# EUROPEAN PATENT SPECIFICATION

(11) **EP 3 893 645 B1**
(45) Date of publication and mention of the grant of the patent: **17.04.2024**
(21) Application number: 19895997.5
(22) Date of filing: 11.12.2019
(51) Int. Cl.: C07D 217/24, C07D 401/12, C07D 471/04, A61K 31/472, A61P 31/12

(54) **SUBSTITUTED ARYLMETHYLUREAS AND HETEROARYLMETHYLUREAS, ANALOGUES THEREOF, AND METHODS USING SAME**
SUBSTITUIERTE ARYLMETHYLHARNSTOFFE UND HETEROARYLMETHYLHARNSTOFFE, ANALOG DAVON UND VERFAHREN DAMIT
ARYLMÉTHYLURÉES ET HÉTÉROARYLMÉTHYLURÉES SUBSTITUÉES, ANALOGUES DE CES DERNIÈRES ET PROCÉDÉS D'UTILISATION DE CELLES-CI

(30) Priority: 12.12.2018 US 201862778471 P; 05.09.2019 US 201962896237 P
(43) Date of publication of application: 20.10.2021
(73) Proprietor: Arbutus Biopharma Corporation, Vancouver, BC V7Y 1B3 (CA)
(72) Inventor: COLE, Andrew G., Cranbury, New Jersey 08512 (US); FAN, Yi, Doylestown, Pennsylvania 18901 (US); KULTGEN, Steven, Warminster, Pennsylvania 18974 (US); MESAROS, Eugen, Wallingford, Pennsylvania 19086 (US)
(74) Representative: Grünecker Patent- und Rechtsanwälte PartG mbB
(86) International application number: PCT/US2019/065756
(87) International publication number: WO 2020/123674

(56) References cited:
- US-A- 4 623 662
- US-A- 5 527 811
- US-A1- 2013 096 119
- US-A1- 2018 016 243
- US-B1- 6 211 373
- DATABASE Pubchem 29 April 2006 (2006-04-29), "1-(3-Chloro-4-fluorophenyl)-3-(pyridin-3- ylmethyl)urea | C13H11ClFN3O", XP055718524,

## Description

### CROSS-REFERENCE TO RELATED APPLICATION

The present application claims priority under 35 U.S.C. § 119(e) to U.S. Provisional Patent Application No. 62/778,471, filed December 12, 2018, and U.S. Provisional Patent Application No. 62/896,237, filed September 5, 2019.

### BACKGROUND OF THE INVENTION

Hepatitis B is one of the world's most prevalent diseases, being listed by National Institute of Allergy and Infectious Diseases (NIAID) as a High Priority Area of Interest. Although most individuals resolve the infection following acute symptoms, approximately 30% of cases become chronic. 350-400 million people worldwide are estimated to have chronic hepatitis B, leading to 0.5-1 million deaths per year, due largely to the development of hepatocellular carcinoma, cirrhosis and/or other complications.

A limited number of drugs are currently approved for the management of chronic hepatitis B, including two formulations of alpha-interferon (standard and pegylated) and five nucleoside/nucleotide analogues (lamivudine, adefovir, entecavir, telbivudine, and tenofovir) that inhibit hepatitis B virus (HBV) DNA polymerase. At present, the first-line treatment choices are entecavir, tenofovir and/or peg-interferon alfa-2a. However, peg-interferon alfa-2a achieves desirable serological milestones in only one third of treated patients, and is frequently associated with severe side effects. Entecavir and tenofovir are potent HBV inhibitors, but require long-term or possibly lifetime administration to continuously suppress HBV replication, and may eventually fail due to emergence of drug-resistant viruses. There is thus a pressing need for the introduction of novel, safe, and effective therapies for chronic hepatitis B.

HBV is a noncytopathic, liver tropic DNA virus belonging to *Hepadnaviridae* family. Pregenomic (pg) RNA is the template for reverse transcriptional replication of HBV DNA. The encapsidation of pg RNA, together with viral DNA polymerase, into a nucleocapsid is essential for the subsequent viral DNA synthesis. Inhibition of pg RNA encapsidation may block HBV replication and provide a new therapeutic approach to HBV treatment. A capsid inhibitor acts by inhibiting the expression and/or function of a capsid protein either directly or indirectly: for example, it may inhibit capsid assembly, induce formation of non-capsid polymers, promote excess capsid assembly or misdirected capsid assembly, affect capsid stabilization, and/or inhibit RNA encapsidation. A capsid inhibitor may also act by inhibiting capsid function in one or more downstream events within the replication process, such as, but not limited to, viral DNA synthesis, transport of relaxed circular DNA (rcDNA) into the nucleus, covalently closed circular DNA (cccDNA) formation, virus maturation, budding and/or release.

Clinically, inhibition of pg RNA encapsidation, or more generally inhibition of nucleocapsid assembly, may offer certain therapeutic advantages. In one aspect, inhibition of pg RNA encapsidation may complement the current medications by providing an option for a subpopulation of patients that do not tolerate or benefit from the current medications. In another aspect, based on their distinct antiviral mechanism, inhibition of pg RNA encapsidation may be effective against HBV variants resistant to the currently available DNA polymerase inhibitors. In yet another aspect, combination therapy of the pg RNA encapsidation inhibitors with DNA polymerase inhibitors may synergistically suppress HBV replication and prevent drug resistance emergence, thus offering a more effective treatment for chronic hepatitis B infection.

Hepatitis D virus (HDV) is a small circular enveloped RNA virus that can propagate only in the presence of HBV. In particular, HDV requires the HBV surface antigen protein to propagate itself. Infection with both HBV and HDV results in more severe complications compared to infection with HBV alone. These complications include a greater likelihood of experiencing liver failure in acute infections and a rapid progression to liver cirrhosis, with an increased chance of developing liver cancer in chronic infections. In combination with hepatitis B, hepatitis D has the highest mortality rate of all the hepatitis infections. The routes of transmission of HDV are similar to those for HBV. Infection is largely restricted to persons at high risk of HBV infection, particularly injecting drug users and persons receiving clotting factor concentrates.

Currently, there is no effective antiviral therapy available for the treatment of acute or chronic type D hepatitis. Interferon-alfa given weekly for 12 to 18 months is the only licensed treatment for hepatitis D. Response to this therapy is limited, as only about one-quarter of patients is serum HDV RNA undetectable 6 months post therapy.

Clinically, inhibition of pg RNA encapsidation, or more generally inhibition of nucleocapsid assembly, may offer certain therapeutic advantages for treatment of hepatitis B and/or hepatitis D. In one aspect, inhibition of pg RNA encapsidation may complement the current medications by providing an option for a subpopulation of patients that do not tolerate or benefit from the current medications. In another aspect, based on their distinct antiviral mechanism, inhibition of pg RNA encapsidation may be effective against HBV and/or HDV variants resistant to the currently available DNA polymerase inhibitors. In yet another aspect, combination therapy of the pg RNA encapsidation inhibitors with DNA polymerase inhibitors may synergistically suppress HBV and/or HDV replication and prevent drug resistance emergence, thus offering a more effective treatment for chronic hepatitis B and/or hepatis D infection.

There is thus a need in the art for the identification of novel compounds that can be used to treat and/or prevent HBV and/or HDV infection in a subject. In certain embodiments, the novel compounds inhibit HBV and/or HDV nucleocapsid assembly. In other embodiments, the novel compounds can be used in patients that are HBV and/or HBV-HDV infected, patients who are at risk of becoming HBV and/or HBV-HDV infected, and/or patients that are infected with drug-resistant HBV and/or HDV. The present invention addresses this need.

### BRIEF SUMMARY OF THE INVENTION

The invention provides a compound of formula (I), or a salt, solvate, stereoisomer, tautomer, or isotopically labelled derivative thereof, or any mixtures thereof: 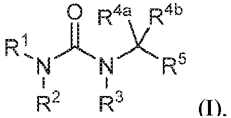 wherein R¹, R², R³, R^{4a}, R^{4b}, and R⁵ are defined elsewhere herein. The invention further provides a pharmaceutical composition comprising at least one compound described herein and a pharmaceutically acceptable carrier. The invention further provides a method of treating or preventing hepatitis B virus (HBV) infection in a subject. The invention further provides a method of inhibiting expression and/or function of a viral capsid protein directly or indirectly in a heptatis B virus-infected subject. In certain embodiments, the method comprises administering to the subject in need thereof a therapeutically effective amount of at least one compound described herein. The methods referred to in the description as part of the invention are to be interpreted as referring to compounds of formula (I) for use in said methods.

### BRIEF DESCRIPTION OF THE FIGURES

The drawings illustrate generally, by way of example, but not by way of limitation, various embodiments of the present invention.

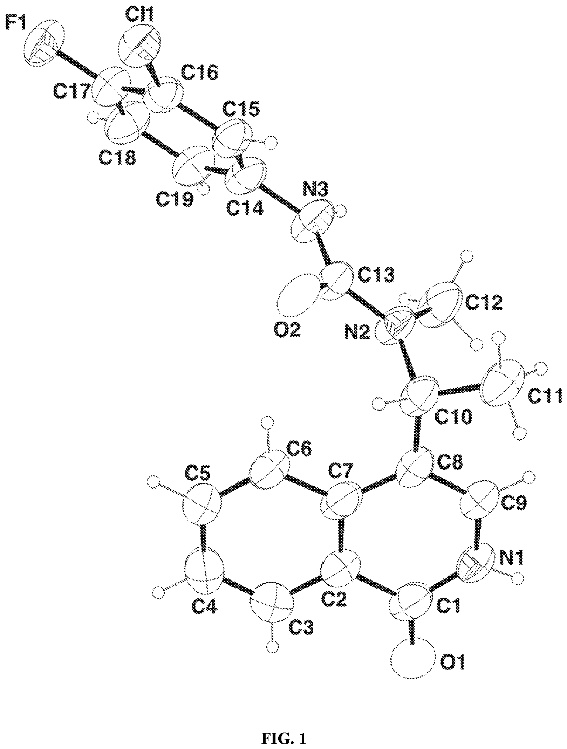

FIG. 1 is an ORTEP representation of (R)-3-(3-chloro-4-fluorophenyl)-1-methyl-1-(1-(1-oxo-1,2-dihydroisoquinolin-4-yl)ethyl)urea **(Compound 14**).

### DETAILED DESCRIPTION OF THE INVENTION

The invention relates, in certain aspects, to the discovery of certain substituted urea-containing compounds that are useful to treat and/or prevent hepatitis B virus (HBV) and/or hepatitis D virus (HDV) infection and related conditions in a subject. In certain embodiments, the compounds of the invention are viral capsid inhibitors.

### Definitions

As used herein, each of the following terms has the meaning associated with it in this section. Unless defined otherwise, all technical and scientific terms used herein generally have the same meaning as commonly understood by one of ordinary skill in the art to which this invention belongs. Generally, the nomenclature used herein and the laboratory procedures in animal pharmacology, pharmaceutical science, separation science, and organic chemistry are those well-known and commonly employed in the art. It should be understood that the order of steps or order for performing certain actions is immaterial, so long as the present teachings remain operable. Any use of section headings is intended to aid reading of the document and is not to be interpreted as limiting; information that is relevant to a section heading may occur within or outside of that particular section. All publications, patents, and patent documents referred to in this document are incorporated by reference herein in their entirety, as though individually incorporated by reference.

In the application, where an element or component is said to be included in and/or selected from a list of recited elements or components, it should be understood that the element or component can be any one of the recited elements or components and can be selected from a group consisting of two or more of the recited elements or components.

In the methods described herein, the acts can be carried out in any order, except when a temporal or operational sequence is explicitly recited. Furthermore, specified acts can be carried out concurrently unless explicit claim language recites that they be carried out separately. For example, a claimed act of doing X and a claimed act of doing Y can be conducted simultaneously within a single operation, and the resulting process will fall within the literal scope of the claimed process.

In this document, the terms "a," "an," or "the" are used to include one or more than one unless the context clearly dictates otherwise. The term "or" is used to refer to a nonexclusive "or" unless otherwise indicated. The statement "at least one of A and B" or "at least one of A or B" has the same meaning as "A, B, or A and B."

As used herein, the term "about" will be understood by persons of ordinary skill in the art and will vary to some extent on the context in which it is used. As used herein, "about" when referring to a measurable value such as an amount, a temporal duration, and the like, is meant to encompass variations of ±20%, ±10%, ±5%, ±1%, or ±0.1% from the specified value, as such variations are appropriate to perform the disclosed methods.

As used herein, the term "alkenyl," employed alone or in combination with other terms, means, unless otherwise stated, a stable monounsaturated or diunsaturated straight chain or branched chain hydrocarbon group having the stated number of carbon atoms. Examples include vinyl, propenyl (or allyl), crotyl, isopentenyl, butadienyl, 1,3-pentadienyl, 1,4-pentadienyl, and the higher homologs and isomers. A functional group representing an alkene is exemplified by -CH₂-CH=CH₂.

As used herein, the term "alkoxy" employed alone or in combination with other terms means, unless otherwise stated, an alkyl group having the designated number of carbon atoms, as defined elsewhere herein, connected to the rest of the molecule via an oxygen atom, such as, for example, methoxy, ethoxy, 1-propoxy, 2-propoxy (or isopropoxy) and the higher homologs and isomers. A specific example is (C₁-C₃)alkoxy, such as, but not limited to, ethoxy and methoxy.

As used herein, the term "alkyl" by itself or as part of another substituent means, unless otherwise stated, a straight or branched chain hydrocarbon having the number of carbon atoms designated *(i.e.,* C₁-C₁₀ means one to ten carbon atoms) and includes straight, branched chain, or cyclic substituent groups. Examples include methyl, ethyl, propyl, isopropyl, butyl, isobutyl, tert-butyl, pentyl, neopentyl, hexyl, and cyclopropylmethyl. A specific embodiment is (C₁-C₆)alkyl, such as, but not limited to, ethyl, methyl, isopropyl, isobutyl, *n*-pentyl, n-hexyl, and cyclopropylmethyl.

As used herein, the term "alkynyl" employed alone or in combination with other terms means, unless otherwise stated, a stable straight chain or branched chain hydrocarbon group with a triple carbon-carbon bond, having the stated number of carbon atoms. Non-limiting examples include ethynyl and propynyl, and the higher homologs and isomers. The term "propargylic" refers to a group exemplified by -CH₂-C≡CH. The term "homopropargylic" refers to a group exemplified by -CH₂CH₂-C≡CH.

As used herein, the term "aromatic" refers to a carbocycle or heterocycle with one or more polyunsaturated rings and having aromatic character, *i.e.,* having (4n+2) delocalized π (pi) electrons, where 'n' is an integer.

As used herein, the term "aryl" employed alone or in combination with other terms means, unless otherwise stated, a carbocyclic aromatic system containing one or more rings (typically one, two or three rings) wherein such rings may be attached together in a pendent manner, such as a biphenyl, or may be fused, such as naphthalene. Examples include phenyl, anthracyl and naphthyl. Aryl groups also include, for example, phenyl or naphthyl rings fused with one or more saturated or partially saturated carbon rings (*e.g*., bicyclo[4.2.0]octa-1,3,5-trienyl, or indanyl), which can be substituted at one or more carbon atoms of the aromatic and/or saturated or partially saturated rings.

As used herein, the term "aryl-(C₁-C₆)alkyl" refers to a functional group wherein a one-to-six carbon alkylene chain is attached to an aryl group, e.g., -CH₂CH₂-phenyl or -CH₂-phenyl (or benzyl). Specific examples are aryl-CH₂- and aryl-CH(CH₃)-. The term "substituted aryl-(C₁-C₆)alkyl" refers to an aryl-(C₁-C₆)alkyl functional group in which the aryl group is substituted. A specific example is substituted aryl(CH₂)-. Similarly, the term "heteroaryl-(C₁-C₆)alkyl" refers to a functional group wherein a one-to-three carbon alkylene chain is attached to a heteroaryl group, *e.g.,* -CH₂CH₂-pyridyl. A specific example is heteroaryl-(CH₂)-. The term "substituted heteroaryl-(C₁-C₆)alkyl" refers to a heteroaryl-(C₁-C₆)alkyl functional group in which the heteroaryl group is substituted. A specific example is substituted heteroaryl-(CH₂)-.

In one aspect, the terms "co-administered" and "co-administration" as relating to a subject refer to administering to the subject a compound and/or composition of the invention along with a compound and/or composition that may also treat or prevent a disease or disorder contemplated herein. In certain embodiments, the co-administered compounds and/or compositions are administered separately, or in any kind of combination as part of a single therapeutic approach. The co-administered compound and/or composition may be formulated in any kind of combinations as mixtures of solids and liquids under a variety of solid, gel, and liquid formulations, and as a solution.

As used herein, the term "cycloalkyl" by itself or as part of another substituent refers to, unless otherwise stated, a cyclic chain hydrocarbon having the number of carbon atoms designated (*i.e*., C₃-C₆ refers to a cyclic group comprising a ring group consisting of three to six carbon atoms) and includes straight, branched chain or cyclic substituent groups. Examples of (C₃-C₆)cycloalkyl groups are cyclopropyl, cyclobutyl, cyclopentyl and cyclohexyl. Cycloalkyl rings can be optionally substituted. Non-limiting examples of cycloalkyl groups include: cyclopropyl, 2-methyl-cyclopropyl, cyclopropenyl, cyclobutyl, 2,3-dihydroxycyclobutyl, cyclobutenyl, cyclopentyl, cyclopentenyl, cyclopentadienyl, cyclohexyl, cyclohexenyl, cycloheptyl, cyclooctanyl, decalinyl, 2,5-dimethylcyclopentyl, 3,5-dichlorocyclohexyl, 4-hydroxycyclohexyl, 3,3,5-trimethylcyclohex-1-yl, octahydropentalenyl, octahydro-1*H*-indenyl, 3a,4,5,6,7,7a-hexahydro-3*H*-inden-4-yl, decahydroazulenyl; bicyclo[6.2.0]decanyl, decahydronaphthalenyl, and dodecahydro-1*H-*fluorenyl. The term "cycloalkyl" also includes bicyclic hydrocarbon rings, non-limiting examples of which include, bicyclo[2.1.1]hexanyl, bicyclo[2.2.1]heptanyl, bicyclo[3.1.1]heptanyl, 1,3-dimethyl[2.2.1]heptan-2-yl, bicyclo[2.2.2]octanyl, and bicyclo[3.3.3]undecanyl.

As used herein, a "disease" is a state of health of a subject wherein the subject cannot maintain homeostasis, and wherein if the disease is not ameliorated then the subject's health continues to deteriorate.

As used herein, a "disorder" in a subject is a state of health in which the subject is able to maintain homeostasis, but in which the subject's state of health is less favorable than it would be in the absence of the disorder. Left untreated, a disorder does not necessarily cause a further decrease in the subject's state of health.

As used herein, the term "halide" refers to a halogen atom bearing a negative charge. The halide anions are fluoride (F⁻), chloride (Cl⁻), bromide (Br⁻), and iodide (I⁻).

As used herein, the term "halo" or "halogen" alone or as part of another substituent refers to, unless otherwise stated, a fluorine, chlorine, bromine, or iodine atom.

As used herein, the term "heteroalkenyl" by itself or in combination with another term refers to, unless otherwise stated, a stable straight or branched chain monounsaturated or diunsaturated hydrocarbon group consisting of the stated number of carbon atoms and one or two heteroatoms selected from the group consisting of O, N, and S, and wherein the nitrogen and sulfur atoms may optionally be oxidized and the nitrogen heteroatom may optionally be quaternized. Up to two heteroatoms may be placed consecutively. Examples include - CH=CH-O-CH₃, -CH=CH-CH₂-OH, -CH₂-CH=N-OCH₃, -CH=CH-N(CH₃)-CH₃, and -CH₂-CH=CH-CH₂-SH.

As used herein, the term "heteroalkyl" by itself or in combination with another term refers to, unless otherwise stated, a stable straight or branched chain alkyl group consisting of the stated number of carbon atoms and one or two heteroatoms selected from the group consisting of O, N, and S, and wherein the nitrogen and sulfur atoms may be optionally oxidized and the nitrogen heteroatom may be optionally quatemized. The heteroatom(s) may be placed at any position of the heteroalkyl group, including between the rest of the heteroalkyl group and the fragment to which it is attached, as well as attached to the most distal carbon atom in the heteroalkyl group. Examples include: -OCH₂CH₂CH₃, - CH₂CH₂CH₂OH, -CH₂CH₂NHCH₃, -CH₂SCH₂CH₃, and -CH₂CH₂S(=O)CH₃. Up to two heteroatoms may be consecutive, such as, for example, -CH₂NH-OCH₃, or -CH₂CH₂SSCH₃.

As used herein, the term "heteroaryl" or "heteroaromatic" refers to a heterocycle having aromatic character. A polycyclic heteroaryl may include one or more rings that are partially saturated. Examples include tetrahydroquinoline and 2,3-dihydrobenzofuryl.

As used herein, the term "heterocycle" or "heterocyclyl" or "heterocyclic" by itself or as part of another substituent refers to, unless otherwise stated, an unsubstituted or substituted, stable, mono- or multi-cyclic heterocyclic ring system that comprises carbon atoms and at least one heteroatom selected from the group consisting of N, O, and S, and wherein the nitrogen and sulfur heteroatoms may be optionally oxidized, and the nitrogen atom may be optionally quaternized. The heterocyclic system may be attached, unless otherwise stated, at any heteroatom or carbon atom that affords a stable structure. A heterocycle may be aromatic or non-aromatic in nature. In certain embodiments, the heterocycle is a heteroaryl.

Examples of non-aromatic heterocycles include monocyclic groups such as aziridine, oxirane, thiirane, azetidine, oxetane, thietane, pyrrolidine, pyrroline, imidazoline, pyrazolidine, dioxolane, sulfolane, 2,3-dihydrofuran, 2,5-dihydrofuran, tetrahydrofuran, thiophane, piperidine, 1,2,3,6-tetrahydropyridine, 1,4-dihydropyridine, piperazine, morpholine, thiomorpholine, pyran, 2,3-dihydropyran, tetrahydropyran, 1,4-dioxane, 1,3-dioxane, homopiperazine, homopiperidine, 1,3-dioxepane, 4,7-dihydro-1,3-dioxepin, and hexamethyleneoxide.

Examples of heteroaryl groups include pyridyl, pyrazinyl, pyrimidinyl (such as, but not limited to, 2- and 4-pyrimidinyl), pyridazinyl, thienyl, furyl, pyrrolyl, imidazolyl, thiazolyl, oxazolyl, pyrazolyl, isothiazolyl, 1,2,3-triazolyl, 1,2,4-triazolyl, 1,3,4-triazolyl, tetrazolyl, 1,2,3-thiadiazolyl, 1,2,3-oxadiazolyl, 1,3,4-thiadiazolyl, and 1,3,4-oxadiazolyl.

Examples of polycyclic heterocycles include indolyl (such as, but not limited to, 2-, 3-, 4-, 5-, 6- and 7-indolyl), indolinyl, quinolyl, tetrahydroquinolyl, isoquinolyl (such as, but not limited to, 1- and 5-isoquinolyl), 1,2,3,4-tetrahydroisoquinolyl, cinnolinyl, quinoxalinyl (such as, but not limited to, 2- and 5-quinoxalinyl), quinazolinyl, phthalazinyl, 1,8-naphthyridinyl, 1,4-benzodioxanyl, coumarin, dihydrocoumarin, 1,5-naphthyridinyl, benzofuryl (such as, but not limited to, 3-, 4-, 5-, 6- and 7-benzofuryl), 2,3-dihydrobenzofuryl, 1,2-benzisoxazolyl, benzothienyl (such as, but not limited to, 3-, 4-, 5-, 6-, and 7-benzothienyl), benzoxazolyl, benzothiazolyl (such as, but not limited to, 2-benzothiazolyl and 5-benzothiazolyl), purinyl, benzimidazolyl, benztriazolyl, thioxanthinyl, carbazolyl, carbolinyl, acridinyl, pyrrolizidinyl, and quinolizidinyl.

The aforementioned listing of heterocyclyl and heteroaryl moieties is intended to be representative and not limiting.

As used herein, the term "pharmaceutical composition" or "composition" refers to a mixture of at least one compound useful within the invention with a pharmaceutically acceptable carrier. The pharmaceutical composition facilitates administration of the compound to a subject.

As used herein, the term "pharmaceutically acceptable" refers to a material, such as a carrier or diluent, which does not abrogate the biological activity or properties of the compound useful within the invention, and is relatively non-toxic, *i.e*., the material may be administered to a subject without causing undesirable biological effects or interacting in a deleterious manner with any of the components of the composition in which it is contained.

As used herein, the term "pharmaceutically acceptable carrier" means a pharmaceutically acceptable material, composition or carrier, such as a liquid or solid filler, stabilizer, dispersing agent, suspending agent, diluent, excipient, thickening agent, solvent or encapsulating material, involved in carrying or transporting a compound useful within the invention within or to the subject such that it may perform its intended function. Typically, such constructs are carried or transported from one organ, or portion of the body, to another organ, or portion of the body. Each carrier must be "acceptable" in the sense of being compatible with the other ingredients of the formulation, including the compound useful within the invention, and not injurious to the subject. Some examples of materials that may serve as pharmaceutically acceptable carriers include: sugars, such as lactose, glucose and sucrose; starches, such as corn starch and potato starch; cellulose, and its derivatives, such as sodium carboxymethyl cellulose, ethyl cellulose and cellulose acetate; powdered tragacanth; malt; gelatin; talc; excipients, such as cocoa butter and suppository waxes; oils, such as peanut oil, cottonseed oil, safflower oil, sesame oil, olive oil, corn oil and soybean oil; glycols, such as propylene glycol; polyols, such as glycerin, sorbitol, mannitol and polyethylene glycol; esters, such as ethyl oleate and ethyl laurate; agar; buffering agents, such as magnesium hydroxide and aluminum hydroxide; surface active agents; alginic acid; pyrogen-free water; isotonic saline; Ringer's solution; ethyl alcohol; phosphate buffer solutions; and other non-toxic compatible substances employed in pharmaceutical formulations. As used herein, "pharmaceutically acceptable carrier" also includes any and all coatings, antibacterial and antifungal agents, and absorption delaying agents, and the like that are compatible with the activity of the compound useful within the invention, and are physiologically acceptable to the subject. Supplementary active compounds may also be incorporated into the compositions. The "pharmaceutically acceptable carrier" may further include a pharmaceutically acceptable salt of the compound useful within the invention. Other additional ingredients that may be included in the pharmaceutical compositions used in the practice of the invention are known in the art and described, for example in Remington's Pharmaceutical Sciences (Genaro, Ed., Mack Publishing Co., 1985, Easton, PA), which is incorporated herein by reference.

As used herein, the language "pharmaceutically acceptable salt" refers to a salt of the administered compound prepared from pharmaceutically acceptable non-toxic acids and/or bases, including inorganic acids, inorganic bases, organic acids, inorganic bases, solvates (including hydrates) and clathrates thereof.

As used herein, a "pharmaceutically effective amount," "therapeutically effective amount," or "effective amount" of a compound is that amount of compound that is sufficient to provide a beneficial effect to the subject to which the compound is administered.

The term "prevent," "preventing," or "prevention" as used herein means avoiding or delaying the onset of symptoms associated with a disease or condition in a subject that has not developed such symptoms at the time the administering of an agent or compound commences. Disease, condition and disorder are used interchangeably herein.

By the term "specifically bind" or "specifically binds" as used herein is meant that a first molecule preferentially binds to a second molecule (*e.g*., a particular receptor or enzyme), but does not necessarily bind only to that second molecule.

As used herein, the terms "subject" and "individual" and "patient" can be used interchangeably and may refer to a human or non-human mammal or a bird. Non-human mammals include, for example, livestock and pets, such as ovine, bovine, porcine, canine, feline and murine mammals. In certain embodiments, the subject is human.

As used herein, the term "substituted" refers to that an atom or group of atoms has replaced hydrogen as the substituent attached to another group.

As used herein, the term "substituted alkyl," "substituted cycloalkyl," "substituted alkenyl," or "substituted alkynyl" refers to alkyl, cycloalkyl, alkenyl, or alkynyl, as defined elsewhere herein, substituted by one, two or three substituents independently selected from the group consisting of halogen, -OH, alkoxy, tetrahydro-2-H-pyranyl, -NH₂, -NH(C₁-C₆ alkyl), -N(C₁-C₆ alkyl)₂, 1-methyl-imidazol-2-yl, pyridin-2-yl, pyridin-3-yl, pyridin-4-yl,-C(=O)OH, -C(=O)O(C₁-C₆)alkyl, trifluoromethyl, -C=N, -C(=O)NH₂, -C(=O)NH(C₁-C₆)alkyl, -C(=O)N((C₁-C₆)alkyl)₂, -SO₂NH₂, -SO₂NH(C₁-C₆ alkyl), -SO₂N(C₁-C₆ alkyl)₂,-C(=NH)NH₂, and -NO₂, in certain embodiments containing one or two substituents independently selected from halogen, -OH, alkoxy, -NH₂, trifluoromethyl, -N(CH₃)₂, and-C(=O)OH, in certain embodiments independently selected from halogen, alkoxy and -OH. Examples of substituted alkyls include, but are not limited to, 2,2-difluoropropyl, 2-carboxycyclopentyl and 3-chloropropyl.

For aryl, aryl-(C₁-C₃)alkyl and heterocyclyl groups, the term "substituted" as applied to the rings of these groups refers to any level of substitution, namely mono-, di-, tri-, tetra-, or penta-substitution, where such substitution is permitted. The substituents are independently selected, and substitution may be at any chemically accessible position. In certain embodiments, the substituents vary in number between one and four. In other embodiments, the substituents vary in number between one and three. In yet another embodiments, the substituents vary in number between one and two. In yet other embodiments, the substituents are independently selected from the group consisting of C₁-C₆ alkyl, -OH, C₁-C₆ alkoxy, halogen, amino, acetamido and nitro. As used herein, where a substituent is an alkyl or alkoxy group, the carbon chain may be branched, straight or cyclic.

Unless otherwise noted, when two substituents are taken together to form a ring having a specified number of ring atoms (*e.g*., R² and R³ taken together with the nitrogen to which they are attached to form a ring having from 3 to 7 ring members), the ring can have carbon atoms and optionally one or more (*e.g*., 1 to 3) additional heteroatoms independently selected from nitrogen, oxygen, or sulfur. The ring can be saturated or partially saturated, and can be optionally substituted.

Whenever a term or either of their prefix roots appear in a name of a substituent the name is to be interpreted as including those limitations provided herein. For example, whenever the term "alkyl" or "aryl" or either of their prefix roots appear in a name of a substituent (*e.g*., arylalkyl, alkylamino) the name is to be interpreted as including those limitations given elsewhere herein for "alkyl" and "aryl" respectively.

In certain embodiments, substituents of compounds are disclosed in groups or in ranges. It is specifically intended that the description include each and every individual subcombination of the members of such groups and ranges. For example, the term "C₁₋₆ alkyl" is specifically intended to individually disclose C₁, C₂, C₃, C₄, C₅, C₆, C₁-C₆, C₁-C₅, C₁-C₄, C₁-C₃, C₁-C₂, C₂-C₆, C₂-C₅, C₂-C₄, C₂-C₃, C₃-C₆, C₃-C₅, C₃-C₄, C₄-C₆, C₄-C₅, and C₅-C₆ alkyl.

The terms "treat," "treating" and "treatment," as used herein, means reducing the frequency or severity with which symptoms of a disease or condition are experienced by a subject by virtue of administering an agent or compound to the subject.

Certain abbreviations used herein follow: cccDNA, covalently closed circular DNA; DMSO, dimethylsulfoxide; HBsAg, HBV surface antigen; HBV, hepatitis B virus; HDV, hepatitis D virus; HPLC, high pressure liquid chromatography; LCMS, liquid chromatography mass spectrometry; NARTI or NRTI, reverse-transcriptase inhibitor; NMR, Nuclear Magnetic Resonance; NtARTI or NtRTI, nucleotide analog reverse-transcriptase inhibitor; pg RNA, pregenomic RNA; rcDNA, relaxed circular DNA; RT, retention time; sAg, surface antigen; TLC, thin layer chromatography.

Ranges: throughout this disclosure, various aspects of the present invention can be presented in a range format. It should be understood that the description in range format is merely for convenience and brevity and should not be construed as an inflexible limitation on the scope of the present invention. Accordingly, the description of a range should be considered to have specifically disclosed all the possible subranges as well as individual numerical values within that range. For example, description of a range such as from 1 to 6 should be considered to have specifically disclosed subranges such as from 1 to 3, from 1 to 4, from 1 to 5, from 2 to 4, from 2 to 6, from 3 to 6 etc., as well as individual numbers within that range, for example, 1, 2, 2.7, 3, 4, 5, 5.3, and 6. For example, a range of "about 0.1% to about 5%" or "about 0.1% to 5%" should be interpreted to include not just about 0.1% to about 5%, but also the individual values *(e.g.,* 1%, 2%, 3%, and 4%) and the sub-ranges (e.g., 0.1% to 0.5%, 1.1% to 2.2%, 3.3% to 4.4%) within the indicated range. The statement "about X to Y" has the same meaning as "about X to about Y," unless indicated otherwise. Likewise, the statement "about X, Y, or about Z" has the same meaning as "about X, about Y, or about Z," unless indicated otherwise. This applies regardless of the breadth of the range.

### Compounds

The invention includes a compound of formula (I), or a salt, solvate, isotopically labelled derivative, stereoisomer (such as, in a non-limiting example, an enantiomer or diastereoisomer, and/or any mixtures thereof, such as, in a non-limiting example, mixtures in any proportions of enantiomers and/or diastereoisomers thereof), tautomer and any mixtures thereof, and/or geometric isomer and any mixtures thereof: 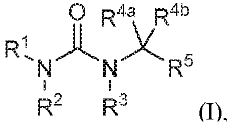 wherein in (I):
R¹ is selected from the group consisting of optionally substituted C₃-C₈ cycloalkyl, optionally substituted phenyl, optionally substituted benzyl, optionally substituted heteroaryl, and -(CH₂)(optionally substituted heteroaryl);
each occurrence of R² is independently selected from the group consisting of H and C₁-C₆ alkyl;
R³ is selected from the group consisting of C₁-C₆ alkyl, and C₃-C₈ cycloalkyl, wherein the alkyl or cycloalkyl is optionally substituted with at least one substituent selected from the group consisting of C₁-C₆ alkyl, C₃-C₈ cycloalkyl, halogen, cyano, -OH, C₁-C₆ alkoxy, C₃-C₈ cycloalkoxy, C₁-C₆ haloalkoxy, C₃-C₈ halocycloalkoxy, optionally substituted phenyl, optionally substituted heteroaryl, optionally substituted heterocyclyl, -C(=O)OR⁶,-OC(=O)R⁶, -SR⁶, -S(=O)R⁶, -S(=O)₂R⁶, -S(=O)₂NR⁶R⁶, -N(R⁶)S(=O)₂R⁶, -N(R⁶)C(=O)R⁶,-C(=O)NR⁶R⁶, and -NR⁶R⁶;
R^{4a} is selected from the group consisting of H, C₁-C₆ alkyl, C₃-C₈ cycloalkyl, and phenyl, wherein the alkyl, cycloalkyl, or phenyl is optionally substituted with at least one substituent selected from the group consisting of C₁-C₆ alkyl, C₃-C₈ cycloalkyl, halogen, cyano, -OH, C₁-C₆ alkoxy, C₃-C₈ cycloalkoxy, C₁-C₆ haloalkoxy, C₃-C₈ halocycloalkoxy, -NR⁶R⁶, and optionally substituted phenyl;
R^{4b} selected from the group consisting of H and optionally substituted C₁-C₆ alkyl;
R⁵ is selected from the group consisting of: 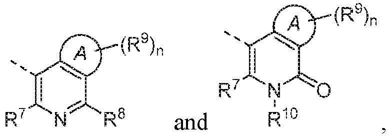 wherein each ring *A* is independently selected from the group consisting of benzene, pyridine, pyrimidine, pyridazine, and pyrazine;
each occurrence of R⁶ is independently selected from the group consisting of H, optionally substituted C₁-C₆ alkyl, optionally substituted C₃-C₈ cycloalkyl, optionally substituted phenyl, and optionally substituted hetereoaryl;
each occurrence of R⁷ is independently selected from the group consisting of H, halogen, optionally substituted C₁-C₆ alkyl, optionally substituted C₃-C₈ cycloalkyl, optionally substituted C₁-C₆ alkoxy, and optionally substituted C₃-C₈ cycloalkoxy;
each occurrence of R⁸ is independently selected from the group consisting of halogen, -CN, optionally substituted C₁-C₆ alkyl, optionally substituted C₃-C₈ cycloalkyl, optionally substituted C₁-C₆ alkoxy, optionally substituted C₃-C₈ cycloalkoxy, heterocyclyl, heteroaryl,-S(optionally substituted C₁-C₆ alkyl), -SO(optionally substituted C₁-C₆ alkyl),-SO₂(optionally substituted C₁-C₆ alkyl), -C(=O)OH, -C(=O)O(optionally substituted C₁-C₆ alkyl), -C(=O)O(optionally substituted C₃-C₈ cycloalkyl), -O(optionally substituted C₁-C₆ alkyl), -O(optionally substituted C₃-C₈ cycloalkyl), -NH₂, -NH(optionally substituted C₁-C₆ alkyl), -NH(optionally substituted C₃-C₈ cycloalkyl), -N(optionally substituted C₁-C₆ alkyl)(optionally substituted C₁-C₆ alkyl), -N(optionally substituted C₃-C₈ cycloalkyl)(optionally substituted C₃-C₈ cycloalkyl), -N(optionally substituted C₁-C₆ alkyl)(optionally substituted C₃-C₈ cycloalkyl), -C(=O)NH₂, -C(=O)NH(optionally substituted C₁-C₆ alkyl), -C(=O)NH(optionally substituted C₃-C₈ cycloalkyl),-C(=O)N(optionally substituted C₁-C₆ alkyl)(optionally substituted C₁-C₆ alkyl),-C(=O)N(optionally substituted C₃-C₈ cycloalkyl)(optionally substituted C₃-C₈ cycloalkyl), and -C(=O)N(optionally substituted C₁-C₆ alkyl)(optionally substituted C₃-C₈ cycloalkyl;
each occurrence of R⁹ is independently selected from the group consisting of H, halogen, optionally substituted C₁-C₆ alkyl, optionally substituted C₃-C₈ cycloalkyl, optionally substituted C₁-C₆ alkoxy, and optionally substituted C₃-C₈ cycloalkoxy;
each occurrence of n is independently 0, 1, 2, 3, or 4; and
R¹⁰ is selected from the group consisting of H, optionally substituted C₁-C₆ alkyl, and optionally substituted C₃-C₈ cycloalkyl.

In certain embodiments, the compound of formula (I) is a compound of formula (Ia):

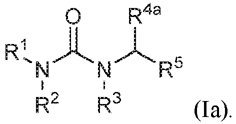

In certain embodiments, the compound of formula (I) is a compound of formula (Ib):

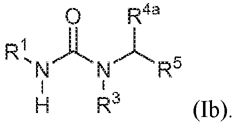

In certain embodiments, the compound of formula (I) is selected from:

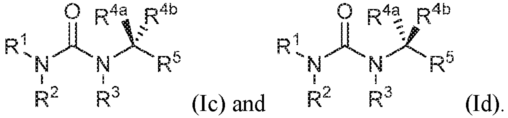

In certain embodiments, the compound of formula (I) is selected from:

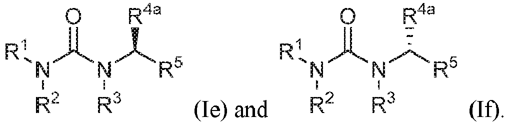

In certain embodiments, the compound of formula (I) is selected from:

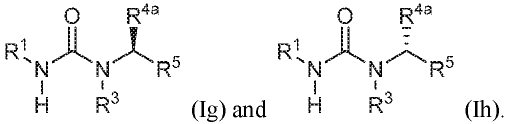

In certain embodiments, R⁵ is selected from:

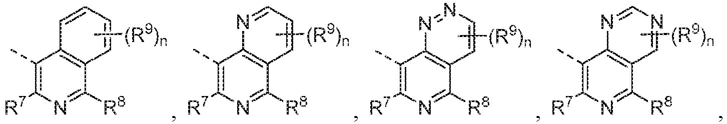

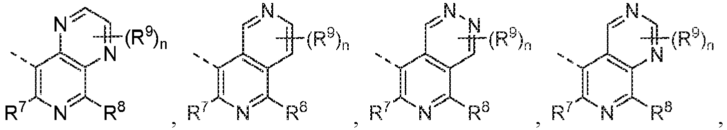

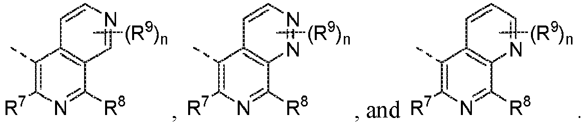

In certain embodiments, R⁵ is selected from:

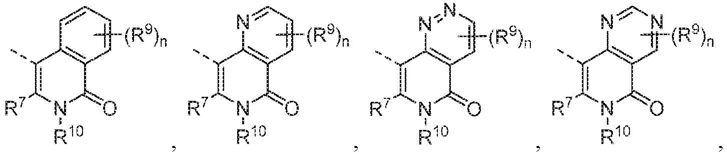

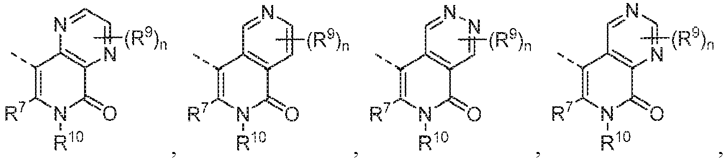

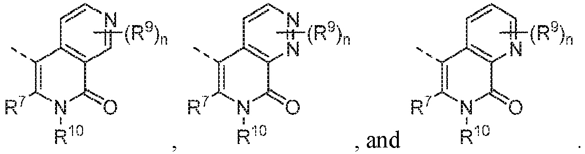

In certain embodiments, R⁵ is 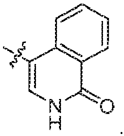 In certain embodiments, R⁵ is 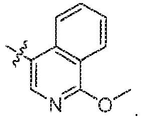 In certain embodiments, R⁵ is 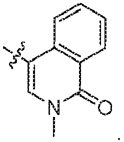 In certain embodiments, R⁵ is 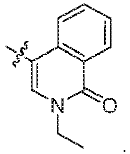 In certain embodiments, R⁵ is 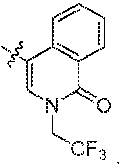 In certain embodiments, R⁵ is 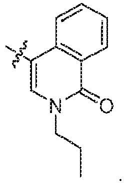 In certain embodiments, R⁵ is 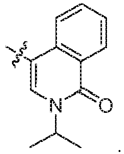 In certain embodiments, R⁵ is 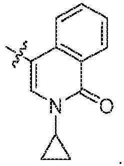 In certain embodiments, R⁵ is 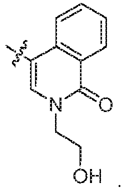 In certain embodiments, R⁵ is 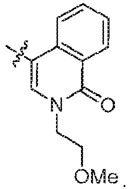 In certain embodiments, R⁵ is 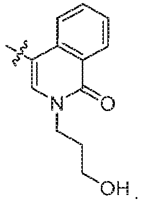 In certain embodiments, R⁵ is 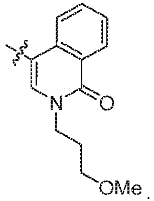 In certain embodiments, R⁵ is 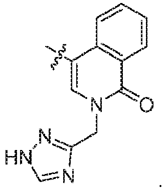 In certain embodiments, R⁵ is 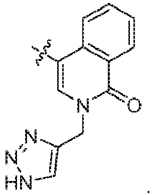 In certain embodiments, R⁵ is 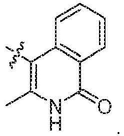 In certain embodiments, R⁵ is 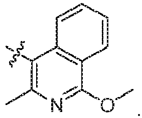 In certain embodiments, R⁵ is 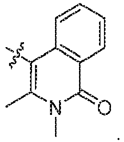 In certain embodiments, R⁵ is 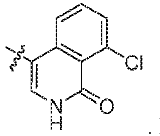 In certain embodiments, R⁵ is . In certain embodiments, R⁵ is 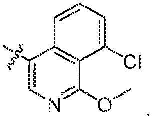 In certain embodiments, R⁵ is 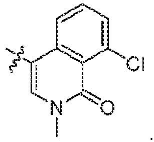 In certain embodiments, R⁵ is 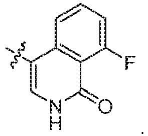 In certain embodiments, R⁵ is 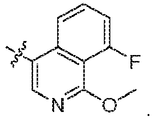 In certain embodiments, R⁵ is 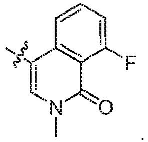 In certain embodiments, R⁵ is 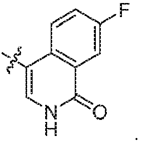 In certain embodiments, R⁵ is 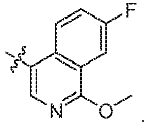 In certain embodiments, R⁵ is 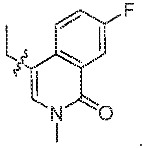 In certain embodiments, R⁵ is 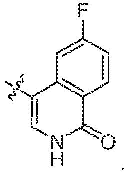 In certain embodiments, R⁵ is 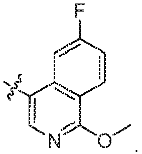 In certain embodiments, R⁵ is 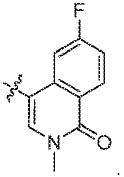 In certain embodiments, R⁵ is 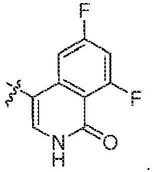 In certain embodiments, R⁵ is 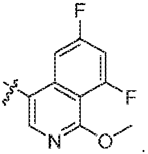 In certain embodiments, R⁵ is 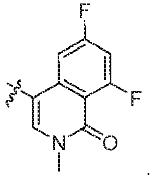 In certain embodiments, R⁵ is 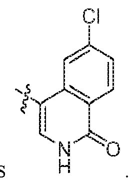 In certain embodiments, R⁵ is 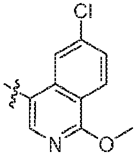 In certain embodiments, R⁵ is 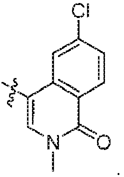 In certain embodiments, R⁵ is 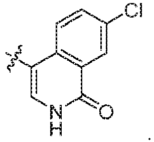 In certain embodiments, R⁵ is 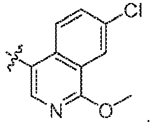 In certain embodiments, R⁵ is 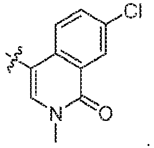 In certain embodiments, R⁵ is 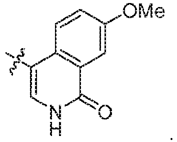 In certain embodiments, R⁵ is 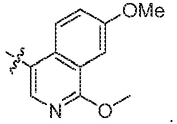 In certain embodiments, R⁵ is 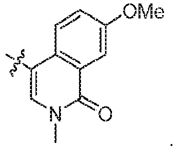 In certain embodiments, R⁵ is 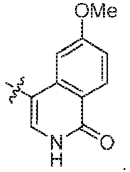 In certain embodiments, R⁵ is 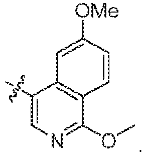 In certain embodiments, R⁵ is 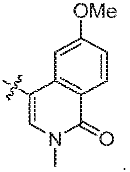 In certain embodiments, R⁵ is 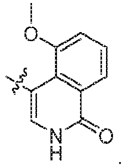 In certain embodiments, R⁵ is 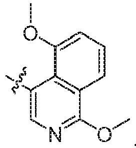 In certain embodiments, R⁵ is 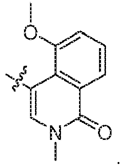 In certain embodiments, R⁵ is 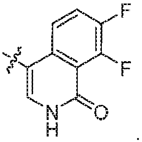 In certain embodiments, R⁵ is 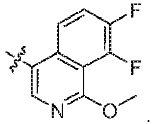 In certain embodiments, R⁵ is 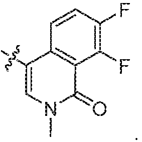 In certain embodiments, R⁵ is 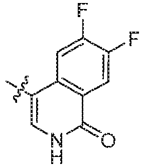 In certain embodiments, R⁵ is 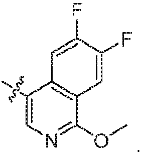 In certain embodiments, R⁵ is 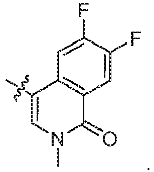 In certain embodiments, R⁵ is 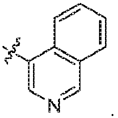 In certain embodiments, R⁵ is 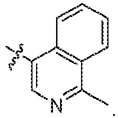 In certain embodiments, R⁵ is 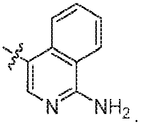 In certain embodiments, R⁵ is 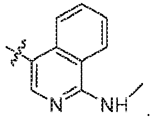 In certain embodiments, R⁵ is 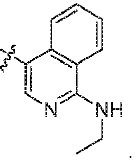 In certain embodiments, R⁵ is 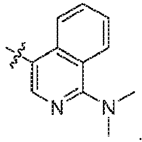 In certain embodiments, R⁵ is 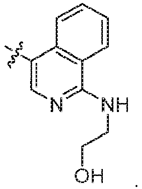 In certain embodiments, R⁵ is 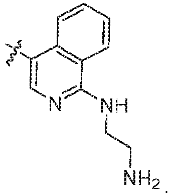 In certain embodiments, R⁵ is 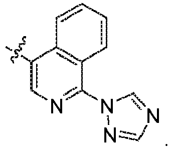 In certain embodiments, R⁵ is 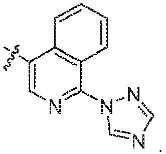 In certain embodiments, R⁵ is 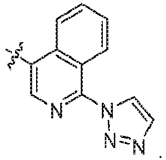 In certain embodiments, R⁵ is 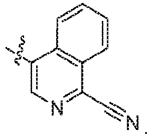 In certain embodiments, R⁵ is 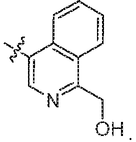 In certain embodiments, R⁵ is 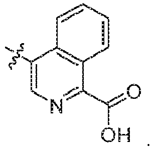 In certain embodiments, R⁵ is 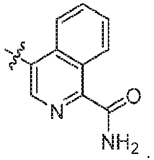 In certain embodiments, R⁵ is 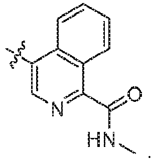 In certain embodiments, R⁵ is 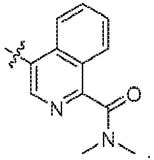 In certain embodiments, R⁵ is 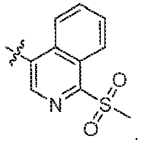 In certain embodiments, R⁵ is 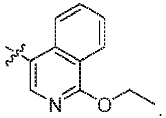 In certain embodiments, R⁵ is 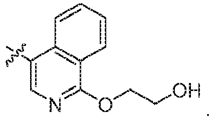 In certain embodiments, R⁵ is 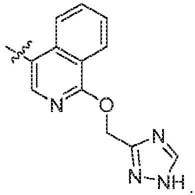 In certain embodiments, R⁵ is 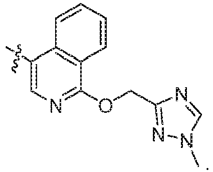 In certain embodiments, R⁵ is 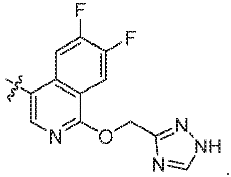 In certain embodiments, R⁵ is 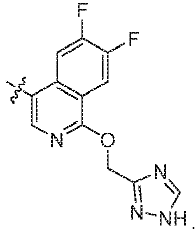 In certain embodiments, R⁵ is 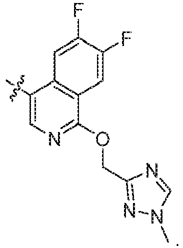 In certain embodiments, R⁵ is 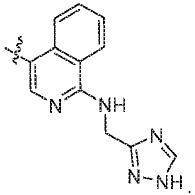 In certain embodiments, R⁵ is 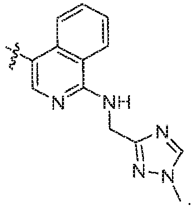 In certain embodiments, R⁵ is 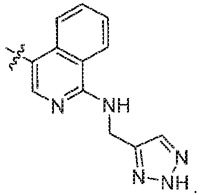 In certain embodiments, R⁵ is 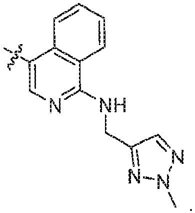 In certain embodiments, R⁵ is 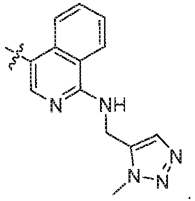 In certain embodiments, R⁵ is 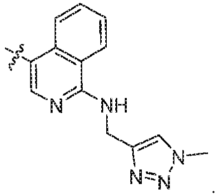 In certain embodiments, R⁵ is 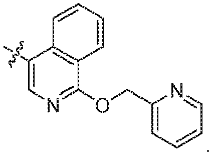 In certain embodiments, R⁵ is 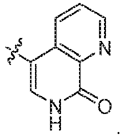 In certain embodiments, R⁵ is 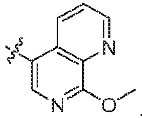 In certain embodiments, R⁵ is 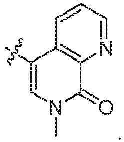 In certain embodiments, R⁵ is 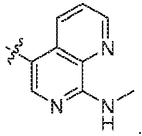 In certain embodiments, R⁵ is 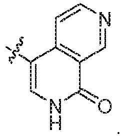 In certain embodiments, R⁵ is 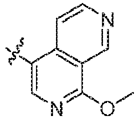 In certain embodiments, R⁵ is 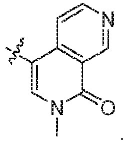 In certain embodiments, R⁵ is 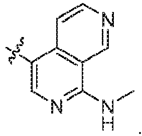 In certain embodiments, R⁵ is 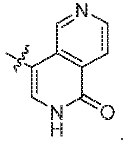 In certain embodiments, R⁵ is 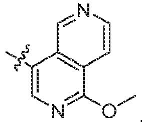 In certain embodiments, R⁵ is 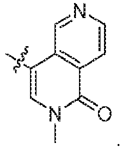 In certain embodiments, R⁵ is 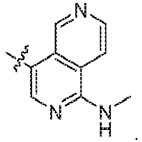 In certain embodiments, R⁵ is 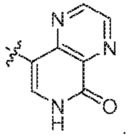 In certain embodiments, R⁵ is 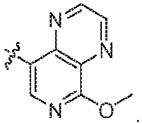 In certain embodiments, R⁵ is 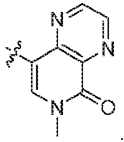 In certain embodiments, R⁵ is

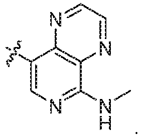

In certain embodiments, each occurrence of alkyl, alkenyl, alkynyl, or cycloalkyl is independently optionally substituted with at least one substituent selected from the group consisting of C₁-C₆ alkyl, C₃-C₈ cycloalkyl, halogen, cyano (-CN), -OR^{a}, optionally substituted phenyl (thus yielding, in non-limiting examples, optionally substituted phenyl-(C₁-C₃ alkyl), such as, but not limited to, benzyl or substituted benzyl), optionally substituted heteroaryl, optionally substituted heterocyclyl, -C(=O)OR^{a}, -OC(=O)R^{a}, -SR^{a}, -S(=O)R^{a},-S(=O)₂R^{a}, -S(=O)₂NR^{a}R^{a}, -N(R^{a})S(=O)₂R^{a}, -N(R^{a})C(=O)R^{a}, -C(=O)NR^{a}R^{a}, and -N(R^{a})(R^{a}), wherein each occurrence of R^{a} is independently H, optionally substituted C₁-C₆ alkyl, optionally substituted C₃-C₈ cycloalkyl, optionally substituted aryl, or optionally substituted heteroaryl, or two R^{a} groups combine with the N to which they are bound to form a heterocycle.

In certain embodiments, each occurrence of aryl or heteroaryl is independently optionally substituted with at least one substituent selected from the group consisting of C₁-C₆ alkyl, C₃-C₈ cycloalkyl, phenyl, C₁-C₆ hydroxyalkyl, (C₁-C₆ alkoxy)-C₁-C₆ alkyl, C₁-C₆ haloalkyl, C₁-C₆ haloalkoxy, halogen, -CN, -OR^{b}, -N(R^{b})(R^{b}), -NO₂, -C(=O)N(R^{b})(R^{b}),-C(=O)OR^{b}, -OC(=O)R^{b}, -SR^{b}, -S(=O)R^{b}, -S(=O)₂R^{b}, -N(R^{b})S(=O)₂R^{b}, -S(=O)₂N(R^{b})(R^{b}), acyl, and C₁-C₆ alkoxycarbonyl, wherein each occurrence of R^{b} is independently H, C₁-C₆ alkyl, or C₃-C₈ cycloalkyl, wherein in R^{b} the alkyl or cycloalkyl is optionally substituted with at least one substituent selected from the group consisting of halogen, -OH, C₁-C₆ alkoxy, and heteroaryl; or substituents on two adjacent carbon atoms combine to form -O(CH₂)₁₋₃O-.

In certain embodiments, each occurrence of aryl or heteroaryl is independently optionally substituted with at least one substituent selected from the group consisting of C₁-C₆ alkyl, C₃-C₈ cycloalkyl, phenyl, C₁-C₆ hydroxyalkyl, (C₁-C₆ alkoxy)-C₁-C₆ alkyl, C₁-C₆ haloalkyl, C₁-C₆ haloalkoxy, halogen, -OR^{b}, -C(=O)N(R^{b})(R^{b}), -C(=O)OR^{b}, -OC(=O)R^{b},-SR^{b}, -S(=O)R^{b}, -S(=O)₂R^{b}, and -N(R^{b})S(=O)₂R^{b}, wherein each occurrence of R^{b} is independently H, C₁-C₆ alkyl, or C₃-C₈ cycloalkyl, wherein in R^{b} the alkyl or cycloalkyl is optionally substituted with at least one substituent selected from the group consisting of halogen, -OH, C₁-C₆ alkoxy, and heteroaryl; or substituents on two adjacent carbon atoms combine to form -O(CH₂)₁₋₃O-.

In certain embodiments, the alkyl, alkenyl, alkynyl, cycloalkyl, heteroaryl, heterocyclyl, aryl, or benzyl group is optionally independently substituted with at least one group selected from the group consisting of C₁-C₆ alkyl; C₁-C₆ alkoxy; C₁-C₆ haloalkyl; C₁-C₆ haloalkoxy; -NH₂, -NH(C₁-C₆ alkyl), -N(C₁-C₆ alkyl)(C₁-C₆ alkyl), halogen, -OH; -CN; phenoxy, -NHC(=O)H, -NHC(=O)C₁-C₆ alkyl, -C(=O)NH₂, -C(=O)NHC₁-C₆ alkyl,-C(=O)N(C₁-C₆ alkyl)(C₁-C₆ alkyl), tetrahydropyranyl, morpholinyl, -C(=O)CH₃,-C(=O)CH₂OH, -C(=O)NHCH₃, -C(=O)CH₂OMe, or an *N*-oxide thereof.

In certain embodiments, each occurrence of the heteroaryl is independently selected from the group consisting of quinolinyl, imidazo[1,2-a]pyridyl, pyridyl, pyrimidyl, pyrazinyl, imidazolyl, thiazolyl, pyrazolyl, isoxazolyl, indolyl (such as, but not limited to, 2-, 3-, 4-, 5-, 6- and 7-indolyl), oxadiazolyl (including 1,2,3-, 1,2,4-, 1,2,5-, and 1,3,4-oxadiazole), and triazolyl (such as 1,2,3-triazolyl and 1,2,4-triazolyl).

In certain embodiments, each occurrence of the heterocyclyl group is independently selected from the group consisting of tetrahydrofuranyl, tetrahydropyranyl, piperidinyl, piperazinyl, pyrrolidinyl, morpholinyl, thiomorpholinyl, 1-oxido-thiomorpholinyl, 1,1-dioxido-thiomorpholinyl, oxazolidinyl, azetidinyl, and the corresponding oxo analogues (where a methylene ring group is replaced with a carbonyl) thereof.

In certain embodiments, R¹ is optionally substituted C₃-C₈ cycloalkyl. In other embodiments, R¹ is cyclobutyl. In yet other embodiments, R¹ is cyclopentyl. In yet other embodiments, R¹ is cyclohexyl. In yet other embodiments, R¹ is cycloheptyl.

In certain embodiments, R¹ is selected from the group consisting of optionally substituted phenyl, optionally substituted benzyl, and -(CH₂)(optionally substituted heteroaryl), wherein the phenyl, benzyl, or heteroaryl is optionally substituted with at least one substituent selected from the group consisting of C₁-C₆ alkyl (such as, for example, methyl, ethyl, and isopropyl), halogen (such as, for example, F, Cl, Br, and I), C₁-C₃ haloalkyl (such as, for example, monofluoromethyl, difluoromethyl, and trifluoromethyl), and -CN.

In certain embodiments, R¹ is selected from the group consisting of: benzyl, 1-phenylethyl, 4-fluorophenylmethyl, 3,4-difluorophenylmethyl, 3-chloro-4-fluorophenylmethyl, 3,4,5-trifluorophenylmethyl, phenyl, 2-chlorophenyl, 3-chlorophenyl, 4-chlorophenyl, 2-fluorophenyl, 3-fluorophenyl, 4-fluorophenyl, 2-bromophenyl, 3-bromophenyl, 4-bromophenyl, 3,4-difluorophenyl, 3,5-difluorophenyl, 2,4,5-trifluorophenyl, 3,4,5-trifluorophenyl, 3,5-dichlorophenyl, 3,5-difluorophenyl, 3-chloro-5-fluorophenyl, 3,4-dichlorophenyl, 3-chloro-4-fluorophenyl, 4-chloro-3-fluorophenyl, 4-chloro-3-methylphenyl, 3-chloro-4-methylphenyl, 4-fluoro-3-methylphenyl, 3-fluoro-4-methylphenyl, 4-chloro-3-methoxyphenyl, 3-chloro-4-methoxyphenyl, 4-fluoro-3-methoxyphenyl, 3-fluoro-4-methoxyphenyl, 3-trifluoromethylphenyl, 4-trifluoromethylphenyl, 3-trifluoromethyl-4-fluorophenyl, 4-trifluoromethyl-3-fluorophenyl, 2,3-difluorophenyl, 2,3,4-trifluorophenyl, 3,5-dichloro-4-fluorophenyl, 3-cyanophenyl, 4-cyanophenyl, 3-cyano-4-fluorophenyl, 4-cyano-3-fluorophenyl, 3-difluoromethyl-4-fluorophenyl, 4-difluoromethyl-3-fluorophenyl, benzo[d][1,3]dioxol-5-yl, 2,3-dihydrobenzo[b][1,4]dioxin-6-yl, benzyl, 3-fluorobenzyl, 4-fluorobenzyl, 3-chlorobenzyl, 4-chlorobenzyl, 2-pyridyl, 4-methyl-2-pyridyl, 5-methyl-2-pyridyl, 6-methyl-2-pyridyl, 3-pyridyl, 2-methyl-3-pyridyl, 3-methyl-3-pyridyl, 4-pyridyl, 2-methyl-4-pyridyl, 2-fluoro-4-pyridyl, 2-chloro-4-pyridyl, 2-trifluoromethyl-4-pyridyl, 6-methyl-4-pyridyl, and 1H-indol-6-yl.

In certain embodiments, R¹ is phenyl. In other embodiments, R¹ is benzyl. In yet other embodiments, R¹ is 1-phenylethyl. In yet other embodiments, R¹ is 4-fluorophenylmethyl. In yet other embodiments, R¹ is 3,4-difluorophenylmethyl. In yet other embodiments, R¹ is 3-chloro-4-fluorophenylmethyl. In yet other embodiments, R¹ is 3,4,5-trifluorophenylmethyl. In yet other embodiments, R¹ is 3,4-difluorophenyl. In yet other embodiments, R¹ is 3-difluoromethyl-4-fluorophenyl. In yet other embodiments, R¹ is 3-chlorophenyl. In yet other embodiments, R¹ is 3-chloro-4-fluorophenyl. In yet other embodiments, R¹ is 4-chloro-3-fluorophenyl. In yet other embodiments, R¹ is 3-fluoro-4-methylphenyl. In yet other embodiments, R¹ is 4-fluoro-3-methylphenyl. In yet other embodiments, R¹ is 3-cyano-4-fluorophenyl. In yet other embodiments, R¹ is 3-difluoromethyl-4-fluorophenyl. In yet other embodiments, R¹ is 2-fluorophenyl. In yet other embodiemnts, R¹ is 2,3-difluorophenyl. In yet other embodiments, R¹ is 3-fluorophenyl. In yet other embodiments, R¹ is 4-fluorophenyl. In yet other embodiments, R¹ is 4-chlorophenyl. In yet other embodiments, R¹ is 4-bromophenyl. In yet other embodiments, R¹ is 3,5-dichlorophenyl. In yet other embodiments, R¹ is 3,5-dichloro-4-fluorophenyl. In yet other embodiments, R¹ is 2,3,4-trifluorophenyl. In yet other embodiments, R¹ is 4-pyridyl. In yet other embodiments, R¹ is 2-chloro-4-pyridyl. In yet other embodiments, R¹ is 2-trifluoromethyl-4-pyridyl. In yet other embodiments, R¹ is 1H-indol-6-yl.

In certain embodiments, each occurrence of R² is independently selected from the group consisting of H and methyl. In other embodiments, R² is H. In yet other embodiments, R² is methyl.

In certain embodiments, R³ is selected from the group consisting of methyl, ethyl, isopropyl, n-propyl, cyclopropyl, n-butyl, isobutyl, sec-butyl, tert-butyl, cyclobutyl, isopropylmethyl, -(CH₂)₂₋₆OH, -(CH₂)₂₋₆O(C₁-C₆ alkyl), optionally substituted benzyl, and optionally substituted phenyl.

In other embodiments, R³ is methyl. In yet other embodiments, R³ is trideuteromethyl. In yet other embodiments, R³ is ethyl. In yet other embodiments, R³ is 1-(2,2-difluoroethyl). In yet other embodiments, R³ is 1-(2,2,2-trifluoroethyl). In yet other embodiments, R³ is 1-pentadeuteroethyl. In yet other embodiments, R³ is isopropyl. In yet other embodiments, R³ is cyclopropyl. In yet other embodiments, R³ is 1-propyl. In yet other embodiments, R³ is cyclopropylmethyl. In yet other embodiments, R³ is 1-butyl. In yet other embodiments, R³ is isobutyl.

In certain embodiments, R³ is methoxymethyl. In certain embodiments, R³ is ethoxymethyl. In certain embodiments, R³ is (acetylamino)methyl. In certain embodiments, R³ is (aminocarbonyl)methyl. In certain embodiments, R³ is (methylaminocarbonyl)methyl. In certain embodiments, R³ is (dimethylaminocarbonyl)methyl. In certain embodiments, R³ is cyanomethyl. In certain embodiments, R³ is methylsulfonylmethyl. In certain embodiments, R³ is aminosulfonylmethyl. In certain embodiments, R³ is (tetrahydro-2H-pyran-4-yl)methyl. In certain embodiments, R³ is (tetrahydrofuran-2-yl)methyl. In certain embodiments, R³ is (tetrahydrofuran-3-yl)methyl. In certain embodiments, R³ is cyclopentylmethyl. In certain embodiments, R³ is cyclohexylmethyl. In certain embodiments, R³ is (1-methylpiperidin-4-yl)methyl. In certain embodiments, R³ is (4-hydroxycyclohexyl)methyl. In certain embodiments, R³ is (1H-1,2,3-triazol-4-yl)methyl. In certain embodiments, R³ is (4H-1,2,4-triazol-3-yl)methyl. In certain embodiments, R³ is (2,2-dimethyl-1,3-dioxan-5-yl)methyl. In certain embodiments, R³ is pyridin-2-ylmethyl. In certain embodiments, R³ is pyridin-3-ylmethyl. In certain embodiments, R³ is pyridin-4-ylmethyl. In certain embodiments, R³ is thiazol-2-ylmethyl. In certain embodiments, R³ is thiazol-4-ylmethyl. In certain embodiments, R³ is pyrimidin-5-ylmethyl. In certain embodiments, R³ is pyrimidin-4-ylmethyl. In certain embodiments, R³ is thiazol-5-ylmethyl.

In certain embodiments, R³ is 1-(2-amino)ethyl. In other embodiments, R³ is 1-(2-hydroxy)ethyl. In yet other embodiments, R³ is 1-(2-methoxy)ethyl. In yet other embodiments, R³ is 1-(2-ethoxy)ethyl. In yet other embodiments, R³ is 1-(2-acetylamino)ethyl. In yet other embodiments, R³ is 1-(2-aminocarbonyl)ethyl. In yet other embodiments, R³ is 1-(2-methylaminocarbonyl)ethyl. In yet other embodiments, R³ is 1-(2-dimethylaminocarbonyl)ethyl. In yet other embodiments, R³ is 1-(2-cyano)ethyl. In yet other embodiments, R³ is 1-(2-methylsulfonyl)ethyl. In yet other embodiments, R³ is 1-(2-aminosulfonyl)ethyl. In yet other embodiments, R³ is 1-(2-(2-methoxyethoxy))ethyl. In yet other embodiments, R³ is 1-(2-(2,2-difluoroethyl)amino)ethyl. In yet other embodiments, R³ is 1-(2-(2,2,2-trifluoroethyl)amino)ethyl. In yet other embodiments, R³ is 1-(1-oxo-1,2-dihydroisoquinolin-4-yl)ethyl. In yet other embodiments, R³ is 1-(1-oxo-1,2-dihydroisoquinolin-4-yl)ethyl. In yet other embodiments, R³ is 1-(2-carboxy)ethyl.

In certain embodiments, R³ is 1-(3-amino)propyl. In other embodiments, R³ is 1-(3-hydroxy)propyl. In yet other embodiments, R³ is 1-(3-methoxy)propyl. In other embodiments, R³ is 1-(3-ethoxy)propyl. In other embodiments, R³ is 1-(3-acetylamino)propyl. In other embodiments, R³ is 1-(3-aminocarbonyl)propyl. In other embodiments, R³ is 1-(3-methylaminocarbonyl)propyl. In other embodiments, R³ is 1-(3-dimethylaminocarbonyl)propyl. In other embodiments, R³ is 1-(3-cyano)propyl. In other embodiments, R³ is 1-(3-methylsulfonyl)propyl. In other embodiments, R³ is 1-(3-aminosulfonyl)propyl. In other embodiments, R³ is 1-(3-hydroxy-2,2-dimethyl)propyl. In other embodiments, R³ is 1-(2-hydroxy)propyl. In other embodiments, R³ is 1-(3-carboxy)propyl. In other embodiments, R³ is 1-(3-hydroxy-2-hydroxymethyl)propyl.

In certain embodiments, R³ is 1-(4-amino)butyl. In other embodiments, R³ is 1-(4-hydroxy)butyl. In certain embodiments, R³ is 1-(4-methoxy)butyl. In certain embodiments, R³ is 1-(4-ethoxy)butyl. In certain embodiments, R³ is 1-(4-acetylamino)butyl. In certain embodiments, R³ is 1-(4-aminocarbonyl)butyl. In certain embodiments, R³ is 1-(4-methylaminocarbonyl)butyl. In certain embodiments, R³ is 1-(4-dimethylaminocarbonyl)butyl. In certain embodiments, R³ is 1-(4-cyano)butyl. In certain embodiments, R³ is 1-(4-methylsulfonyl)butyl.. In certain embodiments, R³ is 1-(4-aminosulfonyl)butyl. In certain embodiments, R³ is 1-(3-hydroxy)butyl. In certain embodiments, R³ is 1-(4-carboxy)butyl.. In certain embodiments, R³ is benzyl.

In certain embodiments, R^{4a} is selected from the group consisting of H, methyl, ethyl, isopropyl, n-propyl, cyclopropyl, n-butyl, isobutyl, sec-butyl, tert-butyl, cyclobutyl, isopropylmethyl, -(CH₂)₂₋₆OH, -(CH₂)₂₋₆O(C₁-C₆ alkyl), optionally substituted benzyl, optionally substituted phenyl, fluoromethyl, difluoromethyl, and trifluoromethyl.

In certain embodiments, R^{4a} is H. In other embodiments, R^{4a} is methyl. In yet other embodiments, R^{4a} is trifluoromethyl. In certain embodiments, R^{4a} is ethyl. In certain embodiments, R^{4a} is cyclopropyl. In certain embodiments, R^{4a} is isopropyl. In certain embodiments, R^{4a} is 1-propyl. In certain embodiments, R^{4a} is phenyl. In certain embodiments, R^{4a} is 2-hydroxyethyl.

In certain embodiments, R^{4b} is selected from the group consisting of H and methyl. In other embodiments, R^{4b} is H. In other embodiments, R^{4b} is methyl.

In certain embodiments, R⁷ is H. In other embodiments, R⁷ is methyl.

In other embodiments, R⁸ is methoxy. In yet other embodiments, R⁸ is ethoxy. In yet other embodiments, R⁸ is methyl. In yet other embodiments, R⁸ is ethyl. In yet other embodiments, R⁸ is 2-hydroxyethoxy. In yet other embodiments, R⁸ is amino. In yet other embodiments, R⁸ is methylamino. In yet other embodiments, R⁸ is ethylamino. In yet other embodiments, R⁸ is dimethylamino. In yet other embodiments, R⁸ is (2-hydroxyethyl)amino. In yet other embodiments, R⁸ is 2-aminoethyl)amino. In yet other embodiments, R⁸ is triazolyl. In yet other embodiments, R⁸ is triazolylmethoxy. In yet other embodiments, R⁸ is (N-methyltriazolyl)methyl. In yet other embodiments, R⁸ is triazolylmethylamino. In yet other embodiments, R⁸ is (N-methyltriazolyl)methylamino. In yet other embodiments, R⁸ is CN. In yet other embodiments, R⁸ is hydroxymethyl. In yet other embodiments, R⁸ is carboxy. In yet other embodiments, R⁸ is aminocarbonyl. In yet other embodiments, R⁸ is methylaminocarbonyl. In yet other embodiments, R⁸ is dimethylaminocarbonyl. In yet other embodiments, R⁸ is methylsulfonyl. In yet other embodiments, R⁸ is pyridylmethoxy.

In certain embodiments, R⁹ is H. In other embodiments, R⁹ is Cl. In yet other embodiments, R⁹ is F. In yet other embodiments, R⁹ is Br. In yet other embodiments, R⁹ is methoxy. In yet other embodiments, R⁹ is ethoxy.

In certain embodiments, R¹⁰ is H. In other embodiments, R¹⁰ is methyl. In yet other embodiments, R¹⁰ is ethyl. In yet other embodiments, R¹⁰ is 1-(2,2,2-trifluoroethyl). In yet other embodiments, R¹⁰ is 1-propyl. In yet other embodiments, R¹⁰ is isopropyl. In yet other embodiments, R¹⁰ is cyclopropyl. In yet other embodiments, R¹⁰ is 1-(2-hydroxy)ethyl. In yet other embodiments, R¹⁰ is 1-(2-methoxy)ethyl. In yet other embodiments, R¹⁰ is 1-(3-hydroxy)propyl. In yet other embodiments, R¹⁰ is 1-(3-methoxy)propyl. In yet other embodiments, R¹⁰ is triazolylmethyl.

In certain embodiments, the compound of the invention is any compound disclosed herein, or a salt, solvate, isotopically labelled, stereoisomer, any mixture of stereoisomers, tautomer, and/or any mixture of tautomers thereof.

In certain embodiments, the compound is at least one compound selected from Table 4, or a salt, solvate, isotopically labelled, stereoisomer, any mixture of stereoisomers, tautomer, and/or any mixture of tautomers thereof.

In certain embodiments, the compound is at least one selected from: (R)-3-(3-chloro-4-fluorophenyl)-1-methyl-1-((1-oxo-1,2-dihydroisoquinolin-4-yl)methyl)urea; (S)-3-(3-chloro-4-fluorophenyl)-1-methyl-1-((1-oxo-1,2-dihydroisoquinolin-4-yl)methyl)urea; (R)-3-(3-chloro-4-fluorophenyl)-1-((1-methoxyisoquinolin-4-yl)methyl)-1-methylurea; (S)-3-(3-chloro-4-fluorophenyl)-1-((1-methoxyisoquinolin-4-yl)methyl)-1-methylurea; (R)-3-(4-fluoro-3-methylphenyl)-1-((1-methoxyisoquinolin-4-yl)methyl)-1-methylurea; (S)-3-(4-fluoro-3-methylphenyl)-1-((1-methoxyisoquinolin-4-yl)methyl)-1-methylurea; (R)-1-(4-fluoro-3-methylphenyl)-3-((1-oxo-1,2-dihydroisoquinolin-4-yl)methyl)urea; (S)-1-(4-fluoro-3-methylphenyl)-3-((1-oxo-1,2-dihydroisoquinolin-4-yl)methyl)urea; (R)-3-(4-fluoro-3-methylphenyl)-1-methyl-1-((1-oxo-1,2-dihydroisoquinolin-4-yl)methyl)urea; (S)-3-(4-fluoro-3-methylphenyl)-1-methyl-1-((1-oxo-1,2-dihydroisoquinolin-4-yl)methyl)urea; (R)-1-(3-chloro-4-fluorophenyl)-3-((1-methoxyisoquinolin-4-yl)methyl)urea; (S)-1-(3-chloro-4-fluorophenyl)-3-((1-methoxyisoquinolin-4-yl)methyl)urea; (R)-1-(4-fluoro-3-methylphenyl)-3-((1-methoxyisoquinolin-4-yl)methyl)urea; (S)-1-(4-fluoro-3-methylphenyl)-3-((1-methoxyisoquinolin-4-yl)methyl)urea; (R)-1-(3-chloro-4-fluorophenyl)-3-((1-oxo-1,2-dihydroisoquinolin-4-yl)methyl)urea; (S)-1-(3-chloro-4-fluorophenyl)-3-((1-oxo-1,2-dihydroisoquinolin-4-yl)methyl)urea; (R)-3-(3-chloro-4-fluorophenyl)-1-ethyl-1-((1-oxo-1,2-dihydroisoquinolin-4-yl)methyl)urea; (S)-3-(3-chloro-4-fluorophenyl)-1-ethyl-1-((1-oxo-1,2-dihydroisoquinolin-4-yl)methyl)urea; (R)-1-(3-chloro-4-fluorophenyl)-3-(1-(1-oxo-1,2-dihydroisoquinolin-4-yl)ethyl)urea; (S)-1-(3-chloro-4-fluorophenyl)-3-(1-(1-oxo-1,2-dihydroisoquinolin-4-yl)ethyl)urea; (R)-3-(3-chloro-4-fluorophenyl)-1-methyl-1-(1-(1-oxo-1,2-dihydroisoquinolin-4-yl)ethyl)urea; (S)-3-(3-chloro-4-fluorophenyl)-1-methyl-1-(1-(1-oxo-1,2-dihydroisoquinolin-4-yl)ethyl)urea; (R)-3-(3-chloro-4-fluorophenyl)-1-ethyl-1-((1-methoxyisoquinolin-4-yl)methyl)urea; (S)-3-(3-chloro-4-fluorophenyl)-1-ethyl-1-((1-methoxyisoquinolin-4-yl)methyl)urea; (R)-3-(3-chloro-4-fluorophenyl)-1-ethyl-1-(isoquinolin-4-ylmethyl)urea; (S)-3-(3-chloro-4-fluorophenyl)-1-ethyl-1-(isoquinolin-4-ylmethyl)urea; (R)-3-(3-chloro-4-fluorophenyl)-1-((1-ethoxyisoquinolin-4-yl)methyl)-1-ethylurea; (S)-3-(3-chloro-4-fluorophenyl)-1-((1-ethoxyisoquinolin-4-yl)methyl)-1-ethylurea; (R)-3-(3-chloro-4-fluorophenyl)-1-ethyl-1-((2-methyl-1-oxo-1,2-dihydroisoquinolin-4-yl)methyl)urea; (S)-3-(3-chloro-4-fluorophenyl)-1-ethyl-1-((2-methyl-1 -oxo-1,2-dihydroisoquinolin-4-yl)methyl)urea; (R)-3-(3-chloro-4-fluorophenyl)-1-isopropyl-1-((1-methoxyisoquinolin-4-yl)methyl)urea; (S)-3-(3-chloro-4-fluorophenyl)-1-isopropyl-1-((1-methoxyisoquinolin-4-yl)methyl)urea; (R)-3-(3-chloro-4-fluorophenyl)-1-((1-methoxyisoquinolin-4-yl)methyl)-1-propylurea; (S)-3-(3-chloro-4-fluorophenyl)-1-((1-methoxyisoquinolin-4-yl)methyl)-1-propylurea; (R)-3-(3-chloro-4-fluorophenyl)-1-(2-hydroxyethyl)-1-((1-methoxyisoquinolin-4-yl)methyl)urea; (S)-3-(3-chloro-4-fluorophenyl)-1-(2-hydroxyethyl)-1-((1-methoxyisoquinolin-4-yl)methyl)urea; (R)-3-(3-chloro-4-fluorophenyl)-1-ethyl-1-(1-(1-methoxyisoquinolin-4-yl)ethyl)urea; (S)-3-(3-chloro-4-fluorophenyl)-1-ethyl-1-(1-(1-methoxyisoquinolin-4-yl)ethyl)urea; (R)-3-(3-chloro-4-fluorophenyl)-1-ethyl-1-(1-(1-oxo-1,2-dihydroisoquinolin-4-yl)ethyl)urea; (S)-3-(3-chloro-4-fluorophenyl)-1-ethyl-1-(1-(1-oxo-1,2-dihydroisoquinolin-4-yl)ethyl)urea; (R)-3-(3-chloro-4-fluorophenyl)-1-isopropyl-1-((1-oxo-1,2-dihydroisoquinolin-4-yl)methyl)urea; (S)-3-(3-chloro-4-fluorophenyl)-1-isopropyl-1-((1-oxo-1,2-dihydroisoquinolin-4-yl)methyl)urea; (R)-3-(3-chloro-4-fluorophenyl)-1-(2-hydroxyethyl)-1-((1-oxo-1,2-dihydroisoquinolin-4-yl)methyl)urea; (S)-3-(3-chloro-4-fluorophenyl)-1-(2-hydroxyethyl)-1-((1-oxo-1,2-dihydroisoquinolin-4-yl)methyl)urea; (R)-3-(3-chloro-4-fluorophenyl)-1-((1-oxo-1,2-dihydroisoquinolin-4-yl)methyl)-1-propylurea; (S)-3-(3-chloro-4-fluorophenyl)-1-((1-oxo-1,2-dihydroisoquinolin-4-yl)methyl)-1-propylurea; (R)-3-(3-chloro-4-fluorophenyl)-1-ethyl-1-((1-(2-hydroxyethoxy)isoquinolin-4-yl)methyl)urea; (S)-3-(3-chloro-4-fluorophenyl)-1-ethyl-1-((1-(2-hydroxyethoxy)isoquinolin-4-yl)methyl)urea; (R)-3-(3-chloro-4-fluorophenyl)-1-(1-(1-methoxyisoquinolin-4-yl)ethyl)-1-methylurea; (S)-3-(3-chloro-4-fluorophenyl)-1-(1-(1-methoxyisoquinolin-4-yl)ethyl)-1-methylurea; (R)-3-(3-chloro-4-fluorophenyl)-1-(cyclopropylmethyl)-1-((1-methoxyisoquinolin-4-yl)methyl)urea; (S)-3-(3-chloro-4-fluorophenyl)-1-(cyclopropylmethyl)-1-((1-methoxyisoquinolin-4-yl)methyl)urea; (R)-3-(4-fluoro-3-methylphenyl)-1-methyl-1-(1-(1-oxo-1,2-dihydroisoquinolin-4-yl)ethyl)urea; (S)-3-(4-fluoro-3-methylphenyl)-1-methyl-1-(1-(1-oxo-1,2-dihydroisoquinolin-4-yl)ethyl)urea; (R)-1-butyl-3-(3-chloro-4-fluorophenyl)-1-((1-methoxyisoquinolin-4-yl)methyl)urea; (S)-1-butyl-3-(3-chloro-4-fluorophenyl)-1-((1-methoxyisoquinolin-4-yl)methyl)urea; (R)-3-(3-chloro-4-fluorophenyl)-1-cyclopropyl-1-((1-methoxyisoquinolin-4-yl)methyl)urea; (S)-3-(3-chloro-4-fluorophenyl)-1-cyclopropyl-1-((1-methoxyisoquinolin-4-yl)methyl)urea; (R)- (3-(3-chloro-4-fluorophenyl)-1-(1-(1-oxo-1,2-dihydroisoquinolin-4-yl)ethyl)-1-propylurea; (S)- (3-(3-chloro-4-fluorophenyl)-1-(1-(1-oxo-1,2-dihydroisoquinolin-4-yl)ethyl)-1-propylurea; (R)-3-(3-chloro-4-fluorophenyl)-1-ethyl-1-(1-(1-methoxyisoquinolin-4-yl)propyl)urea; (S)-3-(3-chloro-4-fluorophenyl)-1-ethyl-1-(1-(1-methoxyisoquinolin-4-yl)propyl)urea; (R)-3-(3-chloro-4-fluorophenyl)-1-ethyl-1-(1-(1-oxo-1,2-dihydroisoquinolin-4-yl)propyl)urea; (S)-3-(3-chloro-4-fluorophenyl)-1-ethyl-1-(1-(1-oxo-1,2-dihydroisoquinolin-4-yl)propyl)urea; (R)-3-(3-chloro-4-fluorophenyl)-1-(cyclopropyl(1-methoxyisoquinolin-4-yl)methyl)-1-ethylurea; (S)-3-(3-chloro-4-fluorophenyl)-1-(cyclopropyl(1-methoxyisoquinolin-4-yl)methyl)-1-ethylurea; (R)-3-(3-chloro-4-fluorophenyl)-1-(2-methoxyethyl)-1-((1-methoxyisoquinolin-4-yl)methyl)urea; (S)-3-(3-chloro-4-fluorophenyl)-1-(2-methoxyethyl)-1-((1-methoxyisoquinolin-4-yl)methyl)urea; (R)-3-(3-chloro-4-fluorophenyl)-1-cyclopropyl-1-((1-oxo-1,2-dihydroisoquinolin-4-yl)methyl)urea; (S)-3-(3-chloro-4-fluorophenyl)-1-cyclopropyl-1-((1-oxo-1,2-dihydroisoquinolin-4-yl)methyl)urea; (R)-3-(4-fluoro-3-methylphenyl)-1-(1-(1-oxo-1,2-dihydroisoquinolin-4-yl)ethyl)-1-propylurea; (S)-3-(4-fluoro-3-methylphenyl)-1-(1-(1-oxo-1,2-dihydroisoquinolin-4-yl)ethyl)-1-propylurea; (R)-3-(3-chloro-4-fluorophenyl)-1-ethyl-1-(1-(1-methoxyisoquinolin-4-yl)-2-methylpropyl)urea; (S)-3-(3-chloro-4-fluorophenyl)-1-ethyl-1-(1-(1-methoxyisoquinolin-4-yl)-2-methylpropyl)urea; (R)-3-(3-chloro-4-fluorophenyl)-1-(cyclopropyl(1-oxo-1,2-dihydroisoquinofin-4-yl)methyl)-1-ethylurea; (S)-3-(3-chloro-4-fluorophenyl)-1-(cyclopropyl(1-oxo-1,2-dihydroisoquinolin-4-yl)methyl)-1-ethylurea; (R)-3-(3-chloro-4-fluorophenyl)-1-((1-methoxyisoquinolin-4-yl)methyl)-1-(3-methoxypropyl)urea; (S)-3-(3-chloro-4-fluorophenyl)-1-((1-methoxyisoquinolin-4-yl)methyl)-1-(3-methoxypropyl)urea; (R)-1-benzyl-3-(3-chloro-4-fluorophenyl)-1-((1-methoxyisoquinolin-4-yl)methyl)urea; (S)-1-benzyl-3-(3-chloro-4-fluorophenyl)-1-((1-methoxyisoquinolin-4-yl)methyl)urea; (R)-3-(3-chloro-4-fluorophenyl)-1-(2-methoxyethyl)-1-((1-oxo-1,2-dihydroisoquinolin-4-yl)methyl)urea; (S)-3-(3-chloro-4-fluorophenyl)-1-(2-methoxyethyl)-1-((1-oxo-1,2-dihydroisoquinolin-4-yl)methyl)urea; (R)-3-(3-chloro-4-fluorophenyl)-1-(3-methoxypropyl)-1-((1-oxo-1,2-dihydroisoquinolin-4-yl)methyl)urea; (S)-3-(3-chloro-4-fluorophenyl)-1-(3-methoxypropyl)-1-((1-oxo-1,2-dihydroisoquinolin-4-yl)methyl)urea; (R)-1-butyl-3-(3-chloro-4-fluorophenyl)-1-((1-oxo-1,2-dihydroisoquinolin-4-yl)methyl)urea; (S)-1-butyl-3-(3-chloro-4-fluorophenyl)-1-((1-oxo-1,2-dihydroisoquinolin-4-yl)methyl)urea; (R)-3-(3-chloro-4-fluorophenyl)-1-(cyclopropylmethyl)-1-((1-oxo-1,2-dihydroisoquinolin-4-yl)methyl)urea; (S)-3-(3-chloro-4-fluorophenyl)-1-(cyclopropylmethyl)-1-((1-oxo-1,2-dihydroisoquinolin-4-yl)methyl)urea; (R)-3-(3-chloro-4-fluoropheny1)-1-ethyl-1-(2-methy1-1-(1-oxo-1,2-dihydroisoquinolin-4-yl)propyl)urea; (S)-3-(3-chloro-4-fluorophenyl)-1-ethyl-1-(2-methyl-1-(1-oxo-1,2-dihydroisoquinolin-4-yl)propyl)urea; (R)-3-(3-chloro-4-fluorophenyl)-1-cyclopropyl-1-(1-(1-methoxyisoquinolin-4-yl)ethyl)urea; (S)-3-(3-chloro-4-fluorophenyl)-1-cyclopropyl-1-(1-(1-methoxyisoquinolin-4-yl)ethyl)urea; (R)-3-(3-chloro-4-fluorophenyl)-1-ethyl-1-((1-methoxyisoquinolin-4-yl)(phenyl)methyl)urea; (S)-3-(3-chloro-4-fluorophenyl)-1-ethyl-1-((1-methoxyisoquinolin-4-yl)(phenyl)methyl)urea; (R)-1-butyl-3-(3-chloro-4-fluorophenyl)-1-(1-(1-methoxyisoquinolin-4-yl)ethyl)urea; (S)-1-butyl-3-(3-chloro-4-fluorophenyl)-1-(1-(1-methoxyisoquinolin-4-yl)ethyl)urea; (R)-3-(3-chloro-4-fluorophenyl)-1-(cyclopropylmethyl)-1-(1-(1-methoxyisoquinolin-4-yl)ethyl)urea; (S)-3-(3-chloro-4-fluorophenyl)-1-(cyclopropylmethyl)-1-(1-(1-methoxyisoquinolin-4-yl)ethyl)urea; (R)-3-(3-chloro-4-fluorophenyl)-1-(2-methoxyethyl)-1-(1-(1-oxo-1,2-dihydroisoquinolin-4-yl)ethyl)urea; (S)-3-(3-chloro-4-fluorophenyl)-1-(2-methoxyethyl)-1-(1-(1-oxo-1,2-dihydroisoquinolin-4-yl)ethyl)urea; (R)-3-(3-chloro-4-fluorophenyl)-1-cyclopropyl-1-(1-(1-oxo-1,2-dihydroisoquinolin-4-yl)ethyl)urea; (S)-3-(3-chloro-4-fluorophenyl)-1-cyclopropyl-1-(1-(1-oxo-1,2-dihydroisoquinolin-4-yl)ethyl)urea; (R)-3-(3-chloro-4-fluorophenyl)-1-ethyl-1-((1-oxo-1,2-dihydroisoquinolin-4-yl)(phenyl)methyl)urea; (S)-3-(3-chloro-4-fluorophenyl)-1-ethyl-1-((1-oxo-1,2-dihydroisoquinolin-4-yl)(phenyl)methyl)urea; (R)-1-(3-chloro-4-fluorophenyl)-3-(3-hydroxy-1-(1-methoxyisoquinolin-4-yl)propyl)urea; (S)-1-(3-chloro-4-fluorophenyl)-3-(3-hydroxy-1-(1-methoxyisoquinolin-4-yl)propyl)urea; (R)-3-(3-chloro-4-fluorophenyl)-1-(2-methoxyethyl)-1-(1-(1-methoxyisoquinolin-4-yl)ethyl)urea; (S)-3-(3-chloro-4-fluorophenyl)-1-(2-methoxyethyl)-1-(1-(1-methoxyisoquinolin-4-yl)ethyl)urea; (R)-3-(3-chloro-4-fluorophenyl)-1-ethyl-1-(2,2,2-trifluoro-1-(1-oxo-1,2-dihydroisoquinolin-4-yl)ethyl)urea; (S)-3-(3-chloro-4-fluorophenyl)-1-ethyl-1-(2,2,2-trifluoro-1-(1-oxo-1,2-dihydroisoquinolin-4-yl)ethyl)urea; (R)-3-(3-chloro-4-fluorophenyl)-1-ethyl-1-(2,2,2-trifluoro-1-(1-methoxyisoquinolin-4-yl)ethyl)urea; (S)-3-(3-chloro-4-fluorophenyl)-1-ethyl-1 -(2,2,2-trifluoro-1 -(1 -methoxyisoquinolin-4-yl)ethyl)urea; (R)-3-(3-chloro-4-fluorophenyl)-1-(3-methoxypropyl)-1-(1-(1-oxo-1,2-dihydroisoquinolin-4-yl)ethyl)urea; (S)-3-(3-chloro-4-fluorophenyl)-1-(3-methoxypropyl)-1-(1-(1-oxo-1,2-dihydroisoquinolin-4-yl)ethyl)urea; (R)-3-(3-chloro-4-fluorophenyl)-1-(1-(1-methoxyisoquinolin-4-yl)ethyl)-1-(3-methoxypropyl)urea; (S)-3-(3-chloro-4-fluorophenyl)-1-(1-(1-methpxyisoquinolin-4-yl)ethyl)-1-(3-methoxypropyl)urea; (R)-1-butyl-3-(3-chloro-4-fluorophenyl)-1-(1-(1-oxo-1,2-dihydroisoquinolin-4-yl)ethyl)urea; (S)-1-butyl-3-(3-chloro-4-fluorophenyl)-1-(1-(1-oxo-1,2-dihydroisoquinolin-4-yl)ethyl)urea; (R)-3-(3-chloro-4-fluorophenyl)-1-(cyclopropylmethyl)-1-(1-(1-oxo-1,2-dihydroisoquinolin-4-yl)ethyl)urea; (S)-3-(3-chloro-4-fluorophenyl)-1-(cyclopropylmethyl)-1-(1-(1-oxo-1,2-dihydroisoquinolin-4-yl)ethyl)urea; (R)-3-(3-chloro-4-fluorophenyl)-1-methyl-1-((1-((1-methyl-1H-1,2,4-triazol-3-yl)methoxy)isoquinolin-4-yl)methyl)urea; (S)-3-(3-chloro-4-fluorophenyl)-1-methyl-1-((1-((1-methyl-1H-1,2,4-triazol-3-yl)methoxy)isoquinolin-4-yl)methyl)urea; (R)-3-(3-chloro-4-fluorophenyl)-1-methyl-1-((1-(pyridin-2-ylmethoxy)isoquinolin-4-yl)methyl)urea; (S)-3-(3-chloro-4-fluorophenyl)-1-methyl-1-((1-(pyridin-2-ylmethoxy)isoquinolin-4-yl)methyl)urea; (R)-3-(4-fluoro-3-(trifluoromethyl)phenyl)-1-methyl-1-(1-(1-oxo-1,2-dihydroisoquinolin-4-yl)ethyl)urea; (S)-3-(4-fluoro-3-(trifluoromethyl)phenyl)-1-methyl-1-(1-(1-oxo-1,2-dihydroisoquinolin-4-yl)ethyl)urea; (R)-3-(4-fluorophenyl)-1-methyl-1-(1-(1-oxo-1,2-dihydroisoquinolin-4-yl)ethyl)urea; (S)-3-(4-fluorophenyl)-1-methyl-1-(1-(1-oxo-1,2-dihydroisoquinolin-4-yl)ethyl)urea; (R)-1-methyl-1-(1-(1-oxo-1,2-dihydroisoquinolin-4-yl)ethyl)-3-(3,4,5-trifluorophenyl)urea; (S)-1-methyl-1-(1-(1-oxo-1,2-dihydroisoquinolin-4-yl)ethyl)-3-(3,4,5-trifluorophenyl)urea; (R)-3-(3,4-difluorophenyl)-1-methyl-1-(1-(1-oxo-1,2-dihydroisoquinolin-4-yl)ethyl)urea; (S)-3-(3,4-difluorophenyl)-1-methyl-1-(1-(1-oxo-1,2-dihydroisoquinolin-4-yl)ethyl)urea; (R)-3-(3-fluorophenyl)-1-methyl-1-(1-(1-oxo-1,2-dihydroisoquinolin-4-yl)ethyl)urea; (S)-3-(3-fluorophenyl)-1-methyl-1-(1-(1-oxo-1,2-dihydroisoquinolin-4-yl)ethyl)urea; (R)-3-(2-Chloropyridin-4-yl)-1-methyl-1-(1-(1-oxo-1,2-dihydroisoquinolin-4-yl)ethyl)urea; (S)-3-(2-Chloropyridin-4-yl)-1-methyl-1-(1-(1-oxo-1,2-dihydroisoquinolin-4-yl)ethyl)urea; (R)-1-Methyl-1-(1-(1-oxo-1,2-dihydroisoquinolin-4-yl)ethyl)-3-(2-(trifluoromethyl)pyridin-4-yl)urea; (S)-1-Methyl-1-(1-(1-oxo-1,2-dihydroisoquinolin-4-yl)ethyl)-3-(2-(trifluoromethyl)pyridin-4-yl)urea; (R)-3-(3-chloro-4-fluorophenyl)-1-(2,2-difluoroethyl)-1-(1-(1-oxo-1,2-dihydroisoquinolin-4-yl)ethyl)urea; (S)-3-(3-chloro-4-fluorophenyl)-1-(2,2-difluoroethyl)-1-(1-(1-oxo-1,2-dihydroisoquinolin-4-yl)ethyl)urea; (R)-1-(2,2-difluoroethyl)-3-(4-fluoro-3-methylphenyl)-1-(1-(1-oxo-1,2-dihydroisoquinolin-4-yl)ethyl)urea; (S)-1-(2,2-difluoroethyl)-3-(4-fluoro-3-methylphenyl)-1-(1-(1-oxo-1,2-dihydroisoquinolin-4-yl)ethyl)urea; (R)-3-(3-cyano-4-fluorophenyl)-1-methyl-1-(1-(1-oxo-1,2-dihydroisoquinolin-4-yl)ethyl)urea; (S)-3-(3-cyano-4-fluorophenyl)-1-methyl-1-(1-(1-oxo-1,2-dihydroisoquinolin-4-yl)ethyl)urea; (R)-3-(3-chloro-4-fluorophenyl)-1-(ethyl-d5)-1-(1-(1-oxo-1,2-dihydroisoquinolin-4-yl)ethyl)urea; (S)-3-(3-chloro-4-fluorophenyl)-1-(ethyl-d5)-1-(1-(1-oxo-1,2-dihydroisoquinolin-4-yl)ethyl)urea; (R)-N-(2-(3-(3-chloro-4-fluorophenyl)-1-(1-(1-oxo-1,2-dihydroisoquinolin-4-yl)ethyl)ureido)ethyl)acetamide; (S)-N-(2-(3-(3-chloro-4-fluorophenyl)-1-(1-(1-oxo-1,2-dihydroisoquinolin-4-yl)ethyl)ureido)ethyl)acetamide; (R)- (3-(3-chloro-4-fluorophenyl)-1-(1-(1-methoxyisoquinolin-4-yl)ethyl)-1-(2,2,2-trifluoroethyl)urea; (S)- (3-(3-chloro-4-fluorophenyl)-1-(1-(1-methoxyisoquinolin-4-yl)ethyl)-1-(2,2,2-trifluoroethyl)urea; (R)-3-(3-chloro-4-fluorophenyl)-1-ethyl-1-(1-(1-oxo-1,2-dihydro-2, 7-naphthyridin-4-yl)ethyl)urea; (S)-3-(3-chloro-4-fluoropheny1)-1-ethyl-1-(1-(1-oxo-1,2-dihydro-2,7-naphthyridin-4-yl)ethyl)urea; (R)-3-(3-chloro-4-fluorophenyl)-1-(2-cyanoethyl)-1-(1-(1-oxo-1,2-dihydroisoquinolin-4-yl)ethyl)urea; (S)-3-(3-chloro-4-fluorophenyl)-1-(2-cyanoethyl)-1-(1-(1-oxo-1,2-dihydroisoquinolin-4-yl)ethyl)urea; (R)-3-(3-(3-chloro-4-fluorophenyl)-1-(1-(1-oxo-1,2-dihydroisoquinolin-4-yl)ethyl)ureido)-N-methylpropanamide; (S)-3-(3-(3-chloro-4-fluorophenyl)-1-(1-(1-oxo-1,2-dihydroisoquinolin-4-yl)ethyl)ureido)-N-methylpropanamide; (R)-3-(3-(3-chloro-4-fluorophenyl)-1-(1-(1-oxo-1,2-dihydroisoquinolin-4-yl)ethyl)ureido)-N,N-dimethylpropanamide; (S)-3-(3-(3-chloro-4-fluorophenyl)-1-(1-(1-oxo-1,2-dihydroisoquinolin-4-yl)ethyl)ureido)-N,N-dimethylpropanamide; (R)-3-(3-chloro-4-fluorophenyl)-1-(2-(methylsulfonyl)ethyl)-1-(1-(1-oxo-1,2-dihydroisoquinolin-4-yl)ethyl)urea; (S)-3-(3-chloro-4-fluorophenyl)-1-(2-(methylsulfonyl)ethyl)-1-(1-(1-oxo-1,2-dihydroisoquinolin-4-yl)ethyl)urea; (R)-3-(3-chloro-4-fluorophenyl)-I-(2-ethoxyethyl)-I-(1-(1-oxo-1,2-dihydroisoquinolin-4-yl)ethyl)urea; (S)-3-(3-chloro-4-fluorophenyl)-1-(2-ethoxyethyl)-1-(1-(1-oxo-1,2-dihydroisoquinolin-4-yl)ethyl)urea; (R)-3-(3-chloro-4-fluorophenyl)-1-methyl-1-(1-(1-oxo-1,2-dihydro-2,7-naphthyridin-4-yl)ethyl)urea; (S)-3-(3-chloro-4-fluorophenyl)-1-methyl-1-(1-(1-oxo-1,2-dihydro-2,7-naphthyridin-4-yl)ethyl)urea; (R)-3-(3-chloro-4-fluorophenyl)-1-(3-cyanopropyl)-1-(1-(1-oxo-l,2-dihydroisoquinolin-4-yl)ethyl)urea; (S)-3-(3-chloro-4-fluorophenyl)-1-(3-cyanopropyl)-1-(1-(1-oxo-1,2-dihydroisoquinolin-4-yl)ethyl)urea; (R)-2-(3-(3-chloro-4-fluorophenyl)-1-(1-(1-oxo-1,2-dihydroisoquinolin-4-yl)ethyl)ureido)-N-methylacetamide; (S)-2-(3-(3-chloro-4-fluorophenyl)-1-(1-(1-oxo-1,2-dihydroisoquinolin-4-yl)ethyl)ureido)-N-methylacetamide; (R)-2-(3-(3-chloro-4-fluorophenyl)-1-(1-(1-oxo-1,2-dihydroisoquinolin-4-yl)ethyl)ureido)ethane-1-sulfonamide; (S)-2-(3-(3-chloro-4-fluorophenyl)-1-(1-(1-oxo-1,2-dihydroisoquinolin-4-yl)ethyl)ureido)ethane-1-sulfonamide; (R)-3-(3-chloro-4-fluorophenyl)-1-(2-(2-methoxyethoxy)ethyl)-1-(1-(1-methoxyisoquinolin-4-yl)ethyl)urea; (S)-3-(3-chloro-4-fluorophenyl)-1-(2-(2-methoxyethoxy)ethyl)-1-(1-(1-methoxyisoquinolin-4-yl)ethyl)urea; (R)-3-(3-chloro-4-fluorophenyl)-1-(2-(2-methoxyethoxy)ethyl)-1-(1-(1-oxo-1,2-dihydroisoquinolin-4-yl)ethyl)urea; (S)-3-(3-chloro-4-fluorophenyl)-1-(2-(2-methoxyethoxy)ethyl)-1-(1-(1-oxo-1,2-dihydroisoquinolin-4-yl)ethyl)urea; (R)-3-(3-chloro-4-fluorophenyl)-1-(2-ethoxyethyl)-1-(1-(1-methoxylsoquinolin-4-yl)ethyl)urea; (S)-3-(3-chloro-4-fluorophenyl)-1-(2-ethoxyethyl)-1-(1-(1-methoxyisoquinolin-4-yl)ethyl)urea; (R)-3-(3-chloro-4-fluorophenyl)-1-methyl-1-(1-(1-oxo-1,2-dihydro-2,6-naphthyridin-4-yl)ethyl)urea; (S)-3-(3-chloro-4-fluorophenyl)-1-methyl-1-(1-(1-oxo-1,2-dihydro-2,6-naphthyridin-4-yl)ethyl)urea; (R)-2-(3-(3-chloro-4-fluorophenyl)-1-(1-(1-oxo-1,2-dihydroisoquinolin-4-yl)ethyl)ureido)acetamide; (S)-2-(3-(3-chloro-4-fluorophenyl)-1-(1-(1-oxo-1,2-dihydroisoquinolin-4-yl)ethyl)ureido)acetamide; (R)-3-(3-chloro-4-fluorophenyl)-1-(3-hydroxypropyl)-1-(1-(1-oxo-1,2-dihydroisoquinolin-4-yl)ethyl)urea; (S)-3-(3-chloro-4-fluorophenyl)-1-(3-hydroxypropyl)-1-(1-(1-oxo-1,2-dihydroisoquinolin-4-yl)ethyl)urea; (R)-3-(3-chloro-4-fluorophenyl)-1-methyl-1-(1-(1-((1-methyl-1H-1,2,4-triazol-3-yl)methoxy)isoquinolin-4-yl)ethyl)urea; (S)-3-(3-chloro-4-fluorophenyl)-1-methyl-1-(1-(1-((1-methyl-1H-1,2,4-triazol-3-yl)methoxy)isoquinolin-4-yl)ethyl)urea; (R)-3-(3-chloro-4-fluorophenyl)-1-(1-(1-oxo-1,2-dihydroisoquinolin-4-yl)ethyl)-1-((tetrahydro-2H-pyran-4-yl)methyl)urea; (S)-3-(3-chloro-4-fluorophenyl)-1-(1-(1-oxo-1,2-dihydroisoquinolin-4-yl)ethyl)-1-((tetrahydro-2H-pyran-4-yl)methyl)urea; (R)-3-(3-chloro-4-fluorophenyl)-1-isobutyl-1-(1-(1-oxo-1,2-dihydroisoquinolin-4-yl)ethyl)urea; (S)-3-(3-chloro-4-fluorophenyl)-1-isobutyl-1-(1-(1-oxo-1,2-dihydroisoquinolin-4-yl)ethyl)urea; (R)-3-(3-chloro-4-fluorophenyl)-1-methyl-1-(1-(5-(methylamino)pyrido[3,4-b]pyrazin-8-yl)ethyl)urea; (S)-3-(3-chloro-4-fluorophenyl)-1-methyl-1-(1-(5-(methylamino)pyrido[3,4-b]pyrazin-8-yl)ethyl)urea; (R)-3-(3-chloro-4-fluorophenyl)-1-(1-(5-methoxypyrido[3,4-b]pyrazin-8-yl)ethyl)-1-methylurea; (S)-3-(3-chloro-4-fluorophenyl)-1-(1-(5-methoxypyrido[3,4-b]pyrazin-8-yl)ethyl)-1-methylurea; (R)-3-(3-chloro-4-fluorophenyl)-1-(3-hydroxypropyl)-1-(1-(1-methoxyisoquinolin-4-yl)ethyl)urea; (S)-3-(3-chloro-4-fluorophenyl)-1-(3-hydroxypropyl)-1-(1-(1-methoxyisoquinolin-4-yl)ethyl)urea; (R)-1-(1-(1-((1H-1,2,4-triazol-3-yl)methoxy)isoquinolin-4-yl)ethyl)-3-(3-chloro-4-fluorophenyl)-1-methylurea; (S)-1-(1-(1-((1H-1,2,4-triazol-3-yl)methoxy)isoquinolin-4-yl)ethyl)-3-(3-chloro-4-fluorophenyl)-1-methylurea; (R)-2-(3-(3-chloro-4-fluorophenyl)-1-(1-(1-oxo-1,2-dihydroisoquinolin-4-yl)ethyl)ureido)-N,N-dimethylacetamide; (S)-2-(3-(3-chloro-4-fluorophenyl)-1-(1-(1-oxo-1,2-dihydroisoquinolin-4-yl)ethyl)ureido)-N,N-dimethylacetamide; (R)-3-(3-chloro-4-fluorophenyl)-1-methyl-1-(1-(5-oxo-5,6-dihydropyrido[3,4-b]pyrazin-8-yl)ethyl)urea; (S)-3-(3-chloro-4-fluorophenyl)-1-methyl-1-(1-(5-oxo-5,6-dihydropyrido[3,4-b]pyrazin-8-yl)ethyl)urea; (R)-3-(3-chloro-4-fluorophenyl)-1-(4-hydroxybutyl)-1-(1-(1-oxo-1,2-dihydroisoquinolin-4-yl)ethyl)urea; (S)-3-(3-chloro-4-fluorophenyl)-1-(4-hydroxybutyl)-1-(1-(1-oxo-1,2-dihydroisoquinolin-4-yl)ethyl)urea; (R)-3-(3-chloro-4-fluorophenyl)-1-methyl-1-(1-(8-oxo-7,8-dihydro-1,7-naphthyridin-5-yl)ethyl)urea; (S)-3-(3-chloro-4-fluorophenyl)-1-methyl-1-(1-(8-oxo-7,8-dihydro-1,7-naphthyridin-5-yl)ethyl)urea; (R)-3-(3-chloro-4-fluorophenyl)-1-isobutyl-1-(1-(1-oxo-1,2-dihydro-2,7-naphthyridin-4-yl)ethyl)urea; (S)-3-(3-chloro-4-fluorophenyl)-1-isobutyl-1-(1-(1-oxo-1, 2-dihydro-2,7-naphthyridin-4-yl)ethyl)urea; 3-(3-chloro-4-fluorophenyl)-1-(1(R)-(1-oxo-1,2-dihydroisoquinolin-4-yl)ethyl)-1-(((R)-tetrahydrofuran-2-yl)methyl)urea; 3-(3-chloro-4-fluorophenyl)-1-(1(S)-(1-oxo-1,2-dihydroisoquinolin-4-yl)ethyl)-1-(((R)-tetrahydrofuran-2-yl)methyl)urea; (R)-1-(3-aminopropyl)-3-(3-chloro-4-fluorophenyl)-1-(1-(1-oxo-1,2-dihydroisoquinolin-4-yl)ethyl)urea; (S)-1-(3-aminopropyl)-3-(3-chloro-4-fluorophenyl)-1-(1-(1-oxo-1,2-dihydroisoquinolin-4-yl)ethyl)urea; (R)-3-(3-chloro-4-fluorophenyl)-1-(1-(1-methoxyisoquinolin-4-yl)ethyl)-1-(2-((2,2,2-trifluoroethyl)amino)ethyl)urea; (S)-3-(3-chloro-4-fluorophenyl)-1-(1-(1-methoxyisoquinolin-4-yl)ethyl)-1-(2-((2,2,2-trifluoroethyl)amino)ethyl)urea; (R)-3-(3-chloro-4-fluorophenyl)-1-(3-hydroxypropyl)-1-(1-(1-oxo-1,2-dihydro-2,7-naphthyridin-4-yl)ethyl)urea; (S)-3-(3-chloro-4-fluorophenyl)-1-(3-hydroxypropyl)-1-(1-(1-oxo-1,2-dihydro-2,7-naphthyridin-4-yl)ethyl)urea; 3-(3-chloro-4-fluorophenyl)-1-(1(R)-(1-oxo-1,2-dihydroisoquinolin-4-yl)ethyl)-1-(((S)-tetrahydrofuran-2-yl)methyl)urea; 3-(3-chloro-4-fluorophenyl)-1-(1(S)-(1-oxo-1,2-dihydroisoquinolin-4-yl)ethyl)-1-(((S)-tetrahydrofuran-2-yl)methyl)urea; (R)-3-(3-chloro-4-fluorophenyl)-1-(1-(1-oxo-1,2-dihydroisoquinolin-4-yl)ethyl)-1-(2-((2,2,2-trifluoroethyl)amino)ethyl)urea; (S)-3-(3-chloro-4-fluorophenyl)-1-(1-(1-oxo-1,2-dihydroisoquinolin-4-yl)ethyl)-1-(2-((2,2,2-trifluoroethyl)amino)ethyl)urea; 3-(3-chloro-4-fluorophenyl)-1-(1(R)-(1-oxo-1,2-dihydroisoquinolin-4-yl)ethyl)-1-(((S)-tetrahydrofuran-3-yl)methyl)urea; 3-(3-chloro-4-fluorophenyl)-1-(1(S)-(1-oxo-1,2-dihydroisoquinolin-4-yl)ethyl)-1-(((S)-tetrahydrofuran-3 -yl)methyl)urea; 3-(3-chloro-4-fluorophenyl)-1-(1(R)-(1-oxo-1,2-dihydroisoquinolin-4-yl)ethyl)-1-(((R)-tetrahydrofuran-3-yl)methyl)urea; 3-(3-chloro-4-fluorophenyl)-1-(1(S)-(1-oxo-1,2-dihydroisoquinolin-4-yl)ethyl)-1-(((R)-tetrahydrofuran-3-yl)methyl)urea; (R)-1-(1-(8-chloro-1-oxo-1,2-dihydroisoquinolin-4-yl)ethyl)-3-(3-chloro-4-fluorophenyl)-1-methylurea; (S)-1-(1-(8-chloro-1-oxo-1,2-dihydroisoquinolin-4-yl)ethyl)-3-(3-chloro-4-fluorophenyl)-1-methylurea; (R)-3-(3-chloro-4-fluorophenyl)-1-(1-(6-fluoro-1-oxo-1,2-dihydroisoquinolin-4-yl)ethyl)-1-methylurea; (S)-3-(3-chloro-4-fluorophenyl)-1-(1-(6-fluoro-1-oxo-1,2-dihydroisoquinolin-4-yl)ethyl)-1-methylurea; (R)-3-(3-chloro-4-fluorophenyl)-1-(1-(6-chloro-1-oxo-1,2-dihydroisoquinolin-4-yl)ethyl)-1-methylurea; (S)-3-(3-chloro-4-fluorophenyl)-1-(1-(6-chloro-1-oxo-1,2-dihydroisoquinolin-4-yl)ethyl)-1-methylurea; (R)-3-(3-chloro-4-fluorophenyl)-1-(1-(7-methoxy-1-oxo-1,2-dihydroisoquinolin-4-yl)ethyl)-1-methylurea; (S)-3-(3-chloro-4-fluorophenyl)-1-(1-(7-methoxy-1-oxo-1,2-dihydroisoquinolin-4-yl)ethyl)-1-methylurea; (R)-3-(3-chloro-4-fluorophenyl)-1-(1-(6-methoxy-1-oxo-1,2-dihydroisoquinolin-4-yl)ethyl)-1-methylurea; (S)-3-(3-chloro-4-fluorophenyl)-1-(1-(6-methoxy-1-oxo-1,2-dihydroisoquinolin-4-yl)ethyl)-1-methylurea; (R)-3-(3-chloro-4-fluorophenyl)-1-methyl-1-(1-(2-methyl-1-oxo-1,2-dihydroisoquinolin-4-yl)ethyl)urea; (S)-3-(3-chloro-4-fluorophenyl)-1-methyl-1-(1-(2-methyl-1-oxo-1,2-dihydroisoquinolin-4-yl)ethyl)urea; (R)-3-(3-chloro-4-fluorophenyl)-1-methyl-1-(1-(2-ethyl-1-oxo-1,2-dihydroisoquinolin-4-yl)ethyl)urea; (S)-3-(3-chloro-4-fluorophenyl)-1-methyl-1-(1-(2-ethyl-1-oxo-1,2-dihydroisoquinolin-4-yl)ethyl)urea; (R)-3-(3-chloro-4-fluorophenyl)-1-methyl-1-(1-(2-propyl-1-oxo-1,2-dihydroisoquinolin-4-yl)ethyl)urea; (S)-3-(3-chloro-4-fluorophenyl)-1-methyl-1-(1-(2-propyl-1-oxo-1,2-dihydroisoquinolin-4-yl)ethyl)urea; (R)-3-(3-chloro-4-fluorophenyl)-1-methyl-1-(1-(2-isopropyl-1-oxo-1,2-dihydroisoquinolin-4-yl)ethyl)urea; (S)-3-(3-chloro-4-fluorophenyl)-1-methyl-1-(1-(2-isopropyl-1-oxo-1,2-dihydroisoquinolin-4-yl)ethyl)urea; (R)-3-(3-chloro-4-fluorophenyl)-1-methyl-1-(1-(2-cyclopropyl-1-oxo-1,2-dihydroisoquinolin-4-yl)ethyl)urea; (S)-3-(3-chloro-4-fluorophenyl)-1-methyl-1-(1-(2-cyclopropyl-1-oxo-1,2-dihydroisoquinolin-4-yl)ethyl)urea; (R)-3-(3-chloro-4-fluorophenyl)-1-(1-(2-(2-methoxyethyl)-1-oxo-1,2-dihydroisoquinolin-4-yl)ethyl)-1-methylurea; (S)-3-(3-chloro-4-fluorophenyl)-1-(1-(2-(2-methoxyethyl)-1-oxo-1,2-dihydroisoquinolin-4-yl)ethyl)-1-methylurea; (R)-3-(3-chloro-4-fluorophenyl)-1-(cyclopentylmethyl)-1-(1-(1-oxo-1,2-dihydroisoquinolin-4-yl)ethyl)urea; (S)-3-(3-chloro-4-fluorophenyl)-1-(cyclopentylmethyl)-1-(1-(1-oxo-1,2-dihydroisoquinolin-4-yl)ethyl)urea; (R)-3-(3-chloro-4-fluorophenyl)-1-(cyclohexylmethyl)-1-(1-(1-oxo-1,2-dihydroisoquinolin-4-yl)ethyl)urea; (S)-3-(3-chloro-4-fluorophenyl)-1-(cyclohexylmethyl)-1-(1-(1-oxo-1,2-dihydroisoquinolin-4-yl)ethyl)urea; (R)-3-(3-chloro-4-fluorophenyl)-1-(2-hydroxyethyl)-1-(1-(1-oxo-1,2-dihydroisoquinolin-4-yl)ethyl)urea; (S)-3-(3-chloro-4-fluoropheny1)-1-(2-hydroxyethyl)-1-(1-(1-oxo-1,2-dihydroisoquinolin-4-yl)ethyl)urea; (R)-3-(3-chloro-4-fluorophenyl)-1-(2-((2,2-difluoroethyl)amino)ethyl)-1-(1-(1-oxo-1,2-dihydroisoquinolin-4-yl)ethyl)urea; (S)-3-(3-chloro-4-fluorophenyl)-1-(2-((2,2-difluoroethyl)amino)ethyl)-1-(1-(1-oxo-1,2-dihydroisoquinolin-4-yl)ethyl)urea; (R)-3-(3-chloro-4-fluorophenyl)-1-(1-(2-(3-methoxypropyl)-1-oxo-1,2-dihydroisoquinolin-4-yl)ethyl)-1-methylurea; (S)-3-(3-chloro-4-fluorophenyl)-1-(1-(2-(3-methoxypropyl)-1-oxo-1,2-dihydroisoquinolin-4-yl)ethyl)-1-methylurea; (R)-1-((1-acetylpiperidin-4-yl)methyl)-3-(3-chloro-4-fluorophenyl)-1-(1-(1-oxo-1,2-dihydroisoquinolin-4-yl)ethyl)urea; (S)-1-((1-acetylpiperidin-4-yl)methyl)-3-(3-chloro-4-fluorophenyl)-1-(1-(1-oxo-1,2-dihydroisoquinolin-4-yl)ethyl)urea; (R)-3-(3-chloro-4-fluorophenyl)-1-((1-(methylsulfonyl)piperidin-4-yl)methyl)-1-(1-(1-oxo-1,2-dihydroisoquinolin-4-yl)ethyl)urea; (S)-3-(3-chloro-4-fluorophenyl)-1-((1-(methylsulfonyl)piperidin-4-yl)methyl)-1-(1-(1-oxo-1,2-dihydroisoquinolin-4-yl)ethyl)urea; (R)-3-(3-chloro-4-fluorophenyl)-1-(1-(2-(2-hydroxyethyl)-l-oxo-1,2-dihydroisoquinolin-4-yl)ethyl)-1-methylurea; (S)-3-(3-chloro-4-fluorophenyl)-1-(1-(2-(2-hydroxyethyl)-1-oxo-1,2-dihydroisoquinolin-4-yl)ethyl)-1-methylurea; (R)-3-(3-chloro-4-fluorophenyl)-1-(1-(2-(3-hydroxypropyl)-1-oxo-1,2-dihydroisoquinolin-4-yl)ethyl)-1-methylurea; (S)-3-(3-chloro-4-fluorophenyl)-1-(1-(2-(3-hydroxypropyl)-1-oxo-1,2-dihydroisoquinolin-4-yl)ethyl)-1-methylurea; (R)-3-(3-chloro-4-fluorophenyl)-1-((1-methylpiperidin-4-yl)methyl)-1-(1-(1-oxo-1,2-dihydroisoquinolin-4-yl)ethyl)urea; (S)-3-(3-chloro-4-fluorophenyl)-1-((1-methylpiperidin-4-yl)methyl)-1-(1-(1-oxo-1,2-dihydroisoquinolin-4-yl)ethyl)urea; (R)-3-(3-(3-chloro-4-fluorophenyl)-1-(1-(1-oxo-1,2-dihydroisoquinolin-4-yl)ethyl)ureido)propanoic acid; (S)-3-(3-(3-chloro-4-fluorophenyl)-1-(1-(1-oxo-1-oxo-1,2-dihydroisoquinolin-4-yl)ethyl)ureido)propanoic acid; (R)-3-(3-chloro-4-fluorophenyl)-1-(3-hydroxy-2,2-dimethylpropyl)-1-(1-(1-oxo-1,2-dihydroisoquinolin-4-yl)ethyl)urea; (S)-3-(3-chloro-4-fluorophenyl)-1-(3-hydroxy-2,2-dimethylpropyl)-1-(1-(1-oxo-1,2-dihydroisoquinolin-4-yl)ethyl)urea; 3-(3-chloro-4-fluorophenyl)-1-((R)-3-hydroxybutyl)-1-(1(R)-(1-oxo-1,2-dihydroisoquinolin-4-yl)ethyl)urea; 3-(3-chloro-4-fluorophenyl)-1-((R)-3-hydroxybutyl)-1-(1(S)-(1-oxo-1,2-dihydroisoquinolin-4-yl)ethyl)urea; 3-(3-chloro-4-fluorophenyl)-1-((S)-3-hydroxybutyl)-1-(1(R)-(1-oxo-1,2-dihydroisoquinolin-4-yl)ethyl)urea; 3-(3-chloro-4-fluorophenyl)-1-((S)-3-hydroxybutyl)-1-(1(S)-(1-oxo-1,2-dihydroisoquinolin-4-yl)ethyl)urea; 3-(3-chloro-4-fluorophenyl)-1-((R)-2-hydroxypropyl)-1-(1(R)-(1-oxo-1,2-dihydroisoquinolin-4-yl)ethyl)urea; 3-(3-chloro-4-fluorophenyl)-1-((R)-2-hydroxypropyl)-1-((S)1-(1-oxo-1,2-dihydroisoquinolin-4-yl)ethyl)urea; 3-(3-chloro-4-fluorophenyl)-1-((S)-2-hydroxypropyl)-1-(1(R)-(1-oxo-1,2-dihydroisoquinolin-4-yl)ethyl)urea; 3-(3-chloro-4-fluorophenyl)-1-((S)-2-hydroxypropyl)-1-(1(S)-(1-oxo-1,2-dihydroisoquinolin-4-yl)ethyl)urea; (R)-3-(3-(3-chloro-4-fluorophenyl)-1-(1-(1-oxo-1,2-dihydroisoquinolin-4-yl)ethyl)ureido)-N-methylpropane-1-sulfonamide; (S)-3-(3-(3-chloro-4-fluorophenyl)-1-(1-(1-oxo-1,2-dihydroisoquinolin-4-yl)ethyl)ureido)-N-methylpropane-1-sulfonamide; (R)-3-(3-chloro-4-fluorophenyl)-1-(((1r,4r)-4-hydroxycyclohexyl)methyl)-1-(1-(1-oxo-1,2-dihydroisoquinolin-4-yl)ethyl)urea; (S)-3-(3-chloro-4-fluorophenyl)-1-(((1r,4r)-4-hydroxycyclohexyl)methyl)-1-(1-(1-oxo-1,2-dihydroisoquinolin-4-yl)ethyl)urea; (R)-3-(3-chloro-4-fluorophenyl)-1-methyl-1-(1-(3-methyl-1-oxo-1,2-dihydroisoquinolin-4-yl)ethyl)urea; (S)-3-(3-chloro-4-fluorophenyl)-1-methyl-1-(1-(3-methyl-1-oxo-1,2-dihydroisoquinolin-4-yl)ethyl)urea; (R)-3-(3-chloro-4-fluorophenyl)-1-((4-cis-hydroxycyclohexyl)methyl)-1-(1-(1-oxo-1,2-dihydroisoquinolin-4-yl)ethyl)urea; (S)-3-(3-chloro-4-fluorophenyl)-1-((4-cis-hydroxycyclohexyl)methyl)-1-(1-(1-oxo-1,2-dihydroisoquinolin-4-yl)ethyl)urea; (R)-4-(3-(3-chloro-4-fluorophenyl)-1-(1-(1-oxo-1,2-dihydroisoquinolin-4-yl)ethyl)ureido)butanoic acid; (S)-4-(3-(3-chloro-4-fluorophenyl)-1-(1-(1-oxo-1,2-dihydroisoquinolin-4-yl)ethyl)ureido)butanoic acid; (R)-3-(3-chloro-4-fluorophenyl)-1-methyl-1-(1-(1-oxo-2-(2,2,2-trifluoroethyl)-1,2-dihydroisoquinolin-4-yl)ethyl)urea; (S)-3-(3-chloro-4-fluorophenyl)-1-methyl-1-(1-(1-oxo-2-(2,2,2-trifluoroethyl)-1,2-dihydroisoquinolin-4-yl)ethyl)urea; (R)-3-(3-chloro-4-fluorophenyl)-1-isobutyl-1-(1-(1-oxo-2-(2,2,2-trifluoroethyl)-1,2-dihydroisoquinolin-4-yl)ethyl)urea; (S)-3-(3-chloro-4-fluorophenyl)-1-isobutyl-1-(1-(1-oxo-2-(2,2,2-trifluoroethyl)-1,2-dihydroisoquinolin-4-yl)ethyl)urea; (R)-3-(4-fluoro-3-methylphenyl)-1-methyl-1-(1-(3-methyl-1-oxo-1,2-dihydroisoquinolin-4-yl)ethyl)urea; (S)-3-(4-fluoro-3-methylphenyl)-1-methyl-1-(1-(3-methyl-1-oxo-1,2-dihydroisoquinolin-4-yl)ethyl)urea; (R)-3-(3-chloro-4-fluorophenyl)-1-methyl-1-(1-(1-(methylamino)isoquinolin-4-yl)ethyl)urea; (S)-3-(3-chloro-4-fluorophenyl)-1-methyl-1-(1-(1-(methylamino)isoquinolin-4-yl)ethyl)urea; (R)-3-(3-chloro-4-fluorophenyl)-1-methyl-1-(1-(1-(dimethylamino)isoquinolin-4-yl)ethyl)urea; (S)-3-(3-chloro-4-fluorophenyl)-1-methyl-1-(1-(1-(dimethylamino)isoquinolin-4-yl)ethyl)urea; (R)-3-(4-fluorophenyl)-1-isobutyl-1-(1-(1-oxo-2-(2,2,2-trifluoroethyl)-1,2-dihydroisoquinolin-4-yl)ethyl)urea; (S)-3-(4-fluorophenyl)-1-isobutyl-1-(1-(1-oxo-2-(2,2,2-trifluoroethyl)-1,2-dihydroisoquinolin-4-yl)ethyl)urea; (R)-3-(4-fluoro-3-methylphenyl)-1-methyl-1-(1-(3-methyl-1-oxo-1,2-dihydroisoquinolin-4-yl)ethyl)urea; (S)-3-(4-fluoro-3-methylphenyl)-1-methyl-1-(1-(3-methyl-1-oxo-1,2-dihydroisoquinolin-4-yl)ethyl)urea; (R)-1-(1-(1-aminoisoquinolin-4-yl)ethyl)-3-(3-chloro-4-fluorophenyl)-1-methylurea; (S)-1-(1-(1-aminoisoquinolin-4-yl)ethyl)-3-(3-chloro-4-fluorophenyl)-1-methylurea; (R)-3-(3-chloro-4-fluorophenyl)-1-(1-(1-(ethylamino)isoquinolin-4-yl)ethyl)-1-methylurea; (S)-3-(3-chloro-4-fluorophenyl)-1-(1-(1-(ethylamino)isoquinolin-4-yl)ethyl)-1-methylurea; (R)-3-(3-chloro-4-ftuorophenyl)-1-(1-(1-((2-hydroxyethyl)amino)isoquinolin-4-yl)ethyl)-1-methylurea; (S)-3-(3-chloro-4-fluorophenyl)-1-(1-(1-((2-hydroxyethyl)amino)isoquinolin-4-yl)ethyl)-1-methylurea; (R)-1-(1-(1-((2-aminoethyl)amino)isoquinolin-4-yl)ethyl)-3-(3-chloro-4-fluorophenyl)-1-methylurea; (S)-1-(1-(1-((2-aminoethyl)amino)isoquinolin-4-yl)ethyl)-3-(3-chloro-4-fluorophenyl)-1-methylurea; (R)-3-(3-chloro-4-fluorophenyl)-1-methyl-1-(1-(1-(((1-methyl-1H-1,2,4-triazol-3-yl)methyl)amino)isoquinolin-4-yl)ethyl)urea; (S)-3-(3-chloro-4-fluorophenyl)-1-methyl-1-(1-(1-(((1-methyl-1H-1,2,4-triazol-3-yl)methyl)amino)isoquinolin-4-yl)ethyl)urea; (R)-1-(1-(1-(((1H-1,2,4-triazol-3-yl)methyl)amino)isoquinolin-4-yl)ethyl)-3-(3-chloro-4-fluorophenyl)-1-methylurea; (S)-1-(1-(1-(((1H-1,2,4-triazol-3-yl)methyl)amino)isoquinolin-4-yl)ethyl)-3-(3-chloro-4-fluorophenyl)-1-methylurea; (R)-3-(3-chloro-4-fluorophenyl)-1-(3-hydroxypropyl)-1-(1-(1-((1-methyl-1H-1,2,4-triazol-3-yl)methoxy)isoquinolin-4-yl)ethyl)urea; (S)-3-(3-chloro-4-fluorophenyl)-1-(3-hydroxypropyl)-1-(1-(1-((1-methyl-1H-1,2,4-triazol-3-yl)methpxy)isoquinolin-4-yl)ethyl)urea; (R)-3-(3-chloro-4-fluorophenyl)-1-(1-(7-fluoro-1-oxo-1,2-dihydroisoquinolin-4-yl)ethyl)-1-methylurea; (S)-3-(3-chloro-4-fluorophenyl)-1-(1-(7-fluoro-1-oxo-1,2-dihydroisoquinolin-4-yl)ethyl)-1-methylurea; (R)-1-(1-(7-fluoro-1-oxo-1,2-dihydroisoquinolin-4-yl)ethyl)-3-(4-fluorophenyl)-1-methylurea; (S)-1-(1-(7-fluoro-1-oxo-1,2-dihydroisoquinolin-4-yl)ethyl)-3-(4-fluorophenyl)-1-methylurea; (R)-1-(1-(1-((1H-1,2,4-triazol-3-yl)methoxy)isoquinolin-4-yl)ethyl)-3-(3-chloro-4-fluorophenyl)-1-(3-hydroxypropyl)urea; (S)-1-(1-(1-((1H-1,2,4-triazol-3-yl)methoxy)isoquinolin-4-yl)ethyl)-3-(3-chloro-4-fluorophenyl)-1-(3-hydroxypropyl)urea; (R)-1-(1-(1-(((2H-1,2,3-triazol-4-yl)methyl)amino)isoquinolin-4-yl)ethyl)-3-(3-chloro-4-fluorophenyl)-1-methylurea; (S)-1-(1-(1-(((2H-1,2,3-triazol-4-yl)methyl)amino)isoquinolin-4-yl)ethyl)-3-(3-chloro-4-fluorophenyl)-1-methylurea; (R)-3-(3-chloro-4-fluorophenyl)-1-(1-(5-methoxy-1-oxo-1,2-dihydroisoquinolin-4-yl)ethyl)-1-methylurea; (S)-3-(3-chloro-4-fluorophenyl)-1-(1-(5-methoxy-1-oxo-1,2-dihydroisoquinolin-4-yl)ethyl)-1-methylurea; (R)-1-((1H-1,2,3-triazol-4-yl)methyl)-3-(3-chloro-4-fluorophenyl)-1-(1-(1-oxo-1,2-dihydroisoquinolin-4-yl)ethyl)urea; (S)-1-((1H-1,2,3-triazol-4-yl)methyl)-3-(3-chloro-4-fluorophenyl)-1-(1-(1-oxo-1,2-dihydroisoquinolin-4-yl)ethyl)urea; (R)-1-((4H-1,2,4-triazol-3-yl)methyl)-3-(3-chloro-4-fluorophenyl)-1-(1-(1-oxo-1,2-dihydroisoquinolin-4-yl)ethyl)urea; (S)-1-((4H-1,2,4-triazol-3-yl)methyl)-3-(3-chloro-4-fluorophenyl)-1-(1-(1-oxo-1,2-dihydroisoquinolin-4-yl)ethyl)urea; (R)-1-(1-(2-((1H-1,2,4-triazol-3-yl)methyl)-1-oxo-1,2-dihydroisoquinolin-4-yl)ethyl)-3-(3-chloro-4-fluorophenyl)-1-methylurea; (S)-1-(1-(2-((1H-1,2,4-triazol-3-yl)methyl)-1-oxo-1,2-dihydroisoquinolin-4-yl)ethyl)-3-(3-chloro-4-fluorophenyl)-1-methylurea; (R)-1-(1-(7-chloro-1-oxo-1,2-dihydroisoquinolin-4-yl)ethyl)-3-(3-chloro-4-fluorophenyl)-1-methylurea; (S)-1-(1-(7-chloro-1-oxo-1,2-dihydroisoquinolin-4-yl)ethyl)-3-(3-chloro-4-fluorophenyl)-1-methylurea; (R)-3-(3-chloro-4-fluorophenyl)-1-((2,2-dimethyl-1,3-dioxan-5-yl)methyl)-1-(1-(1-oxo-1,2-dihydroisoquinolin-4-yl)ethyl)urea; (S)-3-(3-chloro-4-fluorophenyl)-1-((2,2-dimethyl-1,3-dioxan-5-yl)methyl)-1-(1-(1-oxo-1,2-dihydroisoquinolin-4-yl)ethyl)urea; (R)-1-((2,2-dimethyl-1,3-dioxan-5-yl)methyl)-3-(4-fluorophenyl)-1-(1-(1-oxo-1,2-dihydroisoquinolin-4-yl)ethyl)urea; (S)-1-((2,2-dimethyl-1,3-dioxan-5-yl)methyl)-3-(4-fluorophenyl)-1-(1-(1-oxo-1,2-dihydroisoquinolin-4-yl)ethyl)urea; (R)-1-((2,2-dimethyl-1,3-dioxan-5-yl)methyl)-1-(1-(1-oxo-1,2-dihydroisoquinolin-4-yl)ethyl)-3-phenylurea; (S)-1-((2,2-dimethyl-1,3-dioxan-5-yl)methyl)-1-(1-(1-oxo-1,2-dihydroisoquinolin-4-yl)ethyl)-3-phenylurea; (R)-1-(1-(2-((1H-1,2,3-triazol-4-yl)methyl)-1-oxo-1,2-dihydroisoquinolin-4-yl)ethyl)-3-(3-chloro-4-fluorophenyl)-1-methylurea; (S)-1-(1-(2-((1H-1,2,3-triazol-4-yl)methyl)-1-oxo-1,2-dihydroisoquinolin-4-yl)ethyl)-3-(3-chloro-4-fluorophenyl)-1-methylurea; (R)-3-(3-chloro-4-fluorophenyl)-1-(1-(6,7-difluoro-1-oxo-1,2-dihydroisoquinolin-4-yl)ethyl)-1-methylurea; (S)-3-(3-chloro-4-fluorophenyl)-1-(1-(6,7-difluoro-1-oxo-1,2-dihydroisoquinolin-4-yl)ethyl)-1-methylurea; (R)-3-(3-chloro-4-fluorophenyl)-1-(1-(6,7-difluoro-1-oxo-1,2-dihydroisoquinolin-4-yl)ethyl)-1-isobutylurea; (S)-3-(3-chloro-4-fluorophenyl)-1-(1-(6,7-difluoro-1-oxo-1,2-dihydroisoquinolin-4-yl)ethyl)-1-isobutylurea; (R)-3-(3-chloro-4-fluorophenyl)-1-((2,2-dimethyl-1,3-dioxan-5-yl)methyl)-1-(1-(6-fluoro-1-oxo-1,2-dihydroisoquinofin-4-yl)ethyl)urea; (S)-3-(3-chloro-4-fluorophenyl)-1-((2,2-dimethyl-1,3-dioxan-5-yl)methyl)-1-(1-(6-fluoro-1-oxo-1,2-dihydroisoquinolin-4-yl)ethyl)urea; (R)-3-(3-chloro-4-fluorophenyl)-1-(3-hydroxy-2-(hydroxymethyl)propyl)-1-(1-(1-oxo-1,2-dihydroisoquinolin-4-yl)ethyl)urea; (S)-3-(3-chloro-4-fluorophenyl)-1-(3-hydroxy-2-(hydroxymethyl)propyl)-1-(1-(1-oxo-1,2-dihydroisoquinolin-4-yl)ethyl)urea; (R)-1-(1-(6,7-difluoro-1-oxo-1,2-dihydroisoquinolin-4-yl)ethyl)-3-(4-fluorophenyl)-1-methylurea; (S)-1-(1-(6,7-difluoro-1-oxo-1,2-dihydroisoquinolin-4-yl)ethyl)-3-(4-fluorophenyl)-1-methylurea; (R)-1-(1-(6,7-difluoro-1-oxo-1,2-dihydroisoquinolin-4-yl)ethyl)-3-(4-fluorophenyl)-1-isobutylurea; (S)-1-(1-(6,7-difluoro-1-oxo-1,2-dihydroisoquinolin-4-yl)ethyl)-3-(4-fluorophenyl)-1-isobutylurea; (R)-3-(3-chloro-4-fluorophenyl)-1-(1-(6,7-difluoro-1-oxo-1,2-dihydroisoquinolin-4-yl)ethyl)-1-(3-hydroxypropyl)urea; (S)-3-(3-chloro-4-fluorophenyl)-1-(1-(6,7-difluoro-1-oxo-1,2-dihydroisoquinolin-4-yl)ethy1)-1-(3-hydroxypropyl)urea; (R)-1-(1-(6,7-difluoro-1-oxo-1,2-dihydroisoquinolin-4-yl)ethyl)-3-(4-fluorophenyl)-l-(3-hydroxypropyl)urea; (S)-1-(1-(6,7-difluoro-1-oxo-1,2-dihydroisoquinolin-4-yl)ethyl)-3-(4-fluorophenyl)-1-(3-hydroxypropyl)urea; (R)-3-(4-chlorophenyl)-1-methyl-1-(1-(1-oxo-1,2-dihydroisoquinolin-4-yl)ethyl)urea; (S)-3-(4-chlorophenyl)-1-methyl-1-(1-(1-oxo-1,2-dihydroisoquinolin-4-yl)ethyl)urea; (R)-3-(4-bromophenyl)-1-methyl-1-(1-(1-oxo-1,2-dihydroisoquinolin-4-yl)ethyl)urea; (S)-3-(4-bromophenyl)-1-methyl-1-(1-(1-oxo-1,2-dihydroisoquinolin-4-yl)ethyl)urea; (R)-3-(3-chloro-4-fluorophenyl)-1-(1-(6-fluoro-1-oxo-1,2-dihydroisoquinolin-4-yl)ethyl)-1-(3-hydroxy-2-(hydroxymethyl)propyl)urea; (S)-3-(3-chloro-4-fluorophenyl)-1-(1-(6-fluoro-1-oxo-1,2-dihydroisoquinolin-4-yl)ethyl)-1-(3-hydroxy-2-(hydroxymethyl)propyl)urea; (R)-1-(1-(1-(1H-1,2,4-triazol-1-yl)isoquinolin-4-yl)ethyl)-3-(3-chloro-4-fluorophenyl)-1-methylurea; (S)-1-(1-(1-(1H-1,2,4-triazol-1-yl)isoquinolin-4-yl)ethyl)-3-(3-chloro-4-fluorophenyl)-1-methylurea; (R)-1,3-dimethyl-1-(1-(1-oxo-1,2-dihydroisoquinolin-4-yl)ethyl)urea; (S)-1,3-dimethyl-1-(1-(1-oxo-1,2-dihydroisoquinolin-4-yl)ethyl)urea; (R)-3-cyclopentyl-1-methyl-1-(1-(1-oxo-1,2-dihydroisoquinolin-4-yl)ethyl)urea; (S)-3-cyclopentyl-1-methyl-1-(1-(1-oxo-1,2-dihydroisoquinolin-4-yl)ethyl)urea; (R)-1-(1-(1-(1H-1,2,4-triazol-1-yl)isoquinolin-4-yl)ethyl)-3-(3-chloro-4-fluorophenyl)-1-isobutylurea; (S)-1-(1-(1-(1H-1,2,4-triazol-1-yl)isoquinofin-4-yl)ethyl)-3-(3-chloro-4-fluorophenyl)-1-isobutylurea; (R)-1-(1-(1-(1H-1,2,4-triazol-1-yl)isoquinolin-4-yl)ethyl)-3-(4-fluorophenyl)-1-isobutylurea; (S)-1-(1-(1-(1H-1,2,4-triazol-1-yl)isoquinolin-4-yl)ethyl)-3-(4-fluorophenyl)-1-isobutylurea; (R)-3-(3-chloro-4-fluorophenyl)-1-(1-(7-fluoro-2-methyl-1-oxo-1,2-dihydroisoquinolin-4-yl)ethyl)-1-methylurea; (S)-3-(3-chloro-4-fluorophenyl)-1-(1-(7-fluoro-2-methyl-1-oxo-1,2-dihydroisoquinolin-4-yl)ethyl)-1-methylurea; (R)-3-(3-chloro-4-fluorophenyl)-1-(1-(1-oxo-1,2-dihydroisoquinolin-4-yl)ethyl)-1-(pyridin-2-ylmethyl)urea; (S)-3-(3-chloro-4-fluorophenyl)-1-(1-(1-oxo-1,2-dihydroisoquinolin-4-yl)ethyl)-1-(pyridin-2-ylmethyl)urea; (R)-3-(3-chloro-4-fluorophenyl)-1-(1-(1-oxo-1,2-dihydroisoquinolin-4-yl)ethyl)-1-(pyridin-3-ylmethyl)urea; (S)-3-(3-chloro-4-fluorophenyl)-1-(1-(1-oxo-1,2-dihydroisoquinolin-4-yl)ethyl)-1-(pyridin-3-ylmethyl)urea; (R)-3-(3-chloro-4-fluorophenyl)-1-(1-(1-oxo-1,2-dihydroisoquinolin-4-yl)ethyl)-1-(pyridin-4-ylmethyl)urea; (S)-3-(3-chloro-4-fluorophenyl)-1-(1-(1-oxo-1,2-dihydroisoquinolin-4-yl)ethyl)-1-(pyridin-4-ylmethyl)urea; (R)-3-(3-chloro-4-fluorophenyl)-1-(1-(1-oxo-1,2-dihydroisoquinolin-4-yl)ethyl)-1-(thiazol-2-ylmethyl)urea; (S)-3-(3-chloro-4-fluorophenyl)-1-(1-(1-oxo-1,2-dihydroisoquinolin-4-yl)ethyl)-1-(thiazol-2-ylmethyl)urea; (R)-3-(3-chloro-4-fluorophenyl)-1-(1-(1-oxo-1,2-dihydroisoquinolin-4-yl)ethyl)-1-(thiazol-4-ylmethyl)urea; (S)-3-(3-chloro-4-fluorophenyl)-1-(1-(1-oxo-1,2-dihydroisoquinolin-4-yl)ethyl)-1-(thiazol-4-ylmethyl)urea; (R)-3-(3-chloro-4-fluorophenyl)-1-methyl-1-(1-(1-(methylsulfonyl)isoquinolin-4-yl)ethyl)urea; (S)-3-(3-chloro-4-fluorophenyl)-1-methyl-1-(1-(1-(methylsulfonyl)isoquinolin-4-yl)ethyl)urea; (R)-4-(1-(3-(3-chloro-4-fluorophenyl)-1-methylureido)ethyl)-N-methylisoquinoline-1-carboxamide; (S)-4-(1-(3-(3-chloro-4-fluorophenyl)-1-methylureido)ethyl)-N-methylisoquinoline-1-carboxamide; (R)-4-(1-(3-(3-chloro-4-fluorophenyl)-1 -methylureido)ethyl)-N,N-dimethylisoquinoline-1 -carboxamide; (S)-4-(1-(3-(3-chloro-4-fluorophenyl)-1-methylureido)ethyl)-N,N-dimethylisoquinoline-1-carboxamide; (R)-3-(3-chloro-4-fluorophenyl)-1-(1-(1-oxo-1,2-dihydroisoquinolin-4-yl)ethyl)-1-(pyrimidin-5-ylmethyl)urea; (S)-3-(3-chloro-4-fluorophenyl)-1-(1-(1-oxo-1,2-dihydroisoquinolin-4-yl)ethyl)-1-(pyrimidin-5-ylmethyl)urea; (R)-3-(3-chloro-4-fluorophenyl)-1-(1-(1-oxo-1,2-dihydroisoquinolin-4-yl)ethyl)-1-(pyrimidin-4-ylmethyl)urea; (S)-3-(3-chloro-4-fluorophenyl)-1-(1-(1-oxo-1,2-dihydroisoquinolin-4-yl)ethyl)-1-(pyrimidin-4-ylmethyl)urea; (R)-3-(3-chloro-4-fluorophenyl)-1-(1-(1-oxo-1,2-dihydroisoquinolin-4-yl)ethyl)-1-(thiazol-5-ylmethyl)urea; (S)-3-(3-chloro-4-fluorophenyl)-1-(1-(1-oxo-1,2-dihydroisoquinolin-4-yl)ethyl)-1-(thiazol-5-ylmethyl)urea; (R)-4-(1-(3-(3-chloro-4-fluorophenyl)-1-methylureido)ethyl)isoquinoline-1-carboxylic acid; (S)-4-(1-(3-(3-chloro-4-fluorophenyl)-1-methylureido)ethyl)isoquinoline-1-carboxylic acid; (R)-3-(3-chloro-4-fluorophenyl)-1-(1-(1-(hydroxymethyl)isoquinolin-4-yl)ethyl)-1-methylurea; (S)-3-(3-chloro-4-fluorophenyl)-1-(1-(1-(hydroxymethyl)isoquinolin-4-yl)ethyl)-1-methylurea; (R)-4-(1-(3-(3-chloro-4-fluorophenyl)-1-methylureido)ethyl)isoquinoline-1-carboxamide; (S)-4-(1-(3-(3-chloro-4-fluorophenyl)-1-methylureido)ethyl)isoquinoline-1-carboxamide; (R)-3-(3-chloro-4-fluorophenyl)-1-(1-(7,8-difluoro-1-oxo-1,2-dihydroisoquinolin-4-yl)ethyl)-1-methylurea; (S)-3-(3-chloro-4-fluorophenyl)-1-(1-(7,8-difluoro-1-oxo-1,2-dihydroisoquinolin-4-yl)ethyl)-1-methylurea; (R)-3-(3-chloro-4-fluorophenyl)-1-(1-(7,8-difluoro-2-methyl-1-oxo-1,2-dihydroisoquinolin-4-yl)ethyl)-1-methylurea; (S)-3-(3-chloro-4-fluorophenyl)-1-(1-(7,8-difluoro-2-methyl-1-oxo-1,2-dihydroisoquinolin-4-yl)ethyl)-1-methylurea; (R)-3-(3-chloro-4-fluorophenyl)-1-(1-(7,8-difluoro-1-oxo-1,2-dihydroisoquinolin-4-yl)ethyl)-1-(3-hydroxypropyl)urea; (S)-3-(3-chloro-4-fluorophenyl)-1-(1-(7,8-difluoro-1-oxo-1,2-dihydroisoquinolin-4-yl)ethyl)-1-(3-hydroxypropyl)urea; (R)-3-(3-chloro-4-fluorophenyl)-1-(1-(1-cyanoisoquinolin-4-yl)ethyl)-1-methylurea; (S)-3-(3-chloro-4-fluorophenyl)-1-(1-(1-cyanoisoquinolin-4-yl)ethyl)-1-methylurea; (R)-3-(3-chloro-4-fluorophenyl)-1-(1-(7,8-difluoro-1-oxo-1,2-dihydroisoquinolin-4-yl)ethyl)-1-isobutylurea; (S)-3-(3-chloro-4-fluorophenyl)-1-(1-(7,8-difluoro-1-oxo-1,2-dihydroisoquinolin-4-yl)ethyl)-1-isobutylurea; (R)-3-(3-chloro-4-fluorophenyl)-1-(1-(6,7-difluoro-2-methyl-1-oxo-1,2-dihydroisoquinolin-4-yl)ethyl)-1-methylurea; (S)-3-(3-chloro-4-fluorophenyl)-1-(1-(6,7-difluoro-2-methyl-1-oxo-1,2-dihydroisoquinolin-4-yl)ethyl)-1-methylurea; (R)-3-(3-chloro-4-fluorophenyl)-1-(1-(6,7-difluoro-2-methyl-1-oxo-1,2-dihydroisoquinolin-4-yl)ethyl)-1-isobutylurea; (S)-3-(3-chloro-4-fluorophenyl)-1-(1-(6,7-difluoro-2-methyl-1-oxo-1,2-dihydroisoquinolin-4-yl)ethyl)-1-isobutylurea; (R)-3-(3-chloro-4-fluorophenyl)-1-(1-(6,7-difluoro-1-((1-methyl-1H-1,2,4-triazol-3-yl)methoxy)isoquinolin-4-yl)ethyl)-1-methylurea; (S)-3-(3-chloro-4-fluorophenyl)-1-(1-(6,7-difluoro-1-((1-methyl-1H-1,2,4-triazol-3-yl)methoxy)isoquinolin-4-yl)ethyl)-1-methylurea; (R)-3-(3-chloro-4-fluorophenyl)-1-(1-(6,7-difluoro-1-((1-methyl-1H-1,2,4-triazol-3-yl)methoxy)isoquinolin-4-yl)ethyl)-1-(3-hydroxypropyl)urea; (S)-3-(3-chloro-4-fluorophenyl)-1-(1-(6,7-difluoro-1-((1-methyl-1H-1,2,4-triazol-3-yl)methoxy)isoquinolin-4-yl)ethyl)-1-(3-hydroxypropyl)urea; (R)-3-(3-chloro-4-fluorophenyl)-1-(1-(6,7-difluoro-2-methyl-1-oxo-1,2-dihydroisoquinolin-4-yl)ethyl)-1-(3-hydroxypropyl)urea; (S)-3-(3-chloro-4-fluorophenyl)-1-(1-(6,7-difluoro-2-methyl-1-oxo-1,2-dihydroisoquinolin-4-yl)ethyl)-1-(3-hydroxypropyl)urea; (R)-3-(3-chloro-4-fluorophenyl)-1-methyl-1-(1-(1-methylisoquinolin-4-yl)ethyl)urea; (S)-3-(3-chloro-4-fluorophenyl)-1-methyl-1-(1-(1-methylisoquinolin-4-yl)ethyl)urea; (R)-1-(1-(1-(1H-1,2,3-triazol-1-yl)isoquinolin-4-yl)ethyl)-3-(3-chloro-4-fluorophenyl)-1-methylurea; (S)-1-(1-(1-(1H-1,2,3-triazol-1-yl)isoquinolin-4-yl)ethyl)-3-(3-chloro-4-fluorophenyl)-1-methylurea; (R)-1-(1-(1-(1H-1,2,3-triazol-1-yl)isoquinolin-4-yl)ethyl)-3-(3-chloro-4-fluorophenyl)-1-isobutylurea; (S)-1-(1-(1-(1H-1,2,3-triazol-1-yl)isoquinolin-4-yl)ethyl)-3-(3-chloro-4-fluorophenyl)-1-isobutylurea; (R)-3-(3-chloro-4-fluorophenyl)-1-(1-(8-fluoro-1-oxo-1,2-dihydroisoquinolin-4-yl)ethyl)-1-methylurea; (S)-3-(3-chloro-4-fluorophenyl)-1-(1-(8-fluoro-1-oxo-1,2-dihydroisoquinolin-4-yl)ethyl)-1-methylurea; (R)-3-(3-chloro-4-fluorophenyl)-1-(1-(8-fluoro-2-methyl-1-oxo-1,2-dihydroisoquinolin-4-yl)ethyl)-1-methylurea; (S)-3-(3-chloro-4-fluorophenyl)-1-(1-(8-fluoro-2-methyl-1-oxo-1,2-dihydroisoquinolin-4-yl)ethyl)-1-methylurea; (R)-3-(3-chloro-4-fluorophenyl)-1-(1-(8-fluoro-1-oxo-1,2-dihydroisoquinolin-4-yl)ethyl)-1-isobutylurea; (S)-3-(3-chloro-4-fluorophenyl)-1-(1-(8-fluoro-1-oxo-1,2-dihydroisoquinolin-4-yl)ethyl)-1-isobutylurea; (R)-1-(1-(1-((1H-1,2,4-triazol-3-yl)methoxy)-6,7-difluoroisoquinolin-4-yl)ethyl)-3-(3-chloro-4-fluorophenyl)-1-methylurea; (S)-1-(1-(1-((1H-1,2,4-triazol-3-yl)methoxy)-6,7-difluoroisoquinolin-4-yl)ethyl)-3-(3-chloro-4-fluorophenyl)-1-methylurea; (R)-1-(1-(1-((1H-1,2,4-triazol-3-yl)methoxy)-6,7-difluoroisoquinolin-4-yl)ethyl)-3-(3-chloro-4-fluorophenyl)-1-(3-hydroxypropyl)urea; (S)-1-(1-(1-((1H-1,2,4-triazol-3-yl)methoxy)-6,7-difluoroisoquinolin-4-yl)ethyl)-3-(3-chloro-4-fluorophenyl)-1-(3-hydroxypropyl)urea; (R)-3-(3-chloro-4-fluorophenyl)-1-(1-(8-fluoro-1-oxo-1,2-dihydroisoquinolin-4-yl)ethyl)-1-(3-hydroxypropyl)urea; (S)-3-(3-chloro-4-fluorophenyl)-1-(1-(8-fluoro-1-oxo-1,2-dihydroisoquinolin-4-yl)ethyl)-1-(3-hydroxypropyl)urea; (R)-3-(3-chloro-4-fluorophenyl)-1-(1-(6,8-difluoro-1-oxo-1,2-dihydroisoquinolin-4-yl)ethyl)-1-methylurea; (S)-3-(3-chloro-4-fluorophenyl)-1-(1-(6,8-difluoro-1-oxo-1,2-dihydroisoquinolin-4-yl)ethyl)-1-methylurea; (R)-1-(1-(6,8-difluoro-1-oxo-1,2-dihydroisoquinolin-4-yl)ethyl)-3-(4-fluorophenyl)-1-methylurea; (S)-1-(1-(6,8-difluoro-1-oxo-1,2-dihydroisoquinolin-4-yl)ethyl)-3-(4-fluorophenyl)-1-methylurea; (R)-3-(3-chloro-4-fluorophenyl)-1-(1-(6,8-difluoro-1-oxo-1,2-dihydroisoquinolin-4-yl)ethyl)-1-(3-hydroxypropyl)urea; (S)-3-(3-chloro-4-fluorophenyl)-1-(1-(6,8-difluoro-1-oxo-1,2-dihydroisoquinolin-4-yl)ethyl)-1-(3-hydroxypropyl)urea; (R)-1-(1-(6,8-difluoro-1-oxo-1,2-dihydroisoquinolin-4-yl)ethyl)-3-(4-fluorophenyl)-1-isobutylurea; (S)-1-(1-(6,8-difluoro-1-oxo-1,2-dihydroisoquinolin-4-yl)ethyl)-3-(4-fluorophenyl)-1-isobutylurea; (R)-1-(1-(6,8-difluoro-1-oxo-1,2-dihydroisoquinolin-4-yl)ethyl)-3-(4-fluorophenyl)-1-(3-hydroxypropyl)urea; (S)-1-(1-(6,8-difluoro-1-oxo-1,2-dihydroisoquinolin-4-yl)ethyl)-3-(4-fluorophenyl)-1-(3-hydroxypropyl)urea; (R)-3-(3-chloro-4-fluorophenyl)-1-(2-hydroxy-1-(1-oxo-1,2-dihydroisoquinolin-4-yl)ethyl)-1-methylurea; (S)-3-(3-chloro-4-fluorophenyl)-1-(2-hydroxy-1-(1-oxo-1,2-dihydroisoquinolin-4-yl)ethyl)-1-methylurea; (R)-2-(3-(3-chloro-4-fluorophenyl)-1-(1-(6-fluoro-1-oxo-1,2-dihydroisoquinolin-4-yl)ethyl)ureido)ethane-1-sulfonamide; (S)-2-(3-(3-chloro-4-fluorophenyl)-1-(1-(6-fluoro-1-oxo-1,2-dihydroisoquinolin-4-yl)ethyl)ureido)ethane-1-sulfonamide; (R)-2-(3-(3-chloro-4-fluorophenyl)-1-(1-(7-fluoro-1-oxo-1,2-dihydroisoquinolin-4-yl)ethyl)ureido)ethane-1-sulfonamide; (S)-2-(3-(3-chloro-4-fluorophenyl)-1-(1-(7-fluoro-1-oxo-1,2-dihydroisoquinolin-4-yl)ethyl)ureido)ethane-1-sulfonamide; (R)-2-(3-(3-chloro-4-fluorophenyl)-1-(1-(6,7-difluoro-1-oxo-1,2-dihydroisoquinolin-4-yl)ethyl)ureido)ethane-1-sulfonamide; (S)-2-(3-(3-chloro-4-fluorophenyl)-1-(1-(6,7-difluoro-1-oxo-1,2-dihydroisoquinolin-4-yl)ethyl)ureido)ethane-1-sulfonamide; (R)-2-(1-(1-(6,7-difluoro-1-oxo-1,2-dihydroisoquinolin-4-yl)ethyl)-3-(4-fluorophenyl)ureido)ethane-1-sulfonamide; (S)-2-(1-(1-(6,7-difluoro-1-oxo-1,2-dihydroisoquinolin-4-yl)ethyl)-3-(4-fluorophenyl)ureido)ethane-1-sulfonamide; (R)-2-(3-(3-chloro-4-fluorophenyl)-1-(1-(6,8-difluoro-1-oxo-1,2-dihydroisoquinolin-4-yl)ethyl)ureido)ethane-1-sulfonamide; (S)-2-(3-(3-chloro-4-fluoropheny1)-1-(1-(6,8-difluoro-1-oxo-1,2-dihydroisoquinolin-4-yl)ethyl)ureido)ethane-1-sulfonamide; (R)-2-(3-(3-chloro-4-fluorophenyl)-1-(1-(7,8-difluoro-1-oxo-1,2-dihydroisoquinolin-4-yl)ethyl)ureido)ethane-1-sulfonamide; (S)-2-(3-(3-chloro-4-fluorophenyl)-1 -(1 -(7,8-difluoro-1 -oxo-1,2-dihydroisoquinolin-4-yl)ethyl)ureido)ethane-1 - sulfonamide; (R)-2-(3-(4-fluorophenyl)-1-(1-(7,8-difluoro-1-oxo-1,2-dihydroisoquinolin-4-yl)ethyl)ureido)ethane-1-sulfonamide; (S)-2-(3-(4-fluorophenyl)-1-(1-(7,8-difluoro-1-oxo-1,2-dihydroisoquinolin-4-yl)ethyl)ureido)ethane-1-sulfonamide; (R)-2-(3-(3-chloro-4-fluorophenyl)-1-(1-(8-fluoro-1-oxo-1,2-dihydroisoquinolin-4-yl)ethyl)ureido)ethane-1-sulfonamide; (S)-2-(3-(3-chloro-4-fluorophenyl)-1-(1-(8-fluoro-1-oxo-1,2-dihydroisoquinolin-4-yl)ethyl)ureido)ethane-1-sulfonamide; (R)-1-(1-(6,7-difluoro-1-oxo-1,2-dihydroisoquinolin-4-yl)ethyl)-1-methyl-3-phenylurea; (S)-1-(1-(6,7-difluoro-1-oxo-1,2-dihydroisoquinolin-4-yl)ethyl)-1-methyl-3-phenylurea; (R)-3-(4-chlorophenyl)-1-(1-(6,7-difluoro-1-oxo-1,2-dihydroisoquinolin-4-yl)ethyl)-1-methylurea; (S)-3-(4-chlorophenyl)-1-(1-(6,7-difluoro-1-oxo-1,2-dihydroisoquinolin-4-yl)ethyl)-1-methylurea; (R)-1-(1-(6,7-difluoro-1-oxo-1,2-dihydroisoquinolin-4-yl)ethyl)-1-ethyl-3-(4-fluorophenyl)urea; (S)-1-(1-(6,7-difluoro-1-oxo-1,2-dihydroisoquinolin-4-yl)ethyl)-1-ethyl-3-(4-fluorophenyl)urea; (R)-1-(1-(6,7-difluoro-1-oxo-1,2-dihydroisoquinolin-4-yl)ethyl)-1-ethyl-3-phenylurea; (S)-1-(1-(6,7-difluoro-1-oxo-1,2-dihydroisoquinolin-4-yl)ethyl)-1-ethyl-3-phenylurea; (R)-3-(3-chloro-4-fluorophenyl)-1-(1-(6,7-difluoro-1-oxo-1,2-dihydroisoquinolin-4-yl)ethyl)-1-ethylurea; (S)-3-(3-chloro-4-fluorophenyl)-1-(1-(6,7-difluoro-1-oxo-1,2-dihydroisoquinolin-4-yl)ethyl)-1-ethylurea; (R)-1-(1-(6,7-difluoro-1-oxo-1,2-dihydroisoquinolin-4-yl)ethyl)-3-(3,4-difluorophenyl)-1-methylurea; (S)-1-(1-(6,7-difluoro-1-oxo-1,2-dihydroisoquinolin-4-yl)ethyl)-3-(3,4-difluorophenyl)-1-methylurea; (R)-1-(1-(6,7-difluoro-1-oxo-1,2-dihydroisoquinolin-4-yl)ethyl)-3-(3-fluorophenyl)-1-methylurea; (S)-1-(1-(6,7-difluoro-1-oxo-1,2-dihydroisoquinolin-4-yl)ethyl)-3-(3-fluorophenyl)-1-methylurea; (R)-3-(3-chlorophenyl)-1-(1-(6,7-difluoro-1-oxo-1,2-dihydroisoquinolin-4-yl)ethyl)-1-methylurea; (S)-3-(3-chlorophenyl)-1-(1-(6,7-difluoro-1-oxo-1,2-dihydroisoquinolin-4-yl)ethyl)-1-methylurea; (R)-1-(1-(6,7-difluoro-1-oxo-1,2-dihydroisoquinolin-4-yl)ethyl)-1-methyl-3-(3,4,5-trifluorophenyl)urea; (S)-1-(1-(6,7-difluoro-1-oxo-1,2-dihydroisoquinolin-4-yl)ethyl)-1-methyl-3-(3,4,5-trifluorophenyl)urea; (R)-3-(3,5-dichlorophenyl)-1-(1-(6,7-difluoro-1-oxo-1,2-dihydroisoquinolin-4-yl)ethyl)-1-methylurea; (S)-3-(3,5-dichlorophenyl)-1-(1-(6,7-difluoro-1-oxo-1,2-dihydroisoquinolin-4-yl)ethyl)-1-methylurea; (R)-3-(2-chloropyridin-4-yl)-1-(1-(6,7-difluoro-1-oxo-1,2-dihydroisoquinolin-4-yl)ethyl)-1-methylurea; (S)-3-(2-chloropyridin-4-yl)-1-(1-(6,7-difluoro-1-oxo-1,2-dihydroisoquinolin-4-yl)ethyl)-1-methylurea; (R)-1-(1-(6,7-difluoro-1-oxo-1,2-dihydroisoquinolin-4-yl)ethyl)-3-(2-fluorophenyl)-1-methylurea; (S)-1-(1-(6,7-difluoro-1-oxo-1,2-dihydroisoquinolin-4-yl)ethyl)-3-(2-fluorophenyl)-1-methylurea; (R)-1-(1-(6,7-difluoro-1-oxo-1,2-dihydroisoquinolin-4-yl)ethyl)-3-(3,4-difluorophenyl)-1-ethylurea; (S)-1-(1-(6,7-difluoro-1-oxo-1,2-dihydroisoquinolin-4-yl)ethyl)-3-(3,4-difluorophenyl)-1-ethylurea; (R)-1-(1-(6,7-Difluoro-1-oxo-1,2-dihydroisoquinolin-4-yl)ethyl)-3-(4-fluoro-3-methylphenyl)-1-methylurea; (S)-1-(1-(6,7-Difluoro-1-oxo-1,2-dihydroisoquinolin-4-yl)ethyl)-3-(4-fluoro-3-methylphenyl)-1-methylurea; (R)-3-(3-cyano-4-fluorophenyl)-1-(1-(6,7-difluoro-1-oxo-1,2-dihydroisoquinolin-4-yl)ethyl)-1-methylurea; (S)-3-(3-cyano-4-fluorophenyl)-1-(1-(6,7-difluoro-1-oxo-1,2-dihydroisoquinolin-4-yl)ethyl)-1-methylurea; (R)-1-(1-(6,7-Difluoro-1-oxo-1,2-dihydroisoquinolin-4-yl)ethyl)-3-(2,3-difluorophenyl)-1-methylurea; (S)-1-(1-(6,7-Difluoro-1-oxo-1,2-dihydroisoquinolin-4-yl)ethyl)-3-(2,3-difluorophenyl)-1-methylurea; (R)-1-(1-(7,8-Difluoro-1-oxo-1,2-dihydroisoquinolin-4-yl)ethyl)-3-(4-fluorophenyl)-1-methylurea; (S)-1-(1-(7,8-Difluoro-1-oxo-1,2-dihydroisoquinolin-4-yl)ethyl)-3-(4-fluorophenyl)-1-methylurea; (R)-1-(1-(7,8-Difluoro-1-oxo-1,2-dihydroisoquinolin-4-yl)ethyl)-3-(3,4-difluorophenyl)-1-methylurea; (S)-1-(1-(7,8-Difluoro-1-oxo-1,2-dihydroisoquinolin-4-yl)ethyl)-3-(3,4-difluorophenyl)-1-methylurea; (R)-1-(1-(7,8-Difluoro-1-oxo-1,2-dihydroisoquinolin-4-yl)ethyl)-1-methyl-3-phenylurea; (S)-1-(1-(7,8-Difluoro-1-oxo-1,2-dihydroisoquinolin-4-yl)ethyl)-1-methyl-3-phenylurea; (R)-3-(3,5-dichloro-4-fluorophenyl)-1-(1-(6,7-difluoro-1-oxo-1,2-dihydroisoquinolin-4-yl)ethyl)-1-methylurea; (S)-3-(3,5-dichloro-4-fluorophenyl)-1-(1-(6,7-difluoro-1-oxo-1,2-dihydroisoquinolin-4-yl)ethyl)-1-methylurea; (R)-1-(1-(7,8-Difluoro-1-oxo-1,2-dihydroisoquinolin-4-yl)ethyl)-3-(4-fluoro-3-methylphenyl)-1-methylurea; (S)-1-(1-(7,8-Difluoro-1-oxo-1,2-dihydroisoquinolin-4-yl)ethyl)-3-(4-fluoro-3-methylphenyl)-1-methylurea; (R)-1-(1-(7,8-Difluoro-1-oxo-1,2-dihydroisoquinolin-4-yl)ethyl)-1-methyl-3-(3,4,5-trifluorophenyl)urea; (S)-1-(1-(7,8-Difluoro-1-oxo-1,2-dihydroisoquinolin-4-yl)ethyl)-1-methyl-3-(3,4,5-trifluorophenyl)urea; (R)-3-(4-Chlorophenyl)-1-(1-(7,8-difluoro-1-oxo-1,2-dihydroisoquinolin-4-yl)ethyl)-1-methylurea; (S)-3-(4-Chlorophenyl)-1-(1-(7,8-difluoro-1-oxo-1,2-dihydroisoquinolin-4-yl)ethyl)-1-methylurea; (R)-3-(3-chloro-4-fluorophenyl)-1-(1-(6,8-difluoro-1-oxo-1,2-dihydroisoquinolin-4-yl)ethyl)-1-isobutylurea; (S)-3-(3-chloro-4-fluorophenyl)-1-(1-(6,8-difluoro-1-oxo-1,2-dihydroisoquinolin-4-yl)ethyl)-1-isobutylurea; (R)-3-(3,5-dichlorophenyl)-1-(1-(7,8-difluoro-1-oxo-1,2-dihydroisoquinolin-4-yl)ethyl)-1-methylurea; (S)-3-(3,5-dichlorophenyl)-1-(1-(7,8-difluoro-1-oxo-1,2-dihydroisoquinolin-4-yl)ethyl)-1-methylurea; (R)-1-(1-(7,8-difluoro-1-oxo-1,2-dihydroisoquinolin-4-yl)ethyl)-1-methyl-3-(pyridin-4-yl)urea; (S)-1-(1-(7,8-difluoro-1-oxo-1,2-dihydroisoquinolin-4-yl)ethyl)-1-methyl-3-(pyridin-4-yl)urea; (R)-1-(1-(7,8-difluoro-1-oxo-1,2-dihydroisoquinolin-4-yl)ethyl)-1-methyl-3-(pyridin-3-yl)urea; (S)-1-(1-(7,8-difluoro-1-oxo-1,2-dihydroisoquinolin-4-yl)ethyl)-1-methyl-3-(pyridin-3-yl)urea; (R)-3-(3,5-dichloro-4-fluorophenyl)-1-(1-(7,8-difluoro-1-oxo-1,2-dihydroisoquinolin-4-yl)ethyl)-1-methylurea; (S)-3-(3,5-dichloro-4-fluorophenyl)-1-(1-(7,8-difluoro-1-oxo-1,2-dihydroisoquinolin-4-yl)ethyl)-1-methylurea; (R)-3-benzyl-1-(1-(7,8-difluoro-1-oxo-1,2-dihydroisoquinolin-4-yl)ethyl)-1-methylurea; (S)-3-benzyl-1-(1-(7,8-difluoro-1-oxo-1,2-dihydroisoquinolin-4-yl)ethyl)-1-methylurea; (R)-3-(2-chloropyridin-4-yl)-1-(1-(7,8-difluoro-1-oxo-1,2-dihydroisoquinolin-4-yl)ethyl)-1-methylurea; (S)-3-(2-chloropyridin-4-yl)-1-(1-(7,8-difluoro-1-oxo-1,2-dihydroisoquinolin-4-yl)ethyl)-1-methylurea; (R)-1-(1-(7,8-difluoro-1-oxo-1,2-dihydroisoquinolin-4-yl)ethyl)-3-(3-fluorophenyl)-1-methylurea; (S)-1-(1-(7,8-difluoro-1-oxo-1,2-dihydroisoquinolin-4-yl)ethyl)-3-(3-fluorophenyl)-1-methylurea; (R)-1-(1-(7,8-difluoro-1-oxo-1,2-dihydroisoquinolin-4-yl)ethyl)-3-(2-fluorophenyl)-1-methylurea; (S)-1-(1-(7,8-difluoro-1-oxo-1,2-dihydroisoquinolin-4-yl)ethyl)-3-(2-fluorophenyl)-1-methylurea; (R)-1-(1-(7,8-difluoro-1-oxo-1,2-dihydroisoquinolin-4-yl)ethyl)-3-(2,3-difluorophenyl)-1-methylurea; (S)-1-(1-(7,8-difluoro-1-oxo-1,2-dihydroisoquinolin-4-yl)ethyl)-3-(2,3-difluorophenyl)-1-methylurea; (R)-1-(1-(7,8-difluoro-1-oxo-1,2-dihydroisoquinolin-4-yl)ethyl)-3-(3-chlorophenyl)-1-methylurea; (S)-1-(1-(7,8-difluoro-1-oxo-1,2-dihydroisoquinolin-4-yl)ethyl)-3-(3-chlorophenyl)-1-methylurea; (R)-1-(1-(7,8-difluoro-1-oxo-1,2-dihydroisoquinolin-4-yl)ethyl)-3-(3,4-difluorophenyl)-1-ethylurea; (S)-1-(1-(7,8-difluoro-1-oxo-1,2-dihydroisoquinolin-4-yl)ethyl)-3-(3,4-difluorophenyl)-1-ethylurea; (R)-1-(1-(7,8-difluoro-1-oxo-1,2-dihydroisoquinolin-4-yl)ethyl)-3-(4-fluorophenyl)-1-ethylurea; (S)-1-(1-(7,8-difluoro-1-oxo-1,2-dihydroisoquinolin-4-yl)ethyl)-3-(4-fluorophenyl)-1-ethylurea; (R)-1-(1-(7,8-difluoro-1-oxo-1,2-dihydroisoquinolin-4-yl)ethyl)-3-phenyl-1-ethylurea; (S)-1-(1-(7,8-difluoro-1-oxo-1,2-dihydroisoquinolin-4-yl)ethyl)-3-phenyl-1-ethylurea; (R)-1-(1-(7,8-difluoro-1-oxo-1,2-dihydroisoquinolin-4-yl)ethyl)-3-(3-chloro-4-fluorophenyl)-1-ethylurea; (S)-1-(1-(7,8-difluoro-1-oxo-1,2-dihydroisoquinolin-4-yl)ethyl)-3-(3-chloro-4-fluorophenyl)-1-ethylurea; (R)-3-(3-cyano-4-fluorophenyl)-1-(1-(7,8-difluoro-1-oxo-1,2-dihydroisoquinolin-4-yl)ethyl)-1-methylurea; (S)-3-(3-cyano-4-fluorophenyl)-1-(1-(7,8-difluoro-1-oxo-1,2-dihydroisoquinolin-4-yl)ethyl)-1-methylurea; (R)-3-(3-chloro-4-fluorophenyl)-1-(1-(6, 7-difluoro-1-oxo-1,2-dihy droisoquinolin-4-yl)ethyl)-1-(methyl)urea; (S)-3-(3-chloro-4-fluorophenyl)-1-(1-(6,7-difluoro-1-oxo-1,2-dihydroisoquinolin-4-yl)ethyl)-1-(methyl)urea; (R)-3-(3-chloro-4-fluorophenyl)-1-(1-(6,7-difluoro-1-oxo-1,2-dihydroisoquinolin-4-yl)ethyl)-1-(methyl-d₃)urea; (S)-3-(3-chloro-4-fluorophenyl)-1-(1-(6,7-difluoro-1-oxo-1,2-dihydroisoquinolin-4-yl)ethyl)-1-(methyl-d₃)urea; (R)-3-(3-chloro-4-fluorophenyl)-1-(1-(7,8-difluoro-1-oxo-1,2-dihydroisoquinolin-4-yl)ethyl)-1-(methyl)urea; (S)-3-(3-chloro-4-fluorophenyl)-1-(1-(7,8-difluoro-1-oxo-1,2-dihydroisoquinolin-4-yl)ethyl)-1-(methyl)urea; (R)-3-(3-chloro-4-fluorophenyl)-1-(1-(7,8-difluoro-1-oxo-1,2-dihydroisoquinolin-4-yl)ethyl)-1-(methyl-d₃)urea; (S)-3-(3-chloro-4-fluorophenyl)-1-(1-(7,8-difluoro-1-oxo-1,2-dihydroisoquinolin-4-yl)ethyl)-1-(methyl-d₃)urea; (R)-3-(3-chlorophenyl)-1-(1-(7,8-difluoro-1-oxo-1,2-dihydroisoquinolin-4-yl)ethyl)-1-(methyl)urea; (S)-3-(3-chlorophenyl)-1-(1-(7,8-difluoro-1-oxo-1,2-dihydroisoquinolin-4-yl)ethyl)-1-(methyl)urea; (R)-3-(3-chlorophenyl)-1-(1-(7,8-difluoro-1-oxo-1,2-dihydroisoquinolin-4-yl)ethyl)-1-(methyl-d₃)urea; (S)-3-(3-chlorophenyl)-1-(1-(7,8-difluoro-1-oxo-1,2-dihydroisoquinolin-4-yl)ethyl)-1-(methyl-d₃)urea; 1-(1(R)-(6,7-difluoro-1-oxo-1,2-dihydroisoquinolin-4-yl)ethyl)-1-methyl-3-((S)-1-phenylethyl)urea; 1-(1(S)-(6,7-difluoro-1-oxo-1,2-dihydroisoquinolin-4-yl)ethyl)-1-methyl-3-((S)-1-phenylethyl)urea; (R)-1-(1-(6,7-difluoro-1-oxo-1,2-dihydroisoquinolin-4-yl)ethyl)-3-(3,4-difluorobenzyl)-1-methylurea; (S)-1-(1-(6,7-difluoro-1-oxo-1,2-dihydroisoquinolin-4-yl)ethyl)-3-(3,4-difluorobenzyl)-1-methylurea; (R)-1-(1-(6,7-difluoro-1-oxo-1,2-dihydroisoquinolin-4-yl)ethyl)-3-(4-fluorobenzyl)-1-methylurea; (S)-1-(1-(6,7-difluoro-1-oxo-1,2-dihydroisoquinolin-4-yl)ethyl)-3-(4-fluorobenzyl)-1-methylurea; 1-(1(R)-(6,7-difluoro-1-oxo-1,2-dihydroisoquinolin-4-yl)ethyl)-1-methyl-3-((R)-1-phenylethyl)urea; 1-(1(S)-(6,7-difluoro-1-oxo-1,2-dihydroisoquinolin-4-yl)ethyl)-1-methyl-3-((R)-1-phenylethyl)urea; (R)-1-(3-chloro-4-fluorophenyl)-3-(1-(6,7-difluoro-1 -oxo-1,2-dihydroisoquinolin-4-yl)ethyl)urea; (S)-1-(3-chloro-4-fluorophenyl)-3-(1-(6,7-difluoro-1-oxo-1,2-dihydroisoquinolin-4-yl)ethyl)urea; (R)-1-(1-(6,7-difluoro-1-oxo-1 ,2-dihydroisoquinolin-4-yl)ethyl)-1-methyl-3-(3,4,5-trifluorobenzyl)urea; (S)1-(1-(6,7-difluoro-1-oxo-1,2-dihydroisoquinolin-4-yl)ethyl)-1-methyl-3-(3,4,5-trifluorobenzyl)urea; (R)-1-(1-(6,7-difluoro-1-oxo-1,2-dihydroisoquinolin-4-yl)ethyl)-1-methyl-3-(3-chloro-4-fluorobenzyl)urea; (S)1-(1-(6,7-difluoro-1-oxo-1,2-dihydroisoquinolin-4-yl)ethyl)-1-methyl-3-(3-chloro-4-fluorobenzyl)urea; (R)-1-(1-(6,7-difluoro-1-oxo-1,2-dihydroisoquinolin-4-yl)ethyl)-3-(1H-indol-6-yl)-1-methylurea; (S)-1-(1-(6,7-difluoro-1-oxo-1,2-dihydroisoquinolin-4-yl)ethyl)-3-(1H-indol-6-yl)-1-methylurea; 1-(1(R)-(7,8-difluoro-1-oxo-1,2-dihydroisoquinolin-4-yl)ethyl)-1-methyl-3-((S)-1-phenylethyl)urea; 1-(1(S)-(7,8-difluoro-1-oxo-1,2-dihydroisoquinolin-4-yl)ethyl)-1-methyl-3-((S)-1-phenylethyl)urea; 1-(1(R)-(7,8-difluoro-1-oxo-1,2-dihydroisoquinolin-4-yl)ethyl)-1-methyl-3-((R)-1-phenylethyl)urea; 1-(1(S)-(7,8-difluoro-1-oxo-1,2-dihydroisoquinolin-4-yl)ethyl)-1-methyl-3-((R)-1-phenylethyl)urea; (R)-1-(1-(7,8-difluoro-1-oxo-1,2-dihydroisoquinolin-4-yl)ethyl)-3-(3,4-difluorobenzyl)-1-methylurea; (S)1-(1-(7,8-difluoro-1-oxo-1,2-dihydroisoquinolin-4-yl)ethyl)-3-(3,4-difluorobenzyl)-1-methylurea; (R)-1-(1-(7,8-difluoro-1-oxo-1,2-dihydroisoquinolin-4-yl)ethyl)-3-(4-fluorobenzyl)-1-methylurea; (S)1-(1-(7,8-difluoro-1-oxo-1,2-dihydroisoquinolin-4-yl)ethyl)-3-(4-fluorobenzyl)-1-methylurea; (R)-1-(1-(7,8-difluoro-1-oxo-1,2-dihydroisoquinolin-4-yl)ethyl)-3-(3,4,5-trifluorobenzyl)-1-methylurea; (S)-1-(1-(7,8-difluoro-1-oxo-1,2-dihydroisoquinolin-4-yl)ethyl)-3-(3,4,5-trifluorobenzyl)-1-methylurea; (R)-1-(3-cyano-4-fluorophenyl)-3-(1-(6,7-difluoro-1-oxo-1,2-dihydroisoquinolin-4-yl)ethyl)urea; (S)-1-(3-cyano-4-fluorophenyl)-3-(1-(6,7-difluoro-1-oxo-1,2-dihydroisoquinolin-4-yl)ethyl)urea; (R)-3-(3-chloro-4-fluorobenzyl)-1-(1-(7,8-difluoro-1-oxo-1,2-dihydroisoquinolin-4-yl)ethyl)-1-methylurea; (S)-3-(3-chloro-4-fluorobenzyl)-1-(1-(7,8-difluoro-1-oxo-1,2-dihydroisoquinolin-4-yl)ethyl)-1-methylurea; (R)-1-(1-(7,8-difluoro-1-oxo-1,2-dihydroisoquinolin-4-yl)ethyl)-3-(1H-indol-6-yl)-1-methylurea; (S)-1-(1-(7,8-difluoro-1-oxo-1,2-dihydroisoquinolin-4-yl)ethyl)-3-(1H-indol-6-yl)-1-methylurea; (R)-1-(3-chloro-4-fluorophenyl)-3-(1-(7,8-difluoro-1-oxo-1,2-dihydroisoquinolin-4-yl)ethyl)urea; (S)-1-(3-chloro-4-fluorophenyl)-3-(1-(7,8-difluoro-1-oxo-1,2-dihydroisoquinolin-4-yl)ethyl)urea; (R)-1-(1-(6,7-difluoro-1-oxo-1,2-dihydroisoquinolin-4-yl)ethyl)-3-(3-(difluoromethyl)-4-fluorophenyl)-1-methylurea; (S)-1-(1-(6,7-difluoro-1-oxo-1,2-dihydroisoquinolin-4-yl)ethyl)-3-(3-(difluoromethyl)-4-fluorophenyl)-1-methylurea; (R)-1-(1-(7,8-difluoro-1-oxo-1,2-dihydroisoquinolin-4-yl)ethyl)-3-(3-(difluoromethyl)-4-fluorophenyl)-1-methylurea; (S)-1-(1-(7,8-difluoro-1-oxo-1,2-dihydroisoquinolin-4-yl)ethyl)-3-(3-(difluoromethyl)-4-fluorophenyl)-1-methylurea; or a salt, solvate, isotopically labelled, stereoisomer, any mixture of stereoisomers, tautomer, and/or any mixture of tautomers thereof.

In certain embodiments, the compound is at leats one selected from: (R)-3-(3-chloro-4-fluorophenyl)-1-methyl-1-(1-(1-oxo-1,2-dihydroisoquinolin-4-yl)ethyl)urea; (S)-3-(3-chloro-4-fluorophenyl)-1-methyl-1-(1-(1-oxo-1,2-dihydroisoquinolin-4-yl)ethyl)urea; (R)-3-(4-fluoro-3-methylphenyl)-1-methyl-1-(1-(1-oxo-1,2-dihydroisoquinolin-4-yl)ethyl)urea; (R)-3-(3-chloro-4-fluorophenyl)-1-(1-(1-oxo-1,2-dihydroisoquinolin-4-yl)ethyl)-1-propylurea; (R)-3-(4-fluoro-3-methylphenyl)-1-(1-(1-oxo-1,2-dihydroisoquinolin-4-yl)ethyl)-1-propylurea; (R)-3-(4-fluoro-3-(trifluoromethyl)phenyl)-1-methyl-1-(1-(1-oxo-1,2-dihydroisoquinolin-4-yl)ethyl)urea; (R)-3-(4-fluorophenyl)-1-methyl-1-(1-(1-oxo-1,2-dihydroisoquinolin-4-yl)ethyl)urea; (R)-1-methyl-1-(1-(1-oxo-1,2-dihydroisoquinolin-4-yl)ethyl)-3-(3,4,5-trifluorophenyl)urea; (R)-3-(3,4-difluorophenyl)-1-methyl-1-(1-(1-oxo-1,2-dihydroisoquinolin-4-yl)ethyl)urea; (R)-3-(3-fluorophenyl)-1-methyl-1-(1-(1-oxo-1,2-dihydroisoquinolin-4-yl)ethyl)urea; (R)-3-(2-Chloropyridin-4-yl)-1-methyl-1-(1-(1-oxo-1,2-dihydroisoquinolin-4-yl)ethyl)urea; (R)-1-Methyl-1-(1-(1-oxo-1,2-dihydroisoquinolin-4-yl)ethyl)-3-(2-(trifluoromethyl)pyridin-4-yl)urea; (R)-3-(3-chloro-4-fluorophenyl)-1-(2,2-difluoroethyl)-1-(1-(1-oxo-1,2-dihydroisoquinolin-4-yl)ethyl)urea; (R)-1-(2,2-difluoroethyl)-3-(4-fluoro-3-methylphenyl)-1-(1-(1-oxo-1,2-dihydroisoquinolin-4-yl)ethyl)urea; (R)-3-(3-cyano-4-fluorophenyl)-1-methyl-1-(1-(1-oxo-1,2-dihydroisoquinolin-4-yl)ethyl)urea; (R)-3-(3-chloro-4-fluorophenyl)-1-(ethyl-d5)-1-(1-(1-oxo-1,2-dihydroisoquinolin-4-yl)ethyl)urea; (R)-3-(3-chloro-4-fluorophenyl)-1-(1-(1-methoxyisoquinolin-4-yl)ethyl)-1-(2,2,2-trifluoroethyl)urea; (R)-3-(4-chlorophenyl)-1-methyl-1-(1-(1-oxo-l,2-dihydroisoquinolin-4-yl)ethyl)urea; and (R)-3-(4-bromophenyl)-1 -methyl-1 -(1-(1-oxo-1,2-dihydroisoquinolin-4-yl)ethyl)urea; or a salt, solvate, isotopically labelled, stereoisomer, any mixture of stereoisomers, tautomer, and/or any mixture of tautomers thereof.

The compounds of the invention may possess one or more stereocenters, and each stereocenter may exist independently in either the (*R*)- or (*S*)-configuration. In certain embodiments, compounds described herein are present in optically active or racemic forms. The compounds described herein encompass racemic, optically active, regioisomeric and stereoisomeric forms, or combinations thereof that possess the therapeutically useful properties described herein. Preparation of optically active forms is achieved in any suitable manner, including, by way of non-limiting example, by resolution of the racemic form with recrystallization techniques, synthesis from optically active starting materials, chiral synthesis, or chromatographic separation using a chiral stationary phase. A compound illustrated herein by the racemic formula further represents either of the two enantiomers or any mixtures thereof, or in the case where two or more chiral centers are present, all diastereomers or any mixtures thereof.

In certain embodiments, the compounds of the invention exist as tautomers. All tautomers are included within the scope of the compounds recited herein.

Compounds described herein also include isotopically labeled compounds wherein one or more atoms is replaced by an atom having the same atomic number, but an atomic mass or mass number different from the atomic mass or mass number usually found in nature. Examples of isotopes suitable for inclusion in the compounds described herein include and are not limited to ²H , ³H , ¹¹C, ¹³C, ¹⁴C, ³⁶Cl, ¹⁸F, ¹²³I, ¹²⁵I, ¹³N, ¹⁵N, ¹⁵O, ¹⁷O, ¹⁸O, ³²P, and ³⁵S. In certain embodiments, substitution with heavier isotopes such as deuterium affords greater chemical stability. Isotopically labeled compounds are prepared by any suitable method or by processes using an appropriate isotopically labeled reagent in place of the non-labeled reagent otherwise employed.

In certain embodiments, the compounds described herein are labeled by other means, including, but not limited to, the use of chromophores or fluorescent moieties, bioluminescent labels, or chemiluminescent labels.

In all of the embodiments provided herein, examples of suitable optional substituents are not intended to limit the scope of the claimed invention. The compounds of the invention may contain any of the substituents, or combinations of substituents, provided herein.

### Salts

The compounds described herein may form salts with acids or bases, and such salts are included in the present invention. The term "salts" embraces addition salts of free acids or bases that are useful within the methods of the invention. The term "pharmaceutically acceptable salt" refers to salts that possess toxicity profiles within a range that affords utility in pharmaceutical applications. In certain embodiments, the salts are pharmaceutically acceptable salts. Pharmaceutically unacceptable salts may nonetheless possess properties such as high crystallinity, which have utility in the practice of the present invention, such as for example utility in process of synthesis, purification or formulation of compounds useful within the methods of the invention.

Suitable pharmaceutically acceptable acid addition salts may be prepared from an inorganic acid or from an organic acid. Examples of inorganic acids include sulfate, hydrogen sulfate, hydrochloric, hydrobromic, hydriodic, nitric, carbonic, sulfuric, and phosphoric acids (including hydrogen phosphate and dihydrogen phosphate). Appropriate organic acids may be selected from aliphatic, cycloaliphatic, aromatic, araliphatic, heterocyclic, carboxylic and sulfonic classes of organic acids, examples of which include formic, acetic, propionic, succinic, glycolic, gluconic, lactic, malic, tartaric, citric, ascorbic, glucuronic, maleic, fumaric, pyruvic, aspartic, glutamic, benzoic, anthranilic, 4-hydroxybenzoic, phenylacetic, mandelic, embonic (or pamoic), methanesulfonic, ethanesulfonic, benzenesulfonic, pantothenic, sulfanilic, 2-hydroxyethanesulfonic, trifluoromethanesulfonic, p-toluenesulfonic, cyclohexylaminosulfonic, stearic, alginic, β-hydroxybutync, salicylic, galactaric, galacturonic acid, glycerophosphonic acids and saccharin (e.g., saccharinate, saccharate). Salts may be comprised of a fraction of one, one or more than one molar equivalent of acid or base with respect to any compound of the invention.

Suitable pharmaceutically acceptable base addition salts of compounds of the invention include, for example, ammonium salts and metallic salts including alkali metal, alkaline earth metal and transition metal salts such as, for example, calcium, magnesium, potassium, sodium and zinc salts. Pharmaceutically acceptable base addition salts also include organic salts made from basic amines such as, for example, N,N'-dibenzylethylenediamine, chloroprocaine, choline, diethanolamine, ethylenediamine, meglumine (or N-methylglucamine) and procaine. All of these salts may be prepared from the corresponding compound by reacting, for example, the appropriate acid or base with the compound.

### Combination Therapies

In one aspect, the compounds of the invention are useful within the methods of the invention in combination with one or more additional agents useful for treating HBV and/or HDV infections. These additional agents may comprise compounds or compositions identified herein, or compounds (*e.g.*, commercially available compounds) known to treat, prevent, or reduce the symptoms of HBV and/or HDV infections.

Non-limiting examples of one or more additional agents useful for treating HBV and/or HDV infections include: (a) reverse transcriptase inhibitors; (b) capsid inhibitors; (c) cccDNA formation inhibitors; (d) RNA destabilizers; (e) oligomeric nucleotides targeted against the HBV genome; (f) immunostimulators, such as checkpoint inhibitors (*e.g.*, PD-L1 inhibitors); and (g) GalNAc-siRNA conjugates targeted against an HBV gene transcript.

### (a) Reverse Transcriptase Inhibitors

In certain embodiments, the reverse transcriptase inhibitor is a reverse-transcriptase inhibitor (NARTI or NRTI). In other embodiments, the reverse transcriptase inhibitor is a nucleotide analog reverse-transcriptase inhibitor (NtARTI or NtRTI).

Reported reverse transcriptase inhibitors include, but are not limited to, entecavir, clevudine, telbivudine, lamivudine, adefovir, and tenofovir, tenofovir disoproxil, tenofovir alafenamide, adefovir dipovoxil, (1*R*,2*R*,3*R*,S*R*)-3-(6-amino-9*H*-9-purinyl)-2-fluoro-5-(hydroxymethyl)-4-methylenecyclopentan-1-ol (described in U.S. Patent No. 8,816,074, incorporated herein in its entirety by reference), emtricitabine, abacavir, elvucitabine, ganciclovir, lobucavir, famciclovir, penciclovir, and amdoxovir.

Reported reverse transcriptase inhibitors further include, but are not limited to, entecavir, lamivudine, and (1*R*,2*R*,3*R*,5*R*)-3-(6-amino-9*H*-9-purinyl)-2-fluoro-5-(hydroxymethyl)-4-methylenecyclopentan-1-ol.

Reported reverse transcriptase inhibitors further include, but are not limited to, a covalently bound phosphoramidate or phosphonamidate moiety of the above-mentioned reverse transcriptase inhibitors, or as described in for example U.S. Patent No. 8,816,074, US Patent Application Publications No. US 2011/0245484 A1, and US 2008/0286230A1, all of which incorporated herein in their entireties by reference.

Reported reverse transcriptase inhibitors further include, but are not limited to, nucleotide analogs that comprise a phosphoramidate moiety, such as, for example, methyl ((((1*R*,3*R*,4*R*,5*R*)-3-(6-amino-9*H*-purin-9-yl)-4-fluoro-5-hydroxy-2-methylenecyclopentyl) methoxy)(phenoxy) phosphoryl)-(D or L)-alaninate and methyl ((((1*R*,2*R*,3*R*,4*R*)-3-fluoro-2-hydroxy-5-methylene-4-(6-oxo-1,6-dihydro-9*H*-purin-9-yl)cyclopentyl)methoxy)(phenoxy) phosphoryl)-(D or L)-alaninate. Also included are the individual diastereomers thereof, which include, for example, methyl ((*R*)-(((1*R*,3*R*,4*R*,5*R*)-3-(6-amino-9*H*-purin-9-yl)-4-fluoro-5-hydroxy-2-methylenecyclopentyl)methoxy)(phenoxy)phosphoryl)-(D or L)-alaninate and methyl ((*S*)-(((1*R*,3*R*,4*R*,5*R*)-3-(6-amino-9*H*-purin-9-yl)-4-fluoro-5-hydroxy-2-methylenecyclopentyl) methoxy)(phenoxy)phosphoryl)-(D or L)-alaninate.

Reported reverse transcriptase inhibitors further include, but are not limited to, compounds comprising a phosphonamidate moiety, such as, for example, tenofovir alafenamide, as well as those described in U.S. Patent Application Publication No. US 2008/0286230 A1, incorporated herein in its entirety by reference. Methods for preparing stereoselective phosphoramidate or phosphonamidate containing actives are described in, for example, U.S. Patent No. 8,816,074, as well as U.S. Patent Application Publications No. US 2011/0245484 A1 and US 2008/0286230 A1, all of which incorporated herein in their entireties by reference.

### (b) Capsid Inhibitors

As described herein, the term "capsid inhibitor" includes compounds that are capable of inhibiting the expression and/or function of a capsid protein either directly or indirectly. For example, a capsid inhibitor may include, but is not limited to, any compound that inhibits capsid assembly, induces formation of non-capsid polymers, promotes excess capsid assembly or misdirected capsid assembly, affects capsid stabilization, and/or inhibits encapsidation of RNA (pgRNA). Capsid inhibitors also include any compound that inhibits capsid function in a downstream event(s) within the replication process (e.g., viral DNA synthesis, transport of relaxed circular DNA (rcDNA) into the nucleus, covalently closed circular DNA (cccDNA) formation, virus maturation, budding and/or release, and the like). For example, in certain embodiments, the inhibitor detectably inhibits the expression level or biological activity of the capsid protein as measured, *e.g.*, using an assay described herein. In certain embodiments, the inhibitor inhibits the level of rcDNA and downstream products of viral life cycle by at least 5%, at least 10%, at least 20%, at least 50%, at least 75%, or at least 90%.

Reported capsid inhibitors include, but are not limited to, compounds described in International Patent Applications Publication Nos WO 2013006394, WO 2014106019, and WO2014089296, all of which incorporated herein in their entireties by reference.

Reported capsid inhibitors also include, but are not limited to, the following compounds and pharmaceutically acceptable salts and/or solvates thereof: Bay-41-4109 (see Int'l Patent Application Publication No. WO 2013144129), AT-61 (see Int'l Patent Application Publication No. WO 1998033501; and King, et al., 1998, Antimicrob. Agents Chemother. 42(12):3179-3186), DVR-01 and DVR-23 (see Int'l Patent Application Publication No. WO 2013006394; and Campagna, et al., 2013, J. Virol. 87(12):6931, all of which incorporated herein in their entireties by reference.

In addition, reported capsid inhibitors include, but are not limited to, those generally and specifically described in U.S. Patent Application Publication Nos. US 2015/0225355, US 2015/0132258, US 2016/0083383, US 2016/0052921 and Int'l Patent Application Publication Nos. WO 2013096744, WO 2014165128, WO 2014033170, WO 2014033167, WO 2014033176, WO 2014131847, WO 2014161888, WO 2014184350, WO 2014184365, WO 2015059212, WO 2015011281, WO 2015118057, WO 2015109130, WO 2015073774, WO 2015180631, WO 2015138895, WO 2016089990, WO 2017015451, WO 2016183266, WO 2017011552, WO 2017048950, WO2017048954, WO 2017048962, WO 2017064156 and are incorporated herein in their entirety by reference.

### (c) cccDNA Formation Inhibitors

Covalently closed circular DNA (cccDNA) is generated in the cell nucleus from viral rcDNA and serves as the transcription template for viral mRNAs. As described herein, the term "cccDNA formation inhibitor" includes compounds that are capable of inhibiting the formation and/or stability of cccDNA either directly or indirectly. For example, a cccDNA formation inhibitor may include, but is not limited to, any compound that inhibits capsid disassembly, rcDNA entry into the nucleus, and/or the conversion of rcDNA into cccDNA. For example, in certain embodiments, the inhibitor detectably inhibits the formation and/or stability of the cccDNA as measured, e.g., using an assay described herein. In certain embodiments, the inhibitor inhibits the formation and/or stability of cccDNA by at least 5%, at least 10%, at least 20%, at least 50%, at least 75%, or at least 90%.

Reported cccDNA formation inhibitors include, but are not limited to, compounds described in Int'l Patent Application Publication No. WO 2013130703, and are incorporated herein in their entirety by reference.

In addition, reported cccDNA formation inhibitors include, but are not limited to, those generally and specifically described in U.S. Patent Application Publication No. US 2015/0038515 A1, and are incorporated herein in their entirety by reference.

### (d) RNA Destabilizer

As used herein, the term "RNA destabilizer" refers to a molecule, or a salt or solvate thereof, that reduces the total amount of HBV RNA in mammalian cell culture or in a live human subject. In a non-limiting example, an RNA destabilizer reduces the amount of the RNA transcript(s) encoding one or more of the following HBV proteins: surface antigen, core protein, RNA polymerase, and e antigen. In certain embodiments, the RNA destabilizer reduces the total amount of HBV RNA in mammalian cell culture or in a live human subject by at least 5%, at least 10%, at least 20%, at least 50%, at least 75%, or at least 90%.

Reported RNA destabilizers include compounds described in U.S. Patent No. 8,921,381, as well as compounds described in U.S. Patent Application Publication Nos. US 2015/0087659 and US 2013/0303552, all of which are incorporated herein in their entireties by reference.

In addition, reported RNA destabilizers include, but are not limited to, those generally and specifically described in Int'l Patent Application Publication Nos. WO 2015113990, WO 2015173164, US 2016/0122344, WO 2016107832, WO 2016023877, WO 2016128335, WO 2016177655, WO 2016071215, WO 2017013046, WO 2017016921, WO 2017016960, WO 2017017042, WO 2017017043, WO 2017102648, WO 2017108630, WO 2017114812, WO 2017140821, WO 2018085619, and are incorporated herein in their entirety by reference.

### (e) Oligomeric Nucleotides Targeted Against the HBV Genome

Reported oligomeric nucleotides targeted against the HBV genome include, but are not limited to, Arrowhead-ARC-520 (see U.S. Patent No. 8,809,293; and Wooddell et al., 2013, Molecular Therapy 21(5):973-985, all of which incorporated herein in their entireties by reference).

In certain embodiments, the oligomeric nucleotides can be designed to target one or more genes and/or transcripts of the HBV genome. Oligomeric nucleotide targeted to the HBV genome also include, but are not limited to, isolated, double stranded, siRNA molecules, that each include a sense strand and an antisense strand that is hybridized to the sense strand. In certain embodiments, the siRNA target one or more genes and/or transcripts of the HBV genome.

### (f) Immunostimulators

### Checkpoint Inhibitors

As described herein, the term "checkpoint inhibitor" includes any compound that is capable of inhibiting immune checkpoint molecules that are regulators of the immune system (e.g., stimulate or inhibit immune system activity). For example, some checkpoint inhibitors block inhibitory checkpoint molecules, thereby stimulating immune system function, such as stimulation of T cell activity against cancer cells. A non-limting example of a checkpoint inhibitor is a PD-L1 inhibitor.

As described herein, the term "PD-L1 inhibitor" includes any compound that is capable of inhibiting the expression and/or function of the protein Programmed Death-Ligand 1 (PD-L1) either directly or indirectly. PD-L1, also known as cluster of differentiation 274 (CD274) or B7 homolog 1 (B7-H1), is a type 1 transmembrane protein that plays a major role in suppressing the adaptive arm of immune system during pregnancy, tissue allograft transplants, autoimmune disease, and hepatitis. PD-L1 binds to its receptor, the inhibitory checkpoint molecule PD-1 (which is found on activated T cells, B cells, and myeloid cells) so as to modulate activation or inhibition of the adaptive arm of immune system. In certain embodiments, the PD-L1 inhibitor inhibits the expression and/or function of PD-L1 by at least 5%, at least 10%, at least 20%, at least 50%, at least 75%, or at least 90%.

Reported PD-L1 Inhibitors include, but are not limited to, compounds recited in one of the following patent application publications: US 2018/0057455; US 2018/0057486; WO 2017/106634; WO 2018/026971; WO 2018/045142; WO 2018/118848; WO 2018/119221; WO 2018/119236; WO 2018/119266; WO 2018/119286; WO 2018/121560; WO 2019/076343; WO 2019/087214; and are incorporated herein in their entirety by reference.

### (g) GalNAc-siRNA Conjugates Targeted Against an HBV Gene Transcript

"GalNAc" is the abbreviation for N-acetylgalactosamine, and "siRNA" is the abbreviation for small interfering RNA. An siRNA that targets an HBV gene transcript is covalently bonded to GalNAc in a GalNAc-siRNA conjugate useful in the practice of the present invention. While not wishing to be bound by theory, it is believed that GalNAc binds to asialoglycoprotein receptors on hepatocytes thereby facilitating the targeting of the siRNA to the hepatocytes that are infected with HBV. The siRNA enter the infected hepatocytes and stimulate destruction of HBV gene transcripts by the phenomenon of RNA interference.

Examples of GalNAc-siRNA conjugates useful in the practice of this aspect of the present invention are set forth in published international application PCT/CA2017/050447 (PCT Application Publication number WO/2017/177326, published on October 19, 2017) which is hereby incorporated by reference in its entirety.

A synergistic effect may be calculated, for example, using suitable methods such as, for example, the Sigmoid-Eₘₐₓ equation (Holford & Scheiner, 1981, Clin. Pharmacokinet. 6:429-453), the equation of Loewe additivity (Loewe & Muischnek, 1926, Arch. Exp. Pathol Pharmacol. 114: 313-326) and the median-effect equation (Chou & Talalay, 1984, Adv. Enzyme Regul. 22:27-55). Each equation referred to elsewhere herein may be applied to experimental data to generate a corresponding graph to aid in assessing the effects of the drug combination. The corresponding graphs associated with the equations referred to elsewhere herein are the concentration-effect curve, isobologram curve and combination index curve, respectively.

### Synthesis

The present invention further provides methods of preparing compounds of the present invention. Compounds of the present teachings can be prepared in accordance with the procedures outlined herein, from commercially available starting materials, compounds known in the literature, or readily prepared intermediates, by employing standard synthetic methods and procedures known to those skilled in the art. Standard synthetic methods and procedures for the preparation of organic molecules and functional group transformations and manipulations can be readily obtained from the relevant scientific literature or from standard textbooks in the field.

It is appreciated that where typical or preferred process conditions *(i.e.,* reaction temperatures, times, mole ratios of reactants, solvents, pressures, and so forth) are given, other process conditions can also be used unless otherwise stated. Optimum reaction conditions can vary with the particular reactants or solvent used, but such conditions can be determined by one skilled in the art by routine optimization procedures. Those skilled in the art of organic synthesis will recognize that the nature and order of the synthetic steps presented can be varied for the purpose of optimizing the formation of the compounds described herein.

The processes described herein can be monitored according to any suitable method known in the art. For example, product formation can be monitored by spectroscopic means, such as nuclear magnetic resonance spectroscopy (*e.g.*, ¹H or ¹³C), infrared spectroscopy, spectrophotometry (*e.g.*, UV-visible), mass spectrometry, or by chromatography such as high-performance liquid chromatograpy (HPLC), gas chromatography (GC), gel-permeation chromatography (GPC), or thin layer chromatography (TLC).

Preparation of the compounds can involve protection and deprotection of various chemical groups. The need for protection and deprotection and the selection of appropriate protecting groups can be readily determined by one skilled in the art. The chemistry of protecting groups can be found, for example, in Greene, et al., Protective Groups in Organic Synthesis, 2d. Ed. (Wiley & Sons, 1991), the entire disclosure of which is incorporated by reference herein for all purposes.

The reactions or the processes described herein can be carried out in suitable solvents that can be readily selected by one skilled in the art of organic synthesis. Suitable solvents typically are substantially nonreactive with the reactants, intermediates, and/or products at the temperatures at which the reactions are carried out, *i.e.,* temperatures that can range from the solvent's freezing temperature to the solvent's boiling temperature. A given reaction can be carried out in one solvent or a mixture of more than one solvent. Depending on the particular reaction step, suitable solvents for a particular reaction step can be selected.

A compound of formula (I) can be prepared from commercially available or previously documented starting materials, for example, according to the synthetic methods outlined in Scheme 1. (Un)substituted isoquinolin-1(2*H*)-ones (**II**) can either be commercially acquired or synthesized according to procedures outlined in, for example, Tetrahedron, 2002, 58:5761-5766. Bromination of **II** using, in non-limiting examples, pyridinium hydrobromide perbromide as described in J. Med. Chem., 2014, 57:1299-1322 or *N*-bromosuccinimide as described in Angew. Chem. Int. Ed., 2011, 50:8416-8419, provides **III.** Chlorination of **III** with, in a non-limiting example, phosphorus oxychloride as described in Bioorg. Med. Chem. Lett., 2017, 27:217-222, followed by chloride displacement with, for example, an alcohol in the presence of a base (Y=O), in a non-limiting example, as exemplified in WO200472033, provides **IV.** Halogen metal exchange of the heteroaryl bromide of **IV**, followed by quenching of the resulting heteroaryl anion with a suitable electrophile such as, but not limited to, DMF, carbon dioxide, a dialkyl dicarbonate, an anhydride, an aldehyde, a ketone, and/or a Weinreb amide, or manipulation of the bromine using transition metal catalyzed coupling techniques, provides **V.** Reductive alkylation utilizing **V** subsequently provides **VI.** When **V** is an aldehyde or a ketone, reductive alkylation can be achieved by reacting that compound with a primary amine to form an imine, which is then reacted with a reducing agent, such as but not limited to sodium borohydride, or a carbon-based nucleophile, such as but not limited to a Grignard reagent or an alkyl/aryl lithium. Alternatively, when **V** is an aldehyde or a ketone, reductive alkylation can be achieved by reacting that compound with a primary sulfinamide to form a sulfinimine, which is subsequently reacted with a reducing agent, such as but not limited to sodium borohydride, or a carbon-based nucleophile, such as but not limited to a Grignard reagent or an alkyl/aryl lithium. In certain embodiments, the primary sulfinamide can be racemic, scalemic, or enantiopure, and can be used to influence the stereochemical outcome of the sulfinimine reduction. The resulting secondary sulfinamide can be further functionalized with an electrophile, such as but not limited to an alkyl halide, in the presence of base, such as but not limited to sodium hydride, and the sulfonamido group can be removed to provide **VI.** Under certain conditions, sulfinamido removal can be concomitant with R'-dealkylation to provide **VIII** directly. Alternatively, when **V** is an aldehyde or a ketone, the compound can be reduced to the corresponding primary or secondary alcohol using a reducing agent, such as but not limited to sodium borohydride. The primary or secondary alcohol can be functionalized with, for example, para-toluene sulfonyl chloride to provide the corresponding tosylate, or converted to the alkyl halide, using for example thionyl chloride, and subsequently reacted with a primary amine to provide **VI.** Functionalization of **VI** with a variety of electrophiles, for example an isocyanate, provides **VII.** Alternatively, acid mediated *O*-dealkylation of **VI** (Y=O), using for example hydrochloric or hydrobromic acid, provides **VIII,** which can be functionalized with a variety of electrophiles, for example isocyanates, to provide **IX.** Alternatively, ketone **XX** can be synthesized from bromoisoquinolinone **III** *via* palladium catalyzed coupling with a vinyl stannane followed by hydrolysis of the resulting enol ether. Reductive alkylation utilizing a primary amine can subsequently provide **VIII** which can be functionalized to afford **IX** (Scheme 2). The protocols incorporated elsewhere herein exemplify synthesis of representative compounds of the present invention. Analogous compounds can be synthesized in a similar fashion to those exemplified using the appropriately substituted intermediates and reagents.

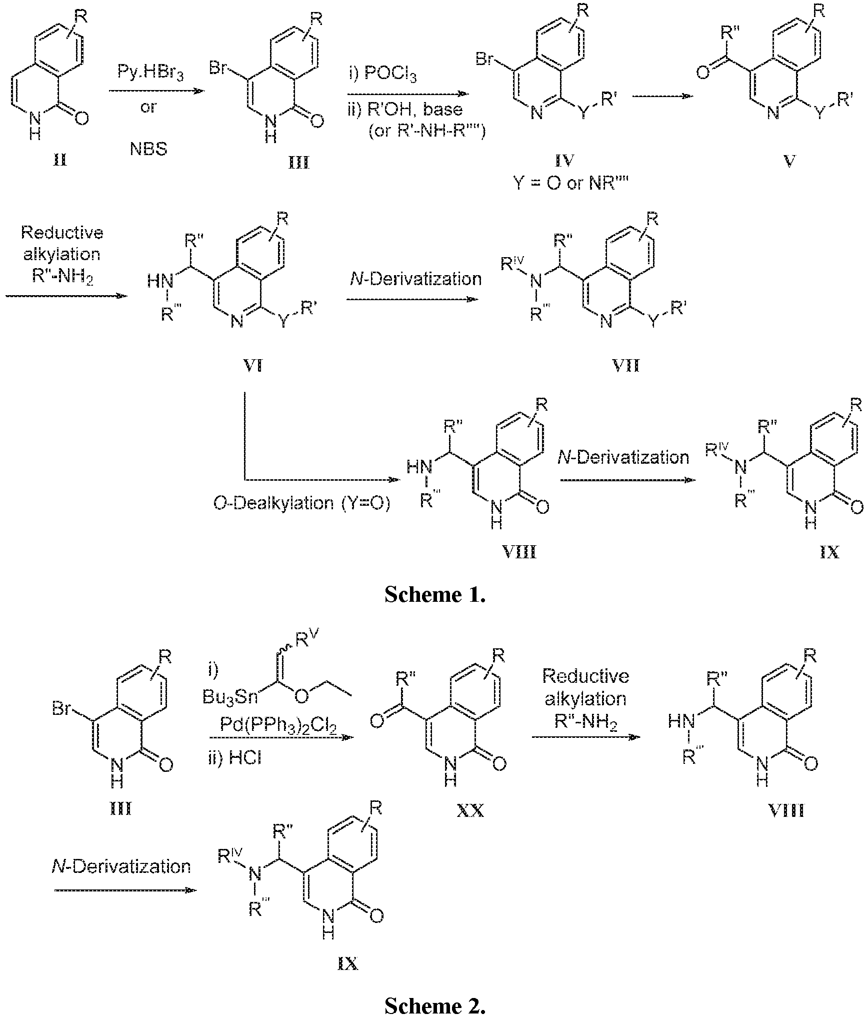

### Methods

The invention provides a method of treating or preventing hepatitis virus infection in a subject. In certain embodiments, the infection comprises hepatitis B virus (HBV) infection. In other embodiments, the method comprises administering to the subject in need thereof a therapeutically effective amount of at least one compound of the invention. In yet other embodiments, the at least one compound of the invention is the only antiviral agent administered to the subject. In yet other embodiments, the at least one compound is administered to the subject in a pharmaceutically acceptable composition. In yet other embodiments, the subject is further administered at least one additional agent useful for treating the hepatitis infection. In yet other embodiments, the at least one additional agent comprises at least one agent selected from the group consisting of reverse transcriptase inhibitor; capsid inhibitor; cccDNA formation inhibitor; RNA destabilizer; oligomeric nucleotide targeted against the HBV genome; immunostimulator, such as checkpoint inhibitor (*e.g.*, PD-L1 inhibitor); and GalNAc-siRNA conjugate targeted against an HBV gene transcript. In yet other embodiments, the subject is co-administered the at least one compound and the at least one additional agent. In yet other embodiments, the at least one compound and the at least one additional agent are coformulated.

The invention further provides a method of inhibiting expression and/or function of a viral capsid protein either directly or indirectly in a subject. In certain embodiments, the method comprises administering to the subject in need thereof a therapeutically effective amount of at least one compound of the invention. In other embodiments, the at least one compound is administered to the subject in a pharmaceutically acceptable composition. In yet other embodiments, the at least one compound of the invention is the only antiviral agent administered to the subject. In yet other embodiments, the subject is further administered at least one additional agent useful for treating HBV infection. In yet other embodiments, the at least one additional agent comprises at least one agent selected from the group consisting of reverse transcriptase inhibitor; capsid inhibitor; cccDNA formation inhibitor; RNA destabilizer; oligomeric nucleotide targeted against the HBV genome; immunostimulator, such as checkpoint inhibitor (*e.g.*, PD-L1 inhibitor); and GalNAc-siRNA conjugate targeted against an HBV gene transcript. In yet other embodiments, the subject is co-administered the at least one compound and the at least one additional agent. In yet other embodiments, the at least one compound and the at least one additional agent are coformulated.

In certain embodiments, the subject is a mammal. In other embodiments, the mammal is a human.

### Pharmaceutical Compositions and Formulations

The invention provides pharmaceutical compositions comprising at least one compound of the invention or a salt or solvate thereof, which are useful to practice methods of the invention. Such a pharmaceutical composition may consist of at least one compound of the invention or a salt or solvate thereof, in a form suitable for administration to a subject, or the pharmaceutical composition may comprise at least one compound of the invention or a salt or solvate thereof, and one or more pharmaceutically acceptable carriers, one or more additional ingredients, or any combinations of these. At least one compound of the invention may be present in the pharmaceutical composition in the form of a physiologically acceptable salt, such as in combination with a physiologically acceptable cation or anion, as is well known in the art.

In certain embodiments, the pharmaceutical compositions useful for practicing the method of the invention may be administered to deliver a dose of between 1 ng/kg/day and 100 mg/kg/day. In other embodiments, the pharmaceutical compositions useful for practicing the invention may be administered to deliver a dose of between 1 ng/kg/day and 1,000 mg/kg/day.

The relative amounts of the active ingredient, the pharmaceutically acceptable carrier, and any additional ingredients in a pharmaceutical composition of the invention will vary, depending upon the identity, size, and condition of the subject treated and further depending upon the route by which the composition is to be administered. By way of example, the composition may comprise between 0.1% and 100% (w/w) active ingredient.

Pharmaceutical compositions that are useful in the methods of the invention may be suitably developed for nasal, inhalational, oral, rectal, vaginal, pleural, peritoneal, parenteral, topical, transdermal, pulmonary, intranasal, buccal, ophthalmic, epidural, intrathecal, intravenous, or another route of administration. A composition useful within the methods of the invention may be directly administered to the brain, the brainstem, or any other part of the central nervous system of a mammal or bird. Other contemplated formulations include projected nanoparticles, microspheres, liposomal preparations, coated particles, polymer conjugates, resealed erythrocytes containing the active ingredient, and immunologically-based formulations.

In certain embodiments, the compositions of the invention are part of a pharmaceutical matrix, which allows for manipulation of insoluble materials and improvement of the bioavailability thereof, development of controlled or sustained release products, and generation of homogeneous compositions. By way of example, a pharmaceutical matrix may be prepared using hot melt extrusion, solid solutions, solid dispersions, size reduction technologies, molecular complexes (*e.g.*, cyclodextrins, and others), microparticulate, and particle and formulation coating processes. Amorphous or crystalline phases may be used in such processes.

The route(s) of administration will be readily apparent to the skilled artisan and will depend upon any number of factors including the type and severity of the disease being treated, the type and age of the veterinary or human patient being treated, and the like.

The formulations of the pharmaceutical compositions described herein may be prepared by any method known or hereafter developed in the art of pharmacology and pharmaceutics. In general, such preparatory methods include the step of bringing the active ingredient into association with a carrier or one or more other accessory ingredients, and then, if necessary or desirable, shaping or packaging the product into a desired single-dose or multi-dose unit.

As used herein, a "unit dose" is a discrete amount of the pharmaceutical composition comprising a predetermined amount of the active ingredient. The amount of the active ingredient is generally equal to the dosage of the active ingredient that would be administered to a subject or a convenient fraction of such a dosage such as, for example, one-half or one-third of such a dosage. The unit dosage form may be for a single daily dose or one of multiple daily doses (e.g., about 1 to 4 or more times per day). When multiple daily doses are used, the unit dosage form may be the same or different for each dose.

Although the descriptions of pharmaceutical compositions provided herein are principally directed to pharmaceutical compositions suitable for ethical administration to humans, it will be understood by the skilled artisan that such compositions are generally suitable for administration to animals of all sorts. Modification of pharmaceutical compositions suitable for administration to humans in order to render the compositions suitable for administration to various animals is well understood, and the ordinarily skilled veterinary pharmacologist can design and perform such modification with merely ordinary, if any, experimentation. Subjects to which administration of the pharmaceutical compositions of the invention is contemplated include, but are not limited to, humans and other primates, mammals including commercially relevant mammals such as cattle, pigs, horses, sheep, cats, and dogs.

In certain embodiments, the compositions of the invention are formulated using one or more pharmaceutically acceptable excipients or carriers. In certain embodiments, the pharmaceutical compositions of the invention comprise a therapeutically effective amount of at least one compound of the invention and a pharmaceutically acceptable carrier. Pharmaceutically acceptable carriers, which are useful, include, but are not limited to, glycerol, water, saline, ethanol, recombinant human albumin (*e.g.*, RECOMBUMIN^{®}), solubilized gelatins (*e.g.*, GELOFUSINE^{®}), and other pharmaceutically acceptable salt solutions such as phosphates and salts of organic acids. Examples of these and other pharmaceutically acceptable carriers are described in Remington's Pharmaceutical Sciences (1991, Mack Publication Co., New Jersey).

The carrier may be a solvent or dispersion medium containing, for example, water, ethanol, polyol (for example, glycerol, propylene glycol, and liquid polyethylene glycol, and the like), recombinant human albumin, solubilized gelatins, suitable mixtures thereof, and vegetable oils. The proper fluidity may be maintained, for example, by the use of a coating such as lecithin, by the maintenance of the required particle size in the case of dispersion and by the use of surfactants. Prevention of the action of microorganisms may be achieved by various antibacterial and antifungal agents, for example, parabens, chlorobutanol, phenol, ascorbic acid, thimerosal, and the like. In many cases, isotonic agents, for example, sugars, sodium chloride, or polyalcohols such as mannitol and sorbitol, are included in the composition. Prolonged absorption of the injectable compositions may be brought about by including in the composition an agent that delays absorption, for example, aluminum monostearate or gelatin.

Formulations may be employed in admixtures with conventional excipients, *i.e.*, pharmaceutically acceptable organic or inorganic carrier substances suitable for oral, parenteral, nasal, inhalational, intravenous, subcutaneous, transdermal enteral, or any other suitable mode of administration, known to the art. The pharmaceutical preparations may be sterilized and if desired mixed with auxiliary agents, e.g., lubricants, preservatives, stabilizers, wetting agents, emulsifiers, salts for influencing osmotic pressure buffers, coloring, flavoring, and/or fragrance-conferring substances and the like. They may also be combined where desired with other active agents, *e.g.*, other analgesic, anxiolytics or hypnotic agents. As used herein, "additional ingredients" include, but are not limited to, one or more ingredients that may be used as a pharmaceutical carrier.

The composition of the invention may comprise a preservative from about 0.005% to 2.0% by total weight of the composition. The preservative is used to prevent spoilage in the case of exposure to contaminants in the environment. Examples of preservatives useful in accordance with the invention include but are not limited to those selected from the group consisting of benzyl alcohol, sorbic acid, parabens, imidurea and any combinations thereof. One such preservative is a combination of about 0.5% to 2.0% benzyl alcohol and 0.05-0.5% sorbic acid.

The composition may include an antioxidant and a chelating agent that inhibit the degradation of the compound. Antioxidants for some compounds are BHT, BHA, alpha-tocopherol and ascorbic acid in the exemplary range of about 0.01% to 0.3%, or BHT in the range of 0.03% to 0.1% by weight by total weight of the composition. The chelating agent may be present in an amount of from 0.01% to 0.5% by weight by total weight of the composition. Exemplary chelating agents include edetate salts (*e.g.* disodium edetate) and citric acid in the weight range of about 0.01% to 0.20%, or in the range of 0.02% to 0.10% by weight by total weight of the composition. The chelating agent is useful for chelating metal ions in the composition that may be detrimental to the shelf life of the formulation. While BHT and disodium edetate are exemplary antioxidant and chelating agent, respectively, for some compounds, other suitable and equivalent antioxidants and chelating agents may be substituted therefore as would be known to those skilled in the art.

Liquid suspensions may be prepared using conventional methods to achieve suspension of the active ingredient in an aqueous or oily vehicle. Aqueous vehicles include, for example, water, and isotonic saline. Oily vehicles include, for example, almond oil, oily esters, ethyl alcohol, vegetable oils such as arachis, olive, sesame, or coconut oil, fractionated vegetable oils, and mineral oils such as liquid paraffin. Liquid suspensions may further comprise one or more additional ingredients including, but not limited to, suspending agents, dispersing or wetting agents, emulsifying agents, demulcents, preservatives, buffers, salts, flavorings, coloring agents, and sweetening agents. Oily suspensions may further comprise a thickening agent. Known suspending agents include, but are not limited to, sorbitol syrup, hydrogenated edible fats, sodium alginate, polyvinylpyrrolidone, gum tragacanth, gum acacia, and cellulose derivatives such as sodium carboxymethylcellulose, methylcellulose, hydroxypropylmethyl cellulose. Known dispersing or wetting agents include, but are not limited to, naturally-occurring phosphatides such as lecithin, condensation products of an alkylene oxide with a fatty acid, with a long chain aliphatic alcohol, with a partial ester derived from a fatty acid and a hexitol, or with a partial ester derived from a fatty acid and a hexitol anhydride (*e.g.*, polyoxyethylene stearate, heptadecaethyleneoxycetanol, polyoxyethylene sorbitol monooleate, and polyoxyethylene sorbitan monooleate, respectively). Known emulsifying agents include, but are not limited to, lecithin, acacia, and ionic or non-ionic surfactants. Known preservatives include, but are not limited to, methyl, ethyl, or n-propyl para-hydroxybenzoates, ascorbic acid, and sorbic acid. Known sweetening agents include, for example, glycerol, propylene glycol, sorbitol, sucrose, and saccharin.

Liquid solutions of the active ingredient in aqueous or oily solvents may be prepared in substantially the same manner as liquid suspensions, the primary difference being that the active ingredient is dissolved, rather than suspended in the solvent. As used herein, an "oily" liquid is one which comprises a carbon-containing liquid molecule and which exhibits a less polar character than water. Liquid solutions of the pharmaceutical composition of the invention may comprise each of the components described with regard to liquid suspensions, it being understood that suspending agents will not necessarily aid dissolution of the active ingredient in the solvent. Aqueous solvents include, for example, water, and isotonic saline. Oily solvents include, for example, almond oil, oily esters, ethyl alcohol, vegetable oils such as arachis, olive, sesame, or coconut oil, fractionated vegetable oils, and mineral oils such as liquid paraffin.

Powdered and granular formulations of a pharmaceutical preparation of the invention may be prepared using known methods. Such formulations may be administered directly to a subject, used, for example, to form tablets, to fill capsules, or to prepare an aqueous or oily suspension or solution by addition of an aqueous or oily vehicle thereto. Each of these formulations may further comprise one or more of dispersing or wetting agent, a suspending agent, ionic and non-ionic surfactants, and a preservative. Additional excipients, such as fillers and sweetening, flavoring, or coloring agents, may also be included in these formulations.

A pharmaceutical composition of the invention may also be prepared, packaged, or sold in the form of oil-in-water emulsion or a water-in-oil emulsion. The oily phase may be a vegetable oil such as olive or arachis oil, a mineral oil such as liquid paraffin, or a combination of these. Such compositions may further comprise one or more emulsifying agents such as naturally occurring gums such as gum acacia or gum tragacanth, naturally-occurring phosphatides such as soybean or lecithin phosphatide, esters or partial esters derived from combinations of fatty acids and hexitol anhydrides such as sorbitan monooleate, and condensation products of such partial esters with ethylene oxide such as polyoxyethylene sorbitan monooleate. These emulsions may also contain additional ingredients including, for example, sweetening or flavoring agents.

Methods for impregnating or coating a material with a chemical composition are known in the art, and include, but are not limited to methods of depositing or binding a chemical composition onto a surface, methods of incorporating a chemical composition into the structure of a material during the synthesis of the material *(i.e.,* such as with a physiologically degradable material), and methods of absorbing an aqueous or oily solution or suspension into an absorbent material, with or without subsequent drying. Methods for mixing components include physical milling, the use of pellets in solid and suspension formulations and mixing in a transdermal patch, as known to those skilled in the art.

### Administration/Dosing

The regimen of administration may affect what constitutes an effective amount. The therapeutic formulations may be administered to the patient either prior to or after the onset of a disease or disorder. Further, several divided dosages, as well as staggered dosages may be administered daily or sequentially, or the dose may be continuously infused, or may be a bolus injection. Further, the dosages of the therapeutic formulations may be proportionally increased or decreased as indicated by the exigencies of the therapeutic or prophylactic situation.

Administration of the compositions of the present invention to a patient, such as a mammal, such as a human, may be carried out using known procedures, at dosages and for periods of time effective to treat a disease or disorder contemplated herein. An effective amount of the therapeutic compound necessary to achieve a therapeutic effect may vary according to factors such as the activity of the particular compound employed; the time of administration; the rate of excretion of the compound; the duration of the treatment; other drugs, compounds or materials used in combination with the compound; the state of the disease or disorder, age, sex, weight, condition, general health and prior medical history of the patient being treated, and like factors well-known in the medical arts. Dosage regimens may be adjusted to provide the optimum therapeutic response. For example, several divided doses may be administered daily or the dose may be proportionally reduced as indicated by the exigencies of the therapeutic situation. A non-limiting example of an effective dose range for a therapeutic compound of the invention is from about 0.01 mg/kg to 100 mg/kg of body weight/per day. One of ordinary skill in the art would be able to study the relevant factors and make the determination regarding the effective amount of the therapeutic compound without undue experimentation.

The compound may be administered to an animal as frequently as several times daily, or it may be administered less frequently, such as once a day, once a week, once every two weeks, once a month, or even less frequently, such as once every several months or even once a year or less. It is understood that the amount of compound dosed per day may be administered, in non-limiting examples, every day, every other day, every 2 days, every 3 days, every 4 days, or every 5 days. For example, with every other day administration, a 5 mg per day dose may be initiated on Monday with a first subsequent 5 mg per day dose administered on Wednesday, a second subsequent 5 mg per day dose administered on Friday, and so on. The frequency of the dose is readily apparent to the skilled artisan and depends upon a number of factors, such as, but not limited to, type and severity of the disease being treated, and type and age of the animal.

Actual dosage levels of the active ingredients in the pharmaceutical compositions of this invention may be varied so as to obtain an amount of the active ingredient that is effective to achieve the desired therapeutic response for a particular patient, composition, and mode of administration, without being toxic to the patient.

A medical doctor, *e.g.*, physician or veterinarian, having ordinary skill in the art may readily determine and prescribe the effective amount of the pharmaceutical composition required. For example, the physician or veterinarian could start doses of the compounds of the invention employed in the pharmaceutical composition at levels lower than that required in order to achieve the desired therapeutic effect and gradually increase the dosage until the desired effect is achieved.

In particular embodiments, it is especially advantageous to formulate the compound in dosage unit form for ease of administration and uniformity of dosage. Dosage unit form as used herein refers to physically discrete units suited as unitary dosages for the patients to be treated; each unit containing a predetermined quantity of therapeutic compound calculated to produce the desired therapeutic effect in association with the required pharmaceutical vehicle. The dosage unit forms of the invention are dictated by and directly dependent on (a) the unique characteristics of the therapeutic compound and the particular therapeutic effect to be achieved, and (b) the limitations inherent in the art of compounding/formulating such a therapeutic compound for the treatment of a disease or disorder in a patient.

In certain embodiments, the compositions of the invention are administered to the patient in dosages that range from one to five times per day or more. In other embodiments, the compositions of the invention are administered to the patient in range of dosages that include, but are not limited to, once every day, every two days, every three days to once a week, and once every two weeks. It will be readily apparent to one skilled in the art that the frequency of administration of the various combination compositions of the invention will vary from subject to subject depending on many factors including, but not limited to, age, disease or disorder to be treated, gender, overall health, and other factors. Thus, the invention should not be construed to be limited to any particular dosage regime and the precise dosage and composition to be administered to any patient will be determined by the attending physician taking all other factors about the patient into account.

Compounds of the invention for administration may be in the range of from about 1 µg to about 7,500 mg, about 20 µg to about 7,000 mg, about 40 µg to about 6,500 mg, about 80 µ g to about 6,000 mg, about 100 µ g to about 5,500 mg, about 200 µ g to about 5,000 mg, about 400 µ g to about 4,000 mg, about 800 µ g to about 3,000 mg, about 1 mg to about 2,500 mg, about 2 mg to about 2,000 mg, about 5 mg to about 1,000 mg, about 10 mg to about 750 mg, about 20 mg to about 600 mg, about 30 mg to about 500 mg, about 40 mg to about 400 mg, about 50 mg to about 300 mg, about 60 mg to about 250 mg, about 70 mg to about 200 mg, about 80 mg to about 150 mg, and any and all whole or partial increments there-in-between.

In some embodiments, the dose of a compound of the invention is from about 0.5 µg and about 5,000 mg. In some embodiments, a dose of a compound of the invention used in compositions described herein is less than about 5,000 mg, or less than about 4,000 mg, or less than about 3,000 mg, or less than about 2,000 mg, or less than about 1,000 mg, or less than about 800 mg, or less than about 600 mg, or less than about 500 mg, or less than about 200 mg, or less than about 50 mg. Similarly, in some embodiments, a dose of a second compound as described herein is less than about 1,000 mg, or less than about 800 mg, or less than about 600 mg, or less than about 500 mg, or less than about 400 mg, or less than about 300 mg, or less than about 200 mg, or less than about 100 mg, or less than about 50 mg, or less than about 40 mg, or less than about 30 mg, or less than about 25 mg, or less than about 20 mg, or less than about 15 mg, or less than about 10 mg, or less than about 5 mg, or less than about 2 mg, or less than about 1 mg, or less than about 0.5 mg, and any and all whole or partial increments thereof.

In certain embodiments, the present invention is directed to a packaged pharmaceutical composition comprising a container holding a therapeutically effective amount of a compound of the invention, alone or in combination with a second pharmaceutical agent; and instructions for using the compound to treat, prevent, or reduce one or more symptoms of a disease or disorder in a patient.

The term "container" includes any receptacle for holding the pharmaceutical composition or for managing stability or water uptake. For example, in certain embodiments, the container is the packaging that contains the pharmaceutical composition, such as liquid (solution and suspension), semisolid, lyophilized solid, solution and powder or lyophilized formulation present in dual chambers. In other embodiments, the container is not the packaging that contains the pharmaceutical composition, *i.e.*, the container is a receptacle, such as a box or vial that contains the packaged pharmaceutical composition or unpackaged pharmaceutical composition and the instructions for use of the pharmaceutical composition. Moreover, packaging techniques are well known in the art. It should be understood that the instructions for use of the pharmaceutical composition may be contained on the packaging containing the pharmaceutical composition, and as such the instructions form an increased functional relationship to the packaged product. However, it should be understood that the instructions may contain information pertaining to the compound's ability to perform its intended function, e.g., treating, preventing, or reducing a disease or disorder in a patient.

### Administration

Routes of administration of any of the compositions of the invention include inhalational, oral, nasal, rectal, parenteral, sublingual, transdermal, transmucosal (e.g., sublingual, lingual, (trans)buccal, (trans)urethral, vaginal (e.g., trans- and perivaginally), (intra)nasal, and (trans)rectal), intravesical, intrapulmonary, intraduodenal, intragastrical, intrathecal, epidural, intrapleural, intraperitoneal, subcutaneous, intramuscular, intradermal, intra-arterial, intravenous, intrabronchial, inhalation, and topical administration.

Suitable compositions and dosage forms include, for example, tablets, capsules, caplets, pills, gel caps, troches, emulsions, dispersions, suspensions, solutions, syrups, granules, beads, transdermal patches, gels, powders, pellets, magmas, lozenges, creams, pastes, plasters, lotions, discs, suppositories, liquid sprays for nasal or oral administration, dry powder or aerosolized formulations for inhalation, compositions and formulations for intravesical administration and the like. It should be understood that the formulations and compositions that would be useful in the present invention are not limited to the particular formulations and compositions that are described herein.

### Oral Administration

For oral application, particularly suitable are tablets, dragees, liquids, drops, capsules, caplets and gelcaps. Other formulations suitable for oral administration include, but are not limited to, a powdered or granular formulation, an aqueous or oily suspension, an aqueous or oily solution, a paste, a gel, toothpaste, a mouthwash, a coating, an oral rinse, or an emulsion. The compositions intended for oral use may be prepared according to any method known in the art and such compositions may contain one or more agents selected from the group consisting of inert, non-toxic, generally recognized as safe (GRAS) pharmaceutically excipients which are suitable for the manufacture of tablets. Such excipients include, for example an inert diluent such as lactose; granulating and disintegrating agents such as cornstarch; binding agents such as starch; and lubricating agents such as magnesium stearate.

Tablets may be non-coated or they may be coated using known methods to achieve delayed disintegration in the gastrointestinal tract of a subject, thereby providing sustained release and absorption of the active ingredient. By way of example, a material such as glyceryl monostearate or glyceryl distearate may be used to coat tablets. Further by way of example, tablets may be coated using methods described in U.S. Patents Nos. 4,256,108; 4,160,452; and 4,265,874 to form osmotically controlled release tablets. Tablets may further comprise a sweetening agent, a flavoring agent, a coloring agent, a preservative, or some combination of these in order to provide for pharmaceutically elegant and palatable preparation. Hard capsules comprising the active ingredient may be made using a physiologically degradable composition, such as gelatin. The capsules comprise the active ingredient, and may further comprise additional ingredients including, for example, an inert solid diluent such as calcium carbonate, calcium phosphate, or kaolin.

Hard capsules comprising the active ingredient may be made using a physiologically degradable composition, such as gelatin. Such hard capsules comprise the active ingredient, and may further comprise additional ingredients including, for example, an inert solid diluent such as calcium carbonate, calcium phosphate, or kaolin.

Soft gelatin capsules comprising the active ingredient may be made using a physiologically degradable composition, such as gelatin from animal-derived collagen or from a hypromellose, a modified form of cellulose, and manufactured using optional mixtures of gelatin, water and plasticizers such as sorbitol or glycerol. Such soft capsules comprise the active ingredient, which may be mixed with water or an oil medium such as peanut oil, liquid paraffin, or olive oil.

For oral administration, the compounds of the invention may be in the form of tablets or capsules prepared by conventional means with pharmaceutically acceptable excipients such as binding agents; fillers; lubricants; disintegrates; or wetting agents. If desired, the tablets may be coated using suitable methods and coating materials such as OPADRY^{®} film coating systems available from Colorcon, West Point, Pa. (e.g., OPADRY^{®} OY Type, OYC Type, Organic Enteric OY-P Type, Aqueous Enteric OY-A Type, OY-PM Type and OPADRY^{®} White, 32K18400). It is understood that similar type of film coating or polymeric products from other companies may be used.

A tablet comprising the active ingredient may, for example, be made by compressing or molding the active ingredient, optionally with one or more additional ingredients. Compressed tablets may be prepared by compressing, in a suitable device, the active ingredient in a free-flowing form such as a powder or granular preparation, optionally mixed with one or more of a binder, a lubricant, an excipient, a surface-active agent, and a dispersing agent. Molded tablets may be made by molding, in a suitable device, a mixture of the active ingredient, a pharmaceutically acceptable carrier, and at least sufficient liquid to moisten the mixture. Pharmaceutically acceptable excipients used in the manufacture of tablets include, but are not limited to, inert diluents, granulating and disintegrating agents, binding agents, and lubricating agents. Known dispersing agents include, but are not limited to, potato starch and sodium starch glycolate. Known surface-active agents include, but are not limited to, sodium lauryl sulphate. Known diluents include, but are not limited to, calcium carbonate, sodium carbonate, lactose, microcrystalline cellulose, calcium phosphate, calcium hydrogen phosphate, and sodium phosphate. Known granulating and disintegrating agents include, but are not limited to, corn starch and alginic acid. Known binding agents include, but are not limited to, gelatin, acacia, pre-gelatinized maize starch, polyvinylpyrrolidone, and hydroxypropyl methylcellulose. Known lubricating agents include, but are not limited to, magnesium stearate, stearic acid, silica, and talc.

Granulating techniques are well known in the pharmaceutical art for modifying starting powders or other particulate materials of an active ingredient. The powders are typically mixed with a binder material into larger permanent free-flowing agglomerates or granules referred to as a "granulation." For example, solvent-using "wet" granulation processes are generally characterized in that the powders are combined with a binder material and moistened with water or an organic solvent under conditions resulting in the formation of a wet granulated mass from which the solvent must then be evaporated.

Melt granulation generally consists in the use of materials that are solid or semi-solid at room temperature *(i.e.,* having a relatively low softening or melting point range) to promote granulation of powdered or other materials, essentially in the absence of added water or other liquid solvents. The low melting solids, when heated to a temperature in the melting point range, liquefy to act as a binder or granulating medium. The liquefied solid spreads itself over the surface of powdered materials with which it is contacted, and on cooling, forms a solid granulated mass in which the initial materials are bound together. The resulting melt granulation may then be provided to a tablet press or be encapsulated for preparing the oral dosage form. Melt granulation improves the dissolution rate and bioavailability of an active *(i.e.,* drug) by forming a solid dispersion or solid solution.

U.S. Patent No. 5,169,645 discloses directly compressible wax-containing granules having improved flow properties. The granules are obtained when waxes are admixed in the melt with certain flow improving additives, followed by cooling and granulation of the admixture. In certain embodiments, only the wax itself melts in the melt combination of the wax(es) and additives(s), and in other cases both the wax(es) and the additives(s) will melt.

The present invention also includes a multi-layer tablet comprising a layer providing for the delayed release of one or more compounds useful within the methods of the invention, and a further layer providing for the immediate release of one or more compounds useful within the methods of the invention. Using a wax/pH-sensitive polymer mix, a gastric insoluble composition may be obtained in which the active ingredient is entrapped, ensuring its delayed release.

Liquid preparation for oral administration may be in the form of solutions, syrups or suspensions. The liquid preparations may be prepared by conventional means with pharmaceutically acceptable additives such as suspending agents (*e.g.*, sorbitol syrup, methyl cellulose or hydrogenated edible fats); emulsifying agent (*e.g.*, lecithin or acacia); nonaqueous vehicles (*e.g.*, almond oil, oily esters or ethyl alcohol); and preservatives (*e.g.*, methyl or propyl para-hydroxy benzoates or sorbic acid). Liquid formulations of a pharmaceutical composition of the invention which are suitable for oral administration may be prepared, packaged, and sold either in liquid form or in the form of a dry product intended for reconstitution with water or another suitable vehicle prior to use.

### Parenteral Administration

As used herein, "parenteral administration" of a pharmaceutical composition includes any route of administration characterized by physical breaching of a tissue of a subject and administration of the pharmaceutical composition through the breach in the tissue. Parenteral administration thus includes, but is not limited to, administration of a pharmaceutical composition by injection of the composition, by application of the composition through a surgical incision, by application of the composition through a tissue-penetrating non-surgical wound, and the like. In particular, parenteral administration is contemplated to include, but is not limited to, subcutaneous, intravenous, intraperitoneal, intramuscular, intrasternal injection, and kidney dialytic infusion techniques.

Formulations of a pharmaceutical composition suitable for parenteral administration comprise the active ingredient combined with a pharmaceutically acceptable carrier, such as sterile water or sterile isotonic saline. Such formulations may be prepared, packaged, or sold in a form suitable for bolus administration or for continuous administration. Injectable formulations may be prepared, packaged, or sold in unit dosage form, such as in ampules or in multidose containers containing a preservative. Injectable formulations may also be prepared, packaged, or sold in devices such as patient-controlled analgesia (PCA) devices. Formulations for parenteral administration include, but are not limited to, suspensions, solutions, emulsions in oily or aqueous vehicles, pastes, and implantable sustained-release or biodegradable formulations. Such formulations may further comprise one or more additional ingredients including, but not limited to, suspending, stabilizing, or dispersing agents. In one embodiment of a formulation for parenteral administration, the active ingredient is provided in dry (*i.e.*, powder or granular) form for reconstitution with a suitable vehicle (*e.g.*, sterile pyrogen-free water) prior to parenteral administration of the reconstituted composition.

The pharmaceutical compositions may be prepared, packaged, or sold in the form of a sterile injectable aqueous or oily suspension or solution. This suspension or solution may be formulated according to the known art, and may comprise, in addition to the active ingredient, additional ingredients such as the dispersing agents, wetting agents, or suspending agents described herein. Such sterile injectable formulations may be prepared using a non-toxic parenterally acceptable diluent or solvent, such as water or 1,3-butanediol, for example. Other acceptable diluents and solvents include, but are not limited to, Ringer's solution, isotonic sodium chloride solution, and fixed oils such as synthetic mono- or di-glycerides. Other parentally-administrable formulations which are useful include those which comprise the active ingredient in microcrystalline form in a recombinant human albumin, a fluidized gelatin, in a liposomal preparation, or as a component of a biodegradable polymer system. Compositions for sustained release or implantation may comprise pharmaceutically acceptable polymeric or hydrophobic materials such as an emulsion, an ion exchange resin, a sparingly soluble polymer, or a sparingly soluble salt.

### Topical Administration

An obstacle for topical administration of pharmaceuticals is the stratum corneum layer of the epidermis. The stratum corneum is a highly resistant layer comprised of protein, cholesterol, sphingolipids, free fatty acids and various other lipids, and includes cornified and living cells. One of the factors that limit the penetration rate (flux) of a compound through the stratum corneum is the amount of the active substance that can be loaded or applied onto the skin surface. The greater the amount of active substance which is applied per unit of area of the skin, the greater the concentration gradient between the skin surface and the lower layers of the skin, and in turn the greater the diffusion force of the active substance through the skin. Therefore, a formulation containing a greater concentration of the active substance is more likely to result in penetration of the active substance through the skin, and more of it, and at a more consistent rate, than a formulation having a lesser concentration, all other things being equal.

Formulations suitable for topical administration include, but are not limited to, liquid or semi-liquid preparations such as liniments, lotions, oil-in-water or water-in-oil emulsions such as creams, ointments or pastes, and solutions or suspensions. Topically administrable formulations may, for example, comprise from about 1% to about 10% (w/w) active ingredient, although the concentration of the active ingredient may be as high as the solubility limit of the active ingredient in the solvent. Formulations for topical administration may further comprise one or more of the additional ingredients described herein.

Enhancers of permeation may be used. These materials increase the rate of penetration of drugs across the skin. Typical enhancers in the art include ethanol, glycerol monolaurate, PGML (polyethylene glycol monolaurate), dimethylsulfoxide, and the like. Other enhancers include oleic acid, oleyl alcohol, ethoxydiglycol, laurocapram, alkanecarboxylic acids, dimethylsulfoxide, polar lipids, or *N*-methyl-2-pyrrolidone.

One acceptable vehicle for topical delivery of some of the compositions of the invention may contain liposomes. The composition of the liposomes and their use are known in the art *(i.e.,* U.S. Patent No. 6,323,219).

In alternative embodiments, the topically active pharmaceutical composition may be optionally combined with other ingredients such as adjuvants, anti-oxidants, chelating agents, surfactants, foaming agents, wetting agents, emulsifying agents, viscosifiers, buffering agents, preservatives, and the like. In other embodiments, a permeation or penetration enhancer is included in the composition and is effective in improving the percutaneous penetration of the active ingredient into and through the stratum corneum with respect to a composition lacking the permeation enhancer. Various permeation enhancers, including oleic acid, oleyl alcohol, ethoxydiglycol, laurocapram, alkanecarboxylic acids, dimethylsulfoxide, polar lipids, or N-methyl-2-pyrrolidone, are known to those of skill in the art. In another aspect, the composition may further comprise a hydrotropic agent, which functions to increase disorder in the structure of the stratum corneum, and thus allows increased transport across the stratum corneum. Various hydrotropic agents such as isopropyl alcohol, propylene glycol, or sodium xylene sulfonate, are known to those of skill in the art.

The topically active pharmaceutical composition should be applied in an amount effective to affect desired changes. As used herein "amount effective" shall mean an amount sufficient to cover the region of skin surface where a change is desired. An active compound should be present in the amount of from about 0.0001% to about 15% by weight volume of the composition. For example, it should be present in an amount from about 0.0005% to about 5% of the composition; for example, it should be present in an amount of from about 0.001% to about 1% of the composition. Such compounds may be synthetically-or naturally derived.

### Buccal Administration

A pharmaceutical composition of the invention may be prepared, packaged, or sold in a formulation suitable for buccal administration. Such formulations may, for example, be in the form of tablets or lozenges made using conventional methods, and may contain, for example, 0.1 to 20% (w/w) of the active ingredient, the balance comprising an orally dissolvable or degradable composition and, optionally, one or more of the additional ingredients described herein. Alternately, formulations suitable for buccal administration may comprise a powder or an aerosolized or atomized solution or suspension comprising the active ingredient. Such powdered, aerosolized, or aerosolized formulations, when dispersed, may have an average particle or droplet size in the range from about 0.1 to about 200 nanometers, and may further comprise one or more of the additional ingredients described herein. The examples of formulations described herein are not exhaustive and it is understood that the invention includes additional modifications of these and other formulations not described herein, but which are known to those of skill in the art.

### Rectal Administration

A pharmaceutical composition of the invention may be prepared, packaged, or sold in a formulation suitable for rectal administration. Such a composition may be in the form of, for example, a suppository, a retention enema preparation, and a solution for rectal or colonic irrigation.

Suppository formulations may be made by combining the active ingredient with a non-irritating pharmaceutically acceptable excipient which is solid at ordinary room temperature *(i.e.,* about 20°C) and which is liquid at the rectal temperature of the subject (*i.e.*, about 37°C in a healthy human). Suitable pharmaceutically acceptable excipients include, but are not limited to, cocoa butter, polyethylene glycols, and various glycerides. Suppository formulations may further comprise various additional ingredients including, but not limited to, antioxidants, and preservatives.

Retention enema preparations or solutions for rectal or colonic irrigation may be made by combining the active ingredient with a pharmaceutically acceptable liquid carrier. As is well known in the art, enema preparations may be administered using, and may be packaged within, a delivery device adapted to the rectal anatomy of the subject. Enema preparations may further comprise various additional ingredients including, but not limited to, antioxidants, and preservatives.

### Additional Administration Forms

Additional dosage forms of this invention include dosage forms as described in U.S. Patents Nos. 6,340,475, 6,488,962, 6,451,808, 5,972,389, 5,582,837, and 5,007,790. Additional dosage forms of this invention also include dosage forms as described in U.S. Patent Applications Nos. 20030147952, 20030104062, 20030104053, 20030044466, 20030039688, and 20020051820. Additional dosage forms of this invention also include dosage forms as described in PCT Applications Nos. WO 03/35041, WO 03/35040, WO 03/35029, WO 03/35177, WO 03/35039, WO 02/96404, WO 02/32416, WO 01/97783, WO 01/56544, WO 01/32217, WO 98/55107, WO 98/11879, WO 97/47285, WO 93/18755, and WO 90/11757.

### Controlled Release Formulations and Drug Delivery Systems:

In certain embodiments, the compositions and/or formulations of the present invention may be, but are not limited to, short-term, rapid-offset, as well as controlled, for example, sustained release, delayed release and pulsatile release formulations.

The term sustained release is used in its conventional sense to refer to a drug formulation that provides for gradual release of a drug over an extended period of time, and that may, although not necessarily, result in substantially constant blood levels of a drug over an extended time period. The period of time may be as long as a month or more and should be a release which is longer that the same amount of agent administered in bolus form.

For sustained release, the compounds may be formulated with a suitable polymer or hydrophobic material which provides sustained release properties to the compounds. As such, the compounds for use the method of the invention may be administered in the form of microparticles, for example, by injection or in the form of wafers or discs by implantation.

In certain embodiments of the invention, the compounds useful within the invention are administered to a subject, alone or in combination with another pharmaceutical agent, using a sustained release formulation.

The term delayed release is used herein in its conventional sense to refer to a drug formulation that provides for an initial release of the drug after some delay following drug administration and that may, although not necessarily, include a delay of from about 10 minutes up to about 12 hours.

The term pulsatile release is used herein in its conventional sense to refer to a drug formulation that provides release of the drug in such a way as to produce pulsed plasma profiles of the drug after drug administration.

The term immediate release is used in its conventional sense to refer to a drug formulation that provides for release of the drug immediately after drug administration.

As used herein, short-term refers to any period of time up to and including about 8 hours, about 7 hours, about 6 hours, about 5 hours, about 4 hours, about 3 hours, about 2 hours, about 1 hour, about 40 minutes, about 20 minutes, or about 10 minutes and any or all whole or partial increments thereof after drug administration after drug administration.

As used herein, rapid-offset refers to any period of time up to and including about 8 hours, about 7 hours, about 6 hours, about 5 hours, about 4 hours, about 3 hours, about 2 hours, about 1 hour, about 40 minutes, about 20 minutes, or about 10 minutes, and any and all whole or partial increments thereof after drug administration.

Those skilled in the art will recognize, or be able to ascertain using no more than routine experimentation, numerous equivalents to the specific procedures, embodiments, claims, and examples described herein. Such equivalents were considered to be within the scope of this invention and covered by the claims appended hereto. For example, it should be understood, that modifications in reaction conditions, including but not limited to reaction times, reaction size/volume, and experimental reagents, such as solvents, catalysts, pressures, atmospheric conditions, e.g., nitrogen atmosphere, and reducing/oxidizing agents, with art-recognized alternatives and using no more than routine experimentation, are within the scope of the present application.

It is to be understood that, wherever values and ranges are provided herein, the description in range format is merely for convenience and brevity and should not be construed as an inflexible limitation on the scope of the invention. Accordingly, all values and ranges encompassed by these values and ranges are meant to be encompassed within the scope of the present invention. Moreover, all values that fall within these ranges, as well as the upper or lower limits of a range of values, are also contemplated by the present application. The description of a range should be considered to have specifically disclosed all the possible sub-ranges as well as individual numerical values within that range and, when appropriate, partial integers of the numerical values within ranges. For example, description of a range such as from 1 to 6 should be considered to have specifically disclosed sub-ranges such as from 1 to 3, from 1 to 4, from 1 to 5, from 2 to 4, from 2 to 6, from 3 to 6 etc., as well as individual numbers within that range, for example, 1, 2, 2.7, 3, 4, 5, 5.3, and 6. This applies regardless of the breadth of the range.

The following examples further illustrate aspects of the present invention. However, they are in no way a limitation of the teachings or disclosure of the present invention as set forth herein.

### EXAMPLES

The invention is now described with reference to the following Examples. These Examples are provided for the purpose of illustration only, and the invention is not limited to these Examples, but rather encompasses all variations that are evident as a result of the teachings provided herein.

### Materials & Methods

The following procedures can be utilized in evaluating and selecting compounds that inhibit hepatitis B virus infection.

### HepDE19 assay with bDNA quantitation of HBV rcDNA:

HepDE19 cell culture system is a HepG2 (human hepatocarcinoma) derived cell line that supports HBV DNA replication and cccDNA formation in a tetracycline (Tet)-regulated manner and produces HBV rcDNA and a detectable reporter molecule dependent on the production and maintenance of cccDNA (Guo, et al.,2007, J. Virol. 81:12472-12484).

HepDE19 (50,000 cells/well) were plated in 96-well collagen-coated tissue-culture treated microtiter plates in DMEM/F12 medium supplemented with 10% fetal bovine serum, 1% penicillin-streptomycin and 1 µg/mL tetracycline and incubated in a humidified incubator at 37 °C and 5% CO₂ overnight. Next day, the cells were switched to fresh medium without tetracycline and incubated for 4 hours at 37 °C and 5% CO₂. The cells were treated with fresh Tet-free medium with compounds at concentrations starting at 25 µM and a serial, ½ log, 8-point, titration series in duplicate. The final DMSO concentration in the assay was 0.5%. The plates were incubated for 7 days in a humidified incubator at 37 °C and 5% CO₂. Following a 7 day-incubation, the level of rcDNA present in the inhibitor-treated wells was measured using a Quantigene 2.0 bDNA assay kit (Affymetrix, Santa Clara, CA) with HBV specific custom probe set and manufacturers instructions. Concurrently, the effect of compounds on cell viability was assessed using replicate plates, plated at a density of 5,000 cells/well and incubated for 4 days, to determine the ATP content as a measure of cell viability using the cell-titer glo reagent (CTG; Promega Corporation, Madison, WI) as per manufacturer's instructions. The plates were read using a Victor luminescence plate reader (PerkinElmer Model 1420 Multilabel counter) and the relative luminescence units (RLU) data generated from each well was calculated as % inhibition of the untreated control wells and analyzed using XL-Fit module in Microsoft Excel to determine EC₅₀ and EC₉₀ (bDNA) and CC₅₀ (CTG) values using a 4-parameter curve fitting algorithm.

### LCMS Methods:

**LCMS Method A:** Waters Acquity UPLC system employing a Waters Acquity UPLC BEH C18, 1.7 µm, 50 x 2.1 mm column with an aqueous acetonitrile based solvent gradient of 2-98% CH₃CN/H₂O (0.05 % TFA) over 9.5 mins. Flow rate = 0.8 mL/min.
**LCMS Method B:** Shimadzu UFLC system employing an ACE UltraCore Super PhenylHexyl, 2.5 µm, 50 x 2.1 mm column with an aqueous acetonitrile based solvent gradient of 5-100% CH₃CN/H₂O (0.05 % Formic acid) over 4.0 mins followed by maintenance of 100% CH₃CN (0.05 % Formic acid) for a further 1 min. Flow rate = 1.0 mL/min.

As described herein, "Enantiomer I" or "Diastereomer I" refers to the first enantiomer or diastereomer eluded from the chiral column under the specific chiral analytical conditions detailed for examples provided elsewhere herein; and "Enantiomer 11" or "Diastereomer II" refers to the second enantiomer or diastereomer eluded from the chiral column under the specific chiral analytical conditions detailed for examples provided elsewhere herein. Such nomenclature does not imply or impart any particular relative and/or absolute configuration for these compounds.

### EXAMPLE 1: COMPOUNDS

### 4-Bromoisoquinolin-1(2H)-one (IIIa)

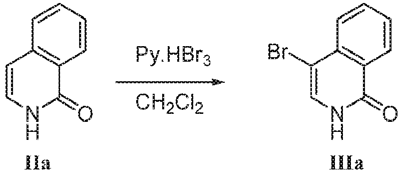

To a solution of 100.0 g (690 mmol, 1.0 eq.) of isoquinolin-1(2*H*)-one (IIa) in 2 L of methylene chloride at room temperature was added 225 g (703 mmol, 1.02 eq.) of pyridinium hydrobromide perbromide, and the mixture was stirred at room temperature for 3 h. The mixture was then filtered, and the filtrate was basified with saturated sodium bicarbonate solution. The precipitated solid was collected by filtration, washed with 4 x 500 mL of water, 2 x 400 mL of petroleum ether, and then dried under high vacuum to provide 130 g (583 mmol, 84%) of 4-bromoisoquinolin-1(2*H*)-one (**IIIa**). LCMS: *m*/*z* found 223.9/225.9 [M+H]⁺; ¹H NMR (400 MHz, CDCl₃): δ 10.52 (bs, 1H), 8.43 (dd, 1H), 7.89 (d, 1H), 7.78-7.82 (m, 1H), 7.57-7.61 (m, 1H), 7.41 (s, 1H).

### 4-Bromo-1-chloroisoquinoline

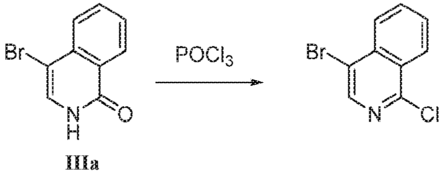

A solution of 100.0 g (447 mmol, 1.0 eq.) of 4-bromoisoquinolin-1(2*H*)-one (**IIIa**) in 250 mL of phosphorus oxychloride was heated at 100 °C for 3 h. The mixture was allowed to cool to room temperature, and the volatiles were evaporated under reduced pressure. The residue was suspended in with 1 L of ice cold water and stirred for 10 min. The precipitated solid was collected by filtration, washed with 500 mL of water and 500 mL of petroleum ether, and dried under high vacuum to provide 96.0 g (397 mmol, 88%) of 4-bromo-1-chloroisoquinoline. LCMS: *m*/*z* found 241.9/243.9; ¹H NMR (400 MHz, CDCl₃): δ 8.49 (s, 1H), 8.36 (d, 1H), 8.20 (d, 1H), 7.88 (dd, 1H), 7.77 (dd, 1H).

### 4-Bromo-1-methoxyisoquinoline (IVa)

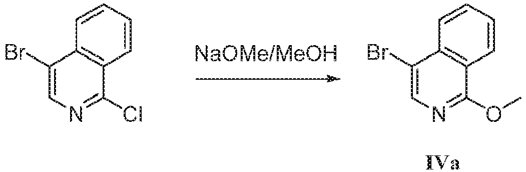

To a solution of 96.0 g (397 mmol, 1.0 eq.) of 4-bromo-1-chloroisoquinoline in 750 mL of methanol at 0 °C was added 32.1 g (1.19 mol, 4.0 eq.) of sodium methoxide portionwise over approximately 30 min, and the mixture was then heated at reflux for 16 h. The mixture was allowed to cool to room temperature and the solvent was removed *in vacuo.* The residue was suspended in 2 L of ice cold water and stirred for 10 min. The precipitated solid was collected by filtration, washed with 2 x 500 mL of water and dried under high vacuum to provide 78 g (329 mmol, 82%) of 4-bromo-1-methoxyisoquinoline (**IVa**). LCMS: *m*/*z* found 237.9/239.9 [M+H]⁺; ¹H NMR (500 MHz, CDCl₃): δ 8.24-8.26 (m, 1H), 8.18 (s, 1H), 8.06 (d, 1H), 7.76-7.95 (m, 1H), 7.59-7.62 (m, 1H), 4.11 (s, 3H).

### 1-Methoxyisoquinoline-4-carbaldehyde (Va)

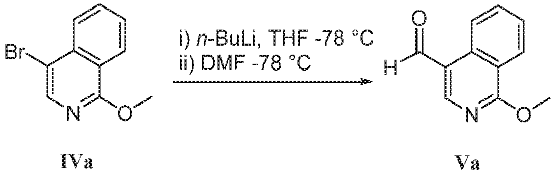

To a solution of 5.0 g (21.0 mmol, 1.0 eq.) of 4-bromo-1-methoxyisoquinoline **(IVa)** in 50 mL of anhydrous THF at -78 °C under a nitrogen atmosphere was added 26 mL (42.0 mmol, 2.0 eq.) of a 1.6 M solution of n-BuLi in hexanes. The mixture was stirred at -78 °C for 1 h, and 6.8 mL (98.5 mmol, 4.7 eq.) of anhydrous DMF was then added. The mixture was stirred at -78 °C for an additional 1.5 h and then quenched by the addition of 50 mL of saturated ammonium chloride solution. The mixture was diluted with 500 mL of ethyl acetate and the layers were separated. The organic phase was washed with 200 mL of brine, dried (Na₂SO₄), filtered and the solvent was removed *in vacuo.* The residue was purified by flash chromatography (SiO₂, eluting with a linear gradient of 0-30% ethyl acetate/hexane) to provide 2.5 g (13.4 mmol, 64%) of 1-methoxyisoquinoline-4-carbaldehyde **(Va).** LCMS: *m*/*z* found 188.2 [M+H] ⁺; ¹H NMR (400 MHz, CDCl₃): δ 10.16 (s, 1H), 9.18 (d, 1H), 8.47 (s, 1H), 8.32 (d, 1H), 7.82-7.99 (m, 1H), 7.62-7.68 (m, 1H), 4.24 (s, 3H). The above detailed reaction was performed in multiple batches on 5 gram scale with consistent results.

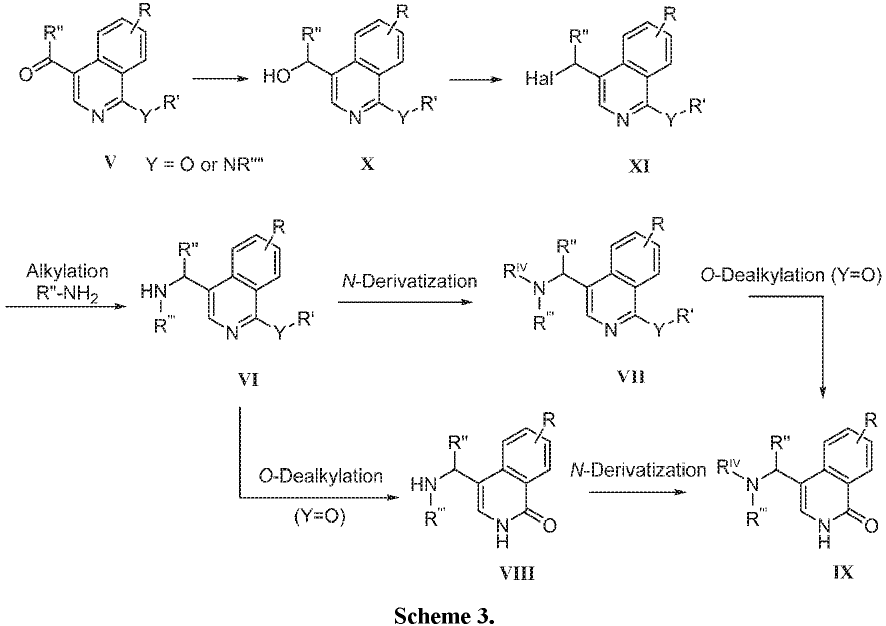

### (1-Methoxyisoquinolin-4-yl)methanol (Xa)

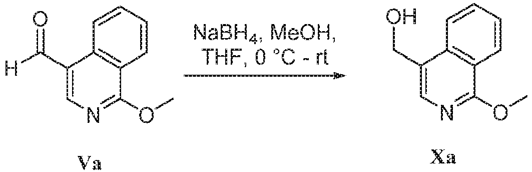

To a solution of 1.0 g (5.35 mmol, 1.0 eq.) of 1-methoxyisoquinoline-4-carbaldehyde (**Va**) in 20 mL of 1:1 (*v*/*v*) MeOH:THF at 0 °C was added 0.61 g (16.04 mmol, 3.0 eq.) of sodium borohydride. The mixture was allowed to warm to room temperature and stirred for 2 h. The solvent was removed *in vacuo,* and the residue was purified by flash chromatography (SiO₂, eluting with a linear gradient of 0-20% ethyl acetate/hexane) to provide 0.60 g (3.17 mmol, 59%) of (1-methoxyisoquinolin-4-yl) methanol (**Xa**). LCMS: *m*/*z* found 190.2 [M+H]⁺; ¹H NMR (400 MHz, CDCl₃): δ 8.29 (d, 1H), 8.08 (d, 1H), 7.97 (s, 1H), 7.72-7.76 (m, 1H), 7.55-7.59 (m, 1H), 4.99 (m, 2H), 4.11 (s, 3H).

### 4-(Chloromethyl)-1-methoxyisoquinoline (XIa)

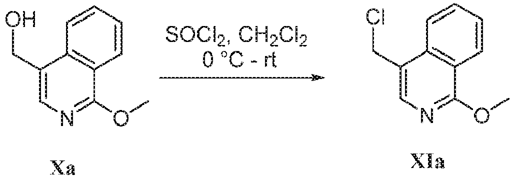

To a solution of 0.6 g (3.17 mmol, 1.0 eq.) of (1-methoxyisoquinolin-4-yl)methanol (**Xa**) in 6 mL of methylene chloride at 0 °C was added 0.4 mL (6.34 mmol, 2.0 eq.) of thionyl chloride. The mixture was allowed to warm to room temperature and stirred for 3 h. The volatiles were removed *in vacuo,* and the residue was dried under high vacuum to provide 0.7 g of 4-(chloromethyl)-1-methoxyisoquinoline (**XIa**). ¹H NMR (400 MHz, CDCl₃): δ 8.23-8.26 (d, 1H), 8.16 (s, 1H), 8.10 (d, 1H), 7.87-7.91 (m, 1H), 7.61-7.72 (m, 1H), 5.18 (s, 2H), 4.07 (s, 3H).

### N-((1-Methoxyisoquinolin-4-yl)methyl)ethanamine (VIa)

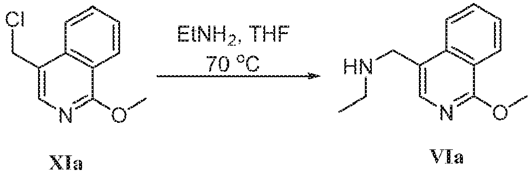

To 0.7 g (3.4 mmol, 1.0 eq.) of 4-(chloromethyl)-1-methoxyisoquinoline (**XIa**) in a pressure vessel was added 14 mL of a 2 M solution of ethyl amine in THF. The vessel was sealed and heated at 70 °C for 16 h. The mixture was allowed to cool to room temperature, diluted with 20 mL of water and extracted with 3 x 30 mL of ethyl acetate. The combined organic extracts were dried (Na₂SO₄), filtered and the solvent was removed *in vacuo* to provide 0.42 g (1.9 mmol, 60% from **Xa**) of *N*-((1-methoxy isoquinolin-4-yl)methyl)ethanamine (**VIa**). LCMS: *m*/*z* found 217.3 [M+H] ⁺; ¹H NMR (400 MHz, CDCl₃): δ 8.26 (d, 1H), 8.15 (s, 1H), 8.12 (m, 1H), 7.82 (dd, 1H), 7.59 (dd, 1H), 4.36 (s, 2H), 4.09 (s, 3H), 2.88 (q, 2H), 1.37 (t, 3H).

### 3-(3-Chloro-4-fluorophenyl)-1-ethyl-1-((1-methoxyisoquinolin-4-yl)methyl)urea (Compound 15)

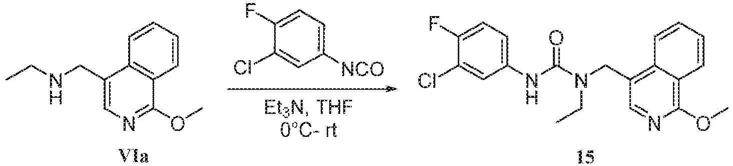

To a solution of 0.3 g (1.38 mmol, 1.0 eq.) of *N-*((1-methoxyisoquinolin-4-yl)methyl) ethanamine (**VIa**) in 10 mL of THF at 0 °C was added 0.38 mL (2.77 mmol, 2.0 eq.) of trimethylamine, followed by 0.24 g (1.38 mmol, 1.0 eq.) of 4-fluoro-3-chlorophenylisocyanate. The mixture was allowed to warm to room temperature and stirred for 6 h. The solvent was removed *in vacuo* and the residue was redissolved in 50 mL of methylene chloride. The organic solution was washed with 20 mL of brine, dried (Na₂SO₄), filtered and the solvent was removed *in vacuo.* The residue was purified by semi-preparative-HPLC to provide 90 mg (0.23 mmol) of 3-(3-chloro-4-fluorophenyl)-1-ethyl-1-((1-methoxyisoquinolin-4-yl)methyl)urea (**Compound 15**). LCMS: *m*/*z* found 388.2/390.2 [M+H]⁺, RT = 5.09 min (Method A); ¹H NMR (400 MHz, DMSO-d₆): δ 8.57 (s, 1H), 8.32 (d, 1H), 8.08 (d, 1H), 7.73 (s, 1H), 7.79-7.83 (m, 2H), 7.63-7.67 (dd, 1H), 7.47-7.51 (m, 1H), 7.28-7.33 (dd, 1H), 4.88 (s, 2H), 4.06 (s, 3H), 3.22-3.27 (q, 2H), 0.99-1.03 (t, 3H).

### 4-((Ethylamino)methyl)isoquinolin-1(2H)-one (VIIIa)

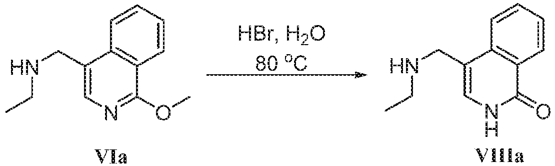

A solution of 0.42 g (1.9 mmol, 1.0 eq.) of *N*-((1-methoxyisoquinolin-4-yl)methyl)ethanamine (**VIa**) in 4.2 mL of 47% aqueous hydrobromic acid was heated at 80 °C for 6 h. The mixture was allowed to cool to room temperature and the solvent was removed *in vacuo* to provide crude 4-((ethylamino)methyl)isoquinolin-l(2*H*)-one (**VIIIa)**, which was taken forward to next step without further purification. LCMS: *m*/*z* found 203.1 [M+H]⁺.

### 3-(3-Chloro-4-fluorophenyl)-1-ethyl-1-((1-oxo-1,2-dihydroisoquinolin-4-yl)methyl)urea (Compound 9)

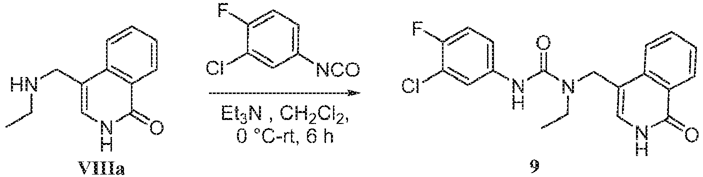

To a solution of crude **VIIIa** as detailed elsewhere herein in 20 mL of methylene chloride at 0 °C was added 1.17 mL (8.43 mmol) of trimethylamine, followed by 0.18 g (1.05 mmol) of 4-fluoro-3-chloro-phenylisocyanate. The mixture was allowed to warm to room temperature and stirred for 6 h. The solvent was removed *in vacuo* and the residue was redissolved in 50 mL of methylene chloride. The organic solution was washed with 20 mL of brine, dried (Na₂SO₄), filtered and the solvent was removed *in vacuo.* The residue was purified by semi-preparative-HPLC to provide 75 mg (0.20 mmol, 20% from **VIa**) of 3-(3-chloro-4-fluorophenyl)-1-ethyl-1-((1-oxo-1,2-dihydroisoquinolin-4-yl)methyl)urea (**Compound 9**). LCMS: *m*/*z* found 374.3/376.3 [M+H]⁺, RT = 3.91 min (Method A); ¹H NMR (400 MHz, DMSO-d₆): δ 11.22 (d, 1H), 8.54 (s, 1H), 8.23 (d, 1H), 7.08-7.82 (m, 2H), 7.71-7.75 (m, 1H), 7.47-7.53 (m, 2H), 7.28-7.32 (dd, 1H), 7.13 (d, 1H), 4.63 (s, 2H), 3.30 (t, 2H), 0.99-1.02 (t, 3H).

### (1-Methoxyisoquinolin-4-yl)methanamine (VIh)

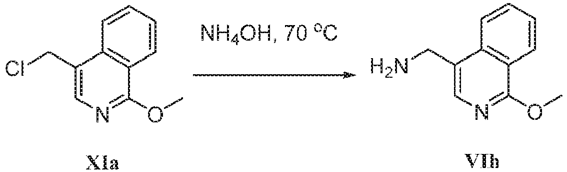

A solution of 0.38 g (1.8 mmol, 1.0 eq.) of 4-(chloromethyl)-1-methoxyisoquinoline (**XIa**) in 10 mL of 35% ammonia was heated at 70 °C for 16 h. The mixture was allowed to cool to room temperature and the volatiles were removed *in vacuo* to provide 0.35 g of crude (1-methoxyisoquinolin-4-yl)methanamine (**VIh**) which was used directly in the next synthetic step.

### 1-(3-Chloro-4-fluorophenyl)-3-((l-methoxyisoquinolin-4-yl)methyl)urea (Compound 6)

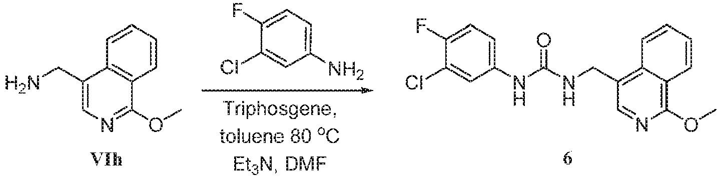

To a solution of 0.33 g (2.23 mmol, 1.2 eq.) of 3-chloro-4-fluoroaniline in 10 mL of toluene was added 0.45 g (1.53 mmol, 0.8 eq.) of triphosgene and the mixture was heated at 80 °C for 4 h. The mixture was allowed to cool to room temperature and then added to a precooled 0 °C solution of 0.35 g of crude (1-methoxyisoquinolin-4-yl)methanamine **(VIh)** and 0.78 mL (5.58 mmol, 3.0 eq.) of triethylamine in 10 mL of DMF. The resulting mixture was allowed to warm to room temperature and stirred for 6 h. The reaction was quenched with 20 mL of water and extracted with 3 x 30 mL of ethyl acetate. The combined organic extracts were washed with 10 mL of saturated sodium bicarbonate solution, dried (Na₂SO₄), filtered and the solvent was removed *in vacuo.* The residue was purified by semi-preparative HPLC to provide 1-(3-chloro-4-fluorophenyl)-3-((1-methoxyisoquinolin-4-yl)methyl)urea (**Compound 6**). LCMS: *m*/*z* found 360.0/362.0 [M+H]⁺; ¹H NMR (400 MHz, DMSO-d₆): δ 1H NMR: 9.00 (bs, 1H), 8.22 (d, 1H), 8.09 (d, 1H), 7.98 (s, 1H), 7.81-7.85 (m, 1H), 7.78 (dd, 1H), 7.63-7.67 (m, 1H), 7.22-7.27 (m, 2H), 7.02 (bs, 1H), 4.59 (d, 2H), 4.05 (s, 3H).

### 1-(3-Chloro-4-fluorophenyl)-3-((1-oxo-1,2-dihydroisoquinolin-4-yl)methyl)urea (Compound 8)

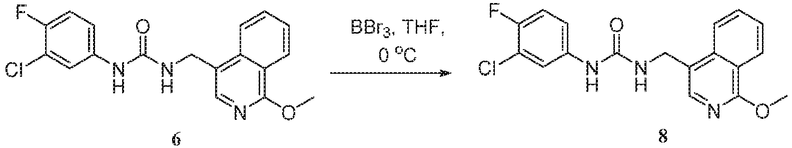

To a solution of 0.06 g (0.17 mmol, 1.0 eq.) of 1-(3-chloro-4-fluorophenyl)-3-((1-methoxyisoquinolin-4-yl)methyl)urea **(Compound 6**) in 5 mL of anhydrous THF at 0 °C was added 0.10 g (0.42 mmol, 2.5 eq.) of boron tribromide. The mixture was allowed to warm to room temperature and then heated at 70 °C for 12 h. The mixture was then allowed to cool to room temperature and quenched by the addition of 5 mL of ice water. The mixture was extracted with 3 x 20 mL of ethyl acetate and the combined organic extracts were dried (Na₂SO₄), filtered and the solvent was removed *in vacuo.* The residue was purified by flash chromatography (SiO₂, eluting with a linear gradient of 0-10% methanol in methylene chloride) to provide 0.04 g (0.12 mmol, 70%) of 1-(3-chloro-4-fluorophenyl)-3-((1-oxo-1,2-dihydroisoquinolin-4-yl)methyl)urea (**Compound 8**). LCMS: *m*/*z* found 346.0/348.0 [M+H]⁺; ¹H NMR (400 MHz, DMSO-d₆): δ ¹H NMR: 11.23 (d, 1H), 8.92 (s, 1H), 8.23 (dd, 1H), 7.74-7.81(m, 3H), 7.50-7.53 (m, 1H), 7.22-7.28 (m, 2H), 7.15 (d, 1H), 6.69 (s, 1H), 4.36 (d, 2H).

### 1-(4-Fluoro-3-methylphenyl)-3-((1-methoxyisoquinolin-4-yl)methyl)urea (Compound 7)

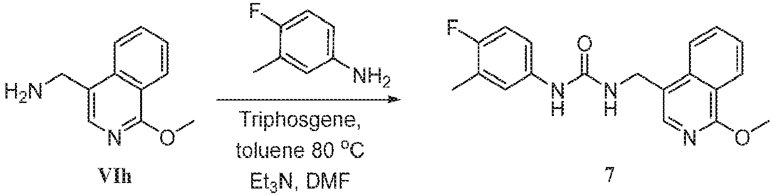

1-(4-Fluoro-3-methylphenyl)-3-((1-methoxyisoquinolin-4-yl)methyl)urea (**Compound 7**) was synthesized in a similar manner as described above from 3-methyl-4-fluoroaniline and (1-methoxyisoquinolin-4-yl)methanamine (**VIh**). LCMS: *m*/*z* found 340.1 [M+H]⁺; ¹H NMR (400 MHz, DMSO-d₆) δ ¹H NMR 8.55 (s, 1H), 8.22 (d, 1H), 8.08 (d, 1H), 7.98 (s, 1H), 7.81-7.85 (m, 1H), 7.63-7.67 (m, 1H), 7.29 (dd, 1H), 7.17-7.21 (m, 1H), 6.97 (t, 1H), 6.73 (d, 1H), 4.59 (d, 2H), 4.05 (s, 3H), 2.17 (d, 3H).

### 1-(4-Fluoro-3-methylphenyl)-3-((1-oxo-1,2-dihydroisoquinolin-4-yl)methyl)urea (Compound 4)

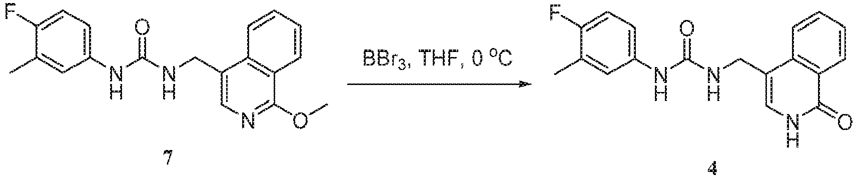

1-(4-Fluoro-3-methylphenyl)-3-((1-oxo-1,2-dihydroisoquinolin-4-yl)methyl)urea (**Compound 4**) was synthesized in a similar manner as described above from 1-(4-fluoro-3-methylphenyl)-3-((1-methoxyisoquinolin-4-yl)methyl)urea (**Compound 7**) and boron tribromide. LCMS: *m*/*z* found 326.4 [M+H]⁺; ¹H NMR (400 MHz, DMSO-d₆) δ ¹H NMR 11.21 (s, 1H), 8.38 (s, 1H), 8.23 (dd, 1H), 7.74-7.81 (m, 2H), 7.50-7.53 (m, 1H), 7.27-7.29 (m, 1H), 7.13-7.20 (m, 2H), 6.97 (t, 1H), 6.43 (t, 1H), 4.36 (d, 2H), 2.17 (d, 3H).

### 1-(1-Methoxyisoquinolin-4-yl)-N-methylmethanamine (VIi)

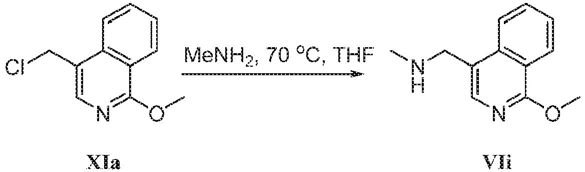

To a solution of 0.50 g (2.4 mmol, 1.0 eq.) of 4-(chloromethyl)-1-methoxyisoquinoline (**XIa**) in 10 mL of THF in a sealed tube was added 4.8 mL (9.6 mmol, 4.0 eq.) of a 2 M solution of methyl amine in THF and the mixture was heated at 70 °C for 16 h. The mixture was allowed to cool to room temperature and the volatiles were removed *in vacuo.* The resulting solid was washed with 10 mL of diethyl ether and dried under high vacuum to provide 0.30 g (1.5 mmol, 63%) of 1-(1-methoxyisoquinolin-4-yl)-*N-*methylmethanamine (**VIi**). ¹H NMR (400 MHz, DMSO-d₆) δ 8.28 (d, 1H), 8.07 (d, 2H), 7.78 (d, 1H), 7.58 (t, 1H), 4.29 (d, 2H), 4.10 (s, 3H), 2.53 (s, 3H).

### 3-(4-Fluoro-3-methylphenyl)-1-((1-methoxyisoquinolin-4-yl)methyl)-1-methylurea (Compound 3)

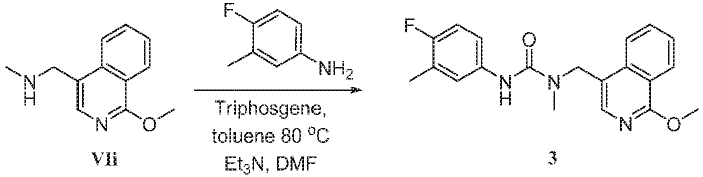

3-(4-Fluoro-3-methylphenyl)-1-((l-methoxyisoquinolin-4-yl)methyl)-1-methylurea (**Compound 3**) was synthesized in a similar manner as described above from 3-methyl-4-fluoroaniline and 1-(1-methoxyisoquinolin-4-yl)-*N*-methylmethanamine (**VIi**). LCMS: *m*/*z* found 354.3 [M+H]⁺; ¹H NMR (400 MHz, DMSO-d₆) δ 8.34 (s, 1H), 8.22 (dd, 1H), 8.11 (d, 1H), 7.93 (s, 1H), 7.79-7.82 (m, 1H), 7.63-7.66 (m, 1H), 7.40 (dd, 1H), 7.30-7.33 (m, 1H), 7.01 (t, 1H), 4.85 (s, 2H), 4.05 (s, 3H), 2.85 (s, 3H), 2.19 (d, 3H).

### 3-(3-Chloro-4-fluorophenyl)-1-((1-methoxyisoquinolin-4-yl)methyl)-1-methylurea (Compound 2)

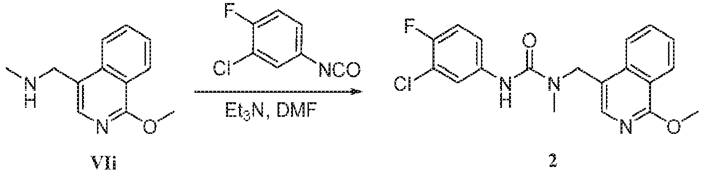

To a solution of 0.05 g (0.24 mmol, 1.0 eq.) of 1-(1-methoxyisoquinolin-4-yl)-N-methylmethanamine (**VIi**) in 5 mL of DMF was added 0.1 mL (0.74 mmol, 3.0 eq.) of triethylamine followed by 0.05 g (0.29 mmol, 1.2 eq.) of 2-chloro-1-fluoro-4-isocyanatobenzene and the mixture was stirred at room temperature for 16 h. The mixture was then diluted with 10 mL of water and extracted with 3 x 15 mL of ethyl acetate. The combined organic extracts were washed with 10 mL of saturated sodium bicarbonate solution, dried (Na₂SO₄), filtered and the solvent was removed *in vacuo.* The residue was purified by semi-preparative HPLC to provide 0.04 g (0.11 mmol, 46%) of 3-(3-chloro-4-fluorophenyl)-1-((1-methoxyisoquinolin-4-yl)methyl)-1-methylurea (**Compound 2**). LCMS: *m*/z found 374.2/376.2 [M+H]⁺; ¹H NMR (400 MHz, DMSO-d₆) δ 8.95 (s, 1H), 8.23 (d, 1H), 8.09 (d, 1H), 7.94 (s, 1H), 7.79-7.83 (m, 2H), 7.63-7.69 (m, 1H), 7.46-7.49 (s, 1H), 7.31 (t, 1H), 4.86 (s, 2H), 4.06 (s, 3H), 2.87 (s, 3H).

### 3-(3-chloro-4-fluorophenyl)-1-((1-ethoxyisoquinolin-4-yl)methyl)-1-ethylurea (Compound 17)

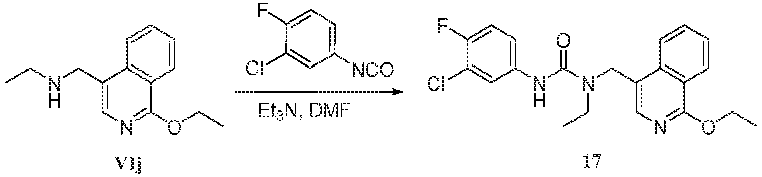

3-(3-Chloro-4-fluorophenyl)-1-((l-ethoxyisoquinolin-4-yl)methyl)-1-methylurea (**Compound 17**) was synthesized in a similar manner as described above fromN-((1-ethoxyisoquinolin-4-yl)methyl)ethanamine (**VIj**) and 2-chloro-1-fluoro-4-isocyanatobenzene. LCMS: *m*/*z* found 402.2/404.2 [M+H]⁺, RT = 5.40 min (Method A); ¹H NMR (400 MHz, DMSO-d₆) δ 8.56 (s, 1H), 8.23-8.25 (d, 1H), 8.05-8.08 (d, 1H), 7.90 (s, 1H), 7.78-7.83 (m, 2H), 7.64-7.68 (t, 1H), 7.46-7.50 (m, 1H), 7.28-7.33 (t, 1H), 4.87 (s, 2H), 4.49-4.54 (q, 2H), 3.27-3.32 (q, 2H), 1.42-1.45 (t, 3H), 0.968-1.02 (t, 3H)

### 4-((Methylamino)methyl)isoquinolin-1(2H)-one (VIIIaf)

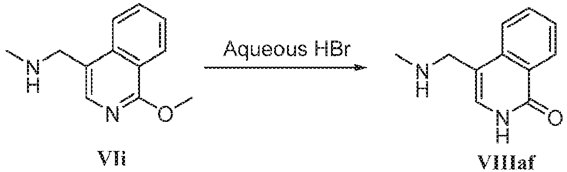

A solution of 0.45 g (2.2 mmol, 1.0 eq.) of 1-(1-methoxyisoquinolin-4-yl)-*N-*methylmethanamine (**VIi**) in 5 mL of 47% aqueous HBr was heated at 80 °C for 6 h. The mixture was allowed to cool to room temperature and the solvent was removed *in vacuo.* The residue was triturated with diethyl ether and dried under high vacuum to provide 0.35 g (1.9 mmol, 84%) of 4-((methylamino)methyl)isoquinolin-1(2*H*)-one (**VIIIaf**). ¹H NMR (400 MHz, DMSO-d₆) δ 11.55 (s, 1H), 8.63 (s, 1H), 8.26 (dd, 1H), 7.95 (d, 1H), 7.80-7.84 (m, 1H), 7.48 (d, 1H), 4.30 (t, 2H), 2.61 (d, 3H).

### 3-(4-Fluoro-3-methylphenyl)-1-methyl-1-((1-oxo-1,2-dihydroisoquinolin-4-yl)methyl)urea (Compound 5)

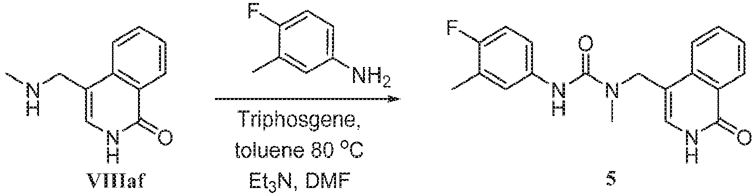

3-(4-Fluoro-3-methylphenyl)-1-methyl-1-((1-oxo-1,2-dihydroisoquinolin-4-yl)methyl)urea (**Compound 5**) was synthesized in a similar manner as described above from 3-methyl-4-fluoroaniline and 4-((methylamino)methyl)isoquinolin-1(2*H*)-one (**VIIIaf**). LCMS: *m*/*z* found 340.3 [M+H]⁺; ¹H NMR (400 MHz, DMSO-d₆) δ 11.27 (d, 1H), 8.32 (s, 1H), 8.23 (dd, 1H), 7.84 (d, 1H), 7.71-7.74 (m, 1H), 7.50 (dd, 1H), 7.39 (dd, 1H), 7.29-7.32 (m, 1H), 7.13 (d, 1H), 7.01 (t, 1H), 4.60 (s, 2H), 2.84 (s, 3H), 2.19 (d, 3H).

### 3-(3-Chloro-4-fluorophenyl)-1-methyl-1-((1-oxo-1,2-dihydroisoquinolin-4-yl)methyl)urea (Compound 1)

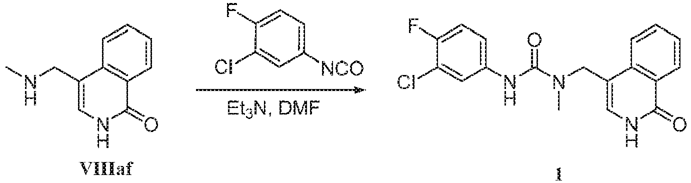

3-(3-Chloro-4-fluorophenyl)-1-methyl-1-((1-oxo-1,2-dihydroisoquinolin-4-yl)methyl)urea (**Compound 1**) was synthesized in a similar manner as described above from 4-((methylamino)methyl)isoquinolin-1(21*H*)-one (**VIIIaf**) and 2-chloro-1-fluoro-4-isocyanatobenzene. LCMS: *m*/*z* found 360.0/362.0 [M+H]⁺; ¹H NMR (400 MHz, DMSO-d₆) δ 11.29 (1H, d), 8.56 (s, 1H), 8.23 (dd, 1H), 7.80-7.83 (m, 2H), 7.71-7.75 (m, 1H), 7.45-7.52 (m, 2H), 7.30 (t, 1H), 7.15 (d, 1H), 4.61 (s, 2H), 2.85 (s, 3H).

### 1-Chloroisoquinoline-4-carbaldehyde

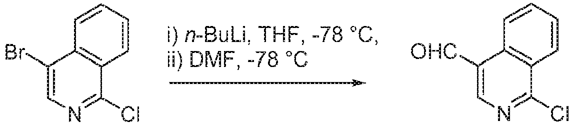

To a solution of 3.0 g (12.5 mmol, 1.0 eq.) of 4-bromo-l-chloroisoquinoline in 30 mL of anhydrous THF at -78 °C under a nitrogen atmosphere was added 15.5 mL (24.8 mmol, 2.0 eq.) of a 1.6 M solution of n-butyl lithium in hexanes. The mixture was stirred at -78 °C for 30 min and 3.62 g (49.6 mmol, 4.0 eq.) of anhydrous DMF was added. Stirring at -78°C was continued for 1 h and the reaction was quenched with 50 mL of saturated ammonium chloride solution. The resulting solution was extracted with 3 x 100 mL of ethyl acetate and the combined organic extracts were washed with 100 mL of brine, dried (Na₂SO₄), filtered the solvent was removed *in vacuo.* The residue was purified by flash chromatography (SiO₂, eluting with a linear gradient of 0-18% ethyl acetate/petroleum ether) to provide 1.3 g (6.80 mmol, 55%) of 1-chloroisoquinoline-4-carbaldehyde. LCMS: *m*/*z* found 191.9/193.9 [M+H]⁺; ¹H NMR (400 MHz, CDCl₃) δ 10.36 (s, 1H), 9.25-9.27 (m, 1H), 8.71 (s, 1H), 8.47-8.49 (m, 1H), 7.95-7.99 (m, 1H), 7.79-7.84 (m, 1H).

### (1-Chloroisoquinolin-4-yl)methanol

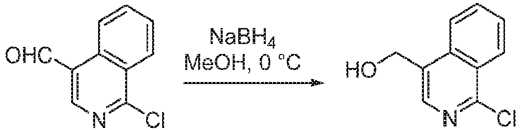

To a solution of 1.3 g (6.80 mmol, 1.0 eq.) of 1-chloroisoquinoline-4-carbaldehyde in 13 mL of methanol at 0 °C was added 0.26 g (6.80 mmol, 1.0 eq.) of sodium borohydride. The mixture was allowed to warm to room temperature and stirred for1 h. The solvent was removed *in vacuo* and the residue was resuspended in 50 mL of water and extracted with 3 x 50 mL of ethyl acetate. The combined organic extracts were dried (Na₂SO₄), filtered and the solvent was removed *in vacuo* to provide 1.3 g (6.71 mmol, 98%) of (1-chloroisoquinolin-4-yl)methanol. LCMS: *m*/*z* found 194.2/196.2 [M+H]⁺; ¹H NMR (400 MHz, CDCl₃) δ 8.39 (d, 1H), 8.27 (s, 1H), 8.17 (d, 1H), 7.83-7.85 (m, 1H), 7.70-7.71 (m, 1H), 5.09 (s, 2H), 1.96 (bs, 1H).

### 1-Chloro-4-(chloromethyl)isoquinoline

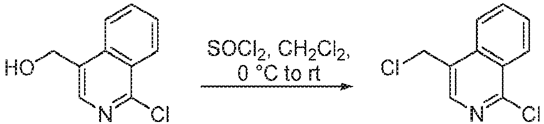

To a solution of 1.3 g (6.73 mmol, 1.0 eq.) of (1-chloroisoquinolin-4-yl)methanol in 13 mL of methylene chloride at 0 °C was added 4.1 g (33.67 mmol, 5.0 eq.) of thionyl chloride. The mixture was allowed to warm to room temperature and stirred for 2 h. The reaction was quenched by the slow addition of 20 mL of water and extracted with 3 x 30 mL of methylene chloride. The combined organic extracts were dried (Na₂SO₄), filtered and the solvent was removed *in vacuo* to provide 1.3 g (6.13 mmol, 91%) of 1-chloro-4-(chloromethyl)isoquinoline. LCMS: *m*/*z* found 212.3/214.3/216.3 [M+H]⁺; ¹H NMR (400 MHz, CDCl₃) δ 8.41 (d, 1H), 8.31 (s, 1H), 8.15 (d, 1H), 7.86-7.90 (m, 1H), 7.73-7.77 (m, 1H), 4.96 (s, 2H).

### 1-(1-Chloroisoquinolin-4-yl)-N methylmethanamine

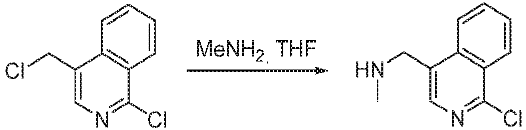

To solution of 1.1 g (5.19 mmol, 1.0 eq.) of 1-chloro-4-(chloromethyl)isoquinoline in 11 mL of THF in a sealed tube, was added 7.8 mL (15.6 mmol, 3.0 eq.) of a 2 M solution of methylamine in THF. The vessel was sealed, and the mixture was stirred at room temperature for 16 h. The volatiles were removed *in* vacuo and the residue was purified by flash chromatography (SiO₂, eluting with a linear gradient of 0-10% methanol in methylene chloride) to provide 0.9 g (4.35 mmol, 84%) of 1-(1-chloroisoquinolin-4-yl)-*N-*methylmethanamine. LCMS: *m*/*z* found 207.2/209.2 [M+H]⁺; ¹H NMR (400 MHz, CDCl₃) δ 8.37 (d, 1H), 8.22 (s, 1H), 8.18 (d, 1H), 7.78-7.82 (m, 1H), 7.67-7.71 (m, 1H), 4.12 (s, 2H), 2.54 (s, 3H), 1.69 (bs, 1H).

### 3-(3-Chloro-4-fluorophenyl)-1-((1-chloroisoquinolin-4-yl)methyl)-1-methylurea

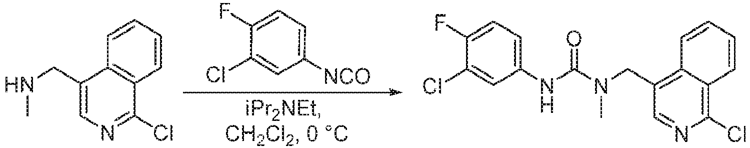

To a solution of 0.8 g (3.88 mmol, 1.0 eq.) of 1-(1-chloroisoquinolin-4-yl)-*N-*methylmethanamine in 8 mL of methylene chloride at 0 °C was added 1.51 g (11.65 mmol, 3.0 eq.) of *N,N-*diisopropylethylamine followed by 0.66 g (3.88 mmol, 1.0 eq.) of 4-fluoro-3-chloro-phenylisocyanate and the mixture was stirred for at 0 °C for 1 h. The mixture was then diluted with 30 mL of water and extracted with 3 x 40 mL of ethyl acetate. The combined organic extracts were washed with 30 mL of brine, dried (Na₂SO₄), filtered and the solvent was removed *in vacuo* to provide 0.6 g (1.58 mmol, 41%) of 3-(3-chloro-4-fluorophenyl)-1-((1-chloroisoquinolin-4-yl)methyl)-1-methylurea. LCMS: *m*/*z* found 378.4/380.4 [M+H]⁺; ¹H NMR (400 MHz, CDCl₃) δ 8.42 (d, 1H), 8.22 (d, 1H), 8.18 (s, 1H), 7.83 (t, 1H), 7.73 (t, 1H), 7.58-7.61 (m, 1H), 7.19-7.23 (m, 1H), 7.07 (t, 1H), 6.39 (s, 1H), 5.01 (s, 2H), 2.92 (s, 3H).

### 3-(3-Chloro-4-fluorophenyl)-1-methyl-1-((1-((1-methyl-1H 1,2,4-triazol-3-yl)methoxy)isoquinolin-4-yl)methyl)urea (Compound 87)

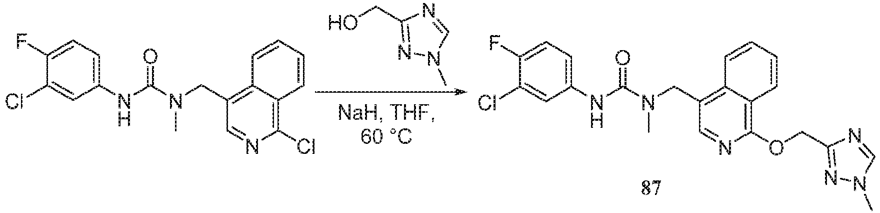

To a solution of 0.3 g (0.79 mmol, 1.0 eq.) of 3-(3-chloro-4-fluorophenyl)-1-((1-chloroisoquinolin-4-yl)methyl)-1-methylurea in 5 mL of anhydrous THF under a nitrogen atmosphere was added 64 mg (1.59 mmol, 2.0 eq.) of a 60% dispersion of sodium hydride in mineral oil followed by 180 mg (1.59 mol, 2.0 eq.) of (1-methyl-1*H*-1,2,4-triazol-3-yl)methanol. The mixture was heated at 60 °C for 8 h, quenched with 25 mL of ice-cold water and extracted with 3 x 40 mL of ethyl acetate. The combined organic extracts were washed with 20 mL of brine, dried (Na₂SO₄), filtered and the solvent was removed *in vacuo.* The residue was purified by flash chromatography (SiO₂, eluting with a linear gradient of 0-10% methanol in methylene chloride) to provide 0.14 g (0.30 mmol, 38%) of 3-(3-chloro-4-fluorophenyl)-1-methyl-1-((1-((1-methyl-1*H*-1,2,4-triazol-3-yl)methoxy)isoquinolin-4-yl)methyl)urea (**Compound 87**). LCMS: *m*/*z* found 455.1/457.2 [M+H]⁺, RT = 5.31 min (Method A); ¹H NMR (400 MHz, DMSO-d₆) δ 8.60 (s, 1H), 8.47 (s, 1H), 8.18 (d, 1H), 8.11 (d, 1H), 7.94 (s, 1H), 7.80-7.85 (m, 2H), 7.65 (t, 1H), 7.46-7.51 (m, 1H), 7.31 (t, 1H), 5.53 (s, 2H), 4.87 (s, 2H), 3.87 (s, 3H), 2.89 (s, 3H).

### 3-(3-Chloro-4-fluorophenyl)-1-methyl-1-((1-(pyridin-2-ylmethoxy)isoquinolin-4-yl)methyl)urea (Compound 88)

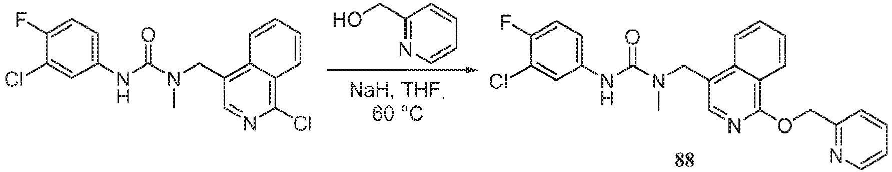

3-(3-Chloro-4-fluorophenyl)-1-methyl-1-((1-(pyridin-2-ylmethoxy)isoquinolin-4-yl)methyl)urea (**Compound 88**) was synthesized in a similar manner as described above from 3-(3-chloro-4-fluorophenyl)-1-((1-chloroisoquinolin-4-yl)methyl)-1-methylurea and pyridin-2-ylmethanol. LCMS: *m*/*z* found 451.1/453.2 [M+H]⁺, RT = 5.00 min (Method A); ¹H NMR (400 MHz, DMSO-d₆) δ 8.58-8.61 (m, 2H), 8.36 (d, 1H), 8.13 (d, 1H), 7.93 (s, 1H), 7.80-7.87 (m, 3H), 7.69 (t, 1H), 7.58 (d, 1H), 7.46-7.50 (m, 1H), 7.28-7.37 (m, 2H), 5.65 (s, 2H), 4.87 (s, 2H), 2.88 (s, 3H).

### 1-((1-(2-((tert-Butyldimethylsilyl)oxy)ethoxy)isoquinolin-4-yl)methyl)-3-(3-chloro-4-fluorophenyl)-1-ethylurea (VIIb)

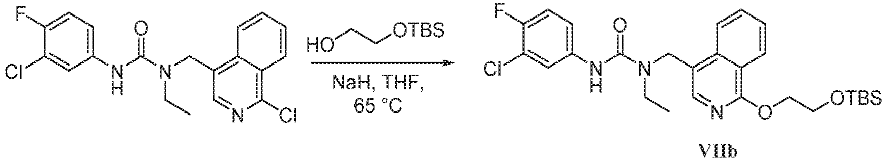

1-((1-(2-((*tert*-Butyldimethylsilyl)oxy)ethoxy)isoquinolin-4-yl)methyl)-3-(3-chloro-4-fluorophenyl)-1-ethylurea (**VIIb**) was synthesized in a similar manner as described above from 3-(3-chloro-4-fluorophenyl)-1-((1-chloroisoquinolin-4-yl)methyl)-1-ethylurea and 2-((*tert*-butyldimethylsilyl)oxy)ethan-1-ol. LCMS: *m*/*z* found 532.5/534.5 [M+H]⁺.

### 3-(3-Chloro-4-fluorophenyl)-1-ethyl-1-((l-(2-hydroxyethoxy)isoquinolin-4-yl)methyl)urea (Compound 29)

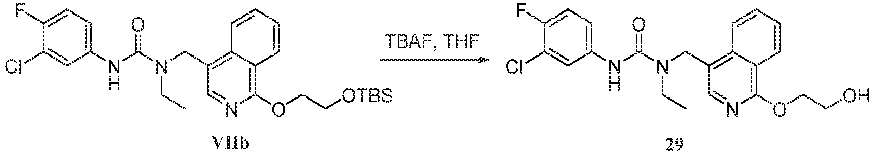

To a solution of 250 mg of 1-((1-(2-((tert-butyldimethylsilyl)oxy)ethoxy)isoquinolin-4-yl)methyl)-3-(3-chloro-4-fluorophenyl)-1-ethylurea **(VIIb)** in 4 mL of THF was added 2.5 mL (2.5 mmol) of a 1 M solution of tetra-*n*-butylammonium fluoride in THF and stirred the mixture was stirred at room temperature for 16 h. The solvent was removed *in vacuo* and the residue was purified by reverse phase chromatography (C18, eluting with linear gradient of 0-50% [0.1% formic acid in water]/acetonitrile) to provide 45 mg of 3-(3-chloro-4-fluorophenyl)-1-ethyl-1-((1-(2-hydroxy ethoxy)isoquinolin-4-yl)methyl)urea (**Compound 29**). LCMS: *m*/*z* found 418.2/420.2 [M+H]⁺; ¹H NMR (400 MHz, DMSO-d₆) δ 8.60 (s, 1H), 8.31 (d, 1H), 8.07 (d, 1H), 7.91 (s, 1H), 7.79-7.84 (m, 2H), 7.65 (t, 1H), 7.49-7.53 (m, 1H), 7.31 (t, 1H), 4.95 (t, 1H), 4.88 (s, 2H), 4.47 (t, 2H), 3.83 (q, 2H), 3.29 (q, 2H), 1.01 (t, 3H).

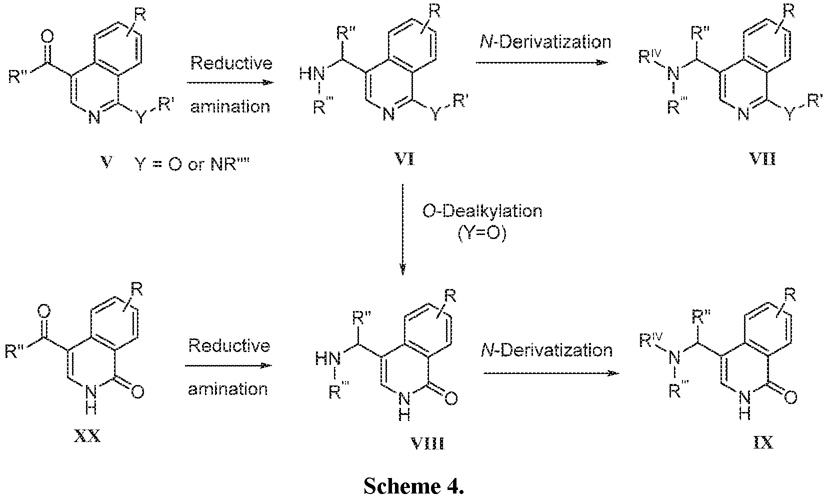

### 1-Cyclopropyl-N-((1-methoxyisoquinolin-4-yl)methyl)methanamine (VIb)

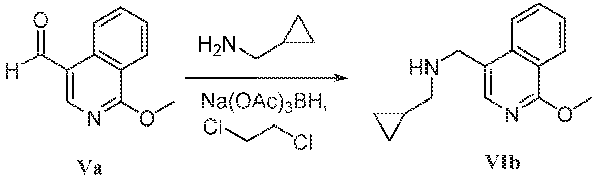

To a solution of 0.10 g (0.52 mmol, 1.0 eq.) of 1-methoxyisoquinoline-4-carbaldehyde (**Va**) in 3 mL of 1,2-dichloroethane was added 0.11 g (1.55 mmol, 3.0 eq.) of cyclopropylmethylamine. The mixture was stirred at room temperature for 10 min, and 0.33 g (1.55 mmol, 3.0 eq.) of solid sodium triacetoxyborohydride was added. The mixture was then stirred vigorously at room temperature for 18 h. The mixture was then diluted with 5 mL of methylene chloride and 10 mL of saturated sodium carbonate solution and stirred vigorously for a further 15 mins. The layers were separated, and the aqueous phase was extracted with 5 mL of methylene chloride. The combined organic extracts were dried (Na₂SO₄), filtered and the solvent was removed *in vacuo,* to provide 0.11 g of crude 1-cyclopropyl-*N*-((1-methoxyisoquinolin-4-yl)methyl)methanamine (**VIb**) which was used without further purification. LCMS: *m*/*z* found 243.2 [M+H]⁺.

### 3-(3-Chloro-4-fluorophenyl)-1-(cyclopropylmethyl)-1-((1-methoxyisoquinolin-4-yl)methyl)urea (Compound 31)

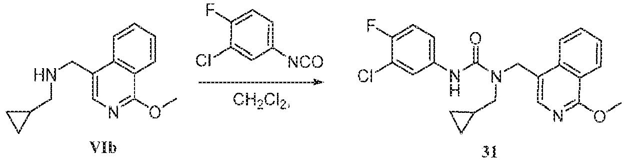

To a solution of 0.11 g of crude N-(cyclopropylmethyl)-1-(1-methoxy-4-isoquinolyl) methanamine in 1.5 mL of methylene chloride was added 0.08 g (0.5 mmol) of 4-fluoro-3-chlorophenylisocyanate, and the mixture was stirred at room temperature for 2 h. The solvent was removed *in vacuo* and the residue was purified by flash chromatography (eluting with a linear gradient of 10-35% ethyl acetate/hexanes over 10 mins) to provide 0.15 g (0.37 mmol, 71% from **Va**) of 3-(3-chloro-4-fluorophenyl)-1-(cyclopropylmethyl)-1-((1-methoxyisoquinolin-4-yl)methyl)urea (**Compound 31**). LCMS: *m*/*z* found 414.1/416.1 [M+H]⁺, RT = 6.33 min (Method A); ¹H NMR (400 MHz, DMSO-d₆) δ 8.63 (s, 1H), 8.11-8.37 (m, 1H), 7.94-8.07 (m, 1H), 7.72-7.91 (m, 3H), 7.63 (m, 1H), 7.46 (m, 1H), 7.29 (t, 1H), 4.96 (s, 2H), 4.03 (d, 3H), 3.18 (d, 2H), 1.02 (td, 1H), 0.27-0.42 (m, 2H), 0.07-0.23 (m, 2H).

### 4-(((Cyclopropylmethyl)amino)methyl)isoquinolin-1(2H)-one hydrochloride (VIIIam)

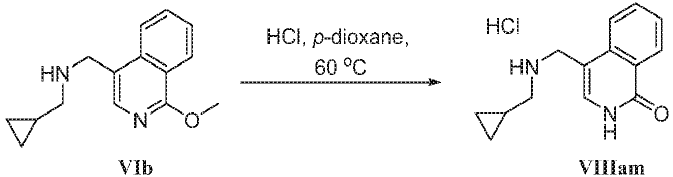

A solution of 0.25 g (1.03 mmol, 1.0 eq.) of 1-cyclopropyl-*N*-((1-methoxyisoquinolin-4-yl)methyl)methanamine (**VIb**) in 3 mL of a 4 M solution of HCl in *p*-dioxane was heated at 60 °C for 2 h. The mixture was allowed to cool to room temperature and the solvent was removed *in vacuo.* The residue was triturated with 10 mL of n-pentane and dried under high vacuum to provide 0.25 g (0.94 mmol, 91%) of 4-(((cyclopropylmethyl)amino)methyl)isoquinolin-l(2H)-one hydrochloride (**VIIIam**). LCMS: *m*/*z* found 229.3 [M+H]⁺.

### 3-(3-Chloro-4-fluorophenyl)-1-(cyclopropylmethyl)-1-((1-oxo-1,2-dihydroisoquinolin-4-yl)methyl)urea (Compound 53)

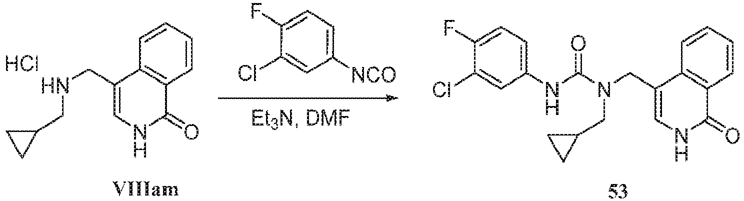

3-(3-Chloro-4-fluorophenyl)-1-(cyclopropylmethyl)-1-((1-oxo-1,2-dihydroisoquinolin-4-yl)methyl)urea (**Compound 53**) was synthesized in a similar manner as described above from 4-(((cyclopropylmethyl)amino)methyl)isoquinolin-1(2*H*)-one hydrochloride (**VIIIam**) and 2-chloro-1-fluoro-4-isocyanatobenzene. LCMS: *m*/*z* found 400.1/402.1 [M+H]⁺, RT = 4.34 min (Method A); ¹H NMR (400 MHz, DMSO-d₆) δ 11.24 (bs, 1H), 8.61 (s, 1H), 8.24 (d, 1H), 7.72-7.81 (m, 3H), 7.45-7.54 (m, 2H), 7.31 (t, 1H), 7.06 (s, 1H), 4.72 (s, 2H), 3.20 (d, 2H), 1.01-1.07 (m, 1H), 0.36-0.42 (m, 2H), 0.17-0.22 (m, 2H).

### N-((1-Methoxyisoquinolin-4-yl)methyl)propan-2-amine (VIc)

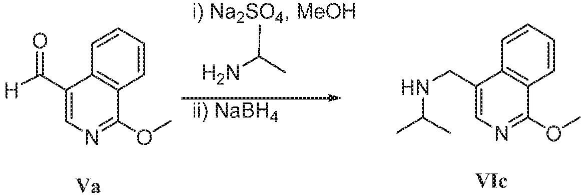

To a solution 2.0 g (10.69 mmol, 1.0 eq.) of 1-methoxyisoquinoline-4-carbaldehyde (**Va**) in 20 mL of methanol at room temperature was added 2 g of solid sodium sulfate and 2.6 mL (32.1 mmol, 3.0 eq.) of isopropyl amine. The mixture was stirred at room temperature for 16 h, and 0.61 g (16.0 mmol, 1.5 eq.) of sodium borohydride was added portionwise. The mixture was stirred at room temperature for a further 16 h, and the solvent was removed *in vacuo.* The residue was suspended in 100 mL of ice cold water and extracted with 3 x 80 mL of ethyl acetate. The combined organic extracts were washed with 80 mL of brine, dried (Na₂SO₄), filtered and the solvent was removed *in vacuo* to provide 1 g crude of *N*-((1-methoxyisoquinolin-4-yl)methyl)propan-2-amine (**VIc**), which was used without further purification. LCMS: *m*/*z* found 231.22 [M+H]⁺.

### 3-(3-Chloro-4-fluorophenyl)-1-isopropyl-1-((1-methoxyisoquinolin-4-yl)methyl)urea (Compound 19)

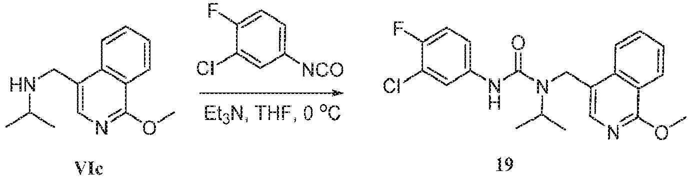

To a solution of 1 g of crude *N*-((1-methoxyisoquinolin-4-yl)methyl)propan-2-amine **(VIc)** in 10 mL of THF at 0 °C was added 1.2 mL (8.7 mmol) of trimethylamine, followed by 0.74 g (4.6 mmol) of 4-fluoro-3-chlorophenylisocyanate. The mixture was allowed to warm to room temperature and stirred for 16 h. The solvent was removed *in vacuo* and the residue was redissolved in 110 mL of methylene chloride. The organic solution was washed with 50 mL of brine, dried (Na₂SO₄), filtered and the solvent was removed *in vacuo.* The residue was purified by semi-preparative HPLC to provide 3-(3-chloro-4-fluorophenyl)-1-isopropyl-1-((1-methoxyisoquinolin-4-yl)methyl)urea **(Compound 19);** LCMS: *m*/*z* found 402.2/404.2 [M+H]⁺, RT = 5.18 min (Method A); ¹H NMR (400 MHz, DMSO-d₆): δ 8.54 (s, 1H), 8.23 (d, 1H), 8.09 (d, 1H), 7.84 (t, 1H), 7.73-7.75 (m, 2H), 7.67 (t, 1H), 7.40-7.44 (m, 1H), 7.24-7.28 (m, 1H), 4.85 (s, 2H), 4.53 (q, 1H), 4.02 (s, 3H), 1.14 (d, 6H).

### 4-((Isopropylamino)methyl)isoquinolin-1(2H)-one (VIIIc)

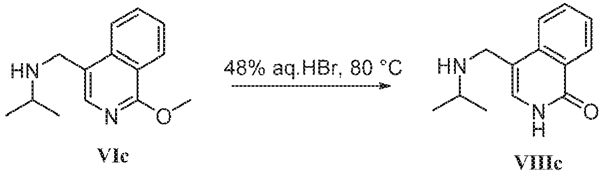

A suspension of 0.5 g of *N*-((1-methoxyisoquinolin-4-yl)methyl)propan-2-amine **(VIc)** in 5 mL of 48% aqueous hydrobromic acid was heated at 80 °C for 4 h. The mixture was allowed to cool to room temperature and evaporated under reduced pressure to provide 0.55 g of crude of 4-((isopropylamino)methyl)isoquinolin-1(2*H*)-one **(VIIIc),** which was used without further purification. LCMS: *m*/*z* found 217.19 [M+H]⁺.

### 3-(3-Chloro-4-fluorophenyl)-1-isopropyl-1-((1-oxo-1,2-dihydroisoquinolin-4-yl)methyl)urea (Compound 26)

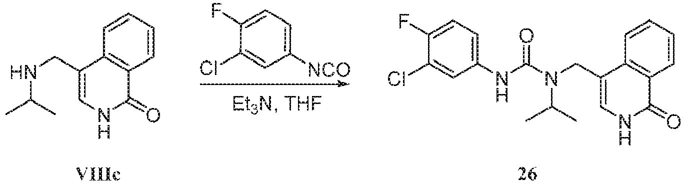

To a solution of 0.5 g of crude 4-((isopropylamino)methyl)isoquinolin-1(2*H*)-one **(VIIIc)** in 10 mL of anhydrous THF at 0 °C was added 0.64 mL (4.32 mmol) of trimethylamine, followed by 0.4 g (2.31 mmol) of 4-fluoro-3-chlorophenylisocyanate. The mixture was allowed to warm to room temperature and stirred for 4 h. The solvent was removed *in vacuo,* and the residue was redissolved in 100 mL of methylene chloride. The organic solution was washed with 50 mL of brine, dried (Na₂SO₄), filtered and the solvent was removed *in vacuo.* The residue was purified by reserve-phase chromatography to provide 0.18 g (0.46 mmol, 21% from **VIc**) of 3-(3-chloro-4-fluorophenyl)-1-isopropyl-1-((1-oxo-1,2-dihydroisoquinolin-4-yl)methyl)urea **(Compound 26).** LCMS: *m*/*z* found 388.1/390.1 [M+H]⁺, RT = 4.04 min (Method A); ¹H NMR (400 MHz, DMSO-d₆): δ 11.13 (d, 1H), 8.51 (s, 1H), 8.25 (d, 1H), 7.73-7.84 (m, 3H), 7.55 (t, 1H), 7.40-7.44 (m, 1H), 7.27 (t, 1H), 6.78 (d, 1H), 4.57 (s, 2H), 4.46-4.49 (m, 1H), 1.13 (d, 6H).

### N-((1-Methoxyisoquinolin-4-yl)methyl)propan-1-amine (VIk)

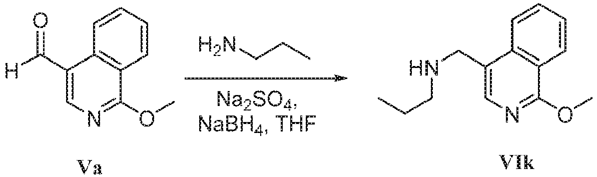

To a solution of 3.8 g (20.3 mmol, 1.0 eq.) of 1-methoxyisoquinoline-4-carbaldehyde **(Va)** in 40 mL of methanol was added 1.7 g (30.5 mmol, 1.5 eq.) of *n*-propylamine followed by 3.8 g of sodium sulfate. The mixture was stirred at room temperature for 16 h and 0.76 g (20.3 mmol, 1.0 eq.) of sodium borohydride was added. The mixture was stirred at room temperature for 16 h and the volatiles were removed *in vacuo.* The residue was resuspended in 200 mL of ice-cold water and extracted with 3 × 100 mL of ethyl acetate. The combined organic extracts were washed with 100 mL of water, 100 mL of brine, dried (Na₂SO₄), filtered and the solvent was removed *in vacuo,* to provide 3.8 g of crude *N-*((1-methoxyisoquinolin-4-yl)methyl)propan-1-amine **(VIk)** which was used without further purification. LCMS: *m*/*z* found 231.2 [M+H]⁺.

### 3-(3-Chloro-4-fluorophenyl)-1-((1-methoxyisoquinolin-4-yl)methyl)-1-propylurea (Compound 20)

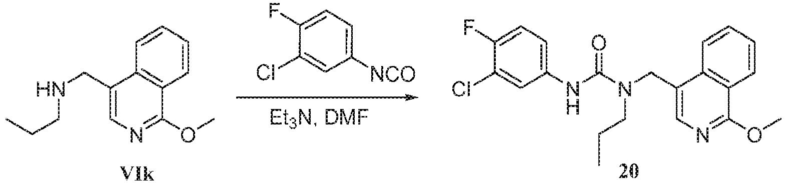

3-(3-Chloro-4-fluorophenyl)-1-((1-methoxyisoquinolin-4-yl)methyl)-1-propylurea **(Compound 20)** was synthesized in a similar manner as described above from *N*-((1-methoxyisoquinolin-4-yl)methyl)propan-1-amine **(VIk)** and 2-chloro-1-fluoro-4-isocyanatobenzene. LCMS: *m*/*z* found 402.2/404.2 [M+H]⁺, RT = 5.46 min (Method A); ¹H NMR (400 MHz, DMSO-d₆) δ 8.56 (s, 1H), 8.21-8.24 (d, 1H), 8.05-8.07 (d, 1H), 7.91 (s, 1H), 7.71-7.83 (m, 2H), 7.63-7.67 (t, 1H), 7.45-7.49 (m, 1H), 7.28-7.33 (t, 1H), 4.89 (s, 2H), 4.06 (s, 3H), 3.18-3.22 (m, 2H), 1.46-1.51 (m, 2H), 0.77-0.81 (t, 3H).

### 4-((Propylamino)methyl)isoquinolin-1(2H)-one (VIIIag)

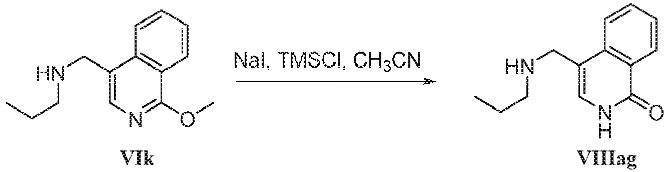

To a solution of 1.0 g (4.35 mmol, 1.0 eq.) of *N*-((1-methoxyisoquinolin-4-yl)methyl)propan-1-amine **(VIk)** in 10 mL of acetonitrile at room temperature was added 1.9 g (13.04 mmol, 3.0 eq.) of sodium iodide followed by 2.3 g (21.7 mmol, 5.0 eq.) of trimethylsilyl chloride and the mixture was stirred at room temperature for 16 h. The volatiles were removed *in vacuo* and the residue was suspended in 100 mL of water and extracted 3 × 80 mL of 10% methanol in methylene chloride. The combined organic extracts were washed with 50 mL of brine, dried (Na₂SO₄), filtered and the solvent was removed *in vacuo.* The residue was purified by trituration with diethyl ether to provide 0.5 g (2.31 mmol, 53%) of 4-((propylamino)methyl)isoquinolin-1(2H)-one **(VIIIag).** ¹H NMR (400 MHz, CDCl₃): δ 10.20-10.80 (bs, 1H), 8.43 (dd, 1H), 7.83 (d, 1H), 7.72 (td, 1H), 7.51 (m, 1H), 7.16 (s, 1H), 3.89 (s, 2H), 2.69 (t, 2H), 1.56 (m, 2H), 0.94 (t, 3H).

### 4-Nitrophenyl (3-chloro-4-fluorophenyl)carbamate

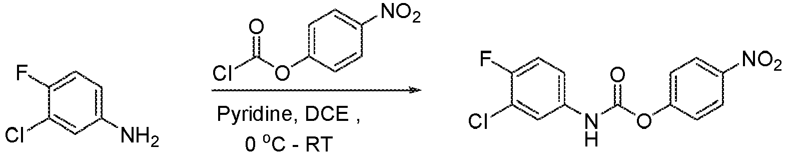

To a solution of 3.0 g (20.6 mmol, 1.0 eq.) of 3-chloro-4-fluoroaniline in 30 mL of 1,2-dichloroethane at 0 °C was added 4.8 g (61.8 mmol, 3.0 eq.) of pyridine followed by 5.0 g (24.7 mmol, 1.2 eq.) of 4-nitrophenyl chloroformate. The mixture was allowed to warm to room temperature and stirred for 5 h. The solvent was removed *in vacuo* and the residue was resuspended in 20 mL of cold water. The solids were collected by filtration and washed with 250 mL of water followed by 125 mL of ethyl acetate, and dried under high vacuum to provide 3.6g, (11.6 mmol, 57%) of 4-nitrophenyl (3-chloro-4-fluorophenyl)carbamate. ¹H NMR (400 MHz, DMSO-d₆): δ 10.70 (s, 1H), 8.31-8.40 (d, 2H), 7.73-7.75 (d, 1H), 7.55-7.57 (d, 2H), 7.40-7.49 (dd, 2H).

### 3-(3-Chloro-4-fluorophenyl)-1-((1-oxo-1,2-dihydroisoquinolin-4-yl)methyl)-1-propylurea (Compound 28)

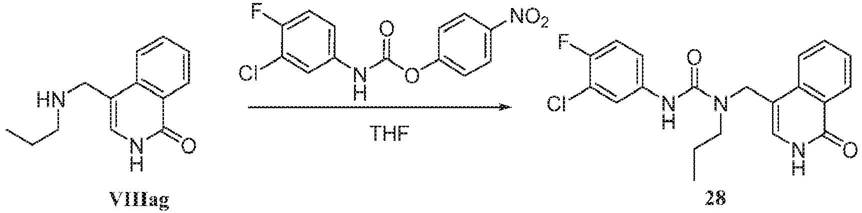

To a solution of 0.50 g (2.31 mmol, 1.0 eq.) of 4-((propylamino)methyl)isoquinolin-1(2H)-one **(VIIIag)** in 5 mL of THF in a sealed tube was added 1.0 g (3.47 mmol, 1.5 eq.) of 4-nitrophenyl (3-chloro-4-fluorophenyl) carbamate. The vessel was sealed, and the mixture was heated at 80 °C for 16 h. The solvent was removed *in vacuo* and the residue was purified by semi-preparative HPLC to provide 101 mg (0.26 mmol, 11%) of 3-(3-chloro-4-fluorophenyl)-1-((1-oxo-1,2-dihydroisoquinolin-4-yl)methyl)-1-propylurea **(Compound 28).** LCMS: *m*/*z* found 388.3/390.3 [M+H]⁺; ¹H NMR (400 MHz, DMSO-d₆): δ 11.25 (s, 1H), 8.53 (s, 1H), 8.24 (d, 1H), 7.72-7.81 (m, 3H), 7.52 (d, 1H), 7.45-7.50 (m, 1H), 7.30 (t, 1H), 7.11 (d, 1H), 4.64 (s, 2H), 3.21 (t, 2H), 1.47 (m, 2H), 0.80 (t, 3H).

### N-((1-Methoxyisoquinolin-4-yl)methyl)cyclopropanamine (VIm)

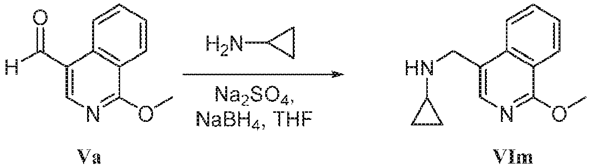

To a solution of 0.2 g (1.07 mmol, 1.0 eq.) of 1-methoxyisoquinoline-4-carbaldehyde (Va) in 4 mL of methanol was added 0.22 mL (3.21 mmol, 3.0 eq.) of cyclopropyl amine followed by 0.2 g of sodium sulfate. The mixture was stirred at room temperature for 16 h and 0.08 g (2.13 mmol, 2.0 eq.) of sodium borohydride was added. The mixture was stirred at room temperature for 2 h and the volatiles were removed *in vacuo.* The residue was resuspended in 20 mL of ice-cold water and extracted with 2 × 50 mL of ethyl acetate. The combined organic extracts were washed with 50 mL of water, 50 mL of brine, dried (Na₂SO₄), filtered and the solvent was removed *in vacuo* to provide 0.2 g of N-((1-methoxyisoquinolin-4-yl)methyl)cyclopropanamine **(VIm).** LCMS: *m*/*z* found 229.5 [M+H]⁺.

### 3-(3-Chloro-4-fluorophenyl)-1-cyclopropyl-1-((1-methoxyisoquinolin-4-yl)methyl)urea (Compound 34)

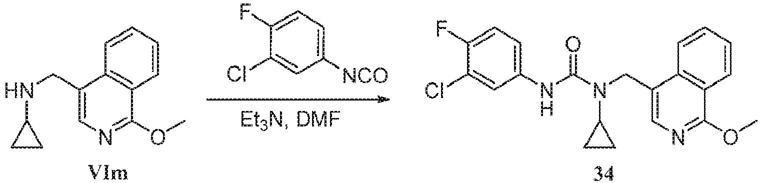

3-(3-Chloro-4-fluorophenyl)-1-cyclopropyl-1-((1-methoxyisoquinolin-4-yl)methyl)urea **(Compound 34)** was synthesized in a similar manner as described above from *N*-((1-methoxyisoquinolin-4-yl)methyl)cyclopropanamine **(VIm)** and 2-chloro-1-fluoro-4-isocyanatobenzene. LCMS: *m*/*z* found 400.2/402.2 [M+H]⁺, RT = 5.40 min (Method A); ¹H NMR (400 MHz, DMSO-d₆) δ 8.54 (bs, 1H), 8.23 (d, 1H), 8.06 (d, 1H), 7.97 (s, 1H), 7.79-7.85 (m, 2H), 7.62-7.67 (m, 1H), 7.48-7.53 (m, 1H), 7.29-7.35 (m, 1H), 4.84 (s, 2H), 4.06 (s, 3H), 2.27-2.33 (m, 1H), 0.83-0.92 (m, 4H).

### 4-((Cyclopropylamino)methyl)isoquinolin-1(2H)-one hydrobromide (VIIIah)

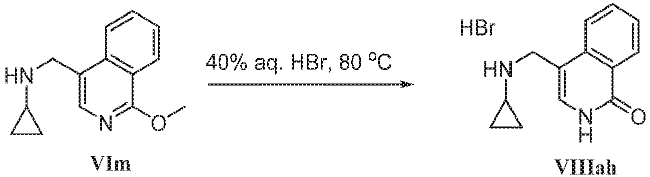

A solution of 0.5 g (2.19 mmol, 1.0 eq) of *N*-((1-methoxyisoquinolin-4-yl)methyl)cyclopropanamine **(VIm)** in 5 mL of 40% aqueous HBr was stirred at 80 °C for 3 h. The mixture was allowed to cool to room temperature and the solvent was removed *in vacuo.* The residue was triturated with 20 mL of diethyl ether and dried under high vacuum to provide 0.3 g of 4-((cyclopropylamino)methyl)isoquinolin-1(2*H*)-one hydrobromide **(VIIIah).** LCMS: *m*/*z* found 215.3 [M+H]⁺; ¹H NMR (400 MHz, CDCl₃) δ 8.93 (bs, 2H), 8.27 (d, 1H), 7.97 (d, 1H), 7.82 (t, 1H), 7.53-7.60 (m, 2H), 4.42 (t, 2H), 2.09 (bs, 1H), 1.79 (s, 1H), 0.73-0.91 (m, 4H).

### 3-(3-Chloro-4-fluorophenyl)-1-cyclopropyl-1-((1-oxo-1,2-dihydroisoquinolin-4-yl)methyl)urea (Compound 42)

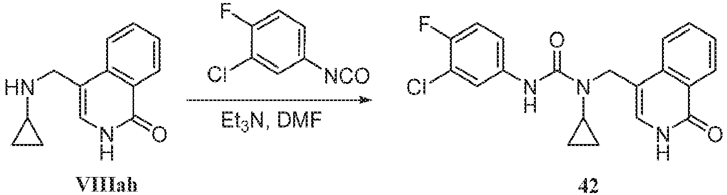

3-(3-Chloro-4-fluorophenyl)-1-cyclopropyl-1-((1-oxo-1,2-dihydroisoquinolin-4-yl)methyl)urea **(Compound 42)** was synthesized in a similar manner as described above from 4-((cyclopropylamino)methyl)isoquinolin-1(2*H*)-one hydrobromide **(VIIIah)** and 2-chloro-1-fluoro-4-isocyanatobenzene. LCMS: *m*/*z* found 386.2/388.2 [M+H]⁺, RT = 4.13 min (Method A); ¹H NMR (400 MHz, DMSO-d₆) δ 11.28 (bd, 1H), 8.54 (bs, 1H), 8.25 (d, 1H), 7.82-7.86 (m, 1H), 7.71-7.80 (m, 2H), 7.47-7.54 (m, 2H), 7.32 (t, 1H), 7.14 (d, 1H), 4.60 (s, 2H), 2.26-2.32 (m, 1H), 0.77-0.92 (m, 4H).

### N-((1-Methoxyisoquinolin-4-yl)methyl)butan-1-amine (VIn)

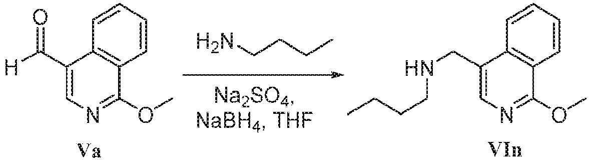

To a solution of 0.5 g (2.67 mmol, 1.0 eq.) of 1-methoxyisoquinoline-4-carbaldehyde **(Va)** in 5 mL of methanol was added 0.49 g (8.02 mmol, 3.0 eq.) of n-butylamine followed by 0.5 g of sodium sulfate. The mixture was stirred at room temperature for 16 h and 0.30 g (8.02 mmol, 3.0 eq.) of sodium borohydride was added. The mixture was then stirred at room temperature for a further 2 h and the volatiles were removed *in vacuo.* The residue was resuspended in 50 mL of ice-cold water and extracted with 2 × 50 mL of ethyl acetate. The combined organic extracts were washed with 50 mL of water, 50 mL of brine, dried (Na₂SO₄), filtered and the solvent was removed *in vacuo* to provide 0.56 g (2.27 mmol, 85%) of *N*-((1-methoxyisoquinolin-4-yl)methyl)butan-1-amine **(VIn).** LCMS: *m*/*z* found 245.4 [M+H]⁺. ¹H NMR (400 MHz, CDCl₃) δ 8.27 (d, 1H), 8.03 (d, 1H), 7.93 (s, 1H), 7.68-7.72 (m 1H), 7.51-7.55 (m, 1H), 4.12 (s, 3H), 4.05 (s, 2H), 2.71 (t, 2H), 1.47-1.53 (m, 2H), 1.32-1.38 (m, 2H), 1.31 (bs, 1H), 0.92 (t, 3H).

### 1-Butyl-3-(3-chloro-4-fluorophenyl)-1-((1-methoxyisoquinolin-4-yl)methyl)urea (Compound 33)

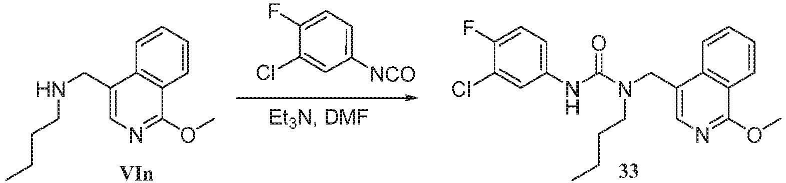

1-Butyl-3-(3-chloro-4-fluorophenyl)-1-((1-methoxyisoquinolin-4-yl)methyl)urea **(Compound 33)** was synthesized in a similar manner as described above from N-((1-methoxyisoquinolin-4-yl)methyl)butan-1-amine **(VIn)** and 2-chloro-1-fluoro-4-isocyanatobenzene. LCMS: *m*/*z* found 416.2/418.2 [M+H]⁺, RT = 6.02 min (Method A); ¹H NMR (400 MHz, DMSO-d₆) δ 8.57 (s, 1H), 8.21-8.24 (m, 1H), 8.05-8.06 (m, 1H), 7.91 (s, 1H), 7.77-7.84 (m, 2H), 7.63-7.67 (m, 1H), 7.45-7.49 (m, 1H), 7.28-7.33 (m, 1H), 4.89 (s, 2H), 4.06 (s, 3H), 3.22-3.26 (m, 2H), 1.44-1.49 (m, 2H), 1.17-1.27 (m, 2H), 0.82 (t, 3H).

### 4-((Butylamino)methyl)isoquinolin-1(2H)-one (VIIIai)

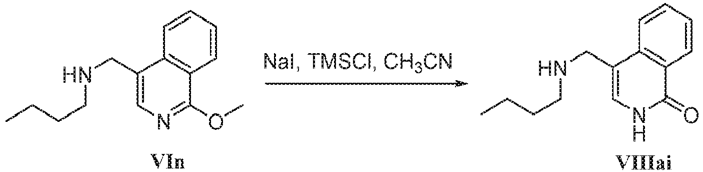

To a solution of 0.55 g (2.25 mmol, 1.0 eq.) of *N*-((1-methoxyisoquinolin-4-yl)methyl)butan-1-amine **(VIn)** in 10 mL of acetonitrile at room temperature was added 1.22 g (11.3 mmol, 5.0 eq.) of trimethylsilyl chloride followed by 1.0 g (6.76 mmol, 3.0 eq.) of sodium iodide and the mixture was stirred at room temperature for 16 h. The mixture was diluted with 15 mL of saturated sodium bicarbonate solution and extracted 3 × 60 mL of ethyl acetate. The combined organic extracts were washed with 30 mL of brine, dried (Na₂SO₄), filtered and the solvent was removed *in vacuo.* The residue was purified by trituration with diethyl ether to provide 0.45 g of 4-((butylamino)methyl)isoquinolin-1(2H)-one **(VIIIai).** LCMS: *m*/*z* found 230.9 [M+H]⁺.

### 1-Butyl-3-(3-chloro-4-fluorophenyl)-1-((1-oxo-1,2-dihydroisoquinolin-4-yl)methyl)urea (Compound 52)

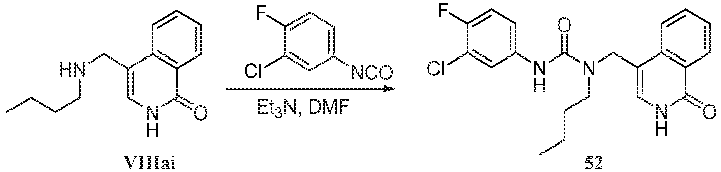

1-Butyl-3-(3-chloro-4-fluorophenyl)-1-((1-oxo-1,2-dihydroisoquinolin-4-yl)methyl)urea **(Compound 52)** was synthesized in a similar manner as described above from 4-((butylamino)methyl)isoquinolin-1(2*H*)-one **(VIIIai)** and 2-chloro-1-fluoro-4-isocyanatobenzene. LCMS: *m*/*z* found 402.1/404.1 [M+H]⁺, RT = 4.62 min (Method A); ¹H NMR (400 MHz, DMSO-d₆) δ 11.25 (bs, 1H), 8.54 (s, 1H), 8.24 (dd, 1H), 7.72-7.80 (m, 3H), 7.47-7.54 (m, 2H), 7.30 (t, 1H), 7.11 (s, 1H), 4.64 (s, 2H), 3.24 (t, 2H), 1.39-1.48 (m, 2H), 1.16-1.27 (m, 2H), 0.82 (t, 3H).

### 2-((tert-Butyldimethylsilyl)oay)-N-((1-methoayisoquinolin-4-yl)methyl)ethan-1-amine (VIo)

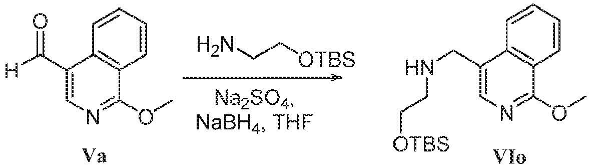

To a solution of 0.4 g (2.13 mmol, 1.0 eq.) of 1-methoxyisoquinoline-4-carbaldehyde **(Va)** in 5 mL of methanol was added 1.2 g (6.38 mmol, 3.0 eq.) of 2-((*tert*butyldimethylsilyl)oxy)ethan-1-amine followed by 0.4 g of sodium sulfate. The mixture was stirred at room temperature for 16 h and 0.12 g (3.19 mmol, 1.0 eq.) of sodium borohydride was added. The mixture was stirred at room temperature for a further 2 h and the volatiles were removed *in vacuo.* The residue was resuspended in 50 mL of ice-cold water and extracted with 3 × 50 mL of ethyl acetate. The combined organic extracts were washed with 100 mL of water, 100 mL of brine, dried (Na₂SO₄), filtered and the solvent was removed *in vacuo.* The residue was purified by flash chromatography (SiO₂, eluting with a linear gradient of 0-25% ethyl acetate/hexane) to provide 0.4 g (1.15 mmol, 54%) of 2-((*tert*butyldimethylsilyl)oxy)-*N*-((1-methoxyisoquinolin-4-yl)methyl)ethan-1-amine **(VIo).** LCMS: *m*/*z* found 347.3 [M+H]⁺.

### 1-(2-((tert-Butyldimethylsilyl)oxy)ethyl)-3-(3-chloro-4-fluorophenyl)-1-((1-methoxyisoquinolin-4-yl)methyl)urea (VIIa)

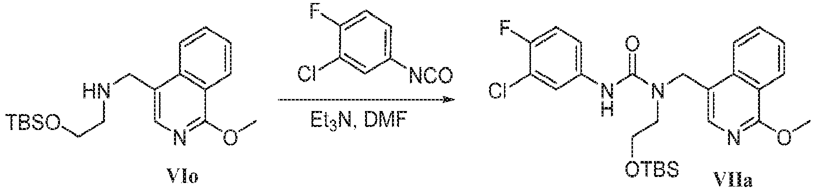

1-(2-((*tert*-Butyldimethylsilyl)oxy)ethyl)-3-(3-chloro-4-fluorophenyl)-1-((1-methoxyisoquinolin-4-yl)methyl)urea **(VIIa)** was synthesized in a similar manner as described above 2-((tert-butyldimethylsilyl)oxy)-*N*-((1-methoxyisoquinolin-4-yl)methyl)ethan-1-amine **(VIo)** and 2-chloro-1-fluoro-4-isocyanatobenzene. LCMS: *m*/*z* found 518.3/520.3 [M+H]⁺.

### 3-(3-Chloro-4-fluorophenyl)-1-(2-hydroxyethyl)-1-((1-methoxyisoquinolin-4-yl)methyl)urea (Compound 21)

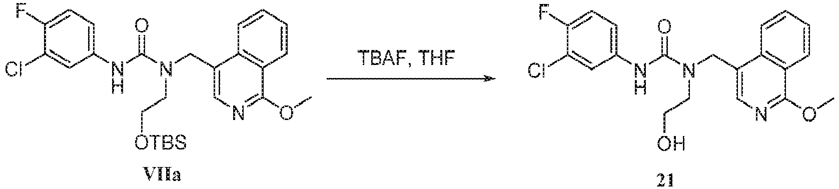

To a solution of 300 mg of 1-(2-((tert-butyldimethylsilyl)oxy)ethyl)-3-(3-chloro-4-fluorophenyl)-1-((1-methoxyisoquinolin-4-yl)methyl)urea **(VIIa)** in 3 mL of THF was added 1.7 mL (1.74 mmol, 3.0 eq.) of a 1 M solution of tetrabutyl ammonium fluoride in THF and the mixture was stirred at room temperature for 2 h. The solvent was removed *in vacuo* and the residue was purified by flash chromatography (SiO₂, eluting with a linear gradient of 40-70% ethyl acetate/hexane) to provide 75 mg (0.19 mmol, 21% from **VIo**) of 3-(3-chloro-4-fluorophenyl)-1-(2-hydroxyethyl)-1-((1-methoxyisoquinolin-4-yl)methyl)urea **(Compound 21).** LCMS: *m*/*z* found 404.2/406.2 [M+H]⁺, RT = 4.44 min (Method A); ¹H NMR (400 MHz, DMSO-d₆) δ 8.89 (s, 1H), 8.23 (d, 1H), 8.06 (d, 1H), 7.93 (s, 1H), 7.82 (t, 1H), 7.76 (dd, 1H), 7.66 (t, 1H), 7.35-7.38 (m, 1H), 7.31 (t, 1H), 5.19-5.21 (m, 1H), 4.93 (s, 2H), 4.06 (s, 3H), 3.46 (q, 2H), 3.33-3.39 (m, 2H).

### 3-(3-Chloro-4-fluorophenyl)-1-(2-hydroxyethyl)-1-((1-oxo-1,2-dihydroisoquinolin-4-yl)methyl)urea (Compound 27)

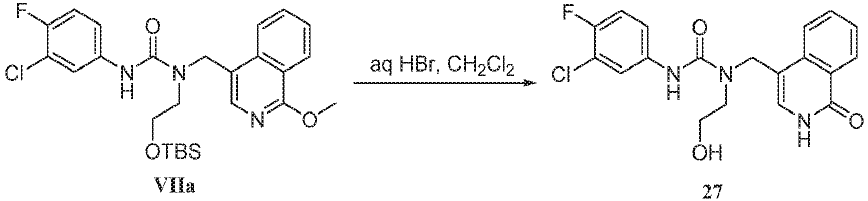

To a solution of 300 mg of 1-(2-((*tert*-butyldimethylsilyl)oxy)ethyl)-3-(3-chloro-4-fluorophenyl)-1-((1-methoxyisoquinolin-4-yl)methyl)urea **(VIIa)** in 3 mL of methylene chloride was added 0.24 g (2.9 mmol, 5.0 eq.) of 47% aqueous HBr and the mixture was stirred at room temperature for 6 h. The mixture was then diluted with 100 mL of ethyl acetate and washed with 50 mL of saturated sodium bicarbonate solution, 50 mL of brine, dried (Na₂SO₄), filtered and the solvent was removed *in vacuo.* The residue was purified by reverse-phase chromatography (eluting with a linear gradient of 10-40% [0.1% formic acid in water]/acetonitrile) to provide 0.036 g (0.09 mmol, 11% from **VIo**) of 3-(3-chloro-4-fluorophenyl)-1-(2-hydroxyethyl)-1-((1-oxo-1,2-dihydroisoquinolin-4-yl)methyl)urea **(Compound 27).** LCMS: *m*/*z* found 390.2/392.1 [M+H]⁺, RT = 3.39 min (Method A); ¹H NMR (400 MHz, DMSO-d₆) δ 11.28 (s, 1H), 8.87 (s, 1H), 8.24 (d, 1H), 7.74-7.81 (m, 3H), 7.52 (t, 1H), 7.35-7.36 (m, 1H), 7.31 (t, 1H), 7.12 (d, 1H), 5.19 (bs, 1H), 4.68 (s, 2H), 3.44-3.47 (m, 2H), 3.32-3.37 (m, 2H).

### 2-Methoxy-N-((1-methoayisoquinolin-4-yl)methyl)ethan-1-amine (VIp)

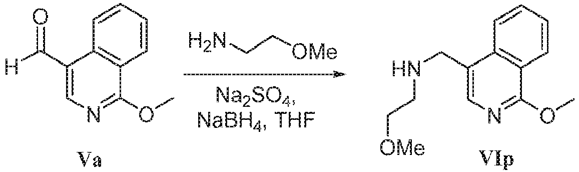

To a solution of 1.0 g (5.3 mmol, 1.0 eq.) of 1-methoxyisoquinoline-4-carbaldehyde **(Va)** in 10 mL of methanol was added 1.19 g (15.9 mmol, 3.0 eq.) of 2-methoxyethan-1-amine followed by 1.0 g of sodium sulfate. The mixture was stirred at room temperature for 16 h and 0.47 g (12.7 mmol, 2.4 eq.) of sodium borohydride was added. The mixture was then stirred at room temperature for a further 4 h and the volatiles were removed *in vacuo.* The residue was resuspended in 50 mL of ice-cold water and extracted with 2 × 50 mL of ethyl acetate. The combined organic extracts were washed with 50 mL of water, 50 mL of brine, dried (Na₂SO₄), filtered and the solvent was removed *in vacuo* to provide 1.2 g (4.9 mmol, 91%) of 2-methoxy-*N*-((1-methoxyisoquinolin-4-yl)methyl)ethan-1-amine **(VIp).** LCMS: *m*/*z* found 247.5 [M+H]⁺. ¹H NMR (400 MHz, DMSO-d₆) δ 8.27 (d, 1H), 8.04 (d, 1H), 7.94 (s, 1H), 7.69-7.73 (m 1H), 7.51-7.56 (m, 1H), 4.11 (s, 3H), 4.08 (s, 2H), 3.52 (t, 2H), 3.34 (s, 3H), 2.88 (t, 2H), 1.69 (bs, 1H)

### 3-(3-Chloro-4-fluorophenyl)-1-(2-methoxyethyl)-1-((1-methoxyisoquinolin-4-yl)methyl)urea (Compound 41)

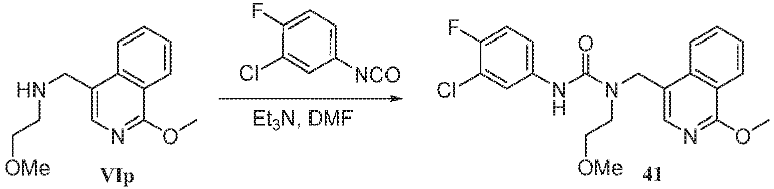

3-(3-Chloro-4-fluorophenyl)-1-(2-methoxyethyl)-1-((1-methoxyisoquinolin-4-yl)methyl)urea **(Compound 41)** was synthesized in a similar manner as described above from 2-methoxy-*N*-((1-methoxyisoquinolin-4-yl)methyl)ethan-1-amine **(VIp)** and 2-chloro-1-fluoro-4-isocyanatobenzene. LCMS: *m*/*z* found 418.2/420.2 [M+H]⁺, RT = 5.36 min (Method A); ¹H NMR (400 MHz, DMSO-d₆) δ 8.65 (bs, 1H), 8.23 (m, 1H), 8.04 (d, 1H), 7.90 (s, 1H), 7.76-7.85 (m, 2H), 7.63-7.68 (m, 1H), 7.38-7.44 (m, 1H), 7.31 (t, 1H), 4.94 (s, 2H), 4.06 (s, 3H), 3.41-3.47 (m, 4H), 3.21 (s, 3H).

### 4-(((2-Methoxyethyl)amino)methyl)isoquinolin-1(2H)-one hydrochloride (VIIIaj)

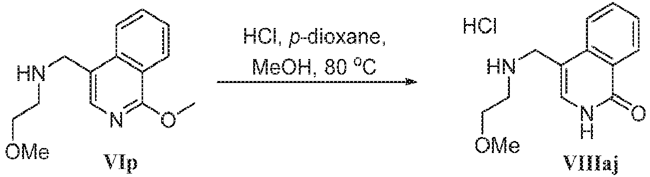

To a solution of 0.60 g (2.44 mmol, 1.0 eq.) of 2-methoxy-*N*-((1-methoxyisoquinolin-4-yl)methyl)ethan-1-amine **(VIp)** in 2.5 mL of methanol in a sealed tube was added 5 mL of a 4 M solution of HCl in *p*-dioxane. The vessel was sealed, and the mixture was heated at 80 °C for 6 h. The mixture was allowed to cool to room temperature and the solvent was removed *in vacuo.* The residue was triturated with 10 mL of *n*-pentane and dried under high vacuum to provide 0.55 g (2.0 mmol, 84%) 4-(((2-methoxyethyl)amino)methyl)isoquinolin-1(2*H*)-one hydrochloride **(VIIIaj).** LCMS: *m*/*z* found 233.3 [M+H]⁺; ¹H NMR (400 MHz, DMSO-d₆) δ 11.57 (bd, 1H), 8.89 (bs, 2H), 8.26 (d, 1H), 7.90-7.92 (m, 1H), 7.80-7.84 (m, 1H), 7.55-7.59 (m, 1H), 7.48 (d, 1H), 4.27-4.30 (m, 2H), 3.61-3.64 (m, 2H), 3.31 (s, 3H), 3.16-3.22 (m, 2H).

### 3-(3-Chloro-4-fluorophenyl)-1-(2-methoxyethyl)-1-((1-oxo-1,2-dihydroisoquinolin-4-yl)methyl)urea (Compound 50)

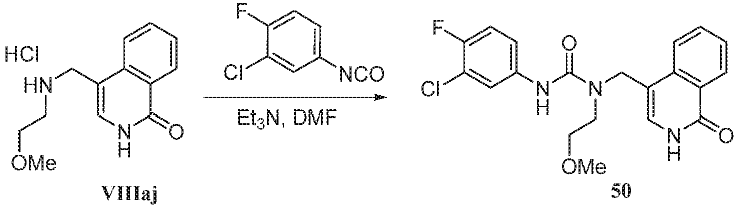

3-(3-Chloro-4-fluorophenyl)-1-(2-methoxyethyl)-1-((1-oxo-1,2-dihydroisoquinolin-4-yl)methyl)urea **(Compound 50)** was synthesized in a similar manner as described above from 4-(((2-methoxyethyl)amino)methyl)isoquinolin-1(2H)-one hydrochloride **(VIIIaj)** and 2-chloro-1-fluoro-4-isocyanatobenzene. LCMS: *m*/*z* found 404.1/406.1 [M+H]⁺, RT = 4.15 min (Method A); ¹H NMR (400 MHz, DMSO-d₆) δ 11.29 (d, 1H), 8.62 (bs, 1H), 8.24 (d, 1H), 7.72-7.78 (m, 3H), 7.50-7.54 (m, 1H), 7.38-7.43 (m, 1H), 7.31 (t, 1H), 7.09 (d, 1H), 4.68 (d, 2H), 3.45-3.48 (m, 2H), 3.38-3.41 (m, 2H), 3.21 (s, 3H).

### 2-Methoxy-N-((1-methoxyisoquinolin-4-yl)methyl)propan-1-amine (VIq)

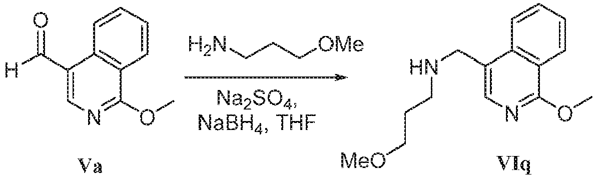

To a solution of 1.0 g (5.3 mmol, 1.0 eq.) of 1-methoxyisoquinoline-4-carbaldehyde **(Va)** in 20 mL of methanol was added 1.42 g (3.21 mmol, 3.0 eq.) of 2-methoxypropan-1-amine followed by 1.0 g of sodium sulfate. The mixture was stirred at room temperature for 16 h and 0.30 g (8.0 mmol, 1.5 eq.) of sodium borohydride was added. The mixture was then stirred at room temperature for a further 4 h and the volatiles were removed *in vacuo.* The residue was resuspended in 30 mL of ice-cold water and extracted with 2 × 60 mL of ethyl acetate. The combined organic extracts were washed with 50 mL of water, 50 mL of brine, dried (Na₂SO₄), filtered and the solvent was removed *in vacuo.* The residue was triturated with 20 mL of *n*-pentane to provide 1.1 g (4.2 mmol, 79%) of 2-methoxy-*N*-((1-methoxyisoquinolin-4-yl)methyl)propan-1-amine **(VIq).** LCMS: *m*/*z* found 261.4 [M+H]⁺; ¹H NMR (400 MHz, CDCl₃) δ 8.28 (dd, 1H), 8.05 (d, 1H), 7.93 (s, 1H), 7.68-7.72 (m, 1H), 7.51-7.56 (m, 1H), 4.11 (s, 3H), 4.06 (d, 2H), 3.44 (t, 2H), 3.30 (s, 3H), 2.80 (t, 2H), 1.80 (m, 2H), 1.68 (bs, 1H).

### 3-(3-Chloro-4-fluorophenyl)-1-((1-methoxyisoquinolin-4-yl)methyl)-1-(3-methoxypropyl)urea (Compound 47)

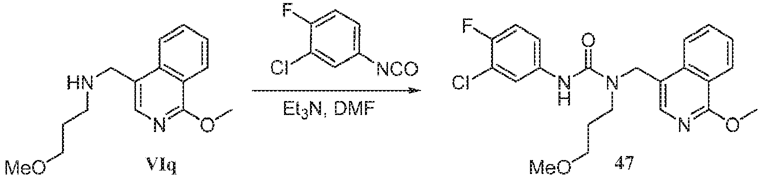

3-(3-Chloro-4-fluorophenyl)-1-((1-methoxyisoquinolin-4-yl)methyl)-1-(3-methoxypropyl)urea **(Compound 47)** was synthesized in a similar manner as described above from 2-methoxy-*N*-((1-methoxyisoquinolin-4-yl)methyl)propan-1-amine **(VIq)** and 2-chloro-1-fluoro-4-isocyanatobenzene. LCMS: *m*/*z* found 432.3/434.2 [M+H]⁺, RT = 5.52 min (Method A); ¹H NMR (400 MHz, DMSO-d₆) δ 8.57 (s, 1H), 8.23 (d, 1H), 8.06 (d, 1H), 7.91 (s, 1H), 7.78-7.85 (m, 2H), 7.66 (t, 1H), 7.42-7.47 (m, 1H), 7.31 (t, 1H), 4.89 (s, 2H), 4.06 (s, 3H), 3.26-3.31 (m, 4H), 3.17 (s, 3H), 1.67-1.74 (m, 2H).

### 4-(((2-Methoxypropyl)amino)methyl)isoquinolin-1(2H)-one hydrochloride (VIIIak)

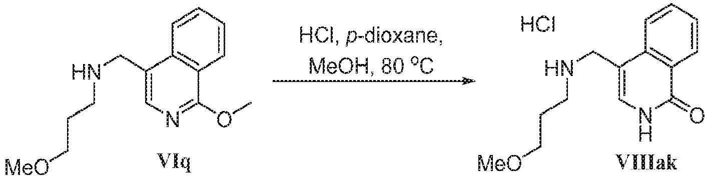

To a solution of 0.50 g (2.44 mmol, 1.0 eq.) of 2-methoxy-*N*-((1-methoxyisoquinolin-4-yl)methyl)propan-1-amine **(VIq)** in 5 mL of methanol in a sealed tube was added 5 mL of a 4 M solution of HCl in *p*-dioxane. The vessel was sealed, and the mixture was heated at 80 °C for 6 h. The mixture was allowed to cool to room temperature and the solvent was removed *in vacuo.* The residue was triturated with 10 mL of diethyl ether and dried under high vacuum to provide 0.4 g (1.42 mmol, 58%) 4-(((2-methoxypropyl)amino)methyl)isoquinolin-1(2*H*)-one hydrochloride **(VIIIak).** LCMS: *m*/*z* found 247.5 [M+H]⁺.

### 3-(3-Chloro-4-fluorophenyl)-1-(2-methoxypropyl)-1-((1-oxo-1,2-dihydroisoquinolin-4-yl)methyl)urea (Compound 51)

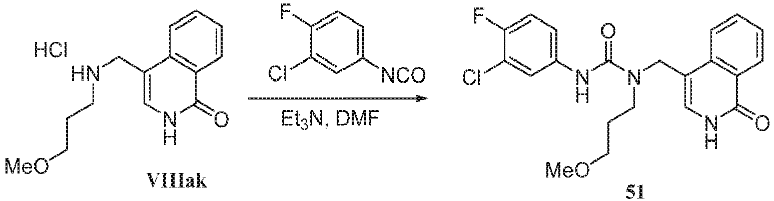

3-(3-Chloro-4-fluorophenyl)-1-(2-methoxypropyl)-1-((1-oxo-1,2-dihydroisoquinolin-4-yl)methyl)urea **(Compound 51)** was synthesized in a similar manner as described above from 4-(((2-methoxypropyl)amino)methyl)isoquinolin-1(2*H*)-one hydrochloride **(VIIIak)** and 2-chloro-1-fluoro-4-isocyanatobenzene. LCMS: *m*/*z* found 418.2/420.2 [M+H]⁺, RT = 4.15 min (Method A); ¹H NMR (400 MHz, DMSO-d₆) δ 11.14 (bd, 1H), 8.54 (bs, 1H), 8.25 (d, 1H), 7.71-7.81 (m, 3H), 7.42-7.55 (m, 2H), 7.31 (t, 1H), 7.11 (d, 1H), 4.64 (s, 2H), 3.26-3.31 (m, 4H), 3.17 (s, 3H), 1.65-1.72 (m, 2H).

### N-Benzyl-1-(1-methoxyisoquinolin-4-yl)methanamine (VIr)

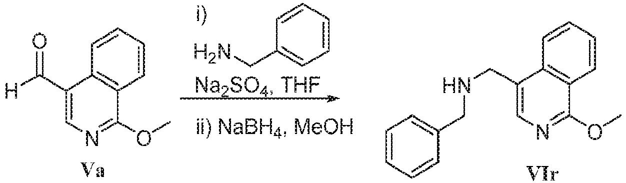

To a solution of 0.05 g (0.27 mmol, 1.0 eq.) of 1-methoxyisoquinoline-4-carbaldehyde **(Va)** in 3 mL of 1,2-dichloroethane was added 0.32 uL (0.29 mmol, 1.1 eq.) of benzylamine and 0.05 g of sodium sulfate. The mixture was stirred at room temperature for 16 h and then added to a stirred solution of 0.039 g (1.03 mmol, 3.8 eq.) of sodium borohydride in 1 mL of methanol. The mixture was stirred at room temperature for 30 min, diluted with 0.5 mL of acetone and stirred for a further 20 min. The solvent was removed *in vacuo* and the residue was partitioned between 5 mL of ethyl acetate and 5 mL of sat. sodium carbonate solution. The layers were separated, and the aqueous phase was extracted with 5 mL of ethyl acetate. The combined organic extracts were dried (Na₂SO₄). filtered and the solvent was removed *in vacuo* to provide 0.07 g of *N*-benzyl-1-(1-methoxyisoquinolin-4-yl)methanamine **(VIr).** LCMS: *m*/*z* found 279.2 [M+H]⁺, RT = 2.90 min (Method A).

### 1-Benzyl-3-(3-chloro-4-fluorophenyl)-1-((1-methoxyisoquinolin-4-yl)methyl)urea (Compound 48)

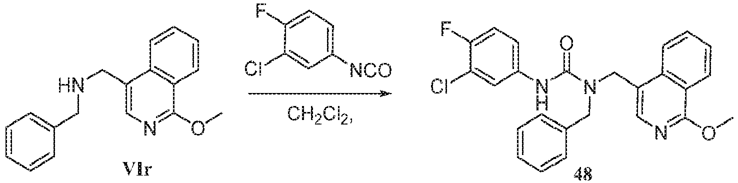

1-Benzyl-3-(3-chloro-4-fluorophenyl)-1-((1-methoxyisoquinolin-4-yl)methyl)urea **(Compound 48)** was synthesized in a similar manner as described above from N-benzyl-1-(1-methoxyisoquinolin-4-yl)methanamine **(VIr)** and 4-fluoro-3-chlorophenylisocyanate. LCMS: *m*/*z* found 450.4.1/452.1 [M+H]⁺, RT = 6.11 min (Method A).

### Isoquinoline-4-carbaldehyde

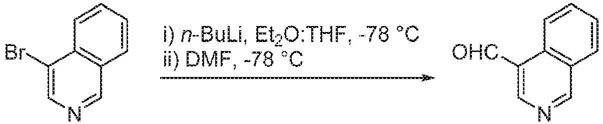

To a solution of 5.0 g (24.0 mmol, 1.0 eq.) of 4-bromoisoquinoline in 100 mL of 1:3 v/v diethyl ether:THF at -78 °C under a nitrogen atmosphere was added 30 mL (48.1 mmol, 2.0 eq,) of a 1.6 M solution of n-butyl lithium in hexanes. The mixture was stirred at -78 °C for 30 min and 4.5 mL (60.1 mmol, 2.5 eq) of DMF was added. The mixture was stirred at -78 °C for a further 30 min, quenched with 50 mL of saturated ammonium chloride solution (50 mL) and extracted with 2 × 100 mL of ethyl acetate. The combined organic extracts were washed with 50 mL of water, 50 mL of brine, dried (Na₂SO₄), filtered and the solvent was removed *in vacuo.* The residue was purified by flash chromatography (SiO₂, eluting with a linear gradient of 10-15% ethyl acetate/petroleum ether) to provide 1.0 g (6.4 mmol, 26%) of isoquinoline-4-carbaldehyde. LCMS: *m*/*z* found 158.0 [M+H]⁺; ¹H NMR (400 MHz, CDCl₃) δ 10.40 (s, 1H), 9.44 (s, 1H), 9.21 (d, 1H), 9.0 (d, 1H), 8.10 (d, 1H), 7.92 (t, 1H), 7.75 (t, 1H).

### N (Isoquinolin-4-ylmethyl)ethanamine

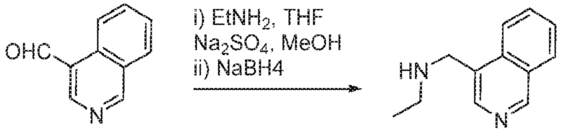

To a solution of 1.0 g (6.36 mmol, 1.0 eq.) of isoquinoline-4-carbaldehyde in 10 mL of methanol was added 10 mL (20.0 mmol, 3.1 eq.) of a 2 M solution of ethyl amine in THF followed by 1.0 g of sodium sulphate. The mixture was stirred at room temperature for 16 h and 0.24 g (6.36 mmol, 1.0 eq.) of sodium borohydride was added. The mixture was stirred at room temperature for a further 4 h and then diluted with 30 mL of ice-cold water. The mixture was extracted with 3 × 70 mL of ethyl acetate and the combined organic extracts were washed with 50 mL of water, 50 mL of brine, dried (Na₂SO₄), filtered and the solvent was removed *in vacuo* to provide 0.8 g (4.30 mmol, 67%) of jV-(isoquinolin-4-ylmethyl)ethanamine. ¹H NMR (400 MHz, CDCl₃) δ 9.18 (s, 1H), 8.48 (s, 1H), 8.15 (d, 1H), 8.00 (d, 1H), 7.77 (t, 1H), 7.61 (t, 1H), 4.20 (s, 2H), 2.80 (q, 2H), 1.20 (t, 3H).

### 3-(3-Chloro-4-fluorophenyl)-1-ethyl-1-(isoquinolin-4-ylmethyl)urea (Compound 16)

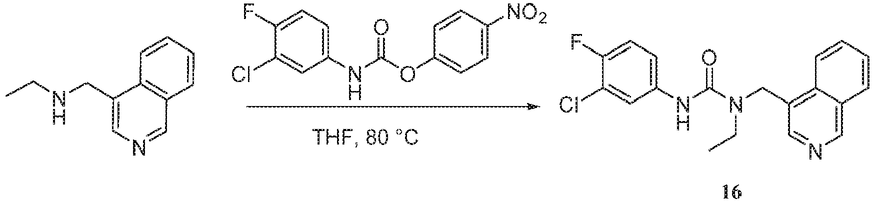

To a stirred solution of 0.5 g (2.68 mmol, 1.0 eq.) of jV-(isoquinolin-4-ylmethyl)ethanamine in 10 mL of anhydrous THF, was added 0.83 g (2.68 mmol, 1.0 eq.) of 4-nitrophenyl-(3-chloro-4-fluorophenyl) carbamate and the mixture was heated at 80 °C for 16 h. The reaction was quenched with 50 mL of water and extracted with 3 × 50 mL of water. The combined organic extracts were washed with 50 mL of water, 50 mL of brine, dried (Na₂SO₄), filtered and the solvent was removed *in vacuo.* The residue was purified by flash chromatography (SiO₂, eluting with a linear gradient of 20-30% ethyl acetate/hexanes) to provide 0.14 g (0.64 mmol, 26%) of 3-(3-chloro-4-fluorophenyl)-1-ethyl-1-(isoquinolin-4-ylmethyl)urea **(Compound 16).** LCMS: *m*/*z* found 358.2/360.2 [M+H]⁺, RT = 2.86 min (Method A); ¹H NMR (300 MHz, DMSO-d₆) δ 9.27 (s, 1H), 8.62 (s, 1H), 8.39 (s, 1H), 8.17 (d, 2H), 7.79-7.87 (m, 2H), 7.72 (t, 1H), 7.46-7.52 (m, 1H), 7.30 (t, 1H), 5.02 (s, 2H), 3.33-3.39 (q, 2H), 1.04 (t, 3H).

### 1-Methoxyisoquinoline-4-carboxylic acid (Vd)

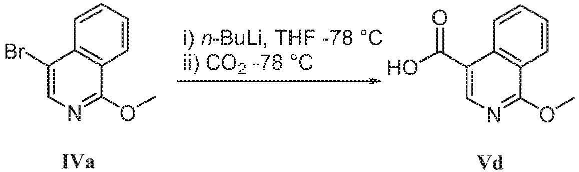

To a solution of 10.0 g (42 mmol, 1.0 eq.) of 4-bromo-1-methoxyisoquinoline **(IVa)** in 600 mL of anhydrous THF at -78 °C was added 52.5 mL (84 mmol, 1.5 eq.) of a 1.6 M solution of *n*-BuLi. The mixture was stirred at -78 °C for 2 h and then poured on to crushed dry-ice and allowed to warm to room temperature. The mixture was diluted with 1.5 L of water and acidified with 10% citric acid solution. The resulting precipitate was collected by filtration and dried under high vacuum to provide 5.3 g (26.1 mmol, 61%) of 1-methoxyisoquinoline-4-carboxylic acid **(Vd).** LCMS: *m*/*z* found 204.0 [M+H] ⁺; ¹H NMR (400 MHz, DMSO-d₆): δ 13.02 (bs,1H), 8.93-8.95 (d, 1H), 8.73 (s, 1H), 8.26-8.29 (d, 1H), 7.86-7.92 (dd, 1H), 7.67-7.72 (dd, 1H), 4.14 (s, 3H). The above detailed reaction was performed in multiple batches on 10 g scale with consistent results.

### N,1-Dimethoxy-N-methylisoquinoline-4-carboxamide (Ve)

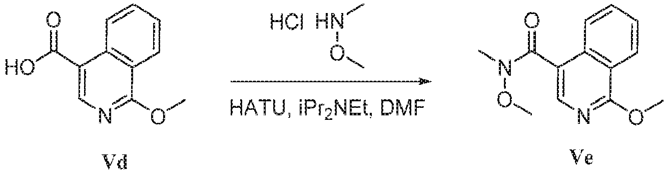

To a solution of 10.6 g (52.2 mmol, 1.0 eq.) of 1-methoxyisoquinoline-4-carboxylic acid **(Vd)** in 50 mL of anhydrous DMF was added 14 mL (156.6 mmol, 3.0 eq.) of *N,N-*diisopropyl ethylamine followed by 3.81 g (78.3 mmol, 1.5 eq.) of *N,O-*dimethylhydroxylamine and 14.9 g (78.3 mmol, 1.5 eq.) of HATU and the mixture was stirred at room temperature for 16 h. The mixture was then diluted with 100 mL of water and extracted with 3 × 50 mL of ethyl acetate. The combined organic extracts were washed with 50 mL of brine, dried (Na₂SO₄), filtered and the solvent was removed *in vacuo.* The residue was purified by flash chromatography (SiO₂, eluting with a linear gradient of 0-20% ethyl acetate/hexane) to provide 9.0 g (36.6 mmol, 70%) of *N*-1-dimethoxy-*N*-methylisoquinoline-4-carboxamide **(Ve).** LCMS: *m*/*z* found 247.05 [M+H]⁺; ¹H NMR (400 MHz, CDCl₃): δ 8.29 (d, 1H), 8.14 (s, 1H), 7.87 (d, 1H), 7.71 (dd, 1H), 7.57 (dd, 1H), 4.16 (s, 3H), 3.56 (s, 3H), 3.42 (s, 3H).

### 1-(1-Methoxyisoquinolin-4-yl)ethan-1-one (Vf)

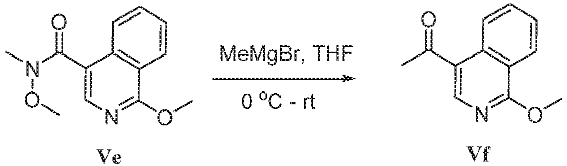

To a solution of 9.0 g (36.6 mmol, 1.0 eq.) of *N*-1-dimethoxy-*N*-methylisoquinoline-4-carboxamide **(Ve)** in 90 mL of anhydrous THF at 0 °C was added 60.9 mL (182.9 mmol, 5 eq.) of a 3.0 M solution of methyl magnesium bromide in THF. The mixture was allowed to warm to room temperature and stirred for 16 h. The reaction was slowly quenched with 750 mL of saturated ammonium chloride solution and extracted with 3 × 250 mL of ethyl acetate. The combined organic extracts were washed with 250 mL of brine, dried (Na₂SO₄), filtered and the solvent removed *in vacuo.* The residue was triturated with *n*-pentane to provide 6.7 g (27.2 mmol, 91%) of 1-(1-methoxyisoquinolin-4-yl)ethan-1-one **(Vf).** LCMS: *m*/*z* found 202.3 [M+H]⁺; ¹H NMR (300 MHz, CDCl₃): δ 9.03 (d, 1H), 8.71 (s, 1H), 8.30 (d, 1H), 7.79 (dd, 1H), 7.59 (dd, 1H), 4.20 (s, 3H), 2.71 (s, 3H).

### (1-(1-Methoayisoquinolin-4-yl)ethanamine (VId)

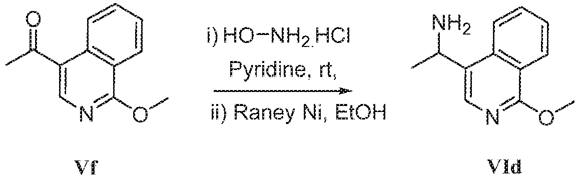

To a solution of 2.4 g (11.9 mmol, 1.0 eq.) of 1-(1-methoxyisoquinolin-4-yl)ethan-1-one **(Vf)** in 24 mL of pyridine was added 1.64 g (23.7 mmol, 2.0 eq.) of hydroxylamine hydrochloride and the mixture was stirred at room temperature for 4 h. The solvent was removed *in vacuo,* and the residue was triturated with n-pentane to provide 1.2 g of 1-(1-methoxyisoquinolin-4-yl)ethan-1-one oxime. LCMS: *m*/*z* found 217.3 [M+H]⁺. The residue was redissolved in 10 mL of ethanol and 1.0 g of wet Raney-Ni was added. The mixture was then stirred under a hydrogen atmosphere (1 atm) at room temperature for 16 h. The mixture was filtered through CELITE^{®} and the pad washed with 2 × 10 mL of ethanol. The solvent was removed *in vacuo* to provide 0.7 g of racemic 1-(1-methoxyisoquinolin-4-yl)ethanamine **(VId).** ¹H NMR (400 MHz, CDCl₃): δ 8.30 (d, 1H), 8.11 (s, 1H), 8.06 (d, 1H), 7.71 (t, 1H), 7.54 (t, 1H), 4.72 (q, 1H), 4.12 (s, 3H), 1.57 (d, 3H).

### 4-(1-Aminoethyl)isoquinolin-1(2H)-one (VIIId)

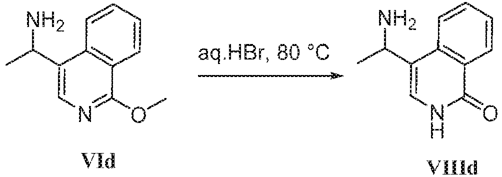

A solution of 0.5 g (2.47 mmol, 1.0 eq.) of 1-(1-methoxyisoquinolin-4-yl)ethan-1-amine **(VId)** in 5 mL of 47% aqueous HBr was heated at 80 °C for 6 h. The mixture was allowed to cool to room temperature and the solvent was removed *in vacuo.* The residue was dried under high vacuum to provide 0.5 g of 4-(1-aminoethyl)isoquinolin-1(2*H*)-one **(VIIId).**

### 1-(3-Chloro-4-fluorophenyl)-3-(1-(1-oxo-1,2-dihydroisoquinolin-4-yl)ethyl)urea

### (Compounds 10 & 11)

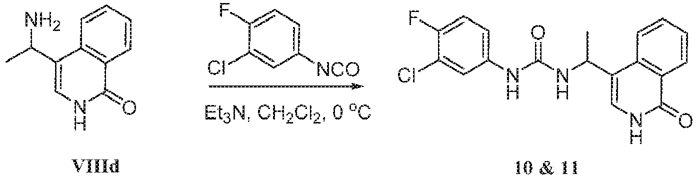

To a solution of 0.5 g (2.65 mmol, 1.0 eq.) of racemic 4-(1-aminoethyl)isoquinolin-1(2H)-one **(VIIId)** in 20 mL of methylene chloride at 0 °C was added 1.9 mL (13.2 mmol, 3.0 eq.) of triethyl amine followed by 0.27 g (1.59 mmol, 0.6 eq.) of 4-fluoro-3-chlorophenylisocyanate. The mixture was allowed to warm to room temperature, stirred for 1 h and the solvent was removed *in vacuo.* The residue was redissolved in 50 mL of methylene chloride and washed with 20 mL of brine, dried (Na₂SO₄), filtered and the solvent was removed *in vacuo.* The residue was purified by trituration with n-pentane to provide 0.51 g (1.42 mmol, 53%) of racemic 1-(3-chloro-4-fluorophenyl)-3-(1-(1-oxo-1,2-dihydroisoquinolin-4-yl)ethyl)urea. LCMS: *m*/*z* found 360.1/362.1 [M+H]⁺. The enantiomers were subsequently separated by SFC - Waters SFC-200, Column - Chiralpak IC (30 × 250 mm), 5µ, 75% CO₂:MeOH, Flow rate 100 g/min.

1-(3-Chloro-4-fluorophenyl)-3-(1-(1-oxo-1,2-dihydroisoquinolin-4-yl)ethyl)urea: Enantiomer I **(Compound 10)**, 92 mg obtained. LCMS: *m*/*z* found 360.2/362.2 [M+H]⁺, RT = 3.72 min (Method A); ¹H NMR (400 MHz, DMSO-d₆): δ 11.25 (d, 1H), 8.53 (s, 1H), 8.25 (d, 1H), 7.76-7.79 (m, 3H), 7.50-7.54 (m, 1H), 7.27 (t, 1H), 7.18-7.22 (m, 1H), 7.11 (d, 1H), 6.62 (d, 1H), 5.17 (t, 1H), 1.47 (d, 3H); Chiral analytical SFC, RT = 2.18 min, Column: Chiralpak IC (150 × 4.6 mm) 3 µ, 70% CO₂:MeOH, Flow rate = 3.0 g/min.

1-(3-Chloro-4-fluorophenyl)-3-(1-(1-oxo-1,2-dihydroisoquinolin-4-yl)ethyl)urea: Enantiomer II **(Compound 11)**, 93 mg obtained. LCMS: *m*/*z* found 360.2/362.2 [M+H]⁺, RT = 3.72 min (Method A); ¹H NMR (400 MHz, DMSO-d₆): δ 11.22 (d, 1H), 8.53 (s, 1H), 8.25 (d, 1H), 7.76-7.79 (m, 3H), 7.50-7.54 (m, 1H), 7.27 (t, 1H), 7.18-7.22 (m, 1H), 7.11 (d, 1H), 6.62 (d, 1H), 5.17 (t, 1H), 1.47 (d, 3H); Chiral analytical SFC, RT = 3.15 min, Column: Chiralpak IC (150 × 4.6 mm) 3 µ, 70% CO₂:MeOH, Flow rate = 3.0 g/min.

### tert-Butyl (1-(1-methoxyisoquinolin-4-yl)ethyl)carbamate (VIe)

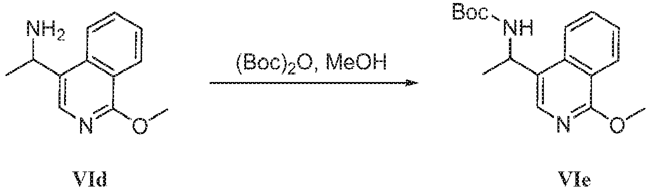

To a solution of 0.5 g (2.46 mmol, 1.0 eq.) of 1-(1-methoxyisoquinolin-4-yl)ethan-1-one **(VId)** in 5 mL of methanol was added 0.54 g (2.46 mmol, 1.0 eq.) of di-*tert*-butyl dicarbonate and the mixture was stirred at room temperature for 16 h. The solvent was removed *in vacuo* and the residue was redissolved in 50 mL of ethyl acetate. The organic solution was washed with 20 mL of brine, dried (Na₂SO₄), filtered and solvent was removed *in vacuo* to provide 0.40 g (1.32 mmol, 54%) of *tert*-butyl (1-(1-methoxyisoquinolin-4-yl)ethyl)carbamate **(VIe).** ¹H NMR (400 MHz, CDCl₃): δ 8.29 (d, 1H), 7.97-8.01 (m, 2H), 7.72 (t, 1H), 7.56 (t, 1H), 5.41 (brs, 1H), 4.75-4.80 (m, 1H), 4.12 (s, 3H), 1.63 (d, 3H), 1.44 (s, 9H).

### 1-(1-Methoxyisoquinolin-4-yl)-N-methylethan-1-amine (VIf)

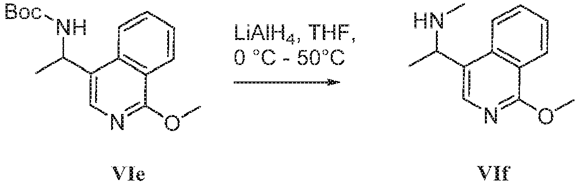

To a solution of 0.40 g (1.32 mmol, 1.0 eq.) of *tert*-butyl (1-(1-methoxyisoquinolin-4-yl)ethyl)carbamate **(VIe)** in 10 mL of THF at 0 °C under a nitrogen atmosphere was added 0.2 g (5.28 mmol, 4.0 eq.) of lithium aluminum hydride. The reaction mixture was then heated to 50 °C for 16 h. The mixture was cooled to 0 °C and slowly quenched with aqueous sodium sulfate solution and filtered through CELITE^{®}. The pad was washed with 2 × 10 mL of THF and the solvent was removed *in vacuo* to provide 0.20 g (0.92 mmol, 70%) of 1-(1-methoxyisoquinolin-4-yl)-*N*-methylethan-1-amine **(VIf).** LCMS: *m*/*z* found 186.0 [M-NHMe]⁺; ¹H NMR (400 MHz, CDCl₃): δ 8.30 (d, 1H), 8.15 (d, 1H), 8.06 (s, 1H), 7.69 (t, 1H), 7.53 (t, 1H), 4.26 (q, 1H), 4.12 (s, 3H), 2.41 (s, 3H), 1.52 (d, 3H).

### 4-(1-(Methylamino)ethyl)isoquinolin-1(2H)-one (VIIIf)

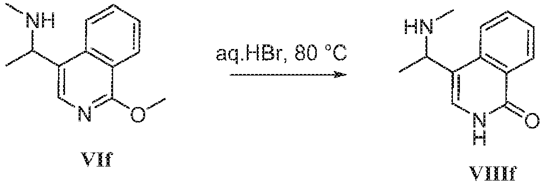

A solution of 0.20 g (0.92 mmol, 1.0 eq.) of 1-(1-methoxyisoquinolin-4-yl)-*N-*methylethan-1-amine **(VIf)** in 2.5 mL of 47% aqueous hydrobromic acid was heated at 80 °C for 6 h. The mixture was allowed to cool to room temperature and was evaporated to dryness to provide 0.35 g of crude of 4-(1-(methylamino)ethyl)isoquinolin-1(2*H*)-one hydrobromide **(VIIIf).** LCMS: *m*/*z* found 172.16 [M-NHMe]⁺.

### 3-(3-Chloro-4-fluorophenyl)-1-methyl-1-(1-(1-oxo-1,2-dihydroisoquinolin-4-yl)ethyl)urea (Compounds 12, 13 & 14)

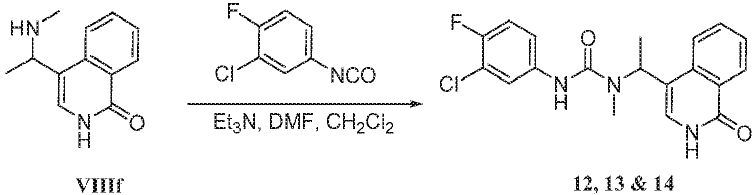

To a solution of 0.35 g of crude 4-(1-(methylamino)ethyl)isoquinolin-1(2*H*)-one hydrobromide **(VIIIf)** in 10 mL of 1:1 (v/v) methylene chloride:DMF at 0 °C was added 1.2 mL (8.7 mmol) of triethylamine followed by 0.30 g (1.73 mmol) of 4-fluoro-3-chlorophenylisocyanate. The mixture was allowed to warm to room temperature and stirred for 2 h. The solvent was removed *in vacuo* and the residue was redissolved in 100 mL of ethyl acetate. The organic solution was washed 50 mL of brine, dried (Na₂SO₄), filtered and the solvent was removed *in vacuo.* The residue was purified by semi-preparative HPLC to provide 90 mg of racemic 1-(3-chloro-4-fluorophenyl)-3-(1-(1-oxo-1,2-dihydroisoquinolin-4-yl)ethyl)urea **(Compound 12).** LCMS: *m*/*z* found 374.1/376.1 [M+H]⁺. The enantiomers were subsequently separated by SFC (Waters SFC-80), Column Chiralcel OD-H (21 × 250 mm), 5 µ, 85% CO₂:MeOH, Flow rate 70 g/min.

3-(3-Chloro-4-fluorophenyl)-1-methyl-1-(1-(1-oxo-1,2-dihydroisoquinolin-4-yl)ethyl)urea: Enantiomer I **(Compound 13).** LCMS: *m*/*z* found 374.2/376.2 [M+H]⁺, RT = 3.83 min (Method A); ¹H NMR (400 MHz, DMSO-d₆): δ 11.45 (bs, 1H), 8.48 (bs, 1H), 8.24 (d, 1H), 7.87 (dd, 1H), 7.69-7.73 (m, 2H), 7.47-7.53 (m, 2H), 7.31 (t, 1H), 7.16 (s, 1H), 5.85 (q, 1H), 2.58 (s, 3H), 1.43 (d, 3H); Chiral analytical SFC, RT = 4.13 min, Column: Chiralcel OD-H (250 × 4.6 mm) 5 µ, 70% CO₂:MeOH, Flow rate = 3.0 g/min.

3-(3-Chloro-4-fluorophenyl)-1-methyl-1-(1-(1-oxo-1,2-dihydroisoquinolin-4-yl)ethyl)urea: Enantiomer II **(Compound 14).** LCMS: *m*/*z* found 374.2/376.2 [M+H]⁺, RT = 3.83 min (Method A); ¹H NMR (400 MHz, DMSO-d₆): δ 11.45 (bs, 1H), 8.48 (bs, 1H), 8.24 (d, 1H), 7.87 (dd, 1H), 7.69-7.73 (m, 2H), 7.47-7.53 (m, 2H), 7.31 (t, 1H), 7.16 (s, 1H), 5.85 (q, 1H), 2.58 (s, 3H), 1.43 (d, 3H); Chiral analytical SFC, RT = 5.58 min, Column: Chiralcel OD-H (250 × 4.6 mm) 5 µ, 70% CO₂:MeOH, Flow rate = 3.0 g/min.

### 1,3-Dimethyl-1-(1-(1-oxo-1,2-dihydroisoquinolin-4-yl)ethyl)urea (Compound 298)

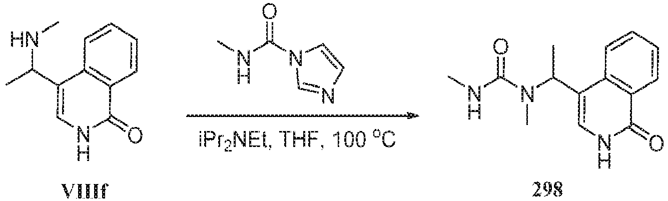

To a solution of 0.3g (1.48 mmol, 1.0 eq.) of 4-(1-(methylamino)ethyl)isoquinolin-1(2H)-one **(VIIIf)** in 3 mL of THF in a sealed tube under a nitrogen atmosphere was added 0.8 mL (4.45 mmol, 3.0 eq.) of N,N-diisopropylethylamine followed by 0.28 g (2.22 mmol, 1.5 eq.) of *N*-methyl-1*H*-imidazole-1-carboxamide and the mixture was heated at 100 °C for 16 h. The mixture was allowed to cool to room temperature, diluted with 20 mL of ice-cold water and extracted with 3 × 100 mL of ethyl acetate. The combined organic extracts were washed with 80 mL of brine, dried (Na₂SO₄), filtered and the solvent was removed *in vacuo.* The residue was purified by semi-preparative HPLC to provide 120 mg (0.46 mmol, 31%) of racemic 1,3-dimethyl-1-(1-(1-oxo-1,2-dihydroisoquinolin-4-yl)ethyl)urea. LCMS: *m*/*z* found 260.0 [M+H]⁺. The enantiomers were subsequently separated by SFC (Waters SFC-80), Column Chiralcel OD-H (21 × 250 mm), 5 µ, 75% CO₂:MeOH, Flow rate 70 g/min.

1,3-Dimethyl-1-(1-(1-oxo-1,2-dihydroisoquinolin-4-yl)ethyl)urea: Enantiomer I **(Compound 298).** LCMS: *m*/*z* found 260.2 [M+H]⁺, RT = 1.64 min (Method A); ¹H NMR (400 MHz, DMSO-d₆) δ 11.30 (bs, 1H), 8.21-8.23 (m, 1H), 7.75 (d, 1H), 7.67-7.72 (m, 1H), 7.46-7.51 (m, 1H), 7.07 (s, 1H), 6.22 (m, 1H), 5.75-5.80 (m, 1H), 2.65 (d, 3H), 2.37 (s, 3H), 1.32 (d, 3H); Chiral analytical SFC, RT = 2.98 min, Column: Chiralcel OD-H (250 × 4.6 mm) 5 µ, 75% CO₂:MeOH, Flow rate = 3.0 g/min.

1,3-Dimethyl-1-(1-(1-oxo-1,2-dihydroisoquinolin-4-yl)ethyl)urea: Enantiomer II. LCMS: *m*/*z* found 260.2 [M+H]⁺, RT = 1.64 min (Method A); ¹H NMR (400 MHz, DMSO-d₆) δ 11.30 (bs, 1H), 8.21-8.23 (m, 1H), 7.75 (d, 1H), 7.67-7.72 (m, 1H), 7.46-7.51 (m, 1H), 7.07 (s, 1H), 6.22 (m, 1H), 5.75-5.80 (m, 1H), 2.65 (d, 3H), 2.37 (s, 3H), 1.32 (d, 3H); Chiral analytical SFC, RT = 4.28 min, Column: Chiralcel OD-H (250 × 4.6 mm) 5 µ, 75% CO₂:MeOH, Flow rate = 3.0 g/min.

### 3-Cyclopentyl-1-methyl-1-(1-(1-oxo-1,2-dihydroisoquinolin-4-yl)ethyl)urea (Compound 299)

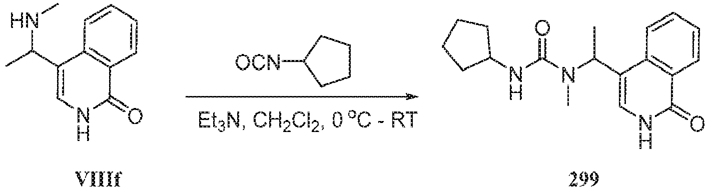

Racemic 3-cyclopentyl-1-methyl-1-(1-(1-oxo-1,2-dihydroisoquinolin-4-yl)ethyl)urea was synthesized in a similar manner as described above from 4-(1-(methylamino)ethyl) isoquinolin-1(2*H*)-one **(VIIIf)** and cyclopentyl isocyanate. The enantiomers were subsequently separated by SFC (Waters SFC-80), Column Chiralcel OX-H (21 × 250 mm), 5 µ, 75% CO₂:MeOH, Flow rate 70 g/min.

3-Cyclopentyl-1-methyl-1-(1-(1-oxo-1,2-dihydroisoquinolin-4-yl)ethyl)urea: Enantiomer I **(Compound 299).** LCMS: *m*/*z* found 314.1 [M+H]⁺, RT = 3.11 min (Method A); ¹H NMR (400 MHz, DMSO-d₆) δ 11.30 (bs, 1H), 8.22 (d, 1H), 7.75 (d, 1H), 7.65-7.69 (m, 1H), 7.45-7.51 (m, 1H), 7.06 (d, 1H), 5.95 (bd, 1H), 5.75-5.80 (m, 1H), 3.98-4.05 (m, 1H), 2.37 (s, 3H), 1.79-1.89 (m, 2H), 1.57-1.66 (m, 2H), 1.34-1.51 (m, 4H), 1.33 (d, 3H); Chiral analytical SFC, RT = 3.18 min, Column: Chiralcel OX-3 (150 × 4.6 mm) 3 µ, 70% CO₂:MeOH, Flow rate = 3.0 g/min.

3-Cyclopentyl-1-methyl-1-(1-(1-oxo-1,2-dihydroisoquinolin-4-yl)ethyl)urea: Enantiomer II. LCMS: *m*/*z* found 314.1 [M+H]⁺, RT = 3.11 min (Method A); ¹H NMR (400 MHz, DMSO-d₆) δ 11.30 (bs, 1H), 8.22 (d, 1H), 7.75 (d, 1H), 7.65-7.69 (m, 1H), 7.45-7.51 (m, 1H), 7.06 (d, 1H), 5.95 (bd, 1H), 5.75-5.80 (m, 1H), 3.98-4.05 (m, 1H), 2.37 (s, 3H), 1.79-1.89 (m, 2H), 1.57-1.66 (m, 2H), 1.34-1.51 (m, 4H), 1.33 (d, 3H); Chiral analytical SFC, RT = 6.07 min, Column: Chiralcel OX-3 (150 × 4.6 mm) 3 µ, 70% CO₂:MeOH, Flow rate = 3.0 g/min.

### 4-Acetylisoquinolin-1(2H)-one (XXa)

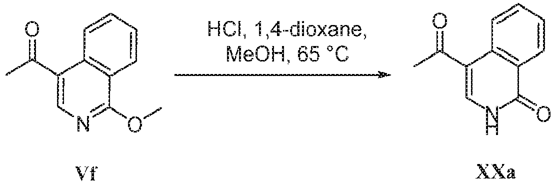

To a solution of 3.0 g (14.92 mmol, 1.0 eq.) of 1-(1-methoxyisoquinolin-4-yl)ethan-1-one **(Vf)** in 30 mL of methanol under a nitrogen atmosphere was added 15 mL of a 4 M solution of HCl in 1,4-dioxane. The mixture was then heated at 70 °C for 3 h. The mixture was allowed to cool to room temperature and the solvent was removed *in vacuo.* The residue triturated with diethyl ether and dried under high vacuum to provide 2.2 g (11.76 mmol, 78%) of 4-acetylisoquinolin-1(2*H*)-one **(XXa).** LCMS: *m*/*z* found 188.2 [M+H]⁺; ¹H NMR (300 MHz, DMSO-d₆): δ 12.00 (bs, 1H), 8.91 (d, 1H), 8.25 (d, 1H), 8.19 (d, 1H), 7.78 (t, 1H), 7.55 (t, 1H), 2.53 (s, 3H).

### 4-(1-((Cyclopentylmethyl)amino)ethyl)isoquinolin-1(2H)-one (VIIIb)

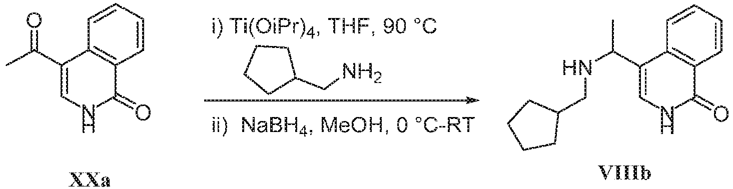

To a solution of 0.5 g (2.67 mmol, 1.0 eq.) of 4-acetylisoquinolin-1(2*H*)-one **(XXa)** in 5 mL of titanium (IV) isopropoxide under a nitrogen atmosphere was added a solution of 0.4 g (4.01 mmol 1.5 eq.) of cyclopentylmethanamine in 5 mL of anhydrous THF and the mixture was heated at 90 °C for 16 h. The mixture was allowed to cool to room temperature and then further cooled to 0 °C. The mixture was diluted with 2 mL of methanol and 0.20 g (5.34 mmol, 2.0 eq.) of sodium borohydride was added. The mixture was then allowed to warm to room temperature and stirred for 1 h. The reaction was quenched by the addition of 20 mL of water and filtered through CELITE^{®}. The pad was washed with 5 mL of ethyl acetate and the biphasic mixture was extracted with 2 × 30 mL of ethyl acetate. The combined organic extracts were dried (Na₂SO₄), filtered and the solvent was removed *in vacuo* to provide 0.6 g (2.21 mmol, 82%) of 4-(1-((cyclopentylmethyl)amino)ethyl)isoquinolin-1(2*B*)-one **(VIIIb).** LCMS: m/z found 271.1 [M+H]⁺; ¹H NMR (300 MHz, DMSO-d₆): δ 11.16 (bs, 1H), 8.21-8.25 (m, 1H), 8.02 (d, 1H), 7.67-7.73 (m, 1H), 7.45-7.49 (m, 1H), 7.15 (d, 1H), 3.99-4.03 (m, 1H), 2.40-2.43 (m, 1H), 2.29-2.33 (m, 1H), 1.87-1.96 (m, 2H), 1.39-1.71 (m, 6H), 1.32 (d, 3H), 1.12-1.19 (m, 2H).

### 3-(3-Chloro-4-fluorophenyl)-1-(cyclopentylmethyl)-1-(1-(1-oxo-1,2-dihydroisoquinolin-4-yl)ethyl)urea (Compounds 192 & 193)

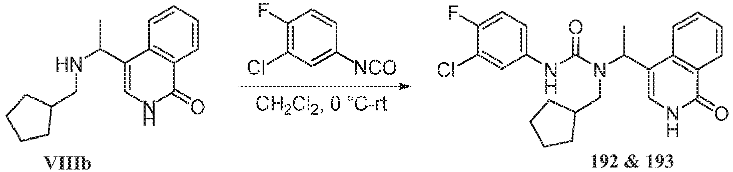

To a stirred solution of 0.6 g (2.21 mmol, 1.0 eq.) of 4-(1-((cyclopentylmethyl)amino) ethyl) isoquinolin-1(2*H*)-one **(VIIIb)** in 6 mL of methylene chloride at 0 °C under a nitrogen atmosphere was added 0.27 mL (2.22 mmol, 1.0 eq.) of 2-chloro-1-fluoro-4-isocyanatobenzene. The mixture was allowed to warm to room temperature and stirred for 1 h. The mixture was diluted with 10 mL of water and extracted with 2 × 20 mL of methylene chloride. The combined organic extracts were dried (Na₂SO₄), filtered and the solvent was removed *in vacuo.* The residue was redissolved in 5 mL of methanol and 0.61 g (4.44 mmol, 2.0 eq.) of potassium carbonate was added. The resulting mixture was stirred at room temperature for 2 h. The volatiles were removed *in vacuo* and the residue was suspended in 10 mL of water and extracted with 2 × 30 mL of ethyl acetate. The combined organic extracts were washed with 30 mL of brine, dried (Na₂SO₄), filtered and the solvent was removed *in vacuo.* The residue was purified by column chromatography (SiO₂, eluting with a linear gradient of 0-5% methanol in methylene chloride) to provide 0.30 g (0.68 mmol, 31%) of racemic 3-(3-chloro-4-fluorophenyl)-1-(cyclopentylmethyl)-1-(1-(1-oxo-1,2-dihydroisoquinolin-4-yl)ethyl)urea. LCMS: *m*/*z* found 442.39 [M+H]⁺. The enantiomers were subsequently separated by SFC (Waters SFC-80), Column Lux Cellulose-2 (30 × 250 mm) 5µ, 70% CO₂:MeOH, Flow rate 70 g/min to provide 92 mg and 89 mg of the resolved enantiomers.

3-(3-Chloro-4-fluorophenyl)-1-(cyclopentylmethyl)-1-(1-(1-oxo-1,2-dihydroisoquinolin-4-yl)ethyl)urea: Enantiomer I **(Compound 192).** LCMS: *m*/*z* found 442.3/444.3 [M+H]⁺, RT = 7.72 min (Method: A); ¹H NMR (400 MHz, DMSO-d₆): δ 11.39 (br s, 1H), 8.45 (br s, 1H), 8.24 (d, 1H), 7.84-7.73 (m, 3H), 7.52-7.47 (m, 2H), 7.32 (t, 1H), 7.22 (d, 1H), 5.81-5.84 (m, 1H), 2.99-3.11 (m, 2H), 1.64-1.69 (m, 1H), 1.46 (d, 3H), 1.33-1.42 (m, 2H), 0.99-1.29 (m, 5H), 0.66-0.72 (m, 1H); Chiral analytical SFC: RT = 7.07 min, Column: Lux Cellulose-2 (250 × 4.6 mm), 5 µ, 70% CO₂:MeOH, Flow rate = 3.0 mL/min.

3-(3-Chloro-4-fluorophenyl)-1-(cyclopentylmethyl)-1-(1-(1-oxo-1,2-dihydroisoquinolin-4-yl)ethyl)urea: Enantiomer II **(Compound 193).** LCMS: *m*/*z* found 442.3/444.3 [M+H]⁺, RT = 7.66 min (Method: A); ¹H NMR (400 MHz, DMSO-d₆): δ 11.39 (br s, 1H), 8.45 (br s, 1H), 8.24 (d, 1H), 7.84-7.73 (m, 3H), 7.52-7.47 (m, 2H), 7.32 (t, 1H), 7.22 (d, 1H), 5.81-5.84 (m, 1H), 2.99-3.11 (m, 2H), 1.64-1.69 (m, 1H), 1.46 (d, 3H), 1.33-1.42 (m, 2H), 0.99-1.29 (m, 5H), 0.66-0.72 (m, 1H); Chiral analytical SFC: RT = 11.35 min, Column: Lux Cellulose-2 (250 × 4.6 mm), 5 µ, 70% CO₂:MeOH, Flow rate = 3.0 mL/min.

### 4-(1-(Isobutylamino)ethyl)isoquinolin-1(2H)-one (VIIIe)

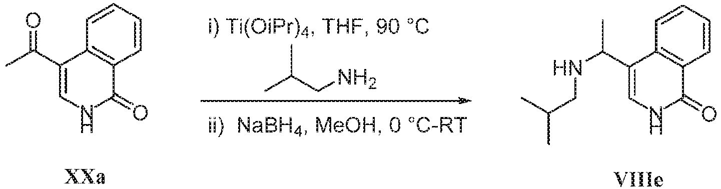

To a solution of 0.2 g (1.06 mmol, 1.0 eq.) of 4-acetylisoquinolin-1(2*H*)-one **(XXa)** in 5 mL of THF under a nitrogen atmosphere was added 0.12 g (1.6 mmol, 1.5 eq.) of isobutylamine followed by 2 mL of titanium (IV) isopropoxide and the mixture was heated to 90 °C for 6 h. The mixture was allowed to cool to room temperature and then further cooled to 0 °C. The mixture was then diluted with 2 mL of methanol and 0.08 g (2.13 mmol, 2.0 eq.) of sodium borohydride was added. The mixture was allowed to warm to room temperature and stirred for 2 h. The reaction was quenched by the addition of 20 mL of water and filtered through CELITE^{®}. The pad was washed with 5 mL of ethyl acetate and the biphasic mixture was extracted with 2 × 30 mL of ethyl acetate. The combined organic extracts were washed with 20 mL of brine, dried (Na₂SO₄), filtered and the solvent was removed *in vacuo* to provide 0.25 g (1.02 mmol, 96%) of 4-(1-(isobutylamino)ethyl)isoquinolin-1(2*H*)-one **(VIIIe).** LCMS: *m*/*z* found 245.1/247.1 [M+H]⁺; ¹H NMR (300 MHz, DMSO-d₆): δ 11.09 (br s 1H), 8.22-8.25 (m, 1H), 8.02 (d, 1H), 7.68-7.74 (m, 1H), 7.48 (t, 1H), 7.15 (d, 1H), 3.99-4.06 (m, 2H), 2.31-2.37 (m, 1H), 2.16-2.22 (m, 1H), 1.58-1.69 (m, 1H), 1.33 (d, 3H), 0.82-0.87 (m, 6H).

### 3-(3-Chloro-4-fluorophenyl)-1-isobutyl-1-(1-(1-oxo-1,2-dihydroisoquinolin-4-yl)ethyl)urea (Compounds 135, 143 & 144)

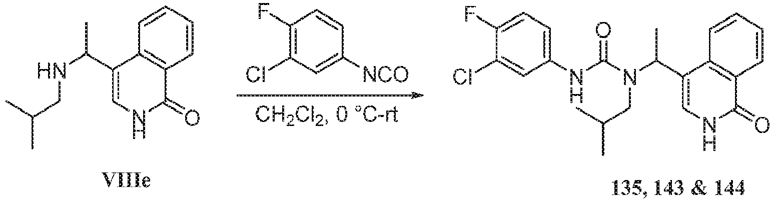

To a stirred solution of 0.25 g (1.02 mmol, 1.0 eq.) of racemic 4-(1-(isobutylamino) ethyl)isoquinolin-1(2H)-one **(VIIIe)** in 10 mL of methylene chloride at 0 °C under a nitrogen atmosphere was added 0.18 g (1.02 mmol, 1.0 eq.) of 2-chloro-1-fluoro-4-isocyanatobenzene and the mixture was stirred at 0 °C for 2 h. The mixture was diluted with 10 mL of water and extracted with 2 × 30 mL of ethyl acetate. The combined organic extracts were washed with 30 mL of brine, dried (Na₂SO₄), filtered and the solvent was removed *in vacuo.* The residue was purified by trituration with 25 mL of methyl *tert*-butyl ether to provide 0.37 g (0.85 mmol, 83%) of racemic 3-(3-chloro-4-fluorophenyl)-1-isobutyl-1-(1-(1-oxo-1,2-dihydroisoquinolin-4-yl)ethyl)urea **(Compound 135).** LCMS: *m*/*z* found 416.1/418.1 [M+H]⁺. The enantiomers were subsequently separated by SFC (Waters SFC-80), Column Lux Cellulose-2 (30 × 250 mm) 5 µ, 65% CO₂:MeOH, Flow rate 70 g/min to provide 86 mg and 90 mg of the resolved enantiomers.

3-(3-chloro-4-fluorophenyl)-1-isobutyl-1-(1-(1-oxo-1,2-dihydroisoquinolin-4-yl)ethyl)urea: Enantiomer I **(Compound 143).** LCMS: *m*/*z* found 416.3/418.3 [M+H]⁺; RT = 6.91 min, (Method A); ¹H NMR (400 MHz, DMSO-*d₆*): δ 11.40 (bd, 1H), 8.40 (bs, 1H), 8.23 (d, 1H), 7.73-7.83 (m, 3H), 7.47-7.52 (m, 2H), 7.32 (t, 1H), 7.22 (d, 1H), 5.83-5.88 (m, 1H), 2.89-2.93 (m, 2H), 1.47 (d, 3H), 1.32-1.38 (m, 1H), 0.61 (d, 3H), 0.48 (d, 3H); Chiral analytical SFC: RT = 1.09 min, Column: CHIRALCEL OZ-3 (150 mm x 4.6 mm) 3 µm; 60% CO₂:MeOH); Flow rate = 3.0 mL/min.

3-(3-chloro-4-fluorophenyl)-1-isobutyl-1-(1-(1-oxo-1,2-dihydroisoquinolin-4-yl)ethyl)urea: Enantiomer II **(Compound 144).** LCMS: *m*/*z* found 416.3/418.3 [M+H]⁺; RT = 6.91 min, (Method A); ¹H NMR (400 MHz, DMSO-d₆): δ 11.40 (bd, 1H), 8.40 (bs, 1H), 8.23 (d, 1H), 7.73-7.83 (m, 3H), 7.47-7.52 (m, 2H), 7.32 (t, 1H), 7.22 (d, 1H), 5.83-5.88 (m, 1H), 2.89-2.93 (m, 2H), 1.47 (d, 3H), 1.32-1.38 (m, 1H), 0.61 (d, 3H), 0.48 (d, 3H); Chiral analytical SFC: RT = 1.49 min, Column: CHIRALCEL OZ-3 (150 mm × 4.6 mm) 3 µm; 60% CO₂:MeOH); Flow rate = 3.0 g/min.

### N-Ethyl-1-(1-methoxyisoquinolin-4-yl)ethan-1-amine (VIw)

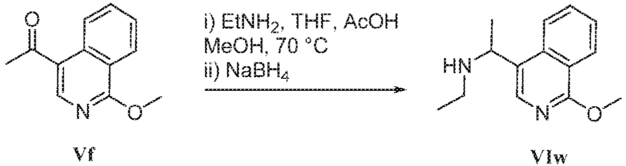

To a solution of 1.2 g (6.0 mmol, 1.0 eq.) of 1-(1-methoxyisoquinolin-4-yl)ethan-1-one **(Vf)** in 12 mL of methanol in a seal tube was added 15 mL (30.0 mmol, 5.0 eq.) of a 2 M solution of ethyl amine in THF followed by 0.06 mL (1.19 mmol, 0.2 eq.) of acetic acid. The mixture was then heated at 70 °C for 24 h. The mixture was allowed to cool to room temperature and 0.43 g (11.9 mmol, 2.0 eq.) of sodium borohydride was added. Stirring was continued for a further 2 h and the solvent was removed *in vacuo.* The residue was diluted with 70 mL of ice-cold water and extracted with 3 × 70 mL of ethyl acetate. The combined organic extracts were washed with 50 mL of water, 50 mL of brine, dried (Na₂SO₄), filtered and the solvent was removed *in vacuo.* The residue was purified by chromatography (neutral alumina, eluting with a linear gradient of 0-10% ethyl acetate/hexanes) to provide 0.78 g (3.39 mmol, 57%) of N-ethyl-1-(1-methoxyisoquinolin-4-yl)ethan-1-amine **(VIw).** LCMS: *m*/*z* found 231.2 [M+H]⁺; ¹H NMR (400 MHz, CDCl₃) δ 8.30 (d, 1H), 8.15 (d, 1H), 8.09 (s, 1H), 7.69 (t, 1H), 7.54 (t, 1H), 4.41 (q, 1H), 4.12 (s, 3H), 2.64 (q, 2H), 1.53 (d, 3H), 1.12 (t, 3H).

### 3-(3-Chloro-4-fluorophenyl)-1-ethyl-1-(1-(1-methoxyisoquinolin-4-yl)ethyl)urea (Compounds 22 & 23)

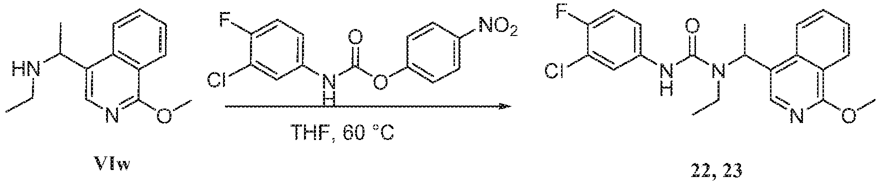

To a solution of 0.7 g (3.04 mmol, 1.0 eq.) of *N*-ethyl-1-(1-methoxyisoquinolin-4-yl)ethan-1-amine **(VIw)** in 7 mL of anhydrous THF was added 0.94 g (6.08 mmol, 2.0 eq.) of 4-nitrophenyl (3-chloro-4-fluorophenyl)carbamate and the mixture was heated at 60 °C for 16 h. The mixture was then allowed to cool to room temperature and the solvent was removed *in vacuo.* The residue was redissolved in 100 mL of ethyl acetate and washed with 50 mL of water, 50 mL of brine, dried (Na₂SO₄), filtered and the solvent was removed *in vacuo.* The residue product was purified by semi-preparative HPLC to provide 0.39 g (0.98 mmol, 32%) of racemic 3-(3-chloro-4-fluorophenyl)-1-ethyl-1-(1-(1-methoxyisoquinolin-4-yl)ethyl)urea. LCMS: *m*/*z* found 402.2/404.2 [M+H]⁺. The enantiomers were subsequently separated by SFC (Waters SFC-80), Column: Chiralpak IC (30 × 250 mm) 5µ, 85% CO₂:MeOH, flow rate 100 g/min to provide 110 mg and 105 mg of the resolved enantiomers.

3-(3-Chloro-4-fluorophenyl)-1-ethyl-1-(1-(1-methoxyisoquinolin-4-yl)ethyl)urea: Enantiomer I **(Compound 22).** LCMS: *m*/*z* found 402.2/401.1 [M+H]⁺, RT = 5.34 min (Method: A); ¹H NMR (400 MHz, DMSO-d6): δ 8.39 (s, 1H), 8.23 (d, 1H), 8.16 (s, 1H), 7.99 (d, 1H), 7.88 (dd, 1H), 7.82 (t, 1H), 7.63 (t, 1H), 7.52-7.56 (m, 1H), 7.33 (t, 1H), 6.15 (q, 1H), 4.09 (s, 3H), 3.11-3.16 (m, 2H), 1.60 (d, 3H), 0.55 (t, 3H); Chiral analytical SFC: RT = 2.78 min, Column: Chiralpak IC (250 × 4.6 mm), 5 µ, 75% CO₂:MeOH, Flow rate = 3.0 mL/min.

3-(3-Chloro-4-fluorophenyl)-1-ethyl-1-(1-(1-methoxyisoquinolin-4-yl)ethyl)urea: Enantiomer II **(Compound 23).** LCMS: *m*/*z* found 402.2/401.1 [M+H]⁺, RT = 5.34 min (Method: A); ¹H NMR (400 MHz, DMSO-d6): δ 8.39 (s, 1H), 8.23 (d, 1H), 8.16 (s, 1H), 7.99 (d, 1H), 7.88 (dd, 1H), 7.82 (t, 1H), 7.63 (t, 1H), 7.52-7.56 (m, 1H), 7.33 (t, 1H), 6.15 (q, 1H), 4.09 (s, 3H), 3.11-3.16 (m, 2H), 1.60 (d, 3H), 0.55 (t, 3H); Chiral analytical SFC: RT = 3.84 min, Column: Chiralpak IC (250 × 4.6 mm), 5 µ, 75% CO₂:MeOH, Flow rate = 3.0 mL/min.

### 4-(1-(Ethylamino)ethyl)isoquinolin-1(2H)-one (VIIIaq)

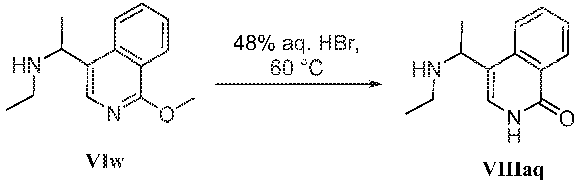

A solution of 0.78 g (3.39 mmol, 1.0 eq.) of *N*-ethyl-1-(1-methoxyisoquinolin-4-yl)ethan-1-amine **(VIw)** in 8 mL of 48% aqueous HBr was heated at 60 °C for 16 h. The mixture was allowed to cool to room temperature, quenched with 200 mL of saturated sodium bicarbonate solution and extracted with 3 × 75 mL of 10% methanol in methylene chloride. The combined organic extracts were washed with 50 mL of brine, dried (Na₂SO₄), filtered and the solvent was removed *in vacuo.* The residue was purified by trituration with *n-*pentane and diethyl ether to provide 0.68 g (3.15 mmol, 92%) of 4-(1-(ethylamino)ethyl)isoquinolin-1(2H)-one **(VIIIaq).** LCMS: *m*/*z* found 217.2 [M+H]⁺; ¹H NMR (300 MHz, DMSO-d₆) δ 11.19 (s, 1H), 8.23 (d, 1H), 8.01 (d, 1H), 7.71 (t, 1H), 7.48 (t, 1H), 7.15 (s, 1H), 4.05 (q, 1H), 2.42-2.47 (m, 2H), 1.92 (bs, 1H), 1.31 (d, 3H), 1.01 (t, 3H).

### 3-(3-Chloro-4-fluorophenyl)-1-ethyl-1-(1-(1-oxo-1,2-dihydroisoquinolin-4-yl)ethyl)urea (Compounds 24 & 25)

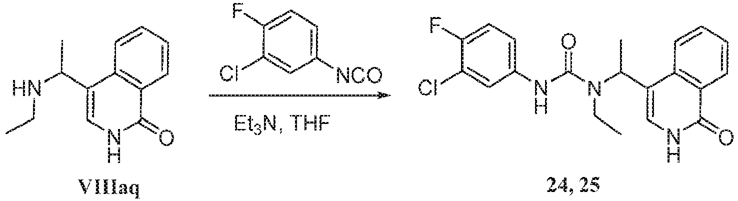

To a solution of 0.55 g (2.55 mmol, 1.0 eq.) of 4-(1-(ethylamino)ethyl)isoquinolin-1(2H)-one **(VIIIaq)** in 10 mL of THF at 0 °C was added 0.7 mL (5.09 mmol, 2.0 eq.) of triethylamine followed by 0.43 g (2.55 mmol, 1.0 eq.) of 2-chloro-1-fluoro-4-isocyanatobenzene. The mixture was allowed to warm to room temperature and stirred for 3 h. The solvent was removed *in vacuo* and the reside was redissolved in 100 mL of ethyl acetate, washed with 50 mL of water, 50 mL of brine, dried (Na₂SO₄), filtered and the solvent was removed *in vacuo.* The residue was purified by reverse-phase chromatography (C-18 column, eluting with a linear gradient of 10-40% [0.1% formic acid in water]/acetonitrile) to provide 0.33 g (0.84 mmol, 33%) of racemic 3-(3-chloro-4-fluorophenyl)-1-ethyl-1-(1-(1-oxo-1,2-dihydroisoquinolin-4-yl)ethyl)urea. LCMS: *m*/*z* found 388.2 [M+H]⁺. The enantiomers were subsequently separated by SFC (Waters SFC-80), Column: Chiralcel OD-H (30 × 250 mm) 5µ, 65% CO₂:MeOH, flow rate 90 g/min to provide 110 mg and 93 mg of the resolved enantiomers.

3-(3-Chloro-4-fluorophenyl)-1-ethyl-1-(1-(1-oxo-1,2-dihydroisoquinolin-4-yl)ethyl)urea: Enantiomer I **(Compound 24).** LCMS: *m*/*z* found 388.2/390.2 [M+H]⁺, RT = 4.05 min (Method: A); 1H NMR (400 MHz, DMSO-d₆): δ 11.41 (s, 1H), 8.36 (s, 1H), 8.24 (d, 1H), 7.87 (dd, 1H), 7.71-7.74 (m, 2H), 7.48-7.56 (m, 2H), 7.32 (t, 1H), 7.22 (d, 1H), 5.87 (q, 1H), 3.10-3.17 (m, 2H), 1.45 (d, 3H), 0.66 (t, 3H); Chiral analytical SFC: RT = 2.74 min, Column: Chiralcel OD-H (250 × 4.6 mm), 5 µ, 65% CO₂:MeOH, Flow rate = 3.0 mL/min.

3-(3-Chloro-4-fluorophenyl)-1-ethyl-1-(1-(1-oxo-1,2-dihydroisoquinolin-4-yl)ethyl)urea: Enantiomer II **(Compound 25).** LCMS: *m*/*z* found 388.2/390.2 [M+H]⁺, RT = 4.06 min (Method: A); 1H NMR (400 MHz, DMSO-d₆): δ 11.41 (s, 1H), 8.36 (s, 1H), 8.24 (d, 1H), 7.87 (dd, 1H), 7.71-7.74 (m, 2H), 7.48-7.56 (m, 2H), 7.32 (t, 1H), 7.22 (d, 1H), 5.87 (q, 1H), 3.10-3.17 (m, 2H), 1.45 (d, 3H), 0.66 (t, 3H); Chiral analytical SFC: RT = 3.91 min, Column: Chiralcel OD-H (250 × 4.6 mm), 5 µ, 65% CO₂:MeOH, Flow rate = 3.0 mL/min.

### N-(1-(1-Methoxyisoquinolin-4-yl)ethyl)cyclopropanamine (VIx)

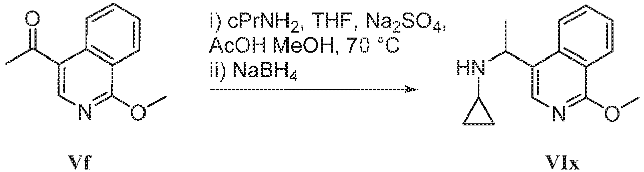

To a solution of 0.2 g (1.0 mmol, 1.0 eq.) of 1-(1-methoxyisoquinolin-4-yl)ethan-1-one **(Vf)** in 5 mL of methanol in a seal tube was added 0.17 g (3.0 mmol, 3.0 eq.) of cyclopropyl amine followed by 0.01 mL (0.2 mmol, 0.2 eq.) of acetic acid and 0.2 g of sodium sulfate. The mixture was then heated at 50 °C for 16 h. The mixture was allowed to cool to room temperature and 0.08 g (2.0 mmol, 2.0 eq.) of sodium borohydride was added. Stirring was continued for a further 30 min and the solvent was removed *in vacuo.* The residue was diluted with 20 mL of ice-cold water and extracted with 3 × 40 mL of ethyl acetate. The combined organic extracts were washed with 50 mL of water, 50 mL of brine, dried (Na₂SO₄), filtered and the solvent was removed *in vacuo* to provide 0.2 g (0.82 mmol, 82%) of *N*-(1-(1-methoxyisoquinolin-4-yl)ethyl) cyclopropanamine **(VIx).** LCMS: *m*/*z* found 243.5 [M+H]⁺; ¹H NMR (400 MHz, CDCl₃) δ 8.30 (d, 1H), 8.17 (d, 1H), 8.05 (s, 1H), 7.66-7.70 (m, 1H), 7.51-7.55 (m, 1H), 4.46-4.51 (q, 1H), 4.12 (s, 3H), 2.09-2.14 (m, 1H), 1.68 (bs, 1H), 1.55 (d, 3H), 0.30-0.41 (m, 4H).

### 3-(3-Chloro-4-fluorophenyl)-1-cyclopropyl-1-(1-(1-methoxyisoquinolin-4-yl)ethyl)urea (Compounds 56 & 57)

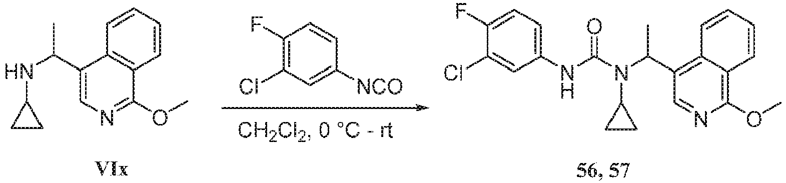

To a stirred solution of 0.2 g (0.82 mmol, 1.0 eq) of *N*-(1-(1-methoxyisoquinolin-4-yl)ethyl)cyclopropanamine **(VIx)** in 5 mL of methylene chloride at 0 °C was added 0.14 g (0.82 mmol, 1.0 eq.) of 2-chloro-1-fluoro-4-isocyanatobenzene. The mixture was allowed to warm to room temperature and stirred for 16 h. The solvent was removed *in vacuo* and the residue was purified by reverse-phase chromatography (Column: REVELERIS^{®} C18, eluting with a linear gradient of 10-70% [0.1 % formic acid in acetonitrile]/[0.1 % formic acid in water]) to provide 0.64 g (0.64 mmol, 77%) of racemic 3-(3-chloro-4-fluorophenyl)-1-cyclopropyl-1-(1-(1-methoxyisoquinolin-4-yl)ethyl)urea. LCMS: *m*/*z* found 414.5/416.5 [M+H]⁺. The enantiomers were subsequently separated by SFC (Waters SFC-80), Column: Chiralpak IG (30 × 250 mm) 5µ, 85% CO₂:MeOH, flow rate 100 g/min to provide 40 mg and 69 mg of the resolved enantiomers.

3-(3-Chloro-4-fluorophenyl)-1-cyclopropyl-1-(1-(1-methoxyisoquinolin-4-yl)ethyl)urea: Enantiomer I **(Compound 56).** LCMS: *m*/*z* found 414.2/416.1 [M+H]⁺, RT = 5.61 min (Method: A); 1H NMR (400 MHz, DMSO-d₆): δ 8.51 (bs, 1H), 8.24 (d, 1H), 8.15 (s, 1H), 7.87-7.92 (dd, 2H), 7.79-7.85 (m, 1H), 7.60-7.66 (m, 1H), 7.52-7.57 (m, 1H), 7.33 (t, 1H), 5.94 (q, 1H), 4.09 (s, 3H), 1.95-1.98 (m, 1H), 1.70 (d, 3H), 0.57-0.73 (m, 3H), 0.11-0.16 (m, 1H); Chiral analytical SFC: RT = 5.55 min, Column: Chiralpak IG (250 × 4.6 mm), 5 µ, 75% CO₂:MeOH, Flow rate = 3.0 mL/min.

3-(3-Chloro-4-fluorophenyl)-1-cyclopropyl-1-(1-(1-methoxyisoquinolin-4-yl)ethyl)urea: Enantiomer II **(Compound 57).** LCMS: *m*/*z* found 414.2/416.1 [M+H]⁺, RT = 5.61 min (Method: A); 1H NMR (400 MHz, DMSO-d₆): δ 8.51 (bs, 1H), 8.24 (d, 1H), 8.15 (s, 1H), 7.87-7.92 (dd, 2H), 7.79-7.85 (m, 1H), 7.60-7.66 (m, 1H), 7.52-7.57 (m, 1H), 7.33 (t, 1H), 5.94 (q, 1H), 4.09 (s, 3H), 1.95-1.98 (m, 1H), 1.70 (d, 3H), 0.57-0.73 (m, 3H), 0.11-0.16 (m, 1H); Chiral analytical SFC: RT = 7.27 min, Column: Chiralpak IG (250 × 4.6 mm), 5 µ, 75% CO₂:MeOH, Flow rate = 3.0 mL/min.

### 4-(1-(Cyclopropylamino)ethyl)isoquinolin-1(2H)-one (VIIIar)

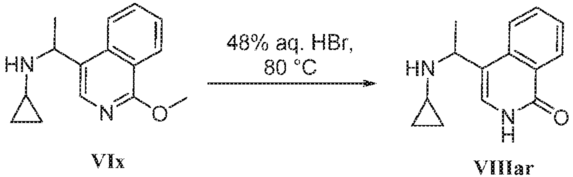

A solution of 0.40 g (1.65 mmol, 1.0 eq.) of *N*-(1-(1-methoxyisoquinolin-4-yl)ethyl) cyclopropanamine **(VIx)** in 10 mL of 48% aqueous HBr was heated at 80 °C for 4 h. The mixture was allowed to cool to room temperature, quenched with 20 mL of saturated sodium bicarbonate solution and extracted with 3 × 40 mL of 10% methanol in methylene chloride. The combined organic extracts were washed with 40 mL of brine, dried (Na₂SO₄), filtered and the solvent was removed *in vacuo.* The residue was purified by trituration with n-pentane to provide 0.35 of 4-(1-(cyclopropylamino)ethyl)isoquinolin-1(2*H*)-one **(VIIIar).** LCMS: *m*/*z* found 229.0 [M+H]⁺; ¹H NMR (300 MHz, DMSO-d₆) δ 9.91 (bs, 1H), 8.47 (dd, 1H), 7.92 (d, 1H), 7.69-7.74 (m, 1H), 7.49-7.54 (m, 1H), 7.25 (d, 1H), 4.26-4.32 (m, 1H), 2.10-2.18 (m, 1H), 1.67 (bs, 1H), 1.48 (d, 3H), 0.32-0.46 (m, 4H).

### 3-(3-Chloro-4-fluorophenyl)-1-cyclopropyl-1-(1-(1-oxo-1,2-dihydroisoquinolin-4-yl)ethyl)urea (Compounds 67 & 68)

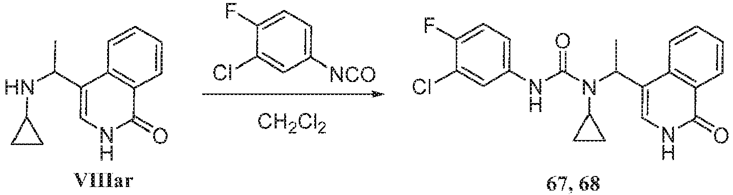

To a solution of 0.35 g of 4-(1-(cyclopropylamino)ethyl)isoquinolin-1(2*H*)-one **(VIIIar)** in 10 mL of methylene chloride was added 0.24 g (1.4 mmol, 0.9 eq.) of 2-chloro-1-fluoro-4-isocyanatobenzene and the mixture was stirred at room temperature for 3 h. The solvent was removed *in vacuo* and the reside purified by semi-preparative HPLC to provide 0.32 g (0.80 mmol, 48% from **VIx**) of racemic 3-(3-chloro-4-fluorophenyl)-1-cyclopropyl-1-(1-(1-oxo-1,2-dihydroisoquinolin-4-yl)ethyl)urea. LCMS: *m*/*z* found 400.2/402.2 [M+H]⁺. The enantiomers were subsequently separated by SFC (Waters SFC-80), Column: Chiralpak AD-H (30 × 250 mm) 5µ, 90% CO₂:MeOH, flow rate 90 g/min to provide 121 mg and 78 mg of the resolved enantiomers.

3-(3-Chloro-4-fluorophenyl)-1-cyclopropyl-1-(1-(1-oxo-1,2-dihydroisoquinolin-4-yl)ethyl)urea: Enantiomer I **(Compound 67).** LCMS: *m*/*z* found 400.2/402.2 [M+H]⁺, RT = 5.28 min (Method: A); ¹H NMR (400 MHz, DMSO-d6): δ 11.37 (d, 1H), 8.49 (s, 1H), 8.24-8.27 (m, 1H), 7.88-7.91 (m, 1H), 7.73-7.77 (m, 1H), 7.63 (d, 1H), 7.48-7.56 (m, 2H), 7.30-7.35 (m, 1H), 7.18-7.19 (m, 1H), 5.63-5.69 (m, 1H), 1.98-2.02 (m, 1H), 1.55 (d, 3H), 0.66-0.71 (m, 3H), 0.23-0.26 (m, 1H); Chiral analytical SFC: RT = 3.18 min, Column: Chiralpak AD-3 (250 × 4.6 mm), 5 µ, 80% CO₂:MeOH, Flow rate = 3.0 mL/min.

3-(3-Chloro-4-fluorophenyl)-1-cyclopropyl-1-(1-(1-oxo-1,2-dihydroisoquinolin-4-yl)ethyl)urea: Enantiomer II **(Compound 68).** LCMS: *m*/*z* found 400.2/402.2 [M+H]⁺, RT = 5.28 min (Method: A); ¹H NMR (400 MHz, DMSO-d6): δ 11.37 (d, 1H), 8.49 (s, 1H), 8.24-8.27 (m, 1H), 7.88-7.91 (m, 1H), 7.73-7.77 (m, 1H), 7.63 (d, 1H), 7.48-7.56 (m, 2H), 7.30-7.35 (m, 1H), 7.18-7.19 (m, 1H), 5.63-5.69 (m, 1H), 1.98-2.02 (m, 1H), 1.55 (d, 3H), 0.66-0.71 (m, 3H), 0.23-0.26 (m, 1H); Chiral analytical SFC: RT = 4.69 min, Column: Chiralpak AD-3 (250 × 4.6 mm), 5 µ, 80% CO₂:MeOH, Flow rate = 3.0 mL/min.

### N-(Cyclopropylmethyl)-1-(1-methoayisoquinolin-4-yl)ethan-1-amine (VIy)

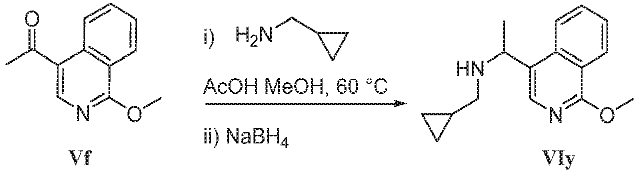

To a solution of 0.5 g (2.5 mmol, 1.0 eq.) of 1-(1-methoxyisoquinolin-4-yl)ethan-1-one **(Vf)** in 20 mL of methanol was added 0.53 g (3.0 mmol, 3.0 eq.) of cyclopropylmethyl amine followed by 0.03 g (0.5 mmol, 0.2 eq.) of acetic acid and the mixture was heated at 60 °C for 16 h. The mixture was allowed to cool to room temperature and 0.19 g (5.0 mmol, 2.0 eq.) of sodium borohydride was added. Stirring was continued for a further 30 min and the solvent was removed *in vacuo.* The residue was diluted with 20 mL of ice-cold water and extracted with 3 × 40 mL of ethyl acetate. The combined organic extracts were washed with 50 mL of water, 50 mL of brine, dried (Na₂SO₄), filtered and the solvent was removed *in vacuo.* The residue was purified by reverse phase chromatography (REVELERIS ^{®} C18 column, eluting with a linear gradient of 10-70% acetonitrile/water) to provide 0.4 g (1.56 mmol, 82%) of *N*-(cyclopropylmethyl)-1-(1-methoxyisoquinolin-4-yl)ethan-1-amine **(VIy).** LCMS: *m*/*z* found 257.5 [M+H]⁺.

### 3-(3-Chloro-4-fluorophenyl)-1-(cyclopropylmethyl)-1-(1-(1-methoxyisoquinolin-4-yl)ethyl)urea (Compounds 63 & 64)

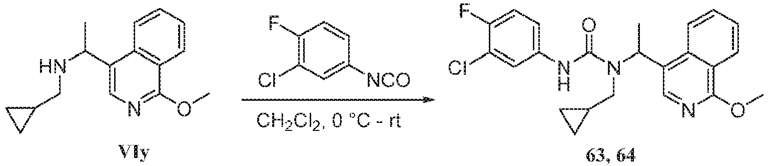

Racemic 3-(3-chloro-4-fluorophenyl)-1-(cyclopropylmethyl)-1-(1-(1-methoxyisoquinolin-4-yl)ethyl)urea was synthesized in a similar manner as described above from *N-*(cyclopropylmethyl)-1-(1-methoxyisoquinolin-4-yl)ethan-1-amine **(VIy)** and 2-chloro-1-fluoro-4-isocyanatobenzene. The enantiomers were subsequently separated by SFC, Column: Chiralpak IC (30 × 250 mm) 5 µ, 85% CO₂:MeOH, flow rate 70 g/min.

3-(3-Chloro-4-fluorophenyl)-1-(cyclopropylmethyl)-1-(1-(1-methoxyisoquinolin-4-yl)ethyl)urea: Enantiomer I **(Compound 63).** LCMS: *m*/*z* found 428.1/430.1 [M+H]⁺, RT = 6.22 min (Method: A); 1H NMR (400 MHz, DMSO-d₆): δ 8.51 (bs, 1H), 8.23 (d, 1H), 8.11 (s, 1H), 7.99 (d, 1H), 7.86-7.90 (m, 1H), 7.79-7.85 (m, 1H), 7.60-7.66 (m, 1H), 7.51-7.57 (m, 1H), 7.30-7.36 (m, 1H), 6.07-6.15 (m, 1H), 4.08 (s, 3H), 3.03 (dd, 1H), 2.88 (dd, 1H), 1.63 (d, 3H), 0.49-0.56 (m, 1H), 0.15-0.23 (m, 1H), 0.01-0.041 (m, 1H), -0.14 - -0.19 (m, 1H), - 0.62 - -0.67 (m, 1H); Chiral analytical SFC: RT = 5.55 min, Column: Chiralpak IC (250 x 4.6 mm), 5 µ, 80% CO₂:MeOH, Flow rate = 3.0 mL/min.

3-(3-Chloro-4-fluorophenyl)-1-(cyclopropylmethyl)-1-(1-(1-methoxyisoquinolin-4-yl)ethyl)urea: Enantiomer II **(Compound 64).** LCMS: *m*/*z* found 428.2/430.2 [M+H]⁺, RT = 6.25 min (Method: A); 1H NMR (400 MHz, DMSO-d₆): δ 8.51 (bs, 1H), 8.23 (d, 1H), 8.11 (s, 1H), 7.99 (d, 1H), 7.86-7.90 (m, 1H), 7.79-7.85 (m, 1H), 7.60-7.66 (m, 1H), 7.51-7.57 (m, 1H), 7.30-7.36 (m, 1H), 6.07-6.15 (m, 1H), 4.08 (s, 3H), 3.03 (dd, 1H), 2.88 (dd, 1H), 1.63 (d, 3H), 0.49-0.56 (m, 1H), 0.15-0.23 (m, 1H), 0.01-0.041 (m, 1H), -0.14 - -0.19 (m, 1H), - 0.62 - -0.67 (m, 1H); Chiral analytical SFC: RT = 7.37 min, Column: Chiralpak IC (250 × 4.6 mm), 5 µ, 80% CO₂:MeOH, Flow rate = 3.0 mL/min.

### 4-(1-((Cyclopropylmethyl)amino)ethyl)isoquinolin-1(2H)-one (Villas)

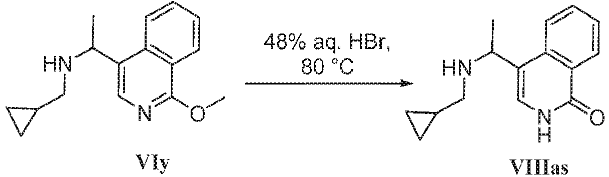

A solution of 0.30 g (1.17 mmol, 1.0 eq.) of *N*-(cyclopropylmethyl)-1-(1-methoxyisoquinolin-4-yl)ethan-1-amine **(VIy)** in 3 mL of 48% aqueous HBr was heated at 80 °C for 3 h. The mixture was allowed to cool to room temperature, quenched with 20 mL of saturated sodium bicarbonate solution and extracted with 3 × 40 mL of 10% methanol in methylene chloride. The combined organic extracts were washed with 40 mL of brine, dried (Na₂SO₄), filtered and the solvent was removed *in vacuo* to provide 0.28 of 4-(1-((cyclopropylmethyl)amino)ethyl)isoquinolin-1(2H)-one **(Villas).** LCMS: *m*/*z* found 243.4 [M+H]⁺.

### 3-(3-Chloro-4-fluorophenyl)-1-(cyclopropylmethyl)-1-(1-(1-oxo-1,2-dihydroisoquinolin-4-yl)ethyl)urea (Compounds 85 & 86)

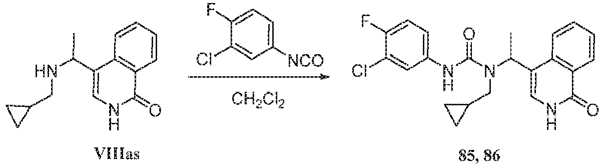

Racemic 3-(3-chloro-4-fluorophenyl)-1-(cyclopropylmethyl)-1-(1-(1-oxo-1,2-dihydroisoquinolin-4-yl)ethyl)urea was synthesized in a similar manner as described above from 4-(1-(ethylamino)ethyl)isoquinolin-1(2*H*)-one **(Villas)** and 2-chloro-1-fluoro-4-isocyanatobenzene. The enantiomers were subsequently separated by SFC, Column: Chiralcel OD-H (30 × 250 mm) 5 µ, 70% CO₂:MeOH, flow rate 60 g/min.

3-(3-Chloro-4-fluorophenyl)-1-(cyclopropylmethyl)-1-(1-(1-oxo-1,2-dihydroisoquinolin-4-yl)ethyl)urea: Enantiomer I **(Compound 85).** LCMS: *m*/*z* found 414.2/416.1 [M+H]⁺, RT = 5.56 min (Method: A); ¹H NMR (400 MHz, DMSO-d₆): δ 11.40 (d, 1H), 8.46 (bs, 1H), 8.23 (d, 1H), 7.85-7.88 (m, 1H), 7.72-7.75 (m, 2H), 7.46-7.55 (m, 2H), 7.33 (t, 1H), 7.17 (d, 1H), 5.82-5.85 (m, 1H), 3.01-3.07 (m, 1H), 2.88-2.94 (m, 1H), 1.47 (d, 3H), 0.54-0.60 (m, 1H), 0.19-0.25 (m, 1H), -0.02-0.08 (m, 1H), -0.02--0.08 (m, 1H), -0.34-0.38 (m, 1H); Chiral analytical SFC: RT = 1.76 min, Column: Chiralpak AD-3 (250 × 4.6 mm), 5 µ, 80% CO₂:MeOH, Flow rate = 3.0 mL/min.

3-(3-Chloro-4-fluorophenyl)-1-(cyclopropylmethyl)-1-(1-(1-oxo-1,2-dihydroisoquinolin-4-yl)ethyl)urea: Enantiomer II **(Compound 86).** LCMS: *m*/*z* found 414.2/416.1 [M+H]⁺, RT = 5.56 min (Method: A); ¹H NMR (400 MHz, DMSO-d₆): δ 11.40 (d, 1H), 8.46 (bs, 1H), 8.23 (d, 1H), 7.85-7.88 (m, 1H), 7.72-7.75 (m, 2H), 7.46-7.55 (m, 2H), 7.33 (t, 1H), 7.17 (d, 1H), 5.82-5.85 (m, 1H), 3.01-3.07 (m, 1H), 2.88-2.94 (m, 1H), 1.47 (d, 3H), 0.54-0.60 (m, 1H), 0.19-0.25 (m, 1H), -0.02-0.08 (m, 1H), -0.02--0.08 (m, 1H), -0.34-0.38 (m, 1H); Chiral analytical SFC: RT = 2.36 min, Column: Chiralpak AD-3 (250 × 4.6 mm), 5 µ, 80% CO₂:MeOH, Flow rate = 3.0 mL/min.

### N-(1-(1-Methoxyisoquinolin-4-yl)ethyl)butan-1-amine (VIz)

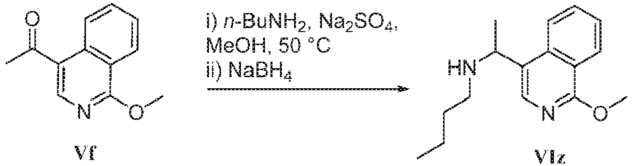

To a solution of 0.5 g (2.5 mmol, 1.0 eq.) of 1-(1-methoxyisoquinolin-4-yl)ethan-1-one **(Vf)** in 10 mL of methanol was added 0.17 g (3.0 mmol, 3.0 eq.) of *n*-butyl amine followed by 0.5 g of sodium sulfate and the mixture was then heated at 50 °C for 16 h. The mixture was allowed to cool to room temperature and 0.28 g (7.5 mmol, 3.0 eq.) of sodium borohydride was added. Stirring was continued for a further 1 h and the solvent was removed *in vacuo.* The residue was diluted with 20 mL of ice-cold water and extracted with 3 × 100 mL of ethyl acetate. The combined organic extracts were washed with 50 mL of brine, dried (Na₂SO₄), filtered and the solvent was removed *in vacuo.* The residue was purified by flash chromatography (SiO₂, eluting with a linear gradient of 10-40% ethyl acetate/petroleum ether) to provide 0.3 g (1.16 mmol, 47%) of *N*-(1-(1-methoxyisoquinolin-4-yl)ethyl)butan-1-amine **(VIz).** LCMS: *m*/*z* found 259.0 [M+H]⁺.

### 1-Butyl-3-(3-chloro-4-fluorophenyl)-1-(1-(1-methoayisoquinolin-4-yl)ethyl)urea (Compounds 61 & 62)

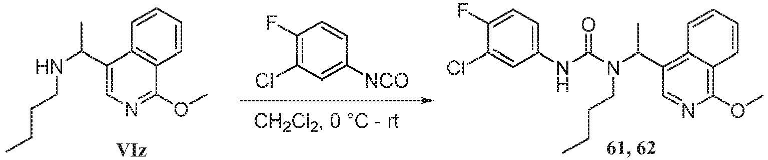

Racemic 1-butyl-3-(3-chloro-4-fluorophenyl)-1-(1-(1-methoxyisoquinolin-4-yl)ethyl)urea was synthesized in a similar manner as described above from *N*-(1-(1-methoxyisoquinolin-4-yl)ethyl)butan-1-amine **(VIz)** and 2-chloro-1-fluoro-4-isocyanatobenzene. The enantiomers were subsequently separated by SFC, Column: Chiralcel OD-H (30 × 250 mm) 5 µ, 85% CO₂:MeOH, flow rate 90 g/min.

1-Butyl-3-(3-chloro-4-fluorophenyl)-1-(1-(1-methoxyisoquinolin-4-yl)ethyl)urea: Enantiomer I **(Compound 61).** LCMS: *m*/*z* found 430.2/432.2 [M+H]⁺, RT = 6.60 min (Method: A); 1H NMR (400 MHz, DMSO-d₆): δ 8.38 (bs, 1H), 8.23 (d, 1H), 8.16 (s, 1H), 7.99 (d, 1H), 7.79-7.86 (m, 2H), 7.61-7.67 (m, 1H), 7.49-7.54 (m, 1H), 7.30-7.35 (m, 1H), 6.11-6.18 (m, 1H), 4.08 (s, 3H), 2.98-3.07 (m, 2H), 1.61 (d, 3H), 1.12-1.23 (m, 1H), 0.85-0.90 (m, 2H), 0.53-0.61 (m, 1H), 0.52 (t, 3H); Chiral analytical SFC: RT = 2.03 min, Column: Chiralcel OD-3 (250 × 4.6 mm), 5 µ, 80% CO₂:MeOH, Flow rate = 3.0 mL/min.

1-Butyl-3-(3-chloro-4-fluorophenyl)-1-(1-(1-methoxyisoquinolin-4-yl)ethyl)urea: Enantiomer II **(Compound 62).** LCMS: *m*/*z* found 430.2/432.0 [M+H]⁺, RT = 6.44 min (Method: A); 1H NMR (400 MHz, DMSO-d₆): δ 8.38 (bs, 1H), 8.23 (d, 1H), 8.16 (s, 1H), 7.99 (d, 1H), 7.79-7.86 (m, 2H), 7.61-7.67 (m, 1H), 7.49-7.54 (m, 1H), 7.30-7.35 (m, 1H), 6.11-6.18 (m, 1H), 4.08 (s, 3H), 2.98-3.07 (m, 2H), 1.61 (d, 3H), 1.12-1.23 (m, 1H), 0.85-0.90 (m, 2H), 0.53-0.61 (m, 1H), 0.52 (t, 3H); Chiral analytical SFC: RT = 2.61 min, Column: Chiralcel OD-3 (250 × 4.6 mm), 5 µ, 80% CO₂:MeOH, Flow rate = 3.0 mL/min.

### 4-(1-(Butylamino)ethyl)isoquinolin-1(2H)-one hydrochloride (VIIIat)

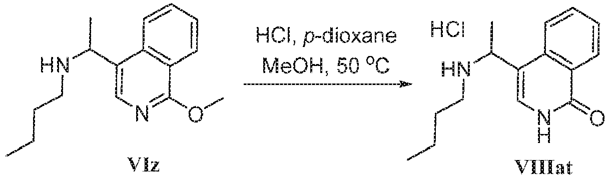

To a solution of 0.30 g (1.16 mmol, 1.0 eq.) of *N*-(1-(1-methoxyisoquinolin-4-yl)ethyl)butan-1-amine **(VIz)** in 10 mL of methanol was added 1 mL of a 4 M solution of HCl in p-dioxane and the mixture was heated at 50 °C for 4 h. The mixture was allowed to cool to room temperature and the solvent was removed *in vacuo.* The residue was triturated with 10 mL of petroleum ether and the resulting solid dried under high vacuum to provide 0.30 (1.07 mmol, 92%) of 4-(1-(butylamino)ethyl)isoquinolin-1(2*H*)-one hydrochloride **(VIIIat).** LCMS: *m*/*z* found 245.1 [M+H]⁺.

### 1-Butyl-3-(3-chloro-4-fluorophenyl)-1-(1-(1-oxo-1,2-dihydroisoquinolin-4-yl)ethyl)urea (Compounds 83 & 84)

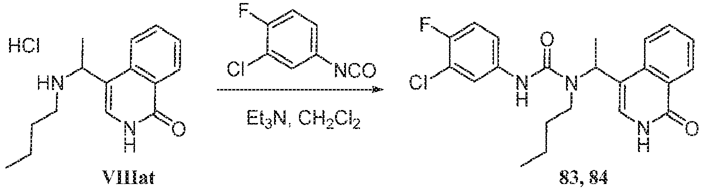

To a stirred solution of 0.3 g (1.07 mmol, 1.0 eq.) of 4-(1-(butylamino)ethyl)isoquinolin-1(2H)-one hydrochloride **(VIIIat)** in 10 mL of methylene chloride at 0 °C was added 0.25 g (2.46 mmol, 2.3 eq.) of triethylamine followed by 0.17 g (1.22 mmol, 1.1 eq.) of 2-chloro-1-fluoro-4-isocyanatobenzene and the mixture was stirred at 0 °C for 1 h. The mixture was diluted with 10 mL of ice-cold water and extracted with 3 × 10 mL of methylene chloride. The combined organic extracts were dried (Na₂SO₄), filtered and the solvent was removed *in vacuo.* The residue was purified by flash chromatography (SiO₂, eluting with a linear gradient of 20-70% ethyl acetate/petroleum ether) to provide 0.27 g (0.65 mmol, 60%) of racemic 1-butyl-3-(3-chloro-4-fluorophenyl)-1-(1-(1-oxo-1,2-dihydroisoquinolin-4-yl)ethyl)urea. The enantiomers were subsequently separated by SFC, Column: Chiralcel OD-H (30 × 250 mm) 5 µ, 85% CO₂:MeOH, flow rate 60 g/min.

1-Butyl-3-(3-chloro-4-fluorophenyl)-1-(1-(1-oxo-1,2-dihydroisoquinolin-4-yl)ethyl)urea: Enantiomer I **(Compound 83).** LCMS: *m*/*z* found 416.1/418.1 [M+H]⁺, RT = 5.71 min (Method: A); ¹H NMR (400 MHz, DMSO-d₆): δ 11.40 (d, 1H), 8.34 (bs, 1H), 8.24 (d, 1H), 7.82-7.84 (m, 1H), 7.71-7.75 (m, 2H), 7.47-7.53 (m, 2H), 7.32 (t, 1H), 7.22 (d, 1H), 5.85-5.87 (m, 1H), 2.94-3.12 (m, 2H), 1.45 (d, 3H), 1.19-1.23 (m, 1H), 0.95-1.05 (m, 2H), 0.68-0.72 (m, 1H), 0.59 (t, 3H); Chiral analytical SFC: RT = 3.10 min, Column: Chiralcel OD-3 (250 × 4.6 mm), 5 µ, 80% CO₂:MeOH, Flow rate = 3.0 mL/min.

1-Butyl-3-(3-chloro-4-fluorophenyl)-1-(1-(1-oxo-1,2-dihydroisoquinolin-4-yl)ethyl)urea: Enantiomer II **(Compound 84).** LCMS: *m*/*z* found 416.1/418.1 [M+H]⁺, RT = 5.71 min (Method: A); ¹H NMR (400 MHz, DMSO-d₆): δ 11.40 (d, 1H), 8.34 (bs, 1H), 8.24 (d, 1H), 7.82-7.84 (m, 1H), 7.71-7.75 (m, 2H), 7.47-7.53 (m, 2H), 7.32 (t, 1H), 7.22 (d, 1H), 5.85-5.87 (m, 1H), 2.94-3.12 (m, 2H), 1.45 (d, 3H), 1.19-1.23 (m, 1H), 0.95-1.05 (m, 2H), 0.68-0.72 (m, 1H), 0.59 (t, 3H); Chiral analytical SFC: RT = 3.80 min, Column: Chiralcel OD-3 (250 × 4.6 mm), 5 µ, 80% CO₂:MeOH, Flow rate = 3.0 mL/min.

### 3-((1-(1-Methoxyisoquinolin-4-yl)ethyl)amino)propan-1-ol (VIg)

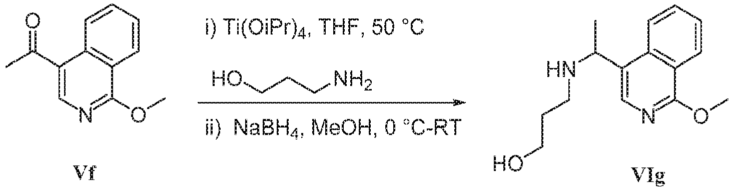

To a solution of 1.0 g (5.0 mmol, 1.0 eq.) of 1-(1-methoxyisoquinolin-4-yl)ethan-1-one **(Vf)** in 10 mL of anhydrous THF under a nitrogen atmosphere was added 0.56 g (7.5 mmol, 1.5 eq.) of 3-aminopropan-1-ol followed by 14.1 g (50.0 mmol, 10.0 eq.) of titanium (IV) isopropoxide and the mixture was heated to 50 °C for 16 h. The mixture was allowed to cool to room temperature and then further cooled to 0 °C. The mixture was then diluted with 2 mL of methanol and 0.38 g (10.0 mmol, 2.0 eq.) of sodium borohydride was added. The mixture was allowed to warm to room temperature and stirred for 2 h. The reaction was quenched by the addition of 50 mL of water and filtered through CELITE^{®}. The pad was washed with 20 mL of ethyl acetate and the biphasic mixture was extracted with 2 × 60 mL of ethyl acetate. The combined organic extracts were washed with 60 mL of brine, dried (Na₂SO₄), filtered and the solvent was removed *in vacuo.* The residue was purified by trituration with 20% ethanol in pentane to provide 0.86 g (3.3 mmol, 66%) of 3-((1-(1-methoxyisoquinolin-4-yl)ethyl)amino)propan-1-ol **(VIg).** LCMS: *m*/*z* found 261.4/263.4 [M+H]⁺; ¹H NMR (400 MHz, DMSO-d₆): δ 8.24-8.28 (m, 3H), 7.82-7.86 (m, 1H), 7.65-7.69 (m, 1H), 4.76-4.83 (m, 1H), 4.07 (s, 3H), 3.33 (bs, 2H), 3.42 (t, 2H), 2.78-2.83 (m, 1H), 2.67-2.71 (m, 1H), 1.67-1.71 (m, 2H), 1.57 (d, 3H).

### 4-(1-((3-Hydroxypropyl)amino)ethyl)isoquinolin-1(2H)-one hydrochloride (VIIIg)

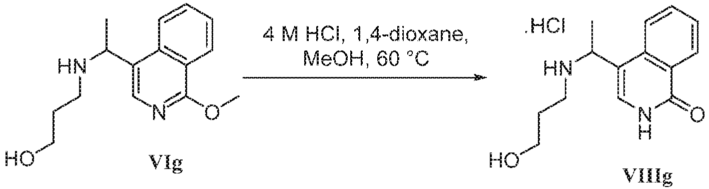

To a stirred solution of 0.4 g (1.54 mmol, 1.0 eq.) of 3-((1-(1-methoxyisoquinolin-4-yl)ethyl) amino)propan-1-ol **(VIg)** in 15 mL of methanol in a pressure vessel was added 13 mL of a 4 M solution of HCl in 1,4-dioxane. The vessel was sealed, and the mixture was heated at 60 °C for 16 h. The mixture was allowed to cool to room temperature and concentrated *in vacuo,* and the residue was dried under high vacuum to provide 0.36 g (1.27 mmol, 82%) of 4-(1-((3-hydroxypropyl)amino)ethyl)isoquinolin-1(2*H*)-one hydrochloride **(VIIIg).** LCMS: *m*/*z* found 247.06 [M+H]⁺; ¹H NMR (400 MHz, DMSO-d₆): δ 9.60 (bs, 1H), 9.06 (bs, 1H), 8.29 (d, 1H), 7.98 (d, 1H), 7.77-7.83 (m, 1H), 7.69 (d, 1H), 7.54-7.59 (m, 1H), 4.82-4.89 (m, 1H), 4.69 (bs, 1H), 3.43 (t, 2H), 2.97-3.04 (m, 1H), 2.84-2.91 (m, 1H), 1.75-1.85 (m, 2H), 1.63 (d, 3H).

### 3-(3-Chloro-4-fluorophenyl)-1-(3-hydroxypropyl)-1-(1-(1-methoxyisoquinolin-4-yl)ethyl)urea (Compound 140)

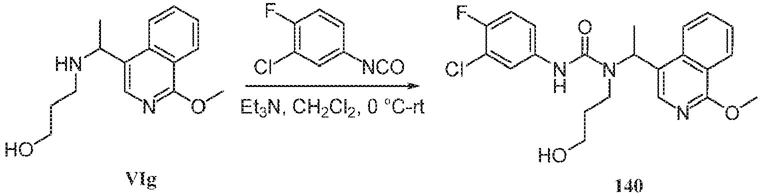

To a stirred solution of 0.4 g (1.53 mmol, 1.0 eq.) of 3-((1-(1-methoxyisoquinolin-4-yl)ethyl) amino)propan-1-ol **(VIg)** in 5 mL of methylene chloride at 0 °C under a nitrogen atmosphere was added 0.7 mL (7.69 mmol, 5.0 eq.) of triethylamine followed by 0.26 g (1.53 mmol, 1.0 eq.) of 2-chloro-1-fluoro-4-isocyanatobenzene. The mixture was allowed to warm to room temperature and stirred for 2 h. The solvent was removed *in vacuo* and the residue was suspended in 10 mL of water and stirred for 20 min. The precipitate was collected by filtration and dried under vacuum. The obtained solid was triturated with 20 mL of diethyl ether and dried under vacuum to provide 0.35 g (0.81 mmol, 53%) of racemic 3-(3-chloro-4-fluorophenyl)-1-(3-hydroxypropyl)-1-(1-(1-methoxyisoquinolin-4-yl)ethyl)urea. LCMS: *m*/*z* found 432.4/434.4 [M+H]⁺. The enantiomers were subsequently separated by SFC (Waters SFC-80), Column - (R,R) Whelk-01 (30 × 250 mm) 5 µ, 60% CO₂:MeOH, Flow rate 90 g/min to provide 103 mg and 108 mg of the resolved enantiomers.

3-(3-Chloro-4-fluorophenyl)-1-(3-hydroxypropyl)-1-(1-(1-methoxyisoquinolin-4-yl)ethyl)urea: Enantiomer I. LCMS: *m*/*z* found 432.2/434.3 [M+H]⁺; RT = 7.17 min, (Method A); ¹H NMR (400 MHz, DMSO-d₆): δ 8.78 (br s, 1H), 8.22 (d, 1H), 8.14 (s, 1H), 7.93 (d, 1H), 7.85-7.79 (m, 2H), 7.63 (t, 1H), 7.45-7.40 (m, 1H), 7.33 (t, 1H), 6.16-6.11 (m, 1H), 5.04 (br s, 1H), 4.08 (s, 3H), 3.17-3.08 (m, 4H), 1.61 (d, 3H), 1.04-0.89 (m, 2H); Chiral analytical SFC: RT = 3.20 min, Column - (R,R) Whelk-01 (4.6 × 250 mm) 5 µ, 60% CO₂: MeOH, Flow rate 4.0 g/min.

3-(3-Chloro-4-fluorophenyl)-1-(3-hydroxypropyl)-1-(1-(1-methoxyisoquinolin-4-yl)ethyl)urea: Enantiomer II **(Compound 140).** LCMS: *m*/*z* found 432.2/434.3 [M+H]⁺; RT = 7.17 min, (Method A); ¹H NMR (400 MHz, DMSO-d₆): δ 8.78 (br s, 1H), 8.22 (d, 1H), 8.14 (s, 1H), 7.93 (d, 1H), 7.85-7.79 (m, 2H), 7.63 (t, 1H), 7.45-7.40 (m, 1H), 7.33 (t, 1H), 6.16-6.11 (m, 1H), 5.04 (br s, 1H), 4.08 (s, 3H), 3.17-3.08 (m, 4H), 1.61 (d, 3H), 1.04-0.89 (m, 2H); Chiral analytical SFC: RT = 5.46 min, Column - (R,R) Whelk-01 (4.6 × 250 mm) 5 µ, 60% CO₂: MeOH, Flow rate 4.0 g/min.

### 3-(3-Chloro-4-fluorophenyl)-1-(3-hydroxypropyl)-1-(1-(1-oxo-1,2-dihydroisoquinolin-4-yl)ethyl)urea (Compounds 130 & 131)

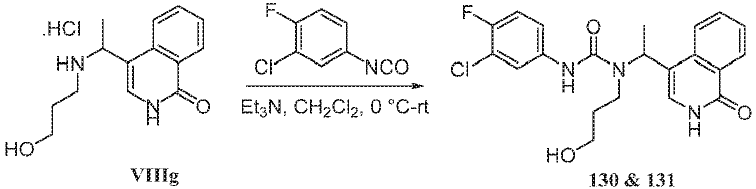

To a stirred solution of 0.3 g (1.06 mmol, 1.0 eq.) of 4-(1-((3-hydroxypropyl)amino)ethyl) isoquinolin-1(2*H*)-one hydrochloride **(VIIIg)** in 10 mL of methylene chloride at 0 °C under a nitrogen atmosphere was added 0.7 mL (5.32 mmol, 5.0 eq.) of triethylamine followed by 0.18 g (1.06 mmol, 1.0 eq.) of 2-chloro-1-fluoro-4-isocyanatobenzene, and the mixture was stirred at room temperature for 2 h. The solvent was removed *in vacuo* and the residue was diluted with 30 mL of water and stirred for 20 min. The resulting precipitated was collected by filtration, washed with 15 mL of diethyl ether and dried under high vacuum to provide 0.25 g (0.60 mmol, 56%) of racemic 3-(3-chloro-4-fluorophenyl)-1-(3-hydroxypropyl)-1-(1-(1-oxo-1,2-dihydroisoquinolin-4-yl)ethyl)urea. LCMS: *m*/*z* found 418.3/420.3 [M+H]⁺; The enantiomers were subsequently separated by SFC (Waters SFC-80), Column: Chiralcel OD-H (250 × 30 mm) 5 µ, 75% CO₂:MeOH, Flow rate 90 g/min to provide 83 mg and 78 mg of the resolved enantiomers.

3-(3-Chloro-4-fluorophenyl)-1-(3-hydroxypropyl)-1-(1-(1-oxo-1,2-dihydroisoquinolin-4-yl)ethyl)urea: Enantiomer I **(Compound 130).** LCMS: *m*/*z* found 418.2/420.2 [M+H]⁺; RT = 6.24 min, (Method A); ¹H NMR (400 MHz, DMSO-*d₆*): δ 11.40 (bd, 1H), 8.75 (bs, 1H), 8.23 (d, 1H), 7.79-7.82 (m, 1H), 7.72-7.76 (m, 1H), 7.66 (d, 1H), 7.49 (t, 1H), 7.40-7.44 (m, 1H), 7.32 (t, 1H), 7.20 (d, 1H), 5.84-5.89 (m, 1H), 5.05 (bs, 1H), 3.12-3.19 (m, 4H), 1.46 (d, 3H), 1.06-1.18 (m, 2H); Chiral analytical SFC: RT = 1.74 min, Column: Chiralcel OD-3 (4.6 × 150 mm) 3 µ, 70% CO₂: MeOH, Flow rate 3.0 g/min.

3-(3-Chloro-4-fluorophenyl)-1-(3-hydroxypropyl)-1-(1-(1-oxo-1,2-dihydroisoquinolin-4-yl)ethyl)urea: Enantiomer II **(Compound 131).** LCMS: *m*/*z* found 418.2/420.2 [M+H]⁺; RT = 6.24 min, (Method A); ¹H NMR (400 MHz, DMSO-d₆): δ 11.40 (bd, 1H), 8.75 (bs, 1H), 8.23 (d, 1H), 7.79-7.82 (m, 1H), 7.72-7.76 (m, 1H), 7.66 (d, 1H), 7.49 (t, 1H), 7.40-7.44 (m, 1H), 7.32 (t, 1H), 7.20 (d, 1H), 5.84-5.89 (m, 1H), 5.05 (bs, 1H), 3.12-3.19 (m, 4H), 1.46 (d, 3H), 1.06-1.18 (m, 2H); Chiral analytical SFC: RT = 2.49 min, Column: Chiralcel OD-3 (4.6 × 150 mm) 3 µ, 70% CO₂: MeOH, Flow rate 3.0 g/min.

### 2-Methoxy-N (1-(1-methoxyisoquinolin-4-yl)ethyl)ethan-1-amine (VIs)

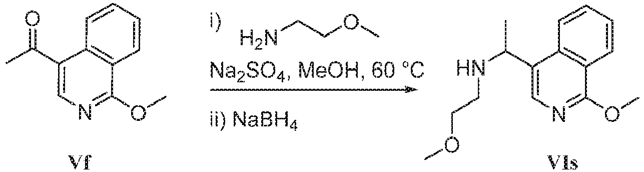

To a solution of 0.5 g (2.5 mmol, 1.0 eq.) of 1-(1-methoxyisoquinolin-4-yl)ethan-1-one **(Vf)** in 10 mL of methanol was added 0.92 g (12.4 mmol, 5.0 eq.) of 2-methoxyethan-1-amine followed by 0.5 g of sodium sulfate and the mixture was then heated at 60 °C for 16 h. The mixture was allowed to cool to room temperature and 0.28 g (7.5 mmol, 3.0 eq.) of sodium borohydride was added. Stirring was continued for a further 1 h and the solvent was removed *in vacuo.* The residue was diluted with 20 mL of ice-cold water and extracted with 3 × 100 mL of ethyl acetate. The combined organic extracts were washed with 50 mL of brine, dried (Na₂SO₄), filtered and the solvent was removed *in vacuo* to provide 0.3 g (1.15 mmol, 47%) of 2-methoxy-*N*-(1-(1-methoxyisoquinolin-4-yl)ethyl)ethan-1-amine **(VIs).** LCMS: *m*/*z* found 261.4 [M+H]⁺.

### 3-(3-chloro-4-fluorophenyl)-1-(2-methoxyethyl)-1-(1-(1-methoxyisoquinolin-4-yl)ethyl)urea (Compounds 72 & 73)

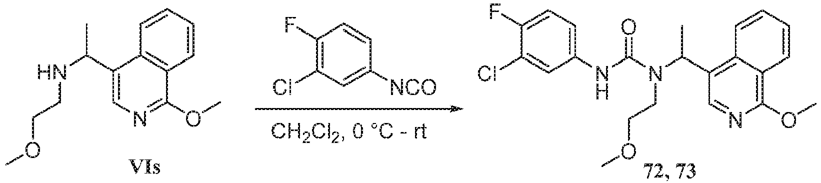

Racemic 3-(3-chloro-4-fluorophenyl)-1-(2-methoxyethyl)-1-(1-(1-methoxyisoquinolin-4-yl)ethyl)urea was synthesized in a similar manner as described above from 2-methoxy-*N*-(1-(1-methoxyisoquinolin-4-yl)ethyl)ethan-1-amine **(VIs)** and 2-chloro-1-fluoro-4-isocyanatobenzene. The enantiomers were subsequently separated by SFC, Column: Chiralpak IG (30 × 250 mm) 5 µ, 80% CO₂:MeOH, flow rate 90 g/min.

3-(3-Chloro-4-fluorophenyl)-1-(2-methoxyethyl)-1-(1-(1-methoxylsoquinolin-4-yl)ethyl)urea: Enantiomer I **(Compound 72).** LCMS: *m*/*z* found 432.2/434.2 [M+H]⁺, RT = 6.32 min (Method: A); ¹H NMR (400 MHz, DMSO-d₆): δ 8.65 (bs, 1H), 8.24 (d, 1H), 8.14 (s, 1H), 7.90 (d, 1H), 7.80-7.85 (m, 2H), 7.63-7.67 (m, 1H), 7.30-7.39 (m, 2H), 6.08-6.12 (m, 1H), 4.08 (s, 3H), 3.24-3.30 (m, 2H), 2.90 (s, 3H), 2.82-2.85 (m, 1H), 2.61-2.68 (m, 1H), 1.61 (d, 3H); Chiral analytical SFC: RT = 3.11 min, Chiralpak IG (250 × 4.6 mm), 5 µ, 70% CO₂:MeOH, Flow rate = 3.0 mL/min.

3-(3-Chloro-4-fluorophenyl)-1-(2-methoxyethyl)-1-(1-(1-methoxylsoquinolin-4-yl)ethyl)urea: Enantiomer II **(Compound 73).** LCMS: *m*/*z* found 432.2/434.2 [M+H]⁺, RT = 6.32 min (Method: A); ¹H NMR (400 MHz, DMSO-d₆): δ 8.65 (bs, 1H), 8.24 (d, 1H), 8.14 (s, 1H), 7.90 (d, 1H), 7.80-7.85 (m, 2H), 7.63-7.67 (m, 1H), 7.30-7.39 (m, 2H), 6.08-6.12 (m, 1H), 4.08 (s, 3H), 3.24-3.30 (m, 2H), 2.90 (s, 3H), 2.82-2.85 (m, 1H), 2.61-2.68 (m, 1H), 1.61 (d, 3H); Chiral analytical SFC: RT = 4.62 min, Chiralpak IG (250 × 4.6 mm), 5 µ, 70% CO₂:MeOH, Flow rate = 3.0 mL/min.

### 3-(3-Chloro-4-fluorophenyl)-1-(2-methoxyethyl)-1-(1-(1-oxo-1,2-dihydroisoquinolin-4-yl)ethyl)urea (Compounds 65 & 66)

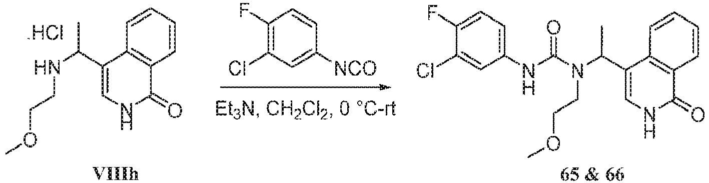

3-(3-Chloro-4-fluorophenyl)-1-(2-methoxyethyl)-1-(1-(1-oxo-1,2-dihydroisoquinolin-4-yl)ethyl)urea was synthesized in an analogous manner as described above from racemic 4-(1-((2-methoxyethyl)amino)ethyl)isoquinolin-1(2*H*)-one hydrochloride **(VIIIh)** and 2-chloro-1-fluoro-4-isocyanatobenzene. The enantiomers were subsequently separated by SFC, Column: Chiralpak IG (30 × 250 mm), 5 µ, 70% CO₂:MeOH, Flow rate 90 g/min.

3-(3-Chloro-4-fluorophenyl)-1-(2-methoxyethy1)-1-(1-(1-oxo-1,2-dihydroisoquinolin-4-yl)ethyl)urea: Enantiomer I **(Compound 65).** LCMS: *m*/*z* found 418.1/420.1 [M+H]⁺; RT = 5.04 min (Method A); ¹H NMR (400 MHz, DMSO-d₆): δ 11.40 (d, 1H), 8.62 (br s, 1H), 8.24-8.26 (m, 1H), 7.79-7.82 (m, 1H), 7.73-7.77 (m, 1H), 7.63 (d, 1H), 7.49-7.53 (m, 1H), 7.29-7.37 (m, 2H), 7.20 (d, 1H), 5.81-5.84 (m, 1H), 3.31-3.34 (m, 2H), 2.95-2.97 (m, 1H), 2.94 (s, 3H), 2.81-2.85 (m, 1H), 1.46 (d, 3H); Chiral analytical SFC: RT = 2.28 min, Column: Chiralpak IG (4.6 × 250 mm), 5 µ, 60% CO₂:MeOH, Flow = 4.0 mL/min.

3-(3-Chloro-4-fluorophenyl)-1-(2-methoxyethyl)-1-(1-(1-oxo-1,2-dihydroisoquinolin-4-yl)ethyl)urea: Enantiomer II **(Compound 66).** LCMS: *m*/*z* found 418.1/420.1 [M+H]⁺; RT = 5.04 min (Method A); ¹H NMR (400 MHz, DMSO-*d₆*): δ 11.40 (d, 1H), 8.62 (br s, 1H), 8.24-8.26 (m, 1H), 7.79-7.82 (m, 1H), 7.73-7.77 (m, 1H), 7.63 (d, 1H), 7.49-7.53 (m, 1H), 7.29-7.37 (m, 2H), 7.20 (d, 1H), 5.81-5.84 (m, 1H), 3.31-3.34 (m, 2H), 2.95-2.97 (m, 1H), 2.94 (s, 3H), 2.81-2.85 (m, 1H), 1.46 (d, 3H); Chiral analytical SFC: RT = 5.18 min, Column: Chiralpak IG (4.6 × 250 mm), 5 µ, 60% CO₂:MeOH, Flow = 4.0 mL/min.

### 3-(3-Chloro-4-fluorophenyl)-1-(1-(1-methoxyisoquinolin-4-yl)ethyl)-1-(3-methoxypropyl)urea (Compounds 81 & 82)

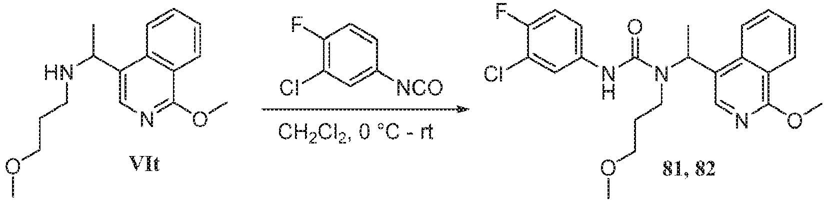

Racemic 3-(3-chloro-4-fluorophenyl)-1-(1-(1-methoxyisoquinolin-4-yl)ethyl)-1-(3-methoxypropyl)urea was synthesized in a similar manner as described above from 3-methoxy-N-(1-(1-methoxyisoquinolin-4-yl)ethyl)propan-1-amine **(VIt)** and 2-chloro-1-fluoro-4-isocyanatobenzene. The enantiomers were subsequently separated by SFC, Column: R,R Whelk-01 (30 × 250 mm) 5 µ, 70% CO₂:MeOH, flow rate 90 g/min.

3-(3-Chloro-4-fluorophenyl)-1-(1-(1-methoxyisoquinolin-4-yl)ethyl)-1-(3-methoxypropyl)urea: Enantiomer I **(Compound 81).** LCMS: *m*/*z* found 446.3/448.2 [M+H]⁺, RT = 7.12 min (Method: A); ¹H NMR (400 MHz, DMSO-d₆): δ 8.39 (s, 1H), 8.23 (d, 1H), 8.14 (s, 1H), 7.97 (d, 1H), 7.80-7.85 (m, 2H), 7.64 (t, 1H), 7.44-7.48 (m, 1H), 7.33 (t, 1H), 6.11-6.14 (m, 1H), 4.08 (s, 3H), 3.05-3.14 (m, 2H), 2.89-3.03 (m, 5H), 1.60 (d, 3H), 1.23-1.30 (m, 1H), 0.93-0.98 (m, 1H); Chiral analytical SFC: RT = 6.31 min, Column: (R,R) Whelk -01 (250 × 4.6 mm), 5 µ, 65% CO₂:MeOH, Flow rate = 3.0 mL/min.

3-(3-Chloro-4-fluorophenyl)-1-(1-(1-methoxyisoquinolin-4-yl)ethyl)-1-(3-methoxypropyl)urea: Enantiomer II **(Compound 82).** LCMS: *m*/*z* found 446.3/448.2 [M+H]⁺, RT = 7.12 min (Method: A); ¹H NMR (400 MHz, DMSO-d₆): δ 8.39 (s, 1H), 8.23 (d, 1H), 8.14 (s, 1H), 7.97 (d, 1H), 7.80-7.85 (m, 2H), 7.64 (t, 1H), 7.44-7.48 (m, 1H), 7.33 (t, 1H), 6.11-6.14 (m, 1H), 4.08 (s, 3H), 3.05-3.14 (m, 2H), 2.89-3.03 (m, 5H), 1.60 (d, 3H), 1.23-1.30 (m, 1H), 0.93-0.98 (m, 1H); Chiral analytical SFC: RT = 8.70 min, Column: (R,R) Whelk -01 (250 × 4.6 mm), 5 µ, 65% CO₂:MeOH, Flow rate = 3.0 mL/min.

### 3-(3-Chloro-4-fluorophenyl)-1-(1-(1-methoxyisoquinolin-4-yl)ethyl)-1-(3-methoxypropyl)urea (Compounds 79 & 80)

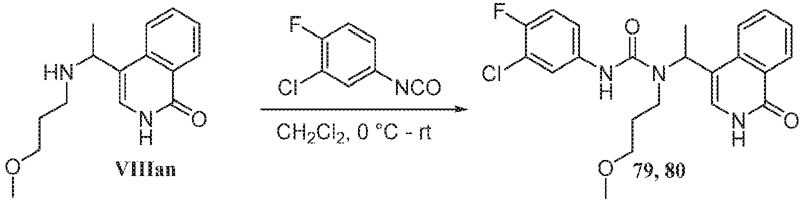

Racemic 3-(3-chloro-4-fluorophenyl)-1-(1-(1-methoxyisoquinolin-4-yl)ethyl)-1-(3-methoxypropyl)urea was synthesized in a similar manner as described above from 4-(1-((3-methoxypropyl)amino)ethyl)isoquinolin-1(2*H*)-one **(VIIIan)** and 2-chloro-1-fluoro-4-isocyanatobenzene. The enantiomers were subsequently separated by SFC, Column: Chiralpak IC (30 × 250 mm) 5 µ, 80% CO₂:MeOH, flow rate 100 g/min.

3-(3-Chloro-4-fluorophenyl)-1-(1-(1-methoxyisoquinolin-4-yl)ethyl)-1-(3-methoxypropyl)urea: Enantiomer I **(Compound 79).** LCMS: *m*/*z* found 432.2/434.2 [M+H]⁺, RT = 5.38 min (Method: A); ¹H NMR (400 MHz, DMSO-d₆): δ 11.40 (bd, 1H), 8.36 (s, 1H), 8.24 (d, 1H), 7.82-7.86 (3, 1H), 7.69-7.75 (m, 2H), 7.44-7.52 (m, 2H), 7.33 (t, 1H), 7.21 (d, 1H), 5.85-5.87 (m, 1H), 2.98-3.15 (m, 7H), 1.45 (d, 3H), 1.32-1.39 (m, 1H), 1.05-1.11 (m, 1H); Chiral analytical SFC: RT = 10.85 min, Column: Chiralpak IC (250 × 4.6 mm), 5 µ, 65% CO₂:MeOH, Flow rate = 3.0 mL/min.

3-(3-Chloro-4-fluorophenyl)-1-(1-(1-methoxyisoquinolin-4-yl)ethyl)-1-(3-methoxypropyl)urea: Enantiomer II **(Compound 80).** LCMS: *m*/*z* found 432.2/434.2 [M+H]⁺, RT = 5.38 min (Method: A); ¹H NMR (400 MHz, DMSO-d₆): δ 11.40 (bd, 1H), 8.36 (s, 1H), 8.24 (d, 1H), 7.82-7.86 (3, 1H), 7.69-7.75 (m, 2H), 7.44-7.52 (m, 2H), 7.33 (t, 1H), 7.21 (d, 1H), 5.85-5.87 (m, 1H), 2.98-3.15 (m, 7H), 1.45 (d, 3H), 1.32-1.39 (m, 1H), 1.05-1.11 (m, 1H); Chiral analytical SFC: RT = 14.54 min, Column: Chiralpak IC (250 × 4.6 mm), 5 µ, 65% CO₂:MeOH, Flow rate = 3.0 mL/min.

### 2-Ethoxy-N-(1-(1-methoxyisoquinolin-4-yl)ethyl)ethan-1-amine (VIu)

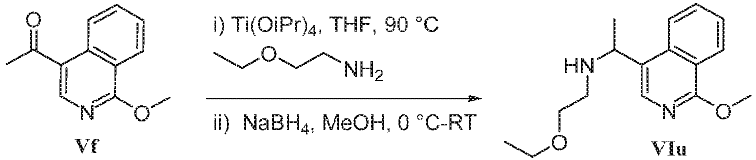

To a solution of 1.0 g (5.0 mmol, 1.0 eq.) of 1-(1-methoxyisoquinolin-4-yl)ethan-1-one **(Vf)** in 5 mL of THF under a nitrogen atmosphere was added 0.66 g (7.5 mmol 1.5 eq.) of 2-ethoxyethan-1-amine followed by 14.1 g (49.8 mmol, 10.0 eq.) titanium (IV) isopropoxide and the mixture was heated at 90 °C for 6 h. The mixture was allowed to cool to room temperature and then further cooled to 0 °C. The mixture was diluted with 2 mL of methanol and 0.38 g (10.0 mmol, 2.0 eq.) of sodium borohydride was added. The mixture was then allowed to warm to room temperature and stirred for 2 h. The reaction was quenched by the addition of 20 mL of water and filtered through CELITE^{®}. The pad was washed with 5 mL of ethyl acetate and the biphasic mixture was extracted with 2 × 30 mL of ethyl acetate. The combined organic extracts were dried (Na₂SO₄), filtered and the solvent was removed *in vacuo.* The residue was purified by flash chromatography (SiO₂, eluting with a linear gradient of 0-4% methanol/methylene chloride) to provide 1.3 g (4.06 mmol, 82%) of 2-ethoxy-*N*-(1-(1-methoxyisoquinolin-4-yl)ethyl)ethan-1-amine **(VIu).** LCMS: *m*/*z* found 275.3 [M+H]⁺; ¹H NMR (400 MHz, DMSO-d₆): δ 8.31 (d, 1H), 8.19-8.22 (m, 1H), 8.06 (s, 1H), 7.74-7.79 (m, 1H), 7.58-7.64 (m, 1H), 4.32-4.39 (m, 1H), 4.04 (s, 3H), 3.32-3.44 (m, 4H), 2.50-2.67 (m, 2H), 2.08 (bs, 1H), 1.40 (d, 3H), 1.08 (t, 3H).

### 3-(3-Chloro-4-fluorophenyl)-1-(2-ethoxyethyl)-1-(1-(1-methoxyisoquinolin-4-yl)ethyl)urea (Compounds 125 & 126)

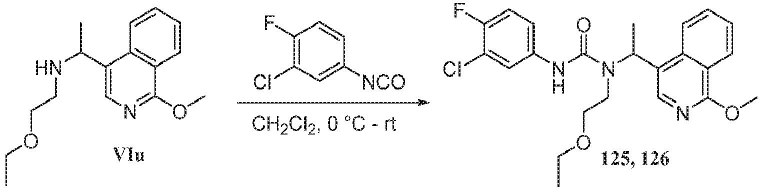

Racemic 3-(3-chloro-4-fluorophenyl)-1-(2-ethoxyethyl)-1-(1-(1-methoxyisoquinolin-4-yl)ethyl)urea was synthesized in a similar manner as described above from 2-ethoxy-N-(1-(1-methoxyisoquinolin-4-yl)ethyl)ethan-1-amine **(VIu)** and 2-chloro-1-fluoro-4-isocyanatobenzene. The enantiomers were subsequently separated by SFC, Chiralpak IG (30 × 250 mm) 5 µ, 70% CO₂:MeOH, flow rate 100 g/min.

3-(3-Chloro-4-fluorophenyl)-1-(2-ethoxyethyl)-1-(1-(1-methoxyisoquinolin-4-yl)ethyl)urea: Enantiomer I **(Compound 125).** LCMS: *m*/*z* found 446.3/448.2 [M+H]⁺, RT = 7.70 min (Method: A); ¹H NMR (400 MHz, DMSO-d₆): 8.68 (bs, 1H), 8.24 (d, 1H), 8.13 (s, 1H), 7.90 (d, 1H), 7.79-7.85 (m, 2H), 7.65 (t, 1H), 7.32-7.34 (m, 2H), 6.08-6.13 (m, 1H), 4.08 (s, 3H), 3.22-3.31 (m, 2H), 3.07-3.11 (m, 1H), 2.85-2.91 (m, 2H), 2.53-2.59 (m, 1H), 1.61 (d, 3H), 0.84 (t, 3H); Chiral analytical SFC: RT = 1.18 min, Column: Chiralpak IG (250 × 4.6 mm), 5 µ, 60% CO₂:MeOH, Flow rate = 3.0 mL/min.

3-(3-Chloro-4-fluorophenyl)-1-(2-ethoxyethyl)-1-(1-(1-methoxyisoquinolin-4-yl)ethyl)urea: Enantiomer II **(Compound 126).** LCMS: *m*/*z* found 446.3/448.2 [M+H]⁺, RT = 7.70 min (Method: A); ¹H NMR (400 MHz, DMSO-d₆): 8.68 (bs, 1H), 8.24 (d, 1H), 8.13 (s, 1H), 7.90 (d, 1H), 7.79-7.85 (m, 2H), 7.65 (t, 1H), 7.32-7.34 (m, 2H), 6.08-6.13 (m, 1H), 4.08 (s, 3H), 3.22-3.31 (m, 2H), 3.07-3.11 (m, 1H), 2.85-2.91 (m, 2H), 2.53-2.59 (m, 1H), 1.61 (d, 3H), 0.84 (t, 3H); Chiral analytical SFC: RT = 1.57 min, Column: Chiralpak IG (250 × 4.6 mm), 5 µ, 60% CO₂:MeOH, Flow rate = 3.0 mL/min.

### 4-(1-((2-Ethoxyethyl)amino)ethyl)isoquinolin-1(2H)-one hydrochloride (VIIIao)

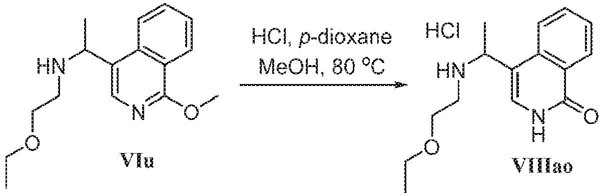

To a solution of 0.7 g (2.55 mmol, 1.0 eq.) of 2-ethoxy-*N*-(1-(1-methoxyisoquinolin-4-yl)ethyl)ethan-1-amine **(VIu)** in 5 mL methanol in a sealed tube was added 15 mL of a 4 M solution of HCl in p-dioxane and the mixture was heated at 80 °C for 16 h. The mixture was allowed to cool to room temperature and the solvent was removed *in vacuo.* The residue was triturated with 10 mL of petroleum ether and the resulting solid dried under high vacuum to provide 0.68 g (2.29 mmol, 89%) of 4-(1-((2-ethoxyethyl)amino)ethyl)isoquinolin-1(2*H*)-one hydrochloride **(VIIIao).** LCMS: *m*/*z* found 261.4 [M+H]⁺; ¹H NMR (400 MHz, DMSO-d₆): δ 11.63 (bd, 1H), 9.45 (bs, 1H), 8.90 (bs, 1H), 8.27-8.30 (m, 1H), 7.95 (d, 1H), 7.78-7.83 (m, 1H), 7.55-7.61 (m, 2H), 4.87-4.92 (m, 1H), 3.60-3.64 (m, 2H), 3.48 (q, 2H), 3.13-3.18 (m, 1H), 3.01-3.06 (m, 1H), 1.63 (d, 3H), 1.13 (t, 3H).

### 3-(3-Chloro-4-fluorophenyl)-1-(2-ethoxyethyl)-1-(1-(1-oxo-1,2-dihydroisoquinolin-4-yl)ethyl)urea (Compounds 109 & 110)

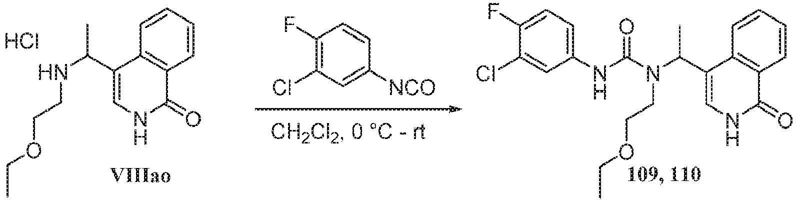

Racemic 3-(3-chloro-4-fluorophenyl)-1-(2-ethoxyethyl)-1-(1-(1-oxo-1,2-dihydroisoquinolin-4-yl)ethyl)urea was synthesized in a similar manner as described above from 4-(1-((2-ethoxyethyl)amino)ethyl)isoquinolin-1(2*H*)-one hydrochloride **(VIIIao)** and 2-chloro-1-fluoro-4-isocyanatobenzene. The enantiomers were subsequently separated by SFC, Column: Chiralpak IG (30 × 250 mm) 5 µ, 50% CO₂:MeOH, flow rate 100 g/min.

3-(3-Chloro-4-fluorophenyl)-1-(2-ethoxyethyl)-1-(1-(1-oxo-1,2-dihydroisoquinolin-4-yl)ethyl)urea: Enantiomer I **(Compound 109).** LCMS: *m*/*z* found 432.3/434.2 [M+H]⁺, RT = 6.38 min (Method: A); ¹H NMR (400 MHz, DMSO-d₆): δ 11.40 (bd, 1H), 8.67 (bs, 1H), 8.25 (d, 1H), 7.73-7.80 (m, 2H), 7.63 (d, 1H), 7.51 (t, 1H), 7.31-7.34 (m, 2H), 7.19-7.21 (m, 1H), 5.81-5.84 (m, 1H), 3.22-3.32 (m, 2H), 3.11-3.18 (m, 1H), 2.90-3.02 (m, 2H), 2.72-2.79 (m, 1H), 1.46 (d, 3H), 0.88 (t, 3H); Chiral analytical SFC: RT = 1.46 min, Column: Chiralpak IG (250 × 4.6 mm), 5 µ, 60% CO₂:MeOH, Flow rate = 3.0 mL/min.

3-(3-Chloro-4-fluorophenyl)-1-(2-ethoxyethyl)-1-(1-(1-oxo-1,2-dihydroisoquinolin-4-yl)ethyl)urea: Enantiomer II **(Compound 110).** LCMS: *m*/*z* found 432.3/434.2 [M+H]⁺, RT = 6.32 min (Method: A); ¹H NMR (400 MHz, DMSO-d₆): δ 11.40 (bd, 1H), 8.67 (bs, 1H), 8.25 (d, 1H), 7.73-7.80 (m, 2H), 7.63 (d, 1H), 7.51 (t, 1H), 7.31-7.34 (m, 2H), 7.19-7.21 (m, 1H), 5.81-5.84 (m, 1H), 3.22-3.32 (m, 2H), 3.11-3.18 (m, 1H), 2.90-3.02 (m, 2H), 2.72-2.79 (m, 1H), 1.46 (d, 3H), 0.88 (t, 3H); Chiral analytical SFC: RT = 2.99 min, Column: Chiralpak IG (250 × 4.6 mm), 5 µ, 60% CO₂:MeOH, Flow rate = 3.0 mL/min.

### 2-(2-Methoxyethoxy)-N-(1-(1-methoxyisoquinolin-4-yl)ethyl)ethan-1-amine (VIv)

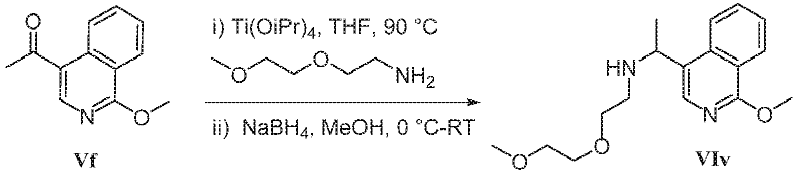

To a solution of 1.0 g (5.0 mmol, 1.0 eq.) of 1-(1-methoxyisoquinolin-4-yl)ethan-1-one **(Vf)** in 5 mL of THF under a nitrogen atmosphere was added 0.88 g (7.5 mmol 1.5 eq.) of 2-(2-methoxyethoxy)ethan-1-amine followed by 14.1 g (49.8 mmol, 10.0 eq.) titanium (IV) isopropoxide and the mixture was heated at 90 °C for 6 h. The mixture was allowed to cool to room temperature and then further cooled to 0 °C. The mixture was diluted with 2 mL of methanol and 0.38 g (10.0 mmol, 2.0 eq.) of sodium borohydride was added. The mixture was then allowed to warm to room temperature and stirred for 2 h. The reaction was quenched by the addition of 20 mL of water and filtered through CELITE^{®}. The pad was washed with 5 mL of ethyl acetate and the biphasic mixture was extracted with 2 × 30 mL of ethyl acetate. The combined organic extracts were dried (Na₂SO₄), filtered and the solvent was removed *in vacuo.* The residue was purified by flash chromatography (SiO₂, eluting with a linear gradient of 0-5% methanol/methylene chloride) to provide 1.4 g (4.59 mmol, 92%) of 2-(2-methoxyethoxy)-*N*-(1-(1-methoxyisoquinolin-4-yl)ethyl)ethan-1-amine **(VIv).** LCMS: *m*/*z* found 305.5 [M+H]⁺; ¹H NMR (400 MHz, DMSO-d₆): δ 8.31 (d, 1H), 8.21 (d, 1H), 8.06 (s, 1H), 7.74-7.79 (m, 1H), 7.58-7.64 (m, 1H), 4.32-4.39 (m, 1H), 4.04 (s, 3H), 3.39-3.49 (m, 6H), 3.22 (s, 3H), 2.51-2.68 (m, 2H), 2.10 (bs, 1H), 1.40 (d, 3H).

### 3-(3-Chloro-4-fluorophenyl)-1-(2-(2-methoxyethoxy)ethyl)-1-(1-(1-methoxyisoquinolin-4-yl)ethyl)urea (Compounds 121 & 122)

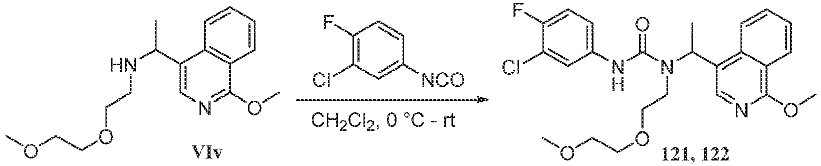

Racemic 3-(3-chloro-4-fluorophenyl)-1-(2-(2-methoxyethoxy)ethyl)-1-(1-(1-methoxyisoquinolin-4-yl)ethyl)urea was synthesized in a similar manner as described above from 2-(2-methoxyethoxy)-*N*-(1-(1-methoxyisoquinolin-4-yl)ethyl)ethan-1-amine **(VIv)** and 2-chloro-1-fluoro-4-isocyanatobenzene. The enantiomers were subsequently separated by SFC, Chiralpak IG (30 × 250 mm) 5 µ, 75% CO₂:MeOH, flow rate 90 g/min.

3-(3-Chloro-4-fluorophenyl)-1-(2-(2-methoxyethoxy)ethyl)-1-(1-(1-methoxyisoquinolin-4-yl)ethyl)urea: Enantiomer I **(Compound 121).** LCMS: *m*/*z* found 476.3/478.2 [M+H]⁺, RT = 7.54 min (Method: A); ¹H NMR (400 MHz, DMSO-d₆): 8.68 (bs, 1H), 8.24 (d, 1H), 8.13 (s, 1H), 7.90 (m, 1H), 7.83 (m, 1H), 7.74 (m, 1H), 7.65 (t, 1H), 7.31-7.41 (m, 2H), 6.08-6.13 (m, 1H), 4.08 (s, 3H), 3.19-3.28 (m, 6H), 3.17 (s, 3H), 2.92-2.99 (m, 2H), 1.61 (d, 3H); Chiral analytical SFC: RT = 1.62 min, Column: Chiralpak IG-3 (250 × 4.6 mm), 5 µ, 70% CO₂:MeOH, Flow rate = 3.0 mL/min.

3-(3-Chloro-4-fluorophenyl)-1-(2-(2-methoxyethoxy)ethyl)-1-(1-(1-methoxyisoquinolin-4-yl)ethyl)urea: Enantiomer II **(Compound 122).** LCMS: *m*/*z* found 476.3/478.2 [M+H]⁺, RT = 7.46 min (Method: A); ¹H NMR (400 MHz, DMSO-d₆): 8.68 (bs, 1H), 8.24 (d, 1H), 8.13 (s, 1H), 7.90 (m, 1H), 7.83 (m, 1H), 7.74 (m, 1H), 7.65 (t, 1H), 7.31-7.41 (m, 2H), 6.08-6.13 (m, 1H), 4.08 (s, 3H), 3.19-3.28 (m, 6H), 3.17 (s, 3H), 2.92-2.99 (m, 2H), 1.61 (d, 3H); Chiral analytical SFC: RT = 2.01 min, Column: Chiralpak IG-3 (250 × 4.6 mm), 5 µ, 70% CO₂:MeOH, Flow rate = 3.0 mL/min.

### 4-(1-((2-(2-Methoxyethoxy)ethyl)amino)ethyl)isoquinolin-1(2H)-one hydrochloride (VIIIap)

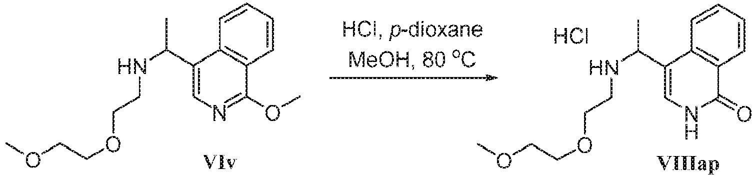

To a solution of 0.7 g (2.55 mmol, 1.0 eq.) of 2-(2-methoxyethoxy)-*N*-(1-(1-methoxyisoquinolin-4-yl)ethyl)ethan-1-amine **(VIv)** in 5 mL methanol in a sealed tube was added 15 mL of a 4 M solution of HCl in *p*-dioxane and the mixture was heated at 80 °C for 16 h. The mixture was allowed to cool to room temperature and the solvent was removed *in vacuo.* The residue was triturated with 10 mL of petroleum ether and the resulting solid dried under high vacuum to provide 0.65 g (2.0 mmol, 86%) of 4-(1-((2-(2-methoxyethoxy)ethyl)amino) ethyl)isoquinolin-1(2*H*)-one hydrochloride **(VIIIap).** LCMS: *m*/*z* found 291.4 [M+H]⁺; ¹H NMR (400 MHz, DMSO-d₆): δ 11.62 (bs, 1H), 9.11 (bs, 1H), 8.28-8.30 (m, 1H), 7.95 (d, 1H), 7.79-7.84 (m, 1H), 7.56-7.60 (m, 1H), 7.51 (d, 1H), 4.89-4.94 (m, 1H), 3.65-3.67 (m, 2H), 3.55-3.58 (m, 2H), 3.44-3.49 (m, 2H), 3.23 (s, 3H), 3.17-3.21 (m, 1H), 3.04-3.11 (m, 1H), 1.61 (d, 3H).

### 3-(3-chloro-4-fluorophenyl)-1-(2-(2-methoxyethoxy)ethyl)-1-(1-(1-oxo-1,2-dihydroisoquinolin-4-yl)ethyl)urea (Compounds 123 & 124)

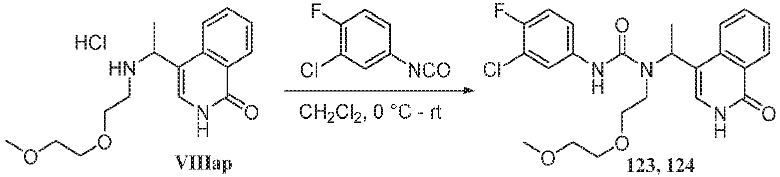

Racemic 3-(3-chloro-4-fluorophenyl)-1-(2-(2-methoxyethoxy)ethyl)-1-(1-(1-oxo-1,2-dihydroisoquinolin-4-yl)ethyl)urea was synthesized in a similar manner as described above from 4-(1-((2-(2-methoxyethoxy)ethyl)amino)ethyl)isoquinolin-1(2*H*)-one hydrochloride **(VIIIap)** and 2-chloro-1-fluoro-4-isocyanatobenzene. The enantiomers were subsequently separated by SFC, Column: Chiralcel OD-H (30 × 250 mm) 5 µ, 70% CO₂:MeOH, flow rate 100 g/min.

3-(3-Chloro-4-fluorophenyl)-1-(2-(2-methoxyethoxy)ethyl)-1-(1-(1-oxo-1,2-dihydroisoquinolin-4-yl)ethyl)urea: Enantiomer I **(Compound 123).** LCMS: *m*/*z* found 462.3/464.4 [M+H]⁺, RT = 6.90 min (Method: A); ¹H NMR (400 MHz, DMSO-d₆): δ 11.40 (bd, 1H), 8.67 (bs, 1H), 8.25 (d, 1H), 7.71-7.77 (m, 2H), 7.62 (d, 1H), 7.49-7.53 (m, 1H), 7.30-7.40 (m, 2H), 7.20 (d, 1H), 5.79-5.83 (m, 1H), 3.18-3.38 (m, 5H), 3.14 (s, 3H), 3.13-3.15 (m, 1H), 2.96-3.01 (m, 1H), 2.68-2.63 (m, 1H), 1.46 (d, 3H); Chiral analytical SFC: RT = 1.62 min, Column: Chiralcel OD-3 (250 × 4.6 mm), 5 µ, 60% CO₂:MeOH, Flow rate = 3.0 mL/min.

3-(3-Chloro-4-fluorophenyl)-1-(2-(2-methoxyethoxy)ethyl)-1-(1-(1-oxo-1,2-dihydroisoquinolin-4-yl)ethyl)urea: Enantiomer II **(Compound 124).** LCMS: *m*/*z* found 462.2/464.4 [M+H]⁺, RT = 6.78 min (Method: A); ¹H NMR (400 MHz, DMSO-d₆): δ 11.40 (bd, 1H), 8.67 (bs, 1H), 8.25 (d, 1H), 7.71-7.77 (m, 2H), 7.62 (d, 1H), 7.49-7.53 (m, 1H), 7.30-7.40 (m, 2H), 7.20 (d, 1H), 5.79-5.83 (m, 1H), 3.18-3.38 (m, 5H), 3.14 (s, 3H), 3.13-3.15 (m, 1H), 2.96-3.01 (m, 1H), 2.68-2.63 (m, 1H), 1.46 (d, 3H); Chiral analytical SFC: RT = 2.33 min, Column: Chiralcel OD-3 (250 × 4.6 mm), 5 µ, 60% CO₂:MeOH, Flow rate = 3.0 mL/min.

### 3-(3-Chloro-4-fluorophenyl)-1-((S)-3-hydroxybutyl)-1-(1-(1-oxo-1,2-dihydroisoquinolin-4-yl)ethyl)urea (Compounds 213 & 214)

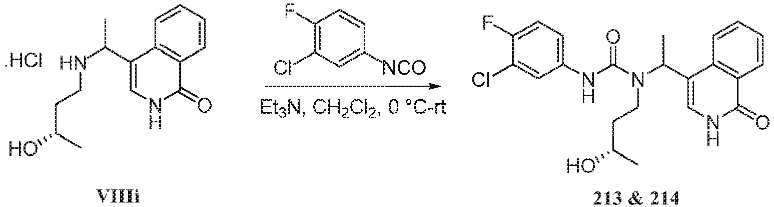

3-(3-Chloro-4-fluoropheny 1)-1 -((S)-3-hy droxy butyl)-1 -(1 -(1 -oxo-1,2-dihydroisoquinolin-4-yl)ethyl)urea was synthesized in an analogous manner as described above from diastereomeric 4-(1-(((*S*)-3-hydroxybutyl)amino)ethyl)isoquinolin-1(2*H*)-one **(VIIIi)** and 2-chloro-1-fluoro-4-isocyanatobenzene. The diastereoisomers were subsequently separated by SFC, Column: Chiralcel OD-H (30 × 250 mm), 5 µ, 75% CO₂:MeOH, Flow rate 100 g/min.

3-(3-Chloro-4-fluorophenyl)-1-((*S*)-3-hydroxybutyl)-1-(1-(1-oxo-1,2-dihydroisoquinolin-4-yl)ethyl)urea: Diastereoisomer I **(Compound 213).** LCMS: *m*/*z* found 432.3/434.3 [M+H]⁺, RT = 7.21 min (Method A); ¹H NMR (400 MHz, DMSO-*d₆*): δ 11.39 (bs, 1H), 8.73 (s, 1H), 8.22-8.25 (m, 1H), 7.80-7.83 (m, 1H), 7.72-7.76 (m, 1H), 7.67 (d, 1H), 7.48-7.52 (m, 1H), 7.42-7.46 (m, 1H), 7.32 (t, 1H), 7.20 (s, 1H), 5.84-5.87 (m, 1H), 5.10 (bs, 1H), 3.41-3.48 (m, 1H), 3.06-3.14 (m, 2H), 1.45 (d, 3H), 1.02-1.14 (m, 2H), 0.86 (d, 3H); Chiral analytical SFC: RT = 3.12 min, Column: Chiralcel OD-3 (4.6 × 250 mm), 3 µ, 60% CO₂:MeOH, Flow = 3.0 mL/min.

3-(3-Chloro-4-fluorophenyl)-1-((*S*)-3-hydroxybutyl)-1-(1-(1-oxo-1,2-dihydroisoquinolin-4-yl)ethyl)urea: Diastereoisomer II **(Compound 214).** LCMS: *m*/*z* found 432.3/434.3 [M+H]⁺, RT = 7.21 min (Method A); ¹H NMR (400 MHz, DMSO-*d₆*): δ 11.39 (bs, 1H), 8.73 (s, 1H), 8.22-8.25 (m, 1H), 7.80-7.83 (m, 1H), 7.72-7.76 (m, 1H), 7.67 (d, 1H), 7.48-7.52 (m, 1H), 7.42-7.46 (m, 1H), 7.32 (t, 1H), 7.20 (s, 1H), 5.84-5.87 (m, 1H), 5.10 (bs, 1H), 3.41-3.48 (m, 1H), 3.06-3.14 (m, 2H), 1.45 (d, 3H), 1.02-1.14 (m, 2H), 0.86 (d, 3H); Chiral analytical SFC: RT = 4.79 min, Column: CHIRALCEL OD-3 (4.6 × 250 mm), 3 µ, 60% CO₂:MeOH, Flow = 3.0 mL/min.

### 4-(1-(((R)-3-Hydroxybutyl)amino)ethyl)isoquinolin-1(2H)-one (VIIIbs)

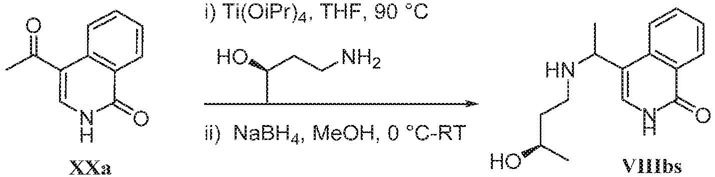

To a solution of 0.3 g (1.6 mmol, 1.0 eq.) of 4-acetylisoquinolin-1(2*H*)-one **(XXa)** in 3 mL of THF under a nitrogen atmosphere was added 0.29 g (3.2 mmol, 2.0 eq.) of (R)-4-aminobutan-2-ol followed by 3 mL of titanium (IV) isopropoxide and the mixture was heated at 90 °C for 16 h. The mixture was allowed to cool to room temperature and then further cooled to 0 °C. The mixture was diluted with 5 mL of methanol and 0.12 g (3.2 mmol, 2.0 eq.) of sodium borohydride was added. The mixture was then allowed to warm to room temperature and stirred for 2 h. The reaction was diluted with 10 mL of water, filtered through CELITE^{®} and the filtrate was extracted with 2 × 100 mL of 10% methanol in methylene chloride. The combined organic extracts were washed with 100 mL of brine, dried (Na₂SO₄), filtered and the solvent was removed *in vacuo.* The residue was purified by flash chromatography (SiO₂, eluting with a linear gradient of 0-6% methanol/methylene chloride) to provide 0.28 g (0.63 mmol, 17%) of 4-(1-(((R)-3-hydroxybutyl)amino)ethyl) isoquinolin-1(2*H*)-one **(VIIIbs).** LCMS: *m*/*z* found 261.2 [M+H]⁺.

### 3-(3-Chloro-4-fluorophenyl)-1-((R)-3-hydroxybutyl)-1-(1-(1-oxo-1,2-dihydroisoquinolin-4-yl)ethyl)urea (Compounds 211 & 212)

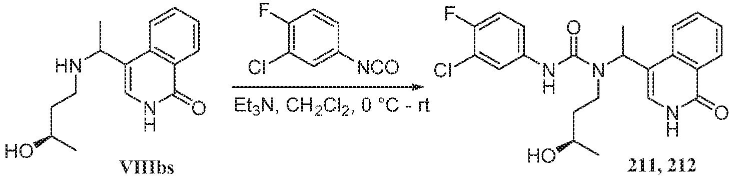

Racemic 3-(3-chloro-4-fluorophenyl)-1-((R)-3-hydroxybutyl)-1-(1-(1-oxo-1,2-dihydroisoquinolin-4-yl)ethyl)urea was synthesized in a similar manner as described above from 4-(1-(((R)-3-hydroxybutyl)amino)ethyl) isoquinolin-1(2*H*)-one **(VIIIbs)** and 2-chloro-1-fluoro-4-isocyanatobenzene. The enantiomers were subsequently separated by SFC, Column: (R,R) Whelk-01 (30 × 250 mm) 5 µ, 65% CO₂:MeOH, flow rate 90 g/min.

3-(3-Chloro-4-fluorophenyl)-1-((R)-3-hydroxybutyl)-1-(1-(1-oxo-1 ,2-dihydroisoquinolin-4-yl)ethyl)urea: Enantiomer I **(Compound 211).** LCMS: *m*/*z* found 432.3/434.3 [M+H]⁺, RT = 7.26 min (Method A); ¹H NMR (400 MHz, DMSO-d₆): 11.39 (bd, 1H), 9.02 (s, 1H), 8.24 (d, 1H), 7.72-7.80 (m, 2H), 7.62 (d, 1H), 7.48-7.52 (m, 1H), 7.30-7.41 (m, 2H), 7.19 (d, 1H), 5.81-5.87 (m, 1H), 5.29 (bs, 1H), 3.10-3.30 (m, 3H), 1.46 (d, 3H), 0.78-0.97 (m, 2H), 0.64 (d, 3H); Chiral analytical SFC: RT = 5.77 min, Column: (R,R) Whelk-01 (250 × 4.6 mm), 5 µ, 60% CO₂:MeOH, Flow rate = 3.0 mL/min.

3-(3-Chloro-4-fluorophenyl)-1-((R)-3-hydroxybutyl)-1-(1-(1-oxo-1 ,2-dihydroisoquinolin-4-yl)ethyl)urea: Enantiomer II **(Compound 212).** LCMS: *m*/*z* found 432.3/434.3 [M+H]⁺, RT = 7.23 min (Method A); ¹H NMR (400 MHz, DMSO-d₆): 11.39 (bd, 1H), 9.02 (s, 1H), 8.24 (d, 1H), 7.72-7.80 (m, 2H), 7.62 (d, 1H), 7.48-7.52 (m, 1H), 7.30-7.41 (m, 2H), 7.19 (d, 1H), 5.81-5.87 (m, 1H), 5.29 (bs, 1H), 3.10-3.30 (m, 3H), 1.46 (d, 3H), 0.78-0.97 (m, 2H), 0.64 (d, 3H); Chiral analytical SFC: RT = 5.85 min, Column: (R,R) Whelk-01 (250 × 4.6 mm), 5 µ, 60% CO₂:MeOH, Flow rate = 3.0 mL/min.

### 4-(1-((3-Hydroxy-2,2-dimethylpropyl)amino)ethyl)isoquinolin-1(2H)-one (VIIIbt)

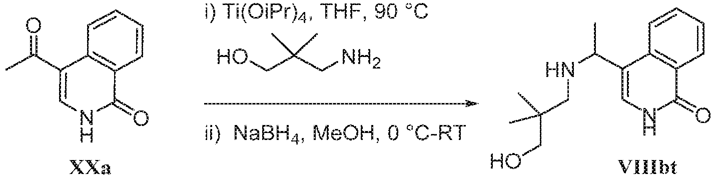

To a solution of 0.5 g (2.7 mmol, 1.0 eq.) of 4-acetylisoquinolin-1(2*H*)-one **(XXa)** in 5 mL of THF under a nitrogen atmosphere was added 0.55 g (5.3 mmol, 2.0 eq.) of 3-amino-2,2-dimethylpropan-1-ol followed by 5 mL of titanium (IV) isopropoxide and the mixture was heated at 90 °C for 16 h. The mixture was allowed to cool to room temperature and then further cooled to 0 °C. The mixture was diluted with 5 mL of methanol and 0.31 g (8.0 mmol, 3.0 eq.) of sodium borohydride was added. The mixture was then allowed to warm to room temperature and stirred for 2 h. The reaction was diluted with 20 mL of water, filtered through CELITE^{®} and the filtrate was extracted with 3 × 30 mL of 10% methanol in methylene chloride. The combined organic extracts were washed with 60 mL of brine, dried (Na₂SO₄), filtered and the solvent was removed *in vacuo.* The residue was purified by flash chromatography (SiO₂, eluting with a linear gradient of 0-6% methanol/methylene chloride) to provide 0.35 g (1.3 mmol, 47%) of 4-(1-((3-hydroxy-2,2-dimethylpropyl)amino)ethyl)isoquinolin-1(2*H*)-one **(VIIIbt).** LCMS: *m*/*z* found 275.3 [M+H]⁺; ¹H NMR (400 MHz, DMSO-d₆) δ 11.15 (bd, 1H), 8.21-8.24 (m, 1H), 7.98 (d, 1H), 7.68-7.73 (m, 1H), 7.45-7.49 (m, 1H), 7.14 (d, 1H), 4.53 (t, 1H), 4.06-4.10 (m, 1H), 3.11-3.16 (m, 2H), 2.31-2.37 (m, 1H), 2.22-2.29 (m, 1H), 1.55 (bs, 1H), 1.33 (d, 3H), 0.79 (s, 3H), 0.78 (s, 3H).

### 3-(3-Chloro-4-fluorophenyl)-1-(3-hydroxy-2,2-dimethylpropyl)-1-(1-(1-oxo-1,2-dihydroisoquinolin-4-yl)ethyl)urea (Compounds 209 & 210)

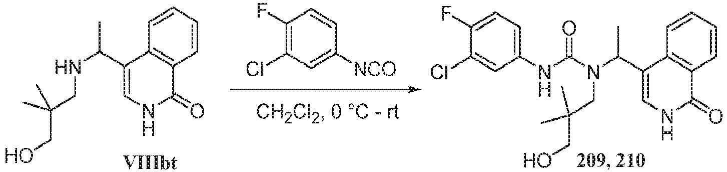

Racemic 3-(3-chloro-4-fluorophenyl)-1-(3-hydroxy-2,2-dimethylpropyl)-1-(1-(1-oxo-1,2-dihydroisoquinolin-4-yl)ethyl)urea was synthesized in a similar manner as described above from 4-(1-((3-hydroxy-2,2-dimethylpropyl)amino)ethyl)isoquinolin-1(2*H*)-one **(VIIIbt)** and 2-chloro-1-fluoro-4-isocyanatobenzene. The enantiomers were subsequently separated by SFC, Column: (R,R) Whelk-01 (30 × 250 mm) 5 µ, 75% CO₂:MeOH, flow rate 70 g/min.

3-(3-Chloro-4-fluorophenyl)-1-(3-hydroxy-2,2-dimethylpropyl)-1-(1-(1-oxo-1,2-dihydroisoquinolin-4-yl)ethyl)urea: Enantiomer I **(Compound 209).** LCMS: *m*/*z* found 446.3/448.3 [M+H]⁺, RT = 7.51 min (Method A); ¹H NMR (400 MHz, DMSO-d₆): 11.38 (bs, 1H), 9.36 (bs, 1H), 8.23 (d, 1H), 7.83-7.86 (m, 1H), 7.73-7.79 (m, 2H), 7.48-7.52 (m, 1H), 7.40-7.44 (m, 1H), 7.33 (t, 1H), 7.21 (s, 1H), 5.93-5.99 (m, 2H), 3.13 (d, 1H), 3.01 (s, 2H), 2.93 (d, 1H), 1.46 (d, 3H), 0.52 (s, 3H), 0.17 (s, 3H); Chiral analytical SFC: RT = 10.83 min, Column: (R,R)-Whelk-01 (250 × 4.6 mm), 5 µ, 70% CO₂:MeOH, Flow rate = 3.0 mL/min.

3-(3-Chloro-4-fluorophenyl)-1-(3-hydroxy-2,2-dimethylpropyl)-1-(1-(1-oxo-1,2-dihydroisoquinolin-4-yl)ethyl)urea: Enantiomer II **(Compound 210).** LCMS: *m*/*z* found 446.3/448.3 [M+H]⁺, RT = 7.47 min (Method A); ¹H NMR (400 MHz, DMSO-d₆): 11.38 (bs, 1H), 9.36 (bs, 1H), 8.23 (d, 1H), 7.83-7.86 (m, 1H), 7.73-7.79 (m, 2H), 7.48-7.52 (m, 1H), 7.40-7.44 (m, 1H), 7.33 (t, 1H), 7.21 (s, 1H), 5.93-5.99 (m, 2H), 3.13 (d, 1H), 3.01 (s, 2H), 2.93 (d, 1H), 1.46 (d, 3H), 0.52 (s, 3H), 0.17 (s, 3H); Chiral analytical SFC: RT = 13.48 min, Column: (R,R)-Whelk-01 (250 × 4.6 mm), 5 µ, 70% CO₂:MeOH, Flow rate = 3.0 mL/min.

### 4-(1-(((R)-2-Hydroxypropyl)amino)ethyl)isoquinolin-1(2H)-one (VIIIbu)

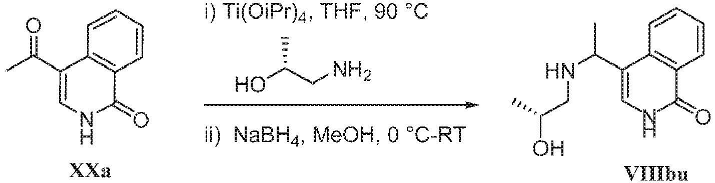

To a solution of 0.5 g (2.7 mmol, 1.0 eq.) of 4-acetylisoquinolin-1(2*H*)-one **(XXa)** in 5 mL of THF under a nitrogen atmosphere was added 0.3 g (4.0 mmol, 1.5 eq.) of (R)-1-aminopropan-2-ol followed by 5 mL of titanium (IV) isopropoxide and the mixture was heated at 90 °C for 16 h. The mixture was allowed to cool to room temperature and then further cooled to 0 °C. The mixture was diluted with 10 mL of methanol and 0.20 g (5.3 mmol, 2.0 eq.) of sodium borohydride was added. The mixture was then allowed to warm to room temperature and stirred for 2 h. The reaction was diluted with 50 mL of water, filtered through CELITE^{®} and the filtrate was extracted with 2 × 50 mL of 10% methanol in methylene chloride. The combined organic extracts were washed with 60 mL of brine, dried (Na₂SO₄), filtered and the solvent was removed *in vacuo.* The residue triturated with 10 mL of 9:1 *v*/*v* diethyl ether/ethanol to provide 0.43 g (1.7 mmol, 65%) of 4-(1-(((R)-2-hydroxypropyl)amino)ethyl)isoquinolin-1(2*H*)-one **(VIIIbu).** LCMS: *m*/*z* found 247.4 [M+H]⁺; ¹H NMR (400 MHz, DMSO-d₆) δ 11.15 (bs, 1H), 8.26 (d, 1H), 7.98-8.03 (m, 1H), 7.73-7.77 (m, 1H), 7.50-7.54 (m, 1H), 7.18 (s, 1H), 4.45-4.49 (m, 1H), 4.02-4.07 (m, 1H), 3.65-3.68 (m, 1H), 2.33-2.50 (m, 2H), 1.98 (bs, 1H), 1.32 (d, 3H), 1.02 (d, 3H).

### 3-(3-Chloro-4-fluorophenyl)-1-((R)-2-hydroxypropyl)-1-(1-(1-oxo-1,2-dihydroisoquinolin-4-yl)ethyl)urea (Compounds 215 & 216)

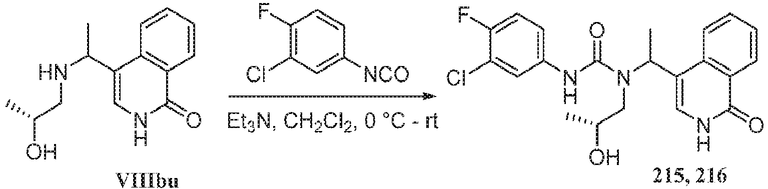

Racemic 3-(3-chloro-4-fluorophenyl)-1-((R)-2-hydroxypropyl)-1-(1-(1-oxo-1,2-dihydroisoquinolin-4-yl)ethyl)urea was synthesized in a similar manner as described above from 4-(1-(((R)-2-hydroxypropyl)amino)ethyl)isoquinolin-1(2*H*)-one **(VIIIbu)** and 2-chloro-1-fluoro-4-isocyanatobenzene. The enantiomers were subsequently separated by SFC, Column: (R,R) Whelk-01 (30 × 250 mm) 5 µ, 60% CO₂:MeOH, flow rate 90 g/min.

3-(3-Chloro-4-fluorophenyl)-1 -((R)-2-hydroxypropyl)-1 -(1 -(1 -oxo-1,2-dihydroisoquinolin-4-yl)ethyl)urea: Enantiomer I **(Compound 215).** LCMS: *m*/*z* found 418.3/420.3 [M+H]⁺, RT = 7.19 min (Method A); ¹H NMR (400 MHz, DMSO-d₆): 11.40 (bs, 1H), 9.62 (bs, 1H), 8.24 (d, 1H), 7.71-7.79 (m, 2H), 7.58 (d, 1H), 7.48-7.52 (m, 1H), 7.31 (t, 1H), 7.23 (s, 1H), 7.15-7.19 (m, 1H), 5.76-5.82 (m, 2H), 3.07-3.10 (m, 2H), 2.73-2.77 (m, 1H), 1.45 (d, 3H), 0.76 (d, 3H); Chiral analytical SFC: RT = 2.22 min, Column: (R,R) Whelk-01 (250 × 4.6 mm), 5 µ, 50% CO₂:MeOH, Flow rate = 3.0 mL/min.

3-(3-Chloro-4-fluorophenyl)-1 -((R)-2-hydroxypropyl)-1 -(1 -(1 -oxo-1,2-dihydroisoquinolin-4-yl)ethyl)urea: Enantiomer II **(Compound 216).** LCMS: *m*/*z* found 418.3/420.3 [M+H]⁺, RT = 7.19 min (Method A); ¹H NMR (400 MHz, DMSO-d₆): 11.40 (bs, 1H), 9.62 (bs, 1H), 8.24 (d, 1H), 7.71-7.79 (m, 2H), 7.58 (d, 1H), 7.48-7.52 (m, 1H), 7.31 (t, 1H), 7.23 (s, 1H), 7.15-7.19 (m, 1H), 5.76-5.82 (m, 2H), 3.07-3.10 (m, 2H), 2.73-2.77 (m, 1H), 1.45 (d, 3H), 0.76 (d, 3H); Chiral analytical SFC: RT = 4.61 min, Column: (R,R) Whelk-01 (250 × 4.6 mm), 5 µ, 50% CO₂:MeOH, Flow rate = 3.0 mL/min.

### 4-(1-(((S)-2-Hydroxypropyl)amino)ethyl)isoquinolin-1(2H)-one (VIIIbv)

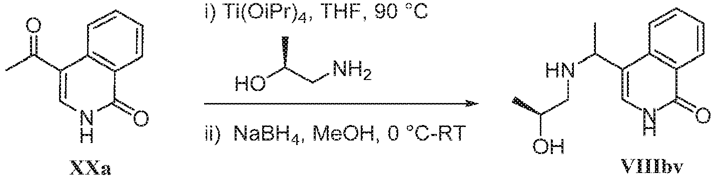

To a solution of 0.4 g (2.1 mmol, 1.0 eq.) of 4-acetylisoquinolin-1(2*H*)-one **(XXa)** in 5 mL of THF under a nitrogen atmosphere was added 0.48 g (6.4 mmol, 3.0 eq.) of (S)-1-aminopropan-2-ol followed by 5 mL of titanium (IV) isopropoxide and the mixture was heated at 90 °C for 16 h. The mixture was allowed to cool to room temperature and then further cooled to 0 °C. The mixture was diluted with 10 mL of methanol and 0.15 g (4.3 mmol, 1.5 eq.) of sodium borohydride was added. The mixture was then allowed to warm to room temperature and stirred for 2 h. The reaction was diluted with 50 mL of water, filtered through CELITE^{®} and the filtrate was extracted with 2 × 100 mL of 10% methanol in methylene chloride. The combined organic extracts were washed with 100 mL of brine, dried (Na₂SO₄), filtered and the solvent was removed *in vacuo.* The residue triturated with 10 mL diethyl ether to provide 0.5 g (2.0 mmol, 95%) of 4-(1-(((S)-2-hydroxypropyl)amino)ethyl)isoquinolin-1(2*H*)-one **(VIIIbv).** LCMS: *m*/*z* found 247.4 [M+H]⁺.

### 3-(3-Chloro-4-fluorophenyl)-1-((S)-2-hydroxypropyl)-1-(1-(1-oxo-1,2-dihydroisoquinolin-4-yl)ethyl)urea (Compounds 217 & 218)

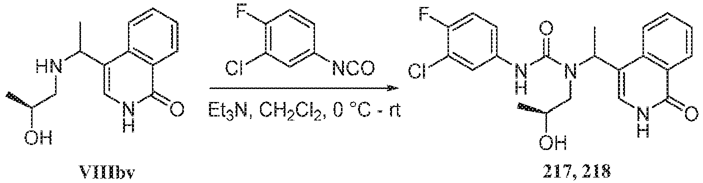

Racemic 3-(3-chloro-4-fluorophenyl)-1-((S)-2-hydroxypropyl)-1-(1-(1-oxo-1,2-dihydroisoquinolin-4-yl)ethyl)urea was synthesized in a similar manner as described above from 4-(1-(((S)-2-hydroxypropyl)amino)ethyl)isoquinolin-1(2*H*)-one **(VIIIbv)** and 2-chloro-1-fluoro-4-isocyanatobenzene. The enantiomers were subsequently separated by SFC, Column: Chiralcel OD-H (30 × 250 mm) 5 µ, 85% CO₂:MeOH, flow rate 70 g/min.

3-(3-Chloro-4-fluorophenyl)-1-((S)-2-hydroxypropyl)-1-(1-(1-oxo-1,2-dihydroisoquinolin-4-yl)ethyl)urea: Enantiomer I **(Compound 217).** LCMS: *m*/*z* found 418.3/420.3 [M+H]⁺, RT = 7.17 min (Method A); ¹H NMR (400 MHz, DMSO-d₆): 11.39 (s, 1H), 9.62 (s, 1H), 8.23 (d, 1H), 7.72-7.80 (m, 3H), 7.50 (t, 1H), 7.25-7.35 (m, 2H), 7.18 (d, 1H), 5.88-5.96 (m, 2H), 3.69 (bs, 1H), 3.02-3.14 (m, 2H), 1.45 (d, 3H), 0.63 (d, 3H); Chiral analytical SFC: RT = 7.66 min, Column: Chiralcel-OD-H (250 × 4.6 mm), 5 µ, 50% CO₂:MeOH, Flow rate = 3.0 mL/min.

3-(3-Chloro-4-fluorophenyl)-1-((S)-2-hydroxypropyl)-1-(1-(1-oxo-1,2-dihydroisoquinolin-4-yl)ethyl)urea: Enantiomer II **(Compound 218).** LCMS: *m*/*z* found 418.3/420.3 [M+H]⁺, RT = 7.15 min (Method A); ¹H NMR (400 MHz, DMSO-d₆): 11.39 (s, 1H), 9.62 (s, 1H), 8.23 (d, 1H), 7.72-7.80 (m, 3H), 7.50 (t, 1H), 7.25-7.35 (m, 2H), 7.18 (d, 1H), 5.88-5.96 (m, 2H), 3.69 (bs, 1H), 3.02-3.14 (m, 2H), 1.45 (d, 3H), 0.63 (d, 3H); Chiral analytical SFC: RT = 10.09 min, Column: Chiralcel-OD-H (250 × 4.6 mm), 5 µ, 50% CO₂:MeOH, Flow rate = 3.0 mL/min.

### 4-(1-(((4-Hydroxycyclohexyl)methyl)amino)ethyl)isoquinolin-1(2H)-one (VIIIbw)

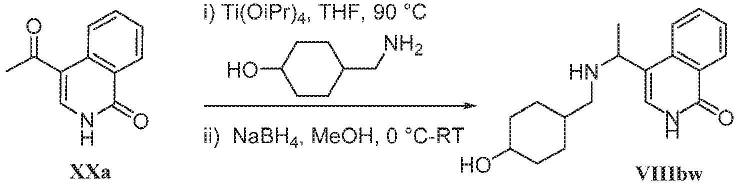

To a solution of 0.3 g (1.6 mmol, 1.0 eq.) of 4-acetylisoquinolin-1(2*H*)-one **(XXa)** in 5 mL of THF under a nitrogen atmosphere was added 0.62 g (4.7 mmol, 3.0 eq.) of 4-(aminomethyl)cyclohexan-1-ol followed by 3 mL of titanium (IV) isopropoxide and the mixture was heated at 90 °C for 16 h. The mixture was allowed to cool to room temperature and then further cooled to 0 °C. The mixture was diluted with 3 mL of methanol and 0.12 g (3.2 mmol, 2.0 eq.) of sodium borohydride was added. The mixture was then allowed to warm to room temperature and stirred for 2 h. The reaction was diluted with 10 mL of water, filtered through CELITE^{®} and the filtrate was extracted with 2 × 10 mL of ethyl acetate. The combined organic extracts were washed with 100 mL of brine, dried (Na₂SO₄), filtered and the solvent was removed *in vacuo* to provide 0.5 g of crude 4-(1-(((4-hydroxycyclohexyl)methyl) amino)ethyl)isoquinolin-1(2*H*)-one **(VIIIbw).** LCMS: *m*/*z* found 301.4 [M+H]⁺.

### 3-(3-Chloro-4-fluorophenyl)-1-((4-hydroaycycloheayl)methyl)-1-(1-(1-oxo-1,2-dihydroisoquinolin-4-yl)ethyl)urea (Compounds 221 & 225)

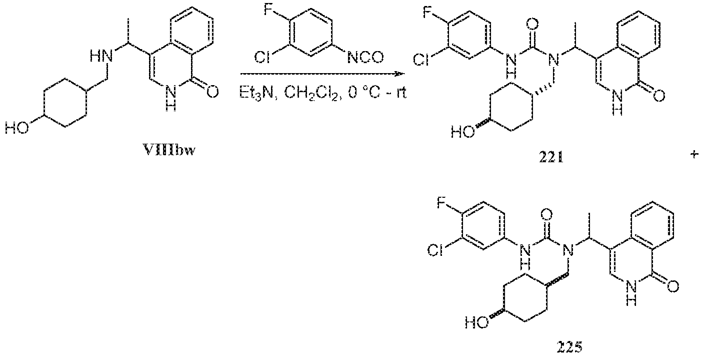

3-(3-Chloro-4-fluorophenyl)-1-((4-hydroxycyclohexyl)methyl)-1-(1-(1-oxo-1,2-dihydroisoquinolin-4-yl)ethyl)urea was synthesized in a similar manner as described above from 4-(1-(((4-hydroxycyclohexyl)methyl)amino)ethyl)isoquinolin-1(2*H*)-one **(VIIIbw).** The *cis-* and *trans*-isomers were subsequently separated by semi-preparative HPLC. Racemic *trans*-3-(3-Chloro-4-fluorophenyl)-1-((4-hydroxycyclohexyl)methyl)-1-(1-(1-oxo-1,2-dihydroisoquinolin-4-yl)ethyl)urea: **(Compound 221).** LCMS: *m*/*z* found 472.3/474.3 [M+H]⁺, RT = 7.07 min (Method A); ¹H NMR (400 MHz, DMSO-d₆): 11.37 (bd, 1H), 8.37 (bs, 1H), 8.25 (d, 1H), 7.80-7.83 (m, 1H), 7.73-7.76 (m, 2H), 7.46-7.53 (m, 2H), 7.32 (t, 1H), 7.21 (d, 1H), 5.85-5.89 (m, 1H), 4.29 (d, 1H), 3.03-3.13 (m, 1H), 2.87-3.01 (m, 2H), 2.48-2.58 (m, 2H), 1.46 (d, 3H), 1.14-1.31 (m, 2H), 0.58-0.80 (m, 4H), 0.36-0.43 (m, 1H).

Racemic *cis*-3-(3-Chloro-4-fluorophenyl)-1-((4-hydroxycyclohexyl)methyl)-1-(1-(1-oxo-1,2-dihydroisoquinolin-4-yl)ethyl)urea: **(Compound 225).** LCMS: *m*/*z* found 472.3/474.3 [M+H]⁺; ¹H NMR (400 MHz, DMSO-d₆): 11.36 (bd, 1H), 8.43 (bs, 1H), 8.24 (d, 1H), 7.72-7.83 (m, 3H), 7.46-7.52 (m, 2H), 7.32 (t, 1H), 7.21 (d, 1H), 5.84-5.87 (m, 1H), 4.10 (d, 1H), 3.53-3.56 (m, 1H), 2.89-3.01 (m, 2H), 1.47 (d, 3H), 1.31-1.38 (m, 2H), 1.15-1.22 (m, 1H), 0.87-1.06 (m, 5H), 0.71-.080 (m, 1H).

### 3-(3-Chloro-4-fluorophenyl)-1-((2,2-dimethyl-1,3-dioxan-5-yl)methyl)-1-(1-(1-oxo-1,2-dihydroisoquinolin-4-yl)ethyl)urea (Compounds 272 & 273)

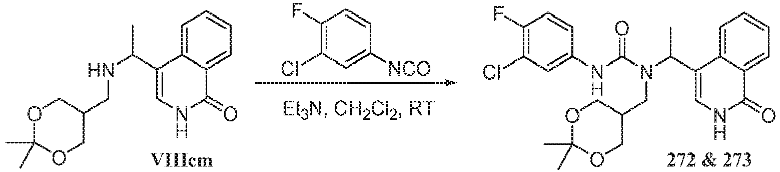

Racemic 3-(3-chloro-4-fluorophenyl)-1-((2,2-dimethyl-1,3-dioxan-5-yl)methyl)-1-(1-(1-oxo-1,2-dihydroisoquinolin-4-yl)ethyl)urea was synthesized in a similar manner as described above from 4-(1-(((2,2-dimethyl-1,3-dioxan-5-yl)methyl)amino)ethyl)isoquinolin-1(2*H*)-one **(VIIIcm)** and 2-chloro-1-fluoro-4-isocyanatobenzene. The enantiomers were subsequently separated by SFC, Column: Chiralcel OD-H (30 × 250 mm) 5 µ, 65% CO₂:MeOH, flow rate 70 g/min.

3-(3-Chloro-4-fluorophenyl)-1-((2,2-dimethyl-1,3-dioxan-5-yl)methyl)-1-(1-(1-oxo-1,2-dihydroisoquinolin-4-yl)ethyl)urea- Enantiomer I **(Compound 272):** LCMS: *m*/*z* found 488.1/490.1 [M+H]⁺, RT = 4.98 min (method A); ¹H NMR (400 MHz, DMSO-*d₆*) δ 11.39 (bs, 1H), 8.47 (s, 1H), 8.22-8.24 (m, 1H), 7.73-7.79 (m, 2H), 7.66 (d, 1H), 7.49-7.53 (m, 1H), 7.31-7.40 (m, 2H), 7.20 (d, 1H), 5.84-5.88 (m, 1H), 3.56-3.63 (m, 2H), 3.27-3.33 (m, 2H), 3.13-3.20 (m, 2H), 1.49 (d, 3H), 1.26 (s, 3H), 1.23 (s, 3H), 1.05-1.09 (m, 1H); Chiral analytical SFC: RT = 1.80 min, Column: Chiralcel OD-3 (4.6 × 150 mm), 5 µ, 70% CO₂/MeOH, Flow = 4.0 g/min.

3-(3-Chloro-4-fluorophenyl)-1-((2,2-dimethyl-1,3-dioxan-5-yl)methyl)-1-(1-(1-oxo-1,2-dihydroisoquinolin-4-yl)ethyl)urea- Enantiomer II **(Compound 273):** LCMS: *m*/*z* found 488.1/490.1 [M+H]⁺, RT = 4.98 min (method A); ¹H NMR (400 MHz, DMSO-*d₆*) δ 11.39 (bs, 1H), 8.47 (s, 1H), 8.22-8.24 (m, 1H), 7.73-7.79 (m, 2H), 7.66 (d, 1H), 7.49-7.53 (m, 1H), 7.31-7.40 (m, 2H), 7.20 (d, 1H), 5.84-5.88 (m, 1H), 3.56-3.63 (m, 2H), 3.27-3.33 (m, 2H), 3.13-3.20 (m, 2H), 1.49 (d, 3H), 1.26 (s, 3H), 1.23 (s, 3H), 1.05-1.09 (m, 1H); Chiral analytical SFC: RT = 3.00 min, Column: Chiralcel OD-3 (4.6 × 150 mm), 5 µ, 70% CO₂/MeOH, Flow = 4.0 g/min.

### 3-(3-Chloro-4-fluorophenyl)-1-(3-hydroxy-2-(hydroxymethyl)propyl)-1-(1-(1-oxo-1,2-dihydro isoquinolin-4-yl)ethyl)urea (Compound 284)

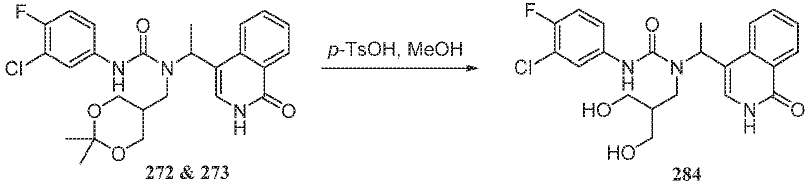

To a solution of 0.2 g (0.41 mmol, 1.0 eq.) of racemic 3-(3-chloro-4-fluorophenyl)-1-((2,2-dimethyl-1,3-dioxan-5-yl)methyl)-1-(1-(1-oxo-1,2-dihydroisoquinolin-4-yl)ethyl)urea **(Compounds 272 & 273)** in 5 mL of methanol was added 8 mg (0.04 mml, 0.1 eq.) of *para*toluene sulfonic acid monohydrate and the mixture was stirred at room temperature for 2 h. The mixture was diluted with 10 mL of saturated sodium bicarbonate solution and extracted with 3 × 30 mL of ethyl acetate. The combined organic extracts were dried (Na₂SO₄) and the solvent was removed *in vacuo.* The residue was purified by MPLC (REVELERIS^{®} Silica column, eluting with a linear gradient of 0-10% methanol/methylene chloride) to provide 0.13 g (0.29 mmol, 70%) of racemic 3-(3-chloro-4-fluorophenyl)-1-(3-hydroxy-2-(hydroxymethyl)propyl)-1-(1-(1-oxo-1,2-dihydroisoquinolin-4-yl)ethyl)urea. The enantiomers were subsequently separated by SFC, Column: Lux Cellulose-2 (30 × 250 mm) 5 µ, 70% CO₂:MeOH, flow rate 70 g/min.

3-(3-Chloro-4-fluorophenyl)-1-(3-hydroxy-2-(hydroxymethyl)propyl)-1-(1-(1-oxo-1,2-dihydro isoquinolin-4-yl)ethyl)urea- Enantiomer I: LCMS: *m*/*z* found 448.2/450.2 [M+H]⁺, RT = 3.78 min (Method A); ¹H NMR (400 MHz, DMSO-*d₆*) δ 11.35 (bs, 1H), 9.29 (s, 1H), 8.22-8.24 (m, 1H), 7.72-7.80 (m, 2H), 7.62 (d, 1H), 7.48-7.52 (m, 1H), 7.30-7.37 (m, 2H), 7.15 (d, 1H), 5.89 (d, 1H), 5.42 (bs, 1H), 4.58 (bs, 1H), 3.10-3.18 (m, 3H), 2.99-3.05 (m, 3H), 1.46 (d, 3H), 0.94-0.99 (m, 1H); Chiral analytical SFC: RT = 3.39 min, Column: Chiralcel OZ-3 (4.6 × 150 mm), 3 µ, 75% CO₂/MeOH, Flow = 4.0 g/min.

3-(3-Chloro-4-fluorophenyl)-1-(3-hydroxy-2-(hydroxymethyl)propyl)-1-(1-(1-oxo-1,2-dihydro isoquinolin-4-yl)ethyl)urea- Enantiomer II **(Compound 284):** LCMS: *m*/*z* found 448.2/450.2 [M+H]⁺, RT = 3.78 min (Method A); ¹H NMR (400 MHz, DMSO-*d₆*) δ 11.35 (bs, 1H), 9.29 (s, 1H), 8.22-8.24 (m, 1H), 7.72-7.80 (m, 2H), 7.62 (d, 1H), 7.48-7.52 (m, 1H), 7.30-7.37 (m, 2H), 7.15 (d, 1H), 5.89 (d, 1H), 5.42 (bs, 1H), 4.58 (bs, 1H), 3.10-3.18 (m, 3H), 2.99-3.05 (m, 3H), 1.46 (d, 3H), 0.94-0.99 (m, 1H); Chiral analytical SFC: RT = 5.67 min, Column: Chiralcel OZ-3 (4.6 × 150 mm), 3 µ, 75% CO₂/MeOH, Flow = 4.0 g/min.

### 3-(4-Fluorophenyl)-1-((2,2-dimethyl-1,3-dioxan-5-yl)methyl)-1-(1-(1-oxo-1,2-dihydroisoquinolin-4-yl)ethyl)urea (Compound 274)

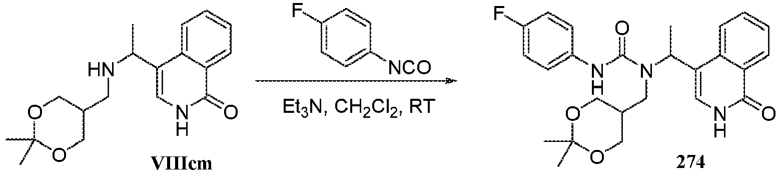

Racemic 3-(4-fluorophenyl)-1-((2,2-dimethyl-1,3-dioxan-5-yl)methyl)-1-(1-(1-oxo-1,2-dihydroisoquinolin-4-yl)ethyl)urea was synthesized in a similar manner as described above from 4-(1-(((2,2-dimethyl-1,3-dioxan-5-yl)methyl)amino)ethyl)isoquinolin-1(2*H*)-one **(VIIIcm)** and 4-fluorophenyl isocyanate. The enantiomers were subsequently separated by SFC, Column: Chiralpak IC (30 × 250 mm) 5 µ, 60% CO₂:MeOH, flow rate 70 g/min.

3-(4-Fluorophenyl)-1-((2,2-dimethyl-1,3-dioxan-5-yl)methyl)-1-(1-(1-oxo-1,2-dihydroisoquinolin-4-yl)ethyl)urea - Enantiomer I: LCMS: *m*/*z* found 454.3 [M+H]⁺, RT = 4.28 min (Method A); ¹H NMR (400 MHz, DMSO-*d₆*) δ 11.40 (bs, 1H), 8.33 (bs, 1H), 8.22 (m, 1H), 7.67-7.77 (m, 2H), 7.43-7.53 (m, 3H), 7.19 (d, 1H), 7.10-7.15 (m, 2H), 5.84-5.90 (m, 1H), 3.58-3.62 (m, 2H), 3.26-3.34 (m, 2H), 3.14-3.20 (m, 2H), 1.49 (d, 3H), 1.26 (s, 3H), 1.24 (s, 3H), 1.01-1.05 (m, 1H); Chiral analytical SFC: RT = 2.31 min, Column: Chiralpak AD-3 (4.6 × 150 mm), 3 µ, 70% CO₂/MeOH, Flow = 4.0 g/min.

3-(4-Fluorophenyl)-1-((2,2-dimethyl-1,3-dioxan-5-yl)methyl)-1-(1-(1-oxo-1,2-dihydroisoquinolin-4-yl)ethyl)urea - Enantiomer II **(Compound 274):** LCMS: *m*/*z* found 454.3 [M+H]⁺, RT = 4.28 min (Method A); ¹H NMR (400 MHz, DMSO-*d₆*) δ 11.40 (bs, 1H), 8.33 (bs, 1H), 8.22 (m, 1H), 7.67-7.77 (m, 2H), 7.43-7.53 (m, 3H), 7.19 (d, 1H), 7.10-7.15 (m, 2H), 5.84-5.90 (m, 1H), 3.58-3.62 (m, 2H), 3.26-3.34 (m, 2H), 3.14-3.20 (m, 2H), 1.49 (d, 3H), 1.26 (s, 3H), 1.24 (s, 3H), 1.01-1.05 (m, 1H); Chiral analytical SFC: RT = 4.13 min, Column: Chiralpak AD-3 (4.6 × 150 mm), 3 µ, 70% CO₂/MeOH, Flow = 4.0 g/min.

### 1-((2,2-Dimethyl-1,3-dioxan-5-yl)methyl)-1-(1-(1-oxo-1,2-dihydroisoquinolin-4-yl)ethyl)-3-phenylurea (Compound 275)

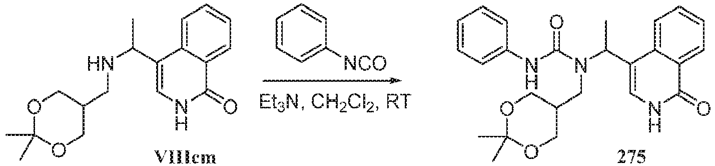

Racemic 1-((2,2-dimethyl-1,3-dioxan-5-yl)methyl)-1-(1-(1-oxo-1,2-dihydroisoquinolin-4-yl)ethyl)-3-phenylurea was synthesized in a similar manner as described above from 4-(1-(((2,2-dimethyl-1,3-dioxan-5-yl)methyl)amino)ethyl)isoquinolin-1(2*H*)-one **(VIIIcm)** and phenyl isocyanate. The enantiomers were subsequently separated by SFC, Column: Chiralpak IC (30 × 250 mm) 5 µ, 60% CO₂:MeOH, flow rate 70 g/min.

1-((2,2-Dimethyl-1,3-dioxan-5-yl)methyl)-1-(1-(1-oxo-1,2-dihydroisoquinolin-4-yl)ethyl)-3-phenylurea - Enantiomer I: LCMS: *m*/*z* found 436.1 [M+H]⁺, RT = 4.16 min (Method A); ¹H NMR (400 MHz, DMSO-*d₆*) δ 11.38 (bd, 1H), 8.31 (bs, 1H), 8.23 (dd, 1H), 7.68-7.77 (m, 2H), 7.45-7.53 (m, 3H), 7.25-7.30 (m, 2H), 7.20 (d, 1H), 6.96-7.00 (m, 1H), 5.87-5.91 (m, 1H), 3.57-3.65 (m, 2H), 3.28-3.38 (m, 2H), 3.16-3.22 (m, 2H), 1.49 (d, 3H), 1.27 (s, 3H), 1.24 (s, 3H), 0.97-1.02 (m, 1H); Chiral analytical SFC: RT = 2.75 min, Column: Chiralpak IC-3 (4.6 × 150 mm), 3 µ, 60% CO₂/MeOH, Flow = 3.0 g/min.

1-((2,2-Dimethyl-1,3-dioxan-5-yl)methyl)-1-(1-(1-oxo-1,2-dihydroisoquinolin-4-yl)ethyl)-3-phenylurea - Enantiomer II **(Compound 275):** LCMS: *m*/*z* found 436.1 [M+H]⁺, RT = 4.16 min (Method A); ¹H NMR (400 MHz, DMSO-*d₆*) δ 11.38 (bd, 1H), 8.31 (bs, 1H), 8.23 (dd, 1H), 7.68-7.77 (m, 2H), 7.45-7.53 (m, 3H), 7.25-7.30 (m, 2H), 7.20 (d, 1H), 6.96-7.00 (m, 1H), 5.87-5.91 (m, 1H), 3.57-3.65 (m, 2H), 3.28-3.38 (m, 2H), 3.16-3.22 (m, 2H), 1.49 (d, 3H), 1.27 (s, 3H), 1.24 (s, 3H), 0.97-1.02 (m, 1H); Chiral analytical SFC: RT = 4.77 min, Column: Chiralpak IC-3 (4.6 × 150 mm), 3 µ, 60% CO₂/MeOH, Flow = 3.0 g/min.

### 4-(1-((Cyclohexylmethyl)amino)ethyl)isoquinolin-1(2H)-one (VIIIau)

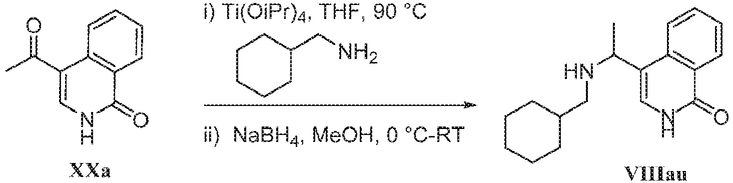

To a solution of 0.5 g (2.7 mmol, 1.0 eq.) of 4-acetylisoquinolin-1(2*H*)-one **(XXa)** in 5 mL of THF under a nitrogen atmosphere was added 0.45 g (4.0 mmol 1.5 eq.) of cyclohexylmethanamine followed by 5 mL of titanium (IV) isopropoxide and the mixture was heated at 90 °C for 16 h. The mixture was allowed to cool to room temperature and then further cooled to 0 °C. The mixture was diluted with 5 mL of methanol and 0.20 g (5.3 mmol, 2.0 eq.) of sodium borohydride was added. The mixture was then allowed to warm to room temperature and stirred for 2 h. The reaction was filtered through CELITE^{®} and the pad was washed with 5 mL of methanol. The filtrate was concentrated *in vacuo* and the residue was triturated with 15 mL of n-pentane to provide 0.65 g (2.28 mmol, 52%) of 4-(1-((cyclohexylmethyl)amino)ethyl)isoquinolin-1(2*H*)-one **(VIIIau).** LCMS: *m*/*z* found 285.1 [M+H]⁺; ¹H NMR (400 MHz, DMSO-d₆): 10.73 (bs, 1H), 8.49 (d, 1H), 7.91-7.94 (m, 1H), 7.68-7.73 (m, 1H), 7.49-7.53 (m, 1H), 7.33 (s, 1H), 4.08-4.14 (m, 1H), 2.37-2.49 (m, 2H), 1.63-1.81 (m, 6H), 1.42 (d, 3H), 1.12-1.29 (m, 4H), 0.85-0.95 (m, 2H).

### 3-(3-Chloro-4-fluorophenyl)-1-(cyclohexylmethyl)-1-(1-(1-oxo-1,2-dihydroisoquinolin-4-yl)ethyl)urea (Compounds 194 & 195)

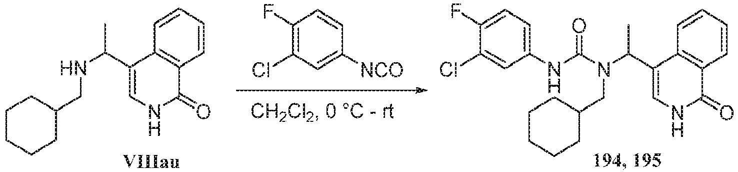

Racemic 3-(3-chloro-4-fluorophenyl)-1-(cyclohexylmethyl)-1-(1-(1-oxo-1,2-dihydroisoquinolin-4-yl)ethyl)urea was synthesized in a similar manner as described above from 4-(1-((cyclohexylmethyl)amino)ethyl)isoquinolin-1(2*H*)-one **(VIIIau)** and 2-chloro-1-fluoro-4-isocyanatobenzene. The enantiomers were subsequently separated by SFC, Column: Lux Cellulose-2 (30 × 250 mm) 5 µ, 70% CO₂:MeOH, flow rate 70 g/min.

3-(3-Chloro-4-fluorophenyl)-1-(cyclohexylmethyl)-1-(1-(1-oxo-1,2-dihydroisoquinolin-4-yl)ethyl)urea: Enantiomer I **(Compound 194).** LCMS: *m*/*z* found 456.3/458.3 [M+H]⁺, 7.71 min (Method A); ¹H NMR (400 MHz, DMSO-d₆): 11.39 (bs, 1H), 8.38 (bs, 1H), 8.24 (d, 1H), 7.81-7.84 (m, 1H), 7.72-7.78 (m, 2H), 7.46-7.52 (m, 2H), 7.32 (t, 1H), 7.21 (d, 1H), 5.85-5.88 (m, 1H), 2.86-3.00 (m, 2H), 1.31-1.48 (m, 7H), 1.18-1.23 (m, 1H), 0.81-0.92 (m, 2H), 0.69-0.80 (m, 2H), 0.49-0.60 (m, 2H); Chiral analytical SFC: RT = 2.97 min, Column: Chiralcel OZ-3 (150 × 4.6 mm), 3 µ, 75% CO₂:MeOH, Flow rate = 3.0 mL/min.

3-(3-Chloro-4-fluorophenyl)-1-(cyclohexylmethyl)-1-(1-(1-oxo-1,2-dihydroisoquinolin-4-yl)ethyl)urea: Enantiomer II **(Compound 195).** LCMS: *m*/*z* found 456.3/458.3 [M+H]⁺, 7.68 min (Method A); ¹H NMR (400 MHz, DMSO-d₆): 11.39 (bs, 1H), 8.38 (bs, 1H), 8.24 (d, 1H), 7.81-7.84 (m, 1H), 7.72-7.78 (m, 2H), 7.46-7.52 (m, 2H), 7.32 (t, 1H), 7.21 (d, 1H), 5.85-5.88 (m, 1H), 2.86-3.00 (m, 2H), 1.31-1.48 (m, 7H), 1.18-1.23 (m, 1H), 0.81-0.92 (m, 2H), 0.69-0.80 (m, 2H), 0.49-0.60 (m, 2H); Chiral analytical SFC: RT = 5.11 min, Column: Chiralcel OZ-3 (150 × 4.6 mm), 3 µ, 75% CO₂:MeOH, Flow rate = 3.0 mL/min.

### 4-(1-(((1-Methylpiperidin-4-yl)methyl)amino)ethyl)isoquinolin-1(2H)-one (VIIIav)

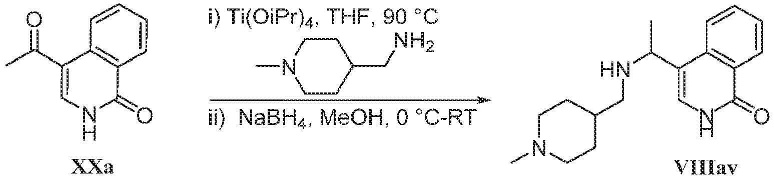

To a solution of 0.5 g (2.7 mmol, 1.0 eq.) of 4-acetylisoquinolin-1(2*H*)-one **(XXa)** in 5 mL of THF under a nitrogen atmosphere was added 1.7 g (13.3 mmol 5.0 eq.) of (1-methylpiperidin-4-yl)methanamine followed by 5 mL of titanium (IV) isopropoxide and the mixture was heated at 90 °C for 16 h. The mixture was allowed to cool to room temperature and then further cooled to 0 °C. The mixture was diluted with 2 mL of methanol and 0.19 g (5.3 mmol, 2.0 eq.) of sodium borohydride was added. The mixture was then allowed to warm to room temperature and stirred for 2 h. The reaction was quenched by the addition of 50 mL of water and filtered through CELITE^{®}. The pad was washed with 5 mL of ethyl acetate and the filtrate was extracted with 3 × 50 mL of 10% methanol in methylene chloride. The combined organic extracts were washed with 100 mL of brine dried (Na₂SO₄), filtered and the solvent was removed *in vacuo* to provide 0.26 g (0.79 mmol, 37%) of 4-(1-(((1-methylpiperidin-4-yl)methyl)amino)ethyl)isoquinolin-1(2*H*)-one **(VIIIav).** LCMS: *m*/*z* found 300.3 [M+H]⁺.

### 3-(3-Chloro-4-fluorophenyl)-1-((1-methylpiperidin-4-yl)methyl)-1-(1-(1-oxo-1,2-dihydroisoquinolin-4-yl)ethyl)urea (Compound 207)

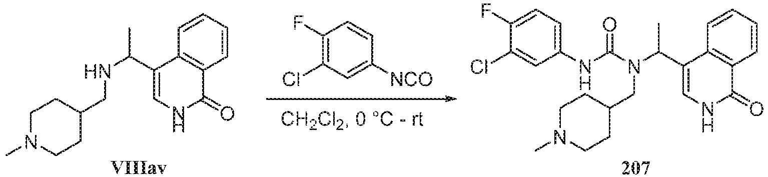

Racemic 3-(3-chloro-4-fluorophenyl)-1-((1-methylpiperidin-4-yl)methyl)-1-(1-(1-oxo-1,2-dihydroisoquinolin-4-yl)ethyl)urea was synthesized in a similar manner as described above from 4-(1-(((1-methylpiperidin-4-yl)methyl)amino)ethyl)isoquinolin-1(2*H*)-one **(VIIIav)** and 2-chloro-1-fluoro-4-isocyanatobenzene. The enantiomers were subsequently separated by SFC, Column: Lux cellulose-2 (30 × 250 mm) 5 µ, 60% CO₂:MeOH, flow rate 90 g/min.

3-(3-Chloro-4-fluorophenyl)-1-((1-methylpiperidin-4-yl)methyl)-1-(1-(1-oxo-1,2-dihydroisoquinolin-4-yl)ethyl)urea: Enantiomer I. LCMS: *m*/*z* found 471.4/473.4 [M+H]⁺, RT = 5.25 min (Method: A); ¹H NMR (400 MHz, DMSO-d₆) δ 11.37 (bs, 1H), 8.41 (bs, 1H), 8.24 (d, 1H), 7.72-7.83 (m, 3H), 7.47-7.52 (m, 2H), 7.32 (t, 1H), 7.21 (s, 1H), 5.86-5.88 (m, 1H), 3.02-3.06 (m, 1H), 2.89-2.94 (m, 1H), 2.42-2.50 (m, 2H), 1.93 (s, 3H), 1.45-1.47 (m, 3H), 1.20-1.33 (m, 3H), 0.97-1.04 (m, 2H), 0.72-0.83 (m, 2H); Chiral analytical SFC: RT = 3.92 min, Column: Lux cellulose-2 (250 × 4.6 mm), 5 µ, 60% CO₂:MeOH, Flow rate = 3.0 mL/min.

3-(3-Chloro-4-fluorophenyl)-1-((1-methylpiperidin-4-yl)methyl)-1-(1-(1-oxo-1,2-dihydroisoquinolin-4-yl)ethyl)urea: Enantiomer II **(Compound 207).** LCMS: *m*/*z* found 471.4/473.4 [M+H]⁺, RT = 5.24 min (Method: A); ¹H NMR (400 MHz, DMSO-d₆) δ 11.37 (bs, 1H), 8.41 (bs, 1H), 8.24 (d, 1H), 7.72-7.83 (m, 3H), 7.47-7.52 (m, 2H), 7.32 (t, 1H), 7.21 (s, 1H), 5.86-5.88 (m, 1H), 3.02-3.06 (m, 1H), 2.89-2.94 (m, 1H), 2.42-2.50 (m, 2H), 1.93 (s, 3H), 1.45-1.47 (m, 3H), 1.20-1.33 (m, 3H), 0.97-1.04 (m, 2H), 0.72-0.83 (m, 2H); Chiral analytical SFC: RT = 7.29 min, Column: Lux cellulose-2 (250 × 4.6 mm), 5 µ, 60% CO₂:MeOH, Flow rate = 3.0 mL/min.

### 4-(1-(((1-Acetylpiperidin-4-yl)methyl)amino)ethyl)isoquinolin-1(2H)-one (VIIIaw)

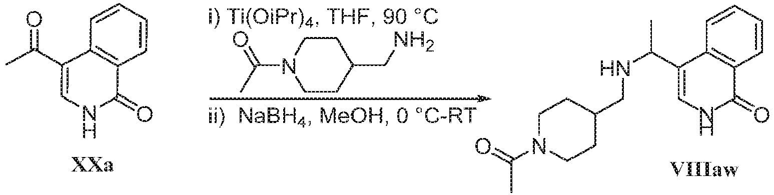

To a solution of 0.3 g (1.6 mmol, 1.0 eq.) of 4-acetylisoquinolin-1(2*H*)-one **(XXa)** in 4 mL of THF under a nitrogen atmosphere was added 0.5 g (3.2 mmol 1.5 eq.) of 1-(4-(aminomethyl)piperidin-1-yl)ethan-1-one followed by 4 mL of titanium (IV) isopropoxide and the mixture was heated at 90 °C for 6 h. The mixture was allowed to cool to room temperature and then further cooled to 0 °C. The mixture was diluted with 2 mL of methanol and 0.16 g (4.3 mmol, 2.0 eq.) of sodium borohydride was added. The mixture was then allowed to warm to room temperature and stirred for 2 h. The reaction was quenched by the addition of 20 mL of water and filtered through CELITE^{®}. The pad was washed with 5 mL of ethyl acetate and the filtrate was extracted with 3 × 50 mL of ethyl acetate. The combined organic extracts were washed with 30 mL of brine dried (Na₂SO₄), filtered and the solvent was removed *in vacuo* to provide 0.26 g (0.79 mmol, 37%) of 4-(1-(((1-acetylpiperidin-4-yl)methyl)amino) ethyl)isoquinolin-1(2*H*)-one **(VIIIaw).** LCMS: *m*/*z* found 328.5 [M+H]⁺; ¹H NMR (400 MHz, DMSO-d₆) δ 11.16 (bs, 1H), 8.21-8.25 (m, 1H), 8.02 (d, 1H), 7.67-7.73 (m, 1H), 7.45-7.49 (m, 1H), 7.14 (d, 1H), 4.28-4.34 (m, 1H), 3.98-4.04 (m, 1H), 3.69-3.77 (m, 1H), 2.91-2.99 (m, 1H), 2.22-2.49 (m, 3H), 1.95 (s, 3H), 1.54-1.79 (m, 4H), 1.32 (d, 3H), 0.86-1.05 (m, 2H).

### 1-((1-Acetylpiperidin-4-yl)methyl)-3-(3-chloro-4-fluorophenyl)-1-(1-(1-oxo-1,2-dihydroisoquinolin-4-yl)ethyl)urea (Compounds 201 & 202)

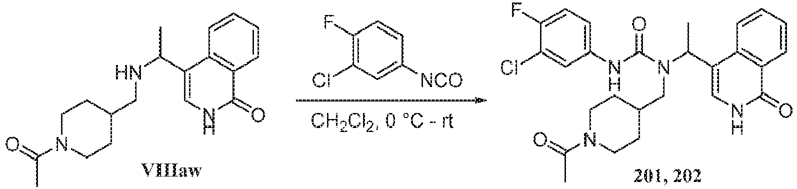

Racemic 1-((1-acetylpiperidin-4-yl)methyl)-3-(3-chloro-4-fluorophenyl)-1-(1-(1-oxo-1,2-dihydroisoquinolin-4-yl)ethyl)urea was synthesized in a similar manner as described above from 4-(1-(((1-acetylpiperidin-4-yl)methyl)amino) ethyl)isoquinolin-1(2*H*)-one **(VIIIaw)** and 2-chloro-1-fluoro-4-isocyanatobenzene. The enantiomers were subsequently separated by SFC, Column: Chiralpak IG (30 × 250 mm) 5 µ, 60% CO₂:MeOH, flow rate 70 g/min.

1-((1-Acetylpiperidin-4-yl)methyl)-3-(3-chloro-4-fluorophenyl)-1-(1-(1-oxo-1,2-dihydroisoquinolin-4-yl)ethyl)urea: Enantiomer I **(Compound 201).** LCMS: *m*/*z* found 499.4/501.4 [M+H]⁺, RT = 7.06 min (Method: A); ¹H NMR (400 MHz, DMSO-d₆) δ 11.42 (bs, 1H), 8.46 (bs, 1H), 8.22 (d, 1H), 7.77-7.80 (m, 1H), 7.66-7.72 (m, 2H), 7.45-7.50 (m, 2H), 7.29 (t, 1H), 7.20 (s, 1H), 5.80-5.85 (m, 1H), 4.05-4.17 (m, 1H), 3.51-3.58 (m, 1H), 2.91-2.99 (m, 2H), 2.29-2.54 (m, 1H), 1.81-2.01 (m, 4H), 1.44-1.45 (m, 3H), 1.07-1.31 (m, 3H), 0.70-0.94 (m, 2H); Chiral analytical SFC: RT = 1.31 min, Column: Chiralpak IG-3 (150 × 4.6 mm), 3 µ, 60% CO₂:MeOH, Flow rate = 3.0 mL/min.

1-((1-Acetylpiperidin-4-yl)methyl)-3-(3-chloro-4-fluorophenyl)-1-(1-(1-oxo-1,2-dihydroisoquinolin-4-yl)ethyl)urea: Enantiomer II **(Compound 202).** LCMS: *m*/*z* found 499.4/501.4 [M+H]⁺, RT = 7.06 min (Method: A); ¹H NMR (400 MHz, DMSO-d₆) δ 11.42 (bs, 1H), 8.46 (bs, 1H), 8.22 (d, 1H), 7.77-7.80 (m, 1H), 7.66-7.72 (m, 2H), 7.45-7.50 (m, 2H), 7.29 (t, 1H), 7.20 (s, 1H), 5.80-5.85 (m, 1H), 4.05-4.17 (m, 1H), 3.51-3.58 (m, 1H), 2.91-2.99 (m, 2H), 2.29-2.54 (m, 1H), 1.81-2.01 (m, 4H), 1.44-1.45 (m, 3H), 1.07-1.31 (m, 3H), 0.70-0.94 (m, 2H); Chiral analytical SFC: RT = 1.99 min, Column: Chiralpak IG-3 (150 × 4.6 mm), 3 µ, 60% CO₂:MeOH, Flow rate = 3.0 mL/min.

### 4-(1-(((1-(Methylsulfonyl)piperidin-4-yl)methyl)amino)ethyl)isoquinolin-1(2H)-one (VIIIax)

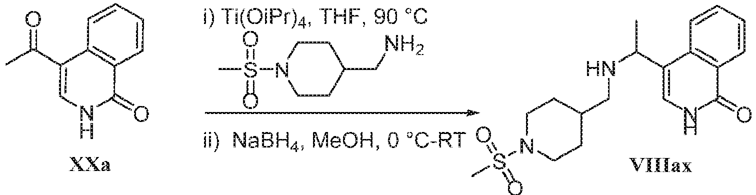

To a solution of 0.3 g (1.6 mmol, 1.0 eq.) of 4-acetylisoquinolin-1(2*H*)-one **(XXa)** in 3 mL of THF under a nitrogen atmosphere was added 0.46 g (2.4 mmol 1.5 eq.) of (1-(methylsulfonyl)piperidin-4-yl)methanamine followed by 3 mL of titanium (IV) isopropoxide and the mixture was heated at 90 °C for 16 h. The mixture was allowed to cool to room temperature and then further cooled to 0 °C. The mixture was diluted with 2 mL of methanol and 0.12 g (3.2 mmol, 2.0 eq.) of sodium borohydride was added. The mixture was then allowed to warm to room temperature and stirred for 2 h. The reaction was quenched by the addition of 20 mL of water and filtered through CELITE^{®}. The pad was washed with 5 mL of ethyl acetate and the filtrate was extracted with 3 × 50 mL of ethyl acetate. The combined organic extracts were washed with 30 mL of brine, dried (Na₂SO₄), filtered and the solvent was removed *in vacuo.* The residue was triturated with 10 mL of *n*-pentane to provide 0.40 g (1.1 mmol, 68%) of 4-(1-(((1-(methylsulfonyl)piperidin-4-yl)methyl)amino)ethyl)isoquinolin-1(2*H*)-one (**VIIIax**). LCMS: *m*/*z* found 364.4 [M+H]⁺.

### 3-(3-Chloro-4-fluorophenyl)-1-((1-(methylsulfonyl)piperidin-4-yl)methyl)-1-(1-(1-oxo-1,2-dihydroisoquinolin-4-yl)ethyl)urea (Compounds 203 & 204)

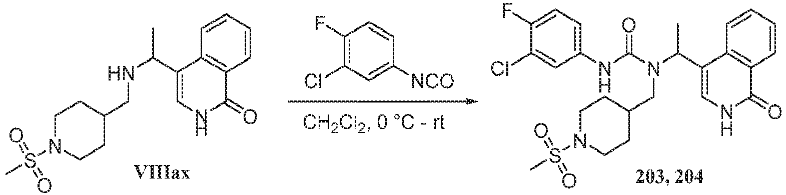

Racemic 3-(3-chloro-4-fluorophenyl)-1-((1-(methylsulfonyl)piperidin-4-yl)methyl)-1-(1-(1-oxo-1,2-dihydroisoquinolin-4-yl)ethyl)urea was synthesized in a similar manner as described above from 4-(1-(((1-(methylsulfonyl)piperidin-4-yl)methyl)amino)ethyl)isoquinolin-1(2*H*)-one **(VIIIax)** and 2-chloro-1-fluoro-4-isocyanatobenzene. The enantiomers were subsequently separated by SFC, Column: Chiralcel OD-H (30 × 250 mm) 5 µ, 65% CO₂:MeOH, flow rate 100 g/min.

3-(3-Chloro-4-fluorophenyl)-1-((1-(methylsulfonyl)piperidin-4-yl)methyl)-1-(1-(1-oxo-1,2-dihydroisoquinolin-4-yl)ethyl)urea: Enantiomer I **(Compound 203).** LCMS: *m*/*z* found 535.4/537.4 [M+H]⁺, RT = 7.19 min (Method: A); ¹H NMR (400 MHz, DMSO-d₆) δ 11.41 (bs, 1H), 8.47 (bs, 1H), 8.26 (d, 1H), 7.81-7.84 (m, 1H), 7.73-7.77 (m, 2H), 7.47-7.53 (m, 2H), 7.34 (t, 1H), 7.24 (s, 1H), 5.84-5.87 (m, 1H), 3.33-3.39 (m, 1H), 3.21-3.27 (m, 1H), 2.97-3.02 (m, 2H), 2.71 (s, 3H), 2.24-2.30 (m, 1H), 2.14-2.20 (m, 1H), 1.46-1.50 (m, 4H), 1.06-1.22 (m, 3H), 0.74-0.80 (m, 1H); Chiral analytical SFC: RT = 1.42 min, Column: Chiralcel OD-3 (150 × 4.6 mm), 3 µ, 60% CO₂:MeOH, Flow rate = 3.0 mL/min.

3-(3-Chloro-4-fluorophenyl)-1-((1-(methylsulfonyl)piperidin-4-yl)methyl)-1-(1-(1-oxo-1,2-dihydroisoquinolin-4-yl)ethyl)urea: Enantiomer II **(Compound 204).** LCMS: *m*/*z* found 535.4/537.4 [M+H]⁺, RT = 7.10 min (Method: A); ¹H NMR (400 MHz, DMSO-d₆) δ 11.41 (bs, 1H), 8.47 (bs, 1H), 8.26 (d, 1H), 7.81-7.84 (m, 1H), 7.73-7.77 (m, 2H), 7.47-7.53 (m, 2H), 7.34 (t, 1H), 7.24 (s, 1H), 5.84-5.87 (m, 1H), 3.33-3.39 (m, 1H), 3.21-3.27 (m, 1H), 2.97-3.02 (m, 2H), 2.71 (s, 3H), 2.24-2.30 (m, 1H), 2.14-2.20 (m, 1H), 1.46-1.50 (m, 4H), 1.06-1.22 (m, 3H), 0.74-0.80 (m, 1H); Chiral analytical SFC: RT = 1.95 min, Column: Chiralcel OD-3 (150 × 4.6 mm), 3 µ, 60% CO₂:MeOH, Flow rate = 3.0 mL/min.

### 4-(1-(((Tetrahydro-2H-pyran-4-yl)methyl)amino)ethyl)isoquinolin-1(2H)-one (VIIIay)

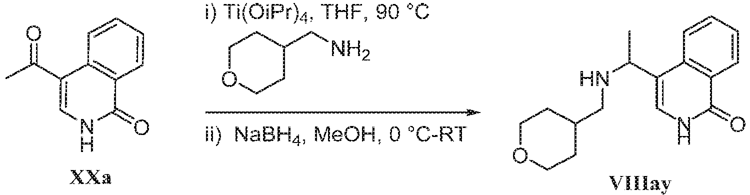

To a solution of 0.2 g (1.1 mmol, 1.0 eq.) of 4-acetylisoquinolin-1(2*H*)-one **(XXa)** in 2 mL of THF under a nitrogen atmosphere was added 0.18 g (1.6 mmol 1.5 eq.) of (tetrahydro-2*H-*pyran-4-yl)methanamine followed by 2 mL of titanium (IV) isopropoxide and the mixture was heated at 90 °C for 6 h. The mixture was allowed to cool to room temperature and then further cooled to 0 °C. The mixture was diluted with 2 mL of methanol and 0.08 g (2.1 mmol, 2.0 eq.) of sodium borohydride was added. The mixture was then allowed to warm to room temperature and stirred for 2 h. The reaction was quenched by the addition of 20 mL of water and filtered through CELITE^{®}. The pad was washed with 5 mL of ethyl acetate and the filtrate was extracted with 3 × 30 mL of ethyl acetate. The combined organic extracts were washed with 30 mL of brine dried (Na₂SO₄), filtered and the solvent was removed *in vacuo* to provide 0.25 g (0.87 mmol, 82%) of 4-(1-(((tetrahydro-2*H*-pyran-4-yl)methyl)amino)ethyl)isoquinolin-1(2*H*)-one **(VIIIay).** LCMS: *m*/*z* found 287.1 [M+H]⁺; ¹H NMR (400 MHz, DMSO-d₆): δ 11.18 (bs 1H), 8.21-8.25 (m, 1H), 8.01 (d, 1H), 7.68-7.74 (m, 1H), 7.47 (t, 1H), 7.14 (bs, 1H), 3.99-4.04 (m, 1H), 3.77-3.83 (m, 2H), 3.19-3.28 (m, 2H), 2.36-2.42 (m, 1H), 2.24-2.30 (m, 1H), 1.53-1.69 (m, 4H), 1.31 (d, 3H), 1.02-1.15 (m, 2H).

### 3-(3-Chloro-4-fluorophenyl)-1-(1-(1-oxo-1,2-dihydroisoquinolin-4-yl)ethyl)-1-((tetrahydro-2H-pyran-4-yl)methyl)urea (Compounds 134, 141 & 142)

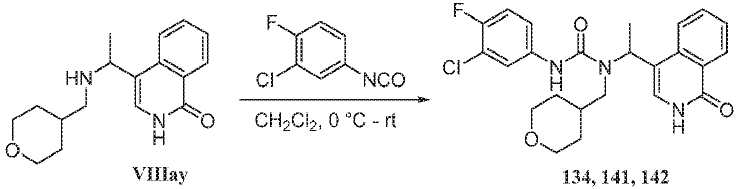

Racemic 3-(3-chloro-4-fluorophenyl)-1-(1-(1-oxo-1,2-dihydroisoquinolin-4-yl)ethyl)-1-((tetrahydro-2*H*-pyran-4-yl)methyl)urea **(Compound 134)** was synthesized in a similar manner as described above from 4-(1-(((tetrahydro-2*H*-pyran-4-yl)methyl)amino)ethyl)isoquinolin-1(2*H*)-one **(VIIIay)** and 2-chloro-1-fluoro-4-isocyanatobenzene. The enantiomers were subsequently separated by SFC, Column: Lux cellulose-2 (30 × 250 mm) 5 µ, 60% CO₂:MeOH, flow rate 70 g/min.

3-(3-Chloro-4-fluorophenyl)-1-(1-(1-oxo-1,2-dihydroisoquinolin-4-yl)ethyl)-1-((tetrahydro-2*H*-pyran-4-yl)methyl)urea: Enantiomer I **(Compound 141).** LCMS: *m*/*z* found 458.3/460.3 [M+H]⁺, RT = 6.59 min (Method: A); ¹H NMR (400 MHz, DMSO-d₆): 11.40 (bs, 1H), 8.45 (bs, 1H), 8.25 (d, 1H), 7.81-7.84 (m, 1H), 7.71-7.75 (m, 2H), 7.47-7.53 (m, 2H), 7.33 (t, 1H), 7.23 (d, 1H), 5.84-5.88 (m, 1H), 3.54-3.63 (m, 2H), 2.91-3.06 (m, 2H), 2.80-2.86 (m, 1H), 2.67-2.71 (m, 1H), 1.48 (d, 3H), 1.15-1.23 (m, 2H), 0.98-1.10 (m, 2H), 0.76-0.82 (m, 1H); Chiral analytical SFC: RT = 1.56 min, Column: Chiralcel OZ-3 (250 × 4.6 mm), 5 µ, 60% CO₂:MeOH, Flow rate = 3.0 mL/min.

3-(3-Chloro-4-fluorophenyl)-1-(1-(1-oxo-1,2-dihydroisoquinolin-4-yl)ethyl)-1-((tetrahydro-2H-pyran-4-yl)methyl)urea: Enantiomer II **(Compound 142).** LCMS: *m*/*z* found 458.3/460.3 [M+H]⁺, RT = 6.58 min (Method: A); ¹H NMR (400 MHz, DMSO-d₆): 11.40 (bs, 1H), 8.45 (bs, 1H), 8.25 (d, 1H), 7.81-7.84 (m, 1H), 7.71-7.75 (m, 2H), 7.47-7.53 (m, 2H), 7.33 (t, 1H), 7.23 (d, 1H), 5.84-5.88 (m, 1H), 3.54-3.63 (m, 2H), 2.91-3.06 (m, 2H), 2.80-2.86 (m, 1H), 2.67-2.71 (m, 1H), 1.48 (d, 3H), 1.15-1.23 (m, 2H), 0.98-1.10 (m, 2H), 0.76-0.82 (m, 1H); Chiral analytical SFC: RT = 2.55 min, Column: Chiralcel OZ-3 (250 × 4.6 mm), 5 µ, 60% CO₂:MeOH, Flow rate = 3.0 mL/min.

### 4-(1-((((R)-Tetrahydrofuran-2-yl)methyl)amino)ethyl)isoquinolin-1(2H)-one (VIIIaz)

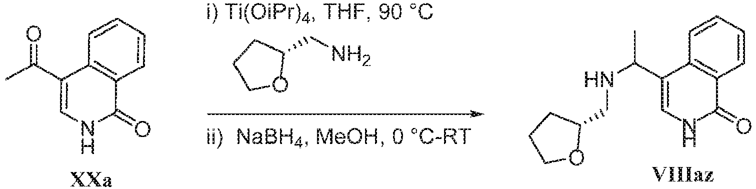

To a solution of 0.3 g (1.6 mmol, 1.0 eq.) of 4-acetylisoquinolin-1(2*H*)-one **(XXa)** in 3 mL of THF under a nitrogen atmosphere was added 0.24 g (2.4 mmol 1.5 eq.) of (R)-(tetrahydrofuran-2-yl)methanamine followed by 3 mL of titanium (IV) isopropoxide and the mixture was heated at 90 °C for 6 h. The mixture was allowed to cool to room temperature and then further cooled to 0 °C. The mixture was diluted with 3 mL of methanol and 0.12 g (3.2 mmol, 2.0 eq.) of sodium borohydride was added. The mixture was then allowed to warm to room temperature and stirred for 2 h. The reaction was quenched by the addition of 20 mL of water and filtered through CELITE^{®}. The pad was washed with 5 mL of ethyl acetate and the filtrate was extracted with 3 × 30 mL of ethyl acetate. The combined organic extracts were washed with 20 mL of brine, dried (Na₂SO₄), filtered and the solvent was removed *in vacuo* to provide 0.32 g (1.17 mmol, 73%) of 4-(1-((((R)-tetrahydrofuran-2-yl)methyl)amino)ethyl)isoquinolin-1(2*H*)-one **(VIIIaz).** LCMS: *m*/*z* found 273.1 [M+H]⁺; ¹H NMR (400 MHz, DMSO-d₆) δ 11.16 (bs, 1H), 8.22-8.24 (m, 1H), 8.01 (t, 1H), 7.68-7.74 (m, 1H), 7.45-7.51 (m, 1H), 7.14 (d, 1H), 4.04-4.10 (m, 1H), 3.82-3.87 (m, 1H), 3.66-3.74 (m, 1H), 3.54-3.62 (m, 1H), 2.44-2.54 (m, 2H), 1.71-1.88 (m, 4H), 1.48-1.54 (m, 1H), 1.32 (d, 3H).

### 3-(3-Chloro-4-fluorophenyl)-1-(1-(1-oxo-1,2-dihydroisoquinolin-4-yl)ethyl)-1-(((R)-tetrahydrofuran-2-yl)methyl)urea (Compound 155 & 156)

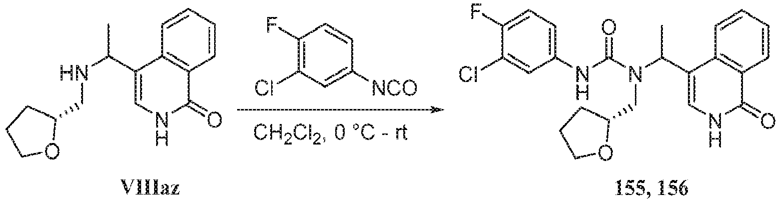

Diastereomeric 3-(3-chloro-4-fluorophenyl)-1-(1-(1-oxo-1,2-dihydroisoquinolin-4-yl)ethyl)-1-(((R)-tetrahydrofuran-2-yl)methyl)urea was synthesized in a similar manner as described above from 4-(1-((((R)-tetrahydrofuran-2-yl)methyl)amino)ethyl)isoquinolin-1(2*H*)-one **(VIIIaz)** and 2-chloro-1-fluoro-4-isocyanatobenzene. The diastereoisomers were subsequently separated by semi-preparative HPLC.

3-(3-Chloro-4-fluorophenyl)-1-(1-(1-oxo-1,2-dihydroisoquinolin-4-yl)ethyl)-1-(((R)-tetrahydrofuran-2-yl)methyl)urea: Diastereomer I **(Compound 155).** LCMS: *m*/*z* found 444.1/446.1 [M+H]⁺, RT = 7.41 min (Method: A); ¹H NMR (400 MHz, DMSO-d₆) δ 11.40 (bs, 1H), 8.94 (bs, 1H), 8.24 (d, 1H), 7.79-7.82 (m, 1H), 7.72-7.76 (m, 1H), 7.59 (d, 1H), 7.50 (t, 1H), 7.32 (t, 1H), 7.21-7.25 (m, 2H), 5.78-5.83 (m, 1H), 3.76-3.81 (m, 1H), 3.50-3.56 (m, 1H), 3.16-3.26 (m, 2H), 2.98-3.04 (m, 1H), 1.68-1.75 (m, 1H), 1.50-1.60 (m, 2H), 1.46 (d, 3H), 1.27-1.33 (m, 1H); Chiral analytical SFC: RT = 2.06 min, Column: Chiralcel OZ-3 (250 × 4.6 mm), 5 µ, 60% CO₂:MeOH, Flow rate = 3.0 mL/min.

3-(3-Chloro-4-fluorophenyl)-1-(1-(1-oxo-1,2-dihydroisoquinolin-4-yl)ethyl)-1-(((R)-tetrahydrofuran-2-yl)methyl)urea: Diastereomer II **(Compound 156).** LCMS: *m*/*z* found 444.1/446.1 [M+H]⁺, RT = 7.11 min (Method: A); ¹H NMR (400 MHz, DMSO-d₆) δ 11.39 (bs, 1H), 8.97 (bs, 1H), 8.24 (d, 1H), 7.79-7.82 (m, 1H), 7.72-7.76 (m, 2H), 7.48-7.52 (m, 1H), 7.30-7.35 (m, 2H), 7.18-7.20 (m, 1H), 5.90-5.93 (m, 1H), 3.74-3.78 (m, 1H), 3.63-3.68 (m, 1H), 3.53-3.59 (m, 1H), 3.17-3.22 (m, 2H), 1.60-1.66 (m, 2H), 1.46-1.55 (m, 4H), 1.15-1.23 (m, 1H); Chiral analytical SFC: RT = 3.50 min, Column: Chiralcel OZ-3 (250 × 4.6 mm), 5 µ, 60% CO₂:MeOH, Flow rate = 3.0 mL/min.

### 3-(3-Chloro-4-fluorophenyl)-1-(1-(1-oxo-1,2-dihydroisoquinolin-4-yl)ethyl)-1-(((S)-tetrahydrofuran-2-yl)methyl)urea (Compounds 160 & 161)

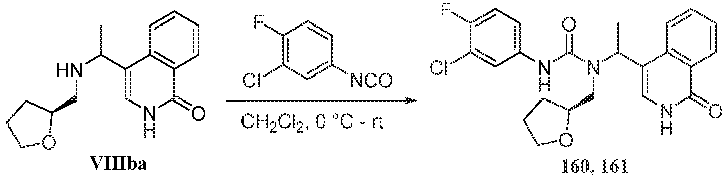

Diastereomeric 3-(3-chloro-4-fluorophenyl)-1-(1-(1-oxo-1,2-dihydroisoquinolin-4-yl)ethyl)-1-(((S)-tetrahydrofuran-2-yl)methyl)urea was synthesized in a similar manner as described above from 4-(1-((((S)-tetrahydrofuran-2-yl)methyl)amino)ethyl)isoquinolin-1(2H)-one **(VIIIba,** synthesized from 4-acetylisoquinolin-1(2*H*)-one **(XXa)** and (S)-(tetrahydrofuran-2-yl)methanamine) and 2-chloro-1-fluoro-4-isocyanatobenzene. The diastereoisomers were subsequently separated by semi-preparative HPLC.

3-(3-Chloro-4-fluorophenyl)-1-(1-(1-oxo-1,2-dihydroisoquinolin-4-yl)ethyl)-1-(((S)-tetrahydrofuran-2-yl)methyl)urea: Diastereomer I **(Compound 160).** LCMS: *m*/*z* found 444.1/446.1 [M+H]⁺, RT = 7.60 min (Method: A); ¹H NMR (400 MHz, DMSO-d₆) δ 11.39 (bs, 1H), 8.97 (bs, 1H), 8.24 (d, 1H), 7.79-7.82 (m, 1H), 7.72-7.76 (m, 2H), 7.48-7.52 (m, 1H), 7.30-7.35 (m, 2H), 7.18-7.20 (m, 1H), 5.90-5.93 (m, 1H), 3.74-3.78 (m, 1H), 3.63-3.68 (m, 1H), 3.53-3.59 (m, 1H), 3.17-3.22 (m, 2H), 1.60-1.66 (m, 2H), 1.46-1.55 (m, 4H), 1.15-1.23 (m, 1H); Chiral analytical SFC: RT = 5.02 min, Column: Lux Cellulose-2 (250 × 4.6 mm), 5 µ, 55% CO₂:MeOH, Flow rate = 3.0 mL/min.

3-(3-Chloro-4-fluorophenyl)-1-(1-(1-oxo-1,2-dihydroisoquinolin-4-yl)ethyl)-1-(((S)-tetrahydrofuran-2-yl)methyl)urea: Diastereomer II **(Compound 161).** LCMS: *m*/*z* found 444.1/446.1 [M+H]⁺, RT = 7.47 min (Method: A); ¹H NMR (400 MHz, DMSO-d₆) δ 11.40 (bs, 1H), 8.94 (bs, 1H), 8.24 (d, 1H), 7.79-7.82 (m, 1H), 7.72-7.76 (m, 1H), 7.59 (d, 1H), 7.50 (t, 1H), 7.32 (t, 1H), 7.21-7.25 (m, 2H), 5.78-5.53 (m, 1H), 3.76-3.81 (m, 1H), 3.50-3.56 (m, 1H), 3.16-3.26 (m, 2H), 2.98-3.04 (m, 1H), 1.68-1.75 (m, 1H), 1.50-1.60 (m, 2H), 1.46 (d, 3H), 1.27-1.33 (m, 1H); Chiral analytical SFC: RT = 8.51 min, Column: Lux Cellulose-2 (250 × 4.6 mm), 5 µ, 55% CO₂:MeOH, Flow rate = 3.0 mL/min.

### 4-(1-(((Tetrahydrofuran-3-yl)methyl)amino)ethyl)isoquinolin-1(2H)-one (VIIIbb)

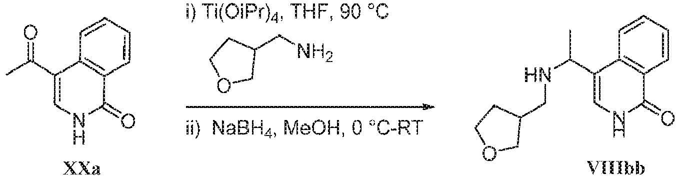

To a solution of 0.5 g (2.7 mmol, 1.0 eq.) of 4-acetylisoquinolin-1(2*H*)-one **(XXa)** in 15 mL of THF under a nitrogen atmosphere was added 0.41 g (4.0 mmol 1.5 eq.) of racemic (tetrahydrofuran-3-yl)methanamine followed by 7 mL of titanium (IV) isopropoxide and the mixture was heated at 90 °C for 6 h. The mixture was allowed to cool to room temperature and then further cooled to 0 °C. The mixture was diluted with 10 mL of methanol and 0.20 g (5.2 mmol, 2.0 eq.) of sodium borohydride was added. The mixture was then allowed to warm to room temperature and stirred for 2 h. The reaction was quenched by the addition of 30 mL of water and filtered through CELITE^{®}. The pad was washed with 20 mL of ethyl acetate and the filtrate was extracted with 3 × 100 mL of ethyl acetate. The combined organic extracts were washed with 20 mL of brine, dried (Na₂SO₄), filtered and the solvent was removed *in vacuo.* The residue was purified by flash chromatography (SiO₂, eluting with a linear gradient of 70-10% ethyl acetate/petroleum ether) to provide 0.35 g (1.28 mmol, 48%) of 4-(1-((tetrahydrofuran-3-yl)methyl)amino)ethyl)isoquinolin-1(2*H*)-one **(VIIIbb)** as a mixture of racemic diastereoisomers. LCMS: *m*/*z* found 273.1 [M+H]⁺.

### 3-(3-Chloro-4-fluorophenyl)-1-(1-(1-oxo-1,2-dihydroisoquinolin-4-yl)ethyl)-1-(((S)-tetrahydrofuran-2-yl)methyl)urea (Compounds 165, 166, 175 & 176)

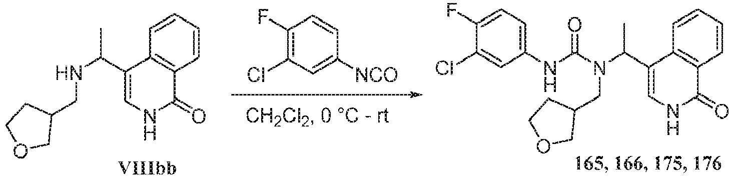

Diastereomeric 3-(3-chloro-4-fluorophenyl)-1-(1-(1-oxo-1,2-dihydroisoquinolin-4-yl)ethyl)-1-(((R)-tetrahydrofuran-2-yl)methyl)urea was synthesized in a similar manner as described above from 4-(1-(((tetrahydrofuran-2-yl)methyl)amino)ethyl)isoquinolin-1(2*H*)-one **(VIIIbb)** and 2-chloro-1-fluoro-4-isocyanatobenzene. The diastereoisomers were subsequently separated by reverse-phase chromatography (REVELERIS^{®} C18 column, eluted with a linear gradient of 30-70% acetonitrile with water) to provide Diastereoisomeric pairs A and B. Each diastereoisomer was subsequently resolved into the individual enantiomers by SFC, Column: (R,R) Whelk-01 (30 × 250 mm) 5 µ, 60% CO₂:MeOH, flow rate 70 g/min.

3-(3-Chloro-4-fluorophenyl)-1-(1-(1-oxo-1,2-dihydroisoquinolin-4-yl)ethyl)-1-(((S)-tetrahydrofuran-2-yl)methyl)urea: Diastereomer IA **(Compound 165).** LCMS: *m*/*z* found 444.1/446.1 [M+H]⁺, RT = 7.34 min (Method: A); ¹H NMR (400 MHz, DMSO-d₆) δ 11.41 (bd, 1H), 8.54 (bs, 1H), 8.25 (d, 1H), 7.81-7.83 (m, 1H), 7.75-7.78 (m, 2H), 7.47-7.53 (m, 2H), 7.34 (t, 1H), 7.23 (bd, 1H), 5.79-5.83 (m, 1H), 3.55-3.60 (m, 1H), 3.33-3.40 (m, 1H), 3.17-3.21 (m, 1H), 3.00-3.06 (m, 2H), 2.71-2.76 (m, 1H), 2.03-2.09 (m, 1H), 1.61-1.70 (m, 1H), 1.47 (d, 3H), 1.38-1.43 (m, 1H); Chiral analytical SFC: RT = 6.54 min, Column: (R,R) Whelk-01 (250 × 4.6 mm), 5 µ, 55% CO₂:MeOH, Flow rate = 3.0 mL/min.

3-(3-Chloro-4-fluorophenyl)-1-(1-(1-oxo-1,2-dihydroisoquinolin-4-yl)ethyl)-1-(((S)-tetrahydrofuran-2-yl)methyl)urea: Diastereomer IIA **(Compound 166).** LCMS: *m*/*z* found 444.1/446.1 [M+H]⁺, RT = 7.34 min (Method: A); ¹H NMR (400 MHz, DMSO-d₆) δ 11.41 (bd, 1H), 8.54 (bs, 1H), 8.25 (d, 1H), 7.81-7.83 (m, 1H), 7.75-7.78 (m, 2H), 7.47-7.53 (m, 2H), 7.34 (t, 1H), 7.23 (bd, 1H), 5.79-5.83 (m, 1H), 3.55-3.60 (m, 1H), 3.33-3.40 (m, 1H), 3.17-3.21 (m, 1H), 3.00-3.06 (m, 2H), 2.71-2.76 (m, 1H), 2.03-2.09 (m, 1H), 1.61-1.70 (m, 1H), 1.47 (d, 3H), 1.38-1.43 (m, 1H); Chiral analytical SFC: RT = 9.27 min, Column: (R,R) Whelk-01 (250 × 4.6 mm), 5 µ, 55% CO₂:MeOH, Flow rate = 3.0 mL/min.

3-(3-Chloro-4-fluorophenyl)-1-(1-(1-oxo-1,2-dihydroisoquinolin-4-yl)ethyl)-1-(((S)-tetrahydrofuran-2-yl)methyl)urea: Diastereomer IB **(Compound 175).** LCMS: *m*/*z* found 444.1/446.1 [M+H]⁺, RT = 7.55 min (Method: A); ¹H NMR (400 MHz, DMSO-d₆) δ 11.41 (bd, 1H), 8.48 (bs, 1H), 8.24 (d, 1H), 7.80-7.83 (m, 1H), 7.74-7.78 (m, 2H), 7.45-7.53 (m, 2H), 7.34 (t, 1H), 7.24 (bd, 1H), 5.80-5.84 (m, 1H), 3.42-3.47 (m, 1H), 3.31-3.37 (m, 1H), 3.22-3.27 (m, 2H), 3.07-3.16 (m, 2H), 1.85-1.91 (m, 1H), 1.47 (d, 3H), 1.34-1.40 (m, 1H), 1.01-1.06 (m, 1H); Chiral analytical SFC: RT = 3.30 min, Column: Chiralpak IC-3 (250 × 4.6 mm), 5 µ, 55% CO₂:MeOH, Flow rate = 3.0 mL/min.

3-(3-Chloro-4-fluorophenyl)-1-(1-(1-oxo-1,2-dihydroisoquinolin-4-yl)ethyl)-1-(((S)-tetrahydrofuran-2-yl)methyl)urea: Diastereomer IIB **(Compound 176).** LCMS: *m*/*z* found 444.1/446.1 [M+H]⁺, RT = 7.55 min (Method: A); ¹H NMR (400 MHz, DMSO-d₆) δ 11.41 (bd, 1H), 8.48 (bs, 1H), 8.24 (d, 1H), 7.80-7.83 (m, 1H), 7.74-7.78 (m, 2H), 7.45-7.53 (m, 2H), 7.34 (t, 1H), 7.24 (bd, 1H), 5.80-5.84 (m, 1H), 3.42-3.47 (m, 1H), 3.31-3.37 (m, 1H), 3.22-3.27 (m, 2H), 3.07-3.16 (m, 2H), 1.85-1.91 (m, 1H), 1.47 (d, 3H), 1.34-1.40 (m, 1H), 1.01-1.06 (m, 1H); Chiral analytical SFC: RT = 4.68 min, Column: Chiralpak IC-3 (250 × 4.6 mm), 5 µ, 55% CO₂:MeOH, Flow rate = 3.0 mL/min.

### 4-(1-((2-Hydroxyethyl)amino)ethyl)isoquinolin-1(2H)-one (VIIIbc)

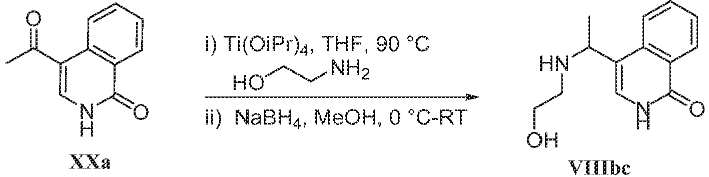

To a solution of 0.3 g (1.6 mmol, 1.0 eq.) of 4-acetylisoquinolin-1(2*H*)-one **(XXa)** in 3 mL of THF under a nitrogen atmosphere was added 0.14 g (2.4 mmol 1.5 eq.) of 2-aminoethan-1-ol followed by 3 mL of titanium (IV) isopropoxide and the mixture was heated at 90 °C for 6 h. The mixture was allowed to cool to room temperature and then further cooled to 0 °C. The mixture was diluted with 2 mL of methanol and 0.12 g (3.2 mmol, 2.0 eq.) of sodium borohydride was added. The mixture was then allowed to warm to room temperature and stirred for 2 h. The reaction was quenched by the addition of 20 mL of water and filtered through CELITE^{®}. The pad was washed with 5 mL of ethyl acetate and the filtrate was extracted with 3 × 50 mL of ethyl acetate. The combined organic extracts were washed with 30 mL of brine dried (Na₂SO₄), filtered and the solvent was removed *in vacuo* to provide 0.26 g (1.1 mmol, 70%) of 4-(1-((2-hydroxyethyl)amino) ethyl)isoquinolin-1(2*H*)-one **(VIIIbc).** LCMS: *m*/*z* found 222.1 [M+H]⁺; ¹H NMR (400 MHz, DMSO-d₆) δ 11.23 (bs, 1H), 8.22-8.25 (m, 1H), 8.00 (d, 1H), 7.69-7.74 (m, 1H), 7.46-7.50 (m, 1H), 7.15 (s, 1H), 4.48 (bs, 1H), 4.07-4.14 (m, 1H), 3.40-3.46 (m, 2H), 3.37 (bs, 1H), 2.51-2.59 (m, 2H), 1.32 (d, 3H).

### 3-(3-Chloro-4-fluorophenyl)-1-(2-hydroxyethyl)-1-(1-(1-oxo-1,2-dihydroisoquinolin-4-yl)ethyl)urea (Compounds 196 & 197)

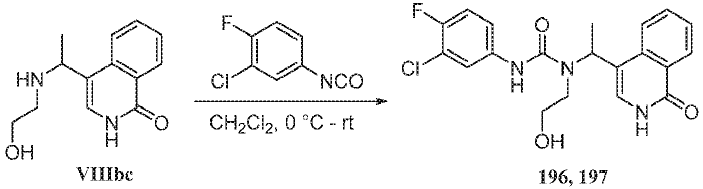

Racemic 3-(3-chloro-4-fluorophenyl)-1-(2-hydroxyethyl)-1-(1-(1-oxo-1,2-dihydroisoquinolin-4-yl)ethyl)urea was synthesized in a similar manner as described above from 4-(1-((2-hydroxyethyl)amino)ethyl)isoquinolin-1(2*H*)-one **(VIIIbc)** and 2-chloro-1-fluoro-4-isocyanatobenzene. The enantiomers were subsequently separated by SFC, Column: Chiralcel OD-H (30 × 250 mm) 5 µ, 80% CO₂:MeOH, flow rate 70 g/min.

3-(3-Chloro-4-fluorophenyl)-1-(2-hydroxyethyl)-1-(1-(1-oxo-1,2-dihydroisoquinolin-4-yl)ethyl)urea: Enantiomer I **(Compound 196).** LCMS: *m*/*z* found 404.2/406.2 [M+H]⁺, RT = 4.90 min (Method: A); ¹H NMR (400 MHz, DMSO-d₆): 11.40 (bs, 1H), 9.20 (bs, 1H), 8.24 (d, 1H), 7.73-7.79 (m, 2H), 7.65 (d, 1H), 7.51 (t, 1H), 7.30-7.35 (m, 2H), 7.19 (d, 1H), 5.80-5.84 (m, 1H), 5.37 (bs, 1H), 3.11-3.21 (m, 3H), 2.85-2.91 (m, 1H), 1.45 (d, 3H); Chiral analytical SFC: RT = 3.21 min, Column: Chiralcel OD-H (150 × 4.6 mm), 3 µ, 70% CO₂:MeOH, Flow rate = 3.0 mL/min.

3-(3-Chloro-4-fluorophenyl)-1-(2-hydroxyethyl)-1-(1-(1-oxo-1,2-dihydroisoquinolin-4-yl)ethyl)urea: Enantiomer II **(Compound 197).** LCMS: *m*/*z* found 404.2/406.2 [M+H]⁺, RT = 4.90 min (Method: A); ¹H NMR (400 MHz, DMSO-d₆): 11.40 (bs, 1H), 9.20 (bs, 1H), 8.24 (d, 1H), 7.73-7.79 (m, 2H), 7.65 (d, 1H), 7.51 (t, 1H), 7.30-7.35 (m, 2H), 7.19 (d, 1H), 5.80-5.84 (m, 1H), 5.37 (bs, 1H), 3.11-3.21 (m, 3H), 2.85-2.91 (m, 1H), 1.45 (d, 3H); Chiral analytical SFC: RT = 5.81 min, Column: Chiralcel OD-H (150 × 4.6 mm), 3 µ, 70% CO₂:MeOH, Flow rate = 3.0 mL/min.

### 4-(1-((4-Hydroxybutyl)amino)ethyl)isoquinolin-1(2H)-one (VIIIbd)

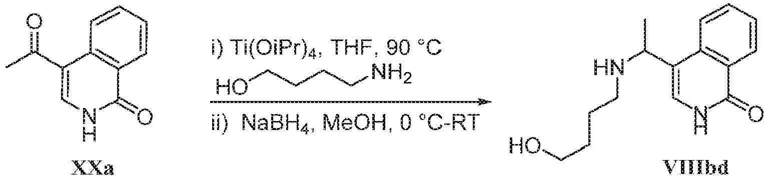

To a solution of 0.3 g (1.6 mmol, 1.0 eq.) of 4-acetylisoquinolin-1(2*H*)-one **(XXa)** in 3 mL of THF under a nitrogen atmosphere was added 0.21 g (2.4 mmol 1.5 eq.) of 4-aminobutan-1-ol followed by 3 mL of titanium (IV) isopropoxide and the mixture was heated at 90 °C for 6 h. The mixture was allowed to cool to room temperature and then further cooled to 0 °C. The mixture was diluted with 2 mL of methanol and 0.12 g (3.2 mmol, 2.0 eq.) of sodium borohydride was added. The mixture was then allowed to warm to room temperature and stirred for 2 h. The reaction was quenched by the addition of 20 mL of water and filtered through CELITE^{®}. The pad was washed with 5 mL of ethyl acetate and the filtrate was extracted with 3 × 50 mL of ethyl acetate. The combined organic extracts were washed with 30 mL of brine, dried (Na₂SO₄), filtered and the solvent was removed *in vacuo.* The residue was triturated with 30 mL of *tert*-butylmethyl ether to provide 0.2 g (0.76 mmol, 48%) of 4-(1-((4-hydroxybutyl)amino)ethyl)isoquinolin-1(2*H*)-one **(VIIIbd).** LCMS: *m*/*z* found 261.5 [M+H]⁺; ¹H NMR (400 MHz, DMSO-d₆): δ 10.38 (bs 1H), 8.48 (d, 1H), 7.81 (d, 1H), 7.72 (t, 1H), 7.52 (t, 1H), 7.26 (s, 1H), 4.18-4.23 (m, 1H), 3.61-3.64 (m, 2H), 2.67-2.70 (m, 2H), 1.74 (bs, 1H), 1.53-1.68 (m, 5H), 1.47 (d, 3H).

### 3-(3-Chloro-4-fluorophenyl)-1-(4-hydroxybutyl)-1-(1-(1-oxo-1,2-dihydroisoquinolin-4-yl)ethyl)urea (Compounds 149 & 150)

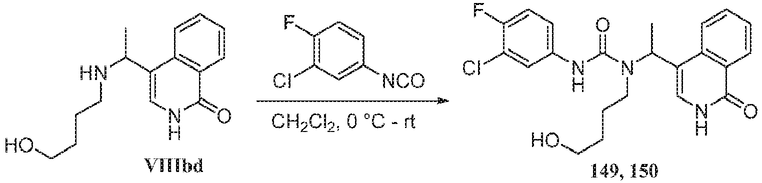

Racemic 3-(3-chloro-4-fluorophenyl)-1-(4-hydroxybutyl)-1-(1-(1-oxo-1,2-dihydroisoquinolin-4-yl)ethyl)urea was synthesized in a similar manner as described above from 4-(1-((4-hydroxybutyl)amino)ethyl)isoquinolin-1(2*H*)-one **(VIIIbd)** and 2-chloro-1-fluoro-4-isocyanatobenzene. The enantiomers were subsequently separated by SFC, Column: Chiralcel OD-H (30 × 250 mm) 5 µ, 80% CO₂:MeOH, flow rate 60 g/min.

3-(3-Chloro-4-fluorophenyl)-1-(4-hydroxybutyl)-1-(1-(1-oxo-1,2-dihydroisoquinolin-4-yl)ethyl)urea: Enantiomer I **(Compound 149).** LCMS: *m*/*z* found 432.2/434.2 [M+H]⁺, RT = 6.80 min (Method: A); ¹H NMR (400 MHz, DMSO-d₆): 11.40 (bs, 1H), 8.39 (bs, 1H), 8.23 (d, 1H), 7.82-7.85 (m, 1H), 7.69-7.75 (m, 2H), 7.47-7.52 (m, 2H), 7.32 (t, 1H), 7.22 (s, 1H), 5.84-5.87 (m, 1H), 4.46 (bs, 1H), 3.11-3.19 (m, 2H), 3.02-3.10 (m, 2H), 1.45 (d, 3H), 1.16-1.24 (m, 3H), 0.81-0.87 (m, 1H); Chiral analytical SFC: RT = 1.59 min, Column: Chiralcel OD-H (250 × 4.6 mm), 5 µ, 70% CO₂:MeOH, Flow rate = 3.0 mL/min.

3-(3-Chloro-4-fluorophenyl)-1-(4-hydroxybutyl)-1-(1-(1-oxo-1,2-dihydroisoquinolin-4-yl)ethyl)urea: Enantiomer II **(Compound 150).** LCMS: *m*/*z* found 432.2/434.2 [M+H]⁺, RT = 6.80 min (Method: A); ¹H NMR (400 MHz, DMSO-d₆): 11.40 (bs, 1H), 8.39 (bs, 1H), 8.23 (d, 1H), 7.82-7.85 (m, 1H), 7.69-7.75 (m, 2H), 7.47-7.52 (m, 2H), 7.32 (t, 1H), 7.22 (s, 1H), 5.84-5.87 (m, 1H), 4.46 (bs, 1H), 3.11-3.19 (m, 2H), 3.02-3.10 (m, 2H), 1.45 (d, 3H), 1.16-1.24 (m, 3H), 0.81-0.87 (m, 1H); Chiral analytical SFC: RT = 2.07 min, Column: Chiralcel OD-H (250 × 4.6 mm), 5 µ, 70% CO₂:MeOH, Flow rate = 3.0 mL/min.

### N-(2-(1-(1-Hydroxyisoquinolin-4-yl)ethylamino)ethyl)acetamide (VIIIbe)

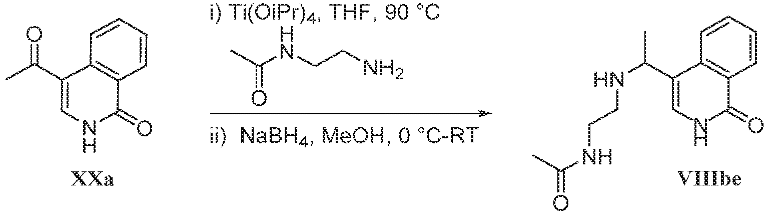

To a solution of 0.5 g (2.7 mmol, 1.0 eq.) of 4-acetylisoquinolin-1(2*H*)-one **(XXa)** in 5 mL of THF under a nitrogen atmosphere was added 0.41 g (4.0 mmol 1.5 eq.) of *N*-(2-aminoethyl)acetamide followed by 5 mL of titanium (IV) isopropoxide and the mixture was heated at 90 °C for 6 h. The mixture was allowed to cool to room temperature and then further cooled to 0 °C. The mixture was diluted with 5 mL of methanol and 0.20 g (5.3 mmol, 2.0 eq.) of sodium borohydride was added. The mixture was then allowed to warm to room temperature and stirred for 2 h. The reaction was filtered through CELITE^{®} and the pad was washed with 5 mL of ethyl acetate. The filtrate was concentrated *in vacuo* and the residue was purified by reverse-phase chromatography (REVELERIS^{®} C18 column, eluting with a linear gradient of 10-60% methanol/water) to provide 0.4 g (1.46 mmol, 54%) of *N*-(2-(1-(1-hydroxyisoquinolin-4-yl)ethylamino)ethyl)acetamide **(VIIIbe).**

### N (2-(3-(3-Chloro-4-fluorophenyl)-1-(1-(1-oxo-1,2-dihydroisoquinolin-4-yl)ethyl)ureido)ethyl)acetamide (Compounds 100,138 & 139)

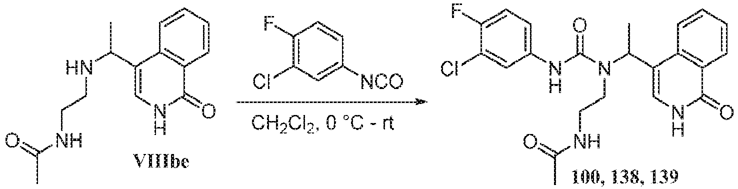

Racemic *N-*(2-(3-(3-chloro-4-fluorophenyl)-1-(1-(1-oxo-1,2-dihydroisoquinolin-4-yl)ethyl)ureido)ethyl)acetamide **(Compound 100)** was synthesized in a similar manner as described above from *N*-(2-(1-(1-hydroxyisoquinolin-4-yl)ethylamino)ethyl)acetamide **(VIIIbe)** and 2-chloro-1-fluoro-4-isocyanatobenzene. The enantiomers were subsequently separated by SFC, Column: Chiralpak IA (30 × 250 mm) 5 µ, 70% CO₂:MeOH, flow rate 90 g/min.

*N-*(2-(3-(3-Chloro-4-fluorophenyl)-1-(1-(1-oxo-1,2-dihydroisoquinolin-4-yl)ethyl)ureido)ethyl)acetamide: Enantiomer I **(Compound 138).** LCMS: *m*/*z* found 445.0/447.0 [M+H]⁺; ¹H NMR (400 MHz, DMSO-d₆): 11.40 (s, 1H), 9.31 (s, 1H), 8.25 (d, 2H), 8.05 (d, 1H), 7.76 (t, 1H), 7.60-7.65 (m, 2H), 7.52 (t, 1H), 7.37 (t, 1H), 7.23 (d, 1H), 5.84 (q, 1H), 3.08 (t, 2H), 2.67 (s, 1H), 2.09 (s, 1H), 1.78 (s, 3H), 1.43 (d, 3H); Chiral analytical SFC: RT = 1.13 min, Column: Chiralcel OD-3 (250 × 4.6 mm), 5 µ, 60% CO₂:MeOH, Flow rate = 3.0 mL/min.

*N-*(2-(3-(3-Chloro-4-fluorophenyl)-1-(1-(1-oxo-1,2-dihydroisoquinolin-4-yl)ethyl)ureido)ethyl)acetamide: Enantiomer II **(Compound 139).** LCMS: *m*/*z* found 445.0/447.0 [M+H]⁺; ¹H NMR (400 MHz, DMSO-d₆): 11.40 (s, 1H), 9.31 (s, 1H), 8.25 (d, 2H), 8.05 (d, 1H), 7.76 (t, 1H), 7.60-7.65 (m, 2H), 7.52 (t, 1H), 7.37 (t, 1H), 7.23 (d, 1H), 5.84 (q, 1H), 3.08 (t, 2H), 2.67 (s, 1H), 2.09 (s, 1H), 1.78 (s, 3H), 1.43 (d, 3H); Chiral analytical SFC: RT = 1.64 min, Column: Chiralcel OD-3 (250 × 4.6 mm), 5 µ, 60% CO₂:MeOH, Flow rate = 3.0 mL/min.

### 2-(1-(1-Oxo-1,2-dihydroisoquinolin-4-yl)ethylamino)ethanesulfonamide (VIIIbf)

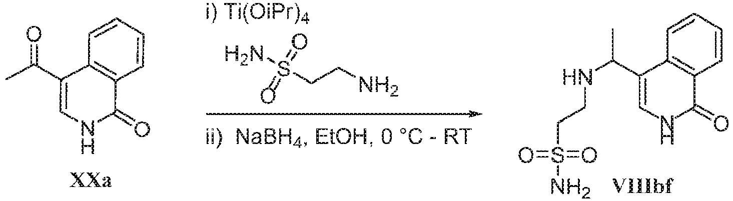

To a solution of 0.5 g (2.7 mmol, 1.0 eq.) of 4-acetylisoquinolin-1(2*H*)-one **(XXa)** in 5 mL of titanium (IV) isopropoxide under a nitrogen atmosphere was added 0.49 g (4.0 mmol 1.5 eq.) of 2-aminoethanesulfonamide and the mixture was stirred at room temperature for 6 h. The mixture was cooled to 0 °C, diluted with 5 mL of ethanol and 0.20 g (5.3 mmol, 2.0 eq.) of sodium borohydride was added. The mixture was then allowed to warm to room temperature and stirred for 2 h. The reaction was filtered through CELITE^{®} and the pad was washed with 5 mL of ethanol. The filtrate was concentrated *in vacuo* and the residue was purified by reverse-phase chromatography (REVELERIS^{®} C18 column, eluting with a linear gradient of 10-60% methanol and water) to provide 0.4 g (1.35 mmol, 50%) of 2-(1-(1-oxo-1,2-dihydroisoquinolin-4-yl)ethylamino) ethanesulfonamide **(VIIIbf).** ¹H NMR (300 MHz, DMSO-d₆): δ 11.20 (s, 1H), 8.25 (d, 1H), 8.03 (d, 1H), 7.74 (t, 1H), 7.51 (t, 1H), 7.17 (d, 1H), 4.12 (q, 1H), 3.12 (m, 2H), 2.86 (m, 2H), 2.36 (s, 1H), 1.43 (d, 3H).

### 2-(3-(3-Chloro-4-fluorophenyl)-1-(1-(1-oxo-1,2-dihydroisoquinolin-4-yl)ethyl)ureido)ethanesulfonamide (Compounds 119 & 120)

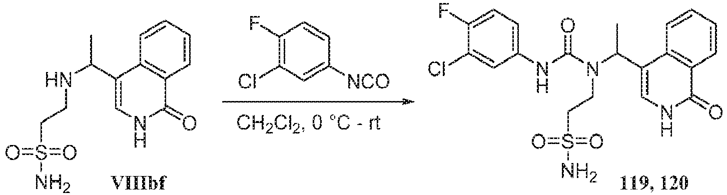

Racemic 2-(3-(3-chloro-4-fluorophenyl)-1-(1-(1-oxo-1,2-dihydroisoquinolin-4-yl)ethyl)ureido)ethanesulfonamide was synthesized in a similar manner as described above from 2-(1-(1-oxo-1,2-dihydroisoquinolin-4-yl)ethylamino) ethanesulfonamide **(VIIIbf)** and 2-chloro-1-fluoro-4-isocyanatobenzene. The enantiomers were subsequently separated by SFC, Column: Chiralpak IA (30 × 250 mm) 5 µ, 50% CO₂:MeOH, flow rate 90 g/min.

2-(3-(3-Chloro-4-fluorophenyl)-1-(1-(1-oxo-1,2-dihydroisoquinolin-4-yl)ethyl)ureido) ethanesulfonamide: Enantiomer I **(Compound 119).** LCMS: *m*/*z* found 467.3/469.2 [M+H]⁺, RT = 6.16 min (Method A); ¹H NMR (400 MHz, DMSO-d₆): 11.22 (bs, 1H), 8.70 (bs, 1H), 8.27 (d, 1H), 7.83 (dd, 1H), 7.77 (t, 1H), 7.61 (d, 1H), 7.47-7.54 (m, 2H), 7.37 (t, 1H), 7.23 (s, 1H), 6.84 (bs, 2H), 5.75 (m, 1H), 3.46 (t, 2H), 3.03 (m, 1H), 2.66 (m, 1H), 1.52 (d, 3H); Chiral analytical SFC: RT = 1.16 min, Chiralpak IG-3 (250 × 4.6 mm), 5 µ, 60% CO₂:MeOH, Flow rate = 3.0 mL/min.

2-(3-(3-Chloro-4-fluorophenyl)-1-(1-(1-oxo-1,2-dihydroisoquinolin-4-yl)ethyl)ureido) ethanesulfonamide: Enantiomer II **(Compound 120).** LCMS: *m*/*z* found 467.2/469.2 [M+H]⁺, RT = 6.07 min (Method A); ¹H NMR (400 MHz, DMSO-d₆): 11.22 (bs, 1H), 8.70 (bs, 1H), 8.27 (d, 1H), 7.83 (dd, 1H), 7.77 (t, 1H), 7.61 (d, 1H), 7.47-7.54 (m, 2H), 7.37 (t, 1H), 7.23 (s, 1H), 6.84 (bs, 2H), 5.75 (m, 1H), 3.46 (t, 2H), 3.03 (m, 1H), 2.66 (m, 1H), 1.52 (d, 3H); Chiral analytical SFC: RT = 3.29 min, Chiralpak IG-3 (250 × 4.6 mm), 5 µ, 60% CO₂:MeOH, Flow rate = 3.0 mL/min.

### N Methyl-3-((1-(1-oxo-1,2-dihydroisoquinolin-4-yl)ethyl)amino)propane-1-sulfonamide (VIIIbg)

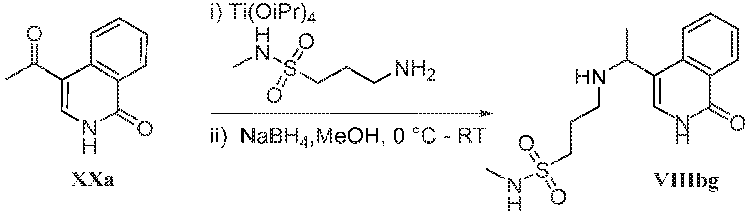

To a solution of 0.3 g (1.6 mmol, 1.0 eq.) of 4-acetylisoquinolin-1(2*H*)-one **(XXa)** in 3 mL of THF under a nitrogen atmosphere was added 0.49 g (3.2 mmol, 2.0 eq.) of 3-amino-V-methylpropane-1-sulfonamide followed by 3 mL of titanium (IV) isopropoxide and the mixture was stirred at 90 °C for 16 h. The mixture was cooled to 0 °C, diluted with 3 mL of ethanol and 0.20 g (5.3 mmol, 2.0 eq.) of sodium borohydride was added. The mixture was then allowed to warm to room temperature and stirred for 2 h. The mixture was then diluted with 15 mL of water and filtered through CELITE^{®}. The pad was washed with 15 mL of ethyl acetate and the filtrate was extracted with 2 × 30 ml of ethyl acetate. The combined organic extracts were washed with 15 mL of brine, dried (Na₂SO₄), filtered and the solvent was removed *in vacuo.* The residue was purified by reverse-phase chromatography (REVELERIS^{®} C18 column, eluting with 40% methanol in water) to provide 0.14 g (0.43 mmol, 27%) of *N*-methyl-3-((1-(1-oxo-1,2-dihydroisoquinolin-4-yl)ethyl)amino)propane-1-sulfonamide (**VIIIbg**). ¹H NMR (300 MHz, DMSO-d₆) δ 10.19 (bs, 1H), 8.92 (d, 1H), 8.23-8.27 (m, 1H), 8.18 (s, 1H), 9.97-8.01 (m, 1H), 7.85-7.81 (m, 1H), 7.53-7.58 (m, 1H), 6.82 (bs, 1H), 4.02-4.07 (m, 1H), 2.99-3.12 (m, 4H), 2.53-2.54 (m, 3H), 1.71-1.82 (m, 2H), 1.31-1.36 (m, 3H).

### 3-(3-(3-Chloro-4-fluorophenyl)-1-(1-(1-oxo-1,2-dihydroisoquinolin-4-yl)ethyl)ureido)-N-methylpropane-1-sulfonamide (Compound 220)

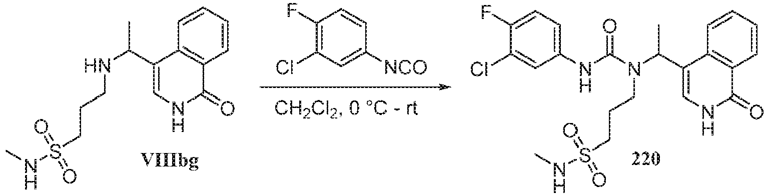

Racemic 3-(3-(3-chloro-4-fluorophenyl)-1-(1-(1-oxo-1,2-dihydroisoquinolin-4-yl)ethyl)ureido)-*N*-methylpropane-1-sulfonamide was synthesized in a similar manner as described above from *N*-methyl-3-((1-(1-oxo-1,2-dihydroisoquinolin-4-yl)ethyl)amino)propane-1-sulfonamide **(VIIIbg)** and 2-chloro-1-fluoro-4-isocyanatobenzene. The enantiomers were subsequently separated by SFC, Column: Lux Cellulose-2 (30 × 250 mm) 5 µ, 60% CO₂:MeOH, flow rate 70 g/min.

3-(3-(3-Chloro-4-fluorophenyl)-1-(1-(1-oxo-1,2-dihydroisoquinolin-4-yl)ethyl)ureido)-*N*-methylpropane-1-sulfonamide: Enantiomer I. LCMS: *m*/*z* found 495.1/497.1 [M+H]⁺, RT = 7.20 min (Method A); ¹H NMR (400 MHz, DMSO-d₆): 11.41 (bs, 1H), 8.41 (bs, 1H), 8.24 (d, 1H), 7.82-7.85 (m, 1H), 7.68-7.76 (m, 2H), 7.48-7.52 (m, 2H), 7.35 (t, 1H), 7.22 (s, 1H), 6.79 (bs, 1H), 5.82-5.86 (m, 1H), 3.14-3.18 (m, 2H), 2.75-2.81 (m, 2H), 2.37 (d, 3H), 1.53-1.61 (m, 1H), 1.47 (d, 3H), 1.36-1.40 (m, 1H); Chiral analytical SFC: RT = 5.26 min, Column: Lux Cellulose-2 (250 × 4.6 mm), 5 µ, 60% CO₂:MeOH, Flow rate = 3.0 mL/min.

3-(3-(3-Chloro-4-fluorophenyl)-1-(1-(1-oxo-1,2-dihydroisoquinolin-4-yl)ethyl)ureido)-*N*-methylpropane-1-sulfonamide: Enantiomer II **(Compound 220).** LCMS: *m*/*z* found 495.1/497.1 [M+H]⁺, RT = 7.17 min (Method A); ¹H NMR (400 MHz, DMSO-d₆): 11.41 (bs, 1H), 8.41 (bs, 1H), 8.24 (d, 1H), 7.82-7.85 (m, 1H), 7.68-7.76 (m, 2H), 7.48-7.52 (m, 2H), 7.35 (t, 1H), 7.22 (s, 1H), 6.79 (bs, 1H), 5.82-5.86 (m, 1H), 3.14-3.18 (m, 2H), 2.75-2.81 (m, 2H), 2.37 (d, 3H), 1.53-1.61 (m, 1H), 1.47 (d, 3H), 1.36-1.40 (m, 1H); Chiral analytical SFC: RT = 8.12 min, Column: Lux Cellulose-2 (250 × 4.6 mm), 5 µ, 60% CO₂:MeOH, Flow rate = 3.0 mL/min.

### 4-(1-(1-Hydroxyisoquinolin-4-yl)ethylamino)butanenitrile (VIIIbh)

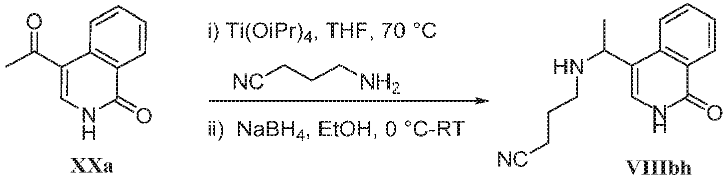

To a solution of 0.5 g (2.7 mmol, 1.0 eq.) of 4-acetylisoquinolin-1(2*H*)-one **(XXa)** in 5 mL of THF under a nitrogen atmosphere was added 0.32 g (2.7 mmol 1.0 eq.) of 4-aminobutanenitrile followed by 5 mL of titanium (IV) isopropoxide and the mixture was heated at 70 °C for 6 h. The mixture was allowed to cool to room temperature and then further cooled to 0 °C. The mixture was diluted with 5 mL of ethanol and 0.20 g (5.3 mmol, 2.0 eq.) of sodium borohydride was added. The mixture was then allowed to warm to room temperature and stirred for 2 h. The reaction was filtered through CELITE^{®} and the pad was washed with 5 mL of ethanol. The filtrate was concentrated *in vacuo* and the residue was purified by reverse-phase chromatography (REVELERIS^{®} C18 column, eluting with a linear gradient of 10-60% methanol and water) to provide 0.47 g (1.39 mmol, 52%) of 4-(1-(1-hydroxyisoquinolin-4-yl)ethylamino)butanenitrile **(VIIIbh).**

### 3-(3-Chloro-4-fluorophenyl)-1-(3-cyanopropyl)-1-(1-(1-oxo-1,2-dihydroisoquinolin-4-yl)ethyl)urea (Compounds 114 & 115)

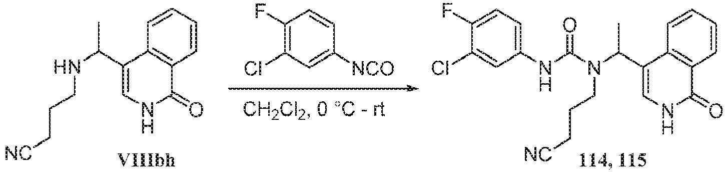

Racemic 3-(3-chloro-4-fluorophenyl)-1-(3-cyanopropyl)-1-(1-(1-oxo-1,2-dihydroisoquinolin-4-yl)ethyl)urea was synthesized in a similar manner as described above from 4-(1-(1-hydroxyisoquinolin-4-yl)ethylamino)butanenitrile **(VIIIbh)** and 2-chloro-1-fluoro-4-isocyanatobenzene. The enantiomers were subsequently separated by SFC, Column: Chiralpak IA (30 × 250 mm) 5 µ, 70% CO₂:MeOH, flow rate 90 g/min.

3-(3-Chloro-4-fluorophenyl)-1-(3-cyanopropyl)-1-(1-(1-oxo-1,2-dihydroisoquinolin-4-yl)ethyl)urea: Enantiomer I **(Compound 114).** LCMS: *m*/*z* found 427.2/429.2 [M+H]⁺, 6.44 min (Method A); ¹H NMR (400 MHz, DMSO-d₆): 11.40 (s, 1H), 8.46 (s, 1H), 8.26 (d, 1H), 7.82 (d, 1H), 7.76 (t, 1H), 7.68 (d, 1H), 7.52 (t, 2H), 7.36 (t, 1H), 7.22 (s, 1H), 5.83 (q, 1H), 3.16 (m, 2H), 2.26 (t, 2H), 1.55 (m, 1H), 1.48 (d, 3H), 1.12 (m, 1H); Chiral analytical SFC: RT = 1.75 min, Column: Chiralcel IG-3 (250 × 4.6 mm), 5 µ, 60% CO₂:MeOH, Flow rate = 3.0 mL/min.

3-(3-Chloro-4-fluorophenyl)-1-(3-cyanopropyl)-1-(1-(1-oxo-1,2-dihydroisoquinolin-4-yl)ethyl)urea: Enantiomer II **(Compound 115).** LCMS: *m*/*z* found 427.2/429.2 [M+H]⁺, 6.44 min (Method A); ¹H NMR (400 MHz, DMSO-d₆): 11.40 (s, 1H), 8.46 (s, 1H), 8.26 (d, 1H), 7.82 (d, 1H), 7.76 (t, 1H), 7.68 (d, 1H), 7.52 (t, 2H), 7.36 (t, 1H), 7.22 (s, 1H), 5.83 (q, 1H), 3.16 (m, 2H), 2.26 (t, 2H), 1.55 (m, 1H), 1.48 (d, 3H), 1.12 (m, 1H); Chiral analytical SFC: RT = 2.56 min, Column: Chiralcel IG-3 (250 × 4.6 mm), 5 µ, 60% CO₂:MeOH, Flow rate = 3.0 mL/min.

### 3-(1-(1-Hydroxyisoquinolin-4-yl)ethylamino)-N,N-dimethylpropanamide (VIIIbi)

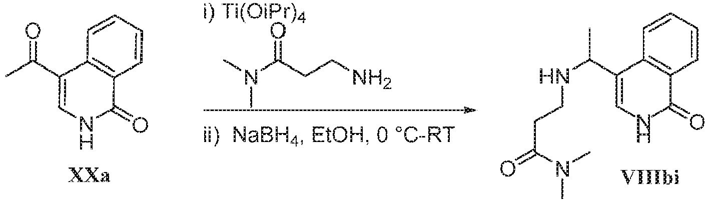

To a solution of 0.5 g (2.7 mmol, 1.0 eq.) of 4-acetylisoquinolin-1(2*H*)-one **(XXa)** in 5 mL of titanium (IV) isopropoxide under a nitrogen atmosphere was added 0.46 g (4.0 mmol 1.5 eq.) of 3-amino-*N*,*N-*dimethylpropanamide and the mixture was stirred at room temperature for 16 h. The mixture was allowed to cool to room temperature and then further cooled to 0 °C. The mixture was diluted with 5 mL of ethanol and 0.20 g (5.3 mmol, 2.0 eq.) of sodium borohydride was added. The mixture was then allowed to warm to room temperature and stirred for 2 h. The reaction was filtered through CELITE^{®} and the pad was washed with 5 mL of ethanol. The filtrate was concentrated *in vacuo* and the residue was purified by reverse-phase chromatography (REVELERIS^{®} C18 column, eluting with a linear gradient of 10-60% methanol/water) to provide 0.4 g (1.39 mmol, 52%) of 3-(1-(1-hydroxyisoquinolin-4-yl)ethylamino)-*N*,*N-*dimethylpropanamide **(VIIIbi).** ¹H NMR (400 MHz, DMSO-d₆): 11.18 (s, 1H), 8.25 (d, 1H), 8.01 (d, 1H), 7.73 (t, 1H), 7.50 (t, 1H), 7.17 (d, 1H), 4.10 (q, 1H), 2.95 (s, 3H), 2.80 (s, 3H), 2.69 (t, 2H), 2.50 (t, 2H), 1.34 (d, 3H).

### 3-(3-(3-Chloro-4-fluorophenyl)-1-(1-(1-oxo-1,2-dihydroisoquinolin-4-yl)ethyl)ureido)-N,N-dimethylpropanamide (Compounds 105 & 106)

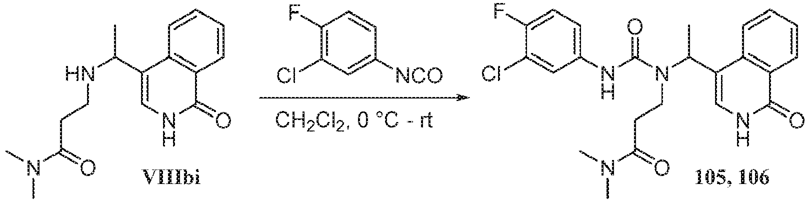

Racemic 3-(3-(3-chloro-4-fluorophenyl)-1-(1-(1-oxo-1,2-dihydroisoquinolin-4-yl)ethyl)ureido)-*N*,*N*-dimethylpropanamide was synthesized in a similar manner as described above from 3-(1-(1-hydroxyisoquinolin-4-yl)ethylamino)-*N*,*N*-dimethylpropanamide **(VIIIbi)** and 2-chloro-1-fluoro-4-isocyanatobenzene. The enantiomers were subsequently separated by SFC, Column: Chiralpak IA (30 × 250 mm) 5 µ, 70% CO₂:MeOH, flow rate 100 g/min.

3-(3-(3-Chloro-4-fluorophenyl)-1-(1-(1-oxo-1,2-dihydroisoquinolin-4-yl)ethyl)ureido)-*N*,*N*-dimethylpropanamide: Enantiomer I **(Compound 105).** LCMS: *m*/*z* found 459.3/461.3 [M+H]⁺, 5.89 min (Method A); ¹H NMR (400 MHz, DMSO-d₆): 11.22 (s, 1H), 9.50 (s, 1H), 8.26 (d, 1H), 7.87 (dd, 1H), 7.77 (t, 1H), 7.61 (d, 1H),7.54 (t, 1H), 7.45 (t, 1H), 7.35 (t, 1H), 7.23 (s, 1H), 5.84 (q, 1H), 3.44 (t, 2H), 2.69 (s, 3H), 2.39 (t, 3H), 1.93-2.07 (m, 2H), 1.47 (d, 3H); Chiral analytical SFC: RT = 1.29 min, Column: Chiralcel OD (150 × 4.6 mm), 5 µ, 60% CO₂:MeOH, Flow rate = 3.0 mL/min.

3-(3-(3-Chloro-4-fluorophenyl)-1-(1-(1-oxo-1,2-dihydroisoquinolin-4-yl)ethyl)ureido)-*N*,*N*-dimethylpropanamide: Enantiomer II **(Compound 106).** LCMS: *m*/*z* found 459.3/461.3 [M+H]⁺, 5.86 min (Method A); ¹H NMR (400 MHz, DMSO-d₆): 11.22 (s, 1H), 9.50 (s, 1H), 8.26 (d, 1H), 7.87 (dd, 1H), 7.77 (t, 1H), 7.61 (d, 1H),7.54 (t, 1H), 7.45 (t, 1H), 7.35 (t, 1H), 7.23 (s, 1H), 5.84 (q, 1H), 3.44 (t, 2H), 2.69 (s, 3H), 2.39 (t, 3H), 1.93-2.07 (m, 2H), 1.47 (d, 3H); Chiral analytical SFC: RT = 1.84 min, Column: Chiralcel OD (150 × 4.6 mm), 5 µ, 60% CO₂:MeOH, Flow rate = 3.0 mL/min.

### 2-((1-(1-Oxo-1,2-dihydroisoquinolin-4-yl)ethyl)amino)acetamide (VIIIbj)

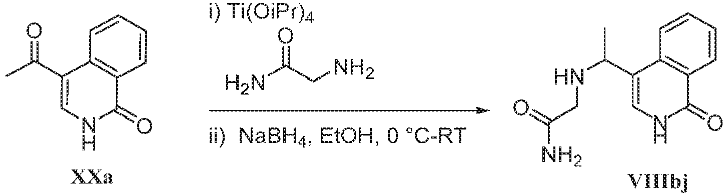

To a solution of 0.5 g (2.7 mmol, 1.0 eq.) of 4-acetylisoquinolin-1(2*H*)-one **(XXa)** in 5 mL of titanium (IV) isopropoxide under a nitrogen atmosphere was added 0.29 g (4.0 mmol 1.5 eq.) of 2-aminoacetamide and the mixture was stirred at room temperature for 16 h. The mixture was allowed to cool to room temperature and then further cooled to 0 °C. The mixture was diluted with 5 mL of ethanol and 0.20 g (5.3 mmol, 2.0 eq.) of sodium borohydride was added. The mixture was then allowed to warm to room temperature and stirred for 2 h. The reaction was filtered through CELITE^{®} and the pad was washed with 5 mL of ethanol. The filtrate was concentrated *in vacuo* and the residue was purified by reverse-phase chromatography (REVELERIS^{®} C18 column, eluting with a linear gradient of 10-60% methanol and water) to provide 0.29 g (1.1 mmol, 44%) of 2-((1-(1-oxo-1,2-dihydroisoquinolin-4-yl)ethyl)amino)acetamide **(VIIIbj).** ¹H NMR (400 MHz, DMSO-d₆) δ 11.20 (bs, 1H), 8.25 (d, 1H), 7.99 (d, 1H), 7.74 (t, 1H), 7.51 (t, 2H), 7.27 (s, 1H), 7.16 (d, 1H), 7.04 (s, 1H), 4.06 (q, 1H), 2.95-3.09 (m, 2H), 1.35 (d, 3H).

### 2-(3-(3-Chloro-4-fluorophenyl)-1-(1-(1-oxo-1,2-dihydroisoquinolin-4-yl)ethyl)ureido) acetamide (Compound 128)

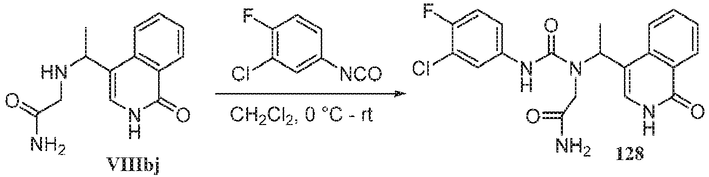

Racemic 2-(3-(3-chloro-4-fluorophenyl)-1-(1-(1-oxo-1,2-dihydroisoquinolin-4-yl)ethyl)ureido) acetamide **(Compound 128)** was synthesized in a similar manner as described above from 2-((1-(1-oxo-1,2-dihydroisoquinolin-4-yl)ethyl)amino)acetamide **(VIIIbj)** and 2-chloro-1-fluoro-4-isocyanatobenzene. LCMS: *m*/*z* found 417.1/419.1 [M+H]⁺; ¹H NMR (400 MHz, DMSO-d₆) δ 11.20 (bs, 1H), 8.76 (s, 1H), 8.23 (d, 1H), 7.79 (d, 1H), 7.65-7.73 (m, 2H), 7.50 (t, 1H), 7.70 (s, 1H), 7.32 (t, 1H), 7.17 (s, 1H), 7.11 (d, 1H), 6.96 (s, 1H), 5.85 (d, 1H), 3.85 (d, 1H), 3.56 (d, 1H), 1.41 (d, 3H).

### N-Methyl-2-((1-(1-oxo-1,2-dihydroisoquinolin-4-yl)ethyl)amino)acetamide (VIIIbk)

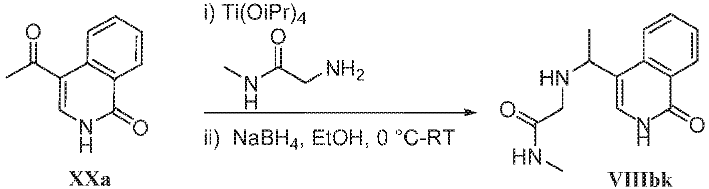

To a solution of 0.5 g (2.7 mmol, 1.0 eq.) of 4-acetylisoquinolin-1(2*H*)-one **(XXa)** in 5 mL of titanium (IV) isopropoxide under a nitrogen atmosphere was added 0.35 g (4.0 mmol 1.5 eq.) of 2-amino-*N*-methylacetamide hydrochloride and the mixture was stirred at room temperature for 16 h. The mixture was allowed to cool to room temperature and then further cooled to 0 °C. The mixture was diluted with 5 mL of ethanol and 0.20 g (5.3 mmol, 2.0 eq.) of sodium borohydride was added. The mixture was then allowed to warm to room temperature and stirred for 2 h. The reaction was filtered through CELITE^{®} and the pad was washed with 5 mL of ethanol. The filtrate was concentrated *in vacuo* and the residue was purified by reverse-phase chromatography (REVELERIS^{®} C18 column, eluting with a linear gradient of 10-60% methanol and water) to provide 0.46 g (1.4 mmol, 52%) of *N*-methyl-2-((1-(1-oxo-1,2-dihydroisoquinolin-4-yl)ethyl)amino)acetamide **(VIIIbk).** ¹H NMR (400 MHz, DMSO-d₆) δ 11.20 (bs, 1H), 8.25 (d, 1H), 7.97 (d, 1H), 7.75 (t, 1H), 7.52 (t, 1H), 7.23 (d, 1H), 7.18 (s, 1H), 4.10 (q, 1H), 3.07 (t, 2H), 2.58 (d, 3H), 1.36 (d, 3H).

### 2-(3-(3-Chloro-4-fluorophenyl)-1-(1-(1-oxo-1,2-dihydroisoquinolin-4-yl)ethyl)ureido)-N-methylacetamide (Compounds 116 & 129)

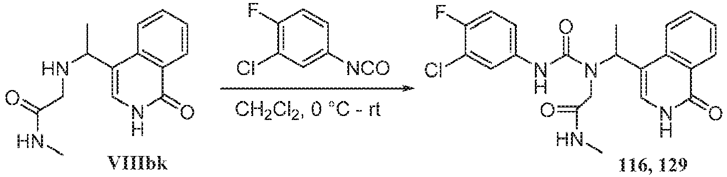

2-(3-(3-Chloro-4-fluorophenyl)-1-(1-(1-oxo-1,2-dihydroisoquinolin-4-yl)ethyl)ureido)-N methylacetamide **(Compound 116)** was synthesized in a similar manner as described above from *N*-methyl-2-((1-(1-oxo-1,2-dihydroisoquinolin-4-yl)ethyl)amino)acetamide **(VIIIbk)** and 2-chloro-1-fluoro-4-isocyanatobenzene. The enantiomers were subsequently separated by SFC, Column: Chiralpak IA (30 × 250 mm) 5 µ, 70% CO₂:MeOH, flow rate 90 g/min.

2-(3-(3-Chloro-4-fluorophenyl)-1-(1-(1-oxo-1,2-dihydroisoquinolin-4-yl)ethyl)ureido)-N-methylacetamide: Enantiomer I. LCMS: *m*/*z* found 431.3/433.3 [M+H]⁺, RT = 6.29 min (Method A); ¹H NMR (400 MHz, DMSO-d₆): δ 11.40 (s, 1H), 8.90 (s, 1H), 8.23 (d, 1H), 7.79 (dd, 1H), 7.73 (t, 1H), 7.59-7.66 (d, 2H), 7.50 (t, 1H), 7.40 (t, 1H), 7.33 (t, 1H), 7.11 (s, 1H), 5.84 (q, 1H), 3.81 (d, 1H), 3.60 (d, 1H), 2.36 (d, 3H), 1.41 (d, 3H); Chiral analytical SFC: RT = 1.66 min, Column: Chiralcel OD-3 (150 × 4.6 mm), 5 µ, 60% CO₂:MeOH, Flow rate = 3.0 mL/min.

2-(3-(3 -Chloro-4-fluorophenyl)-1 -(1 -(1 -oxo-1,2-dihydroisoquinolin-4-yl)ethyl)ureido)-N-methylacetamide: Enantiomer II **(Compound 129).** LCMS: *m*/*z* found 431.3/433.3 [M+H]⁺, RT = 6.29 min (Method A); ¹H NMR (400 MHz, DMSO-d₆): δ 11.40 (s, 1H), 8.90 (s, 1H), 8.23 (d, 1H), 7.79 (dd, 1H), 7.73 (t, 1H), 7.59-7.66 (d, 2H), 7.50 (t, 1H), 7.40 (t, 1H), 7.33 (t, 1H), 7.11 (s, 1H), 5.84 (q, 1H), 3.81 (d, 1H), 3.60 (d, 1H), 2.36 (d, 3H), 1.41 (d, 3H); Chiral analytical SFC: RT = 4.03 min, Column: Chiralcel OD-3 (150 × 4.6 mm), 5 µ, 60% CO₂:MeOH, Flow rate = 3.0 mL/min.

### N,N-Dimethyl-2-(1-(1-oxo-1,2-dihydroisoquinolin-4-yl)ethylamino)acetamide (VIIIbm)

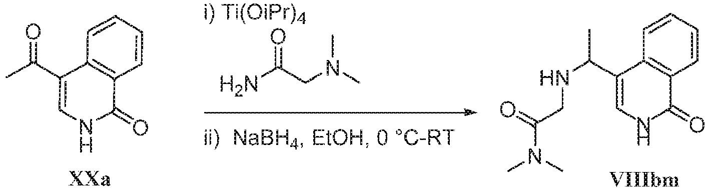

To a solution of 0.5 g (2.7 mmol, 1.0 eq.) of 4-acetylisoquinolin-1(2*H*)-one **(XXa)** in 5 mL of titanium (IV) isopropoxide under a nitrogen atmosphere was added 0.4 g (4.0 mmol 1.5 eq.) of 2-amino-*N*,*N-*dimethylacetamide and the mixture was stirred at room temperature for 16 h. The mixture was allowed to cool to room temperature and then further cooled to 0 °C. The mixture was diluted with 5 mL of ethanol and 0.20 g (5.3 mmol, 2.0 eq.) of sodium borohydride was added. The mixture was then allowed to warm to room temperature and stirred for 2 h. The reaction was filtered through CELITE^{®} and the pad was washed with 5 mL of ethanol. The filtrate was concentrated *in vacuo* and the residue was purified by reverse-phase chromatography (REVELERIS^{®} C18 column, eluting with a linear gradient of 10-60% methanol and water) to provide 0.2 g (0.7 mmol, 27%) of *N,N-*dimethyl-2-(1-(1-oxo-1,2-dihydroisoquinolin-4-yl)ethylamino)acetamide **(VIIIbm).** LCMS: *m*/*z* found 274.5 [M+H]⁺.

### 2-(3-(3-Chloro-4-fluorophenyl)-1-(1-(1-oxo-1,2-dihydroisoquinolin-4-yl)ethyl)ureido)-N,N-dimethylacetamide (Compound 147)

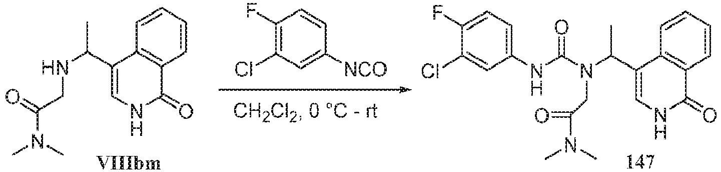

Racemic 2-(3-(3-chloro-4-fluorophenyl)-1-(1-(1-oxo-1,2-dihydroisoquinolin-4-yl)ethyl)ureido)-*N*,*N*-dimethylacetamide **(Compound 147)** was synthesized in a similar manner as described above from *N*,*N*-dimethyl-2-(1-(1-oxo-1,2-dihydroisoquinolin-4-yl)ethylamino)acetamide **(VIIIbm)** and 2-chloro-1-fluoro-4-isocyanatobenzene. LCMS: *m*/*z* found 445.1/447.1 [M+H]⁺; ¹H NMR (400 MHz, DMSO-d₆) δ 11.40 (s, 1H), 8.51 (s, 1H), 8.22 (d, 1H), 7.65-7.74 (m, 3H), 7.49 (t, 1H), 7.27-7.35 (m, 2H), 7.16 (d, 1H), 5.86 (q, 1H), 4.13 (d, 1H), 3.75 (d,1H), 2.83 (s, 3H), 2.63 (s,3H), 1.40 (d, 3H).

### tert-Butyl (3-((1-(1-oxo-1,2-dihydroisoquinolin-4-yl)ethyl)amino)propyl)carbamate (VIIIbn)

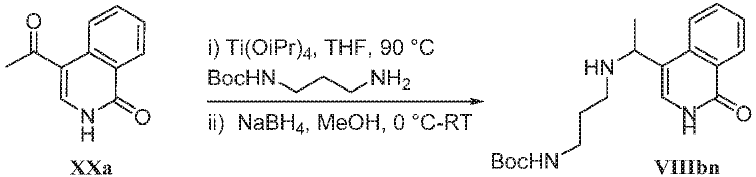

To a solution of 0.3 g (1.6 mmol, 1.0 eq.) of 4-acetylisoquinolin-1(2*H*)-one **(XXa)** in 3 mL of THF under a nitrogen atmosphere was added 0.42 g (2.4 mmol 1.5 eq.) of tert-butyl (3-aminopropyl)carbamate followed by 3 mL of titanium (IV) isopropoxide and the mixture was heated at 90 °C for 6 h. The mixture was allowed to cool to room temperature and then further cooled to 0 °C. The mixture was diluted with 2 mL of methanol and 0.12 g (3.2 mmol, 2.0 eq.) of sodium borohydride was added. The mixture was then allowed to warm to room temperature and stirred for 2 h. The reaction was quenched by the addition of 20 mL of water and filtered through CELITE^{®}. The pad was washed with 5 mL of ethyl acetate and the filtrate was extracted with 3 × 50 mL of ethyl acetate. The combined organic extracts were washed with 30 mL of brine, dried (Na₂SO₄), filtered and the solvent was removed *in vacuo* to provide 0.39 g (1.1 mmol, 70%) of tert-butyl (3-((1-(1-oxo-1,2-dihydroisoquinolin-4-yl)ethyl)amino)propyl)carbamate **(VIIIbn).** LCMS: *m*/*z* found 346.4 [M+H]⁺.

### tert-Butyl (3-(3-(3-chloro-4-fluorophenyl)-1-(1-(1-oxo-1,2-dihydroisoquinolin-4-yl)ethyl)ureido)propyl)carbamate (IXa)

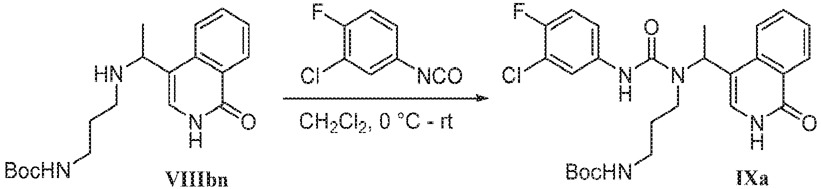

Racemic *tert*-butyl (3-(3-(3-chloro-4-fluorophenyl)-1-(1-(1-oxo-1,2-dihydroisoquinolin-4-yl)ethyl)ureido)propyl)carbamate **(IXa)** was synthesized in a similar manner as described above from tert-butyl (3-((1-(1-oxo-1,2-dihydroisoquinolin-4-yl)ethyl)amino) propyl)carbamate **(VIIIbn)** and 2-chloro-1-fluoro-4-isocyanatobenzene. LCMS: *m*/*z* found 517.1/519.1 [M+H]⁺; ¹H NMR (400 MHz, DMSO-d₆): 11.40 (bd, 1H), 6.34 (bs, 1H), 8.23 (d, 1H), 7.79-7.83 (m, 1H), 7.66-7.75 (m, 2H), 7.46-7.55 (m, 2H), 7.33 (t, 1H), 7.17 (d, 1H), 6.64 (bt, 1H), 5.80-5.84 (m, 1H), 3.01-3.08 (m, 2H), 2.68-2.75 (m, 2H), 1.45 (d, 3H), 1.29-1.36 (m, 10H), 1.11-1.16 (m, 1H).

### 1-(3-Aminopropyl)-3-(3-chloro-4-fluorophenyl)-1-(1-(1-oxo-1,2-dihydroisoquinolin-4-yl)ethyl)urea (Compounds 157 & 164)

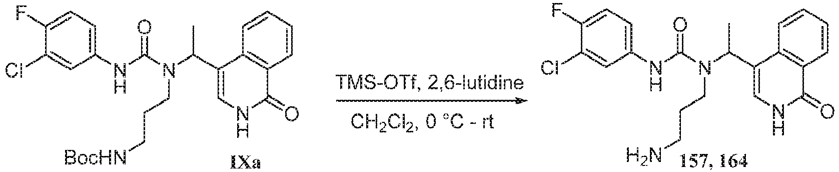

To a solution of 0.9 g (1.7 mmol, 1.0 eq.) of *tert*-butyl (3-(3-(3-chloro-4-fluorophenyl)-1-(1-(1-oxo-1,2-dihydroisoquinolin-4-yl)ethyl)ureido)propyl)carbamate **(IXa)** in 10 mL of methylene chloride at 0 °C was added 2.48 mL (20.9 mmol, 12.0 eq.) of 2,6-lutidine followed by the slow addition of 1.9 mL (10.5 mmol, 6.0 eq.) of trimethylsilyl trifluoromethanesulfonate over approximately 10 min. The mixture was allowed to warm to room temperature and stirred for 16 h. The reaction was quenched with 20 mL of saturated sodium bicarbonate solution and extracted with 3 × 30 mL of 20% methanol in methylene chloride. The combined organic extracts were dried (Na₂SO₄), filtered and the solvent was removed *in vacuo.* The residue was purified by reverse-phase column chromatography (REVERLIS^{®} C18 column, eluting with a linear gradient of 10-20% [0.1% formic acid in acetonitrile]/[0.1% formic acid in water]) to provide 0.35 g (0.84 mmol, 48%) of racemic 1-(3-aminopropyl)-3-(3-chloro-4-fluorophenyl)-1-(1-(1-oxo-1,2-dihydroisoquinolin-4-yl)ethyl)urea **(Compound 157).** LCMS: *m*/*z* found 417.4/419.4 [M+H]⁺. The enantiomers were subsequently separated by SFC, Column: Lux Cellulose-2 (30 × 250 mm) 5 µ, 75% CO₂:MeOH, flow rate 70 g/min.

1-(3-Aminopropyl)-3-(3-chloro-4-fluorophenyl)-1-(1-(1-oxo-1,2-dihydroisoquinolin-4-yl)ethyl)urea: Enantiomer I. LCMS: *m*/*z* found 417.4/419.4 [M+H]⁺; ¹H NMR (400 MHz, DMSO-d₆): 11.40 (bs, 1H), 8.23 (d, 1H), 7.81-7.83 (m, 1H), 7.72-7.76 (m, 2H), 7.62 (d, 1H), 7.48-7.52 (m, 1H), 7.38-7.42 (m, 1H), 7.28 (t, 1H), 7.20 (s, 1H), 5.85-5.89 (m, 1H), 3.17-3.20 (m, 2H), 2.21-2.26 (m, 1H), 2.08-2.12 (m, 1H), 1.46 (d, 3H), 1.08-1.12 (m, 1H), 0.69-0.76 (m, 1H); Chiral analytical SFC: RT = 1.35 min, Column: Chiralcel OZ-3 (150 × 4.6 mm), 3 µ, 60% CO₂:MeOH, Flow rate = 3.0 mL/min.
1-(3-Aminopropyl)-3-(3-chloro-4-fluorophenyl)-1-(1-(1-oxo-1,2-dihydroisoquinolin-4-yl)ethyl)urea: Enantiomer II **(Compound 164).** LCMS: *m*/*z* found 417.4/419.4 [M+H]⁺; ¹H NMR (400 MHz, DMSO-d₆): 11.40 (bs, 1H), 8.23 (d, 1H), 7.81-7.83 (m, 1H), 7.72-7.76 (m, 2H), 7.62 (d, 1H), 7.48-7.52 (m, 1H), 7.38-7.42 (m, 1H), 7.28 (t, 1H), 7.20 (s, 1H), 5.85-5.89 (m, 1H), 3.17-3.20 (m, 2H), 2.21-2.26 (m, 1H), 2.08-2.12 (m, 1H), 1.46 (d, 3H), 1.08-1.12 (m, 1H), 0.69-0.76 (m, 1H); Chiral analytical SFC: RT = 2.03 min, Column: Chiralcel OZ-3 (150 × 4.6 mm), 3 µ, 60% CO₂:MeOH, Flow rate = 3.0 mL/min.

### N¹-(1-(1-methoxyisoquinolin-4-yl)ethyl)-N²-(2,2,2-trifluoroethyl)ethane-1,2-diamine (VIaa)

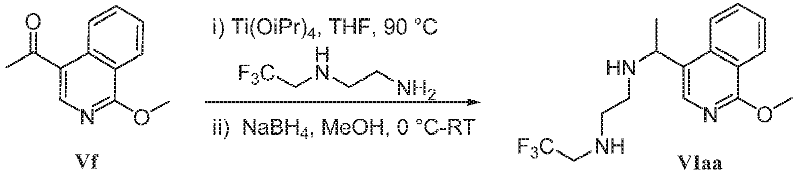

To a solution of 2.0 g (10.0 mmol, 1.0 eq.) of 1-(1-methoxyisoquinolin-4-yl)ethan-1-one (Vf) in 20 mL of THF under a nitrogen atmosphere was added 1.1 g (7.5 mmol 1.5 eq.) of *N*¹-(2,2,2-trifluoroethyl)ethane-1,2-diamine followed by 20 mL titanium (IV) isopropoxide and the mixture was heated at 90 °C for 16 h. The mixture was allowed to cool to room temperature and then further cooled to 0 °C. The mixture was diluted with 10 mL of methanol and 0.76 g (20.0 mmol, 2.0 eq.) of sodium borohydride was added. The mixture was then allowed to warm to room temperature and stirred for 2 h. The reaction was quenched by the addition of 20 mL of water and filtered through CELITE^{®}. The pad was washed with 25 mL of ethyl acetate and the biphasic mixture was extracted with 2 × 50 mL of ethyl acetate. The combined organic extracts were dried (Na₂SO₄), filtered and the solvent was removed *in vacuo.* The residue was purified by flash chromatography (SiO₂, eluting with a linear gradient of 30-45% ethyl acetate/hexanes) to provide 1.0 g (3.1 mmol, 30%) of *N*¹-(1-(1-methoxyisoquinolin-4-yl)ethyl)-*N*²-(2,2,2-trifluoroethyl)ethane-1,2-diamine **(VIaa).** LCMS: *m*/*z* found 328.2 [M+H]⁺; ¹H NMR (400 MHz, DMSO-d₆): δ 8.32 (d, 1H), 8.21 (d, 1H), 8.07 (s, 1H), 7.73-7.79 (m, 1H), 7.58-7.64 (m, 1H), 4.28-4.35 (m, 1H), 4.04 (s, 3H), 3.11-3.21 (m, 2H), 2.61-2.69 (m, 3H), 2.43-2.54 (m, 2H), 2.33 (bs, 1H), 1.41 (d, 3H).

### 3-(3-Chloro-4-fluorophenyl)-1-(1-(1-methoxyisoquinolin-4-yl)ethyl)-1-(2-((2,2,2-trifluoroethyl) amino)ethyl)urea (Compound 158)

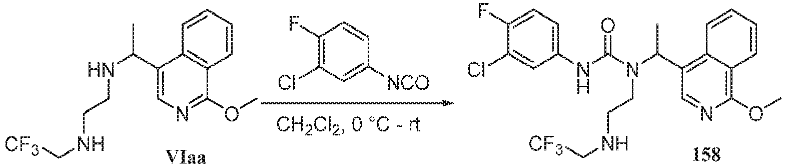

Racemic 3-(3-chloro-4-fluorophenyl)-1-(1-(1-methoxyisoquinolin-4-yl)ethyl)-1-(2-((2,2,2-trifluoroethyl) amino)ethyl)urea **(Compound 158)** was synthesized in a similar manner as described above from *N*¹-(1-(1-methoxyisoquinolin-4-yl)ethyl)-*N*²-(2,2,2-trifluoroethyl)ethane-1,2-diamine **(VIaa)** and 2-chloro-1-fluoro-4-isocyanatobenzene. LCMS: *m*/*z* found 499.1/501.1 [M+H]⁺; ¹H NMR (400 MHz, DMSO-d₆) δ 9.50 (bs, 1H), 8.23 (d, 1H), 8.15 (s, 1H), 7.92 (d, 1H), 7.74-7.83 (m, 2H), 7.62-7.66 (m, 1H), 7.38-7.43 (m, 1H), 7.33 (t, 1H), 6.07-6.11 (m, 1H), 4.08 (s, 3H), 3.10-3.14 (m, 2H), 2.76-2.93 (m, 2H), 2.67-2.73 (m, 1H), 2.08-2.14 (m, 1H), 1.90-1.98 (m, 1H), 1.60 (d, 3H).

### 4-(1-((2-((2,2,2-Trifluoroethyl)amino)ethyl)amino)ethyl)isoquinolin-1(2H)-one (VIIIbo)

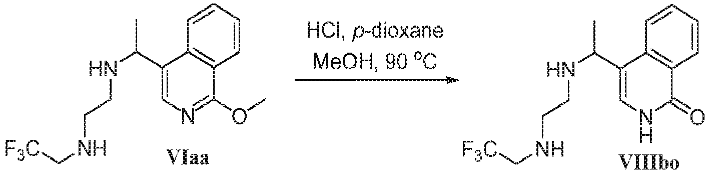

To a solution of 0.5 g (1.52 mmol, 1.0 eq.) of *N*¹-(1-(1-methoxylsoquinolin-4-yl)ethyl)-*N*²-(2,2,2-trifluoroethyl)ethane-1,2-diamine **(VIaa)** in 2 mL methanol in a sealed tube was added 5 mL of a 4 M solution of HCl in *p*-dioxane and the mixture was heated at 90 °C for 16 h. The mixture was allowed to cool to room temperature and the solvent was removed *in vacuo.* The residue was triturated with 10 mL of n-pentane and the resulting solid dried under high vacuum. The residue was redissolved in 5 mL of methanol and 2.5 g of Amberlyst A-21 basic resin was added. The mixture was stirred at room temperature for 2 h, filtered and the resin washed with 2 × 5 ml of methanol. The filtrate was concentrated *in vacuo* and the residue dried under high vacuum to provide 0.5 g of 4-(1-((2-((2,2,2-trifluoroethyl)amino)ethyl)amino)ethyl)isoquinolin-1(2*H*)-one (**VIIIbo**). LCMS: *m*/*z* found 314.4 [M+H]⁺; ¹H NMR (400 MHz, DMSO-d₆): δ 11.46 (bs, 1H), 8.25-8.28 (m, 1H), 7.96-7.99 (m, 1H), 7.72-7.79 (m, 1H), 7.54 (t, 1H), 7.45 (bs, 1H), 4.51-4.57 (m, 1H), 3.17-3.27 (m, 4H), 2.72-2.87 (m, 4H), 1.52 (d, 3H).

### 3-(3-Chloro-4-fluorophenyl)-1-(1-(1-oxo-1,2-dihydroisoquinolin-4-yl)ethyl)-1-(2-((2,2,2-trifluoroethyl)amino)ethyl)urea (Compounds 162 & 163)

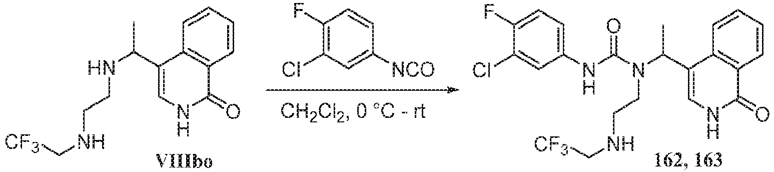

Racemic 3-(3-chloro-4-fluorophenyl)-1-(1-(1-oxo-1,2-dihydroisoquinolin-4-yl)ethyl)-1-(2-((2,2,2-trifluoroethyl)amino)ethyl)urea was synthesized in a similar manner as described above from 4-(1-((2-((2,2,2-trifluoroethyl)amino)ethyl)amino)ethyl)isoquinolin-1(2*H*)-one **(VIIIbo)** and 2-chloro-1-fluoro-4-isocyanatobenzene. The enantiomers were subsequently separated by SFC, Column: Chiralpak IG (30 × 250 mm) 5 µ, 70% CO₂:MeOH, flow rate 90 g/min.

3-(3-Chloro-4-fluorophenyl)-1-(1-(1-oxo-1,2-dihydroisoquinolin-4-yl)ethyl)-1-(2-((2,2,2-trifluoroethyl)amino)ethyl)urea: Enantiomer I **(Compound 162).** LCMS: *m*/*z* found 485.3/487.4 [M+H]⁺, RT = 6.68 min (Method: A); ¹H NMR (400 MHz, DMSO-d₆): δ 11.40 (bs, 1H), 9.51 (s, 1H), 8.23-8.25 (m, 1H), 7.71-7.78 (m, 2H), 7.63-7.65 (m, 1H), 7.48-7.52 (m, 1H), 7.36-7.41 (m, 1H), 7.32 (t, 1H), 7.19-7.21 (m, 1H), 5.80-5.83 (m, 1H), 3.12-3.16 (m, 2H), 2.83-3.01 (m, 2H), 2.76-2.81 (m, 1H), 2.30-2.36 (m, 1H), 2.04-2.09 (m, 1H), 1.45 (d, 3H); Chiral analytical SFC: RT = 2.72 min, Column: Chiralpak IG-3 (150 × 4.6 mm), 3 µ, 60% CO₂:MeOH, Flow rate = 3.0 mL/min.

3-(3-Chloro-4-fluorophenyl)-1-(1-(1-oxo-1,2-dihydroisoquinolin-4-yl)ethyl)-1-(2-((2,2,2-trifluoroethyl)amino)ethyl)urea: Enantiomer II **(Compound 163).** LCMS: *m*/*z* found 485.3/487.4 [M+H]⁺, RT = 6.68 min (Method: A); ¹H NMR (400 MHz, DMSO-d₆): δ 11.40 (bs, 1H), 9.51 (s, 1H), 8.23-8.25 (m, 1H), 7.71-7.78 (m, 2H), 7.63-7.65 (m, 1H), 7.48-7.52 (m, 1H), 7.36-7.41 (m, 1H), 7.32 (t, 1H), 7.19-7.21 (m, 1H), 5.80-5.83 (m, 1H), 3.12-3.16 (m, 2H), 2.83-3.01 (m, 2H), 2.76-2.81 (m, 1H), 2.30-2.36 (m, 1H), 2.04-2.09 (m, 1H), 1.45 (d, 3H); Chiral analytical SFC: RT = 4.05 min, Column: Chiralpak IG-3 (150 × 4.6 mm), 3 µ, 60% CO₂:MeOH, Flow rate = 3.0 mL/min.

### N¹-(2,2-Difluoroethyl)-N²-(1-(1-methoxyisoquinolin-4-yl)ethyl)ethane-1,2-diamine (VIab)

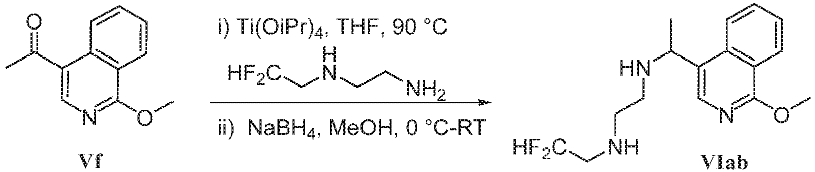

To a solution of 1.5 g (7.5 mmol, 1.0 eq.) of 1-(1-methoxyisoquinolin-4-yl)ethan-1-one **(Vf)** in 15 mL of THF under a nitrogen atmosphere was added 0.9 g (7.5 mmol 1.5 eq.) of *N*¹-(2,2-difluoroethyl)ethane-1,2-diamine followed by 15 mL titanium (IV) isopropoxide and the mixture was heated at 90 °C for 16 h. The mixture was allowed to cool to room temperature and then further cooled to 0 °C. The mixture was diluted with 10 mL of methanol and 0.57 g (14.9 mmol, 2.0 eq.) of sodium borohydride was added. The mixture was then allowed to warm to room temperature and stirred for 2 h. The reaction was quenched by the addition of 20 mL of water and filtered through CELITE^{®}. The pad was washed with 25 mL of ethyl acetate and the biphasic mixture was extracted with 2 × 50 mL of ethyl acetate. The combined organic extracts were dried (Na₂SO₄), filtered and the solvent was removed *in vacuo.* The residue was purified by flash chromatography (SiO₂, eluting with a linear gradient of 0-3% methanol/methylene chloride) to provide 0.45 g (1.51 mmol, 20%) of *N¹*-(2,2-difluoroethyl)-*N*²-(1-(1-methoxyisoquinolin-4-yl)ethyl)ethane-1,2-diamine **(VIab).** LCMS: *m*/*z* found 310.3 [M+H]⁺; ¹H NMR (400 MHz, DMSO-d₆): δ 8.31 (d, 1H), 8.21 (d, 1H), 8.07 (s, 1H), 7.74-7.79 (m, 1H), 7.59-7.63 (m, 1H), 5.95 (m, 1H), 4.31-4.37 (m, 1H), 4.05 (s, 3H), 2.77-2.87 (m, 2H), 2.44-2.65 (m, 4H), 2.34 (bs, 2H), 1.41 (d, 3H).

### 4-(1-((2-((2,2-difluoroethyl)amino)ethyl)amino)ethyl)isoquinolin-1(2H)-one dihydrochloride (VIIIbp)

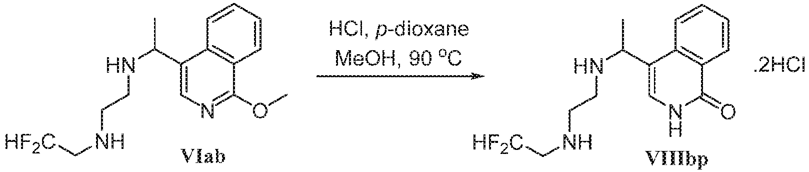

To a solution of 0.45 g (1.45 mmol, 1.0 eq.) of *N¹*-(2,2-difluoroethyl)-*N*²-(1-(1-methoxyisoquinolin-4-yl)ethyl)ethane-1,2-diamine **(VIab)** in 5 mL methanol in a sealed tube was added 5 mL of a 4 M solution of HCl inp-dioxane and the mixture was heated at 90 °C for 3 h. The mixture was allowed to cool to room temperature and the solvent was removed *in vacuo.* The residue was triturated with 10 mL of *n*-pentane and the resulting solid dried under high vacuum to provide 0.45 g of 4-(1-((2-((2,2-difluoroethyl)amino)ethyl) amino)ethyl)isoquinolin-1(2*H*)-one dihydrochloride salt **(VIIIbp).** ¹H NMR (400 MHz, DMSO-d₆): δ 11.67 (bd, 1H), 9.85 (bs, 2H), 8.30 (d, 1H), 7.97 (d, 1H), 7.79-7.84 (m, 1H), 7.71 (d, 1H), 7.55-7.61 (m, 1H), 6.44 (m, 1H), 4.93-4.98 (m, 1H), 3.59-3.61 (m, 1H), 3.37-3.46 (m, 4H), 3.21-3.30 (m, 1H), 1.67 (d, 3H).

### 3-(3-Chloro-4-fluorophenyl)-1-(2-((2,2-difluoroethyl)amino)ethyl)-1-(1-(1-oxo-1,2-dihydroisoquinolin-4-yl)ethyl)urea (Compounds 198 & 199)

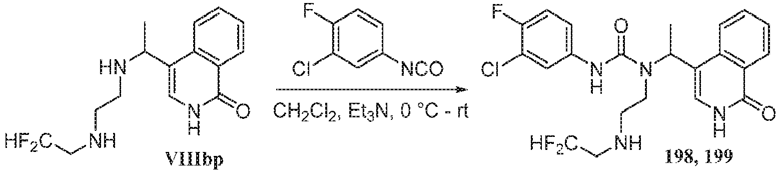

Racemic 3-(3-chloro-4-fluorophenyl)-1-(2-((2,2-difluoroethyl)amino)ethyl)-1-(1-(1-oxo-1,2-dihydroisoquinolin-4-yl)ethyl)urea was synthesized in a similar manner as described above from 4-(1-((2-((2,2-difluoroethyl)amino)ethyl) amino)ethyl)isoquinolin-1(2*H*)-one dihydrochloride salt **(VIIIbp)** and 2-chloro-1-fluoro-4-isocyanatobenzene. The enantiomers were subsequently separated by SFC, Column: Chiralcel OD-H (30 × 250 mm) 5 µ, 85% CO₂:MeOH, flow rate 60 g/min.

3-(3-Chloro-4-fluorophenyl)-1-(2-((2,2-difluoroethyl)amino)ethyl)-1-(1-(1-oxo-1,2-dihydroisoquinolin-4-yl)ethyl)urea: Enantiomer I **(Compound 198).** LCMS: *m*/*z* found 467.3/469.3 [M+H]⁺, RT = 7.06 min (Method: A); ¹H NMR (400 MHz, DMSO-d₆): δ 11.41 (d, 1H), 10.04 (bs, 1H), 8.25 (d, 1H), 7.72-7.78 (m, 2H), 7.64 (d, 1H), 7.49-7.53 (m, 1H), 7.31-7.38 (m, 2H), 7.19 (d, 1H), 5.62-5.90 (m, 2H), 3.12-3.15 (m, 2H), 2.61-2.67 (m, 2H), 2.32-4.40 (m, 1H), 1.92-1.98 (m, 1H), 1.44 (d, 3H); Chiral analytical SFC: RT = 4.13 min, Column: Chiralcel OD-H (250 × 4.6 mm), 5 µ, 75% CO₂:MeOH, Flow rate = 3.0 mL/min.

3-(3-Chloro-4-fluorophenyl)-1-(2-((2,2-difluoroethyl)amino)ethyl)-1-(1-(1-oxo-1,2-dihydroisoquinolin-4-yl)ethyl)urea: Enantiomer II **(Compound 199).** LCMS: *m*/*z* found 467.3/469.3 [M+H]⁺, RT = 7.06 min (Method: A); ¹H NMR (400 MHz, DMSO-d₆): δ 11.41 (d, 1H), 10.04 (bs, 1H), 8.25 (d, 1H), 7.72-7.78 (m, 2H), 7.64 (d, 1H), 7.49-7.53 (m, 1H), 7.31-7.38 (m, 2H), 7.19 (d, 1H), 5.62-5.90 (m, 2H), 3.12-3.15 (m, 2H), 2.61-2.67 (m, 2H), 2.32-4.40 (m, 1H), 1.92-1.98 (m, 1H), 1.44 (d, 3H); Chiral analytical SFC: RT = 5.74 min, Column: Chiralcel OD-H (250 × 4.6 mm), 5 µ, 75% CO₂:MeOH, Flow rate = 3.0 mL/min.

### Isopropyl 3-((1-(1-oxo-1,2-dihydroisoquinolin-4-yl)ethyl)amino)propanoate (VIIIbq)

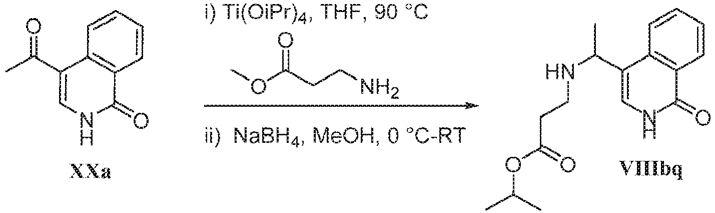

To a solution of 0.5 g (2.7 mmol, 1.0 eq.) of 4-acetylisoquinolin-1(2*H*)-one **(XXa)** in 5 mL of THF under a nitrogen atmosphere was added 0.54 g (5.3 mmol, 2.0 eq.) of methyl 3-aminopropanoate followed by 5 mL of titanium (IV) isopropoxide and the mixture was heated at 90 °C for 16 h. The mixture was allowed to cool to room temperature and then further cooled to 0 °C. The mixture was diluted with 5 mL of methanol and 0.20 g (5.3 mmol, 2.0 eq.) of sodium borohydride was added. The mixture was then allowed to warm to room temperature and stirred for 2 h. The reaction was diluted with 10 mL of water, filtered through CELITE^{®} and the pad was washed with 5 mL of ethyl acetate. The filtrate was extracted with 2 × 30 mL of ethyl acetate and the combined organic extracts were dried (NazSOa), filtered and the solvent was removed *in vacuo* and the residue was triturated with 15 mL of n-pentane to provide 0.48 g (1.58 mmol, 59%) of isopropyl 3-((1-(1-oxo-1,2-dihydroisoquinolin-4-yl)ethyl)amino)propanoate **(VIIIbq).** ¹H NMR (400 MHz, DMSO-d₆) δ 9.89 (bs, 1H), 8.46-8.49 (m, 1H), 7.90 (d, 1H), 7.69-7.73 (m, 1H), 7.49-7.53 (m, 1H), 7.31 (s, 1H), 4.99-5.05 (m, 1H), 4.11-4.18 (m, 1H), 2.81-2.89 (m, 2H), 2.45-2.49 (m, 2H), 1.61 (bs, 1H), 1.43 (d, 3H), 1.23 (d, 6H).

### 3-(3-(3-Chloro-4-fluorophenyl)-1-(1-(1-oxo-1,2-dihydroisoquinolin-4-yl)ethyl)ureido)propanoic acid (Compound 208)

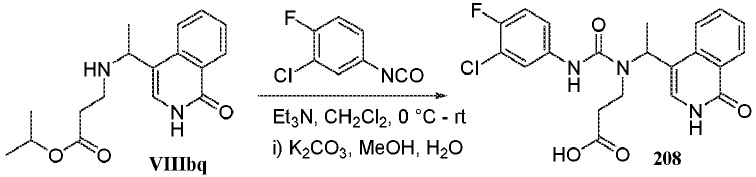

To a stirred solution of 0.48 g (1.58 mmol, 1.0 eq.) of isopropyl 3-((1-(1-oxo-1,2-dihydroisoquinolin-4-yl)ethyl)amino)propanoate **(VIIIbq)** in 5 mL of methylene chloride at 0 °C under a nitrogen atmosphere was added 1.0 mL (4.76 mmol, 3.0 eq.) of triethylamine followed by 0.28 mL (1.58 mmol, 1.0 eq.) of 2-chloro-1-fluoro-4-isocyanatobenzene. The mixture was allowed to warm to room temperature and stirred for 1 h. The mixture was diluted 20 mL of ice-cold water and extracted with 2 × 30 mL of methylene chloride. The combined organic extracts were washed with 30 mL of brine, dried (Na₂SO₄), filtered and the solvent was removed *in vacuo.* The residue was re-dissolved in 6 mL of 5:1 *v*/*v* methanol: water and 0.4 g of potassium carbonate was added. The solvent was removed *in vacuo* and residue was diluted with 10 mL of water and washed with 20 mL of 10% methanol in metyhylene chloride. The aqueous layer was acidified to pH~4 with 2 M aqueous HCl and the precipitated solid was collected by filtration, washed with 5 mL of diethyl ether and dried under vacuum to provide 0.40 g (0.92 mmol, 58%) of racemic 3-(3-(3-chloro-4-fluorophenyl)-1 -(1 -(1 -oxo-1,2-dihydroisoquinolin-4-yl)ethyl)ureido)propanoic acid. The enantiomers were subsequently separated by SFC, Column: (R,R) Whelk-01 (30 × 250 mm) 5 µ, 60% CO₂:MeOH, flow rate 90 g/min.

3-(3-(3-Chloro-4-fluorophenyl)-1-(1-(1-oxo-1,2-dihydroisoquinolin-4-yl)ethyl)ureido)propanoic acid: Enantiomer I. LCMS: *m*/*z* found 432.4/434.4 [M+H]⁺, 7.27 min (Method A); ¹H NMR (400 MHz, DMSO-d₆): 12.20 (bs, 1H), 11.39 (bs, 1H), 9.72 (bs, 1H), 8.23-8.44 (m, 1H), 7.83-7.85 (m, 1H), 7.72-7.76 (m, 1H), 7.65 (d, 1H), 7.49-7.54 (m, 2H), 7.31 (t, 1H), 7.23 (d, 1H), 5.83-5.86 (m, 1H), 3.23-3.31 (m, 2H), 1.89-1.96 (m, 1H), 1.69-1.77 (m, 1H), 1.45 (d, 3H); Chiral analytical SFC: RT = 7.57 min, Column: (R,R) Whelk-01 (150 × 4.6 mm), 5 µ, 55% CO₂:MeOH, Flow rate = 3.0 mL/min.

3-(3-(3-Chloro-4-fluorophenyl)-1-(1-(1-oxo-1,2-dihydroisoquinolin-4-yl)ethyl)ureido)propanoic acid: Enantiomer II **(Compound 208)** LCMS: *m*/*z* found 432.4/434.4 [M+H]⁺, 7.27 min (Method A); ¹H NMR (400 MHz, DMSO-d₆): 12.20 (bs, 1H), 11.39 (bs, 1H), 9.72 (bs, 1H), 8.23-8.44 (m, 1H), 7.83-7.85 (m, 1H), 7.72-7.76 (m, 1H), 7.65 (d, 1H), 7.49-7.54 (m, 2H), 7.31 (t, 1H), 7.23 (d, 1H), 5.83-5.86 (m, 1H), 3.23-3.31 (m, 2H), 1.89-1.96 (m, 1H), 1.69-1.77 (m, 1H), 1.45 (d, 3H); Chiral analytical SFC: RT = 10.50 min, Column: (R,R) Whelk-01 (150 × 4.6 mm), 5 µ, 55% CO₂:MeOH, Flow rate = 3.0 mL/min.

### Isopropyl 4-(3-(3-chloro-4-fluorophenyl)-1-(1-(1-oxo-1,2-dihydroisoquinolin-4-yl)ethyl)ureido)butanoate (VIIIbr)

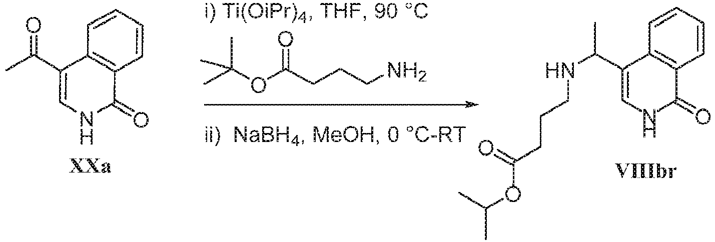

To a solution of 0.7 g (3.7 mmol, 1.0 eq.) of 4-acetylisoquinolin-1(2*H*)-one **(XXa)** in 7 mL of THF under a nitrogen atmosphere was added 1.2 g (7.5 mmol, 2.0 eq.) of tert-butyl 4-aminobutanoate followed by 7 mL of titanium (IV) isopropoxide and the mixture was heated at 90 °C for 16 h. The mixture was allowed to cool to room temperature and then further cooled to 0 °C. The mixture was diluted with 5 mL of methanol and 0.28 g (7.5 mmol, 2.0 eq.) of sodium borohydride was added. The mixture was then allowed to warm to room temperature and stirred for 2 h. The reaction was diluted with 10 mL of water, filtered through CELITE^{®} and the pad was washed with 5 mL of ethyl acetate. The filtrate was extracted with 2 × 30 mL of ethyl acetate and the combined organic extracts were dried (Na₂SO₄), filtered and the solvent was removed *in vacuo.* The residue was purified by flash chromatography (SiO₂, eluting with a linear gradient of 0-6% methanol/methylene chloride) to provide 0.28 g (0.63 mmol, 17%) of isopropyl 4-((1-(1-oxo-1,2-dihydroisoquinolin-4-yl)ethyl)amino)butanoate **(VIIIbr).** LCMS: *m*/*z* found 317.3 [M+H]⁺; ¹H NMR (400 MHz, DMSO-d₆) δ 11.26 (bs, 1H), 8.25 (d, 1H), 7.98 (d, 1H), 7.71-7.76 (m, 1H), 7.47-7.53 (m, 1H), 7.25 (bs, 1H), 4.80-4.87 (m, 1H), 4.18-4.24 (m, 1H), 2.42-2.64 (m, 3H), 2.26-2.32 (m, 2H), 1.65-1.73 (m, 2H), 1.39 (d, 3H), 1.12 (d, 6H).

### 3-(3-(3-Chloro-4-fluorophenyl)-1-(1-(1-oxo-1,2-dihydroisoquinolin-4-yl)ethyl)ureido)propanoic acid (IXb)

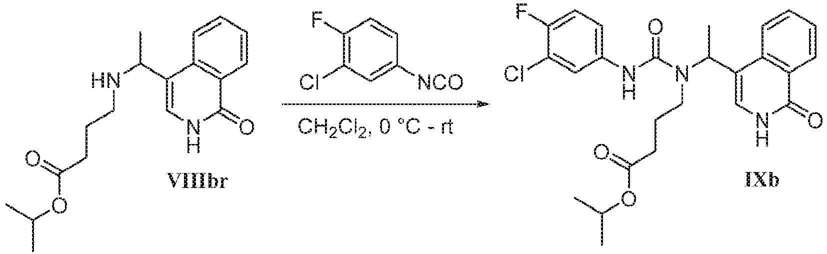

To a stirred solution of 0.17 g (0.53 mmol, 1.0 eq.) of isopropyl 4-((1-(1-oxo-1,2-dihydroisoquinolin-4-yl)ethyl)amino)butanoate **(VIIIbr)** in 2 mL of methylene chloride at 0 °C under a nitrogen atmosphere was added 0.05 mL (0.45 mmol, 0.8 eq.) of 2-chloro-1-fluoro-4-isocyanatobenzene. The mixture was allowed to warm to room temperature and stirred for 1 h. The mixture was diluted with 20 mL of ice-cold water and extracted with 2 × 30 mL of 10% methanol in methylene chloride. The combined organic extracts were washed with 30 mL of brine, dried (Na₂SO₄), filtered and the solvent was removed *in vacuo.* The residue was purified by flash chromatography (SiO₂, eluting with a linear gradient of 0-3% methanol in methylene chloride) to provide 0.15 g (0.30 mmol, 57%) of isopropyl 4-(3-(3-chloro-4-fluorophenyl)-1-(1-(1-oxo-1,2-dihydroisoquinolin-4-yl)ethyl)ureido)butanoate **(IXb).** LCMS: *m*/*z* found 488.34 [M+H]⁺.

### 4-(3-(3-Chloro-4-fluorophenyl)-1-(1-(1-oxo-1,2-dihydroisoquinolin-4-yl)ethyl)ureido)butanoic acid (Compounds 226 & 227)

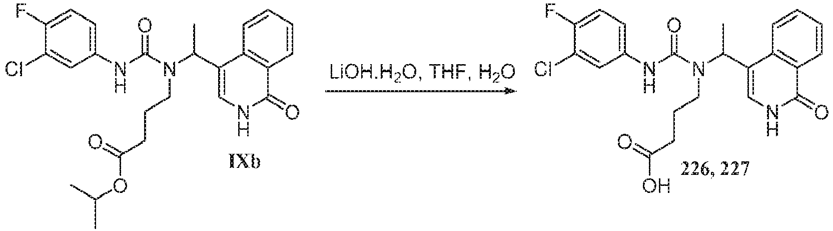

To a stirred solution of 0.11 g (0.22 mmol, 1.0 eq.) of isopropyl 4-(3-(3-chloro-4-fluorophenyl)-1-(1-(1-oxo-1,2-dihydroisoquinolin-4-yl)ethyl)ureido)butanoate **(IXb)** in 1 mL of THF was added a solution of 0.09 g (2.25 mmol, 10.0 eq.) of lithium hydroxide monohydrate in 1 mL of water, and the mixture was stirred at room temperature for 16 h. The mixture was diluted 5 mL of water and washed with 20 mL of diethyl ether. The aqueous phase was acidified to pH~3 with 2 M aqueous HCl and the precipitated solid was collected by filtration, washed with 5 mL of diethyl ether and dried under vacuum to provide 0.09 g (0.20 mmol, 91%) of racemic 4-(3-(3-chloro-4-fluorophenyl)-1-(1-(1-oxo-1,2-dihydroisoquinolin-4-yl)ethyl)ureido)butanoic acid. The enantiomers were subsequently separated by SFC, Column: Chiralcel OD-H (30 × 250 mm) 5 µ, 65% CO₂:MeOH, flow rate 70 g/min.

4-(3-(3-Chloro-4-fluorophenyl)-1-(1-(1-oxo-1,2-dihydroisoquinolin-4-yl)ethyl)ureido)butanoic acid: Enantiomer I **(Compound 226)** LCMS: *m*/*z* found 446.3/448.3 [M+H]⁺, 5.81 min (Method A); ¹H NMR (400 MHz, DMSO-d₆): 12.41 (bs, 1H), 11.39 (bd, 1H), 8.85 (bs, 1H), 8.23 (d, 1H), 7.92-7.94 (m, 1H), 7.68-7.76 (m, 2H), 7.58-7.63 (m, 1H), 7.47-7.51 (m, 1H), 7.33 (t, 1H), 7.19 (d, 1H), 5.85-5.89 (m, 1H), 2.98-3.04 (m, 2H), 1.96-2.03 (m, 2H), 1.44 (d, 3H), 1.32-1.41 (m, 1H), 0.91-1.01 (m, 1H); Chiral analytical SFC: RT = 2.40 min, Column: Chiralcel OD-3 (150 × 4.6 mm), 3 µ, 70% CO₂:MeOH, Flow rate = 3.0 mL/min.

4-(3-(3-Chloro-4-fluorophenyl)-1-(1-(1-oxo-1,2-dihydroisoquinolin-4-yl)ethyl)ureido)butanoic acid: Enantiomer II **(Compound 227)** LCMS: *m*/*z* found 446.3/448.3 [M+H]⁺, 5.81 min (Method A); ¹H NMR (400 MHz, DMSO-d₆): 12.41 (bs, 1H), 11.39 (bd, 1H), 8.85 (bs, 1H), 8.23 (d, 1H), 7.92-7.94 (m, 1H), 7.68-7.76 (m, 2H), 7.58-7.63 (m, 1H), 7.47-7.51 (m, 1H), 7.33 (t, 1H), 7.19 (d, 1H), 5.85-5.89 (m, 1H), 2.98-3.04 (m, 2H), 1.96-2.03 (m, 2H), 1.44 (d, 3H), 1.32-1.41 (m, 1H), 0.91-1.01 (m, 1H); Chiral analytical SFC: RT = 3.64 min, Column: Chiralcel OD-3 (150 × 4.6 mm), 3 µ, 70% CO₂:MeOH, Flow rate = 3.0 mL/min.

### 4-(1-(((2H-1,2,3-Triazol-4-yl)methyl)amino)ethyl)isoquinolin-1(2H)-one (VIIIbx)

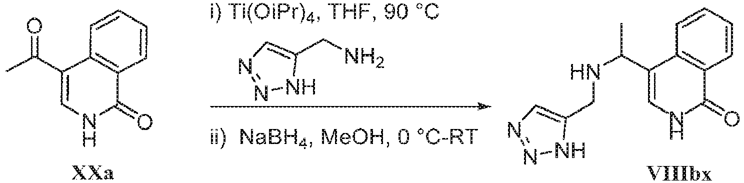

To a solution of 0.5 g (2.7 mmol, 1.0 eq.) of 4-acetylisoquinolin-1(2*H*)-one **(XXa)** in 5 mL of THF under a nitrogen atmosphere was added 0.4 g (4.0 mmol, 1.5 eq.) of (2*H*-1,2,3-triazol-4-yl)methanamine followed by 5 mL of titanium (IV) isopropoxide and the mixture was heated at 90 °C for 16 h. The mixture was allowed to cool to room temperature and then further cooled to 0 °C. The mixture was diluted with 5 mL of methanol and 0.2 g (5.3 mmol, 2.0 eq.) of sodium borohydride was added. The mixture was then allowed to warm to room temperature and stirred for 2 h. The reaction was diluted with 50 mL of water, filtered through CELITE^{®} and the filtrate was extracted with 2 × 100 mL of 10% methanol in methylene chloride. The combined organic extracts were washed with 100 mL of brine, dried (Na₂SO₄), filtered and the solvent was removed *in vacuo* to provide 0.5 g of crude 4-(1-(((2H-1,2,3-triazol-4-yl)methyl)amino)ethyl)isoquinolin-1(2*H*)-one **(VIIIbx).** LCMS: *m*/*z* found 270.1 [M+H]⁺.

### 1-((2H-1,2,3-triazol-4-yl)methyl)-3-(3-chloro-4-fluorophenyl)-1-(1-(1-oxo-1,2-dihydroisoquinolin-4-yl)ethyl)urea (Compounds 266 & 267)

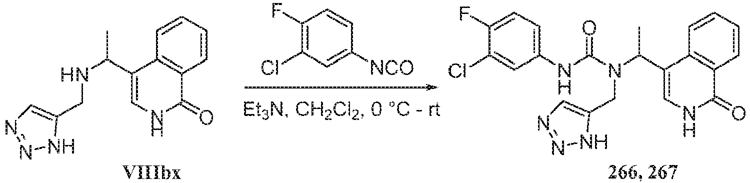

Racemic 1-((2*H*-1,2,3-triazol-4-yl)methyl)-3-(3-chloro-4-fluorophenyl)-1-(1-(1-oxo-1,2-dihydroisoquinolin-4-yl)ethyl)urea was synthesized in a similar manner as described above from 4-(1-(((2*H*-1,2,3-triazol-4-yl)methyl)amino)ethyl)isoquinolin-1(2*H*)-one **(VIIIbx)** and 2-chloro-1-fluoro-4-isocyanatobenzene. The enantiomers were subsequently separated by SFC, Column: Chiralcel OD-H (30 × 250 mm) 5 µ, 85% CO₂:MeOH, flow rate 90 g/min.

1-((2*H*-1,2,3-Triazol-4-yl)methyl)-3-(3-chloro-4-fluorophenyl)-1-(1-(1-oxo-1,2-dihydroisoquinolin-4-yl)ethyl)urea: Enantiomer I **(Compound 266).** LCMS: *m*/*z* found 441.1/443.1 [M+H]⁺, RT = 8.11 min (Method A); ¹H NMR (400 MHz, DMSO-d₆) δ 11.38 (bs, 1H), 9.27 (bs, 1H), 8.18 (d, 2H), 7.82-7.84 (m, 1H), 7.60-7.67 (m, 2H), 7.42-7.47 (m, 2H), 7.33 (t, 1H), 7.25 (d, 1H), 6.88 (s, 1H), 5.88-5.91 (m, 1H), 4.39 (d, 1H), 4.32 (d, 1H), 1.46 (d, 3H); Chiral analytical SFC: RT = 2.13 min, Column: Chiralcel-OD-3 (250 × 4.6 mm), 3 µ, 70% CO₂:MeOH, Flow rate = 3.0 mL/min.

1-((2*H*-1,2,3-Triazol-4-yl)methyl)-3-(3-chloro-4-fluorophenyl)-1-(1-(1-oxo-1,2-dihydroisoquinolin-4-yl)ethyl)urea: Enantiomer II **(Compound 267).** LCMS: *m*/*z* found 441.1/443.1 [M+H]⁺, RT = 8.14 min (Method A); ¹H NMR (400 MHz, DMSO-d₆) δ 11.38 (bs, 1H), 9.27 (bs, 1H), 8.18 (d, 2H), 7.82-7.84 (m, 1H), 7.60-7.67 (m, 2H), 7.42-7.47 (m, 2H), 7.33 (t, 1H), 7.25 (d, 1H), 6.88 (s, 1H), 5.88-5.91 (m, 1H), 4.39 (d, 1H), 4.32 (d, 1H), 1.46 (d, 3H); Chiral analytical SFC: RT = 2.86 min, Column: Chiralcel-OD-3 (250 × 4.6 mm), 3 µ, 70% CO₂:MeOH, Flow rate = 3.0 mL/min.

### 4-(1-(((1H-1,2,4-Triazol-5-yl)methyl)amino)ethyl)isoquinolin-1(2H)-one (VIII by)

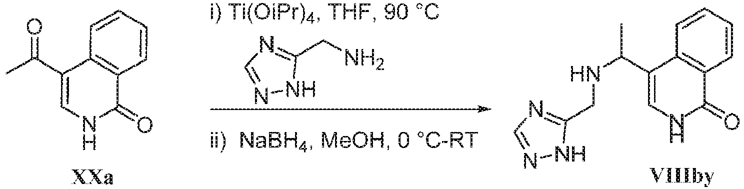

To a solution of 0.45 g (2.4 mmol, 1.0 eq.) of 4-acetylisoquinolin-l(2*H*)-one **(XXa)** in 5 mL of THF under a nitrogen atmosphere was added 0.47 g (4.0 mmol, 1.5 eq.) of (1*H*-1,2,4-triazol-5-yl)methanamine followed by 5 mL of titanium (IV) isopropoxide and the mixture was heated at 90 °C for 16 h. The mixture was allowed to cool to room temperature and then further cooled to 0 °C. The mixture was diluted with 5 mL of methanol and 0.33 g (8.7 mmol, 3.6 eq.) of sodium borohydride was added. The mixture was then allowed to warm to room temperature and stirred for 2 h. The reaction was diluted with 50 mL of water, filtered through CELITE^{®} and the filtrate was extracted with 2 x 100 mL of 10% methanol in methylene chloride. The combined organic extracts were washed with 100 mL of brine, dried (Na₂SO₄), filtered and the solvent was removed *in vacuo.* The residue was purified by reverse-phase chromatography (C-18, eluting with a linear gradient of 10-60% acetonitrile in water) to provide 0.12 g (0.44 mmol, 19%) of 4-(1-(((1*H*-1,2,4-triazol-5-yl)methyl)amino)ethyl)isoquinolin-1(2*H*)-one **(VIIIby).** LCMS: *m*/*z* found 270.2 [M+H]⁺

### 1-((1H-1,2,4-Triazol-5-yl)methyl)-3-(3-chloro-4-fluorophenyl)-1-(1-(1-oxo-1,2-dihydroisoquinolin-4-yl)ethyl)urea (Compound 268)

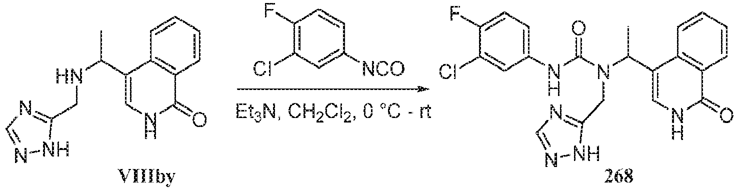

Racemic 1-((1*H*-1,2,4-triazol-5-yl)methyl)-3-(3-chloro-4-fluorophenyl)-1-(1-(1-oxo-1,2-dihydroisoquinolin-4-yl)ethyl)urea was synthesized in a similar manner as described above from 4-(1-(((1*H*-1,2,4-triazol-5-yl)methyl)amino)ethyl)isoquinolin-1(2*H*)-one **(VIII by)** and 2-chloro-1-fluoro-4-isocyanatobenzene. The enantiomers were subsequently separated by SFC, Column: (R,R)Whelk-01 (30 x 250 mm) 5 µ, 80% CO₂:MeOH, flow rate 70 g/min.

1-((1*H*-1,2,4-Triazol-5-yl)methyl)-3-(3-chloro-4-fluorophenyl)-1-(1-(1-oxo-1,2-dihydroisoquinolin-4-yl)ethyl)urea: Enantiomer I. LCMS: *m*/*z* found 441.1/443.1 [M+H]⁺; ¹H NMR (400 MHz, DMSO-d₆) δ 13.60 (bs, 1H), 11.34 (bs, 1H), 9.23 (bs, 1H), 8.12-8.17 (m, 2H), 7.81-7.83 (m, 1H), 7.60-7.64 (m, 1H), 7.53-7.55 (m, 1H), 7.40-7.44 (m, 2H), 7.33 (t, 1H), 7.18 (s, 1H), 5.85-5.88 (m, 1H), 4.43 (d, 1H), 4.27 (d, 1H), 1.42 (d, 3H); Chiral analytical SFC: RT = 5.53 min, Column: (R,R)Whelk-01 (250 x 4.6 mm), 5 µ, 70% CO₂:MeOH, Flow rate = 3.0 mL/min.

1-((1*H*-1,2,4-Triazol-5-yl)methyl)-3-(3-chloro-4-fluorophenyl)-1-(1-(1-oxo-1,2-dihydroisoquinolin-4-yl)ethyl)urea: Enantiomer II **(Compound 268).** LCMS: *m*/*z* found 441.1/443.1 [M+H]⁺; ¹H NMR (400 MHz, DMSO-d₆) δ 13.60 (bs, 1H), 11.34 (bs, 1H), 9.23 (bs, 1H), 8.12-8.17 (m, 2H), 7.81-7.83 (m, 1H), 7.60-7.64 (m, 1H), 7.53-7.55 (m, 1H), 7.40-7.44 (m, 2H), 7.33 (t, 1H), 7.18 (s, 1H), 5.85-5.88 (m, 1H), 4.43 (d, 1H), 4.27 (d, 1H), 1.42 (d, 3H); Chiral analytical SFC: RT = 10.00 min, Column: (R,R)Whelk-01 (250 x 4.6 mm), 5 µ, 70% CO₂:MeOH, Flow rate = 3.0 mL/min.

### 4-(1-((Pyridin-2-ylmethyl)amino)ethyl)isoquinolin-1(2H)-one (VIIIbz)

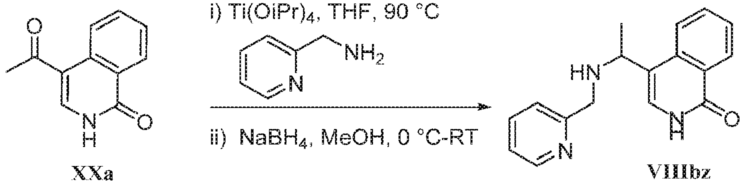

To a solution of 0.2 g (1.1 mmol, 1.0 eq.) of 4-acetylisoquinolin-l(2*H*)-one **(XXa)** in 2 mL of THF under a nitrogen atmosphere was added 0.23 g (2.1 mmol, 2.0 eq.) of pyridin-2-ylmethanamine followed by 2 mL of titanium (IV) isopropoxide and the mixture was heated at 90 °C for 16 h. The mixture was allowed to cool to room temperature and then further cooled to 0 °C. The mixture was diluted with 2 mL of methanol and 0.12 g (3.2 mmol, 3.0 eq.) of sodium borohydride was added. The mixture was then allowed to warm to room temperature and stirred for 2 h. The reaction was diluted with 10 mL of water, filtered through CELITE^{®} and the filtrate was extracted with 2 x 100 mL of 10% methanol in methylene chloride. The combined organic extracts were washed with 50 mL of brine, dried (Na₂SO₄), filtered and the solvent was removed *in vacuo.* The residue was triturated with 10 mL of diethyl ether followed by 10 mL of n-pentane to provide 0.2 g of (0.71 mmol, 66%) of 4-(1-((pyridin-2-ylmethyl)amino)ethyl)isoquinolin-1(2*H*)-one (**VIIIbz**)**.** LCMS: *m*/*z* found 280.2 [M+H]⁺.

### 3-(3-Chloro-4-fluorophenyl)-1-(1-(1-oxo-1,2-dihydroisoquinolin-4-yl)ethyl)-1-(pyridin-2-ylmethyl) urea (Compounds 304 & 305)

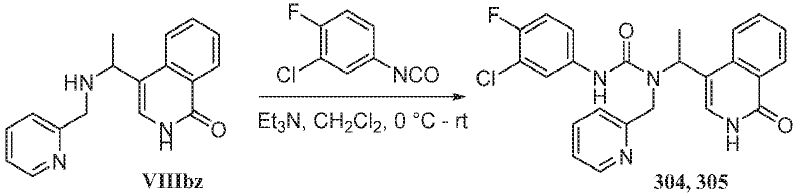

Racemic 3-(3-chloro-4-fluorophenyl)-1-(1-(1-oxo-1,2-dihydroisoquinolin-4-yl)ethyl)-1-(pyridin-2-ylmethyl) urea was synthesized in a similar manner as described above from 4-(1-((pyridin-2-ylmethyl)amino)ethyl)isoquinolin-1(2*H*)-one **(VIIIbz)** and 2-chloro-1-fluoro-4-isocyanatobenzene. The enantiomers were subsequently separated by SFC, Column: Lux Cellulose-2 (30 x 250 mm) 5 µ, 60% CO₂:MeOH, flow rate 70 g/min.

3-(3-Chloro-4-fluorophenyl)-1-(1-(1-oxo-1,2-dihydroisoquinolin-4-yl)ethyl)-1-(pyridin-2-ylmethyl) urea: Enantiomer I **(Compound 304).** LCMS: *m*/*z* found 451.2/453.1 [M+H]⁺, RT = 3.50 min (Method A); ¹H NMR (400 MHz, DMSO-d₆) δ 11.30 (bs, 1H), 9.78 (bs, 1H), 8.25-8.28 (m, 1H), 8.03-8.06 (m, 1H), 7.82-7.85 (m, 1H), 7.54-7.59 (m, 1H), 7.48 (d, 1H), 7.28-7.42 (m, 4H), 7.21 (s, 1H), 6.95-6.99 (m, 1H), 6.65 (d, 1H), 5.85-5.90 (m, 1H), 4.50 (d, 1H), 4.38 (d, 1H), 1.50 (d, 3H); Chiral analytical SFC: RT = 4.90 min, Column: Chiralcel-OZ-3 (250 x 4.6 mm), 3 µ, 70% CO₂:MeOH, Flow rate = 3.0 mL/min.

3-(3-Chloro-4-fluorophenyl)-1-(1-(1-oxo-1,2-dihydroisoquinolin-4-yl)ethyl)-1-(pyridin-2-ylmethyl) urea: Enantiomer II (Compound 305). LCMS: *m*/*z* found 451.2/453.1 [M+H]⁺, RT = 3.50 min (Method A); ¹H NMR (400 MHz, DMSO-d₆) δ 11.30 (bs, 1H), 9.78 (bs, 1H), 8.25-8.28 (m, 1H), 8.03-8.06 (m, 1H), 7.82-7.85 (m, 1H), 7.54-7.59 (m, 1H), 7.48 (d, 1H), 7.28-7.42 (m, 4H), 7.21 (s, 1H), 6.95-6.99 (m, 1H), 6.65 (d, 1H), 5.85-5.90 (m, 1H), 4.50 (d, 1H), 4.38 (d, 1H), 1.50 (d, 3H); Chiral analytical SFC: RT = 9.21 min, Column: Chiralcel-OZ-3 (250 x 4.6 mm), 3 µ, 70% CO₂:MeOH, Flow rate = 3.0 mL/min.

### 4-(1-((Pyridin-3-ylmethyl)amino)ethyl)isoquinolin-1(2H)-one (VIIIca)

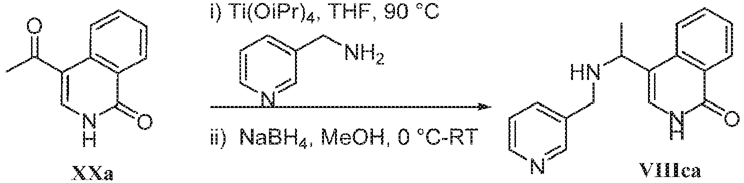

To a solution of 0.3 g (1.6 mmol, 1.0 eq.) of 4-acetylisoquinolin-l(2*H*)-one **(XXa)** in 10 mL of THF under a nitrogen atmosphere was added 0.35 g (3.2 mmol, 2.0 eq.) of pyridin-3-ylmethanamine followed by 3 mL of titanium (IV) isopropoxide and the mixture was heated at 90 °C for 16 h. The mixture was allowed to cool to room temperature and then further cooled to 0 °C. The mixture was diluted with 5 mL of methanol and 0.18 g (4.8 mmol, 3.0 eq.) of sodium borohydride was added. The mixture was then allowed to warm to room temperature and stirred for 2 h. The reaction was diluted with 10 mL of water, filtered through CELITE^{®} and the filtrate was extracted with 2 x 50 mL of 10% methanol in methylene chloride. The combined organic extracts were washed with 50 mL of brine, dried (Na₂SO₄), filtered and the solvent was removed *in vacuo.* The residue was triturated with 10 mL of diethyl ether followed by 10 mL of n-pentane to provide 0.15 g of (0.53 mmol, 34%) of 4-(1-((pyridin-3-ylmethyl)amino)ethyl)isoquinolin-1(2*H*)-one **(VIIIca).** LCMS: *m*/*z* found 280.1 [M+H]⁺.

### 3-(3-Chloro-4-fluorophenyl)-1-(1-(1-oxo-1,2-dihydroisoquinolin-4-yl)ethyl)-1-(pyridin-3-ylmethyl) urea (Compounds 306 & 307)

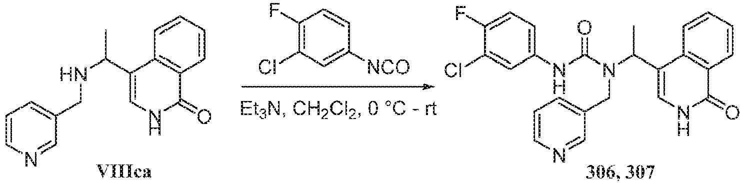

Racemic 3-(3-chloro-4-fluorophenyl)-1-(1-(1-oxo-1,2-dihydroisoquinolin-4-yl)ethyl)-1-(pyridin-3-ylmethyl) urea was synthesized in a similar manner as described above from 4-(1-((pyridin-3-ylmethyl)amino)ethyl)isoquinolin-1(2*H*)-one **(VIIIca)** and 2-chloro-1-fluoro-4-isocyanatobenzene. The enantiomers were subsequently separated by SFC, Column: Chiralpak IG (30 x 250 mm) 5 µ, 70% CO₂:MeOH, flow rate 100 g/min.

3-(3-Chloro-4-fluorophenyl)-1-(1-(1-oxo-1,2-dihydroisoquinolin-4-yl)ethyl)-1-(pyridin-3-ylmethyl) urea: Enantiomer I **(Compound 306).** LCMS: *m*/*z* found 451.1/453.2 [M+H]⁺, RT = 3.22 min (Method A); ¹H NMR (400 MHz, DMSO-d₆) δ 11.29 (bs, 1H), 8.76 (bs, 1H), 8.04-8.10 (m, 3H), 7.82-7.84 (m, 1H), 7.65-7.69 (m, 2H), 7.45-7.50 (m, 1H), 7.39-7.44 (m, 1H), 7.32 (t, 1H), 7.17-7.21 (m, 2H), 6.86-6.90 (m, 1H), 5.92-5.96 (m, 1H), 4.54 (d, 1H), 4.34 (d, 1H), 1.51 (d, 3H); Chiral analytical SFC: RT = 1.39 min, Column: Chiralpak-IG-3 (250 x 4.6 mm), 3 µ, 70% CO₂:MeOH, Flow rate = 3.0 mL/min.

3-(3-Chloro-4-fluorophenyl)-1-(1-(1-oxo-1,2-dihydroisoquinolin-4-yl)ethyl)-1-(pyridin-3-ylmethyl) urea: Enantiomer II **(Compound 307).** LCMS: *m*/*z* found 451.1/453.2 [M+H]⁺, RT = 3.22 min (Method A); ¹H NMR (400 MHz, DMSO-d₆) δ 11.29 (bs, 1H), 8.76 (bs, 1H), 8.04-8.10 (m, 3H), 7.82-7.84 (m, 1H), 7.65-7.69 (m, 2H), 7.45-7.50 (m, 1H), 7.39-7.44 (m, 1H), 7.32 (t, 1H), 7.17-7.21 (m, 2H), 6.86-6.90 (m, 1H), 5.92-5.96 (m, 1H), 4.54 (d, 1H), 4.34 (d, 1H), 1.51 (d, 3H); Chiral analytical SFC: RT = 3.91 min, Column: Chiralpak-IG-3 (250 x 4.6 mm), 3 µ, 70% CO₂:MeOH, Flow rate = 3.0 mL/min.

### 4-(1-((Pyridin-4-ylmethyl)amino)ethyl)isoquinolin-1(2H)-one (VIIIcb)

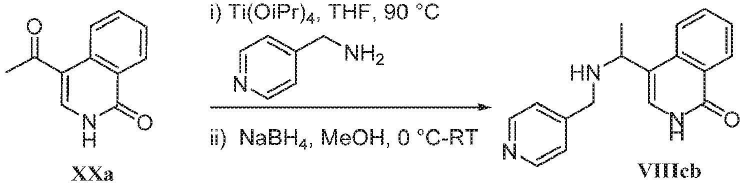

To a solution of 0.6 g (3.2 mmol, 1.0 eq.) of 4-acetylisoquinolin-l(2*H*)-one **(XXa)** in 10 mL of THF under a nitrogen atmosphere was added 0.7 g (6.4 mmol, 2.0 eq.) of pyridin-4-ylmethanamine followed by 6 mL of titanium (IV) isopropoxide and the mixture was heated at 90 °C for 16 h. The mixture was allowed to cool to room temperature and then further cooled to 0 °C. The mixture was diluted with 5 mL of methanol and 0.36 g (9.6 mmol, 3.0 eq.) of sodium borohydride was added. The mixture was then allowed to warm to room temperature and stirred for 2 h. The reaction was diluted with 10 mL of water, filtered through CELITE^{®} and the filtrate was extracted with 2 x 60 mL of 10% methanol in methylene chloride. The combined organic extracts were washed with 60 mL of brine, dried (Na₂SO₄), filtered and the solvent was removed *in vacuo.* The residue was purified by chromatography (SiO₂, eluting with a linear gradient of 0-6% methanol/methylene chloride) to provide 0.2 g of (0.72 mmol, 34%) of 4-(1-((pyridin-4-ylmethyl)amino)ethyl) isoquinolin-1(2*H*)-one **(VIIIcb).** LCMS: *m*/*z* found 280.1 [M+H]⁺.

### 3-(3-Chloro-4-fluorophenyl)-1-(1-(1-oxo-1,2-dihydroisoquinolin-4-yl)ethyl)-1-(pyridin-4-ylmethyl) urea (Compounds 308 & 309)

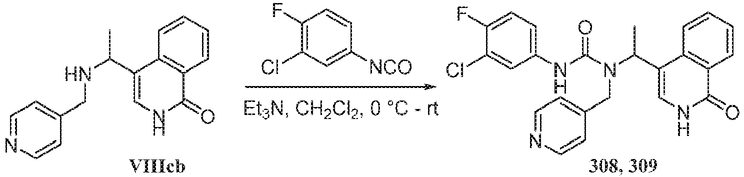

Racemic 3-(3-chloro-4-fluorophenyl)-1-(1-(1-oxo-1,2-dihydroisoquinolin-4-yl)ethyl)-1-(pyridin-4-ylmethyl) urea was synthesized in a similar manner as described above from 4-(1-((pyridin-4-ylmethyl)amino)ethyl)isoquinolin-1(2*H*)-one **(VIIIcb)** and 2-chloro-1-fluoro-4-isocyanatobenzene. The enantiomers were subsequently separated by SFC, Column: Chiralpak IG (30 x 250 mm) 5 µ, 70% CO₂:MeOH, flow rate 90 g/min.

3-(3-Chloro-4-fluorophenyl)-1-(1-(1-oxo-1,2-dihydroisoquinolin-4-yl)ethyl)-1-(pyridin-4-ylmethyl) urea: Enantiomer I **(Compound 308).** LCMS: *m*/*z* found 451.1/453.2 [M+H]⁺, RT = 3.22 min (Method A); ¹H NMR (400 MHz, DMSO-d₆) δ 11.27 (bs, 1H), 8.70 (bs, 1H), 8.06-8.11 (m, 3H), 7.79-7.82 (m, 1H), 7.70-7.73 (m, 2H), 7.42-7.47 (m, 2H), 7.31 (t, 1H), 7.14 (d, 1H), 6.84 (d, 2H), 5.96-6.00 (m, 1H), 4.51 (d, 1H), 4.35 (d, 1H), 1.51 (d, 3H); Chiral analytical SFC: RT = 2.11 min, Column: Chiralpak-IG-3 (250 x 4.6 mm), 3 µ, 70% CO₂:MeOH, Flow rate = 3.0 mL/min.

3-(3-Chloro-4-fluorophenyl)-1-(1-(1-oxo-1,2-dihydroisoquinolin-4-yl)ethyl)-1-(pyridin-4-ylmethyl) urea: Enantiomer II **(Compound 309).** LCMS: *m*/*z* found 451.1/453.2 [M+H]⁺, RT = 3.22 min (Method A); ¹H NMR (400 MHz, DMSO-d₆) δ 11.27 (bs, 1H), 8.70 (bs, 1H), 8.06-8.11 (m, 3H), 7.79-7.82 (m, 1H), 7.70-7.73 (m, 2H), 7.42-7.47 (m, 2H), 7.31 (t, 1H), 7.14 (d, 1H), 6.84 (d, 2H), 5.96-6.00 (m, 1H), 4.51 (d, 1H), 4.35 (d, 1H), 1.51 (d, 3H); Chiral analytical SFC: RT = 4.94 min, Column: Chiralpak-IG-3 (250 x 4.6 mm), 3 µ, 70% CO₂:MeOH, Flow rate = 3.0 mL/min.

### 4-(1-((Pyrimidin-5-ylmethyl)amino)ethyl)isoquinolin-1(2H)-one (VIIIcc)

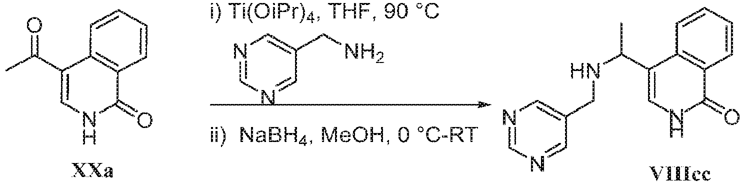

To a solution of 0.3 g (1.6 mmol, 1.0 eq.) of 4-acetylisoquinolin-l(2*H*)-one **(XXa)** in 10 mL of THF under a nitrogen atmosphere was added 0.35 g (3.2 mmol, 2.0 eq.) of pyrimidin-5-ylmethanamine followed by 3 mL of titanium (IV) isopropoxide and the mixture was heated at 90 °C for 16 h. The mixture was allowed to cool to room temperature and then further cooled to 0 °C. The mixture was diluted with 5 mL of methanol and 0.18 g (4.8 mmol, 3.0 eq.) of sodium borohydride was added. The mixture was then allowed to warm to room temperature and stirred for 2 h. The reaction was diluted with 10 mL of water, filtered through CELITE^{®} and the filtrate was extracted with 2 x 50 mL of 10% methanol in methylene chloride. The combined organic extracts were washed with 50 mL of brine, dried (Na₂SO₄), filtered and the solvent was removed *in vacuo.* The residue was purified by chromatography (SiO₂, eluting with a linear gradient of 0-6% methanol/methylene chloride) to provide 0.06 g of (0.21 mmol, 13%) of 4-(1-((pyrimidin-5-ylmethyl)amino)ethyl)isoquinolin-1(2*H*)-one **(VIIIcc).** LCMS: *m*/*z* found 281.1 [M+H]⁺.

### 3-(3-Chloro-4-fluorophenyl)-1-(1-(1-oxo-1,2-dihydroisoquinolin-4-yl)ethyl)-1-(pyrimidin-5-ylmethyl)urea (Compound 320)

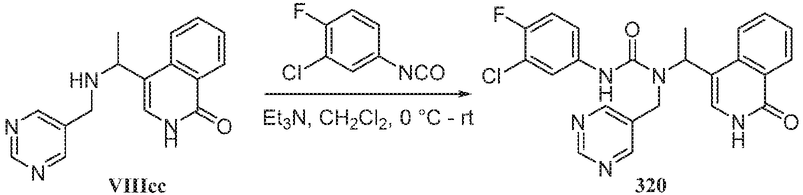

Racemic 3-(3-chloro-4-fluorophenyl)-1-(1-(1-oxo-1,2-dihydroisoquinolin-4-yl)ethyl)-1-(pyrimidin-5-ylmethyl)urea was synthesized in a similar manner as described above 4-(1-((pyrimidin-5-ylmethyl)amino)ethyl)isoquinolin-1(2*H*)-one **(VIIIcc)** and 2-chloro-1-fluoro-4-isocyanatobenzene. The enantiomers were subsequently separated by SFC, Column: Chiralpak IG (30 x 250 mm) 5 µ, 60% CO₂:MeOH, flow rate 70 g/min.

3-(3-Chloro-4-fluorophenyl)-1-(1-(1-oxo-1,2-dihydroisoquinolin-4-yl)ethyl)-1-(pyrimidin-5-ylmethyl)urea: Enantiomer I **(Compound 320).** LCMS: *m*/*z* found 452.2/454.1 [M+H]⁺, RT = 3.66 min (Method A); ¹H NMR (400 MHz, DMSO-d₆) δ 11.29 (bs, 1H), 8.89 (bs, 1H), 8.63 (s, 1H), 8.18 (s, 2H), 8.03 (d, 1H), 7.82-7.86 (m, 1H), 7.66-7.70 (m, 1H), 7.58-7.62 (m, 1H), 7.48-7.53 (m, 1H), 7.36-7.42 (m, 1H), 7.32 (t, 1H), 7.22 (s, 1H), 5.87-5.90 (m, 1H), 4.62 (d, 1H), 4.28 (d, 1H), 1.56 (d, 3H); Chiral analytical SFC: RT = 1.16 min, Column: Chiralpak-IG-3 (250 x 4.6 mm), 3 µ, 60% CO₂:MeOH, Flow rate = 3.0 mL/min.

3-(3-Chloro-4-fluorophenyl)-1-(1-(1-oxo-1,2-dihydroisoquinolin-4-yl)ethyl)-1-(pyrimidin-5-ylmethyl)urea: Enantiomer II. LCMS: *m*/*z* found 452.2/454.1 [M+H]⁺, RT = 3.67 min (Method A); ¹H NMR (400 MHz, DMSO-d₆) δ 11.29 (bs, 1H), 8.89 (bs, 1H), 8.63 (s, 1H), 8.18 (s, 2H), 8.03 (d, 1H), 7.82-7.86 (m, 1H), 7.66-7.70 (m, 1H), 7.58-7.62 (m, 1H), 7.48-7.53 (m, 1H), 7.36-7.42 (m, 1H), 7.32 (t, 1H), 7.22 (s, 1H), 5.87-5.90 (m, 1H), 4.62 (d, 1H), 4.28 (d, 1H), 1.56 (d, 3H); Chiral analytical SFC: RT = 2.60 min, Column: Chiralpak-IG-3 (250 x 4.6 mm), 3 µ, 60% CO₂:MeOH, Flow rate = 3.0 mL/min.

### 4-(1-((Pyrimidin-4-ylmethyl)amino)ethyl)isoquinolin-1(2H)-one (VIIIcd)

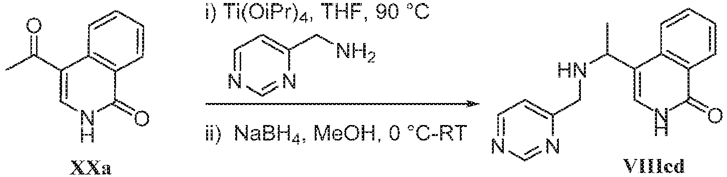

To a solution of 0.3 g (1.6 mmol, 1.0 eq.) of 4-acetylisoquinolin-l(2*H*)-one **(XXa)** in 10 mL of THF under a nitrogen atmosphere was added 0.31 g (2.7 mmol, 1.7 eq.) of pyrimidin-4-ylmethanamine followed by 3 mL of titanium (IV) isopropoxide and the mixture was heated at 90 °C for 16 h. The mixture was allowed to cool to room temperature and then further cooled to 0 °C. The mixture was diluted with 3 mL of methanol and 0.12 g (4.8 mmol, 2.0 eq.) of sodium borohydride was added. The mixture was then allowed to warm to room temperature and stirred for 2 h. The reaction was diluted with 10 mL of water, filtered through CELITE^{®} and the filtrate was extracted with 2 x 50 mL of 10% methanol in methylene chloride. The combined organic extracts were washed with 50 mL of brine, dried (Na₂SO₄), filtered and the solvent was removed *in vacuo.* The residue was purified by chromatography (SiO₂, eluting with a linear gradient of 0-6% methanol/methylene chloride) to provide 0.2 g of (0.71 mmol, 44%) of 4-(1-((pyrimidin-4-ylmethyl)amino) ethyl)isoquinolin-1(2*H*)-one **(VIIIcd).** LCMS: *m*/*z* found 281.1 [M+H]⁺.

### 3-(3-Chloro-4-fluorophenyl)-1-(1-(1-oxo-1,2-dihydroisoquinolin-4-yl)ethyl)-1-(pyrimidin-4-ylmethyl)urea (Compounds 321 & 322)

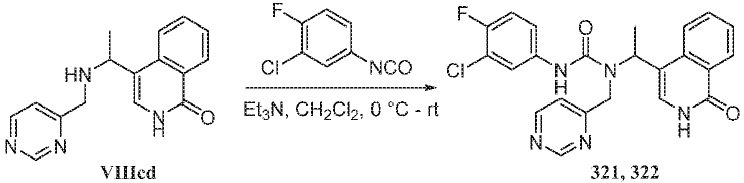

Racemic 3-(3-chloro-4-fluorophenyl)-1-(1-(1-oxo-1,2-dihydroisoquinolin-4-yl)ethyl)-1-(pyrimidin-4-ylmethyl)urea was synthesized in a similar manner as described above 4-(1-((pyrimidin-4-ylmethyl)amino)ethyl)isoquinolin-1(2*H*)-one **(VIIIcd)** and 2-chloro-1-fluoro-4-isocyanatobenzene. The enantiomers were subsequently separated by SFC, Column: Chiralpak IC (30 x 250 mm) 5 µ, 60% CO₂:MeOH, flow rate 100 g/min.

3-(3-Chloro-4-fluorophenyl)-1-(1-(1-oxo-1,2-dihydroisoquinolin-4-yl)ethyl)-1-(pyrimidin-4-ylmethyl)urea: Enantiomer I **(Compound 321).** LCMS: *m*/*z* found 452.2/454.2 [M+H]⁺, RT = 4.01 min (Method A); ¹H NMR (400 MHz, DMSO-d₆) δ 11.29 (bs, 1H), 8.94 (bs, 1H), 8.76 (d, 1H), 8.24 (d, 1H), 8.05 (d, 1H), 7.80-7.83 (m, 1H), 7.62-7.70 (m, 2H), 7.40-7.48 (m, 2H), 7.32 (t, 1H), 7.17 (s, 1H), 6.86 (d, 1H), 5.93-5.97 (m, 1H), 4.59 (d, 1H), 4.38 (d, 1H), 1.51 (d, 3H); Chiral analytical SFC: RT = 4.21 min, Column: Chiralpak-IC-3 (150 x 4.6 mm), 3 µ, 60% CO₂:MeOH, Flow rate = 3.0 mL/min.

3-(3-Chloro-4-fluorophenyl)-1-(1-(1-oxo-1,2-dihydroisoquinolin-4-yl)ethyl)-1-(pyrimidin-4-ylmethyl)urea: Enantiomer II **(Compound 322).** LCMS: *m*/*z* found 452.2/454.2 [M+H]⁺, RT = 4.01 min (Method A); ¹H NMR (400 MHz, DMSO-d₆) δ 11.29 (bs, 1H), 8.94 (bs, 1H), 8.76 (d, 1H), 8.24 (d, 1H), 8.05 (d, 1H), 7.80-7.83 (m, 1H), 7.62-7.70 (m, 2H), 7.40-7.48 (m, 2H), 7.32 (t, 1H), 7.17 (s, 1H), 6.86 (d, 1H), 5.93-5.97 (m, 1H), 4.59 (d, 1H), 4.38 (d, 1H), 1.51 (d, 3H); Chiral analytical SFC: RT = 6.78 min, Column: Chiralpak-IC-3 (150 x 4.6 mm), 3 µ, 60% CO₂:MeOH, Flow rate = 3.0 mL/min.

### 4-(1-((Thiazol-2-ylmethyl)amino)ethyl)isoquinolin-1(2H)-one (VIIIce)

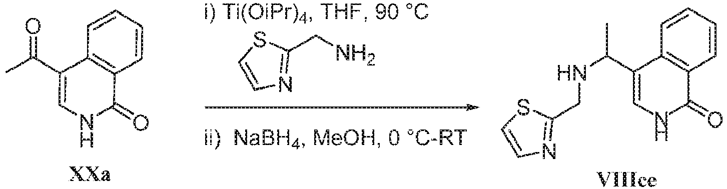

To a solution of 0.3 g (1.6 mmol, 1.0 eq.) of 4-acetylisoquinolin-l(2*H*)-one **(XXa)** in 10 mL of THF under a nitrogen atmosphere was added 0.3 g (2.4 mmol, 2.0 eq.) of thiazol-2-ylmethanamine followed by 3 mL of titanium (IV) isopropoxide and the mixture was heated at 90 °C for 16 h. The mixture was allowed to cool to room temperature and then further cooled to 0 °C. The mixture was diluted with 3 mL of methanol and 0.12 g (4.8 mmol, 3.0 eq.) of sodium borohydride was added. The mixture was then allowed to warm to room temperature and stirred for 2 h. The reaction was diluted with 10 mL of water, filtered through CELITE^{®} and the filtrate was extracted with 3 x 50 mL of 10% methanol in methylene chloride. The combined organic extracts were washed with 100 mL of brine, dried (Na₂SO₄), filtered and the solvent was removed *in vacuo.* The residue was triturated with 10 mL of diethyl ether followed by 10 mL of n-pentane to provide 0.3 g of 4-(1-((thiazol-2-ylmethyl)amino)ethyl)isoquinolin-1(2*H*)-one **(VIIIce).** LCMS: *m*/*z* found 286.0 [M+H]⁺.

### 3-(3-Chloro-4-fluorophenyl)-1-(1-(1-oxo-1,2-dihydroisoquinolin-4-yl)ethyl)-1-(thiazol-2-ylmethyl)urea (Compounds 310 & 311)

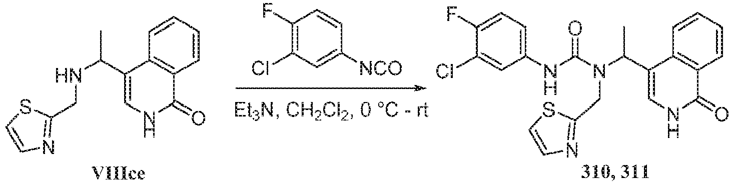

Racemic 3-(3-chloro-4-fluorophenyl)-1-(1-(1-oxo-1,2-dihydroisoquinolin-4-yl)ethyl)-1-(thiazol-2-ylmethyl)urea was synthesized in a similar manner as described above 4-(1-((thiazol-2-ylmethyl)amino)ethyl)isoquinolin-1(2*B*)-one **(VIIIce)** and 2-chloro-1-fluoro-4-isocyanatobenzene. The enantiomers were subsequently separated by SFC, Column: Chiralcel OD-H (30 x 250 mm) 5 µ, 75% CO₂:MeOH, flow rate 100 g/min.

3-(3-Chloro-4-fluorophenyl)-1-(1-(1-oxo-1,2-dihydroisoquinolin-4-yl)ethyl)-1-(thiazol-2-ylmethyl)urea: Enantiomer I **(Compound 310).** LCMS: *m*/*z* found 457.1/459.1 [M+H]⁺, RT = 4.50 min (Method A); ¹H NMR (400 MHz, DMSO-d₆) δ 11.36 (bs, 1H), 9.11 (bs, 1H), 8.12-8.15 (m, 1H), 7.80-7.83 (m, 1H), 7.59-7.66 (m, 2H), 7.40-7.48 (m, 3H), 7.32-7.37 (m, 2H), 7.26 (s, 1H), 5.88-5.92 (m, 1H), 4.64-4.74 (m, 2H), 1.50 (d, 3H); Chiral analytical SFC: RT = 2.92 min, Column: Chiralcel-OD-3 (250 x 4.6 mm), 3 µ, 70% CO₂:MeOH, Flow rate = 3.0 mL/min.

3-(3-Chloro-4-fluorophenyl)-1-(1-(1-oxo-1,2-dihydroisoquinolin-4-yl)ethyl)-1-(thiazol-2-ylmethyl)urea: Enantiomer II **(Compound 311).** LCMS: *m*/*z* found 457.1/459.1 [M+H]⁺, RT = 4.50 min (Method A); ¹H NMR (400 MHz, DMSO-d₆) δ 11.36 (bs, 1H), 9.11 (bs, 1H), 8.12-8.15 (m, 1H), 7.80-7.83 (m, 1H), 7.59-7.66 (m, 2H), 7.40-7.48 (m, 3H), 7.32-7.37 (m, 2H), 7.26 (s, 1H), 5.88-5.92 (m, 1H), 4.64-4.74 (m, 2H), 1.50 (d, 3H); Chiral analytical SFC: RT = 3.66 min, Column: Chiralcel-OD-3 (250 x 4.6 mm), 3 µ, 70% CO₂:MeOH, Flow rate = 3.0 mL/min.

### 4-(1-((Thiazol-4-ylmethyl)amino)ethyl)isoquinolin-1(2H)-one (VIIIcf)

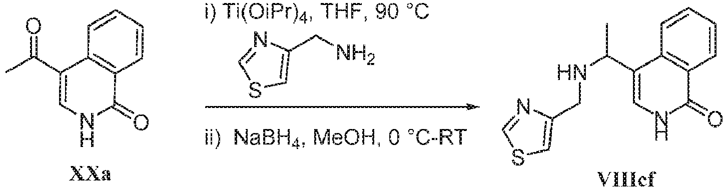

To a solution of 0.3 g (1.6 mmol, 1.0 eq.) of 4-acetylisoquinolin-l(2*H*)-one **(XXa)** in 10 mL of THF under a nitrogen atmosphere was added 0.3 g (2.4 mmol, 2.0 eq.) of thiazol-4-ylmethanamine followed by 3 mL of titanium (IV) isopropoxide and the mixture was heated at 90 °C for 16 h. The mixture was allowed to cool to room temperature and then further cooled to 0 °C. The mixture was diluted with 3 mL of methanol and 0.12 g (4.8 mmol, 3.0 eq.) of sodium borohydride was added. The mixture was then allowed to warm to room temperature and stirred for 2 h. The reaction was diluted with 10 mL of water, filtered through CELITE^{®} and the filtrate was extracted with 3 x 50 mL of 10% methanol in methylene chloride. The combined organic extracts were washed with 100 mL of brine, dried (Na₂SO₄), filtered and the solvent was removed *in vacuo.* The residue was triturated with 10 mL of diethyl ether followed by 10 mL of n-pentane to provide 0.3 g of 4-(1-((thiazol-4-ylmethyl)amino)ethyl)isoquinolin-1(2*H*)-one **(VIIIcf).** LCMS: *m*/*z* found 286.0 [M+H]⁺.

### 3-(3-Chloro-4-fluorophenyl)-1-(1-(1-oxo-1,2-dihydroisoquinolin-4-yl)ethyl)-1-(thiazol-4-ylmethyl)urea (Compounds 312 & 313)

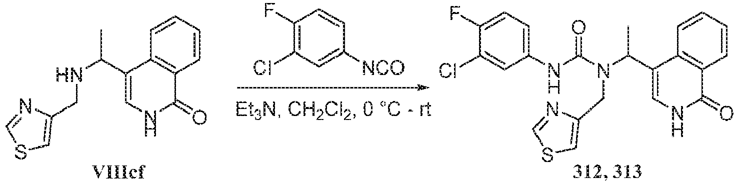

Racemic 3-(3-chloro-4-fluorophenyl)-1-(1-(1-oxo-1,2-dihydroisoquinolin-4-yl)ethyl)-1-(thiazol-4-ylmethyl)urea was synthesized in a similar manner as described above 4-(1-((thiazol-4-ylmethyl)amino)ethyl)lsoquinolin-1(2*H*)-one **(VIIIcf)** and 2-chloro-1-fluoro-4-isocyanatobenzene. The enantiomers were subsequently separated by SFC, Column: Chiralcel OD-H (30 x 250 mm) 5 µ, 70% CO₂:MeOH, flow rate 60 g/min.

3-(3-Chloro-4-fluorophenyl)-1-(1-(1-oxo-1,2-dihydroisoquinolin-4-yl)ethyl)-1-(thiazol-4-ylmethyl)urea: Enantiomer I **(Compound 312).** LCMS: *m*/*z* found 457.1/459.1 [M+H]⁺, RT = 4.56 min (Method A); ¹H NMR (400 MHz, DMSO-d₆) δ 11.36 (bs, 1H), 9.17 (bs, 1H), 8.81 (d, 1H), 8.10-8.13 (m, 1H), 7.82-7.85 (m, 1H), 7.59-7.63 (m, 1H), 7.52 (d, 1H), 7.38-7.45 (m, 2H), 7.32 (t, 1H), 7.21 (s, 1H), 6.82 (d, 1H), 5.85-5.90 (m, 1H), 4.43-4.53 (m, 2H), 1.47 (d, 3H); Chiral analytical SFC: RT = 1.72 min, Column: Chiralcel-OD-3 (250 x 4.6 mm), 3 µ, 70% CO₂:MeOH, Flow rate = 3.0 mL/min.

3-(3-Chloro-4-fluorophenyl)-1-(1-(1-oxo-1,2-dihydroisoquinolin-4-yl)ethyl)-1-(thiazol-4-ylmethyl)urea: Enantiomer II **(Compound 313).** LCMS: *m*/*z* found 457.1/459.1 [M+H]⁺, RT = 4.56 min (Method A); ¹H NMR (400 MHz, DMSO-d₆) δ 11.36 (bs, 1H), 9.17 (bs, 1H), 8.81 (d, 1H), 8.10-8.13 (m, 1H), 7.82-7.85 (m, 1H), 7.59-7.63 (m, 1H), 7.52 (d, 1H), 7.38-7.45 (m, 2H), 7.32 (t, 1H), 7.21 (s, 1H), 6.82 (d, 1H), 5.85-5.90 (m, 1H), 4.43-4.53 (m, 2H), 1.47 (d, 3H); Chiral analytical SFC: RT = 2.61 min, Column: Chiralcel-OD-3 (250 x 4.6 mm), 3 µ, 70% CO₂:MeOH, Flow rate = 3.0 mL/min.

### 4-(1-((Thiazol-5-ylmethyl)amino)ethyl)isoquinolin-1(2H)-one (VIIIcg)

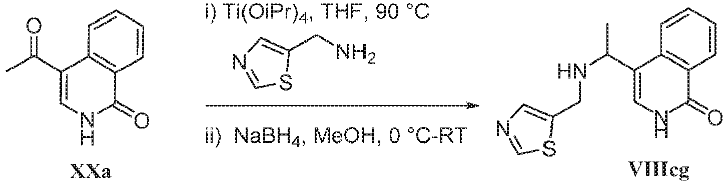

To a solution of 0.16 g (0.9 mmol, 1.0 eq.) of 4-acetylisoquinolin-1(2*B*)-one **(XXa)** in 1.6 mL of THF under a nitrogen atmosphere was added 0.2 g (1.7 mmol, 2.0 eq.) of thiazol-5-ylmethanamine followed by 1.6 mL of titanium (IV) isopropoxide and the mixture was heated at 90 °C for 16 h. The mixture was allowed to cool to room temperature and then further cooled to 0 °C. The mixture was diluted with 1.6 mL of methanol and 0.07 g (1.7 mmol, 2.0 eq.) of sodium borohydride was added. The mixture was then allowed to warm to room temperature and stirred for 2 h. The reaction was diluted with 10 mL of water, filtered through CELITE^{®} and the filtrate was extracted with 3 x 50 mL of 10% methanol in methylene chloride. The combined organic extracts were washed with 100 mL of brine, dried (Na₂SO₄), filtered and the solvent was removed *in vacuo* to provide 0.25 g of crude 4-(1-((thiazol-5-ylmethyl)amino)ethyl)isoquinolin-1(2*H*)-one **(VIIIcg).** LCMS: *m*/*z* found 286.0 [M+H]⁺.

### 3-(3-Chloro-4-fluorophenyl)-1-(1-(1-oxo-1,2-dihydroisoquinolin-4-yl)ethyl)-1-(thiazol-5-ylmethyl)urea (Compounds 323 & 324)

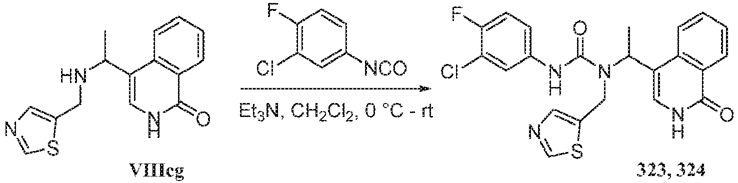

Racemic 3-(3-chloro-4-fluorophenyl)-1-(1-(1-oxo-1,2-dihydroisoquinolin-4-yl)ethyl)-1-(thiazol-5-ylmethyl)urea was synthesized in a similar manner as described above 4-(1-((thiazol-5-ylmethyl)amino)ethyl)isoquinolin-1(2*B*)-one **(VIIIcg)** and 2-chloro-1-fluoro-4-isocyanatobenzene. The enantiomers were subsequently separated by SFC, Column: Chiralpak IG (30 x 250 mm) 5 µ, 80% CO₂:MeOH, flow rate 100 g/min.

3-(3-Chloro-4-fluorophenyl)-1-(1-(1-oxo-1,2-dihydroisoquinolin-4-yl)ethyl)-1-(thiazol-5-ylmethyl)urea: Enantiomer I **(Compound 323).** LCMS: *m*/*z* found 457.1/459.1 [M+H]⁺, RT = 3.90 min (Method A); ¹H NMR (400 MHz, DMSO-d₆) δ 11.40 (bs, 1H), 8.82 (bs, 1H), 8.61 (s, 1H), 8.15 (d, 1H), 7.81-7.83 (m, 1H), 7.59-7.66 (m, 2H), 7.46-7.51 (m, 1H), 7.40-7.45 (m, 1H), 7.35 (t, 1H), 7.31 (s, 1H), 7.28 (s, 1H), 5.82-5.86 (m, 1H), 4.54-4.65 (m, 2H), 1.50 (d, 3H); Chiral analytical SFC: RT = 1.50 min, Column: Chiralpak-IG-3 (150 x 4.6 mm), 3 µ, 60% CO₂:MeOH, Flow rate = 3.0 mL/min.

3-(3-Chloro-4-fluorophenyl)-1-(1-(1-oxo-1,2-dihydroisoquinolin-4-yl)ethyl)-1-(thiazol-5-ylmethyl)urea: Enantiomer II **(Compound 324).** LCMS: *m*/*z* found 457.1/459.1 [M+H]⁺, RT = 3.90 min (Method A); ¹H NMR (400 MHz, DMSO-d₆) δ 11.40 (bs, 1H), 8.82 (bs, 1H), 8.61 (s, 1H), 8.15 (d, 1H), 7.81-7.83 (m, 1H), 7.59-7.66 (m, 2H), 7.46-7.51 (m, 1H), 7.40-7.45 (m, 1H), 7.35 (t, 1H), 7.31 (s, 1H), 7.28 (s, 1H), 5.82-5.86 (m, 1H), 4.54-4.65 (m, 2H), 1.50 (d, 3H); Chiral analytical SFC: RT = 2.46 min, Column: Chiralpak-IG-3 (150 x 4.6 mm), 3 µ, 60% CO₂:MeOH, Flow rate = 3.0 mL/min.

### 3-(1-(1-Methoxyisoquinolin-4-yl)ethylamino)propanenitrile (VIac)

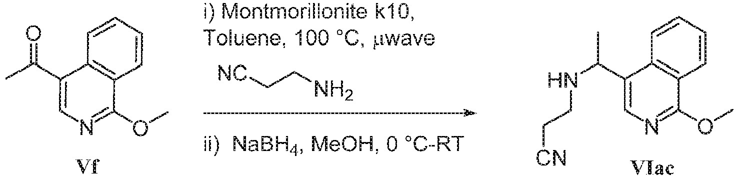

To a solution of 0.5 g (2.5 mmol, 1.0 eq.) of 1-(1-methoxyisoquinolin-4-yl)ethanone **(Vf)** in 5 mL of toluene under a nitrogen atmosphere was added 0.19 g (2.7 mmol 1.1 eq.) of 3-aminopropanenitrile followed by 0.5 g of montmorillonite-K10 and the mixture was subjected to microwave irradiation maintaining a reaction temperature of 100 °C for 36 h. The mixture was allowed to cool to room temperature and then further cooled to 0 °C. The mixture was diluted with 5 mL of methanol and 0.18 g (5.3 mmol, 2.0 eq.) of sodium borohydride was added. The mixture was then allowed to warm to room temperature and stirred for 2 h. The mixture was filtered through CELITE^{®} and the pad was washed with 5 mL of ethyl acetate. The filtrate was concentrated *in vacuo* and the residue was purified by chromatography (REVELERIS^{®} SiO₂ column, eluting with a linear gradient of 20-60% ethyl acetate/petroleum ether) to provide 0.2 g (0.78 mmol, 31%) of 3-(1-(1-methoxyisoquinolin-4-yl)ethylamino)propanenitrile **(VIac).** LCMS: *m*/*z* found 256.5 [M+H]⁺.

### 3-(1-(1-Hydroxyisoquinolin-4-yl)ethylamino)propanenitrile (VIIIch)

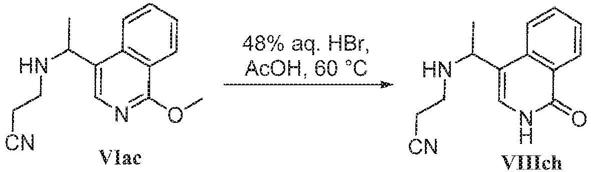

To a solution of 0.2 g (0.78 mmol, 1.0 eq.) of 3-(1-(1-methoxyisoquinolin-4-yl)ethylamino)propanenitrile **(VIac)** in 1 mL of acetic acid at 0 °C was added 5 mL of 48% aqueous HBr. The mixture was allowed to warm to room temperature and stirred for 72 h. The reaction was quenched by the slow addition of 20 mL of saturated sodium bicarbonate solution and extracted with 3 x 75 mL of ethyl acetate. The combined organic extracts were washed with 50 mL of brine, dried (Na₂SO₄), filtered and the solvent was removed *in vacuo.* The residue was purified by reverse phase chromatography (Reveleris^{®} C18 column-40 g; eluted with linear gradient of 10-50% water/methanol) to provide 0.13 g (0.53 mmol, 68%) of 3-(1-(1-hydroxyisoquinolin-4-yl)ethylamino)propanenitrile **(VIIIch).** LCMS: *m*/*z* found 242.4 [M+H]⁺.

### 3-(3-Chloro-4-fluorophenyl)-1-(2-cyanoethyl)-1-(1-(1-oxo-1,2-dihydroisoquinolin-4-yl)ethyl)urea (Compounds 103, 112 & 113)

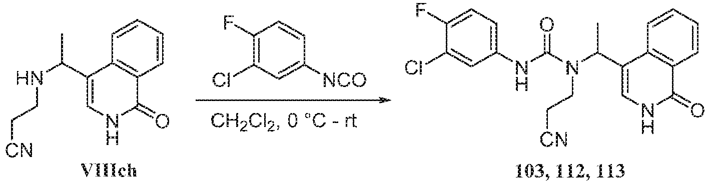

Racemic 3-(3-chloro-4-fluorophenyl)-1-(2-cyanoethyl)-1-(1-(1-oxo-1,2-dihydroisoquinolin-4-yl)ethyl)urea **(Compound 103)** was synthesized in a similar manner as described above from 3-(1-(1-hydroxyisoquinolin-4-yl)ethylamino)propanenitrile **(VIIIch)** and 2-chloro-1-fluoro-4-isocyanatobenzene. The enantiomers were subsequently separated by SFC, Column: Chiralpak IA (30 x 250 mm) 5 µ, 70% CO₂:MeOH, flow rate 90 g/min.

3-(3-Chloro-4-fluorophenyl)-1-(2-cyanoethyl)-1-(1-(1-oxo-1,2-dihydroisoquinolin-4-yl)ethyl)urea: Enantiomer I **(Compound 112).** LCMS: *m*/*z* found 413.20/415.2 [M+H]⁺, 6.46 min (Method A); ¹H NMR (400 MHz, DMSO-d₆): 11.45 (s, 1H), 8.71 (s, 1H), 8.26 (d, 1H), 7.82 (dd, 1H), 7.76 (t, 1H), 7.64 (d, 1H), 7.53 (t, 2H), 7.37 (t, 1H), 7.30 (s, 1H), 5.81 (q, 1H), 3.41 (t, 2H), 2.50 (m, 1H), 2.11 (m, 1H), 1.51 (d, 3H); Chiral analytical SFC: RT = 0.89 min, Chiralcel OD-3 (250 x 4.6 mm), 5 µ, 60% CO₂:MeOH, Flow rate = 3.0 mL/min.

3-(3-Chloro-4-fluorophenyl)-1-(2-cyanoethyl)-1-(1-(1-oxo-1,2-dihydroisoquinolin-4-yl)ethyl)urea: Enantiomer II **(Compound 113).** LCMS: *m*/*z* found 413.2/415.2 [M+H]⁺, 6.46 min (Method A); ¹H NMR (400 MHz, DMSO-d₆): 11.45 (s, 1H), 8.71 (s, 1H), 8.26 (d, 1H), 7.82 (dd, 1H), 7.76 (t, 1H), 7.64 (d, 1H), 7.53 (t, 2H), 7.37 (t, 1H), 7.30 (s, 1H), 5.81 (q, 1H), 3.41 (t, 2H), 2.50 (m, 1H), 2.11 (m, 1H), 1.51 (d, 3H); Chiral analytical SFC: RT = 1.93 min, Chiralcel OD-3 (250 x 4.6 mm), 5 µ, 60% CO₂:MeOH, Flow rate = 3.0 mL/min.

### 1-(1-Methoxyisoquinolin-4-yl)-N-(2-(methylsulfonyl)ethyl)ethanamine (VIad)

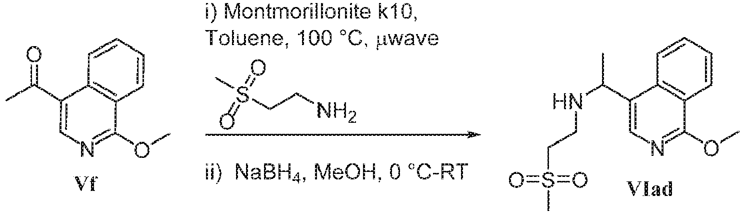

To a solution of 0.5 g (2.5 mmol, 1.0 eq.) of 1-(1-methoxyisoquinolin-4-yl)ethanone **(Vf)** in 5 mL of toluene under a nitrogen atmosphere was added 0.19 g (2.7 mmol 1.1 eq.) of 2-(methylsulfonyl)ethanamine followed by 0.5 g of montmorillonite-K10 and the mixture was subjected to microwave irradiation, maintaining a reaction temperature of 100 °C for 36 h. The mixture was allowed to cool to room temperature and then further cooled to 0 °C. The mixture was diluted with 5 mL of methanol and 0.18 g (5.3 mmol, 2.0 eq.) of sodium borohydride was added. The mixture was then allowed to warm to room temperature and stirred for 2 h. The mixture was filtered through CELITE^{®} and the pad was washed with 5 mL of ethyl acetate. The filtrate was concentrated *in vacuo* and the residue was purified by chromatography (REVELERIS^{®} SiO₂ column, eluting with a linear gradient of 20-60% ethyl acetate/petroleum ether) to provide 0.2 g (0.64 mmol, 26%) of 1-(1-methoxyisoquinolin-4-yl)-*N*-(2-(methylsulfonyl)ethyl)ethanamine **(VIad).** ¹H NMR (400 MHz, CDCl₃): δ 8.32 (d, 1H), 8.29 (d, 1H), 8.05 (s, 1H), 7.71 (t, 1H), 7.57 (t, 1H), 4.41 (q, 1H), 4.16 (s, 3H), 3.18 (m, 4H), 2.94 (s, 3H), 1.54 (d, 3H).

### 4-(1-((2-(Methylsulfonyl)ethyl)amino)ethyl)isoquinolin-1(2H)-one (VIIIcj)

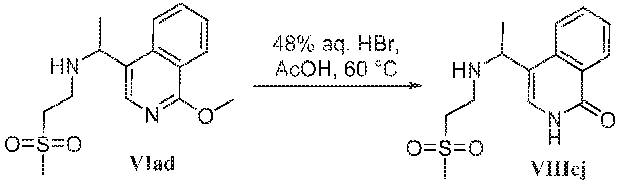

To a solution of 0.2 g (0.65 mmol, 1.0 eq.) of 1-(1-methoxyisoquinolin-4-yl)-*N*-(2-(methylsulfonyl)ethyl)ethanamine **(VIad)** in 1 mL of acetic acid at 0 °C was added 5 mL of 48% aqueous HBr. The mixture was allowed to warm to room temperature and stirred for 72 h. The reaction was quenched by the slow addition of 20 mL of saturated sodium bicarbonate solution and extracted with 3 x 75 mL of ethyl acetate. The combined organic extracts were washed with 50 mL of brine, dried (Na₂SO₄), filtered and the solvent was removed *in vacuo.* The residue was purified by reverse-phase chromatography (REVELERIS^{®} C18 column-40 g; eluting with a linear gradient of 10-50% water/methanol) to provide 0.13 g (0.53 mmol, 68%) of 4-(1-((2-(methylsulfonyl)ethyl)amino)ethyl)isoquinolin-1(2*H*)-one (**VIIIcj**). ¹H NMR (300 MHz, DMSO-d₆): δ11.2 (s, 1H), 8.24 (d, 1H), 8.02 (d, 1H), 7.90 (s, 1H), 7.68-7.76 (m, 2H), 7.50 (t, 1H), 4.06 (q, 1H), 3.04-3.11 (m, 7H), 1.33 (d, 3H).

### 3-(3-Chloro-4-fluorophenyl)-1-(2-(methylsulfonyl)ethyl)-1-(1-(1-oxo-1,2-dihydroisoquinolin-4-yl)ethyl)urea (Compounds 107 & 108)

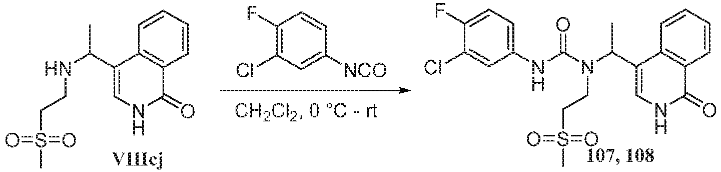

Racemic 3-(3-chloro-4-fluorophenyl)-1-(2-(methylsulfonyl)ethyl)-1-(1-(1-oxo-1,2-dihydroisoquinolin-4-yl)ethyl)urea was synthesized in a similar manner as described above from 4-(1-((2-(methylsulfonyl)ethyl)amino)ethyl)isoquinolin-1(2*H*)-one **(VIIIcj)** and 2-chloro-1-fluoro-4-isocyanatobenzene. The enantiomers were subsequently separated by SFC, Column: Chiralpak IA (30 x 250 mm) 5 µ, 70% CO₂:MeOH, flow rate 90 g/min.

3-(3-Chloro-4-fluorophenyl)-1-(2-(methylsulfonyl)ethyl)-1-(1-(1-oxo-1,2-dihydroisoquinolin-4-yl)ethyl)urea - Enantiomer I **(Compound 107).** LCMS: *m*/*z* found 466.2/468.2 [M+H]⁺, 5.66 min (Method A); ¹H NMR (400 MHz, DMSO-d₆): 11.22 (bs, 1H), 8.72 (bs, 1H), 8.27 (d, 1H), 7.83 (dd, 1H), 7.78 (t, 1H), 7.62 (d, 1H), 7.49-7.55 (m, 2H), 7.38 (t, 1H), 7.27 (s, 1H), 5.77 (q, 1H), 3.47 (t, 2H), 3.21 (t, 1H), 2.97 (s, 3H), 2.48-2.60 (m, 1H), 1.53 (d, 3H); Chiral analytical SFC: RT = 1.29 min, Chiralcel OD-3 (250 x 4.6 mm), 5 µ, 60% CO₂:MeOH, Flow rate = 3.0 mL/min.

3-(3-Chloro-4-fluorophenyl)-1-(2-(methylsulfonyl)ethyl)-1-(1-(1-oxo-1,2-dihydroisoquinolin-4-yl)ethyl)urea - Enantiomer II **(Compound 108).** LCMS: *m*/*z* found 466.2/468.2 [M+H]⁺, 5.64 min (Method A); ¹H NMR (400 MHz, DMSO-d₆): 11.22 (bs, 1H), 8.72 (bs, 1H), 8.27 (d, 1H), 7.83 (dd, 1H), 7.78 (t, 1H), 7.62 (d, 1H), 7.49-7.55 (m, 2H), 7.38 (t, 1H), 7.27 (s, 1H), 5.77 (q, 1H), 3.47 (t, 2H), 3.21 (t, 1H), 2.97 (s, 3H), 2.48-2.60 (m, 1H), 1.53 (d, 3H); Chiral analytical SFC: RT = 1.95 min, Chiralcel OD-3 (250 x 4.6 mm), 5 µ, 60% CO₂:MeOH, Flow rate = 3.0 mL/min.

### 3-(1-(1-Methoxyisoquinolin-4-yl)ethylamino)-N-methylpropanamide (VIae)

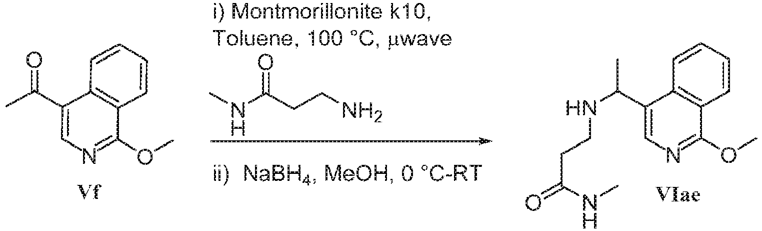

To a solution of 0.5 g (2.5 mmol, 1.0 eq.) of 1-(1-methoxyisoquinolin-4-yl)ethanone **(Vf)** in 5 mL of toluene under a nitrogen atmosphere was added 0.28 g (2.7 mmol 1.1 eq.) of 3-amino-N-methylpropanamide followed by 0.5 g of montmorillonite-K10 and the mixture was subjected to microwave irradiation maintaining a reaction temperature of 100 °C for 36 h. The mixture was allowed to cool to room temperature and then further cooled to 0 °C. The mixture was diluted with 5 mL of methanol and 0.18 g (5.3 mmol, 2.0 eq.) of sodium borohydride was added. The mixture was then allowed to warm to room temperature and stirred for 2 h. The mixture was filtered through CELITE^{®} and the pad was washed with 5 mL of ethyl acetate. The filtrate was concentrated *in vacuo* and the residue was purified by chromatography (REVELERIS^{®} SiO₂ column, eluting with a linear gradient of 20-60% ethyl acetate/petroleum ether) to provide 0.2 g (0.69 mmol, 28%) of 3-(1-(1-methoxyisoquinolin-4-yl)ethylamino)-N-methylpropanamide **(VIae).** LCMS: *m*/*z* found 288.4 [M+H]⁺.

### 3-(1-(1-Hydroxyisoquinolin-4-yl)ethylamino)-N-methylpropanamide (VIIIck)

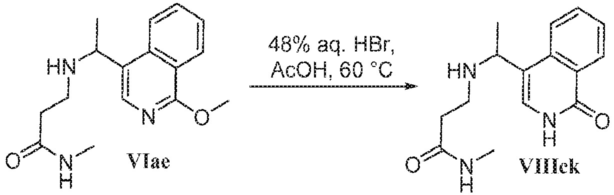

To a solution of 0.2 g (0.78 mmol, 1.0 eq.) of 3-(1-(1-methoxyisoquinolin-4-yl)ethylamino)-N-methylpropanamide **(VIae)** in 1 mL of acetic acid at 0 °C was added 5 mL of 48% aqueous HBr. The mixture was allowed to warm to room temperature and stirred for 72 h. The reaction was quenched by the slow addition of 20 mL of saturated sodium bicarbonate solution and extracted with 3 x 75 mL of ethyl acetate. The combined organic extracts were washed with 50 mL of brine, dried (Na₂SO₄), filtered and the solvent was removed *in vacuo.* The residue was purified by reverse phase chromatography (Reveleris^{®} C18 column-40 g; eluted with linear gradient of 10-50% water/methanol) to provide 0.13 g (0.48 mmol, 62%) of 3-(1-(1-hydroxyisoquinolin-4-yl)ethylamino)-*N*-methylpropanamide **(VIIIck).** LCMS: *m*/*z* found 274.4 [M+H]⁺.

### 3-(3-(3-Chloro-4-fluorophenyl)-1-(1-(1-oxo-1,2-dihydroisoquinolin-4-yl)ethyl)ureido)-N-methylpropanamide (Compounds 104, 117 & 118)

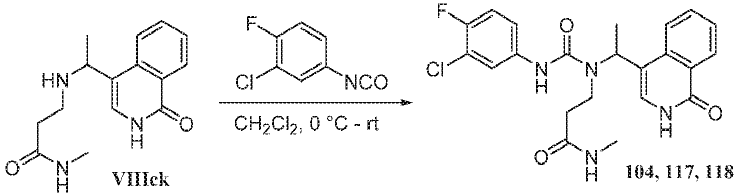

Racemic 3-(3-(3-chloro-4-fluorophenyl)-1-(1-(1-oxo-1,2-dihydroisoquinolin-4-yl)ethyl)ureido)-N-methylpropanamide **(Compound 104)** was synthesized in a similar manner as described above from 3-(1-(1-hydroxyisoquinolin-4-yl)ethylamino)-*N-*methylpropanamide **(VIIIck)** and 2-chloro-1-fluoro-4-isocyanatobenzene. The enantiomers were subsequently separated by SFC, Column: Chiralpak IA (30 x 250 mm) 5 µ, 70% CO₂:MeOH, flow rate 90 g/min.

3-(3-(3-Chloro-4-fluorophenyl)-1-(1-(1-oxo-1,2-dihydroisoquinolin-4-yl)ethyl)ureido)-N-methylpropanamide - Enantiomer I **(Compound 117).** LCMS: *m*/*z* found 445.1/447.1 [M+H]⁺; ¹H NMR (400 MHz, DMSO-d₆): 11.40 (s, 1H), 9.50 (s, 1H), 8.25 (d, 1H), 7.88 (dd, 1H), 7.68-7.80 (m, 2H), 7.63 (d, 1H), 7.45-7.51 (m, 2H), 7.36 (t, 1H), 7.20 (m, 1H), 5.84 (m, 1H), 3.40 (t, 2H), 2.46 (m, 3H), 1.89 (m, 2H), 1.45 (d, 3H); Chiral analytical SFC: RT = 1.88 min, Column: Chiralcel OD-3 (250 x 4.6 mm), 5 µ, 60% CO₂:MeOH, Flow rate = 3.0 mL/min.

3-(3-(3-Chloro-4-fluorophenyl)-1-(1-(1-oxo-1,2-dihydroisoquinolin-4-yl)ethyl)ureido)-N-methylpropanamide - Enantiomer II **(Compound 118).** LCMS: *m*/*z* found 445.1/447.1 [M+H]⁺; ¹H NMR (400 MHz, DMSO-d₆): 11.40 (s, 1H), 9.50 (s, 1H), 8.25 (d, 1H), 7.88 (dd, 1H), 7.68-7.80 (m, 2H), 7.63 (d, 1H), 7.45-7.51 (m, 2H), 7.36 (t, 1H), 7.20 (m, 1H), 5.84 (m, 1H), 3.40 (t, 2H), 2.46 (m, 3H), 1.89 (m, 2H), 1.45 (d, 3H); Chiral analytical SFC: RT = 2.47 min, Column: Chiralcel OD-3 (250 x 4.6 mm), 5 µ, 60% CO₂:MeOH, Flow rate = 3.0 mL/min.

### tert-Butyl 2-(1-methoxyisoquinolin-4-yl)-2-oxoacetate (Vx)

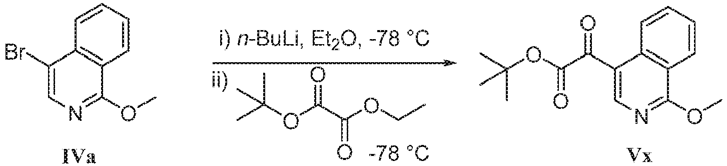

To a solution of 1.3 g (5.48 mmol, 1.0 eq.) of 4-bromo-1-methoxyisoquinoline **(IVa)** in 55 mL of anhydrous diethyl ether at -78 °C under a nitrogen atmosphere was added 6.85 mL (10.9 mmol, 2.0 eq.) of a 1.6 M solution of *n-*BuLi in hexane drop wise over approximately 15 min. The mixture was stirred at -78 °C for 30 min and 4.8 g (27.38 mmol, 5.0 eq.) of tert-butyl ethyl oxalate was added. After stirring at -78 °C for 3 h, the reaction was quenched with 25 mL of saturated aqueous ammonium chloride solution and extracted with 2 x 100 mL of ethyl acetate. The combined organic extracts were dried (Na₂SO₄), filtered and the solvent was removed *in vacuo.* The residue was purified by chromatography (SiO₂, eluting with a linear gradient of 15-20% ethyl acetate/petroleum ether) to provide 1.3 g (4.52 mmol, 82%) of tert-butyl 2-(1-methoxyisoquinolin-4-yl)-2-oxoacetate **(Vx).** LCMS: *m*/*z* found 288.2 [M+H]⁺; ¹H NMR (400 MHz, CDCl₃) δ 9.11 (d, 1H), 8.58 (s, 1H), 8.32 (d, 1H), 7.82-7.86 (m, 1H), 7.61-7.65 (m, 1H), 4.23 (s, 3H), 1.65 (s, 9H).

### tert-Butyl 2-(1-methoxyisoquinolin-4-yl)-2-(methylamino)acetate (VIbi)

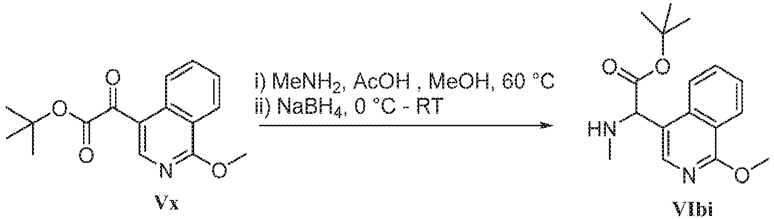

To a solution of 1.3 g (4.52 mmol) of tert-butyl 2-(1-methoxyisoquinolin-4-yl)-2-(methylamino)acetate **(Vx)** in 60 mL of methanol in a sealed tube at 0 °C under a nitrogen atmosphere was added 0.54 ml (9.05 mmol, 2.0 eq.) of glacial acetic acid followed by 9.1 mL (18.1 mmol, 4.0 eq.) of a 2 M solution of methyl amine in THF. The vessel was sealed, and the mixture was heated at 60 °C for 52 h. The mixture was then allowed to cool to room temperature, further cooled to 0 °C, and 0.34 g (9.0 mmol, 2.0 eq.) of sodium borohydride was added. On warming to room temperature, the mixture was stirred for a further 30 min. The reaction was quenched with 100 mL of ice-cold water and extracted with 3 x 100 mL of 10% methanol in methylene chloride. The combined organic extracts were washed with 100 mL of brine, dried (Na₂SO₄), filtered and the solvent was removed *in vacuo.* The residue was purified by MPLC (Silica gel column, eluting with a linear gradient of 50-60% of ethyl acetate/petroleum ether) to provide 0.45 g (1.48 mmol) of *tert*-butyl 2-(1-methoxyisoquinolin-4-yl)-2-(methylamino)acetate **(VIbi).** LCMS: *m*/*z* found 303.1 [M+H]⁺, RT = 1.45 min; ¹H NMR (400 MHz, CDCl₃) δ 8.26-8.29 (m, 1H), 8.08 (d, 1H), 7.93 (s, 1H), 7.67-7.71 (m, 1H), 7.51-7.56 (m, 1H), 4.55 (s, 1H), 4.14 (s, 3H), 2.45 (s, 3H), 1.78 (bs, 1H), 1.37 (s, 9H).

### 2-(1-Methoxyisoquinolin-4-yl)-2-(methylamino)ethan-1-ol (VIbj)

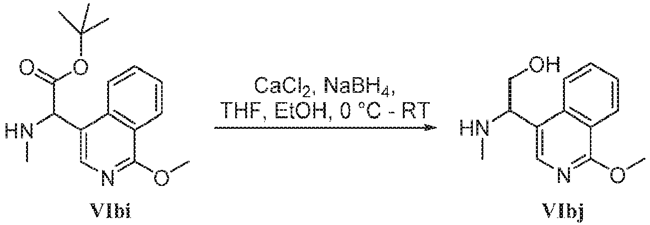

To a solution of 0.43 g (1.48 mmol, 1.0 eq.) of *tert*-butyl 2-(1-methoxyisoquinolin-4-yl)-2-(methylamino)acetate **(VIbi)** in 12 mL of 1: 1 *v*/*v* THF:ethanol at 0 °C was added 0.52 g (4.70 mmol, 3.0 eq.) of calcium chloride followed by 0.30 g (7.84 mmol, 5.0 eq.) of sodium borihydride. The mixture was allowed to warm to room temperature and stirred for 4 h. The mixture was then diluted with 50 mL of water and extracted with 2 x 100 mL of ethyl acetate. The combined organic extracts were washed with 50 mL of brine, dried (Na₂SO₄), filtered and the solvent was removed *in vacuo.* The residue was purified by MPLC (Silica gel column, eluting with a linear gradient of 15-20% of methanol in methylene chloride) to provide 0.16 g (0.69 mmol, 46%) of 2-(1-methoxyisoquinolin-4-yl)-2-(methylamino)ethanol **(VIbj).** LCMS: *m*/*z* found 233.1 [M+H]⁺, RT = 1.18 min; ¹H NMR (400 MHz, CDCl₃) δ 8.31-8.33 (m, 1H), 8.12 (s, 1H), 8.06 (d, 1H), 7.71-7.75 (m, 1H), 7.55-7.59 (m, 1H), 4.44-4.47 (m, 1H), 4.12 (s, 3H), 3.92-3.96 (m, 1H), 3.80-3.85 (m, 1H), 2.48 (s, 3H), 2.32 (bs, 2H).

### 4-(2-Hydroxy-1-(methylamino)ethyl)isoquinolin-1(2H)-one.hydrochloride (VIIIdd)

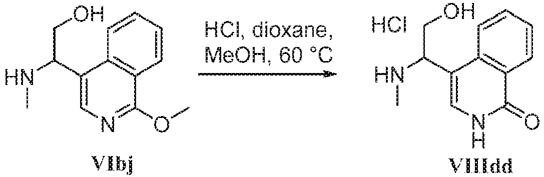

To a solution of 0.16 g (0.69 mmol, 1.0 eq.) 2-(1-methoxyisoquinolin-4-yl)-2-(methylamino)ethanol **(VIbj)** in 0.8 mL of methanol in a sealed tube at 0 °C was added 0.96 mL of a 4 M solution of HCl in 1,4-dioxane. The vessel was sealed, and the mixture was heated at 60 °C for 8 h. The mixture was allowed to cool to room temperature and concentrated *in vacuo.* The residue was triturated with 5 mL of n-pentane and dried under high vacuum to provide 0.16 g of 4-(2-hydroxy-1-(methylamino)ethyl)isoquinolin-1(2*H*)-one hydrochloride **(VIIIdd).** LCMS: *m*/*z* found 219.2 [M+H]⁺.

### 3-(3-Chloro-4-fluorophenyl)-1-(2-hydroxy-1-(1-oxo-1,2-dihydroisoquinolin-4-yl)ethyl)-1-methylurea (Compounds 379 & 380)

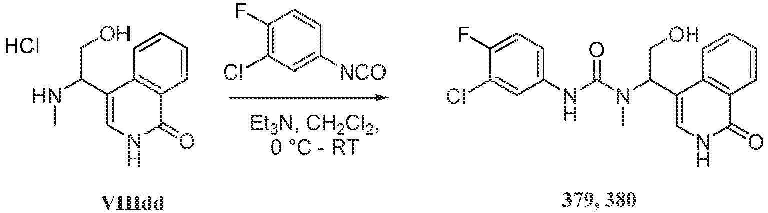

Racemic 3-(3-chloro-4-fluorophenyl)-1-(2-hydroxy-1-(1-oxo-1,2-dihydroisoquinolin-4-yl)ethyl)-1-methylurea was synthesised in an analogous manner as described above from 4-(2-hydroxy-1-(methylamino)ethyl)isoquinolin-1(2*H*)-one hydrochloride (**VIIIdd**) and 2-chloro-1-fluoro-4-isocyanatobenzene. The enantiomers were subsequently separated by chiral SFC, Column: Chiralpak IG (250 x 30 mm) 5 µ, 60% CO₂/MeOH, Flow rate 90 g/min.

3-(3-Chloro-4-fluorophenyl)-1-(2-hydroxy-1-(1-oxo-1,2-dihydroisoquinolin-4-yl)ethyl)-1-methylurea - Enantiomer I **(Compound 379).** LCMS: *m*/*z* found 390.2/392.2 [M+H]⁺; RT = 3.76 min (Method A); ¹H NMR (400 MHz, DMSO-d₆): δ 11.40 (bs, 1H), 8.44 (bs, 1H), 8.24 (d, 1H), 7.85-7.87 (m, 1H), 7.71-7.79 (m, 2H), 7.47-7.53 (m, 2H), 7.31 (t, 1H), 7.23 (bd, 1H), 5.70-5.73 (m, 1H), 5.00 (bt, 1H), 3.83-3.91 (m, 2H), 2.66 (s, 3H); Chiral analytical SFC: RT = 3.75 min, Column: Chiralpak IG (4.6 x 250 mm) 5 µ, 65 % CO₂/MeOH, Flow rate: 3.0 mL/min.

3-(3-Chloro-4-fluorophenyl)-1-(2-hydroxy-1-(1-oxo-1,2-dihydroisoquinolin-4-yl)ethyl)-1-methylurea - Enantiomer II **(Compound 380).** LCMS: *m*/*z* found 390.2/392.2 [M+H]⁺; RT = 3.76 min (Method A); ¹H NMR (400 MHz, DMSO-d₆): δ 11.40 (bs, 1H), 8.44 (bs, 1H), 8.24 (d, 1H), 7.85-7.87 (m, 1H), 7.71-7.79 (m, 2H), 7.47-7.53 (m, 2H), 7.31 (t, 1H), 7.23 (bd, 1H), 5.70-5.73 (m, 1H), 5.00 (bt, 1H), 3.83-3.91 (m, 2H), 2.66 (s, 3H); Chiral analytical SFC: RT = 6.89 min, Column: Chiralpak IG (4.6 x 250 mm) 5 µ, 65 % CO₂/MeOH, Flow rate: 3.0 mL/min.

### 3-Amino-3-(1-methoxyisoquinolin-4-yl)propanoic acid (VIbk)

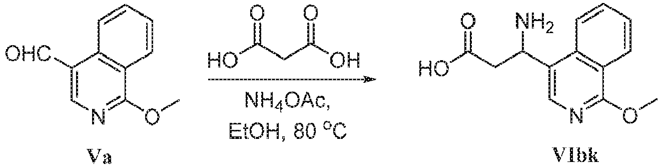

To a solution of 3.0 g (16.0 mmol, 1.0 eq.) of 1-methoxyisoquinoline-4-carbaldehyde **(Va)** in 60 mL of ethanol was added 1.66 g (16.0 mmol, 1.0 eq.) of malonic acid followed by 2.5 g (32.1 mmol, 2.0 eq.) of ammonium acetate and the mixture was heated at 80 °C for 16 h. The mixture was allowed to cool to room temperature, further cooled to 0 °C and stirred for 10 minutes. The resulting solid was collected by filtration and purified by reverse phase chromatography (C-18, eluting with a linear gradient of 0-50% [0.1% formic acid in acetonitrile]/water) to provide 0.5 g (2.03 mmol, 13%) 3-amino-3-(1-methoxyisoquinolin-4-yl)propanoic acid **(VIbk).** LCMS: *m*/*z* found 247.3 [M+H]⁺, RT = 1.21 min.

### 3-Amino-3-(1-methoxyisoquinolin-4-yl)propan-1-ol (VIbm)

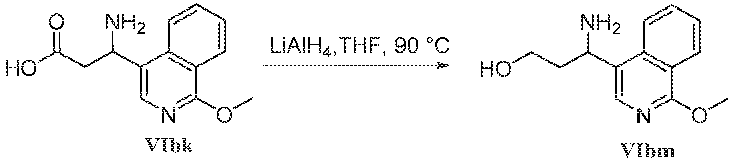

To a solution of 0.45 g (1.83 mmol, 1.0 eq.) of 3-amino-3-(1-methoxyisoquinolin-4-yl)propanoic acid **(VIbk)** in 9 mL of THF at 0 °C under a nitrogen atmosphere was added 2.7 mL (5.49 mmol, 3.0 eq.) of a 2 M solution of lithium aluminium hydride in THF. The mixture was allowed to warm to room temperature and then heated at 90 °C for 3 h. On cooling to room temperature, the reaction was quenched with 20 mL of water and then diluted with 30 mL of THF. The mixture was filtered through CELITE^{®} and the filtrate was evaporated *in vacuo* to provide 0.29 g of crude 3-amino-3-(1-methoxyisoquinolin-4-yl)propan-1-ol **(VIbm).** LCMS: *m*/*z* found 233.42 [M+H] ⁺.

### 1-(3-Chloro-4-fluorophenyl)-3-(3-hydroxy-1-(1-methoxyisoquinolin-4-yl)propyl)urea (Compounds 71, 77 & 78)

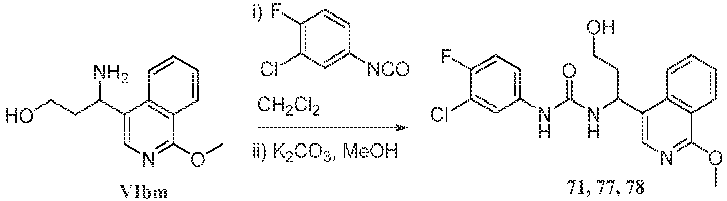

To a solution of 0.29 g of crude 3-amino-3-(1-methoxyisoquinolin-4-yl)propan-1-ol **(VIbm)** in 3 mL of methylene chloride at 0 °C was added 0.32 g (1.87 mmol, 1.5 eq.) of 2-chloro-1-fluoro-4-isocyanatobenzene. The mixture was allowed to warm to room temperature and stirred for 2 h. The solvent was removed *in vacuo* and the residue was resuspended in 5 mL of methanol and a solution of 0.1 g (3.08 mmol, 3.0 eq.) of sodium hydroxide in 5 mL of water was added. The mixture was stirred room temperature for 16 h and the methanol was removed *in vacuo.* The precipitated solid was collected by filtration and purified by reverse phase chromatography (C-18, eluting with a linear gradient 0-50% [0.1% formic acid in acetonitrile]/water) to provide 50 mg (0.12 mmol) of racemic 1-(3-Chloro-4-fluorophenyl)-3-(3-hydroxy-1-(1-methoxyisoquinolin-4-yl)propyl)urea **(Compound 71).** The enantiomers were subsequently separated by chiral SFC, Column: Chiralpak AD-H (250 x 30 mm) 5 µ, 60% CO₂/MeOH, Flow rate 60 g/min.

1-(3-Chloro-4-fluorophenyl)-3-(3-hydroxy-1-(1-methoxyisoquinolin-4-yl)propyl)urea - Enantiomer I **(Compound 77).** LCMS: *m*/*z* found 404.1/406.1 [M+H]⁺; ¹H NMR (400 MHz, DMSO-d₆): δ 8.71 (s, 1H), 8.23 (d, 1H), 8.14 (d, 1H), 8.01 (s, 1H), 7.83 (t, 1H), 7.72-7.74 (m, 1H), 7.66 (t, 1H), 7.16-7.27 (m, 2H), 6.93 (d, 1H), 5.48 (m, 1H), 4.72 (t, 1H), 4.04 (s, 3H), 3.31-3.55 (m, 2H), 1.95-2.05 (m, 2H); Chiral analytical SFC: RT = 1.58 min, Column: Chiralpak AD-H (4.6 x 250mm) 5 µ, 60 % CO₂/MeOH, Flow rate: 3.0 mL/min.

1-(3-Chloro-4-fluorophenyl)-3-(3-hydroxy-1-(1-methoxyisoquinolin-4-yl)propyl)urea - Enantiomer II **(Compound 78).** LCMS: *m*/*z* found 404.1/406.1 [M+H]⁺; ¹H NMR (400 MHz, DMSO-d₆): δ 8.71 (s, 1H), 8.23 (d, 1H), 8.14 (d, 1H), 8.01 (s, 1H), 7.83 (t, 1H), 7.72-7.74 (m, 1H), 7.66 (t, 1H), 7.16-7.27 (m, 2H), 6.93 (d, 1H), 5.48 (m, 1H), 4.72 (t, 1H), 4.04 (s, 3H), 3.31-3.55 (m, 2H), 1.95-2.05 (m, 2H); Chiral analytical SFC: RT = 3.02 min, Column: Chiralpak AD-H (4.6 x 250mm) 5 µ, 60 % CO₂/MeOH, Flow rate: 3.0 mL/min.

### 1-(1-Chloroisoquinolin-4-yl)ethan-1-one

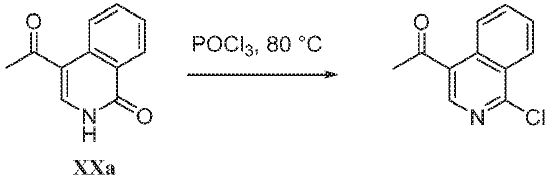

A solution of 2.40 g (12.83 mmol, 1.0 eq) of 4-acetylisoquinolin-1(2*H*)-one **(XXa)** in 24 mL of phosphorus oxychloride was heated at 80 °C for 3 h. The mixture was allowed to cool to room temperature and poured into 100 mL ice-cold water. The precipitated solid was collected by filtration and washed with 30 ml of chilled water followed by 30 ml of diethyl ether and then dried under high vacuum to provide 1.6 g (7.78 mmol, 61%) of 1-(1-chloroisoquinolin-4-yl)ethan-1-one. **LCMS:** *m*/*z* found 206.1/208.1 [M+H]⁺.

### 1-(1-Aminoisoquinolin-4-yl)ethan-1-one (Vh)

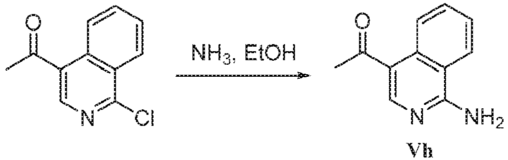

A solution of 0.5 g (2.43 mmol, 1.0 eq) of 1-(1-chloroisoquinolin-4-yl)ethan-1-one in 5 mL of saturated ethanolic ammonia was stirred at room temperature in a sealed vessel for 22 h. The mixture was then diluted with 20 mL of water and extracted with 3 x 30 mL of ethyl acetate. The combined organic extracts were washed with 30 mL of brine, dried (Na₂SO₄), filtered and the solvent was removed *in vacuo.* The residue was purified by trituration with 10 mL of n-pentane and dried under high vacuum to provide 0.35 g (1.88 mmol, 77%) of 1-(1-aminoisoquinolin-4-yl)ethan-1-one **(Vh).** LCMS: *m*/*z* found 187.0 [M+H]⁺; ¹H NMR (400 MHz, DMSO-d₆) δ 9.06 (d, 1H), 8.68 (s, 1H), 8.27 (d, 1H), 7.69-7.80 (m, 3H), 7.50-7.55 (m, 1H), 2.57 (s, 3H).

### 4-(1-(Methylamino)ethyl)isoquinolin-1-amine (VIaf)

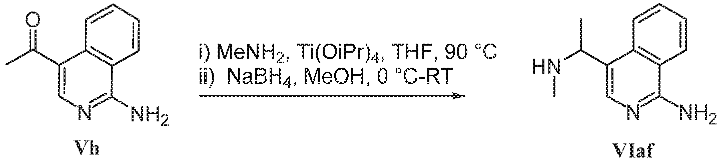

To a solution of 0.3 g (1.6 mmol, 1.0 eq.) of 1-(1-aminoisoquinolin-4-yl)ethan-1-one **(Vh)** in 3 mL of titanium isopropoxide in a sealed tube under a nitrogen atmosphere was added 5 mL (10 mmol, 6.3 eq.) of a 2 M solution of methylamine in THF and the mixture was heated at 90 °C for 16 h. The mixture was allowed to cool to room temperature and then further cooled to 0 °C. The mixture was diluted with 3 mL of methanol and 0.12 g (3.2 mmol, 2.0 eq.) of sodium borohydride was added. The mixture was then allowed to warm to room temperature and stirred for 2 h. The reaction was quenched by the addition of 30 mL of water and filtered through CELITE^{®}. The pad was washed with 10 mL of ethyl acetate and the filtrate was extracted with 3 x 30 mL of ethyl acetate. The combined organic extracts were washed with 40 mL of brine, dried (Na₂SO₄), filtered and the solvent was removed *in vacuo.* The residue was purified by chromatography (SiO₂, eluting with a linear gradient of 70-100% ethyl acetate/petroleum ether) to provide 0.2 g of 4-(1-(methylamino)ethyl)isoquinolin-1-amine **(VIaf).** ¹H NMR (400 MHz, DMSO-d₆): δ 8.19-8.14 (m, 2H), 7.83 (s, 1H), 7.64-7.59 (m, 1H), 7.46-7.42 (m, 1H), 7.60 (br s, 2H), 4.09-4.03 (m, 1H), 2.20 (s, 3H), 2.19 (br s, 1H), 1.35 (d, 3H).

### 1-(1-(1-Aminoisoquinolin-4-yl)ethyl)-3-(3-chloro-4-fluorophenyl)-1-methylurea (Compounds 238 & 239)

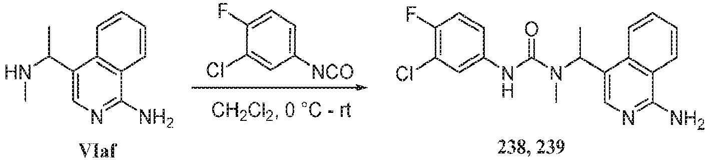

To a solution of 0.15 g of crude 4-(1-(methylamino)ethyl)isoquinolin-1-amine **(VIaf)** in 2 mL of methylene chloride under a nitrogen atmosphere at 0 °C was added 64 mg (0.37 mmol) of 2-chloro-1-fluoro-4-isocyanatobenzene. The reaction mixture was allowed to warm to room temperature and stirred for 1 h. The reaction mixture was then diluted with 10 mL of water and extracted with 2 x 30 ml of methylene chloride. The combined organic extracts were dried over (Na₂SO₄), filtered and the solvent was removed *in vacuo* to provide 0.2 g of racemic 1-(1-(1-aminoisoquinolin-4-yl)ethyl)-3-(3-chloro-4-fluorophenyl)-1-methylurea. LCMS: *m*/*z* found 373.1/375.1 [M+H]⁺. The enantiomers were subsequently separated by SFC, Column: Chiralpak IC (30 x 250 mm) 5 µ, 50% CO₂:MeOH, flow rate 100 g/min to provide 48 mg and 53 mg, respectively of the resolved enantiomers.

1-(1-(1-Aminoisoquinolin-4-yl)ethyl)-3-(3-chloro-4-fluorophenyl)-1-methylurea - Enantiomer I **(Compound 238).** LCMS: *m*/*z* found 373.3/375.3 [M+H]⁺, RT = 7.03 min (Method: A); ¹H NMR (400 MHz, DMSO-d₆): 8.42 (bs, 1H), 8.22 (d, 1H), 7.85-7.88 (m, 2H), 7.80 (d, 1H), 7.62-7.67 (m, 1H), 7.44-7.53 (m, 2H), 7.31 (t, 1H), 6.84 (bs, 2H), 5.99-6.03 (m, 1H), 2.53 (s, 3H), 1.52 (d, 3H); Chiral analytical SFC: RT = 3.98 min, Column: Chiralpak IC-3 (150 x 4.6 mm), 3 µ, 70% CO₂:MeOH, Flow rate = 3.0 mL/min.

1-(1-(1-Aminoisoquinolin-4-yl)ethyl)-3-(3-chloro-4-fluorophenyl)-1-methylurea - Enantiomer II **(Compound 239).** LCMS: *m*/*z* found 373.3/375.3 [M+H]⁺, RT = 7.03 min (Method: A); ¹H NMR (400 MHz, DMSO-d₆): 8.42 (bs, 1H), 8.22 (d, 1H), 7.85-7.88 (m, 2H), 7.80 (d, 1H), 7.62-7.67 (m, 1H), 7.44-7.53 (m, 2H), 7.31 (t, 1H), 6.84 (bs, 2H), 5.99-6.03 (m, 1H), 2.53 (s, 3H), 1.52 (d, 3H); Chiral analytical SFC: RT = 8.29 min, Column: Chiralpak IC-3 (150 x 4.6 mm), 3 µ, 70% CO₂:MeOH, Flow rate = 3.0 mL/min.

### 1-(1-Aminoisoquinolin-4-yl)ethan-1-one (Vi)

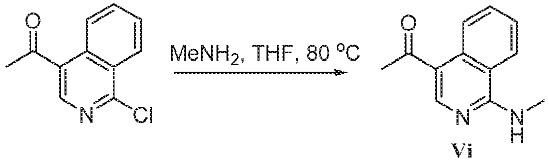

A solution of 0.6 g (2.92 mmol, 1.0 eq) of 1-(1-chloroisoquinolin-4-yl)ethan-1-one in 5 mL (10 mmol, 3.4 eq.) of a 2 M solution of methylamine in THF in a sealed vessel was heated at 80 °C for 16 h. The mixture was allowed to cool to room temperature and the solvent was removed *in vacuo.* The residue was triturated with 10 mL of n-pentane and dried under high vacuum to provide 0.52 g (2.60 mmol, 89%) of 1-(1-(methylamino)isoquinolin-4-yl)ethan-1-one (**Vi**). LCMS: *m*/*z* found 201.1 [M+H]⁺; ¹H NMR (400 MHz, DMSO-d₆) δ 9.05-9.08 (m, 1H), 8.78 (s, 1H), 8.36 (m, 1H), 8.24-8.26 (m, 1H), 7.70-7.74 (m, 1H), 7.52-7.57 (m, 1H), 3.06 (d, 3H), 2.57 (s, 3H).

### N-Methyl-4-(1-(methylamino)ethyl)isoquinolin-1-amine (VIag)

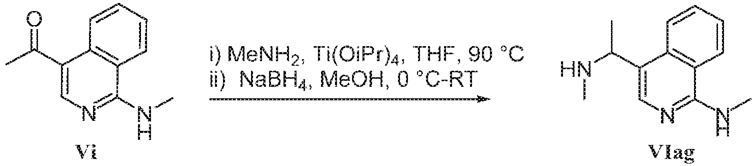

To a solution of 0.52 g (2.6 mmol, 1.0 eq.) of 1-(1-(methylamino)isoquinolin-4-yl)ethan-1-one **(Vi)** in 5 mL of titanium isopropoxide in a sealed tube under a nitrogen atmosphere was added 10 mL (20 mmol, 7.7 eq.) of a 2 M solution of methylamine in THF and the mixture was heated at 90 °C for 16 h. The mixture was allowed to cool to room temperature and then further cooled to 0 °C. The mixture was diluted with 5 mL of methanol and 0.20 g (5.2 mmol, 2.0 eq.) of sodium borohydride was added. The mixture was then allowed to warm to room temperature and stirred for 2 h. The reaction was quenched by the addition of 30 mL of water and filtered through CELITE^{®}. The pad was washed with 30 mL of ethyl acetate and the filtrate was extracted with 2 x 20 mL of ethyl acetate. The combined organic extracts were washed with 40 mL of brine, dried (Na₂SO₄), filtered and the solvent was removed *in vacuo* to provide 0.16 g of *N*-methyl-4-(1-(methylamino)ethyl)isoquinolin-1-amine **(VIag).** LCMS: *m*/*z* found 216.4 [M+H]⁺; ¹H NMR (400 MHz, DMSO-d₆) δ 8.13-8.20 (m, 2H), 7.92 (s, 1H), 7.58-7.65 (m, 1H), 7.43-7.49 (m, 1H), 7.30 (m, 1H), 4.04-4.10 (m, 1H), 3.32 (bs, 1H), 2.95 (d, 3H), 2.21 (s, 3H), 1.37 (d, 3H).

### 3-(3-Chloro-4-fluorophenyl)-1-methyl-1-(1-(1-(methylamino)isoquinolin-4-yl)ethyl)urea (Compounds 231 & 232)

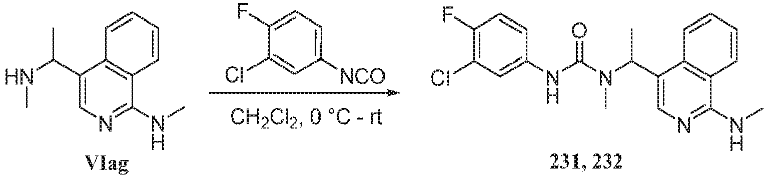

Racemic 3-(3-chloro-4-fluorophenyl)-1-methyl-1-(1-(1-(methylamino)isoquinolin-4-yl)ethyl)urea was synthesized in a similar manner as described above from N-methyl-4-(1-(methylamino)ethyl)isoquinolin-1-amine **(VIag)** and 2-chloro-1-fluoro-4-isocyanatobenzene. The enantiomers were subsequently separated by SFC, Column: Chiralpak IC (30 x 250 mm) 5 µ, 75% CO₂:MeOH, flow rate 70 g/min.

3-(3-Chloro-4-fluorophenyl)-1-methyl-1-(1-(1-(methylamino)isoquinolin-4-yl)ethyl)urea: Enantiomer I **(Compound 231).** LCMS: *m*/*z* found 387.2/389.2 [M+H]⁺, RT = 7.04 min (Method: A); ¹H NMR (400 MHz, DMSO-d₆) δ 8.42 (bs, 1H), 8.20 (d, 1H), 7.98 (s, 1H), 7.85-7.88 (m, 1H), 7.81 (d, 1H), 7.62-7.66 (m, 1H), 7.46-7.53 (m, 3H), 7.31 (t, 1H), 5.97-6.02 (m, 1H), 2.98 (d, 3H), 2.53 (s, 3H), 1.53 (d, 3H); Chiral analytical SFC: RT = 1.88 min, Column: Chiralpak IC (150 x 4.6 mm), 3 µ, 70% CO₂:MeOH, Flow rate = 3.0 mL/min.

3-(3-Chloro-4-fluorophenyl)-1-methyl-1-(1-(1-(methylamino)isoquinolin-4-yl)ethyl)urea: Enantiomer II **(Compound 232).** LCMS: *m*/*z* found 387.2/389.2 [M+H]⁺, RT = 7.00 min (Method: A); ¹H NMR (400 MHz, DMSO-d₆) δ 8.42 (bs, 1H), 8.20 (d, 1H), 7.98 (s, 1H), 7.85-7.88 (m, 1H), 7.81 (d, 1H), 7.62-7.66 (m, 1H), 7.46-7.53 (m, 3H), 7.31 (t, 1H), 5.97-6.02 (m, 1H), 2.98 (d, 3H), 2.53 (s, 3H), 1.53 (d, 3H); Chiral analytical SFC: RT = 3.12 min, Column: Chiralpak IC (150 x 4.6 mm), 3 µ, 70% CO₂:MeOH, Flow rate = 3.0 mL/min.

### 1-(1-(Ethylamino)isoquinolin-4-yl)ethan-1-one (Vj)

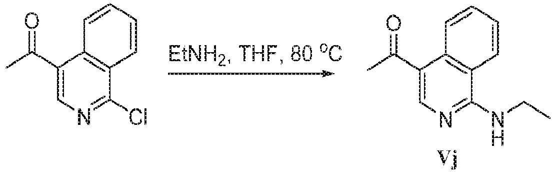

A solution of 0.5 g (2.4 mmol, 1.0 eq) of 1-(1-chloroisoquinolin-4-yl)ethan-1-one in 5 mL (10 mmol, 4 eq.) of a 2 M solution of ethylamine in THF in a sealed vessel was heated at 80 °C for 16 h. The mixture was allowed to cool to room temperature and the solvent was removed *in vacuo.* The residue was purified by chromatography (SiO₂, eluting with a linear gradient of 2-6% methanol/methylene chloride) to provide 0.28 g (1.31 mmol, 53%) of 1-(1-(ethylamino)isoquinolin-4-yl)ethan-1-one **(Vj).** LCMS: *m*/*z* found 215.1 [M+H]⁺; ¹H NMR (400 MHz, DMSO-d₆) δ 9.06-9.08 (m, 1H), 8.77 (s, 1H), 8.27-8.30 (m, 2H), 7.69-7.74 (m, 1H), 7.52-7.56 (m, 1H), 3.59-3.66 (m, 2H), 2.57 (d, 3H), 1.25 (t, 3H).

### N-Ethyl-4-(1-(methylamino)ethyl)isoquinolin-1-amine (VIah)

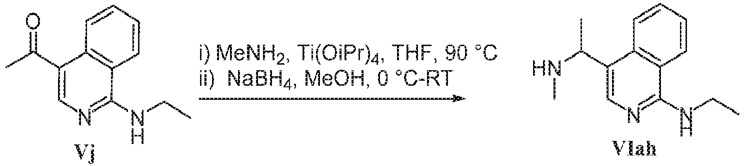

To a solution of 0.28 g (1.3 mmol, 1.0 eq.) of 1-(1-(ethylamino)isoquinolin-4-yl)ethan-1-one **(Vj)** in 3 mL of titanium isopropoxide in a sealed tube under a nitrogen atmosphere was added 5 mL (10 mmol, 7.7 eq.) of a 2 M solution of methylamine in THF and the mixture was heated at 90 °C for 16 h. The mixture was allowed to cool to room temperature and then further cooled to 0 °C. The mixture was diluted with 5 mL of methanol and 0.10 g (2.6 mmol, 2.0 eq.) of sodium borohydride was added. The mixture was then allowed to warm to room temperature and stirred for 2 h. The reaction was quenched by the addition of 30 mL of water and filtered through CELITE^{®}. The pad was washed with 30 mL of ethyl acetate and the filtrate was extracted with 2 x 20 mL of ethyl acetate. The combined organic extracts were washed with 40 mL of brine, dried (Na₂SO₄), filtered and the solvent was removed *in vacuo* to provide 0.30 g of *N*-ethyl-4-(1-(methylamino)ethyl)isoquinolin-1-amine **(VIah).** LCMS: *m*/*z* found 230.1 [M+H]⁺; ¹H NMR (400 MHz, DMSO-d₆) δ 8.16-8.22 (m, 2H), 7.89 (s, 1H), 7.58-7.63 (m, 1H), 7.43-7.47 (m, 1H), 7.21 (bt, 1H), 4.04-4.08 (m, 1H), 3.45-3.53 (m, 2H), 2.21 (s, 3H), 1.36 (d, 3H), 1.22 (t, 3H).

### 3-(3-Chloro-4-fluorophenyl)-1-(1-(1-(ethylamino)isoquinolin-4-yl)ethyl)-1-methylurea (Compounds 240 &, 241)

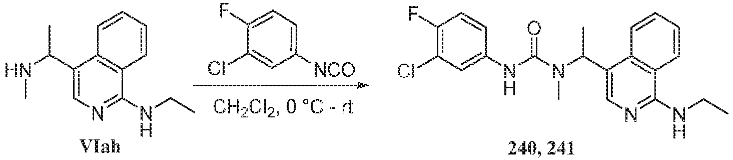

Racemic 3-(3-chloro-4-fluorophenyl)-1-(1-(1-(ethylamino)isoquinolin-4-yl)ethyl)-1-methylurea was synthesized in a similar manner as described above from *N*-ethyl-4-(1-(methylamino)ethyl)isoquinolin-1-amine **(VIah)** and 2-chloro-1-fluoro-4-isocyanatobenzene. The enantiomers were subsequently separated by SFC, Column: Chiralpak IC (30 x 250 mm) 5 µ, 70% CO₂:MeOH, flow rate 90 g/min.

3-(3-Chloro-4-fluorophenyl)-l-(l-(l-(ethylamino)isoquinolin-4-yl)ethyl)-1-methylurea: Enantiomer I **(Compound 240).** LCMS: *m*/*z* found 401.3/403.3 [M+H]⁺, RT = 7.45 min (Method: A); ¹H NMR (400 MHz, DMSO-d₆) δ 8.42 (bs, 1H), 8.25 (d, 1H), 7.96 (s, 1H), 7.84-7.88 (m, 1H), 7.80 (d, 1H), 7.61-7.67 (m, 1H), 7.41-7.54 (m, 3H), 7.31 (t, 1H), 5.97-6.01 (m, 1H), 3.50-3.57 (m, 2H), 2.54 (s, 3H), 1.52 (d, 3H), 1.24 (t, 3H); Chiral analytical SFC: RT = 4.60 min, Column: Chiralpak IC (250 x 4.6 mm), 3 µ, 75% CO₂:MeOH, Flow rate = 3.0 mL/min.

3-(3-Chloro-4-fluorophenyl)-1-(1-(1-(ethylamino)isoquinolin-4-yl)ethyl)-1-methylurea: Enantiomer II **(Compound 241).** LCMS: *m*/*z* found 401.3/403.3 [M+H]⁺, RT = 7.45 min (Method: A); ¹H NMR (400 MHz, DMSO-d₆) δ 8.42 (bs, 1H), 8.25 (d, 1H), 7.96 (s, 1H), 7.84-7.88 (m, 1H), 7.80 (d, 1H), 7.61-7.67 (m, 1H), 7.41-7.54 (m, 3H), 7.31 (t, 1H), 5.97-6.01 (m, 1H), 3.50-3.57 (m, 2H), 2.54 (s, 3H), 1.52 (d, 3H), 1.24 (t, 3H); Chiral analytical SFC: RT = 7.27 min, Column: Chiralpak IC (250 x 4.6 mm), 3 µ, 75% CO₂:MeOH, Flow rate = 3.0 mL/min.

### 1-(1-(Dimethylamino)isoquinolin-4-yl)ethan-1-one (Vk)

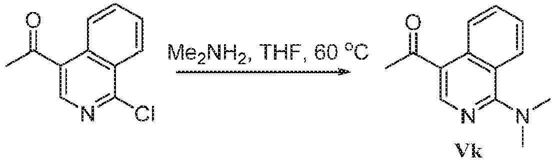

A solution of 0.5 g (2.4 mmol, 1.0 eq) of 1-(1-chloroisoquinolin-4-yl)ethan-1-one in 5 mL (10 mmol, 4 eq.) of a 2 M solution of dimethylamine in THF in a sealed vessel was heated at 60 °C for 8 h. The mixture was allowed to cool to room temperature and the solvent was removed *in vacuo.* The residue was triturated with 10 mL of n-pentane and dried under high vacuum to provide 0.50 g (2.33 mmol, 96%) of 1-(1-(dimethylamino)isoquinolin-4-yl)ethan-1-one **(Vk).** LCMS: *m*/*z* found 215.2 [M+H]⁺; ¹H NMR (400 MHz, DMSO-d₆) δ 9.99 (d, 1H), 8.77 (s, 1H), 8.14 (d, 1H), 7.71-7.77 (m, 1H), 7.51-7.55 (m, 1H), 3.25 (s, 6H), 2.61 (s, 3H).

### N,N-Dimethyl-4-(1-(methylamino)ethyl)isoquinolin-1-amine (VIai)

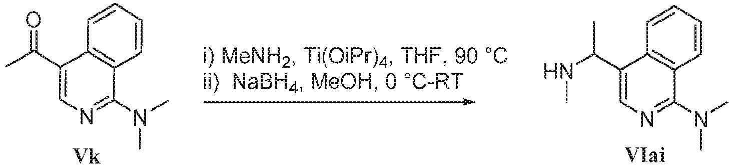

To a solution of 0.5 g (2.3 mmol, 1.0 eq.) of 1-(1-(dimethylamino)isoquinolin-4-yl)ethan-1-one **(Vk)** in 5 mL of titanium isopropoxide in a sealed tube under a nitrogen atmosphere was added 10 mL (20 mmol, 8.7 eq.) of a 2 M solution of methylamine in THF and the mixture was heated at 90 °C for 16 h. The mixture was allowed to cool to room temperature and then further cooled to 0 °C. The mixture was diluted with 5 mL of methanol and 0.18 g (4.7 mmol, 2.0 eq.) of sodium borohydride was added. The mixture was then allowed to warm to room temperature and stirred for 2 h. The reaction was quenched by the addition of 20 mL of water and filtered through CELITE^{®}. The pad was washed with 30 mL of ethyl acetate and the filtrate was extracted with 2 x 30 mL of ethyl acetate. The combined organic extracts were washed with 40 mL of brine, dried (Na₂SO₄), filtered and the solvent was removed *in vacuo* to provide 0.36 g (1.57 mmol, 67%) of *N,N-*dimethyl-4-(1-(methylamino)ethyl)isoquinolin-1-amine **(VIai).** LCMS: *m*/*z* found 230.4 [M+H]⁺; ¹H NMR (400 MHz, DMSO-d₆) δ 8.27 (d, 1H), 8.15 (d, 1H), 8.12 (s, 1H), 7.66-7.71 (m, 1H), 7.53-7.57 (m, 1H), 4.18-4.23 (m, 1H), 3.30 (bs, 1H), 2.97 (s, 6H), 2.22 (s, 3H), 1.38 (d, 3H).

### 3-(3-Chloro-4-fluorophenyl)-1-(1-(1-(dimethylamino)isoquinolin-4-yl)ethyl)-1-methylurea (Compounds 233 & 234)

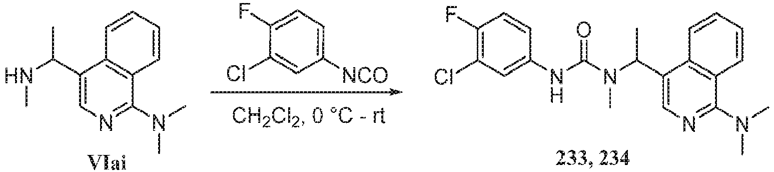

Racemic 3-(3-chloro-4-fluorophenyl)-1-(1-(1-(dimethylamino)isoquinolin-4-yl)ethyl)-1-methylurea was synthesized in a similar manner as described above from *N,N-*dimethyl-4-(1-(methylamino)ethyl)isoquinolin-1-amine **(VIai)** and 2-chloro-1-fluoro-4-isocyanatobenzene. The enantiomers were subsequently separated by SFC, Column: (R,R) Whelk-01 (30 x 250 mm) 5 µ, 65% CO₂:MeOH, flow rate 90 g/min.

3-(3-Chloro-4-fluorophenyl)-1-(1-(1-(dimethylamino)isoquinolin-4-yl)ethyl)-1-methylurea: Enantiomer I **(Compound 233).** LCMS: *m*/*z* found 401.3/403.3 [M+H]⁺, RT = 6.90 min (Method: A); ¹H NMR (400 MHz, DMSO-d₆) δ 8.45 (bs, 1H), 8.14-8.17 (m, 2H), 7.95 (d, 1H), 7.85-7.88 (m, 1H), 7.69-7.73 (m, 1H), 7.54-7.58 (m, 1H), 7.48-7.52 (m, 1H), 7.31 (t, 1H), 6.07-6.11 (m, 1H), 3.04 (s, 6H), 2.56 (s, 3H), 1.57 (d, 3H); Chiral analytical SFC: RT = 5.88 min, Column: (R,R) Whelk-01 (250 x 4.6 mm), 3 µ, 65% CO₂:MeOH, Flow rate = 3.0 mL/min.

3-(3-Chloro-4-fluorophenyl)-1-(1-(1-(dimethylamino)isoquinolin-4-yl)ethyl)-1-methylurea: Enantiomer II **(Compound 234).** LCMS: *m*/*z* found 401.3/403.3 [M+H]⁺, RT = 6.90 min (Method: A); ¹H NMR (400 MHz, DMSO-d₆) δ 8.45 (bs, 1H), 8.14-8.17 (m, 2H), 7.95 (d, 1H), 7.85-7.88 (m, 1H), 7.69-7.73 (m, 1H), 7.54-7.58 (m, 1H), 7.48-7.52 (m, 1H), 7.31 (t, 1H), 6.07-6.11 (m, 1H), 3.04 (s, 6H), 2.56 (s, 3H), 1.57 (d, 3H); Chiral analytical SFC: RT = 8.16 min, Column: (R,R) Whelk-01 (250 x 4.6 mm), 3 µ, 65% CO₂:MeOH, Flow rate = 3.0 mL/min.

### 1-(1-((2-Hydroayethyl)amino)isoquinolin-4-yl)ethan-1-one (Vm)

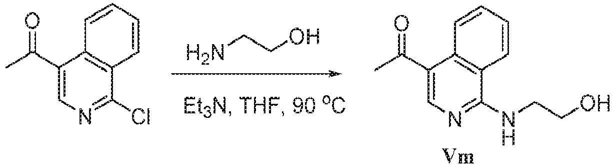

To a solution of 0.5 g (2.5 mmol, 1.0 eq) of 1-(1-chloroisoquinolin-4-yl)ethan-1-one in 4 mL of THF in a sealed vessel was added 1.0 g of triethylamine followed by 0.24 g (3.9 mmol, 1.6 eq.) of 2-aminoethan-1-ol and the mixture was heated at 90 °C for 6 h. The mixture was allowed to cool to room temperature, diluted with 30 mL of water and extracted with 3 x 30 mL of ethyl acetate. The combined organic extracts were dried (Na₂SO₄), filtered and the solvent was removed *in vacuo.* The residue was triturated with 10 mL of *n*-pentane and dried under high vacuum to provide 0.45 g (1.95 mmol, 80%) of 1-(1-((2-hydroxyethyl)amino)isoquinolin-4-yl)ethan-1-one **(Vm).** LCMS: *m*/*z* found 231.1 [M+H]⁺

### 2-((4-(1-(Methylamino)ethyl)isoquinolin-1-yl)amino)ethan-1-ol (VIaj)

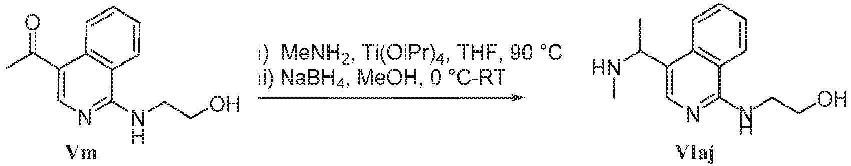

To a solution of 0.45 g (1.95 mmol, 1.0 eq.) of 1-(1-((2-hydroxyethyl)amino) isoquinolin-4-yl)ethan-1-one **(Vm)** in 4.5 mL of titanium isopropoxide in a sealed tube under a nitrogen atmosphere was added 5 mL (10 mmol, 5 eq.) of a 2 M solution of methylamine in THF and the mixture was heated at 90 °C for 16 h. The mixture was allowed to cool to room temperature and then further cooled to 0 °C. The mixture was diluted with 30 mL of methanol and 0.22 g (5.9 mmol, 2.0 eq.) of sodium borohydride was added. The mixture was then allowed to warm to room temperature and stirred for 2 h. The reaction was quenched by the addition of 30 mL of water and filtered through CELITE^{®}. The pad was washed with 30 mL of ethyl acetate and the filtrate was extracted with 2 x 30 mL of ethyl acetate. The combined organic extracts were washed with 40 mL of brine, dried (Na₂SO₄), filtered and the solvent was removed *in vacuo* to provide 0.4 g of 2-((4-(1-(methylamino)ethyl)isoquinolin-1-yl)amino)ethan-1-ol **(VIaj).** LCMS: *m*/*z* found 246.2 [M+H]⁺.

### 3-(3-Chloro-4-fluorophenyl)-1-(1-(1-((2-hydroxyethyl)amino)isoquinolin-4-yl)ethyl)-1-methylurea (Compounds 242 & 243)

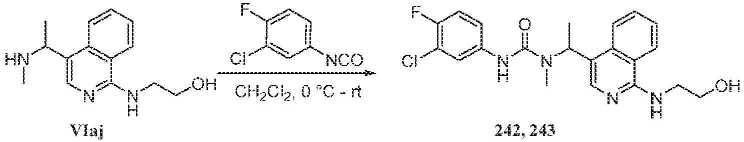

Racemic 3-(3-chloro-4-fluorophenyl)-1(1(1((2-hydroxyethyl)amino)isoquinolin-4-yl)ethyl)-1-methylurea was synthesized in a similar manner as described above from 2-((4-(1-(methylamino)ethyl)isoquinolin-1-yl)amino)ethan-1-ol **(VIaj)** and 2-chloro-1-fluoro-4-isocyanatobenzene. The enantiomers were subsequently separated by SFC, Column: Chiralpak AD-H (30 x 250 mm) 5 µ, 85% CO₂:MeOH, flow rate 100 g/min.

3-(3-Chloro-4-fluorophenyl)-1-(1-(1-((2-hydroxyethyl)amino)isoquinolin-4-yl)ethyl)-1-methylurea: Enantiomer I **(Compound 242).** LCMS: *m*/*z* found 417.2/419.2 [M+H]⁺, RT = 7.34 min (Method: A); ¹H NMR (400 MHz, DMSO-d₆) δ 8.43 (bs, 1H), 8.25 (d, 1H), 7.95 (s, 1H), 7.85-7.88 (m, 1H), 7.81 (d, 1H), 7.63-7.67 (m, 1H), 7.46-7.53 (m, 3H), 7.31 (t, 1H), 5.98-6.01 (m, 1H), 4.86 (t, 1H), 3.54-3.67 (m, 4H), 2.54 (s, 3H), 1.52 (d, 3H); Chiral analytical SFC: RT = 1.72 min, Column: Chiralpak AD-3 (150 x 4.6 mm), 3 µ, 75% CO₂:MeOH, Flow rate = 3.0 mL/min.

3-(3-Chloro-4-fluorophenyl)-1-(1-(1-((2-hydroxyethyl)amino)isoquinolin-4-yl)ethyl)-1-methylurea: Enantiomer II **(Compound 243).** LCMS: *m*/*z* found 417.2/419.2 [M+H]⁺, RT = 7.34 min (Method: A); ¹H NMR (400 MHz, DMSO-d₆) δ 8.43 (bs, 1H), 8.25 (d, 1H), 7.95 (s, 1H), 7.85-7.88 (m, 1H), 7.81 (d, 1H), 7.63-7.67 (m, 1H), 7.46-7.53 (m, 3H), 7.31 (t, 1H), 5.98-6.01 (m, 1H), 4.86 (t, 1H), 3.54-3.67 (m, 4H), 2.54 (s, 3H), 1.52 (d, 3H); Chiral analytical SFC: RT = 2.67 min, Column: Chiralpak AD-3 (150 x 4.6 mm), 3 µ, 75% CO₂:MeOH, Flow rate = 3.0 mL/min.

### tert-Butyl (2-((4-acetylisoquinolin-1-yl)amino)ethyl)carbamate (Vn)

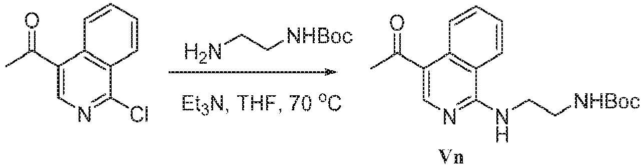

To a solution of 0.7 g (3.4 mmol, 1.0 eq) of 1-(1-chloroisoquinolin-4-yl)ethan-1-one in 7 mL of THF in a sealed vessel was added 2.3 mL (17.1 mmol, 5.0 eq.) of triethylamine followed by 1.1 g (6.8 mmol, 2.0 eq.) of tert-butyl (2-aminoethyl)carbamate and the mixture was heated at 70 °C for 16 h. The mixture was allowed to cool to room temperature, diluted with 40 mL of water and extracted with 3 x 50 mL of ethyl acetate. The combined organic extracts were dried (Na₂SO₄), filtered and the solvent was removed *in vacuo.* The residue was triturated with 20 mL of n-pentane and dried under high vacuum to provide 0.7 g (2.1 mmol, 62%) of tert-butyl (2-((4-acetylisoquinolin-1-yl)amino)ethyl)carbamate **(Vn).** LCMS: *m*/*z* found 330.3 [M+H]⁺; ¹H NMR (400 MHz, CDCl₃) δ 9.18 (d, 1H), 8.73 (s, 1H), 7.88-7.91 (m, 1H), 7.68-7.73 (m, 1H), 7.48-7.53 (m, 1H), 7.27 (bs, 1H), 5.09 (bt, 1H), 3.73-3.76 (m, 2H), 3.55-3.59 (m, 2H), 2.65 (s, 3H), 1.45 (s, 9H).

### tert-Butyl (2-((4-(1-(methylamino)ethyl)isoquinolin-1-yl)amino)ethyl)carbamate (VIak)

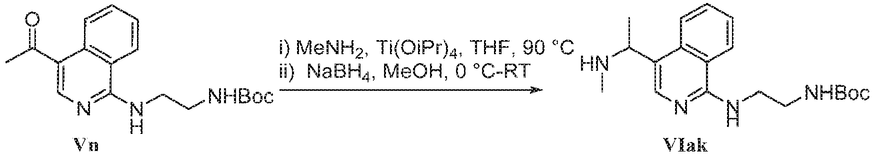

To a solution of 0.7 g (2.1 mmol, 1.0 eq.) of tert-butyl (2-((4-acetylisoquinolin-1-yl)amino)ethyl)carbamate **(Vn)** in 10 mL of THF in a sealed tube under a nitrogen atmosphere was added 10 mL of titanium isopropoxide followed by 15 mL (30 mmol, 14 eq.) of a 2 M solution of methylamine in THF and the mixture was heated at 90 °C for 16 h. The mixture was allowed to cool to room temperature and then further cooled to 0 °C. The mixture was diluted with 10 mL of methanol and 0.16 g (4.25 mmol, 2.0 eq.) of sodium borohydride was added. The mixture was then allowed to warm to room temperature and stirred for 2 h. The reaction was quenched by the addition of 30 mL of water and filtered through CELITE^{®}. The pad was washed with 30 mL of ethyl acetate and the filtrate was extracted with 50 mL of ethyl acetate. The organic extracts were washed with 50 mL of brine, dried (Na₂SO₄), filtered and the solvent was removed *in vacuo* to provide 0.4 g of *tert*-butyl (2-((4-(1-(methylamino)ethyl)isoquinolin-1-yl)amino)ethyl)carbamate **(VIak).** LCMS: *m*/*z* found 345.2 [M+H]⁺.

### 1-(1-(1-((2-Aminoethyl)amino)isoquinolin-4-yl)ethyl)-3-(3-chloro-4-fluorophenyl)-1-methylurea (Compounds 244, 249 & 250)

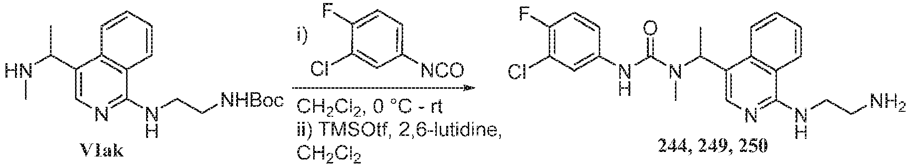

Racemic 1-(1-(1-((2-aminoethyl)amino)isoquinolin-4-yl)ethyl)-3-(3-chloro-4-fluorophenyl)-1-methylurea **(Compound 244)** was synthesized in a similar manner as described above from *tert*-butyl (2-((4-(1-(methylamino)ethyl)isoquinolin-1-yl)amino)ethyl)carbamate **(VIak)** and 2-chloro-1-fluoro-4-isocyanatobenzene followed by TMS triflate-mediated Boc deprotection. The enantiomers were subsequently separated by SFC, Column: Chiralpak IC (30 x 250 mm) 5 µ, 70% CO₂:MeOH, flow rate 70 g/min.

1-(1-(1-((2-Aminoethyl)amino)isoquinolin-4-yl)ethyl)-3-(3-chloro-4-fluorophenyl)-1-methylurea: Enantiomer I **(Compound 249).** LCMS: *m*/*z* found 416.2/418.2 [M+H]⁺, RT = 6.13 min (Method: A); ¹H NMR (400 MHz, DMSO-d₆) δ 8.42 (bs, 1H), 8.27 (d, 1H), 7.95 (s, 1H), 7.85-7.88 (m, 1H), 7.80 (d, 1H), 7.62-7.66 (m, 1H), 7.46-7.53 (m, 2H), 7.40 (m, 1H), 7.31 (t, 1H), 5.98-6.01 (m, 1H), 3.49-3.54 (m, 2H), 2.79-2.83 (m, 2H), 2.53 (s, 3H), 1.52 (d, 3H); Chiral analytical SFC: RT = 3.97 min, Column Chiralpak IC-3 (150 x 4.6 mm), 3 µ, 70% CO₂:MeOH, Flow rate = 3.0 mL/min.

1-(1-(1-((2-Aminoethyl)amino)isoquinolin-4-yl)ethyl)-3-(3-chloro-4-fluorophenyl)-1-methylurea: Enantiomer II **(Compound 250).** LCMS: *m*/*z* found 416.2/418.2 [M+H]⁺, RT = 6.13 min (Method: A); ¹H NMR (400 MHz, DMSO-d₆) δ 8.42 (bs, 1H), 8.27 (d, 1H), 7.95 (s, 1H), 7.85-7.88 (m, 1H), 7.80 (d, 1H), 7.62-7.66 (m, 1H), 7.46-7.53 (m, 2H), 7.40 (m, 1H), 7.31 (t, 1H), 5.98-6.01 (m, 1H), 3.49-3.54 (m, 2H), 2.79-2.83 (m, 2H), 2.53 (s, 3H), 1.52 (d, 3H); Chiral analytical SFC: RT = 5.64 min, Column: Chiralpak IC-3 (150 x 4.6 mm), 3 µ, 70% CO₂:MeOH, Flow rate = 3.0 mL/min.

### 1-(1-(((1-Methyl-1H-1,2,4-triazol-3-yl)methyl)amino)isoquinolin-4-yl)ethan-1-one (Vo)

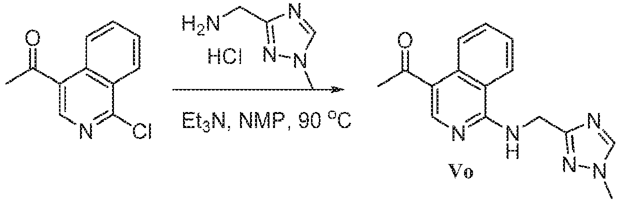

To a solution of 0.4 g (1.95 mmol, 1.0 eq) of 1-(1-chloroisoquinolin-4-yl)ethan-1-one in 5 mL of NMP was added 1.3 mL (9.75 mmol, 5.0 eq.) of triethylamine followed by 0.58 g (3.9 mmol, 2.0 eq.) of (1-methyl-1*H*-1,2,4-triazol-3-yl)methanamine hydrochloride and the mixture was heated at 90 °C for 3 h. The mixture was allowed to cool to room temperature, diluted with 40 mL of water and extracted with 3 x 30 mL of 10% methanol in methylene chloride. The combined organic extracts were dried (Na₂SO₄), filtered and the solvent was removed *in vacuo.* The residue was purified column chromatography (SiO₂, eluting with a linear gradient of 30-65% ethyl acetate in petroleum ether) to provide 0.45 g of 1-(1-(((1-methyl-1*H*-1,2,4-triazol-3-yl)methyl)amino)isoquinolin-4-yl)ethan-1-one **(Vo).** LCMS: *m*/*z* found 282.3 [M+H]⁺.

### N-((1-Methyl-1H-1,2,4-triazol-3-yl)methyl)-4-(1-(methylamino)ethyl)isoquinolin-1-amine (VIam)

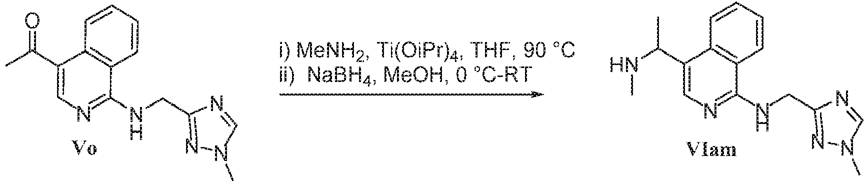

To a solution of 0.45 g (2.1 mmol, 1.0 eq.) of 1-(1-(((1-methyl-1*H*-1,2,4-triazol-3-yl)methyl)amino)isoquinolin-4-yl)ethan-1-one **(Vo)** in 5 mL of THF in a sealed tube under a nitrogen atmosphere was added 5 mL of titanium isopropoxide followed by 5 mL (10 mmol) of a 2 M solution of methylamine in THF and the mixture was heated at 90 °C for 6 h. The mixture was allowed to cool to room temperature and then further cooled to 0 °C. The mixture was diluted with 5 mL of methanol and 0.12 g (3.2 mmol) of sodium borohydride was added. The mixture was then allowed to warm to room temperature and stirred for 2 h. The reaction was quenched by the addition of 30 mL of water and filtered through CELITE^{®}. The pad was washed with 30 mL of ethyl acetate and the filtrate was extracted with 50 mL of ethyl acetate. The organic extracts were washed with 50 mL of brine, dried (Na₂SO₄), filtered and the solvent was removed *in vacuo* to provide 0.36 g of *N*-((1-methyl-1*H*-1,2,4-triazol-3-yl)methyl)-4-(1-(methylamino)ethyl)isoquinolin-1-amine **(VIam).** LCMS: *m*/*z* found 297.3 [M+H]⁺.

### 3-(3-Chloro-4-fluorophenyl)-1-methyl-1-(1-(1-(((1-methyl-1H-1,2,4-triazol-3-yl)methyl)amino) isoquinolin-4-yl)ethyl)urea (Compounds 245 & 246)

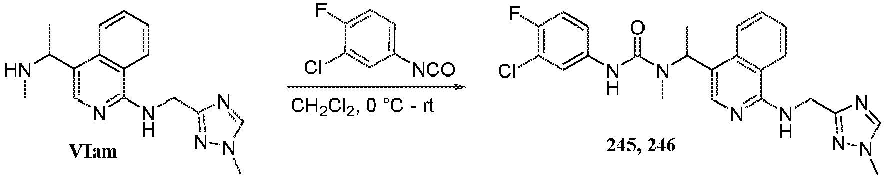

Racemic 3-(3-chloro-4-fluorophenyl)-1-methyl-1-(1-(1-(((1-methyl-1*H*-1,2,4-triazol-3-yl)methyl)amino) isoquinolin-4-yl)ethyl)urea was synthesized in a similar manner as described above from *N*-((1-methyl-1*H*-1,2,4-triazol-3-yl)methyl)-4-(1-(methylamino)ethyl)isoquinolin-1-amine **(VIam)** and 2-chloro-1-fluoro-4-isocyanatobenzene. The enantiomers were subsequently separated by SFC, Column: Chiralpak IC (30 x 250 mm) 5 µ, 60% CO₂:MeOH, flow rate 100 g/min.

3-(3-Chloro-4-fluorophenyl)-1-methyl-1-(1-(1-(((1-methyl-1*H*-1,2,4-triazol-3-yl)methyl)amino) isoquinolin-4-yl)ethyl)urea - Enantiomer I **(Compound 245).** LCMS: *m*/*z* found 468.3/470.3 [M+H]⁺, RT = 7.34 min (Method: A); ¹H NMR (400 MHz, DMSO-d₆) δ 8.43 (bs, 1H), 8.31-8.34 (m, 2H), 7.81-7.94 (m, 4H), 7.64-7.69 (m, 1H), 7.48-7.53 (m, 2H), 7.31 (t, 1H), 5.98-6.01 (m, 1H), 4.68-4.81 (m, 2H), 3.80 (s, 3H), 2.54 (s, 3H), 1.51 (d, 3H); Chiral analytical SFC: RT = 5.16 min, Column: Chiralpak IC (250 x 4.6 mm), 5 µ, 60% CO₂:MeOH, Flow rate = 3.0 mL/min.

3-(3-Chloro-4-fluorophenyl)-1-methyl-1-(1-(1-(((1-methyl-1H-1,2,4-triazol-3-yl)methyl)amino) isoquinolin-4-yl)ethyl)urea: Enantiomer II **(Compound 246).** LCMS: *m*/*z* found 468.3/470.3 [M+H]⁺, RT = 7.34 min (Method: A); ¹H NMR (400 MHz, DMSO-d₆) δ 8.43 (bs, 1H), 8.31-8.34 (m, 2H), 7.81-7.94 (m, 4H), 7.64-7.69 (m, 1H), 7.48-7.53 (m, 2H), 7.31 (t, 1H), 5.98-6.01 (m, 1H), 4.68-4.81 (m, 2H), 3.80 (s, 3H), 2.54 (s, 3H), 1.51 (d, 3H); Chiral analytical SFC: RT = 9.12 min, Column: Chiralpak IC (250 x 4.6 mm), 5 µ, 60% CO₂:MeOH, Flow rate = 3.0 mL/min.

### 1-(1-(((1H-1,2,4-Triazol-3-yl)methyl)amino)isoquinolin-4-yl)ethan-1-one (Vp)

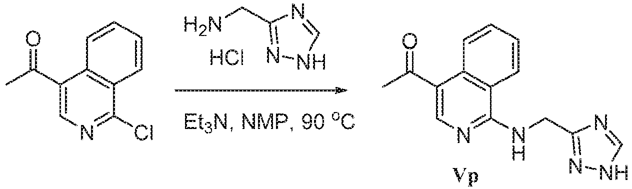

To a solution of 0.4 g (1.95 mmol, 1.0 eq) of 1-(1-chloroisoquinolin-4-yl)ethan-1-one in 5 mL of NMP was added 1.3 mL (9.75 mmol, 5.0 eq.) of triethylamine followed by 0.52 g (3.9 mmol, 2.0 eq.) of (1*H*-1,2,4-triazol-3-yl)methanamine hydrochloride and the mixture was heated at 90 °C for 3 h. The mixture was allowed to cool to room temperature, diluted with 40 mL of water and extracted with 3 x 30 mL of 10% methanol in methylene chloride. The combined organic extracts were dried (Na₂SO₄), filtered and the solvent was removed *in vacuo* to provide 0.5 g of crude 1-(1-(((1*H*-1,2,4-triazol-3-yl)methyl)amino)isoquinolin-4-yl)ethan-1-one **(Vp).** LCMS: *m*/*z* found 268.2 [M+H]⁺.

### N-((1H-1,2,4-Triazol-3-yl)methyl)-4-(1-(methylamino)ethyl)isoquinolin-1-amine (VIan)

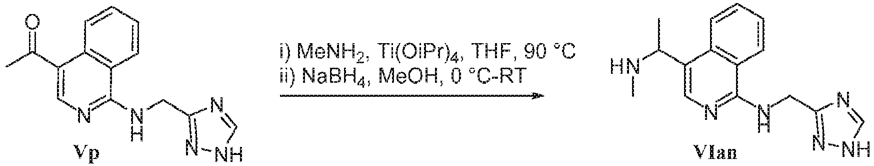

To a solution of 0.3 g of crude 1-(1-(((1*H*-1,2,4-triazol-3-yl)methyl)amino) isoquinolin-4-yl)ethan-1-one **(Vp)** in 3 mL of THF in a sealed tube under a nitrogen atmosphere was added 3 mL of titanium isopropoxide followed by 5 mL (10 mmol) of a 2 M solution of methylamine in THF and the mixture was heated at 90 °C for 6 h. The mixture was allowed to cool to room temperature and then further cooled to 0 °C. The mixture was diluted with 5 mL of methanol and 0.12 g (3.2 mmol) of sodium borohydride was added. The mixture was then allowed to warm to room temperature and stirred for 2 h. The reaction was quenched by the addition of 30 mL of water and filtered through CELITE^{®}. The pad was washed with 30 mL of ethyl acetate and the filtrate was extracted with 50 mL of ethyl acetate. The organic extracts were washed with 50 mL of brine, dried (Na₂SO₄), filtered and the solvent was removed *in vacuo* to provide 0.39 g ofN ((1H-1,2,4-triazol-3-yl)methyl)-4-(1-(methylamino)ethyl)isoquinolin-1-amine **(VIan).** LCMS: *m*/*z* found 283.2 [M+H]⁺.

### 1-(1-(1-(((1H-1,2,4-Triazol-3-yl)methyl)amino)isoquinolin-4-yl)ethyl)-3-(3-chloro-4-fluorophenyl)-1-methylurea (Compounds 247 & 248)

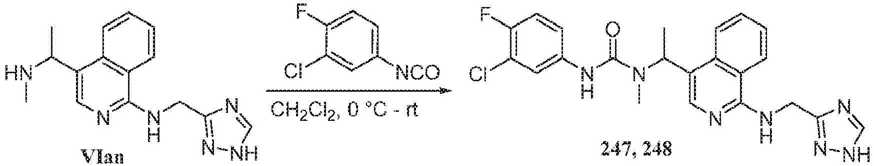

Racemic 1-(1-(1-(((1*H*-1,2,4-triazol-3-yl)methyl)amino)isoquinolin-4-yl)ethyl)-3-(3-chloro-4-fluorophenyl)-1-methylurea was synthesized in a similar manner as described above from *N-*((1*H*-1,2,4-triazol-3-yl)methyl)-4-(1-(methylamino)ethyl)isoquinolin-1-amine **(VIan)** and 2-chloro-1-fluoro-4-isocyanatobenzene. The enantiomers were subsequently separated by SFC, Column: Chiralpak AD-H (30 x 250 mm) 5 µ, 80% CO₂:MeOH, flow rate 100 g/min.

1-(1-(1-(((1*H*-1,2,4-Triazol-3-yl)methyl)amino)isoquinolin-4-yl)ethyl)-3-(3-chloro-4-fluorophenyl)-1-methylurea: Enantiomer I **(Compound 247).** LCMS: *m*/*z* found 454.2/456.2 [M+H]⁺, RT = 7.19 min (Method: A); ¹H NMR (400 MHz, DMSO-d₆) δ 13.69 (bs, 1H), 8.43 (bs, 1H), 8.31-8.34 (m, 1H), 8.12 (bs, 1H), 7.94 (s, 1H), 7.81-7.88 (m, 3H), 7.65-7.70 (m, 1H), 7.49-7.56 (m, 2H), 7.31 (t, 1H), 5.98-6.01 (m, 1H), 4.78-4.83 (m, 2H), 2.54 (s, 3H), 1.51 (d, 3H); Chiral analytical SFC: RT = 1.75 min, Column: Chiralpak AD-H (250 x 4.6 mm), 5 µ, 60% CO₂:MeOH, Flow rate = 3.0 mL/min.

1-(1-(1-(((1*H*-1,2,4-Triazol-3-yl)methyl)amino)isoquinolin-4-yl)ethyl)-3-(3-chloro-4-fluorophenyl)-1-methylurea: Enantiomer II **(Compound 248).** LCMS: *m*/*z* found 454.2/456.2 [M+H]⁺, RT = 7.21 min (Method: A); ¹H NMR (400 MHz, DMSO-d₆) δ 13.69 (bs, 1H), 8.43 (bs, 1H), 8.31-8.34 (m, 1H), 8.12 (bs, 1H), 7.94 (s, 1H), 7.81-7.88 (m, 3H), 7.65-7.70 (m, 1H), 7.49-7.56 (m, 2H), 7.31 (t, 1H), 5.98-6.01 (m, 1H), 4.78-4.83 (m, 2H), 2.54 (s, 3H), 1.51 (d, 3H); Chiral analytical SFC: RT = 5.57 min, Column: Chiralpak AD-H (250 x 4.6 mm), 5 µ, 60% CO₂:MeOH, Flow rate = 3.0 mL/min.

### 1-(1-(((2H-1,2,3-Triazol-4-yl)methyl)amino)isoquinolin-4-yl)ethan-1-one (Vq)

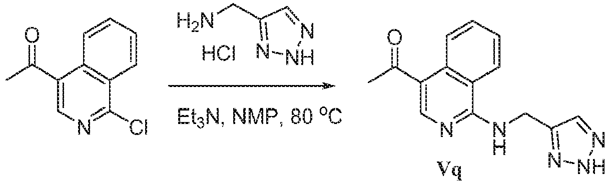

To a solution of 0.6 g (2.92 mmol, 1.0 eq.) of 1-(1-chloroisoquinolin-4-yl)ethan-1-one in 8 mL of NMP was added 1.8 mL (13.2 mmol, 4.5 eq.) of triethylamine followed by 0.78 g (5.8 mmol, 2.0 eq.) of (2H-1,2,3-triazol-4-yl)methanamine hydrochloride and the mixture was heated at 90 °C for 3 h. The mixture was allowed to cool to room temperature, diluted with 40 mL of water and extracted with 3 x 30 mL of 10% methanol in methylene chloride. The combined organic extracts were dried (Na₂SO₄), filtered and the solvent was removed *in vacuo.* The residue was purified column chromatography (SiO₂, eluting with a linear gradient of 0-3% of methanol in methylene chloride) to provide 0.4 g (1.49 mmol, 51%) of 1-(1-(((2H-1,2,3-triazol-4-yl)methyl)amino)isoquinolin-4-yl)ethan-1-one **(Vq).** LCMS: *m*/*z* found 268.4 [M+H]⁺; ¹H NMR (300 MHz, DMSO-d₆) δ 14.76 (bs, 1H), 9.07 (d, 1H), 8.77-8.81 (m, 2H), 8.33 (d, 1H), 7.71-7.77 (m, 2H), 7.54-7.59 (m, 1H), 4.88 (d, 2H), 2.59 (s, 3H).

### N-((2H-1,2,3-Triazol-4-yl)methyl)-4-(1-(methylamino)ethyl)isoquinolin-1-amine (VIao)

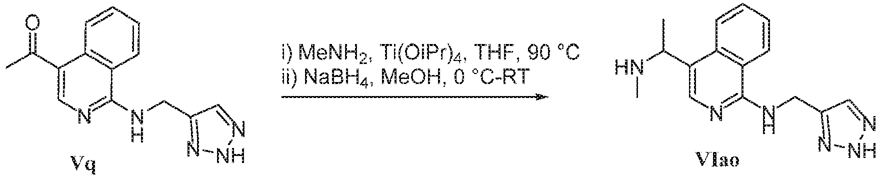

To a solution of 0.35 g (1.31 mmol, 1.0 eq.) of 1-(1-(((2*H*-1,2,3-triazol-4-yl)methyl) amino)isoquinolin-4-yl)ethan-1-one **(Vq)** in 3 mL of THF in a sealed tube under a nitrogen atmosphere was added 3 mL of titanium isopropoxide followed by 5 mL (10 mmol, 7.6 eq.) of a 2 M solution of methylamine in THF and the mixture was heated at 90 °C for 16 h. The mixture was allowed to cool to room temperature and then further cooled to 0 °C. The mixture was diluted with 3 mL of methanol and 0.10 g (2.6 mmol, 2.0 eq.) of sodium borohydride was added. The mixture was then allowed to warm to room temperature and stirred for 2 h. The reaction was quenched by the addition of 30 mL of water and filtered through CELITE^{®}. The pad was washed with 30 mL of ethyl acetate and the filtrate was extracted with 50 mL of ethyl acetate. The organic extracts were washed with 50 mL of brine, dried (Na₂SO₄), filtered and the solvent was removed *in vacuo* to provide 0.4 g of *N-*((2*H-*1,2,3-triazol-4-yl)methyl)-4-(1-(methylamino)ethyl)isoquinolin-1-amine **(VIao).** LCMS: *m*/*z* found 283.4 [M+H]⁺.

### 1-(1-(1-(((2H 1,2,3-Triazol-4-yl)methyl)amino)isoquinolin-4-yl)ethyl)-3-(3-chloro-4-fluorophenyl)-1-methylurea (Compounds 261 & 262)

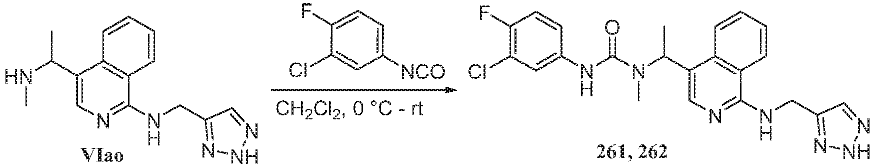

Racemic 1-(1-(1-(((2*H*-1,2,3-triazol-4-yl)methyl)amino)isoquinolin-4-yl)ethyl)-3-(3-chloro-4-fluorophenyl)-1-methylurea was synthesized in a similar manner as described above from *N*-((2*H*-1,2,3-triazol-4-yl)methyl)-4-(1-(methylamino)ethyl)isoquinolin-1-amine **(VIao)** and 2-chloro-1-fluoro-4-isocyanatobenzene. The enantiomers were subsequently separated by SFC, Column: (R,R) Whelk-01 (30 x 250 mm) 5 µ, 65% CO₂:MeOH, flow rate 100 g/min.

1-(1-(1-(((2*H*-1,2,3-Triazol-4-yl)methyl)amino)isoquinolin-4-yl)ethyl)-3-(3-chloro-4-fluorophenyl)-1-methylurea: Enantiomer I **(Compound 261).** LCMS: *m*/*z* found 454.1/456.1 [M+H]⁺, RT = 6.27 min (Method: A); ¹H NMR (400 MHz, DMSO-d₆) δ 14.72 (bs, 1H), 8.43 (bs, 1H), 8.29 (d, 1H), 7.91-8.00 (m, 2H), 7.82-7.88 (m, 2H), 7.65-7.69 (m, 2H), 7.48-7.53 (m, 2H), 7.31 (t, 1H), 5.98-6.03 (m, 1H), 4.72-4.83 (m, 2H), 2.54 (s, 3H), 1.53 (d, 3H); Chiral analytical SFC: RT = 4.58 min, Column: (R,R) Whelk-01 (250 x 4.6 mm), 5 µ, 60% CO₂:MeOH, Flow rate = 3.0 mL/min.

1-(1-(1-(((2*H*-1,2,3-Triazol-4-yl)methyl)amino)isoquinolin-4-yl)ethyl)-3-(3-chloro-4-fluorophenyl)-1-methylurea: Enantiomer II **(Compound 262).** LCMS: *m*/*z* found 454.1/456.1 [M+H]⁺, RT = 6.25 min (Method: A); ¹H NMR (400 MHz, DMSO-d₆) δ 14.72 (bs, 1H), 8.43 (bs, 1H), 8.29 (d, 1H), 7.91-8.00 (m, 2H), 7.82-7.88 (m, 2H), 7.65-7.69 (m, 2H), 7.48-7.53 (m, 2H), 7.31 (t, 1H), 5.98-6.03 (m, 1H), 4.72-4.83 (m, 2H), 2.54 (s, 3H), 1.53 (d, 3H); Chiral analytical SFC: RT = 6.10 min, Column: (R,R) Whelk-01 (250 x 4.6 mm), 5 µ, 60% CO₂:MeOH, Flow rate = 3.0 mL/min.

### 4-Bromoisoquinoline 2-oxide

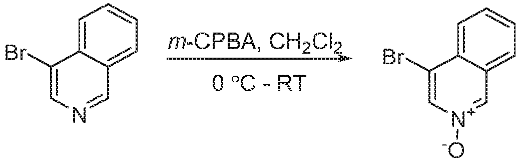

To a solution of 2.0 g (9.61 mmol, 1.0 eq.) of 4-bromoisoquinoline in 50 mL of methylene chloride at 0 °C was added 2.49 g (14.4 mmol, 1.5 eq.) of 77% *m-*chloroperbenzoic acid. The mixture was allowed to warm to room temperature and stirred for 4 h. An additional portion of 0.30 g (1.74 mmol, 0.2 eq.) of 77% m-chloroperbenzoic acid was added and the mixture was stirred at room temperature for an additional 72 h. The reaction was then quenched with 50 mL of saturated aqueous sodium bicarbonate solution and extracted with 3 x 80 mL of methylene chloride. The combined organic extracts were dried (Na₂SO₄), filtered and the solvent was removed *in vacuo* to provide 2.13 g (9.6 mmol, 99 %) of 4-bromoisoquinoline 2-oxide. ¹H NMR (400 MHz, CDCl₃) δ 8.74 (m, 1H), 8.44 (d, 1H), 8.04-8.13 (m, 1H), 7.63-7.78 (m, 3H).

### 4-Bromo-1-(1,2,4-triazol-1-yl)isoquinoline

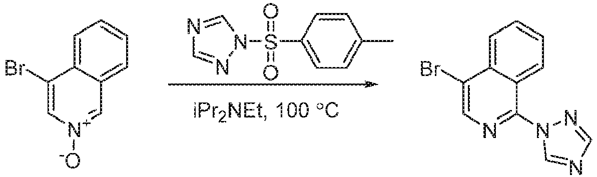

To 0.3 g (1.34 mmol, 1.0 eq.) of 4-bromoisoquinoline 2-oxide in a sealed tube was added 0.7 mL (4.02 mmol, 3.0 eq.) of *N,N-*diisopropylethylamine followed by 0.45 g (2.01 mmol, 1.5 eq.) of 1-(p-tolylsulfonyl)-1,2,4-triazole. The vessel was sealed and heated at 100 °C for 16 h. The mixture was allowed to cool to room temperature, diluted with 4 ml of methylene chloride and purified by flash chromatography (SiO₂, eluting with a gradient of 15-100% ethyl acetate/hexanes) to provide 0.13 g (0.48 mmol, 36%) of 4-bromo-1-(1,2,4-triazol-1-yl)isoquinoline. ¹H NMR (400 MHz, CDCl₃) δ 9.04 (s, 1H), 8.92 (m, 1H), 8.60 (s, 1H), 8.23-8.33 (m, 2H), 7.92 (m, 1H), 7.78 (m, 1H).

### 1-[1-(1,2,4-Triazol-1-yl)-4-isoquinolyl]ethenone (Vr)

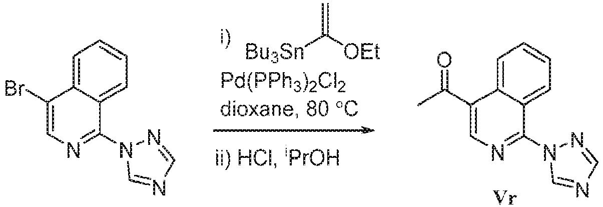

A solution of 0.14 g (0.51 mmol, 1.0 eq.) of 4-bromo-1-(1,2,4-triazol-1-yl)isoquinoline in 4 mL of 1,4-dioxane in a sealed tube was degassed with nitrogen and 0.22 mL (0.66 mmol, 1.3 eq.) of tributyl(1-ethoxyvinyl)stannane was added followed by 29 mg (0.04 mmol, 0.08 eq.) of dichlorobis(triphenylphosphine)palladium(II). The vessel was sealed, and the mixture was heated at 80 °C for 70 min. The mixture was allowed to cool to room temperature, diluted with 25 mL of ethyl acetate and filtered through CELITE^{®}. The pad was washed with 15 mL of ethyl acetate and the combined filtrate was concentrated *in vacuo* and purified by flash chromatography (SiO₂, eluting with a gradient of 10%-60% ethyl acetate/hexanes) to provide 0.13 g (0.49 mmol, 96%) of 4-(1-ethoxyvinyl)-1-(1,2,4-triazol-1-yl)isoquinoline. ¹H NMR (400 MHz, CDCl₃) δ 9.02 (s, 1H), 8.81 (m, 1H), 8.44 (s, 1H), 8.20-8.28 (m, 2H), 7.79 (m, 1H), 7.69 (m, 1H), 4.62 (d, 1H), 4.50 (d, 1H), 4.07 (q, 2H), 1.45 (t, 3H). The purified 4-(1-ethoxyvinyl)-1-(1,2,4-triazol-1-yl)isoquinoline was dissolved in 10 mL of isopropanol and 0.73 mL (1.46 mmol, 3.0 eq.) of 2 M aqueous HCl was added. The mixture was stirred at room temperature for 50 min and the volatiles were removed *in vacuo.* The resulting solid was dried under high vacuum to provide 0.11 g (0.48 mmol, 98%) of 1-[1-(1,2,4-triazol-1-yl)-4-isoquinolyl]ethenone **(Vr).** ¹H NMR (400 MHz, DMSO-d₆) δ 9.41 (d, 1H), 9.11 (s, 1H), 8.77 (m, 1H), 8.67 (m, 1H), 8.49 (d, 1H), 8.03 (m, 1H), 7.86 (m, 1H), 2.83 (s, 3H).

### 1-(1-(1H-1,2,4-Triazol-1-yl)isoquinolin-4-yl)-N-methylethan-1-amine (VIap)

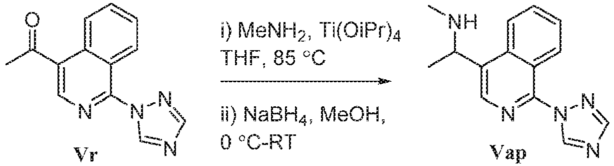

To a mixture of 56 mg (0.24 mmol, 1.0 eq.) of 1-[1-(1,2,4-triazol-1-yl)-4-isoquinolyl]ethanone **(Vr)** in 1.2 mL (2.4 mmol, 10.0 eq.) of a 2 M solution of methylamine in THF in a microwave vial was added 0.28 mL (0.94 mmol, 4.0 eq.) of tetraisopropoxytitanium. The mixture was subjected to microwave irradiation, maintaining a reaction temperature of 85 °C for 30 min. The mixture was allowed to cool to room temperature, further cooled to 0 °C, diluted 0.7 mL of methanol and 13 mg (0.35 mmol, 1.5 eq.) of sodium borohydride was added. The mixture was stirred at 0 °C for 30 min, allowed to warm to room temperature and stirred for an additional 1 h. The reaction mixture was then slowly added to 0.5 mL of a rapidly stirred brine solution and diluted with 20 mL of 9:1 *v*/*v* ethyl acetate:acetonitrile. The mixture was filtered through CELITE^{®} and the pad was washed with 15 mL of ethyl acetate. The combined filtrate was evaporated *in vacuo* and the residue was dried under high vacuum to provide 71 mg of crude 1-(1-(1H-1,2,4-triazol-1-yl)isoquinolin-4-yl)-*N*-methylethan-1-amine **(VIap).**

### 1-(1-(1-(1H-1,2,4-triazol-1-yl)isoquinolin-4-yl)ethyl)-3-(3-chloro-4-fluorophenyl)-1-methylurea (Compound 297)

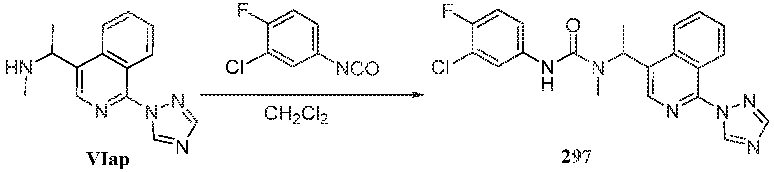

To 71 mg of crude *N-*methyl-1-[1-(1,2,4-triazol-1-yl)-4-isoquinolyl]ethanamine **(VIap)** in 2 mL of methylene chloride at 0 °C was added a solution of 31 µL (0.25 mmol) of 2-chloro-1-fluoro-4-isocyanato-benzene in 0.5 mL of methylene chloride and the mixture was stirred at 0 °C for 15 min. The mixture was diluted with 2 mL of methylene chloride and 50 µL of MeOH, loaded directly onto a pre-equilibrated silica column, and purified by flash chromatography (SiO₂, eluting with a gradient of 0.5-10% methanol/methylene chloride) to provide 12.5 mg (11% from **Vr)** of racemic 3-(3-chloro-4-fluoro-phenyl)-1-methyl-1-[1-[1-(1,2,4-triazol-1-yl)-4-isoquinolyl] ethyl] urea **(Compound** 297). LCMS: *m*/*z* found 425.2/427.2 [M+H]⁺, RT = 4.57 min (Method A); ¹H NMR (400 MHz, CDCl₃) δ 9.04 (s, 1H), 8.83 (d, 1H), 8.45 (s, 1H), 8.27 (d, 2H), 7.85 (m, 1H), 7.70 (m, 2H), 7.24 (m, 1H), 7.10 (t, 1H), 6.52 (q, 1H), 6.31 (s, 1H), 2.66 (s, 3H), 1.76 (d, 3H).

### 1-(1-(1-(1H-1,2,4-Triazol-1-yl)isoquinolin-4-yl)ethyl)-3-(3-chloro-4-fluorophenyl)-1-isobutylurea (Compounds 300, 344 & 345)

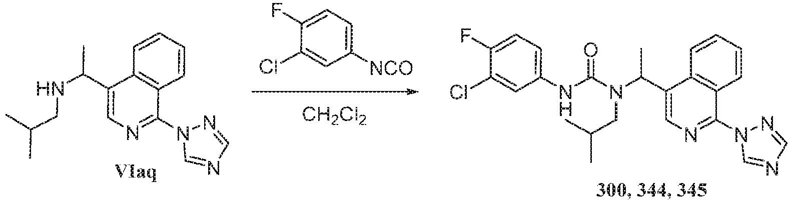

Racemic 1-(1-(1-(1*H*-1,2,4-triazol-1-yl)isoquinolin-4-yl)ethyl)-3-(3-chloro-4-fluorophenyl)-1-isobutylurea **(Compound 300)** was synthesized in an analogous manner as described above from *N*-(1-(1-(1*H*-1,2,4-triazol-1-yl)isoquinolin-4-yl)ethyl)-2-methylpropan-1-amine **(VIaq)** and 2-chloro-1-fluoro-4-isocyanatobenzene. LCMS: *m*/*z* found 467.2/469.2 [M+H]⁺, RT = 5.41 min (Method A); ¹H NMR (400 MHz, CDCl₃) δ 9.06 (s, 1H), 8.87 (m, 1H), 8.44 (d, 1H), 8.24-8.32 (m, 2H), 7.86 (m, 1H), 7.72 (m, 1H), 7.64 (m, 1H), 7.22 (m, 1H), 7.10 (t, 1H), 6.57 (q, 1H), 6.37 (s, 1H), 2.80-2.97 (m, 2H), 1.77 (d, 3H), 1.29 (m, 1H), 0.75 (d, 3H), 0.39 (d, 3H). The enantiomers were subsequently separated by SFC (Waters SFC-80), Column: Diacel IG (250 x 10 mm) 5µ, 70% CO₂:MeOH, flow rate 9 g/min.

1-(1-(1-(1*H*-1,2,4-triazol-1-yl)isoquinolin-4-yl)ethyl)-3-(3-chloro-4-fluorophenyl)-1-isobutylurea: Enantiomer I **(Compound 344).** LCMS: *m*/*z* found 467.2/469.2 [M+H]⁺, RT = 5.48 min (Method A); ¹H NMR (400 MHz, CDCl₃) δ 9.06 (s, 1H), 8.87 (m, 1H), 8.44 (d, 1H), 8.24-8.32 (m, 2H), 7.86 (m, 1H), 7.72 (m, 1H), 7.64 (m, 1H), 7.22 (m, 1H), 7.10 (t, 1H), 6.57 (q, 1H), 6.37 (s, 1H), 2.80-2.97 (m, 2H), 1.77 (d, 3H), 1.29 (m, 1H), 0.75 (d, 3H), 0.39 (d, 3H). Chiral analytical SFC: RT = 3.69 min, Column: Diacel IG (250 X 4.6 mm) 5 µ, 70% CO₂:MeOH, Flow = 3.0 g/min.

1-(1-(1-(1*H*-1,2,4-triazol-1-yl)isoquinolin-4-yl)ethyl)-3-(3-chloro-4-fluorophenyl)-1-isobutylurea: Enantiomer II **(Compound 345).** LCMS: *m*/*z* found 467.2/469.2 [M+H]⁺, RT = 5.48 min (Method A); ¹H NMR (400 MHz, CDCl₃) δ 9.06 (s, 1H), 8.87 (m, 1H), 8.44 (d, 1H), 8.24-8.32 (m, 2H), 7.86 (m, 1H), 7.72 (m,1H), 7.64 (m, 1H), 7.22 (m, 1H), 7.10 (t, 1H), 6.57 (q, 1H), 6.37 (s, 1H), 2.80-2.97 (m, 2H), 1.77 (d, 3H), 1.29 (m, 1H), 0.75 (d, 3H), 0.39 (d, 3H). Chiral analytical SFC: RT = 4.10 min, Column: Diacel IG (250 X 4.6 mm) 5 µ, 70% CO₂:MeOH, Flow = 3.0 g/min.

### 1-(1-(1-(1H-1,2,4-Triazol-1-yl)isoquinolin-4-yl)ethyl)-3-(4-fluorophenyl)-1-isobutylurea (Compound 301)

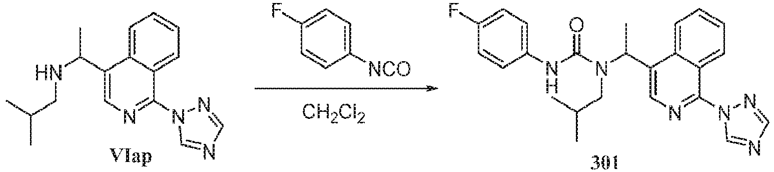

Racemic 1-(1-(1-(1*H*-1,2,4-triazol-1-yl)isoquinolin-4-yl)ethyl)-3-(4-fluorophenyl)-1-isobutylurea **(Compound 301)** was synthesized in a similar manner as described above from *N-*(1-(1-(1*H-*1,2,4-triazol-1-yl)isoquinolin-4-yl)ethyl)-2-methylpropan-1-amine **(VIaq)** and 1-fluoro-4-isocyanato-benzene. LCMS: *m*/*z* found 433.2 [M+H]+, RT = 4.76 min (Method A); ¹H NMR (400 MHz, DMSO-d₆) δ 9.06 (s, 1H), 8.86 (dd, 1H), 8.44 (d, 1H), 8.32 (d, 1H), 8.26 (s, 1H), 7.85 (ddd, 1H), 7.72 (ddd, 1H), 7.34-7.44 (m, 2H), 7.00-7.10 (m, 2H), 6.59 (q, 1H), 6.35 (s, 1H), 2.81-2.97 (m, 2H), 1.77 (d, 3H), 1.30 (td, 1H), 0.76 (d, 3H), 0.37 (d, 3H).

### 1-(p-Tolylsulfonyl)triazole

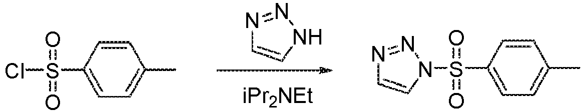

To a solution of 0.8 g (11.6 mmol, 1.0 eq.) of 1*H*-triazole in 80 mL of methylene chloride under a nitrogen atmosphere was added 3.09 g (16.2 mmol, 1.4 eq.) of 4-methylbenzenesulfonyl chloride followed by 8.7 mL (49.8 mmol, 4.3 eq.) of *N,N-*diisopropylethyl amine. The mixture was degassed with nitrogen and then stirred at room temperature for 16 h. The volatiles were removed *in vacuo,* and the residue was purified by flash chromatography (SiO₂, eluting with a linear gradient of 0-30% ethyl acetate/methylene chloride) to provide 2.0 g (8.9 mmol, 77%) of 1-(p-tolylsulfonyl)triazole. ¹H NMR (400 MHz, CDCl₃) δ 8.16 (d, 1H), 7.94-8.04 (m, 2H), 7.70 (d, 1H), 7.35-7.43 (m, 2H), 2.45 (s, 3H).

### 4-Bromo-1-(1H-1,2,3-triazol-1-yl)isoquinoline

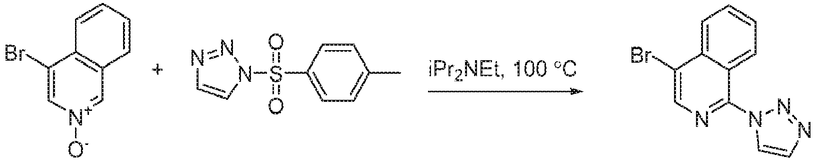

A mixture of 0.54 mL (3.08 mmol, 3.0 eq.) of *N,N*-diisopropylethylamine, 0.34 g (1.54 mmol, 1.5 eq.) of 1-(*p*-tolylsulfonyl)triazole and 0.23 g (1.03 mmol, 1.0 eq.) in a sealed tube was heated at 100 °C for 16 h. Upon cooling to room temperature, the mixture was diluted with 3 mL of methylene chloride and purified by flash chromatography (SiO₂, eluting with a gradient of 15-100 % ethyl acetate/hexanes) to provide 0.1 g (0.12 mmol, 34%) of 4-bromo-1-(1*H*-1,2,3-triazol-1-yl)isoquinoline. ¹H NMR (400 MHz, CDCl₃) δ 8.86 (m, 1H), 8.65 (s, 1H), 8.48 (m, 1H), 8.32 (m, 1H), 7.86-78.02 (m, 2H), 7.75-7.86 (m, 1H).

### 1-[1-(1,2,3-Triazol-1-yl)-4-isoquinolyl]ethan-1-one (Vs)

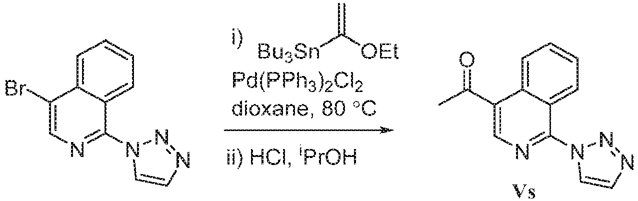

A solution of 0.13 g (0.46 mmol, 1.0 eq.) of 4-bromo-1-(1*H*-1,2,3-triazol-1-yl)isoquinoline in 4 mL of 1,4-dioxane in a sealed tube was degassed with nitrogen and 0.20 mL (0.60 mmol, 1.3 eq.) of tributyl(1-ethoxyvinyl)stannane was added followed by 26 mg (0.04 mmol, 0.09 eq.) of dichlorobis(triphenylphosphine)palladium(II). The vessel was sealed, and the mixture was heated at 80 °C for 70 min. The mixture was allowed to cool to room temperature, diluted with 25 mL of ethyl acetate and filtered through CELITE^{®}. The pad was washed with 15 mL of ethyl acetate and the combined filtrate was concentrated *in vacuo* and purified by flash chromatography (SiO₂, eluting with a gradient of 10%-60% ethyl acetate/hexanes) to provide 0.11 g (89%) of 4-(1-ethoxyvinyl)-1-(1,2,3-triazol-1-yl)isoquinoline. ¹H NMR (400 MHz, CDCl₃) δ 8.76 (m, 1H), 8.44-8.51 (m, 2H), 8.26 (m, 1H), 7.93 (d, 1H), 7.81 (m, 1H), 7.71 (m, 1H), 4.63 (d, 1H), 4.52 (d, 1H), 4.08 (q, 2H), 1.45 (t, 3H). The purified 4-(1-ethoxyvinyl)-1-(1,2,3-triazol-1-yl)isoquinoline was dissolved in 10 mL of isopropanol and 0.61 mL (1.22 mmol, 3.0 eq.) of 2 M aqueous HCl was added. The mixture was stirred at room temperature for 3 h and the volatiles were removed *in vacuo.* The resulting solid was dried under high vacuum to provide 0.11 g of 1-[1-(1,2,3-triazol-1-yl)-4-isoquinolyl]ethenone (Vs). ¹H NMR (400 MHz, DMSO-d₆) δ 9.16 (s, 1H), 8.92 (d, 1H), 8.78 (m, 1H), 8.37 (m, 1H), 8.14 (d, 1H), 8.05 (m, 1H), 7.88 (m, 1H), 2.85 (s, 3H).

### 1-(1-(1H-1,2,3-Triazol-1-yl)isoquinolin-4-yl)-N-methylethan-1-amine (VIar)

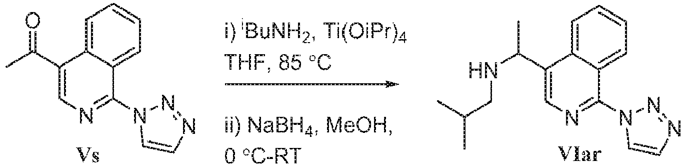

To a mixture of 37 mg (0.16 mmol, 1.0 eq.) of 1-[1-(triazol-1-yl)-4-isoquinolyl]ethenone (Vs) and 17 µL (0.17 mmol, 1.1 eq.) of 2-methylpropan-1-amine in 0.85 mL of THF in a microwave vial was added 0.18 mL (0.62 mmol, 4.0 eq.) of tetraisopropoxytitanium. The mixture was subjected to microwave irradiation, maintaining a reaction temperature of 85 °C for 30 min. The mixture was allowed to cool to room temperature, further cooled to 0 °C, diluted 0.7 mL of methanol and 9 mg (0.23 mmol, 1.5 eq.) of sodium borohydride was added. The mixture was stirred at 0 °C for 30 min, allowed to warm to room temperature and stirred for an additional 1 h. The reaction mixture was then slowly added to 0.5 mL of a rapidly stirred brine solution and diluted with 20 mL of 9:1 *v*/*v* ethyl acetate:acetonitrile. The mixture was filtered through CELITE^{®} and the pad was washed with 15 mL of ethyl acetate. The combined filtrate was evaporated *in vacuo* and the residue was dried under high vacuum to provide 50 mg of crude *N*-(1-(1-(1*H*-1,2,3-triazol-1-yl)isoquinolin-4-yl)ethyl)-2-methylpropan-1-amine **(VIar).** ¹H NMR (400 MHz, Methanol-*d*₄) δ 8.66 (m, 2H), 8.49 (d, 1H), 8.29 (m, 1H), 8.03 (t, 1H), 7.94 (m, 1H), 7.77 (m, 1H), 4.74 (q, 1H), 3.38 - 3.28 (m, 3H), 2.53 (m, 1H), 2.32 (m, 1H), 1.80 (m, 1H), 1.60 (m, 3H), 0.93 (m, 6H).

### 1-(1-(1-(1H-1,2,3-Triazol-1-yl)isoquinolin-4-yl)ethyl)-3-(3-chloro-4-fluorophenyl)-1-isobutylurea (Compound 355)

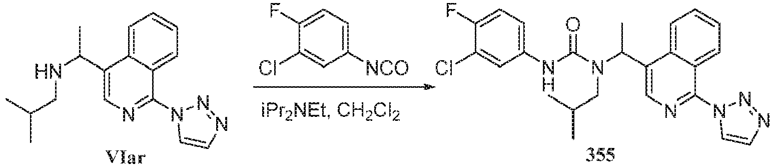

Racemic 1-(1-(1-(1*H*-1,2,3-triazol-1-yl)isoquinolin-4-yl)ethyl)-3-(3-chloro-4-fluorophenyl)-1-isobutylurea **(Compound 355)** was synthesized in a similar manner as described above from *N*-(1-(1-(1*H*-1,2,3-triazol-1-yl)isoquinolin-4-yl)ethyl)-2-methylpropan-1-amine (**VIar**) and 2-chloro-1-fluoro-4-isocyanato-benzene. LCMS: m/z found 467.2/469.2 [M+H]+, RT = 5.54 min (Method A); ¹H NMR (400 MHz, DMSO-d₆) δ 8.88 (d, 1H), 8.69 (s, 1H), 8.52 (s, 1H), 8.36 (d, 1H), 8.23 (m 1H), 8.11 (d, 1H), 8.01 (m, 1H), 7.77-7.86 (m, 2H), 7.50 (m, 1H), 7.34 (t, 1H), 6.37 (q, 1H), 3.02 (d, 2H), 1.73 (d, 3H), 1.21-1.36 (m, 1H), 0.63 (d, 3H), 0.38 (d, 3H).

### 1-(1-(1-(1H-1,2,3-Triazol-1-yl)isoquinolin-4-yl)ethyl)-3-(3-chloro-4-fluorophenyl)-1-methylurea (Compounds 354, 424 & 425)

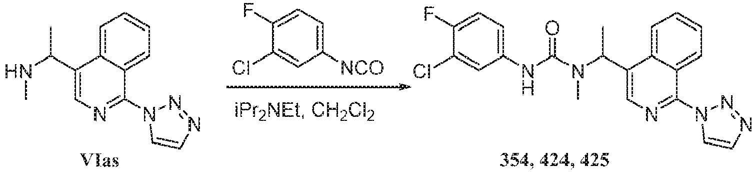

Racemic 1-(1-(1-(1*H*-1,2,3-triazol-1-yl)isoquinolin-4-yl)ethyl)-3-(3-chloro-4-fluorophenyl)-1-methylurea **(Compound 354)** was synthesized in an analogous manner as described above from 1-(1-(1*H*-1,2,3-triazol-1-yl)isoqumolm-4-yl)-*N*-methylethan-1-amine **(VIas)** and 2-chloro-1-fluoro-4-isocyanatobenzene. LCMS: *m*/*z* found 425.1/427.1 [M+H]⁺, RT = 4.78 min. The enantiomers were subsequently separated by SFC (Waters SFC-80), Column: Chiralcel OX-3 (250 x 10 mm) 5µ, 70% CO₂:MeOH, Flow rate 9 g/min to provide 13.8 mg and 14.1 mg of the resolved enantiomers.

1-(1-(1-(1*H*-1,2,3-Triazol-1-yl)isoquinolin-4-yl)ethyl)-3-(3-chloro-4-fluorophenyl)-1-methylurea - Enantiomer I **(Compound 424).** LCMS: *m*/*z* found 425.2/427.1 [M+H]+, RT = 4.48 min (Method A); ¹H NMR (400 MHz, DMSO-*d*₆) δ 8.86 (d, 1H), 8.65 (d, 1H), 8.56 (s, 1H), 8.28 (d, 1H), 8.17-8.24 (m, 1H), 8.11 (d, 1H), 7.99 (m, 1H), 7.88 (m, 1H), 7.81 (m, 1H), 7.52 (m, 1H), 7.33 (t, 1H), 6.36 (q, 1H), 2.67 (s, 3H), 1.70 (d, 3H); Chiral analytical SFC: RT = 2.70 min, Column: Chiralcel OX-3 (150 x 4.6 mm) 3 µ, 70% CO₂:MeOH, Flow = 3.0 g/min.

1-(1-(1-(1*H*-1,2,3-Triazol-1-yl)isoquinolin-4-yl)ethyl)-3-(3-chloro-4-fluorophenyl)-1-methylurea - Enantiomer II **(Compound 425).** LCMS: *m*/*z* found 425.2/427.1 [M+H]+, RT = 4.48 min (Method A); ¹H NMR (400 MHz, DMSO-d₆) δ 8.86 (d, 1H), 8.65 (d, 1H), 8.56 (s, 1H), 8.28 (d, 1H), 8.17-8.24 (m, 1H), 8.11 (d, 1H), 7.99 (m, 1H), 7.88 (m, 1H), 7.81 (m, 1H), 7.52 (m, 1H), 7.33 (t, 1H), 6.36 (q, 1H), 2.67 (s, 3H), 1.70 (d, 3H); Chiral analytical SFC: RT = 3.70 min, Column: Chiralcel OX-3 (150 x 4.6 mm) 3 µ, 70% CO₂:MeOH, Flow = 3.0 g/min.

### 4-Bromo-1-((1-methyl-1H-1,2,4-triazol-3-yl)methoxy)isoquinoline (IVb)

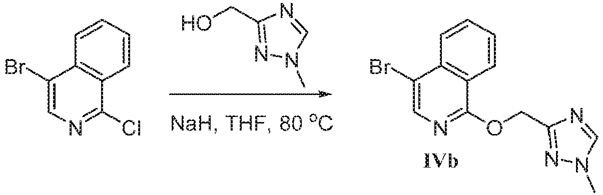

To a solution of 0.61 g (5.37 mmol, 1.3 eq.) of (1-methyl-1*H*-1,2,4-triazol-3-yl)methanol in 25 mL of THF at 0 °C under a nitrogen atmosphere was added 0.25 g (6.19 mmol, 1.5 eq.) of a 60% dispersion of sodium hydride in mineral oil. The mixture was stirred at 0 °C for 20 min and 1.0 g (4.13 mmol, 1.0 eq) 4-bromo-1-chloroisoquinoline was added. The mixture was then heated at 80 °C for 6 h. On cooling to room temperature, the reaction was quenched with 30 mL of ice-cold water and extracted with 3 x 50 mL of ethyl acetate. The combined organic extracts were washed with 40 mL of brine, dried (Na₂SO₄), filtered and the solvent was removed *in vacuo.* The residue was purified by flash chromatography (SiO₂, eluting with a linear gradient of 0-10% ethyl acetate/petroleum ether) to provide 0.9 g (2.82 mmol, 68%) of 4-bromo-1-((1-methyl-1*H*-1,2,4-triazol-3-yl)methoxy)isoquinoline **(IVb).** LCMS: *m*/*z* found 319.3/321.3 [M+H]⁺, RT = 1.87 min; ¹H NMR (400 MHz, CDCl₃) δ 8.30-8.32 (m, 1H), 8.20 (s, 1H), 8.04-8.06 (m, 2H), 7.74-7.78 (t, 1H), 7.26-7.59 (t, 1H), 5.63 (s, 2H), 3.94 (s, 3H).

### 1-(1-((1-Methyl-1H-1,2,4-triazol-3-yl)methoxy)isoquinolin-4-yl)ethanone (Vt)

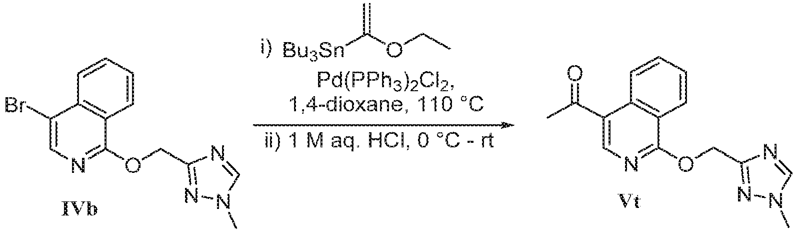

To a solution of 0.90 g (2.8 mmol, 1.0 eq.) of 4-bromo-1-((1-methyl-1*H*-1,2,4-triazol-3-yl)methoxy)isoquinoline **(IVb)** in 3 mL of 1,4-dioxane was added 2.6 g (7.0 mmol, 2.5 eq.) of tributyl(1-ethoxyvinyl)tin. The mixture was degassed by purging with argon gas for 5 min and 0.10 g (0.14 mmol, 0.05 eq.) of bis(triphenylphosphine)palladium(II) dichloride was added. The mixture was then heated at 110 °C under an argon atmosphere for 16 h. The mixture was allowed to cool to room temperature and further cooled to 0 °C. The mixture was then diluted with 15 mL of 1 M aqueous HCl and the resulting solution stirred at room temperature for 3 h. The mixture was basified with 40 mL of saturated sodium bicarbonate solution and filtered through a CELITE^{®} pad. The filtrate was extracted with 3 x 50 mL of ethyl acetate and the combined organic extracts were washed with 30 mL of brine, dried (Na₂SO₄), filtered and the solvent was removed *in vacuo.* The residue was purified by flash chromatography (SiO₂, eluting with a linear gradient 0-10% methanol/methylene chloride) to provide 0.68 g (2.4 mmol, 85%) of 1-(1-((1-methyl-1*H*-1,2,4-triazol-3-yl)methoxy)isoquinolin-4-yl)ethanone **(Vt).** LCMS: *m*/*z* found 283.1 [M+H]⁺, RT = 1.58 min; ¹H NMR (400 MHz, CDCl₃) δ 9.02 (d, 1H), 8.73 (s, 1H), 8.34-8.37 (m, 1H), 8.06 (s, 1H), 7.75-7.78 (m, 1H), 7.54-7.56 (m, 1H), 5.73 (s, 2H), 3.95 (s, 3H), 2.84 (s, 3H).

### N Methyl-1-(1-((1-methyl-1H 1,2,4-triazol-3-yl)methoxy)isoquinolin-4-yl)ethan-1-amine (VIat)

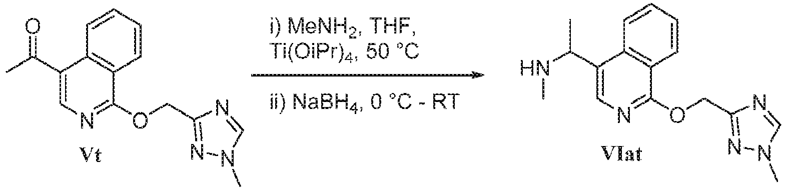

To a solution of 0.68 g (2.4 mmol, 1.0 eq.) of 1-(1-((1-methyl-1H-1,2,4-triazol-3-yl)methoxy)isoquinolin-4-yl)ethanone **(Vt)** in 25 mL of THF in a sealed tube under a nitrogen atmosphere was added 6 mL (12 mmol, 5 eq.) of a 2 M solution of methylamine in THF followed by 6.8 mL of titanium isopropoxide and the mixture was heated at 50 °C for 16 h. The mixture was allowed to cool to room temperature and then further cooled to 0 °C. The mixture was diluted with 2 mL of methanol and 0.27 g (7.2 mmol, 3.0 eq.) of sodium borohydride was added. The mixture was then allowed to warm to room temperature and stirred for 2 h. The reaction was quenched by the addition of 30 mL of water and filtered through CELITE^{®}. The pad was washed with 10 mL of ethyl acetate and the filtrate was extracted with 3 x 50 mL of ethyl acetate. The combined organic extracts were dried (Na₂SO₄), filtered and the solvent was removed *in vacuo.* The residue was purified by chromatography (SiO₂, eluting with a linear gradient of 0-10% methanol/methylene chloride) to provide 0.37 g (1.24 mmol, 51%) of *N*-methyl-1-(1-((1-methyl-1*H*-1,2,4-triazol-3-yl)methoxy)isoquinolin-4-yl)ethanamine **(VIat).** LCMS: *m*/*z* found 298.1 [M+H]⁺, RT = 1.15 min; ¹H NMR (400 MHz, CDCl₃): δ 8.92 (bs, 1H), 8.48 (s, 1H), 8.29 (s, 1H), 8.23 (d, 2H), 7.86-7.92 (m, 1H), 7.68-7.73 (m, 1H), 5.55 (s, 2H), 4.97-5.01 (m, 1H), 3.87 (s, 3H), 2.53 (s, 3H), 1.64 (d, 3H).

### 3-(3-Chloro-4-fluorophenyl)-1-methyl-1-(1-(1-((1-methyl-1H 1,2,4-triazol-3-yl)methoxy)isoquinolin-4-yl)ethyl)urea (Compounds 132 & 133)

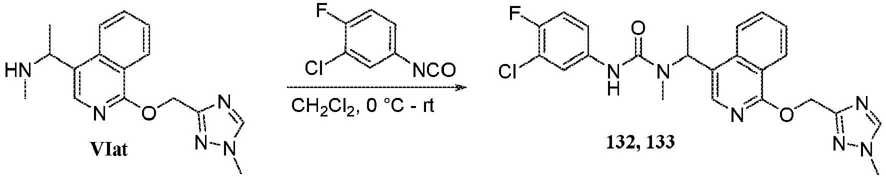

Racemic 3-(3-chloro-4-fluorophenyl)-1-methyl-1-(1-(1-((1-methyl-1*H*-1,2,4-triazol-3-yl)methoxy)isoquinolin-4-yl)ethyl)urea was synthesized in a similar manner as described above from *N-*methyl-1-(1-((1-methyl-1*H*-1,2,4-triazol-3-yl)methoxy)isoquinolin-4-yl)ethanamine **(VIat)** and 2-chloro-1-fluoro-4-isocyanatobenzene. The enantiomers were subsequently separated by SFC, Column: Chiralpak AD-H (30 × 250 mm) 5 µ, 70% CO₂:MeOH, flow rate 90 g/min.

3-(3-Chloro-4-fluorophenyl)-1-methyl-1-(1-(1-((1-methyl-1*H*-1,2,4-triazol-3-yl)methoxy)isoquinolin-4-yl)ethyl)urea: Enantiomer I **(Compound 132)**. LCMS: *m*/*z* found 469.3/471.3 [M+H]⁺, RT = 6.73 min (Method: A); ¹H NMR (400 MHz, DMSO-d₆) δ 8.48 (bs, 2H), 8.18 (d, 1H), 8.11 (bs, 1H), 7.99 (d, 1H), 7.79-7.87 (m, 2H), 7.61-7.65 (m, 1H), 7.49-7.53 (m, 1H), 7.32 (t, 1H), 6.11-6.15 (m, 1H), 5.51-5.58 (m, 2H), 3.88 (s, 3H), 2.57 (s, 3H), 1.59 (d, 3H); Chiral analytical SFC: RT = 1.49 min, Column: Chiralpak AD-3 (150 × 4.6 mm), 3 µ, 60% CO₂:MeOH, Flow rate = 3.0 mL/min.

3-(3-Chloro-4-fluorophenyl)-1-methyl-1-(1-(1-((1-methyl-1*H*-1,2,4-triazol-3-yl)methoxy)isoquinolin-4-yl)ethyl)urea: Enantiomer II **(Compound 133)**. LCMS: *m*/*z* found 469.3/471.3 [M+H]⁺, RT = 6.69 min (Method: A); ¹H NMR (400 MHz, DMSO-d₆) δ 8.48 (bs, 2H), 8.18 (d, 1H), 8.11 (bs, 1H), 7.99 (d, 1H), 7.79-7.87 (m, 2H), 7.61-7.65 (m, 1H), 7.49-7.53 (m, 1H), 7.32 (t, 1H), 6.11-6.15 (m, 1H), 5.51-5.58 (m, 2H), 3.88 (s, 3H), 2.57 (s, 3H), 1.59 (d, 3H); Chiral analytical SFC: RT = 2.74 min, Column: Chiralpak AD-3 (150 × 4.6 mm), 3 µ, 60% CO₂:MeOH, Flow rate = 3.0 mL/min.

### 3-((1-(1-((1-Methyl-1H-1,2,4-triazol-3-yl)methoxy)isoquinolin-4-yl)ethyl)amino)propan-1-ol (VIau)

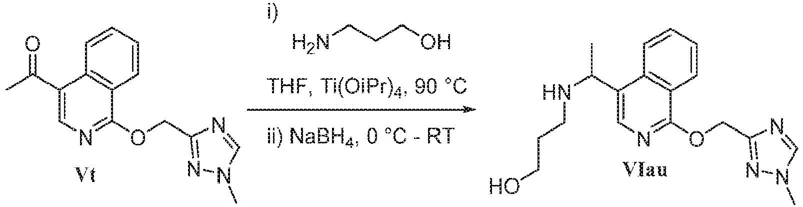

To a solution of 0.22 g (0.78 mmol, 1.0 eq.) of 1-(1-((1-methyl-1*H*-1,2,4-triazol-3-yl)methoxy)isoquinolin-4-yl)ethan-1-one **(Vt)** in 2 mL of THF in a sealed tube under a nitrogen atmosphere was added 0.12 g (1.56 mmol, 2.0 eq.) of 3-aminopropan-1-ol followed by 2 mL of titanium isopropoxide and the mixture was heated at 90 °C for 16 h. The mixture was allowed to cool to room temperature and then further cooled to 0 °C. The mixture was diluted with 2 mL of methanol and 0.06 g (1.56 mmol, 2.0 eq.) of sodium borohydride was added. The mixture was then allowed to warm to room temperature and stirred for 2 h. The reaction was quenched by the addition of 5 mL of water 40 mL and of 10% methanol in methylene chloride and filtered through CELITE^{®}. The pad was washed with 10 mL of 10% methanol in methylene chloride and the layers were separated. The organic phase was dried (Na₂SO₄), filtered and the solvent was removed *in vacuo* to provide 0.17 g of crude 3-((1-(1-((1-methyl-1*H*-1,2,4-triazol-3-yl)methoy)isoquinolin-4-yl)ethyl)amino)propan-1-ol **(VIau).** LCMS: *m*/*z* found 298.1 [M+H]⁺.

### 3-(3-Chloro-4-fluorophenyl)-1-(3-hydroxypropyl)-1-(1-(1-((1-methyl-1H 1,2,4-triazol-3-yl)methoxy)isoquinolin-4-yl)ethyl)urea (Compounds 255 & 256)

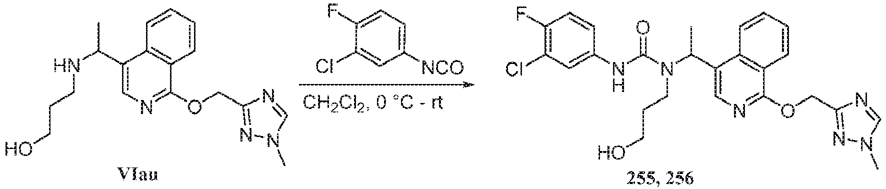

Racemic 3-(3-chloro-4-fluorophenyl)-1-(3-hydroxypropyl)-1-(1-(1-((1-methyl-1*H-*1,2,4-triazol-3-yl)methoxy)isoquinolin-4-yl)ethyl)urea was synthesized in a similar manner as described above from 3-((1-(1-((1-methyl-1*H*-1,2,4-triazol-3-yl)methoxy)isoquinofin-4-yl)ethyl)amino)propan-1-ol **(VIau)** and 2-chloro-1-fluoro-4-isocyanatobenzene. The enantiomers were subsequently separated by SFC, Column: (R,R) Whelk-01 (30 × 250 mm) 5 µ, 60% CO₂:MeOH, flow rate 100 g/min.

3-(3-Chloro-4-fluorophenyl)-1-(3-hydroxypropyl)-1-(1-(1-((1-methyl-1*H*-1,2,4-triazol-3-yl)methoxy)isoquinolin-4-yl)ethyl)urea - Enantiomer I **(Compound 255).** LCMS: *m*/*z* found 513.3/515.3 [M+H]⁺, RT = 7.51 min (Method: A); ¹H NMR (400 MHz, DMSO-d₆) δ 8.78 (bs, 1H), 8.48 (s, 1H), 8.15-8.19 (m, 2H), 7.94 (d, 1H), 7.80-7.85 (m, 2H), 7.61-7.65 (m, 1H), 7.40-7.46 (m, 1H), 7.33 (t, 1H), 6.13-6.17 (m, 1H), 5.54 (q, 2H), 5.04 (bs, 1H), 3.87 (s, 3H), 3.09-3.17 (m, 4H), 1.62 (d, 3H), 0.90-1.05 (m, 2H); Chiral analytical SFC: RT = 7.02 min, Column: (R,R) Whelk-01 (150 × 4.6 mm), 5 µ, 60% CO₂:MeOH, Flow rate = 4.0 mL/min.

3-(3-Chloro-4-fluorophenyl)-1-(3-hydroxypropyl)-1-(1-(1-((1-methyl-1*H*-1,2,4-triazol-3-yl)methoxy)isoquinolin-4-yl)ethyl)urea - Enantiomer II **(Compound 256).** LCMS: *m*/*z* found 513.3/515.3 [M+H]⁺, RT = 7.51 min (Method: A); ¹H NMR (400 MHz, DMSO-d₆) δ 8.78 (bs, 1H), 8.48 (s, 1H), 8.15-8.19 (m, 2H), 7.94 (d, 1H), 7.80-7.85 (m, 2H), 7.61-7.65 (m, 1H), 7.40-7.46 (m, 1H), 7.33 (t, 1H), 6.13-6.17 (m, 1H), 5.54 (q, 2H), 5.04 (bs, 1H), 3.87 (s, 3H), 3.09-3.17 (m, 4H), 1.62 (d, 3H), 0.90-1.05 (m, 2H); Chiral analytical SFC: RT = 9.51 min, Column: (R,R) Whelk-01 (150 × 4.6 mm), 5 µ, 60% CO₂:MeOH, Flow rate = 4.0 mL/min.

### 3-(3-Chloro-4-fluorophenyl)-1-(3-hydroxypropyl)-1-(1-(1-((1-trityl-1H-1,2,4-triazol-3-yl)methoxy) isoquinolin-4-yl)ethyl)urea (VIIc)

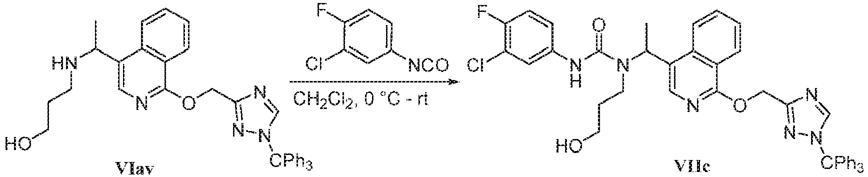

Racemic 3-(3-chloro-4-fluorophenyl)-1-(3-hydroxypropyl)-1-(1-(1-((1-trityl-1*H-*1,2,4-triazol-3-yl)methoxy) isoquinolin-4-yl)ethyl)urea was synthesized in a similar manner as described above from 3-((1-(1-((1-trityl-1*H*-1,2,4-triazol-3-yl)methoxy)isoquinolin-4-yl)ethyl)amino)propan-1-ol **(VIav)** and 2-chloro-1-fluoro-4-isocyanatobenzene. The obtained crude product was purified by MPLC (REVELERS^{®} column, eluting with a linear gradient of 0-5% methanol in methylene chloride). LCMS: *m*/*z* found 741.3/743.3 [M+H]⁺; ¹H NMR (400 MHz, DMSO-d₆) δ 8.79 (s, 1H), 8.15-8.19 (m, 3H), 7.94 (d, 1H), 7.80-7.85 (m, 2H), 7.62-7.66 (m, 1H), 7.31-7.44 (m, 11H), 7.05-7.09 (m, 6H), 6.13-6.17 (m, 1H), 5.63 (d, 1H), 5.56 (d, 1H), 5.05 (bt, 1H), 3.05-3.17 (m, 4H), 1.62 (d, 3H), 0.74-1.02 (m, 2H).

### 1-(1-(1-((1H-1,2,4-Triazol-3-yl)methoxy)isoquinolin-4-yl)ethyl)-3-(3-chloro-4-fluorophenyl)-1-(3-hydroxypropyl)urea (Compounds 259 & 260)

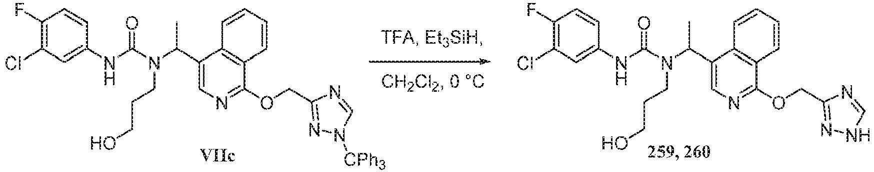

To a solution of 0.15 g (0.20 mmol, 1.0 eq.) of 3-(3-chloro-4-fluorophenyl)-1-(3-hydroxypropyl)-1-(1-(1-((1-trityl-1*H*-1,2,4-triazol-3-yl)methoxy)isoquinolin-4-yl)ethyl)urea **(VIIc)** in 3 mL of methylene chloride at 0 °C was added 14 uL (0.20 mmol, 1.0 eq.) of trifluoroacetic acid followed by 0.1 mL (0.60 mmol, 3.0 eq.) of triethylsilane and the mixture was stirred at 0 °C for 1 h. The mixture was diluted with 5 mL of water and extracted with 10 mL of methylene chloride. The organic extracts were washed with 5 mL of saturated sodium bicarbonate solution, 5 mL of brine, dried (Na₂SO₄), filtered and the solvent was removed *in vacuo.* The residue was triturated with 5 mL of 1:1 *v*/*v* diethyl ether:*n*-pentane and dried under high vacuum to provide 80 mg (0.16 mmol, 80%) of racemic1-(1-(1-((1*H*-1,2,4-triazol-3-yl)methoxy)isoquinolin-4-yl)ethyl)-3-(3-chloro-4-fluorophenyl)-1-(3-hydroxypropyl)urea. LCMS: *m*/*z* found 499.12 [M+H]⁺. The enantiomers were subsequently separated by SFC, Column: Lux Cellulose-2 (30 × 250 mm) 5 µ, 60% CO₂:MeOH, flow rate 70 g/min.

1-(1-(1-((1H-1,2,4-Triazol-3-yl)methoxy)isoquinolin-4-yl)ethyl)-3-(3-chloro-4-fluorophenyl)-1-(3-hydroxypropyl)urea - Enantiomer I **(Compound 259).** LCMS: *m*/*z* found 499.1/501.1 [M+H]⁺, RT = 6.78 min (Method: A); ¹H NMR (400 MHz, DMSO-d₆) δ 14.13 (bs, 1H), 8.79 (bs, 1H), 8.53 (s, 1H), 8.24 (s, 1H), 8.16 (s, 1H), 7.95 (d, 1H), 7.80-7.86 (m, 2H), 7.62-7.66 (m, 1H), 7.40-7.46 (m, 1H), 7.33 (t, 1H), 6.13-6.17 (m, 1H), 5.58-5.63 (m, 2H), 5.06 (bt, 1H), 3.09-3.17 (m, 4H), 1.62 (d, 3H), 0.91-1.04 (m, 2H); Chiral analytical SFC: RT = 1.68 min, Column: Chiralcel OZ-3 (150 × 4.6 mm), 3 µ, 60% CO₂:MeOH, Flow rate = 4.0 mL/min.

1-(1-(1-((1*H*-1,2,4-Triazol-3-yl)methoxy)isoquinolin-4-yl)ethyl)-3-(3-chloro-4-fluorophenyl)-1-(3-hydroxypropyl)urea - Enantiomer II **(Compound 260).** LCMS: *m*/*z* found 499.1/501.1 [M+H]⁺, RT = 6.78 min (Method: A); ¹H NMR (400 MHz, DMSO-d₆) δ 14.13 (bs, 1H), 8.79 (bs, 1H), 8.53 (s, 1H), 8.24 (s, 1H), 8.16 (s, 1H), 7.95 (d, 1H), 7.80-7.86 (m, 2H), 7.62-7.66 (m, 1H), 7.40-7.46 (m, 1H), 7.33 (t, 1H), 6.13-6.17 (m, 1H), 5.58-5.63 (m, 2H), 5.06 (bt, 1H), 3.09-3.17 (m, 4H), 1.62 (d, 3H), 0.91-1.04 (m, 2H); Chiral analytical SFC: RT = 3.04 min, Column: Chiralcel OZ-3 (150 × 4.6 mm), 3 µ, 60% CO₂:MeOH, Flow rate = 4.0 mL/min.

### 1-(1-(1-((1H-1,2,4-Triazol-3-yl)methoxy)isoquinolin-4-yl)ethyl)-3-(3-chloro-4-fluorophenyl)-1-methylurea (Compound 145 & 146)

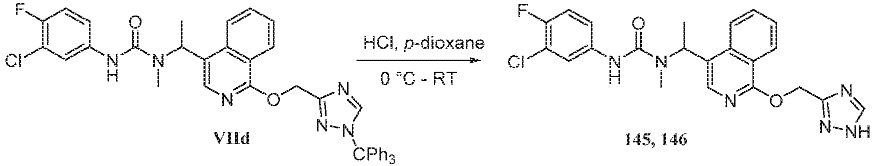

To a solution of 0.4 g (0.58 mmol, 1.0 eq.) of 3-(3-chloro-4-fluorophenyl)-1-methyl-1-(1-(1-((1-trityl-1*H*-1,2,4-triazol-3-yl)methoxy)isoquinolin-4-yl)ethyl)urea **(VIId)** in 4 mL of 1,4-dioxane was added 4 mL of a 1 M aqueous HCl solution drop wise at 0 °C. The mixture was allowed to warm to room temperature and stirred for 3 h. The mixture was then basified with saturated sodium bicarbonate solution to pH~9 and extracted with 3 × 40 mL of methylene chloride. The combined organic extracts were washed with 60 mL of brine, dried (Na₂SO₄), filtered and the solvent was removed *in vacuo.* The residue was purified by column chromatography (REVELERIS^{®} SiO₂ column, eluting with a linear gradient of 0-5% methanol in methylene chloride) to provide 120 mg (0.268 mmol, 46%) of racemic 1-(1-(1-((1*H*-1,2,4-triazol-3-yl)methoxy)isoquinolin-4-yl)ethyl)-3-(3-chloro-4-fluorophenyl)-1-methylurea. LCMS: *m*/*z* found 455.1/457.1 [M+H]⁺. The enantiomers were subsequently separated by SFC, Column: Lux Cellulose-2 (30 × 250 mm) 5 µ, 70% CO₂:MeOH, flow rate 70 g/min.

1-(1-(1-((1*H*-1,2,4-Triazol-3-yl)methoxy)isoquinolin-4-yl)ethyl)-3-(3-chloro-4-fluorophenyl)-1-methylurea - Enantiomer I **(Compound 145).** LCMS: *m*/*z* found 455.3/457.3 [M+H]⁺, RT = 6.52 min (Method: A); ¹H NMR (400 MHz, DMSO-d₆) δ 14.05 (bs, 1H), 8.47 (bs, 1H), 8.38 (bs, 1H), 8.25 (d, 1H), 8.12 (s, 1H), 7.99 (d, 1H), 7.80-7.88 (m, 2H), 7.62-7.66 (m, 1H), 7.49-7.53 (m, 1H), 7.32 (t, 1H), 6.12-6.16 (m, 1H), 5.63 (s, 2H), 2.57 (s, 3H), 1.59 (d, 3H); Chiral analytical SFC: RT = 3.26 min, Column: Chiralcel OZ-3 (150 × 4.6 mm), 3 µ, 70% CO₂:MeOH, Flow rate = 3.0 mL/min.

1-(1-(1-((1*H*-1,2,4-Triazol-3-yl)methoxy)isoquinolin-4-yl)ethyl)-3-(3-chloro-4-fluorophenyl)-1-methylurea - Enantiomer II **(Compound 146).** LCMS: *m*/*z* found 455.3/457.3 [M+H]⁺, RT = 6.51 min (Method: A); ¹H NMR (400 MHz, DMSO-d₆) δ 14.05 (bs, 1H), 8.47 (bs, 1H), 8.38 (bs, 1H), 8.25 (d, 1H), 8.12 (s, 1H), 7.99 (d, 1H), 7.80-7.88 (m, 2H), 7.62-7.66 (m, 1H), 7.49-7.53 (m, 1H), 7.32 (t, 1H), 6.12-6.16 (m, 1H), 5.63 (s, 2H), 2.57 (s, 3H), 1.59 (d, 3H); Chiral analytical SFC: RT = 5.01 min, Column: Chiralcel OZ-3 (150 × 4.6 mm), 3 µ, 70% CO₂:MeOH, Flow rate = 3.0 mL/min.

### 1-(1-Chloroisoquinolin-4-yl)-N-methylethan-1-amine

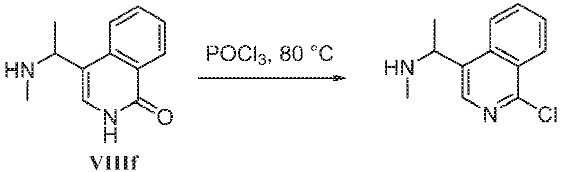

A solution of 2.5 g (12.4 mmol, 1.0 eq.) of 4-(1-(methylamino)ethyl)isoquinolin-1(2H)-one **(VIIIf)** in 25 mL of phosphorus oxychloride was heated at 80 °C for 6 h. The mixture was allowed to cool to room temperature and quenched with 100 mL of ice water, basified with 10% aqueous sodium carbonate solution and extracted with 3 × 50 mL of ethyl acetate. The combined organic extracts were washed with 30 mL of brine, dried (Na₂SO₄), filtered and the solvent was removed *in vacuo* to provide 2.0 g of 1-(1-chloroisoquinolin-4-yl)-*N*-methylethanamine. LCMS: m/z found 221.1/224.1 [M+H]⁺, RT = 1.53 min; ¹H NMR (300 MHz, DMSO-*d*₆) δ 8.46 (d, 1H), 8.38 (s, 1H), 8.32 (d, 1H), 7.88-7.94 (m, 1H), 7.79-7.85 (m, 1H), 4.32-4.39 (m, 1H), 2.38 (bs, 1H), 2.22 (s, 3H), 1.40 (d, 3H).

### Ethyl 4-(1-(methylamino)ethyl)isoquinoline-1-carboxylate (VIaw)

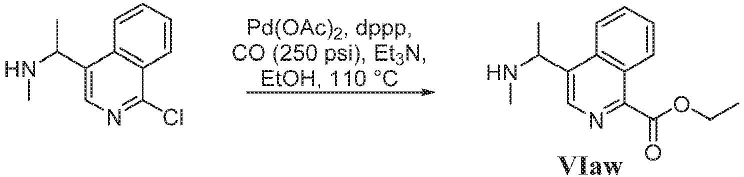

To a solution of 2.0 g (9.1 mmol, 1.0 eq.) of 1-(1-chloroisoquinolin-4-yl)-N-methylethanamine in 20 mL of ethanol in a steel pressure vessel was added 1.87 g (4.54 mmol, 0.5 eq.) of 1,3-bis(diphenylphosphino)propane (dppp) followed by 6.4 mL (45.5 mmol, 5.0 eq.) of triethylamine. The mixture was purged with nitrogen gas for 5 min and 1.02 g (4.55 mmol, 0.5 eq.) of palladium(II)acetate was added. The the vessel was sealed, pressurized with 250 psi of carbon monoxide and heated at 110 °C for 16 h. The mixture was allowed to cool to to room temperature, filtered through CELITE^{®} and the pad was washed with 20 mL of ethanol. The filtrate was concentrated *in vacuo* and the residue was purified by chromatography (REVELERIS^{®} silica gel column, eluting with a linear gradient of 0-10% methanol in methylene chloride) to provide 1.3 g (5.03 mmol, 40% from **VIIIf)** of ethyl 4-(1-(methylamino)ethyl)isoquinoline-1-carboxylate **(VIaw).** LCMS: *m*/*z* found 259.2 [M+H]⁺.

### Ethyl 4-(1-(3-(3-chloro-4-fluorophenyl)-1-methylureido)ethyl)isoquinoline-1-carboxylate (Vile)

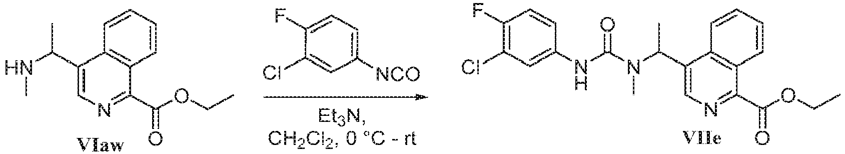

To a solution of 0.5 g (1.93 mmol, 1.0 eq) of ethyl 4-(1-(methylamino)ethyl) isoquinoline-1-carboxylate **(VIaw)** in 5 mL of methylene chloride at 0 °C under a nitrogen atmosphere was added 0.82 mL (5.81 mmol, 3.0 eq) of triethylamine followed by 0.3 g (1.74 mmol, 0.9 eq) of 2-chloro-1-fluoro-4-isocyanatobenzene. The mixture was allowed to warm to room temperature and stirred for 1 h. The mixture was diluted with 50 mL of water and extracted with 3 × 50 mL of methylene chloride. The combined organic extracts were washed with 35 mL of water, 35 mL of brine, dried (Na₂SO₄), filtered and the solvent was removed *in vacuo.* The residue was purified by chromatography (REVELERIS^{®} Silica column, eluting with a linear gradient 0-5% of methanol in methylene chloride) to provide 0.6 g (1.39 mmol, 72%) of ethyl4-(1-(3-(3-chloro-4-fluorophenyl)-1-methylureido)ethyl)isoquinoline-1-carboxylate **(Vile).** LCMS: *m*/*z* found 430.5/432.5 [M+H]⁺, RT = 2.40 min; ¹H NMR (300 MHz, DMSO-*d*₆) δ 8.68 (s, 1H), 8.52 (bs, 1H), 8.41 (d, 1H), 8.20 (d, 1H), 7.87-7.94 (m, 1H), 7.84-7.87 (m, 1H), 7.75-7.81 (m, 1H), 7.48-7.53 (m, 1H), 7.32 (t, 1H), 6.29-6.34 (m, 1H), 4.49 (q, 2H), 2.61 (s, 3H), 1.67 (d, 3H), 1.39 (t, 3H).
The enantiomers were subsequently separated by chiral SFC, Column: Chiralpak AD-H (250 × 30 mm) 5 µ, 90% CO₂/MeOH, Flow rate 100 g/min to provide 130 mg of ethyl 4-(1-(3-(3-chloro-4-fluorophenyl)-1-methylureido)ethyl)isoquinoline-1-carboxylate - Enantiomer I **(VIIe-Enantiomer I)** and 100 mg of ethyl 4-(1-(3-(3-chloro-4-fluorophenyl)-1-methylureido)ethyl)isoquinoline-l-carboxylate - Enantiomer II **(VIIe-Enantiomer II)**.

### 4-(1-(3-(3-Chloro-4-fluorophenyl)-1-methylureido)ethyl)isoquinoline-1-carboxylic acid Enantiomer I (Compound 325)

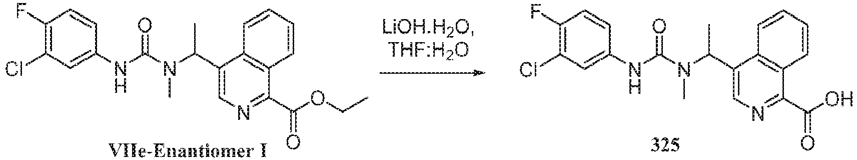

To a solution of 130 mg (0.303 mmol, 1.0 eq) of ethyl 4-(1-(3-(3-chloro-4-fluorophenyl)-1-methylureido)ethyl)isoquinoline-1-carboxylate **(VIIe-Enantiomer I)** in 2 mL of THF was added a solution of 26 mg (0.606 mmol, 2.0 eq) of lithium hydroxide monohydrate in 2 mL of water and the mixture was stirred at room temperature for 6 h. The volatiles were removed *in vacuo* and the residue resuspended in 5 ml of water and acidified to pH ~2 with 1 M HCl. The resulting precipitate was collected by filtration, washed with 5 mL of water followed by 5 ml of n-pentane and dried under high vacuum to provide 89 mg (0.221 mmol, 73%) of 4-(1-(3-(3-chloro-4-fluorophenyl)-1-methylureido)ethyl)isoquinoline-1-carboxylic acid Enantiomer I **(Compound 325).** LCMS: *m*/*z* found 402.0/404.0 [M+H]⁺; RT = 3.49 min (Method A); ¹H NMR (400 MHz, DMSO-d₆): δ 8.65 (s, 1H), 8.52-8.54 (m, 2H), 8.19 (d, 1H), 7.85-7.93 (m, 2H), 7.75-7.79 (m, 1H), 7.49-7.53 (m, 1H), 7.32 (t, 1H), 6.28-6.34 (m, 1H), 2.62 (s, 3H), 1.67 (d, 3H); Chiral analytical SFC: RT = 6.00 min, Column: Chiralpak IE, (4.6 × 250 mm) 5 µm, 70% CO₂/MeOH, Flow = 3.0 g/min.

### 4-(-(3-(3-Chloro-4-fliiorophenyl)-1-methylureido)ethyl)-N-methylisoquinoline-1-carboxamide (Compounds 316 & 317)

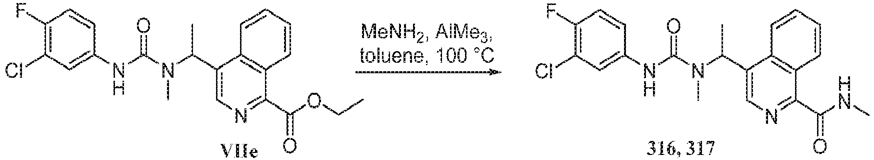

To a solution of 0.35 g (0.82 mmol, 1.0 eq.) of racemic ethyl 4-(1-(3-(3-chloro-4-fluorophenyl)-1-methylureido)ethyl)isoquinoline-1-carboxylate **(VIIe)** in 8 mL of toluene under a nitrogen atmosphere was added 0.62 mL (1.22 mmol, 1.5 eq.) of a 2 M solution of methylamine in THF followed by 0.82 mL (1.63 mmol, 2.0 eq.) of a 2 M solution of trimethylaluminium in toluene and the mixture was heated at 100 °C for 2 h. The mixture was allowed to cool to room temperature, quenched with 10 mL of saturated ammonium chloride solution and extracted with 2 × 50 mL of ethyl acetate. The combined organic extracts were dried (Na₂SO₄), filtered and the solvent was removed *in vacuo.* The residue was purified by chromatography (REVELERIS^{®} silica column, eluting with a linear gradient of 0-10% methanol in methylene chloride) to provide 0.25 g (0.60 mmol, 74%) of racemic 4-(1-(3-(3-chloro-4-fluorophenyl)-1-methylureldo)ethyl)-*N*-methylsoquinoline-1-carboxamide. LCMS: *m*/*z* found 415.3/417.3 [M+H]⁺. The enantiomers were subsequently separated by chiral SFC, Column: (R,R)-Whelk-01 (250 × 30 mm) 5 µ, 75% CO₂/MeOH, Flow rate 90 g/min.

4-(1-(3-(3-Chloro-4-fluorophenyl)-1-methylureido)ethyl)-N methylisoquinoline-1-carboxamide - Enantiomer I **(Compound 316).** LCMS: *m*/*z* found 415.1/417.1 [M+H]⁺; RT = 4.32 min (Method A); ¹H NMR (400 MHz, DMSO-d₆): δ 8.91 (d, 1H), 8.80 (m, 1H), 8.59 (s, 1H), 8.52 (s, 1H), 8.15 (d, 1H), 7.84-7.88 (m, 2H), 7.70-7.74 (m, 1H), 7.48-7.52 (m, 1H), 7.32 (t, 1H), 6.26-6.32 (m, 1H), 2.89 (d, 3H), 2.60 (s, 3H), 1.67 (d, 3H); Chiral analytical SFC: RT = 2.86 min, Column: (R,R)-Whelk-01 (4.6 × 150 mm) 3.5 µm, 70% CO₂/MeOH, Flow = 3.0 g/min.

4-(1-(3-(3-Chloro-4-fluorophenyl)-1-methylureido)ethyl)-N methylisoquinoline-1-carboxamide - Enantiomer II **(Compound 317).** LCMS: *m*/*z* found 415.1/417.1 [M+H]⁺; RT = 4.32 min (Method A); ¹H NMR (400 MHz, DMSO-d₆): δ 8.91 (d, 1H), 8.80 (m, 1H), 8.59 (s, 1H), 8.52 (s, 1H), 8.15 (d, 1H), 7.84-7.88 (m, 2H), 7.70-7.74 (m, 1H), 7.48-7.52 (m, 1H), 7.32 (t, 1H), 6.26-6.32 (m, 1H), 2.89 (d, 3H), 2.60 (s, 3H), 1.67 (d, 3H); Chiral analytical SFC: RT = 4.10 min, Column: (R,R)-Whelk-01 (4.6 × 150 mm) 3.5 µm, 70% CO₂/MeOH, Flow = 3.0 g/min.

### 4-(1-(3-(3-Chloro-4-fluorophenyl)-1-methylureido)ethyl)-N-N-dimethylisoquinoline-1-carboxamide (Compounds 318 & 319)

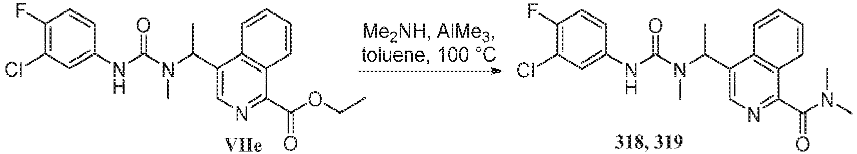

To a solution of 0.35 g (0.82 mmol, 1.0 eq.) of ethyl 4-(1-(3-(3-chloro-4-fluorophenyl)-1-methylureido)ethyl)isoquinoline-1-carboxylate **(VIIe)** in 8 mL of toluene under a nitrogen atmosphere was added 0.62 mL (1.22 mmol, 1.5 eq.) of a 2 M solution of dimethylamine in THF followed by 0.82 mL (1.63 mmol, 2.0 eq.) of a 2 M solution of trimethylaluminium in toluene and the mixture was heated at 100 °C for 2 h. The mixture was allowed to cool to room temperature, quenched with 15 mL of saturated ammonium chloride solution and extracted with 2 × 50 mL of ethyl acetate. The combined organic extracts were dried (Na₂SO₄), filtered and the solvent was removed *in vacuo.* The residue was purified by chromatography (REVELERIS^{®} silica column, eluting with a linear gradient of 0-10% methanol in methylene chloride) to provide 0.15 g (0.35 mmol, 43%) of racemic 4-(1-(3-(3-chloro-4-fluorophenyl)-1-methylureido)ethyl)-*N*,*N*-dimethylisoquinoline-1-carboxamide. LCMS: *m*/*z* found 429.1/431.1 [M+H]⁺. The enantiomers were subsequently separated by chiral SFC, Column: (R,R)-Whelk-01 (250 × 30 mm) 5 µ, 65% CO₂/MeOH, Flow rate 70 g/min.

4-(1-(3-(3-Chloro-4-fluorophenyl)-1-methylureido)ethyl)-N,N dimethylisoquinoline-1-carboxamide - Enantiomer I **(Compound 318).** LCMS: *m*/*z* found 429.2/431.2 [M+H]⁺; RT = 4.13 min (Method A); ¹H NMR (400 MHz, DMSO-d₆): δ 8.59 (s, 1H), 8.51 (s, 1H), 8.16 (d, 1H), 7.85-7.92 (m, 3H), 7.69-7.73 (m, 1H), 7.48-7.53 (m, 1H), 7.32 (t, 1H), 6.26-6.31 (m, 1H), 3.15 (s, 3H), 2.74 (s, 3H), 2.61 (s, 3H), 1.66 (d, 3H); Chiral analytical SFC: RT = 2.16 min, Column: (R,R)-Whelk-01 (4.6 × 150 mm) 3.5 µm, 60% CO₂/MeOH, Flow = 3.0 g/min.

4-(1-(3-(3-Chloro-4-fluorophenyl)-1-methylureido)ethyl)-*N*,*N*-dimethylisoquinoline-1-carboxamide - Enantiomer II **(Compound 319).** LCMS: *m*/*z* found 429.2/431.2 [M+H]⁺; RT = 4.15 min (Method A); ¹H NMR (400 MHz, DMSO-d₆): δ 8.59 (s, 1H), 8.51 (s, 1H), 8.16 (d, 1H), 7.85-7.92 (m, 3H), 7.69-7.73 (m, 1H), 7.48-7.53 (m, 1H), 7.32 (t, 1H), 6.26-6.31 (m, 1H), 3.15 (s, 3H), 2.74 (s, 3H), 2.61 (s, 3H), 1.66 (d, 3H); Chiral analytical SFC: RT = 3.04 min, Column: (R,R)-Whelk-01 (4.6 × 150 mm) 3.5 µm, 60% CO₂/MeOH, Flow = 3.0 g/min.

### 4-(1-(3-(3-Chloro-4-fluorophenyl)-1-methylureido)ethyl)isoquinoline-1-carboxamide (Compounds 328 & 329)

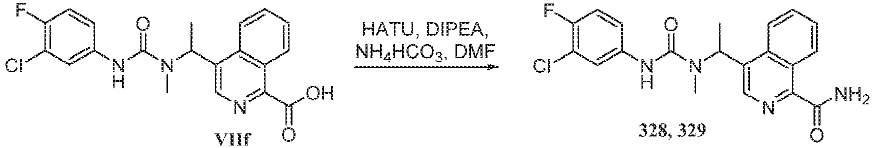

To a solution of 0.3 g (0.74 mmol, 1.0 eq.) of racemic 4-(1-(3-(3-chloro-4-fluorophenyl)-1-methylureido)ethyl)isoquinoline-1-carboxylic acid **(VIIf)** in 5 mL of DMF was added 0.52 mL (3.0 mmol, 4.0 eq.) of *N*,*N*-diisopropylethylamine followed by 0.56 g (1.5 mmol, 2.0 eq.) of (1-[bis(dimethylamino)methylene]-1*H*-1,2,3-triazolo[4,5-b]pyridinium 3-oxide hexafluorophosphate and 0.59 g (7.5 mmol, 10 eq.) of ammonium bicarbonate and the mixture was stirred at room temperature for 16 h. The mixture was diluted with 30 mL of ice-cold water and the solids collected by filtration, washed with 10 mL of water followed by 5 mL of n-pentane to provide 0.2 g (0.5 mmol, 67%) of racemic 4-(1-(3-(3-chloro-4-fluorophenyl)-1-methylureido)ethyl)isoquinoline-1-carboxamide. The enantiomers were subsequently separated by chiral SFC, Column: (R,R)-Whelk-01 (250 × 30 mm) 5 µ, 75% CO₂/MeOH, Flow rate 100 g/min.

4-(1-(3-(3-Chloro-4-fluorophenyl)-1-methylureido)ethyl)isoquinoline-1-carboxamide - Enantiomer I **(Compound 328).** LCMS: *m*/*z* found 401.1/403.2 [M+H]⁺; RT = 4.17 min (Method A); ¹H NMR (400 MHz, DMSO-d₆): δ 8.89 (d, 1H), 8.60 (s, 1H), 8.52 (s, 1H), 8.22 (bs, 1H), 8.14 (d, 1H), 7.84-7.89 (m, 2H), 7.80 (bs, 1H), 7.70-7.74 (m, 1H), 7.48-7.53 (m, 1H), 7.32 (t, 1H), 6.26-6.32 (m, 1H), 2.61 (s, 3H), 1.67 (d, 3H); Chiral analytical SFC: RT = 2.42 min, Column: (R,R)-Whelk-01 (4.6 × 150 mm) 3.5 µm, 60% CO₂/MeOH, Flow = 3.0 g/min.

4-(1-(3-(3-Chloro-4-fluorophenyl)-1-methylureido)ethyl)isoquinoline-1-carboxamide - Enantiomer II **(Compound 329).** LCMS: *m*/*z* found 401.1/403.2 [M+H]⁺; RT = 4.16 min (Method A); ¹H NMR (400 MHz, DMSO-d₆): δ 8.89 (d, 1H), 8.60 (s, 1H), 8.52 (s, 1H), 8.22 (bs, 1H), 8.14 (d, 1H), 7.84-7.89 (m, 2H), 7.80 (bs, 1H), 7.70-7.74 (m, 1H), 7.48-7.53 (m, 1H), 7.32 (t, 1H), 6.26-6.32 (m, 1H), 2.61 (s, 3H), 1.67 (d, 3H); Chiral analytical SFC: RT = 3.57 min, Column: (R,R)-Whelk-01 (4.6 × 150 mm) 3.5 µm, 60% CO₂/MeOH, Flow = 3.0 g/min.

### 3-(3-Chloro-4-fluorophenyl)-1-(1-(1-(hydroxymethyl)isoquinolin-4-yl)ethyl)-1-methylurea (Compounds 326 & 327)

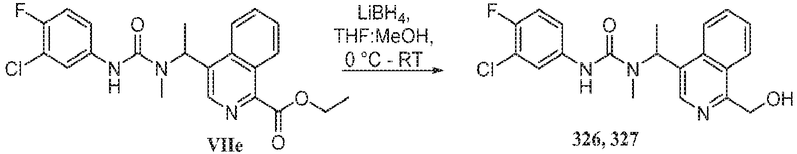

To a solution of 0.15 g (0.35 mmol, 1.0 eq.) of racemic ethyl 4-(1-(3-(3-chloro-4-fluorophenyl)-1-methylureido)ethyl)isoquinoline-1-carboxylate **(VIIe)** in 4 mL of 1:1 *v*/*v* methanol:THF at 0 °C was added 31 mg (1.40 mmol, 4.0 eq.) of lithiumborohydride. The mixture was allowed to warm to room temperature and stirred for 2 h. The reaction was quenched with 20 mL of ice-cold water and filtered through a CELITE^{®}. The pad was washed with 5 mL of THF and 5 mL of methanol and the filtrate was concentrated *in vacuo.* The residue was purified by semi-preparative HPLC to provide 95 mg (0.24 mmol, 70%) of racemic3-(3-chloro-4-fluorophenyl)-1-(1-(1-(hydroxymethyl)isoquinolin-4-yl)ethyl)-1-methylurea. The enantiomers were subsequently separated by chiral SFC, Column: Chiralpak IG (250 × 30 mm) 5 µ, 70% CO₂/MeOH, Flow rate 100 g/min.

3-(3-Chloro-4-fluorophenyl)-1-(1-(1-(hydroxymethyl)isoquinolin-4-yl)ethyl)-1-methylurea - Enantiomer I **(Compound 326).** LCMS: *m*/*z* found 388.2/390.2 [M+H]⁺; RT = 3.31 min (Method A); ¹H NMR (400 MHz, DMSO-d₆): δ 8.48-8.52 (m, 2H), 8.39 (d, 1H), 8.09 (d, 1H), 7.80-7.87 (m, 2H), 7.67-7.71 (m, 1H), 7.48-7.53 (m, 1H), 7.32 (t, 1H), 6.22-6.28 (m, 1H), 5.42 (bs, 1H), 5.06 (s, 2H), 2.57 (s, 3H), 1.63 (d, 3H); Chiral analytical SFC: RT = 1.42 min, Column: Chiralpak IG-3 (4.6 × 150 mm) 3 µm, 70% CO₂/MeOH, Flow = 3.0 g/min.

3-(3-Chloro-4-fluorophenyl)-1-1(-(1-(hydroxymethyl)isoquinolin-4-yl)ethyl)-1-methylurea - Enantiomer II **(Compound 327).** LCMS: *m*/*z* found 388.2/390.2 [M+H]⁺; RT = 3.33 min (Method A); ¹H NMR (400 MHz, DMSO-d₆): δ 8.48-8.52 (m, 2H), 8.39 (d, 1H), 8.09 (d, 1H), 7.80-7.87 (m, 2H), 7.67-7.71 (m, 1H), 7.48-7.53 (m, 1H), 7.32 (t, 1H), 6.22-6.28 (m, 1H), 5.42 (bs, 1H), 5.06 (s, 2H), 2.57 (s, 3H), 1.63 (d, 3H); Chiral analytical SFC: RT = 1.98 min, Column: Chiralpak IG-3 (4.6 × 150 mm) 3 µm, 70% CO₂/MeOH, Flow = 3.0 g/min.

### 3-(3-Chloro-4-fluorophenyl)-1-(1-(1-cyanoisoquinolin-4-yl)ethyl)-1-methylurea (Compounds 336 & 337)

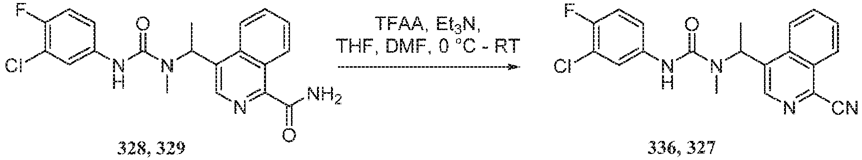

To a solution of 0.15 g (0.38 mmol, 1.0 eq.) of racemic 4-(1-(3-(3-chloro-4-fluorophenyl)-1-methylureido)ethyl)isoquinoline-1-carboxamide **(Compounds 328 & 329)** in 6 mL of 1:1 v/v THF:DMF at 0 °C under a nitrogen atmosphere was added 0.26 mL (1.88 mmol, 5.0 eq.) of triethylamine followed by 0.16 g (0.75 mmol, 2.0 eq.) of trifluoroacetic anhydride. The mixture was allowed to warm to room temperature stirred for 1 h. The reaction was quenched with 10 mL of saturated ammonium chloride solution and extracted with 2 × 30 mL of ethyl acetate. The combined organic extracts were dried (Na₂SO₄), filtered the solvent was removed *in vacuo.* The residue was purified by flash chromatography (eluting with a linear gradient of 10-30% of ethyl acetate in petroleum ether) to provide 0.08 g (0.21 mmol, 55%) of racemic 3-(3-chloro-4-fluorophenyl)-1-(1-(1-cyanoisoquinolin-4-yl)ethyl)-1-methylurea. The enantiomers were subsequently separated by chiral SFC, Column: Chiralpak IC (250 × 30 mm) 5 µ, 60% CO₂/MeOH, Flow rate 70 g/min.

3-(3-Chloro-4-fluorophenyl)-1-(1-(1-cyanoisoquinolin-4-yl)ethyl)-1-methylurea-Enantiomer I **(Compound 336)**. LCMS: *m*/*z* found 383.1/385.1 [M+H]⁺; RT = 5.21 min (Method A); ¹H NMR (400 MHz, DMSO-d₆): δ 8.82 (s, 1H), 8.53 (bs, 1H), 8.28-8.32 (m, 2H), 8.01-8.05 (m, 1H), 7.93-7.97 (m, 1H), 7.83-7.86 (m, 1H), 7.47-7.52 (m, 1H), 7.32 (t, 1H), 6.32-6.37 (m, 1H), 2.64 (s, 3H), 1.67 (d, 3H); Chiral analytical SFC: RT = 1.69 min, Column: Chiralpak IC-3 (4.6 × 150 mm) 3 µm, 60% CO₂/MeOH, Flow = 3.0 g/min.

3-(3-Chloro-4-fluorophenyl)-1-(1-(1-cyanoisoquinolin-4-yl)ethyl)-1-methylurea-Enantiomer II **(Compound 337)**. LCMS: *m*/*z* found 383.1/385.1 [M+H]⁺; RT = 5.21 min (Method A); ¹H NMR (400 MHz, DMSO-d₆): δ 8.82 (s, 1H), 8.53 (bs, 1H), 8.28-8.32 (m, 2H), 8.01-8.05 (m, 1H), 7.93-7.97 (m, 1H), 7.83-7.86 (m, 1H), 7.47-7.52 (m, 1H), 7.32 (t, 1H), 6.32-6.37 (m, 1H), 2.64 (s, 3H), 1.67 (d, 3H); Chiral analytical SFC: RT = 3.32 min, Column: Chiralpak IC-3 (4.6 × 150 mm) 3 µm, 60% CO₂/MeOH, Flow = 3.0 g/min.

### N-Methyl-1-(1-methylisoquinolin-4-yl)ethan-1-amine (VIax)

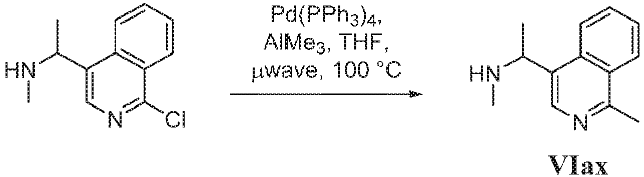

To a solution of 0.3 g (1.36 mmol, 1.0 eq.) of 1-(1-chloroisoquinolin-4-yl)-N-methylethan-1-amine in 5 mL of THF in a microwave vial was added 0.08 g (0.07 mmol, 0.05 eq.) of tetrakis(triphenylphosphine)palladium(0) followed by 0.8 mL (1.6 mmol, 1.2 eq.) of a 2 M solution of trimethyl aluminium in toluene. The mixture was purged with nitrogen gas for 5 min and then subjected to microwave irradiation, maintaining a reaction temperature of 100 °C for 1 h. The mixture was allowed to cool to room temperature and quenched with 15 mL of ice-cold water. The resulting heterogeneous mixture was filtered through CELITE^{®} The filtrate was extracted with 60 mL ethyl acetate and the organic extracts were dried (Na₂SO₄), filtered and the solvent was removed *in* vacuo to provide 0.3 g of *N*-methyl-1-(1-methylisoquinolin-4-yl)ethan-1-amine **(VIax).** ¹H NMR (300 MHz, DMSO-*d*₆) δ 8.62 (s, 1H), 8.26-8.33 (m, 2H), 7.86-7.92 (m, 1H), 7.73-7.79 (m, 1H), 5.11-5.14 (m, 1H), 2.92 (s, 3H), 2.55 (s, 3H), 1.65 (d, 3H).

### 3-(3-Chloro-4-fluorophenyl)-1-methyl-1-(1-(1-methylisoquinolin-4-yl)ethyl)urea (Compounds 352 & 353)

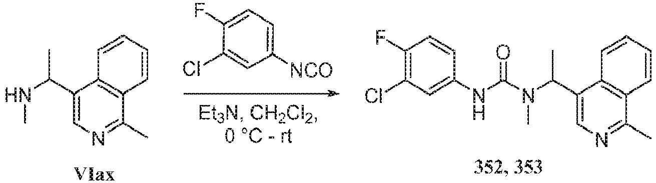

Racemic 3-(3-chloro-4-fluorophenyl)-1-methyl-1-(1-(1-methylisoquinofin-4-yl)ethyl)urea was synthesized in a similar manner as described above from *N*-methyl-1-(1-methylisoquinolin-4-yl)ethan-1-amine **(VIax)** and 2-chloro-1-fluoro-4-isocyanatobenzene. The enantiomers were subsequently separated by chiral SFC, Column: (R,R)-Whelk-01 (250 × 30 mm) 5 µ, 65% CO₂/MeOH, Flow rate 100 g/min.

3-(3-Chloro-4-fluorophenyl)-1-methyl-1-(1-(1-methylisoquinolin-4-yl)ethyl)urea-Enantiomer **I (Compound 352)**. LCMS: *m*/*z* found 372.2/374.4 [M+H]⁺; RT = 3.54 min (Method A); ¹H NMR (400 MHz, DMSO-d₆): δ 8.48 (s, 1H), 8.44 (s, 1H), 8.25 (d, 1H), 8.06 (d, 1H), 7.85-7.87 (m, 1H), 7.79-7.83 (m, 1H), 7.66-7.70 (m, 1H), 7.48-7.53 (m, 1H), 7.32 (t, 1H), 6.20-6.25 (m, 1H), 2.90 (s, 3H), 2.55 (s, 3H), 1.61 (d, 3H); Chiral analytical SFC: RT = 3.26 min, Column: (R,R)-Whelk-01 (4.6 × 150 mm) 3.5 µm, 70% CO₂/MeOH, Flow = 3.0 g/min.

3-(3-Chloro-4-fluorophenyl)-1-methyl-1-(1-(1-methylisoquinolin-4-yl)ethyl)urea-Enantiomer **II (Compound 353)**. LCMS: *m*/*z* found 372.2/374.4 [M+H]⁺; RT = 3.54 min (Method A); ¹H NMR (400 MHz, DMSO-d₆): δ 8.48 (s, 1H), 8.44 (s, 1H), 8.25 (d, 1H), 8.06 (d, 1H), 7.85-7.87 (m, 1H), 7.79-7.83 (m, 1H), 7.66-7.70 (m, 1H), 7.48-7.53 (m, 1H), 7.32 (t, 1H), 6.20-6.25 (m, 1H), 2.90 (s, 3H), 2.55 (s, 3H), 1.61 (d, 3H); Chiral analytical SFC: RT = 4.83 min, Column: (R,R)-Whelk-01 (4.6 × 150 mm) 3.5 µm, 70% CO₂/MeOH, Flow = 3.0 g/min.

### 1-(1-(Methylthio)isoquinolin-4-yl)ethan-1-one (Vu)

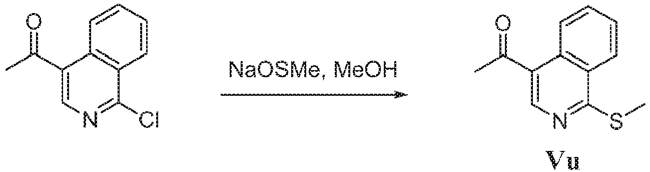

To a solution of 0.55 g (7.80 mmol, 2.0 eq.) of sodium thiomethoxide in 10 mL of methanol was added 0.8 g (3.90 mmol, 1.0 eq.) of 1-(1-chloroisoquinolin-4-yl)ethan-1-one and the mixture was stirred at room temperature for 2 h. The mixture was diluted with 10 mL of 10% aqueous potassium carbonate and extracted with 2 × 40 mL of methylene chloride. The combined organic extracts were washed with 30 mL of brine, dried (Na₂SO₄), filtered and the solvent was removed *in vacuo* to provide 0.7 g (3.22 mmol, 82%) of 1-(1-(methylthio)isoquinolin-4-yl)ethan-1-one **(Vu).** LCMS: *m*/*z* found 218.1 [M+H]⁺; RT = 2.46 min; ¹H NMR (400 MHz, CDCl₃) δ 8.99 (d, 1H), 8.90 (s, 1H), 8.26 (d, 1H), 7.77-7.81 (m, 1H), 7.59-7.63 (m, 1H), 2.77 (s, 6H).

### N-Methyl-1-(1-(methylthio)isoquinolin-4-yl)ethan-1-amine (VIay)

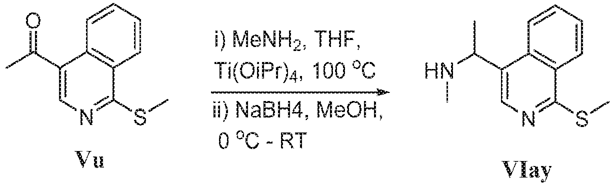

To a solution of 0.2 g (0.92 mmol, 1.0 eq.) of 1-(1-(methylthio)isoquinolin-4-yl)ethan-1-one **(Vu)** in 2 mL of THF in a sealed tube at room temperature under a nitrogen atmosphere was added 2.0 mL (4.0 mmol, 4.3 eq.) of a 2 M solution of methylamine solution in THF followed by 2 mL of titanium isopropoxide, and the mixture was heated to 100 °C for 4 h. The mixture was allowed to cool to room temperature and further cooled to 0 °C. The cooled solution was then diluted with 2 mL of methanol and 0.07 g (1.84 mmol, 2 eq.) of sodium borohydride was added portion-wise. The mixture was then allowed to warm to room temperature and stirred for 2 h. The mixture was diluted with 10 mL of brine and 50 mL of 10% methanol in methylene chloride, filtered through CELITE^{®} and the pad washed with 20 mL of 10% methanol in methylene chloride. The oranic layer was separated, washed with 40 mL of brine, dried (Na₂SO₄), filtered and the solvent was removed *in vacuo* to provide 0.25 g of *N*-methyl-1-(1-(methylthio)isoquinolin-4-yl)ethan-1-amine **(VIay).** LCMS: *m*/*z* found 233.2 [M+H]⁺; ¹H NMR (400 MHz, DMSO-d₆) δ 8.42 (s, 1H), 8.37 (d, 1H), 8.16 (d, 1H), 7.76-7.82 (m, 1H), 7.64-7.69 (m, 1H), 4.24-4.30 (m, 1H), 2.64 (s, 3H), 2.16-2.18 (m, 4H), 1.39 (d, 3H).

### 3-(3-Chloro-4-fluorophenyl)-1-methyl-1-(1-(1-(methylthio)isoquinolin-4-yl)ethyl)urea (VIIg)

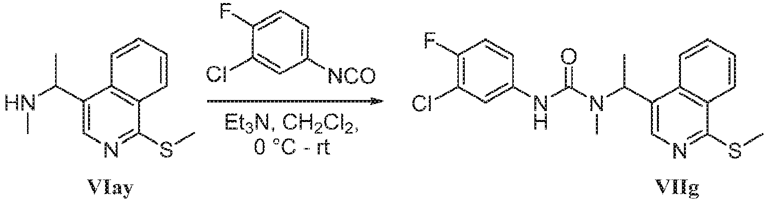

To a solution of 0.25 g of *N*-methyl-1-(1-(methylthio)isoquinolin-4-yl)ethan-1-amine **(VIay)** in 5 mL of methylene chloride at 0 °C under a nitrogen atmosphere was added 0.45 mL (3.23 mmol) of triethylamine followed by 0.13 mL (1.07 mmol) of 2-chloro-1-fluoro-4-isocyanatobenzene. The reaction mixture was allowed to warm to room temperature and stirred for 1 h. The mixture was diluted with 15 mL of water and extracted with 2 × 40 mL of 10% methanol in methylene chloride. The combined organic extracts were dried (Na₂SO₄), filtered and the solvent was removed *in vacuo.* The residue was purified by chromatography (REVELERIS^{®} Silica column, eluting with a linear gradient of 0-6% methanol in methylene chloride) to provide 0.26 g (0.64 mmol, 70% from **Vu)** of 3-(3-chloro-4-fluorophenyl)-1-methyl-1-(1-(1-(methylthio)isoquinolin-4-yl)ethyl)urea **(VIIg).** LCMS: *m*/*z* found 404.3 [M+H]⁺, RT = 2.78 min; ¹H NMR (300 MHz, DMSO-*d*₆) δ 8.45-8.49 (m, 2H), 8.19 (d, 1H), 8.06 (d, 1H), 7.81-7.89 (m, 2H), 7.66-7.72 (m, 1H), 7.68-7.72 (m, 1H), 7.31 (t, 1H), 6.17-6.21 (m, 1H), 2.67 (s, 3H), 2.58 (s, 3H), 1.61 (d, 3H).

### 3-(3-Chloro-4-fluorophenyl)-1-methyl-1-(1-(1-(methylsulfonyl)isoquinolin-4-yl)ethyl)urea (Compounds 314 & 315)

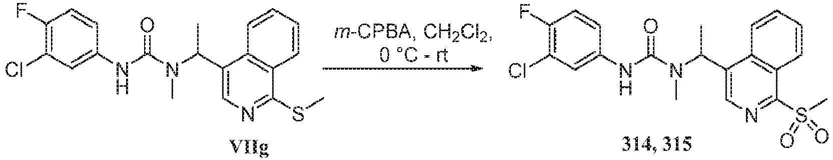

To a solution of 0.26 g (0.64 mmol, 1.0 eq.) of 3-(3-chloro-4-fluorophenyl)-1-methyl-1-(1-(1-(methylthio)isoquinolin-4-yl)ethyl)urea **(VIIg)** in 3 mL of methylene chloride at 0 °C was added 0.38 g (1.55 mmol, 2.0 eq.) of 70% m-chloroperbenzoic acid. The mixture was allowed to warm to room temperature and stirred for 2 h. The mixture was then diluted with 30 mL of saturated sodium bicarbonate solution and extracted with 2 × 30 ml of methylene chloride. The combined organic extracts were dried (Na₂SO₄), filtered and the solvent was removed *in vacuo.* The residue was purified by chromatography (REVELERIS^{®} Silica column, eluting with 2% of methanol in methylene chloride) to provie 0.16 g (0.36 mmol, 57%) of racemic 3-(3-chloro-4-fluorophenyl)-1-methyl-1-(1-(1-(methylsulfonyl)isoquinolin-4-yl)ethyl)urea. The enantiomers were subsequently separated by chiral SFC, Column: (R,R)-Whelk-01 (250 × 30 mm) 5 µ, 60% CO₂/MeOH, Flow rate 100 g/min.

3-(3-Chloro-4-fluorophenyl)-1-methyl-1-(1-(1-(methylsulfonyl)isoquinolin-4-yl)ethyl)urea - Enantiomer I **(Compound 314).** LCMS: *m*/*z* found 436.1/438.1 [M+H]⁺; RT = 4.84 min (Method A); ¹H NMR (400 MHz, DMSO-d₆): δ 8.85 (d, 1H), 8.69 (s, 1H), 8.54 (s, 1H), 8.30 (d, 1H), 7.99-8.03 (m, 1H), 7.84-7.91 (m, 2H), 7.48-7.52 (m, 1H), 7.32 (t, 1H), 6.30-6.34 (m, 1H), 3.60 (s, 3H), 2.65 (s, 3H), 1.68 (d, 3H); Chiral analytical SFC: RT = 2.45 min, Column: (R,R)-Whelk-01 (4.6 × 150 mm) 3.5 µm, 60% CO₂/MeOH, Flow = 3.0 g/min.

3-(3-Chloro-4-fluorophenyl)-1-methyl-1-(1-(1-(methylsulfonyl)isoquinolin-4-yl)ethyl)urea - Enantiomer II **(Compound 315).** LCMS: *m*/*z* found 436.1/438.1 [M+H]⁺; RT = 4.84 min (Method A); ¹H NMR (400 MHz, DMSO-d₆): δ 8.85 (d, 1H), 8.69 (s, 1H), 8.54 (s, 1H), 8.30 (d, 1H), 7.99-8.03 (m, 1H), 7.84-7.91 (m, 2H), 7.48-7.52 (m, 1H), 7.32 (t, 1H), 6.30-6.34 (m, 1H), 3.60 (s, 3H), 2.65 (s, 3H), 1.68 (d, 3H); Chiral analytical SFC: RT = 4.67 min, Column: (R,R)-Whelk-01 (4.6 × 150 mm) 3.5 µm, 60% CO₂/MeOH, Flow = 3.0 g/min.

### Synthesis of 6-Fluoroisoquinolin-1(2H)-one (IIb) - Route I: 6-Fluoroisoquinoline 2-oxide

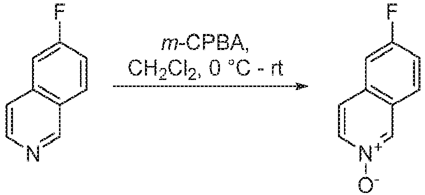

To a stirred solution of 2.0 g (13.6 mmol, 1.0 eq.) of 6-fluoroisoquinoline in 20 mL of methylene chloride at 0 °C was added 4.6 g (27.2 mmol, 2.0 eq.) of m-chloroperoxybenzoic acid portion-wise over 15 min. On completion of addition, the mixture was allowed to warm to room temperature and stirred for 16 h. The mixture was then poured into 50 mL of ice-cold water and extracted with 5 × 60 mL of methylene chloride. The combined organic extracts were washed with 30 mL of 10% aqueous NaOH solution followed by 50 mL of brine, dried (Na₂SO₄), filtered and the solvent was removed *in vacuo.* The resulting residue was triturated with 40 mL of diethyl ether, filtered, and the resulting solid dried under vacuum to provide 1.8 g (11.0 mmol, 81%) of 6-fluoroisoquinoline 2-oxide. LCMS: *m*/*z* found 164.15 [M+H]⁺, RT = 1.37 min; ¹H NMR (300 MHz, DMSO-d₆): δ 8.97 (s, 1H), 8.19-8.16 (m, 1H), 8.00-7.95 (m, 1H), 7.91 (d, 1H), 7.81-7.74 (m, 1H), 7.62-7.56 (m, 1H).

### 6-Fluoroisoquinolin-1(2H)-one (IIb)

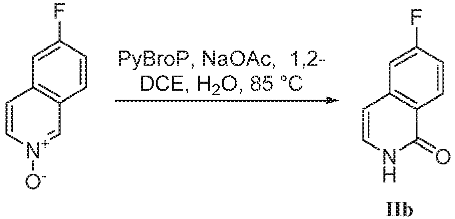

To a stirred suspension of 1.7 g (10.4 mmol, 1.0 eq.) of 6-fluoroisoquinoline 2-oxide in 17 mL of 1,2-dichloroethane at room temperature was added 2.6 g (31.3 mmol, 3.0 eq.) of sodium acetate followed by 9.7 g (20.9 mmol, 2.0 eq.) of benzotriazol-1-yl-oxytripyrrolidinophosphonium hexafluorophosphate (PyBroP) and 2 mL of water. The mixture was then heated at 85 °C for 16 h. The mixture was allowed to cool to room temperature and diluted with 50 mL of ethyl acetate and 50 mL of water. The layers were separated and the organic phase was washed with 30 mL of water followed by 30 mL of brine, dried (Na₂SO₄), filtered and the solvent was removed *in vacuo.* The residue was purified by flash chromatography (SiO₂, eluting with linear gradient of 30-60% ethyl acetate/petroleum ether) to provide 0.8 g (4.9 mmol, 47%) of 6-fluoroisoquinolin-1(2*H*)-one **(IIb).** LCMS: *m*/*z* found 164.0 [M+H]⁺, RT = 1.38 min; ¹H NMR (300 MHz, DMSO-d₆): δ 11.29 (bs, 1H), 8.25-8.20 (m, 1H), 7.50-7.45 (m, 1H), 7.34-7.27 (m, 1H), 7.24-7.20 (m, 1H), 6.54 (d, 1H).

### Synthesis of 6-Fluoroisoquinolin-1(2H)-one (IIb) - Route II: 6-Fluoro-3,4-dihydroisoquinolin-1(2H)-one

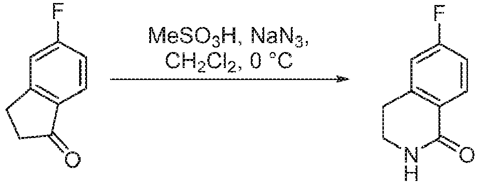

To a solution of 15.0 g (100 mmol, 1.0 eq.) of 5-fluoro-2,3-dihydro-1*H*-inden-1-one in 150 mL of methylene chloride was added 120 mL of methane sulfonic acid. The mixture was cooled to 0 °C and 13.0 g (200 mmol, 2.0 eq.) of sodium azide was added portions wise over 20 min. The resulting mixture was then stirred at 0 °C for 2 h. On completion, the reaction mixture was basified (pH>10) by the slow addition of 350 mL of 20% aqueous NaOH solution at 0 °C over 30 min and then stirred at at 0 °C for an additional 30 min. The resulting solution was extracted with 2 × 500 mL of 10% methanol in methylene chloride. The combined organic extracts were washed with 500 mL of water, 500 mL of brine, dried (Na₂SO₄), filtered and the solvent was removed *in vacuo.* The residue was purified by flash column chromatography (SiO₂, eluting with 0-30% ethyl acetate/petroleum ether) to provide 7.0 g (42.4 mmol, 42%) of 6-fluoro-3,4-dlhydrolsoquinolin-1(2*H*)-one. LCMS: *m*/*z* found 166.3 [M+H]⁺, RT = 1.74 min; ¹H NMR (400 MHz, CDCl₃): δ 8-06-8.10 (m, 1H), 7.00-7.05 (m, 1H), 6.90-6.93 (m, 1H), 6.47 (bs, 1H), 3.56-3.60 (m, 2H), 2.98-3.02 (m, 2H).

### 6-Fluoroisoquinolin-1(2H)-one (IIb)

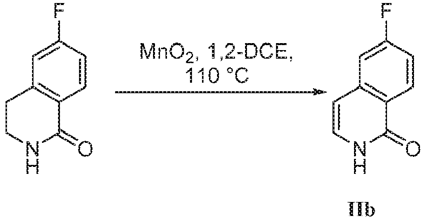

To a solution of 7.0 g (42.4 mmol, 1.0 eq.) of 6-fluoro-3,4-dihydroisoquinolin-1(2*H*)-one in 150 mL of 1,2-dichloroethane at room temperature was added 22.1 g (254 mmol, 6.0 eq.) of activated manganese dioxide. The mixture was then heated at 110 °C for 24 h. The mixture was allowed to cool to room temperature and filtered through CELITE^{®}. The pad was washed with 50 mL of 1,2-dichloroethane and the filtrate was further treated with 14.8 g (170 mmol, 4.0 eq.) of activated manganese dioxide and heated at 110 °C for an additional 48 h. The mixture was allowed to cool to room temperature and again filtered through a CELITE^{®}, and the pad was washed with 2 × 25 mL of DMSO. The 1,2-dichloroethane was removed *in* vacuo and the resulting DMSO solution was diluted with 650 mL of ice-cold water. The precipitated solid was collected by filtration and dried under vacuum to provide 3.0 g (18.4 mmol 43%) of 6-fluoroisoquinolin-1(2*H*)-one (**IIb**). LCMS: *m*/*z* found 163.9 [M+H]⁺, RT = 1.39 min; ¹H NMR (300 MHz, DMSO-d₆): δ 11.29 (bs, 1H), 8.20-8.23 (m, 1H), 7.45-7.50 (m, 1H), 7.27-7.34 (m, 1H), 7.20-7.24 (m, 1H), 6.54 (d, 1H).

### 4-Bromo-6-fluoroisoquinolin-1(2H)-one (IIIb)

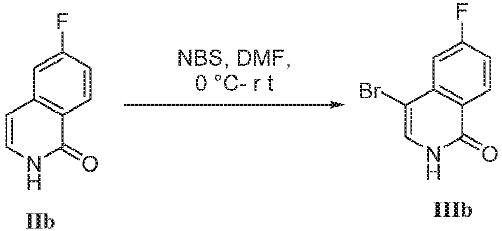

To a solution of 0.8 g (4.9 mmol, 1.0 eq.) of 6-fluoroisoquinolin-1(2*H*)-one (**IIb**) in 8 mL of DMF was added 0.88 g (5.1 mmol, 1.05 eq.) of *N-*bromosuccinimide and the mixture was stirred for 16 h. The mixture was then diluted with 80 mL of water and the resulting precipitated solid was collected by filtration, washed with 2 × 40 mL of water and dried under vacuum. The collected solids were triturated with 10 mL of methyl tert-butyl ether, filtered and dried under vacuum to provide 1.0 g (4.13 mmol, 84%) of 4-bromo-6-fluoroisoquinolin-1(2*H*)-one (**IIIb)**. LCMS: *m*/*z* found 242.1 [M+H]⁺, RT = 2.08 min; ¹H NMR (300 MHz, DMSO-d₆): δ 11.65 (bs, 1H), 8.33-8.27 (m, 1H), 7.65-7.62 (m, 1H), 7.49-7.42 (m, 2H).

### 4-Acetyl-6-fluoroisoquinolin-1(2H)-one (XXb)

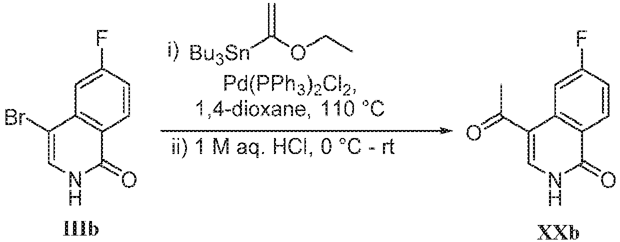

To a solution of 1.0 g (4.1 mmol, 1.0 eq.) of 4-bromo-6-fluorolsoquinolin-1(2*H*)-one **(IIIb)** in 10 mL of 1,4-dioxane was added 3.7 g (10.3 mmol, 2.5 eq.) of tributyl(1-ethoxyvinyl)tin. The mixture was degassed by purging with argon gas for 5 min and 0.29 g (0.41 mmol, 0.1 eq.) of bis(triphenylphosphine)palladium(II) dichloride was added. The mixture was then heated at 110 °C under an argon atmosphere for 16 h. The mixture was allowed to cool to room temperature and further cooled to 0 °C. The mixture was then diluted with 15 mL of 1 M aqueous HCl and the resulting solution stirred at room temperature for 3 h. The mixture was basified with 40 mL of saturated sodium bicarbonate solution and filtered through a CELITE^{®} pad. The filtrate was extracted with 3 × 50 mL of ethyl acetate and the combined organic extracts were washed with 30 mL of brine, dried (Na₂SO₄), filtered and the solvent was removed *in vacuo.* The residue was purified by flash chromatography (SiO₂, eluting with a linear gradient 50-70% ethyl acetate/petroleum ether) to provide 0.4 g (1.95 mmol, 47%) of 4-acetyl-6-fluoroisoquinolin-1(2*H*)-one **(XXb).** LCMS: *m*/*z* found 206.2 [M+H]⁺, RT = 1.86 min.

### 6-Fluoro-4-(1-(methylamino)ethyl)isoquinolin-1(2H)-one (VIIIn)

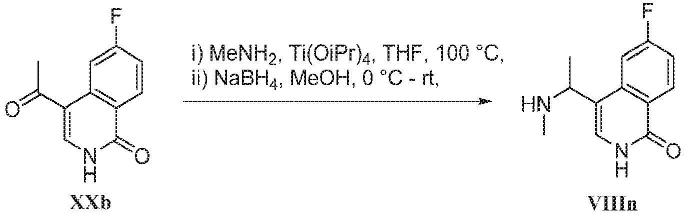

To a solution of 0.4 g (1.95 mmol, 1.0 eq.) of 4-acetyl-6-fluoroisoquinolin-1(2*H*)-one **(XXb)** in 10 mL of THF at room temperature under a nitrogen atmosphere was added 4.88 mL (9.76 mmol, 5.0 eq.) of a 2M solution of methylamine solution in THF followed by 4 mL of titanium isopropoxide, and the mixture was heated to 100 °C for 16 h. The mixture was allowed to cool to room temperature and further cooled to 0 °C. The cooled solution was then diluted with 5 mL of methanol and 0.15 g (3.90 mmol, 2.0 eq.) of sodium borohydride was added portions wise. The mixture was then allowed to warm to room temperature and stirred for 2 h. The reaction mixture was diluted with 20 mL of water, filtered through CELITE^{®}, and the filtrate was extracted with 6 × 20 mL of 10% methanol in dichloromethane. The combined organic extracts were washed with 60 mL of brine, dried (Na₂SO₄), filtered and the solvent was removed *in vacuo.* The residue was purified by reverse phase chromatography (REVELERIS^{®} C-18: 40 g column; eluting with linear gradient 5-15% of 0.1% formic acid in water with acetonitrile) to provide 0.2 g (0.90 mmol, 46%) of 6-fluoro-4-(1-(methylamino)ethyl)isoquinolin-1(2*H*)-one **(VIIIn).** LCMS: *m*/*z* found 221.23 [M+H]⁺; ¹H NMR (300 MHz, DMSO-d₆): δ 11.36 (bs, 1H), 8.32-8.26 (m, 1H), 8.18 (s, 1H), 7.84-7.79 (m, 1H), 7.37-7.31 (m, 1H), 7.23 (s, 1H), 4.09-4.03 (m, 1H), 2.28 (s, 3H), 1.35 (d, 3H).

### 3-(3-Chloro-4-fluorophenyl)-1-(1-(6-fluoro-1-oxo-1,2-dihydroisoquinolin-4-yl)ethyl)-1-methylurea (Compounds 169 & 170)

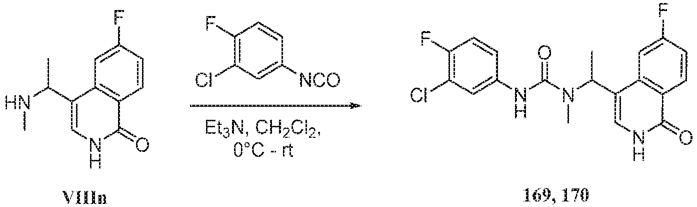

To a solution of 160 mg (0.72 mmol, 1.0 eq.) of 6-fluoro-4-(1-(methylamino)ethyl) isoquinolin-1(2*H*)-one **(VIIIn)** in 20 mL of methylene chloride at 0 °C was added 0.3 mL (2.18 mmol, 3.0 eq.) of triethylamine followed by 0.12 g (0.72 mmol, 1.0 eq.) of 2-chloro-1-fluoro-4-isocyanatobenzene. The mixture was allowed to warm to room temperature and stirred for 1 h. The reaction mixture was then diluted with 30 mL of water and extracted with 3 × 30 mL of 10% methanol in methylene chloride. The combined organic extracts were washed with 50 mL of brine, dried (Na₂SO₄), filtered and the solvent was removed *in vacuo.* The residue was purified by reverse phase chromatography (REVELERIS^{®} C-18: 40 g column eluting with linear gradient 10-22% of [0.1% formic acid in water]/acetonitrile) to provide 235 mg (0.59 mmol mmol, 83%) of racemic 3-(3-chloro-4-fluorophenyl)-1-(1-(6-fluoro-1-oxo-1,2-dihydroisoquinolin-4-yl)ethyl)-1-methylurea. LCMS: *m*/*z* found 392.29 [M+H]⁺. The enantiomers were subsequently separated by chiral SFC, Column: Chiralpak IG (250 × 30 mm) 5 µ, 85% CO₂/MeOH, Flow rate 100 g/min.

3-(3-Chloro-4-fluorophenyl)-1-(1-(6-fluoro-1-oxo-1,2-dihydroisoquinolin-4-yl)ethyl)-1-methylurea - Enantiomer I **(Compound 169)** LCMS: *m*/*z* found 392.2/394.2 [M+H]⁺, RT = 7.09 min (Method A); ¹H NMR (400 MHz, DMSO-*d*₆): δ 11.45 (bs, 1H), 8.48 (bs, 1H), 8.28-8.32 (m, 1H), 7.81-7.83 (m, 1H), 7.46-7.51 (m, 2H), 7.30-7.39 (m, 2H), 7.20-7.23 (m, 1H), 5.76-5.80 (m, 1H), 2.60 (s, 3H), 1.43 (d, 3H); Chiral analytical SFC: RT = 3.47 min, Column: Chiralpak IG (4.6 × 150 mm) 5 µm, 70.0% CO₂/MeOH, Flow = 3.0 g/min.

3-(3-Chloro-4-fluorophenyl)-1-(1-(6-fluoro-1-oxo-1,2-dihydroisoquinolin-4-yl)ethyl)-1-methylurea - Enantiomer II **(Compound 170)** LCMS: *m*/*z* found 392.2/394.2 [M+H]⁺, RT = 7.09 min (Method A); ¹H NMR (400 MHz, DMSO-*d*₆): δ 11.45 (bs, 1H), 8.48 (bs, 1H), 8.28-8.32 (m, 1H), 7.81-7.83 (m, 1H), 7.46-7.51 (m, 2H), 7.30-7.39 (m, 2H), 7.20-7.23 (m, 1H), 5.76-5.80 (m, 1H), 2.60 (s, 3H), 1.43 (d, 3H); Chiral analytical SFC: RT = 5.45 min, Column: Chiralpak IG (4.6 × 150 mm) 5 µm, 70.0% CO₂/MeOH, Flow = 3.0 g/min.

### 4-(1-(((2,2-Dimethyl-1,3-dioxan-5-yl)methyl)amino)ethyl)-6-fluoroisoquinolin-1(2H)-one (VIIIo)

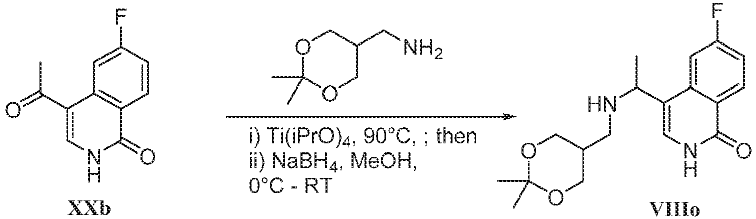

To a solution of 0.8 g (3.9 mmol, 1.0 eq.) of 4-acetyl-6-fluoroisoquinolin-1(2*H*)-one **(XXb)** and 1.13 g (7.8 mmol, 2.0 eq.) of (2,2-dimethyl-1,3-dioxan-5-yl)methanamine in 10 mL of THF in a sealed tube at room temperature was added 11.0 g (39.0 mmol, 10 eq.) of titanium isopropoxide. The reaction vessel was sealed, and the mixture was heated at 90 °C for 16 h. The reaction mixture was allowed to cool to room temperature and further cooled to 0 °C, diluted with 20 mL of methanol and then 0.3 g (7.8 mmol, 2.0 eq.) of sodium borohydride was added portion-wise over approximately 10 mins. The mixture was allowed to warm to room temperature and stirred for 2 h. The reaction mixture was diluted with 20 mL of brine and 100 mL of 20% methanol in methylene chloride. The resulting heterogeneous mixture was stirred for 30 min, filtered through CELITE^{®} and the pad was washed with 50 mL of 10% methanol in methylene chloride. The combined organic filtrates were dried (Na₂SO₄), filtered, and the solvent was removed *in vacuo.* The residue which was purified by MPLC (SiO₂, eluting with linear gradient 0-5% of methanol in methylene chloride) to provide 1.2 g (3.59 mmol, 92%) of 4-(1-(((2,2-dimethyl-1,3-dioxan-5-yl)methyl)amino)ethyl)-6-fluoroisoquinolin-1(2*H*)-one **(VIIIo).** LCMS: *m*/*z* found 335.3 [M+H]⁺, RT = 1.59 min; ¹H NMR (300 MHz, DMSO-*d*₆): δ 11.24 (bs, 1H), 8.25-8.31 (m, 1H), 7.82-7.85 (m, 1H), 7.28-7.35 (m, 1H), 7.15-7.17 (m, 1H), 3.89-3.93 (m, 1H), 3.79-3.82 (m, 2H), 3.53-3.59 (m, 2H), 2.40-2.51 (m, 2H), 1.69-1.78 (m, 2H), 1.25-1.30 (m, 9H).

### 3-(3-Chloro-4-fluorophenyl)-1-((2,2-dimethyl-1,3-dioxan-5-yl)methyl)-1-(1-(6-fluoro-1-oxo-1,2-dihydroisoquinolin-4-yl)ethyl)urea (Compounds 282 & 283)

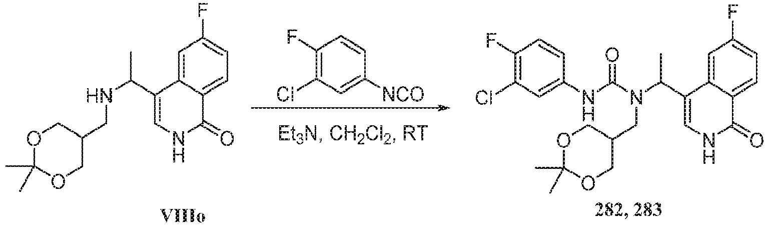

To a solution of 1.2 g (3.59 mmol, 1.0 eq.) of 4-(1-(((2,2-dimethyl-1,3-dioxan-5-yl)methyl)amino)ethyl)-6-fluoroisoquinolin-1(2*H*)-one **(VIIIo)** in 30 mL of methylene chloride under a nitrogen atmosphere was added 1.8 g (17.9 mmol, 5.0 eq.) of triethylamine followed by 0.62 g (3.59 mmol, 1.0 eq) of 2-chloro-1-fluoro-4-isocyanatobenzene and the mixture was stirred at room temperature for 2 h. The mixture was then diluted with 50 mL of water and extracted with 3 × 50 mL of methylene chloride. The combined organic extracts were washed with 2 × 35 mL of water, 35 mL of brine, dried (Na₂SO₄), filtered and the solvent was removed *in vacuo.* The residue was purified by MPLC (REVELERIS^{®} Silica column, eluting with a linear gradient of 0-5% methanol in methylene chloride) to provide 0.55 g (1.08 mmol, 30%) of racemic 3-(3-chloro-4-fluorophenyl)-1-((2,2-dimethyl-1,3-dioxan-5-yl)methyl)-1-(1-(6-fluoro-1-oxo-1,2-dihydroisoquinolin-4-yl)ethyl)urea. LCMS: *m*/*z* found 506.1/508.1 [M+H]⁺. A 70 mg portion of the racemate was subsequently separated by chiral SFC, Column Lux cellulose-2 (250 × 30 mm) 5 µ, 50% CO₂/MeOH, Flow rate 70 g/min.

3-(3-Chloro-4-fluorophenyl)-1-((2,2-dimethyl-1,3-dioxan-5-yl)methyl)-1-(1-(6-fluoro-1-oxo-1,2-dihydroisoquinolin-4-yl)ethyl)urea- Enantiomer I **(Compound 282):** LCMS: *m*/*z* found 506.1/508.1 [M+H]⁺, RT = 5.12 min (method A); ¹H NMR (400 MHz, DMSO-*d*₆): δ 11.46 (bs, 1H), 8.48 (bs, 1H), 8.27-8.31 (m, 1H), 7.73-7.76 (m, 1H), 7.32-7.46 (m, 4H), 7.27 (s, 1H), 5.76-5.80 (m, 1H), 3.66-3.70 (m, 1H), 3.53-3.57 (m, 1H), 3.27-3.33 (m, 2H), 3.09-3.27 (m, 2H), 1.49 (d, 3H), 1.18-1.26 (m, 7H); Chiral analytical SFC: RT = 3.03 min, Column: Lux Cellulose-2 (4.6 × 250) mm, 5 µ, 60% CO₂/MeOH, Flow = 4.0 g/min.

3-(3-Chloro-4-fluorophenyl)-1-((2,2-dimethyl-1,3-dioxan-5-yl)methyl)-1-(1-(6-fluoro-1-oxo-1,2-dihydroisoquinolin-4-yl)ethyl)urea - Enantiomer II **(Compound 283):** LCMS: *m*/*z* found 506.1/508.1 [M+H]⁺, RT = 5.12 min (method A); ¹H NMR (400 MHz, DMSO-*d*₆): δ 11.46 (bs, 1H), 8.48 (bs, 1H), 8.27-8.31 (m, 1H), 7.73-7.76 (m, 1H), 7.32-7.46 (m, 4H), 7.27 (s, 1H), 5.76-5.80 (m, 1H), 3.66-3.70 (m, 1H), 3.53-3.57 (m, 1H), 3.27-3.33 (m, 2H), 3.09-3.27 (m, 2H), 1.49 (d, 3H), 1.18-1.26 (m, 7H); Chiral analytical SFC: RT = 10.96 min, Column: Lux Cellulose-2 (4.6 × 250) mm, 5 µ, 60% CO₂/MeOH, Flow = 4.0 g/min.

### 3-(3-Chloro-4-fluorophenyl)-1-(1-(6-fluoro-1-oxo-1,2-dihydroisoquinolin-4-yl)ethyl)-1-(3-hydroxy-2-(hydroxymethyl)propyl)urea (Compounds 295 & 296)

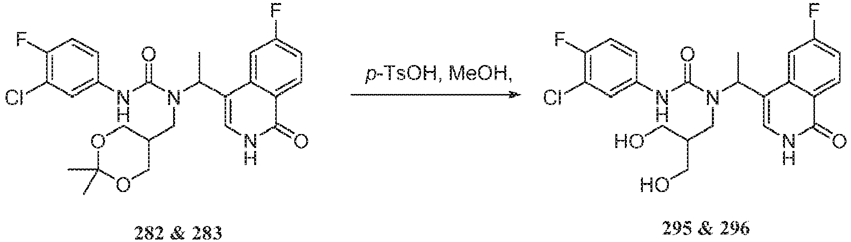

To a solution of 0.48 g (0.95 mmol, 1.0 eq.) of racemic 3-(3-chloro-4-fluorophenyl)-1-((2,2-dimethyl-1,3-dioxan-5-yl)methyl)-1-(1-(6-fluoro-1-oxo-1,2-dihydroisoquinolin-4-yl)ethyl)urea **(Compounds 282 & 283)** in 25 mL of methanol was added 21 mg (0.11 mml, 0.1 eq) of para-toluene sulfonic acid monohydrate and the mixture was stirred at room temperature for 2 h. The mixture was diluted with 20 mL of saturated sodium bicarbonate solution and 35 mL of water. The resultant mixture was stirred for 30 min, and the prepcipitated solids were collected by filtration, washed with 20 mL of pentane and dried under vacuum to provide 380 mg (0.81 mmol, 85%) of racemic 3-(3-chloro-4-fluorophenyl)-1-(1-(6-fluoro-1-oxo-1,2-dihydroisoquinolin-4-yl)ethyl)-1-(3-hydroxy-2-(hydroxymethyl)propyl)urea. LCMS: *m*/*z* found 466.5/468.5 [M+H]⁺. The enantiomers were subsequently separated by chiral SFC, Chiralpak IC (250 × 30 mm) 5 µ, 70% CO₂/MeOH, Flow rate 100 g/min.

3-(3-Chloro-4-fluorophenyl)-1-(1-(6-fluoro-1-oxo-1,2-dihydroisoquinolin-4-yl)ethyl)-1-(3-hydroxy-2-(hydroxymethyl)propyl)urea - Enantiomer I **(Compound 295)**: LCMS: *m*/*z* found 466.5/468.5 [M+H]⁺, RT = 4.02 min (method A); ¹H NMR (400 MHz, DMSO-*d*₆): δ 11.46 (bs, 1H), 9.26 (bs, 1H), 8.27-8.31 (m, 1H), 7.75-7.78 (m, 1H), 7.31-7.39 (m, 4H), 7.23 (d, 1H), 5.81-5.84 (m, 1H), 5.43 (t, 1H), 4.61 (t, 1H), 2.97-3.23 (m, 6H), 1.46 (d, 3H), 1.01-1.06 (m, 1H); Chiral analytical SFC: RT = 7.94 min, Column: Chiralpak IC (4.6 × 250) mm, 5 µ, 80% CO₂/MeOH, Flow = 3.0 g/min.

3-(3-Chloro-4-fluorophenyl)-1-(1-(6-fluoro-1-oxo-1,2-dihydroisoquinolin-4-yl)ethyl)-1-(3-hydroxy-2-(hydroxymethyl)propyl)urea - Enantiomer II **(Compound 296):** LCMS: *m*/*z* found 466.5/468.5 [M+H]⁺, RT = 4.02 min (method A); ¹H NMR (400 MHz, DMSO-*d*₆): δ 11.46 (bs, 1H), 9.26 (bs, 1H), 8.27-8.31 (m, 1H), 7.75-7.78 (m, 1H), 7.31-7.39 (m, 4H), 7.23 (d, 1H), 5.81-5.84 (m, 1H), 5.43 (t, 1H), 4.61 (t, 1H), 2.97-3.23 (m, 6H), 1.46 (d, 3H), 1.01-1.06 (m, 1H); Chiral analytical SFC: RT = 12.82 min, Column: Chiralpak IC (4.6 × 250) mm, 5 µ, 80% CO₂/MeOH, Flow = 3.0 g/min.

### 2-((1-(6-Fluoro-1-oxo-1,2-dihydroisoquinolin-4-yl)ethyl)amino)ethane-1-sulfonamide (VIIIp)

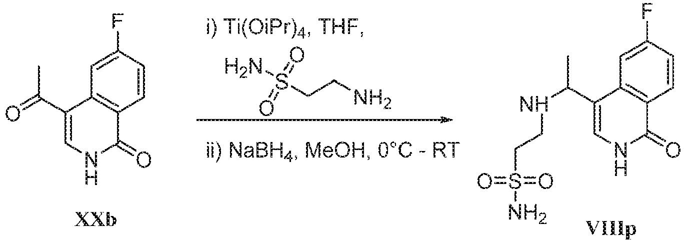

To a solution of 0.5 g (2.43 mmol, 1.0 eq.) of 4-acetyl-6-fluoroisoquinolin-1(2*H*)-one **(XXb)** in 10 mL of THF under a nitrogen atmosphere was added 0.45 g (3.66 mmol, 1.5 eq.) of 2-aminoethane-1-sulfonamide followed by 5 mL of titanium isopropoxide and the mixture was stirred at room temperature for 48 h. The mixture was then cooled to 0 °C, diluted with 2 mL of methanol and 0.37 g (9.72 mmol, 4.0 eq.) of sodium borohydride was added portions wise over approximately 10 minutes. After stirring at 0 °C for 4h, the mixture was diluted with 50 mL of brine and 100 mL of ethyl acetate. The resultant heterogeneous mixture was filtered through CELITE^{®} and the pad was washed with 25 mL of ethyl acetate. The layers were separated, and the aqueous phase was extracted with 2 × 50 mL of ethyl acetate. The combined organic extracts were washed with 100 mL of brine, dried (Na₂SO₄), filtered and the solvent was removed *in vacuo* to provide 0.4 g of 2-((1-(6-fluoro-1-oxo-1,2-dihydroisoquinolin-4-yl)ethyl)amino)ethane-1-sulfonamide **(VIIIp)** which was carried forward to the next step without further purification. LCMS: *m*/*z* found 314.0 [M+H]⁺.

### 2-(3-(3-Chloro-4-fluorophenyl)-1-(1-(6-fluoro-1-oxo-1,2-dihydroisoquinolin-4-yl)ethyl)ureido) ethane-1-sulfonamide (Compounds 381 & 382)

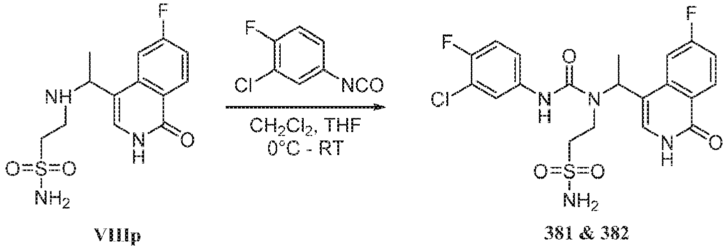

To a suspension of 0.4 g of crude 2-((1-(6-fluoro-1-oxo-1,2-dihydroisoquinolin-4-yl)ethyl)amino)ethane-1-sulfonamide **(VIIIp)** in 10 mL of 1:1 (v/v) methylene chloride:THF at 0 °C was added 0.1 mL (0.6 mmol) of 2-chloro-1-fluoro-4-isocyanatobenzene. The mixture was allowed to warm to room temperature and stirred for 1 h. The mixture was then diluted with 100 mL of water and the resulting precipitate collected by filtration. The crude product was triturated with 10 mL of methyl tert-butyl ether and dried under vacuum to provide 0.36 g (0.74 mmol, 30% over two steps) of racemic 2-(3-(3-chloro-4-fluorophenyl)-1-(1-(6-fluoro-1-oxo-1,2-dihydroisoquinolin-4-yl)ethyl)ureido)ethane-1-sulfonamide. LCMS: *m*/*z* found 485.0/487.0 [M+H]⁺. The enantiomers were subsequently separated by chiral SFC, Chiralpak IC (250 × 30 mm) 5 µ, 75% CO₂/MeOH, Flow rate 100 g/min.

2-(3-(3-Chloro-4-fluorophenyl)-1-(1-(6-fluoro-1-oxo-1,2-dihydroisoquinolin-4-yl)ethyl)ureido) ethane-1-sulfonamide - Enantiomer I **(Compound 381):** LCMS: *m*/*z* found 485.2/487.2 [M+H]⁺, RT = 4.10 min (method A); ¹H NMR (400 MHz, DMSO-*d*₆): δ 8.30-8.34 (m, 1H), 8.02 (m, 4H), 7.76-7.79 (m, 1H), 7.44-7.49 (m, 1H), 7.33-7.38 (m, 3H), 7.29 (s, 1H), 5.68-5.72 (m, 1H), 3.39-3.47 (m, 2H), 2.95-3.02 (m, 1H), 2.53-2.67 (m, 1H), 1.51 (d, 3H); Chiral analytical SFC: RT = 3.85 min, Column: Chiralpak IC (4.6 × 250) mm, 5 µ, 75% CO₂/MeOH, Flow = 3.0 g/min.

2-(3-(3-Chloro-4-fluorophenyl)-1-(1-(6-fluoro-1-oxo-1,2-dihydroisoquinolin-4-yl)ethyl)ureido) ethane-1-sulfonamide - Enantiomer II **(Compound 382):** LCMS: *m*/*z* found 485.2/487.2 [M+H]⁺, RT = 4.10 min (method A); ¹H NMR (400 MHz, DMSO-*d*₆): δ 8.30-8.34 (m, 1H), 8.02 (m, 4H), 7.76-7.79 (m, 1H), 7.44-7.49 (m, 1H), 7.33-7.38 (m, 3H), 7.29 (s, 1H), 5.68-5.72 (m, 1H), 3.39-3.47 (m, 2H), 2.95-3.02 (m, 1H), 2.53-2.67 (m, 1H), 1.51 (d, 3H); Chiral analytical SFC: RT = 4.95 min, Column: Chiralpak IC (4.6 × 250) mm, 5 µ, 75% CO₂/MeOH, Flow = 3.0 g/min.

### Synthesis of 6-methoxyisoquinolin-1(2H)-one (IIg)

### 6-Methoxyisoquinoline 2-oxide

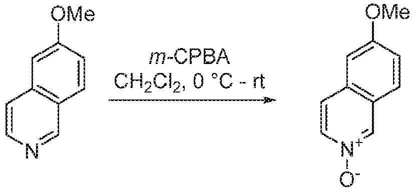

To a solution of 2.0 g (12.6 mmol, 1.0 eq) of 6-methoxyisoquinoline in 20 mL of methylene chloride at 0 °C was added 4.3 g (25.1 mmol, 2.0 eq.) of m-chloroperbenzoic acid portion-wise over approximately 15 min. The mixture was then allowed to warm to room temperature and stirred for 16 h. The mixture was poured into 20 mL of ice-cold water and extracted with 4 × 50 mL of 10% methanol in methylene chloride. The combined organic extracts were washed with 80 mL of 10% aqueous sodium hydroxide solution, 80 mL of brine, dried (Na₂SO₄), filtered and the solvent was removed *in vacuo.* The residue was triturated with 30 mL of methyl tert-butyl ether and dried under high vacuum to provide 2.0 g (11.4 mmol, 90%) of 6-methoxyisoquinoline 2-oxide. LCMS: *m*/*z* found 176.1 [M+H]⁺, RT = 1.53 min; ¹H NMR (300 MHz, DMSO-d₆) δ 8.86 (s, 1H), 8.12-8.09 (m, 1H), 7.84-7.80 (m, 2H), 7.40 (d, 1H), 7.34-7.29 (m, 1H), 3.89 (s, 3H).

### 6-Methoxyisoquinolin-1(2H)-one (IIg)

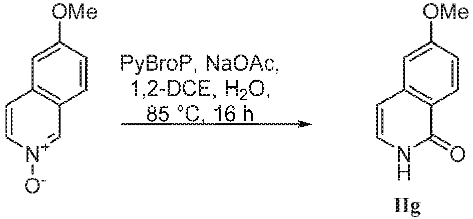

To a suspension of 1.9 g (10.9 mmol, 1.0 eq.) of 6-methoxyisoquinoline 2-oxide in 30 mL of 1,2-dichloroethane was added 2.6 g (32.6 mmol, 3.0 eq.) of sodium acetate followed by 10.1 g (21.71 mmol, 2.0 eq.) of bromotripyrrolidinophosphonium hexafluorophosphate and 5 mL of water, and the mixture was heated at 85 °C for 16 h. The mixture was allowed to cool to room temperature and the solvent was removed *in vacuo.* The residue resuspended in 20 mL of water and extracted with 3 × 50 mL of methylene chloride. The combined organic extracts were washed with 80 mL of brine, dried (Na₂SO₄), filtered and the solvent was removed *in vacuo.* The residue was purified by flash chromatography (SiO₂, eluting with 70% ethyl acetate/petroleum ether in methylene chloride) to provide 1.5 g (8.57 mmol, 78%) of 6-methoxyisoquinolin-1(2*H*)-one (**IIg**). LCMS: *m*/*z* found 176.1 [M+H]⁺, RT = 1.74 min; ¹H NMR (300 MHz, DMSO-d₆) δ 11.04 (bs, 1H), 8.07 (d, 1H), 7.02-7.15 (m, 3H), 6.47 (d, 1H), 3.83 (s, 3H).

### 4-Bromo-6-methoxyisoquinolin-1(2H)-one (IIIg)

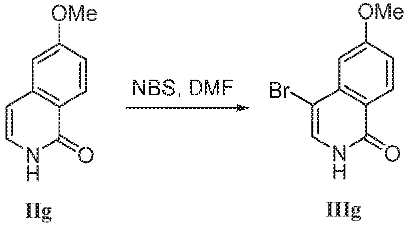

To a solution of 1.0 g (5.7 mmol, 1.0 eq.) of 6-methoxyisoquinolin-1(2*H*)-one **(IIg**) in 5 mL of DMF was added 0.82 g (4.6 mmol, 0.8 eq.) of *N-*bromosuccinimide and the mixture was stirred at room temperature for 24 h. The mixture was diluted with 20 mL of water and the precipitated solid was collected by filtration and dried under high vacuum. The solid was then purified by flash chromatography (SiO₂, eluting with a linear gradient of 30-50% ethyl acetate in petroleum ether) to provide 0.9 g (3.5 mmol, 62%) of 4-bromo-6-methoxyisoquinolin-1(2*H*)-one (**IIIg**). LCMS: *m*/*z* found 253.9/255.9 [M+H]⁺, RT = 1.61 min; ¹H NMR (400 MHz, DMSO-d₆) δ 11.42 (bs, 1H), 8.15 (d, 1H), 7.53 (bd, 1H), 7.16-7.19 (m, 1H), 7.11 (d, 1H), 3.92 (s, 3H).

### 4-Acetyl-6-methoxyisoquinolin-1(2H)-one (XXg)

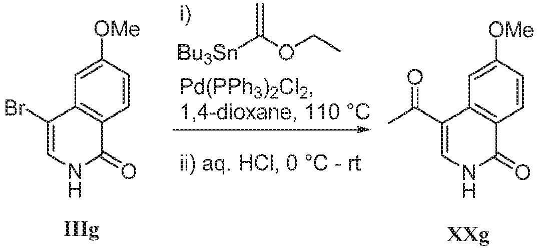

To a solution of 0.80 g (3.16 mmol, 1.0 eq.) of of 4-bromo-6-methoxyisoquinolin-1(2*H*)-one **(IIIg)** in 20 mL of 1,4-dioxane was added 2.81 g (7.0 mmol, 2.5 eq.) of tributyl(1-ethoxyvinyl)tin. The mixture was degassed by purging with argon gas for 5 min and 0.22 g (0.31 mmol, 0.1 eq.) of bis(triphenylphosphine)palladium(II) dichloride was added. The mixture was then heated at 110 °C under an argon atmosphere for 16 h. The mixture was allowed to cool to room temperature and further cooled to 0 °C. The mixture was then diluted with 5 mL of 1 M aqueous HCl and the resulting solution stirred at room temperature for 3 h. The mixture was basified with 40 mL of saturated sodium bicarbonate solution and filtered through CELITE^{®}. The filtrate was extracted with 3 × 50 mL of ethyl acetate and the combined organic extracts were washed with 30 mL of brine, dried (Na₂SO₄), filtered and the solvent was removed *in vacuo.* The residue was purified by flash chromatography (SiO₂, eluting with 40% ethyl acetate/petroleum ether) to provide 0.55 g of 4-acetyl-6-methoxyisoquinolin-1(2*H*)-one (**XXg**). LCMS: *m*/*z* found 218.2 [M+H]⁺, RT = 1.73 min.

### 6-Methoxy-4-(1-(methylamino)ethyl)isoquinolin-1(2H)-one (VIIIcn)

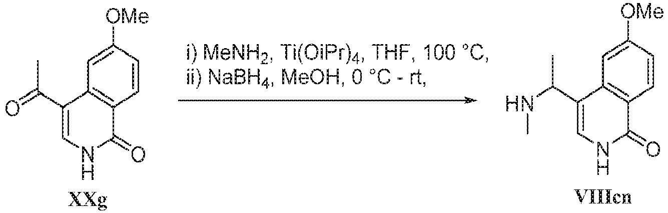

To a solution of 0.55 g of 4-acetyl-6-methoxyisoquinolin-1(2*H*)-one (**XXg**) in 20 mL of THF in a sealed tube under a nitrogen atmosphere was added 6.3 mL (12.6 mmol, 5 eq.) of a 2 M solution of methylamine solution in THF followed by 5.5 mL of titanium isopropoxide, and the mixture was heated to 100 °C for 4 h. The mixture was allowed to cool to room temperature and further cooled to 0 °C. The cooled solution was then diluted with 10 mL of methanol and 0.19 g (5.3 mmol, 2 eq.) of sodium borohydride was added portion-wise. The mixture was then allowed to warm to room temperature and stirred for 2 h. The reaction mixture was diluted with 30 mL of water and extracted with 2 × 60 mL of ethyl acetate. The combined organic extracts were washed with 50 mL of brine, dried (Na₂SO₄), filtered and the solvent was removed *in vacuo.* The residue was purified by reverse-phase chromatography (C18, eluted with a linear gradient of 10-20% [0.1% formic acid in water]/acetonitrile) to provide 0.25 g (1.07 mmol) of 6-methoxy-4-(1-(methylamino)ethyl)isoquion-1(2*H*)-one **(VIIIcn).** LCMS: *m*/*z* found 233.1 [M+H]⁺, RT = 1.05 min; ¹H NMR (400 MHz, DMSO-*d*₆) δ 11.09 (bs, 1H), 8.14-8.17 (m, 2H), 7.34 (d, 1H), 7.08-7.16 (m, 2H), 4.07-4.10 (m, 1H), 3.89 (s, 3H), 2.30 (s, 3H), 1.36 (d, 3H).

### 3-(3-Chloro-4-fluorophenyl)-1-(1-(6-methoxy-1-oxo-1,2-dihydroisoquinolin-4-yl)ethyl)-1-methylurea (Compounds 179 & 180)

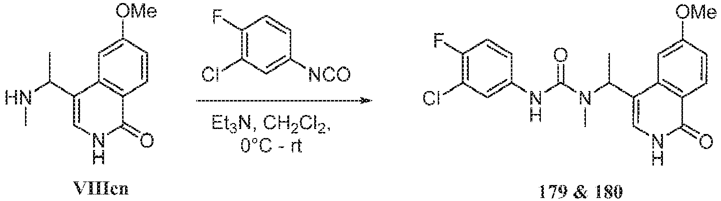

Racemic 3-(3-chloro-4-fluorophenyl)-1-(1-(6-methoxy-1-oxo-1,2-dihydroisoquinolin-4-yl)ethyl)-1-methylurea was synthesized in a similar manner as described above from 6-methoxy-4-(1-(methylamino)ethyl)isoquinolin-1(*2H*)-one (**VIIIcn**) and 2-chloro-1-fluoro-4-isocyanatobenzene. The enantiomers were subsequently separated by SFC, Column: Chiralpak IC (30 × 250 mm) 5 µ, 60% CO₂:MeOH, flow rate 90 g/min.

3-(3-Chloro-4-fluorophenyl)-1-(1-(6-methoxy-1-oxo-1,2-dihydroisoquinolin-4-yl)ethyl)-1-methylurea - Enantiomer I **(Compound 179**) LCMS: *m*/*z* found 404.3/406.3 [M+H]⁺, RT = 7.36 min (Method A); ¹H NMR (400 MHz, DMSO-*d*₆) δ 11.22 (bs, 1H), 8.46 (bs, 1H), 8.14 (d, 1H), 7.82-7.85 (m, 1H), 7.48-7.52 (m, 1H), 7.31 (t, 1H), 7.20 (d, 1H), 7.13 (d, 1H), 7.05-7.08 (m, 1H), 5.81-5.84 (m, 1H), 3.73 (s, 3H), 2.58 (s, 3H), 1.43 (d, 3H); Chiral analytical SFC: RT = 1.93 min, Column: Chiralpak IC-3 (4.6 × 150 mm) 3 µm, 60.0% CO₂/MeOH, Flow = 3.0 g/min.

3-(3-Chloro-4-fluorophenyl)-1-(1-(6-methoxy-1-oxo-1,2-dihydroisoquinolin-4-yl)ethyl)-1-methylurea - Enantiomer II **(Compound 180)** LCMS: *m*/*z* found 404.3/406.3 [M+H]⁺, RT = 7.37 min (Method A); ¹H NMR (400 MHz, DMSO-*d*₆) δ 11.22 (bs, 1H), 8.46 (bs, 1H), 8.14 (d, 1H), 7.82-7.85 (m, 1H), 7.48-7.52 (m, 1H), 7.31 (t, 1H), 7.20 (d, 1H), 7.13 (d, 1H), 7.05-7.08 (m, 1H), 5.81-5.84 (m, 1H), 3.73 (s, 3H), 2.58 (s, 3H), 1.43 (d, 3H); Chiral analytical SFC: RT = 2.66 min, Column: Chiralpak IC-3 (4.6 × 150 mm) 3 µm, 60.0% CO₂/MeOH, Flow = 3.0 g/min.

### Synthesis of 6-chloroisoquinolin-1(2H)-one (IIh)

### 6-Chloroisoquinoline 2-oxide

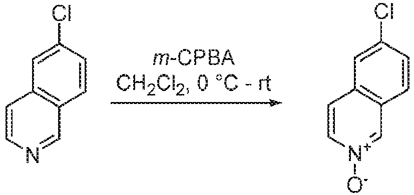

To a solution of 3.0 g (18.4 mmol, 1.0 eq) of 6-chloroisoquinoline in 30 mL of methylene chloride at 0 °C was added 6.3 g (36.8 mmol, 2.0 eq.) of m-chloroperbenzoic acid portion-wise over approximately 15 min. The mixture was then allowed to warm to room temperature and stirred for 6 h. The mixture was poured into 50 mL of ice-cold water and extracted with 5 × 60 mL of methylene chloride. The combined organic extracts were washed with 30 mL of 10% aqueous sodium hydroxide solution, 50 mL of brine, dried (Na₂SO₄), filtered and the solvent was removed *in vacuo.* The residue was triturated with 30 mL of 30 mL of n-pentane and dried under high vacuum to provide 3.4 g of 6-chloroisoquinoline 2-oxide. LCMS: *m*/*z* found 180.1/182.1 [M+H]⁺, RT = 1.53 min; ¹H NMR (300 MHz, DMSO-d₆) δ 8.99 (s, 1H), 8.19-8.22 (m, 1H), 8.11 (d, 1H), 7.90-7.93 (m, 2H), 7.67-7.71 (m, 1H).

### 6-Chloroisoquinolin-1(2H)-one (IIh)

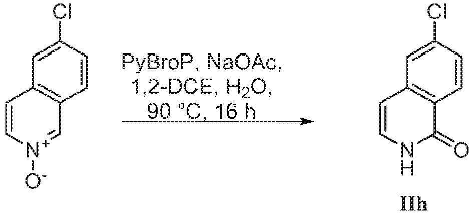

To a suspension of 3.4 g of 6-chloroisoquinoline 2-oxide in 35 mL of 1,2-dichloroethane was added 4.6 g (57.0 mmol) of sodium acetate followed by 17.7 g (38.01 mmol) of bromotripyrrolidinophosphonium hexafluorophosphate and 5 mL of water and the mixture was heated at 90 °C for 16 h. The mixture was allowed to cool to room temperature and the solvent was removed *in vacuo.* The residue resuspended in 20 mL of water and extracted with 3 × 50 mL of methylene chloride. The combined organic extracts were washed with 30 mL of brine, dried (Na₂SO₄), filtered and the solvent was removed *in vacuo.* The residue was triturated with 2 × 20 mL of diethyl ether to provide 1.2 g (6.7 mmol, 35% from 6-chloroisoquinoline) of 6-chloroisoquinolin-1(2*H*)-one (**IIh**). LCMS: *m*/*z* found 180.1/182.1 [M+H]⁺, RT = 1.974 min; ¹H NMR (300 MHz, DMSO-d₆) δ 11.34 (bs, 1H), 8.16 (d, 1H), 7.79 (d, 1H), 7.47-7.50 (m, 1H), 7.21-7.25 (m, 1H), 6.53 (d, 1H).

### 4-Bromo-6-chloroisoquinolin-1(2H)-one (IIIh)

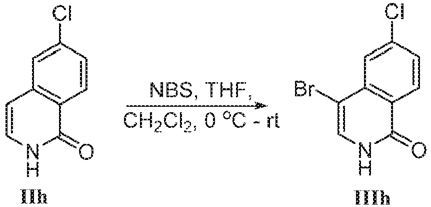

To a solution of 0.6 g (3.4 mmol, 1.0 eq.) of 6-chloroisoquinolin-1(2*H*)-one (**IIh**) in 15 mL of 3:2 v/v THF:methylene chloride at 0 °C was added 0.6 g (3.4 mmol, 1.0 eq.) of *N-*bromosuccinimide. The mixture was allowed to warm to room temperature and stirred for 24 h. The mixture was diluted with 20 mL of water and extracted with 3 × 50 mL of ethyl acetate. The combined organic extracts were washed with 30 mL of brine, dried (Na₂SO₄), filtered and the solvent was removed *in vacuo.* The resulting solid was triturated with 5 mL of chilled methylene chloride and dried under high vacuum to provide 0.6 g (2.3 mmol, 69%) of 4-bromo-6-chloroisoquinolin-1(2*H*)-one (**IIIh**). LCMS: *m*/*z* found 258.0/260.1/262.0 [M+H]⁺, RT = 1.94 min; ¹H NMR (400 MHz, DMSO-d₆) δ 11.76 (bs, 1H), 8.23 (d, 1H), 7.72 (d, 1H), 7.61-7.65 (m, 2H).

### 4-Acetyl-6-chloroisoquinolin-1(2H)-one (XXh)

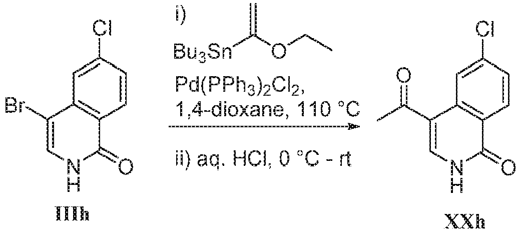

To a solution of 0.80 g (3.1 mmol, 1.0 eq.) of of 4-bromo-6-chloroisoquinolin-1(2*H*)-one (IIIh) in 5 mL of 1,4-dioxane was added 2.81 g (7.0 mmol, 2.5 eq.) of tributyl(1-ethoxyvinyl)tin. The mixture was degassed by purging with argon gas for 5 min and 0.22 g (0.31 mmol, 0.1 eq.) of bis(triphenylphosphine)palladium(II) dichloride was added. The mixture was then heated at 110 °C under an argon atmosphere for 16 h. The mixture was allowed to cool to room temperature and further cooled to 0 °C. The mixture was then diluted with 5 mL of 1 M aqueous HCl and the resulting solution stirred at room temperature for 3 h. The mixture was basified with 40 mL of saturated sodium bicarbonate solution and filtered through a CELITE^{®} pad. The filtrate was extracted with 3 × 50 mL of ethyl acetate and the combined organic extracts were washed with 30 mL of brine, dried (Na₂SO₄), filtered and the solvent was removed *in vacuo.* The residue was purified by flash chromatography (SiO₂, eluting with 60% ethyl acetate/petroleum ether) to provide 0.58 g of 4-acetyl-6-chloroisoquinolin-1(2*H*)-one (**XXh**). LCMS: *m*/*z* found 222.1/224.12 [M+H]⁺, RT = 1.73 min.

### 6-Chloro-4-(1-(methylamino)ethyl)isoquinolin-1(2H)-one (VIIIco)

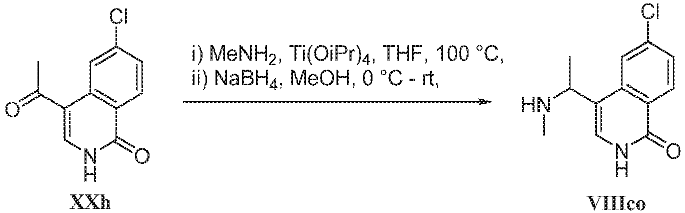

To a solution of 0.5 g (2.26 mmol, 1.0 eq.) of 4-acetyl-6-chloroisoquinolin-1(2*H*)-one **(XXh)** in 5 mL of THF in a sealed tube at room temperature under a nitrogen atmosphere was added 5.6 mL (11.2 mmol, 5 eq.) of a 2 M solution of methylamine solution in THF followed by 5 mL of titanium isopropoxide, and the mixture was heated to 100 °C for 16 h. The mixture was allowed to cool to room temperature and further cooled to 0 °C. The cooled solution was then diluted with 10 mL of methanol and 0.17 g (4.5 mmol, 2 eq.) of sodium borohydride was added portions wise. The mixture was then allowed to warm to room temperature and stirred for 2 h. The reaction mixture was diluted with 30 mL of water and extracted with 2 × 60 mL of ethyl acetate. The combined organic extracts were washed with 50 mL of brine, dried (Na₂SO₄), filtered and the solvent was removed *in vacuo.* The residue was triturated with 10 mL of diethyl ether to provide 0.3 g (1.26 mmol, 56%) of 6-chloro-4-(1-(methylamino)ethyl)lsoquinolin-1(2*H*)-one **(VIIIco).** LCMS: *m*/*z* found 237.1/239.1 [M+H]⁺, RT = 1.11 min; ¹H NMR (400 MHz, DMSO-*d*₆) δ 11.31 (bs, 1H), 8.22 (d, 1H), 8.13 (d, 1H), 7.48-7.52 (m, 1H), 7.16 (s, 1H), 3.84-3.90 (m, 1H), 2.20 (bs, 4H), 1.30 (d, 3H).

### 1-(1-(6-Chloro-1-oxo-1,2-dihydroisoquinolin-4-yl)ethyl)-3-(3-chloro-4-fluorophenyl)-1-methylurea (Compounds 171 & 172)

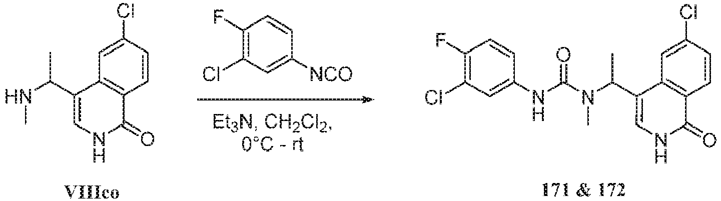

Racemic 1-(1-(6-chloro-1-oxo-1,2-dihydroisoquinolin-4-yl)ethyl)-3-(3-chloro-4-fluorophenyl)-1-methylurea was synthesized in a similar manner as described above from 6-chloro-4-(1-(methylamino)ethyl)isoquinolin-1(2*H*)-one **(VIIIco)** and 2-chloro-1-fluoro-4-isocyanatobenzene. The enantiomers were subsequently separated by SFC, Column: Chiralpak IG (30 × 250 mm) 5 µ, 75% CO₂:MeOH, flow rate 100 g/min.

1-(1-(6-Chloro-1-oxo-1,2-dihydroisoquinolin-4-yl)ethyl)-3-(3-chloro-4-fluorophenyl)-1-methylurea - Enantiomer I **(Compound 171**) LCMS: *m*/*z* found 408.2/410.2/412.2 [M+H]⁺, RT = 7.19 min (Method A); ¹H NMR (400 MHz, DMSO-*d*₆) δ 11.53 (bs, 1H), 8.49 (bs, 1H), 8.23 (d, 1H), 7.80-7.83 (m, 2H), 7.52-7.55 (m, 1H), 7.45-7.50 (m, 1H), 7.20 (t, 1H), 7.21 (s, 1H), 5.78-5.83 (m, 1H), 2.58 (s, 3H), 1.43 (d, 3H); Chiral analytical SFC: RT = 4.43 min, Column: Chiralpak IG-3 (4.6 × 150 mm) 3 µm, 80.0% CO₂/MeOH, Flow = 3.0 g/min.

1-(1-(6-Chloro-1-oxo-1,2-dihydroisoquinolin-4-yl)ethyl)-3-(3-chloro-4-fluorophenyl)-1-methylurea - Enantiomer II **(Compound 172**) LCMS: *m*/*z* found 408.2/410.2/412.2 [M+H]⁺, RT = 7.19 min (Method A); ¹H NMR (400 MHz, DMSO-*d*₆) δ 11.53 (bs, 1H), 8.49 (bs, 1H), 8.23 (d, 1H), 7.80-7.83 (m, 2H), 7.52-7.55 (m, 1H), 7.45-7.50 (m, 1H), 7.20 (t, 1H), 7.21 (s, 1H), 5.78-5.83 (m, 1H), 2.58 (s, 3H), 1.43 (d, 3H); Chiral analytical SFC: RT = 7.16 min, Column: Chiralpak IG-3 (4.6 × 150 mm) 3 µm, 80.0% CO₂/MeOH, Flow = 3.0 g/min.

### Synthesis of 7-fluoroisoquinolin-1(2H)-one (IIc)

### (E)-3-(4-Fluorophenyl)acryloyl azide

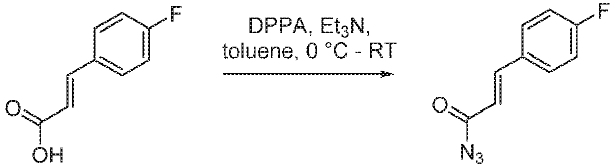

To a solution of 20.0 g (120.5 mmol, 1.0 eq.) of (E)-3-(4-fluorophenyl)acrylic acid in 100 mL of toluene at 0 °C under an argon atmosphere was added 25 mL (180 mmol, 1.5 eq.) of triethylamine followed by 29.8 g (108.4 mmol, 0.9 eq.) of diphenylphosphoryl azide. The mixture was allowed to warm to room temperature and stirred for 2 h. The solvent was removed *in vacuo* and the residue was purified by MPLC (REVELERIS^{®} Silica column, eluting with a linear gradient of 10-20% ethyl acetate/petroleum ether) to provide 13.0 g (62.2 mmol, 51%) of (*E*)-3-(4-fluorophenyl)acryloyl azide. ¹H NMR (400 MHz, CDCl₃): δ 7.71 (d, 1H), 7.51-7.55 (m, 2H), 7.07-7.12 (m, 2H), 6.34 (d, 1H).

### 7-Fluoroisoquinolin-1(2H)-one (IIc)

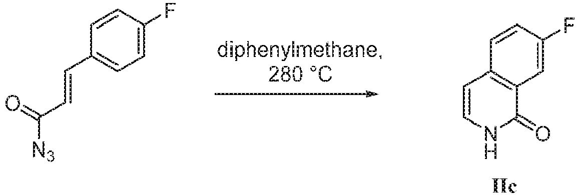

A solution of 13.0 g (73.4 mmol, 1.0 eq.) of (E)-3-(4-fluorophenyl)acryloyl azide in 50 mL of diphenylmethane was heated to 100 °C for 30 min. The temperature was subsequently increased to 280 °C and the mixture stirred for a further 3 h. The mixture was allowed to cool to room temperature and the resulting heterogeneous mixture was diluted with 200 mL of n-heptane and stirred for 30 min. The solids were collected by filtration and dried under vacuum. The above detailed reaction was conducted in duplicate and the obtained solid from both batches were combined, triturated with 100 mL of *n*-heptane, filtered and dried under vacuum to provide 13.0 g (79.8 mmol, 54%) of 7-fluorolsoquinolin-1(2*H*)-one (**IIc**). LCMS: *m*/*z* found 164.12 [M+H]⁺, RT = 1.74min; ¹H NMR (300 MHz, DMSO-*d*₆): δ 11.27 (bs, 1H), 7.74-7.85 (m, 2H), 7.56-7.63 (m, 1H), 7.14-7.21 (m, 1H), 6.59 (d, 1H).

### 4-Bromo-7-fluoroisoquinolin-1(2H)-one (IIIc)

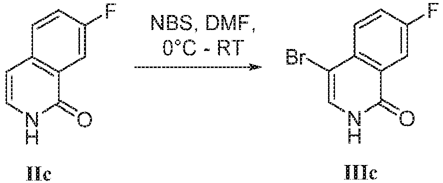

To a solution of 2.0 g (12.2 mmol, 1.0 eq.) of 7-fluoroisoquinolin-1(2*H*)-one **(IIc)** in 20 mL of DMF at 0 °C was added 2.4 g (13.5 mmol, 1.1 eq.) of N-bromo succinimide portion-wise over approximately 15 min. The resulting mixture was allowed to warm to room temperature and stirred for 2 h. The reaction was quenched by the addition of 50 mL of saturated sodium bicarbonate solution and concentrated *in vacuo.* The resulting residue was diluted with 80 mL of water and the resulting solids were collected by filtration, washed with 50 mL of petroleum ether and dried under high vacuum to provide 1.5 g (6.2 mmol, 51%) of 4-bromo-6,7-difluoroisoquinolin-1(2*H*)-one (**IIIc**). LCMS: *m*/*z* found 241.9/243.9 [M+H]⁺, RT = 2.04 min; ¹H NMR (400 MHz, CDCl₃): δ 10.5 (bs, 1H), 8.07-8.10 (m, 1H), 7.89-7.93 (m, 1H), 7.50-7.55 (m, 1H), 7.37 (s, 1H).

### 4-Acetyl-7-fluoroisoquinolin-1(2H)-one (XXc)

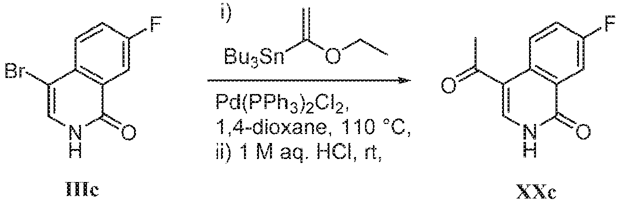

To a solution of 1.5 g (6.2 mmol, 1.0 eq.) of 7-fluoroisoquinolin-1(2*H*)-one (**IIIc**) in 15 mL of 1,4-dioxane was added 6.73 g (18.7 mmol, 3.0 eq.) of tributyl(1-ethoxyvinyl)stannane. The mixture was purged with nitrogen gas for 5 min and 0.22 g (0.31 mmol, 0.05 eq.) of Pd(PPh₃)₂Cl₂ was added, and then heated to 110 °C for 16 h. The reaction mixture was allowed to cool to room temperature and 30 mL of 1 M aqueous HCl was added and stirring was continued for an additional 30 min. The reaction mixture was then basified with 50 mL of saturated sodium bicarbonate solution and extracted with 3 × 100 mL of ethyl acetate. The combined organic extracts were washed with 100 mL of water, 100 mL of brine, dried (Na₂SO₄), filtered and the solvent was removed *in vacuo.* The residue was purified by MPLC (REVELERIS^{®} silica column, eluting with a linear gradient of 30-50% ethyl acetate/petroleum ether) to provide 1.0 g (4.87 mmol, 78%) of 4-acetyl-7-fluoroisoquinolin-1(2*H*)-one (**XXc**). LCMS: *m*/*z* found 206.1 [M+H]⁺, ¹H NMR (400 MHz, CDCl₃): δ 11.5 (bs, 1H), 9.09-9.13(m, 1H), 8.02-8.09 (m, 2H), 7.45-7.55 (m, 1H), 2.60 (s, 3H).

### 7-Fluoro-4-(1-(methylamino)ethyl)isoquinolin-1(2H)-one (VIIIq)

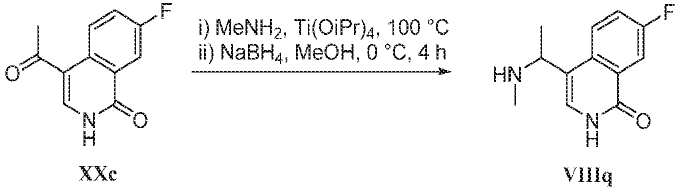

To a solution of 1.0 g (4.9 mmol, 1.0 eq.) of 4-acetyl-7-fluoroisoquinolin-1(2*H*)-one **(XXc)** in 10 mL of THF under an argon atmosphere was added 10 mL (20.0 mmol, 4.1 eq.) of a 2 M solution methylamine in THF followed by 10 mL of titanium isopropoxide and the mixture was heated at 100 °C for 16 h. Th mixture was allowed to cool to room temperature and further cooled to 0 °C. Following dilution with 3 mL of methanol, 0.74 g (19.48 mmol, 4.0 eq.) of sodium borohydride was added portion-wise over approximately 10 min and stirring was continued for 4 h. The reaction mixture was diluted with 100 mL of water and extracted with 4 × 80 mL of 5% methanol in methylene chloride. The combined organic extracts were washed with 50 mL of water, 50 mL of brine, dried (Na₂SO₄), filtered and the solvent was removed *in vacuo* to provide 800 mg of 7-fluoro-4-(1-(methylamino)ethyl)isoquinolin-1(2*H*)-one (**VIIIq**). LCMS: *m*/*z* found 221.0 [M+H]⁺.

### 3-(3-Chloro-4-fluorophenyl)-1-(1-(7-fluoro-1-oxo-1,2-dihydroisoquinolin-4-yl)ethyl)-1-methylurea (Compounds 257, 263 & 264)

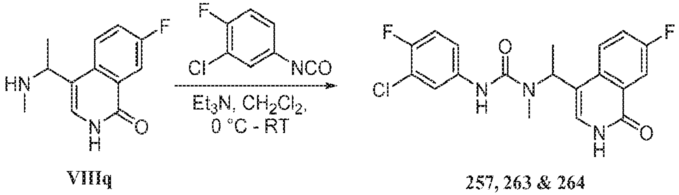

To a solution of 0.6 g of 7-fluoro-4-(1-(methylamino)ethyl)isoquinolin-1(2*H*)-one **(VIIIq)** in 10 mL of methylene chloride at 0 °C was added 1.1 mL (8.16 mmol) of triethylamine followed by 0.37 g (2.18 mmol) of 2-chloro-1-fluoro-4-isocyanatobenzene and the mixture was stirred at room temperature for 1 h. The mixture was then diluted with 100 mL of water and extracted with 3 × 150 mL of methylene chloride. The combined organic extracts were washed with 100 mL of brine, dried (Na₂SO₄), filtered and the solvent was removed *in vacuo.* The residue was purified by MPLC (REVELERIS^{®} silica column, eluting with a linear gradient of 15-20% of [30% methanol in methylene chloride]/methylene chloride) to provide 150 mg (0.51 mmol) of racemic 3-(3-chloro-4-fluorophenyl)-1-(1-(7-fluoro-1-oxo-1,2-dihydroisoquinolin-4-yl)ethyl)-1-methylurea (**Compound 257**). LCMS: *m*/*z* found 392.2/394.2 [M+H]⁺, RT = 6.77 min (method A); ¹H NMR (400 MHz, DMSO-*d*₆): δ 11.54 (bs, 1H), 8.46 (s 1H), 7.85-7.91 (m, 2H), 7.76-7.80 (m, 1H), 7.65-7.71 (m, 1H), 7.48-7.52 (m, 1H), 7.31 (t, 1H), 7.14 (d, 1H), 5.82-5.87 (m, 1H), 2.58 (s, 3H), 1.43 (d, 3H).
The enantiomers were subsequently separated by chiral SFC, Chiralpak IC (250 × 30 mm) 5 µ, 90% CO₂/MeOH, Flow rate 100 g/min.

3-(3-Chloro-4-fluorophenyl)-1-(1-(7-fluoro-1-oxo-1,2-dihydroisoquinolin-4-yl)ethyl)-1-methylurea - Enantiomer I **(Compound 263**): LCMS: *m*/*z* found 392.2/394.2 [M+H]⁺, RT = 6.77 min (method A); ¹H NMR (400 MHz, DMSO-*d*₆): δ 11.54 (bs, 1H), 8.46 (s 1H), 7.85-7.91 (m, 2H), 7.76-7.80 (m, 1H), 7.65-7.71 (m, 1H), 7.48-7.52 (m, 1H), 7.31 (t, 1H), 7.14 (d, 1H), 5.82-5.87 (m, 1H), 2.58 (s, 3H), 1.43 (d, 3H); Chiral analytical SFC: RT = 1.86 min, Column: Chiralpak IC (4.6 × 250) mm, 5 µ, 70% CO₂/MeOH, Flow = 3.0 g/min.

3-(3-Chloro-4-fluorophenyl)-1-(1-(7-fluoro-1-oxo-1,2-dihydroisoquinolin-4-yl)ethyl)-1-methylurea - Enantiomer II **(Compound 264**): LCMS: *m*/*z* found 392.2/394.2 [M+H]⁺, RT = 6.77 min (method A); ¹H NMR (400 MHz, DMSO-*d*₆): δ 11.54 (bs, 1H), 8.46 (s 1H), 7.85-7.91 (m, 2H), 7.76-7.80 (m, 1H), 7.65-7.71 (m, 1H), 7.48-7.52 (m, 1H), 7.31 (t, 1H), 7.14 (d, 1H), 5.82-5.87 (m, 1H), 2.58 (s, 3H), 1.43 (d, 3H); Chiral analytical SFC: RT = 2.57 min, Column: Chiralpak IC (4.6 × 250) mm, 5 µ, 70% CO₂/MeOH, Flow = 3.0 g/min.

### 3-(4-Fluorophenyl)-1-(1-(7-fluoro-1-oxo-1,2-dihydroisoquinolin-4-yl)ethyl)-1-methylurea (Compound 258)

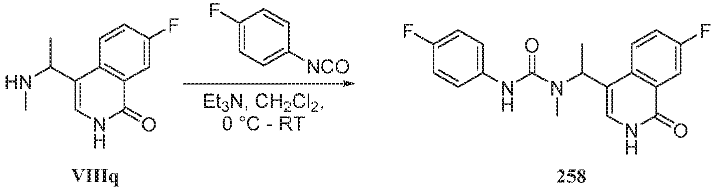

Racemic 3-(4-fluorophenyl)-1-(1-(7-fluoro-1-oxo-1,2-dihydroisoquinolin-4-yl)ethyl)-1-methylurea **(Compound 258**) was synthesized in a similar manner as described above from 7-fluoro-4-(1-(methylamino)ethyl)isoquinolin-1(2*H*)-one (**VIIIq**) and 4-fluorophenylisocyanate. LCMS: *m*/*z* found 358.2/360.2 [M+H]⁺, RT = 7.54 min (Method A); ¹H NMR (400 MHz, CD₃OD) δ 7.99 (dd, 1H), 7.91 (dd, 1H), 7.55 (ddd, 1H), 7.37-7.47 (m, 2H), 7.23 (d, 1H), 6.97-7.10 (m, 2H), 5.93-6.03 (m, 1H), 2.65 (s, 3H), 1.54 (d, 3H).

### 2-((1-(7-Fluoro-1-oxo-1,2-dihydroisoquinolin-4-yl)ethyl)amino)ethane-1-sulfonamide (VIIIr)

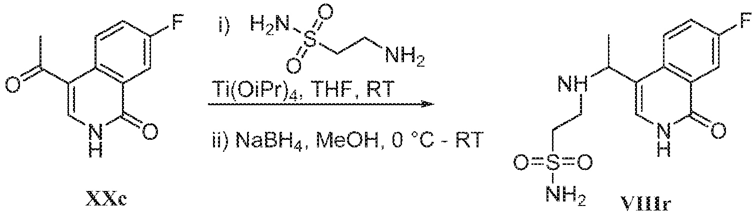

To a solution of 0.5 g (2.4 mmol, 1.0 eq.) of 4-acetyl-7-fluorolsoquinolin-1(2*H*)-one **(XXc)** in 10 mL of THF under a nitrogen atmosphere was added 0.45 g (3.65 mmol, 1.5 eq.) of 2-aminoethane-1-sulfonamide followed by 5 mL of titanium isopropoxide and the mixture was stirred at room temperature for 48 h. The mixture was cooled to 0 °C, diluted with 1 mL of methanol and 0.37 g (9.72 mmol, 4.0 eq.) of sodium borohydride was added portion-wise over approximately 10 min. After stirring at 0 °C for 4 h, the mixture was diluted with 100 mL of brine and 200 mL of ethyl acetate. The resulting heterogeneous mixture was filtered through CELITE^{®} and the pad was washed with 40 mL of ethyl acetate. The layers were separated, and the aqueous phase was extracted with 2 × 100 mL of ethyl acetate. The combined organic extracts were washed with 100 mL of brine, dried (Na₂SO₄), filtered and the solvent was removed *in vacuo* to provide 350 mg of 2-((1-(7-fluoro-1-oxo-1,2-dihydroisoquinolin-4-yl)ethyl)amino)ethane-1-sulfonamide **(VIIIr).** LCMS: *m*/*z* found 314.2 [M+H]⁺.

### 2-(3-(3-Chloro-4-fluorophenyl)-1-(1-(7-fluoro-1-oxo-1,2-dihydroisoquinolin-4-yl)ethyl)ureido) ethane-1-sulfonamide (Compounds 383 & 384)

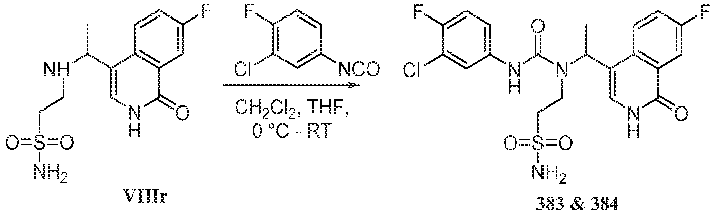

Racemic 2-(3-(3-chloro-4-fluoropheny1)-1-(1-(7-fluoro-1-oxo-1,2-dihydroisoquinolin-4-yl)ethyl)ureido) ethane-1-sulfonamide was synthesized in a similar manner as described above from 2-((1-(7-fluoro-1-oxo-1,2-dihydroisoquinolin-4-yl)ethyl)amino)ethane-1-sulfonamide **(VIIIr)** and 2-chloro-1-fluoro-4-isocyanatobenzene. The enantiomers were subsequently separated by chiral SFC, R,R (Whelk-01) (250 × 30 mm) 5 µ, 70% CO₂/MeOH, Flow rate 100 g/min.

2-(3-(3-Chloro-4-fluorophenyl)-1-(1-(7-fluoro-1-oxo-1,2-dihydroisoquinolin-4-yl)ethyl)ureido) ethane-1-sulfonamide - Enantiomer I **(Compound 383**): LCMS: *m*/*z* found 485.2/487.2 [M+H]⁺, RT = 4.13 min (Method A); ¹H NMR (400 MHz, DMSO-*d*₆): δ 11.58 (bs, 1H), 8.71 (bs, 1H), 7.90-7.93 (m, 1H), 7.81-7.83 (m, 1H), 7.66-7.75 (m, 2H), 7.47-7.51 (m, 1H), 7.35 (t, 1H), 7.22 (s, 1H), 6.84 (bs, 2H), 5.73-5.78 (m, 1H), 3.40-3.47 (m, 2H), 2.98-3.05 (m, 1H), 2.54-2.62 (m, 1H), 1.51 (d, 3H); Chiral analytical SFC: RT = 3.13 min, Column: R,R (Whelk-01) (4.6 × 250) mm, 5 µ, 65% CO₂/MeOH, Flow = 3.0 g/min.

2-(3-(3-Chloro-4-fluorophenyl)-1-(1-(7-fluoro-1-oxo-1,2-dihydroisoquinolin-4-yl)ethyl)ureido) ethane-1-sulfonamide - Enantiomer II **(Compound 384**): LCMS: *m*/*z* found 485.2/487.2 [M+H]⁺, RT = 4.13 min (Method A); ¹H NMR (400 MHz, DMSO-*d*₆): δ 11.58 (bs, 1H), 8.71 (bs, 1H), 7.90-7.93 (m, 1H), 7.81-7.83 (m, 1H), 7.66-7.75 (m, 2H), 7.47-7.51 (m, 1H), 7.35 (t, 1H), 7.22 (s, 1H), 6.84 (bs, 2H), 5.73-5.78 (m, 1H), 3.40-3.47 (m, 2H), 2.98-3.05 (m, 1H), 2.54-2.62 (m, 1H), 1.51 (d, 3H); Chiral analytical SFC: RT = 4.25 min, Column: R,R (Whelk-01) (4.6 × 250) mm, 5 µ, 65% CO₂/MeOH, Flow = 3.0 g/min.

### Synthesis of 7-methoxyisoquinolin-1(2H)-one (IIi)

### 7-Methoxyisoquinoline 2-oxide

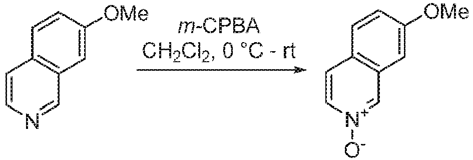

To a solution of 5.0 g (31.4 mmol, 1.0 eq) of 7-methoxyisoquinoline in 50 mL of methylene chloride at 0 °C was added 10.8 g (62.9 mmol, 2.0 eq.) of m-chloroperbenzoic acid portion-wise over approximately 15 min. The mixture was then allowed to warm to room temperature and stirred for 16 h. The mixture was poured into 50 mL of ice-cold water and extracted with 4 × 50 mL methylene chloride. The combined organic extracts were washed with 30 mL of 10% aqueous sodium hydroxide solution, 50 mL of brine, dried (Na₂SO₄), filtered and the solvent was removed *in vacuo.* The residue was triturated with 40 mL of diethyl ether and dried under high vacuum to provide 4.4 g (25.1 mmol, 82%) of 7-methoxyisoquinoline 2-oxide. LCMS: *m*/*z* found 176.1 [M+H]⁺, RT = 1.59 min; ¹H NMR (300 MHz, DMSO-d₆) δ 8.60 (s, 1H), 8.03 (d, 1H), 7.68 (d, 1H), 7.58 (d, 1H), 7.22 (d, 1H), 6.96 (s, 1H), 3.94 (s, 3H).

### 7-Methoxyisoquinolin-1(2H)-one (IIi)

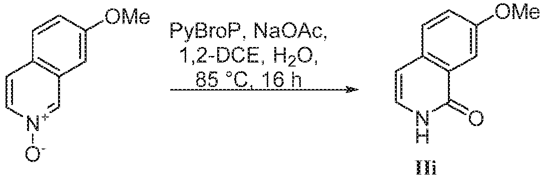

To a suspension of 4.4 g (25.1 mmol, 1.0 eq.) of 7-methoxyisoquinoline 2-oxide in 40 mL of 1,2-dichloroethane was added 6.1 g (75.4 mmol, 2.0 eq.) of sodium acetate followed by 23.4 g (50.3 mmol, 2.0 eq.) of bromotripyrrolidinophosphonium hexafluorophosphate and 5 mL of water and the mixture was heated at 85 °C for 16 h. The mixture was allowed to cool to room temperature and the solvent was removed *in vacuo.* The residue resuspended in 50 mL of water and extracted with 3 × 50 mL of methylene chloride. The combined organic extracts were washed with 80 mL of brine, dried (Na₂SO₄), filtered and the solvent was removed *in vacuo.* The residue was triturated with 50 mL of methyl tert-butyl ether to provide 2.0 g (11.4 mmol, 78%) of 7-methoxyisoquinolin-1(2*H*)-one (**IIi**). LCMS: *m*/*z* found 176.1 [M+H]⁺, RT = 1.78 min; ¹H NMR (300 MHz, DMSO-d₆) δ 11.19 (bs, 1H), 7.59-7.62 (m, 2H), 7.30-7.33 (m, 1H), 7.04 (t, 1H), 6.51 (d, 1H), 3.85 (s, 3H).

### 4-Bromo-7-methoxyisoquinolin-1(2H)-one (IIIi)

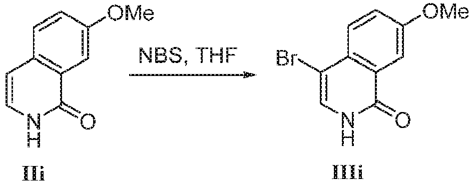

To a solution of 1.9 g (10.9 mmol, 1.0 eq.) of 7-methoxyisoquinolin-1(2*H*)-one **(IIi)** in 40 mL of THF at 0 °C was added 1.7 g (9.8 mmol, 0.9 eq.) of *N-*bromosuccinimide and the mixture was stirred at 0 °C for 24 h. The mixture was diluted with 60 mL of water and the precipitated solid was collected by filtration and dried under high vacuum. The solid was then triturated with 20 mL of ethanol to provide 1.8 g (7.1 mmol, 62%) of 4-bromo-7-methoxyisoquinolin-1(2*H*)-one (**IIIi**). LCMS: *m*/*z* found 253.9/255.9 [M+H]⁺, RT = 1.65 min; ¹H NMR (400 MHz, DMSO-d₆) δ 11.52 (bs, 1H), 7.71 (d, 1H), 7.66 (d, 1H), 7.45-7.48 (m, 1H), 7.41 (s, 1H), 3.90 (s, 3H).

### 4-Acetyl-7-methoxyisoquinolin-1(2H)-one (XXi)

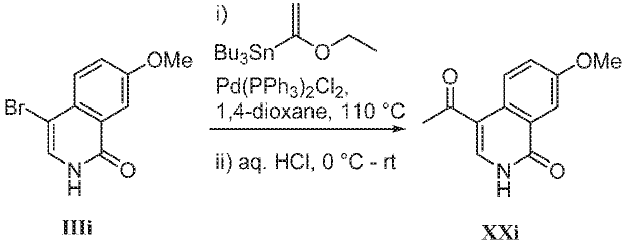

To a solution of 1.3 g (5.13 mmol, 1.0 eq.) of of 4-bromo-7-methoxyisoquinolin-1(2H)-one **(IIIi)** in 15 mL of 1,4-dioxane was added 4.6 g (12.8 mmol, 2.5 eq.) of tributyl(1-ethoxyvinyl)tin. The mixture was degassed by purging with argon gas for 5 min and 0.36 g (0.51 mmol, 0.1 eq.) of bis(triphenylphosphine)palladium(II) dichloride was added. The mixture was then heated at 110 °C under an argon atmosphere for 16 h. The mixture was allowed to cool to room temperature and further cooled to 0 °C. The mixture was then diluted with 15 mL of 1 M aqueous HCl and the resulting solution stirred at room temperature for 3 h. The mixture was basified with 40 mL of saturated sodium bicarbonate solution and filtered through CELITE^{®}. The filtrate was extracted with 3 × 50 mL of ethyl acetate and the combined organic extracts were washed with 30 mL of brine, dried (Na₂SO₄), filtered and the solvent was removed *in vacuo.* The residue was triturated with *n*-pentane to provide 0.8 g (3.68 mmol, 72%) of 4-acetyl-7-methoxyisoquinolin-1(2*H*)-one (**XXi**). LCMS: *m*/*z* found 218.2 [M+H]⁺, RT = 1.84 min.

### 7-Methoxy-4-(1-(methylamino)ethyl)isoquinolin-1(2H)-one (VIIIcp)

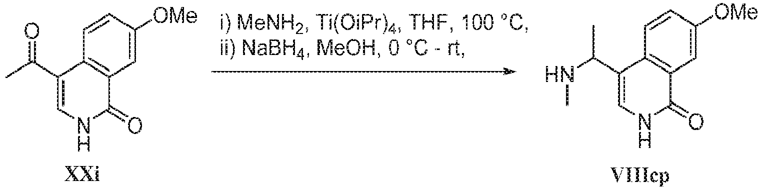

To a solution of 0.28 g (1.3 mmol, 1.0 eq.) of 4-acetyl-7-methoxyisoquinolin-1(2*H*)-one **(XXi)** in 10 mL of THF in a sealed tube under a nitrogen atmosphere was added 3.2 mL (6.4 mmol, 5.0 eq.) of a 2 M solution of methylamine in THF followed by 3 mL of titanium isopropoxide, and the mixture was heated to 100 °C for 4 h. The mixture was allowed to cool to room temperature and further cooled to 0 °C. The cooled solution was then diluted with 10 mL of methanol and 0.09 g (2.6 mmol, 2 eq.) of sodium borohydride was added portion-wise. The mixture was then allowed to warm to room temperature and stirred for 2 h. The reaction mixture was diluted with 30 mL of water and extracted with 2 × 60 mL of ethyl acetate. The combined organic extracts were washed with 50 mL of brine, dried (Na₂SO₄), filtered and the solvent was removed *in vacuo.* The residue was purified by reverse-phase chromatography (C18, eluted with a linear gradient of 5-15% [0.1% formic acid in water]/acetonitrile) to provide 0.15 g (0.64 mmol, 93%) of 7-methoxy-4-(1-(methylamino)ethyl)isoquinolin-1(2*H*)-one **(VIIIcp).** LCMS: *m*/*z* found 233.1 [M+H]⁺, RT = 1.43 min; ¹H NMR (400 MHz, DMSO-d₆) δ 11.23 (bs, 1H), 8.24 (bs, 1H), 7.82 (d, 1H), 7.67 (d, 1H), 7.33-7.36 (m, 1H), 7.07 (s, 1H), 3.86-4.12 (m, 1H), 3.86 (s, 3H), 2.28 (s, 3H), 1.35 (d, 3H).

### 3-(3-Chloro-4-fluorophenyl)-1-(1-(7-methoxy-1-oxo-1,2-dihydroisoquinolin-4-yl)ethyl)-1-methylurea (Compounds 173,177 & 178)

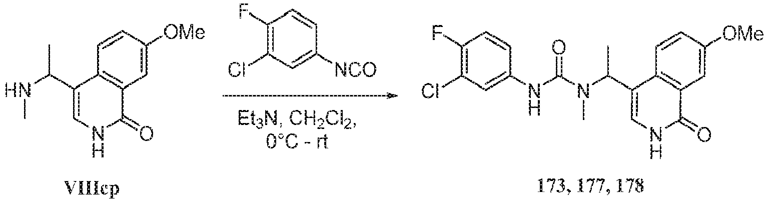

Racemic 3-(3-chloro-4-fluorophenyl)-1-(1-(7-methoxy-1-oxo-1,2-dihydroisoquinolin-4-yl)ethyl)-1-methylurea **(Compound 173)** was synthesized in a similar manner as described above from 7-methoxy-4-(1-(methylamino)ethyl)isoquinolin-1(2*H*)-one **(VIIIcp)** and 2-chloro-1-fluoro-4-isocyanatobenzene. The enantiomers were subsequently separated by SFC, Column: Chiralpak IC (30 × 250 mm) 5 µ, 75% CO₂:MeOH, flow rate 90 g/min.

3-(3-Chloro-4-fluorophenyl)-1-(1-(7-methoxy-1-oxo-1,2-dihydroisoquinolin-4-yl)ethyl)-1-methylurea - Enantiomer I **(Compound 177**) LCMS: *m*/*z* found 404.2/406.2 [M+H]⁺, RT = 7.19 min (Method A); ¹H NMR (400 MHz, DMSO-*d*₆) δ 11.29 (s, 1H), 8.46 (bs, 1H), 7.85-7.87 (m, 1H), 7.65 (d, 2H), 7.48-7.52 (m, 1H), 7.36-7.39 (m, 1H), 7.33 (s, 1H), 7.31 (t, 1H), 5.82 (d, 1H), 3.84 (s, 3H), 2.57 (s, 3H), 1.41 (d, 3H); Chiral analytical SFC: RT = 5.18 min, Column: Chiralpak IC (4.6 × 250 mm) 5 µm, 60.0% CO₂/MeOH, Flow = 3.0 g/min.

3-(3-Chloro-4-fluorophenyl)-1-(1-(7-methoxy-1-oxo-1,2-dihydroisoquinolin-4-yl)ethyl)-1-methylurea - Enantiomer II **(Compound 178)** LCMS: *m*/*z* found 404.2/406.2 [M+H]⁺, RT = 7.19 min (Method A); ¹H NMR (400 MHz, DMSO-*d*₆) δ 11.29 (s, 1H), 8.46 (bs, 1H), 7.85-7.87 (m, 1H), 7.65 (d, 2H), 7.48-7.52 (m, 1H), 7.36-7.39 (m, 1H), 7.33 (s, 1H), 7.31 (t, 1H), 5.82 (d, 1H), 3.84 (s, 3H), 2.57 (s, 3H), 1.41 (d, 3H); Chiral analytical SFC: RT = 9.36 min, Column: Chiralpak IC (4.6 × 250 mm) 5 µm, 60.0% CO₂/MeOH, Flow = 3.0 g/min.

### 1-(1-(7-Chloro-1-oxo-1,2-dihydroisoquinolin-4-yl)ethyl)-3-(3-chloro-4-fluorophenyl)-1-methylurea (Compound 271)

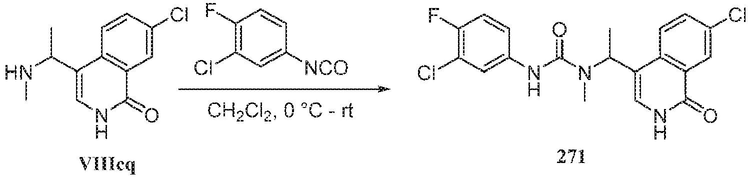

Racemic 1-(1-(7-chloro-1-oxo-1,2-dihydroisoquinolin-4-yl)ethyl)-3-(3-chloro-4-fluorophenyl)-1-methylurea **(Compound 271)** was synthesized in an analogous manner as described above from 7-chloro-4-(1-(methylamino)ethyl)isoquinolin-1(2*H*)-one (**VIIIcq**, derived from 7-chloroisoquinoline) and 2-chloro-1-fluoro-4-isocyanatobenzene. LCMS: *m*/*z* found 408.1/410.1 [M+H]⁺, RT = 4.53 min (Method A); ¹H NMR (400 MHz, DMSO-*d*₆) δ 11.60 (m, 1H), 8.46 (s, 1H), 8.17 (d, 1H), 7.79-7.90 (m, 2H), 7.73 (d, 1H), 7.49 (m, 1H), 7.31 (t, 1H), 7.19 (m, 1H), 5.83 (q, 1H), 2.58 (s, 3H), 1.42 (d, 3H).

### Synthesis of 8-fluoroisoquinolin-1(2H)-one (IIk)

### 2,2-Diethoxy-N (2-fluorobenzyl)ethan-1-amine

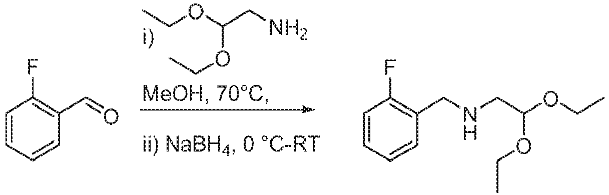

To a solution of 25.0 g (201.6 mmol, 1.0 eq.) of 2-fluorobenzaldehyde in 250 mL of methanol was added 26.8 g (201.6 mmol, 1.0 eq.) of 2,2-diethoxyethanamine and the mixture was heated at 70 °C for 2 h. The mixture was allowed to cool to room temperature, further cooled to 0 °C, and 7.65 g (201.6 mmol, 1.0 eq.) of sodium borohydride was added portion-wise. The mixture was then allowed to warm to room temperature and stirred for 16 h. The solvent was removed *in vacuo* and the residue was resuspended in 250 mL of ice-cold water and extracted with 2 × 500 mL of methylene chloride. The combined organic extracts were washed with 250 mL of brine, dried (Na₂SO₄), filtered and the solvent was removed *in vacuo* to provide 40.0 g (165.7 mmol, 82%) of 2,2-diethoxy-*N*-(2-fluorobenzyl)ethan-1-amine. LCMS: *m*/*z* found 242.1 [M+H]⁺; ¹H NMR (400 MHz, CDCl₃) δ 7.31 -7.35 (m, 1H), 7.20-7.25 (m, 1H), 7.07-7.11 (m, 1H), 7.00-7.04 (m, 1H), 4.61 (t, 1H), 3.86 (s, 2H), 3.64-3.72 (m, 2H), 3.48-3.56 (m, 2H), 2.73-2.75 (d, 2H), 1.56 (bs, 1H), 1.18-1.22 (m, 6H).
The above detailed reaction was conducted on multiple batches as described above with consistent results.

### N-(2,2-Diethoxyethyl)-N-(2-fluorobenzyl)-4-methylbenzenesulfonamide

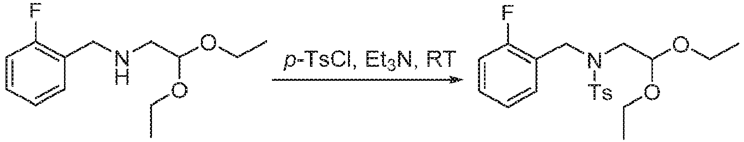

To a solution of 40.0 g (165.7 mmol, 1.0 eq.) of 2,2-diethoxy-N-(2-fluorobenzyl)ethan-1-amine in 400 mL of methylene chloride was added 69.8 mL (497.1 mmol, 3.0 eq.) of triethylamine followed by 49.2 g (248.6 mmol, 1.5 eq.) of *p-*toluenesulfonyl chloride and the mixture was stirred at room temperature for 16 h. The mixture was then diluted with 400 mL of ice-cold water and extracted with 2 × 800 mL of methylene chloride. The combined organic extracts were washed with 500 mL of brine, dried (Na₂SO₄), filtered and the solvent was removed *in vacuo.* The above detailed reaction was repeated in duplicate and the crude products from the three batches were combined and subsequently purified by flash column chromatography (SiO₂, eluting with a linear gradient of 30-50% of ethyl acetate in petroleum ether) to provide 130.0 g (328.69 mmol, 65%) of *N-*(2,2-diethoxyethyl)-*N*-(2-fluorobenzyl)-4-methylbenzenesulfonamide. ¹H NMR (400 MHz, CDCl₃) δ 7.65-7.68 (m, 2H), 7.35-7.38 (m, 1H), 7.25-7.27 (m, 2H), 7.20-7.22 (m, 1H), 7.04-7.09 (m, 1H), 6.92-6.97 (m, 1H), 4.55-4.58 (m, 3H), 3.57-3.65 (m, 2H), 3.34-3.42 (m, 2H), 3.26 (d, 2H), 2.41 (s, 3H), 1.11 (t, 6H).

### 8-Fluoroisoquinoline

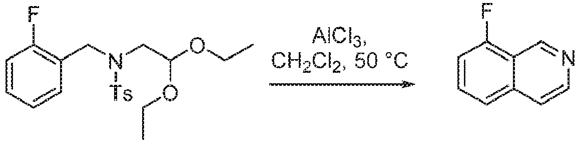

To a solution of 50.0 g (126.4 mmol, 1.0 eq.) of *N*-(2,2-diethoxyethyl)-*N*-(2-fluorobenzyl)-4-methylbenzenesulfonamide in 500 mL of methylene chloride was added 67.4 g (505.7 mmol, 4.0 eq) of AlCl₃ portion-wise over approximately 15 min and the mixture was heated at 50 °C for 4 h. The mixture was allowed to cool to room temperature and poured into 1 L ice-cold water and extracted with 2 × 1 L of dichloromethane. The combined organic extracts were washed with 500 mL of saturated sodium bicarbonate solution, 500 mL of brine, dried (Na₂SO₄), filtered and the solvent was removed *in vacuo.* The above detailed reaction was conducted in duplicate and the crude products from the batches were combined and subsequently purified by flash column chromatography (SiO₂, eluting with a linear gradient of 5-10% of ethyl acetate in petroleum ether) to provide 13.0 g (88.43 mmol, 35%) of 8-fluoroisoquinoline. LCMS: *m*/*z* found 148.0 [M+H]⁺; ¹H NMR (400 MHz, CDCl₃) δ 9.55 (s, 1H), 8.61 (d, 1H), 7.60-7.67 (m, 3H), 7.22-7.25 (m, 1H).

### 8-Fluoroisoquinoline 2-oxide

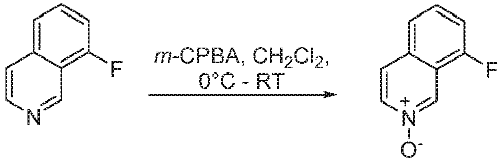

To a solution of 13.0 g (88.4 mmol, 26%) of 8-fluoroisoquinoline in 130 mL of methylene chloride at 0 °C was added 43.5 g (176.6 mmol, 2.0 eq.) of m-chloroperbenzoic acid portion-wise over approximately 20 min. The mixture was then allowed to warm to room temperature and stirred for 16 h. The mixture was quenched with 500 mL of saturated sodium bicarbonate solution and extracted with 2 × 500 mL of 10% methanol in methylene chloride. The combined organic extracts were washed with 400 mL of brine, dried (Na₂SO₄), filtered and the solvent was removed *in vacuo.* The residue was triturated with 50 mL of diethyl ether and dried under high vacuum to provide 10.0 g (61.34 mmol, 69%) of 8-fluoroisoquinoline 2-oxide. LCMS: m/z found 164.0 [M+H]⁺; ¹H NMR (400 MHz, DMSO-d₆) δ 8.84 (s, 1H), 8.23-8.25 (m, 1H), 8.02-8.05 (m, 1H), 7.82 (d, 1H), 7.59-7.64 (m, 1H), 7.49-7.53 (m, 1H).

### 8-Fluoroisoquinolin-1(2H)-one (IIk)

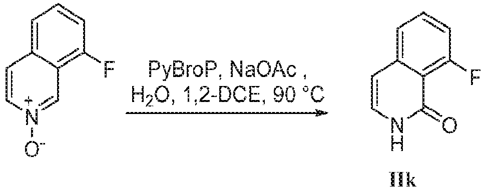

To a suspension of 5.0 g (30.6 mmol, 1.0 eq.) of 8-fluoroisoquinoline 2-oxide in 100 mL of 1,2-dichloroethane was added 7.5 g (91.9 mmol, 3.0 eq.) of sodium acetate followed by 28.2 g (61.2 mmol, 2.0 eq.) of bromotripyrrolidinophosphonium hexafluorophosphate (PyBroP) and 8.2 mL (459.6 mmol, 15 eq.) of water and the mixture was heated at 90 °C for 16 h. The mixture was allowed to cool to room temperature, extracted with 2 × 40 mL of methylene chloride and the combined organic extracts were concentrated *in vacuo.* The above detailed reaction was conducted in duplicate and the crude products from the batches were combined and subsequently purified by flash column chromatography (SiO₂, eluting with a linear gradient of 60-70% of ethyl acetate in petroleum ether) to provide 8.0 g (49.07 mmol, 79%) of 8-fluoroisoquinolin-1(2*H*)-one (**IIk**). LCMS: *m*/*z* found 164.1 [M+H]⁺; ¹H NMR (400 MHz, DMSO-d₆): δ 11.20 (bs, 1H), 7.63-7.68 (m, 1H), 7.44 (d, 1H), 7.14-7.20 (m, 2H), 6.52-6.54 (m, 1H).

### 4-Bromo-8-fluoroisoquinolin-1(2H)-one (IIIk)

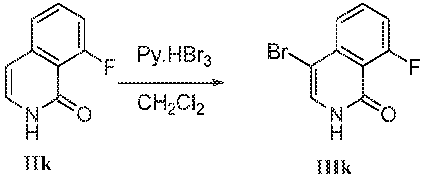

To a solution of 1.6 g (9.80 mmol, 1.0 eq) of 8-fluoroisoquinolin-1(2*H*)-one (**IIk**) in 100 mL of methylene chloride at 0 °C was added 1.88 g (5.80 mmol, 0.6 eq.) of pyridinium hydrobromide perbromide. The mixture was allowed to warm to room temperature and stirred for 1 h. The reaction was quenched with 5 mL of saturated sodium bicarbonate solution and the volatiles were removed *in vacuo.* The resultant residue was resuspended in 10 mL of water and the precipitated solid was collected by filtration, washed with 50 mL of petroleum ether and dried under high vacuum to provide 0.7 g (2.9 mmol, 50%) of 4-bromo-8-fluoroisoquinolin-1(2*H*)-one **(IIIk).** LCMS: *m*/*z* found 242.2/244.2 [M+H]⁺; ¹H NMR (400 MHz, DMSO-d₆) δ 11.57 (bs, 1H), 7.81-7.86 (m, 1H), 7.57-7.60 (m, 2H), 7.32-7.38 (m, 1H).

### 4-Acetyl-8-fluoroisoquinolin-1(2H)-one (XXk)

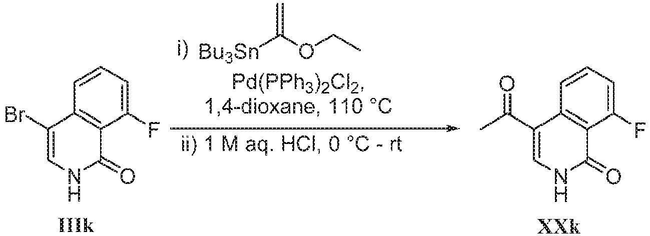

To a solution of 1.4 g (5.7 mmol, 1.0 eq.) of 4-bromo-8-fluoroisoquinolin-1(2*H*)-one **(IIIk)** in 20 mL of 1,4-dioxane was added 5.2 g (14.4 mmol, 2.5 eq.) of tributyl(1-ethoxyvinyl)tin. The mixture was degassed by purging with argon gas for 5 min and 0.41 g (0.57 mmol, 0.1 eq.) of bis(triphenylphosphine)palladium(II) dichloride was added. The mixture was then heated at 110 °C under an argon atmosphere for 16 h. The mixture was allowed to cool to room temperature and further cooled to 0 °C. The mixture was then diluted with 10 mL of 1 M aqueous HCl and the resulting solution stirred at room temperature for 2 h. The mixture was basified with 40 mL of saturated sodium bicarbonate solution and filtered through CELITE^{®}. The filtrate was extracted with 3 × 50 mL of ethyl acetate and the combined organic extracts were washed with 30 mL of brine, dried (Na₂SO₄), filtered and the solvent was removed *in vacuo.* The residue was triturated with 10 mL of *n*-pentane to provide 0.5 g (1.95 mmol, 47%) of 4-acetyl-8-fluoroisoquinolin-1(2*H*)-one **(XXk).** LCMS: *m*/*z* found 206.3 [M+H]+, RT = 1.86 min.

### 8-Fluoro-4-(1-(methylamino)ethyl)isoquinolin-1(2H)-one (VIIIcr)

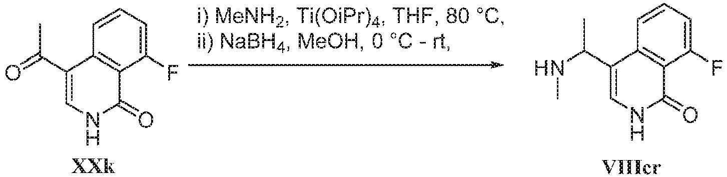

To a solution of 0.25 g (1.22 mmol, 1.0 eq.) of 4-acetyl-8-fluoroisoquinolin-1(2*H*)-one **(XXk)** in 2.5 mL of THF at room temperature under a nitrogen atmosphere was added 1.2 mL (2.4 mmol, 2.0 eq.) of a 2 M solution of methylamine in THF followed by 1.25 mL of titanium isopropoxide, and the mixture was heated to 80 °C for 4 h. The mixture was allowed to cool to room temperature and further cooled to 0 °C. The cooled solution was then diluted with 2 mL of methanol and 0.14 g (3.65 mmol, 3.0 eq.) of sodium borohydride was added portion-wise. The mixture was then allowed to warm to room temperature and stirred for 2 h. The reaction mixture was diluted with 50 mL of water and extracted with 4 × 60 mL of ethyl acetate. The combined organic extracts were washed with 50 mL of brine, dried (Na₂SO₄), filtered and the solvent was removed *in vacuo* to provide 0.25 g of crude 8-fluoro-4-(1-(methylamino)ethyl)isoquinolin-1(2*H*)-one **(VIIIcr).** LCMS: *m*/*z* found 221.4 [M+H]⁺.

### 3-(3-Chloro-4-fluorophenyl)-1-(1-(8-fluoro-1-oxo-1,2-dihydroisoquinolin-4-yl)ethyl)-1-methylurea (Compounds 356 & 357)

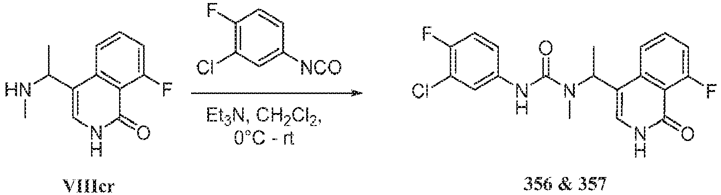

Racemic 3-(3-chloro-4-fluorophenyl)-1-(1-(8-fluoro-1-oxo-1,2-dihydroisoquinolin-4-yl)ethyl)-1-methylurea was synthesized in a similar manner as described above from 8-fluoro-4-(1-(methylamino)ethyl)isoquinolin-1(2*H*)-one (**VIIIcr)** and 2-chloro-1-fluoro-4-isocyanatobenzene. The enantiomers were subsequently separated by SFC, Column: Chiralpak IG (30 × 250 mm) 5 µ, 75% CO₂:MeOH, flow rate 90 g/min.

3-(3-Chloro-4-fluorophenyl)-1-(1-(8-fluoro-1-oxo-1,2-dihydroisoquinolin-4-yl)ethyl)-1-methylurea - Enantiomer I **(Compound 356**) LCMS: *m*/*z* found 392.2/394.2 [M+H]⁺, RT = 4.16 min (Method A); ¹H NMR (400 MHz, DMSO-*d*₆): δ 11.50 (bs, 1H), 8.50 (bs 1H), 7.84-7.87 (m, 1H), 7.68-7.74 (m, 1H), 7.46-7.51 (m, 2H), 7.30 (t, 1H), 7.17-7.23 (m, 2H), 5.75-5.80 (m, 1H), 2.58 (s, 3H), 1.41 (d, 3H); Chiral analytical SFC: RT = 2.01 min, Column: Chiralpak IC-3 (4.6 × 150 mm) 3 µm, 70.0% CO₂/MeOH, Flow = 3.0 g/min.

3-(3-Chloro-4-fluorophenyl)-1-(1-(8-fluoro-1-oxo-1,2-dihydroisoquinolin-4-yl)ethyl)-1-methylurea - Enantiomer II **(Compound 357**) LCMS: *m*/*z* found 392.2/394.2 [M+H]⁺, RT = 4.16 min (Method A); ¹H NMR (400 MHz, DMSO-*d*₆): δ 11.50 (bs, 1H), 8.50 (bs 1H), 7.84-7.87 (m, 1H), 7.68-7.74 (m, 1H), 7.46-7.51 (m, 2H), 7.30 (t, 1H), 7.17-7.23 (m, 2H), 5.75-5.80 (m, 1H), 2.58 (s, 3H), 1.41 (d, 3H); Chiral analytical SFC: RT = 2.93 min, Column: Chiralpak IC-3 (4.6 × 150 mm) 3 µm, 70.0% CO₂/MeOH, Flow = 3.0 g/min.

### 2-((1-(8-Fluoro-1-oxo-1,2-dihydroisoquinolin-4-yl)ethyl)amino)ethane-1-sulfonamide (VIIIcs)

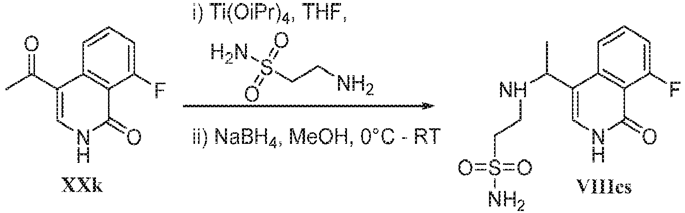

To a solution of 0.4 g (1.9 mmol, 1.0 eq.) of 4-acetyl-8-fluoroisoquinolin-1(2*H*)-one **(XXk)** in 2 mL of THF under a nitrogen atmosphere was added 0.29 g (2.3 mmol, 1.5 eq.) of 2-aminoethane-1-sulfonamide followed by 2 mL of titanium isopropoxide and the mixture was stirred at room temperature for 24 h. The mixture was then cooled to 0 °C, diluted with 4 mL of methanol and 0.22 g (5.8 mmol, 3.0 eq.) of sodium borohydride was added portions wise over approximately 10 minutes. After stirring at 0 °C for 4h, the mixture was diluted with 4 mL of brine and 100 mL of 10% methanol in methylene chloride. The resultant heterogeneous mixture was filtered through CELITE^{®} and the pad was washed with 40 mL of 10% methanol in methylene chloride. The layers were separated, and the organic phase was washed with 50 mL of brine, dried (Na₂SO₄), filtered and the solvent was removed *in vacuo* to provide 0.3 g of 2-((1-(8-fluoro-1-oxo-1,2-dihydroisoquinolin-4-yl)ethyl)amino)ethane-1-sulfonamide **(VIIIcs)** which was carried forward to the next step without further purification. LCMS: *m*/*z* found 314.4 [M+H]⁺.

### 2-(3-(3-Chloro-4-fluorophenyl)-1-(1-(8-fluoro-1-oxo-1,2-dihydroisoquinolin-4-yl)ethyl)ureido) ethane-1-sulfonamide (Compounds 396 & 397)

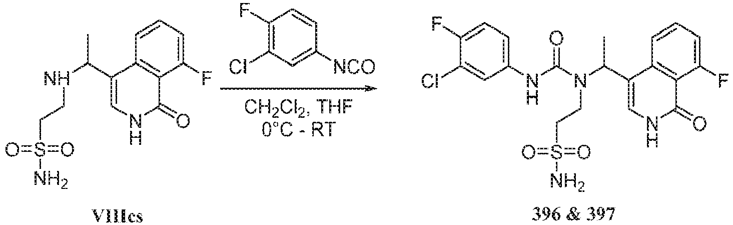

Racemic 2-(3-(3-chloro-4-fluoropheny1)-1-(1-(8-fluoro-1-oxo-1,2-dihydroisoquinolin-4-yl)ethyl)ureido) ethane-1-sulfonamide was synthesized in a similar manner as described above from 2-((1-(8-fluoro-1-oxo-1,2-dihydroisoquinolin-4-yl)ethyl)amino)ethane-1-sulfonamide **(VIIIcs)** and 2-chloro-1-fluoro-4-isocyanatobenzene. The enantiomers were subsequently separated by SFC, Column: Chiralpak IC (30 × 250 mm) 5 µ, 60% CO₂:MeOH, flow rate 90 g/min.

2-(3-(3-Chloro-4-fluorophenyl)-1-(1-(8-fluoro-1-oxo-1,2-dihydroisoquinolin-4-yl)ethyl)ureido) ethane-1-sulfonamide - Enantiomer I **(Compound 396**) LCMS: *m*/*z* found 485.2/487.2 [M+H]⁺, RT = 3.95 min (Method A); ¹H NMR (400 MHz, DMSO-*d*₆): δ 11.39 (bs, 1H), 8.52 (bs, 1H), 7.79-7.82 (m, 1H), 7.71-7.77 (m, 1H), 7.46-7.50 (m, 1H) 7.32-7.40 (m, 2H), 7.20-7.26 (m, 2H), 6.81 (bs, 2H), 5.65-5.70 (d, 1H), 3.39-3.50 (m, 2H), 2.97-3.04 (m, 1H), 2.51-2.59 (m, 1H) 1.50 (d, 3H); Chiral analytical SFC: RT = 1.96 min, Column: Chiralpak IC-3 (4.6 × 150 mm) 3 µm, 60.0% CO₂/MeOH, Flow = 3.0 g/min.

2-(3-(3-Chloro-4-fluorophenyl)-1-(1-(8-fluoro-1-oxo-1,2-dihydroisoquinolin-4-yl)ethyl)ureido) ethane-1-sulfonamide - Enantiomer II **(Compound 397**) LCMS: *m*/*z* found 485.2/487.2 [M+H]⁺, RT = 3.95 min (Method A); ¹H NMR (400 MHz, DMSO-*d*₆): δ 11.39 (bs, 1H), 8.52 (bs, 1H), 7.79-7.82 (m, 1H), 7.71-7.77 (m, 1H), 7.46-7.50 (m, 1H) 7.32-7.40 (m, 2H), 7.20-7.26 (m, 2H), 6.81 (bs, 2H), 5.65-5.70 (d, 1H), 3.39-3.50 (m, 2H), 2.97-3.04 (m, 1H), 2.51-2.59 (m, 1H) 1.50 (d, 3H); Chiral analytical SFC: RT = 2.74 min, Column: Chiralpak IC-3 (4.6 × 150 mm) 3 µm, 60.0% CO₂/MeOH, Flow = 3.0 g/min.

### 8-Fluoro-4-(1-(isobutylamino)ethyl)isoquinolin-1(2H)-one (VIIIct)

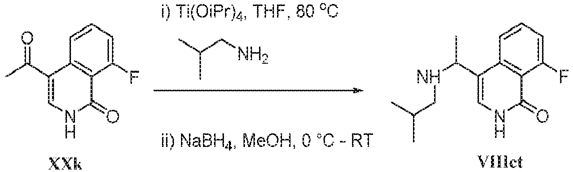

To a solution of 0.4 g (1.9 mmol, 1.0 eq.) of 4-acetyl-8-fluoroisoquinolin-1(2*H*)-one **(XXk)** in 4 mL of THF under a nitrogen atmosphere was added 0.21 g (2.9 mmol, 1.5 eq.) of isobutylamine followed by 2 mL of titanium isopropoxide and the mixture was heated at 80 °C for 5 h. The mixture was allowed to cool to room temperature, further cooled to 0 °C, diluted with 4 mL of methanol and 0.22 g (5.8 mmol, 3.0 eq.) of sodium borohydride was added. After stirring at 0 °C for 4 h, the mixture was diluted with 50 mL of water and extracted with 3 × 150 mL of ethyl acetate. The combined organic extracts were washed with 50 mL of brine, dried (Na₂SO₄), filtered and the solvent was removed *in vacuo* to provide 0.3 g of 8-fluoro-4-(1-(isobutylamino)ethyl)lsoquinolin-1(2*H*)-one **(VIIIct)** which was carried forward to the next step without further purification. LCMS: *m*/*z* found 263.4 [M+H]⁺.

### 3-(3-Chloro-4-fluorophenyl)-1-(1-(8-fluoro-1-oxo-1,2-dihydroisoquinolin-4-yl)ethyl)-1-isobutylurea (Compounds 360 & 361)

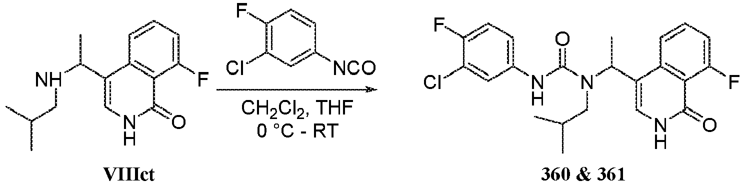

Racemic 3-(3-chloro-4-fluorophenyl)-1-(1-(8-fluoro-1-oxo-1,2-dihydroisoquinolin-4-yl)ethyl)-1-isobutylurea was synthesized in a similar manner as described above from 8-fluoro-4-(1-(isobutylamino)ethyl)isoquinolin-1(2*H*)-one (**VIIIct**) and 2-chloro-1-fluoro-4-isocyanatobenzene. The enantiomers were subsequently separated by SFC, Column: Chiralpak IC (30 × 250 mm) 5 µ, 70% CO₂:MeOH, flow rate 100 g/min.

3-(3-Chloro-4-fluorophenyl)-1-(1-(8-fluoro-1-oxo-1,2-dihydroisoquinolin-4-yl)ethyl)-1-isobutylurea - Enantiomer I (**Compound 360**) LCMS: *m*/*z* found 434.2/436.2 [M+H]⁺, RT = 4.81 min (Method A); ¹H NMR (400 MHz, DMSO-*d₆*): δ 11.45 (bs, 1H), 8.39 (s, 1H), 7.79-7.82 (m, 1H), 7.72-7.77 (m, 1H), 7.59 (d, 1H), 7.46-7.50 (m, 1H), 7.31 (t, 1H), 7.19-7.24 (m, 2H), 5.79-5.81 (m, 1H), 2.84-2.98 (m, 2H), 1.45 (d, 3H), 1.33-1.37 (m, 1H), 0.62 (d, 3H), 0.49 (d, 3H); Chiral analytical SFC: RT = 2.75 min, Column: Chiralpak IC-3 (4.6 × 150 mm) 3 µm, 70.0% CO₂/MeOH, Flow = 3.0 g/min.

3-(3-Chloro-4-fluorophenyl)-1-(1-(8-fluoro-1-oxo-1,2-dihydroisoquinolin-4-yl)ethyl)-1-isobutylurea - Enantiomer II (**Compound 361**) LCMS: *m*/*z* found 434.2/436.2 [M+H]⁺, RT = 4.81 min (Method A); ¹H NMR (400 MHz, DMSO-*d₆*): δ 11.45 (bs, 1H), 8.39 (s, 1H), 7.79-7.82 (m, 1H), 7.72-7.77 (m, 1H), 7.59 (d, 1H), 7.46-7.50 (m, 1H), 7.31 (t, 1H), 7.19-7.24 (m, 2H), 5.79-5.81 (m, 1H), 2.84-2.98 (m, 2H), 1.45 (d, 3H), 1.33-1.37 (m, 1H), 0.62 (d, 3H), 0.49 (d, 3H); Chiral analytical SFC: RT = 3.60 min, Column: Chiralpak IC-3 (4.6 × 150 mm) 3 µm, 70.0% CO₂/MeOH, Flow = 3.0 g/min.

### 8-Fluoro-4-(1-((3-hydroxypropyl)amino)ethyl)isoquinolin-1(2H)-one (VIIIcu)

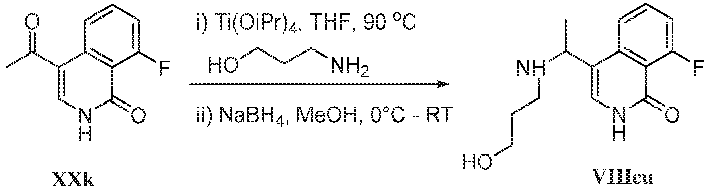

To a solution of 0.5 g (2.4 mmol, 1.0 eq.) of 4-acetyl-8-fluorolsoquinolin-1(2*H*)-one (**XXk**) in 5 mL of THF under a nitrogen atmosphere was added 0.21 g (2.9 mmol, 1.5 eq.) of 3-aminopropan-1-ol followed by 2.5 mL of titanium isopropoxide and the mixture was heated at 90 °C for 4 h. The mixture was allowed to cool to room temperature, further cooled to 0 °C, diluted with 2.5 mL of methanol and 0.28 g (7.3 mmol, 3.0 eq.) of sodium borohydride was added. After stirring at 0 °C for 3 h, the mixture was diluted with 5 mL of brine, filtered through CELITE^{®} and the filtrate was extracted with 2 × 80 mL of 10% methanol in methylene chloride. The combined organic extracts were washed with 50 mL of brine, dried (Na₂SO₄), filtered and the solvent was removed *in vacuo* to provide 0.6 g of 8-fluoro-4-(1-((3-hydroxypropyl)amino)ethyl)isoquinolin-1(2*H*)-one (**VIIIcu**) which was carried forward to the next step without further purification. LCMS: *m*/*z* found 263.4 [M+H]⁺.

### 3-(3-Chloro-4-fluorophenyl)-1-(1-(8-fluoro-1-oxo-1,2-dihydroisoquinolin-4-yl)ethyl)-1-(3-hydroxypropyl)urea (Compounds 366 & 367)

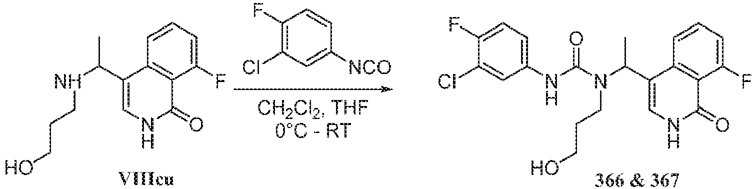

Racemic 3-(3-chloro-4-fluorophenyl)-1-(1-(8-fluoro-1-oxo-1,2-dihydroisoquinolin-4-yl)ethyl)-1-(3-hydroxypropyl)urea was synthesized in a similar manner as described above from 8-fluoro-4-(1-((3-hydroxypropyl)amino)ethyl)isoquinolin-1(2*H*)-one (**VIIIcu**) and 2-chloro-1-fluoro-4-isocyanatobenzene. The enantiomers were subsequently separated by SFC, Column: Lux Cellulose-2 (30 × 250 mm) 5 µ, 65% CO₂:MeOH, flow rate 100 g/min.

3-(3-Chloro-4-fluorophenyl)-1-(1-(8-fluoro-1-oxo-1,2-dihydroisoquinolin-4-yl)ethyl)-1-(3-hydroxypropyl)urea- Enantiomer I (**Compound 366**) LCMS: *m*/*z* found 436.1/438.1 [M+H]⁺, RT = 5.09 min (Method A); ¹H NMR (400 MHz, DMSO-*d₆*): δ 11.39 (bs, 1H), 8.78 (bs 1H), 7.79-7.81 (m, 1H), 7.70-7.76 (m, 1H), 7.39-7.46 (m, 2H), 7.32 (t, 1H), 7.19-7.24 (m, 2H), 5.77-5.81 (m, 1H), 5.16 (bs, 1H), 3.11-3.21 (m, 4H) 1.44 (d, 3H), 1.07-1.14 (m, 2H); Chiral analytical SFC: RT = 1.39 min, Column: Chiralcel OZ-3 (4.6 × 150 mm) 3 µm, 60.0% CO₂/MeOH, Flow = 3.0 g/min.

3-(3-Chloro-4-fluorophenyl)-1-(1-(8-fluoro-1-oxo-1,2-dihydroisoquinolin-4-yl)ethyl)-1-(3-hydroxypropyl)urea- Enantiomer II (**Compound 367**) LCMS: *m*/*z* found 436.1/438.1 [M+H]⁺, RT = 5.09 min (Method A); ¹H NMR (400 MHz, DMSO-*d₆*): δ 11.39 (bs, 1H), 8.78 (bs 1H), 7.79-7.81 (m, 1H), 7.70-7.76 (m, 1H), 7.39-7.46 (m, 2H), 7.32 (t, 1H), 7.19-7.24 (m, 2H), 5.77-5.81 (m, 1H), 5.16 (bs, 1H), 3.11-3.21 (m, 4H) 1.44 (d, 3H), 1.07-1.14 (m, 2H); Chiral analytical SFC: RT = 1.89 min, Column: Chiralcel OZ-3 (4.6 × 150 mm) 3 µm, 60.0% CO₂/MeOH, Flow = 3.0 g/min.

### 1-(1-(8-Chloro-1-oxo-1,2-dihydroisoquinolin-4-yl)ethyl)-3-(3-chloro-4-fluorophenyl)-1-methylurea (Compounds 167 & 168)

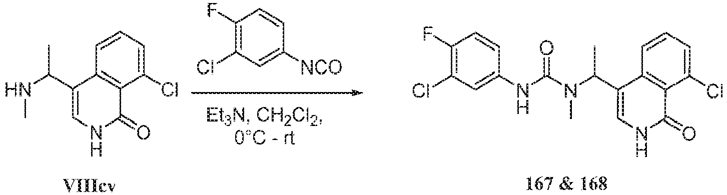

Racemic 1-(1-(8-chloro-1-oxo-1,2-dihydroisoquinolin-4-yl)ethyl)-3-(3-chloro-4-fluorophenyl)-1-methylurea was synthesized in a similar manner as described above from 8-chloro-4-(1-(methylamino)ethyl)isoquinolin-1(2*H*)-one (**VIIIcv**, derived from 8-chloroisoquinoline) and 2-chloro-1-fluoro-4-isocyanatobenzene. The enantiomers were subsequently separated by SFC, Column: Chiralpak IC (30 × 250 mm) 5 µ, 60% CO₂:MeOH, flow rate 100 g/min.

1-(1-(8-Chloro-1-oxo-1,2-dihydroisoquinolin-4-yl)ethyl)-3-(3-chloro-4-fluorophenyl)-1-methylurea - Enantiomer I (**Compound 167**) LCMS: *m*/*z* found 408.2/410.2 [M+H]⁺, RT = 7.25 min (Method A); ¹H NMR (400 MHz, DMSO-*d₆*) δ 11.45 (bs, 1H), 8.46 (bs, 1H), 7.84-7.86 (m, 1H), 7.63-7.68 (m, 2H), 7.48-7.51 (m, 2H), 7.31 (t, 1H), 7.18 (s, 1H), 5.74-5.79 (m, 1H), 2.57 (s, 3H), 1.41 (d, 3H); Chiral analytical SFC: RT = 2.61 min, Column: Chiralpak IC-3 (4.6 × 150 mm) 3 µm, 70.0% CO₂/MeOH, Flow = 3.0 g/min.

1-(1-(8-Chloro-1-oxo-1,2-dihydroisoquinolin-4-yl)ethyl)-3-(3-chloro-4-fluorophenyl)-1-methylurea - Enantiomer I (**Compound 168**) LCMS: *m*/*z* found 408.2/410.2 [M+H]⁺, RT = 7.25 min (Method A); ¹H NMR (400 MHz, DMSO-*d₆*) δ 11.45 (bs, 1H), 8.46 (bs, 1H), 7.84-7.86 (m, 1H), 7.63-7.68 (m, 2H), 7.48-7.51 (m, 2H), 7.31 (t, 1H), 7.18 (s, 1H), 5.74-5.79 (m, 1H), 2.57 (s, 3H), 1.41 (d, 3H); Chiral analytical SFC: RT = 3.77 min, Column: Chiralpak IC-3 (4.6 × 150 mm) 3 µm, 70.0% CO₂/MeOH, Flow = 3.0 g/min.

### 3-(3-Chloro-4-fluorophenyl)-1-methyl-1-(1-(3-methyl-1-oxo-1,2-dihydroisoquinolin-4-yl)ethyl)urea (Compounds 222 & 223)

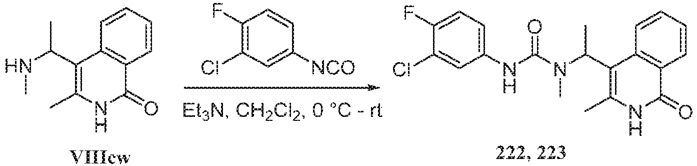

Racemic 3-(3-chloro-4-fluorophenyl)-1-methyl-1-(1-(3-methyl-1-oxo-1,2-dihydroisoquinolin-4-yl)ethyl)urea was synthesized in a similar manner as described above from 3-methyl-4-(1-(methylamino)ethyl)isoquinolin-1(2*H*)-one (**VIIIcw**, derived from 3-methylisoquinoline) and 2-chloro-1-fluoro-4-isocyanatobenzene. The enantiomers were subsequently separated by SFC, Column: Chiralpak IG (30 × 250 mm) 5 µ, 60% CO₂:MeOH, flow rate 70 g/min.

3-(3-Chloro-4-fluorophenyl)-1-methyl-1-(1-(3-methyl-1-oxo-1,2-dihydroisoquinolin-4-yl)ethyl)urea: Enantiomer I (**Compound 222**). LCMS: *m*/*z* found 388.2/390.2 [M+H]⁺, RT = 5.65 min (Method: A); ¹H NMR (400 MHz, DMSO-d₆) δ 11.18 (bs, 1H), 8.47 (s, 1H), 8.19-8.22 (m, 1H), 7.76-7.82 (m, 2H), 7.66-7.71 (m, 1H), 7.40-7.48 (m, 2H), 7.28 (t, 1H), 5.76-5.82 (m, 1H), 2.84 (s, 3H), 2.43 (s, 3H), 1.57 (d, 3H); Chiral analytical SFC: RT = 3.36 min, Column: Chiralpak IG (250 × 4.6 mm), 5 µ, 60% CO₂:MeOH, Flow rate = 3.0 mL/min.

3-(3-Chloro-4-fluorophenyl)-1-methyl-1-(1-(3-methyl-1-oxo-1,2-dihydroisoquinolin-4-yl)ethyl)urea: Enantiomer II (**Compound 223**). LCMS: *m*/*z* found 388.2/390.2 [M+H]⁺, RT = 5.61 min (Method: A); ¹H NMR (400 MHz, DMSO-d₆) δ 11.18 (bs, 1H), 8.47 (s, 1H), 8.19-8.22 (m, 1H), 7.76-7.82 (m, 2H), 7.66-7.71 (m, 1H), 7.40-7.48 (m, 2H), 7.28 (t, 1H), 5.76-5.82 (m, 1H), 2.84 (s, 3H), 2.43 (s, 3H), 1.57 (d, 3H); Chiral analytical SFC: RT = 10.21 min, Column: Chiralpak IG (250 × 4.6 mm), 5 µ, 60% CO₂:MeOH, Flow rate = 3.0 mL/min.

### 3-(4-Fluoro-3-methylphenyl)-1-methyl-1-(1-(3-methyl-1-oxo-1,2-dihydroisoquinolin-4-yl)ethyl)urea (Compounds 230, 236 & 237)

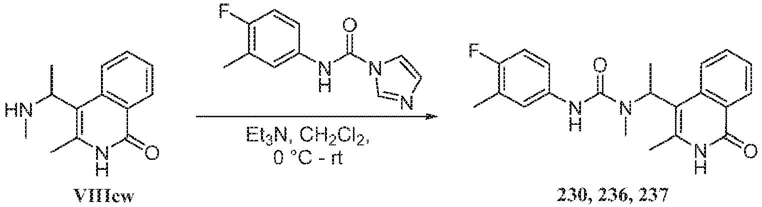

To a solution of 120 mg (0.55 mmol, 1.0 eq.) of 3-methyl-4-(1-(methylamino)ethyl) isoquinolin-1(2*H*)-one (**VIIIcw**) in 5 mL of methylene chloride at 0 °C under a nitrogen atmosphere was added 0.28 g (2.77 mmol, 5.0 eq.) of triethylamine followed by 0.24 g (1.11 mmol, 2.0 eq.) of 2-chloro-1-fluoro-4-isocyanatobenzene. The mixture was allowed to warm to room temperature and stirred for 6 h. The mixture was diluted with 10 mL of water and extracted with 2 × 20 mL of ethyl acetate. The combined organic extracts were dried (Na₂SO₄), filtered and the solvent was removed *in vacuo.* The residue was purified by flash chromatography (SiO₂, eluting with a linear gradient of 70-100% ethyl acetate in petroleum ether) to provide 76 mg (0.21 mmol, 37%) of racemic 3-(4-fluoro-3-methylphenyl)-1-methyl-1-(1-(3-methyl-1-oxo-1,2-dihydroisoquinolin-4-yl)ethyl)urea (**Compound 230**). LCMS: *m*/*z* found 366.4 [M-H]⁻; The enantiomers were subsequently separated by SFC, Column: Chiralpak AD-H (30 × 250 mm) 5 µ, 50% CO₂:MeOH, flow rate 70 g/min.

3-(4-Fluoro-3-methylphenyl)-1-methyl-1-(1-(3-methyl-1-oxo-1,2-dihydroisoquinolin-4-yl)ethyl)urea - Enantiomer I (**Compound 236**). LCMS: *m*/*z* found 368.4 [M+H]⁺, RT = 7.26 min (Method: A); ¹H NMR (400 MHz, DMSO-d₆) δ 11.12 (bs, 1H), 8.22 (s, 1H), 8.19 (d, 1H), 7.80 (d, 1H), 7.66-7.70 (m, 1H), 7.36-7.44 (m, 2H), 7.26-7.31 (m, 1H), 6.99 (t, 1H), 5.76-5.82 (m, 1H), 2.83 (s, 3H), 2.43 (s, 3H), 2.18 (d, 3H), 1.56 (d, 3H); Chiral analytical SFC: RT = 2.90 min, Column: Chiralpak AD-H (250 × 4.6 mm), 5 µ, 60% CO₂:MeOH, Flow rate = 3.0 mL/min.

3-(4-Fluoro-3-methylphenyl)-1-methyl-1-(1-(3-methyl-1-oxo-1,2-dihydroisoquinolin-4-yl)ethyl)urea - Enantiomer II (**Compound 237**). LCMS: *m*/*z* found 368.4 [M+H]⁺, RT = 7.27 min (Method: A); ¹H NMR (400 MHz, DMSO-d₆) δ 11.12 (bs, 1H), 8.22 (s, 1H), 8.19 (d, 1H), 7.80 (d, 1H), 7.66-7.70 (m, 1H), 7.36-7.44 (m, 2H), 7.26-7.31 (m, 1H), 6.99 (t, 1H), 5.76-5.82 (m, 1H), 2.83 (s, 3H), 2.43 (s, 3H), 2.18 (d, 3H), 1.56 (d, 3H); Chiral analytical SFC: RT = 9.91 min, Column: Chiralpak AD-H (250 × 4.6 mm), 5 µ, 60% CO₂:MeOH, Flow rate = 3.0 mL/min.

### Synthesis of 6,7-difluoroisoquinolin-1(2H)-one (IId)

### (E)-3-(3,4-Difluorophenyl)acryloyl azide

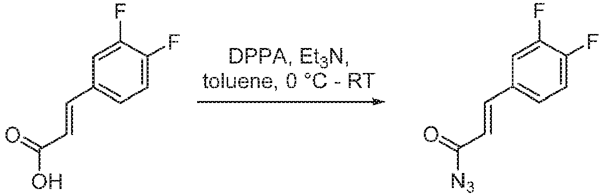

To a solution of 20.0 g (108.1 mmol, 1.0 eq.) of (E)-3-(3,4-difluorophenyl)acrylic acid in 100 mL of toluene under a nitrogen atmosphere at 0 °C was added 45 mL (324.1 mmol, 3.0 eq.) of triethylamine followed by 26.8 g (97.8 mmol, 0.9 eq.) of diphenylphosphoryl azide. The mixture was allowed to warm to room temperature and stirred for 2 h. The solvent was removed *in vacuo* and the product was isolated by MPLC (REVELERIS^{®} silica column; eluting with a linear gradient of 10-20% ethyl acetate/petroleum ether) to provide 10.0 g (47.84 mmol, 44%) of (E)-3-(3,4-difluorophenyl)acryloyl azide. ¹H NMR (400 MHz, CDCl₃): δ 7.65 (d, 1H), 7.33-7.39 (m, 1H), 7.25-7.30 (m, 1H), 7.19-7.23 (m, 1H), 6.34 (d, 1H).

### 6,7-Difluoroisoquinolin-1(2H)-one (IId)

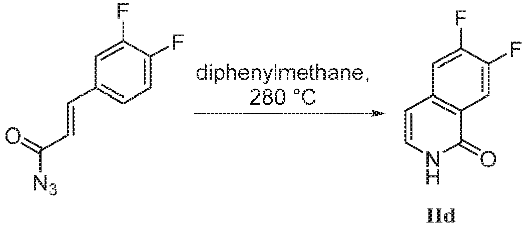

A stirred solution of 10.0 g (47.8 mmol, 1.0 eq.) of (E)-3-(3,4-difluorophenyl)acryloyl azide in 50 mL of diphenylmethane was heated to 100 °C for 30 min. The temperature was subsequently increased to 280 °C and stirring was continued for 3 h. The mixture was allowed to cool to room temperature and diluted with 200 mL of *n*-heptane and stirred for a further 30 min. The solids were collected by filtration and triturated with 100 mL *of n-*heptane and dried under vacuum to provide 6.0 g (33.1 mmol, 69%) of 6,7-difluoroisoquinolin-1(2*H*)-one (**IId**). LCMS: *m*/*z* found 182.4 [M+H]⁺, RT = 1.45 min; ¹H NMR (400 MHz, CDCl₃): δ 10.11 (bs, 1H), 8.15-8.21 (m, 1H), 7.30-7.35 (m, 1H), 7.11-7.14 (m, 1H), 6.48 (d, 1H).

### 4-Bromo-6,7-difluoroisoquinolin-1(2H)-one (IIId)

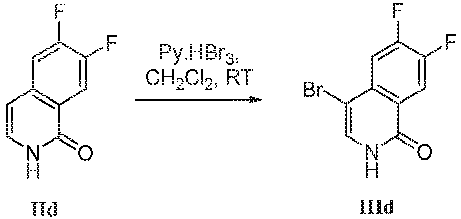

To a solution of 3.0 g (16.6 mmol, 1.0 eq.) of 6,7-difluoroisoquinolin-1(2*H*)-one (**IId**) in 30 mL of methylene chloride was added 5.3 g (16.6 mmol, 1.0 eq.) of pyridinium hydrobromide perbromide and the mixture was stirred at room temperature for 4 h. The reaction was quenched with 50 mL of saturated sodium bicarbonate solution and the solvent was removed *in vacuo.* The residue was suspended in 80 mL of water and the solids were collected by filtration, washed with 50 mL of petroleum ether and dried under vacuum to provide 3.5 g (13.5 mmol, 81%) of 4-bromo-6,7-difluoroisoquinolin-1(2*H*)-one (**IIId**). ¹H NMR (300 MHz, DMSO-d₆): δ 11.81 (bs, 1H), 8.11-8.18 (m, 1H), 7.68-7.75 (m, 1H), 7.64 (s, 1H).

### 4-Acetyl-6,7-difluoroisoquinolin-1(2H)-one (XXd)

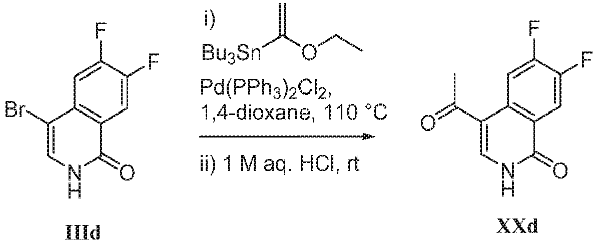

To a stirred solution of 3.5 g (13.5 mmol, 1.0 eq.) of 4-bromo-6,7-difluoroisoquinolin-1(2*H*)-one (**IIId**) in 35 mL of 1,4-dioxane was added 12.2 g (33.8 mmol, 2.5 eq.) of tributyl(1-ethoxyvinyl)stannane. The mixture was purged with nitrogen gas for 5 min and 0.95 g (1.35 mmol, 0.1 eq.) of Pd(PPh₃)₂Cl₂ was added, and then heated to 110 °C for 16 h. The reaction mixture was allowed to cool to room temperature and 60 mL of 1 M aqueous HCl was added and stirring was continued for an additional 1 h. The reaction mixture was then basified with 50 mL of saturated sodium bicarbonate solution and extracted with 3 × 200 mL of ethyl acetate. The combined organic extracts were washed with 100 mL of water, 100 mL of brine, dried (Na₂SO₄), filtered and the solvent was removed *in vacuo.* The residue was purified by MPLC (REVELERIS^{®} silica column, eluting with a linear gradient of 30-50% ethyl acetate/petroleum ether) to provide 1.7 g (7.6 mmol, 56%) of 4-acetyl-6,7-difluoroisoquinolin-1(2*H*)-one (**XXd**). LCMS: *m*/*z* found 224.0 [M+H]⁺, ¹H NMR (300 MHz, DMSO-d₆): δ 12.20 (bs, 1H), 8.87-8.95 (m, 1H), 8.27 (s, 1H), 8.09-8.16 (m, 1H), 2.53 (s, 3H).

The above reaction sequence was performed on multiple batches with consistent results.

### 6,7-Difluoro-4-(1-(methylamino)ethyl)isoquinolin-1(2H)-one (VIIIs)

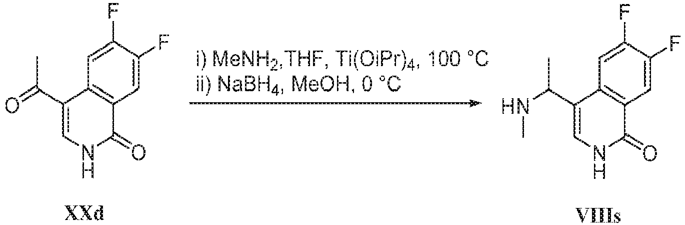

To a solution of 400 mg (1.8 mmol, 1.0 eq.) of 4-acetyl-6,7-difluoroisoquinolin-1(2*H*)-one (**XXd**) in 4 mL of THF was added 4 mL (8.0 mmol, 4.4 eq.) of a 2M solution of methylamine solution in THF followed by 4 mL of titanium isopropoxide and the mixture was heated at 100 °C for 16 h. The mixture was allowed to cool to room temperature and further cooled to 0 °C. Following dilution with 3 mL of methanol, 0.21 g (5.4 mmol, 3.0 eq.) of sodium borohydride was added portion-wise over approximately 10 min and stirring was continued for 4 h. The reaction mixture was diluted with 100 mL of water and extracted with 4 × 60 mL of ethyl acetate. The combined organic extracts were washed with 50 mL of water, 50 mL of brine, dried (Na₂SO₄), filtered and the solvent was removed *in vacuo* to provide 0.4 g of 6,7-difluoro-4-(1-(methylamino)ethyl)isoquinolin-1(2*H*)-one **(VIIIs).** LCMS: *m*/*z* found 239.0 [M+H]⁺.

### 3-(3-Chloro-4-fluorophenyl)-1-(1-(6,7-difluoro-1-oxo-1,2-dihydroisoquinolin-4-yl)ethyl)-1-methylurea (Compounds 278 & 279)

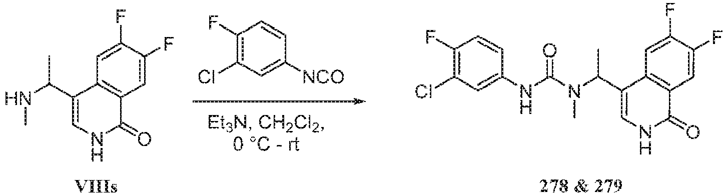

To a solution of 400 mg of 6,7-difluoro-4-(1-(methylamino)ethyl)isoquinolin-1(2*H*)-one (**VIIIs**) in 4 mL of methylene chloride at 0 °C was added 0.7 mL (5.04 mmol) of triethylamine followed by 0.17 mL (1.0 mmol) of 2-chloro-1-fluoro-4-isocyanatobenzene. The mixture was allowed to warm to room temperature and stirred for 1 h. The reaction mixture was then diluted with 100 mL of water and extracted with 3 × 100 mL of methylene chloride. The combined organic extracts were washed with 50 mL of brine, dried (Na₂SO₄), filtered and the solvent was removed *in vacuo.* The residue was purified by reverse phase chromatography (REVELERIS^{®} C-18: 40 g column eluting with linear gradient 10-22% of [0.1% formic acid in water]/acetonitrile) to provide 280 mg (0.68 mmol) of racemic 3-(3-chloro-4-fluorophenyl)-1-(1-(6,7-difluoro-1-oxo-1,2-dihydroisoquinolin-4-yl)ethyl)-1-methylurea. The enantiomers were subsequently separated by chiral SFC, Column: (R,R) Whelk-01 (250 × 30 mm) 5 µ, 85% CO₂/MeOH, Flow rate 90 g/min.

(3-Chloro-4-fluorophenyl)-1-(1-(6,7-difluoro-1-oxo-1,2-dihydroisoquinolin-4-yl)ethyl)-1-methylurea - Enantiomer I (**Compound 278**), LCMS: *m*/*z* found 410.2/412.2 [M+H]⁺, RT = 4.33 min (Method A); ¹H NMR (400 MHz, DMSO-d₆): δ 11.62 (bs, 1H), 8.48 (s 1H), 8.08-8.14 (m, 1H), 7.80-7.83 (m, 1H), 7.70-7.76 (m, 1H), 7.47-7.52 (m, 1H), 7.33 (t, 1H), 7.22 (s, 1H), 5.75-5.81 (m, 1H), 2.60 (s, 3H), 1.43 (d, 3H); Chiral analytical SFC: RT = 2.12 min, Column: (R,R) Whelk-01 (4.6 × 150 mm) 5 µm, 60.0% CO₂/MeOH, Flow = 3.0 g/min.

(3-Chloro-4-fluorophenyl)-1-(1-(6,7-difluoro-1-oxo-1,2-dihydroisoquinolin-4-yl)ethyl)-1-methylurea - Enantiomer II (**Compound 279**), LCMS: *m*/*z* found 410.2/412.2 [M+H]⁺, RT = 4.33 min (Method A); ¹H NMR (400 MHz, DMSO-d₆): δ 11.62 (bs, 1H), 8.48 (s 1H), 8.08-8.14 (m, 1H), 7.80-7.83 (m, 1H), 7.70-7.76 (m, 1H), 7.47-7.52 (m, 1H), 7.33 (t, 1H), 7.22 (s, 1H), 5.75-5.81 (m, 1H), 2.60 (s, 3H), 1.43 (d, 3H); Chiral analytical SFC: RT = 2.64 min, Column: (R,R) Whelk-01 (4.6 × 150 mm) 5 µm, 60.0% CO₂/MeOH, Flow = 3.0 g/min.

### 1-(1-(6,7-Difluoro-1-oxo-1,2-dihydroisoquinolin-4-yl)ethyl)-3-(4-fluorophenyl)-1-methylurea (Compounds 285 & 286)

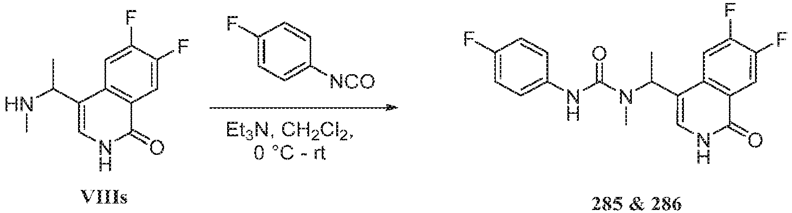

Racemic 1-(1-(6,7-difluoro-1-oxo-1,2-dihydroisoquinolin-4-yl)ethyl)-3-(4-fluorophenyl)-1-methylurea was synthesized in a similar manner as described above from 6,7-difluoro-4-(1-(methylamino)ethyl)isoquinolin-1(2*H*)-one (**VIIIs**) and 4-fluorophenyl isocyanate. The enantiomers were subsequently separated by chiral SFC, Column: Chiralpak IC (250 × 30 mm) 5 µ, 90% CO₂/MeOH, Flow rate 90 g/min.

1-(1-(6,7-Difluoro-1-oxo-1,2-dihydroisoquinolin-4-yl)ethyl)-3-(4-fluorophenyl)-1-methylurea - Enantiomer I (**Compound 285**), LCMS: *m*/*z* found 376.2 [M+H]⁺, RT = 3.81 min (Method A); ¹H NMR (400 MHz, DMSO-d₆): δ 11.61 (bs, 1H), 8.32 (s, 1H), 8.08-8.13 (m, 1H), 7.75-7.80 (m, 1H), 7.50-7.54 (m, 2H), 7.21 (s, 1H), 7.09-7.13 (m, 2H), 5.76-5.82 (m, 1H), 2.60 (s, 3H), 1.42 (d, 3H); Chiral analytical SFC: RT = 1.48 min, Column: Chiralpak IC, (4.6 × 150 mm) 5 µm, 60.0% CO₂/MeOH, Flow = 3.0 g/min.

1-(1-(6,7-Difluoro-1-oxo-1,2-dihydroisoquinolin-4-yl)ethyl)-3-(4-fluorophenyl)-1-methylurea - Enantiomer II (**Compound 286**), LCMS: *m*/*z* found 376.2 [M+H]⁺, RT = 3.81 min (Method A); ¹H NMR (400 MHz, DMSO-d₆): δ 11.61 (bs, 1H), 8.32 (s, 1H), 8.08-8.13 (m, 1H), 7.75-7.80 (m, 1H), 7.50-7.54 (m, 2H), 7.21 (s, 1H), 7.09-7.13 (m, 2H), 5.76-5.82 (m, 1H), 2.60 (s, 3H), 1.42 (d, 3H); Chiral analytical SFC: RT = 2.11 min, Column: Chiralpak IC, (4.6 × 150 mm) 5 µm, 60.0% CO₂/MeOH, Flow = 3.0 g/min.

### 1-(1-(6,7-Difluoro-1-oxo-1,2-dihydroisoquinolin-4-yl)ethyl)-1-methyl-3-phenylurea (Compounds 398 & 399)

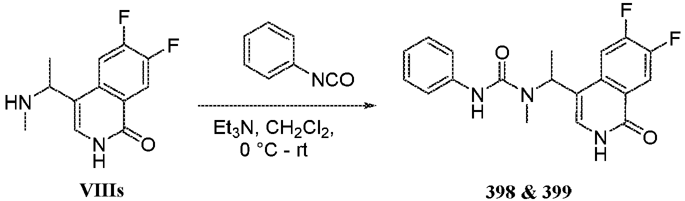

Racemic 1-(1-(6,7-difluoro-1-oxo-1,2-dihydroisoquinolin-4-yl)ethyl)-1-methyl-3-phenylurea was synthesized in a similar manner as described above from 6,7-difluoro-4-(1-(methylamino)ethyl)isoquinolin-1(2*H*)-one (**VIIIs**) and phenyl isocyanate. The enantiomers were subsequently separated by chiral SFC, Column: Chiralpak IC (250 × 30 mm) 5 µ, 75% CO₂/MeOH, Flow rate 100 g/min.

1-(1-(6,7-Difluoro-1-oxo-1,2-dihydroisoquinolin-4-yl)ethyl)-1-methyl-3-phenylurea - Enantiomer I **(Compound 398**), LCMS: *m*/*z* found 358.3 [M+H]⁺, RT = 3.53 min (Method A); ¹H NMR (400 MHz, DMSO-d₆): δ 11.61 (bs, 1H), 8.26 (s, 1H), 8.08-8.13 (m, 1H), 7.76-7.81 (m, 1H), 7.52 (d, 2H), 7.24-7.29 (m, 2H), 7.22 (s, 1H), 6.95-6.99 (m, 1H), 5.77-5.83 (m, 1H), 2.61 (s, 3H), 1.42 (d, 3H); Chiral analytical SFC: RT = 2.89 min, Column: Chiralpak IC, (4.6 × 150 mm) 5 µm, 70.0% CO₂/MeOH, Flow = 3.0 g/min.

1-(1-(6,7-Difluoro-1-oxo-1,2-dihydroisoquinolin-4-yl)ethyl)-1-methyl-3-phenylurea - Enantiomer II (**Compound 399**), LCMS: *m*/*z* found 358.3 [M+H]⁺, RT = 3.53 min (Method A); ¹H NMR (400 MHz, DMSO-d₆): δ 11.61 (bs, 1H), 8.26 (s, 1H), 8.08-8.13 (m, 1H), 7.76-7.81 (m, 1H), 7.52 (d, 2H), 7.24-7.29 (m, 2H), 7.22 (s, 1H), 6.95-6.99 (m, 1H), 5.77-5.83 (m, 1H), 2.61 (s, 3H), 1.42 (d, 3H); Chiral analytical SFC: RT = 4.60 min, Column: Chiralpak IC, (4.6 × 150 mm) 5 µm, 70.0% CO₂/MeOH, Flow = 3.0 g/min.

### 3-(4-Chlorophenyl)-1-(1-(6,7-difluoro-1-oxo-1,2-dihydroisoquinolin-4-yl)ethyl)-1-methylurea (Compounds 400 & 401)

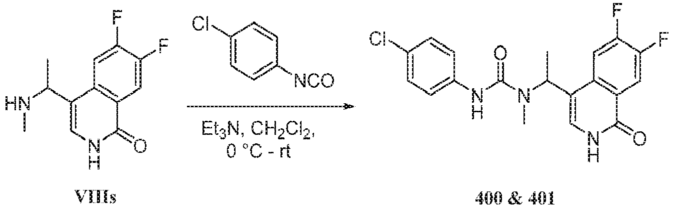

Racemic 3-(4-chlorophenyl)-1-(1-(6,7-difluoro-1-oxo-1,2-dihydroisoquinolin-4-yl)ethyl)-1-methylurea was synthesized in a similar manner as described above from 6,7-difluoro-4-(1-(methylamino)ethyl)isoquinolin-1(2*H*)-one (**VIIIs**) and 4-chlorophenyl isocyanate. The enantiomers were subsequently separated by chiral SFC, Column: Chiralpak IC (250 × 30 mm) 5 µ, 70% CO₂/MeOH, Flow rate 90 g/min.

3-(4-Chlorophenyl)-1-(1-(6,7-difluoro-1-oxo-1,2-dihydroisoquinolin-4-yl)ethyl)-1-methylurea - Enantiomer I (**Compound 400**), LCMS: *m*/*z* found 392.1/394.1 [M+H]⁺, RT = 3.21 min (Method A); ¹H NMR (400 MHz, DMSO-d₆): δ 11.62 (bs, 1H), 8.41 (s, 1H), 8.08-8.13 (m, 1H), 7.72-7.78 (m, 1H), 7.57 (d, 2H), 7.32 (d, 2H), 7.22 (s, 1H), 5.76-5.82 (m, 1H), 2.60 (s, 3H), 1.42 (d, 3H); Chiral analytical SFC: RT = 1.95 min, Column: Chiralpak IC, (4.6 × 150 mm) 5 µm, 70.0% CO₂/MeOH, Flow = 3.0 g/min.

3-(4-Chlorophenyl)-1-(1-(6,7-difluoro-1-oxo-1,2-dihydroisoquinolin-4-yl)ethyl)-1-methylurea - Enantiomer II (**Compound 401**), LCMS: *m*/*z* found 392.1/394.1 [M+H]⁺, RT = 3.21 min (Method A); ¹H NMR (400 MHz, DMSO-d₆): δ 11.62 (bs, 1H), 8.41 (s, 1H), 8.08-8.13 (m, 1H), 7.72-7.78 (m, 1H), 7.57 (d, 2H), 7.32 (d, 2H), 7.22 (s, 1H), 5.76-5.82 (m, 1H), 2.60 (s, 3H), 1.42 (d, 3H); Chiral analytical SFC: RT = 3.53 min, Column: Chiralpak IC, (4.6 × 150 mm) 5 µm, 70.0% CO₂/MeOH, Flow = 3.0 g/min.

### 1-(1-(6,7-Difluoro-1-oxo-1,2-dihydroisoquinolin-4-yl)ethyl)-3-(3,4-difluorophenyl)-1-methylurea (Compounds 408 & 409)

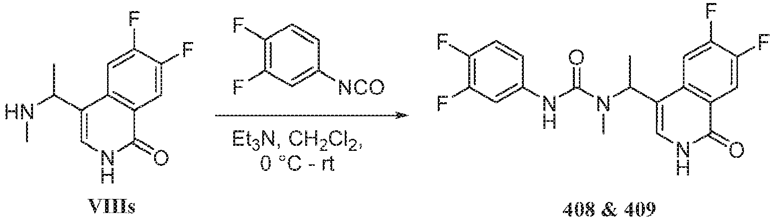

Racemic 1-(1-(6,7-difluoro-1-oxo-1,2-dihydroisoquinolin-4-yl)ethyl)-3-(3,4-difluorophenyl)-1-methylurea was synthesized in a similar manner as described above from 6,7-difluoro-4-(1-(methylamino)ethyl)isoquinolin-1(2*H*)-one (**VIIIs**) and 3,4-difluorophenyl isocyanate. The enantiomers were subsequently separated by chiral SFC, Column: Chiralpak IC (250 × 30 mm, 5 µm) 75% CO₂/MeOH, Flow rate 90 g/min.

1-(1-(6,7-Difluoro-1-oxo-1,2-dihydroisoquinolin-4-yl)ethyl)-3-(3,4-difluorophenyl)-1-methylurea - Enantiomer I (**Compound 409**), LCMS: *m*/*z* found 394.2 [M+H]⁺, RT = 4.03 min (Method A); ¹H NMR (400 MHz, DMSO-d₆): δ 11.62 (bs, 1H), 8.48 (bs, 1H), 8.08-8.13 (m, 1H), 7.66-7.75 (m, 2H), 7.30-7.36 (m, 2H), 7.22 (s, 1H), 5.75-5.80 (m, 1H), 2.60 (s, 3H), 1.42 (d, 3H); Chiral analytical SFC: RT = 4.36 min, Column: Chiralpak IC-3, (4.6 × 150 mm) 3 µm, 70.0% CO₂/MeOH, Flow = 3.0 g/min.

1-(1-(6,7-Difluoro-1-oxo-1,2-dihydroisoquinolin-4-yl)ethyl)-3-(3,4-difluorophenyl)-1-methylurea - Enantiomer II (**Compound 409**), LCMS: *m*/*z* found 394.2 [M+H]⁺, RT = 4.03 min (Method A); ¹H NMR (400 MHz, DMSO-d₆): δ 11.62 (bs, 1H), 8.48 (bs, 1H), 8.08-8.13 (m, 1H), 7.66-7.75 (m, 2H), 7.30-7.36 (m, 2H), 7.22 (s, 1H), 5.75-5.80 (m, 1H), 2.60 (s, 3H), 1.42 (d, 3H); Chiral analytical SFC: RT = 7.92 min, Column: Chiralpak IC-3, (4.6 × 150 mm) 3 µm, 70.0% CO₂/MeOH, Flow = 3.0 g/min.

### 1-(1-(6,7-Difluoro-1-oxo-1,2-dihydroisoquinolin-4-yl)ethyl)-3-(3-fluorophenyl)-1-methylurea (Compounds 410 & 411)

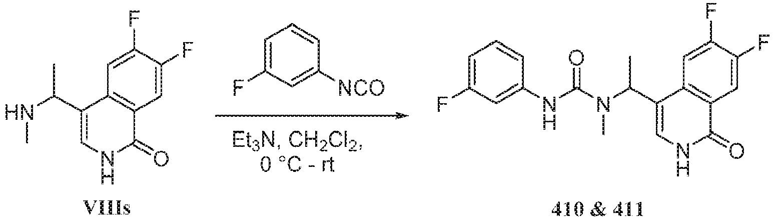

Racemic 1-(1-(6,7-difluoro-1-oxo-1,2-dihydroisoquinolin-4-yl)ethyl)-3-(3-fluorophenyl)-1-methylurea was synthesized in a similar manner as described above from 6,7-difluoro-4-(1-(methylamino)ethyl)isoquinolin-1(2*H*)-one (**VIIIs**) and 3-fluorophenyl isocyanate. The enantiomers were subsequently separated by chiral SFC, Column: Chiralpak IC (250 × 30 mm, 5 µm) 75% CO₂/MeOH, Flow rate 90 g/min.

1-(1-(6,7-Difluoro-1-oxo-1,2-dihydroisoquinolin-4-yl)ethyl)-3-(3-fluorophenyl)-1-methylurea - Enantiomer I (**Compound 410**), LCMS: *m*/*z* found 376.3 [M+H]⁺, RT = 3.85 min (Method A); ¹H NMR (400 MHz, DMSO-d₆): δ 11.65 (bs, 1H), 8.47 (s, 1H), 8.08-8.13 (m, 1H), 7.71-7.76 (m, 1H), 7.49-7.53 (m, 1H), 7.25-7.35 (m, 2H), 7.22 (s, 1H), 6.76-6.80 (m, 1H), 5.76-5.81 (m, 1H), 2.61 (s, 3H), 1.43 (d, 3H); Chiral analytical SFC: RT = 2.09 min, Column: Chiralpak IC-3, (4.6 × 150 mm) 3 µm, 70.0% CO₂/MeOH, Flow = 3.0 g/min.

1-(1-(6,7-Difluoro-1-oxo-1,2-dihydroisoquinolin-4-yl)ethyl)-3-(3-fluorophenyl)-1-methylurea - Enantiomer II (**Compiund 411**), LCMS: *m*/*z* found 376.3 [M+H]⁺, RT = 3.84 min (Method A); ¹H NMR (400 MHz, DMSO-d₆): δ 11.65 (bs, 1H), 8.47 (s, 1H), 8.08-8.13 (m, 1H), 7.71-7.76 (m, 1H), 7.49-7.53 (m, 1H), 7.25-7.35 (m, 2H), 7.22 (s, 1H), 6.76-6.80 (m, 1H), 5.76-5.81 (m, 1H), 2.61 (s, 3H), 1.43 (d, 3H); Chiral analytical SFC: RT = 3.43 min, Column: Chiralpak IC-3, (4.6 × 150 mm) 3 µm, 70.0% CO₂/MeOH, Flow = 3.0 g/min.

### 1-(1-(6,7-Difluoro-1-oxo-1,2-dihydroisoquinolin-4-yl)ethyl)-3-(3-chlorophenyl)-1-methylurea (Compounds 412 & 413)

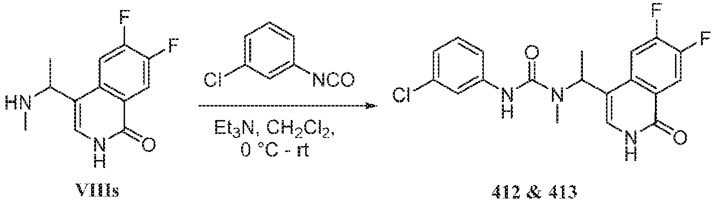

Racemic 1-(1-(6,7-difluoro-1-oxo-1,2-dihydroisoquinolin-4-yl)ethyl)-3-(3-chlorophenyl)-1-methylurea was synthesized in a similar manner as described above from 6,7-difluoro-4-(1-(methylamino)ethyl)isoquinolin-1(2*H*)-one **(VIIIs)** and 3-chlorophenyl isocyanate. The enantiomers were subsequently separated by chiral SFC, Column: (R,R) Whelk-01 (250 × 30 mm, 5 µm) 60% CO₂/MeOH, Flow rate 90 g/min.

1-(1-(6,7-Difluoro-1-oxo-1,2-dihydroisoquinolin-4-yl)ethyl)-3-(3-chlorophenyl)-1-methylurea - Enantiomer I (**Compound 412**), LCMS: *m*/*z* found 392.2/394.2 [M+H]⁺, RT = 4.27 min (Method A); ¹H NMR (400 MHz, DMSO-d₆): δ 11.71 (bs, 1H), 8.46 (bs, 1H), 8.08-8.13 (m, 1H), 7.71-7.76 (m, 2H), 7.46-7.49 (m, 1H), 7.29 (t, 1H), 7.22 (s, 1H), 7.00-7.03 (m, 1H), 5.76-5.81 (m, 1H), 2.61 (s, 3H), 1.43 (d, 3H); Chiral analytical SFC: RT = 2.95 min, Column: (R,R) Whelk-01, (4.6 × 150 mm) 3.5 µm, 60.0% CO₂/MeOH, Flow = 3.0 g/min.

1-(1-(6,7-Difluoro-1-oxo-1,2-dihydroisoquinolin-4-yl)ethyl)-3-(3-chlorophenyl)-1-methylurea - Enantiomer II (**Compound 413**), LCMS: *m*/*z* found 392.2/394.2 [M+H]⁺, RT = 4.27 min (Method A); ¹H NMR (400 MHz, DMSO-d₆): δ 11.71 (bs, 1H), 8.46 (bs, 1H), 8.08-8.13 (m, 1H), 7.71-7.76 (m, 2H), 7.46-7.49 (m, 1H), 7.29 (t, 1H), 7.22 (s, 1H), 7.00-7.03 (m, 1H), 5.76-5.81 (m, 1H), 2.61 (s, 3H), 1.43 (d, 3H); Chiral analytical SFC: RT = 4.07 min, Column: (R,R) Whelk-01, (4.6 × 150 mm) 3.5 µm, 60.0% CO₂/MeOH, Flow = 3.0 g/min.

### 1-(1-(6,7-Difluoro-1-oxo-1,2-dihydroisoquinolin-4-yl)ethyl)-3-(3,4,5-trifluorophenyl)-1-methylurea (Compounds 414 & 415)

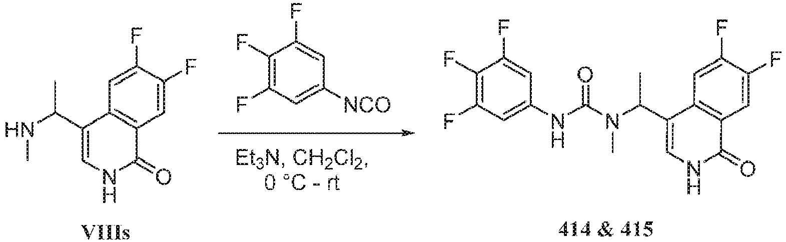

Racemic 1-(1-(6,7-difluoro-1-oxo-1,2-dihydroisoquinolin-4-yl)ethyl)-3-(3,4,5-trifluorophenyl)-1-methylurea was synthesized in a similar manner as described above from 6,7-difluoro-4-(1-(methylamino)ethyl)isoquinolin-1(2*H*)-one **(VIIIs)** and 3,4,5-trifluorophenyl isocyanate. The enantiomers were subsequently separated by chiral SFC, Column: Chiralpak AD-H (250 × 30 mm, 5 µm) 75% CO₂/MeOH, Flow rate 90 g/min.

1-(1-(6,7-Difluoro-1-oxo-1,2-dihydroisoquinolin-4-yl)ethyl)-3-(3,4,5-trifluorophenyl)-1-methylurea - Enantiomer I (**Compound 414**), LCMS: *m*/*z* found 412.2 [M+H]⁺, RT = 4.49 min (Method A); ¹H NMR (400 MHz, DMSO-d₆): δ 11.63 (bs, 1H), 8.62 (bs, 1H), 8.09-8.14 (m, 1H), 7.66-7.71 (m, 1H), 7.50-7.55 (m, 2H), 7.22 (s, 1H), 5.74-5.79 (m, 1H), 2.60 (s, 3H), 1.43 (d, 3H); Chiral analytical SFC: RT = 1.45 min, Column: Chiralpak AD-H, (4.6 × 150 mm) 5 µm, 70.0% CO₂/MeOH, Flow = 3.0 g/min.

1-(1-(6,7-Difluoro-1-oxo-1,2-dihydroisoquinolin-4-yl)ethyl)-3-(3,4,5-trifluorophenyl)-1-methylurea - Enantiomer II (**Compound 415**), LCMS: *m*/*z* found 412.1 [M+H]⁺, RT = 4.49 min (Method A); ¹H NMR (400 MHz, DMSO-d₆): δ 11.63 (bs, 1H), 8.62 (bs, 1H), 8.09-8.14 (m, 1H), 7.66-7.71 (m, 1H), 7.50-7.55 (m, 2H), 7.22 (s, 1H), 5.74-5.79 (m, 1H), 2.60 (s, 3H), 1.43 (d, 3H); Chiral analytical SFC: RT = 3.64 min, Column: Chiralpak AD-H, (4.6 × 150 mm) 5 µm, 70.0% CO₂/MeOH, Flow = 3.0 g/min.

### 3-(3,5-Dichlorophenyl)-1-(1-(6,7-difluoro-1-oxo-1,2-dihydroisoquinolin-4-yl)ethyl)-1-methylurea (Compounds 416 & 417)

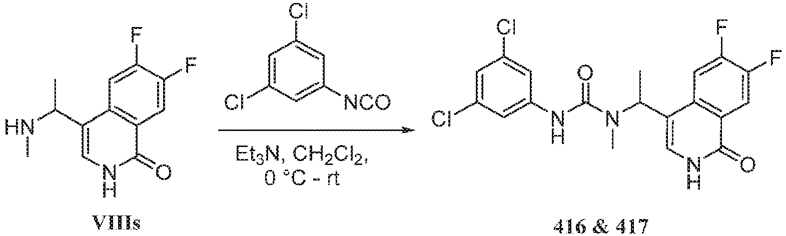

Racemic 3-(3,5-dichlorophenyl)-1-(1-(6,7-difluoro-1-oxo-1,2-dihydroisoquinolin-4-yl)ethyl)-1-methylurea was synthesized in a similar manner as described above from 6,7-difluoro-4-(1-(methylamino)ethyl)isoquinolin-1(2*H*)-one (**VIIIs**) and 3,5-dichlorophenyl isocyanate. The enantiomers were subsequently separated by chiral SFC, Column: (R,R) Whelk-01 (250 × 30 mm, 5 µm) 7 0% CO₂/MeOH, Flow rate 90 g/min.

3-(3,5-Dichlorophenyl)-1-(1-(6,7-difluoro-1-oxo-1,2-dihydroisoquinolin-4-yl)ethyl)-1-methylurea - Enantiomer I (**Compound 416**), LCMS: *m*/*z* found 426.1/428.0/430.0 [M+H]⁺, RT = 5.05 min (Method A); ¹H NMR (400 MHz, DMSO-d₆): δ 11.61 (bs, 1H), 8.52 (bs, 1H), 8.08-8.13 (m, 1H), 7.65-7.71 (m, 3H), 7.24 (s, 1H), 7.16 (t, 1H), 5.74-5.79 (m, 1H), 2.60 (s, 3H), 1.43 (d, 3H); Chiral analytical SFC: RT = 2.84 min, Column: (R,R) Whelk-01, (4.6 × 150 mm) 3.5 µm, 60.0% CO₂/MeOH, Flow = 3.0 g/min.

3-(3,5-Dichlorophenyl)-1-(1-(6,7-difluoro-1-oxo-1,2-dihydroisoquinolin-4-yl)ethyl)-1-methylurea - Enantiomer II (**Compound 417**), LCMS: *m*/*z* found 426.1/428.0/430.0 [M+H]⁺, RT = 5.05 min (Method A); ¹H NMR (400 MHz, DMSO-d₆): δ 11.61 (bs, 1H), 8.52 (bs, 1H), 8.08-8.13 (m, 1H), 7.65-7.71 (m, 3H), 7.24 (s, 1H), 7.16 (t, 1H), 5.74-5.79 (m, 1H), 2.60 (s, 3H), 1.43 (d, 3H); Chiral analytical SFC: RT = 4.11 min, Column: (R,R) Whelk-01, (4.6 × 150 mm) 3.5 µm, 60.0% CO₂/MeOH, Flow = 3.0 g/min.

### 1-(1-(6,7-Difluoro-1-oxo-1,2-dihydroisoquinolin-4-yl)ethyl)-3-(2-fluorophenyl)-1-methylurea (Compounds 420 & 421)

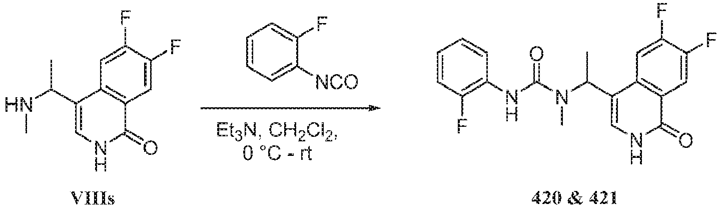

Racemic 1-(1-(6,7-difluoro-1-oxo-1,2-dihydroisoquinolin-4-yl)ethyl)-3-(2-fluorophenyl)-1-methylurea was synthesized in a similar manner as described above from 6,7-difluoro-4-(1-(methylamino)ethyl)isoquinolin-1(2*H*)-one (**VIIIs**) and 2-fluorophenyl isocyanate. The enantiomers were subsequently separated by chiral SFC, Column: Chiralcel OX-H (250 × 30 mm, 5 µm) 75% CO₂/MeOH, Flow rate 90 g/min.

1-(1-(6,7-Difluoro-1-oxo-1,2-dihydroisoquinolin-4-yl)ethyl)-3-(2-fluorophenyl)-1-methylurea - Enantiomer I (**Compound 420**), LCMS: *m*/*z* found 376.2 [M+H]⁺, RT = 3.50 min (Method A); ¹H NMR (400 MHz, DMSO-d₆): δ 11.61 (bs, 1H), 8.08-8.13 (m, 2H), 7.76-7.81 (m, 1H), 7.42-7.47 (m, 1H), 7.13-7.25 (m, 4H), 5.72-5.77 (m, 1H), 2.59 (s, 3H), 1.42 (d, 3H); Chiral analytical SFC: RT = 1.73 min, Column: Chiralcel OX-H, (4.6 × 150 mm) 5 µm, 70.0% CO₂/MeOH, Flow = 3.0 g/min.

1-(1-(6,7-Difluoro-1-oxo-1,2-dihydroisoquinolin-4-yl)ethyl)-3-(2-fluorophenyl)-1-methylurea - Enantiomer II (**Compounds 421**), LCMS: *m*/*z* found 376.2 [M+H]⁺, RT = 3.50 min (Method A); ¹H NMR (400 MHz, DMSO-d₆): δ 11.61 (bs, 1H), 8.08-8.13 (m, 2H), 7.76-7.81 (m, 1H), 7.42-7.47 (m, 1H), 7.13-7.25 (m, 4H), 5.72-5.77 (m, 1H), 2.59 (s, 3H), 1.42 (d, 3H); Chiral analytical SFC: RT = 2.22 min, Column: Chiralcel OX-H, (4.6 × 150 mm) 5 µm, 70.0% CO₂/MeOH, Flow = 3.0 g/min.

### 1-(1-(6,7-Difluoro-1-oxo-1,2-dihydroisoquinolin-4-yl)ethyl)-3-(4-fluoro-3-methylphenyl)-1-methylurea (Compounds 426 & 427)

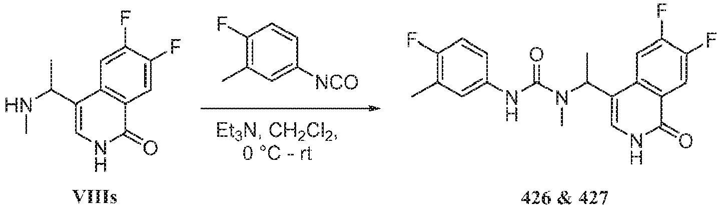

Racemic 1-(1-(6,7-difluoro-1-oxo-1,2-dihydroisoquinolin-4-yl)ethyl)-3-(4-fluoro-3-methylphenyl)-!-methylurea was synthesized in a similar manner as described above from 6,7-difluoro-4-(1-(methylamino)ethyl)isoquinolin-1(2*H*)-one (**VIIIs**) and 1-fluoro-4-isocyanato-2-methylbenzene. The enantiomers were subsequently separated by chiral SFC, Column: Chiralpak IC (250 × 30 mm, 5 µm) 70% CO₂/MeOH, Flow rate 90 g/min. 1-(1-(6,7-Difluoro-1-oxo-1,2-dihydroisoquinolin-4-yl)ethyl)-3-(4-fluoro-3-methylphenyl)-1-methylurea - Enantiomer I (**Compound 426**), LCMS: *m*/*z* found 390.3 [M+H]⁺, RT = 4.07 min (Method A); ¹H NMR (400 MHz, DMSO-d₆): δ 11.60 (bs, 1H), 8.24 (bs, 1H), 8.08-8.13 (m, 1H), 7.74-7.79 (m, 1H), 7.40-7.43 (m, 1H), 7.30-7.35 (m, 1H), 7.20 (s, 1H), 7.03 (t, 1H), 5.60-5.76 (m, 1H), 2.58 (s, 3H), 2.21 (s, 3H), 1.41 (d, 3H); Chiral analytical SFC: RT = 2.09 min, Column: Chiralpak IC, (4.6 × 150 mm) 5 µm, 70.0% CO₂/MeOH, Flow = 3.0 g/min.

1-(1-(6,7-Difluoro-1-oxo-1,2-dihydroisoquinofin-4-yl)ethyl)-3-(4-fluoro-3-methylphenyl)-1-methylurea - Enantiomer II (**Compound 427**), LCMS: *m*/*z* found 390.3 [M+H]⁺, RT = 4.07 min (Method A); ¹H NMR (400 MHz, DMSO-d₆): δ 11.60 (bs, 1H), 8.24 (bs, 1H), 8.08-8.13 (m, 1H), 7.74-7.79 (m, 1H), 7.40-7.43 (m, 1H), 7.30-7.35 (m, 1H), 7.20 (s, 1H), 7.03 (t, 1H), 5.60-5.76 (m, 1H), 2.58 (s, 3H), 2.21 (s, 3H), 1.41 (d, 3H); Chiral analytical SFC: RT = 3.22 min, Column: Chiralpak IC, (4.6 × 150 mm) 5 µm, 70.0% CO₂/MeOH, Flow = 3.0 g/min.

### N-(3-Cyano-4-fluorophenyl)-1H-imidazole-1-carboxamide

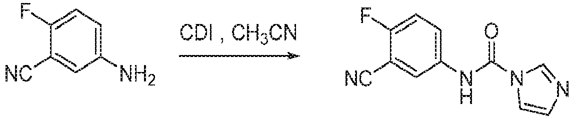

To a solution of 0.15 g (1.0 mmol, 1.0 eq.) of 5-amino-2-fluorobenzonitrile in 5 mL of acetonitrile was added 0.20 g (1.2 mmol, 1.2 eq.) of 1,1'-carbonyldiimidazole and the mixture was stirred at room temperature for 4 h. The mixture was then diluted with 20 mL of water, the precipitated solid was collected by filtration and dried under high vacuum to provide 0.22 g of *N*-(3-cyano-4-fluorophenyl)-1*H*-imidazole-1-carboxamide. ¹H NMR (400 MHz, CDCl₃): δ 8.33 (s, 1H), 7.86-7.90 (m, 2H), 7.61 (s, 1H), 7.32-7.35 (m, 1H), 7.22-7.25 (m, 1H), 7.10-7.13 (m, 1H).

### 3-(3-Cyano-4-fluorophenyl)-1-(1-(6,7-difluoro-1-oxo-1,2-dihydroisoquinolin-4-yl)ethyl)-1-methylurea (Compounds 428 & 429)

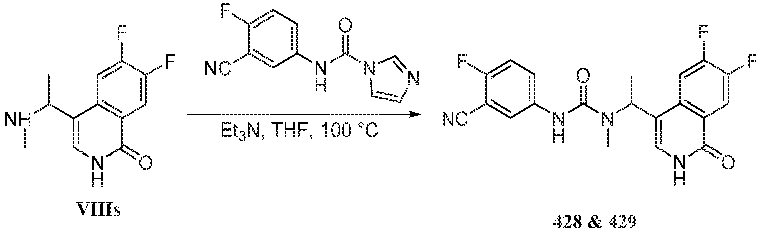

To a suspension of 0.15 g (0.63 mmol, 1.0 eq.) of 6,7-difluoro-4-(1-(methylamino)ethyl)isoquinolin-1(2*H*)-one **(VIIIs)** in 5 mL of THF under a nitrogen atmosphere in a sealed tube was added 0.19 g (1.89 mmol, 3.0 eq.) of triethylamine followed by 0.22 g (0.94 mmol, 1.5 eq.) of *N*-(3-cyano-4-fluorophenyl)-1*H*-imidazole-1-carboxamide and the mixture was heated at 100 °C for 16 h. The mixture was allowed to cool to room temperature, diluted with 20 mL of water and extracted with 3 × 50 mL of ethyl acetate. The combined organic extracts were washed with 50 mL of brine, dried (Na₂SO₄), filtered and the solvent was removed *in vacuo.* The residue was purified by MPLC (SiO₂, eluting with linear gradient 15-18% of [30% methanol in methylene chloride]/methylene chloride) to provide 0.11 g (0.27 mmol, 43%) of racemic 3-(3-cyano-4-fluorophenyl)-1-(1-(6,7-difluoro-1-oxo-1,2-dihydroisoquinolin-4-yl)ethyl)-1-methylurea. LCMS: *m*/*z* found 401.06 [M+H]⁺. The enantiomers were subsequently separated by chiral SFC, Column: Chiralpak IG (250 × 30 mm, 5 µm) 60% CO₂/MeOH, Flow rate 90 g/min.

3-(3-Cyano-4-fluorophenyl)-1-(1-(6,7-difluoro-1-oxo-1,2-dihydroisoquinolin-4-yl)ethyl)-1-methylurea - Enantiomer I (**Compound 428**), LCMS: *m*/*z* found 401.2 [M+H]⁺, RT = 3.81 min (Method A); ¹H NMR (400 MHz, DMSO-d₆): δ 11.62 (bs, 1H), 8.65 (bs, 1H), 8.09-8.14 (m, 1H), 8.00-8.03 (m, 1H), 7.85-7.89 (m, 1H), 7.68-7.73 (m, 1H), 7.46 (t, 1H), 7.23 (d, 1H), 5.76-5.81 (m, 1H), 2.61 (s, 3H), 1.43 (d, 3H); Chiral analytical SFC: RT = 0.88 min, Column: Chiralpak IG, (4.6 × 150 mm) 3 µm, 60.0% CO₂/MeOH, Flow = 3.0 g/min.

3-(3-Cyano-4-fluorophenyl)-1-(1-(6,7-difluoro-1-oxo-1,2-dihydroisoquinolin-4-yl)ethyl)-1-methylurea - Enantiomer II (**Compound 429**), LCMS: *m*/*z* found 401.2 [M+H]⁺, RT = 3.81 min (Method A); ¹H NMR (400 MHz, DMSO-d₆): δ 11.62 (bs, 1H), 8.65 (bs, 1H), 8.09-8.14 (m, 1H), 8.00-8.03 (m, 1H), 7.85-7.89 (m, 1H), 7.68-7.73 (m, 1H), 7.46 (t, 1H), 7.23 (d, 1H), 5.76-5.81 (m, 1H), 2.61 (s, 3H), 1.43 (d, 3H); Chiral analytical SFC: RT = 2.93 min, Column: Chiralpak IG, (4.6 × 150 mm) 3 µm, 60.0% CO₂/MeOH, Flow = 3.0 g/min.

### Phenyl (3-cyano-4-fluorophenyl)carbamate

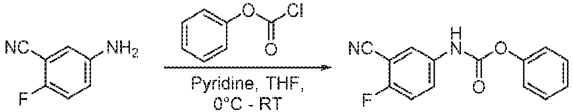

To a solution of 1.0 g (7.35 mmol, 1.0 eq.) of 5-amino-2-fluorobenzonitrile in 10 mL of THF at 0 °C under a nitrogen atmosphere was added 0.85 mL (11.02 mmol, 1.5 eq.) of pyridine followed by 0.8 mL (6.6 mmol, 0.9 eq.) of phenyl chloroformate. The mixture was allowed to warm to room temperature and the stirred for 2 h. The mixture was diluted with 30 mL of water and extracted with 2 × 100 mL of ethyl acetate. The combined organic extracts were washed with 20 mL of water, 20 mL of brine, dried (Na₂SO₄), filtered and the solvent was removed *in vacuo.* The residue was triturated with 10 mL of water, filtered the resulting solid was dried under high vacuum to provide 1.3 g (5.07 mmol, 68%) of phenyl (3-cyano-4-fluorophenyl)carbamate. LCMS: *m*/*z* found 257.10 [M+H]⁺; ¹H NMR (400 MHz, CDCl₃): δ 7.78-7.82 (m, 1H), 7.63-7.66 (m, 1H), 7.41 (t, 2H), 7.23-7.30 (m, 2H), 7.18 (d, 2H), 7.01 (bs, 1H).

### 3-(3-Cyano-4-fluorophenyl)-1-(1-(6,7-difluoro-1-oxo-1,2-dihydroisoquinolin-4-yl)ethyl)-1-methylurea (Compounds 428 & 429)

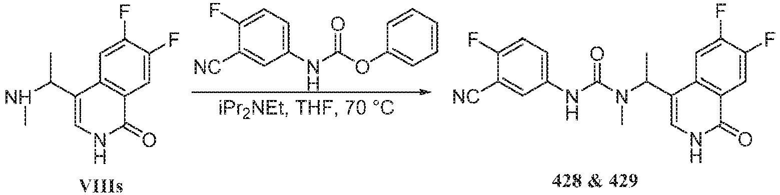

To a solution of 0.5 g (2.10 mmol, 1.0 eq.) of 6,7-difluoro-4-(1-(methylamino)ethyl) isoquinolin-1(2*H*)-one (**VIIIs**) in 5 mL of DMF at room temperature was added 0.9 mL (5.25 mmol, 2.5 eq) of *N*,*N*-diisopropylethylamine followed by 0.65 g (2.52 mmol, 1.2 eq.) of phenyl (3-cyano-4-fluorophenyl)carbamate. The mixture was heated to 70 °C and stirred for 2 h. The mixture was poured into 40 mL of ice-cold water and the precipitated solid was collected by filtration, washed with 10 mL of water and dried under high vacuum to provide 650 mg (1.6 mmol, 77%) of racemic 3-(3-cyano-4-fluorophenyl)-1-(1-(6,7-difluoro-1-oxo-1,2-dihydroisoquinolin-4-yl)ethyl)-1-methylurea. LCMS: *m*/*z* found 401.22 [M+H]⁺. The enantiomers were subsequently separated by chiral SFC, Column: Chiralpak IG (250 × 30 mm) 5 µ, 50% CO₂/MeOH, Flow rate 70 g/min to provide 0.14 g of 3-(3-cyano-4-fluorophenyl)-1-(1-(6,7-difluoro-1-oxo-1,2-dihydroisoquinolin-4-yl)ethyl)-1-methylurea - Enantiomer I (**Compound 428**) and 0.14 g of 3-(3-cyano-4-fluorophenyl)-1-(1-(6,7-difluoro-1-oxo-1,2-dihydroisoquinolin-4-yl)ethyl)-1-methylurea - Enantiomer II **(Compound 429**).

3-(3-Cy ano-4-fluorophenyl)-1 -(1 -(6,7-difluoro-1 -oxo-1,2-dihydroisoquinolin-4-yl)ethyl)-1-methylurea - Enantiomer I (**Compound 428**), LCMS: *m*/*z* found 401.2 [M+H]⁺, RT = 3.81 min (Method A); ¹H NMR (400 MHz, DMSO-d₆): δ 11.62 (bs, 1H), 8.65 (bs, 1H), 8.09-8.14 (m, 1H), 8.00-8.03 (m, 1H), 7.85-7.89 (m, 1H), 7.68-7.73 (m, 1H), 7.46 (t, 1H), 7.23 (d, 1H), 5.76-5.81 (m, 1H), 2.61 (s, 3H), 1.43 (d, 3H); Chiral analytical SFC: RT = 0.88 min, Column: Chiralpak IG, (4.6 × 150 mm) 3 µm, 60.0% CO₂/MeOH, Flow = 3.0 g/min.

3-(3-Cyano-4-fluorophenyl)-1-(1-(6,7-difluoro-1-oxo-1,2-dihydroisoquinolin-4-yl)ethyl)-1-methylurea - Enantiomer II (**Compound 429**), LCMS: *m*/*z* found 401.2 [M+H]⁺, RT = 3.81 min (Method A); ¹H NMR (400 MHz, DMSO-d₆): δ 11.62 (bs, 1H), 8.65 (bs, 1H), 8.09-8.14 (m, 1H), 8.00-8.03 (m, 1H), 7.85-7.89 (m, 1H), 7.68-7.73 (m, 1H), 7.46 (t, 1H), 7.23 (d, 1H), 5.76-5.81 (m, 1H), 2.61 (s, 3H), 1.43 (d, 3H); Chiral analytical SFC: RT = 2.93 min, Column: Chiralpak IG, (4.6 × 150 mm) 3 µm, 60.0% CO₂/MeOH, Flow = 3.0 g/min.

### Phenyl 2,3-difluorophenylcarbamate

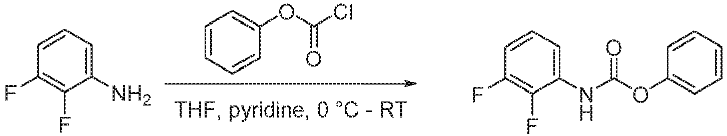

To a solution of 0.5 g (3.9 mmol, 1.0 eq.) of 2,3-difluoroaniline in 5 mL of THF at 0 °C was added 1.2 mL (15.5 mmol, 4.0 eq.) of pyridine followed by 0.53 mL (4.2 mmol, 1.1 eq.) of phenyl chloroformate and the mixture was stirred at room temperature for 2 h. The mixture was then diluted with 30 mL of water and extracted with 3 × 100 mL of ethyl acetate. The combined organic extracts were washed with 50 mL of brine, dried (Na₂SO₄), filtered and the solvent was removed in *vacuo.* The residue was purified by MPLC (SiO₂, eluting with 30% ethyl acetate in petroleum ether) to provide 0.38 g (1.5 mmol, 39%) of phenyl 2,3-difluorophenylcarbamate. LCMS: *m*/*z* found 250.0 [M+H]⁺, ¹H NMR (400 MHz, CDCl₃): δ 7.88-7.93 (t, 1H), 7.39-7.43 (m, 2H), 7.24-7.32 (m, 2H), 7.17-7.22 (m, 2H), 7.06-7.11 (m, 1H), 6.87-6.93 (m, 1H).

### 1-(1-(6,7-Difluoro-1-oxo-1,2-dihydroisoquinolin-4-yl)ethyl)-3-(2,3-difluorophenyl)-1-methylurea (Compounds 430 & 431)

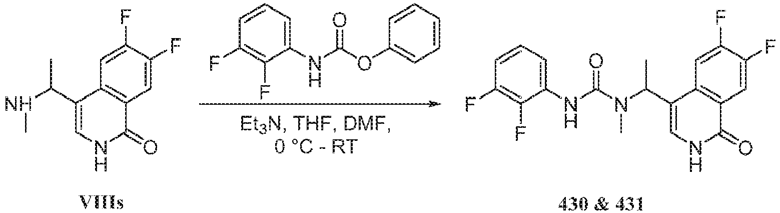

To a solution of 0.1 g (0.42 mmol, 1.0 eq.) of 6,7-difluoro-4-(1-(methylamino)ethyl)isoquinolin-1(2*H*)-one **(VIIIs)** in 2 mL of 2:1 *v*/*v* THF:DMF at 0 °C was added 0.14 mL (1.05 mmol, 2.5 eq.) of triethylamine followed by 94 mg (0.37 mmol, 0.9 eq.) of phenyl 2,3-difluorophenylcarbamate and the mixture was stirred at room temperature for 16 h. The solvent was then removed *in vacuo* and the residue was purified by MPLC (SiO₂, eluting with 8% methanol in methylane chloride) to provide 60 mg (0.15 mmol, 36%) of racemic 1-(1-(6,7-difluoro-1-oxo-1,2-dihydroisoquinolin-4-yl)ethyl)-3-(2,3-difluorophenyl)-1-methylurea. LCMS: *m*/*z* found 394.1 [M+H]⁺. The enantiomers were subsequently separated by chiral SFC, Column: Chiralpak IC (250 × 30 mm, 5 µm) 85% CO₂/MeOH, Flow rate 90 g/min.

1-(1-(6,7-Difluoro-1-oxo-1,2-dihydroisoquinolin-4-yl)ethyl)-3-(2,3-difluorophenyl)-1-methylurea - Enantiomer I (**Compound 430**), LCMS: *m*/*z* found 394.1; ¹H NMR (400 MHz, DMSO-d₆): δ 11.46 (bs, 1H), 8.44 (bs, 1H), 8.09-8.14 (m, 1H), 7.55-7.80 (m, 1H), 7.14-7.22 (m, 4H), 5.73-5.76 (m, 1H), 2.60 (s, 3H), 1.43 (d, 3H); Chiral analytical SFC: RT = 5.02 min, Column: Chiralpak IC-3, (4.6 × 150 mm) 5 µm, 85% CO₂/MeOH, Flow = 3.0 g/min.

1-(1-(6,7-Difluoro-1-oxo-1,2-dihydroisoquinolin-4-yl)ethyl)-3-(2,3-difluorophenyl)-1-methylurea - Enantiomer II (**Compound 431**), LCMS: *m*/*z* found 394.1; ¹H NMR (400 MHz, DMSO-d₆): δ 11.46 (bs, 1H), 8.44 (bs, 1H), 8.09-8.14 (m, 1H), 7.55-7.80 (m, 1H), 7.14-7.22 (m, 4H), 5.73-5.76 (m, 1H), 2.60 (s, 3H), 1.43 (d, 3H); Chiral analytical SFC: RT = 5.92 min, Column: Chiralpak IC-3, (4.6 × 150 mm) 5 µm, 85% CO₂/MeOH, Flow = 3.0 g/min.

### Phenyl 3,5-dichloro-4-fluorophenylcarbamate

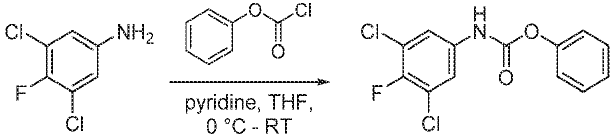

To a solution of 0.5 g (2.7 mmol, 1.0 eq.) of 3,5-dichloro-4-fluoroaniline in 15 mL of THF at 0 °C under a nitrogen atmosphere was added 0.8 mL (11.1 mmol, 4.0 eq.) of pyridine followed by 0.52 g (3.3 mmol, 1.2 eq.) of phenyl chlorformate. The mixture was allowed to warm to room temperature and stirred for 16 h. The mixture was then diluted with 100 mL of water and extracted with 2 × 100 mL of ethyl acetate. The combined organic extracts were washed with 100 mL of brine, dried (Na₂SO₄), filtered and the solvent was removed *in vacuo.* The residue was triturated with 30 mL of n-pentane, filtered and the solid was dried under vacuum to provide 0.45 g (1.5 mmol, 55%) of phenyl 3,5-dichloro-4-fluorophenylcarbamate. LCMS: *m*/*z* found 300.3 [M+H]⁺, ¹H NMR (400 MHz, CDCl₃): δ 7.47 (d, 2H), 7.38-7.42 (m, 2H), 7.25-7.28 (m, 1H), 7.16-7.19 (m, 2H), 6.88 (bs, 1H).

### 3-(3,5-dichloro-4-fluorophenyl)-1-(1-(6,7-difluoro-1-oxo-1,2-dihydroisoquinolin-4-yl)ethyl)-1-methylurea (Compounds 438 & 439)

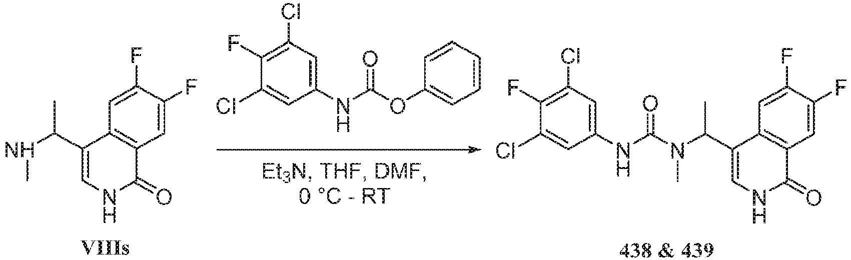

To a solution of 100 mg (0.42 mmol, 1.0 eq.) of 6,7-difluoro-4-(1-(methylamino)ethyl)isoquinolin-1(2*H*)-one **(VIIIs)** in 6 mL of THF at 0 °C was added 0.15 mL (1.4 mmol, 2.5 eq.) of triethylamine followed by 0.12 g (0.42 mmol, 1.0 eq.) of phenyl 3,5-dichloro-4-fluorophenylcarbamate. The mixture was allowed to warm to room temperature and stirred for 16 h. The mixture was then diluted with 20 mL of water and extracted with 3 × 50 mL of methylene chloride. The combined organic extracts were washed with 30 mL of brine, dried (Na₂SO₄), filtered and the solvent was removed *in vacuo.* The residue was triturated with 30 mL of *n*-pentane to provide 130 mg (0.29 mmol, 69%) of racemic 3-(3,5-dichloro-4-fluorophenyl)-1-(1-(6,7-difluoro-1-oxo-1,2-dihydroisoquinolin-4-yl)ethyl)-1-methylurea. LCMS: *m*/*z* found 443.97 [M+H]⁺. The enantiomers were subsequently separated by chiral SFC, Column: (R,R)Whelk-01 (250 × 30 mm, 5 µm) 60% CO₂/MeOH, Flow rate 90 g/min.

3-(3,5-Dichloro-4-fluorophenyl)-1-(1-(6,7-difluoro-1-oxo-1,2-dihydroisoquinolin-4-yl)ethyl)-1-methylurea- Enantiomer I (**Compound 438**), LCMS: *m*/*z* found 444.1/446.1/448.1, RT = 5.19 min (Method A); ¹H NMR (400 MHz, DMSO-d₆): δ 11.63 (bs, 1H), 8.60 (bs 1H), 8.09-8.14 (m, 1H), 7.80 (d, 2H), 7.66-7.71 (m, 1H), 7.22 (s, 1H), 5.74-5.79 (m, 1H), 2.60 (s, 3H), 1.43 (d, 3H); Chiral analytical SFC: RT = 2.17 min, Column: (R,R)Whelk-01, (4.6 × 150 mm) 5 µm, 60% CO₂/MeOH, Flow = 3.0 g/min.

3-(3,5-Dichloro-4-fluorophenyl)-1-(1-(6,7-difluoro-1-oxo-1,2-dihydroisoquinolin-4-yl)ethyl)-1-methylurea- Enantiomer II (**Compound 439**), LCMS: *m*/*z* found 444.1/446.1/448.1, RT = 5.19 min (Method A); ¹H NMR (400 MHz, DMSO-d₆): δ 11.63 (bs, 1H), 8.60 (bs 1H), 8.09-8.14 (m, 1H), 7.80 (d, 2H), 7.66-7.71 (m, 1H), 7.22 (s, 1H), 5.74-5.79 (m, 1H), 2.60 (s, 3H), 1.43 (d, 3H); Chiral analytical SFC: RT = 2.84 min, Column: (R,R)Whelk-01, (4.6 × 150 mm) 5 µm, 60% CO₂/MeOH, Flow = 3.0 g/min.

### 1-(1-(6,7-Difluoro-1-oxo-1,2-dihydroisoquinolin-4-yl)ethyl)-1-methyl-3-((S)-1-phenylethyl)urea (Compound 475)

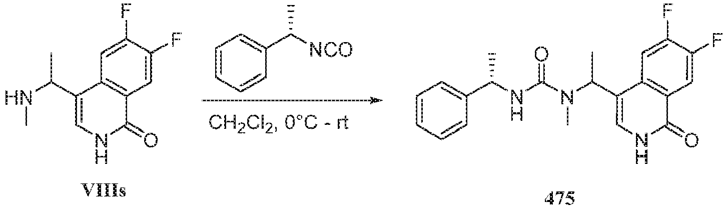

Diastereomeric 1-(1-(6,7-difluoro-1-oxo-1,2-dihydroisoquinolin-4-yl)ethyl)-1-methyl-3-((S)-1-phenylethyl)urea was synthesized in a similar manner as described above from 6,7-difluoro-4-(1-(methylamino)ethyl)isoquinolin-1(2*H*)-one (**VIIIs**) and (S)-(1-isocyanatoethyl)benzene. The diastereoisomers were subsequently separated by chiral SFC, Column: (R,R)-Whelk-01 (250 × 30 mm) 5 µ, 80% CO₂/MeOH, Flow rate 100 g/min.

1-(1-(6,7-Difluoro-1-oxo-1,2-dihydroisoquinolin-4-yl)ethyl)-1-methyl-3-((S)-1-phenylethyl)urea - Diastereomer I (**Compound 475**), LCMS: *m*/*z* found 386.3 [M+H]⁺, RT = 3.71 min (Method A); ¹H NMR (400 MHz, DMSO-d₆): δ 11.59 (bs, 1H), 8.05-8.10 (m, 1H), 7.53-7.58 (m, 1H), 7.17-7.29 (m, 5H), 7.13 (s, 1H), 6.57 (bd, 1H), 5.64-5.70 (m, 1H), 4.92-4.99 (m, 1H), 2.48 (s, 3H), 1.41 (d, 3H), 1.35 (d, 3H); Chiral analytical SFC: RT = 2.80 min, Column: (R,R)-Whelk-01, (4.6 × 150 mm) 3.5 µm, 75% CO₂/MeOH, Flow = 3.0 g/min.

1-(1-(6,7-Difluoro-1-oxo-1,2-dihydroisoquinolin-4-yl)ethyl)-1-methyl-3-((S)-1-phenylethyl)urea- Diastereomer II, LCMS: *m*/*z* found 386.3 [M+H]⁺, RT = 3.71 min (Method A); ¹H NMR (400 MHz, DMSO-d₆): δ 11.59 (bs, 1H), 8.05-8.10 (m, 1H), 7.53-7.58 (m, 1H), 7.17-7.29 (m, 5H), 7.13 (s, 1H), 6.57 (bd, 1H), 5.64-5.70 (m, 1H), 4.92-4.99 (m, 1H), 2.48 (s, 3H), 1.41 (d, 3H), 1.35 (d, 3H); Chiral analytical SFC: RT = 4.05 min, Column: (R,R)-Whelk-01, (4.6 × 150 mm) 3.5 µm, 75% CO₂/MeOH, Flow = 3.0 g/min.

### 1-(1-(6,7-Difluoro-1-oxo-1,2-dihydroisoquinolin-4-yl)ethyl)-1-methyl-3-((R)-1-phenylethyl)urea (Compound 479)

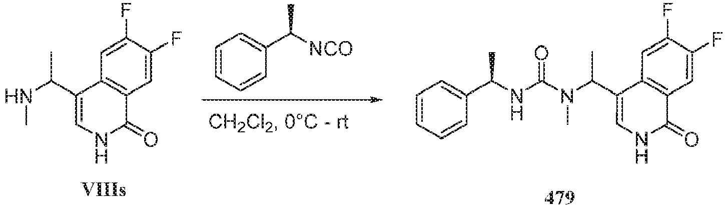

Diastereomeric 1-(1-(6,7-difluoro-1-oxo-1,2-dihydroisoquinolin-4-yl)ethyl)-1-methyl-3-((R)-1-phenylethyl)urea was synthesized in a similar manner as described above from 6,7-difluoro-4-(1-(methylamino)ethyl)isoquinolin-1(2H)-one (**VIIIs**) and (S)-(1-isocyanatoethyl)benzene. The diastereoisomers were subsequently separated by chiral SFC, Column: Chiralpak IC (250 × 30 mm) 5 µ, 80% CO₂/MeOH, Flow rate 100 g/min.

1-(1-(6,7-Difluoro-1-oxo-1,2-dihydroisoquinolin-4-yl)ethyl)-1-methyl-3-((R)-1-phenylethyl)urea - Diastereomer I. LCMS: *m*/*z* found 386.3 [M+H]⁺, RT = 3.71 min (Method A); ¹H NMR (400 MHz, DMSO-d₆): δ 11.57 (bs, 1H), 8.05-8.10 (m, 1H), 7.53-7.59 (m, 1H), 7.17-7.29 (m, 5H), 7.13 (s, 1H), 6.57 (bd, 1H), 5.65-5.70 (m, 1H), 4.92-4.99 (m, 1H), 2.50 (s, 3H), 1.41 (d, 3H), 1.35 (d, 3H); Chiral analytical SFC: RT = 2.21 min, Column: Chiralpak IC-3, (4.6 × 150 mm) 3 µm, 70% CO₂/MeOH, Flow = 3.0 g/min.

1-(1-(6,7-Difluoro-1-oxo-1,2-dihydroisoquinolin-4-yl)ethyl)-1-methyl-3-((R)-1-phenylethyl)urea- Diastereomer II (**Compound 479**), LCMS: *m*/*z* found 386.3 [M+H]⁺, RT = 3.80 min (Method A); ¹H NMR (400 MHz, DMSO-d₆): δ 11.59 (bs, 1H), 8.07-8.12 (m, 1H), 7.74-7.79 (m, 1H), 7.29-7.38 (m, 4H), 7.19-7.22 (m, 1H), 7.15 (s, 1H), 6.57 (bd, 1H), 5.66-5.71 (m, 1H), 4.95-5.01 (m, 1H), 2.47 (s, 3H), 1.38 (d, 3H), 1.31 (d, 3H); Chiral analytical SFC: RT = 3.45 min, Column: Chiralpak IC-3, (4.6 × 150 mm) 3 µm, 70% CO₂/MeOH, Flow = 3.0 g/min.

### 1-(1-(6,7-Difluoro-1-oxo-1,2-dihydroisoquinolin-4-yl)ethyl)-3-(3,4-difluorobenzyl)-1-methylurea (Compounds 476 & 477)

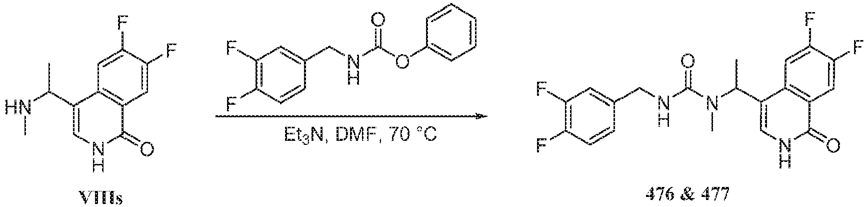

Racemic 1-(1-(6,7-difluoro-1-oxo-1,2-dihydroisoquinolin-4-yl)ethyl)-3-(3,4-difluorobenzyl)-1-methylurea was synthesized in a similar manner as described above from 6,7-difluoro-4-(1-(methylamino)ethyl)isoquinolin-1(2*H*)-one (**VIIIs**) and phenyl (3,4-difluorobenzyl)carbamate. The enantiomers were subsequently separated by chiral SFC, Column: (R,R)-Whelk-01 (250 × 30 mm) 5 µ, 80% CO₂/MeOH, Flow rate 100 g/min.

1-(1-(6,7-Difluoro-1-oxo-1,2-dihydroisoquinolin-4-yl)ethyl)-3-(3,4-difluorobenzyl)-1-methylurea - Enantiomer I (**Compound 476**), LCMS: *m*/*z* found 408.3 [M+H]⁺, RT 3.72 min (Method A); ¹H NMR (400 MHz, DMSO-d₆): δ 11.58 (bs, 1H), 8.06-8.11 (m, 1H), 7.68-7.73 (m, 1H), 7.30-7.37 (m, 1H), 7.22-7.27 (m, 1H), 7.15 (s, 1H), 7.05-7.12 (m, 1H), 7.03 (bt, 1H), 5.68-5.74 (m, 1H), 4.36-4.41 (m, 1H), 4.16-4.21 (m, 1H), 2.45 (s, 3H), 1.35 (d, 3H); Chiral analytical SFC: RT = 2.47 min, Column: (R,R)-Whelk-01, (4.6 × 150 mm) 3 µm, 60% CO₂/MeOH, Flow = 3.0 g/min.

1-(1-(6,7-Difluoro-1-oxo-1,2-dihydroisoquinolin-4-yl)ethyl)-3-(3,4-difluorobenzyl)-1-methylurea - Enantiomer II (**Compound 477**), LCMS: *m*/*z* found 408.3 [M+H]⁺, RT 3.71 min (Method A); ¹H NMR (400 MHz, DMSO-d₆): δ 11.58 (bs, 1H), 8.06-8.11 (m, 1H), 7.68-7.73 (m, 1H), 7.30-7.37 (m, 1H), 7.22-7.27 (m, 1H), 7.15 (s, 1H), 7.05-7.12 (m, 1H), 7.03 (bt, 1H), 5.68-5.74 (m, 1H), 4.36-4.41 (m, 1H), 4.16-4.21 (m, 1H), 2.45 (s, 3H), 1.35 (d, 3H); Chiral analytical SFC: RT = 3.13 min, Column: (R,R) Whelk-01, (4.6 × 150 mm) 3 µm, 60% CO₂/MeOH, Flow = 3.0 g/min.

### 1-(1-(6,7-Difluoro-1-oxo-1,2-dihydroisoquinolin-4-yl)ethyl)-3-(4-fluorobenzyl)-1-methylurea (Compound 478)

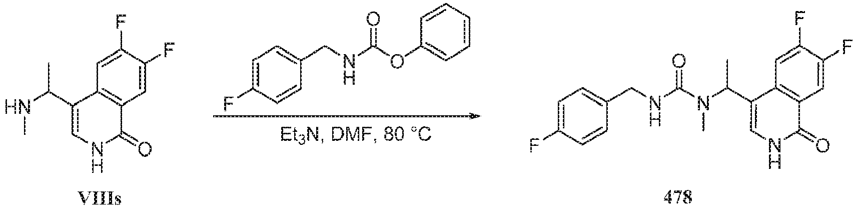

Racemic 1-(1-(6,7-difluoro-1-oxo-1,2-dihydroisoquinolin-4-yl)ethyl)-3-(4-fluorobenzyl)-1-methylurea was synthesized in a similar manner as described above from 6,7-difluoro-4-(1-(methylamino)ethyl)isoquinolin-1(2*H*)-one (**VIIIs**) and phenyl (4-fluorobenzyl)carbamate. The enantiomers were subsequently separated by chiral SFC, Column: (R,R)-Whelk-01 (250 × 30 mm) 5 µ, 80% CO₂/MeOH, Flow rate 100 g/min.

1-(1-(6,7-Difluoro-1-oxo-1,2-dihydroisoquinolin-4-yl)ethyl)-3-(4-fluorobenzyl)-1-methylurea - Enantiomer I (**Compound 478**), LCMS: *m*/*z* found 390.3 [M+H]⁺, RT = 3.58 min (Method A); ¹H NMR (400 MHz, DMSO-d₆): δ 11.58 (bs, 1H), 8.06-8.11 (m, 1H), 7.70-7.76 (m, 1H), 7.28-7.32 (m, 2H), 7.16 (s, 1H), 7.08-7.12 (m, 2H), 6.99 (bt, 1H), 5.70-5.75 (m, 1H), 4.36-4.41 (m, 1H), 4.18-4.24 (m, 1H), 2.44 (s, 3H), 1.35 (d, 3H); Chiral analytical SFC: RT = 2.71 min, Column: (R,R)-Whelk-01, (4.6 × 150 mm) 3.5 µm, 75% CO₂/MeOH, Flow = 3.0 g/min.

1-(1-(6,7-Difluoro-1-oxo-1,2-dihydroisoquinolin-4-yl)ethyl)-3-(4-fluorobenzyl)-1-methylurea - Enantiomer II (**Compound 479**), LCMS: *m*/*z* found 390.3 [M+H]⁺, RT = 3.58 min (Method A); ¹H NMR (400 MHz, DMSO-d₆): δ 11.58 (bs, 1H), 8.06-8.11 (m, 1H), 7.70-7.76 (m, 1H), 7.28-7.32 (m, 2H), 7.16 (s, 1H), 7.08-7.12 (m, 2H), 6.99 (bt, 1H), 5.70-5.75 (m, 1H), 4.36-4.41 (m, 1H), 4.18-4.24 (m, 1H), 2.44 (s, 3H), 1.35 (d, 3H); Chiral analytical SFC: RT = 3.49 min, Column: (R,R)-Whelk-01, (4.6 × 150 mm) 3.5 µm, 75% CO₂/MeOH, Flow = 3.0 g/min.

### 1-(1-(6,7-Difluoro-1-oxo-1,2-dihydroisoquinolin-4-yl)ethyl)-1-methyl-3-(3,4,5-trifluorobenzyl)urea (Compounds 481 & 482)

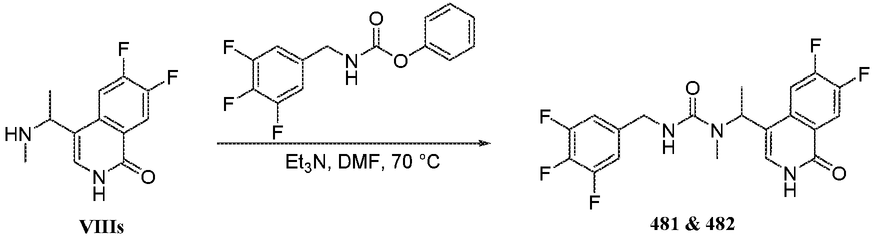

Racemic 1-(1-(6,7-difluoro-1-oxo-1,2-dihydroisoquinolin-4-yl)ethyl)-1-methyl-3-(3,4,5-trifluorobenzyl)urea was synthesized in a similar manner as described above from 6,7-difluoro-4-(1-(methylamino)ethyl)isoquinolin-1(2*H*)-one (**VIIIs**) and phenyl (3,4,5-trifluorobenzyl)carbamate. The enantiomers were subsequently separated by chiral SFC, Column: (R,R)-Whelk-01 (250 × 30 mm) 5 µ, 80% CO₂/MeOH, Flow rate 60 g/min.

1-(1-(6,7-Difluoro-1-oxo-1,2-dihydroisoquinolin-4-yl)ethyl)-1-methyl-3-(3,4,5-trifluorobenzyl)urea - Enantiomer I (**Compound 481**), LCMS: *m*/*z* found 426.3 [M+H]⁺, RT = 3.90 min (Method A); ¹H NMR (400 MHz, DMSO-d₆) δ 11.57 (bs, 1H), 8.06-8.11 (m, 1H), 7.65-7.70 (m, 1H), 7.12-7.16 (m, 3H), 7.06 (bt, 1H), 5.68-5.73 (m, 1H), 4.36-4.42 (m, 1H), 4.13-4.19 (m, 1H), 2.47 (s, 3H), 1.36 (d, 3H); Chiral analytical SFC: RT = 3.16 min, Column: (R,R)-Whelk-01, (4.6 × 250 mm) 3.5 µm, 80% CO₂/MeOH, Flow = 3.0 g/min.

1-(1-(6,7-Difluoro-1-oxo-1,2-dihydroisoquinofin-4-yl)ethyl)-1-methyl-3-(3,4,5-trifluorobenzyl)urea - Enantiomer II (**Compound 482**), LCMS: *m*/*z* found 426.3 [M+H]⁺, RT = 3.91 min (Method A); ¹H NMR (400 MHz, DMSO-d₆) δ 11.57 (bs, 1H), 8.06-8.11 (m, 1H), 7.65-7.70 (m, 1H), 7.12-7.16 (m, 3H), 7.06 (bt, 1H), 5.68-5.73 (m, 1H), 4.36-4.42 (m, 1H), 4.13-4.19 (m, 1H), 2.47 (s, 3H), 1.36 (d, 3H); Chiral analytical SFC: RT = 4.01 min, Column: (R,R)-Whelk-01, (4.6 × 250 mm) 3.5 µm, 80% CO₂/MeOH, Flow = 3.0 g/min.

### 3-(3-Chloro-4-fluorobenzyl)-1-(1-(6,7-difluoro-1-oxo-1,2-dihydroisoquinolin-4-yl)ethyl)-1-methylurea (Compounds 483 & 484)

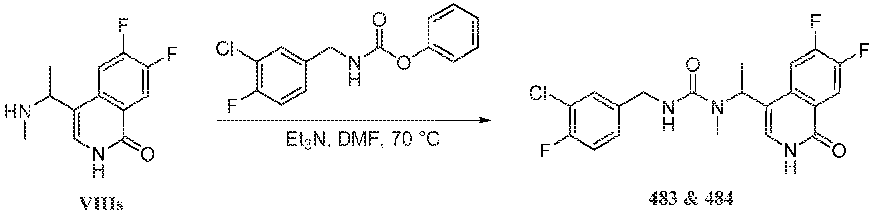

Racemic 3-(3-chloro-4-fluorobenzyl)-1-(1-(6,7-difluoro-1-oxo-1,2-dihydroisoquinolin-4-yl)ethyl)-1-methylurea was synthesized in a similar manner as described above from 6,7-difluoro-4-(1-(methylamino)ethyl)isoquinolin-1(2*H*)-one (**VIIIs**) and phenyl 3-chloro-4-fluorobenzylcarbamate. The enantiomers were subsequently separated by chiral SFC, Column: (R,R)-Whelk-01 (250 × 30 mm) 5 µ, 70% CO₂/MeOH, Flow rate 60 g/min.

3-(3-Chloro-4-fluorobenzyl)-1-(1-(6,7-difluoro-1-oxo-1,2-dihydroisoquinolin-4-yl)ethyl)-1-methylurea - Enantiomer I (**Compound 483**), LCMS: *m*/*z* found 424.2/426.2 [M+H]⁺, RT = 3.99 min (Method A); ¹H NMR (400 MHz, DMSO-d₆): δ 11.50 (bs, 1H), 8.06-8.11 (m, 1H), 7.67-7.73 (m, 1H), 7.39-7.42 (m, 1H), 7.25-7.35 (m, 2H), 7.16 (s, 1H), 7.03 (bt, 1H), 5.69-5.74 (m, 1H), 4.35-4.41 (m, 1H), 4.17-4.22 (m, 1H), 2.46 (s, 3H), 1.35 (d, 3H); Chiral analytical SFC: RT = 2.33 min, Column: (R,R)-Whelk-01, (4.6 × 250 mm) 3.5 µm, 75% CO₂/MeOH, Flow = 3.0 g/min.

3-(3-Chloro-4-fluorobenzyl)-1-(1-(6,7-difluoro-1-oxo-1,2-dihydroisoquinolin-4-yl)ethyl)-1-methylurea - Enantiomer II (**Compound 484**), LCMS: *m*/*z* found 424.2/426.2 [M+H]⁺, RT = 3.99 min (Method A); ¹H NMR (400 MHz, DMSO-d₆): δ 11.50 (bs, 1H), 8.06-8.11 (m, 1H), 7.67-7.73 (m, 1H), 7.39-7.42 (m, 1H), 7.25-7.35 (m, 2H), 7.16 (s, 1H), 7.03 (bt, 1H), 5.69-5.74 (m, 1H), 4.35-4.41 (m, 1H), 4.17-4.22 (m, 1H), 2.46 (s, 3H), 1.35 (d, 3H); Chiral analytical SFC: RT = 3.00 min, Column: (R,R)-Whelk-01, (4.6 × 250 mm) 3.5 µm, 75% CO₂/MeOH, Flow = 3.0 g/min.

### 1-(1-(6,7-Difluoro-1-oxo-1,2-dihydroisoquinolin-4-yl)ethyl)-3-(1H-indol-6-yl)-1-methylurea (Compounds 485 & 486)

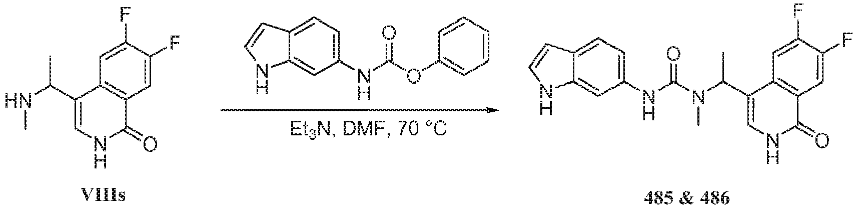

Racemic 1-(1-(6,7-difluoro-1-oxo-1,2-dihydroisoquinolin-4-yl)ethyl)-3-(1*H*-indol-6-yl)-1-methylurea was synthesized in a similar manner as described above from 6,7-difluoro-4-(1-(methylamino)ethyl)isoquinolin-1(2*H*)-one (**VIIIs**) and phenyl 1*H*-indol-6-ylcarbamate. The enantiomers were subsequently separated by chiral SFC, Column: Chiralpak IC (250 × 30 mm) 5 µ, 50% CO₂/MeOH, Flow rate 60 g/min.

1-(1-(6,7-Difluoro-1-oxo-1,2-dihydroisoquinolin-4-yl)ethyl)-3-(1*H*-indol-6-y1)-1-methylurea - Enantiomer I (**Compound 485**), LCMS: *m*/*z* found 397.3 [M+H]⁺, RT = 3.37 min (Method A); ¹H NMR (400 MHz, DMSO-d₆) δ 11.60 (bs, 1H), 10.93 (s, 1H), 8.08-8.13 (m, 2H), 7.83-7.88 (m, 1H), 7.72 (s, 1H), 7.39 (d, 1H), 7.21-7.23 (m, 2H), 7.03-7.06 (m, 1H), 6.33 (bs, 1H), 5.81-5.86 (m, 1H), 2.62 (s, 3H), 1.43 (d, 3H); Chiral analytical SFC: RT = 2.30 min, Column: Chiralpak IC-3, (4.6 × 150 mm) 3 µm, 60% CO₂/MeOH, Flow = 3.0 g/min.

1-(1-(6,7-Difluoro-1-oxo-1,2-dihydroisoquinolin-4-yl)ethyl)-3-(1*H*-indol-6-y1)-1-methylurea - Enantiomer II (**Compound 486**), LCMS: *m*/*z* found 397.3 [M+H]⁺, RT = 3.37 min (Method A); ¹H NMR (400 MHz, DMSO-d₆) δ 11.60 (bs, 1H), 10.93 (s, 1H), 8.08-8.13 (m, 2H), 7.83-7.88 (m, 1H), 7.72 (s, 1H), 7.39 (d, 1H), 7.21-7.23 (m, 2H), 7.03-7.06 (m, 1H), 6.33 (bs, 1H), 5.81-5.86 (m, 1H), 2.62 (s, 3H), 1.43 (d, 3H); Chiral analytical SFC: RT = 4.21 min, Column: Chiralpak IC-3, (4.6 × 150 mm) 3 µm, 60% CO₂/MeOH, Flow = 3.0 g/min.

### 3-(2-Chloropyridin-4-yl)-1-(1-(7,8-difluoro-1-oxo-1,2-dihydroisoquinolin-4-yl)ethyl)-1-methylurea (Compounds 418 & 419)

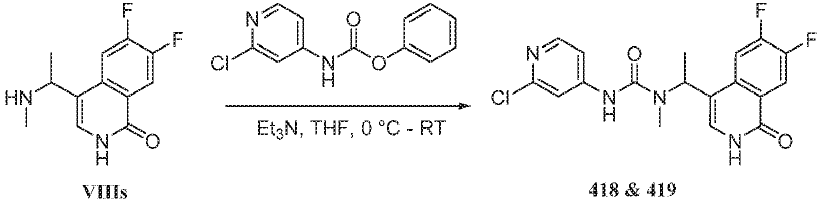

Racemic 3-(2-chloropyridin-4-yl)-1-(1-(6,7-difluoro-1-oxo-1,2-dihydroisoquinolin-4-yl)ethyl)-1-methylurea was synthesized in a similar manner as described above from 6,7-difluoro-4-(1-(methylamino)ethyl)isoquinolin-1(2*H*)-one (**VIIIs**) and phenyl 2-chloropyridin-4-ylcarbamate. The enantiomers were subsequently separated by chiral SFC, Column: Lux Cellulose-2 (250 × 30 mm) 5 µ, 50% CO₂/MeOH, Flow rate 100 g/min.

3-(2-Chloropyridin-4-yl)-1-(1-(6,7-difluoro-1-oxo-1,2-dihydroisoquinolin-4-yl)ethyl)-1-methylurea - Enantiomer I (**Compound 418**), LCMS: *m*/*z* found 393.2/395.2 [M+H]⁺, RT = 3.22 min (Method A); ¹H NMR (400 MHz, DMSO-d₆): δ 11.60 (bs, 1H), 9.11 (bs, 1H), 8.16 (d, 1H), 8.09-8.14 (m, 1H), 7.73-7.74 (m, 1H), 7.63-7.68 (m, 1H), 7.53-7.55 (m, 1H), 7.24 (s, 1H), 5.74-5.79 (m, 1H), 2.63 (s, 3H), 1.44 (d, 3H); Chiral analytical SFC: RT = 1.82 min, Column: Chiralcel OZ-3, (4.6 × 150 mm) 3 µm, 60% CO₂/MeOH, Flow = 3.0 g/min.

3-(2-Chloropyridin-4-yl)-1-(1-(6,7-difluoro-1-oxo-1,2-dihydroisoquinolin-4-yl)ethyl)-1-methylurea - Enantiomer II (**Compound 419**), LCMS: *m*/*z* found 393.2/395.2 [M+H]⁺, RT = 3.22 min (Method A); ¹H NMR (400 MHz, DMSO-d₆): δ 11.60 (bs, 1H), 9.11 (bs, 1H), 8.16 (d, 1H), 8.09-8.14 (m, 1H), 7.73-7.74 (m, 1H), 7.63-7.68 (m, 1H), 7.53-7.55 (m, 1H), 7.24 (s, 1H), 5.74-5.79 (m, 1H), 2.63 (s, 3H), 1.44 (d, 3H); Chiral analytical SFC: RT = 3.06 min, Column: Chiralcel OZ-3, (4.6 × 150 mm) 3 µm, 60% CO₂/MeOH, Flow = 3.0 g/min.

### 1-(1-(6,7-Difluoro-1-oxo-1,2-dihydroisoquinolin-4-yl)ethyl)-3-(3-(difluoromethyl)-4-fluorophenyl)-1-methylurea (Compounds 501 & 502)

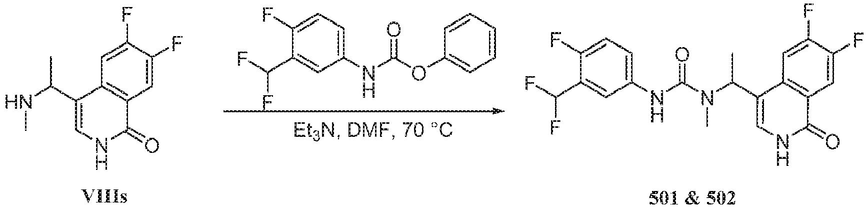

Racemic 1-(1-(6,7-difluoro-1-oxo-1,2-dihydroisoquinolin-4-yl)ethyl)-3-(3-(difluoromethyl)-4-fluorophenyl)-1-methylurea was synthesized in a similar manner as described above from 6,7-difluoro-4-(1-(methylamino)ethyl)isoquinolin-1(2*H*)-one (**VIIIs**) and 3-(difluoromethyl)-4-fluorophenylcarbamate. The enantiomers were subsequently separated by chiral SFC, Column: Chiralpak IC (250 × 30 mm) 5 µ, 80% CO₂/MeOH, Flow rate 90 g/min.

1-(1-(6,7-Difluoro-1-oxo-1,2-dihydroisoquinolin-4-yl)ethyl)-3-(3-(difluoromethyl)-4-fluorophenyl)-1-methylurea - Enantiomer I (**Compound 501**), LCMS: *m*/*z* found 426.2 [M+H]⁺, RT = 4.07 min (Method A); ¹H NMR (400 MHz, DMSO-d₆) δ 11.61 (bs, 1H), 8.53 (bs, 1H), 8.08-8.13 (m, 1H), 7.83-7.86 (m, 1H), 7.69-7.76 (m, 2H), 7.06-7.34 (m, 3H), 5.76-5.82 (m, 1H), 2.61 (s, 3H), 1.43 (d, 3H); Chiral analytical SFC: RT = 2.03 min, Column: Chiralpak IC-3, (4.6 × 150 mm) 3 µm, 65% CO₂/MeOH, Flow = 3.0 g/min.

1-(1-(6,7-Difluoro-1-oxo-1,2-dihydroisoquinolin-4-yl)ethyl)-3-(3-(difluoromethyl)-4-fluorophenyl)-1-methylurea - Enantiomer II (**Compound 502**), LCMS: *m*/*z* found 426.2 [M+H]⁺, RT = 4.07 min (Method A); ¹H NMR (400 MHz, DMSO-d₆) δ 11.61 (bs, 1H), 8.53 (bs, 1H), 8.08-8.13 (m, 1H), 7.83-7.86 (m, 1H), 7.69-7.76 (m, 2H), 7.06-7.34 (m, 3H), 5.76-5.82 (m, 1H), 2.61 (s, 3H), 1.43 (d, 3H); Chiral analytical SFC: RT = 2.86 min, Column: Chiralpak IC-3, (4.6 × 150 mm) 3 µm, 65% CO₂/MeOH, Flow = 3.0 g/min.

### 1-(1-(6,7-Difluoro-1-oxo-1,2-dihydroisoquinolin-4-yl)ethyl)-1-ethyl-3-(4-fluorophenyl)urea (Compounds 402 & 403)

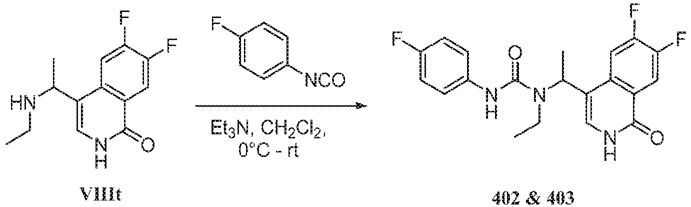

Racemic 1-(1-(6,7-difluoro-1-oxo-1,2-dihydroisoquinolin-4-yl)ethyl)-1-ethyl-3-(4-fluorophenyl)urea was synthesized in a similar manner as described above from 6,7-difluoro-4-(1-(ethylamino)ethyl)isoquinolin-1(2*H*)-one (**VIIIt**) and 4-fluorophenyl isocyanate. The enantiomers were subsequently separated by chiral SFC, Column: Chiralpak IC (250 × 30 mm) 5 µ, 85% CO₂/MeOH, Flow rate 100 g/min.

1-(1-(6,7-Difluoro-1-oxo-1,2-dihydroisoquinofin-4-yl)ethyl)-1-ethyl-3-(4-fluorophenyl)urea - Enantiomer I (**Compound 402**), LCMS: *m*/*z* found 390.3 [M+H]⁺, RT = 3.86 min (Method A); ¹H NMR (400 MHz, DMSO-d₆): δ 11.62 (bs, 1H), 8.24 (bs, 1H), 8.08-8.11 (m, 1H), 7.76-7.81 (m, 1H), 7.50-7.54 (m, 2H), 7.27 (s, 1H), 7.09-7.14 (m, 2H), 5.79-5.83 (m, 1H), 3.20-3.30 (m, 1H), 3.07-3.15 (m, 1H), 1.44 (d, 3H), 0.67 (t, 3H); Chiral analytical SFC: RT = 4.47 min, Column: Chiralpak IC, (4.6 × 150 mm) 5 µm, 80% CO₂/MeOH, Flow = 3.0 g/min.

1-(1-(6,7-Difluoro-1-oxo-1,2-dihydroisoquinofin-4-yl)ethyl)-1-ethyl-3-(4-fluorophenyl)urea - Enantiomer II (**Compound 403**), LCMS: *m*/*z* found 390.3 [M+H]⁺, RT = 3.86 min (Method A); ¹H NMR (400 MHz, DMSO-d₆): δ 11.62 (bs, 1H), 8.24 (bs, 1H), 8.08-8.11 (m, 1H), 7.76-7.81 (m, 1H), 7.50-7.54 (m, 2H), 7.27 (s, 1H), 7.09-7.14 (m, 2H), 5.79-5.83 (m, 1H), 3.20-3.30 (m, 1H), 3.07-3.15 (m, 1H), 1.44 (d, 3H), 0.67 (t, 3H); Chiral analytical SFC: RT = 6.72 min, Column: Chiralpak IC, (4.6 × 150 mm) 5 µm, 80% CO₂/MeOH, Flow = 3.0 g/min.

### 3-(3-Chloro-4-fluorophenyl)-1-(1-(6,7-difluoro-1-oxo-1,2-dihydroisoquinolin-4-yl)ethyl)-1-ethylurea (Compounds 406 & 407)

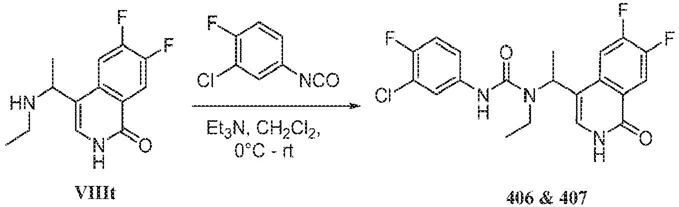

Racemic 3-(3-chloro-4-fluorophenyl)-1-(1-(6,7-difluoro-1-oxo-1,2-dihydroisoquinolin-4-yl)ethyl)-1-ethylurea was synthesized in a similar manner as described above from 6,7-difluoro-4-(1-(ethylamino)ethyl)isoquinolin-1(2*H*)-one (**VIIIt**) and 2-chloro-1-fluoro-4-isocyanatobenzene. The enantiomers were subsequently separated by chiral SFC, Column: Chiralpak IC (250 × 30 mm) 5 µ, 80% CO₂/MeOH, Flow rate 90 g/min.

3-(3-Chloro-4-fluorophenyl)-1-(1-(6,7-difluoro-1-oxo-1,2-dihydroisoquinolin-4-yl)ethyl)-1-ethylurea- Enantiomer I (**Compound 406**), LCMS: *m*/*z* found 424.2/426.2 [M+H]⁺, RT = 4.53 min (Method A); ¹H NMR (400 MHz, DMSO-d₆): δ 11.68 (bs, 1H), 8.38 (bs, 1H), 8.08-8.14 (m, 1H), 7.80-7.83 (m, 1H), 7.71-7.77 (m, 1H), 7.50-7.55 (m, 1H), 7.28-7.35 (m, 2H), 5.78-5.81 (m, 1H), 3.21-3.31 (m, 1H), 3.06-3.18 (m, 1H), 1.45 (d, 3H), 0.67 (t, 3H); Chiral analytical SFC: RT = 2.74 min, Column: Chiralpak IC, (4.6 × 150 mm) 5 µm, 70% CO₂/MeOH, Flow = 3.0 g/min.

3-(3-Chloro-4-fluorophenyl)-1-(1-(6,7-difluoro-1-oxo-1,2-dihydroisoquinolin-4-yl)ethyl)-1-ethylurea- Enantiomer II (**Compound 407**), LCMS: *m*/*z* found 424.2/426.2 [M+H]⁺, RT = 4.53 min (Method A); ¹H NMR (400 MHz, DMSO-d₆): δ 11.68 (bs, 1H), 8.38 (bs, 1H), 8.08-8.14 (m, 1H), 7.80-7.83 (m, 1H), 7.71-7.77 (m, 1H), 7.50-7.55 (m, 1H), 7.28-7.35 (m, 2H), 5.78-5.81 (m, 1H), 3.21-3.31 (m, 1H), 3.06-3.18 (m, 1H), 1.45 (d, 3H), 0.67 (t, 3H); Chiral analytical SFC: RT = 4.33 min, Column: Chiralpak IC, (4.6 × 150 mm) 5 µm, 70% CO₂/MeOH, Flow = 3.0 g/min.

### 1-(1-(6,7-Difluoro-1-oxo-1,2-dihydroisoquinolin-4-yl)ethyl)-1-ethyl-3-phenylurea (Compounds 404 & 405)

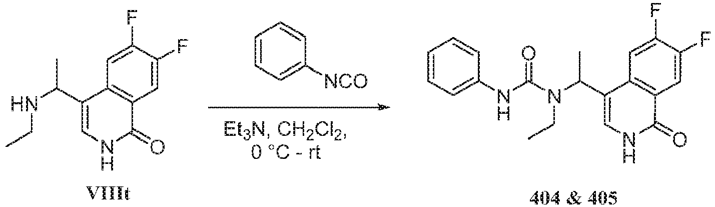

Racemic 1-(1-(6,7-difluoro-1-oxo-1,2-dihydroisoquinolin-4-yl)ethyl)-1-ethyl-3-phenylurea was synthesized in a similar manner as described above from 6,7-difluoro-4-(1-(ethylamino)ethyl)isoquinolin-1(2*H*)-one (**VIIIt**) and phenyl isocyanate. The enantiomers were subsequently separated by chiral SFC, Column: (R, R) Whelk-01 (250 × 30 mm) 5 µ, 60% CO₂/MeOH, Flow rate 100 g/min.

1-(1-(6,7-Difluoro-1-oxo-1,2-dihydroisoquinolin-4-yl)ethyl)-1-ethyl-3-phenylurea - Enantiomer I (**Compound 404**), LCMS: *m*/*z* found 372.3 [M+H]⁺, RT = 3.74 min (Method A); ¹H NMR (400 MHz, DMSO-d₆): δ 11.61 (bs, 1H), 8.18 (bs, 1H), 8.08-8.13 (m, 1H), 7.78-7.83 (m, 1H), 7.51-7.54 (m, 2H), 7.25-7.29 (m, 3H), 6.96-7.00 (m, 1H), 5.80-5.84 (m, 1H), 3.23-3.30 (m, 1H), 3.07-3.15 (m, 1H), 1.44 (d, 3H), 0.68 (t, 3H); Chiral analytical SFC: RT = 2.48 min, Column: (R, R) Whelk-01, (4.6 × 150 mm) 5 µm, 70% CO₂/MeOH, Flow = 3.0 g/min.

1-(1-(6,7-Difluoro-1-oxo-1,2-dihydroisoquinolin-4-yl)ethyl)-1-ethyl-3-phenylurea - Enantiomer II (**Compound 405**), LCMS: *m*/*z* found 372.3 [M+H]⁺, RT = 3.74 min (Method A); ¹H NMR (400 MHz, DMSO-d₆): δ 11.61 (bs, 1H), 8.18 (bs, 1H), 8.08-8.13 (m, 1H), 7.78-7.83 (m, 1H), 7.51-7.54 (m, 2H), 7.25-7.29 (m, 3H), 6.96-7.00 (m, 1H), 5.80-5.84 (m, 1H), 3.23-3.30 (m, 1H), 3.07-3.15 (m, 1H), 1.44 (d, 3H), 0.68 (t, 3H); Chiral analytical SFC: RT = 3.44 min, Column: (R, R) Whelk-01, (4.6 × 150 mm) 5 µm, 70% CO₂/MeOH, Flow = 3.0 g/min.

### 1-(1-(6,7-Difluoro-1-oxo-1,2-dihydroisoquinolin-4-yl)ethyl)-3-(3,4-difluorophenyl)-1-ethylurea (Compounds 422 & 423)

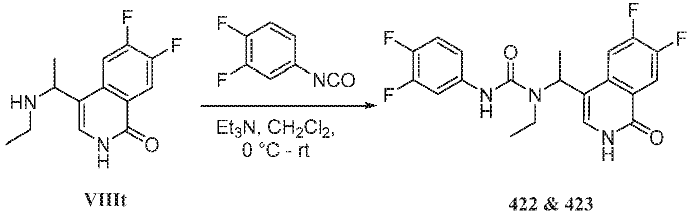

Racemic 1-(1-(6,7-difluoro-1-oxo-1,2-dihydroisoquinolin-4-yl)ethyl)-3-(3,4-difluorophenyl)-1-ethylurea was synthesized in a similar manner as described above from 6,7-difluoro-4-(1-(ethylamino)ethyl)isoquinolin-1(2*H*)-one (**VIIIt**) and 3,4-difluorophenyl isocyanate. The enantiomers were subsequently separated by chiral SFC, Column: Chiralpak IC (250 × 30 mm) 5 µ, 80% CO₂/MeOH, Flow rate 100 g/min.

1-(1-(6,7-Difluoro-1-oxo-1,2-dihydroisoquinolin-4-yl)ethyl)-3-(3,4-difluorophenyl)-1-ethylurea - Enantiomer I (**Compound 422**), LCMS: *m*/*z* found 408.2 [M+H]⁺, RT = 4.26 min (Method A); ¹H NMR (400 MHz, DMSO-d₆): δ 11.68 (bs, 1H), 8.39 (bs, 1H), 8.08-8.14 (m, 1H), 7.67-7.77 (m, 2H), 7.28-7.37 (m, 3H), 5.77-5.83 (m, 1H), 3.20-3.27 (m, 1H), 3.08-3.14 (m, 1H), 1.44 (d, 3H), 0.67 (t, 3H); Chiral analytical SFC: RT = 2.07 min, Column: Chiralpak IC-3, (4.6 × 150 mm) 3 µm, 70% CO₂/MeOH, Flow = 3.0 g/min.

1-(1-(6,7-Difluoro-1-oxo-1,2-dihydroisoquinolin-4-yl)ethyl)-3-(3,4-difluorophenyl)-1-ethylurea - Enantiomer II (**Compound 423**), LCMS: *m*/*z* found 408.2 [M+H]⁺, RT = 4.26 min (Method A); ¹H NMR (400 MHz, DMSO-d₆): δ 11.68 (bs, 1H), 8.39 (bs, 1H), 8.08-8.14 (m, 1H), 7.67-7.77 (m, 2H), 7.28-7.37 (m, 3H), 5.77-5.83 (m, 1H), 3.20-3.27 (m, 1H), 3.08-3.14 (m, 1H), 1.44 (d, 3H), 0.67 (t, 3H); Chiral analytical SFC: RT = 3.02 min, Column: Chiralpak IC-3, (4.6 × 150 mm) 3 µm, 70% CO₂/MeOH, Flow = 3.0 g/min.

### 6,7-Difluoro-4-(1-(isobutylamino)ethyl)isoquinolin-1(2H)-one (VIIIu)

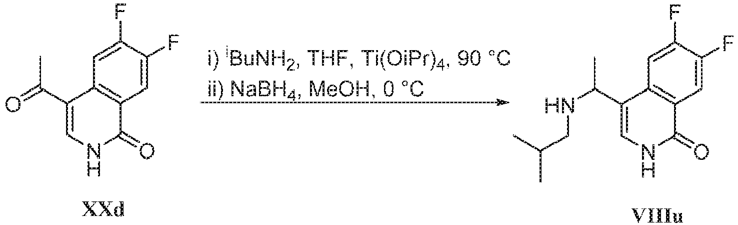

To a solution of 0.4 g (1.8 mmol, 1.0 eq.) of 4-acetyl-6,7-difluoroisoquinolin-1(2*H*)-one (**XXd**) in 4 mL of THF under a nitrogen atmosphere was added 0.34 mL (3.58 mmol, 2.0 eq.) of isobutylamine followed by 4 mL of titanium isopropoxide and the mixture was heated at 90 °C for 16 h. The mixture was allowed to room temperature and further cooled to 0 °C. Following dilution with 3 mL of methanol, 0.20 g (5.4 mmol, 3.0 eq.) of sodium borohydride was added portion-wise over approximately 10 min and stirring was continued for 4 h. The reaction mixture was diluted with 40 mL of water and extracted with 50 mL of ethyl acetate. The combined organic extracts were washed with 50 mL of water, 50 mL of brine, dried (Na₂SO₄) and the solvent was removed *in vacuo* to provide 420 mg of 6,7-difluoro-4-(1-(isobutylamino)ethyl)isoquinolin-1(2*H*)-one (**VIIIu**)**.** LCMS: *m*/*z* found 281.10 [M+H]⁺.

### 3-(3-Chloro-4-fluorophenyl)-1-(1-(6,7-difluoro-1-oxo-1,2-dihydroisoquinolin-4-yl)ethyl)-1-isobutylurea (Compounds 280 & 281)

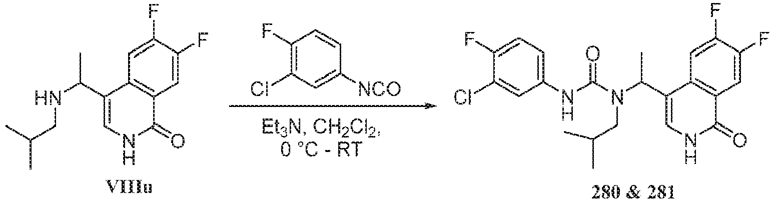

Racemic 3-(3-chloro-4-fluorophenyl)-1-(1-(6,7-difluoro-1-oxo-1,2-dihydroisoquinolin-4-yl)ethyl)-1-isobutylurea was synthesized in a similar manner as described above from 6,7-difluoro-4-(1-(isobutylamino)ethyl)isoquinolin-1(2*H*)-one (**VIIIu**) and 2-chloro-1-fluoro-4-isocyanatobenzene. The enantiomers were subsequently separated by chiral SFC, Column: Chiralpak IC (250 × 30 mm) 5 µ, 90% CO₂/MeOH, Flow rate 100 g/min.

3-(3-Chloro-4-fluorophenyl)-1-(1-(6,7-difluoro-1-oxo-1,2-dihydroisoquinolin-4-yl)ethyl)-1-isobutylurea - Enantiomer I (**Compound 280**), LCMS: *m*/*z* found 452.1/454.1 [M+H]⁺, RT = 5.15 min (Method A); ¹H NMR (400 MHz, DMSO-d₆): δ 11.68 (bs, 1H), 8.43 (s, 1H), 8.09-8.14 (m, 1H), 7.84-7.89 (m, 1H), 7.75-7.78 (m, 1H), 7.44-7.49 (m, 1H), 7.34 (t, 1H), 7.28 (s, 1H), 5.78-5.81 (m, 1H), 3.02-3.08 (m, 1H), 2.82-2.88 (m, 1H), 1.46 (d, 3H), 1.33-1.40 (m, 1H), 0.63 (d, 3H), 0.42 (d, 3H); Chiral analytical SFC: RT = 6.33 min, Column: Chiralpak IC, (4.6 × 150 mm) 5 µm, 85% CO₂/MeOH, Flow = 3.0 g/min.

3-(3-Chloro-4-fluorophenyl)-1-(1-(6,7-difluoro-1-oxo-1,2-dihydroisoquinolin-4-yl)ethyl)-1-isobutylurea - Enantiomer II (**Compound 281**), LCMS: *m*/*z* found 452.1/454.1 [M+H]⁺, RT = 5.19 min (Method A); ¹H NMR (400 MHz, DMSO-d₆): δ 11.68 (bs, 1H), 8.43 (s, 1H), 8.09-8.14 (m, 1H), 7.84-7.89 (m, 1H), 7.75-7.78 (m, 1H), 7.44-7.49 (m, 1H), 7.34 (t, 1H), 7.28 (s, 1H), 5.78-5.81 (m, 1H), 3.02-3.08 (m, 1H), 2.82-2.88 (m, 1H), 1.46 (d, 3H), 1.33-1.40 (m, 1H), 0.63 (d, 3H), 0.42 (d, 3H); Chiral analytical SFC: RT = 9.10 min, Column: Chiralpak IC, (4.6 × 150 mm) 5 µm, 85% CO₂/MeOH, Flow = 3.0 g/min.

### 1-(1-(6,7-Difluoro-1-oxo-1,2-dihydroisoquinolin-4-yl)ethyl)-3-(4-fluorophenyl)-1-isobutylurea (Compounds 287 & 288)

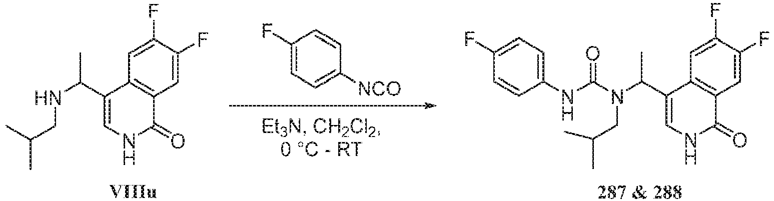

Racemic 1-(1-(6,7-difluoro-1-oxo-1,2-dihydroisoquinolin-4-yl)ethyl)-3-(4-fluorophenyl)-1-isobutylurea was synthesized in a similar manner as described above from 6,7-difluoro-4-(1-(isobutylamino)ethyl)isoquinolin-1(2*H*)-one (**VIIIu**) and 4-fluorophenyl isocyanate. The enantiomers were subsequently separated by chiral SFC, Column: Lux Cellulose-2 (250 × 30 mm) 5 µ, 85% CO₂/MeOH, Flow rate 70 g/min.

1-(1-(6,7-Difluoro-1-oxo-1,2-dihydroisoquinolin-4-yl)ethyl)-3-(4-fluorophenyl)-1-isobutylurea - Enantiomer I **(Compound 287**), LCMS: *m*/*z* found 418.2 [M+H]⁺, RT = 4.59 min (Method A); ¹H NMR (400 MHz, DMSO-d₆): δ 11.62 (bs, 1H), 8.27 (s, 1H), 8.08-8.14 (m, 1H), 7.89-7.94 (m, 1H), 7.46-7.50 (m, 2H), 7.27 (s, 1H), 7.09-7.14 (m, 2H), 5.79-5.83 (m, 1H), 3.03-3.09 (m, 1H), 2.80-2.86 (m, 1H), 1.45 (d, 3H), 1.33-1.39 (m, 1H), 0.64 (d, 3H), 0.41 (d, 3H); Chiral analytical SFC: RT = 4.36 min, Column: Lux Cellulose-2, (4.6 × 150 mm) 5 µm, 85% CO₂/MeOH, Flow = 3.0 g/min.

1-(1-(6,7-Difluoro-1-oxo-1,2-dihydroisoquinolin-4-yl)ethyl)-3-(4-fluorophenyl)-1-isobutylurea - Enantiomer II **(Compound 288**), LCMS: *m*/*z* found 418.2 [M+H]⁺, RT = 4.59 min (Method A); ¹H NMR (400 MHz, DMSO-d₆): δ 11.62 (bs, 1H), 8.27 (s, 1H), 8.08-8.14 (m, 1H), 7.89-7.94 (m, 1H), 7.46-7.50 (m, 2H), 7.27 (s, 1H), 7.09-7.14 (m, 2H), 5.79-5.83 (m, 1H), 3.03-3.09 (m, 1H), 2.80-2.86 (m, 1H), 1.45 (d, 3H), 1.33-1.39 (m, 1H), 0.64 (d, 3H), 0.41 (d, 3H); Chiral analytical SFC: RT = 7.60 min, Column: Lux Cellulose-2, (4.6 × 150 mm) 5 µm, 85% CO₂/MeOH, Flow = 3.0 g/min.

### 6,7-Difluoro-4-(1-((3-hydroxypropyl)amino)ethyl)isoquinolin-1(2H)-one (VIIIv)

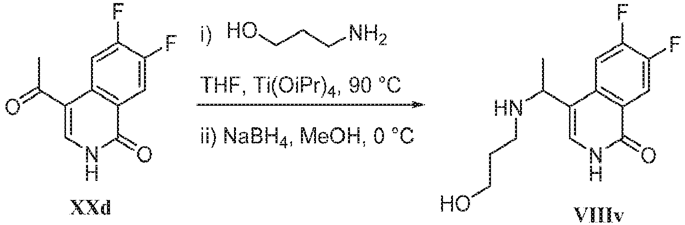

To a solution of 0.8 mg (3.6 mmol, 1.0 eq.) of 4-acetyl-6,7-difluoroisoquinolin-1(2*H*)-one (**XXd**) in 8 mL THF under a nitrogen atmosphere was added 0.81 g (10.8 mmol, 3.0 eq.) of 3-aminopropan-1-ol followed by 8 mL of titanium isopropoxide and the mixture was heated at 90 °C for 16 h. The mixture was allowed to cool to room temperature and further cooled to 0 °C. Following dilution with 4 mL of methanol, 0.40 g (10.8 mmol, 3.0 eq.) of sodium borohydride was added portion-wise over approximately 10 min and stirring was continued for 4 h. The reaction mixture was diluted with 100 mL of water and filtered through CELITE^{®}. The pad was washed with 50 mL of ethyl acetate and the filtrate was extracted with 3 × 100 mL of ethyl acetate. The combined organic extracts were washed with 50 mL of water, 50 mL of brine, dried (Na₂SO₄), filtered and the solvent was removed *in vacuo* to provide 1.0 g of 6,7-difluoro-4-(1-((3-hydroxypropyl)amino)ethyl)isoquinolin-1(2*H*)-one (**VIIIv**). LCMS: *m*/*z* found 283.0 [M+H]⁺.

### 3-(3-Chloro-4-fluorophenyl)-1-(1-(6,7-difluoro-1-oxo-1,2-dihydroisoquinolin-4-yl)ethyl)-1-(3-hydroxypropyl)urea (Compounds 289 & 290)

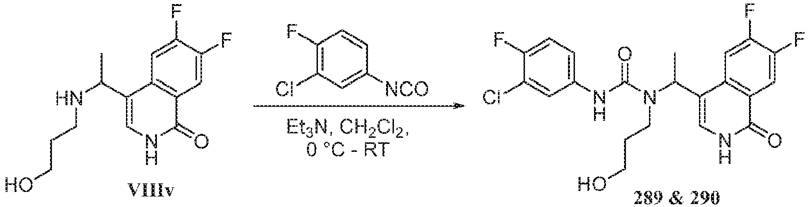

To a solution of 0.5 g of 6,7-difluoro-4-(1-((3-hydroxypropyl)amino)ethyl)isoquinolin-1(2*H*)-one (**VIIIv**) in 5 mL of methylene chloride was added 0.54 mg (5.31 mmol) of triethylamine followed by 0.18 g (1.06 mmol) of 2-chloro-1-fluoro-4-isocyanatobenzene and the mixture was stirred at room temperature for 4 h. The resulting precipitate was collected by filtration, washed with 10 mL of water and dried under vacuum. The solids were then triturated with 5 mL of diethyl ether and 20 mL and *n-*pentane and dried under high vacuum to provide 0.4 g (0.88 mmol) of racemic 3-(3-chloro-4-fluorophenyl)-1-(1-(6,7-difluoro-1-oxo-1,2-dihydroisoquinolin-4-yl)ethyl)-1-(3-hydroxypropyl)urea. The enantiomers were subsequently separated by chiral SFC, Column: (R,R) Whelk-001 (250 × 30 mm) 5 µ, 60% CO₂/MeOH, Flow rate 90 g/min.

3-(3-Chloro-4-fluorophenyl)-1-(1-(6,7-difluoro-1-oxo-1,2-dihydroisoquinolin-4-yl)ethyl)-1-(3-hydroxypropyl)urea- Enantiomer I (**Compound 289**), LCMS: *m*/*z* found 454.1/456.1 [M+H]⁺, RT = 4.37 min (Method A); ¹H NMR (400 MHz, DMSO-d₆): 11.63 (bs, 1H), 8.71 (s, 1H), 8.09-8.14 (m, 1H), 7.75-7.78 (m, 1H), 7.65-7.70 (m, 1H), 7.41-7.45 (m, 1H), 7.34 (t, 1H), 7.26 (s, 1H), 5.77-5.82 (m, 1H), 4.99 (t, 1H), 3.12-3.21 (m, 4H), 1.45 (d, 3H), 1.13-1.24 (m, 2H); Chiral analytical SFC: RT = 6.00 min, Column: (R,R) Whelk-001, (4.6 × 150 mm) 5 µm, 65% CO₂/MeOH, Flow = 3.0 g/min.

3-(3-Chloro-4-fluorophenyl)-1-(1-(6,7-difluoro-1-oxo-1,2-dihydroisoquinolin-4-yl)ethyl)-1-(3-hydroxypropyl)urea- Enantiomer II (**Compound 290**), LCMS: *m*/*z* found 454.1/456.1 [M+H]⁺, RT = 4.37 min (Method A); ¹H NMR (400 MHz, DMSO-d₆): 11.63 (bs, 1H), 8.71 (s, 1H), 8.09-8.14 (m, 1H), 7.75-7.78 (m, 1H), 7.65-7.70 (m, 1H), 7.41-7.45 (m, 1H), 7.34 (t, 1H), 7.26 (s, 1H), 5.77-5.82 (m, 1H), 4.99 (t, 1H), 3.12-3.21 (m, 4H), 1.45 (d, 3H), 1.13-1.24 (m, 2H); Chiral analytical SFC: RT = 8.82 min, Column: (R,R) Whelk-001, (4.6 × 150 mm) 5 µm, 65% CO₂/MeOH, Flow = 3.0 g/min.

### 1-(1-(6,7-Difluoro-1-oxo-1,2-dihydroisoquinolin-4-yl)ethyl)-3-(4-fluorophenyl)-1-(3-hydroxypropyl) urea (Compounds 291 & 292)

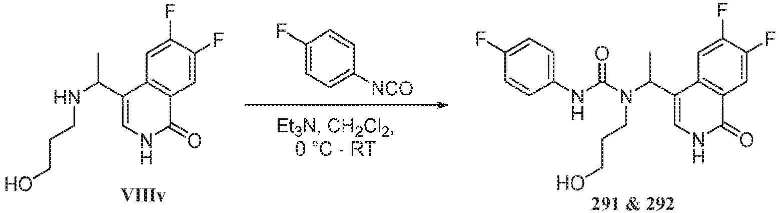

Racemic 1-(1-(6,7-difluoro-1-oxo-1,2-dihydroisoquinolin-4-yl)ethyl)-3-(4-fluorophenyl)-1-(3-hydroxypropyl) urea was synthesized in a similar manner as described above from 6,7-difluoro-4-(1-((3-hydroxypropyl)amino)ethyl)isoquinolin-1(2*H*)-one (**VIIIv**) and 4-fluorophenyl isocyanate. The enantiomers were subsequently separated by chiral SFC, Column: Lux Celulose-2 (250 × 30 mm) 5 µ, 80% CO₂/MeOH, Flow rate 60 g/min.

1-(1-(6,7-Difluoro-1-oxo-1,2-dihydroisoquinolin-4-yl)ethyl)-3-(4-fluorophenyl)-1-(3-hydroxypropyl) urea - Enantiomer I **(Compound 291**), LCMS: *m*/*z* found 420.1 [M+H]⁺, RT = 3.71 min (Method A); ¹H NMR (400 MHz, DMSO-d₆): δ 11.62 (bs, 1H), 8.55 (s, 1H), 8.08-8.14 (m, 1H), 7.69-7.74 (m, 1H), 7.46-7.51 (m, 2H), 7.25 (s, 1H), 7.09-7.15 (m, 2H), 5.78-5.84 (m, 1H), 4.95 (t, 1H), 3.11-3.22 (m, 4H), 1.45 (d, 3H), 1.14-1.24 (m, 2H); Chiral analytical SFC: RT = 7.72 min, Column: (R,R) Whelk-001, (4.6 × 150 mm) 5 µm, 80% CO₂/MeOH, Flow = 3.0 g/min.

1-(1-(6,7-Difluoro-1-oxo-1,2-dihydroisoquinolin-4-yl)ethyl)-3-(4-fluorophenyl)-1-(3-hydroxypropyl) urea - Enantiomer II **(Compound 292**), LCMS: *m*/*z* found 420.1 [M+H]⁺, RT = 3.71 min (Method A); ¹H NMR (400 MHz, DMSO-d₆): δ 11.62 (bs, 1H), 8.55 (s, 1H), 8.08-8.14 (m, 1H), 7.69-7.74 (m, 1H), 7.46-7.51 (m, 2H), 7.25 (s, 1H), 7.09-7.15 (m, 2H), 5.78-5.84 (m, 1H), 4.95 (t, 1H), 3.11-3.22 (m, 4H), 1.45 (d, 3H), 1.14-1.24 (m, 2H); Chiral analytical SFC: RT = 11.79 min, Column: (R,R) Whelk-001, (4.6 × 150 mm) 5 µm, 80% CO₂/MeOH, Flow = 3.0 g/min.

### 2-((1-(6,7-Difluoro-1-oxo-1,2-dihydroisoquinolin-4-yl)ethyl)amino)ethane-1-sulfonamide (VIIIw)

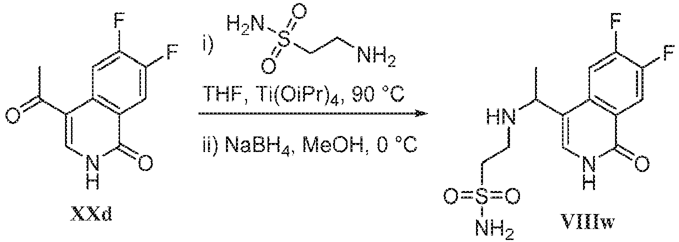

To a solution of 0.5 g (2.2 mmol, 1.0 eq.) of 4-acetyl-6,7-difluoroisoquinolin-1(2*H*)-one (**XXd**) in 5 mL of THF under a nitrogen atmosphere was added 0.42 mg (3.4 mmol, 1.5 eq.) of 2-aminoethanesulfonamide followed by 5 mL of titanium isopropoxide and the mixture was heated at 90 °C for 16 h. The mixture was allowed to cool to room temperature and further cooled to 0 °C. Following dilution with 5 mL of methanol, 0.17 g (4.48 mmol, 2.0 eq.) of sodium borohydride was added portion-wise over approximately 10 min and stirring was continued for 2 h. The reaction mixture was diluted with 5 mL of brine and 100 mL of 10% methanol in methylene chloride and the mixture was filtered through CELITE^{®}. The pad was washed with 40 mL of 10% methanol in methylene chloride and the layers were separated. The organic phase was dried (Na₂SO₄), filtered and the solvent was removed *in vacuo.* The residue was purified by MPLC (SiO₂, eluting with a linear gradient 10-20% of methanol/ methylene chloride) to provide 150 mg of 2-((1-(6,7-difluoro-1-oxo-1,2-dihydroisoquinolin-4-yl)ethyl)amino)ethane-1-sulfonamide (**VIIIw**). LCMS: *m*/*z* found 332.4 [M+H]⁺.

### 1-(1-Chloro-6,7-difluoroisoquinolin-4-yl)ethanone

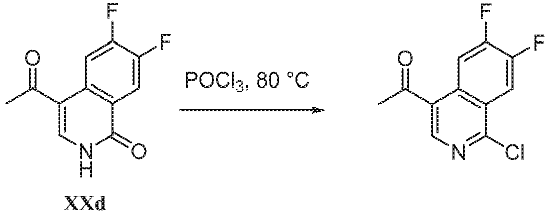

A solution of 1.0 g (4.48 mmol, 1.0 eq) of 4-acetyl-6,7-difluoroisoquinolin-1(2*H*)-one (**XXd**) in 10 mL of phosphorus oxychloride was heated at 80 °C for 4 h. The mixture was allowed to cool to room temperature and poured into 200 mL ice-cold water. The precipitated solid was collected by filtration and washed with 20 ml of chilled water followed by 20 ml of diethyl ether and then dried under high vacuum to provide 0.8 g (3.32 mmol, 86%) of 1-(1-chloro-6,7-difluoroisoquinolin-4-yl)ethanone. LCMS: *m*/*z* found 242.0/244.0 [M+H]⁺.

### 1-(6,7-Difluoro-1-((1-methyl-1H-1,2,4-triazol-3-yl)methoxy)isoquinolin-4-yl)ethanone (Vv)

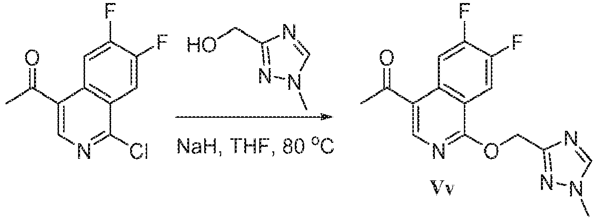

To a solution of 0.56 g (4.97 mmol, 1.5 eq.) of (1-methyl-1*H*-1,2,4-triazol-3-yl)methanol in 10 mL of THF at 0 °C under a nitrogen atmosphere was added 0.20 g (4.97 mmol, 1.5 eq.) of a 60% dispersion of sodium hydride in mineral oil. The mixture was stirred at 0 °C for 20 min and a solution of 0.8 g (3.31 mmol, 1.0 eq) 1-(1-chloro-6,7-difluoroisoquinolin-4-yl)ethanone in 2 mL of THF in was added. The mixture was allowed to warm to room temperature and stirred for 2 h. The reaction was then quenched with 20 mL of ice-cold water and extracted with 3 × 50 mL of ethyl acetate. The combined organic extracts were washed with 40 mL of brine, dried (Na₂SO₄), filtered ant the solvent was removed *in vacuo.* The residue was purified by chromatography (REVELERIS^{®} SiO₂ column, eluting with a linear gradient of 0-6% methanol /methylene chloride) to provide 0.32 g (1.01 mmol, 30%) of 1-(6,7-difluoro-1-((1-methyl-1*H*-1,2,4-triazol-3-yl)methoxy)isoquinolin-4-yl)ethanone (**Vv**). LCMS: *m*/*z* found 319.2 [M+H]⁺, RT = 2.13 min; ¹H NMR (400 MHz, CDCl₃) δ 8.94 (s, 1H), 8-87-8.93 (m, 1H), 8.48 (s, 1H), 8.07-8.12 (m, 1H), 5.66 (s, 2H), 3.87 (s, 3H), 2.71 (s, 3H).

### 1-(6,7-Difluoro-1-((1-methyl-1H-1,2,4-triazol-3-yl)methoxy)isoquinolin-4-yl)-N-methylethanamine (VIaz)

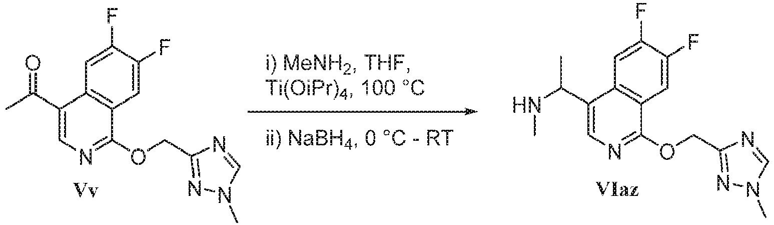

To a solution of 0.1 g (0.31 mmol, 1.0 eq.) of 1-(6,7-difluoro-1-((1-methyl-1*H*-1,2,4-triazol-3-yl)methoxy)isoquinolin-4-yl)ethanone (**Vv**) in 2 mL of THF in a sealed tube under a nitrogen atmosphere was added 1.0 mL (2.0 mmol, 6.5 eq.) of a 2 M solution of methylamine in THF followed by 1 mL of titanium isopropoxide and the mixture was heated at 100 °C for 2 h. The mixture was allowed to cool to room temperature and then further cooled to 0 °C. The mixture was diluted with 2 mL of methanol and 0.024 g (0.63 mmol, 2.0 eq.) of sodium borohydride was added. The mixture was then allowed to warm to room temperature and stirred for 2 h. The reaction was quenched by the addition of 10 mL of water and filtered through CELITE^{®}. The pad was washed with 10 mL of 20% methanol in methylene chloride and the filtrate was extracted with 3 × 50 mL of 20% methanol in methylene chloride. The combined organic extracts were dried (Na₂SO₄), filtered and the solvent was removed *in vacuo* to provide 0.15 g of crude 1-(6,7-difluoro-1-((1-methyl-1*H*-1,2,4-triazol-3-yl)methoxy)isoquinolin-4-yl)-N-methylethanamine (**VIaz**). LCMS: *m*/*z* found 334.1 [M+H]⁺, RT = 1.23 min.

### 3-(3-Chloro-4-fluorophenyl)-1-(1-(6,7-difluoro-1-((1-methyl-1H-1,2,4-triazol-3-yl)methoxy)isoquinolin-4-yl)ethyl)-1-methylurea (Compounds 346 & 347)

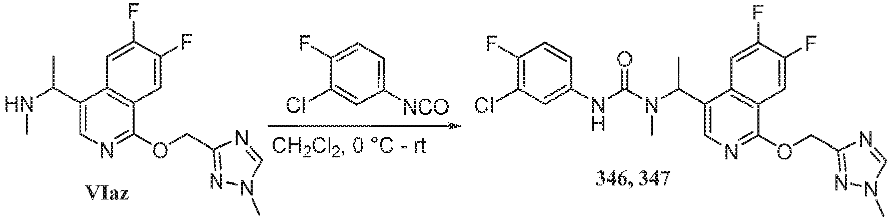

Racemic 3-(3-chloro-4-fluorophenyl)-1-(1-(6,7-difluoro-1-((1-methyl-1*H*-1,2,4-triazol-3-yl)methoxy) isoquinolin-4-yl)ethyl)-1-methylurea was synthesized in a similar manner as described above from 1-(6,7-difluoro-1-((1-methyl-1*H*-1,2,4-triazol-3-yl)methoxy)isoquinolin-4-yl)-N-methylethanamine (**VIaz**) and 2-chloro-1-fluoro-4-isocyanatobenzene. The enantiomers were subsequently separated by SFC, Column: Chiralpak IC (30 × 250 mm) 5 µ, 65% CO₂:MeOH, flow rate 100 g/min.

3-(3-Chloro-4-fluorophenyl)-1-(1-(6,7-difluoro-1-((1-methyl-1*H*-1,2,4-triazol-3-yl)methoxy)isoquinolin-4-yl)ethyl)-1-methylurea: Enantiomer I (**Compound 346**). LCMS: *m*/*z* found 505.2/507.2 [M+H]⁺, RT = 5.05 min (Method: A); ¹H NMR (400 MHz, DMSO-d₆) δ 8.48-8.50 (m, 2H), 8.16 (s, 1H), 8.00-8.05 (m, 2H), 7.81-7.83 (m, 1H), 7.48-7.52 (m, 1H), 7.33 (t, 1H), 6.04-6.09 (m, 1H), 5.52-5.59 (m, 2H), 3.87 (s, 3H), 2.59 (s, 3H), 1.58 (d, 3H); Chiral analytical SFC: RT = 3.45 min, Chiralpak IC-3 (150 × 4.6 mm), 3 µ, 60% CO₂:MeOH, Flow rate = 3.0 mL/min.

3-(3-Chloro-4-fluorophenyl)-1-(1-(6,7-difluoro-1-((1-methyl-1*H*-1,2,4-triazol-3-yl)methoxy)isoquinolin-4-yl)ethyl)-1-methylurea: Enantiomer II (**Compound 347**). LCMS: *m*/*z* found 505.2/507.2 [M+H]⁺, RT = 5.05 min (Method: A); ¹H NMR (400 MHz, DMSO-d₆) δ 8.48-8.50 (m, 2H), 8.16 (s, 1H), 8.00-8.05 (m, 2H), 7.81-7.83 (m, 1H), 7.48-7.52 (m, 1H), 7.33 (t, 1H), 6.04-6.09 (m, 1H), 5.52-5.59 (m, 2H), 3.87 (s, 3H), 2.59 (s, 3H), 1.58 (d, 3H); Chiral analytical SFC: RT = 5.62 min, Chiralpak IC-3 (150 × 4.6 mm), 3 µ, 60% CO₂:MeOH, Flow rate = 3.0 mL/min.

### 3-((1-(6,7-difluoro-1-((1-methyl-1H-1,2,4-triazol-3-yl)methoay)isoquinolin-4-yl)ethyl)amino) propan-1-ol (VIba)

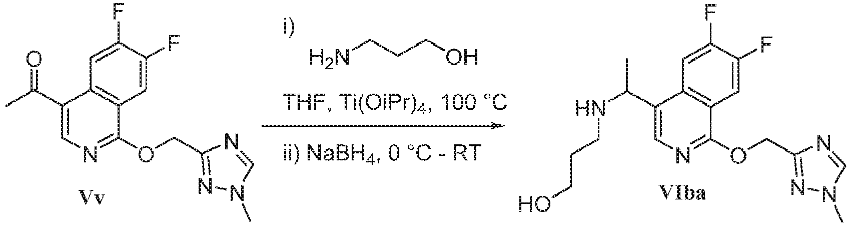

To a solution of 0.2 g (0.63 mmol, 1.0 eq.) of 1-(6,7-difluoro-1-((1-methyl-1*H*-1,2,4-triazol-3-yl)methoxy)isoquinolin-4-yl)ethanone (**Vv**) in 2 mL of THF in a sealed tube under a nitrogen atmosphere was added 0.07 g (0.94 mmol, 1.5 eq.) of 3-aminopropan-1-ol followed by 2 mL of titanium isopropoxide and the mixture was heated at 100 °C for 2 h. The mixture was allowed to cool to room temperature and then further cooled to 0 °C. The mixture was diluted with 2 mL of methanol and 0.05 g (1.25 mmol, 2.0 eq.) of sodium borohydride was added. The mixture was then allowed to warm to room temperature and stirred for 2 h. The reaction was quenched by the addition of 5 mL of water and 20 mL of 10% methanol in methylene chloride and filtered through CELITE^{®}. The pad was washed with 20 mL of 10% methanol in methylene chloride and the layers were separated. The organic phase was dried (Na₂SO₄), filtered and the solvent was removed *in vacuo* to provide 0.2 g of crude 3-(1-(6,7-difluoro-1-((1-methyl-1*H*-1,2,4-triazol-3-yl)methoxy)isoquinolin-4-yl)ethylamino)propan-1-ol (**VIba**). LCMS: *m*/*z* found 378.3 [M+H]⁺.

### 3-(3-Chloro-4-fluorophenyl)-1-(1-(6,7-difluoro-1-((1-methyl-1H-1,2,4-triazol-3-yl)methoxy) isoquinolin-4-yl)ethyl)-1-(3-hydroxypropyl)urea (Compounds 348 & 349)

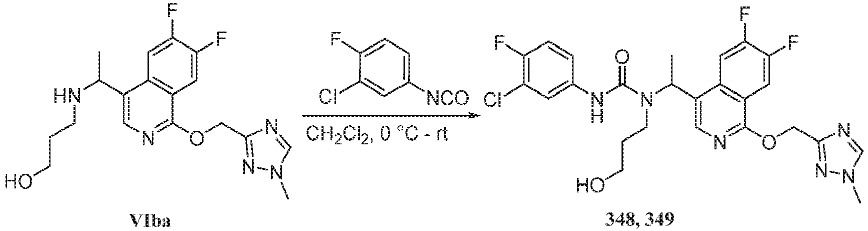

Racemic 3-(3-chloro-4-fluorophenyl)-1-(1-(6,7-difluoro-1-((1-methyl-1*H*-1,2,4-triazol-3-yl)methoxy) isoquinolin-4-yl)ethyl)-1-(3-hydroxypropyl)urea was synthesized in a similar manner as described above from 3-(1-(6,7-difluoro-1-((1-methyl-1*H*-1,2,4-triazol-3-yl)methoxy)isoquinolin-4-yl)ethylamino)propan-1-ol (**VIba**) and 2-chloro-1-fluoro-4-isocyanatobenzene. The enantiomers were subsequently separated by SFC, Column: Chiralpak IC (30 × 250 mm) 5 µ, 70% CO₂:MeOH, flow rate 100 g/min.

3-(3-chloro-4-fluorophenyl)-1-(1-(6,7-difluoro-1-((1-methyl-1*H*-1,2,4-triazol-3-yl)methoxy)isoquinolin-4-yl)ethyl)-1-(3-hydroxypropyl)urea - Enantiomer I (**Compound 348**). LCMS: *m*/*z* found 549.2/515.3 [M+H]⁺, RT = 4.91 min (Method: A); ¹H NMR (400 MHz, DMSO-d₆) δ 8.74 (s, 1H), 8.48 (s, 1H), 8.19 (s, 1H), 7.93-8.05 (m, 2H), 7.76-7.78 (m, 1H), 7.42-7.45 (m, 1H), 7.33 (t, 1H), 6.08-6.10 (m, 1H), 5.50-5.60 (m, 2H), 5.02 (bs, 1H), 3.87 (s, 3H), 3.13-3.17 (m, 4H), 1.60 (d, 3H), 1.01-1.06 (m, 2H); Chiral analytical SFC: RT = 3.83 min, Chiralpak IC-3 (150 × 4.6 mm), 3 µ, 60% CO₂:MeOH, Flow rate = 3.0 mL/min.

3-(3-chloro-4-fluorophenyl)-1-(1-(6,7-difluoro-1-((1-methyl-1*H*-1,2,4-triazol-3-yl)methoxy)isoquinolin-4-yl)ethyl)-1-(3-hydroxypropyl)urea - Enantiomer II (**Compound 349**). LCMS: *m*/*z* found 549.2/515.3 [M+H]⁺, RT = 4.91 min (Method: A); ¹H NMR (400 MHz, DMSO-d₆) δ 8.74 (s, 1H), 8.48 (s, 1H), 8.19 (s, 1H), 7.93-8.05 (m, 2H), 7.76-7.78 (m, 1H), 7.42-7.45 (m, 1H), 7.33 (t, 1H), 6.08-6.10 (m, 1H), 5.50-5.60 (m, 2H), 5.02 (bs, 1H), 3.87 (s, 3H), 3.13-3.17 (m, 4H), 1.60 (d, 3H), 1.01-1.06 (m, 2H); Chiral analytical SFC: RT = 6.06 min, Chiralpak IC-3 (150 × 4.6 mm), 3 µ, 60% CO₂:MeOH, Flow rate = 3.0 mL/min.

### 3-(3-Chloro-4-fluorophenyl)-1-(1-(6,7-difluoro-1-((1-trityl-1H-1,2,4-triazol-3-yl)methoxy) isoquinolin-4-yl)ethyl)-1-(3-hydroxypropyl)urea (VIIh)

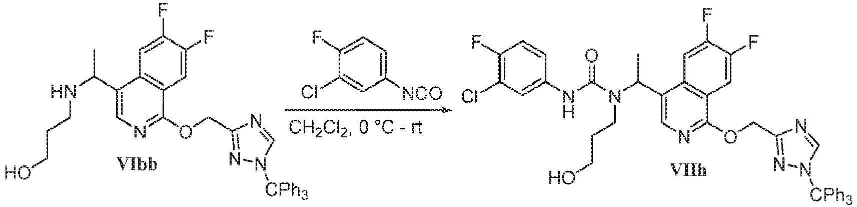

Racemic 3-(3-chloro-4-fluorophenyl)-1-(1-(6,7-difluoro-1-((1-trityl-1*H*-1,2,4-triazol-3-yl)methoxy) isoquinolin-4-yl)ethyl)-1-(3-hydroxypropyl)urea (**VIIh**) was synthesized in a similar manner as described above from 3-(1-(6,7-difluoro-1-((1-trityl-1H-1,2,4-triazol-3-yl)methoxy)isoquinolin-4-yl)ethylamino)propan-1-ol (**VIbb**) and 2-chloro-1-fluoro-4-isocyanatobenzene. LCMS: *m*/*z* found 777.5/779.5 [M+H]⁺.

### 1-(1-(1-((1H-1,2,4-Triazol-3-yl)methoxy)-6,7-difluoroisoquinolin-4-yl)ethyl)-3-(3-chloro-4-fluorophenyl)-1-(3-hydroxypropyl)urea (Compounds 364 & 365)

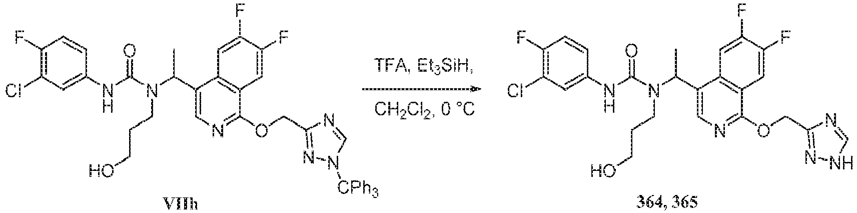

To a solution of 0.6 g (0.77 mmol, 1.0 eq.) of 3-(3-chloro-4-fluorophenyl)-1-(1-(6,7-difluoro-1-((1-trityl-1*H*-1,2,4-triazol-3-yl)methoxy)isoquinolin-4-yl)ethyl)-1-(3-hydroxypropyl)urea (**VIIh**) in 6 mL of methylene chloride at 0 °C was added 0.09 g (0.77 mmol, 1.0 eq.) of trifluoroacetic acid followed by 0.27 g (2.32 mmol, 3.0 eq.) of triethylsilane and the mixture was stirred at 0 °C for 2 h. The mixture was basified with 3 mL of saturated sodium bicarbonate solution to pH~9 and extracted with 3 × 50 mL of methylene chloride. The combined organic extracts were washed with 60 mL of brine, dried (Na₂SO₄), filtered and the solvent was removed *in vacuo.* The residue was purified flash chromatography (SiO₂, eluting with a linear gradient of 0-5% methanol in methylene chloride) to provide 0.3 g (0.56 mmol, 72%) of racemic 1-(1-(1-((1*H*-1,2,4-triazol-3-yl)methoxy)-6,7-difluoroisoquinotin-4-yl)ethyl)-3-(3-chloro-4-fluorophenyl)-1-(3-hydroxypropyl)urea. LCMS: *m*/*z* found 535.1/537.1 [M+H]⁺. The enantiomers were subsequently separated by SFC, Column: (R,R)-Whelk-01 (30 × 250 mm) 5 µ, 60% CO₂:MeOH, flow rate 100 g/min.

1-(1-(1-((1*H*-1,2,4-Triazol-3-yl)methoxy)-6,7-difluoroisoquinolin-4-yl)ethyl)-3-(3-chloro-4-fluorophenyl)-1-(3-hydroxypropyl)urea: Enantiomer I **(Compound 364**). LCMS: *m*/*z* found 535.3/537.3 [M+H]⁺, RT = 4.66 min (Method: A); ¹H NMR (400 MHz, DMSO-d₆) δ 14.06 (bs, 1H), 8.75 (s, 1H), 8.36 (s, 1H), 8.16-8.20 (m, 2H), 7.93-7.98 (m, 1H), 7.76-7.79 (m, 1H), 7.42-7.47 (m, 1H), 7.34 (t, 1H), 6.08-6.10 (m, 1H), 5.59-5.68 (m, 2H), 5.02 (bs, 1H), 3.09-3.16 (m, 4H), 1.61 (d, 3H), 1.01-1.10 (m, 2H); Chiral analytical SFC: RT = 2.20 min, (R,R)-Whelk-01 (150 × 4.6 mm), 3.5 µ, 60% CO₂:MeOH, Flow rate = 4.0 mL/min.

1-(1-(1-((1*H*-1,2,4-Triazol-3-yl)methoxy)-6,7-difluoroisoquinolin-4-yl)ethyl)-3-(3-chloro-4-fluorophenyl)-1-(3-hydroxypropyl)urea: Enantiomer II **(Compound 365**). LCMS: *m*/*z* found 535.3/537.3 [M+H]⁺, RT = 4.66 min (Method: A); ¹H NMR (400 MHz, DMSO-d₆) δ 14.06 (bs, 1H), 8.75 (s, 1H), 8.36 (s, 1H), 8.16-8.20 (m, 2H), 7.93-7.98 (m, 1H), 7.76-7.79 (m, 1H), 7.42-7.47 (m, 1H), 7.34 (t, 1H), 6.08-6.10 (m, 1H), 5.59-5.68 (m, 2H), 5.02 (bs, 1H), 3.09-3.16 (m, 4H), 1.61 (d, 3H), 1.01-1.10 (m, 2H); Chiral analytical SFC: RT = 3.78 min, (R,R)-Whelk-01 (150 × 4.6 mm), 3.5 µ, 60% CO₂:MeOH, Flow rate = 4.0 mL/min.

### 1-(1-(1-((1H-1,2,4-Triazol-3-yl)methoxy)-6,7-difluoroisoquinolin-4-yl)ethyl)-3-(3-chloro-4-fluorophenyl)-1-methylurea (Compounds 362 & 363)

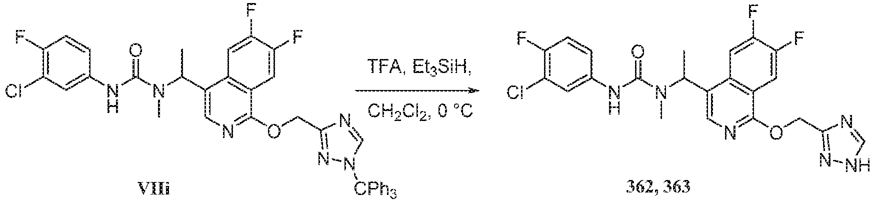

Racemic 1-(1-(1-((1*H*-1,2,4-triazol-3-yl)methoxy)-6,7-difluoroisoquinolin-4-yl)ethyl)-3-(3-chloro-4-fluorophenyl)-1-methylurea was synthesized in a similar manner as described above from 3-(3-chloro-4-fluorophenyl)-1-(1-(6,7-difluoro-1-((1-trityl-1*H*-1,2,4-triazol-3-yl)methoxy)isoquinolin-4-yl)ethyl)-1-methylurea (**VIIi**) and trifluoroacetic acid/triethylsilane. The enantiomers were subsequently separated by SFC, Column: (R,R)-Whelk-01 (30 × 250 mm) 5 µ, 65% CO₂:MeOH, flow rate 90 g/min.

1-(1-(1-((1*H*-1,2,4-Triazol-3-yl)methoxy)-6,7-difluoroisoquinolin-4-yl)ethyl)-3-(3-chloro-4-fluorophenyl)-1-methylurea: Enantiomer I (**Compound 362**). LCMS: *m*/*z* found 491.2/493.2 [M+H]⁺, RT = 4.77 min (Method: A); ¹H NMR (400 MHz, DMSO-d₆) δ 14.0 (bs, 1H), 8.50 (s, 1H), 8.34 (s, 1H), 8.15-8.21 (m, 2H), 8.00-8.05 (m, 1H), 7.81-7.84 (m, 1H), 7.48-7.53 (m, 1H), 7.33 (t, 1H), 6.04-6.10 (m, 1H), 5.60-5.67 (m, 2H), 2.59 (s, 3H), 1.58 (d, 3H); Chiral analytical SFC: RT = 3.84 min, (R,R)-Whelk-01 (150 × 4.6 mm), 3.5 µ, 70% CO₂:MeOH, Flow rate = 4.0 mL/min.

1-(1-(1-((1*H*-1,2,4-Triazol-3-yl)methoxy)-6,7-difluoroisoquinolin-4-yl)ethyl)-3-(3-chloro-4-fluorophenyl)-1-methylurea: Enantiomer II (**Compound 363**). LCMS: *m*/*z* found 491.2/493.2 [M+H]⁺, RT = 4.77 min (Method: A); ¹H NMR (400 MHz, DMSO-d₆) δ 14.0 (bs, 1H), 8.50 (s, 1H), 8.34 (s, 1H), 8.15-8.21 (m, 2H), 8.00-8.05 (m, 1H), 7.81-7.84 (m, 1H), 7.48-7.53 (m, 1H), 7.33 (t, 1H), 6.04-6.10 (m, 1H), 5.60-5.67 (m, 2H), 2.59 (s, 3H), 1.58 (d, 3H); Chiral analytical SFC: RT = 5.70 min, (R,R)-Whelk-01 (150 × 4.6 mm), 3.5 µ, 70% CO₂:MeOH, Flow rate = 4.0 mL/min.

### Synthesis of 6,7-difluoroisoquinolin-1(2H)-one (IIf)

### 1-(2,3-Difluorophenyl)-N-(2,2-dimethoxyethyl)methanamine

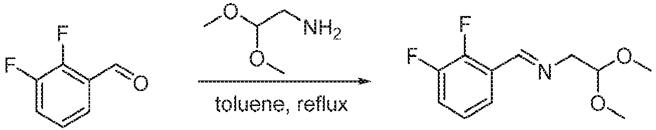

To a solution of 25.0 g (176.1 mmol, 1.0 eq.) of 2,3-difluorobenzaldehyde in 200 mL of toluene in an apparatus equipped with a Dean-Stark trap was added 18.5 g (176.1 mmol, 1.0 eq.) of 2,2-dimethoxyethanamine and the mixture was heated to azeotropic reflux for 16 h. The mixture was allowed to cool to room temperature and the solvent was removed *in vacuo* to provide 35 g of crude (E)-1-(2,3-difluorophenyl)-N-(2,2-dimethoxyethyl)methanimine which was used without further purification. ¹H NMR (400 MHz, CDCl₃): δ 8.57 (s, 1H), 7.72-7.77 (m, 1H), 7.18-7.25 (m, 1H), 7.07-7.13 (m, 1H), 4.69 (t, 1H), 3.81 (d, 2H), 3.43 (s, 6H).

### 7,8-Difluoroisoquinoline

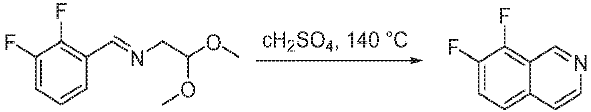

A mixture of 35.0 g (152.8 mmol, 1.0 eq.) of (E)-1-(2,3-difluorophenyl)-N-(2,2-dimethoxyethyl)methanimine and 250 mL of chilled conc. H₂SO₄ was stirred at 140 °C for 30 min. The mixture was allowed to cool to room temperature and slowly poured into 600 mL of ice-cold water. The mixture was then filtered through CELITE^{®} and the pad was washed with 100 mL of water. The filtrate was washed with 2 × 500 mL of methylene chloride and the aqueous layer was basified with 500 mL of 10 M aqueous sodium hydroxide solution, and extracted with 3 × 500 mL of methylene chloride. The combined organic extracts were dried (Na₂SO₄), filtered and the solvent was removed *in vacuo* to provide 5.0 g (30.30 mmol, 17%) of 7,8-difluoroisoquinoline. LCMS: *m*/*z* found 166.0 [M+H]⁺, ¹H NMR (400 MHz, DMSO-d₆): δ 9.51 (s, 1H), 8.63 (d, 1H), 7.89-7.97 (m, 3H).

### 7,8-Difluoroisoquinoline 2-oxide

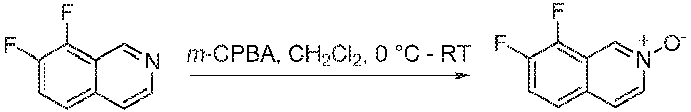

To a stirred solution of 5.0 g (30.3 mmol, 1.0 eq.) of 7,8-difluoroisoquinoline in 100 mL of methylene chloride at 0 °C was added 10.4 g (60.6 mmol, 2.0 eq.) of *m-*chloroperoxybenzoic acid portion-wise over approximately 15 min. The mixture was allowed to warm to room temperature and stirred for 7 h. The reaction mixture was re-cooled to 0 °C and quenched by the addition of 200 mL of saturated sodium bicarbonate solution and then extracted 3 × 200 mL of 10% methanol in methylene chloride. The combined organic extracts were washed with 50 mL of brine, dried (Na₂SO₄), filtered and the solvent was removed *in vacuo.* The residue was triturated with 2 × 100 mL of *n*-pentane, filtered, and dried under high vacuum to provide 4.5 g (24.8 mmol, 90%) of 7,8-difluoroisoquinoline 2-oxide. LCMS: *m*/*z* found 181.9 [M+H]⁺, ¹H NMR (400 MHz, DMSO-d₆): δ 8.89 (s, 1H), 8.21-8.24 (m, 1H), 8.03-8.06 (m, 1H), 7.87-7.90 (m, 1H), 7.68-7.76 (m, 1H).

### 7,8-Difluoroisoquinolin-1(2H)-one (IIf)

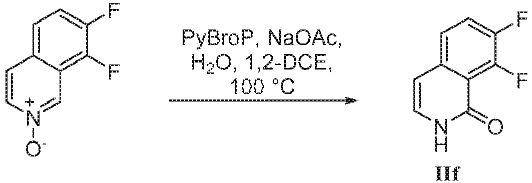

To a suspension of 2.5 g (13.8 mmol, 1.0 eq.) of 7,8-difluoroisoquinoline 2-oxide in 25 mL of 1,2-dichloroethane was added 3.4 g (41.4 mmol, 3.0 eq.) of sodium acetate followed by 12.9 g (27.6 mmol, 2.0 eq.) of bromotripyrrolidinophosphonium hexafluorophosphate (PyBroP) and 3.7 mL (207.2 mmol, 15 eq.) of water and the mixture was heated at 100 °C for 12 h. The mixture was allowed to cool to room temperature and diluted with 30 mL of methylene chloride and 50 mL of water. The resulting suspension was filtered and the solid was dissolved in 200 mL of 50% methanol in methylene chloride, dried (Na₂SO₄), filtered and the solvent was removed *in vacuo* to provide 1.3 g (7.2 mmol, 52%) of 7,8-difluoroisoquinolin-1(2*H*)-one (**IIf**). LCMS: *m*/*z* found 182.1 [M+H]⁺, ¹H NMR (400 MHz, DMSO-d₆): δ 11.34 (bs, 1H), 7.74-7.82 (m, 1H), 7.49-7.53 (m, 1H), 7.14-7.17 (m, 1H), 6.53-6.54 (m, 1H).

The above detailed reaction sequence was performed on multiple batches with consistent results.

### 4-Bromo-7,8-difluoroisoquinolin-1(2H)-one (IIIf)

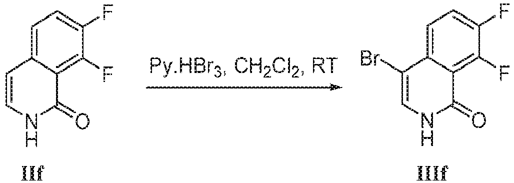

To a suspension of 3.8 g (21.0 mmol, 1.0 eq) of 7,8-difluoroisoquinolin-1(2*H*)-one (**IIf**) in 10 mL of methylene chloride was added 5.26 g (16.6 mmol, 0.8 eq.) of pyridinium bromide perbromide and the mixture was stirred at room temperature for 3 h. The reaction was then quenched by the addition of 100 mL of saturated sodium bicarbonate solution and the solvent was removed *in vacuo.* The residue was suspended in 150 mL of water, filtered and the solids were washed with 60 mL of *n*-pentane and dried under high vacuum to provide 4.0 g (15.38 mmol, 93%) of 4-bromo-7,8-difluoroisoquinolin-1(2*H*)-one (**IIIf**). LCMS: *m*/*z* found 260.0/262.0 [M+H]⁺, ¹H NMR (400 MHz, DMSO-d₆): δ 11.67 (bs, 1H), 7.90-7.97 (m, 1H), 7.57-7.63 (m, 2H).

### 4-Acetyl-7,8-difluoroisoquinolin-1(2H)-one (XXf)

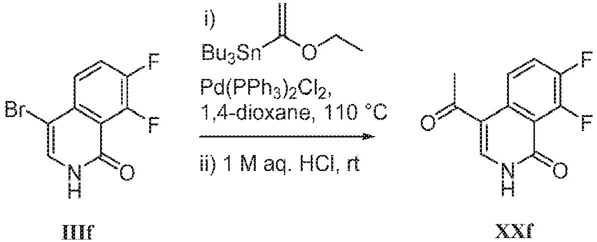

To a solution of 3.0 g (11.58 mmol, 1.0 eq.) of 4-bromo-7,8-difluoroisoquinolin-1(2*H*)-one (**IIIf**) in 30 mL of 1,4-dioxane was added 10.48 g (28.95 mmol, 2.5 eq) of tributyl(1-ethoxyvinyl)stannane. The mixture was purged with nitrogen gas for 5 min and 0.81 g (1.15 mmol, 0.1 eq.) of Pd(PPh₃)₂Cl₂ was added, and then heated to 110 °C for 16 h. The reaction mixture was allowed to cool to room temperature and 30 mL of 1 M aqueous HCl was added and stirring was continued for an additional 3 h. The reaction mixture was then basified with 50 mL of saturated sodium bicarbonate solution and extracted with 3 × 200 mL of ethyl acetate. The combined organic extracts were washed with 100 mL of water, 100 mL of brine, dried (Na₂SO₄), filtered and the solvent was removed *in vacuo.* The residue was triturated with 60 mL of *n*-pentane, filtered the solids were dried under high vacuum to provide 2.3 g of 4-acetyl-7,8-difluoroisoquinolin-1(2*H*)-one (**XXf**). LCMS: *m*/*z* found 224.0 [M+H]⁺.
The above reaction sequence was performed on multiple batches with consistent results.

### 7,8-Difluoro-4-(1-(methylamino)ethyl)isoquinolin-1(2H)-one (VIIIx)

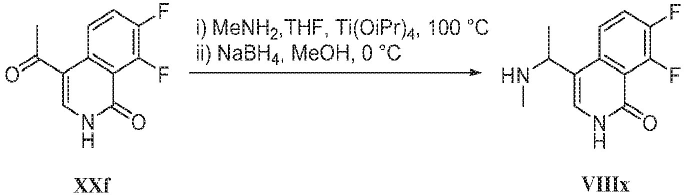

To a solution of 300 mg (1.34 mmol, 1.0 eq.) of 4-acetyl-7,8-difluoroisoquinolin-1(*2H*)-one **(XXf)** was added 3 mL (6 mmol, 4.4 eq.) of a 2 M solution of methylamine in THF followed by 1.5 mL of titanium isopropoxide and the mixture was heated at 100 °C for 16 h. The mixture was allowed to cool to room temperature and further cooled to 0 °C. Following dilution with 3 mL of methanol, 0.15 g (4.0 mmol, 3.0 eq.) of sodium borohydride was added portion-wise over approximately 10 min and stirring was continued for 3 h. The reaction mixture was diluted with 50 mL of brine and extracted with 100 mL of 10% methanol in methylene chloride. The combined organic extracts were washed with 50 mL of brine, dried (Na₂SO₄) and the solvent was removed *in vacuo* to provide 0.21 g of 7,8-difluoro-4-(1-(methylamino)ethyl)isoquinolin-1(2*H*)-one **(VIIIx).** LCMS: *m*/*z* found 239.1 [M+H]⁺.
The above reaction sequence was performed on multiple batches with consistent results.

### 3-(3-Chloro-4-fluorophenyl)-1-(1-(7,8-difluoro-1-oxo-1,2-dihydroisoquinolin-4-yl)ethyl)-1-methylurea (Compounds 330 & 331)

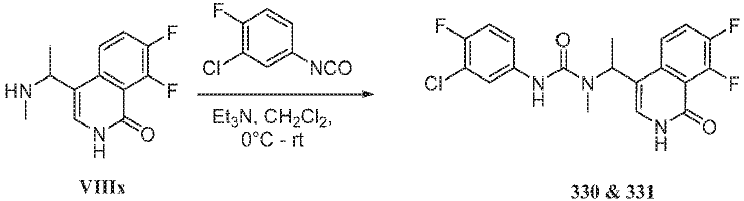

Racemic 3-(3-chloro-4-fluorophenyl)-1-(1-(7,8-difluoro-1-oxo-1,2-dihydroisoquinolin-4-yl)ethyl)-1-methylurea was synthesized in a similar manner as described above from 7,8-difluoro-4-(1-(methylamino)ethyl)isoquinolin-1(*2H*)-one **(VIIIx)** and 2-chloro-1-fluoro-4-isocyanatobenzene. The enantiomers were subsequently separated by chiral SFC, Column: Chiralpak IC (250 × 30 mm) 5 µ, 65% CO₂/MeOH, Flow rate 90 g/min.

3-(3-Chloro-4-fluorophenyl)-1-(1-(7,8-difluoro-1-oxo-1,2-dihydroisoquinolin-4-yl)ethyl)-1-methylurea - Enantiomer I **(Compound 330),** LCMS: *m*/*z* found 410.2/412.2 [M+H]⁺, RT = 4.33 min (Method A); ¹H NMR (400 MHz, DMSO-d₆): δ 11.49 (bs, 1H), 8.46 (s 1H), 7.84-7.93 (m, 2H), 7.47-7.53 (m, 2H), 7.31 (t, 1H), 7.15 (s, 1H), 5.75-5.81 (m, 1H), 2.58 (s, 3H), 1.41 (d, 3H); Chiral analytical SFC: RT = 2.24 min, Column: Chiralpak IC, (4.6 × 150 mm) 5 µm, 60% CO₂/MeOH, Flow = 3.0 g/min.

3-(3-Chloro-4-fluorophenyl)-1-(1-(7,8-difluoro-1-oxo-1,2-dihydroisoquinolin-4-yl)ethyl)-1-methylurea - Enantiomer II **(Compound 331),** LCMS: *m*/*z* found 410.2/412.2 [M+H]⁺, RT = 4.33 min (Method A); ¹H NMR (400 MHz, DMSO-d₆): δ 11.49 (bs, 1H), 8.46 (s 1H), 7.84-7.93 (m, 2H), 7.47-7.53 (m, 2H), 7.31 (t, 1H), 7.15 (s, 1H), 5.75-5.81 (m, 1H), 2.58 (s, 3H), 1.41 (d, 3H); Chiral analytical SFC: RT = 3.39 min, Column: Chiralpak IC, (4.6 × 150 mm) 5 µm, 60% CO₂/MeOH, Flow = 3.0 g/min.

### 1-(1-(7,8-Difluoro-1-oxo-1,2-dihydroisoquinolin-4-yl)ethyl)-3-(4-fluorophenyl)-1-methylurea (Compounds 432 & 433)

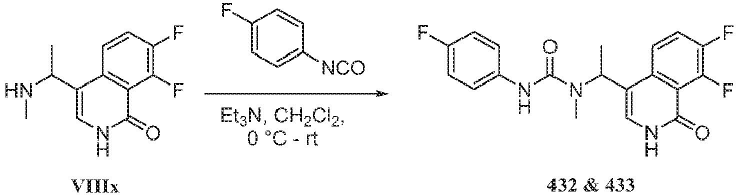

Racemic 1-(1-(7,8-difluoro-1-oxo-1,2-dihydroisoquinolin-4-yl)ethyl)-3-(4-fluorophenyl)-1-methylurea was synthesized in a similar manner as described above from 7,8-difluoro-4-(1-(methylamino)ethyl)isoquinolin-1(*2H*)-one **(VIIIx)** and 4-fluorophenyl isocyanate. The enantiomers were subsequently separated by chiral SFC, Column: Chiralpak IC (250 × 30 mm) 5 µ, 65% CO₂/MeOH, Flow rate 90 g/min.

1-(1-(7,8-Difluoro-1-oxo-1,2-dihydroisoquinolin-4-yl)ethyl)-3-(4-fluorophenyl)-1-methylurea - Enantiomer I **(Compound 432),** LCMS: *m*/*z* found 376.2 [M+H]⁺, RT = 3.36 min (Method A); ¹H NMR (400 MHz, DMSO-d₆): δ 11.46 (bs, 1H), 8.30 (bs, 1H), 7.83-7.90 (m, 1H), 7.51-7.57 (m, 3H), 7.14 (s, 1H), 7.06-7.11 (m, 2H), 5.76-5.82 (m, 1H), 2.57 (s, 3H), 1.40 (d, 3H); Chiral analytical SFC: RT = 1.58 min, Column: Chiralpak IC-3, (4.6 × 150 mm) 3 µm, 60% CO₂/MeOH, Flow = 3.0 g/min.

1-(1-(7,8-Difluoro-1-oxo-1,2-dihydroisoquinolin-4-yl)ethyl)-3-(4-fluorophenyl)-1-methylurea - Enantiomer II **(Compound 433),** LCMS: *m*/*z* found 376.3 [M+H]⁺, RT = 3.36 min (Method A); ¹H NMR (400 MHz, DMSO-d₆): δ 11.46 (bs, 1H), 8.30 (bs, 1H), 7.83-7.90 (m, 1H), 7.51-7.57 (m, 3H), 7.14 (s, 1H), 7.06-7.11 (m, 2H), 5.76-5.82 (m, 1H), 2.57 (s, 3H), 1.40 (d, 3H); Chiral analytical SFC: RT = 2.72 min, Column: Chiralpak IC-3, (4.6 × 150 mm) 3 µm, 60% CO₂/MeOH, Flow = 3.0 g/min.

### 1-(1-(7,8-Difluoro-1-oxo-1,2-dihydroisoquinolin-4-yl)ethyl)-3-(3,4-difluorophenyl)-1-methylurea (Compounds 434 & 435)

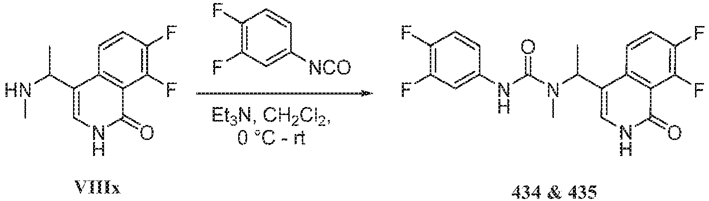

Racemic 1-(1-(7,8-difluoro-1-oxo-1,2-dihydroisoquinolin-4-yl)ethyl)-3-(3,4-difluorophenyl)-1-methylurea was synthesized in a similar manner as described above from 7,8-difluoro-4-(1-(methylamino)ethyl)isoquinolin-1(*2H*)-one **(VIIIx)** and 3,4-difluorophenyl isocyanate. The enantiomers were subsequently separated by chiral SFC, Column: Chiralpak IC (250 × 30 mm) 5 µ, 85% CO₂/MeOH, Flow rate 100 g/min.

1-(1-(7,8-Difluoro-1-oxo-1,2-dihydroisoquinolin-4-yl)ethyl)-3-(3,4-difluorophenyl)-1-methylurea - Enantiomer I **(Compound 434),** LCMS: *m*/*z* found 394.2 [M+H]⁺, RT = 3.76 min (Method A); ¹H NMR (400 MHz, DMSO-d₆): δ 11.51 (bs, 1H), 8.47 (bs, 1H), 7.83-7.92 (m, 1H), 7.70-7.75 (m, 1H), 7.49-7.53 (m, 1H), 7.29-7.33 (m, 2H), 7.15 (s, 1H), 5.75-5.80 (m, 1H), 2.58 (s, 3H), 1.41 (d, 3H); Chiral analytical SFC: RT = 3.75 min, Column: Chiralpak IC-3, (4.6 × 150 mm) 5 µm, 60% CO₂/MeOH, Flow = 3.0 g/min.

1-(1-(7,8-Difluoro-1-oxo-1,2-dihydroisoquinolin-4-yl)ethyl)-3-(3,4-difluorophenyl)-1-methylurea - Enantiomer II **(Compound 435),** LCMS: *m*/*z* found 394.2 [M+H]⁺, RT = 3.76 min (Method A); ¹H NMR (400 MHz, DMSO-d₆): δ 11.51 (bs, 1H), 8.47 (bs, 1H), 7.83-7.92 (m, 1H), 7.70-7.75 (m, 1H), 7.49-7.53 (m, 1H), 7.29-7.33 (m, 2H), 7.15 (s, 1H), 5.75-5.80 (m, 1H), 2.58 (s, 3H), 1.41 (d, 3H); Chiral analytical SFC: RT = 5.74 min, Column: Chiralpak IC-3, (4.6 × 150 mm) 3 µm, 60% CO₂/MeOH, Flow = 3.0 g/min.

### 1-(1-(7,8-Difluoro-1-oxo-1,2-dihydroisoquinolin-4-yl)ethyl)-1-methyl-3-phenylurea (Compounds 436 & 437)

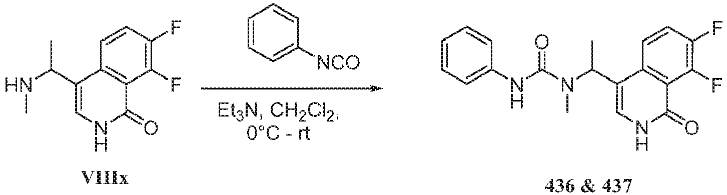

Racemic 1-(1-(7,8-difluoro-1-oxo-1,2-dihydroisoquinolin-4-yl)ethyl)-1-methyl-3-phenylurea was synthesized in a similar manner as described above from 7,8-difluoro-4-(1-(methylamino)ethyl)isoquinolin-1(*2H*)-one **(VIIIx)** and phenyl isocyanate. The enantiomers were subsequently separated by chiral SFC, Column: Chiralpak IG (250 × 30 mm) 5 µ, 70% CO₂/MeOH, Flow rate 100 g/min.

1-(1-(7,8-difluoro-1-oxo-1,2-dihydroisoquinolin-4-yl)ethyl)-1-methyl-3-phenylurea-Enantiomer I **(Compound 436),** LCMS: *m*/*z* found 358.3 [M+H]⁺, RT = 3.21 min (Method A); ¹H NMR (400 MHz, DMSO-d₆): δ 11.49 (bs, 1H), 8.24 (bs, 1H), 7.84-7.91 (m, 1H), 7.52-7.60 (m, 3H), 7.23-7.27 (m, 2H), 7.14 (s, 1H), 6.94-6.98 (m, 1H), 5.78-5.82 (m, 1H), 2.59 (s, 3H), 1.41 (d, 3H); Chiral analytical SFC: RT = 2.86 min, Column: Chiralpak IC-3, (4.6 × 150 mm) 3 µm, 60% CO₂/MeOH, Flow = 3.0 g/min.

1-(1-(7,8-difluoro-1-oxo-1,2-dihydroisoquinolin-4-yl)ethyl)-1-methyl-3-phenylurea - Enantiomer II **(Compound 437),** LCMS: *m*/*z* found 358.2 [M+H]⁺, RT = 3.21 min (Method A); ¹H NMR (400 MHz, DMSO-d₆): δ 11.49 (bs, 1H), 8.24 (bs, 1H), 7.84-7.91 (m, 1H), 7.52-7.60 (m, 3H), 7.23-7.27 (m, 2H), 7.14 (s, 1H), 6.94-6.98 (m, 1H), 5.78-5.82 (m, 1H), 2.59 (s, 3H), 1.41 (d, 3H); Chiral analytical SFC: RT = 4.23 min, Column: Chiralpak IC-3, (4.6 × 150 mm) 3 µm, 60% CO₂/MeOH, Flow = 3.0 g/min.

### 1-(1-(7,8-Difluoro-1-oxo-1,2-dihydroisoquinolin-4-yl)ethyl)-3-(4-fluoro-3-methylphenyl)-1-methylurea (Compounds 440 & 441)

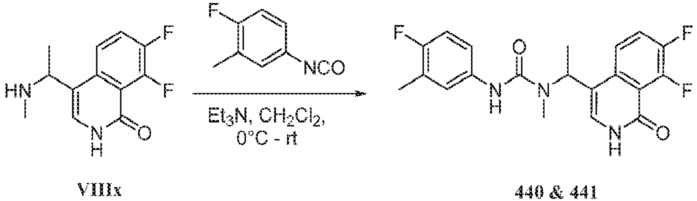

Racemic 1-(1-(7,8-difluoro-1-oxo-1,2-dihydroisoquinolin-4-yl)ethyl)-3-(4-fluoro-3-methylphenyl)-1-methylurea was synthesized in a similar manner as described above from 7,8-difluoro-4-(1-(methylamino)ethyl)isoquinolin-1(*2H*)-one **(VIIIx)** and 1-fluoro-4-isocyanato-2-methylbenzene. The enantiomers were subsequently separated by chiral SFC, Column: (R,R) Whelk-01 (250 × 30 mm) 5 µ, 65% CO₂/MeOH, Flow rate 100 g/min.

1-(1-(7,8-Difluoro-1-oxo-1,2-dihydroisoquinofin-4-yl)ethyl)-3-(4-fluoro-3-methylphenyl)-1-methylurea - Enantiomer I **(Compound 440),** LCMS: *m*/*z* found 390.3 [M+H]⁺, RT = 3.73 min (Method A); ¹H NMR (400 MHz, DMSO-d₆): δ 11.49 (bs, 1H), 8.21 (bs, 1H), 7.84-7.91 (m, 1H), 7.53-7.56 (m, 1H), 7.43-7.45 (m, 1H), 7.30-7.34 (m, 1H), 7.14 (s, 1H), 7.01 (t, 1H), 5.76-5.81 (m, 1H), 2.56 (s, 3H), 2.20 (s, 3H), 1.40 (d, 3H); Chiral analytical SFC: RT = 3.53 min, Column: (R,R) Whelk-01, (4.6 × 150 mm) 3.5 µm, 60% CO₂/MeOH, Flow = 3.0 g/min.

1-(1-(7,8-Difluoro-1-oxo-1,2-dihydroisoquinofin-4-yl)ethyl)-3-(4-fluoro-3-methylphenyl)-1-methylurea - Enantiomer II **(Compound 441),** LCMS: *m*/*z* found 390.3 [M+H]⁺, RT = 3.73 min (Method A); ¹H NMR (400 MHz, DMSO-d₆): δ 11.49 (bs, 1H), 8.21 (bs, 1H), 7.84-7.91 (m, 1H), 7.53-7.56 (m, 1H), 7.43-7.45 (m, 1H), 7.30-7.34 (m, 1H), 7.14 (s, 1H), 7.01 (t, 1H), 5.76-5.81 (m, 1H), 2.56 (s, 3H), 2.20 (s, 3H), 1.40 (d, 3H); Chiral analytical SFC: RT = 4.72 min, Column: (R,R) Whelk-01, (4.6 × 150 mm) 3.5 µm, 60% CO₂/MeOH, Flow = 3.0 g/min.

### 1-(1-(7,8-Difluoro-1-oxo-1,2-dihydroisoquinolin-4-yl)ethyl)-1-methyl-3-(3,4,5-trifluorophenyl)urea (Compounds 442 & 443)

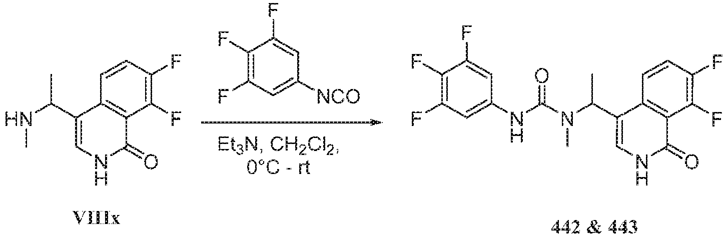

Racemic 1-(1-(7,8-difluoro-1-oxo-1,2-dihydroisoquinolin-4-yl)ethyl)-1-methyl-3-(3,4,5-trifluorophenyl)urea was synthesized in a similar manner as described above from 7,8-difluoro-4-(1-(methylamino)ethyl)isoquinolin-1(*2H*)-one **(VIIIx)** and 1,2,3-trifluoro-5-isocyanatobenzene. The enantiomers were subsequently separated by chiral SFC, Column: Chiralpak IG (250 × 30 mm) 5 µ, 50% CO₂/MeOH, Flow rate 90 g/min.

1-(1-(7,8-Difluoro-1-oxo-1,2-dihydroisoquinofin-4-yl)ethyl)-1-methyl-3-(3,4,5-trifluorophenyl)urea - Enantiomer I **(Compound 442),** LCMS: *m*/*z* found 412.2 [M+H]⁺, RT = 4.16 min (Method A); ¹H NMR (400 MHz, DMSO-d₆): δ 11.52 (bs, 1H), 8.61 (bs, 1H), 7.84-7.91 (m, 1H), 7.46-7.55 (m, 3H), 7.16 (s, 1H), 5.74-5.79 (m, 1H), 2.57 (d, 3H), 1.41 (d, 3H); Chiral analytical SFC: RT = 0.84 min, Column: Chiralpak IG-3, (4.6 × 150 mm) 5 µm, 60% CO₂/MeOH, Flow = 3.0 g/min.

1-(1-(7,8-Difluoro-1-oxo-1,2-dihydroisoquinofin-4-yl)ethyl)-1-methyl-3-(3,4,5-trifluorophenyl)urea - Enantiomer II **(Compound 443),** LCMS: *m*/*z* found 412.2 [M+H]⁺, RT = 4.16 min (Method A); ¹H NMR (400 MHz, DMSO-d₆): δ 11.52 (bs, 1H), 8.61 (bs, 1H), 7.84-7.91 (m, 1H), 7.46-7.55 (m, 3H), 7.16 (s, 1H), 5.74-5.79 (m, 1H), 2.57 (d, 3H), 1.41 (d, 3H); Chiral analytical SFC: RT = 3.96 min, Column: Chiralpak IG-3, (4.6 × 150 mm) 5 µm, 60% CO₂/MeOH, Flow = 3.0 g/min.

### 3-(4-Chlorophenyl)-1-(1-(7,8-difluoro-1-oxo-1,2-dihydroisoquinolin-4-yl)ethyl)-1-methylurea (Compounds 444 & 445)

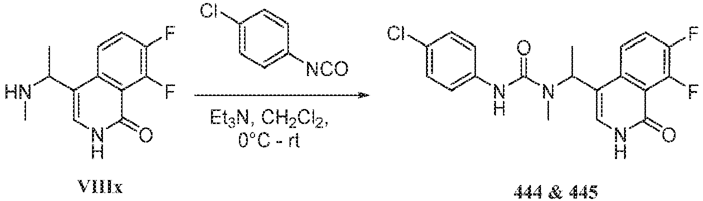

Racemic 3-(4-chlorophenyl)-1-(1-(7,8-difluoro-1-oxo-1,2-dihydroisoquinolin-4-yl)ethyl)-1-methylurea was synthesized in a similar manner as described above from 7,8-difluoro-4-(1-(methylamino)ethyl)isoquinolin-1(*2H*)-one **(VIIIx)** and 4-chlorophenyl isocyanate. The enantiomers were subsequently separated by chiral SFC, Column: Chiralpak IC (250 × 30 mm) 5 µ, 65% CO₂/MeOH, Flow rate 100 g/min.

3-(4-Chlorophenyl)-1-(1-(7,8-difluoro-1-oxo-1,2-dihydroisoquinolin-4-yl)ethyl)-1-methylurea - Enantiomer I **(Compound 444),** LCMS: *m*/*z* found 392.2/394.2 [M+H]⁺, RT = 3.90 min (Method A); ¹H NMR (400 MHz, DMSO-d₆): δ 11.50 (bs, 1H), 8.39 (bs, 1H), 7.84-7.91 (m, 1H), 7.52-7.59 (m, 3H), 7.29-7.31 (m, 2H), 7.14 (s, 1H), 5.76-5.81 (m 1H), 2.59 (s, 3H), 1.41 (d, 3H); Chiral analytical SFC: RT = 1.62 min, Column: Chiralpak IC-3, (4.6 × 150 mm) 3 µm, 60% CO₂/MeOH, Flow = 3.0 g/min.

3-(4-Chlorophenyl)-1-(1-(7,8-difluoro-1-oxo-1,2-dihydroisoquinolin-4-yl)ethyl)-1-methylurea - Enantiomer II **(Compound 445),** LCMS: *m*/*z* found 392.2/394.2 [M+H]⁺, RT = 3.90 min (Method A); ¹H NMR (400 MHz, DMSO-d₆): δ 11.50 (bs, 1H), 8.39 (bs, 1H), 7.84-7.91 (m, 1H), 7.52-7.59 (m, 3H), 7.29-7.31 (m, 2H), 7.14 (s, 1H), 5.76-5.81 (m 1H), 2.59 (s, 3H), 1.41 (d, 3H); Chiral analytical SFC: RT = 2.50 min, Column: Chiralpak IC-3, (4.6 × 150 mm) 3 µm, 60% CO₂/MeOH, Flow = 3.0 g/min.

### 3-(3,5-Dichlorophenyl)-1-(1-(7,8-difluoro-1-oxo-1,2-dihydroisoquinolin-4-yl)ethyl)-1-methylurea (Compounds 447 & 448)

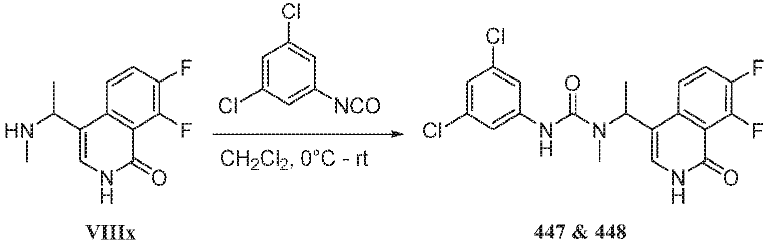

Racemic 3-(3,5-dichlorophenyl)-1-(1-(7,8-difluoro-1-oxo-1,2-dihydroisoquinolin-4-yl)ethyl)-1-methylurea was synthesized in a similar manner as described above from 7,8-difluoro-4-(1-(methylamino)ethyl)isoquinolin-1(*2H*)-one **(VIIIx)** and 1,3-dichloro-5-isocyanatobenzene. The enantiomers were subsequently separated by chiral SFC, Column: Chiralpak IG (250 × 30 mm) 5 µ, 50% CO₂/MeOH, Flow rate 90 g/min.

3-(3,5-Dichlorophenyl)-1-(1-(7,8-difluoro-1-oxo-1,2-dihydroisoquinolin-4-yl)ethyl)-1-methylurea - Enantiomer I **(Compound 447),** LCMS: *m*/*z* found 426.2/428.2/430.2 [M+H]⁺, RT = 4.75 min (Method A); ¹H NMR (400 MHz, DMSO-d₆): δ 11.60 (bs, 1H), 8.60 (bs, 1H), 7.84-7.91 (m, 1H), 7.71 (d, 2H), 7.45-7.48 (m, 1H), 7.14-7.16 (m, 2H), 5.74-5.79 (m, 1H), 2.58 (s, 3H), 1.42 (d, 3H); Chiral analytical SFC: RT = 1.24 min, Column: Chiralpak IG-3, (4.6 × 150 mm) 3 µm, 60% CO₂/MeOH, Flow = 3.0 g/min.

3-(3,5-Dichlorophenyl)-1-(1-(7,8-difluoro-1-oxo-1,2-dihydroisoquinolin-4-yl)ethyl)-1-methylurea - Enantiomer II **(Compound 448),** LCMS: *m*/*z* found 426.2/428.2/430.2 [M+H]⁺, RT = 4.75 min (Method A); ¹H NMR (400 MHz, DMSO-d₆): δ 11.60 (bs, 1H), 8.60 (bs, 1H), 7.84-7.91 (m, 1H), 7.71 (d, 2H), 7.45-7.48 (m, 1H), 7.14-7.16 (m, 2H), 5.74-5.79 (m, 1H), 2.58 (s, 3H), 1.42 (d, 3H); Chiral analytical SFC: RT = 3.31 min, Column: Chiralpak IG-3, (4.6 × 150 mm) 3 µm, 60% CO₂/MeOH, Flow = 3.0 g/min.

### 3-Benzyl-1-(1-(7,8-difluoro-1-oxo-1,2-dihydroisoquinolin-4-yl)ethyl)-1-methylurea (Compound 453)

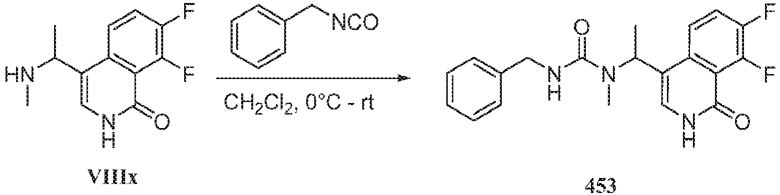

Racemic 3-benzyl-1-(1-(7,8-difluoro-1-oxo-1,2-dihydroisoquinolin-4-yl)ethyl)-1-methylurea was synthesized in a similar manner as described above from 7,8-difluoro-4-(1-(methylamino)ethyl)isoquinolin-1(*2H*)-one **(VIIIx)** and (isocyanatomethyl)benzene. The enantiomers were subsequently separated by chiral SFC, Column: Chiralpak IC (250 × 30 mm) 5 µ, 70% CO₂/MeOH, Flow rate 90 g/min.

3-Benzyl-1-(1-(7,8-difluoro-1-oxo-1,2-dihydroisoquinolin-4-yl)ethyl)-1-methylurea - Enantiomer I (), LCMS: *m*/*z* found 372.2 [M+H]⁺, RT = 3.12 min (Method A); ¹H NMR (400 MHz, DMSO-d₆): δ 11.45 (bs, 1H), 7.69-7.74 (m, 1H), 7.54-7.57 (m, 1H), 7.21-7.34 (m, 5H), 7.08 (s, 1H), 6.92 (t, 1H), 5.71-5.76 (m, 1H), 4.33-4.39 (m, 1H), 4.24-4.30 (m, 1H), 2.43 (s, 3H), 1.34 (d, 3H); Chiral analytical SFC: RT = 5.28 min, Column: Chiralpak IC-3, (4.6 × 150 mm) 3 µm, 75% CO₂/MeOH, Flow = 3.0 g/min.

3-Benzyl-1-(1-(7,8-difluoro-1-oxo-1,2-dihydroisoquinolin-4-yl)ethyl)-1-methylurea - Enantiomer II **(Compound 453),** LCMS: *m*/*z* found 372.2 [M+H]⁺, RT = 3.11 min (Method A); ¹H NMR (400 MHz, DMSO-d₆): δ 11.45 (bs, 1H), 7.69-7.74 (m, 1H), 7.54-7.57 (m, 1H), 7.21-7.34 (m, 5H), 7.08 (s, 1H), 6.92 (t, 1H), 5.71-5.76 (m, 1H), 4.33-4.39 (m, 1H), 4.24-4.30 (m, 1H), 2.43 (s, 3H), 1.34 (d, 3H); Chiral analytical SFC: RT = 8.03 min, Column: Chiralpak IC-3, (4.6 × 150 mm) 3 µm, 75% CO₂/MeOH, Flow = 3.0 g/min.

### 1-(1-(7,8-Difluoro-1-oxo-1,2-dihydroisoquinolin-4-yl)ethyl)-1-methyl-3-((S)-1-phenylethyl)urea (Compound 487)

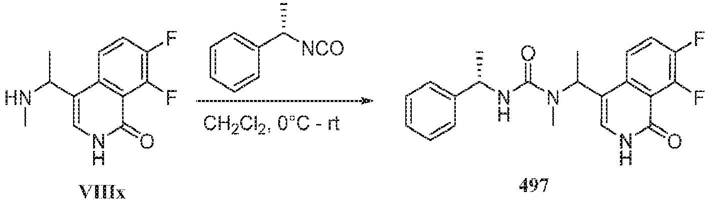

Diastereomeric 1-(1-(7,8-difluoro-1-oxo-1,2-dihydroisoquinolin-4-yl)ethyl)-1-methyl-3-((S)-1-phenylethyl)urea was synthesized in a similar manner as described above from 7,8-difluoro-4-(1-(methylamino)ethyl)isoquinolin-1(*2H*)-one **(VIIIx)** and (S)-(1-isocyanatoethyl)benzene. The diastereoisomers were subsequently separated by chiral SFC, Column: Chiralpak IG (250 × 30 mm) 5 µ, 65% CO₂/MeOH, Flow rate 90 g/min.

1-(1-(7,8-Difluoro-1-oxo-1,2-dihydroisoquinolin-4-yl)ethyl)-1-methyl-3-((S)-1-phenylethyl)urea - Diastereomer I **(Compound 487),** LCMS: *m*/*z* found 386.3 [M+H]⁺, RT = 3.43 min (Method A); ¹H NMR (400 MHz, DMSO-d₆): δ 11.50 (bs, 1H), 7.20-7.44 (m, 7H), 7.06 (s, 1H), 6.53 (bd, 1H), 5.64-5.69 (m, 1H), 4.91-4.95 (m, 1H), 2.45 (s, 3H), 1.40 (d, 3H), 1.33 (d, 3H); Chiral analytical SFC: RT = 2.32 min, Column: Chiralpak IG-3, (4.6 × 150 mm) 3 µm, 75% CO₂/MeOH, Flow = 3.0 g/min.

1-(1-(7,8-Difluoro-1-oxo-1,2-dihydroisoquinolin-4-yl)ethyl)-1-methyl-3-((S)-1-phenylethyl)urea - Diastereomer II. LCMS: *m*/*z* found 386.3 [M+H]⁺, RT = 3.42 min (Method A); ¹H NMR (400 MHz, DMSO-d₆): δ 11.50 (bs, 1H), 7.20-7.44 (m, 7H), 7.06 (s, 1H), 6.53 (bd, 1H), 5.64-5.69 (m, 1H), 4.91-4.95 (m, 1H), 2.45 (s, 3H), 1.40 (d, 3H), 1.33 (d, 3H); Chiral analytical SFC: RT = 3.90 min, Column: Chiralpak IG-3, (4.6 × 150 mm) 3 µm, 75% CO₂/MeOH, Flow = 3.0 g/min.

### 1-(1-(7,8-Difluoro-1-oxo-1,2-dihydroisoquinolin-4-yl)ethyl)-1-methyl-3-((R)-1-phenylethyl)urea (Compound 488)

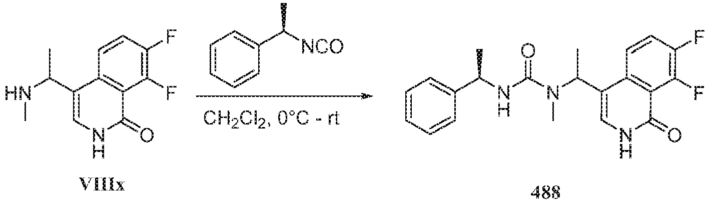

Diastereomeric 1-(1-(7,8-difluoro-1-oxo-1,2-dihydroisoquinolin-4-yl)ethyl)-1-methyl-3-((R)-1-phenylethyl)urea was synthesized in a similar manner as described above from 7,8-difluoro-4-(l-(methylamino)ethyl)isoquinolin-1(*2H*)-one **(VIIIx)** and (R)-(1-isocyanatoethyl)benzene. The diastereoisomers were subsequently separated by chiral SFC, Column: Chiralpak IC (250 × 30 mm) 5 µ, 60% CO₂/MeOH, Flow rate 90 g/min.

1-(1-(7,8-Difluoro-1-oxo-1,2-dihydroisoquinolin-4-yl)ethyl)-1-methyl-3-((R)-1-phenylethyl)urea - Diastereomer I. LCMS: *m*/*z* found 386.3 [M+H]⁺, RT = 3.43 min (Method A); ¹H NMR (400 MHz, DMSO-d₆): δ 11.50 (bs, 1H), 7.20-7.43 (m, 7H), 7.07 (s, 1H), 6.53 (bd, 1H), 5.64-5.69 (m, 1H), 4.89-4.96 (m, 1H), 2.45 (s, 3H), 1.40 (d, 3H), 1.33 (d, 3H); Chiral analytical SFC: RT = 1.67 min, Column: Chiralpak IC-3, (4.6 × 150 mm) 3 µm, 60% CO₂/MeOH, Flow = 3.0 g/min.

1-(1-(7,8-Difluoro-1-oxo-1,2-dihydroisoquinolin-4-yl)ethyl)-1-methyl-3-((R)-1 - phenylethyl)urea - Diastereomer II **(Compound 488),** LCMS: *m*/*z* found 386.3 [M+H]⁺, RT = 3.43 min (Method A); ¹H NMR (400 MHz, DMSO-d₆): δ 11.50 (bs, 1H), 7.20-7.43 (m, 7H), 7.07 (s, 1H), 6.53 (bd, 1H), 5.64-5.69 (m, 1H), 4.89-4.96 (m, 1H), 2.45 (s, 3H), 1.40 (d, 3H), 1.33 (d, 3H); Chiral analytical SFC: RT = 4.48 min, Column: Chiralpak IC-3, (4.6 × 150 mm) 3 µm, 60% CO₂/MeOH, Flow = 3.0 g/min.

### 1-(1-(7,8-Difluoro-1-oxo-1,2-dihydroisoquinolin-4-yl)ethyl)-3-(3-fluorophenyl)-1-methylurea (Compounds 455 & 456)

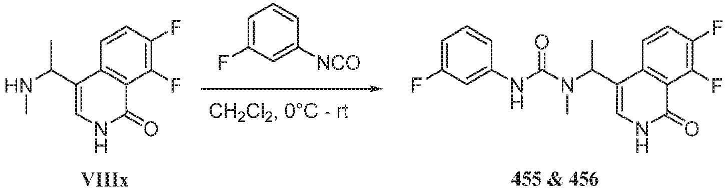

Racemic 1-(1-(7,8-difluoro-1-oxo-1,2-dihydroisoquinolin-4-yl)ethyl)-3-(3-fluorophenyl)-1-methylurea was synthesized in a similar manner as described above from 7,8-difluoro-4-(1-(methylamino)ethyl)isoquinolin-1(*2H*)-one **(VIIIx)** and 1-fluoro-3-isocyanatobenzene. The enantiomers were subsequently separated by chiral SFC, Column: Chiralpak IG (250 × 30 mm) 5 µ, 50% CO₂/MeOH, Flow rate 100 g/min.

1-(1-(7,8-Difluoro-1-oxo-1,2-dihydroisoquinolin-4-yl)ethyl)-3-(3-fluorophenyl)-1-methylurea - Enantiomer I **(Compound 455),** LCMS: *m*/*z* found 376.3 [M+H]⁺, RT = 3.52 min (Method A); ¹H NMR (400 MHz, DMSO-d₆): δ 11.50 (bs, 1H), 8.45 (bs, 1H), 7.84-7.91 (m, 1H), 7.50-7.56 (m, 2H), 7.23-7.34 (m, 2H), 7.15 (s, 1H), 6.74-6.79 (m, 1H), 5.76-5.82 (m, 1H), 2.59 (s, 3H), 1.41 (s, 3H); Chiral analytical SFC: RT = 1.05 min, Column: Chiralpak IG-3, (4.6 × 150 mm) 3 µm, 60% CO₂/MeOH, Flow = 3.0 g/min.

1-(1-(7,8-Difluoro-1-oxo-1,2-dihydroisoquinolin-4-yl)ethyl)-3-(3-fluorophenyl)-1-methylurea - Enantiomer II **(Compound 456),** LCMS: *m*/*z* found 376.3 [M+H]⁺, RT = 3.52 min (Method A); ¹H NMR (400 MHz, DMSO-d₆): δ 11.50 (bs, 1H), 8.45 (bs, 1H), 7.84-7.91 (m, 1H), 7.50-7.56 (m, 2H), 7.23-7.34 (m, 2H), 7.15 (s, 1H), 6.74-6.79 (m, 1H), 5.76-5.82 (m, 1H), 2.59 (s, 3H), 1.41 (s, 3H); Chiral analytical SFC: RT = 2.71 min, Column: Chiralpak IG-3, (4.6 × 150 mm) 3 µm, 60% CO₂/MeOH, Flow = 3.0 g/min.

### 1-(1-(7,8-Difluoro-1-oxo-1,2-dihydroisoquinolin-4-yl)ethyl)-3-(2-fluorophenyl)-1-methylurea (Compound 457)

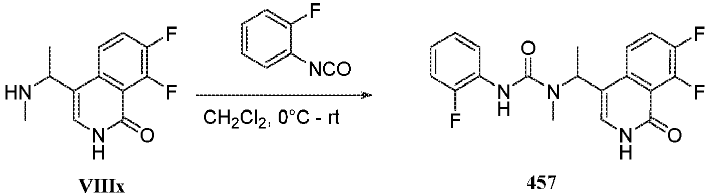

Racemic 1-(1-(7,8-difluoro-1-oxo-1,2-dihydroisoquinolin-4-yl)ethyl)-3-(2-fluorophenyl)-1-methylurea was synthesized in a similar manner as described above from 7,8-difluoro-4-(1-(methylamino)ethyl)isoquinolin-1(*2H*)-one **(VIIIx)** and 2-fluorophenyl isocyanate. The enantiomers were subsequently separated by chiral SFC, Column: Chiralpak IC (250 × 30 mm) 5 µ, 70% CO₂/MeOH, Flow rate 100 g/min.

1-(1-(7,8-Difluoro-1-oxo-1,2-dihydroisoquinolin-4-yl)ethyl)-3-(2-fluorophenyl)-1-methylurea - Enantiomer I. LCMS: *m*/*z* found 376.3 [M+H]⁺, RT = 3.14 min (Method A); ¹H NMR (400 MHz, DMSO-d₆): δ 11.50 (bs, 1H), 8.06 (bs, 1H), 7.78-7.83 (m, 1H), 7.58-7.61 (m, 1H), 7.48-7.52 (m, 1H), 7.12-7.23 (m, 4H), 5.73-5.78 (m, 1H), 2.58 (s, 3H), 1.41 (d, 3H); Chiral analytical SFC: RT = 2.79 min, Column: Chiralpak IC-3, (4.6 × 150 mm) 3 µm, 60% CO₂/MeOH, Flow = 3.0 g/min.

1-(1-(7,8-Difluoro-1-oxo-1,2-dihydroisoquinolin-4-yl)ethyl)-3-(2-fluorophenyl)-1-methylurea - Enantiomer II **(Compound 457),** LCMS: *m*/*z* found 376.3 [M+H]⁺, RT = 3.14 min (Method A); ¹H NMR (400 MHz, DMSO-d₆): δ 11.50 (bs, 1H), 8.06 (bs, 1H), 7.78-7.83 (m, 1H), 7.58-7.61 (m, 1H), 7.48-7.52 (m, 1H), 7.12-7.23 (m, 4H), 5.73-5.78 (m, 1H), 2.58 (s, 3H), 1.41 (d, 3H); Chiral analytical SFC: RT = 3.46 min, Column: Chiralpak IC-3, (4.6 × 150 mm) 3 µm, 60% CO₂/MeOH, Flow = 3.0 g/min.

### 3-(3-Chlorophenyl)-1-(1-(7,8-difluoro-1-oxo-1,2-dihydroisoquinolin-4-yl)ethyl)-1-methylurea (Compounds 460 & 461)

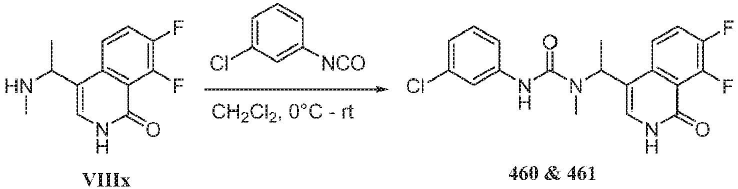

Racemic 3-(3-chlorophenyl)-1-(1-(7,8-difluoro-1-oxo-1,2-dihydroisoquinolin-4-yl)ethyl)-1-methylurea was synthesized in a similar manner as described above from 7,8-difluoro-4-(1-(methylamino)ethyl)isoquinolin-1(*2H*)-one **(VIIIx)** and 3-chlorophenyl isocyanate. The enantiomers were subsequently separated by chiral SFC, Column: Chiralpak IC (250 × 30 mm) 5 µ, 70% CO₂/MeOH, Flow rate 100 g/min.

3-(3-Chlorophenyl)-1-(1-(7,8-difluoro-1-oxo-1,2-dihydroisoquinolin-4-yl)ethyl)-1-methylurea - Enantiomer I **(Compound 460),** LCMS: *m*/*z* found 392.2/394.2 [M+H]⁺, RT = 3.94 min (Method A); ¹H NMR (400 MHz, DMSO-d₆): δ 11.50 (bs, 1H), 8.43 (bs, 1H), 7.84-7.91 (m, 1H), 7.75-7.76 (m, 1H), 7.46-7.53 (m, 2H), 7.27 (t, 1H), 7.15 (s, 1H), 6.99-7.01 (m, 1H), 5.76-5.81 (m, 1H), 2.59 (s, 3H), 1.41 (s, 3H); Chiral analytical SFC: RT = 1.24 min, Column: Chiralpak IG-3, (4.6 × 150 mm) 3 µm, 60% CO₂/MeOH, Flow = 3.0 g/min.

3-(3-Chlorophenyl)-1-(1-(7,8-difluoro-1-oxo-1,2-dihydroisoquinolin-4-yl)ethyl)-1-methylurea - Enantiomer II **(Compound 461),** LCMS: *m*/*z* found 392.2/394.2 [M+H]⁺, RT = 3.94 min (Method A); ¹H NMR (400 MHz, DMSO-d₆): δ 11.50 (bs, 1H), 8.43 (bs, 1H), 7.84-7.91 (m, 1H), 7.75-7.76 (m, 1H), 7.46-7.53 (m, 2H), 7.27 (t, 1H), 7.15 (s, 1H), 6.99-7.01 (m, 1H), 5.76-5.81 (m, 1H), 2.59 (s, 3H), 1.41 (s, 3H); Chiral analytical SFC: RT = 2.50 min, Column: Chiralpak IG-3, (4.6 × 150 mm) 3 µm, 60% CO₂/MeOH, Flow = 3.0 g/min.

### Phenyl 2,3-difluorophenylcarbamate

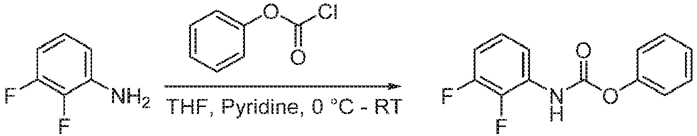

To a solution of 0.5 g (3.87 mmol, 1.0 eq.) of 2,3-difluoroaniline in 5 mL of THF at 0 °C under a nitrogen atmosphere was added 1.2 mL (15.49 mmol, 4.0 eq.) of pyridine followed by 0.53 mL (4.22 mmol, 1.1 eq.) of phenyl chloroformate. The mixture was allowed to warm to room temperature and stirred for 2 h. The mixture was then diluted with 30 mL of water and extracted with 3 × 100 mL of ethyl acetate. The combined organic extracts were washed with 50 mL of brine, dried (Na₂SO₄), filtered and the solvent was removed *in vacuo.* The residue was purified by chromatography (SiO₂, eluting with 30% ethyl acetate in petroleum ether) to provide 0.38 g (1.52 mmol, 39%) of phenyl 2,3-difluorophenylcarbamate. LCMS: *m*/*z* found 250.0 [M+H]⁺; ¹H NMR (400 MHz, CDCl₃): δ 7.88-7.93 (bt, 1H), 7.39-7.43 (m, 2H), 7.24-7.32 (m, 2H), 7.17-7.22 (m, 2H), 7.06-7.11 (m, 1H), 6.87-693 (m, 1H).

### 1-(1-(7,8-Difluoro-1-oxo-1,2-dihydroisoquinolin-4-yl)ethyl)-3-(2,3-difluorophenyl)-1-methylurea (Compounds 458 & 459)

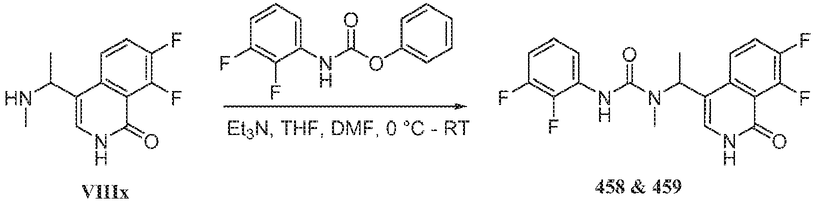

To a solution of 0.12 g (0.50 mmol, 1.0 eq.) of 7,8-difluoro-4-(1-(methylamino)ethyl)isoquinolin-1(*2H*)-one **(VIIIx)** in 4 mL of 3: 1 *v*/*v* THF:DMF mixture at 0 °C under a nitrogen atmosphere was added 0.22 mL (1.51 mmol, 3.0 eq.) of triethylamine followed by 0.13 g (0.50 mmol, 1.0 eq.) of phenyl (2,3-difluorophenyl)carbamate. The mixture was allowed to warm to room temperature and stirred for 16 h. The solvent was removed *in vacuo* and the residue was purified by reverse phase semi-preparative HPLC to provide 100 mg (0.25 mmol, 50%) of racemicl-(1-(7,8-difluoro-1-oxo-1,2-dihydroisoquinolin-4-yl)ethyl)-3-(2,3-difluorophenyl)-1-methylurea. LCMS: *m*/*z* found 394.1 [M+H]⁺, RT = 1.73 min. The diastereoisomers were subsequently separated by chiral SFC, Column: Chiralpak IC (250 × 30 mm) 5 µ, 80% CO₂/MeOH, Flow rate 90 g/min.

1-(1-(7,8-Difluoro-1-oxo-1,2-dihydroisoquinolin-4-yl)ethyl)-3-(2,3-difluorophenyl)-1-methylurea - Enantiomer I **(Compound 458),** LCMS: *m*/*z* found 394.3 [M+H]⁺, RT = 3.37 min (Method A); ¹H NMR (400 MHz, DMSO-d₆): δ 11.41 (bs, 1H), 8.47 (bs, 1H), 7.79-7.85 (m, 1H), 7.55-7.58 (m, 1H), 7.26-7.30 (m, 1H), 7.10-7.20 (m, 3H), 5.73-5.77 (m, 1H), 2.59 (s, 3H), 1.42 (d, 3H); Chiral analytical SFC: RT = 3.24 min, Column: Chiralpak AD-3, (4.6 × 150 mm) 3 µm, 85% CO₂/MeOH, Flow = 3.0 g/min.

1-(1-(7,8-Difluoro-1-oxo-1,2-dihydroisoquinolin-4-yl)ethyl)-3-(2,3-difluorophenyl)-1-methylurea - Enantiomer II **(Compound 459),** LCMS: *m*/*z* found 394.3 [M+H]⁺, RT = 3.37 min (Method A); ¹H NMR (400 MHz, DMSO-d₆): δ 11.41 (bs, 1H), 8.47 (bs, 1H), 7.79-7.85 (m, 1H), 7.55-7.58 (m, 1H), 7.26-7.30 (m, 1H), 7.10-7.20 (m, 3H), 5.73-5.77 (m, 1H), 2.59 (s, 3H), 1.42 (d, 3H); Chiral analytical SFC: RT = 3.85 min, Column: Chiralpak AD-3, (4.6 × 150 mm) 3 µm, 85% CO₂/MeOH, Flow = 3.0 g/min.

### 1-(1-(7,8-Difluoro-1-oxo-1,2-dihydroisoquinolin-4-yl)ethyl)-1-methyl-3-(pyridin-4-yl)urea (Compound 449)

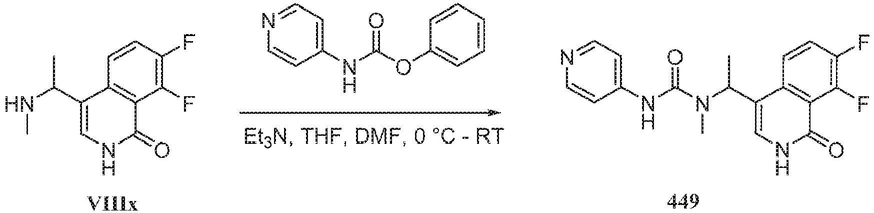

Racemic 1-(1-(7,8-difluoro-1-oxo-1,2-dihydroisoquinolin-4-yl)ethyl)-1-methyl-3-(pyridin-4-yl)urea was synthesized in a similar manner as described above from 7,8-difluoro-4-(1-(methylamino)ethyl)isoquinolin-1(*2H*)-one **(VIIIx)** and phenyl pyridin-4-ylcarbamate. The enantiomers were subsequently separated by chiral SFC, Column: Lux Cellulose-2 (250 × 30 mm) 5 µ, 50% CO₂/MeOH, Flow rate 90 g/min.

1-(1-(7,8-Difluoro-1-oxo-1,2-dihydroisoquinofin-4-yl)ethyl)-1-methyl-3-(pyridin-4-yl)urea - Enantiomer I. LCMS: *m*/*z* found 359.1 [M+H]⁺, RT = 1.63 min (Method A); ¹H NMR (400 MHz, DMSO-d₆): δ 11.52 (bs, 1H), 8.69 (bs, 1H), 8.32-8.34 (m, 2H), 7.85-7.91 (m, 1H), 7.56-7.57 (m, 2H), 7.47-7.50 (m, 1H), 7.16 (s, 1H), 5.75-5.81 (m, 1H), 2.61 (d, 3H), 1.43 (d, 3H); Chiral analytical SFC: RT = 2.04 min, Column: Chiralcel OZ-3, (4.6 × 150 mm) 3 µm, 60% CO₂/MeOH, Flow = 3.0 g/min.

1-(1-(7,8-Difluoro-1-oxo-1,2-dihydroisoquinofin-4-yl)ethyl)-1-methyl-3-(pyridin-4-yl)urea - Enantiomer II **(Compound 449),** LCMS: *m*/*z* found 359.1 [M+H]⁺, RT = 1.62 min (Method A); ¹H NMR (400 MHz, DMSO-d₆): δ 11.52 (bs, 1H), 8.69 (bs, 1H), 8.32-8.34 (m, 2H), 7.85-7.91 (m, 1H), 7.56-7.57 (m, 2H), 7.47-7.50 (m, 1H), 7.16 (s, 1H), 5.75-5.81 (m, 1H), 2.61 (d, 3H), 1.43 (d, 3H); Chiral analytical SFC: RT = 4.03 min, Column: Chiralcel OZ-3, (4.6 × 150 mm) 3 µm, 60% CO₂/MeOH, Flow = 3.0 g/min.

### 1-(1-(7,8-Difluoro-1-oxo-1,2-dihydroisoquinolin-4-yl)ethyl)-1-methyl-3-(pyridin-3-yl)urea (Compound 450)

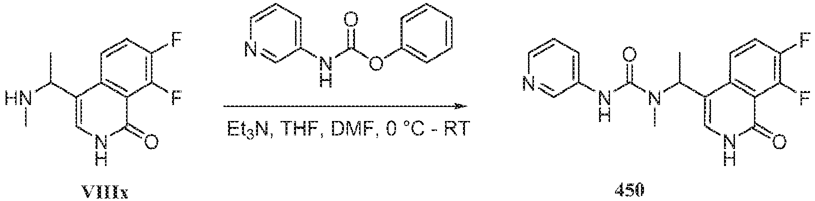

Racemic 1-(1-(7,8-difluoro-1-oxo-1,2-dihydroisoquinolin-4-yl)ethyl)-1-methyl-3-(pyridin-3-yl)urea was synthesized in a similar manner as described above from 7,8-difluoro-4-(l-(methylamino)ethyl)isoquinolin-1(*2H*)-one **(VIIIx)** and phenyl pyridin-3-ylcarbamate. The enantiomers were subsequently separated by chiral SFC, Column: Chiralpak IC (250 × 30 mm) 5 µ, 60% CO₂/MeOH, Flow rate 100 g/min.

1-(1-(7,8-Difluoro-1-oxo-1,2-dihydroisoquinofin-4-yl)ethyl)-1-methyl-3-(pyridin-3-yl)urea - Enantiomer I, LCMS: *m*/*z* found 359.2 [M+H]⁺, RT = 1.52 min (Method A); ¹H NMR (400 MHz, DMSO-d₆): δ 11.51 (bs, 1H), 8.71 (d, 1H), 8.47 (bs, 1H), 8.17-8.18 (m, 1H), 7.94-7.97 (m, 1H), 7.85-7.91 (m, 1H), 7.52-7.55 (m, 1H), 7.27-7.31 (m, 1H), 7.16 (s, 1H), 5.77-5.83 (m, 1H), 2.60 (s, 3H), 1.42 (d, 3H); Chiral analytical SFC: RT = 3.03 min, Column: Chiralpak IC, (4.6 × 150 mm) 3 µm, 60% CO₂/MeOH, Flow = 3.0 g/min.

1-(1-(7,8-Difluoro-1-oxo-1,2-dihydroisoquinofin-4-yl)ethyl)-1-methyl-3-(pyridin-3-yl)urea - Enantiomer II **(Compound 450),** LCMS: *m*/*z* found 359.2 [M+H]⁺, RT = 1.52 min (Method A); ¹H NMR (400 MHz, DMSO-d₆): δ 11.51 (bs, 1H), 8.71 (d, 1H), 8.47 (bs, 1H), 8.17-8.18 (m, 1H), 7.94-7.97 (m, 1H), 7.85-7.91 (m, 1H), 7.52-7.55 (m, 1H), 7.27-7.31 (m, 1H), 7.16 (s, 1H), 5.77-5.83 (m, 1H), 2.60 (s, 3H), 1.42 (d, 3H); Chiral analytical SFC: RT = 4.49 min, Column: Chiralpak IC, (4.6 × 150 mm) 3 µm, 60% CO₂/MeOH, Flow = 3.0 g/min.

### 3-(3,5-Dichloro-4-fluorophenyl)-1-(1-(7,8-difluoro-1-oxo-1,2-dihydroisoquinolin-4-yl)ethyl)-1-methylurea (Compounds 451 & 452)

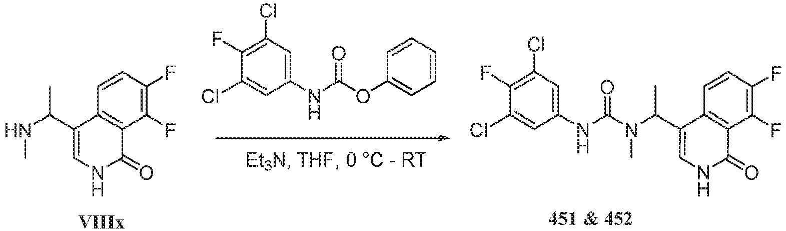

Racemic 3-(3,5-dichloro-4-fluorophenyl)-1-(1-(7,8-difluoro-1-oxo-1,2-dihydroisoquinolin-4-yl)ethyl)-1-methylurea was synthesized in a similar manner as described above from 7,8-difluoro-4-(1-(methylamino)ethyl)isoquinolin-1(*2H*)-one **(VIIIx)** and phenyl 3,5-dichloro-4-fluorophenylcarbamat. The enantiomers were subsequently separated by chiral SFC, Column: Chiralpak IG (250 × 30 mm) 5 µ, 65% CO₂/MeOH, Flow rate 100 g/min.

3-(3,5-Dichloro-4-fluorophenyl)-1-(1-(7,8-difluoro-1-oxo-1,2-dihydroisoquinolin-4-yl)ethyl)-1-methylurea- Enantiomer I **(Compound 451),** LCMS: *m*/*z* found 444.1/446.1/448.1 [M+H]⁺, RT = 4.76 min (Method A); ¹H NMR (400 MHz, DMSO-d₆): δ 11.51 (bs, 1H), 8.60 (bs, 1H), 7.85-7.92 (m, 1H), 7.82 (d, 2H), 7.45-7.49 (m, 1H), 7.15 (s, 1H), 5.74-5.79 (m, 1H), 2.58 (s, 3H), 1.41 (d, 3H); Chiral analytical SFC: RT = 1.69 min, Column: Chiralpak IG, (4.6 × 150 mm) 5 µm, 60% CO₂/MeOH, Flow = 3.0 g/min.

3-(3,5-Dichloro-4-fluorophenyl)-1-(1-(7,8-difluoro-1-oxo-1,2-dihydroisoquinolin-4-yl)ethyl)-1-methylurea - Enantiomer II **(Compound 452),** LCMS: *m*/*z* found 444.1/446.1/448.1 [M+H]⁺, RT = 4.76 min (Method A); ¹H NMR (400 MHz, DMSO-d₆): δ 11.51 (bs, 1H), 8.60 (bs, 1H), 7.85-7.92 (m, 1H), 7.82 (d, 2H), 7.45-7.49 (m, 1H), 7.15 (s, 1H), 5.74-5.79 (m, 1H), 2.58 (s, 3H), 1.41 (d, 3H); Chiral analytical SFC: RT = 4.13 min, Column: Chiralpak IG, (4.6 × 150 mm) 5 µm, 60% CO₂/MeOH, Flow = 3.0 g/min.

### 3-(2-Chloropyridin-4-yl)-1-(1-(7,8-difluoro-1-oxo-1,2-dihydroisoquinolin-4-yl)ethyl)-1-methylurea (Compound 454)

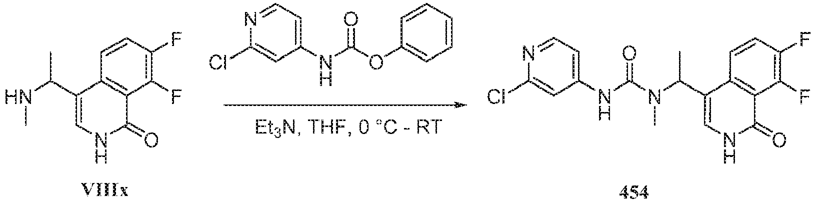

Racemic 3-(2-chloropyridin-4-yl)-1-(1-(7,8-difluoro-1-oxo-1,2-dihydroisoquinolin-4-yl)ethyl)-1-methylurea was synthesized in a similar manner as described above from 7,8-difluoro-4-(1-(methylamino)ethyl)isoquinolin-1(*2H*)-one **(VIIIx)** and phenyl 2-chloropyridin-4-ylcarbamate. The enantiomers were subsequently separated by chiral SFC, Column: Chiralpak IG (250 × 30 mm) 5 µ, 60% CO₂/MeOH, Flow rate 90 g/min.

3-(2-Chloropyridin-4-yl)-1-(1-(7,8-difluoro-1-oxo-1,2-dihydroisoquinolin-4-yl)ethyl)-1-methylurea - Enantiomer I. LCMS: *m*/*z* found 393.2/395.2 [M+H]⁺, RT = 2.88 min (Method A); ¹H NMR (400 MHz, DMSO-d₆): δ 10.11 (bs, 2H), 8.15 (d, 1H), 7.85-7.91 (m, 1H), 7.76 (d, 1H), 7.51-7.53 (m, 1H), 7.43-7.47 (m, 1H), 7.16 (s, 1H), 5.73-5.78 (m, 1H), 2.60 (s, 3H), 1.43 (d, 3H); Chiral analytical SFC: RT = 1.45 min, Column: Chiralpak IG-3, (4.6 × 150 mm) 3 µm, 60% CO₂/MeOH, Flow = 3.0 g/min.

3-(2-Chloropyridin-4-yl)-1-(1-(7,8-difluoro-1-oxo-1,2-dihydroisoquinolin-4-yl)ethyl)-1-methylurea - Enantiomer II **(Compound 454),** LCMS: *m*/*z* found 393.2/395.2 [M+H]⁺, RT = 2.88 min (Method A); ¹H NMR (400 MHz, DMSO-d₆): δ 10.11 (bs, 2H), 8.15 (d, 1H), 7.85-7.91 (m, 1H), 7.76 (d, 1H), 7.51-7.53 (m, 1H), 7.43-7.47 (m, 1H), 7.16 (s, 1H), 5.73-5.78 (m, 1H), 2.60 (s, 3H), 1.43 (d, 3H); Chiral analytical SFC: RT = 4.35 min, Column: Chiralpak IG-3, (4.6 × 150 mm) 3 µm, 60% CO₂/MeOH, Flow = 3.0 g/min.

### Phenyl (3-cyano-4-fluorophenyl)carbamate

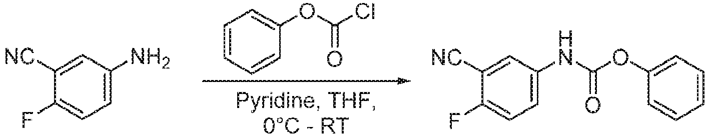

To a solution of 1.0 g (7.35 mmol, 1.0 eq.) of 5-amino-2-fluorobenzonitrile in 10 mL of THF at 0 °C under a nitrogen atmosphere was added 0.85 mL (11.02 mmol, 1.5 eq.) of pyridine followed by 0.8 mL (6.6 mmol, 0.9 eq.) of phenyl chloroformate. The mixture was allowed to warm to room temperature and the stirred for 2 h. The mixture was diluted with 30 mL of water and extracted with 2 × 100 mL of ethyl acetate. The combined organic extracts were washed with 20 mL of water, 20 mL of brine, dried (Na₂SO₄), filtered and the solvent was removed *in vacuo.* The residue was triturated with 10 mL of water, filtered the resulting solid was dried under high vacuum to provide 1.3 g (5.07 mmol, 68%) of phenyl (3-cyano-4-fluorophenyl)carbamate. LCMS: *m*/*z* found 257.10 [M+H]⁺; ¹H NMR (400 MHz, CDCl₃): δ 7.78-7.82 (m, 1H), 7.63-7.66 (m, 1H), 7.41 (t, 2H), 7.23-7.30 (m, 2H), 7.18 (d, 2H), 7.01 (bs, 1H).

### 3-(3-Cyano-4-fluorophenyl)-1-(1-(7,8-difluoro-1-oxo-1,2-dihydroisoquinolin-4-yl)ethyl)-1-methylurea (Compounds 470 & 471)

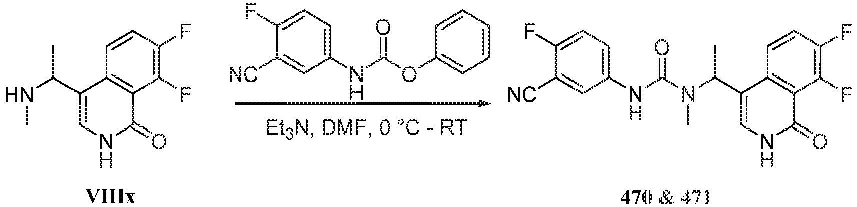

Racemic 3-(3-cyano-4-fluorophenyl)-1-(1-(7,8-difluoro-1-oxo-1,2-dihydroisoquinolin-4-yl)ethyl)-1-methylurea was synthesized in a similar manner as described above from 7,8-difluoro-4-(1-(methylamino)ethyl)isoquinolin-1(*2H*)-one **(VIIIx)** and phenyl (3-cyano-4-fluorophenyl)carbamate. The enantiomers were subsequently separated by chiral SFC, Column: Chiralpak IF (250 × 30 mm) 5 µ, 70% CO₂/MeOH, Flow rate 100 g/min.

3-(3-Cyano-4-fluorophenyl)-1-(1-(7,8-difluoro-1-oxo-1,2-dihydroisoquinolin-4-yl)ethyl)-1-methylurea- Enantiomer I **(Compound 470),** LCMS: *m*/*z* found 401.3 [M+H]⁺, RT 3.42 min (Method A); ¹H NMR (400 MHz, DMSO-d₆): δ 11.52 (bs, 1H), 8.64 (bs, 1H), 8.05-8.07 (m, 1H), 7.83-7.92 (m, 2H), 7.49-7.53 (m, 1H), 7.44 (t, 1H), 7.16 (s, 1H), 5.75-5.80 (m, 1H), 2.59 (s, 3H), 1.42 (d, 3H); Chiral analytical SFC: RT = 2.61 min, Column: Chiralpak IF, (4.6 × 250 mm) 5 µm, 60% CO₂/MeOH, Flow = 3.0 g/min.

3-(3-Cyano-4-fluorophenyl)-1-(1-(7,8-difluoro-1-oxo-1,2-dihydroisoquinolin-4-yl)ethyl)-1-methylurea - Enantiomer II **(Compound 471),** LCMS: *m*/*z* found 401.3 [M+H]⁺, RT 3.42 min (Method A); ¹H NMR (400 MHz, DMSO-d₆): δ 11.52 (bs, 1H), 8.64 (bs, 1H), 8.05-8.07 (m, 1H), 7.83-7.92 (m, 2H), 7.49-7.53 (m, 1H), 7.44 (t, 1H), 7.16 (s, 1H), 5.75-5.80 (m, 1H), 2.59 (s, 3H), 1.42 (d, 3H); Chiral analytical SFC: RT = 4.79 min, Column: Chiralpak IF, (4.6 × 250 mm) 5 µm, 60% CO₂/MeOH, Flow = 3.0 g/min.

### 1-(1-(7,8-Difluoro-1-oxo-1,2-dihydroisoquinolin-4-yl)ethyl)-3-(3,4-difluorobenzyl)-1-methylurea (Compounds 489 & 490)

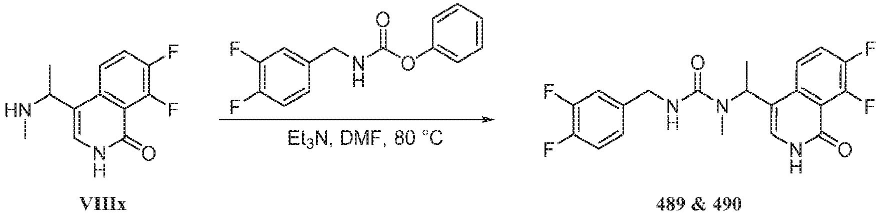

Racemic 1-(1-(7,8-difluoro-1-oxo-1,2-dihydroisoquinolin-4-yl)ethyl)-3-(3,4-difluorobenzyl)-1-methylurea was synthesized in a similar manner as described above from 7,8-difluoro-4-(1-(methylamino)ethyl)isoquinolin-1(*2H*)-one **(VIIIx)** and phenyl (3,4-difluorobenzyl)carbamate. The enantiomers were subsequently separated by chiral SFC, Column: Chiralpak IC (250 × 30 mm) 5 µ, 80% CO₂/MeOH, Flow rate 90 g/min.

1-(1-(7,8-Difluoro-1-oxo-1,2-dihydroisoquinolin-4-yl)ethyl)-3-(3,4-difluorobenzyl)-1-methylurea - Enantiomer I **(Compound 489),** LCMS: *m*/*z* found 408.2 [M+H]⁺, RT 3.45 min (Method A); ¹H NMR (400 MHz, DMSO-d₆): δ 11.48 (bs, 1H), 7.66-7.73 (m, 1H), 7.52-7.55 (m, 1H), 7.36-7.43 (m, 1H), 7.22-7.28 (m, 1H), 7.09-7.12 (m, 2H), 6.98 (bt, 1H), 5.75-5.70 (m, 1H), 4.30-4.36 (m, 1H), 4.20-4.26 (m, 1H), 2.43 (s, 3H), 1.34 (d, 3H); Chiral analytical SFC: RT = 4.41 min, Column: Chiralpak IC-3, (4.6 × 150 mm) 3 µm, 60% CO₂/MeOH, Flow = 3.0 g/min.

1-(1-(7,8-Difluoro-1-oxo-1,2-dihydroisoquinolin-4-yl)ethyl)-3-(3,4-difluorobenzyl)-1-methylurea - Enantiomer II **(Compound 490),** LCMS: *m*/*z* found 408.2 [M+H]⁺, RT 3.45 min (Method A); ¹H NMR (400 MHz, DMSO-d₆): δ 11.48 (bs, 1H), 7.66-7.73 (m, 1H), 7.52-7.55 (m, 1H), 7.36-7.43 (m, 1H), 7.22-7.28 (m, 1H), 7.09-7.12 (m, 2H), 6.98 (bt, 1H), 5.75-5.70 (m, 1H), 4.30-4.36 (m, 1H), 4.20-4.26 (m, 1H), 2.43 (s, 3H), 1.34 (d, 3H); Chiral analytical SFC: RT = 5.35 min, Column: Chiralpak IC-3, (4.6 × 150 mm) 3 µm, 60% CO₂/MeOH, Flow = 3.0 g/min.

### 1-(1-(7,8-Difluoro-1-oxo-1,2-dihydroisoquinolin-4-yl)ethyl)-3-(4-fluorobenzyl)-1-methylurea (Compounds 491 & 492)

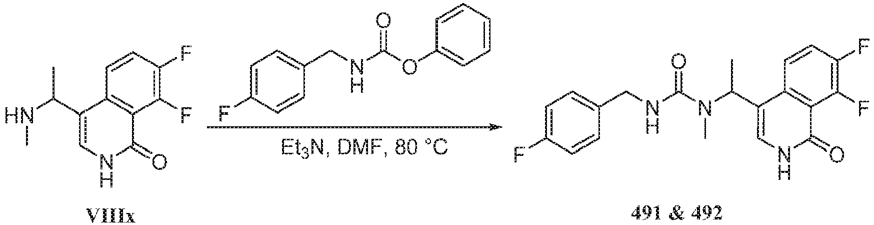

Racemic 1-(1-(7,8-difluoro-1-oxo-1,2-dihydroisoquinolin-4-yl)ethyl)-3-(4-fluorobenzyl)-1-methylurea was synthesized in a similar manner as described above from 7,8-difluoro-4-(1-(methylamino)ethyl)isoquinolin-1(*2H*)-one **(VIIIx)** and phenyl (4-fluorobenzyl)carbamate. The enantiomers were subsequently separated by chiral SFC, Column: Chiralpak IC (250 × 30 mm) 5 µ, 80% CO₂/MeOH, Flow rate 90 g/min.

1-(1-(7,8-Difluoro-1-oxo-1,2-dihydroisoquinolin-4-yl)ethyl)-3-(4-fluorobenzyl)-1-methylurea - Enantiomer I **(Compound 491),** LCMS: *m*/*z* found 390.2 [M+H]⁺, RT = 3.25 min (Method A); ¹H NMR (400 MHz, DMSO-d₆): δ 11.50 (bs, 1H), 7.70-7.77 (m, 1H), 7.52-7.55 (m, 1H), 7.28-7.31 (m, 2H), 7.13-7.18 (m, 2H), 7.08 (s, 1H), 6.94 (bt, 1H), 5.70-5.75 (m, 1H), 4.21-4.36 (m, 2H), 2.42 (s, 3H), 1.34 (d, 3H); Chiral analytical SFC: RT = 3.48 min, Column: Chiralpak IC-3, (4.6 × 150 mm) 3 µm, 60% CO₂/MeOH, Flow = 3.0 g/min.

1-(1-(7,8-Difluoro-1-oxo-1,2-dihydroisoquinolin-4-yl)ethyl)-3-(4-fluorobenzyl)-1-methylurea - Enantiomer II **(Compound 492),** LCMS: *m*/*z* found 390.2 [M+H]⁺, RT = 3.26 min (Method A); ¹H NMR (400 MHz, DMSO-d₆): δ 11.50 (bs, 1H), 7.70-7.77 (m, 1H), 7.52-7.55 (m, 1H), 7.28-7.31 (m, 2H), 7.13-7.18 (m, 2H), 7.08 (s, 1H), 6.94 (bt, 1H), 5.70-5.75 (m, 1H), 4.21-4.36 (m, 2H), 2.42 (s, 3H), 1.34 (d, 3H); Chiral analytical SFC: RT = 4.32 min, Column: Chiralpak IC-3, (4.6 × 150 mm) 3 µm, 60% CO₂/MeOH, Flow = 3.0 g/min.

### 1-(1-(7,8-Difluoro-1-oxo-1,2-dihydroisoquinolin-4-yl)ethyl)-1-methyl-3-(3,4,5-trifluorobenzyl)urea (Compounds 493 & 494)

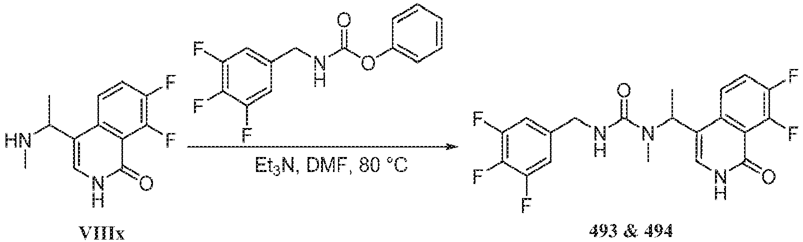

Racemic 1-(1-(7,8-difluoro-1-oxo-1,2-dihydroisoquinolin-4-yl)ethyl)-1-methyl-3-(3,4,5-trifluorobenzyl)urea was synthesized in a similar manner as described above from 7,8-difluoro-4-(1-(methylamino)ethyl)isoquinolin-1(*2H*)-one **(VIIIx)** and phenyl (3,4,5-trifluorobenzyl)carbamate. The enantiomers were subsequently separated by chiral SFC, Column: Chiralpak IC (250 × 30 mm) 5 µ, 80% CO₂/MeOH, Flow rate 60 g/min.

1-(1-(7,8-Difluoro-1-oxo-1,2-dihydroisoquinofin-4-yl)ethyl)-1-methyl-3-(3,4,5-trifluorobenzyl)urea - Enantiomer I **(Compound 493),** LCMS: *m*/*z* found 426.3 [M+H]⁺, RT = 3.70 min (Method A); ¹H NMR (400 MHz, DMSO-d₆): δ 11.46 (bs, 1H), 7.65-7725 (m, 1H), 7.52-7.55 (m, 1H), 7.10-7.16 (m, 3H), 7.01 (bt, 1H), 5.71-5.74 (m, 1H), 4.30-4.36 (m, 1H), 4.19-4.25 (m, 1H), 2.45 (s, 3H), 1.34 (d, 3H); Chiral analytical SFC: RT = 6.02 min, Column: Chiralpak IC-3, (4.6 × 150 mm) 3 µm, 85% CO₂/MeOH, Flow = 3.0 g/min.

1-(1-(7,8-Difluoro-1-oxo-1,2-dihydroisoquinofin-4-yl)ethyl)-1-methyl-3-(3,4,5-trifluorobenzyl)urea - Enantiomer II **(Compound 494),** LCMS: *m*/*z* found 426.3 [M+H]⁺, RT = 3.70 min (Method A); ¹H NMR (400 MHz, DMSO-d₆): δ 11.46 (bs, 1H), 7.65-7725 (m, 1H), 7.52-7.55 (m, 1H), 7.10-7.16 (m, 3H), 7.01 (bt, 1H), 5.71-5.74 (m, 1H), 4.30-4.36 (m, 1H), 4.19-4.25 (m, 1H), 2.45 (s, 3H), 1.34 (d, 3H); Chiral analytical SFC: RT = 7.38 min, Column: Chiralpak IC-3, (4.6 × 150 mm) 3 µm, 85% CO₂/MeOH, Flow = 3.0 g/min.

### 3-(3-Chloro-4-fluorobenzyl)-1-(1-(7,8-difluoro-1-oxo-1,2-dihydroisoquinolin-4-yl)ethyl)-1-methylurea (Compounds 496 & 497)

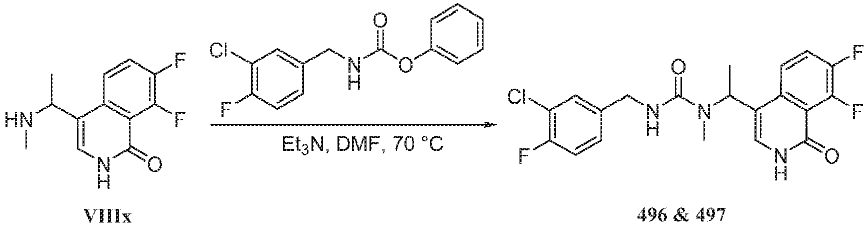

Racemic 3-(3-chloro-4-fluorobenzyl)-1-(1-(7,8-difluoro-1-oxo-1,2-dihydroisoquinolin-4-yl)ethyl)-1-methylurea was synthesized in a similar manner as described above from 7,8-difluoro-4-(l-(methylamino)ethyl)isoquinolin-1(*2H*)-one **(VIIIx)** and phenyl 3-chloro-4-fluorobenzylcarbamate. The enantiomers were subsequently separated by chiral SFC, Column: Chiralpak IC (250 × 30 mm) 5 µ, 80% CO₂/MeOH, Flow rate 70 g/min.

3-(3-Chloro-4-fluorobenzyl)-1-(1-(7,8-difluoro-1-oxo-1,2-dihydroisoquinolin-4-yl)ethyl)-1-methylurea - Enantiomer I **(Compound 496),** LCMS: *m*/*z* found 424.2/426.2 [M+H]⁺, RT = 3.75 min (Method A); ¹H NMR (400 MHz, DMSO-d₆): δ 11.46 (bs, 1H), 7.66-7.73 (m, 1H), 7.51-7.54 (m, 1H), 7.36-7.43 (m, 2H), 7.25-7.29 (m, 1H), 7.09 (s, 1H), 6.99 (bt, 1H), 5.69-5.74 (m, 1H), 4.20-4.36 (m, 2H), 2.43 (s, 3H), 1.34 (d, 3H); Chiral analytical SFC: RT = 3.77 min, Column: Chiralpak IC-3, (4.6 × 150 mm) 3 µm, 75% CO₂/MeOH, Flow = 3.0 g/min.

3-(3-Chloro-4-fluorobenzyl)-1-(1-(7,8-difluoro-1-oxo-1,2-dihydroisoquinolin-4-yl)ethyl)-1-methylurea - Enantiomer II **(Compound 497),** LCMS: *m*/*z* found 424.3/426.3 [M+H]⁺, RT = 3.76 min (Method A); ¹H NMR (400 MHz, DMSO-d₆): δ 11.46 (bs, 1H), 7.66-7.73 (m, 1H), 7.51-7.54 (m, 1H), 7.36-7.43 (m, 2H), 7.25-7.29 (m, 1H), 7.09 (s, 1H), 6.99 (bt, 1H), 5.69-5.74 (m, 1H), 4.20-4.36 (m, 2H), 2.43 (s, 3H), 1.34 (d, 3H); Chiral analytical SFC: RT = 4.69 min, Column: Chiralpak IC-3, (4.6 × 150 mm) 3 µm, 75% CO₂/MeOH, Flow = 3.0 g/min.

### 1-(1-(7,8-Difluoro-1-oxo-1,2-dihydroisoquinolin-4-yl)ethyl)-3-(1H-indol-6-yl)-1-methylurea (Compounds 498 & 499)

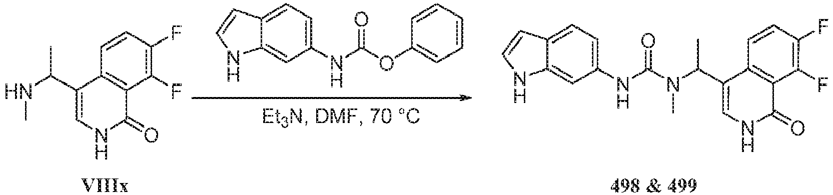

Racemic 1-(1-(7,8-difluoro-1-oxo-1,2-dihydroisoquinolin-4-yl)ethyl)-3-(1*H*-indol-6-yl)-1-methylurea was synthesized in a similar manner as described above from 7,8-difluoro-4-(1-(methylamino)ethyl)isoquinolin-1(*2H*)-one **(VIIIx)** and phenyl 1*H*-indol-6-ylcarbamate. The enantiomers were subsequently separated by chiral SFC, Column: Chiralpak IC (250 × 30 mm) 5 µ, 50% CO₂/MeOH, Flow rate 60 g/min.

1-(1-(7,8-Difluoro-1-oxo-1,2-dihydroisoquinofin-4-yl)ethyl)-3-(1*H*-indol-6-yl)-1-methylurea - Enantiomer I **(Compound 498),** LCMS: *m*/*z* found 397.3 [M+H]⁺, RT = 3.08 min (Method A); ¹H NMR (400 MHz, DMSO-d₆) δ 11.49 (bs, 1H), 10.89 (bs, 1H), 8.11 (s, 1H), 7.82-7.88 (m, 1H), 7.73 (s, 1H), 7.60-7.64 (m, 1H), 7.37 (d, 1H), 7.21 (t, 1H), 7.14 (s, 1H), 7.05-7.08 (m, 1H), 6.32 (d, 1H), 5.81-5.86 (m, 1H), 2.60 (s, 3H), 1.42 (d, 3H); Chiral analytical SFC: RT = 4.06 min, Column: Chiralpak IC-3, (4.6 × 150 mm) 3 µm, 65% CO₂/MeOH, Flow = 3.0 g/min.

1-(1-(7,8-Difluoro-1-oxo-1,2-dihydroisoquinofin-4-yl)ethyl)-3-(1*H*-indol-6-yl)-1-methylurea - Enantiomer II **(Compound 499),** LCMS: *m*/*z* found 397.3 [M+H]⁺, RT = 3.08 min (Method A); ¹H NMR (400 MHz, DMSO-d₆) δ 11.49 (bs, 1H), 10.89 (bs, 1H), 8.11 (s, 1H), 7.82-7.88 (m, 1H), 7.73 (s, 1H), 7.60-7.64 (m, 1H), 7.37 (d, 1H), 7.21 (t, 1H), 7.14 (s, 1H), 7.05-7.08 (m, 1H), 6.32 (d, 1H), 5.81-5.86 (m, 1H), 2.60 (s, 3H), 1.42 (d, 3H); Chiral analytical SFC: RT = 7.81 min, Column: Chiralpak IC-3, (4.6 × 150 mm) 3 µm, 65% CO₂/MeOH, Flow = 3.0 g/min.

### 1-(1-(7,8-Difluoro-1-oxo-1,2-dihydroisoquinolin-4-yl)ethyl)-3-(3-(difluoromethyl)-4-fluorophenyl)-1-methylurea (Compounds 503 & 504)

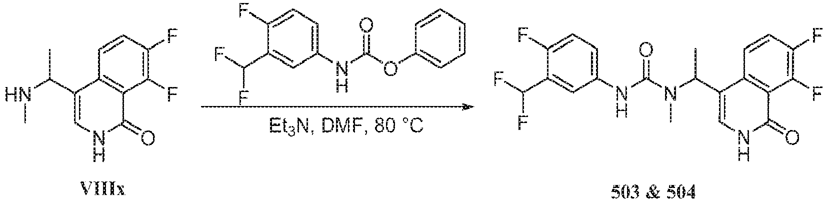

Racemic 1-(1-(7,8-difluoro-1-oxo-1,2-dihydroisoquinolin-4-yl)ethyl)-3-(3-(difluoromethyl)-4-fluorophenyl)-1-methylurea was synthesized in a similar manner as described above from 7,8-difluoro-4-(1-(methylamino)ethyl)isoquinolin-1(*2H*)-one **(VIIIx)** and 3-(difluoromethyl)-4-fluorophenylcarbamate. The enantiomers were subsequently separated by chiral SFC, Column: Chiralcel OD-H (250 × 30 mm) 5 µ, 80% CO₂/MeOH, Flow rate 90 g/min.

1-(1-(7,8-Difluoro-1-oxo-1,2-dihydroisoquinolin-4-yl)ethyl)-3-(3-(difluoromethyl)-4-fluorophenyl)-1-methylurea - Enantiomer I **(Compound 503),** LCMS: *m*/*z* found 426.2 [M+H]⁺, RT = 3.80 min (Method A); ¹H NMR (400 MHz, DMSO-d₆) δ 11.56 (bs, 1H), 8.51 (bs, 1H), 7.82-7.88 (m, 2H), 7.69-7.73 (m, 1H), 7.49-7.53 (m, 1H), 7.05-7.33 (m, 3H), 5.75-5.80 (m, 1H), 2.58 (s, 3H), 1.42 (d, 3H); Chiral analytical SFC: RT = 3.95 min, Column: Chiralcel OD-3, (4.6 × 150 mm) 3 µm, 65% CO₂/MeOH, Flow = 3.0 g/min.

1-(1-(7,8-Difluoro-1-oxo-1,2-dihydroisoquinolin-4-yl)ethyl)-3-(3-(difluoromethyl)-4-fluorophenyl)-1-methylurea - Enantiomer II **(Compound 504),** LCMS: *m*/*z* found 426.2 [M+H]⁺, RT = 3.80 min (Method A); ¹H NMR (400 MHz, DMSO-d₆) δ 11.56 (bs, 1H), 8.51 (bs, 1H), 7.82-7.88 (m, 2H), 7.69-7.73 (m, 1H), 7.49-7.53 (m, 1H), 7.05-7.33 (m, 3H), 5.75-5.80 (m, 1H), 2.58 (s, 3H), 1.42 (d, 3H); Chiral analytical SFC: RT = 5.38 min, Column: Chiralcel OD-3, (4.6 × 150 mm) 3 µm, 65% CO₂/MeOH, Flow = 3.0 g/min.

### 7,8-Difluoro-4-(1-(methylamino)ethyl)isoquinolin-1(2H)-one (VIIIcx)

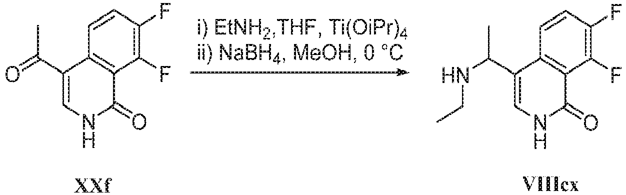

To a solution of 1.0 g (4.48 mmol, 1.0 eq.) of 4-acetyl-7,8-difluoroisoquinolin-(2*H*)-one **(XXf)** in 5 mL of THF was added 2.7 mL (5.38 mmol, 1.2 eq.) of a 2 M solution of ethylamine in THF followed by 5 mL of titanium isopropoxide and the mixture was stirred at room temperature for 16 h. The mixture cooled to 0 °C, diluted with 5 mL of methanol and 0.51 g (13.4 mmol, 3.0 eq.) of sodium borohydride was added portion-wise over approximately 10 min. The mixture was stirred at room temperature for a further 2 h and diluted with 10 mL of brine and 200 mL of 10% methanol in methylene chloride. The heterogenous mixture was filtered through CELITE^{®} and the pad washed with 20 mL of 10% methanol in methylene chloride. The layers were separated and the organic phase was dried (Na₂SO₄), filtered and the solvent was removed *in vacuo* to provide 0.8 g of 7,8-difluoro-4-(1-(ethylamino)ethyl)isoquinolin-1(2*H*)-one **(VIIIcx).** LCMS: *m*/*z* found 253.2 [M+H]⁺.
The above reaction sequence was performed on multiple batches with consistent results.

### 1-(1-(7,8-Difluoro-1-oxo-1,2-dihydroisoquinolin-4-yl)ethyl)-3-(3,4-difluorophenyl)-1-ethylurea (Compounds 462 & 463)

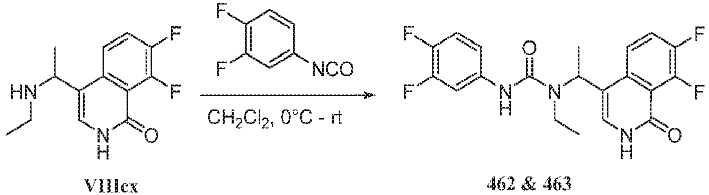

Racemic 1-(1-(7,8-difluoro-1-oxo-1,2-dihydroisoquinolin-4-yl)ethyl)-3-(3,4-difluorophenyl)-1-ethylurea was synthesized in a similar manner as described above from 7,8-difluoro-4-(1-(ethylamino)ethyl)isoquinolin-1(2*H*)-one **(VIIIcx)** and 3,4-difluorophenyl isocyanate. The enantiomers were subsequently separated by chiral SFC, Column: Chiralcel OD-H (250 × 30 mm) 5 µ, 80% CO₂/MeOH, Flow rate 90 g/min.

1-(1-(7,8-Difluoro-1-oxo-1,2-dihydroisoquinolin-4-yl)ethyl)-3-(3,4-difluorophenyl)-1-ethylurea - Enantiomer I **(Compound 462),** LCMS: *m*/*z* found 408.3 [M+H]⁺, RT = 3.93 min (Method A); ¹H NMR (400 MHz, DMSO-d₆): δ 11.50 (bs, 1H), 8.35 (bs, 1H), 7.84-7.91 (m, 1H), 7.71-7.76 (m, 1H), 7.51-7.54 (m, 1H), 7.27-7.33 (m, 2H), 7.23 (s, 1H), 5.77-5.82 (m, 1H), 3.07-3.33 (m, 2H), 1.43 (d, 3H) 0.67 (t, 3H); Chiral analytical SFC: RT = 2.09 min, Column: Chiralcel OD-3, (4.6 × 150 mm) 3 µm, 80% CO₂/MeOH, Flow = 3.0 g/min.

1-(1-(7,8-Difluoro-1-oxo-1,2-dihydroisoquinolin-4-yl)ethyl)-3-(3,4-difluorophenyl)-1-ethylurea - Enantiomer II **(Compound 463),** LCMS: *m*/*z* found 408.3 [M+H]⁺, RT = 3.93 min (Method A); ¹H NMR (400 MHz, DMSO-d₆): δ 11.50 (bs, 1H), 8.35 (bs, 1H), 7.84-7.91 (m, 1H), 7.71-7.76 (m, 1H), 7.51-7.54 (m, 1H), 7.27-7.33 (m, 2H), 7.23 (s, 1H), 5.77-5.82 (m, 1H), 3.07-3.33 (m, 2H), 1.43 (d, 3H) 0.67 (t, 3H); Chiral analytical SFC: RT = 3.37 min, Column: Chiralcel OD-3, (4.6 × 150 mm) 3 µm, 80% CO₂/MeOH, Flow = 3.0 g/min.

### 1-(1-(7,8-Difluoro-1-oxo-1,2-dihydroisoquinolin-4-yl)ethyl)-1-ethyl-3-(4-fluorophenyl)urea (Compounds 464 & 465)

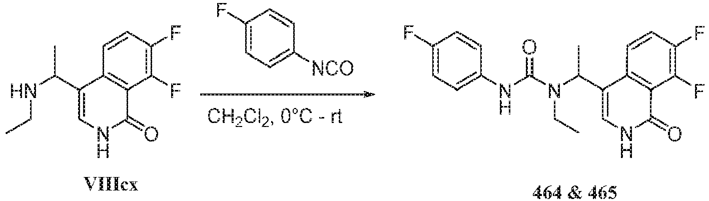

Racemic 1-(1-(7,8-difluoro-1-oxo-1,2-dihydroisoquinolin-4-yl)ethyl)-1-ethyl-3-(3-fluorophenyl)urea was synthesized in a similar manner as described above from 7,8-difluoro-4-(1-(ethylamino)ethyl)isoquinolin-1(2*H*)-one **(VIIIcx)** and 4-fluorophenyl isocyanate. The enantiomers were subsequently separated by chiral SFC, Column: Chiralpak IC (250 × 30 mm) 5 µ, 80% CO₂/MeOH, Flow rate 90 g/min.

1-(1-(7,8-Difluoro-1-oxo-1,2-dihydroisoquinofin-4-yl)ethyl)-1-ethyl-3-(4-fluorophenyl)urea - Enantiomer I **(Compound 464),** LCMS: *m*/*z* found 390.2 [M+H]⁺, RT = 3.54 min (Method A); ¹H NMR (400 MHz, DMSO-d₆): δ 11.50 (bs, 1H), 8.20 (bs, 1H), 7.85-7.91 (m, 1H), 7.52-7.59 (m, 3H), 7.21 (s, 1H), 7.07-7.12 (m, 2H), 5.79-5.84 (m, 1H), 3.08-3.19 (m, 2H), 1.42 (d, 3H), 0.67 (t, 3H); Chiral analytical SFC: RT = 3.85 min, Column: Chiralpak IC-3, (4.6 × 150 mm) 3 µm, 80% CO₂/MeOH, Flow = 3.0 g/min.

1-(1-(7,8-Difluoro-1-oxo-1,2-dihydroisoquinofin-4-yl)ethyl)-1-ethyl-3-(4-fluorophenyl)urea - Enantiomer II **(Compound 465),** LCMS: *m*/*z* found 390.2 [M+H]⁺, RT = 3.54 min (Method A); ¹H NMR (400 MHz, DMSO-d₆): δ 11.50 (bs, 1H), 8.20 (bs, 1H), 7.85-7.91 (m, 1H), 7.52-7.59 (m, 3H), 7.21 (s, 1H), 7.07-7.12 (m, 2H), 5.79-5.84 (m, 1H), 3.08-3.19 (m, 2H), 1.42 (d, 3H), 0.67 (t, 3H); Chiral analytical SFC: RT = 5.34 min, Column: Chiralpak IC-3, (4.6 × 150 mm) 3 µm, 80% CO₂/MeOH, Flow = 3.0 g/min.

### 1-(1-(7,8-Difluoro-1-oxo-1,2-dihydroisoquinolin-4-yl)ethyl)-1-ethyl-3-phenylurea (Compounds 466 & 467)

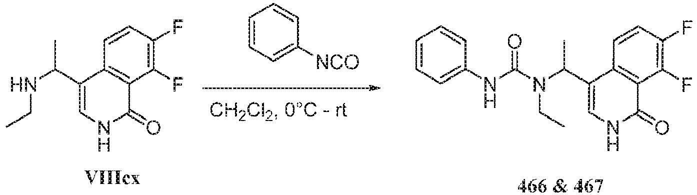

Racemic 1-(1-(7,8-difluoro-1-oxo-1,2-dihydroisoquinolin-4-yl)ethyl)-1-ethyl-3-phenylurea was synthesized in a similar manner as described above from 7,8-difluoro-4-(1-(ethylamino)ethyl)isoquinolin-1(2*H*)-one **(VIIIcx)** and phenyl isocyanate. The enantiomers were subsequently separated by chiral SFC, Column: Chiralpak IC (250 × 30 mm) 5 µ, 75% CO₂/MeOH, Flow rate 100 g/min.

1-(1-(7,8-Difluoro-1-oxo-1,2-dihydroisoquinolin-4-yl)ethyl)-1-ethyl-3-phenylurea - Enantiomer I **(Compound 466),** LCMS: *m*/*z* found 372.3 [M+H]⁺, RT = 3.41 min (Method A); ¹H NMR (400 MHz, DMSO-d₆): δ 11.50 (bs, 1H), 8.14 (bs, 1H), 7.84-7.91 (m, 1H), 7.53-7.60 (m, 3H), 7.21-7.27 (m, 3H), 6.95-6.99 (m, 1H), 5.80-5.85 (m, 1H), 3.08-3.32 (m, 2H), 1.43 (d, 3H), 0.67 (t, 3H); Chiral analytical SFC: RT = 3.66 min, Column: Chiralpak IC-3, (4.6 × 150 mm) 3 µm, 80% CO₂/MeOH, Flow = 3.0 g/min.

1-(1-(7,8-Difluoro-1-oxo-1,2-dihydroisoquinolin-4-yl)ethyl)-1-ethyl-3-phenylurea - Enantiomer II **(Compound 467),** LCMS: *m*/*z* found 372.3 [M+H]⁺, RT = 3.41 min (Method A); ¹H NMR (400 MHz, DMSO-d₆): δ 11.50 (bs, 1H), 8.14 (bs, 1H), 7.84-7.91 (m, 1H), 7.53-7.60 (m, 3H), 7.21-7.27 (m, 3H), 6.95-6.99 (m, 1H), 5.80-5.85 (m, 1H), 3.08-3.32 (m, 2H), 1.43 (d, 3H), 0.67 (t, 3H); Chiral analytical SFC: RT = 4.96 min, Column: Chiralpak IC-3, (4.6 × 150 mm) 3 µm, 80% CO₂/MeOH, Flow = 3.0 g/min.

### 3-(3-Chloro-4-fluorophenyl)-1-(1-(7,8-difluoro-1-oxo-1,2-dihydroisoquinolin-4-yl)ethyl)-1-ethylurea (Compounds 468 & 469)

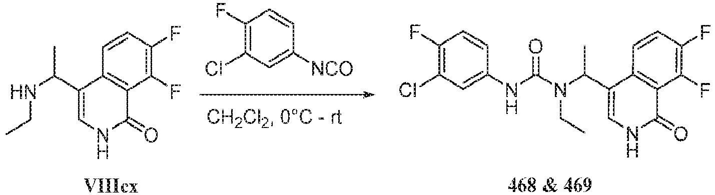

Racemic 3-(3-chloro-4-fluorophenyl)-1-(1-(7,8-difluoro-1-oxo-1,2-dihydroisoquinolin-4-yl)ethyl)-1-ethylurea was synthesized in a similar manner as described above from 7,8-difluoro-4-(l-(ethylamino)ethyl)isoquinolin-1(2*H*)-one **(VIIIcx)** and 1-fluoro-2-chloro-4-isocyanatobenzene. The enantiomers were subsequently separated by chiral SFC, Column: Chiralpak IC (250 × 30 mm) 5 µ, 75% CO₂/MeOH, Flow rate 90 g/min.

3-(3-Chloro-4-fluorophenyl)-1-(1-(7,8-difluoro-1-oxo-1,2-dihydroisoquinolin-4-yl)ethyl)-1-ethylurea - Enantiomer I **(Compound 468),** LCMS: *m*/*z* found 424.2/426.2 [M+H]⁺, RT = 4.25 min (Method A); ¹H NMR (400 MHz, DMSO-d₆): δ 11.50 (bs, 1H), 8.35 (bs, 1H), 7.85-7.92 (m, 2H), 7.50-7.54 (m, 2H), 7.31 (t, 1H), 7.22 (s, 1H), 5.77-5.83 (m, 1H), 3.06-3.22 (m, 2H), 1.43 (d, 3H), 0.67 (t, 3H); Chiral analytical SFC: RT = 2.77 min, Column: Chiralpak IC-3, (4.6 × 150 mm) 3 µm, 75% CO₂/MeOH, Flow = 3.0 g/min.

3-(3-Chloro-4-fluorophenyl)-1-(1-(7,8-difluoro-1-oxo-1,2-dihydroisoquinolin-4-yl)ethyl)-1-ethylurea - Enantiomer II **(Compound 469),** LCMS: *m*/*z* found 424.2/426.2 [M+H]⁺, RT = 4.25 min (Method A); ¹H NMR (400 MHz, DMSO-d₆): δ 11.50 (bs, 1H), 8.35 (bs, 1H), 7.85-7.92 (m, 2H), 7.50-7.54 (m, 2H), 7.31 (t, 1H), 7.22 (s, 1H), 5.77-5.83 (m, 1H), 3.06-3.22 (m, 2H), 1.43 (d, 3H), 0.67 (t, 3H); Chiral analytical SFC: RT = 3.89 min, Column: Chiralpak IC-3, (4.6 × 150 mm) 3 µm, 75% CO₂/MeOH, Flow = 3.0 g/min.

### 3-(3-Chloro-4-fluorophenyl)-1-(1-(7,8-difluoro-1-oxo-1,2-dihydroisoquinolin-4-yl)ethyl)-1-isobutylurea (Compounds 338 & 339)

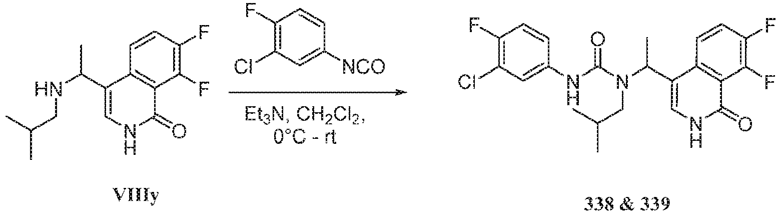

Racemic 3-(3-chloro-4-fluorophenyl)-1-(1-(7,8-difluoro-1-oxo-1,2-dihydroisoquinolin-4-yl)ethyl)-1-isobutylurea (and the associated synthetic intermediates) were synthesized in a similar manner as described previously from 7,8-difluoro-4-(1-(isobutylamino)ethyl)isoquinolin-1(2*H*)-one **(VIIIy)** and 2-chloro-1-fluoro-4-isocyanatobenzene. The enantiomers were subsequently separated by chiral SFC, Column: Chiralpak IC (250 × 30 mm) 5 µ, 80% CO₂/MeOH, Flow rate 100 g/min.

3-(3-Chloro-4-fluorophenyl)-1-(1-(7,8-difluoro-1-oxo-1,2-dihydroisoquinolin-4-yl)ethyl)-1-isobutylurea - Enantiomer I **(Compound 338),** LCMS: *m*/*z* found 452.2/454.1 [M+H]⁺, RT = 5.07 min (Method A); ¹H NMR (400 MHz, DMSO-d₆): δ 11.52 (bs, 1H), 8.39 (bs 1H), 7.88-7.95 (m, 1H), 7.79-7.82 (m, 1H), 7.61-7.65 (m, 1H), 7.45-7.50 (m, 1H), 7.32 (t, 1H), 7.23 (s, 1H), 5.78-5.83 (m, 1H), 2.96-3.02 (m, 1H), 2.83-2.89 (m, 1H), 1.45 (d, 3H), 1.30-1.36 (m, 1H), 0.63 (d, 3H), 0.46 (d, 3H); Chiral analytical SFC: RT = 2.98 min, Column: Chiralpak IC-3, (4.6 × 150 mm) 5 µm, 80% CO₂/MeOH, Flow = 3.0 g/min.

3-(3-Chloro-4-fluorophenyl)-1-(1-(7,8-difluoro-1-oxo-1,2-dihydroisoquinolin-4-yl)ethyl)-1-isobutylurea - Enantiomer II **(Compound 339),** LCMS: *m*/*z* found 452.1/454.2 [M+H]⁺, RT = 5.08 min (Method A); ¹H NMR (400 MHz, DMSO-d₆): δ 11.52 (bs, 1H), 8.39 (bs 1H), 7.88-7.95 (m, 1H), 7.79-7.82 (m, 1H), 7.61-7.65 (m, 1H), 7.45-7.50 (m, 1H), 7.32 (t, 1H), 7.23 (s, 1H), 5.78-5.83 (m, 1H), 2.96-3.02 (m, 1H), 2.83-2.89 (m, 1H), 1.45 (d, 3H), 1.30-1.36 (m, 1H), 0.63 (d, 3H), 0.46 (d, 3H); Chiral analytical SFC: RT = 3.86 min, Column: Chiralpak IC-3, (4.6 × 150 mm) 5 µm, 80% CO₂/MeOH, Flow = 3.0 g/min.

### 3-(3-Chloro-4-fluorophenyl)-1-(1-(7,8-difluoro-1-oxo-1,2-dihydroisoquinolin-4-yl)ethyl)-1-(3-hydroxypropyl)urea (Compounds 334 & 335)

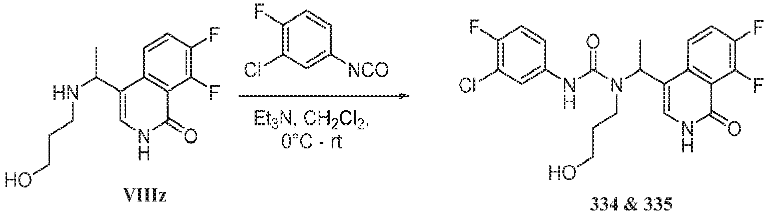

Racemic 3-(3-chloro-4-fluorophenyl)-1-(1-(7,8-difluoro-1-oxo-1,2-dihydroisoquinolin-4-yl)ethyl)-1-(3-hydroxypropyl)urea (and the associated synthetic intermediates) were synthesized in a similar manner as described previously from 7,8-difluoro-4-(1-((3-hydroxypropyl)amino)ethyl)isoquinolin-1(2*H*)-one **(VIIIz)** and 2-chloro-1-fluoro-4-isocyanatobenzene. The enantiomers were subsequently separated by chiral SFC, Column: Chiralpak IC (250 × 30 mm) 5 µ, 70% CO₂/MeOH, Flow rate 90 g/min.

3-(3-Chloro-4-fluorophenyl)-1-(1-(7,8-difluoro-1-oxo-1,2-dihydroisoquinolin-4-yl)ethyl)-1-(3-hydroxypropyl)urea - Enantiomer I **(Compound 334),** LCMS: *m*/*z* found 454.1/456.1 [M+H]⁺, RT = 4.28 min (Method A); ¹H NMR (400 MHz, DMSO-d₆): δ 11.50 (bs, 1H), 8.78 (bs 1H), 7.86-7.93 (m, 1H), 7.79-7.82 (m, 1H), 7.45-7.49 (m, 1H), 7.39-7.43 (m, 1H), 7.32 (t, 1H), 7.20 (s, 1H), 5.77-5.81 (m, 1H), 5.15 (bs, 1H) 3.13-3.27 (m, 4H), 1.44 (d, 3H), 1.10-1.13 (m, 2H); Chiral analytical SFC: RT = 2.74 min, Column: Chiralpak IC-3, (4.6 × 150 mm) 5 µm, 75% CO₂/MeOH, Flow = 3.0 g/min.

3-(3-Chloro-4-fluorophenyl)-1-(1-(7,8-difluoro-1-oxo-1,2-dihydroisoquinolin-4-yl)ethyl)-1-(3-hydroxypropyl)urea- Enantiomer II **(Compound 335),** LCMS: *m*/*z* found 454.1/456.1 [M+H]⁺, RT = 4.28 min (Method A); ¹H NMR (400 MHz, DMSO-d₆): δ 11.50 (bs, 1H), 8.78 (bs 1H), 7.86-7.93 (m, 1H), 7.79-7.82 (m, 1H), 7.45-7.49 (m, 1H), 7.39-7.43 (m, 1H), 7.32 (t, 1H), 7.20 (s, 1H), 5.77-5.81 (m, 1H), 5.15 (bs, 1H) 3.13-3.27 (m, 4H), 1.44 (d, 3H), 1.10-1.13 (m, 2H); Chiral analytical SFC: RT = 3.95 min, Column: Chiralpak IC-3, (4.6 × 150 mm) 5 µm, 75% CO₂/MeOH, Flow = 3.0 g/min.

### 2-(3-(3-Chloro-4-fluorophenyl)-1-(1-(7,8-difluoro-1-oxo-1,2-dihydroisoquinolin-4-yl)ethyl)ureido) ethane-1-sulfonamide (Compounds 393, 391 & 392)

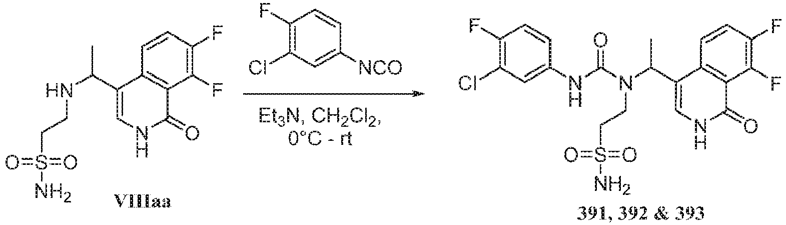

Racemic 2-(3-(3-chloro-4-fluorophenyl)-1-(1-(7,8-difluoro-1-oxo-1,2-dihydroisoquinolin-4-yl)ethyl)ureido) ethane-1-sulfonamide **(Compound 393)** (and the associated synthetic intermediates) were synthesized in a similar manner as described previously from 2-((1-(7,8-difluoro-1-oxo-1,2-dihydroisoquinolin-4-yl)ethyl)amino)ethane-1 - sulfonamide **(VIIIaa)** and 2-chloro-1-fluoro-4-isocyanatobenzene. The enantiomers were subsequently separated by chiral SFC, Column: Chiralpak IC (250 × 30 mm) 5 µ, 75% CO₂/MeOH, Flow rate 90 g/min.

2-(3-(3-Chloro-4-fluorophenyl)-1-(1-(7,8-difluoro-1-oxo-1,2-dihydroisoquinolin-4-yl)ethyl)ureido) ethane-1-sulfonamide - Enantiomer I **(Compound 391),** LCMS: *m*/*z* found 503.2/505.2 [M+H]⁺, RT = 4.12 min (Method A); ¹H NMR (400 MHz, DMSO-d₆): δ 11.56 (bs, 1H), 8.67 (bs, 1H), 7.88-7.95 (m, 1H), 7.80-7.83 (m, 1H), 7.46-7.50 (m, 1H), 7.39-7.43 (m, 1H), 7.34 (t, 1H), 7.23 (s, 1H), 6.84 (bs, 2H), 5.66-5.72 (m, 1H), 3.38-3.50 (m, 2H), 2.99-3.07 (m, 1H), 2.55-2.61 (m, 1H), 1.49 (d, 3H); Chiral analytical SFC: RT = 4.42 min, Column: Chiralpak IC-3, (4.6 × 150 mm) 5 µm, 75% CO₂/MeOH, Flow = 3.0 g/min.

2-(3-(3-Chloro-4-fluorophenyl)-1-(1-(7 ,8-difluoro-l-oxo-1,2-dihydroisoquinolin-4-yl)ethyl)ureido) ethane-1-sulfonamide - Enantiomer II **(Compound 392),** LCMS: *m*/*z* found 503.2/505.2 [M+H]⁺, RT = 4.12 min (Method A); ¹H NMR (400 MHz, DMSO-d₆): δ 11.56 (bs, 1H), 8.67 (bs, 1H), 7.88-7.95 (m, 1H), 7.80-7.83 (m, 1H), 7.46-7.50 (m, 1H), 7.39-7.43 (m, 1H), 7.34 (t, 1H), 7.23 (s, 1H), 6.84 (bs, 2H), 5.66-5.72 (m, 1H), 3.38-3.50 (m, 2H), 2.99-3.07 (m, 1H), 2.55-2.61 (m, 1H), 1.49 (d, 3H); Chiral analytical SFC: RT = 6.87 min, Column: Chiralpak IC-3, (4.6 × 150 mm) 5 µm, 75% CO₂/MeOH, Flow = 3.0 g/min.

### 2-(1-(1-(7,8-Difluoro-1-oxo-1,2-dihydroisoquinolin-4-yl)ethyl)-3-(4-fluorophenyl)ureido)ethane-1-sulfonamide (Compounds 394 & 395)

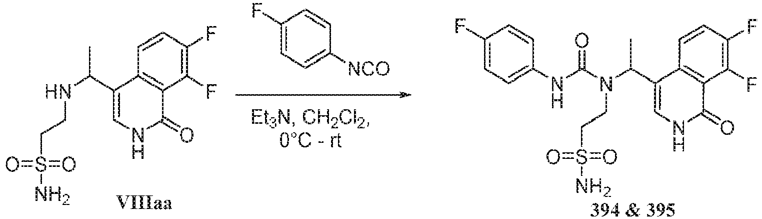

Racemic 2-(1-(1-(7,8-difluoro-1-oxo-1,2-dihydroisoquinolin-4-yl)ethyl)-3-(4-fluorophenyl)ureido)ethane-1-sulfonamide was synthesized in a similar manner as described previously from 2-((1-(7,8-difluoro-1-oxo-1,2-dihydroisoquinolin-4-yl)ethyl)amino)ethane-1-sulfonamide **(VIIIaa)** and 4-fluorophenylisocyanate. The enantiomers were subsequently separated by chiral SFC, Column: Chiralpak IC (250 × 30 mm) 5 µ, 70% CO₂/MeOH, Flow rate 100 g/min.

2-(1-(1-(7,8-Difluoro-1-oxo-1,2-dihydroisoquinolin-4-yl)ethyl)-3-(4-fluorophenyl)ureido)ethane-1-sulfonamide - Enantiomer I **(Compound 394),** LCMS: *m*/*z* found 469.2 [M+H]⁺, RT = 3.70 min (Method A); ¹H NMR (400 MHz, DMSO-d₆): δ 8.16 (bs, 2H), 7.86-7.94 (m, 1H), 7.50-7.54 (m, 2H), 7.43-7.47 (m, 1H), 7.23 (s, 1H), 7.09-7.14 (m, 2H), 7.01 (bs, 2H), 5.69-5.73 (m, 1H), 3.47-3.50 (m, 2H), 2.93-3.02 (m, 1H), 2.49-2.56 (m, 1H), 1.48 (d, 3H); Chiral analytical SFC: RT = 4.75 min, Column: Chiralpak IC, (4.6 × 150 mm) 5 µm, 70% CO₂/MeOH, Flow = 3.0 g/min.

2-(1-(1-(7,8-Difluoro-1-oxo-1,2-dihydroisoquinolin-4-yl)ethyl)-3-(4-fluorophenyl)ureido)ethane-1-sulfonamide - Enantiomer II **(Compound 395),** LCMS: *m*/*z* found 469.2 [M+H]⁺, RT = 3.70 min (Method A); ¹H NMR (400 MHz, DMSO-d₆): δ 8.16 (bs, 2H), 7.86-7.94 (m, 1H), 7.50-7.54 (m, 2H), 7.43-7.47 (m, 1H), 7.23 (s, 1H), 7.09-7.14 (m, 2H), 7.01 (bs, 2H), 5.69-5.73 (m, 1H), 3.47-3.50 (m, 2H), 2.93-3.02 (m, 1H), 2.49-2.56 (m, 1H), 1.48 (d, 3H); Chiral analytical SFC: RT = 7.44 min, Column: Chiralpak IC, (4.6 × 150 mm) 5 µm, 70% CO₂/MeOH, Flow = 3.0 g/min.

### (E)-3-(3,5-difluorophenyl)acryloyl azide

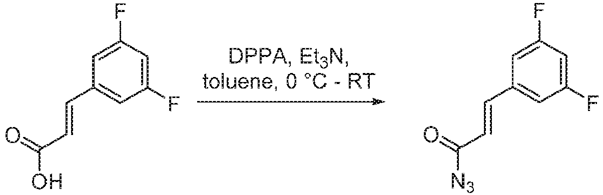

To a solution of 30.0 g (163 mmol, 1.0 eq.) of (E)-3-(3,5-difluorophenyl)acrylic acid in 150 mL of toluene under a nitrogen atmosphere at 0 °C was added 34 mL (245 mmol, 1.5 eq.) of triethylamine followed by 40.3 g (147 mmol, 0.9 eq.) of diphenylphosphoryl azide. The mixture was allowed to warm to room temperature and stirred for 3 h. The mixture was then diluted with 200 mL of ice-cold water and extracted with 3 × 200 mL of ethyl acetate. The combined organic extracts were washed with 200 mL of brine, dried (Na₂SO₄), filtered and the solvent was removed *in vacuo.* The residue was triturated with 30 mL of n-hexane, filtered and dried under high vacuum to provide 15.0 g (71.8 mmol, 44%) of (E)-3-(3,5-difluorophenyl)acryloyl azide. ¹H NMR (400 MHz, CDCl₃): δ 7.63 (d, 1H), 7.02-7.07 (m, 2H), 6.84-6.90 (m, 1H), 6.40 (d, 1H).
The above detailed reaction was performed on multiple batches with consistent results.

### 6,8-Difluoroisoquinolin-1(2H)-one (IIe)

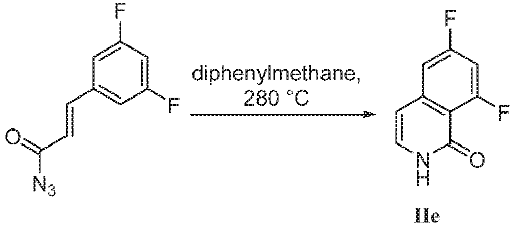

A stirred solution of 10.0 g (47.8 mmol, 1.0 eq.) of (E)-3-(3,5-difluorophenyl)acryloyl azide in 50 mL of diphenylmethane was heated to 130 °C for 30 min. The temperature was subsequently increased to 280 °C and stirring was continued for 3 h. The mixture was allowed to cool to room temperature, diluted with 50 mL of n-heptane and stirred for a further 30 min. The solids were collected by filtration, triturated with 50 mL of acetone and dried under vacuum to provide 4.0 g (22.1 mmol, 46%) of 6,7-difluoroisoquinolin-1(2*H*)-one **(IIe)**. LCMS: *m*/*z* found 182.1 [M+H]⁺, ¹H NMR (400 MHz, CDCl₃): δ 10.63 (bs, 1H), 7.16-7.19 (m, 1H), 6.98-7.02 (m, 1H), 6.87-6.93 (m, 1H), 6.44-6.47 (m, 1H).

The above detailed reaction was performed on multiple batches with consistent results

### 4-Bromo-6,8-difluoroisoquinolin-1(2H)-one (IIIe)

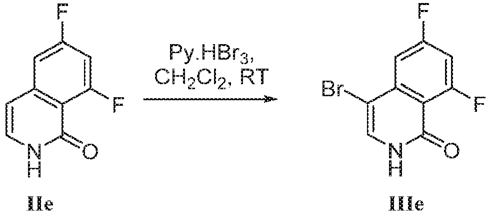

To a solution of 2.5 g (13.8 mmol, 1.0 eq.) of 6,8-difluoroisoquinolin-1(2*H*)-one **(IIe)** in 100 mL of methylene chloride was added 4.85 g (15.1 mmol, 1.1 eq.) of pyridinium hydrobromide perbromide and the mixture was stirred at room temperature for 3 h. The reaction was quenched with 30 mL of saturated sodium bicarbonate solution and the solvent was removed *in vacuo.* The residue was suspended in 80 mL of water and the solids were collected by filtration, washed with 50 mL of petroleum ether and dried under vacuum to provide 2.5 g (13.5 mmol, 69%) of 4-bromo-6,8-difluoroisoquinolin-1(2*H*)-one **(IIIe)**. LCMS: *m*/*z* found 260.0/262.0 [M+H]⁺.

The above detailed reaction was performed on multiple batches with consistent results

### 4-Acetyl-6,8-difluoroisoquinolin-1(2H)-one (XXe)

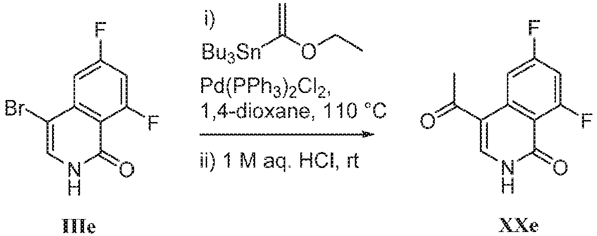

To a stirred solution of 3.8 g (14.6 mmol, 1.0 eq.) of 4-bromo-6,8-difluoroisoquinolin-1(*2H*)-one **(IIIe)** in 50 mL of 1,4-dioxane was added 13.4 g (36.5 mmol, 3.0 eq.) of tributyl(1-ethoxyvinyl)stannane. The mixture was purged with nitrogen gas for 5 min and 1.02 g (1.46 mmol, 0.1 eq.) of Pd(PPh₃)₂Cl₂ was added, and then heated to 110 °C for 16 h. The reaction mixture was allowed to cool to room temperature and 50 mL of 1 M aqueous HCl was added and stirring was continued for an additional 1 h. The reaction mixture was then basified with 50 mL of saturated sodium bicarbonate solution and extracted with 2 × 300 mL of ethyl acetate. The combined organic extracts were washed with 100 mL of brine, dried (Na₂SO₄), filtered and the solvent was removed *in vacuo.* The residue was triturated with 100 mL of n-pentane, filtered and dried under high vacuum to provide 2.0 g (9.0 mmol, 61%) of 4-acetyl-6,8-difluoroisoquinolin-1(2*H*)-one **(XXe).** LCMS: *m*/*z* found 224.1 [M+H]⁺, ¹H NMR (300 MHz, DMSO-d₆): δ 11.30 (bs, 1H), 8.69-8.73 (m, 1H), 8.09-8.10 (m, 1H), 6.96-7.03 (m, 1H), 2.58 (s, 3H).
The above detailed reaction was performed on multiple batches with consistent results

### 6,8-Difluoro-4-(l-(methylamino)ethyl)isoquinolin-1(2H)-one (VIIIab)

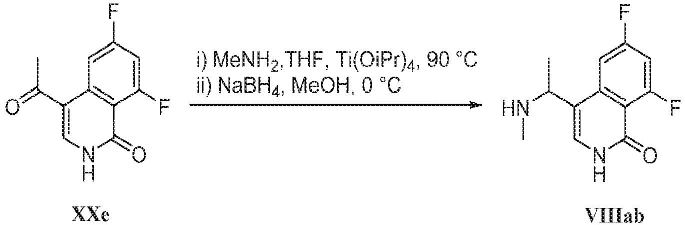

To a solution of 500 mg (2.2 mmol, 1.0 eq.) of 4-acetyl-6,8-difluoroisoquinolin-1(*2H*)-one **(XXe)** in 5 mL of THF in a sealed tube was added 2.3 mL (4.5 mmol, 2.0 eq.) of a 2 M solution of methylamine solution in THF followed by 2.5 mL of titanium isopropoxide and the mixture was heated at 90 °C for 3 h. The mixture was allowed to cool to room temperature and further cooled to 0 °C. Following dilution with 2.5 mL of methanol, 0.25 g (6.7 mmol, 3.0 eq.) of sodium borohydride was added portion-wise over approximately 10 min and stirring was continued for 4 h. The reaction mixture was diluted with 10 mL of brine, filtered through CELITE^{®} and the pad was washed with 50 mL of 10% methanol in methylene chloride. The combined filtrate was evaporated *in vacuo* to provide 0.3 g of 6,8-difluoro-4-(1-(methylamino)ethyl)isoquinolin-1(2*H*)-one **(VIIIab).** LCMS: *m*/*z* found 239.1 [M+H]⁺.

### 3-(3-Chloro-4-fluorophenyl)-1-(1-(6,8-difluoro-1-oxo-1,2-dihydroisoquinolin-4-yl)ethyl)-1-methylurea (Compounds 368 & 369)

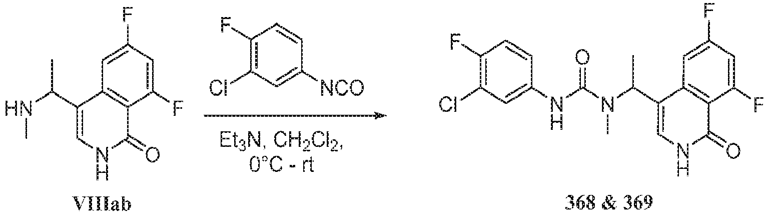

To a solution of 300 mg of 6,8-difluoro-4-(1-(methylamino)ethyl)isoquinolin-1(2*H*)-one **(VIIIab)** in 10 mL of methylene chloride at 0 °C was added 0.53 mL (3.72 mmol) of triethylamine followed by 0.10 mL (0.75 mmol) of 2-chloro-1-fluoro-4-isocyanatobenzene. The mixture was allowed to warm to room temperature and stirred for 1 h. The reaction mixture was then diluted with 100 mL of water and extracted with 3 × 100 mL of methylene chloride. The combined organic extracts were washed with 50 mL of brine, dried (Na₂SO₄), filtered and the solvent was removed *in vacuo.* The residue was purified by reverse phase chromatography (REVELERIS^{®} C-18: 40 g column eluting with linear gradient 10-22% of [0.1% formic acid in water]/acetonitrile) to provide 300 mg (0.73 mmol) of racemic 3-(3-chloro-4-fluorophenyl)-1-(1-(6,8-difluoro-1-oxo-1,2-dihydroisoquinolin-4-yl)ethyl)-1-methylurea. The enantiomers were subsequently separated by chiral SFC, Column: Chiralpak IC (250 × 30 mm) 5 µ, 70% CO₂/MeOH, Flow rate 100 g/min.

(3-Chloro-4-fluorophenyl)-1-(1-(6,8-difluoro-1-oxo-1,2-dihydroisoquinolin-4-yl)ethyl)-1-methylurea- Enantiomer I **(Compound 368),** LCMS: *m*/*z* found 410.2/412.2 [M+H]⁺, RT = 4.36 min (Method A); ¹H NMR (400 MHz, DMSO-d₆): δ 11.48 (bs, 1H), 8.47 (bs, 1H), 7.80-7.82 (m, 1H), 7.46-7.51 (m, 1H), 7.28-7.35 (m, 3H), 7.23 (s, 1H), 5.72-5.75 (m, 1H), 2.59 (s, 3H), 1.41 (d, 3H); Chiral analytical SFC: RT = 3.98 min, Column: Chiralpak IC-3 (4.6 × 150 mm) 3 µm, 60% CO₂/MeOH, Flow = 3.0 g/min.

(3-Chloro-4-fluorophenyl)-1-(1-(6,8-difluoro-1-oxo-1,2-dihydroisoquinolin-4-yl)ethyl)-1-methylurea - Enantiomer II **(Compound 369),** LCMS: *m*/*z* found 410.2/412.2 [M+H]⁺, RT = 4.36 min (Method A); ¹H NMR (400 MHz, DMSO-d₆): δ 11.48 (bs, 1H), 8.47 (bs, 1H), 7.80-7.82 (m, 1H), 7.46-7.51 (m, 1H), 7.28-7.35 (m, 3H), 7.23 (s, 1H), 5.72-5.75 (m, 1H), 2.59 (s, 3H), 1.41 (d, 3H); Chiral analytical SFC: RT = 6.38 min, Column: Chiralpak IC-3 (4.6 × 150 mm) 3 µm, 60% CO₂/MeOH, Flow = 3.0 g/min.

### 1-(1-(6,8-Difluoro-1-oxo-1,2-dihydroisoquinolin-4-yl)ethyl)-3-(4-fluorophenyl)-1-methylurea (Compounds 370 & 371)

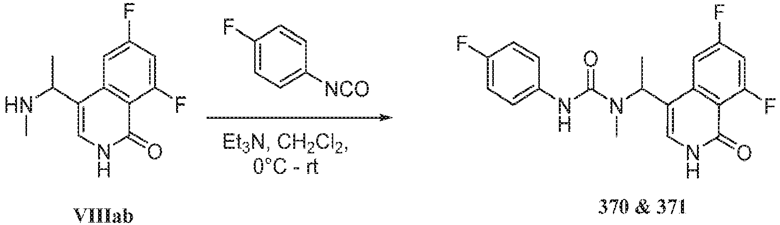

Racemic 1-(1-(6,8-difluoro-1-oxo-1,2-dihydroisoquinolin-4-yl)ethyl)-3-(4-fluorophenyl)-1-methylurea was synthesized in a similar manner as described above from 6,8-difluoro-4-(1-(methylamino)ethyl)isoquinolin-1(2*H*)-one **(VIIIab)** and 4-fluorophenyl isocyanate. The enantiomers were subsequently separated by chiral SFC, Column: Chiralpak IC (250 × 30 mm) 5 µ, 75% CO₂/MeOH, Flow rate 90 g/min.

1-(1-(6,8-Difluoro-1-oxo-1,2-dihydroisoquinolin-4-yl)ethyl)-3-(4-fluorophenyl)-1-methylurea - Enantiomer I **(Compound 370),** LCMS: *m*/*z* found 376.2 [M+H]⁺, RT = 3.87 min (Method A); ¹H NMR (400 MHz, DMSO-d₆): δ 11.47 (bs, 1H), 8.31 (bs 1H), 7.50-7.54 (m, 2H), 7.27-7.38 (m, 2H), 7.22 (s, 1H), 7.08-7.13 (m, 2H), 5.70-5.76 (m, 1H), 2.59 (s, 3H), 1.41 (d, 3H); Chiral analytical SFC: RT = 3.62 min, Column: Chiralpak IC-3, (4.6 × 150 mm) 3 µm, 60% CO₂/MeOH, Flow = 3.0 g/min.

1-(1-(6,8-Difluoro-1-oxo-1,2-dihydroisoquinolin-4-yl)ethyl)-3-(4-fluorophenyl)-1-methylurea - Enantiomer II **(Compound 371),** LCMS: *m*/*z* found 376.2 [M+H]⁺, RT = 3.87 min (Method A); ¹H NMR (400 MHz, DMSO-d₆): δ 11.47 (bs, 1H), 8.31 (bs 1H), 7.50-7.54 (m, 2H), 7.27-7.38 (m, 2H), 7.22 (s, 1H), 7.08-7.13 (m, 2H), 5.70-5.76 (m, 1H), 2.59 (s, 3H), 1.41 (d, 3H); Chiral analytical SFC: RT = 5.19 min, Column: Chiralpak IC-3, (4.6 × 150 mm) 3 µm, 60% CO₂/MeOH, Flow = 3.0 g/min.

### 6,8-Difluoro-4-(1-(isobutylamino)ethyl)isoquinolin-1(2H)-one (VIIIac)

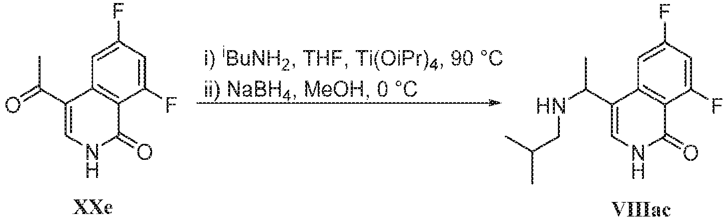

To a solution of 1.0 g (4.5 mmol, 1.0 eq.) of 4-acetyl-6,8-difluoroisoquinolin-1(2*H*)-one **(XXe)** in 10 mL of THF in a sealed tube under a nitrogen atmosphere was added 0.66 mL (6.7 mmol, 2.0 eq.) of isobutylamine followed by 5 mL of titanium isopropoxide and the mixture was heated at 90 °C for 16 h. The mixture was allowed to cool to room temperature and further cooled to 0 °C. Following dilution with 2 mL of methanol, 0.68 g (17.8 mmol, 4.0 eq.) of sodium borohydride was added portion-wise over approximately 10 min and stirring was continued for 3 h. The reaction mixture was diluted with 40 mL of water and extracted with 50 mL of ethyl acetate. The combined organic extracts were washed with 50 mL of water, 50 mL of brine, dried (Na₂SO₄), filtered and the solvent was removed *in vacuo* to provide 1.0 g of 6,8-difluoro-4-(1-(isobutylamino)ethyl)isoquinolin-1(2*H*)-one **(VIIIac)**. LCMS: *m*/*z* found 281.10 [M+H]⁺.

### 3-(3-Chloro-4-fluorophenyl)-1-(1-(6,8-difluoro-1-oxo-1,2-dihydroisoquinolin-4-yl)ethyl)-1-isobutylurea (Compounds 446 & 372)

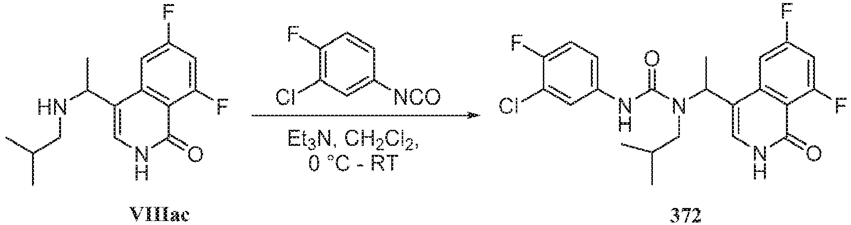

Racemic 3-(3-chloro-4-fluorophenyl)-1-(1-(6,8-difluoro-1-oxo-1,2-dihydroisoquinolin-4-yl)ethyl)-1-isobutylurea was synthesized in a similar manner as described above from 6,8-difluoro-4-(1-(isobutylamino)ethyl)isoquinolin-1(2*H*)-one **(VIIIac)** and 2-chloro-1-fluoro-4-isocyanatobenzene. The enantiomers were subsequently separated by chiral SFC, Column: Lux Cellulose-2 (250 × 30 mm) 5 µ, 70% CO₂/MeOH, Flow rate 90 g/min.

3-(3-Chloro-4-fluorophenyl)-1-(1-(6,8-difluoro-1-oxo-1,2-dihydroisoquinolin-4-yl)ethyl)-1-isobutylurea - Enantiomer I **(Compound 446),** LCMS: *m*/*z* found 452.1/454.1 [M+H]⁺, RT = 4.94 min (Method A); ¹H NMR (400 MHz, DMSO-d₆): δ 11.53 (bs, 1H), 8.41 (s, 1H), 7.74-7.77 (m, 1H), 7.42-7.48 (m, 2H), 7.29-7.35 (m, 3H), 5.72-5.75 (m, 1H), 3.00-3.07 (m, 1H), 2.81-2.87 (m, 1H), 1.44 (d, 3H), 1.32-1.39 (m, 1H), 0.64 (d, 3H), 0.45 (d, 3H); Chiral analytical SFC: RT = 1.35 min, Column: Chiralcel OZ-3, (4.6 × 150 mm) 5 µm, 85% CO₂/MeOH, Flow = 3.0 g/min.

3-(3-Chloro-4-fluorophenyl)-1-(1-(6,8-difluoro-1-oxo-1,2-dihydroisoquinolin-4-yl)ethyl)-1-isobutylurea - Enantiomer II **(Compound 372),** LCMS: *m*/*z* found 452.1/454.1 [M+H]⁺, RT = 4.94 min (Method A); ¹H NMR (400 MHz, DMSO-d₆): δ 11.53 (bs, 1H), 8.41 (s, 1H), 7.74-7.77 (m, 1H), 7.42-7.48 (m, 2H), 7.29-7.35 (m, 3H), 5.72-5.75 (m, 1H), 3.00-3.07 (m, 1H), 2.81-2.87 (m, 1H), 1.44 (d, 3H), 1.32-1.39 (m, 1H), 0.64 (d, 3H), 0.45 (d, 3H); Chiral analytical SFC: RT = 2.06 min, Column: Chiralcel OZ-3, (4.6 × 150 mm) 5 µm, 85% CO₂/MeOH, Flow = 3.0 g/min.

### 1-(1-(6,8-Difluoro-1-oxo-1,2-dihydroisoquinolin-4-yl)ethyl)-3-(4-fluorophenyl)-1-isobutylurea (Compounds 375 & 376)

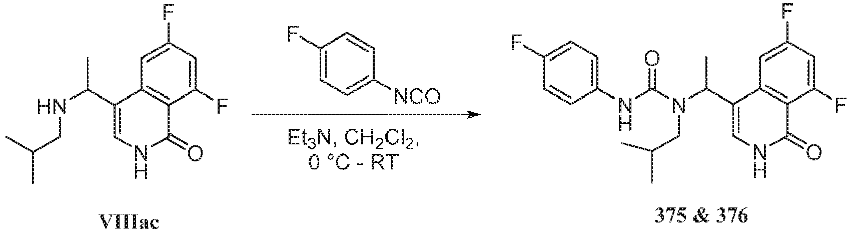

Racemic 1-(1-(6,8-difluoro-1-oxo-1,2-dihydroisoquinolin-4-yl)ethyl)-3-(4-fluorophenyl)-1-isobutylurea was synthesized in a similar manner as described above from 6,8-difluoro-4-(1-(isobutylamino)ethyl)isoquinofin-1(2*H*)-one **(VIIIac)** and 4-fluorophenyl isocyanate. The enantiomers were subsequently separated by chiral SFC, Column: Lux Cellulose-2 (250 × 30 mm) 5 µ, 80% CO₂/MeOH, Flow rate 100 g/min.

1-(1-(6,8-Difluoro-1-oxo-1,2-dihydroisoquinolin-4-yl)ethyl)-3-(4-fluorophenyl)-1-isobutylurea - Enantiomer I **(Compound 375),** LCMS: *m*/*z* found 418.2 [M+H]⁺, RT = 4.44 min (Method A); ¹H NMR (400 MHz, DMSO-d₆): δ 11.50 (bs, 1H), 8.26 (bs, 1H), 7.46-7.53 (m, 3H), 7.27-7.34 (m, 2H), 7.09-7.13 (m, 2H), 5.73-5.78 (m, 1H), 3.01-3.07 (m, 1H), 2.80-2.86 (m, 1H), 1.44 (d, 3H), 1.33-1.39 (m, 1H), 0.65 (d, 3H), 0.45 (d, 3H); Chiral analytical SFC: RT = 1.27 min, Column: Chiralcel OZ-3, (4.6 × 150 mm) 5 µm, 70% CO₂/MeOH, Flow = 3.0 g/min.

1-(1-(6,8-Difluoro-1-oxo-1,2-dihydroisoquinolin-4-yl)ethyl)-3-(4-fluorophenyl)-1-isobutylurea - Enantiomer II **(Compound 376),** LCMS: *m*/*z* found 418.2 [M+H]⁺, RT = 4.44 min (Method A); ¹H NMR (400 MHz, DMSO-d₆): δ 11.50 (bs, 1H), 8.26 (bs, 1H), 7.46-7.53 (m, 3H), 7.27-7.34 (m, 2H), 7.09-7.13 (m, 2H), 5.73-5.78 (m, 1H), 3.01-3.07 (m, 1H), 2.80-2.86 (m, 1H), 1.44 (d, 3H), 1.33-1.39 (m, 1H), 0.65 (d, 3H), 0.45 (d, 3H); Chiral analytical SFC: RT = 1.90 min, Column: Chiralcel OZ-3, (4.6 × 150 mm) 5 µm, 70% CO₂/MeOH, Flow = 3.0 g/min.

### 6.8-Difluoro-4-(1-((3-hydroxypropyl)amino)ethyl)isoquinolin-1(2H)-one (VIIIad)

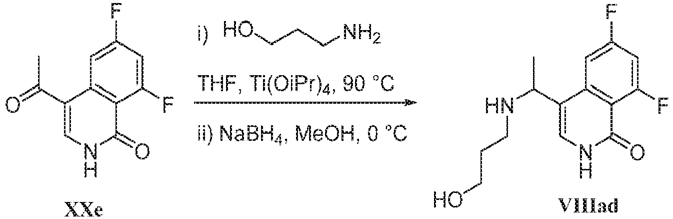

To a solution of 0.8 g (3.6 mmol, 1.0 eq.) of 4-acetyl-6,7-difluoroisoquinolin-1(2*H*)-one **(XXe)** in 5 mL THF under a nitrogen atmosphere was added 0.81 g (10.8 mmol, 3.0 eq.) of 3-aminopropan-1-ol followed by 5 mL of titanium isopropoxide and the mixture was heated at 90 °C for 3 h. The mixture was allowed to cool to room temperature and further cooled to 0 °C. Following dilution with 5 mL of methanol, 0.51 g (13.3 mmol, 3.0 eq.) of sodium borohydride was added portion-wise over approximately 10 min and stirring was continued for 4 h. The reaction mixture was diluted with 10 mL of brine and filtered through CELITE^{®}. The pad was washed with 2 × 100 mL of methylene chloride and the organic phase of the filtrate was dried (Na₂SO₄), filtered and the solvent was removed *in vacuo* to provide 0.8 g of 6,8-difluoro-4-(1-((3-hydroxypropyl)amino)ethyl)isoquinolin-1(2*H*)-one **(VIIIad)**. LCMS: *m*/*z* found 283.2 [M+H]⁺.

### 3-(3-Chloro-4-fluorophenyl)-1-(1-(6,8-difluoro-1-oxo-1,2-dihydroisoquinolin-4-yl)ethyl)-1-(3-hydroxypropyl)urea (Compounds 373 & 374)

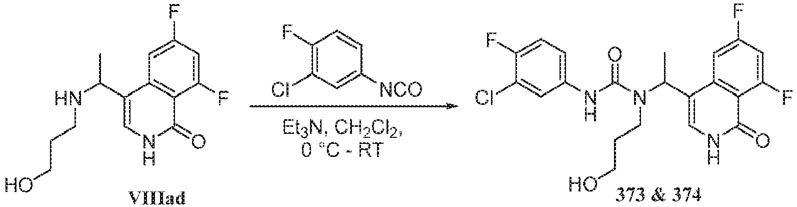

To a solution of 0.4 g of 6,8-difluoro-4-(1-((3-hydroxypropyl)amino)ethyl)isoquinolin-1(2*H*)-one **(VIIIad)** in 5 mL of methylene chloride was added 0.6 mL (4.2 mmol) of triethylamine followed by 0.11 g (0.85 mmol) of 2-chloro-1-fluoro-4-isocyanatobenzene and the mixture was stirred at room temperature for 2 h. The mixture was diluted with 50 mL of ice-cold water and extracted with 2 × 100 ml of methylene chloride. The combined organic extracts were washed with 50 mL of brine, dried (Na₂SO₄), filtered and the solvent was removed *in vacuo.* The residue was purified by flash chromatography (SiO₂, eluting with 80% ethyl acetate/petroleum ether) to provide 150 mg of racemic 3-(3-chloro-4-fluorophenyl)-1-(1-(6,8-difluoro-1-oxo-1,2-dihydroisoquinolin-4-yl)ethyl)-1-(3-hydroxypropyl)urea. The enantiomers were subsequently separated by chiral SFC, Column: Lux Cellulose-2 (250 × 30 mm) 5 µ, 70% CO₂/MeOH, Flow rate 90 g/min.

3-(3-Chloro-4-fluorophenyl)-1-(1-(6,8-difluoro-1-oxo-1,2-dihydroisoquinolin-4-yl)ethyl)-1-(3-hydroxypropyl)urea- Enantiomer I **(Compound 373),** LCMS: *m*/*z* found 454.1/456.2 [M+H]⁺, RT = 4.27 min (Method A); ¹H NMR (400 MHz, DMSO-d₆): 11.50 (bs, 1H), 8.71 (bs, 1H), 7.75-7.77 (m, 1H), 7.39-7.44 (m, 1H), 7.26-7.36 (m, 4H), 5.71-5.75 (m, 1H), 5.01 (bs, 1H), 3.10-3.24 (m, 4H), 1.44 (d, 3H), 1.10-1.23 (m, 2H); Chiral analytical SFC: RT = 2.06 min, Column: Chiralcel OZ-3, (4.6 × 150 mm) 5 µm, 70% CO₂/MeOH, Flow = 3.0 g/min.

3-(3-Chloro-4-fluorophenyl)-1-(1-(6,8-difluoro-1-oxo-1,2-dihydroisoquinolin-4-yl)ethyl)-1-(3-hydroxypropyl)urea- Enantiomer II **(Compound 374),** LCMS: *m*/*z* found 454.1/456.2 [M+H]⁺, RT = 4.27 min (Method A); ¹H NMR (400 MHz, DMSO-d₆): 11.50 (bs, 1H), 8.71 (bs, 1H), 7.75-7.77 (m, 1H), 7.39-7.44 (m, 1H), 7.26-7.36 (m, 4H), 5.71-5.75 (m, 1H), 5.01 (bs, 1H), 3.10-3.24 (m, 4H), 1.44 (d, 3H), 1.10-1.23 (m, 2H); Chiral analytical SFC: RT = 3.24 min, Column: Chiralcel OZ-3, (4.6 × 150 mm) 5 µm, 70% CO₂/MeOH, Flow = 3.0 g/min.

### 1-(1-(6,8-Difluoro-1-oxo-1,2-dihydroisoquinolin-4-yl)ethyl)-3-(4-fluorophenyl)-1-(3-hydroxypropyl) urea (Compounds 377 & 378)

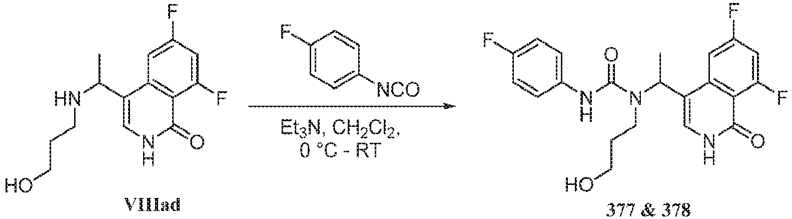

Racemic 1-(1-(6,8-difluoro-1-oxo-1,2-dihydroisoquinolin-4-yl)ethyl)-3-(4-fluorophenyl)-1-(3-hydroxypropyl) urea was synthesized in a similar manner as described above from 6,8-difluoro-4-(1-((3-hydroxypropyl)amino)ethyl)isoquinolin-1(2*H*)-one **(VIIIad)** and 4-fluorophenyl isocyanate. The enantiomers were subsequently separated by chiral SFC, Column: Lux Cellulose-2 (250 × 30 mm) 5 µ, 70% CO₂/MeOH, Flow rate 100 g/min.

1-(1-(6,8-Difluoro-1-oxo-1,2-dihydroisoquinolin-4-yl)ethyl)-3-(4-fluorophenyl)-1-(3-hydroxypropyl) urea - Enantiomer I **(Compound 377),** LCMS: *m*/*z* found 420.2 [M+H]⁺, RT = 3.79 min (Method A); ¹H NMR (400 MHz, DMSO-d₆): δ 11.49 (bs, 1H), 8.55 (bs, 1H), 7.46-7.50 (m, 2H), 7.26-7.32 (m, 3H), 7.09-7.14 (m, 2H), 5.72-5.76 (m, 1H), 4.99 (bs, 1H), 3.11-3.23 (m, 4H), 1.43 (d, 3H), 1.11-1.21 (m, 2H); Chiral analytical SFC: RT = 1.73 min, Column: Chiralcel OZ-3, (4.6 × 150 mm) 5 µm, 70% CO₂/MeOH, Flow = 3.0 g/min.

1-(1-(6,8-Difluoro-1-oxo-1,2-dihydroisoquinolin-4-yl)ethyl)-3-(4-fluorophenyl)-1-(3-hydroxypropyl) urea - Enantiomer II **(Compound 378),** LCMS: *m*/*z* found 420.2 [M+H]⁺, RT = 3.79 min (Method A); ¹H NMR (400 MHz, DMSO-d₆): δ 11.49 (bs, 1H), 8.55 (bs, 1H), 7.46-7.50 (m, 2H), 7.26-7.32 (m, 3H), 7.09-7.14 (m, 2H), 5.72-5.76 (m, 1H), 4.99 (bs, 1H), 3.11-3.23 (m, 4H), 1.43 (d, 3H), 1.11-1.21 (m, 2H); Chiral analytical SFC: RT = 2.66 min, Column: Chiralcel OZ-3, (4.6 × 150 mm) 5 µm, 70% CO₂/MeOH, Flow = 3.0 g/min.

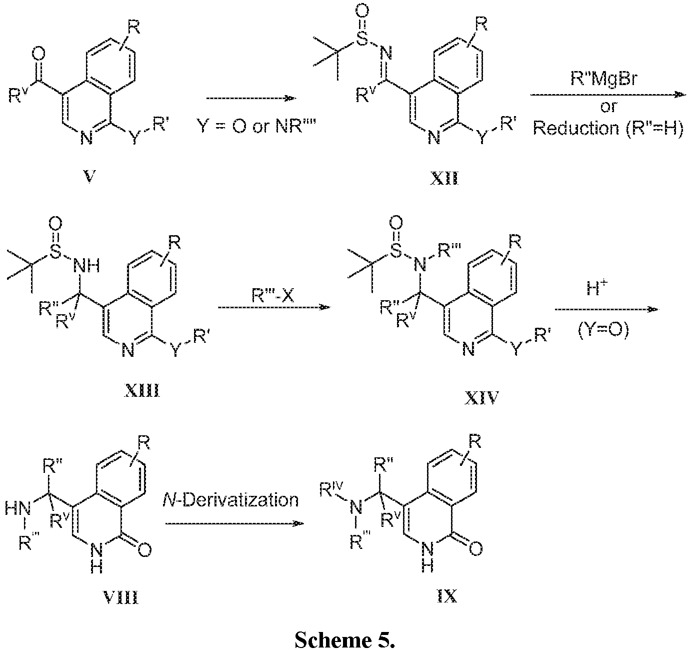

### (S)-N-((1-Methoxyisoquinolin-4-yl)methylene)-2-methylpropane-2-sulfinamide (XIIa)

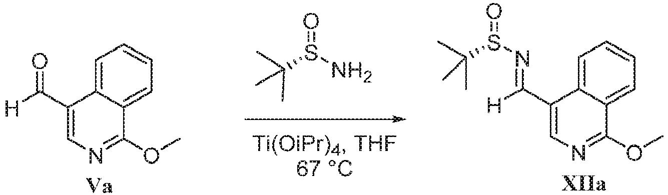

To a solution of 3.0 g (16.0 mmol, 1.0 eq.) of 1-methoxyisoquinoline-4-carbaldehyde in 67 mL of anhydrous THF at room temperature under a nitrogen atmosphere was added 9.5 mL (32.0 mmol, 2.0 eq.) of titanium (IV) isopropoxide followed by 2.14 g (17.6 mmol, 1.1 eq.) of (*S*)-2-methylpropane-2-sulfinamide, and the mixture was heated to 67 °C for 16 h. The mixture was allowed to cool to room temperature and poured into 50 mL of a rapidly stirred brine solution. The mixture was stirred for 10 minutes and then filtered through CELITE^{®}. The filter cake was washed with 200 mL of ethyl acetate, and the filtrate was transferred to a separatory funnel where the layers were separated. The organic phase was washed with 30 mL of brine and the combined aqueous washings were extracted with 30 mL of ethyl acetate. The combined organic extracts were dried (Na₂SO₄), filtered, and the solvent was removed *in vacuo.* The residue was re-dissolved in 80 mL of methylene chloride, evaporated, and dried under high vacuum to provide 4.65 g of crude (*S*)*-N-*((1-methoxyisoquinolin-4-yl)methylene)-2-methylpropane-2-sulfinamide **(XIIa)** which was used without further purification.

### (S)-N-((R)-1-(1-Methoxyisoquinolin-4-yl)ethyl)-2-methylpropane-2-sulfinamide (XIIIa)

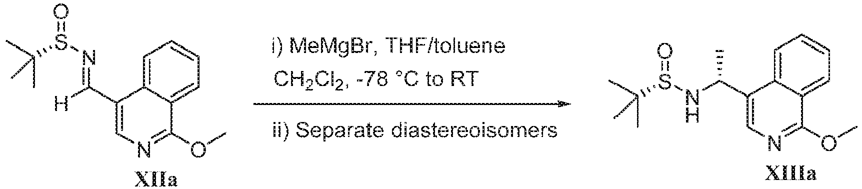

A solution of 4.65 g of crude (*S*)-*N*[(1-methoxy-4-isoquinolyl)methylene]-2-methylpropane-2-sulfinamide **(XIIa)** in 80 mL of anhydrous methylene chloride under a nitrogen atmosphere was cooled to -78 °C and 28.6 mL (40.3 mmol) of a 1.4 M solution of methylmagnesium bromide in 1:3 (*v*/*v*) of THF: toluene was added slowly. The mixture was allowed to warm to room temperature and stirred for 16 h. The reaction mixture was then slowly added to a mixture of 80 mL of saturated aqueous ammonium chloride and ice. The resulting mixture was diluted with 100 mL of ethyl acetate and the layers were separated. The aqueous phase was extracted with 2 × 40 mL of ethyl acetate and the combined organic extracts were washed with 30 mL of saturated sodium bicarbonate solution, dried (Na₂SO₄), filtered, and the solvent was removed *in vacuo.* The residue was purified by flash chromatography (SiO₂, eluting with 40% -100% ethyl acetate/hexanes), isolating 2.63 g (8.58 mmol 54% from **Va)** of the major (second eluting) diastereomer of (*S*)-*N*-(1-(1-methoxyisoquinolin-4-yl)ethyl)-2-methylpropane-2-sulfinamide **(XIIIa)**. ¹H NMR (400 MHz, DMSO-d₆) δ 8.12-8.25 (m, 2H), 8.04 (s, 1H), 7.78 (m, 1H), 7.62 (m, 1H), 5.46 (d, 1H), 4.95 (m, 1H), 4.05 (s, 3H), 1.65 (d, 3H), 1.07 (s, 9H). The stereochemistry of the α-methyl substituent was shown to be (R)-based on X-ray crystallographic analysis of **Compound 14.**

Analogous compounds can be synthesized from intermediate **XII** utilizing different Grignard reagents.

### Alternate synthesis of (S)-N-((R)-1-(1-Methoxyisoquinolin-4-yl)ethyl)-2-methylpropane-2-sulfinamide (XIIIa)

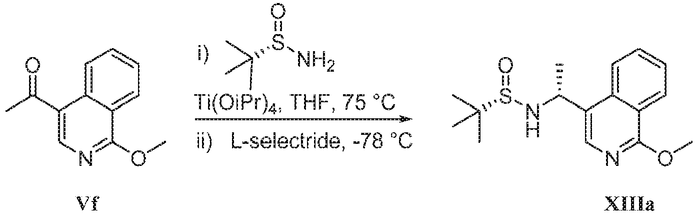

To a mixture of 53 mg (0.26 mmol, 1.0 eq.) of 1-(1-methoxy-4-isoquinolyl)ethanone **(Vf)** and 41.5 mg (0.34 mmol, 1.3 eq.) of (S)-2-methylpropane-2-sulfinamide in 0.15 mL of anhydrous THF in a sealed tube was added 0.16 mL (0.53 mmol, 2.0 eq.) of tetraisopropoxytitanium and the mixture was heated at 75 °C for 21 h. The mixture was allowed to cool to room temperature and diluted with 0.4 mL of THF. The mixture was further cooled to -78 °C under a nitrogen atmosphere and 0.28 mL (0.28 mmol, 1.1 eq.) of a 1 M solution of L-selectride in THF was added and the mixture was allowed to warm to room temperature over 4 h. The reaction mixture was then cooled to -40 °C and quenched with 0.6 mL of methanol and the cooling bath was removed. Upon warming to room temperature, the mixture was slowly added to 0.5 mL of a rapidly stirred brine solution. The mixture was diluted with 15 mL ethyl acetate, stirred for 10 min and then filtered through CELITE^{®}. The pad was washed with 10 mL of ethyl acetate, the combined filtrate was evaporated *in vacuo* and the major diastereoisomer was isolated by flash chromatography (SiO₂, eluting with a gradient of 0-100% of ethyl acetate/hexanes) to provide 55 mg (0.18 mmol, 69% from **Vf)** of diastereomerically pure (*S*)-*N-*(1-(1-methoxyisoquinolin-4-yl)ethyl)-2-methylpropane-2-sulfinamide **(XIIIa)**. ¹H NMR (400 MHz, DMSO-d₆) δ 8.12-8.25 (m, 2H), 8.04 (s, 1H), 7.78 (m, 1H), 7.63 (m, 1H), 5.46 (d, 1H), 4.96 (m, 1H), 4.05 (s, 3H), 1.65 (d, 3H), 1.07 (s, 9H). Comparative analysis of **XIIIa** derived from the above detailed procedure with **XIIIa** derived from the reaction of methyl magnesium bromide with (S)-N-[(1-methoxy-4-isoquinolyl)methylene]-2-methyl-propane-2-sulfinamide **(XIIa**) reveals the same diastereomeric preference for the major diastereoisomer. X-ray crystallographic studies on **Compound 14** (*vide infra*) reveal the absolute configuration of the α-methyl substituent of **XIIIa** as (R)-.

### (S)-N-((R)-1-(1-methoxyisoquinolin-4-yl)ethyl)-N,2-dimethylpropane-2-sulfinamide (XIVa)

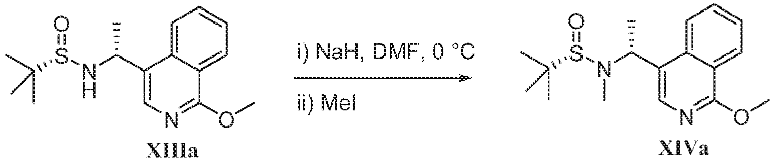

To a solution of 0.65 g (2.1 mmol, 1.0 eq.) of the major diastereoisomer of (*S*)-*N*-(1-(1-methoxyisoquinolin-4-yl)ethyl)-2-methylpropane-2-sulfinamide **(XIIIa)** as described above in 20 mL of anhydrous DMF under a nitrogen atmosphere at 0 °C was added 0.17 g (4.2 mmol, 2.0 eq.) of a 60% dispersion of sodium hydride in mineral oil. The mixture was stirred at 0 °C for 20 min and 0.26 mL (4.2 mmol, 2.0 eq.) of iodomethane was added. The mixture was stirred at 0 °C for an additional 2 h and quenched by the slow addition of 20 mL of water. The mixture was further diluted with 20 mL of water and extracted with 3 × 20 mL of ethyl acetate. The combined organic extracts were washed with 3 × 10 mL of water, 15 mL of brine, dried (Na₂SO₄), filtered, and the solvent was removed *in vacuo.* The residue was purified by flash chromatography (SiO₂, eluting with 25-95% ethyl acetate/hexanes) to provide 0.65 g (1.37 mmol, 96%) of (*S*)-*N*-((R)-1-(1-methoxyisoquinolin-4-yl)ethyl)-*N*,2-dimethylpropane-2-sulfinamide **(XIVa)** as a single diastereoisomer. ¹H NMR (400 MHz, CDCl₃) δ 8.30 (m, 1H), 7.96-8.06 (m, 2H), 7.70 (m, 1H), 7.56 (m, 1H), 5.17-5.28 (m, 1H), 4.13 (s, 3H), 2.41 (s, 3H), 1.76 (d, 3H), 1.21 (s, 9H). The above detailed reactions were performed on multiple batches with consistent results.
Analogous compounds can be synthesized from intermediate **XIII** utilizing different alkylating agents.

### (R)-4-(1-(Methylamino)ethyl)isoquinolin-1(2H)-one (VIIIf)

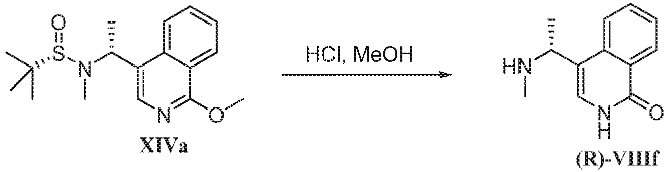

A solution of 1.02 g (3.18 mmol, 1.0 eq.) of diastereomerically pure (*S*)-*N*-((R)-1-(1-methoxy isoquinolin-4-yl)ethyl)-*N*,2-dimethylpropane-2-sulfinamide **(XIVa)** in 64 mL (60 mmol, 25.0 eq.) of a 1.25 M solution of HCl in methanol in a sealed tube was heated at 55 °C for 16 h. The volatiles were removed *in vacuo* to provide a white solid which was suspended in 15 mL of 2-methyl THF and 25 mL of diethyl ether. The mixture was cooled in an ice bath and the resulting white precipitate was collected by vacuum filtration and dried under high vacuum to provide 0.76 g (3.2 mmol, 100%) of (R)-4-(1-(methylamino)ethyl)isoquinolin-1(*2H*)-one hydrochloride **((R)-VIIIf)** as a single enantiomer. ¹H NMR (400 MHz, DMSO-d6) δ 11.62 (d, 1H), 9.64 (s, 1H), 9.13 (s, 1H), 8.26 (m, 1H), 7.94 (d, 1H), 7.78 (m, 1H), 7.51-7.61 (m, 2H), 4.80 (q, 1H), 2.52 (m, 3H, overlapping with DMSO-d₆), 1.58 (d, 3H).

### (R)-3-(3-Chloro-4-fluorophenyl)-1-methyl-1-(1-(1-oxo-1,2-dihydroisoquinolin-4-yl)ethyl)urea (Compound 14)

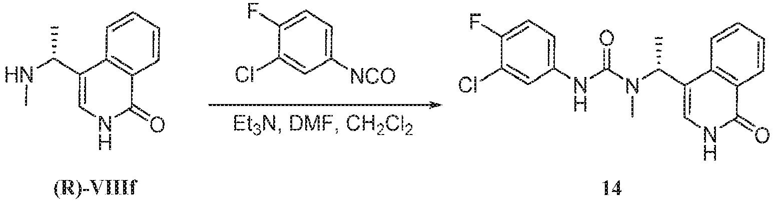

To a mixture of 0.31 g (1.28 mmol, 1.05 eq.) of enantiomerically pure (R)-4-[1-(methylamino)ethyl]-2*H*-isoquinolin-1-one hydrochloride **((R)-VIIIf,** derived from (*S*)-*N-*((R)-1-(1-methoxy isoquinolin-4-yl)ethyl)-2-methylpropane-2-sulfinamide **(XIIIa)** and methyl iodide) in 5 mL of methylene chloride was added 0.56 mL (3.20 mmol, 2.6 eq.) of *N,N-*diisopropylethylamine. The resulting solution was cooled to 0 °C and a solution of 0.15 mL (1.22 mmol, 1.0 eq.) of 2-chloro-1-fluoro-4-isocyanato-benzene in 2.5 mL of methylene chloride was added slowly. The mixture was stirred at 0 °C for 30 minutes and then loaded directly on a preconditioned silica column and purified by flash chromatography (SiO₂, eluting with 0-6% methylene chloride/methanol). The product was dried in a vacuum oven at 50 °C for 16 h to provide 0.43 g (1.10 mmol, 90%) of (R)-3-(3-chloro-4-fluorophenyl)-1-methyl-1-(1-(1-oxo-1,2-dihydroisoquinolin-4-yl)ethyl)urea **(Compound 14).** LCMS: *m*/*z* found 374.1/376.2 [M+H]⁺, RT = 3.99 min (Method A); ¹H NMR (400 MHz, CDCl₃) δ 11.73 (s, 1H), 8.47 (m, 1H), 7.86 (m 1H), 7.74 (m, 1H), 7.67 (m, 1H), 7.56 (m, 1H), 7.23 (m, 2H), 7.08 (t, 1H), 6.35 (s, 1H), 6.07-6.17 (m, 1H), 2.66 (s, 3H), 1.54 (d, 3H).

### X-ray Structure Determination of Compound 14

Crystals of **Compound 14** were grown by vapor diffusion, using ethyl acetate as the solvent and 1:1 v/v diethyl ether:hexanes as the anti-solvent. **Compound 14** (Molecular formula C₁₉H₁₇ClFN₃O₂), crystallizes in the cubic space group I23 (systematic absences hkl: h+k+1=odd) with a=35.3794(15)Å, V=44284(6)Å³, Z=72, and d_{calc}=1.009 g/cm³. X-ray intensity data were collected on a Bruker D8QUEST (APEX3 2018.7-2: Bruker-AXS, Madison, Wisconsin, USA (2016)) CMOS area detector employing graphite-monochromated Mo-Kα radiation (λ=0.71073Å) at a temperature of 100K. Preliminary indexing was performed from a series of twenty-four 0.5° rotation frames with exposures of 10 seconds. A total of 1387 frames were collected with a crystal to detector distance of 50.0 mm, rotation widths of 0.5° and exposures of 40 seconds (Table 1).

**Table 1.**

| **scan type** | **2θ** | **ω** | **ϕ** | **χ** | **Frames** |
|---|---|---|---|---|---|
| ω | 8.52 | 184.90 | 0.00 | 54.72 | 374 |
| ω | 8.52 | 184.90 | 144.00 | 54.72 | 374 |
| ω | 8.52 | 184.90 | 216.00 | 54.72 | 374 |
| ω | 8.52 | 184.90 | 72.00 | 54.72 | 265 |

Rotation frames were integrated using SAINT (SAINT v8.38A: Bruker-AXS Madison, Wisconsin, USA (2014)), producing a listing of unaveraged F² and σ(F²) values. A total of 420033 reflections were measured over the ranges 5.15 ≤ 2θ ≤ 50.686°, -42 ≤ h ≤ 42, -42 ≤ k ≤ 42, -42 ≤ 1 ≤ 42 yielding 13532 unique reflections (Rᵢₙₜ = 0.0598). The intensity data were corrected for Lorentz and polarization effects and for absorption using SADABS (SADABS v2016/2: Krause, L., Herbst-Irmer, R., Sheldrick, G.M. & Stalke, D., J. Appl. Cryst., 48, 3-10 (2015)) (minimum and maximum transmission 0.7110, 0.7452). The structure was solved by direct methods - ShelXT (SHELXT v2014/4: Sheldrick, G.M., Acta Cryst., A, 71, 3-8 (2015)). Refinement was by full-matrix least squares based on F² using SHELXL-2018 (SHELXL-2018/3: Sheldrick, G.M., Acta Cryst., A, 71, 3-8 (2015)). All reflections were used during refinement. The weighting scheme used was w=1/[σ²(Fₒ²)+ (0.0437P)² + 1.0285P] where P = (Fₒ² + 2F_{c}²)/3. Non-hydrogen atoms were refined anisotropically and hydrogen atoms were refined using a riding model. Refinement converged to R1=0.0357 and wR2=0.0849 for 11359 observed reflections for which F > 4σ(F) and R1=0.0544 and wR2=0.0986 and GOF =1.094 for all 13532 unique, non-zero reflections and 718 variables. The maximum Δ/σ in the final cycle of least squares was 0.001 and the two most prominent peaks in the final difference Fourier were +0.18 and -0.17 e/Å³.

Table 2. lists cell information, data collection parameters, and refinement data for **Compound 14.**

**Table 2. Summary of Structure Determination of Compound 14**

| | |
|---|---|
| Empirical formula | C₁₉H₁₇ClFN₃O₂ |
| Formula weight | 373.80 |
| Temperature/K | 100 |
| Crystal system | cubic |
| Space group | I23 |
| a | 35.3794(15)Å |
| Volume | 44284(6)Å³ |
| Z | 72 |
| d_{calc} | 1.009 g/cm³ |
| µ | 0.176 mm⁻¹ |
| F(000) | 13968.0 |
| Crystal size, mm | 0.35 × 0.34 × 0.17 |
| 20 range for data collection | 5.15 - 50.686° |
| Index ranges | -42 ≤ h ≤ 42, -42 ≤ k ≤ 42, -42 ≤ 1 ≤ 42 |
| Reflections collected | 420033 |
| Independent reflections | 13532[R(int) = 0.0598] |
| Data/restraints/parameters | 13532/18/718 |
| Goodness-of-fit on F² | 1.094 |
| Final R indexes [I>=2σ (I)] | R₁ = 0.0357, wR₂ = 0.0849 |
| Final R indexes [all data] | R₁ = 0.0544, wR₂ = 0.0986 |
| Largest diff. peak/hole | 0.18/-0.17 eÅ⁻³ |
| Flack parameter | 0.001(8) |

Final positional and equivalent isotropic thermal parameters for **Compound 14** are given in Table 3.

| **Table 3. Refined Positional Parameters for Compound 14** | | | | |
|---|---|---|---|---|
| **Atom** | ***x*** | ***y*** | ***z*** | **U(eq)** |
| Cl1 | 0.78482(2) | 0.55148(3) | 0.86028(2) | 0.0487(2) |
| F1 | 0.71225(6) | 0.51258(6) | 0.86207(6) | 0.0612(5) |
| O1 | 0.81837(9) | 0.45515(8) | 0.52149(7) | 0.0703(8) |
| O2 | 0.81293(6) | 0.53342(7) | 0.72410(6) | 0.0516(6) |
| N1 | 0.82552(8) | 0.51226(9) | 0.54954(8) | 0.0518(7) |
| N2 | 0.79474(8) | 0.55718(9) | 0.66760(7) | 0.0482(7) |
| N3 | 0.75378(8) | 0.55703(10) | 0.71883(8) | 0.0577(8) |
| C1 | 0.81924(11) | 0.47428(12) | 0.55081(10) | 0.0579(9) |
| C2 | 0.81258(12) | 0.45892(11) | 0.58853(11) | 0.0588(10) |
| C3 | 0.80411(15) | 0.42052(12) | 0.59227(13) | 0.0798(13) |
| C4 | 0.79626(19) | 0.40578(13) | 0.62695(14) | 0.0997(19) |
| C5 | 0.79813(17) | 0.42856(13) | 0.65913(13) | 0.0878(16) |
| C6 | 0.80739(12) | 0.46604(12) | 0.65617(11) | 0.0631(11) |
| C7 | 0.81478(11) | 0.48268(11) | 0.62083(10) | 0.0555(9) |
| C8 | 0.82510(9) | 0.52156(10) | 0.61639(9) | 0.0472(8) |
| C9 | 0.82945(10) | 0.53504(11) | 0.58080(9) | 0.0496(8) |
| C10 | 0.83102(10) | 0.54654(10) | 0.65042(9) | 0.0490(8) |
| C11 | 0.85565(11) | 0.58162(12) | 0.64335(10) | 0.0598(10) |
| C12 | 0.76761(12) | 0.57865(13) | 0.64478(10) | 0.0684(12) |
| C13 | 0.78849(9) | 0.54865(10) | 0.70447(9) | 0.0449(8) |
| C14 | 0.74313(9) | 0.54600(10) | 0.75556(9) | 0.0479(8) |
| C15 | 0.76631(9) | 0.55452(10) | 0.78657(9) | 0.0462(8) |
| C16 | 0.75593(9) | 0.54270(10) | 0.82173(9) | 0.0432(8) |
| C17 | 0.72193(10) | 0.52360(10) | 0.82708(10) | 0.0502(8) |
| C18 | 0.69910(10) | 0.51571(12) | 0.79729(10) | 0.0580(10) |
| C19 | 0.70966(10) | 0.52713(11) | 0.76129(10) | 0.0538(9) |
| Cl1" | 0.76322(7) | 0.69480(8) | 0.63458(8) | 0.0859(8) |
| Cl1' | 0.87508(6) | 0.77777(5) | 0.66351(5) | 0.0645(5) |
| F1' | 0.80149(11) | 0.75238(11) | 0.66912(12) | 0.1429(16) |
| O1' | 0.96691(7) | 0.61531(6) | 0.36667(6) | 0.0467(5) |
| O2' | 0.84975(6) | 0.62396(6) | 0.55183(6) | 0.0424(5) |
| N1' | 0.96543(7) | 0.58465(8) | 0.42278(7) | 0.0390(6) |
| N2' | 0.91144(7) | 0.61972(7) | 0.53452(7) | 0.0355(5) |
| N3' | 0.89314(8) | 0.66121(7) | 0.58164(7) | 0.0403(6) |
| C1' | 0.95115(9) | 0.60974(9) | 0.39786(9) | 0.0405(7) |
| C2' | 0.91692(9) | 0.62933(8) | 0.40973(9) | 0.0383(7) |
| C3' | 0.89953(10) | 0.65416(9) | 0.38408(10) | 0.0473(8) |
| C4' | 0.86713(11) | 0.67259(10) | 0.39461(11) | 0.0564(9) |
| C5' | 0.85138(10) | 0.66685(10) | 0.43019(10) | 0.0499(8) |
| C6' | 0.86795(9) | 0.64224(9) | 0.45530(9) | 0.0417(7) |
| C7' | 0.90080(9) | 0.62216(8) | 0.44567(9) | 0.0378(7) |
| C8' | 0.91899(8) | 0.59522(8) | 0.47030(9) | 0.0375(7) |
| C9' | 0.94987(8) | 0.57706(9) | 0.45761(8) | 0.0382(7) |
| C10' | 0.90295(8) | 0.58721(8) | 0.50981(9) | 0.0384(7) |
| C11' | 0.91641(10) | 0.55046(9) | 0.52768(9) | 0.0464(8) |
| C12' | 0.95133(9) | 0.62775(9) | 0.54282(9) | 0.0416(7) |
| C13' | 0.88298(9) | 0.63441(8) | 0.55587(8) | 0.0361(7) |
| C14' | 0.86790(10) | 0.68351(9) | 0.60340(8) | 0.0433(7) |
| C15' | 0.88403(12) | 0.71406(9) | 0.62256(9) | 0.0505(9) |
| C16' | 0.86139(16) | 0.73719(12) | 0.64440(12) | 0.0748(13) |
| C17' | 0.82349(16) | 0.72999(14) | 0.64810(15) | 0.0873(16) |
| C18' | 0.80744(13) | 0.69994(12) | 0.62870(13) | 0.0703(11) |
| C19' | 0.82931(11) | 0.67692(11) | 0.60695(10) | 0.0548(9) |
| Cl1* | 0.91438(4) | 0.73141(3) | 0.75842(3) | 0.0813(4) |
| F1* | 0.89117(7) | 0.65460(7) | 0.73693(7) | 0.0771(7) |
| O1* | 1.04816(7) | 0.89383(6) | 0.52976(6) | 0.0469(5) |
| O2* | 0.97392(6) | 0.69790(6) | 0.58997(6) | 0.0434(5) |
| N1* | 1.06308(7) | 0.83309(7) | 0.51584(7) | 0.0397(6) |
| N2* | 1.02958(7) | 0.72995(7) | 0.58816(7) | 0.0403(6) |
| N3* | 1.00249(8) | 0.71462(7) | 0.64551(7) | 0.0423(6) |
| C1* | 1.04147(9) | 0.85944(9) | 0.53335(8) | 0.0390(7) |
| C2* | 1.01032(9) | 0.84491(9) | 0.55618(8) | 0.0393(7) |
| C3* | 0.98692(9) | 0.87066(10) | 0.57504(9) | 0.0451(8) |
| C4* | 0.95634(10) | 0.85776(10) | 0.59537(9) | 0.0493(8) |
| C5* | 0.94836(10) | 0.81940(11) | 0.59605(10) | 0.0532(9) |
| C6* | 0.97101(9) | 0.79372(10) | 0.57810(9) | 0.0437(8) |
| C7* | 1.00377(8) | 0.80573(9) | 0.55816(8) | 0.0372(7) |
| C8* | 1.03013(8) | 0.78000(9) | 0.54051(8) | 0.0375(7) |
| C9* | 1.05822(9) | 0.79468(9) | 0.51992(8) | 0.0377(7) |
| C10* | 1.02614(9) | 0.73744(9) | 0.54734(8) | 0.0399(7) |
| C11* | 1.05356(10) | 0.71287(9) | 0.52499(9) | 0.0472(8) |
| C12* | 1.06268(9) | 0.74610(9) | 0.60731(9) | 0.0418(7) |
| C13* | 1.00075(9) | 0.71297(8) | 0.60677(9) | 0.0389(7) |
| C14* | 0.97390(9) | 0.69853(9) | 0.66864(9) | 0.0423(7) |
| C15* | 0.96035(10) | 0.71965(10) | 0.69863(9) | 0.0482(8) |
| C16* | 0.93237(11) | 0.70487(10) | 0.72162(9) | 0.0540(9) |
| C17* | 0.91883(11) | 0.66915(11) | 0.71483(10) | 0.0547(9) |
| C18* | 0.93272(10) | 0.64750(10) | 0.68581(10) | 0.0492(8) |
| C19* | 0.96076(9) | 0.66216(9) | 0.66232(9) | 0.0423(7) |

The ORTEP representation of **Compound 14** defining the absolute configuration of **Compound 14** as (R)- (and consequently the absolute configuration of the α-methyl substituent of the major diastereoisomer of **XIIIa** as (R)-) is shown in FIG. 1.

### (R)-3-(4-Fluoro-3-methylphenyl)-1-methyl-1-(1-(1-oxo-1,2-dihydroisoquinolin-4-yl)ethyl)urea (Compound 32)

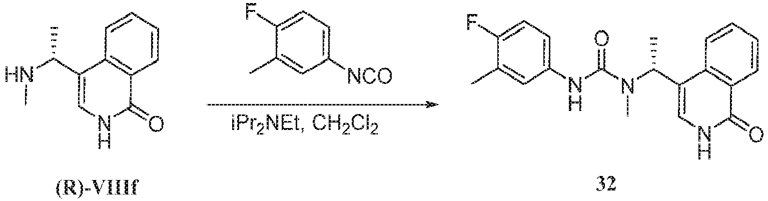

Enantiopure (R)-3-(4-fluoro-3-methylphenyl)-1-methyl-1-(1-(1-oxo-1,2-dihydroisoquinolin-4-yl)ethyl)urea **(Compound 32)** was synthesized in an analogous manner as described above from enantiopure (R)-4-(1-(methylamino)ethyl)isoquinolin-1(2*H*)-one hydrochloride **((R)-VIIIf,** derived from (*S*)-*N*-((R)-1-(1-methoxy isoquinolin-4-yl)ethyl)-2-methylpropane-2-sulfinamide **(XIIIa)** and methyl iodide) and 4-fluoro-3-methylphenyl isocyanate. LCMS: *m*/*z* found 354.2 [M+H]⁺, RT = 3.61 min (Method A); ¹H NMR (400 MHz, CDCl₃) δ 11.37 (s, 1H), 8.44-8.51 (m, 1H), 7.90 (d, 1H), 7.74 (m, 1H), 7.55 (m, 1H), 7.30-7.38 (m, 1H), 7.22 (d, 1H), 7.14 (m, 1H), 6.95 (t, 1H), 6.23 (s, 1H), 6.14 (m, 1H), 2.66 (s, 3H), 2.28 (d, 3H), 1.54 (d, 3H).

### (R)-3-(4-Fluoro-3-(trifluoromethyl)phenyl)-1-methyl-1-(1-(1-oxo-1,2-dihydroisoquinolin-4-yl)ethyl)urea (Compound 89)

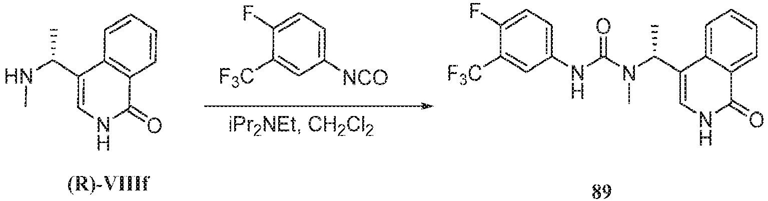

(R)-3-(4-Fluoro-3-(trifluoromethyl)phenyl)-1-methyl-1-(1-(1-oxo-1,2-dihydroisoquinolin-4-yl)ethyl)urea **(Compound 89)** was synthesized in an analogous manner as described above from enantiopure (R)-4-(1-(methylamino)ethyl)isoquinolin-1(2*H*)-one hydrochloride **((R)-VIIIf,** derived from (*S*)-*N*-((R)-1-(1-methoxy isoquinolin-4-yl)ethyl)-2-methylpropane-2-sulfinamide **(XIIIa)** and methyl iodide) and 4-fluoro-3-trifluoromethylphenyl isocyanate. LCMS: *m*/*z* found 408.1 [M+H]⁺, RT = 4.67 min (Method A); ¹H NMR (400 MHz, CDCl₃) δ 11.72 (s, 1H), 8.45-8.58 (m, 2H), 7.86 (dd, 1H), 7.70-7.80 (m, 1H), 7.57 (ddd, 1H), 7.22-7.31 (m, 3H), 6.68 (d, 1H), 6.13 (q, 1H), 2.72 (s, 3H), 1.57 (d, 3H).

### (R)-3-(4-Fluorophenyl)-1-methyl-1-(1-(1-oxo-1,2-dihydroisoquinolin-4-yl)ethyl)urea (Compound 90)

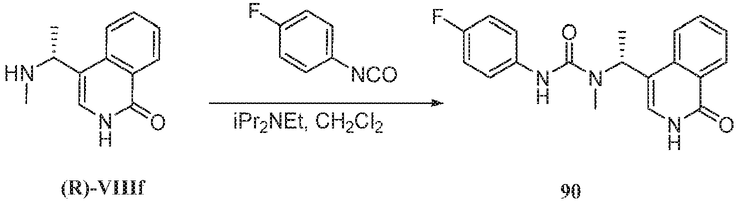

(R)-3-(4-Fluorophenyl)-1-methyl-1-(1-(1-oxo-1,2-dihydroisoquinolin-4-yl)ethyl)urea **(Compound 90)** was synthesized in an analogous manner as described above from enantiopure (R)-4-(1-(methylamino)ethyl)isoquinolin-1(2*H*)-one hydrochloride **((R)-VIIIf** derived from (*S*)-*N*-((R)-1-(1-methoxy isoquinolin-4-yl)ethyl)-2-methylpropane-2-sulfinamide **(XIIIa)** and methyl iodide) and 4-fluorophenyl isocyanate. LCMS: *m*/*z* found 340.2 [M+H]⁺, RT = 4.13 min (Method A); ¹H NMR (400 MHz, CDCl₃) δ 11.63 (s, 1H), 8.44-8.51 (m, 1H), 7.87-7.94 (m, 1H), 7.74 (m, 1H), 7.55 (m, 1H), 7.35-7.45 (m, 2H), 7.21-7.24 (m, 1H), 6.97-7.08 (m, 2H), 6.31 (s, 1H), 6.14 (m, 1H), 2.66 (s, 3H), 1.54 (d, 3H).

### (R)-3-(4-Chlorophenyl)-1-methyl-1-(1-(1-oxo-1,2-dihydroisoquinolin-4-yl)ethyl)urea (Compound 293)

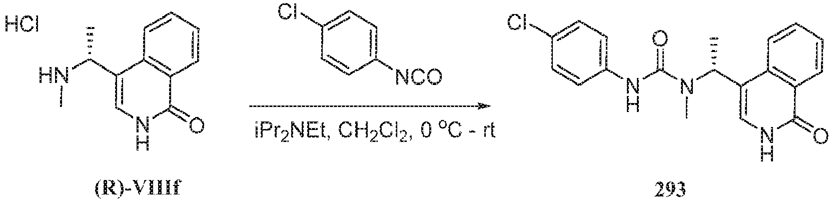

(R)-3-(4-Chlorophenyl)-1-methyl-1-(1-(1-oxo-1,2-dihydroisoquinolin-4-yl)ethyl)urea was synthesized in a similar manner as described above from enantiomerically pure (R)-4-[1-(methylamino)ethyl]-2*H*-isoquinolin-1-one hydrochloride **((R)-VIIIf,** derived from (*S*)-*N-*((R)-1-(1-methoxy isoquinolin-4-yl)ethyl)-2-methylpropane-2-sulfinamide **(XIIIa)** and methyl iodide) and 4-chlorophenyl isocyanate. LCMS: *m*/*z* found 356.2/358.2 [M+H]⁺, RT = 3.77 min (Method A); ¹H NMR (400 MHz, CDCl₃): δ 11.30 (s, 1H), 8.47 (dd, 1H), 7.88 (d, 1H), 7.73 (ddd, 1H), 7.55 (ddd, 1H), 7.37-7.45 (m, 2H), 7.19-7.33 (m, 3H), 6.33 (s, 1H), 6.13 (q, 1H), 2.67 (s, 2H), 1.54 (d, 3H).

### (R)-3-(4-Bromophenyl)-1-methyl-1-(1-(1-oxo-1,2-dihydroisoquinolin-4-yl)ethyl)urea (Compound 294)

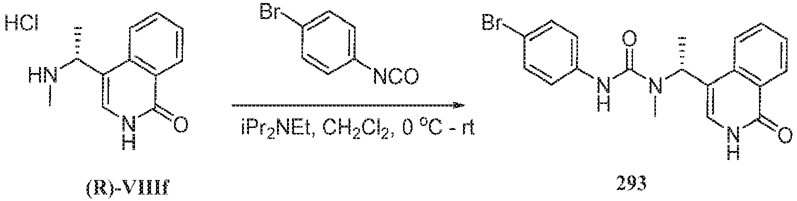

(R)-3-(4-Bromophenyl)-1-methyl-1-(1-(1-oxo-1,2-dihydroisoquinolin-4-yl)ethyl)urea was synthesized in a similar manner as described above from enantiomerically pure (R)-4-[1-(methylamino)ethyl]-2*H*-isoquinolin-1-one hydrochloride **((R)-VIIIf,** derived from (*S*)-*N-*((R)-1-(1-methoxy isoquinolin-4-yl)ethyl)-2-methylpropane-2-sulfinamide (**XIIIa**) and methyl iodide) and 4-chlorophenyl isocyanate. LCMS: *m*/*z* found 400.1/402.1 [M+H]⁺, RT = 3.91 min (Method A); ¹H NMR (400 MHz, CDCl₃): δ 11.24 (s, 1H), 8.51-8.43 (m, 1H), 7.87 (d, 1H), 7.68-7.78 (m, 1H), 7.55 (ddt, 1H), 7.39-7.47 (m, 2H), 7.40-7.32 (m, 2H), 7.21 (d, 1H), 6.33 (s, 1H), 6.13 (q, 1H), 2.66 (s, 3H), 1.52 (d, 3H).

### (R)-1-Methyl-1-(1-(1-oxo-1,2-dihydroisoquinolin-4-yl)ethyl)-3-(3,4,5-trifluorophenyl)urea (Compound 91)

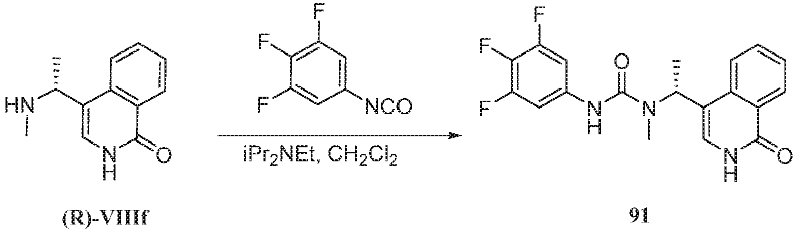

(R)-1-Methyl-1-(1-(1-oxo-1,2-dihydroisoquinolin-4-yl)ethyl)-3-(3,4,5-trifluorophenyl)urea (**Compound 91**) was synthesized in an analogous manner as described above from enantiopure (R)-4-(1-(methylamino) ethyl)isoquinolin-1(2*H*)-one hydrochloride ((**R**)-**VIIIf**, derived from (*S*)-*N*-((R)-1-(1-methoxy isoquinolin-4-yl)ethyl)-2-methylpropane-2-sulfinamide (**XIIIa**) and methyl iodide) and 3,4,5-trifluorophenyl isocyanate. LCMS: *m*/*z* found 376.2 [M+H]⁺, RT = 5.00 min (Method A); ¹H NMR (400 MHz, DMSO-d₆) δ 11.42 (d, 1H), 8.62 (s, 1H), 8.24 (m, 1H), 7.74 (m, 1H), 7.63-7.70 (m, 1H), 7.45-7.60 (m, 3H), 7.16 (d, 1H), 5.83 (m, 1H), 2.58 (s, 3H), 1.43 (d, 3H).

### (R)-3-(3,4-Difluorophenyl)-1-methyl-1-(1-(1-oxo-1,2-dihydroisoquinolin-4-yl)ethyl)urea (Compound 92)

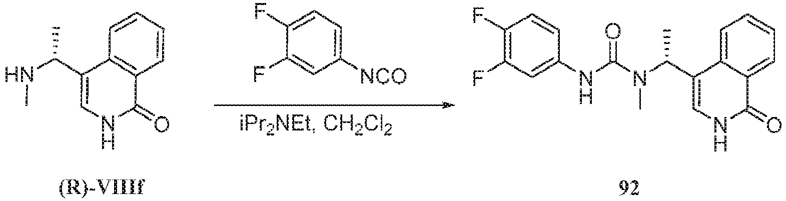

(R)-3-(3,4-Difluorophenyl)-1-methyl-1-(1-(1-oxo-1,2-dihydroisoquinolin-4-yl)ethyl)urea (**Compound 92**) was synthesized in an analogous manner as described above from enantiopure (R)-4-(1-(methylamino)ethyl)isoquinolin-1(2*H*)-one hydrochloride ((**R**)-**VIIIf,** derived from (*S*)-*N*-((R)-1-(1-methoxy isoquinolin-4-yl)ethyl)-2-methylpropane-2-sulfinamide (**XIIIa**) and methyl iodide) and 3,4-difluorophenyl isocyanate. LCMS: *m*/*z* found 358.2 [M+H]⁺, RT = 4.73 min (Method A); ¹H NMR (400 MHz, CDCl₃) δ 11.73 (s, 1H), 8.47 (m, 1H), 7.86 (d, 1H), 7.74 (m, 1H), 7.511-7.62 (m, 2H), 7.24 (d, 1H), 7.09 (m, 1H), 6.95-7.04 (m, 1H), 6.37 (s, 1H), 6.12 (m, 1H), 2.66 (s, 3H), 1.54 (d, 3H).

### (R)-3-(3-Fluorophenyl)-1-methyl-1-(1-(1-oxo-1,2-dihydroisoquinolin-4-yl)ethyl)urea (Compound 93)

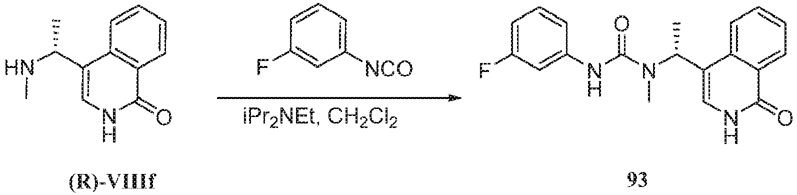

(R)-3-(3-Fluorophenyl)-1-methyl-1-(1-(1-oxo-1,2-dihydroisoquinolin-4-yl)ethyl)urea (**Compound 93**) was synthesized in an analogous manner as described above from enantiopure (R)-4-(1-(methylamino)ethyl)isoquinolin-1(2*H*)-one hydrochloride **((R)-VIIIf,** derived from (*S*)-*N*-((R)-1-(1-methoxy isoquinolin-4-yl)ethyl)-2-methylpropane-2-sulfinamide (**XIIIa**) and methyl iodide) and 3-fluorophenyl isocyanate. LCMS: *m*/*z* found 340.2 [M+H]⁺, RT = 4.40 min (Method A); ¹ H NMR (400 MHz, CDCl₃) δ 11.61 (s, 1H), 8.47 (m, 1H), 7.88 (m, 1H), 7.69-7.79 (m, 1H), 7.55 (m, 1H), 7.46 (m, 1H), 7.20-7.30 (m, 2H), 7.07 (m, 1H), 6.77 (m, 1H), 6.43 (s, 1H), 6.14 (m, 1H), 2.66 (s, 3H), 1.54 (d, 3H).

### (R)-3-(3-Chloro-4-fluorophenyl)-1-(1-(1-oxo-1,2-dihydroisoquinolin-4-yl)ethyl)-1-propylurea (Compound 35)

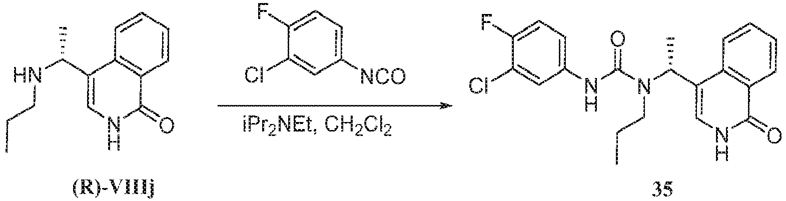

(R)-3-(3-Chloro-4-fluorophenyl)-1-(1-(1-oxo-1,2-dihydroisoquinolin-4-yl)ethyl)-1-propylurea (**Compound 35**) was synthesized in an analogous manner as described above from enantiopure (R)-4-(1-(propylamino) ethyl)isoquinolin-1(2*H*)-one **((R)-VIIIj,** derived from (*S*)-*N*-((R)-1-(1-methoxyisoquinolin-4-yl)ethyl)-2-methylpropane-2-sulfinamide (**XIIIa**) and 1-iodopropane) and 2-chloro-1-fluoro-4-isocyanato-benzene. LCMS: *m*/*z* found 402.1/404.1 [M+H]⁺, RT = 4.63 min (Method A); ¹H NMR (400 MHz, CDCl₃) δ 11.65 (s, 1H), 8.48 (ddd, 1H), 7.84 (dt, 1H), 7.75 (ddd, 1H), 7.64 (dd, 1H), 7.56 (ddd, 1H), 7.18-7.31 (m, 2H), 7.09 (t, 1H), 6.25 (s, 1H), 6.13 (q, 1H), 2.95-3.06 (m, 1H), 2.87-2.98 (m, 1H), 1.55 (d, 3H), 1.22-1.43 (m, 1H), 1.01 (m, 1 H), 0.70 (t, 3H).

### (R)-3-(4-Fluoro-3-methylphenyl)-1-(1-(1-oxo-1,2-dihydroisoquinolin-4-yl)ethyl)-1-propylurea (Compound 43)

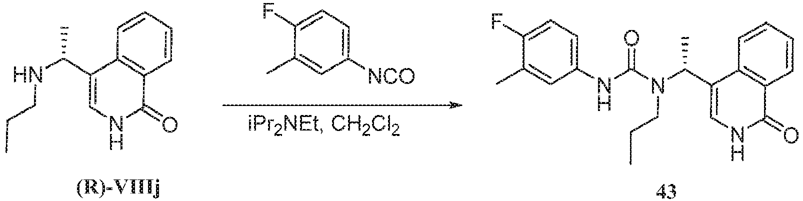

(R)- 3-(4-fluoro-3-methylphenyl)-1-(1-(1-oxo-1,2-dihydroisoquinolin-4-yl)ethyl)-1-propylurea (**Compound 43**) was synthesized in an analogous manner as described above from enantiopure (R)-4-(1-(propylamino)ethyl)isoquinolin-1(2*H*)-one **((R)-VIIIj,** derived from (*S*)-*N*-((R)-1-(1-methoxy isoquinolin-4-yl)ethyl)-2-methylpropane-2-sulfinamide (**XIIIa**) and 1-iodopropane) and 4-fluoro-3-methylphenyl isocyanate. LCMS: *m*/*z* found 382.3 [M+H]⁺, RT = 4.41 min (Method A); ¹ H NMR (400 MHz, CDCl₃) δ 11.43 (s, 1H), 8.47 (m, 1H), 7.87 (d, 1H), 7.74 (m, 1H), 7.55 (m, 1H), 7.22-7.36 (m, 2H), 7.09-7.18 (m, 1H), 6.96 (t, 1H), 6.1-6.21 (m, 2H), 2.96 (m, 2H), 2.28 (d, 3H), 1.56 (d, 3H), 1.35 (m, 1H), 1.01 (m, 1H), 0.70 (t, 3H).

### (R)-2,2,2-Trifluoro-N-[1-(1-methoxy-4-isoquinolyl)ethyl]ethanamine (VIbn)

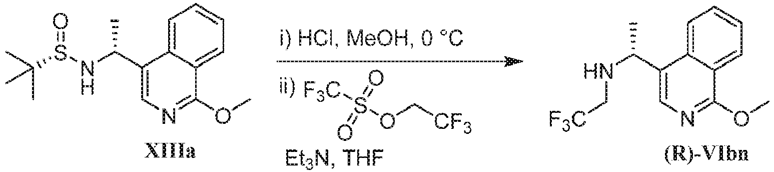

To a solution of 0.24 g (0.78 mmol, 1.0 eq.) of (S)-*N*-[(R)-1-(1-methoxy-4-isoquinolyl)ethyl]-2-methyl-propane-2-sulfinamide (**XIIIa**) in 10 mL of anhydrous methanol at 0 °C was added 2.74 mL (2.74 mmol, 3.5 eq.) of a 1 M solution of HCl in methanol. The mixture was allowed to warm to room temperature and stirred for 30 min. An additional portion of 2.0 mL (2.0 mmol, 2.6 eq.) of a 1 M solution of HCl in methanol was added and stirring was continued for a further 1 h. The mixture was then diluted with 20 mL of saturated sodium bicarbonate solution and extracted with 3 x 25 mL of ethyl acetate. The combined organic extracts were washed with 20 mL of water, 20 mL of brine, dried (Na₂SO₄), filtered and the solvent was removed *in vacuo* to provide 0.18 g of 1-(1-methoxy-4-isoquinolyl)ethanamine, The product was redissolved in 7 mL of anhydrous THF and 0.25 mL (1.78 mmol, 2.3 eq.) of triethylamine was added, followed by 0.16 mL (1.11 mmol, 1.4 eq.) of 2,2,2-trifluoroethyl trifluoromethanesulfonate and the mixture was stirred at room temperature for 16 h. An additional portion of 0.1 mL (0.72 mmol, 0.9 eq.) of 2,2,2-trifluoroethyl trifluoromethanesulfonate was added and stirring was continued for a further 16 h. The mixture was then diluted with 25 mL of ethyl acetate, washed with 8 mL of water, 8 mL of brine, dried (Na₂SO₄), filtered, and the solvent was removed *in vacuo.* The residue was purified by flash chromatography (SiO₂, eluting with a gradient of 10-30% ethyl acetate/hexanes) to provide 0.15 g (0.53 mmol, 68%) of (R)-2,2,2-trifluoro-N [1-(1-methoxy-4-isoquinolyl)ethyl]ethanamine **((R)-VIbn).** ¹HNMR (400 MHz, CDCl₃) δ 8.31 (m, 1H), 8.14-8.22 (m, 1H), 8.07 (d, 1H), 7.70 (m, 1H), 7.55 (m, 1H), 4.53 (q, 1H), 4.13 (s, 3H), 3.14 (m, 2H), 1.55 (d, 3H).

### (R)-3-(3-Chloro-4-fluorophenyl)-1-(1-(1-methoxyisoquinolin-4-yl)ethyl)-1-(2,2,2-trifluoroethyl)urea (Compound 101)

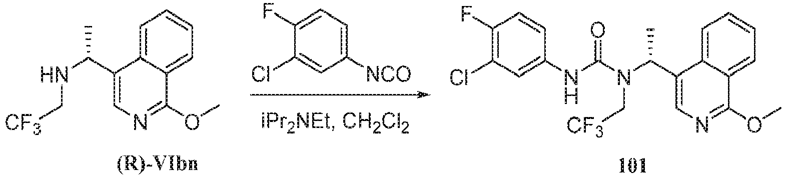

(R)-3-(3-chloro-4-fluorophenyl)-1-(1-(1-methoxyisoquinolin-4-yl)ethyl)-1-(2,2,2-trifluoroethyl)urea (**Compound 101**) was synthesized in a similar manner as described above from (R)-2,2,2-trifluoro-N-[1-(1-methoxy-4-isoquinolyl)ethyl]ethanamine **((R)-VIbn,** derived from (S)-*N*-((R)-1-(1-methoxyisoquinolin-4-yl)ethyl)-2-methylpropane-2-sulfinamide) and 2-chloro-1-fluoro-4-isocyanato-benzene. LCMS: *m*/*z* found 456.2/458.3 [M+H]⁺, RT = 7.22 min (Method A); ¹H NMR (400 MHz, CDCl₃) δ 8.32 (m, 1H), 8.07-8.13 (m, 1H), 7.81 (m, 1H), 7.74 (m, 1H), 7.55-7.64 (m, 2H), 7.17 (m, 1H), 7.10 (t, 1H), 6.56 (s, 1H), 6.18 (q, 1H), 4.16 (s, 3H), 3.73-3.89 (m, 1H), 3.54 (m, 1H), 1.76 (d, 3H).

### (R)-3-(3-Chloro-4-fluorophenyl)-1-(2,2-difluoroethyl)-1-(1-(1-oxo-1,2-dihydroisoquinolin-4-yl)ethyl)urea (Compound 96)

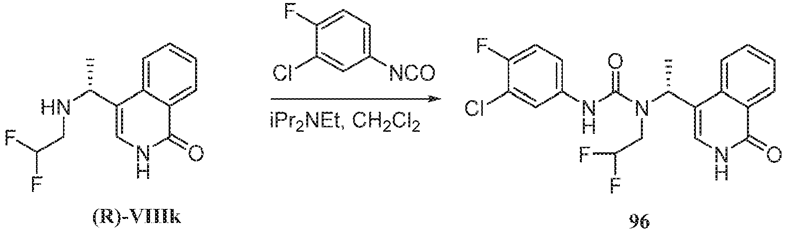

(R)-3-(3-Chloro-4-fluorophenyl)-1-(2,2-difluoroethyl)-1-(1-(1-oxo-1,2-dihydroisoquinolin-4-yl)ethyl)urea (**Compound 96**) was synthesized in an analogous as described above manner from enantiopure (R)-4-(1-((2,2-difluoroethyl)amino)ethyl) isoquinolin-1(2*H*)-one hydrochloride **((R)-VIIIk))** (derived from (*S*)-*N*-((R)-1-(1-methoxy isoquinolin-4-yl)ethyl)-2-methylpropane-2-sulfinamide (**XIIIa**) and 2,2-difluoroethyl trifluoromethanesulfonate) and 3-chloro-4-fluorophenyl isocyanate. LCMS: *m*/*z* found 424.1/426.2 [M+H]⁺, RT = 5.84 min (Method A); ¹H NMR (400 MHz, CDCl₃) δ 11.54 (s, 1H), 8.50 (m, 1H), 7.78 (m, 1H), 7.70 (d, 1H), 7.56-7.65 (m, 2H), 7.29 (d, 1H), 7.18 (m, 1H), 7.10 (t, 1H), 6.82 (d, 1H), 6.07 (d, 1H), 4.87-5.15 (m, 1H), 3.38-3.57 (m, 2H), 1.61 (m, 2H).

### (R)-3-(3-Methyl-4-fluorophenyl)-1-(2,2-difluoroethyl)-1-(1-(1-oxo-1,2-dihydroisoquinolin-4-yl)ethyl)urea (Compound 97)

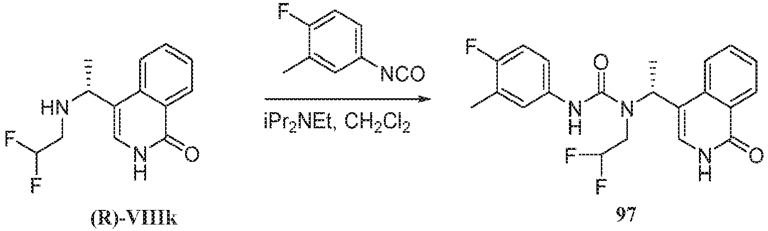

(R)-3-(3-Chloro-4-fluorophenyl)-1-(2,2-difluoroethyl)-1-(1-(1-oxo-1,2-dihydroisoquinolin-4-yl) ethyl)urea (**Compound 97**) was synthesized in an analogous manner from (R)-4-(1-((2,2-difluoroethyl)amino)ethyl)isoquinolin-1(2*H*)-one hydrochloride **((R)-VIIIk,** derived from (*S*)-*N*-((R)-1-(1-methoxy isoquinolin-4-yl)ethyl)-2-methylpropane-2-sulfinamide (**XIIIa**) and 2,2-difluoroethyl trifluoromethanesulfonate) and 3-methyl-4-fluorophenyl isocyanate. LCMS: *m*/*z* found 404.2 [M+H]⁺, RT = 5.52 min (Method A); ¹H NMR (400 MHz, CDCl₃) δ 11.61 (s, 1H), 8.46-8.53 (m, 1H), 7.69-7.82 (m, 2H), 7.60 (m, 1H), 7.21-7.32 (m, 2H), 7.12 (m, 1H), 6.97 (t, 1H), 6.73 (t, 1H), 6.07 (q, 1H), 4.89-5.17 (m, 1H), 3.37-3.58 (m, 2H), 2.28 (d, 3H), 1.60 (d, 3H).

### (R)-3-(3-Chloro-4-fluorophenyl)-1-(ethyl-d₅)-1-(1-(1-oxo-1,2-dihydroisoquinolin-4-yl)ethyl)urea (Compound 99)

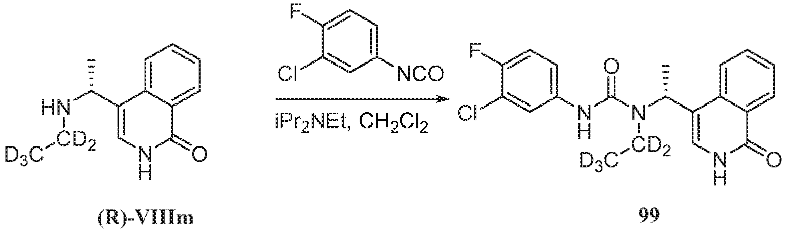

(R)-3-(3-Chloro-4-fluorophenyl)-1-(ethyl-d₅)-1-(1-(1-oxo-1,2-dihydroisoquinolin-4-yl)ethyl)urea (**Compound 99**) was synthesized in an analogous as described above manner from enantiopure (R)-4-(1-((ethyl-d₅)amino)ethyl)isoquinolin-1(2*H*)-one hydrochloride **((R)-VIIIm,** derived from (*S*)-*N*-((R)-1-(1-methoxy isoquinolin-4-yl)ethyl)-2-methylpropane-2-sulfinamide (**XIIIa**) and 1-bromo-1,1,2,2,2-pentadeuterio-ethane) and 3-chloro-4-fluorophenyl isocyanate. LCMS: *m*/*z* found 393.2/395.2 [M+H]⁺, RT = 6.01 min (Method A); ¹H NMR (400 MHz, CDCl₃) δ 11.24 (s, 1H), 8.47 (m, 1H), 7.85 (d, 1H), 7.70-7.80 (m, 1H), 7.66 (m, 1H), 7.51-7.60 (m, 1H), 7.19-.29 (m, 2H), 7.09 (t, 1H), 6.25 (s, 1H), 6.13 (m, 1H), 1.52 (d, 3H).

### (R)-1-Methyl-1-(1-(1-oxo-1,2-dihydroisoquinolin-4-yl)ethyl)-3-(2-(trifluoromethyl)pyridin-4-yl)urea (Compound 95)

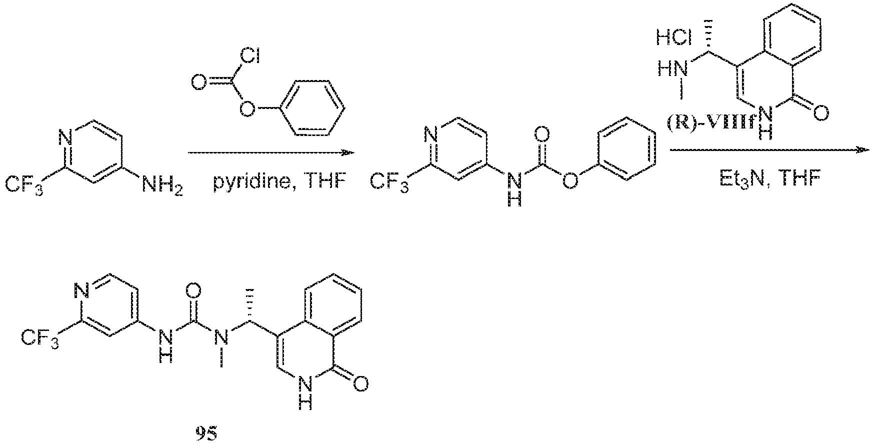

To a solution of 0.83 g (5.12 mmol, 1.0 eq.) of 2-(trifluoromethyl)pyridin-4-amine in 10 mL of anhydrous THF at 0 °C under a nitrogen atmosphere was added 1.66 mL (20.48 mmol, 4.0 eq.) of pyridine followed by the slow addition of 0.71 mL (5.63 mmol, 1.1 eq.) of phenyl chloroformate. The mixture was allowed to warm to room temperature and stirred for 1h. The mixture was then diluted with 25 mL of water and extracted with 3 x 50 mL of ethyl acetate. The combined organic extracts were washed with 10 mL of water and 10 mL of brine, dried (Na₂SO₄), filtered, and the solvent was removed *in vacuo.* The residue was purified by flash chromatography (40g SiO₂, eluting with 10-70% ethyl acetate/hexanes) to provide 1.41 g (4.0 mmol, 97%) of (phenyl (2-(trifluoromethyl)pyridin-4-yl)carbamate. ¹H NMR (400 MHz, CDCl₃) δ 8.63 (d, 1H), 7.85 (d, 1H), 7.58 (dd, 1H), 7.38-7.48 (m, 2H), 7.25-7.35 (m, 1H), 7.15-7.30 (m, 2H).

To a mixture of 53 mg (0.22 mmol, 1.1 eq.) of (R)-4-[1-(methylamino)ethyl]-2H-isoquinolin-1-one hydrocloride **((R)-VIIIf,** derived from (*S*)-*N*-((R)-1-(1-methoxy isoquinolin-4-yl)ethyl)-2-methylpropane-2-sulfinamide (**XIIIa**) and methyl iodide) in 2 mL of anhydrous THF was added 77 µL (0.56 mmol, 2.8 eq.) of triethylamine followed by 56 mg (0.2 mmol, 1.0 eq.) of phenyl (2-(trifluoromethyl) pyridin-4-yl)carbamate and the mixture was stirred at room temperature for 60 h. The reaction mixture was loaded directly onto preconditioned 12 g silica column and the product was isolated by flash chromatography (SiO₂, eluting with 1-8% methanol/methylene chloride) to provide 58 mg (0.13 mmol, 67%) of (R)-(1-methyl-1-[1-(1-oxo-2*H*-isoquinolin-4-yl)ethyl]-3-[2-(trifluoromethyl)-4-pyridyl]urea (**Compound 95**, Enantiomer II). LCMS: *m*/*z* found 391.1 [M+H]⁺, RT = 4.66 min (Method A); ¹H NMR (400 MHz, CD₃OD) δ 8.47 (d, 1H), 8.37 (m, 1H), 8.11 (d, 1H), 7.70-7.83 (m, 3H), 7.55 (m, 1H), 7.28 (d, 1H), 6.00 (m, 1H), 2.70 (s, 3H), 1.58 (d, 3H).

### (R)-3-(2-Chloropyridin-4-yl)-1-methyl-1-(1-(1-oxo-1,2-dihydroisoquinolin-4-yl)ethyl)urea (Compound 94)

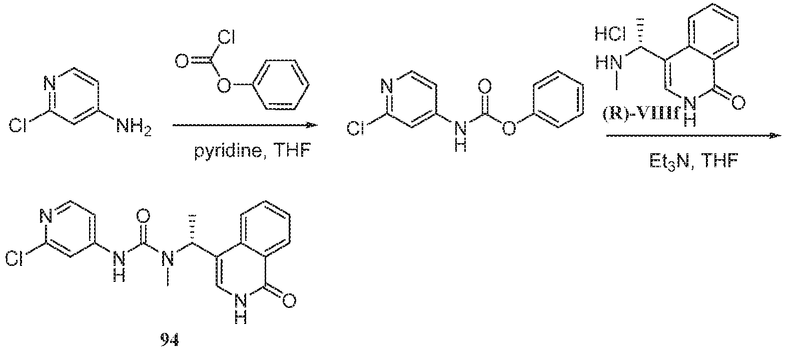

(R)-3-(2-Chloropyridin-4-yl)-1-methyl-1-(1-(1-oxo-1,2-dihydroisoquinolin-4-yl)ethyl)urea (**Compound 94**) was synthesized in an analogous manner as described above from (R)-4-(1-(methylamino)ethyl)isoquinolin-1(2H)-one hydrochloride **((R)-VIIIf,** derived from (S)-N-((R)-1-(1-methoxy isoquinolin-4-yl)ethyl)-2-methylpropane-2-sulfinamide (**XIIIa**)) and methyl iodide and 2-chloropyridin-4-amine. LCMS: *m*/*z* found 357.1/359.1 [M+H]⁺, RT = 4.03 min (Method A); ¹H NMR (400 MHz, DMSO-d₆) δ 11.43 (d, 1H), 8.95 (s, 1H), 8.24 (m, 1H), 8.16 (d, 1H), 7.69-7.80 (m, 2H), 7.60-7.67 (m, 1H), 7.46-7.58 (m, 2H), 7.14-7.21 (m, 1H), 5.83 (m, 1H), 2.61 (s, 3H), 1.45 (d, 3H).

### (R)-3-(3-Cyano-4-fluorophenyl)-1-methyl-1-(1-(1-oxo-1,2-dihydroisoquinolin-4-yl)ethyl)urea (Compound 98)

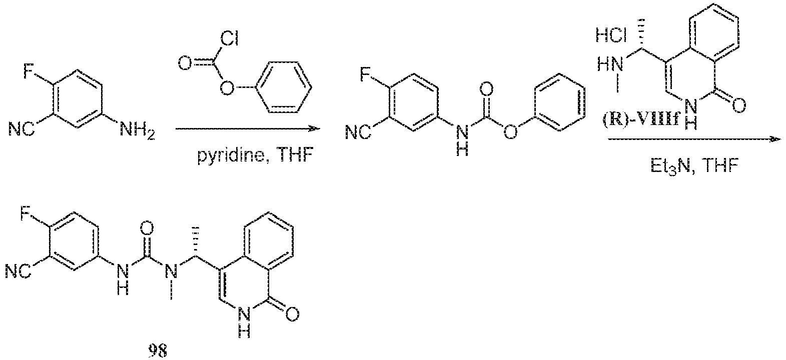

(R)-3-(3-Cyano-4-fluorophenyl)-1-methyl-1-(1-(1-oxo-1,2-dihydroisoquinolin-4-yl)ethyl)urea (**Compound 98**) was synthesized in an analogous manner as described above from (R)-4-(1-(methylamino)ethyl)isoquinolin-1(2*H*)-one hydrochloride **((R)-VIIIf,** derived from (*S*)-*N*-((R)-1-(1-methoxy isoquinolin-4-yl)ethyl)-2-methylpropane-2-sulfinamide (**XIIIa**)) and methyl iodide and 5-amino-2-fluorobenzonitrile. LCMS: *m*/*z* found 365.2 [M+H]⁺, RT = 4.95 min (Method A); ¹H NMR (400 MHz, CDCl₃) δ 11.43 (s, 1H), 8.47 (m, 1H), 7.80-7.88 (m, 2H), 7.74 (m, 1H), 7.64 (m, 1H), 7.56 (m, 1H), 7.24 (d, 1H), 7.17 (m, 1H), 6.47 (s, 1H), 6.12 (m, 1H), 2.68 (s, 3H), 1.58 (d, 3H).

### (R)-N-(Cyclopropyl(1-methoxyisoquinolin-4-yl)methyl)-2-methylpropane-2-sulfinamide (XIIIc)

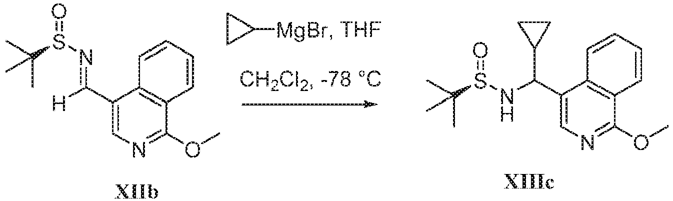

To a solution of 0.5 g (5.17 mmol, 1.0 e) of (R)-N((1-methoxyisoquinolin-4-yl)methylene)-2-methylpropane-2-sulfinamide (**XIIb,** derived from **Va** and (R)-2-methylpropane-2-sulfinamide) in 10 mL of methylene chloride at -78 °C under a nitrogen atmosphere was added 35 mL (17.2 mmol, 10.0 eq.) of a 0.5 M solution of cyclopropylmagnesium bromide in THF and the mixture was stirred at -78 °C for 1 h. The reaction was quenched with 10 mL of saturated ammonium chloride solution and extracted with 3 x 50 mL of ethyl acetate. The combined organic extracts were washed with 50 mL of brine, dried (Na₂SO₄), filtered and the solvent was removed *in vacuo.* The residue was purified by chromatography (neutral alumina, eluting with a linear gradient of 0-20% ethyl acetate/petroleum ether) to provide 0.3 g (0.90 mmol, 53%) of (*R*)-*N*-(cyclopropyl(1-methoxyisoquinolin-4-yl)methyl)-2-methylpropane-2-sulfinamide (**XIIIc**) as an approximately 20:1 mixture of diastereoisomers. LCMS: *m*/*z* found 333.5 [M+H]⁺.

### (R)-N-(Cyclopropyl(1-methoxyisoquinolin-4-yl)methyl)-N-ethyl-2-methylpropane-2-sulfinamide (XIVc)

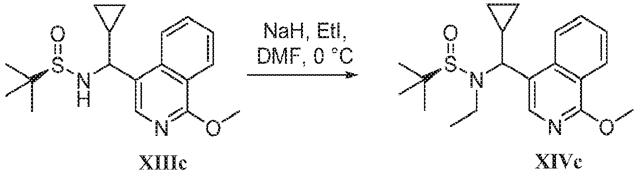

To a suspension of 0.3 g (3.01 mmol, 1.0 eq.) of (*R*)-*N*-(cyclopropyl(1-methoxyisoquinolin-4-yl)methyl)-2-methylpropane-2-sulfinamide (**XIIIc**) in 10 mL of DMF at 0 °C under a nitrogen atmosphere was added 0.07 g (1.84 mmol, 2.0 eq.) of a 60% dispersion of sodium hydride in mineral oil. The mixture was stirred at 0 °C for 15 min and 0.2 mL (1.84 mmol, 2.0 eq.) of ethyl iodide was added. The mixture was then allowed to warm to room temperature and stirred for 3 h. The reaction was quenched with 10 mL of ice-cold water and extracted with 3 x 100 mL of ethyl acetate. The combined organic extracts were washed with 100 mL of brine, dried (Na₂SO₄), filtered and the solvent was removed *in vacuo.* The residue was purified by chromatography (neutral alumina, eluting with a linear gradient of 0-5% ethyl acetate/petroleum ether) to provide 0.15 g (0.42 mmol, 46%) of (*R*)-*N-*(cyclopropyl(1-methoxyisoquinolin-4-yl)methyl)-*N*-ethyl-2-methylpropane-2-sulfinamide (**XIVc**) as an approximately 1:10 mixture of diastereoisomers. LCMS: *m*/*z* found 361.5 [M+H]⁺.

### N-(Cyclopropyl(1-methoxyisoquinolin-4-yl)methyl)ethanamine (VIbc)

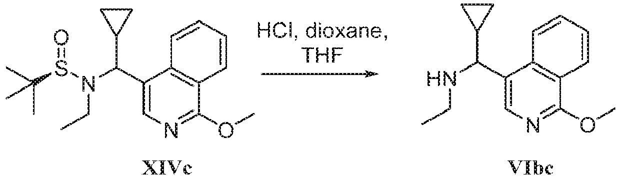

To a solution of 0.25 g (0.69 mmol, 1.0 eq.) of (*R*)-*N*-(cyclopropyl(1-methoxyisoquinolin-4-yl)methyl)-*N*-ethyl-2-methylpropane-2-sulfinamide (**XIVc**) in 10 mL of THF at 0 °C was added 0.5 mL of a 4 M solution of HCl in 1,4-dioxane and the mixture was stirred at 0 °C for 2 h. The mixture was diluted with 5 mL of with ice-cold water, adjusted the pH to 8-9 using saturated sodium bicarbonate solution and extracted with 3 x 30 mL of ethyl acetate. The combined organic extracts were washed with 40 mL of brine, dried (Na₂SO₄), filtered and the solvent was removed *in vacuo* to provide 0.15 g (0.58 mmol, 84%) of *N*-(cyclopropyl(1-methoxyisoquinolin-4-yl)methyl)ethanamine (**VIbc**). LCMS: *m*/*z* found 257.5 [M+H]⁺, RT = 1.79 min; ¹H NMR (400 MHz, CDCl₃): δ 8.40-8.42 (m, 1H), 8.27-8.30 (m, 1H), 8.01 (s, 1H), 7.65-7.69 (m, 1H), 7.50-7.55 (m, 1H), 3.70 (s, 3H), 3.39 (d, 1H), 2.64-2.68 (m, 1H), 2.51-2.56 (m, 1H), 1.41-1.44 (m, 2H), 1.07 (t, 3H), 0.64-0.68 (m, 1H), 0.32-0.39 (m, 2H), 0.15-0.18 (m, 1H).

### 3-(3-Chloro-4-fluorophenyl)-1-(cyclopropyl(1-methoxyisoquinolin-4-yl)methyl)-1-ethylurea - Enantiomer I (Compound 40)

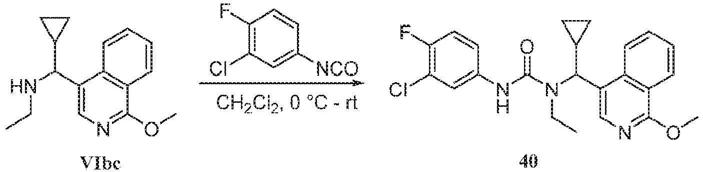

3-(3-Chloro-4-fluorophenyl)-1-(cyclopropyl(1-methoxyisoquinolin-4-yl)methyl)-1-ethylurea Enantiomer I was synthesised in an analogous manner as described above from scalemic *N*-(cyclopropyl(1-methoxyisoquinolin-4-yl)methyl)ethanamine (**VIbc,** ~ 20:1 mixture of enantiomers, derived from (R)-2-methylpropane-2-sulfinamide) and 2-chloro-1-fluoro-4-isocyanatobenzene. Chiral analytical SFC: 92 and 4% with RT = 3.12 and 4.23 min, respectively, Column: Lux Cellulose-2 (4.6 x 250mm) 5 µ, 70 % CO₂:[0.5% diethyl amine in methanol], Flow rate: 3.0 mL/min. The major enantiomer was subsequently isolated by chiral SFC, Column: Chiralpak IC (250 x 30 mm) 5 µ, 70% CO₂/MeOH, Flow rate 70 g/min.

3-(3-Chloro-4-fluorophenyl)-1-(cyclopropyl(1-methoxyisoquinolin-4-yl)methyl)-1-ethylurea - Enantiomer I (**Compound 40**). LCMS: *m*/*z* found 428.2/430.2 [M+H]⁺; RT = 5.87 min (Method A); ¹H NMR (400 MHz, DMSO-d₆): δ 8.42 (s, 1H), 8.35 (bs, 1H), 8.21 (d, 1H), 7.98 (d, 1H), 7.84-7.87 (m, 1H), 7.75-7.79 (m, 1H), 7.58-7.63 (m, 1H), 7.51-7.55 (m, 1H), 7.29-7.34 (m, 1H), 5.23 (d, 1H), 4.09 (s, 3H), 3.32-3.35 (m, 1H), 3.19-3.23 (m, 1H), 1.79-1.83 (m, 1H), 0.59-0.72 (m, 6H), 0.19-0.23 (m, 1H); Chiral analytical SFC: RT = 3.12 min, Column: Lux Cellulose-2 (4.6 x 250 mm) 5 µ, 70 % CO₂:[0.5% diethyl amine in methanol], Flow rate: 3.0 mL/min.

### 4-(Cyclopropyl(ethylamino)methyl)isoquinolin-1(2H)-one hydrochloride (VIIIcy)

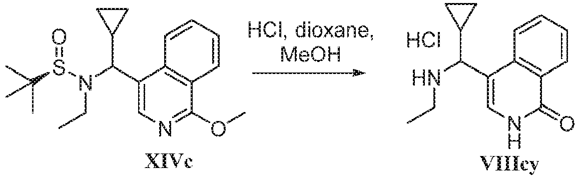

To a solution of 0.50 g (1.39 mmol, 1.0 eq.) of (*R*)-*N*-(cyclopropyl(1-methoxyisoquinolin-4-yl)methyl)-*N*-ethyl-2-methylpropane-2-sulfinamide (**XVIc**) in 10 mL of methanol was added 2 mL of a 4 M solution of HCl in 1,4-dioxane and the mixture was stirred at room temperature for 2 h. The solvent was removed *in vacuo* and the residue was triturated with 20 mL of *n*-pentane and dried under high vacuum to provide 0.30 g of 4-(cyclopropyl(ethylamino)methyl)isoquinolin-1(2*H*)-one hydrochloride (**VIIIcy**). LCMS: *m*/*z* found 243.3 [M+H]⁺, RT = 1.79 min.

### 3-(3-Chloro-4-fluorophenyl)-1-(cyclopropyl(1-oxo-1,2-dihydroisoquinolin-4-yl)methyl)-1-ethylurea - Enantiomer I (Compound 46)

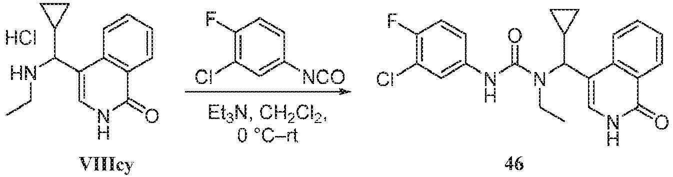

3-(3-Chloro-4-fluorophenyl)-1-(cyclopropyl(1-oxo-1,2-dihydroisoquinolin-4-yl)methyl)-1-ethylurea Enantiomer I was synthesised in an analogous manner as described above from scalemic 4-(cyclopropyl(ethylamino)methyl)isoquinolin-1(2*H*)-one hydrochloride (**VIIIcy,** ~ 20:1 mixture of enantiomers, derived from (R)-2-methylpropane-2-sulfinamide) and 2-chloro-1-fluoro-4-isocyanatobenzene. The major enantiomer was subsequently isolated by chiral SFC, Column: Chiralpak IC (250 x 30 mm) 5 µ, 70% CO₂/MeOH, Flow rate 70 g/min.

3-(3-Chloro-4-fluorophenyl)-1-(cyclopropyl(1-methoxyisoquinolin-4-yl)methyl)-1-ethylurea - Enantiomer I (**Compound 46**). LCMS: *m*/*z* found 414.2/416.2 [M+H]⁺; RT = 4.77 min (Method A); ¹H NMR (400 MHz, DMSO-d₆): δ 12.42 (bs, 1H), 8.34 (bs, 1H), 8.23 (d, 1H), 7.84-7.87 (m, 1H), 7.68-7.73 (m, 2H), 7.44-7.56 (m, 3H), 7.32 (t, 1H), 4.97 (d, 1H), 3.19-3.33 (m, 2H), 1.61-1.66 (m, 1H), 0.73 (t, 3H), 0.52-0.69 (m, 3H), 0.20-0.23 (m, 1H); Chiral analytical SFC: RT = 2.97 min, Column: Lux Cellulose-2 (4.6 x 250mm) 5 µ, 60 % CO₂/[0.5% diethyl amine in methanol], Co-Solvent, Flow rate: 3.0 mL/min.

### (S)-N-(Cyclopropyl(l-methoxyisoquinolin-4-yl)methyl)-2-methylpropane-2-sulfinamide (XIIId)

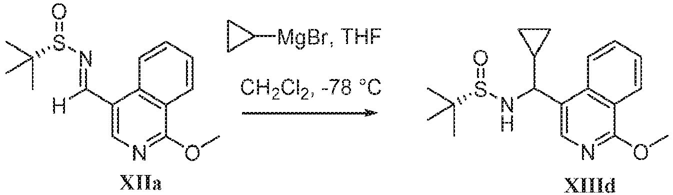

To a solution of 1.5 g (5.17 mmol, 1.0 e) of (*S*)-*N*-((1-methoxyisoquinolin-4-yl)methylene)-2-methylpropane-2-sulfinamide (**XIIa**) in 150 mL of methylene chloride at -78 °C under a nitrogen atmosphere was added 104 mL (51.72 mmol, 10.0 eq.) of a 0.5 M solution of cyclopropylmagnesium bromide in THF and the mixture was stirred at -78 °C for 2 h. The reaction was quenched with 50 mL of saturated ammonium chloride solution and extracted with 3 x 100 mL of ethyl acetate. The combined organic extracts were washed with 50 mL of brine, dried (Na₂SO₄), filtered and the solvent was removed *in vacuo.* The residue was purified by chromatography (neutral alumina, eluting with a linear gradient of 0-20% ethyl acetate/petroleum ether) to provide 1.0 g (3.01 mmol, 58%) of (*S*)-*N*-(cyclopropyl(1-methoxyisoquinolin-4-yl)methyl)-2-methylpropane-2-sulfinamide (**XIIId**) as an approximately 1:10 mixture of diastereoisomers. **LCMS:** *m*/*z* found 333.5 [M+H]⁺.

### (S)-N-(Cyclopropyl(1-methoxyisoquinolin-4-yl)methyl)-N-ethyl-2-methylpropane-2-sulfinamide (XIVd)

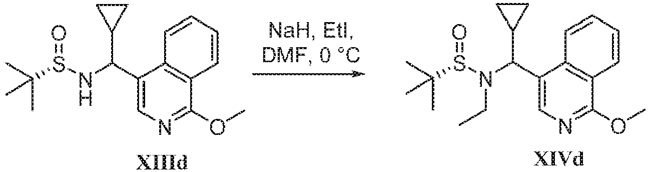

To a suspension of 1.0 g (3.01 mmol, 1.0 eq.) of of (*S*)-*N*-(cyclopropyl(1-methoxyisoquinolin-4-yl)methyl)-2-methylpropane-2-sulfinamide (**XIIId**) in 10 mL of DMF at 0 °C under a nitrogen atmosphere was added 0.24 g (6.02 mmol, 2.0 eq.) of a 60% dispersion of sodium hydride in mineral oil. The mixture was stirred at 0 °C for 15 min and 0.7 mL (6.02 mmol, 2.0 eq.) of ethyl iodide was added. The mixture was then allowed to warm to room temperature and stirred for 3 h. The reaction was quenched with 30 mL of ice-cold water and extracted with 3 x 100 mL of ethyl acetate. The combined organic extracts were washed with 100 mL of brine, dried (Na₂SO₄), filtered and the solvent was removed *in vacuo.* The residue was purified by chromatography (neutral alumina, eluting with a linear gradient of 0-5% ethyl acetate/petroleum ether) to provide 0.65 g (1.80 mmol, 60%) of (*S*)*-N-*(cyclopropyl(1-methoxyisoquinolin-4-yl)methyl)-*N*-ethyl-2-methylpropane-2-sulfinamide (**XIVd**) as an approximately 1:10 mixture of diastereoisomers. LCMS: *m*/*z* found 361.5 [M+H]⁺.

### N-(Cyclopropyl(1-methoxyisoquinolin-4-yl)methyl)ethanamine (VIbc)

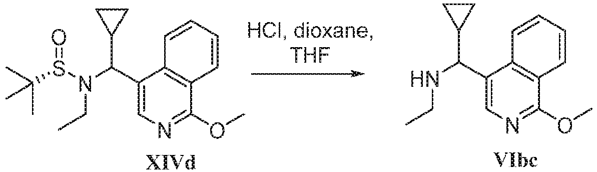

To a solution of 0.25 g (0.69 mmol, 1.0 eq.) of (*S*)-*N*-(cyclopropyl(1-methoxyisoquinolin-4-yl)methyl)-*N*-ethyl-2-methylpropane-2-sulfinamide (**XIVd**) in 10 mL of THF at 0 °C was added 0.5 mL of a 4 M solution of HCl in 1,4-dioxane and the mixture was stirred at 0 °C for 2 h. The mixture was diluted with 5 mL of with ice-cold water and adjusted the pH to 8-9 using saturated sodium bicarbonate solution and extracted with 3 x 30 mL of ethyl acetate. The combined organic extracts were washed with 40 mL of brine, dried (Na₂SO₄), filtered and the solvent was removed *in vacuo* to provide 0.15 g (0.58 mmol, 84%) of *N*-(cyclopropyl(1-methoxyisoquinolin-4-yl)methyl)ethanamine (**VIbc**). LCMS: *m*/*z* found 257.5 [M+H]⁺, RT = 1.79 min; ¹H NMR (400 MHz, CDCl₃): δ 8.40-8.42 (m, 1H), 8.27-8.30 (m, 1H), 8.01 (s, 1H), 7.65-7.69 (m, 1H), 7.50-7.55 (m, 1H), 3.70 (s, 3H), 3.39 (d, 1H), 2.64-2.68 (m, 1H), 2.51-2.56 (m, 1H), 1.41-1.44 (m, 2H), 1.07 (t, 3H), 0.64-0.68 (m, 1H), 0.32-0.39 (m, 2H), 0.15-0.18 (m, 1H).

### 3-(3-Chloro-4-fluorophenyl)-1-(cyclopropyl(1-methoxyisoquinolin-4-yl)methyl)-1-ethylurea - Enantiomer II (Compound 58)

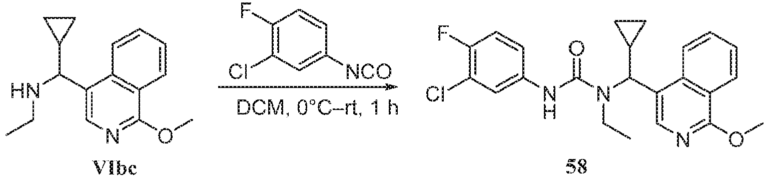

3-(3-Chloro-4-fluorophenyl)-1-(cyclopropyl(1-methoxyisoquinolin-4-yl)methyl)-1-ethylurea Enantiomer II was synthesised in an analogous manner as described above from scalemic *N*-(cyclopropyl(1-methoxyisoquinolin-4-yl)methyl)ethanamine (**VIbc,** ~ 1:10 mixture of enantiomers, derived from (S)-2-methylpropane-2-sulfinamide) and 2-chloro-1-fluoro-4-isocyanatobenzene. Chiral analytical SFC: 8 and 92% with RT = 2.16 and 2.57 min, respectively [Column: Lux Cellulose-2 (4.6 x 250) mm, 5 µ, 70% CO₂/[0.5% diethyl amine in methanol], Flow rate: 3.0 mL/min]. The major enantiomer was subsequently separated by chiral SFC, Column: Lux Cellulose-2 (250 x 30 mm) 5 µ, 80% CO2/MeOH, Flow rate 70 g/min.

3-(3-Chloro-4-fluorophenyl)-1-(cyclopropyl(1-methoxyisoquinolin-4-yl)methyl)-1-ethylurea - Enantiomer II (**Compound 58**). LCMS: *m*/*z* found 428.2/430.2 [M+H]⁺; RT = 5.86 min (Method A); ¹H NMR (400 MHz, DMSO-d₆): δ 8.42 (s, 1H), 8.35 (bs, 1H), 8.21 (d, 1H), 7.98 (d, 1H), 7.84-7.87 (m, 1H), 7.75-7.79 (m, 1H), 7.58-7.63 (m, 1H), 7.51-7.55 (m, 1H), 7.29-7.34 (m, 1H), 5.23 (d, 1H), 4.09 (s, 3H), 3.32-3.35 (m, 1H), 3.19-3.23 (m, 1H), 1.79-1.83 (m, 1H), 0.59-0.72 (m, 6H), 0.19-0.23 (m, 1H); Chiral analytical SFC: RT = 2.57 min, Column: Lux Cellulose-2 (4.6 x 250) mm, 5 µ, 70% CO₂/[0.5% diethyl amine in methanol], 70 %Co-Solvent, Flow rate: 3.0 mL/min

### 4-(Cyclopropyl(ethylamino)methyl)isoquinolin-1(2H)-one hydrochloride (VIIIcy)

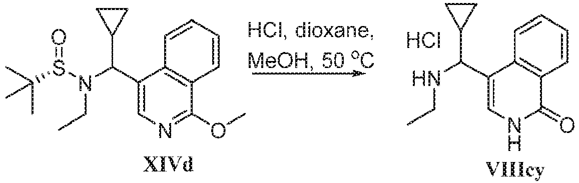

To a solution of 0.35 g (0.97 mmol, 1.0 eq.) of (*S*)-*N*-(cyclopropyl(1-methoxyisoquinolin-4-yl)methyl)-*N*-ethyl-2-methylpropane-2-sulfinamide (**XIVd**) in 10 mL of methanol was added 0.5 mL of a 4 M solution of HCl in 1,4-dioxane and the mixture was heated at 50 °C for 2 h. The solvent was removed *in vacuo* and the residue was triturated with 20 mL of *n*-pentane and dried under high vacuum to provide 0.25 g (0.90 mmol, 92%) of 4-(cyclopropyl(ethylamino)methyl)isoquinolin-1(2*H*)-one hydrochloride (**VIIIcy**). LCMS: *m*/*z* found 243.3 [M+H]⁺, RT = 1.79 min.

### 3-(3-Chloro-4-fluorophenyl)-1-(cyclopropyl(1-oxo-1,2-dihydroisoquinolin-4-yl)methyl)-1-ethylurea Enantiomer II (Compound 49)

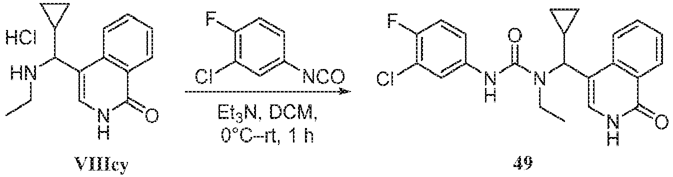

3-(3-Chloro-4-fluorophenyl)-1-(cyclopropyl(1-oxo-1,2-dihydroisoquinolin-4-yl)methyl)-1-ethylurea Enantiomer II was synthesised in an analogous manner as described above from scalemic 4-(cyclopropyl(ethylamino)methyl)isoquinolin-1(2*H*)-one hydrochloride (**VIIIcy,** ~ 1:10 mixture of enantiomers, derived from (S)-2-methylpropane-2-sulfinamide) and 2-chloro-1-fluoro-4-isocyanatobenzene. The major enantiomer was subsequently isolated by chiral SFC, Column: Chiralpak IC (250 x 30 mm) 5 µ, 70% CO₂/MeOH, Flow rate 70 g/min.

3-(3-Chloro-4-fluorophenyl)-1-(cyclopropyl(1-methoxyisoquinolin-4-yl)methyl)-1-ethylurea - Enantiomer II (**Compound 49**). LCMS: *m*/*z* found 414.2/416.2 [M+H]⁺; RT = 5.10min (Method A); ¹H NMR (400 MHz, DMSO-d₆): δ 12.42 (bs, 1H), 8.34 (bs, 1H), 8.23 (d, 1H), 7.84-7.87 (m, 1H), 7.68-7.73 (m, 2H), 7.44-7.56 (m, 3H), 7.32 (t, 1H), 4.97 (d, 1H), 3.19-3.33 (m, 2H), 1.61-1.66 (m, 1H), 0.73 (t, 3H), 0.52-0.69 (m, 3H), 0.20-0.23 (m, 1H); Chiral analytical SFC: RT = 2.69 min, Column: Lux Cellulose-2 (4.6 x 250mm) 5 µ, 60% CO₂/[0.5% diethyl amine in methanol], Flow rate: 3.0 mL/min.

### 3-(3-Chloro-4-fluorophenyl)-1-ethyl-1-(1-(1-methoayisoquinolin-4-yl)propyl)urea - Enantiomer II (Compound 38)

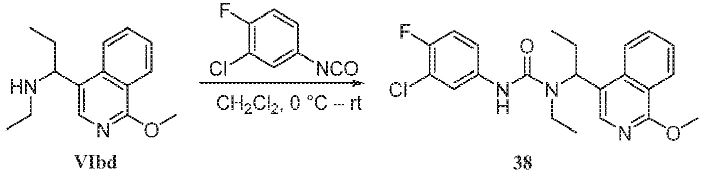

3-(3-Chloro-4-fluorophenyl)-1-ethyl-1-(1-(1-methoxyisoquinolin-4-yl)propyl)urea - Enantiomer II was synthesised in an analogous manner as described above from scalemic *N-*ethyl-1-(1-methoxyisoquinolin-4-yl)propan-1-amine (**VIbd**, derived from (R)-2-methylpropane-2-sulfinamide and ethyl magnesium bromide) and 2-chloro-1-fluoro-4-isocyanatobenzene.

3-(3-Chloro-4-fluorophenyl)-1-ethyl-1-(1-(1-methoxyisoquinolin-4-yl)propyl)urea - Enantiomer II (**Compound 38**). LCMS: *m*/*z* found 416.4/418.4 [M+H]⁺; ¹H NMR (400 MHz, DMSO-d₆): δ 8.34 (s, 1H), 8.22 (d, 1H), 8.15 (s, 1H), 8.13 (s, 1H), 7.85-7.87 (m, 1H), 7.78-7.82 (t, 1H), 7.64 (t, 1H), 7.51-7.54 (m, 1H), 7.39-7.34 (m 1H), 5.96 (t, 1H), 4.08 (s, 3H), 3.25-3.27 (m, 1H), 3.07-3.09 (m, 1H), 2.07-2.13 (m, 2H), 0.97 (t, 3H), 0.52 (t, 3H).

### 3-(3-Chloro-4-fluorophenyl)-1-ethyl-1-(1-(1-oxo-1,2-dihydroisoquinolin-4-yl)propyl)urea - Enantiomer I (Compound 39)

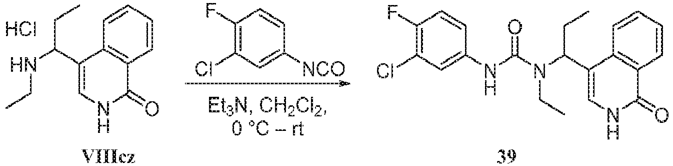

3-(3-Chloro-4-fluorophenyl)-1-ethyl-1-(1-(1-oxo-1,2-dihydroisoquinolin-4-yl)propyl)urea - Enantiomer I was synthesised in an analogous manner as described above from scalemic *N*-ethyl-1-(1-methoxyisoquinolin-4-yl)propan-1-amine hydrochloride (**VIIIcz,** derived from (R)-2-methylpropane-2-sulfinamide and ethyl magnesium bromide) and 2-chloro-1-fluoro-4-isocyanatobenzene. Chiral analytical SFC: 6 and 93% with RT = 4.05 and 5.43 min, respectively [Column: Chiralcel OX-H (4.6 x 250) mm, 5 µ, 70% CO₂/MeOH, Flow rate: 3.0 mL/min]. The major enantiomer was subsequently separated by chiral SFC, Column: Chiralcel OX-H (250 x 30 mm) 5 µ, 85% CO₂/MeOH, Flow rate 70 g/min.

3-(3-Chloro-4-fluorophenyl)-1-ethyl-1-(1-(1-oxo-1,2-dihydroisoquinolin-4-yl)propyl)urea - Enantiomer I (**Compound 39**). LCMS: *m*/*z* found 402.4/404.4 [M+H]⁺; ¹H NMR (400 MHz, DMSO-d₆): δ 11.40 (bd, 1H), 8.33 (bs, 1H), 8.23 (d, 1H), 7.84-7.89 (m, 2H), 7.73 (t, 1H), 7.47-7.56 (m, 2H), 7.32 (t, 1H), 7.21 (d, 1H), 5.66-5.72 (m, 1H), 3.28-3.39 (m, 1H), 3.06-3.12 (m, 1H), 1.86-1.96 (m, 2H), 0.93 (t, 3H), 0.64 (t, 3H); Chiral analytical SFC: RT = 5.43 min, Column: Lux Cellulose-2 (4.6 x 250mm) 5 µ, 70 % CO₂/MeOH, Flow rate: 3.0 mL/min.

### 3-(3-Chloro-4-fluorophenyl)-1-ethyl-1-(1-(1-methoayisoquinolin-4-yl)propyl)urea - Enantiomer I (Compound 36)

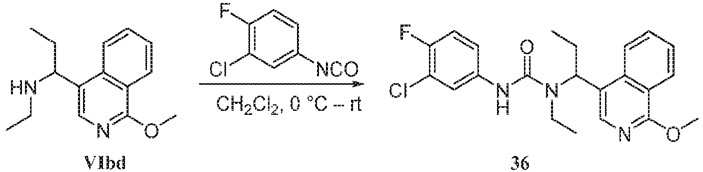

3-(3-Chloro-4-fluorophenyl)-1-ethyl-1-(1-(1-methoxyisoquinolin-4-yl)propyl)urea - Enantiomer I was synthesised in an analogous manner as described above from scalemic *N-*ethyl-1-(1-methoxyisoquinolin-4-yl)propan-1-amine (**VIbd,** derived from (S)-2-methylpropane-2-sulfinamide and ethyl magnesium bromide) and 2-chloro-1-fluoro-4-isocyanatobenzene.

3-(3-Chloro-4-fluorophenyl)-1-ethyl-1-(1-(1-methoxyisoquinolin-4-yl)propyl)urea - Enantiomer I (**Compound 36**). LCMS: *m*/*z* found 416.4/418.4 [M+H]⁺; ¹H NMR (400 MHz, DMSO-d₆): δ 8.34 (s, 1H), 8.22 (d, 1H), 8.15 (s, 1H), 8.13 (s, 1H), 7.85-7.87 (m, 1H), 7.78-7.82 (t, 1H), 7.64 (t, 1H), 7.51-7.54 (m, 1H), 7.39-7.34 (m 1H), 5.96 (t, 1H), 4.08 (s, 3H), 3.25-3.27 (m, 1H), 3.07-3.09 (m, 1H), 2.07-2.13 (m, 2H), 0.97 (t, 3H), 0.52 (t, 3H).

### 3-(3-chloro-4-fluorophenyl)-1-ethyl-1-(1-(1-oxo-1,2-dihydroisoquinolin-4-yl)propyl)urea - Enantiomer II (Compound 37)

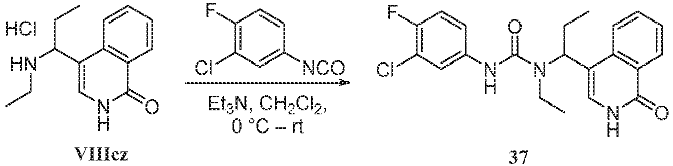

3-(3-Chloro-4-fluorophenyl)-1-ethyl-1-(1-(1-oxo-1,2-dihydroisoquinolin-4-yl)propyl)urea Enantiomer II was synthesised in an analogous manner as described above from scalemic *N*-ethyl-1-(1-methoxyisoquinolin-4-yl)propan-1-amine hydrochloride (**VIIIcz,** derived from (S)-2-methylpropane-2-sulfinamide and ethyl magnesium bromide) and 2-chloro-1-fluoro-4-isocyanatobenzene. The major enantiomer was subsequently separated by chiral SFC, Column: Chiralcel OX-H (250 x 30 mm) 5 µ, 85% CO₂/MeOH, Flow rate 70 g/min.

3-(3-Chloro-4-fluorophenyl)-1-(cyclopropyl(1-methoxyisoquinolin-4-yl)methyl)-1-ethylurea - Enantiomer II (**Compound 37**). LCMS: *m*/*z* found 402.4/404.4 [M+H]⁺; ¹H NMR (400 MHz, DMSO-d₆): δ 11.40 (bd, 1H), 8.33 (bs, 1H), 8.23 (d, 1H), 7.84-7.89 (m, 2H), 7.73 (t, 1H), 7.47-7.56 (m, 2H), 7.32 (t, 1H), 7.21 (d, 1H), 5.66-5.72 (m, 1H), 3.28-3.39 (m, 1H), 3.06-3.12 (m, 1H), 1.86-1.96 (m, 2H), 0.93 (t, 3H), 0.64 (t, 3H); Chiral analytical SFC: RT = 4.05 min, Column: Lux Cellulose-2 (4.6 x 250mm) 5 µ, 70 % CO₂/MeOH, Flow rate: 3.0 mL/min.

### 3-(3-Chloro-4-fluorophenyl)-1-ethyl-1-((1-methoxyisoquinolin-4-yl)(phenyl)methyl)urea - Enantiomer I (Compound 59)

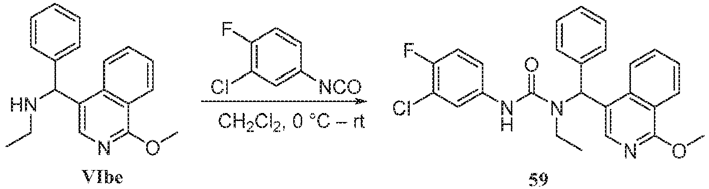

3-(3-Chloro-4-fluorophenyl)-1-ethyl-1-((1-methoxyisoquinolin-4-yl)(phenyl)methyl)urea - Enantiomer I was synthesised in an analogous manner as described above from *N*-((1-methoxyisoquinolin-4-yl)(phenyl)methyl)ethanamine (**VIbe**, derived from (R)-2-methylpropane-2-sulfinamide and phenyl magnesium bromide) and 2-chloro-1-fluoro-4-isocyanatobenzene. The major enantiomer was subsequently isolated by chiral SFC, Column: Chiralpak AD-H (250 x 30 mm) 5 µ, 85% CO₂/MeOH, Flow rate 70 g/min.

3-(3-Chloro-4-fluoropheny 1)-1 -ethyl-1 -((1 -methoxy isoquinolin-4-yl)(phenyl)methyl)urea - Enantiomer I (**Compound 59**). LCMS: *m*/*z* found 464.2/466.2 [M+H]⁺, RT = 6.63 min; ¹H NMR (400 MHz, DMSO-d₆): δ 8.62 (s, 1H), 8.26 (d, 1H), 7.93 (d, 1H), 7.81-7.85 (m, 2H), 7.64-7.69 (m, 1H), 7.56 (s, 1H), 7.50-7.54 (m, 1H), 7.38-7.42 (m, 2H), 7.28-7.34 (m, 3H), 7.23-7.25 (m, 2H), 4.06 (s, 3H), 3.60-3.64 (m, 1H), 3.40-3.47 (m, 1H), 0.37 (t, 3H); Chiral analytical SFC: RT = 5.38 min, Column: Chiralpak AD-H (4.6 x 250mm) 5 m, 70 % CO₂/MeOH, Flow rate: 3.0 mL/min.

### 3-(3-Chloro-4-fluorophenyl)-1-ethyl-1-((1-oxo-1,2-dihydroisoquinolin-4-yl)(phenyl)methyl)urea - Enantiomer I (Compound 69)

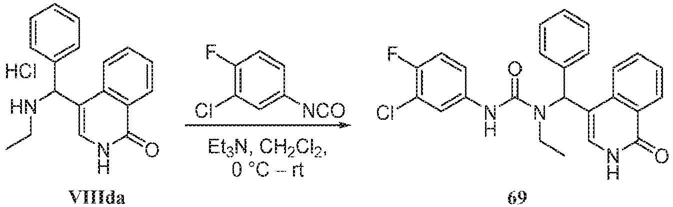

3-(3-Chloro-4-fluorophenyl)-1-ethyl-1-((1-oxo-1,2-dihydroisoquinolin-4-yl)(phenyl)methyl)urea - Enantiomer I was synthesised in an analogous manner as described above from scalemic 4-((ethylamino)(phenyl)methyl)isoquinolin-1(2*H*)-one hydrochloride (**VIIIda,** derived from (R)-2-methylpropane-2-sulfinamide and phenyl magnesium bromide) and 2-chloro-1-fluoro-4-isocyanatobenzene.. The major enantiomer was subsequently separated by chiral SFC, Column: Chiralpak IG (250 x 30 mm) 5 µ, 70% CO₂/MeOH, Flow rate 60 g/min.

3-(3-chloro-4-fluorophenyl)-1-ethyl-1-((1-oxo-1,2-dihydroisoquinolin-4-yl)(phenyl)methyl)urea - Enantiomer I (**Compound 69**). LCMS: *m*/*z* found 450.2/452.2 [M+H]⁺, RT = 5.37 min; ¹H NMR (400 MHz, DMSO-d₆): δ 11.25 (bd, 1H) 8.60 (s, 1H), 8.27 (d, 1H), 7.83-7.85 (m, 1H), 7.76-7.78 (m, 1H), 7.70-7.72 (m, 1H), 7.50-7.56 (m, 2H), 7.38-7.42 (m, 2H), 7.27-7.34 (m, 4H), 7.07 (s, 1H), 6.53 (d, 1H), 3.60-3.68 (m, 1H), 3.35-3.41 (m, 1H), 0.48 (t, 3H); Chiral analytical SFC: RT = 6.76 min, Column: Chiralpak IG (4.6 x 250mm) 5 µ, 70 % CO₂/MeOH, Flow rate: 3.0 mL/min.

### 3-(3-Chloro-4-fluorophenyl)-1-ethyl-1-((1-methoxyisoquinolin-4-yl)(phenyl)methyl)urea - Enantiomer II (Compound 60)

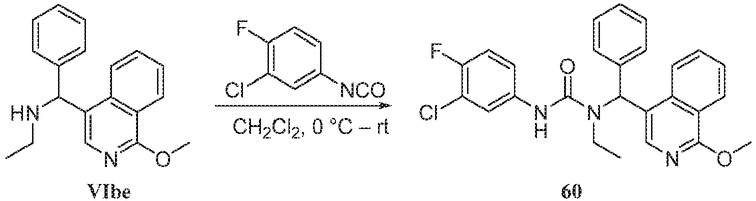

3-(3-Chloro-4-fluorophenyl)-1-ethyl-1-((1-methoxyisoquinolin-4-yl)(phenyl)methyl)urea - Enantiomer II was synthesised in an analogous manner as described above from *N*-((1-methoxyisoquinolin-4-yl)(phenyl)methyl)ethanamine (**VIbe,** derived from (S)-2-methylpropane-2-sulfinamide and phenyl magnesium bromide) and 2-chloro-1-fluoro-4-isocyanatobenzene. The major enantiomer was subsequently separated by chiral SFC, Column: Chiralpak AD-H (250 x 30 mm) 5 µ, 85% CO₂/MeOH, Flow rate 70 g/min.

3-(3-Chloro-4-fluorophenyl)-1-ethyl-1-((1-methoxyisoquinolin-4-yl)(phenyl)methyl)urea - Enantiomer II (**Compound 60**). LCMS: *m*/*z* found 464.2/466.2 [M+H]⁺, RT = 6.63 min; ¹H NMR (400 MHz, DMSO-d₆): δ 8.62 (s, 1H), 8.26 (d, 1H), 7.93 (d, 1H), 7.81-7.85 (m, 2H), 7.64-7.69 (m, 1H), 7.56 (s, 1H), 7.50-7.54 (m, 1H), 7.38-7.42 (m, 2H), 7.28-7.34 (m, 3H), 7.23-7.25 (m, 2H), 4.06 (s, 3H), 3.60-3.64 (m, 1H), 3.40-3.47 (m, 1H), 0.37 (t, 3H); Chiral analytical SFC: RT = 6.97 min, Column: Chiralpak AD-H (4.6 x 250mm) 5 m, 70 % CO₂/MeOH, Flow rate: 3.0 mL/min.

### 3-(3-Chloro-4-fluorophenyl)-1-ethyl-1-((1-oxo-1,2-dihydroisoquinolin-4-yl)(phenyl)methyl)urea - Enantiomer II (Compound 70)

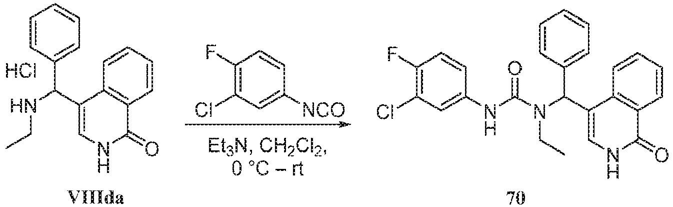

3-(3-Chloro-4-fluorophenyl)-1-ethyl-1-((1-oxo-1,2-dihydroisoquinolin-4-yl)(phenyl)methyl)urea - Enantiomer II was synthesised in an analogous manner as described above from scalemic 4-((ethylamino)(phenyl)methyl)isoquinolin-1(2*H*)-one hydrochloride (**VIIIda**, derived from (S)-2-methylpropane-2-sulfinamide and phenyl magnesium bromide) and 2-chloro-1-fluoro-4-isocyanatobenzene. The major enantiomer was subsequently separated by chiral SFC, Column: Chiralpak IG (250 x 30 mm) 5 µ, 70% CO₂/MeOH, Flow rate 60 g/min.

3-(3-Chloro-4-fluorophenyl)-1-ethyl-1-((1-oxo-1,2-dihydroisoquinolin-4-yl)(phenyl)methyl)urea - Enantiomer II (**Compound 70**). LCMS: *m*/*z* found 450.2/452.2 [M+H]⁺, RT = 5.37 min; ¹H NMR (400 MHz, DMSO-d₆): δ 11.25 (bd, 1H) 8.60 (s, 1H), 8.27 (d, 1H), 7.83-7.85 (m, 1H), 7.76-7.78 (m, 1H), 7.70-7.72 (m, 1H), 7.50-7.56 (m, 2H), 7.38-7.42 (m, 2H), 7.27-7.34 (m, 4H), 7.07 (s, 1H), 6.53 (d, 1H), 3.60-3.68 (m, 1H), 3.35-3.41 (m, 1H), 0.48 (t, 3H); Chiral analytical SFC: RT = 11.55 min, Column: Chiralpak IG (4.6 x 250mm) 5 µ, 70 % CO₂/MeOH, Flow rate: 3.0 mL/min.

### 3-(3-Chloro-4-fluorophenyl)-1-ethyl-1-(1-(1-methoxyisoquinolin-4-yl)-2-methylpropyl)urea (Compounds 44 & 45)

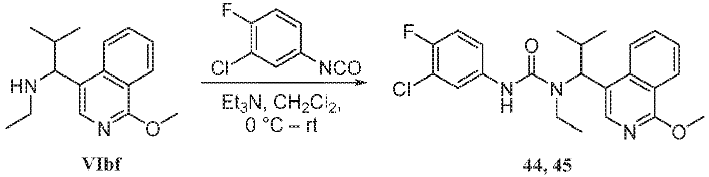

3-(3-Chloro-4-fluorophenyl)-1-ethyl-1-(1-(1-methoxyisoquinolin-4-yl)-2-methylpropyl)urea was synthesised in an analogous manner as described above from scalemic *N*-ethyl-1-(1-methoxyisoquinolin-4-yl)-2-methylpropan-1-amine hydrochloride (**VIbf**, ~ 7:3 mixture of enantiomers, derived from (S)-2-methylpropane-2-sulfinamide and isopropyl magnesium bromide) and 2-chloro-1-fluoro-4-isocyanatobenzene. Chiral analytical SFC: 71% and 29% with RT = 2.87 and 4.76 min, respectively [Column: Chiralpak IC (4.6 x 250) mm, 5 µ, 80 % CO₂/MeOH, Flow rate: 3.0 mL/min]. The enantiomers were subsequently separated by chiral SFC, Column: Chiralpak IC (250 x 30 mm) 5 µ, 80% CO₂/MeOH, Flow rate 70 g/min.

3-(3-Chloro-4-fluorophenyl)-1-ethyl-1-(1-(1-methoxyisoquinolin-4-yl)-2-methylpropyl)urea - Enantiomer I (**Compound 44**). LCMS: *m*/*z* found 430.3/432.3 [M+H]⁺; RT = 6.06 min (Method A); ¹H NMR (400 MHz, DMSO-d₆): δ 8.28-8.33 (m, 2H), 8.20-8.23 (m, 2H), 7.78-7.84 (m, 2H), 7.59-7.64 (m, 1H), 7.48-7.52 (m, 1H), 7.28-7.34 (m, 1H), 5.72 (d, 1H), 4.09 (s, 3H), 3.47-3.37 (m, 1H), 3.08-3.17 (m, 1H), 2.69-2.77 (m, 1H), 1.02 (d, 3H), 0.91 (d, 3H), 0.38 (t, 3H); Chiral analytical SFC: RT = 2.81 min, Column: Lux Cellulose-2 (4.6 x 250) mm, 5 µ, Co-Solvent 0.5% diethyl amine in Methanol, 80 %Co-Solvent, Flow rate: 3.0 mL/min

3-(3-Chloro-4-fluorophenyl)-1-ethyl-1-(1-(1-methoxyisoquinolin-4-yl)-2-methylpropyl)urea - Enantiomer II (**Compound 45**). LCMS: *m*/*z* found 430.3/432.3 [M+H]⁺; RT = 6.07 min (Method A); ¹H NMR (400 MHz, DMSO-d₆): δ 8.28-8.33 (m, 2H), 8.20-8.23 (m, 2H), 7.78-7.84 (m, 2H), 7.59-7.64 (m, 1H), 7.48-7.52 (m, 1H), 7.28-7.34 (m, 1H), 5.72 (d, 1H), 4.09 (s, 3H), 3.47-3.37 (m, 1H), 3.08-3.17 (m, 1H), 2.69-2.77 (m, 1H), 1.02 (d, 3H), 0.91 (d, 3H), 0.38 (t, 3H); Chiral analytical SFC: RT = 3.24 min, Column: Lux Cellulose-2 (4.6 x 250) mm, 5 µ, Co-Solvent 0.5% diethyl amine in Methanol, 80 %Co-Solvent, Flow rate: 3.0 mL/min

### 3-(3-Chloro-4-fluorophenyl)-1-ethyl-1-(2-methyl-1-(1-oxo-1,2-dihydroisoquinolin-4-yl)propyl)urea (Compounds 54 & 55)

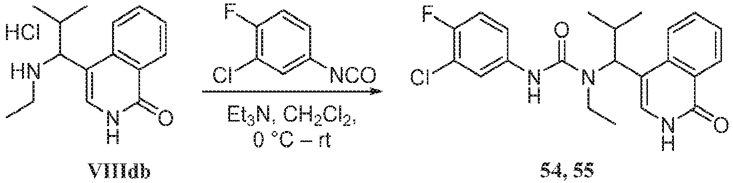

3-(3-Chloro-4-fluorophenyl)-1-ethyl-1-(2-methyl-1-(1 -oxo-1,2-dihydroisoquinolin-4-yl)propyl)urea was synthesised in an analogous manner as described above from scalemic 4-(1-(ethylamino)-2-methylpropyl)isoquinolin-1(2H)-one hydrochloride (**VIIIdb,** ~ 7:3 mixture of enantiomers, derived from (S)-2-methylpropane-2-sulfinamide and isopropyl magnesium bromide) and 2-chloro-1-fluoro-4-isocyanatobenzene. Chiral analytical SFC: 72% and 26% with RT = 4.23 and 6.17 min, respectively [Column: Lux Cellulose-2 (4.6 x 250) mm, 5 µ, 70 % CO₂/MeOH, Flow rate: 3.0 mL/min]. The enantiomers were subsequently separated by chiral SFC, Column: Lux Cellulose-2 (250 x 30 mm) 5 µ, 75% CO₂/MeOH, Flow rate 70 g/min.

3-(3-Chloro-4-fluorophenyl)-1-ethyl-1-(2-methyl-1-(1-oxo-1,2-dihydroisoquinolin-4-yl)propyl)urea - Enantiomer I (**Compound 54**). LCMS: *m*/*z* found 416.2/418.2 [M+H]⁺; RT = 4.60 min (Method A); ¹H NMR (400 MHz, DMSO-d₆): δ 11.42 (bd, 1H), 8.27 (bs, 1H), 8.24 (d, 1H), 8.05 (d, 1H), 7.81-7.83 (m, 1H), 7.70-7.75 (m, 1H), 7.46-7.53 (m, 2H), 7.28-7.35 (m, 2H), 5.44 (d, 1H), 3.38-3.44 (m, 1H), 3.07-3.14 (m, 1H), 2.53-2.56 (m, 1H), 0.96 (d, 3H), 0.92 (d, 3H), 0.55 (t, 3H); Chiral analytical SFC: RT = 4.29 min, Column: Lux Cellulose-2 (4.6 x 250mm), 5 µ, 70% CO₂/MeOH, Flow rate: 3.0 mL/min

3-(3-Chloro-4-fluorophenyl)-1-ethyl-1-(2-methyl-1-(1-oxo-1,2-dihydroisoquinolin-4-yl)propyl)urea - Enantiomer II (**Compound 55**). LCMS: *m*/*z* found 416.2/418.2 [M+H]⁺; RT = 4.61 min (Method A); ¹H NMR (400 MHz, DMSO-d₆): δ 11.42 (bd, 1H), 8.27 (bs, 1H), 8.24 (d, 1H), 8.05 (d, 1H), 7.81-7.83 (m, 1H), 7.70-7.75 (m, 1H), 7.46-7.53 (m, 2H), 7.28-7.35 (m, 2H), 5.44 (d, 1H), 3.38-3.44 (m, 1H), 3.07-3.14 (m, 1H), 2.53-2.56 (m, 1H), 0.96 (d, 3H), 0.92 (d, 3H), 0.55 (t, 3H); Chiral analytical SFC: RT = 6.27 min, Column: Lux Cellulose-2 (4.6 x 250mm), 5 µ, 70% CO₂/MeOH, Flow rate: 3.0 mL/min.

### 2,2,2-Trifluoro-1-(1-methoxyisoquinolin-4-yl)ethan-1-one (Vw)

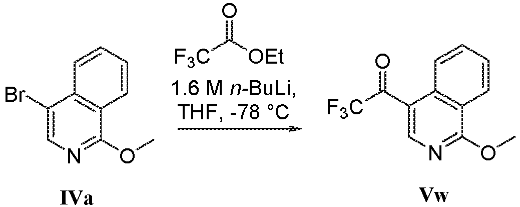

To a stirred solution of 1.0 g (4.20 mmol, 1.0 eq.) of 4-bromo-1-methoxyisoquinoline (**IVa**) in 40 mL of anhydrous THF at -78 °C under a nitrogen atmosphere was added 3.9 mL (6.30 mmol, 1.5 eq.) of a 1.6 M solution of n-BuLi in hexanes. The mixture was stirred at -78 °C for 30 min and 0.72 g (5.04 mmol, 1.2 eq.) of ethyl trifluoroacetate was added drop-wise over approximately 10 min. Stirring was continued for a further 1 h and the reaction was quenched with saturated 30 mL of saturated aqueous ammonium chloride. The resulting solution was allowed to warm to room temperature, diluted with 30 mL of water and extracted with 3 x 50 mL of ethyl acetate. The combined organic extracts were dried (Na₂SO₄), filtered and the solvent was removed *in vacuo.* The residue was purified by chromatography (neutral alumina, eluting with a linear gradient of 5-10% ethyl acetate/petroleum ether) to provide 0.6 g (2.35 mmol, 56%) of 2,2,2-trifluoro-1-(1-methoxyisoquinolin-4-yl)ethan-1-one (**Vw**). LCMS: *m*/*z* found 256.4 [M+H]⁺, RT = 2.76 min; ¹H NMR (400 MHz, CDCl₃) δ 8.99-9.02 (m, 1H), 8.87-8.89 (m, 1H), 8.35-8.37 (m, 1H), 7.85-7.89 (m, 1H), 7.64-7.68 (m, 1H), 4.26 (s, 3H).
The above detailed reaction was performed on multiple batches with consistent results.

### (R)-2-Methyl-N-(2,2,2-trifluoro-1-(1-methoxyisoquinolin-4-yl)ethylidene)propane-2-sulfinamide (XIIc)

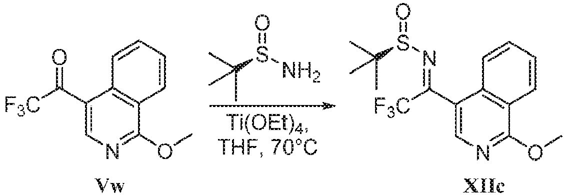

To a solution of 0.6 g (2.35 mmol, 1.0 eq.) of 2,2,2-trifluoro-1-(1-methoxyisoquinolin-4-yl)ethan-1-one (**Vw**) in 10 mL of THF in a sealed tube was added 0.29 g (2.39 mmol, 1.02 eq.) of (*R*)-2-methylpropane-2-sulfinamide followed by 1.07 g (4.70 mmol, 2.0 eq.) of titanium tetraethoxide and the mixture was stirred at 70 °C for 15 h. The mixture was allowed to cool to room temperature, diluted with 40 mL of ice-cold water and extracted with 3 x 50 mL of ethyl acetate. The combined organic extracts were dried (Na₂SO₄), filtered and the solvent was removed *in vacuo* to provide 0.65 g of crude (*R*)-2-methyl-*N*-(2,2,2-trifluoro-1-(1-methoxyisoquinolin-4-yl)ethylidene)propane-2-sulfinamide (**XIIc**). LCMS: *m*/*z* found 359.4 [M+H]⁺, RT = 2.76 min.
The above detailed reaction was performed on multiple batches with consistent results.

### (R)-2-Methyl-N-(2,2,2-trifluoro-1-(1-methoxyisoquinolin-4-yl)ethyl)propane-2-sulfinamide (XIIIe)

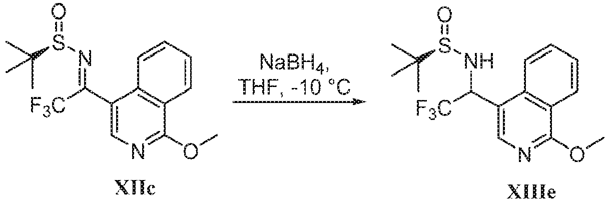

To a solution of 0.65 g of crude (*R*)-2-methyl-*N*-(2,2,2-trifluoro-1-(1-methoxyisoquinolin-4-yl)ethylidene)propane-2-sulfinamide (**XIIc**) in 10 mL of THF at -10 °C was added 69 mg (1.81 mmol, 1.0 eq.) of sodium borohydride and the mixture was stirred for 2 h. The mixture was then diluted with 20 mL of ice-cold water and extracted with 3 x 50 mL of ethyl acetate. The combined organic extracts were washed with 30 mL of brine, dried (Na₂SO₄), filtered and the solvent was removed *in vacuo.* The residue was purified by chromatography (neutral alumina, eluting with a linear gradient of 10-15% ethyl acetate/petroleum ether) to provide 0.4 g (1.11 mmol, 47% from **Vw**) of (R)-2-methyl-N-(2,2,2-trifluoro-1-(1-methoxyisoquinolin-4-yl)ethyl)propane-2-sulfinamide (**XIIIe** as ~ 20:1 mixture of diastereoisomers). LCMS: *m*/*z* found 361.5 [M+H]⁺, RT = 2.43 min; ¹H NMR (400 MHz, CDCl₃): δ 8.34 (d, 1H), 8.21 (s, 1H), 8.01-7.98 (m, 1H), 7.77-7.72 (m, 1H), 7.61-7.57(m, 1H), 5.49 (br s, 1H), 4.16 (s, 3H), 3.96-3.95 (m, 1H), 1.23 (s, 9H). The above detailed reaction was performed on multiple batches with consistent results.

### 2-Methyl-N-(2,2,2-trifluoro-1-(1-methoxyisoquinolin-4-yl)ethyl)propane-2-sulfonamide (VIbg)

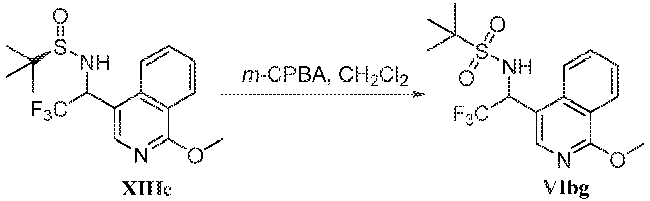

To a solution of 1.3 g (3.61 mmol, 1.0 eq.) of (R)-2-methyl-N-(2,2,2-trifluoro-1-(1-methoxyisoquinolin-4-yl)ethyl)propane-2-sulfinamide (**XIIIe**) in 10 mL of methylene chloride at 0 °C was added 1.8 g (7.22 mmol, 2.0 eq.) of 70 % m-CPBA. The mixture was allowed to warm to room temperature and stirred for 2 h. The mixture was then diluted with 25 mL of water and extracted with 3 x 40 mL of ethyl acetate. The combined organic extracts were washed with 50 mL of brine, dried (Na₂SO₄), filtered and the solvent was removed *in vacuo* to provide 1.2 g (3.19 mmol, 88%) of 2-methyl-*N*-(2,2,2-trifluoro-1-(1-methoxyisoquinolin-4-yl)ethyl)propane-2-sulfonamide (**VIbg**). LCMS: *m*/*z* found 377.5 [M+H]⁺, RT = 2.52 min.

### N-Ethyl-2-methyl-N-(2,2,2-trifluoro-1-(1-methoxyisoquinolin-4-yl)ethyl)propane-2-sulfonamide (VIIj)

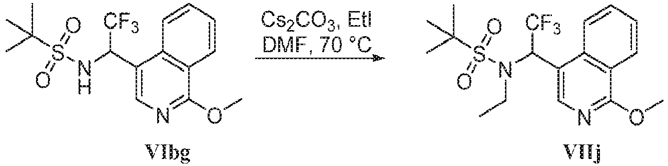

To a solution of 1.2 g (3.19 mmol, 1.0 eq.) of 2-methyl-*N*-(2,2,2-trifluoro-1-(1-methoxyisoquinolin-4-yl)ethyl)propane-2-sulfonamide (**VIbg**, derived from (R)-2-methylpropane-2-sulfinamide) in 10 mL of DMF under a nitrogen atmosphere was added 3.16 g (9.69 mmol, 3.0 eq.) of cesium carbonate followed by 2.49 g (15.47 mmol, 5.0 eq.) of ethyl iodide and the mixture was heated at 70 °C for 8 h. The mixture was then allowed to cool to room temperature, quenched with 50 mL of ice-cold water and extracted with 3 x 100 mL of ethyl acetate. The combined organic extracts were washed with 100 mL of brine, dried (Na₂SO₄), filtered and the solvent was removed *in vacuo.* The residue was purified by chromatography (neutral alumina, eluting with a linear gradient of 0-10% ethyl acetate/petroleum ether) to provide 1.2 g (2.97 mmol, 93%) of *N*-ethyl-2-methyl-N-(2,2,2-trifluoro-1-(1-methoxyisoquinolin-4-yl)ethyl)propane-2-sulfonamide (**VIIj**). ¹H NMR (400 MHz, CDCl₃): δ 8.31-8.34 (m, 2H), 8.17-8.21 (m, 1H), 7.80-7.85 (m, 1H), 7.60-7.64 (m, 1H), 6.48-6.52 (m, 1H), 4.17 (s, 3H), 3.42-3.50 (m, 1H), 2.72-2.83 (m, 1H), 1.48 (bs, 9H), 1.02-1.13 (m, 3H).

### N-Ethyl-2,2,2-trifluoro-1-(1-methoxyisoquinolin-4-yl)ethan-1-amine (VIbh)

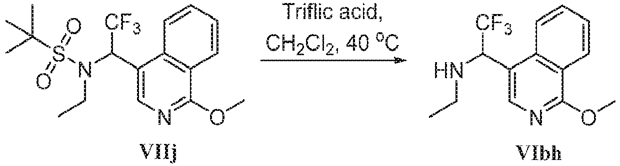

To a solution of 1.2 g (2.97 mmol, 1.0 eq.) of *N*-ethyl-2-methyl-*N*-(2,2,2-trifluoro-1-(1-methoxyisoquinolin-4-yl)ethyl)propane-2-sulfonamide (**VIIj**, derived from (*R*)-2-methylpropane-2-sulfinamide) in 10 mL of methylene chloride was added 2.22 g (14.85 mmol, 5.0 eq.) of triflic acid and the mixture was heated at 40 °C for 6 h. The mixture was allowed to cool to room temperature, slowly diluted with 5 mL saturated sodium bicarbonate solution and extracted with 3 x 50 mL of ethyl acetate. The combined organic extracts were washed with 40 mL brine, dried (Na₂SO₄), filtered and the solvent was removed *in vacuo* to provide 0.8 g (2.81 mmol, 94%) of *N*-ethyl-2,2,2-trifluoro-1-(1-methoxyisoquinolin-4-yl)ethan-1-amine (**VIbh**). LCMS: *m*/*z* found 285.4 [M+H]⁺, RT = 2.28 min.

### 3-(3-Chloro-4-fluorophenyl)-1-ethyl-1-(2,2,2-trifluoro-1-(1-methoayisoquinolin-4-yl)ethyl)urea - Enantiomer I

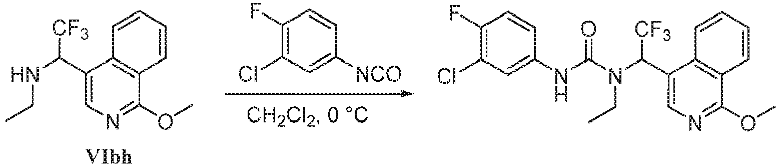

To a solution of 0.8 g (2.81 mmol, 1.0 eq.) of *N*-ethyl-2,2,2-trifluoro-1-(1-methoxyisoquinolin-4-yl)ethan-1-amine (**VIbh,** derived from (R)-2-methylpropane-2-sulfinamide) in 10 mL of methylene chloride at 0 °C was added 0.48 g (2.81 mmol, 1.0 eq.) of 2-chloro-1-fluoro-4-isocyanatobenzene and the mixture was stirred for 2 h. The mixture was then diluted with10 mL of water and extracted with 2 x 30 mL of methylene chloride. The combined organic extracts were dried (Na₂SO₄), filtered and the solvent was removed *in vacuo.* The residue was purified by column chromatography (SiO₂, eluted with a linear gradient of 5-10% ethyl acetate/petroleum ether) to provide 350 mg (0.77 mmol, 27%) of 3-(3 -chloro-4-fluorophenyl)-1 -ethyl-1 -(2,2,2-trifluoro-1 -(1 -methoxy isoquinolin-4-yl)ethyl)urea. LCMS: *m*/*z* found 456.1 [M+H]⁺, RT = 2.61 min; Chiral analytical SFC: 94% and 3 % at RT = 6.71 min and 11.52 min, respectively, Column: (R,R) Whelk-01 (4.6 x 250 mm) 5 µ, 80 % CO₂/ⁱPrOH, Flow rate: 3.0 mL/min. The major enantiomer was subsequently isolated by chiral SFC, Column: (R,R)-Whelk-01 (250 x 30 mm) 5 µ, 80% CO₂/MeOH, Flow rate 100 g/min.

3-(3-Chloro-4-fluorophenyl)-1-ethyl-1-(2,2,2-trifluoro-1-(1-methoxyisoquinolin-4-yl)ethyl)urea - Enantiomer I. LCMS: *m*/*z* found 456.1/458.1 [M+H]⁺, RT = 6.74 min; ¹H NMR (400 MHz, DMSO-d₆): δ 8.75 (bs, 1H), 8.30 (d, 1H), 8.26 (s, 1H), 7.99 (d, 1H), 7.90-7.93 (m, 1H), 7.83-7.86 (m, 1H), 7.70-7.74 (m, 1H), 7.53-7.57 (m, 1H), 7.35-7.40 (m, 1H), 6.89-6.96 (m, 1H), 4.13 (s, 3H), 3.57-3.63 (m, 1H), 3.25-3.28 (m, 1H), 0.46 (t, 3H); Chiral analytical SFC: RT = 6.86 min, Column: (R,R)-Whelk-01 (4.6 x 250 mm) 5 µ, 80 % CO₂/ⁱPrOH, Flow rate: 3.0 mL/min.

### 3-(3-Chloro-4-fluorophenyl)-1-ethyl-1-(2,2,2-trifluoro-1-(1-methoayisoquinolin-4-yl)ethyl)urea - Enantiomer II (Compound 75)

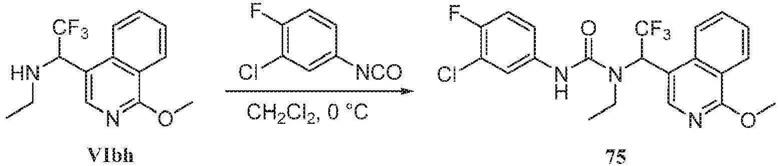

3-(3-Chloro-4-fluorophenyl)-1-ethyl-1-(2,2,2-trifluoro-1-(1-methoxyisoquinolin-4-yl)ethyl)urea wassynthesized in a similar manner as described above from *N*-ethyl-2,2,2-trifluoro-1-(1-methoxyisoquinolin-4-yl)ethan-1-amine (**VIbh,** derived from (*S*)-2-methylpropane-2-sulfinamide) and 2-chloro-1-fluoro-4-isocyanatobenzene. The major enantiomer was subsequently separated by chiral SFC, Column: (R,R)-Whelk-01 (250 x 30 mm) 5 µ, 80% CO₂/MeOH, Flow rate 100 g/min.

3-(3-Chloro-4-fluorophenyl)-1-ethyl-1-(2,2,2-trifluoro-1-(1-methoxyisoquinolin-4-yl)ethyl)urea - Enantiomer II (**Compound 75**). LCMS: *m*/*z* found 456.1/458.1 [M+H]⁺, RT = 6.74 min; ¹H NMR (400 MHz, DMSO-d₆): δ 8.75 (bs, 1H), 8.30 (d, 1H), 8.26 (s, 1H), 7.99 (d, 1H), 7.90-7.93 (m, 1H), 7.83-7.86 (m, 1H), 7.70-7.74 (m, 1H), 7.53-7.57 (m, 1H), 7.35-7.40 (m, 1H), 6.89-6.96 (m, 1H), 4.13 (s, 3H), 3.57-3.63 (m, 1H), 3.25-3.28 (m, 1H), 0.46 (t, 3H); Chiral analytical SFC: RT = 11.29 min, Column: (R,R)-Whelk-01 (4.6 x 250 mm) 5 µ, 80 % CO₂/ⁱPrOH, Flow rate: 3.0 mL/min.

### 4-(l-(Ethylamino)-2,2,2-trifluoroethyl)isoquinolin-1(2H)-one (VIIIdc)

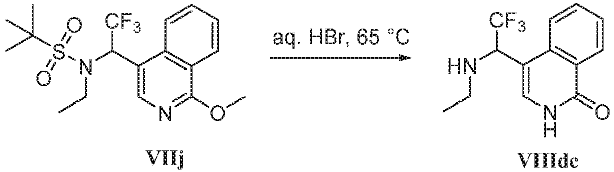

A solution of 0.8 g (1.98 mmol, 1.0 eq.) of *N-*ethyl-2-methyl-*N-*(2,2,2-trifluoro-1-(1-methoxyisoquinolin-4-yl)ethyl)propane-2-sulfonamide (**VIIIdc**, derived from (R)-2-methylpropane-2-sulfinamide) in 4.8 mL of a 47% solution of aqueous HBr was heated at 65 °C for 6 h. The mixture was allowed to cool to room temperature, basified to pH>8 with 25 mL of saturated sodium bicarbonate solution and extracted with 3 x 50 mL of ethyl acetate. The combined organic extracts were washed with 40 mL of brine, dried (Na₂SO₄), filtered and the solvent was removed *in vacuo* to provide 0.4 g (2.81 mmol, 94%) of 4-(1-(ethylamino)-2,2,2-trifluoroethyl)isoquinolin-1(2*H*)-one (**VIIIdc**). LCMS: *m*/*z* found 271.4 [M+H]⁺, RT = 1.72 min.

### 3-(3-chloro-4-fluorophenyl)-1-ethyl-1-(2,2,2-trifluoro-1-(1-oxo-1,2-dihydroisoquinolin-4-yl)ethyl)urea - Enantiomer I (Compound 74)

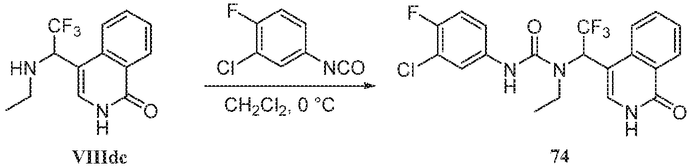

3-(3-Chloro-4-fluorophenyl)-1-ethyl-1-(2,2,2-trifluoro-1-(1-methoxyisoquinolin-4-yl)ethyl)urea - Enantiomer I was synthesized in a similar manner as described above from N-ethyl-2,2,2-trifluoro-1-(1-methoxyisoquinolin-4-yl)ethan-1-amine (**VIIIdc,** derived from (R)-2-methylpropane-2-sulfinamide) and 2-chloro-1-fluoro-4-isocyanatobenzene. LCMS: *m*/*z* found 442.34 [M+H]⁺, RT = 2.47 min; Chiral analytical SFC: 95% and 4 % at RT = 3.41 min and 4.45 min, respectively, Column: Chiralcel OX-H (4.6 x 250 mm) 5 µ, 80 % CO₂/MeOH, Flow rate: 3.0 mL/min. The major enantiomer was subsequently separated by chiral SFC, Column: Chiralcel OX-H (250 x 30 mm) 5 µ, 90% CO₂/MeOH, Flow rate 90 g/min.

3-(3-chloro-4-fluorophenyl)-1-ethyl-1-(2,2,2-trifluoro-1-(1-oxo-1,2-dihydroisoquinolin-4-yl)ethyl)urea - Enantiomer I (**Compound 74**) - LCMS: *m*/*z* found 442.2/444.1 [M+H]⁺, RT = 5.62 min; ¹H NMR (400 MHz, DMSO-d₆): δ 11.58 (bs, 1H), 8.74 (bs, 1H), 8.29 (d, 1H), 7.80-7.85 (m, 2H), 7.71 (d, 1H), 7.52-7.59 (m, 2H), 7.37 (t, 1H), 7.31 (s, 1H), 6.64-6.69 (m, 1H), 3.56-3.63 (m, 1H), 3.28-3.36 (m, 1H), 0.63 (t, 3H); Chiral analytical SFC: RT = 3.45 min, Column: Chiralcel OX-H (4.6 x 250 mm) 5 µ, 80 % CO₂/MeOH, Flow rate: 3.0 mL/min.

### 3-(3-Chloro-4-fluorophenyl)-1-ethyl-1-(2,2,2-trifluoro-1-(1-oxo-1,2-dihydroisoquinolin-4-yl)ethyl)urea - Enantiomer II (Compound 76)

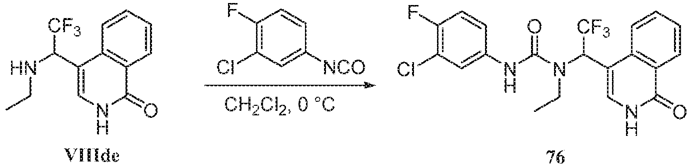

3-(3-Chloro-4-fluorophenyl)-1-ethyl-1-(2,2,2-trifluoro-1-(1-methoxyisoquinolin-4-yl)ethyl)urea - Enantiomer II was synthesized in a similar manner as described above from *N*-ethyl-2,2,2-trifluoro-1-(1-methoxyisoquinolin-4-yl)ethan-1-amine (**VIIIde**, derived from (*S*)-2-methylpropane-2-sulfinamide) and 2-chloro-1-fluoro-4-isocyanatobenzene. LCMS: *m*/*z* found 442.34 [M+H]⁺, RT = 2.47 min; Chiral analytical SFC: 95% and 4 % at RT = 3.43 min and 4.45 min, respectively, Column: Chiralcel OX-H (4.6 x 250 mm) 5 µ, 80 % CO₂/MeOH, Flow rate: 3.0 mL/min. The major enantiomer was subsequently isolated by chiral SFC, Column: Chiralcel OX-H (250 x 30 mm) 5 µ, 90% CO₂/MeOH, Flow rate 90 g/min.

3-(3-Chloro-4-fluorophenyl)-1-ethyl-1-(2,2,2-trifluoro-1-(1-oxo-1,2-dihydroisoquinolin-4-yl)ethyl)urea - Enantiomer II (**Compound 76**) - LCMS: *m*/*z* found 442.2/444.1 [M+H]⁺, RT = 5.62 min; ¹H NMR (400 MHz, DMSO-d₆): δ 11.58 (bs, 1H), 8.74 (bs, 1H), 8.29 (d, 1H), 7.80-7.85 (m, 2H), 7.71 (d, 1H), 7.52-7.59 (m, 2H), 7.37 (t, 1H), 7.31 (s, 1H), 6.64-6.69 (m, 1H), 3.56-3.63 (m, 1H), 3.28-3.36 (m, 1H), 0.63 (t, 3H); Chiral analytical SFC: RT = 4.49 min, Column: Chiralcel OX-H (4.6 x 250 mm) 5 µ, 80 % CO₂/MeOH, Flow rate: 3.0 mL/min.

### 1-(6,7-Difluoro-1-methoxy-4-isoquinolyl)ethenone (Vg)

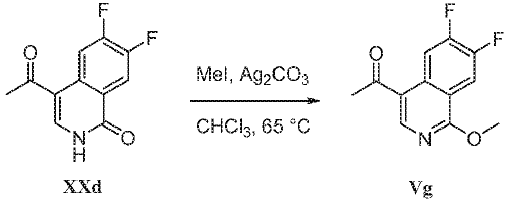

A mixture of 2.5 mL (40.3 mmol) of iodomethane, 7.4 g (26.9 mmol) of silver carbonate and 3.0 g (13.4 mmol) of 4-acetyl-6,7-difluoro-2*H*-isoquinolin-1-one (**XXd**) in 70 mL of chloroform in a sealed tube was heated at 65 °C for 24 h. The mixture was allowed to cool to room temperature and diluted with 32 mL of ethyl acetate and 8 mL of acetonitrile. The mixture was then filtered through a pad of CELITE^{®} and the pad was washed with 100 mL of ethyl acetate. The combined filtrate was evaporated *in vacuo* and the residue was purified by flash chromatography (SiO₂, eluting with a gradient of 0-50% ethyl acetate/hexanes) to provide 2.1 g (8.7 mmol, 65%) of 1-(6,7-difluoro-1-methoxy-4-isoquinolyl)ethenone (**Vg**). ¹H NMR (400 MHz, CDCl₃) δ 9.01(m, 1H), 8.74 (s, 1H), 8.02 (m, 1H), 4.19 (s, 3H), 2.70 (s, 3H).

### (S)-N-[1-(6,7-Difluoro-1-methoxy-4-isoquinolyl)ethyl]-2-methyl-propane-2-sulfinamide (XIIIb)

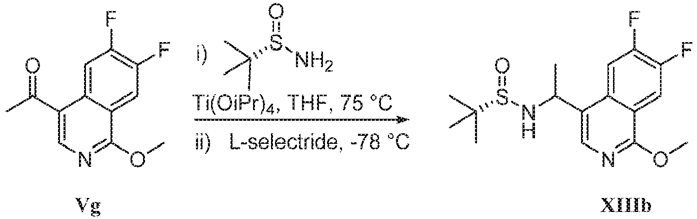

To a mixture of 1.83 g (7.72 mmol, 1.0 eq.) of 1-(6,7-difluoro-1-methoxy-4-isoquinolyl)ethanone (**Vg**) and 1.22 g (10.03 mmol, 1.3 eq.) of (S)-2-methylpropane-2-sulfinamide in 2.2 mL of anhydrous THF in a sealed tube was added 4.57 mL (15.44 mmol, 2.0 eq.) of tetraisopropoxytitanium and the mixture was heated at 80 °C for 26 h. The mixture was allowed to cool to room temperature and diluted with 35 mL of THF. The mixture was further cooled to -78 °C under a nitrogen atmosphere and 7.73 mL (7.73 mmol, 1.0 eq.) of a 1 M solution of L-selectride in THF was added and the mixture was stirred at -78 °C for 90 minutes. An additional portion of 0.3 mL (0.3 mmol) of a 1 M solution of L-selectride in THF was added and stirring was continued for a further 45 min. The mixture then was diluted with 10 mL of methanol and the cooling bath was removed. Upon warming to room temperature, the mixture was diluted with 70 mL of 1:6 v/v acetonitrile/ethyl acetate and added to 10 mL of a rapidly stirred brine solution. After stirring for 10 minutes, the mixture was filtered through CELITE^{®} and the pad was washed with 20 mL of ethyl acetate. The combined filtrate was evaporated *in vacuo* and the major diastereoisomer was isolated by flash chromatography (SiO₂, eluting with a gradient of 0-100% of ethyl acetate/methylene chloride) to provide 1.70 g (4.96 mmol, 64%) of diastereomerically pure (S)-*N*-[1-(6,7-difluoro-1-methoxy-4-isoquinolyl)ethyl]-2-methyl-propane-2-sulfinamide (**XIIIb**). ¹H NMR (400 MHz, CDCl₃) δ 7.98-8.08 (m, 2H), 7.83 (m, 1H), 4.91-5.02 (m, 1H), 4.11 (s, 3H), 3.34 (d, 1H), 1.73 (d, 3H), 1.21 (s, 9H).

### (S)-N-(1-(6,7-difluoro-1-methoxyisoquinolin-4-yl)ethyl)-N,2-dimethylpropane-2-sulfinamide (XIVb)

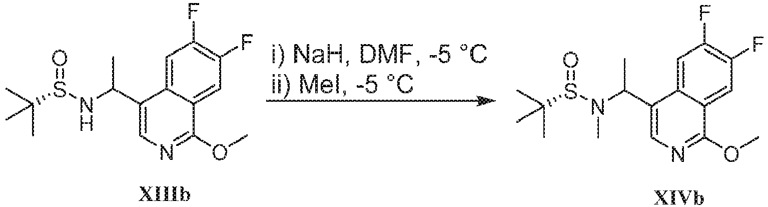

To a solution of 1.47 g (4.29 mmol, 1.0 eq.) of diastereomerically pure (S)-*N*-[1-(6,7-difluoro-1-methoxy-4-isoquinolyl)ethyl]-2-methyl-propane-2-sulfinamide (**XIIIb**) in 22 mL of anhydrous DMF under a nitrogen atmosphere at -5 °C was added 0.31 g (7.73 mmol, 1.8 eq.) of a 60% dispersion of sodium hydride in mineral oil. The mixture was stirred at -5 °C for 25 minutes and 0.53 mL (8.59 mmol, 2.0 eq.) of iodomethane was added. The mixture was stirred at -5 °C for a further 20 min and then quenched by the addition of 30 mL water. The mixture was then extracted with 2 x 50 mL of ethyl acetate and the combined organic extracts were washed with 2 x 30 mL of water followed by 30 mL of brine, dried (Na₂SO₄), filtered and the solvent was removed *in vacuo.* The residue was purified by flash chromatography (SiO₂, eluting with a gradient of 0% to 5% MeOH/DCM) to provide 1.39 (3.90 mmol, 90%) of (S)-*N*-[1-(6,7-difluoro-1-methoxy-4-isoquinolyl)ethyl]-*N*,2-dimethylpropane-2-sulfinamide (**XIVb**). LCMS: *m*/*z* found 357.2 [M+H]⁺, RT = 4.73 min (Method A). ¹H NMR (400 MHz, CDCl₃) δ 7.97-8.07 (m, 2H), 7.76 (m, 1H), 5.00-5.11 (m, 1H), 4.12 (s, 3H), 2.37 (s, 3H), 1.75 (d, 3H), 1.24 (s, 9H).

### 6,7-Difluoro-4-(1-(methylamino)ethyl)isoquinolin-1(2H)-one hydrochloride salt (VIIIs)

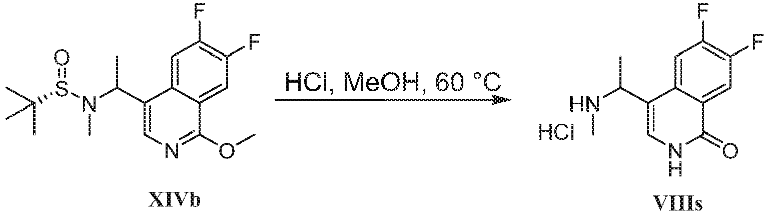

To a solution of 1.39 g (3.90 mmol, 1.0 eq.) of (S)-*N*-[1-(6,7-difluoro-1-methoxy-4-isoquinolyl)ethyl]-*N*,2-dimethyl-propane-2-sulfinamide (**XIVb**) in 14 mL of methanol in a sealed tube was added 13 mL (39.0 mmol, 10 eq.) of a 3 M solution of HCl in methanol and the mixture was heated at 60 °C for 15 h. The mixture was allowed to cool to room temperature and the volatiles were removed *in vacuo.* The residue was subsequently triturated with 2 x 10 mL of diethyl ether to provide 1.09 g of 6,7-difluoro-4-[1-(methylamino)ethyl]-2H-isoquinolin-1-one hydrochloride salt. ¹H NMR (400 MHz, Methanol-*d*₄) δ 8.22 (m, 1H), 7.94 (m, 1H), 7.51 (s, 1H), 4.81-4.92 (m, 1H), 2.72 (s, 3H), 1.73 (d, 3H).

### 3-(3-Chloro-4-fluorophenyl)-1-(1-(6,7-difluoro-1-oxo-1,2-dihydroisoquinolin-4-yl)ethyl)-1-methylurea (Compound 279)

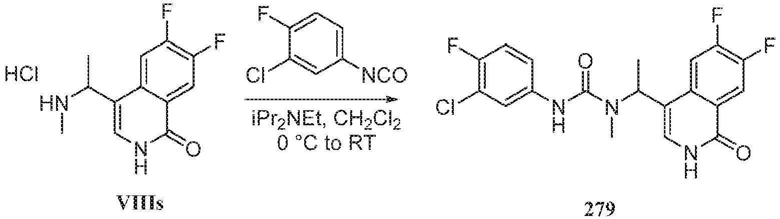

To a mixture of 23 mg (0.08 mmol, 1.0 eq.) of 6,7-difluoro-4-[1-(methylamino)ethyl]-2H-isoquinolin-1-one hydrochloride (**VIIIs**, derived from (S)-2-methylpropane-2-sulfinamide and methyl iodide) in 1.8 mL of methylene chloride at 0 °C was added 37 uL (0.21 mmol, 2.6 eq.) of *N,N*-diisopropylethyl amine followed by a solution of 11 uL (0.08 mmol, 1.0 eq.) of 2-chloro-1-fluoro-4-isocyanato-benzene in 0.5 mL of methylene chloride. The mixture was allowed to warm to room temperature and stirred for 10 min. The mixture was then loaded directly onto a pre-equilibrated silica column and the product isolated by flash chromatography (SiO₂, eluting with a gradient of 0.5% - 7% methanol/methylene chloride) to provide 26 mg (0.06 mmol, 75%) of 3-(3-chloro-4-fluoro-phenyl)-1-[1-(6,7-difluoro-1-oxo-2H-isoquinolin-4-yl)ethyl]-1-methyl-urea as a single enantiomer (**Compound 279**, Enantiomer II). LCMS: *m*/*z* found 410.2/412.3 [M+H]+, RT = 4.32 min (Method A); ¹H NMR (400 MHz, DMSO-*d*₆) δ 11.63 (s, 1H), 8.48 (s, 1H), 8.11 (m, 1H), 7.82 (m, 1H), 7.73 (m, 1H), 7.50 (m, 1H), 7.33 (t, 1H), 7.22 (d, 1H), 5.78 (m, 1H), 2.60 (s, 3H), 1.42 (d, 3H).

### 3-(3-Cyano-4-fluorophenyl)-1-(1-(6,7-difluoro-1-oxo-1,2-dihydroisoquinolin-4-yl)ethyl)-1-methylurea (Compounds 429)

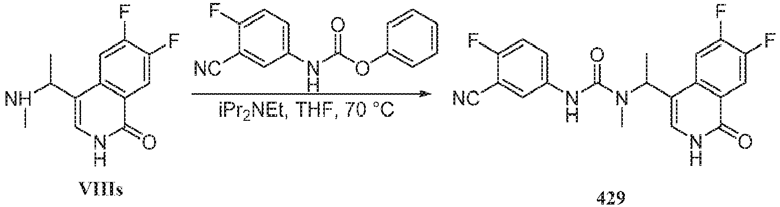

3-(3-Cyano-4-fluorophenyl)-1-(1-(6,7-difluoro-1-oxo-1,2-dihydroisoquinolin-4-yl)ethyl)-1-methylurea as a single enantiomer (**Compound 429**) was synthesized in a similar manner as described above from enantiomerically pure 6,7-difluoro-4-(1-(methylamino)ethyl) isoquinolin-1(2*H*)-one (**VIIIs,** derived from (S)-2-methylpropane-2-sulfinamide and methyl iodide) and phenyl (3-cyano-4-fluorophenyl)carbamate.

3-(3-Cyano-4-fluorophenyl)-1-(1-(6,7-difluoro-1-oxo-1,2-dihydroisoquinolin-4-yl)ethyl)-1-methylurea - Enantiomer II (**Compound 429**), LCMS: *m*/*z* found 401.2 [M+H]⁺, RT = 3.81 min (Method A); ¹H NMR (400 MHz, DMSO-d₆): δ 11.62 (bs, 1H), 8.65 (bs, 1H), 8.09-8.14 (m, 1H), 8.00-8.03 (m, 1H), 7.85-7.89 (m, 1H), 7.68-7.73 (m, 1H), 7.46 (t, 1H), 7.23 (d, 1H), 5.76-5.81 (m, 1H), 2.61 (s, 3H), 1.43 (d, 3H).

### (S)-N-(1-(6,7-Difluoro-1-methoxyisoquinolin-4-yl)ethyl)-2-methyl-N-(methyl-d₃)propane-2-sulfinamide (XIVe)

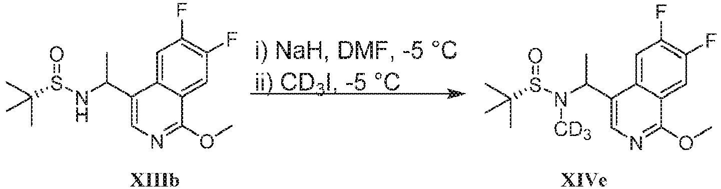

To a solution of 43 mg (0.13 mmol, 1.0 eq.) of diastereomerically pure (S)-*N*-[1-(6,7-difluoro-1-methoxy-4-isoquinolyl)ethyl]-2-methyl-propane-2-sulfinamide (**XIIIb,** derived from (S)-2-methylpropane-2-sulfinamide and L-selectride) in 3 mL of anhydrous DMF under a nitrogen atmosphere at -5 °C was added 10 mg (0.25 mmol, 2.0 eq.) of a 60% dispersion of sodium hydride in mineral oil. The mixture was stirred at -5 °C for 25 minutes and 16 uL (0.23 mmol, 2.0 eq.) of trideuterio(iodo)methane was added. The mixture was stirred at -5 °C for a further 15 min and then quenched by the addition of 15 mL water. The mixture was then extracted with 2 x 15 mL of ethyl acetate and the combined organic extracts were washed with 2 x 10 mL of water followed by 10 mL of brine, dried (Na₂SO₄), filtered and the solvent was removed *in vacuo.* The residue was purified by flash chromatography (SiO₂, eluting with a gradient of 20 % to 100 % ethyl acetate/hexanes) to provide 36 mg (0.1 mmol, 80 %) of (S)-*N*-(1-(6,7-difluoro-1-methoxyisoquinolin-4-yl)ethyl)-2-methyl-*N*-(methyl-d₃)propane-2-sulfinamide (**XIVe**). LCMS: *m*/*z* found 360.3 [M+H]⁺, RT = 1.01 min. ¹H NMR (400 MHz, CDCl₃) δ 7.97-8.07 (m, 2H), 7.76 (m, 1H), 5.05 (q, 1H), 4.12 (s, 3H), 1.75 (d, 3H), 1.24 (s, 9H).

### 6,7-Difluoro-4-(1-((methyl-d₃)amino)ethyl)isoquinolin-1(2H)-one hydrochloride (VIIIde)

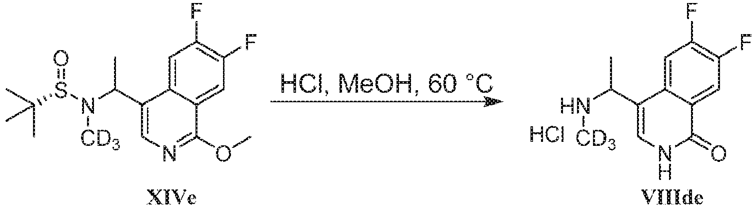

To a solution of 36 mg (0.10 mmol, 1.0 eq.) of (S)-*N*-(1-(6,7-difluoro-1-methoxyisoquinolin-4-yl)ethyl)-2-methyl-*N*-(methyl-d₃)propane-2-sulfinamide (**XIVe**, derived from (S)-2-methylpropane-2-sulfinamide and L-selectride) in 3 mL of methanol in a pressure tube was added 0.34 mL (1.01 mmol, 10 eq.) of a 3 M solution of HCl in methanol. The vessel was sealed, and the mixture was heated at 60 °C for 15 h. The mixture was allowed to cool to room temperature and the volatiles were removed *in vacuo.* The residue was subsequently triturated with 2 x 5 mL of diethyl ether to provide 27 mg (0.08 mmol, 96 %) of 6,7-difluoro-4-(1-((methyl-d₃)amino)ethyl)isoquinolin-1(2*H*)-one hydrochloride. ¹H NMR (400 MHz, CD₃OD) δ 8.21 (m, 2H), 7.95 (m, 1H), 7.51 (s, 1H), 4.89 (m, 1H), 1.73 (d, 3H).

### 3-(3-Chloro-4-fluorophenyl)-1-(1-(6,7-difluoro-1-oxo-1,2-dihydroisoquinolin-4-yl)ethyl)-1-(methyl-d₃)urea (Compound 472)

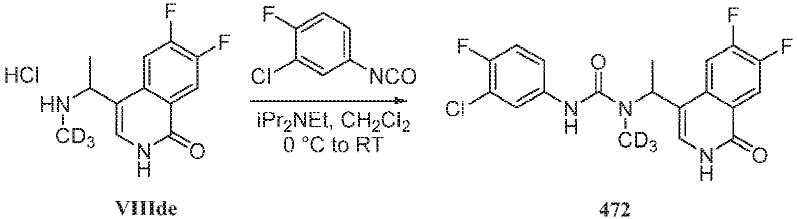

To a mixture of 27 mg (0.10 mmol, 1.0 eq.) of 6,7-difluoro-4-[1-((methyl-d₃)amino)ethyl]-2*H*-isoquinolin-1-one hydrochloride (**VIIIde**, derived from (S)-*N*-(1-(6,7-difluoro-1-methoxyisoquinolin-4-yl)ethyl)-2-methyl-*N*-(methyl-d₃)propane-2-sulfinamide (**XIVe**)) in 1.8 mL of methylene chloride at 0 °C was added 42 uL (0.24 mmol, 2.4 eq.) of *N,N*-diisopropylethyl amine followed by a solution of 12 uL (0.10 mmol, 1.0 eq.) of 2-chloro-1-fluoro-4-isocyanato-benzene in 0.5 mL of methylene chloride. The mixture was allowed to warm to room temperature and stirred for 10 min. The mixture was then loaded directly onto a pre-equilibrated silica column and the product was isolated by flash chromatography (SiO₂, eluting with a gradient of 0.5% - 7% methanol/methylene chloride) to provide 30 mg (0.07 mmol, 75%) of 3-(3-chloro-4-fluoro-phenyl)-1-[1-(6,7-difluoro-1-oxo-2*H*-isoquinolin-4-yl)ethyl]-1-(methyl-d₃)-urea **(Compound 472).** LCMS: *m*/*z* found 413.2/415.2 [M+H]+, RT = 4.31 min (Method A); ¹H NMR (400 MHz, DMSO-*d*₆) δ 11.64 (s, 1H), 8.48 (s, 1H), 8.11 (m, 1H), 7.78 -7.86 (m, 1H), 7.73 (m, 1H), 7.49 (m, 1H), 7.33 (t, 1H), 7.22 (s, 1H), 5.77 (q, 1H), 1.42 (d, 3H).

### 4-(1-Aminoethyl)-6,7-difluoro-2H-isoquinolin-1-one hydrochloride (VIIIdf)

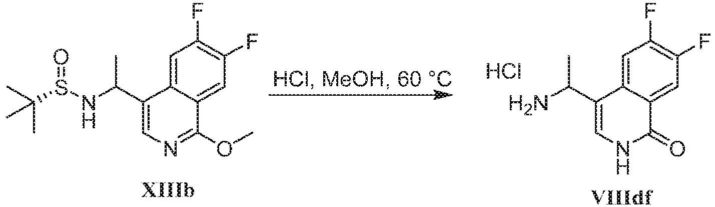

To a solution of 61 mg (0.18 mmol, 1.0 eq.) of diastereomerically pure (S)-N-[1-(6,7-difluoro-1-methoxy-4-isoquinolyl)ethyl]-2-methyl-propane-2-sulfinamide (**XIIIb**, derived from (S)-2-methylpropane-2-sulfinamide and L-selectride) in 3 mL of methanol in a sealed tube was added 6 mL (1.8 mmol, 10.0 eq.) of a 3 M solution HCl in methanol. The vessel was sealed, and the mixture was heated at 70 °C for 16 h. The mixture was allowed to cool to room temperature and the volatiles were removed *in vacuo.* The residue was triturated with 2 x 5 ml of diethyl ether and the residue was dried under high vacuum to provide 48 mg of 4-(1-aminoethyl)-6,7-difluoro-2*H*-isoquinolin-1-one hydrochloride (0.18 mmol, 100%). ¹H NMR (400 MHz, CD₃OD) δ 8.20 (m, 1H), 7.89 (m, 1H), 7.44 (s, 1H), 4.92 (m, 1H), 1.71 (d, 3H).

### 1-(3-Cyano-4-fluorophenyl)-3-(1-(6,7-difluoro-1-oxo-1,2-dihydroisoquinolin-4-yl)ethyl)urea (Compound 495)

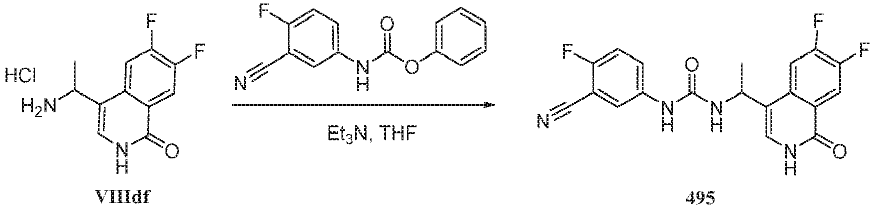

Enantiomerically pure 1-(3-cyano-4-fluorophenyl)-3-(1-(6,7-difluoro-1-oxo-1,2-dihydroisoquinolin-4-yl)ethyl)urea (**Compound 495**) was synthesized in a similar manner as described above from 4-(1-aminoethyl)-6,7-difluoro-2*H*-isoquinolin-1-one hydrochloride (**VIIIdf**, derived from (S)-*N*-[1-(6,7-difluoro-1-methoxy-4-isoquinolyl)ethyl]-2-methylpropane-2-sulfinamide (**XIIIb**)) and phenyl *N*-(3-cyano-4-fluoro-phenyl)carbamate. LCMS: *m*/*z* found 387 [M+H]⁺, RT = 2.76 min (Method B); ¹H NMR (400 MHz, DMSO-d₆) δ 11.51 (s, 1H), 8.72 (s, 1H), 8.12 (m, 1H), 7.91 (m, 1H), 7.84 (m, 1H), 7.65 (m, 1H), 7.40 (t, 1H), 7.17 (s, 1H), 6.80 (d, 1H), 5.11 (q, 1H), 1.45 (d, 3H).

### 1-(3-Chloro-4-fluorophenyl)-3-(1-(6,7-difluoro-1-oxo-1,2-dihydroisoquinolin-4-yl)ethyl)urea (Compound 480)

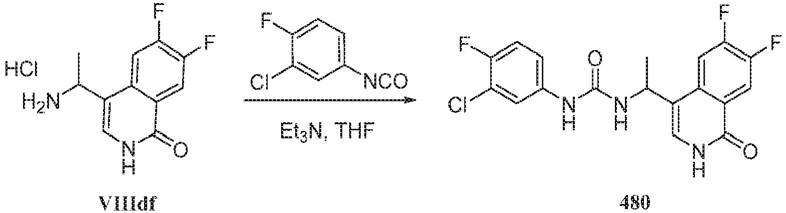

Enantiomerically pure 1-(3-chloro-4-fluorophenyl)-3-(1-(6,7-difluoro-1-oxo-1,2-dihydroisoquinolin-4-yl)ethyl)urea **(Compound 480)** was synthesized in a similar manner as described above from 4-(1-aminoethyl)-6,7-difluoro-2*H*-isoquinolin-1-one hydrochloride (**VIIIdf**, derived from (S)-*N*-[1-(6,7-difluoro-1-methoxy-4-isoquinolyl)ethyl]-2-methylpropane-2-sulfinamide (**XIIIb**)) and 2-chloro-1-fluoro-4-isocyanatobenzene. LCMS: *m*/*z* found 396/398 [M+H]⁺, RT = 2.84 min (Method B); ¹H NMR (400 MHz, DMSO-*d*₆) δ 11.51 (d, 1H), 8.57 (s, 1H), 8.12 (m, 1H), 7.85 (m, 1H), 7.74 (m, 1H), 7.17-7.32 (m, 2H), 7.16 (d, 1H), 6.68 (d, 1H), 5.09 (m, 1H), 1.45 (d, 3H).

### 1-(7,8-Difluoro-1-methoxy-4-isoquinolyl)ethenone (Vy)

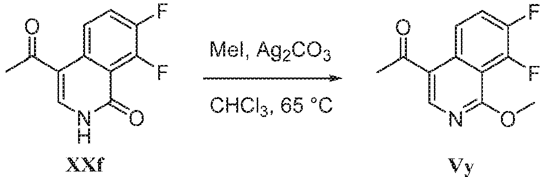

A mixture of 2.51 mL (40.3 mmol, 3.0 eq.) of iodomethane, 7.41 g (26.9 mmol, 2.0 eq.) of silver carbonate and 3.0 g (13.44 mmol, 1.0 eq.) of 4-acetyl-7, 8-difluoro-2*H-*isoquinolin-1-one (**XXf**) in 80 mL of chloroform in a sealed tube was heated at 65 °C for 24 h. The mixture was allowed to cool to room temperature and diluted with 32 mL of ethyl acetate and 8 mL of acetonitrile. The mixture was then filtered through a pad of CELITE^{®} and the pad was washed with 100 mL of ethyl acetate. The combined filtrate was evaporated *in vacuo* and the residue was purified by flash chromatography (SiO₂, eluting with a gradient of 0-90% ethyl acetate/hexanes) to provide 2.03 g (8.56 mmol, 64%) of 1-(6,7-difluoro-1-methoxy-4-isoquinolyl)ethenone (**Vy**). ¹H NMR (400 MHz, CDCl₃) δ 8.87 (m, 1H), 8.67 (s, 1H), 7.61 (m, 1H), 4.20 (s, 3H), 2.71 (s, 3H).

### (S)-N-[1-(7,8-Difluoro-1-methoxy-4-isoquinolyl)ethyl]-2-methyl-propane-2-sulfinamide (XIIIf)

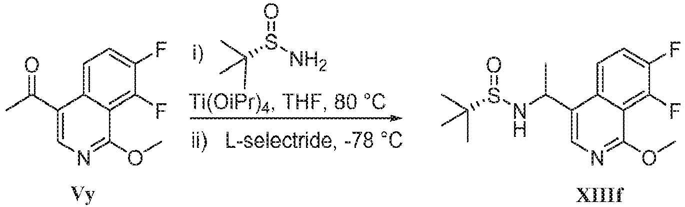

To a mixture of 2.00 g (8.43 mmol, 1.0 eq.) of 1-(7,8-difluoro-1-methoxy-4-isoquinolyl)ethanone (**Vy**) and 1.33 g (10.96 mmol, 1.3 eq.) of (S)-2-methylpropane-2-sulfinamide in 2.4 mL of anhydrous THF in a sealed tube was added 5.00 mL (16.86 mmol, 2.0 eq.) of tetraisopropoxytitanium and the mixture was heated at 80 °C for 25 h. The mixture was allowed to cool to room temperature and diluted with 37 mL of THF. The mixture was further cooled to -78 °C under a nitrogen atmosphere and 8.43 mL (8.43 mmol, 1.0 eq.) of a 1 M solution of L-selectride in THF was added and the mixture was stirred at -78 °C for 160 min. An addition 0.8 mL (0.8 mmol, 0.1 eq.) portion of a 1 M solution of L-selectride in THF was added and the mixture was stirred at -78 °C for an additional 45 min. The mixture was then diluted with 10 mL of methanol, the cooling bath removed, and the mixture was added to 10 mL of a rapidly stirred brine solution and diluted with 70 mL of 1:4 *v*/*v* acetonitrile/ethyl acetate. After stirring for 10 minutes, the mixture was filtered through a pad of CELITE^{®} and the pad was washed with 20 mL of ethyl acetate. The combined filtrate was evaporated *in vacuo* and the major diastereoisomer was isolated by flash chromatography (SiO₂, eluting with a gradient of 0-80% of ethyl acetate/methylene chloride) to provide 1.65 g (4.82 mmol, 57%) of diastereomerically pure (S)-*N*-[1-(7,8-difluoro-1-methoxy-4-isoquinolyl)ethyl]-2-methyl-propane-2-sulfinamide (**XIIIf**). ¹H NMR (400 MHz, CDCl₃) δ 8.05 (t, 1H), 7.80 (m, 1H), 7.48-7.59 (m, 1H), 5.05 (m, 1H), 4.13 (s, 3H), 3.34 (d, 1H), 1.73 (d, 3H), 1.19 (s, 9H).

### (S)-N-(1-(7,8-difluoro-1-methoayisoquinolin-4-yl)ethyl)-N,2-dimethylpropane-2-sulfinamide (XIVf)

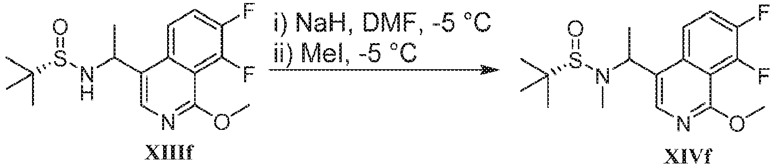

To a solution of 1.64 g (4.79 mmol, 1.0 eq.) of diastereomerically pure (S)-*N*-[1-(7,8-difluoro-1-methoxy-4-isoquinolyl)ethyl]-2-methyl-propane-2-sulfinamide (**XIIIf**) in 24 mL of anhydrous DMF under a nitrogen atmosphere at -5 °C was added 340 mg (8.62 mmol, 1.8 eq.) of a 60% dispersion of sodium hydride in mineral oil. The mixture was stirred at -5 °C for 25 minutes and 0.6 mL (9.58 mmol, 2.0 eq.) of iodomethane was added. The mixture was stirred at -5 °C for a further 30 min and then quenched by the addition of 35 mL of water. The mixture was then extracted with 2 x 50 mL of ethyl acetate and the combined organic extracts were washed with 2 x 30 mL of water followed by 30 mL of brine, dried (Na₂SO₄), filtered and the solvent was removed *in vacuo.* The residue was purified by flash chromatography (SiO₂, eluting with a gradient of 0.5 to 7% MeOH/DCM) to provide 1.54 g (90 %) of (S)-*N*-[1-(7,8-difluoro-1-methoxy-4-isoquinolyl)ethyl]-*N*,2-dimethyl-propane-2-sulfinamide (**XIVf**). LCMS: *m*/*z* found 357.2 [M+H]⁺, RT = 4.47 min (Method A). ¹H NMR (400 MHz, CDCl₃) δ 8.01 (d, 1H), 7.76 (m, 1H), 7.53 (m, 1H), 5.07-5.18 (m, 1H), 4.13 (s, 3H), 2.40 (s, 3H), 1.75 (d, 3H), 1.21 (s, 9H).

### 4-(1-Aminoethyl)-7,8-difluoro-2H-isoquinolin-1-one hydrochloride (VIIIdh)

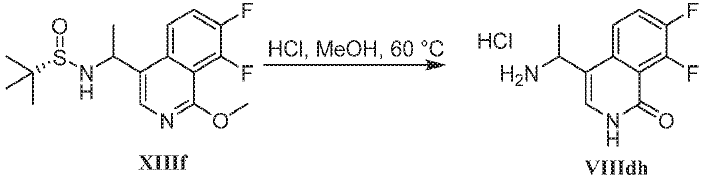

To a solution of 61 mg (0.18 mmol, 1.0 eq.) of diastereomerically pure (S)-*N*-[1-(7,8-difluoro-1-methoxy-4-isoquinolyl)ethyl]-2-methyl-propane-2-sulfinamide (**XIIIf**) in 1 mL of methanol in a sealed tube was added 0.6 mL (1.8 mmol, 10.0 eq.) of a 3 M solution HCl in methanol. The vessel was sealed, and the mixture was heated at 60 °C for 16 h. The mixture was allowed to cool to room temperature and the volatiles were removed *in vacuo.* The residue was triturated with 2 × 8 ml of diethyl ether and the residue was dried under high vacuum to provide 49 mg of 4-(1-aminoethyl)-7,8-difluoro-2*H*-isoquinolin-1-one hydrochloride (0.18 mmol, 100%). ¹HNMR (400 MHz, CD₃OD) δ 7.80 (m, 1H), 7.70 (m, 1H), 7.36 (m, 1H), 4.89 (m, 1H), 1.70 (d, 3H).

### 1-(3-Chloro-4-fluorophenyl)-3-(1-(7,8-difluoro-1-oxo-1,2-dihydroisoquinolin-4-yl)ethyl)urea (Compound 500)

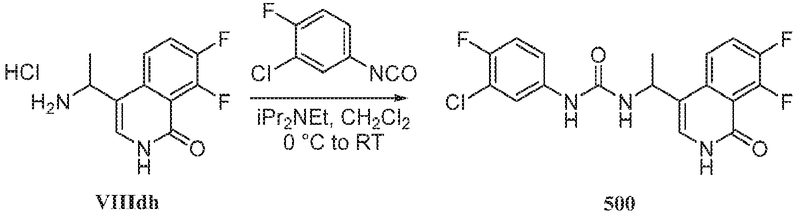

To a mixture of 29 mg (0.11 mmol, 1.0 eq.) of 4-(1-aminoethyl)-7,8-difluoroisoquinolin-1(2*H*)-onehydrochloride (**VIIIdh,** derived from (S)-2-methylpropane-2-sulfinamide and 1-(7,8-difluoro-1-methoxy-4-isoquinolyl)ethanone) in 1.8 mL of methylene chloride at 0 °C was added 39 uL (0.22 mmol, 2.6 eq.) of *N,N*-diisopropylethyl amine followed by a solution of 14 uL (0.11 mmol, 1.0 eq.) of 2-chloro-1-fluoro-4-isocyanato-benzene in 0.5 mL of methylene chloride. The mixture was allowed to warm to room temperature and stirred for 60 min. The volatiles were removed *in vacuo* and the residue was resuspended in 4 mL water. The resulting precipitate was collected by vacuum filtration, and washed with 5 mL of water followed by 5 mL of 1:1 *v:v* methylene chloride/hexanes. The material was triturated with 15 mL of hot ethyl acetate and allowed to stand overnight. The product was collected by vacuum filtration, and dried in a 50 °C vacuum oven to provide 13 mg (28 %) of 1-(3-chloro-4-fluorophenyl)-3-(1-(7,8-difluoro-1-oxo-1,2-dihydroisoquinolin-4-yl)ethyl)urea (**Compound 500**) as a single enantiomer. LCMS: *m*/*z* found 396.2/398.2 [M+H]+, RT = 3.89 min (Method A); ¹H NMR (400 MHz, DMSO-*d*₆) δ 11.41 (d, 1H), 8.56 (s, 1H), 7.83-7.95 (m, 1H), 7.77 (m, 1H), 7.56-7.65 (m, 1H), 7.27 (t, 1H), 7.20 (m, 1H), 7.10 (d, 1H), 6.63 (d, 1H), 5.11 (m, 1H), 1.44 (d, 3H).

### 3-(3-Chloro-4-fluorophenyl)-1-(1-(7,8-difluoro-1-oxo-1,2-dihydroisoquinolin-4-yl)ethyl)-1-(methyl-d₃)urea (Compound 473)

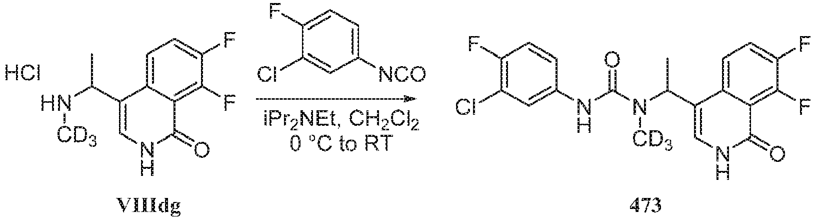

3-(3-Chloro-4-fluorophenyl)-1-(1-(7,8-difluoro-1-oxo-1,2-dihydroisoquinolin-4-yl)ethyl)-1-(methyl-d₃)urea (**Compound 473**) was synthesized in an analogous manner as described above from 7,8-difluoro-4-(1-((methyl-d₃)amino)ethyl)isoquinolin-1(2*H*)-one (**VIIIdg**, derived from (S)-*N*-(1-(7,8-difluoro-1-methoxyisoquinolin-4-yl)ethyl)-2-methyl-*N-*(methyl-d₃)propane-2-sulfinamide) and 2-chloro-1-fluoro-4-isocyanato-benzene. LCMS: *m*/*z* found 413/415 [M+H]⁺, RT = 2.91 min (Method B); ¹H NMR (400 MHz, DMSO-*d*₆) δ 11.51 (s, 1H), 8.46 (s, 1H), 7.82-7.95 (m, 2H), 7.44-7.55 (m, 2H), 7.31 (t, 1H), 7.15 (s, 1H), 5.77 (q, 1H), 1.41 (d, 3H).

### 3-(3-Chlorophenyl)-1-(1-(7,8-difluoro-1-oxo-1,2-dihydroisoquinolin-4-yl)ethyl)-1-(methyl-d3)urea (Compound 474)

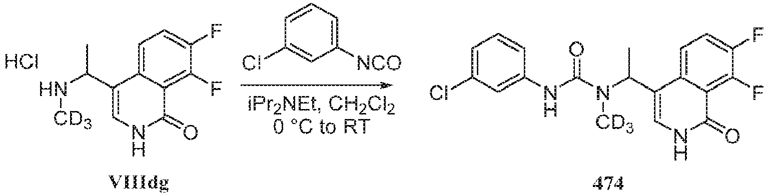

3-(3-Chlorophenyl)-1-(1-(7,8-difluoro-1-oxo-1,2-dihydroisoquinolin-4-yl)ethyl)-1-(methyl-d3)urea (**Compound 474**) was synthesized in an analogous manner as described above from 7,8-difluoro-4-(1-((methyl-d₃)amino)ethyl)isoquinolin-1(2*H*)-one (**VIIIdg**, derived from (S)-*N*-(1-(7,8-difluoro-1-methoxyisoquinolin-4-yl)ethyl)-2-methyl-*N*-(methyl-d₃)propane-2-sulfinamide) and 1-chloro-3-isocyanatobenzene. LCMS: *m*/*z* found 395/397 [M+H]⁺, RT = 2.88 min (Method B); ¹H NMR (400 MHz, DMSO-*d*₆) δ 11.52 (s, 1H), 8.44 (s, 1H), 7.83-7.95 (m, 1H), 7.76 (t, 1H), 7.44-7.56 (m, 2H), 7.27 (t, 1H), 7.15 (s, 1H), 7.00 (m, 1H), 5.78 (q, 1H), 1.41 (d, 3H).

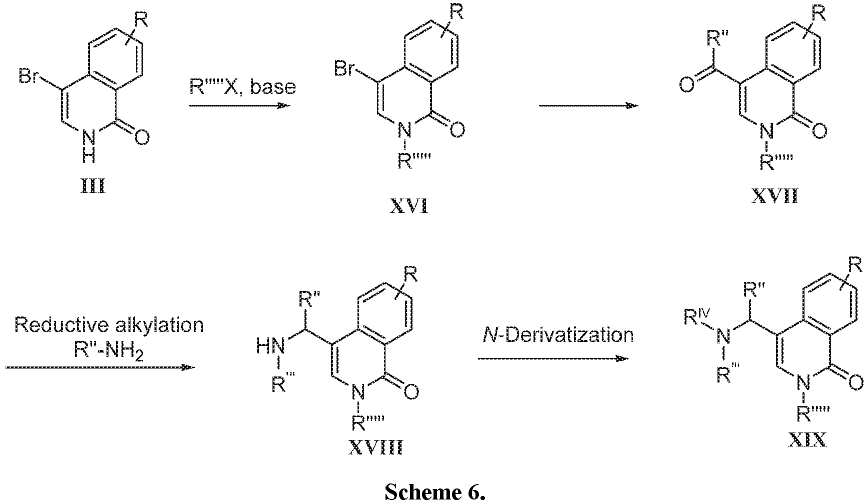

### 2-Methylisoquinolin-1(2H)-one

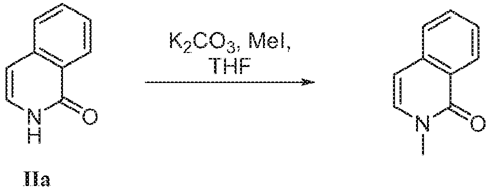

To a solution 5.0 g (34.5 mmol, 1.0 eq.) of isoquinolin-1(2*H*)-one (**IIa**) in 50 mL of THF at 0 °C was added 14.3 g (103.4 mmol, 3.0 eq.) of potassium carbonate followed by 7.0 g (51.7 mmol, 1.5 eq.) of methyl iodide. The mixture was allowed to warm to room temperature and stirred for 24 h. The reaction mixture was diluted with 100 mL of cold water and extracted with 3 x 50 mL of ethyl acetate. The combined organic extracts were washed with 50 mL of brine, dried (Na₂SO₄), filtered and the solvent was removed *in vacuo.* The residue was purified by flash chromatography (SiO₂, eluting with 10% ethyl acetate/petroleum ether) to provide 3.0 g (18.9 mmol, 55%) of 2-methylisoquinolin-1(2*H*)-one. LCMS: *m*/*z* found 160.0 [M+H]⁺; ¹H NMR (400 MHz CDCl₃) δ 8.42 (s, 1H), 7.59-7.64 (dt, 1H), 7.45-7.51 (m, 2H), 7.05-7.07 (d, 1H), 6.46-6.49 (d, 1H), 3.60 (s, 3H).

### 2-Methyl-1-oxo-1,2-dihydroisoquinoline-4-carbaldehyde (XVIIc)

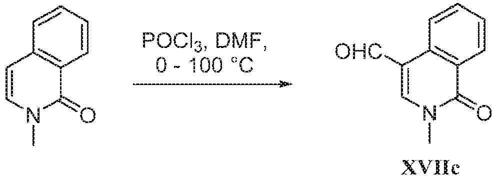

To 5 mL of DMF at 0 °C was slowly added 1.3 mL (13.8 mmol, 1.1 eq.) of phosphorus oxychloride and the mixture was stirred for 30 min. This solution was subsequently added to a solution of 2.0 g (12.6 mmol, 1.0 eq.) of 2-methylisoquinolin-1(2*H*)-one in 15 mL of DMF at 0 °C over approximately 15 min. The mixture was allowed to warm to room temperature and then heated at 100 °C for 5 h. The mixture was then allowed to cool and was poured on to 200 mL of ice-cooled water and extracted with 2 x 100 mL of ethyl acetate. The combined organic extracts were washed with 100 mL of brine, dried (Na₂SO₄), filtered and the solvent was removed *in vacuo.* The residue was purified by flash chromatography (SiO₂, eluting with 20% ethyl acetate/petroleum ether) to provide 1.5 g (8.0 mmol, 63%) of 2-methyl-1-oxo-1,2-dihydroisoquinoline-4-carbaldehyde (**XVIIc**). LCMS: *m*/*z* found 188.0 [M+H]⁺; ¹H NMR (400 MHz, DMSO-d₆) δ 9.80 (s, 1H), 9.00 (d, 1H), 8.44 (dd, 1H), 7.77 (t, 1H), 7.73 (s, 1H), 7.57-7.61 (m, 1H), 3.73 (s, 3H).

### 4-((Ethylamino)methyl)-2-methylisoquinolin-1(2H)-one (XVIIIj)

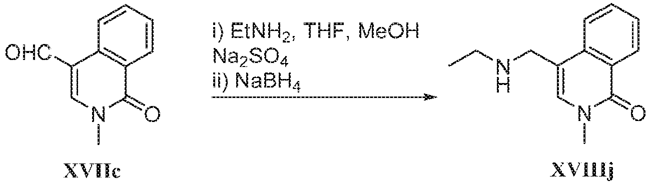

To a solution 1.5 g (8.0 mmol, 1.0 eq.) of 2-methyl-1-oxo-1,2-dihydroisoquinoline-4-carbaldehyde (**XVIIc**) in 7.5 mL of methanol was added 1.5 g of Na₂SO₄ and 40 mL (80.0 mmol, 10.0 eq.) of a 2 M solution of ethyl amine in THF. The mixture was stirred at room temperature for 16 h and 0.48 g (12.65 mmol, 1.5 eq.) of sodium borohydride was added. The mixture was stirred for a further 16 h and the volatiles were removed *in vacuo.* The residue was resuspended in 100 mL of ice-cold water and extracted with 3 x 80 mL of ethyl acetate. The combined organic extracts were washed with 80 mL of brine, dried (Na₂SO₄), filtered and the solvent was removed *in vacuo* to provide 0.7 g of crude 4-((ethylamino)methyl)-2-methylisoquinolin-1(2*H*)-one (**XVIIIj**). LCMS: *m*/*z* found 217.2 [M+H]⁺;

### 3-(3-Chloro-4-fluorophenyl)-1-ethyl-1-((2-methyl-1-oxo-1,2-dihydroisoquinolin-4-yl)methyl)urea (Compound 18)

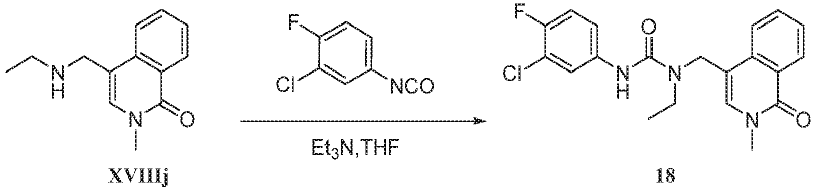

To a solution 0.5 g of 4-((ethylamino)methyl)-2-methylisoquinolin-1(2*H*)-one (**XVIIIj**) in 5 mL of THF at 0 °C was added 0.65 mL (4.62 mmol, 2.0 eq.) of triethylamine followed by 0.4 g (2.31 mmol, 1.0 eq.) of 4-fluoro-3-chloro-phenylisocyanate. The mixture was allowed to warm to room temperature and stirred for 16 h. The mixture was then diluted with 50 mL of ethyl acetate and washed with 50 mL of brine, dried (Na₂SO₄), filtered and the solvent was removed *in vacuo.* The residue was purified by reverse-phase chromatography (C18, Eluting with a linear gradient of 1-100% 0.1 M formic acid in water/acetonitrile) to provide 0.18 g (0.46 mmol) of 3-(3-chloro-4-fluorophenyl)-1-ethyl-1-((2-methyl-1-oxo-1,2-dihydroisoquinolin-4-yl)methyl)urea (**Compound 18**). LCMS: *m*/*z* found 388.2/390.2 [M+H]⁺, RT = 4.18 min (MethodA); ¹H NMR (400 MHz, DMSO-d₆) δ 8.54 (s, 1H), 8.27 (d, 1H), 7.81-7.84 (m, 2H), 7.73 (t, 1H), 7.48-7.54 (m, 3H), 7.30 (t, 1H), 4.62 (s, 2H), 3.38 (s, 3H), 3.28 (q, 2H), 1.02 (t, 3H).

### 4-Bromo-2-methylisoquinolin-1(2H)-one (XVIa)

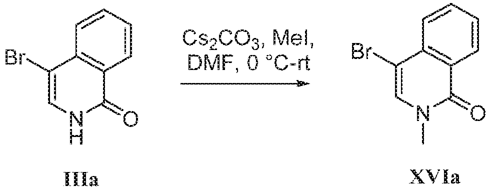

To a stirred solution of 0.5 g (2.23 mmol, 1.0 eq.) of 4-bromoisoquinolin-1(2*H*)-one (**IIIa**) in 5 mL of anhydrous DMF was added 1.8 g (5.54 mmol, 2.5 eq.) of cesium carbonate. The mixture was cooled to 0 °C and 0.26 mL (3.34 mmol, 1.5 eq.) of iodomethane was added. The mixture was allowed to warm to room temperature and stirred for 16 h. The mixture was then diluted with 10 mL of ice-cold water and the precipitated solids were collected by filtration, washed with 5 mL of water, and dried under vacuum to provide 0.5 g (2.10 mmol, 94%) of 4-bromo-2-methylisoquinolin-1(2*H*)-one (**XVIa**). LCMS: *m*/*z* found 238.0/240.0 [M+H]⁺; ¹H NMR (400 MHz, CDCl₃): δ 8.45 (d, 1H), 7.82 (d, 1H), 7.72-7.77 (m, 1H), 7.54-7.58 (m, 1H), 7.37 (s, 1H), 3.61 (s, 3H). The above detailed reaction was performed in multiple batches with consistent results.

The above detailed procedure can be employed utilizing alternate alkyl halide to provide N-substituted analogs on **XVIa.**

### 4-Acetyl-2-methylisoquinolin-1(2H)-one (XVIIa)

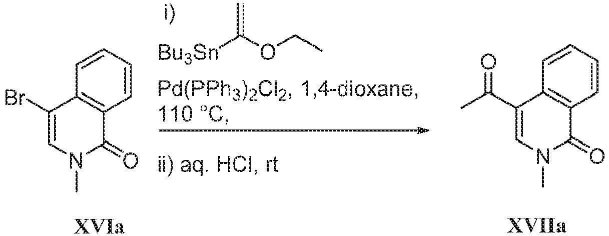

To a stirred solution of 1.0 g (4.20 mmol, 1.0 eq.) of 4-bromo-2-methylisoquinolin-1(2*H*)-one (**XVIa**) in 10 mL of anhydrous 1,4-dioxane was added 3.6 mL (10.50 mmol, 2.5 eq.) of tributyl(1-ethoxyvinyl)stannane. The mixture was purged with nitrogen gas for 10 min and 0.30 g (0.42 mmol, 0.1 eq.) of Pd(PPh₃)₂Cl₂ was added. The mixture was then heated at 110 °C for 16 h. The mixture was allowed to cool to room temperature, diluted with 10 mL of 1 M aqueous HCl solution and stirred at room temperature for an additional 2 h. The mixture was then basified to pH~9 with saturated sodium bicarbonate solution and filtered through CELITE^{®}. The filtrate was extracted with 3 x 50 mL of ethyl acetate and the combined organic extracts were washed with 50 mL of brine, dried (Na₂SO₄), filtered and the solvent was removed *in vacuo.* The residue was purified by column chromatography (SiO₂, eluting with a linear gradient of 0-50% ethyl acetate in petroleum ether) to provide 0.5 g (2.48 mmol, 59%) of 4-acetyl-2-methylisoquinolin-1(2H)-one (**XVIIa**). LCMS: *m*/*z* found 202.0 [M+H]⁺; ¹H NMR (300 MHz, DMSO-d₆): δ 8.90 (d, 1H), 8.57 (s, 1H), 8.26-8.29 (m, 1H), 7.74-7.81 (m, 1H), 7.53-7.59 (m, 1H), 3.63 (s, 3H), 2.55 (s, 3H).

### 2-Methyl-4-(1-(methylamino)ethyl)isoquinolin-1(2H)-one (XVIIIa)

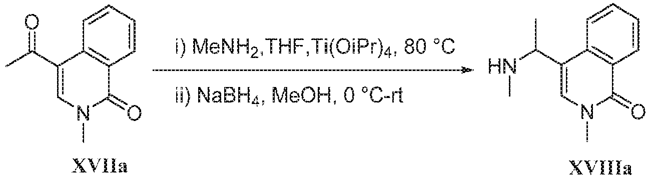

To a solution of 0.6 g (3.0 mmol, 1.0 eq.) of 4-acetyl-2-methylisoquinolin-1(2*H*)-one (**XVIIa**) in 6 mL of anhydrous THF in a pressure vessel under a nitrogen atmosphere was added 7.4 mL (15.0 mmol, 5.0 eq.) of a 2 M solution of methylamine in THF followed by 6 mL of titanium (IV) isopropoxide. The vessel was sealed, and the mixture was heated at 80 °C and for 5 h. The mixture was allowed to cool to room temperature and then further cooled to 0 °C. The mixture was then diluted with 2 mL of methanol and 0.22 g (6.1 mmol, 2.0 eq.) of sodium borohydride was added. The mixture was allowed to warm to room temperature and stirred for 2 h. The reaction was quenched by the addition of 20 mL of water and filtered through CELITE^{®}. The pad was washed with 5 mL of ethyl acetate and the biphasic mixture was extracted with 2 x 40 mL of ethyl acetate. The combined organic extracts were washed with 40 mL of brine, dried (Na₂SO₄), filtered and the solvent was removed *in vacuo* to provide 0.4 g of crude 2-methyl-4-(1-(methylamino)ethyl)isoquinolin-1(2*H*)-one (**XVIIIa**), which was carried to the next step without further purification. LCMS: *m*/*z* found 217.1 [M+H]⁺.

### 3-(3-Chloro-4-fluorophenyl)-1-methyl-1-(1-(2-methyl-1-oxo-1,2-dihydroisoquinolin-4-yl)ethyl)urea (Compounds 181 & 182)

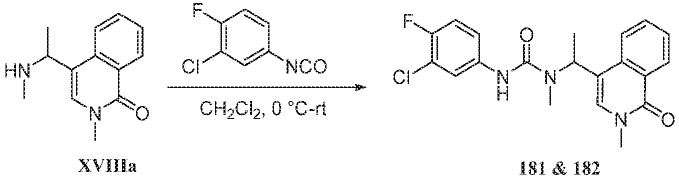

To a stirred solution of 0.4 g of crude 2-methyl-4-(1-(methylamino)ethyl)isoquinolin-1(2H)-one (**XVIIIa**) in 4 mL of methylene chloride at 0 °C under a nitrogen atmosphere was added 0.22 mL (1.85 mmol, 1.0 eq.) of 2-chloro-1-fluoro-4-isocyanatobenzene and the mixture was stirred at room temperature for 1 h. The solvent was removed *in vacuo* and the mixture was diluted with 30 mL of water and stirred for 20 min. The resulting solid was collected by vacuum filtration, washed with 2 x 10 mL of *n*-pentane and dried under high vacuum to provide 0.31 g (0.80 mmol, 32% from **XVIIa**) of racemic 3-(3-chloro-4-fluorophenyl)-1-methyl-1-(1-(2-methyl-1-oxo-1,2-dihydroisoquinolin-4-yl)ethyl)urea. LCMS: *m*/*z* found 388.3/390.3 [M+H]⁺. The enantiomers were subsequently separated by SFC (Waters SFC-80), Column: Chiralpak IC (250 x 30 mm) 5µ, 70% CO₂:MeOH, Flow rate 70 g/min to provide 97 mg and 96 mg of the resolved enantiomers.

3-(3-Chloro-4-fluorophenyl)-1-methyl-1-(1-(2-methyl-1-oxo-1,2-dihydroisoquinolin-4-yl)ethyl)urea: Enantiomer I (**Compound 181**). LCMS: *m*/*z* found 388.2/390.2 [M+H]⁺, RT = 7.14 min (Method A); ¹H NMR (400 MHz, DMSO-d₆): δ 8.46 (br s, 1H), 8.27 (d, 1H), 7.85-7.87 (m, 1H), 7.69-7.75 (m, 2H), 7.48-7.53 (m, 3H), 7.31 (t, 1H), 5.85-5.91 (m, 1H), 3.58 (s, 3H), 2.60 (s, 3H), 1.46 (d, 3H); Chiral analytical SFC: RT = 5.49 min, Column: CHIRALPAK IC-3 (4.6 x 150 mm) 3 µ, 75% CO₂:MeOH, Flow = 3.0 g/min.

3-(3-Chloro-4-fluorophenyl)-1-methyl-1-(1-(2-methyl-1-oxo-1,2-dihydroisoquinolin-4-yl)ethyl)urea: Enantiomer II (**Compound 182**). LCMS: *m*/*z* found 388.2/390.2 [M+H]⁺, RT = 7.14 min (Method A); ¹H NMR (400 MHz, DMSO-d₆): δ 8.46 (br s, 1H), 8.27 (d, 1H), 7.85-7.87 (m, 1H), 7.69-7.75 (m, 2H), 7.48-7.53 (m, 3H), 7.31 (t, 1H), 5.85-5.91 (m, 1H), 3.58 (s, 3H), 2.60 (s, 3H), 1.46 (d, 3H); Chiral analytical SFC, RT = 7.89 min; Column: CHIRALPAK IC-3 (4.6 x 150 mm) 3 µ, 75% CO₂:MeOH, Flow = 3.0 g/min.

### 3-(3-Chloro-4-fluorophenyl)-1-(1-(2-ethyl-1-oxo-1,2-dihydroisoquinolin-4-yl)ethyl)-1-methylurea (Compounds 183 & 184)

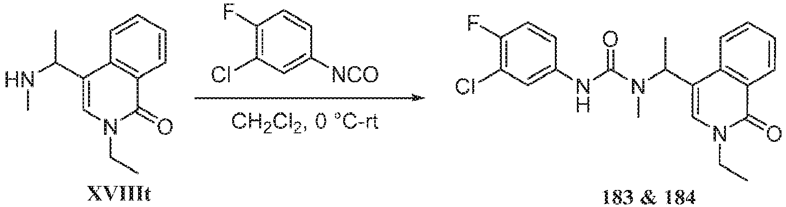

3-(3-Chloro-4-fluorophenyl)-1-(1-(2-ethyl-1-oxo-1,2-dihydroisoquinolin-4-yl)ethyl)-1-methylurea was synthesized in an analogous manner as described above from 2-ethyl-4-(1-(methylamino)ethyl)isoquinolin-1(2*H*)-one (**XVIIIt**) and 2-chloro-1-fluoro-4-isocyanatobenzene. The enantiomers were subsequently separated by SFC (Waters SFC-200) Column: Chiralpak IC (250 x 30 mm) 5 µ, 70% CO₂:MeOH, Flow rate 60 g/min.

3-(3-Chloro-4-fluorophenyl)-1-(1-(2-ethyl-1-oxo-1,2-dihydroisoquinolin-4-yl)ethyl)-1-methylurea: Enantiomer I (**Compound 183**). LCMS: *m*/*z* found 402.3/404.3 [M+H]⁺, RT = 7.28 min (Method A); ¹H NMR (400 MHz, DMSO-d₆): δ 8.46 (bs, 1H), 8.28 (d, 1H), 7.84-7.87 (m, 1H), 7.69-7.75 (m, 2H), 7.48-7.53 (m, 3H), 7.31 (t, 1H), 5.86-5.91 (m, 1H), 4.00-4.13 (m, 2H), 2.60 (s, 3H), 1.47 (d, 3H), 1.29 (t, 3H); Chiral analytical SFC: RT = 6.54 min, Column: CHIRALPAK IC-3 (4.6 x 150 mm) 3 µ, 80% CO₂:MeOH, Flow = 3.0 g/min.

3-(3-Chloro-4-fluorophenyl)-1-(1-(2-ethyl-1-oxo-1,2-dihydroisoquinolin-4-yl)ethyl)-1-methylurea: Enantiomer II (**Compound 184**). LCMS: *m*/*z* found 402.3/404.3 [M+H]⁺, RT = 7.28 min (Method A); ¹H NMR (400 MHz, DMSO-d₆): δ 8.46 (bs, 1H), 8.28 (d, 1H), 7.84-7.87 (m, 1H), 7.69-7.75 (m, 2H), 7.48-7.53 (m, 3H), 7.31 (t, 1H), 5.86-5.91 (m, 1H), 4.00-4.13 (m, 2H), 2.60 (s, 3H), 1.47 (d, 3H), 1.29 (t, 3H); Chiral analytical SFC: RT = 7.55 min, Column: CHIRALPAK IC-3 (4.6 x 150 mm) 3 µ, 80% CO₂:MeOH, Flow = 3.0 g/min

### 3-(3-Chloro-4-fluorophenyl)-1-methyl-1-(1-(1-oxo-2-propyl-1,2-dihydroisoquinolin-4-yl)ethyl)urea (Compounds 185 & 186)

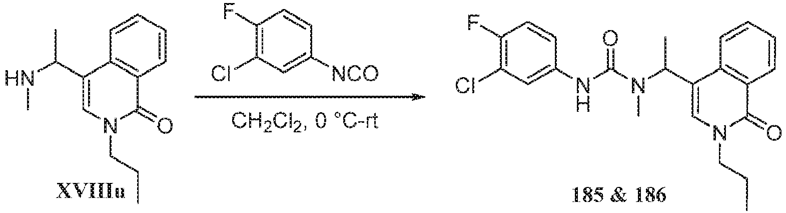

3-(3-Chloro-4-fluorophenyl)-1-methyl-1-(1-(1-oxo-2-propyl-1,2-dihydroisoquinolin-4-yl)ethyl)urea was synthesized in an analogous manner as described above from 4-(1-(methylamino)ethyl)-2-propylisoquinolin-1(2*H*)-one (**XVIIIu**) and 2-chloro-1-fluoro-4-isocyanatobenzene. The enantiomers were subsequently separated by SFC (Waters SFC-200) Column: Chiralpak IC (250 x 30 mm) 5 µ, 65% CO₂:MeOH, Flow rate 90 g/min.

3-(3-Chloro-4-fluorophenyl)-1-methyl-1-(1-(1-oxo-2-propyl-1,2-dihydroisoquinolin-4-yl)ethyl)urea: Enantiomer I **(Compound 185**). LCMS: *m*/*z* found 416.3/418.3 [M+H]⁺, RT = 7.42 min (Method A); ¹H NMR (400 MHz, DMSO-d₆): δ 8.47 (bs, 1H), 8.28 (d, 1H), 7.84-7.87 (m, 1H), 7.69-7.75 (m, 2H), 7.47-7.54 (m, 3H), 7.32 (t, 1H), 5.86-5.92 (m, 1H), 3.95-4.04 (m, 2H), 2.59 (s, 3H), 1.71-1.77 (m, 2H), 1.47 (d, 3H), 0.92 (t, 3H); Chiral analytical SFC: RT = 4.44 min; Column: CHIRALPAK IC-3 (4.6 x 150 mm) 5 µ, 65% CO₂:MeOH, Flow = 3.0 g/min.

3-(3-Chloro-4-fluorophenyl)-1-methyl-1-(1-(1-oxo-2-propyl-1,2-dihydroisoquinolin-4-yl)ethyl)urea: Enantiomer II (**Compound 186**). LCMS: *m*/*z* found 416.3/418.3 [M+H]⁺, RT = 7.42 min (Method A); ¹H NMR (400 MHz, DMSO-d₆): δ 8.47 (bs, 1H), 8.28 (d, 1H), 7.84-7.87 (m, 1H), 7.69-7.75 (m, 2H), 7.47-7.54 (m, 3H), 7.32 (t, 1H), 5.86-5.92 (m, 1H), 3.95-4.04 (m, 2H), 2.59 (s, 3H), 1.71-1.77 (m, 2H), 1.47 (d, 3H), 0.92 (t, 3H); Chiral analytical SFC: RT = 5.99 min; Column: CHIRALPAK IC-3 (4.6 x 150 mm) 5 µ, 65% CO₂:MeOH, Flow = 3.0 g/min.

### 3-(3-Chloro-4-fluorophenyl)-1-(1-(2-isopropyl-1-oxo-1,2-dihydroisoquinolin-4-yl)ethyl)-1-methylurea (Compounds 187 & 188)

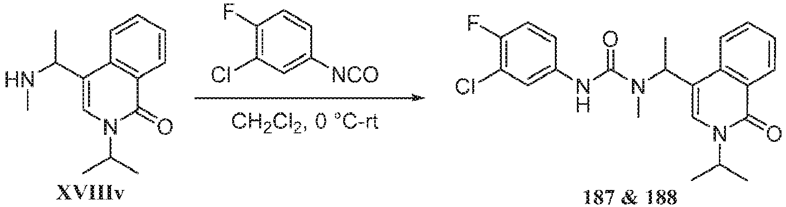

3-(3-Chloro-4-fluorophenyl)-1-(1-(2-isopropyl-1-oxo-1,2-dihydroisoquinolin-4-yl)ethyl)-1-methylurea urea was synthesized in an analogous manner as described above from 2-isopropyl-4-(1-(methylamino)ethyl)isoquinolin-1(2*H*)-one (**XVIIIv**) and 2-chloro-1-fluoro-4-isocyanatobenzene. The enantiomers were subsequently separated by SFC (Waters SFC-200) Column: Chiralcel OD-H (250 x 30 mm) 5 µ, 90% CO₂:MeOH, Flow rate 55 g/min.

3-(3-Chloro-4-fluorophenyl)-1-(1-(2-isopropyl-1-oxo-1,2-dihydroisoquinolin-4-yl)ethyl)-1-methylurea: Enantiomer I (**Compound 187**). LCMS: *m*/*z* found 416.3/418.3 [M+H]⁺, RT = 7.43 min (Method A); ¹H NMR (400 MHz, DMSO-d₆): δ 8.47 (bs, 1H), 8.29 (d, 1H), 7.84-7.87 (m, 1H), 7.70-7.75 (m, 2H), 7.48-7.53 (m, 2H), 7.36 (s, 1H), 7.32 (t, 1H), 5.88-5.93 (m, 1H), 5.17-5.24 (m, 1H), 2.59 (s, 3H), 1.51 (d, 3H), 1.38-1.44 (m, 6H); Chiral analytical SFC: RT = 3.44 min, Column: Chiralcel OD-3 (4.6 x 150 mm) 3 µ, 80% CO₂:MeOH, Flow = 3.0 g/min.

3-(3-Chloro-4-fluorophenyl)-1-(1-(2-isopropyl-1-oxo-1,2-dihydroisoquinolin-4-yl)ethyl)-1-methylurea: Enantiomer II (**Compound 188**). LCMS: *m*/*z* found 416.3/418.3 [M+H]⁺, RT = 7.43 min (Method A); ¹H NMR (400 MHz, DMSO-d₆): δ 8.47 (bs, 1H), 8.29 (d, 1H), 7.84-7.87 (m, 1H), 7.70-7.75 (m, 2H), 7.48-7.53 (m, 2H), 7.36 (s, 1H), 7.32 (t, 1H), 5.88-5.93 (m, 1H), 5.17-5.24 (m, 1H), 2.59 (s, 3H), 1.51 (d, 3H), 1.38-1.44 (m, 6H); Chiral analytical SFC: RT = 4.21 min, Column: Chiralcel OD-3 (4.6 x 150 mm) 3 µ, 80% CO₂:MeOH, Flow = 3.0 g/min.

### 3-(3-Chloro-4-fluorophenyl)-1-(1-(2-cyclopropyl-1-oxo-1,2-dihydroisoquinolin-4-yl)ethyl)-1-methylurea (Compounds 189 & 190)

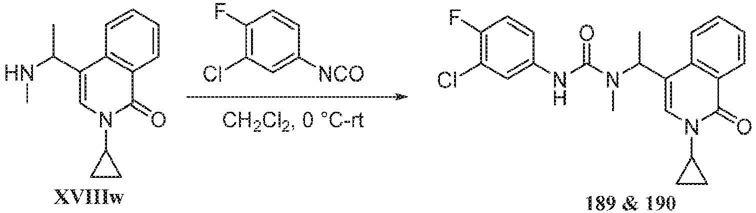

3-(3-Chloro-4-fluorophenyl)-1-(1-(2-cyclopropyl-1-oxo-1,2-dihydroisoquinolin-4-yl)ethyl)-1-methylurea was synthesized in an analogous manner as described above from 2-cyclopropyl-4-(1-(methylamino)ethyl)isoquinolin-1(2*H*)-one (**XVIIIw**) and 2-chloro-1-fluoro-4-isocyanatobenzene. The enantiomers were subsequently separated by SFC (Waters SFC-200) Column: Chiralpak IG (250 x 30 mm) 5 µ, 55% CO₂:MeOH, Flow rate 90 g/min.

3-(3-Chloro-4-fluorophenyl)-1-(1-(2-cyclopropyl-1-oxo-1,2-dihydroisoquinolin-4-yl)ethyl)-1-methylurea: Enantiomer I (**Compound 189**). LCMS: *m*/*z* found 414.3/416.3 [M+H]⁺, RT = 7.25 min (Method A); ¹H NMR (400 MHz, DMSO-d₆): δ 8.46 (br s, 1H), 8.27 (d, 1H), 7.84-7.87 (m, 1H), 7.67-7.75 (m, 2H), 7.48-7.54 (m, 2H), 7.31 (t, 1H), 7.23 (s, 1H), 5.82-5.88 (m, 1H), 3.33-3.39 (m, 1H), 2.59 (s, 3H), 1.47 (d, 3H), 1.03-1.08 (m, 2H), 0.92-0.99 (m, 2H); Chiral analytical SFC: RT = 4.21 min, Column: Chiralpak IG (4.6 x 150 mm) 3 µ, 55% CO₂:MeOH, Flow = 3.0 g/min.

3-(3-Chloro-4-fluorophenyl)-1-(1-(2-cyclopropyl-1-oxo-1,2-dihydroisoquinolin-4-yl)ethyl)-1-methylurea: Enantiomer II (**Compound 190**). LCMS: *m*/*z* found 414.3/416.3 [M+H]⁺, RT = 7.25 min (Method A); ¹H NMR (400 MHz, DMSO-d₆): δ 8.46 (br s, 1H), 8.27 (d, 1H), 7.84-7.87 (m, 1H), 7.67-7.75 (m, 2H), 7.48-7.54 (m, 2H), 7.31 (t, 1H), 7.23 (s, 1H), 5.82-5.88 (m, 1H), 3.33-3.39 (m, 1H), 2.59 (s, 3H), 1.47 (d, 3H), 1.03-1.08 (m, 2H), 0.92-0.99 (m, 2H); Chiral analytical SFC: RT = 5.91 min, Column: Chiralpak IG (4.6 x 150 mm) 3 µ, 55% CO₂:MeOH, Flow = 3.0 g/min.

### 3-(3-Chloro-4-fluorophenyl)-1-(1-(2-(2-methoayethyl)-1-oxo-1,2-dihydroisoquinolin-4-yl)ethyl)-1-methylurea (Compound 191)

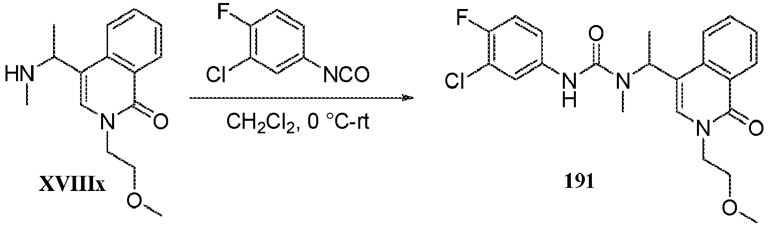

3-(3-Chloro-4-fluorophenyl)-1-(1-(2-(2-methoxyethyl)-1-oxo-1,2-dihydroisoquinolin-4-yl)ethyl)-1-methylurea was synthesized in an analogous manner as described above from 2-(2-methoxyethyl)-4-(1-(methylamino)ethyl)isoquinolin-1(2*H*)-one (**XVIIIx**) and 2-chloro-1-fluoro-4-isocyanatobenzene. The enantiomers were subsequently separated by SFC (Waters SFC-200) Column: Chiralpak IC (250 x 30 mm) 5 µ, 80% CO₂:MeOH, Flow rate 100 g/min.

3-(3-Chloro-4-fluorophenyl)-1-(1-(2-(2-methoxyethyl)-1-oxo-1,2-dihydroisoquinolin-4-yl)ethyl)-1-methylurea: Enantiomer I. LCMS: *m*/*z* found 432.3/434.3 [M+H]⁺, RT = 7.17 min (Method A); ¹H NMR (400 MHz, DMSO-d₆): δ 8.46 (br s, 1H), 8.28 (d, 1H), 7.87-7.84 (m, 1H), 7.76-7.69 (m, 2H), 7.53-7.45 (m, 3H), 7.32 (t, 1H), 5.92-5.87 (m, 1H), 4.27-4.14 (m, 2H), 3.65 (t, 2H), 3.27 (s, 3H), 2.59 (s, 3H), 1.45 (d, 3H); Chiral analytical SFC: RT = 5.39 min, Column: Chiralpak IC (4.6 x 150 mm) 3 µ, 70% CO₂:MeOH, Flow = 3.0 g/min.

3-(3-Chloro-4-fluorophenyl)-1-(1-(2-(2-methoxyethyl)-1-oxo-1,2-dihydroisoquinolin-4-yl)ethyl)-1-methylurea: Enantiomer II (**Compound 191**). LCMS: *m*/*z* found 432.3/434.3 [M+H]⁺, RT = 7.15 min (Method A); ¹H NMR (400 MHz, DMSO-d₆): δ 8.46 (br s, 1H), 8.28 (d, 1H), 7.87-7.84 (m, 1H), 7.76-7.69 (m, 2H), 7.53-7.45 (m, 3H), 7.32 (t, 1H), 5.92-5.87 (m, 1H), 4.27-4.14 (m, 2H), 3.65 (t, 2H), 3.27 (s, 3H), 2.59 (s, 3H), 1.45 (d, 3H); Chiral analytical SFC: RT = 7.14 min, Column: Chiralpak IC (4.6 x 150 mm) 3 µ, 70% CO₂:MeOH, Flow = 3.0 g/min.

### 3-(3-Chloro-4-fluorophenyl)-1-(1-(2-(3-methoxypropyl)-1-oxo-1,2-dihydroisoquinolin-4-yl)ethyl)-1-methylurea (Compound 200)

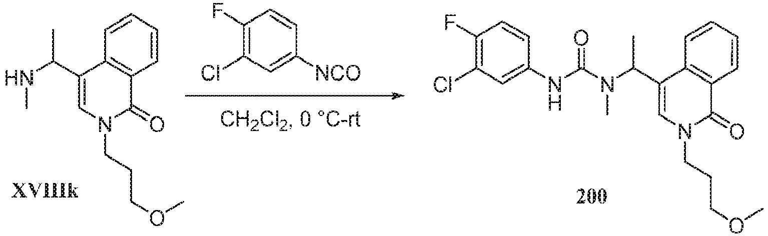

3-(3-chloro-4-fluorophenyl)-1-(1-(2-(3-methoxypropyl)-1-oxo-1,2-dihydroisoquinolin-4-yl)ethyl)-1-methylurea was synthesized in an analogous manner as described above from 2-(3-methoxypropyl)-4-(1-(methylamino)ethyl)isoquinolin-1(2*H*)-one (**XVIIIk**, derived from *N*-alkylation of **IIIa** with 1-bromo-3-methoxypropane) and 2-chloro-1-fluoro-4-isocyanatobenzene. The enantiomers were subsequently separated by SFC (Waters SFC-200) Column: Chiralpak IC (250 x 30 mm) 5 µ, 75% CO₂:MeOH, Flow rate 90 g/min.

3-(3-Chloro-4-fluorophenyl)-1-(1-(2-(3-methoxypropyl)-1-oxo-1,2-dihydroisoquinolin-4-yl)ethyl)-1-methylurea: Enantiomer I. LCMS: *m*/*z* found 446.3/448.3 [M+H]⁺, RT = 7.50 min (Method A); ¹H NMR (400 MHz, DMSO-d₆): δ 8.47 (bs, 1H), 8.28 (d, 1H), 7.84-7.87 (m, 1H), 7.70-7.75 (m, 2H), 7.47-7.53 (m, 2H), 7.45 (s, 1H), 7.32 (t, 1H), 5.87-5.91 (m, 1H), 4.05-4.11 (m, 2H), 3.33-3.39 (m, 2H), 3.25 (s, 3H), 2.60 (s, 3H), 1.93-1.98 (m, 2H), 1.47 (d, 3H); Chiral analytical SFC: RT = 6.04 min, Column: Chiralpak IC-3 (4.6 × 150 mm) 3 µ, 80% CO₂:MeOH, Flow = 3.0 g/min.

3-(3-Chloro-4-fluorophenyl)-1-(1-(2-(3-methoxypropyl)-1-oxo-1,2-dihydroisoquinolin-4-yl)ethyl)-1-methylurea: Enantiomer II (**Compound 200**). LCMS: *m*/*z* found 446.3/448.3 [M+H]⁺, RT = 7.48 min (Method A); ¹H NMR (400 MHz, DMSO-d₆): δ 8.47 (bs, 1H), 8.28 (d, 1H), 7.84-7.87 (m, 1H), 7.70-7.75 (m, 2H), 7.47-7.53 (m, 2H), 7.45 (s, 1H), 7.32 (t, 1H), 5.87-5.91 (m, 1H), 4.05-4.11 (m, 2H), 3.33-3.39 (m, 2H), 3.25 (s, 3H), 2.60 (s, 3H), 1.93-1.98 (m, 2H), 1.47 (d, 3H); Chiral analytical SFC: RT = 7.07 min, Column: Chiralpak IC-3 (4.6 x 150 mm) 3 µ, 80% CO₂:MeOH, Flow = 3.0 g/min.

### 3-(3-chloro-4-fluorophenyl)-1-(1-(2-(2-hydroayethyl)-1-oxo-1,2-dihydroisoquinolin-4-yl)ethyl)-1-methylurea (Compound 205)

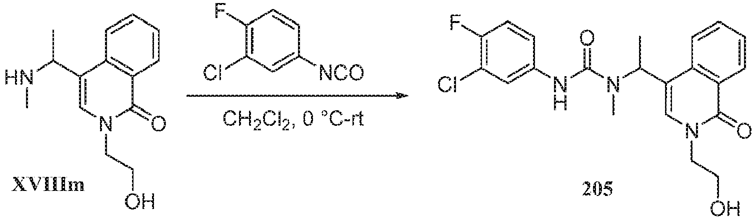

Racemic 3-(3-chloro-4-fluorophenyl)-1-(1-(2-(2-hydroxyethyl)-1-oxo-1,2-dihydroisoquinolin-4-yl)ethyl)-1-methylurea was synthesized in an analogous manner as described above from 2-(3-hydroxyethyl)-4-(1-(methylamino)ethyl)isoquinolin-1(2H)-one (**XVIIIm,** derived from *N*-alkylation of **IIIa** with 2-(2-bromopropoxyy)tetrahydro-2*H*-pyran) and 2-chloro-1-fluoro-4-isocyanatobenzene. The enantiomers were subsequently separated by SFC, Column: Chiralpak AD-H (250 × 30 mm) 5 µ, 75% CO₂:MeOH, Flow rate 90 g/min.

3-(3-Chloro-4-fluorophenyl)-1-(1-(2-(2-hydroxyethyl)-1-oxo-1,2-dihydroisoquinolin-4-yl)ethyl)-1-methylurea: Enantiomer I. LCMS: *m*/*z* found 418.2/420.2 [M+H]⁺, RT = 7.06 min (Method A); ¹H NMR (400 MHz, DMSO-d₆): δ 8.45 (bs, 1H), 8.28 (d, 1H), 7.84-7.87 (m, 1H), 7.68-7.75 (m, 2H), 7.47-7.53 (m, 2H), 7.45 (s, 1H), 7.31 (t, 1H), 5.86-5.90 (m, 1H), 4.88 (bs, 1H), 4.06-4.14 (m, 2H), 3.69-3.73 (m, 2H), 2.59 (s, 3H), 1.45 (d, 3H); Chiral analytical SFC: RT = 3.06 min, Column: Chiralpak IC-3 (4.6 × 150 mm) 3 µ, 80% CO₂:MeOH, Flow = 3.0 g/min.

3-(3-Chloro-4-fluorophenyl)-1-(1-(2-(2-hydroxyethyl)-1-oxo-1,2-dihydroisoquinolin-4-yl)ethyl)-1-methylurea: Enantiomer II (**Compound 205**). LCMS: *m*/*z* found 418.2/420.2 [M+H]⁺, RT = 7.06 min (Method A); ¹H NMR (400 MHz, DMSO-d₆): δ 8.45 (bs, 1H), 8.28 (d, 1H), 7.84-7.87 (m, 1H), 7.68-7.75 (m, 2H), 7.47-7.53 (m, 2H), 7.45 (s, 1H), 7.31 (t, 1H), 5.86-5.90 (m, 1H), 4.88 (bs, 1H), 4.06-4.14 (m, 2H), 3.69-3.73 (m, 2H), 2.59 (s, 3H), 1.45 (d, 3H); Chiral analytical SFC: RT = 3.99 min, Column: Chiralpak IC-3 (4.6 × 150 mm) 3 µ, 80% CO₂:MeOH, Flow = 3.0 g/min.

### 3-(3-Chloro-4-fluorophenyl)-1-(1-(2-(3-hydroxypropyl)-1-oxo-1,2-dihydroisoquinolin-4-yl)ethyl)-1-methylurea (Compound 206)

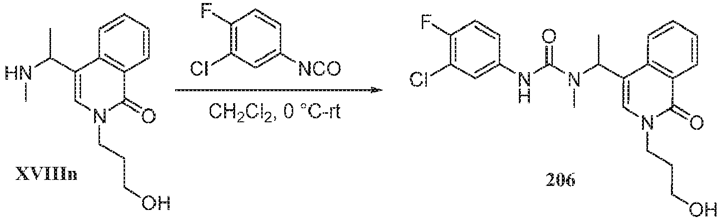

Racemic 3-(3-chloro-4-fluorophenyl)-1-(1-(2-(3-hydroxypropyl)-1-oxo-1,2-dihydroisoquinolin-4-yl)ethyl)-1-methylurea was synthesized in an analogous manner as described above from 2-(3-hydroxypropyl)-4-(1-(methylamino)ethyl)isoquinolm-1(2*H*)-one (**XVIIIn,** derived from *N*-alkylation of **IIIa** with 2-(2-bromopropoxy)tetrahydro-2*H*-pyran) and 2-chloro-1-fluoro-4-isocyanatobenzene. The enantiomers were subsequently separated by SFC, Column: Chiralpak IC (250 × 30 mm) 5 µ, 70% CO₂:MeOH, Flow rate 100 g/min.

3-(3-Chloro-4-fluorophenyl)-1-(1-(2-(3-hydroxypropyl)-1-oxo-1,2-dihydroisoquinolin-4-yl)ethyl)-1-methylurea: Enantiomer I. LCMS: *m*/*z* found 432.3/434.4 [M+H]⁺, RT = 7.12 min (Method A); ¹H NMR (400 MHz, DMSO-d₆): δ 8.46 (bs, 1H), 8.28 (d, 1H), 7.84-7.87 (m, 1H), 7.69-7.75 (m, 2H), 7.46-7.53 (m, 3H), 7.31 (t, 1H), 5.87-5.91 (m, 1H), 4.63 (bs, 1H), 4.06-4.11 (m, 2H), 3.45-3.48 (m, 2H), 2.59 (s, 3H), 1.85-1.90 (m, 2H), 1.46 (d, 3H); Chiral analytical SFC: RT = 2.73 min, Column: Chiralpak IC-3 (4.6 × 150 mm) 3 µ, 70% CO₂:MeOH, Flow = 3.0 g/min.

3-(3-Chloro-4-fluorophenyl)-1-(1-(2-(3-hydroxypropyl)-1-oxo-1,2-dihydroisoquinolin-4-yl)ethyl)-1-methylurea: Enantiomer II (**Compound 206**). LCMS: *m*/*z* found 432.3/434.4 [M+H]⁺, RT = 7.12 min (Method A); ¹H NMR (400 MHz, DMSO-d₆): δ 8.46 (bs, 1H), 8.28 (d, 1H), 7.84-7.87 (m, 1H), 7.69-7.75 (m, 2H), 7.46-7.53 (m, 3H), 7.31 (t, 1H), 5.87-5.91 (m, 1H), 4.63 (bs, 1H), 4.06-4.11 (m, 2H), 3.45-3.48 (m, 2H), 2.59 (s, 3H), 1.85-1.90 (m, 2H), 1.46 (d, 3H); Chiral analytical SFC: RT = 3.66 min, Column: Chiralpak IC-3 (4.6 × 150 mm) 3 µ, 70% CO₂:MeOH, Flow = 3.0 g/min.

### 1-(1-(2-((1H-1,2,4-Triazol-3-yl)methyl)-1-oxo-1,2-dihydroisoquinolin-4-yl)ethyl)-3-(3-chloro-4-fluorophenyl)-1-methylurea (Compounds 269 & 270)

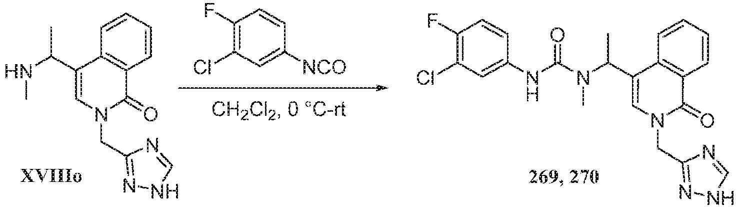

Racemic 1-(1-(2-((1*H*-1,2,4-triazol-3-yl)methyl)-1-oxo-1,2-dihydroisoquinolin-4-yl)ethyl)-3-(3-chloro-4-fluorophenyl)-1-methylurea was synthesized in an analogous manner as described above from 2-((1*H*-1,2,4-triazol-3-yl)methyl)-4-(1-(methylamino)ethyl)isoquinolin-1(2*H*)-one **XVIIIo,** derived from A-alkylation of **IIIa** with (1-trityl-1*H*-1,2,4-triazol-3-yl)methyl methanesulfonate) and 2-chloro-1-fluoro-4-isocyanatobenzene. The enantiomers were subsequently separated by SFC, Column: Lux Cellulose-02 (250 x 30 mm) 5 µ, 65% CO₂:MeOH, Flow rate 90 g/min.

1-(1-(2-((1*H*-1,2,4-Triazol-3-yl)methyl)-1-oxo-1,2-dihydroisoquinolin-4-yl)ethyl)-3-(3-chloro-4-fluorophenyl)-1-methylurea: Enantiomer I (**Compound 269**). LCMS: *m*/*z* found 455.2/457.2 [M+H]⁺, RT = 3.72 min (Method A); ¹H NMR (400 MHz, DMSO-d₆): δ 13.90 (bs, 1H), 8.47 (s, 1H), 8.32 (s, 1H), 8.25 (d, 1H), 7.84-7.87 (m, 1H), 7.71-7.78 (m, 2H), 7.63 (s, 1H), 7.48-7.54 (m, 2H), 7.32 (t, 1H), 5.88-5.93 (m, 1H), 5.38 (d, 1H), 5.29 (d, 1H), 2.61 (s, 3H), 1.43 (d, 3H); Chiral analytical SFC: RT = 1.51 min, Column: Chiralcel OZ-3 (4.6 x 150 mm) 3 µ, 60% CO₂:MeOH, Flow = 3.0 g/min.

1-(1-(2-((1*H*-1,2,4-Triazol-3-yl)methyl)-1-oxo-1,2-dihydroisoquinolin-4-yl)ethyl)-3-(3-chloro-4-fluorophenyl)-1-methylurea: Enantiomer II (**Compound 270**). LCMS: *m*/*z* found 455.2/457.2 [M+H]⁺, RT = 3.72 min (Method A); ¹H NMR (400 MHz, DMSO-d₆): δ 13.90 (bs, 1H), 8.47 (s, 1H), 8.32 (s, 1H), 8.25 (d, 1H), 7.84-7.87 (m, 1H), 7.71-7.78 (m, 2H), 7.63 (s, 1H), 7.48-7.54 (m, 2H), 7.32 (t, 1H), 5.88-5.93 (m, 1H), 5.38 (d, 1H), 5.29 (d, 1H), 2.61 (s, 3H), 1.43 (d, 3H); Chiral analytical SFC: RT = 2.45 min, Column: Chiralcel OZ-3 (4.6 x 150 mm) 3 µ, 60% CO₂:MeOH, Flow = 3.0 g/min.

### 1-(1-(2-((1H-1,2,3-Triazol-4-yl)methyl)-1-oxo-1,2-dihydroisoquinolin-4-yl)ethyl)-3-(3-chloro-4-fluorophenyl)-1-methylurea (Compounds 276 & 277)

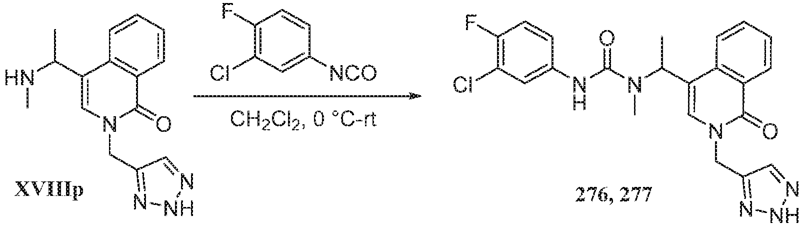

Racemic 1-(1-(2-((1*H*-1,2,3-triazol-4-yl)methyl)-1-oxo-1,2-dihydroisoquinolin-4-yl)ethyl)-3-(3-chloro-4-fluorophenyl)-1-methylurea was synthesized in an analogous manner as described above from 2-((1H-1,2,3-triazol-4-yl)methyl)-4-(1-(methylamino)ethyl)isoquinolin-1(2*H*)-one **(XVIIIp,** derived from *N-*alkylation of **IIIa** with (1-trityl-1*H*-1,2,3-triazol-4-yl)methyl methanesulfonate) and 2-chloro-1-fluoro-4-isocyanatobenzene. The enantiomers were subsequently separated by SFC, Column: Chiralpak AD-H (250 × 30 mm) 5 µ, 65% CO₂:MeOH, Flow rate 90 g/min.

1-(1-(2-((1*H*-1,2,3-Triazol-4-yl)methyl)-1-oxo-1,2-dihydroisoquinolin-4-yl)ethyl)-3-(3-chloro-4-fluorophenyl)-1-methylurea: Enantiomer I (**Compound 276**). LCMS: *m*/*z* found 455.2/457.2 [M+H]⁺, RT = 3.43 min (Method A); ¹H NMR (400 MHz, DMSO-d₆): δ 14.93 (bs, 1H), 8.46 (s, 1H), 8.27 (d, 1H), 7.84-7.87 (m, 1H), 7.81 (s, 1H), 7.70-7.75 (m, 2H), 7.65 (s, 1H), 7.47-7.54 (m, 2H), 7.31 (t, 1H), 5.88-5.91 (m, 1H), 5.40 (d, 1H), 5.28 (d, 1H), 2.60 (s, 3H), 1.45 (d, 3H); Chiral analytical SFC: RT = 9.70 min, Column: Chiralpak AD-H (4.6 x 250 mm) 5 µ, 60% CO₂:MeOH, Flow = 3.0 g/min.

1-(1-(2-((1*H*-1,2,3-Triazol-4-yl)methyl)-1-oxo-1,2-dihydroisoquinolin-4-yl)ethyl)-3-(3-chloro-4-fluorophenyl)-1-methylurea: Enantiomer II (**Compound 277**). LCMS: *m*/*z* found 455.2/457.2 [M+H]⁺, RT = 3.43 min (Method A); ¹H NMR (400 MHz, DMSO-d₆): δ 14.93 (bs, 1H), 8.46 (s, 1H), 8.27 (d, 1H), 7.84-7.87 (m, 1H), 7.81 (s, 1H), 7.70-7.75 (m, 2H), 7.65 (s, 1H), 7.47-7.54 (m, 2H), 7.31 (t, 1H), 5.88-5.91 (m, 1H), 5.40 (d, 1H), 5.28 (d, 1H), 2.60 (s, 3H), 1.45 (d, 3H); Chiral analytical SFC: RT = 12.15 min, Column: Chiralpak AD-H (4.6 x 250 mm) 5 µ, 60% CO₂:MeOH, Flow = 3.0 g/min.

### 3-(3-Chloro-4-fluorophenyl)-1-methyl-1-(1-(1-oxo-2-(2,2,2-trifluoroethyl)-1,2-dihydroisoquinolin-4-yl)ethyl)urea (Compound 228)

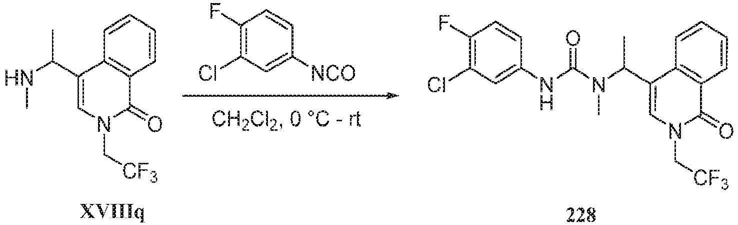

Racemic 3-(3-chloro-4-fluorophenyl)-1-methyl-1-(1-(1-oxo-2-(2,2,2-trifluoroethyl)-1,2-dihydroisoquinolin-4-yl)ethyl)urea was synthesized in an analogous manner as described above from racemic 4-(1-(methylamino)ethyl)-2-(2,2,2-trifluoroethyl)isoquinolin-1(2*H)*-one (**XVIIIq**) and 2-chloro-1-fluoro-4-isocyanatobenzene. LCMS: *m*/*z* found 456.3 [M+H]⁺, RT = 7.74 min (Method A); ¹H NMR (400 MHz, Chloroform-*d*) δ 8.48 (m, 1H), 7.81 (m, 1H), 7.72 (m, 1H), 7.65 (m, 1H), 7.55 (m, 1H), 7.22 (m, 1H), 7.01-7.13 (m, 2H), 6.29 (s, 1H), 6.07-6.17 (m, 1H), 4.86 (m, 1H), 4.60 (m, 1H), 2.66 (s, 3H), 1.52 (d, 3H).

### 3-(3-Chloro-4-fluorophenyl)-1-isobutyl-1-(1-(1-oxo-2-(2,2,2-trifluoroethyl)-1,2-dihydroisoquinolin-4-yl)ethyl)urea (Compound 229)

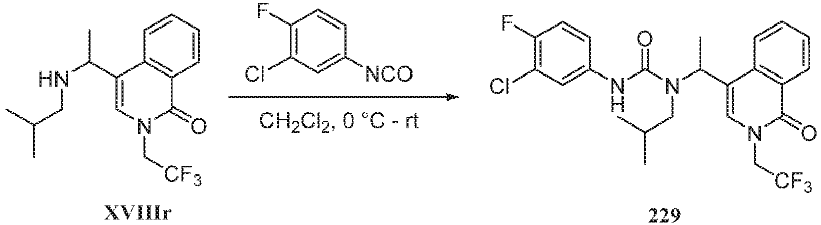

Racemic 3-(3-chloro-4-fluorophenyl)-1-isobutyl-1-(1-(1-oxo-2-(2,2,2-trifluoroethyl)-1,2-dihydroisoquinolin-4-yl)ethyl)urea was synthesized in an analogous manner as described above from racemic 4-(1-(isobutylamino)ethyl)-2-(2,2,2-trifluoroethyl)isoquinolin-1(2*H*)-one (**XVIIIr**) and 2-chloro-1-fluoro-4-isocyanatobenzene. LCMS: *m*/*z* found 498.3/500.5 [M+H]⁺, RT = 8.09 min (Method A); ¹H NMR (400 MHz, CDCl₃) δ 8.48 (m, 1H), 7.79-7.86 (m, 1H), 7.74 (m, 1H), 7.51-7.65 (m, 2H), 7.16-7.28 (m, 1H), 7.03-7.13 (m, 2H), 6.35 (s, 1H), 6.18 (q, 1H), 5.15 (m, 1H), 4.34 (m, 1H), 2.84 (m, 2H), 1.54 (d, 3H), 1.38 (m, 1H), 0.75 (d, 3H), 0.52 (d, 3H).

### 3-(4-Fluorophenyl)-1-isobutyl-1-(1-(1-oxo-2-(2,2,2-trifluoroethyl)-1,2-dihydroisoquinolin-4-yl)ethyl)urea (Compound 235)

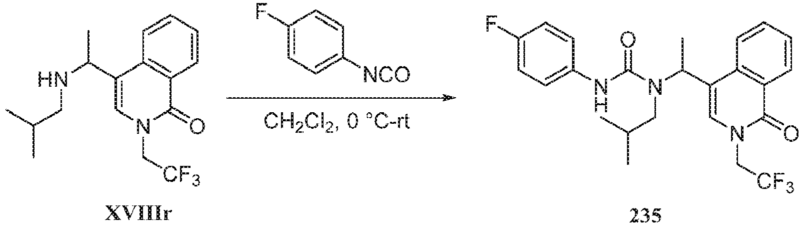

Racemic 3-(4-fluorophenyl)-1-isobutyl-1-(1-(1-oxo-2-(2,2,2-trifluoroethyl)-1,2-dihydroisoquinolin-4-yl)ethyl)urea (**Compound 235**) was synthesized in an similar manner as described above from 4-(1-(isobutylamino)ethyl)-2-(2,2,2-trifluoroethyl)isoquinolin-1(2*H*)-one (**XVIIIr**) and 1-fluoro-4-isocyanatobenzene. LCMS: *m*/*z* found 464.4 [M+H]⁺, RT = 7.85 min (Method A); ¹H NMR (400 MHz, DMSO-*d*₆) δ 8.25-8.34 (m, 2H), 7.89 (d, 1H), 7.82 (m, 1H), 7.46-7.63 (m, 4H), 7.06-7.17 (m, 2H), 5.96 (d, 1H), 5.10 (m, 1H), 4.90 (m, 1H), 2.99 (m, 1H), 2.85 (m, 1H), 1.47 (d, 3H), 1.31-1.42 (m, 1H), 0.61 (d, 3H), 0.43 (d, 3H).

### 4-Bromo-7-fluoro-2-methylisoquinolin-1(2H)-one (XVIb)

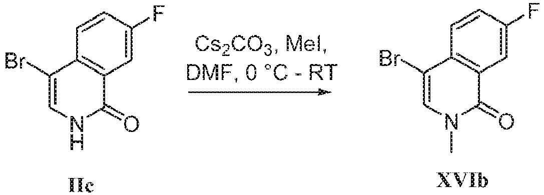

To a solution of 3.5 g (14.5 mmol, 1.0 eq.) of 4-bromo-7-fluoroisoquinolin-1(2*H*)-one (**IIc**) in 30 mL of DMF at 0 °C was added 11.8 g (36.3 mmol, 2.5 eq.) of cesium carbonate followed by 3.1 g (21.8 mmol, 1.5 eq.) of methyl iodide and the mixture was stirred at room temperature for 16 h. The reaction mixture was then was diluted with 80 mL of ice-cold water and the resulting solids were collected by filtration, washed with 50 mL of petroleum ether and dried under vacuum to provide 2.0 g (7.8 mmol, 54%) of 4-bromo-7-fluoro-2-methylisoquinolin-1(2*H*)-one (**XVIb**). LCMS: *m*/*z* found 256.0/258.0 [M+H]⁺, RT = 1.79 min; ¹H NMR (300 MHz, DMSO-d₆): δ 7.90-7.95 (m, 2H), 7.70-7.84 (m, 2H), 3.52 (s, 3H).

### 4-Acetyl-7-fluoro-2-methylisoquinolin-1(2H)-one (XVIIb)

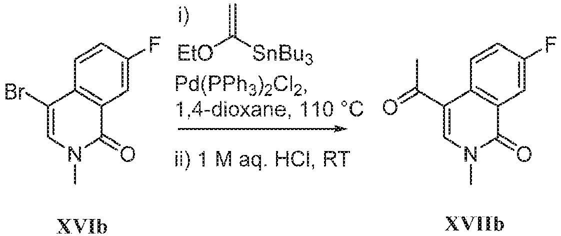

To a solution of 2.0 g (7.8 mmol, 1.0 eq.) of 4-bromo-7-fluoro-2-methylisoquinolin-1(2*H*)-one (**XVIb**) in 15 mL of 1,4-dioxane was added 7.07 g (19.6 mmol, 2.5 eq.) of tributyl(1-ethoxyvinyl)stannane. The solution was purged purged with nitrogen gas for 5 min and 0.55 g (0.78 mmol, 0.1 eq.) of Pd(PPh₃)₂Cl₂ was added and the mixture was heated at 110 °C for 16 h under a nitrogen atmosphere. The mixture was then allowed to cool to room temperature and 30 mL of 1 M aqueous HCl solution was added and stirring was continued for a further 2 h. The mixture was diluted with 60 mL of water and extracted with 3 x 150 mL of ethyl acetate. The combined organic extracts were washed with 100 mL of water, 100 mL of brine, dried (Na₂SO₄), filtered and the solvent was removed *in* vacuo. The residue was triturated with 50 mL of pentane and the resulting solids were collected by filtration and dried under high vacuum to provide 1.6 g (7.3 mmol, 93%) of 4-acetyl-7,8-difluoroisoquinolin-1(2*H*)-one (**XVIIb**). LCMS: *m*/*z* found 220.4 [M+H]⁺, RT = 1.95 min; ¹H NMR (400 MHz, DMSO-d₆): δ 8.96-9.00 (m, 1H), 8.57 (s, 1H), 7.89-7.93 (m, 1H), 7.65-7.71 (m, 1H), 3.63 (s, 3H), 2.55 (s, 3H).

### 7-Fluoro-2-methyl-4-(1-(methylamino)ethyl)isoquinolin-1(2H)-one (XVIIIb)

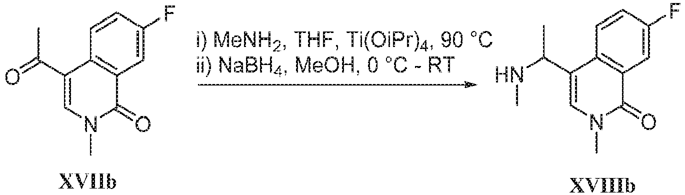

To a solution of 0.8 g (3.65 mmol, 1.0 eq.) of 4-acetyl-7-fluoro-2-methylisoquinolin-1(2*H*)-one (**XVIIb**) in 5 mL of THF under a nitrogen atmosphere was added 8 mL (16.0 mmol, 4.4 eq.) of a 2 M methylamine solution in THF followed by 8 mL of titanium isopropoxide and the mixture was heated at 90 °C for 16 h. The mixture was allowed to cool to room temperature and further cooled to 0 °C. The mixture was then diluted with 5 mL of methanol and 0.41 g (10.95 mmol, 3.0 eq.) of sodium borohydride was added portions wise over approximately 10 min. After stirring for 4 h at room temperature the mixture was diluted with 50 mL of water and extracted with 4 × 60 mL of ethyl acetate. The combined organic extracts were washed with 50 mL of water, 50 mL of brine, dried (Na₂SO₄), filtered and the solvent was removed *in vacuo* to provide 0.62 g of 7-fluoro-2-methyl-4-(1-(methylamino)ethyl) isoquinolin-1(2*H*)-one (**XVIIIb**)**.** LCMS: *m*/*z* found 235.1 [M+H]⁺, RT = 1.40 min; ¹H NMR (300 MHz, DMSO-d₆): δ 8.16-8.21 (m, 1H), 7.88-7.93 (m, 1H), 7.56-7.64 (m, 1H), 7.41 (s, 1H), 3.88-3.94 (m, 1H), 3.52 (s, 3H), 2.23 (s, 3H), 2.07 (bs, 1H), 1.33 (d, 3H).

### 3-(3-Chloro-4-fluorophenyl)-1-(1-(7-fluoro-2-methyl-1-oxo-1,2-dihydroisoquinolin-4-yl)ethyl)-1-methylurea (Compounds 302 & 303)

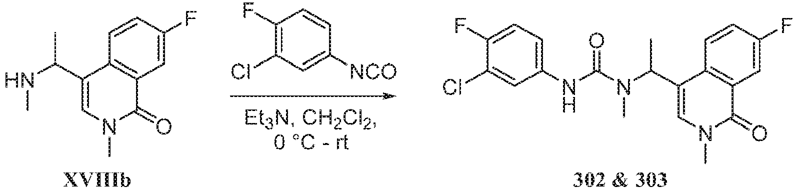

Racemic 3-(3-chloro-4-fluorophenyl)-1-(1-(7-fluoro-2-methyl-1-oxo-1,2-dihydroisoquinolin-4-yl)ethyl)-1-methylurea was synthesized in a similar manner as described above from 7-fluoro-2-methyl-4-(1-(methylamino)ethyl)isoquinolin-1(2*H*)-one (**XVIIIb**) and 2-chloro-1-fluoro-4-isocyanatobenzene. The enantiomers were subsequently separated by chiral SFC, Chiralpak IC (250 × 30 mm) 5 µ, 60% CO₂/MeOH, Flow rate 90 g/min.

3-(3-Chloro-4-fluorophenyl)-1-(1-(7-fluoro-2-methyl-1-oxo-1,2-dihydroisoquinolin-4-yl)ethyl)-1-methylurea - Enantiomer I (**Compound 302**): LCMS: *m*/*z* found 406.2/408.2 [M+H]⁺, RT = 4.63 min (method A); ¹H NMR (400 MHz, DMSO-*d₆*): δ 8.46 (s, 1H), 7.90-7.94 (m, 1H), 7.85-7.88 (m, 1H), 7.77-7.81 (m, 1H), 7.65-7.71 (m, 1H), 7.48-7.52 (m, 2H), 7.31 (t, 1H), 5.85-5.90 (m, 1H), 3.59 (s, 3H), 2.60 (s, 3H), 1.45 (d, 3H); Chiral analytical SFC: RT = 1.55 min, Column: Chiralpak IC-3 (4.6 x 250) mm, 5 µ, 60% CO₂/MeOH, Flow = 3.0 g/min.

3-(3-Chloro-4-fluorophenyl)-1-(1-(7-fluoro-2-methyl-1-oxo-1,2-dihydroisoquinolin-4-yl)ethyl)-1-methylurea - Enantiomer II (**Compound 303**): LCMS: *m*/*z* found 406.2/408.2 [M+H]⁺, RT = 4.63 min (Method A); ¹H NMR (400 MHz, DMSO-*d₆*): δ 8.46 (s, 1H), 7.90-7.94 (m, 1H), 7.85-7.88 (m, 1H), 7.77-7.81 (m, 1H), 7.65-7.71 (m, 1H), 7.48-7.52 (m, 2H), 7.31 (t, 1H), 5.85-5.90 (m, 1H), 3.59 (s, 3H), 2.60 (s, 3H), 1.45 (d, 3H); Chiral analytical SFC: RT = 2.48 min, Column: Chiralpak IC-3 (4.6 × 250) mm, 5 µ, 60% CO₂/MeOH, Flow = 3.0 g/min.

### 4-Acetyl-8-fluoro-2-methylisoquinolin-1(2H)-one (XVIId)

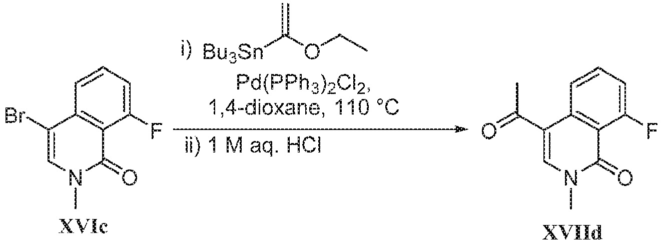

To a solution of 0.8 g (3.1 mmol, 1.0 eq.) of 4-bromo-8-fluoro-2-methylisoquinolin-1(2*H*)-one (**XVIc**) in 16 mL of 1,4-dioxane was added 2.8 g (7.8 mmol, 2.5 eq.) of tributyl(1-ethoxyvinyl)tin. The mixture was degassed by purging with argon gas for 5 min and 0.22 g (0.31 mmol, 0.1 eq.) of bis(triphenylphosphine)palladium(II) dichloride was added. The mixture was then heated at 110 °C under an argon atmosphere for 16 h. The mixture was allowed to cool to room temperature and diluted with 20 mL of 1 M aqueous HCl and the resulting solution stirred at room temperature for 2 h. The mixture was basified with 40 mL of saturated sodium bicarbonate solution and filtered through CELITE^{®}. The filtrate was extracted with 3 × 50 mL of ethyl acetate and the combined organic extracts were washed with 30 mL of brine, dried (Na₂SO₄), filtered and the solvent was removed *in vacuo.* The residue was triturated with 50 mL of *n*-pentane to provide 0.42 g (1.9 mmol, 61%) of 4-acetyl-8-fluoro-2-methylisoquinolin-1(2*H*)-one (**XVIId**). LCMS: *m*/*z* found 220.3 [M+H]⁺. ¹H NMR (400 MHz, CDCl₃): δ 8.75 (d, 1H), 7.96 (s, 1H), 7.64-7.69 (m, 1H), 7.16-7.21 (m, 1H), 3.66 (s, 3H), 2.57 (s, 3H).

### 8-Fluoro-2-methyl-4-(1-(methylamino)ethyl)isoquinolin-1(2H)-one (XVIIIs)

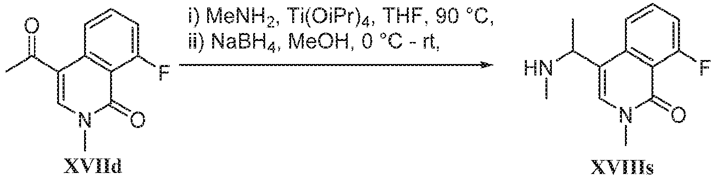

To a solution of 0.42 g (1.8 mmol, 1.0 eq.) of 4-acetyl-8-fluoro-2-methylisoquinolin-1(2*H*)-one (**XVIId**) in 4 mL of THF in a sealed tube under a nitrogen atmosphere was added 1.8 mL (3.6 mmol, 2.0 eq.) of a 2 M solution of methylamine in THF followed by 2 mL of titanium isopropoxide, and the mixture was heated to 90 °C for 4 h. The mixture was allowed to cool to room temperature and further cooled to 0 °C. The cooled solution was then diluted with 2 mL of methanol and 0.21 g (5.45 mmol, 3.0 eq.) of sodium borohydride was added portions wise. The mixture was then allowed to warm to room temperature and stirred for 1 h. The reaction mixture was diluted with 10 mL of ice-cold water and extracted with 4 x 100 mL of methylene chloride. The combined organic extracts were washed with 50 mL of brine, dried (Na₂SO₄), filtered and the solvent was removed *in vacuo* to provide 0.4 g of crude 8-fluoro-2-methyl-4-(1-(methylamino)ethyl)isoquinolin-1(2*H*)-one (**XVIIIs**). LCMS: *m*/*z* found 235.2 [M+H]⁺.

### 3-(3-Chloro-4-fluorophenyl)-1-(1-(8-fluoro-2-methyl-1-oxo-1,2-dihydroisoquinolin-4-yl)ethyl)-1-methylurea (Compounds 358 & 359)

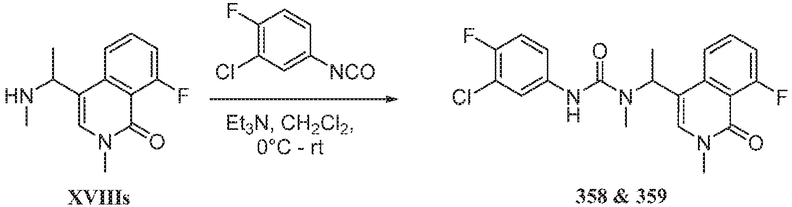

Racemic 3-(3-chloro-4-fluorophenyl)-1-(1-(8-fluoro-2-methyl-1-oxo-1,2-dihydroisoquinolin-4-yl)ethyl)-1-methylurea was synthesized in a similar manner as described above from 8-fluoro-2-methyl-4-(1-(methylamino)ethyl)isoquinolin-1(2*H*)-one (**XVIIIs**) and 2-chloro-1-fluoro-4-isocyanatobenzene. The enantiomers were subsequently separated by SFC, Column: (R,R)-Whelk-01 (30 × 250 mm) 5 µ, 40% CO₂:MeOH, flow rate 90 g/min.

3-(3-Chloro-4-fluorophenyl)-1-(1-(8-fluoro-2-methyl-1-oxo-1,2-dihydroisoquinolin-4-yl)ethyl)-1-methylurea- Enantiomer I (**Compound 358**) LCMS: *m*/*z* found 406.1/408.1 [M+H]⁺, RT = 4.43 min (Method A); ¹H NMR (400 MHz, DMSO-*d*₆) δ 8.46 (bs, 1H), 7.84-7.87 (m, 1H), 7.69-7.74 (m, 1H), 7.57 (s, 1H), 7.48-7.52 (m, 2H), 7.31 (t, 1H), 7.20-7.25 (m, 1H), 5.79-5.84 (m, 1H), 3.52 (s, 3H), 2.59 (s, 3H), 1.45 (d, 3H); Chiral analytical SFC: RT = 3.13 min, Column: (R,R)-Whelk-01 (4.6 × 150 mm) 3.5 µm, 60% CO₂/MeOH, Flow = 3.0 g/min.

3-(3-Chloro-4-fluorophenyl)-1-(1-(8-fluoro-2-methyl-1-oxo-1,2-dihydroisoquinolin-4-yl)ethyl)-1-methylurea- Enantiomer II (**Compound 358**) LCMS: *m*/*z* found 406.1/408.1 [M+H]⁺, RT = 4.43 min (Method A); ¹H NMR (400 MHz, DMSO-*d*₆) δ 8.46 (bs, 1H), 7.84-7.87 (m, 1H), 7.69-7.74 (m, 1H), 7.57 (s, 1H), 7.48-7.52 (m, 2H), 7.31 (t, 1H), 7.20-7.25 (m, 1H), 5.79-5.84 (m, 1H), 3.52 (s, 3H), 2.59 (s, 3H), 1.45 (d, 3H); Chiral analytical SFC: RT = 4.28 min, Column: (R,R)-Whelk-01 (4.6 x 150 mm) 3.5 µm, 60% CO₂/MeOH, Flow = 3.0 g/min.

### 3-(3-Chloro-4-fluorophenyl)-1-(1-(6,7-difluoro-2-methyl-1-oxo-1,2-dihydroisoquinolin-4-yl)ethyl)-1-methylurea (Compounds 340 & 341)

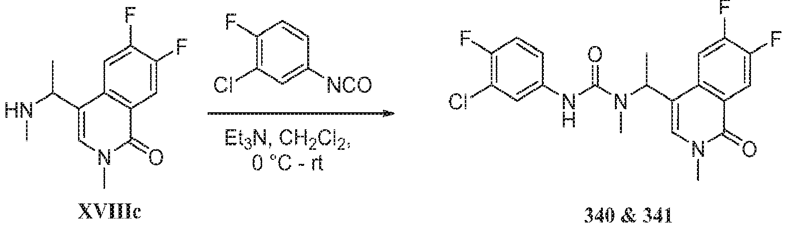

Racemic 3-(3-chloro-4-fluorophenyl)-1-(1-(6,7-difluoro-2-methyl-1-oxo-1,2-dihydroisoquinolin-4-yl)ethyl)-1-methylurea was synthesized in a similar manner as described above from 6,7-difluoro-2-methyl-4-(1-(methylamino)ethyl)isoquinolin-1(2*H*)-one (**XVIIIc**) and 2-chloro-1-fluoro-4-isocyanatobenzene. The enantiomers were subsequently separated by chiral SFC, Column: Chiralpak IC (250 × 30 mm) 5 µ, 65% CO₂/MeOH, Flow rate 90 g/min.

3-(3-Chloro-4-fluorophenyl)-1-(1-(6,7-difluoro-2-methyl-1-oxo-1,2-dihydroisoquinolin-4-yl)ethyl)-1-methylurea - Enantiomer I (**Compound 340**), LCMS: *m*/*z* found 424.1/426.1 [M+H]⁺, RT = 4.94 min (Method A); ¹H NMR (400 MHz, DMSO-d₆): δ 8.48 (bs, 1H), 8.11-8.17 (m, 1H), 7.81-7.83 (m, 1H), 7.71-7.76 (m, 1H), 7.59 (s, 1H), 7.48-7.53 (m, 1H), 7.33 (t, 1H), 5.79-5.84 (m, 1H), 3.58 (s, 3H), 2.62 (s, 3H), 1.45 (d, 3H); Chiral analytical SFC: RT = 1.63 min, Column: Chiralpak IC, (4.6 × 150 mm) 5 µm, 65% CO₂/MeOH, Flow = 3.0 g/min.

3-(3-Chloro-4-fluorophenyl)-1-(1-(6,7-difluoro-2-methyl-1-oxo-1,2-dihydroisoquinolin-4-yl)ethyl)-1-methylurea - Enantiomer II (**Compound 341**), LCMS: *m*/*z* found 424.1/426.1 [M+H]⁺, RT = 4.94 min (Method A); ¹H NMR (400 MHz, DMSO-d₆): δ 8.48 (bs, 1H), 8.11-8.17 (m, 1H), 7.81-7.83 (m, 1H), 7.71-7.76 (m, 1H), 7.59 (s, 1H), 7.48-7.53 (m, 1H), 7.33 (t, 1H), 5.79-5.84 (m, 1H), 3.58 (s, 3H), 2.62 (s, 3H), 1.45 (d, 3H); Chiral analytical SFC: RT = 3.05 min, Column: Chiralpak IC, (4.6 x 150 mm) 5 µm, 65% CO₂/MeOH, Flow = 3.0 g/min.

### 3-(3-Chloro-4-fluorophenyl)-1-(1-(6,7-difluoro-2-methyl-1-oxo-1,2-dihydroisoquinolin-4-yl)ethyl)-1-isobutylurea (Compounds 342 & 343)

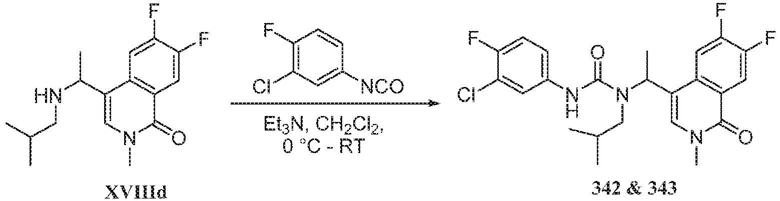

Racemic 3-(3-chloro-4-fluorophenyl)-1-(1-(6,7-difluoro-2-methyl-1-oxo-1,2-dihydroisoquinolin-4-yl)ethyl)-1-isobutylurea was synthesized in a similar manner as described previously from 6,7-difluoro-4-(1-(isobutylamino)ethyl)-2-methyfisoquinolin-1(2*H*)-one (**XVIIId**) and 2-chloro-1-fluoro-4-isocyanatobenzene. The enantiomers were subsequently separated by chiral SFC, Column: (R,R) Whelk-001 (250 × 30 mm) 5 µ, 75% CO₂/MeOH, Flow rate 100 g/min.

3-(3-Chloro-4-fluorophenyl)-1-(1-(6,7-difluoro-2-methyl-1-oxo-1,2-dihydroisoquinolin-4-yl)ethyl)-1-isobutylurea - Enantiomer I (**Compound 342**), LCMS: *m*/*z* found 466.1/468.1 [M+H]⁺, RT = 5.70 min (Method A); ¹H NMR (400 MHz, DMSO-d₆): δ 8.44 (s, 1H), 8.12-8.18 (m, 1H), 7.81-7.88 (m, 1H), 7.73-7.77 (m, 1H), 7.66 (s, 1H), 7.42-7.49 (m, 1H), 7.34 (t, 1H), 5.81-5.84 (m, 1H), 3.58 (s, 3H), 3.01-3.07 (m, 1H), 2.82-2.89 (m, 1H), 1.48 (d, 3H), 1.35-1.44 (m, 1H), 0.65 (d, 3H), 0.41 (d, 3H); Chiral analytical SFC: RT = 3.18 min, Column: (R,R) Whelk-001, (4.6 x 150 mm) 5 µm, 70% CO₂/MeOH, Flow = 3.0 g/min.

3-(3-Chloro-4-fluorophenyl)-1-(1-(6,7-difluoro-2-methyl-1-oxo-1,2-dihydroisoquinolin-4-yl)ethyl)-1-isobutylurea - Enantiomer II (**Compound 343**), LCMS: *m*/*z* found 466.1/468.1 [M+H]⁺, RT = 5.70 min (Method A); ¹H NMR (400 MHz, DMSO-d₆): δ 8.44 (s, 1H), 8.12-8.18 (m, 1H), 7.81-7.88 (m, 1H), 7.73-7.77 (m, 1H), 7.66 (s, 1H), 7.42-7.49 (m, 1H), 7.34 (t, 1H), 5.81-5.84 (m, 1H), 3.58 (s, 3H), 3.01-3.07 (m, 1H), 2.82-2.89 (m, 1H), 1.48 (d, 3H), 1.35-1.44 (m, 1H), 0.65 (d, 3H), 0.41 (d, 3H); Chiral analytical SFC: RT = 4.40 min, Column: (R,R) Whelk-001, (4.6 x 150 mm) 5 µm, 70% CO₂/MeOH, Flow = 3.0 g/min.

### 3-(3-Chloro-4-fluorophenyl)-1-(1-(6,7-difluoro-2-methyl-1-oxo-1,2-dihydroisoquinolin-4-yl)ethyl)-1-(3-hydroxypropyl)urea (Compounds 350 & 351)

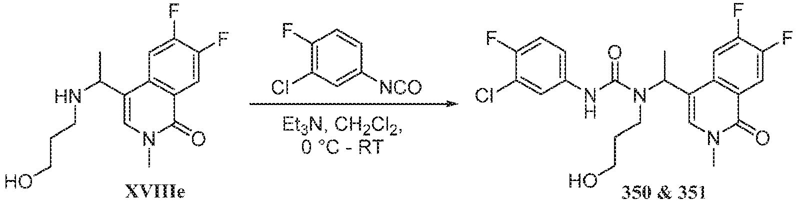

Racemic 3-(3-chloro-4-fluorophenyl)-1-(1-(6,7-difluoro-2-methyl-1-oxo-1,2-dihydroisoquinolin-4-yl)ethyl)-1-(3-hydroxypropyl)urea was synthesized in a similar manner as described previously from 6,7-difluoro-4-(1-(3-hydroxypropylamino)ethyl)-2-methylisoquinolin-1(2*H*)-one (**XVIIIe**) and 2-chloro-1-fluoro-4-isocyanatobenzene. The enantiomers were subsequently separated by chiral SFC, Column: (R,R) Whelk-001 (250 × 30 mm) 3.5 µ, 55% CO₂/MeOH, Flow rate 90 g/min.

3-(3-Chloro-4-fluorophenyl)-1-(1-(6,7-difluoro-2-methyl-1-oxo-1,2-dihydroisoquinolin-4-yl)ethyl)-1-(3-hydroxypropyl)urea- Enantiomer I (**Compound 350**), LCMS: *m*/*z* found 468.1/470.1 [M+H]⁺, RT = 4.79 min (Method A); ¹H NMR (400 MHz, DMSO-d₆): δ 8.72 (bs, 1H), 8.13-8.18 (m, 1H), 7.75-7.76 (m, 1H), 7.66-7.71 (m, 1H), 7.64 (s, 1H), 7.41-7.46 (m, 1H), 7.34 (t, 1H), 5.81-5.85 (m, 1H), 4.99 (br t, 1H), 3.58 (s, 3H), 3.15-3.21 (m, 4H), 1.47 (d, 3H), 1.09-1.23 (m, 2H); Chiral analytical SFC: RT = 2.51 min, Column: (R,R) Whelk-001 (4.6 × 150 mm) 3.5 µm, 60% CO₂/MeOH, Flow = 3.0 g/min.

3-(3-Chloro-4-fluorophenyl)-1-(1-(6,7-difluoro-2-methyl-1-oxo-1,2-dihydroisoquinolin-4-yl)ethyl)-1-(3-hydroxypropyl)urea- Enantiomer II (**Compound 351**), LCMS: *m*/*z* found 468.1/470.1 [M+H]⁺, RT = 4.79 min (Method A); ¹H NMR (400 MHz, DMSO-d₆): δ 8.72 (bs, 1H), 8.13-8.18 (m, 1H), 7.75-7.76 (m, 1H), 7.66-7.71 (m, 1H), 7.64 (s, 1H), 7.41-7.46 (m, 1H), 7.34 (t, 1H), 5.81-5.85 (m, 1H), 4.99 (br t, 1H), 3.58 (s, 3H), 3.15-3.21 (m, 4H), 1.47 (d, 3H), 1.09-1.23 (m, 2H); Chiral analytical SFC: RT = 3.88 min, Column: (R,R) Whelk-001 (4.6 x 150 mm) 3.5 µm, 60% CO₂/MeOH, Flow = 3.0 g/min.

### 2-(3-(3-Chloro-4-fluorophenyl)-1-(1-(6,7-difluoro-1-oxo-1,2-dihydroisoquinolin-4-yl)ethyl)ureido) ethane-1-sulfonamide (Compounds 385 & 386)

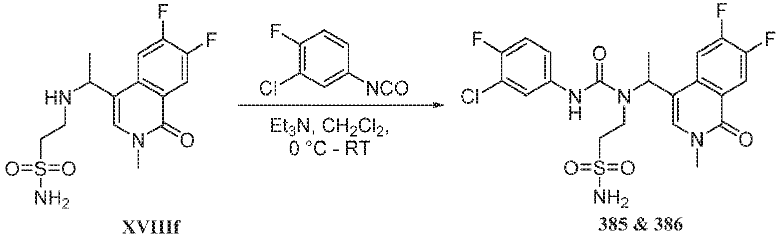

Racemic 2-(3-(3-chloro-4-fluorophenyl)-1-(1-(6,7-difluoro-1-oxo-1,2-dihydroisoquinolin-4-yl)ethyl)ureido) ethane-1-sulfonamide was synthesized in a similar manner as described previously from 2-(1-(6,7-difluoro-1-oxo-1,2-dihydroisoquinolin-4-yl)ethylamino) ethanesulfonamide (**XVIIIf**) and 2-chloro-1-fluoro-4-isocyanatobenzene. The enantiomers were subsequently separated by chiral SFC, Column: (R,R) Whelk-01 (250 × 30 mm) 5 µ, 65% CO₂/MeOH, Flow rate 100 g/min.

2-(3-(3-Chloro-4-fluorophenyl)-1-(1-(6,7-difluoro-1-oxo-1,2-dihydroisoquinolin-4-yl)ethyl)ureido) ethane-1-sulfonamide - Enantiomer I (**Compound 385**), LCMS: *m*/*z* found 503.1/505.1 [M+H]⁺, RT = 4.29 min (Method A); ¹H NMR (400 MHz, DMSO-d₆): δ 8.34 (bs, 2H), 8.11-8.16 (m, 1H), 7.75-7.77 (m, 1H), 7.57-7.62 (m, 1H), 7.44-7.48 (m, 1H), 7.36 (t, 1H), 7.29 (s, 1H), 7.10 (bs, 2H), 5.68-5.72 (m, 1H), 3.43-3.51 (m, 2H), 3.00-3.07 (m, 1H), 2.59-2.67 (m, 1H), 1.51 (d, 3H); Chiral analytical SFC: RT = 2.05 min, Column: (R,R) Whelk-001 (4.6 × 150 mm) 5 µm, 60% CO₂/MeOH, Flow = 3.0 g/min.

2-(3-(3-Chloro-4-fluorophenyl)-1-(1-(6,7-difluoro-1-oxo-1,2-dihydroisoquinolin-4-yl)ethyl)ureido) ethane-1-sulfonamide - Enantiomer II (**Compound 386**), LCMS: *m*/*z* found 503.1/505.1 [M+H]+, RT = 4.29 min (Method A); ¹H NMR (400 MHz, DMSO-d₆): δ 8.34 (bs, 2H), 8.11-8.16 (m, 1H), 7.75-7.77 (m, 1H), 7.57-7.62 (m, 1H), 7.44-7.48 (m, 1H), 7.36 (t, 1H), 7.29 (s, 1H), 7.10 (bs, 2H), 5.68-5.72 (m, 1H), 3.43-3.51 (m, 2H), 3.00-3.07 (m, 1H), 2.59-2.67 (m, 1H), 1.51 (d, 3H); Chiral analytical SFC: RT = 2.93 min, Column: (R,R) Whelk-001 (4.6 × 150 mm) 5 µm, 60% CO₂/MeOH, Flow = 3.0 g/min.

### 2-(1-(1-(6,7-Difluoro-1-oxo-1,2-dihydroisoquinolin-4-yl)ethyl)-3-(4-fluorophenyl) ureido)ethane-1-sulfonamide (Compounds 387 & 388)

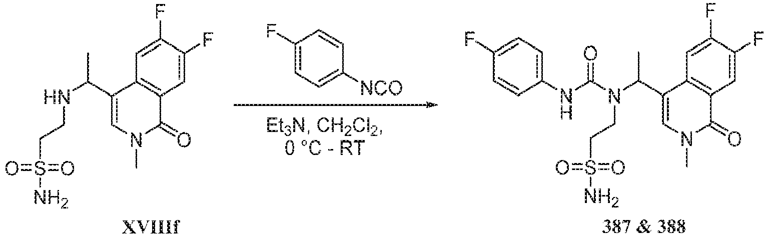

Racemic 2-(1-(1-(6,7-difluoro-1-oxo-1,2-dihydroisoquinolin-4-yl)ethyl)-3-(4-fluorophenyl)ureido)ethane-1-sulfonamide was synthesized in a similar manner as described previously from 2-(1-(6,7-difluoro-1-oxo-1,2-dihydroisoquinolin-4-yl)ethylamino) ethanesulfonamide (**XVIIIf**) and 4-fluorophenyl isocyanate. The enantiomers were subsequently separated by chiral SFC, Column: Chiralpak IG (250 x 30 mm) 5 µ, 70% CO₂/MeOH, Flow rate 100 g/min.

2-(1-(1-(6,7-Difluoro-1-oxo-1,2-dihydroisoquinolin-4-yl)ethyl)-3-(4-fluorophenyl) ureido)ethane-1-sulfonamide - Enantiomer I (**Compound 387**), LCMS: *m*/*z* found 469.2 [M+H]⁺, RT = 3.88 min (Method A); ¹H NMR (400 MHz, DMSO-d₆): δ 11.68 (bs, 1H), 8.53 (bs, 1H), 8.11-8.17 (m, 1H), 7.61-7.66 (m, 1H), 7.47-7.51 (m, 2H), 7.28 (s, 1H), 7.12-7.17 (m, 2H), 6.85 (bs, 2H), 5.69-5.73 (m, 1H), 3.41-3.49 (m, 2H), 3.01-3.08 (m, 1H), 2.58-2.4 (m, 1H), 1.50 (d, 3H); Chiral analytical SFC: RT = 1.09 min, Column: Chiralpak IG (4.6 x 150 mm) 5 µm, 75% CO₂/MeOH, Flow = 3.0 g/min.

2-(1-(1-(6,7-Difluoro-1-oxo-1,2-dihydroisoquinolin-4-yl)ethyl)-3-(4-fluorophenyl) ureido)ethane-1-sulfonamide - Enantiomer II (**Compound 388**), LCMS: *m*/*z* found 469.2 [M+H]⁺, RT = 3.88 min (Method A); ¹H NMR (400 MHz, DMSO-d₆): δ 11.68 (bs, 1H), 8.53 (bs, 1H), 8.11-8.17 (m, 1H), 7.61-7.66 (m, 1H), 7.47-7.51 (m, 2H), 7.28 (s, 1H), 7.12-7.17 (m, 2H), 6.85 (bs, 2H), 5.69-5.73 (m, 1H), 3.41-3.49 (m, 2H), 3.01-3.08 (m, 1H), 2.58-2.4 (m, 1H), 1.50 (d, 3H); Chiral analytical SFC: RT = 2.70 min, Column: Chiralpak IG (4.6 x 150 mm) 5 µm, 75% CO₂/MeOH, Flow = 3.0 g/min.

### 3-(3-Chloro-4-fluorophenyl)-1-(1-(7,8-difluoro-2-methyl-1-oxo-1,2-dihydroisoquinolin-4-yl)ethyl)-1-methylurea (Compounds 332 & 333)

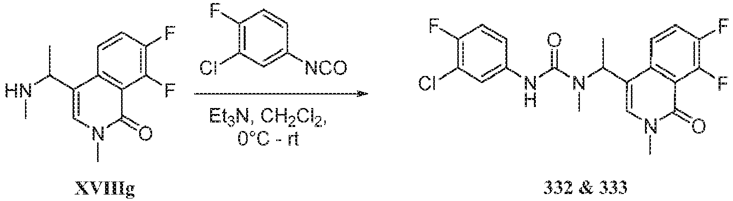

Racemic 3-(3-chloro-4-fluorophenyl)-1-(1-(7,8-difluoro-2-methyl-1-oxo-1,2-dihydroisoquinolin-4-yl)ethyl)-1-methylurea was synthesized in a similar manner as described above from 7,8-difluoro-2-methyl-4-(1-(methylamino)ethyl)isoquinolin-1(2*H*)-one (**XVIIIg**) and 2-chloro-1-fluoro-4-isocyanatobenzene. The enantiomers were subsequently separated by chiral SFC, Column: Chiralpak IC (250 x 30 mm) 5 µ, 65% CO₂/MeOH, Flow rate 100 g/min.

3-(3-Chloro-4-fluorophenyl)-1-(1-(7,8-difluoro-2-methyl-1-oxo-1,2-dihydroisoquinolin-4-yl)ethyl)-1-methylurea - Enantiomer I (**Compound 332**), LCMS: *m*/*z* found 424.1/426.0 [M+H]⁺, RT = 4.66 min (Method A); ¹H NMR (400 MHz, DMSO-d₆): δ 8.47 (s, 1H), 7.85-7.92 (m, 2H), 7.47-7.55 (m, 3H), 7.31 (t, 1H), 5.79-5.84 (m, 1H), 3.53 (s, 3H), 2.60 (s, 3H), 1.44 (d, 3H); Chiral analytical SFC: RT = 2.37 min, Column: Chiralpak IC, (4.6 x 150 mm) 5 µm, 60% CO₂/MeOH, Flow = 4.0 g/min.

3-(3-Chloro-4-fluorophenyl)-1-(1-(7,8-difluoro-2-methyl-1-oxo-1,2-dihydroisoquinolin-4-yl)ethyl)-1-methylurea- Enantiomer II (**Compound 333**), LCMS: *m*/*z* found 424.1/426.0 [M+H]⁺, RT = 4.66 min (Method A); ¹H NMR (400 MHz, DMSO-d₆): δ 8.47 (s, 1H), 7.85-7.92 (m, 2H), 7.47-7.55 (m, 3H), 7.31 (t, 1H), 5.79-5.84 (m, 1H), 3.53 (s, 3H), 2.60 (s, 3H), 1.44 (d, 3H); Chiral analytical SFC: RT = 4.57 min, Column: Chiralpak IC, (4.6 × 150 mm) 5 µm, 60% CO₂/MeOH, Flow = 4.0 g/min.

### 2-((1-(6,8-Difluoro-1-oxo-1,2-dihydroisoquinolin-4-yl)ethyl)amino)ethane-1-sulfonamide (VIIIae)

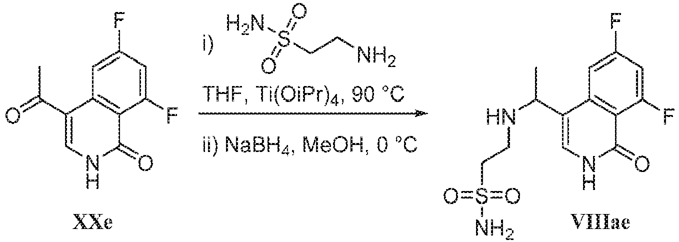

To a solution of 0.3 g (1.34 mmol, 1.0 eq.) of 4-acetyl-6,8-difluoroisoquinolin-1(2*H*)-one (**XXe**) in 1.5 mL of THF under a nitrogen atmosphere was added 0.17 mg (1.34 mmol, 1.0 eq.) of 2-aminoethanesulfonamide followed by 1.5 mL of titanium isopropoxide and the mixture was heated at 90 °C for 24 h. The mixture was allowed to cool to room temperature and further cooled to 0 °C. Following dilution with 3 mL of methanol, 0.15 g (4.02 mmol, 3.0 eq.) of sodium borohydride was added portion-wise over approximately 10 min and stirring was continued for 2 h. The reaction mixture was diluted with 5 mL of brine and 100 mL of 10% methanol in methylene chloride and the mixture was filtered through CELITE^{®}. The pad was washed with 40 mL of 10% methanol in methylene chloride and the layers were separated. The organic phase was dried (Na₂SO₄), filtered and the solvent was removed *in vacuo* to provide 150 mg of 2-((1-(6,8-difluoro-1-oxo-1,2-dihydroisoquinolin-4-yl)ethyl)amino)ethane-1-sulfonamide (**VIIIae**). LCMS: *m*/*z* found 332.0 [M+H]⁺.

### 2-(3-(3-Chloro-4-fluorophenyl)-1-(1-(6,8-difluoro-1-oxo-1,2-dihydroisoquinolin-4-yl)ethyl)ureido) ethane-1-sulfonamide (Compounds 389 & 390)

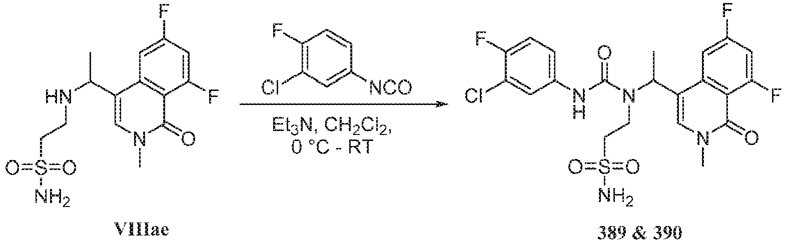

Racemic 2-(3-(3-chloro-4-fluorophenyl)-1-(1-(6,8-difluoro-1-oxo-1,2-dihydroisoquinolin-4-yl)ethyl)ureido) ethane-1-sulfonamide was synthesized in a similar manner as described previously from 2-(1-(6,8-difluoro-1-oxo-1,2-dihydroisoquinolin-4-yl)ethylamino) ethanesulfonamide (**VIIIae**) and 2-chloro-1-fluoro-4-isocyanatobenzene. The enantiomers were subsequently separated by chiral SFC, Column: Chiralpak IC (250 × 30 mm) 5 µ, 70% CO₂/MeOH, Flow rate 100 g/min.

2-(3-(3 -Chloro-4-fluorophenyl)-1-(1-(6,8-difluoro-1-oxo-1,2-dihydroisoquinolin-4-yl)ethyl)ureido) ethane-1-sulfonamide - Enantiomer I (**Compound 389**), LCMS: *m*/*z* found 503.2/505.1 [M+H]⁺, RT = 4.11 min (Method A); ¹H NMR (400 MHz, DMSO-d₆): δ 11.50 (bs, 1H), 8.68 (bs, 1H), 7.74-7.77 (m, 1H), 7.43-7.48 (m, 1H), 7.30-7.38 (m, 3H), 7.19 (d, 1H), 6.86 (br s, 2H), 5.61-5.65 (m, 1H), 3.39-3.51 (m, 2H), 3.03-3.10 (m, 1H), 2.60-2.73 (m, 1H), 1.50 (d, 3H); Chiral analytical SFC: RT = 1.63 min, Column: Chiralpak IC-3 (4.6 x 150 mm) 3 µm, 75% CO₂/MeOH, Flow = 3.0 g/min.
2-(3-(3-Chloro-4-fluorophenyl)-1-(1-(6,8-difluoro-1-oxo-1,2-dihydroisoquinolin-4-yl)ethyl)ureido) ethane-1-sulfonamide - Enantiomer II (**Compound 390**), LCMS: *m*/*z* found 503.2/505.1 [M+H]⁺, RT = 4.11 min (Method A); ¹H NMR (400 MHz, DMSO-d₆): δ 11.50 (bs, 1H), 8.68 (bs, 1H), 7.74-7.77 (m, 1H), 7.43-7.48 (m, 1H), 7.30-7.38 (m, 3H), 7.19 (d, 1H), 6.86 (br s, 2H), 5.61-5.65 (m, 1H), 3.39-3.51 (m, 2H), 3.03-3.10 (m, 1H), 2.60-2.73 (m, 1H), 1.50 (d, 3H); Chiral analytical SFC: RT = 2.30 min, Column: Chiralpak IC-3 (4.6 x 150 mm) 3 µm, 75% CO₂/MeOH, Flow = 3.0 g/min.

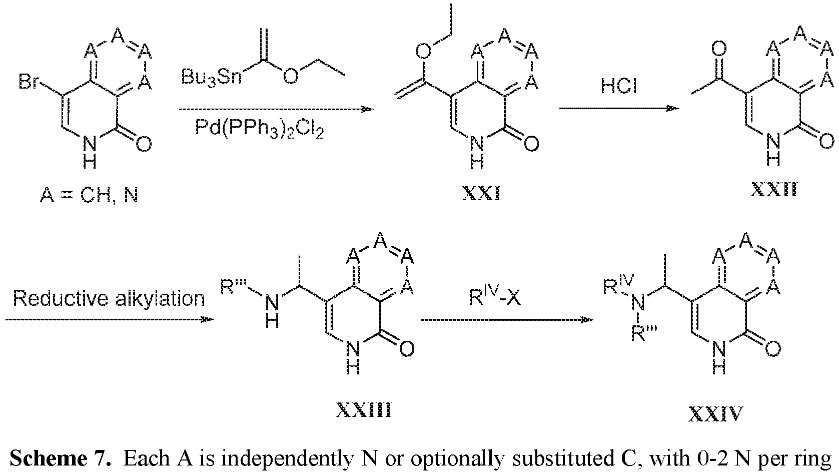

### 4-(1-Ethoxyvinyl)-2,7-naphthyridin-1(2H)-one (XXIa)

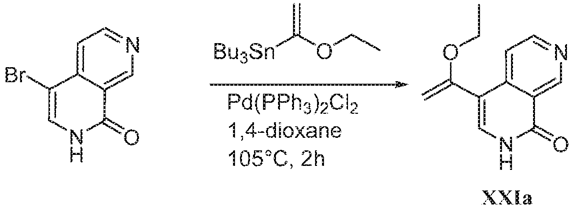

A suspension of 0.3 g (1.33 mmol, 1.0 eq.) of 4-bromo-2*H*-2,7-naphthyridin-1-one in 7 mL of anhydrous 1,4-dioxane was degassed with nitrogen for 5 minutes and 0.12 g (0.17 mmol, 0.13 eq.) of dichlorobis(triphenylphosphine)palladium (II) and 0.58 mL (1.73 mmol, 1.3 eq.) of tributyl(1-ethoxyvinyl)stannane were added. The mixture was then heated at 105 °C under a nitrogen atmosphere for 2 h. The mixture was allowed to cool to room temperature, diluted with 15 ml of ethyl acetate, and filtered through a pad of CELITE^{®}. The pad was washed with 40 mL of ethyl acetate and the combined filtrates were evaporated, absorbed on CELITE^{®}, and purified by flash chromatography (24 g SiO₂, eluting with 0-7% methanol/methylene chloride) to provide 0.22 g (0.98 mmol, 74 %) of (4-(1-ethoxyvinyl)-2*H-*2,7-naphthyridin-1-one (**XXIa**). ¹H NMR (400 MHz, DMSO-d₆) δ 11.76 (s, 1H), 9.33 (d, 1H), 8.73 (d, 1H), 7.64 (m, 1H), 7.41-7.47 (m, 1H), 4.40 (m, 2H), 3.92 (m, 2H), 1.31 (t, 3H).

### 4-Acetyl-2,7-naphthyridin-1(2H)-one (XXIIa)

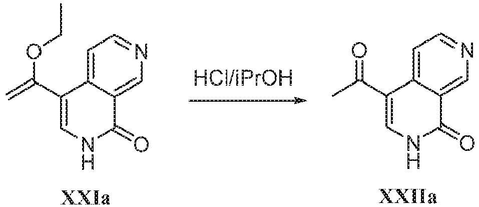

To a solution of 0.21 g (0.97 mmol, 1.0 eq.) of 4-(1-ethoxyvinyl)-2*H*-2,7-naphthyridin-1-one (**XXIa**) in 4 mL of 2-propanol was added 1.2 mL (2.42 mmol, 2.5 eq.) of a 2 M aqueous solution of hydrochloric acid and the mixture was stirred at room temperature for 10 mins. The mixture mixture was cooled in an ice bath and the resulting precipitate was collected by vacuum filtration, washed with 5 mL of acetone, and dried under vacuum to provide 0.22 g of 4-acetyl-2*H*-2,7-naphthyridin-1-one hydrochloride salt (**XXIIa**). ¹H NMR (400 MHz, DMSO-d₆) δ 12.75 (s, 1H), 9.45 (d, 1H), 8.96 (m, 1H), 8.85 (d, 1H), 8.57 (d, 1H), 2.57 (s, 3H).

### 4-(1-(Ethylamino)ethyl)-2,7-naphthytidin-1(2H)-one (XXIIIa)

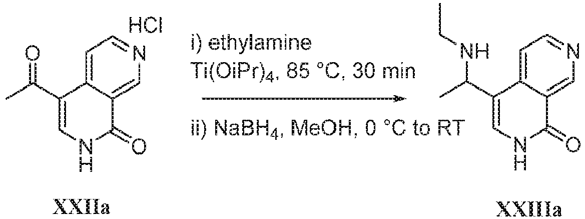

To a mixture of 35 mg (0.19 mmol, 1.0 eq.) of 4-acetyl-2*H-*2,7-naphthyridin-1-one hydrochloride salt (**XXIIa**) and 0.93 mL (1.86 mmol, 10.0 eq.) of a 2 M solution of ethylamine solution THF in a microwave vial was added 0.55 mL (1.86 mmol, 10.0 eq.) of titanium (IV) isopropoxide. The mixture was subjected to microwave irradiation maintaining a temperature of 85 °C for 30 min. The reaction mixture was diluted with 0.3 mL of methanol, cooled in an ice bath and 14 mg (0.37 mmol, 2.0 eq.) of sodium borohydride was added. The mixture was allowed to warm to room temperature and stirred for 90 min. The reaction mixture was then slowly added to a 3 mL of a stirred brine solution, diluted with 20 mL of ethyl acetate and filtered through CELITE^{®}. The pad was washed with 15 mL of ethyl acetate and the combined filtrate was dried (Na₂SO₄), filtered and the solvent was removed *in vacuo* to provide 29 mg of crude 4-[1-(ethylamino)ethyl]-2*H*-2,7-naphthyridin-1-one (**XXIIIa**).

### 3-(3-Chloro-4-fluorophenyl)-1-ethyl-1-(1-(1-oxo-1,2-dihydro-2,7-naphthyridin-4-yl)ethyl)urea (Compounds 102, 151 & 152)

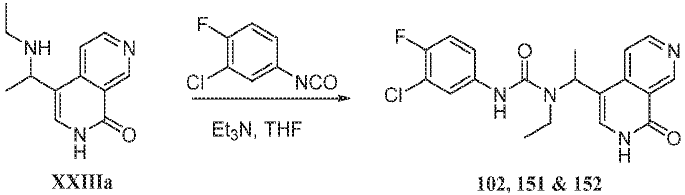

A solution of 14 µL (0.12 mmol, 1.0 eq.) of 2-chloro-1-fluoro-4-isocyanato-benzene in 0.5 mL of THF was added to a suspension of 28 mg (0.13 mmol, 1.1 eq.) of 4-[1-(ethylamino)ethyl]-2*H*-2,7-naphthyridin-1-one (**XXIIIa**) and 18 µL (0.13 mmol, 1.1 eq.) of triethylamine in 1 mL of THF and the mixture was stirred at room temperature for 5 min. The reaction mixture was then loaded on a pre-equilibrated 12 g silica column, and purified by flash chromatography (12 g SiO₂, eluting with 0.5-9.5% methanol/methylene chloride) to provide 23 mg of racemic 3-(3-chloro-4-fluorophenyl)-1-ethyl-1-(1-(1-oxo-1,2-dihydro-2,7-naphthyridin-4-yl)ethyl)urea (**Compound 102**). The enantiomers were subsequently separated by SFC, Column: Chiralpak AD-H (10 × 250 mm), 5 µ, 70% CO₂:MeOH/MeCN (1:1), Flow rate 9.0 g/min.

3-(3-Chloro-4-fluorophenyl)-1-ethyl-1-(1-(1-oxo-1,2-dihydro-2,7-naphthyridin-4-yl)ethyl)urea: Enantiomer I (**Compound 151**). LCMS: *m*/*z* found 389.2/391.2 [M+H]⁺, RT = 5.25 min (Method A); ¹H NMR (400 MHz, DMSO-d₆) δ 11.80 (s, 1H), 9.34 (d, 1H), 8.76 (m, 1H), 8.38 (s, 1H), 7.86 (m, 1H), 7.49-7.61 (m, 2H), 7.46 (s, 1H), 7.32 (m, 1H), 5.83 (m, 1H), 3.15 (m, 2H), 1.45 (d, 3H), 0.67 (t, 3H); Chiral analytical SFC: RT = 2.95 min, Column: Chiralpak AD-H (4.6 x 250 mm) 5 µ, 70% CO₂: MeOH/MeCN (1:1), Flow rate = 3.0 g/min.

3-(3-Chloro-4-fluorophenyl)-1-ethyl-1-(1-(1-oxo-1,2-dihydro-2,7-naphthyridin-4-yl)ethyl)urea: Enantiomer II (**Compound 152**). LCMS: *m*/*z* found 389.2/391.2 [M+H]⁺, RT = 5.25 min (Method A); ¹H NMR (400 MHz, DMSO-d₆) δ 11.80 (s, 1H), 9.34 (d, 1H), 8.76 (m, 1H), 8.38 (s, 1H), 7.86 (m, 1H), 7.49-7.61 (m, 2H), 7.46 (s, 1H), 7.32 (m, 1H), 5.83 (m, 1H), 3.15 (m, 2H), 1.45 (d, 3H), 0.67 (t, 3H). Chiral analytical SFC: RT = 3.55 min, Column: Chiralpak AD-H (4.6 × 250 mm) 5 µ, 70% CO₂: MeOH/MeCN (1:1), Flow rate = 3.0 g/min.

### 3-(3-Chloro-4-fluorophenyl)-1-methyl-1-(1-(1-oxo-1,2-dihydro-2,7-naphthyridin-4-yl)ethyl)urea (Compound 111)

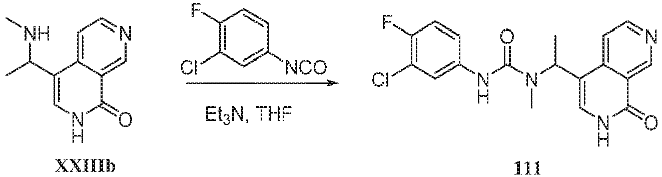

3-(3-Chloro-4-fluorophenyl)-1-methyl-1-(1-(1-oxo-1,2-dihydro-2,7-naphthyridin-4-yl)ethyl)urea (**Compound 111**) was synthesized in an analogous manner as described above from racemic 4-(1-(methylamino)ethyl)-2,7-naphthyridin-1(2*H*)-one (**XXIIIb**) and 2-chloro-1-fluoro-4-isocyanato-benzene. LCMS: *m*/*z* found 375.2/377.2 [M+H]⁺, RT = 5.23 min (Method A); ¹H NMR (400 MHz, DMSO-d₆) δ 11.77 (s, 1H), 9.34 (s, 1H), 8.76 (m, 1H), 8.48 (s, 1H), 7.86 (m, 1H), 7.56 (d, 1H), 7.50 (m, 1H), 7.40 (s, 1H), 7.32 (m, 1H), 5.82 (m, 1H), 2.60 (d, 3H), 1.43 (d, 3H).

### 3-(3-Chloro-4-fluorophenyl)-1-isobutyl-1-(1-(1-oxo-1,2-dihydro-2,7-naphthyridin-4-yl)ethyl)urea (Compounds 154, 253 & 254)

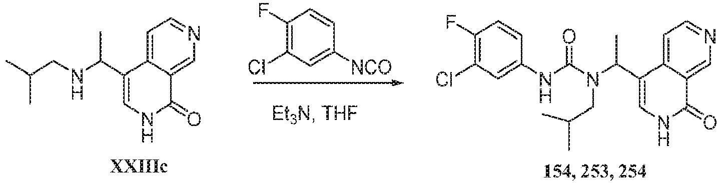

Racemic 3-(3-chloro-4-fluorophenyl)-1-isobutyl-1-(1-(1-oxo-1,2-dihydro-2,7-naphthyridin-4-yl)ethyl)urea (**Compound 154**) was synthesized in an analogous manner as detailed above from racemic 4-(1-(isobutylamino)ethyl)-2,7-naphthyridin-1(2*H*)-one (**XXIIIc**). LCMS: *m*/*z* found 417.3/419.3 [M+H]⁺, RT = 6.39 min (Method A); ¹H NMR (400 MHz, DMSO-d₆) δ 11.78 (m, 1H), 9.34 (d, 1H), 8.78 (m, 1H), 8.42 (s, 1H), 7.81 (m, 1H), 7.64-7.71 (m, 1H), 7.44-7.53 (m, 2H), 7.33 (t, 1H), 5.83 (d, 1H), 3.00 (m, 1H), 2.88 (m, 1H), 1.47 (d, 3H), 1.36 (m, 1H), 0.63 (d, 3H), 0.45 (d, 3H). The enantiomers were subsequently separated by chiral SFC, Column: Chiralpak IC (250 × 30 mm) 5 µ, 70% CO₂/MeOH, Flow rate 100 g/min.

3-(3-Chloro-4-fluoropheny1)-1-isobutyl-1-(1-(1-oxo-1,2-dihydro-2,7-naphthyridin-4-yl)ethyl)urea - Enantiomer I (**Compound 253**), LCMS: *m*/*z* found 417.3/419.3 [M+H]⁺; ¹H NMR (400 MHz, DMSO-d₆) δ 11.78 (m, 1H), 9.34 (d, 1H), 8.78 (m, 1H), 8.42 (s, 1H), 7.81 (m, 1H), 7.64-7.71 (m, 1H), 7.44-7.53 (m, 2H), 7.33 (t, 1H), 5.83 (d, 1H), 3.00 (m, 1H), 2.88 (m, 1H), 1.47 (d, 3H), 1.36 (m, 1H), 0.63 (d, 3H), 0.45 (d, 3H); Chiral analytical SFC: RT = 2.39 min, Column: Chiralpak IC, (4.6 x 150 mm) 3 µm, 70% CO₂/MeOH, Flow = 3.0 g/min.

3-(3-Chloro-4-fluorophenyl)-1-isobutyl-1-(1-(1-oxo-1,2-dihydro-2,7-naphthyridin-4-yl)ethyl)urea- Enantiomer II (**Compound 254**), LCMS: *m*/*z* found 417.3/419.3 [M+H]⁺; ¹H NMR (400 MHz, DMSO-d₆) δ 11.78 (m, 1H), 9.34 (d, 1H), 8.78 (m, 1H), 8.42 (s, 1H), 7.81 (m, 1H), 7.64-7.71 (m, 1H), 7.44-7.53 (m, 2H), 7.33 (t, 1H), 5.83 (d, 1H), 3.00 (m, 1H), 2.88 (m, 1H), 1.47 (d, 3H), 1.36 (m, 1H), 0.63 (d, 3H), 0.45 (d, 3H); Chiral analytical SFC: RT = 3.29 min, Column: Chiralpak IC, (4.6 × 150 mm) 3 µm, 70% CO₂/MeOH, Flow = 3.0 g/min.

### 3-(3-Chloro-4-fluorophenyl)-1-(3-hydroxypropyl)-1-(1-(1-oxo-1,2-dihydro-2,7-naphthyridin-4-yl)ethyl)urea (Compounds 159, 251 & 252)

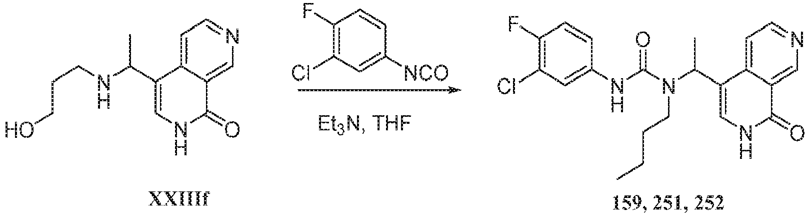

Racemic 3-(3-chloro-4-fluorophenyl)-1-(3-hydroxypropyl)-1-(1-(1-oxo-1,2-dihydro-2,7-naphthyridin-4-yl)ethyl)urea (**Compound 159**) was synthesized in an analogous manner as detailed above from racemic 4-(1-((3-hydroxypropyl)amino)ethyl)-2,7-naphthyridin-1(2*H*)-one (**XXIIIf**). The enantiomers were subsequently separated by chiral SFC, Column: Chiralpak IC-3 (150 × 30 mm) 3 µ, 70% CO₂/MeOH, Flow rate 90 g/min.

3-(3-Chloro-4-fluorophenyl)-1-(3-hydroxypropyl)-1-(1-(1-oxo-1,2-dihydro-2,7-naphthyridin-4-yl)ethyl)urea- Enantiomer I (**Compound 251**), LCMS: *m*/*z* found 419.3/421.3 [M+H]⁺, RT = 5.69 min (Method A); ¹H NMR (400 MHz, DMSO-d₆) δ 11.79 (bs, 1H), 9.31 (s, 1H), 8.76 (d, 1H), 8.72 (bs, 1H), 7.78-7.82 (m, 1H), 7.52-7.54 (m, 1H), 7.41-7.45 (m, 2H), 7.33 (t, 1H), 5.81-5.82 (m, 1H), 5.02 (bs, 1H), 3.11-3.19 (m, 4H), 1.46 (d, 3H), 1.11-1.16 (m, 2H); Chiral analytical SFC: RT = 2.80 min, Column: Chiralpak IC, (4.6 × 150 mm) 3 µm, 70% CO₂/MeOH, Flow = 3.0 g/min.

3-(3-Chloro-4-fluorophenyl)-1-(3-hydroxypropyl)-1-(1-(1-oxo-1,2-dihydro-2,7-naphthyridin-4-yl)ethyl)urea - Enantiomer II (**Compound 252**), LCMS: *m*/*z* found 419.3/421.3 [M+H]⁺, RT = 5.69 min (Method A); ¹H NMR (400 MHz, DMSO-d₆) δ 11.79 (bs, 1H), 9.31 (s, 1H), 8.76 (d, 1H), 8.72 (bs, 1H), 7.78-7.82 (m, 1H), 7.52-7.54 (m, 1H), 7.41-7.45 (m, 2H), 7.33 (t, 1H), 5.81-5.82 (m, 1H), 5.02 (bs, 1H), 3.11-3.19 (m, 4H), 1.46 (d, 3H), 1.11-1.16 (m, 2H); Chiral analytical SFC: RT = 3.78 min, Column: Chiralpak IC, (4.6 × 150 mm) 3 µm, 70% CO₂/MeOH, Flow = 3.0 g/min.

### 3-(3-Chloro-4-fluorophenyl)-1-methyl-1-(1-(1-oxo-1,2-dihydro-2,6-naphthyridin-4-yl)ethyl)urea (Compound 127)

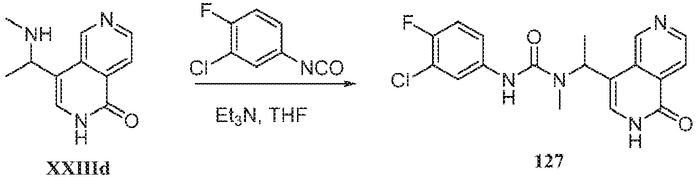

3-(3-Chloro-4-fluorophenyl)-1-methyl-1-(1-(1-oxo-1,2-dihydro-2,6-naphthyridin-4-yl)ethyl)urea (**Compound 127**) was synthesized in an analogous manner as described above from racemic 4-(1-(methylamino)ethyl)-2,6-naphthyridin-1(2*H*)-one (**XXIIId**). LCMS: *m*/*z* found 375.2/377.2 [M+H]⁺, RT = 5.13 min (Method A); ¹H NMR (400 MHz, DMSO-d₆) δ 11.76 (s, 1H), 9.07 (s, 1H), 8.67 (m, 1H), 8.50 (s, 1H), 8.03 (m, 1H), 7.85 (m, 1H), 7.51 (m, 1H), 7.33 (m, 1H), 7.26 (s, 1H), 5.97 (d, 1H), 2.61 (d, 3H), 1.45 (d, 3H).

### 3-(3-Chloro-4-fluorophenyl)-1-methyl-1-(1-(8-oxo-7,8-dihydro-1,7-naphthyridin-5-yl)ethyl)urea (Compound 153)

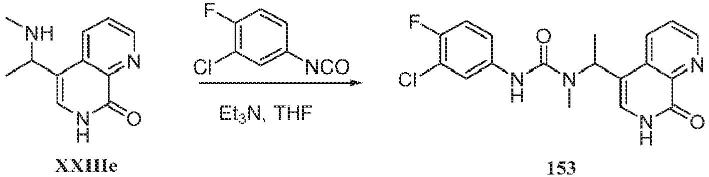

3-(3-Chloro-4-fluorophenyl)-1-methyl-1-(1-(8-oxo-7,8-dihydro-1,7-naphthyridin-5-yl)ethyl)urea (**Compound 153**) was synthesized in an analogous manner as described above from racemic 5-(1-(methylamino)ethyl)-1,7-naphthyridin-8(7*H*)-one5-(1-(methylamino)ethyl)-1,7-naphthyridin-8(7*H*)-one (**XXIIIe**). LCMS: *m*/*z* found 375.2/377.2 [M+H]⁺, RT = 6.10 min (Method A); ¹H NMR (400 MHz, DMSO-d₆) δ 11.97 (m, 1H), 8.84 (d, 1H), 8.51 (s, 1H), 8.30 (d, 1H), 7.82-7.95 (m, 2H), 7.51 (m, 1H), 7.31 (m, 2H), 6.25 (bs, 1H), 5.86 (m, 1H), 2.60 (s, 3H), 1.45 (d, 3H).

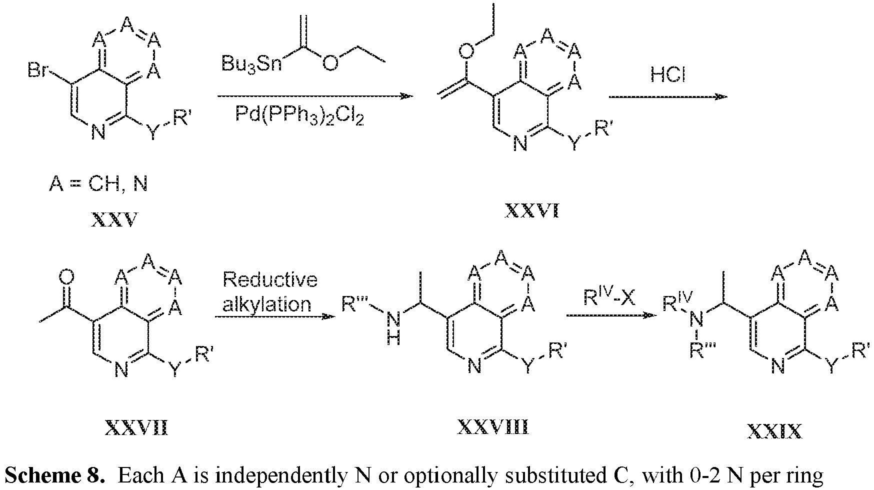

### 5,8-Dibromopyrido[3,4-b]pyrazine

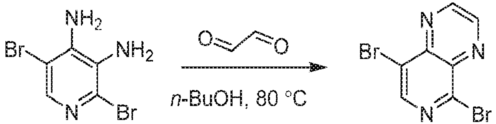

A suspension of 1.98 mL (17.2 mmol, 2.3 eq.) of 40% aqueous glyoxyl and 2.0 g (7.5 mmol, 1.0 eq.) of 2,5-dibromopyridine-3,4-diamine in 6 mL of n-butanol was heated to 80 °C. After reaching the desired temperature, an additional 2.5 mL of *n*-butanol was added and the mixture was heated at 80 °C for 2 h. The mixture was allowed to cool to room temperature and the resulting precipitate was collected by vacuum filtration, washed with 40 mL of petroleum ether and dried *in vacuo.* The material was redissolved in 40 mL of methylene chloride, dried (Na₂SO₄), filtered, and the solvent was removed *in vacuo* to provide 1.93 g 5,8-dibromopyrido[3,4-b]pyrazine (6.7 mmol, 92%). ¹H NMR (400 MHz, CDCl₃) δ 9.19 (m, 1H), 9.12 (m, 1H), 8.85 (m, 1H).

### 8-Bromo-5-methoxy-pyrido[3,4-b]pyrazine (XXVa)

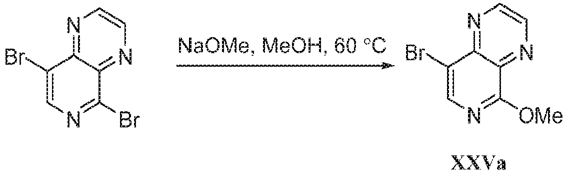

To a suspension of 1.52 g (5.26 mmol, 1.0 eq.) of 5,8-dibromopyrido[3,4-b]pyrazine in 20 mL of anhydrous methanol under a nitrogen atmosphere was added 0.43 g (7.89 mmol, 1.5 eq.) of sodium methoxide and the mixture was heated at 60 °C for 3 h. The mixture was then allowed to cool to room temperature and the solvent was removed *in vacuo.* The residue was redissolved in 70 mL of ethyl acetate and washed with 20 mL of brine. The organic phase was dried (Na₂SO₄), filtered, and the solvent was removed *in vacuo* to provide 1.25 g of 8-bromo-5-methoxy-pyrido[3,4-b]pyrazine (**XXVa**). ¹H NMR (400 MHz, CDCl₃) δ 9.12 (d, 1H), 8.95 (m, 1H), 8.54 (d, 1H), 4.24 (s, 3H).

### 8-(1-Ethoayvinyl)-5-methoxy-pyrido[3,4-b]pyrazine (XXVIa)

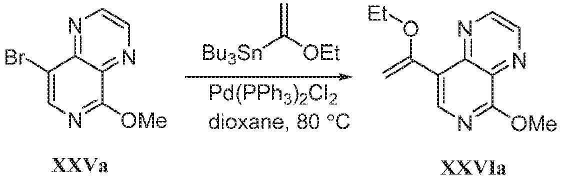

A solution of 0.4 g (1.66 mmol, 1.0 eq.) of 8-bromo-5-methoxy-pyrido[3,4-b]pyrazine (**XXVa**) in 10 mL of anhydrous 1,4-dioxane in a pressure vessel was degassed with nitrogen for 5 minutes and 0.73 mL (2.16 mmol, 1.3 eq.) of tributyl(1-ethoxyvinyl)stannane and 0.11 g (0.15 mmol, 0.09 eq.) of chlorobis(triphenylphosphine)palladium(II) were added. The vessel was sealed, and the mixture was heated at 100 °C for 1 h. The mixture was then allowed to cool to room temperature, diluted with 20 mL of ethyl acetate and filtered through CELITE^{®}. The pad was washed with 30 mL of ethyl acetate and the filtrates were evaporated onto CELITE^{®} and purified by flash chromatography (SiO₂, eluting with a gradient of 5-45% ethyl acetate/hexanes) to provide 0.31 g (1.5 mmol, 80%) of 8-(1-ethoxyvinyl)-5-methoxy-pyrido[3,4-b]pyrazine (**XXVIa**). ¹H NMR (400 MHz, DMSO-*d*₆) δ 9.16 (d, 1H), 9.00 (d, 1H), 8.42 (s, 1H), 4.91 (d, 1H), 4.62 (d, 1H), 4.11 (s, 3H), 3.93 (q, 2H), 1.31 (t, 3H).

### 1-(5-Methoxypyrido[3,4-b]pyrazin-8-yl)ethenone (XXVIIa)

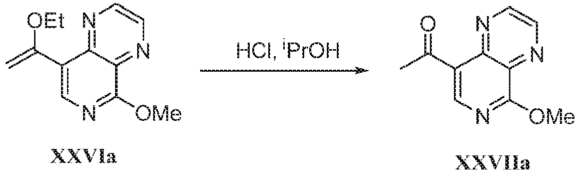

To a suspension of 0.15 g (0.65 mmol, 1.0 eq.) of 8-(1-ethoxyvinyl)-5-methoxy-pyrido[3,4-b]pyrazine (150 mg, 0.65 mmol) in 10.5 mL of isopropanol was added 0.81 mL of 2 M aqueous HCl and the mixture was stirred at room temperature for 20 min. The volatiles were removed *in vacuo* to provide 0.13 g of crude 1-(5-methoxypyrido[3,4-b]pyrazin-8-yl)ethenone (**XXVIIa**). ¹H NMR (400 MHz, DMSO-*d*₆) δ 9.24 (m, 1H), 9.02-9.11 (m, 1H), 8.64 (s, 1H), 4.17 (d, 3H), 2.82 (d, 3H).

### 1-(5-Methoxypyrido[3,4-b]pyrazin-8-yl)-N-methyl-ethanamine (XXVIIIa) and N-Methyl-8-(1-(methylamino)ethyl)pyrido[3,4-b]pyrazin-5-amine (XXVIIIb)

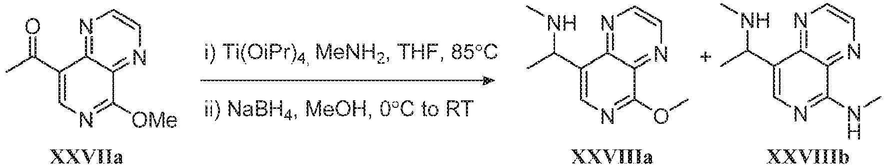

To a mixture of 0.13 g (0.62 mmol, 1.0 eq.) of 1-(5-methoxypyrido[3,4-b]pyrazin-8-yl)ethanone (**XXVIIa**) in 3.1 mL of a 2 M solution of methylamine in THF was added 1.82 mL (6.15 mmol, 10 eq.) of tetraisopropoxytitanium and the mixture was subjected to microwave irradiation, maintaining a reaction temperature 85 °C for 30 min. The mixture was diluted with 1.6 mL of methanol, cooled to 0 °C and 47 mg (1.23 mmol, 2.0 eq.) of sodium borohydride was added. After stirring for 25 min, the reaction mixture was slowly added to 0.75 mL of a stirred brine solution. The mixture was diluted with 20 mL of ethyl acetate and filtered through CELITE^{®}. The pad was washed with 15 mL of ethyl acetate and the combined filtrate was dried (Na₂SO₄), filtered and the solvent was removed *in vacuo.* The resulting crude product mixture comprising 0.13 g as a mixture of 1-(5-methoxypyrido[3,4-b]pyrazin-8-yl)-*N*-methyl-ethanamine (**XXVIIIa**) and *N*-methyl-8-(1-(methylamino)ethyl)pyrido[3,4-b]pyrazin-5-amine (**XXVIIIb)** was carried forward without further manipulation.

### 3-(3-chloro-4-fluorophenyl)-1-(1(5-methoxypyrido[3,4-b]pyrazin-8-yl)ethyl)-1-methylurea (Compound 137) and 3-(3-chloro-4-fluorophenyl)-1-methyl-1-(1-(5-(methylamino)pyrido[3,4-b]pyrazin-8-yl)ethyl)urea (Compound 136)

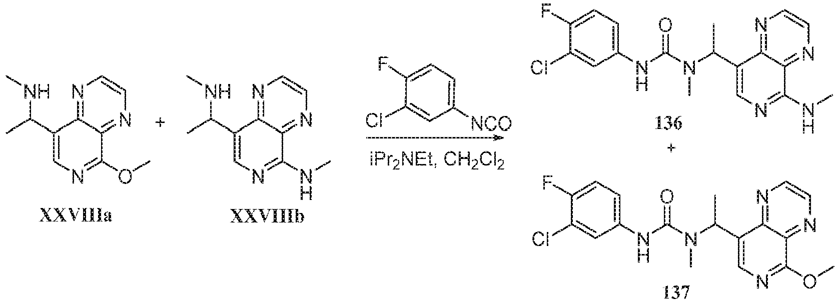

A solution of 28 uL (0.22 mmol) of 2-chloro-1-fluoro-4-isocyanato-benzene in 0.5 mL of methylene chloride was added to a 0 °C suspension of a crude mixture of 54 mg of 1-(5-methoxypyrido[3,4-b]pyrazin-8-yl)-*N*-methyl-ethanamine (**XXVIIIa**) and *N*-methyl-8-(1-(methylamino)ethyl)pyrido[3,4-b]pyrazin-5-amine (**XXVIIIb**) in 1.5 mL of methylene chloride. After 5 min, the crude reaction mixture was loaded onto a preconditioned 12 g silica column, and the products were separated by flash chromatography (SiO₂, eluting with a gradient of 10-80% ethyl acetate/hexanes). The first eluting product was repurified by semi-preparative HPLC to provide 8.5 mg (9%) of racemic 3-(3-chloro-4-fluorophenyl)-1-(1-(5-methoxypyrido[3,4-b]pyrazin-8-yl)ethyl)-1-methylurea (**Compound 137**). LCMS: *m*/*z* found 390.2/392.2 [M+H]+, RT = 6.50 min (Method A); ¹H NMR (400 MHz, CDCl₃) δ 9.29-9.35 (m, 1H), 9.04-9.12 (m, 2H), 8.44 (s, 1H), 7.56 (m, 1H), 7.24-7.32 (m, 1H), 7.10 (t, 1H), 6.06 (q, 1H), 4.28 (s, 3H), 2.78 (s, 3H), 1.77 (d, 3H).

The second eluting product was repurified by flash chromatography (SiO₂, eluting with a gradient of 1-4% methanol/methylene chloride) to provide 17 mg (38%) of racemic 3-(3-chloro-4-fluoro-phenyl)-1-methyl-1-[1-[5-(methylamino)pyrido[3,4-b]pyrazin-8-yl]ethyl]urea (**Compound 136**). LCMS: *m*/*z* found 389.2/391.3 [M+H]+, RT = 5.44 min (Method A); ¹H NMR (400 MHz, CDCl₃) δ 9.56 (s, 1H), 8.98 (d, 1H), 8.77 (d, 1H), 8.35 (s, 1H), 7.59 (m, 1H), 7.29 (m, 1H), 7.08 (t, 1H), 6.90 (d, 1H), 5.85-5.96 (m, 1H), 3.23 (d, 3H), 2.77 (s, 3H), 1.70 (d, 3H).

### 3-(3-Chloro-4-fluorophenyl)-1-methyl-1-(1-(5-oxo-5,6-dihydropyrido[3,4-b]pyrazin-8-yl)ethyl)urea (Compound 148)

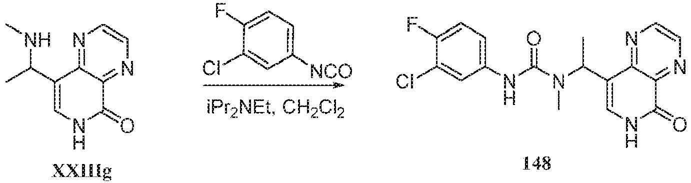

Racemic 3-(3-chloro-4-fluorophenyl)-1-methyl-1-(1-(5-oxo-5,6-dihydropyrido[3,4-b]pyrazin-8-yl)ethyl)urea (**Compound 148**) was synthesized in an similar manner as described above from 8-(1-(methylamino)ethyl)pyrido[3,4-b]pyrazin-5(6*H*)-one (**XXIIIg**) and 2-chloro-1-fluoro-4-isocyanatobenzene. LCMS: *m*/*z* found 376.2/378.2 [M+H]⁺, RT = 5.97 min (Method A); ¹H NMR (400 MHz, DMSO-*d*₆) δ 11.93 (d, 1H), 8.96 (d, 1H), 8.82 (d, 1H), 8.64 (s, 1H), 7.81 (dd, 1H), 7.39-7.50 (m, 2H), 7.29 (t, 1H), 5.87 (q, 1H), 2.63 (s, 3H), 1.50 (d, 3H).

### EXAMPLE 2: BIOLOGICAL RESULTS

Representative compounds of the invention were tested for their abilities to inhibit formation of relaxed circular DNA (rcDNA) in a HepDE19 assay, as described elsewhere herein. Results are illustrated in Table 4.

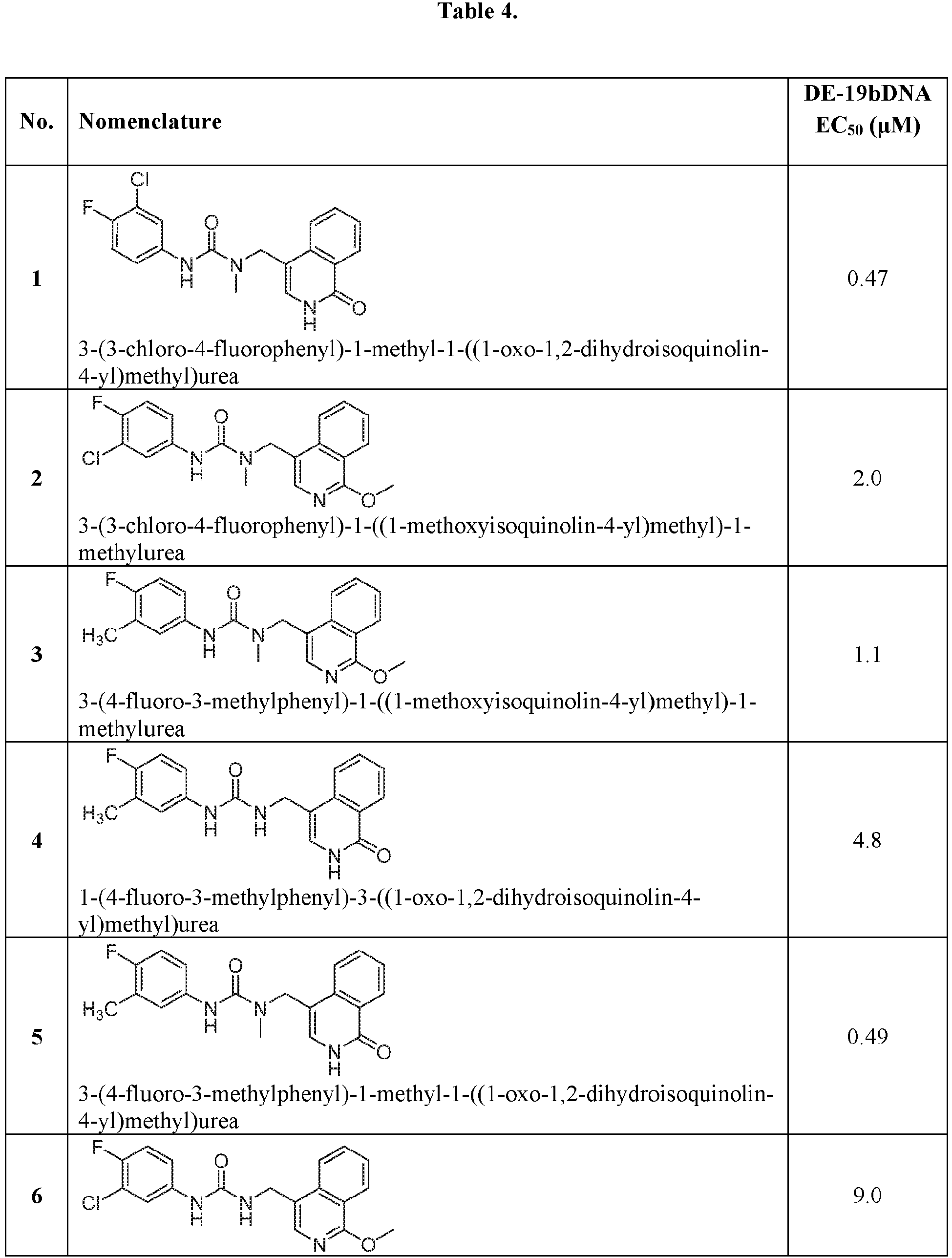

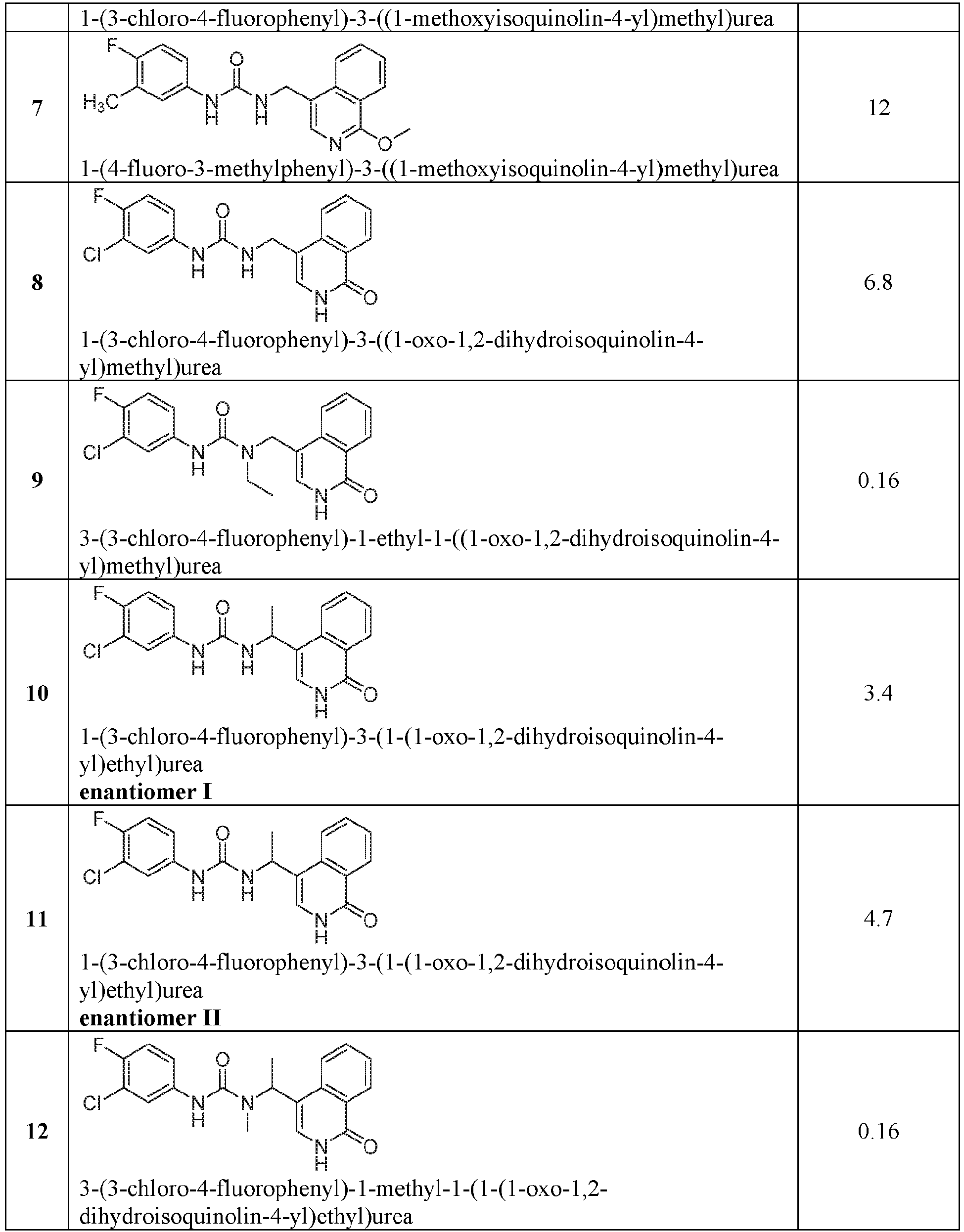

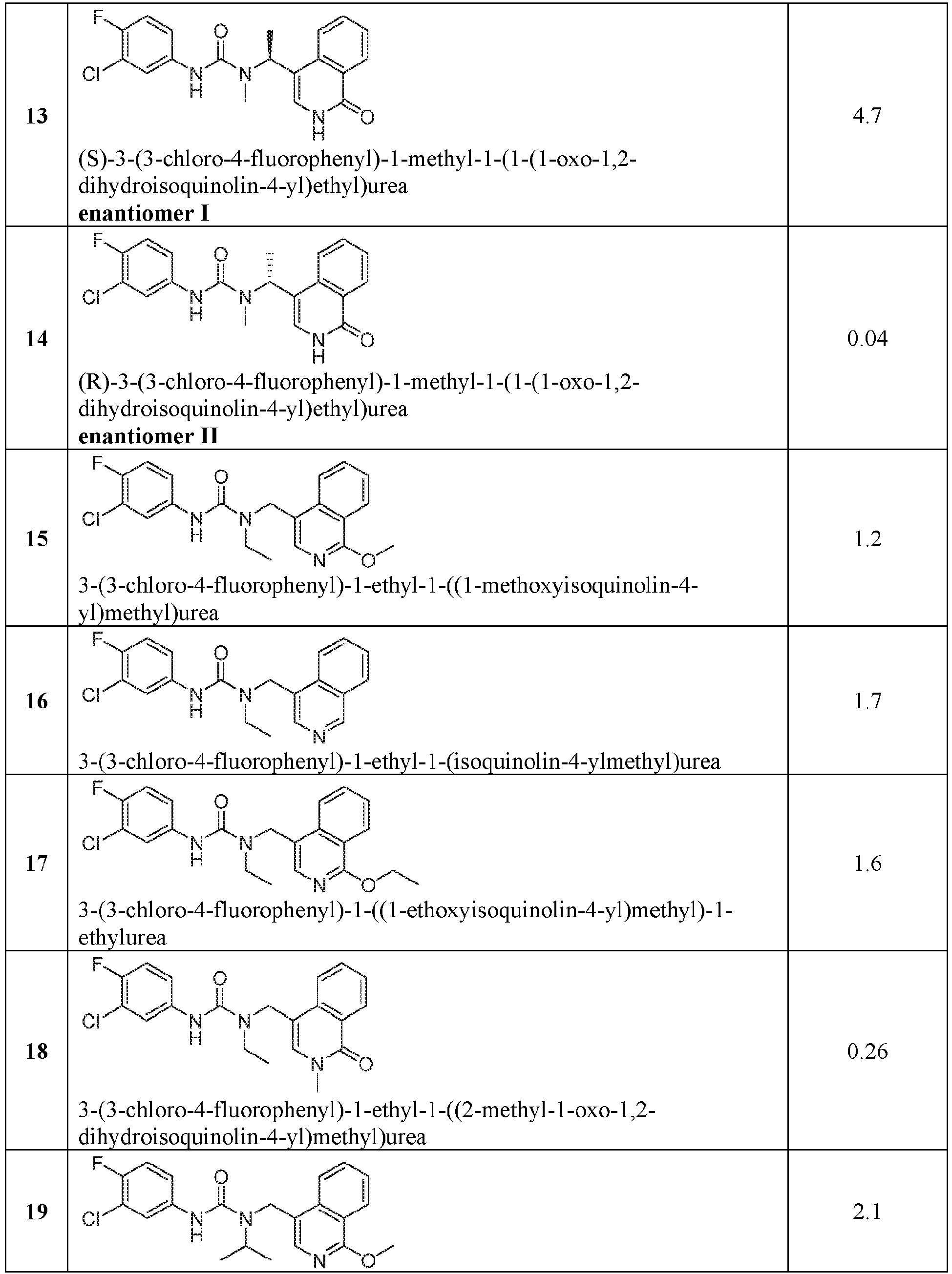

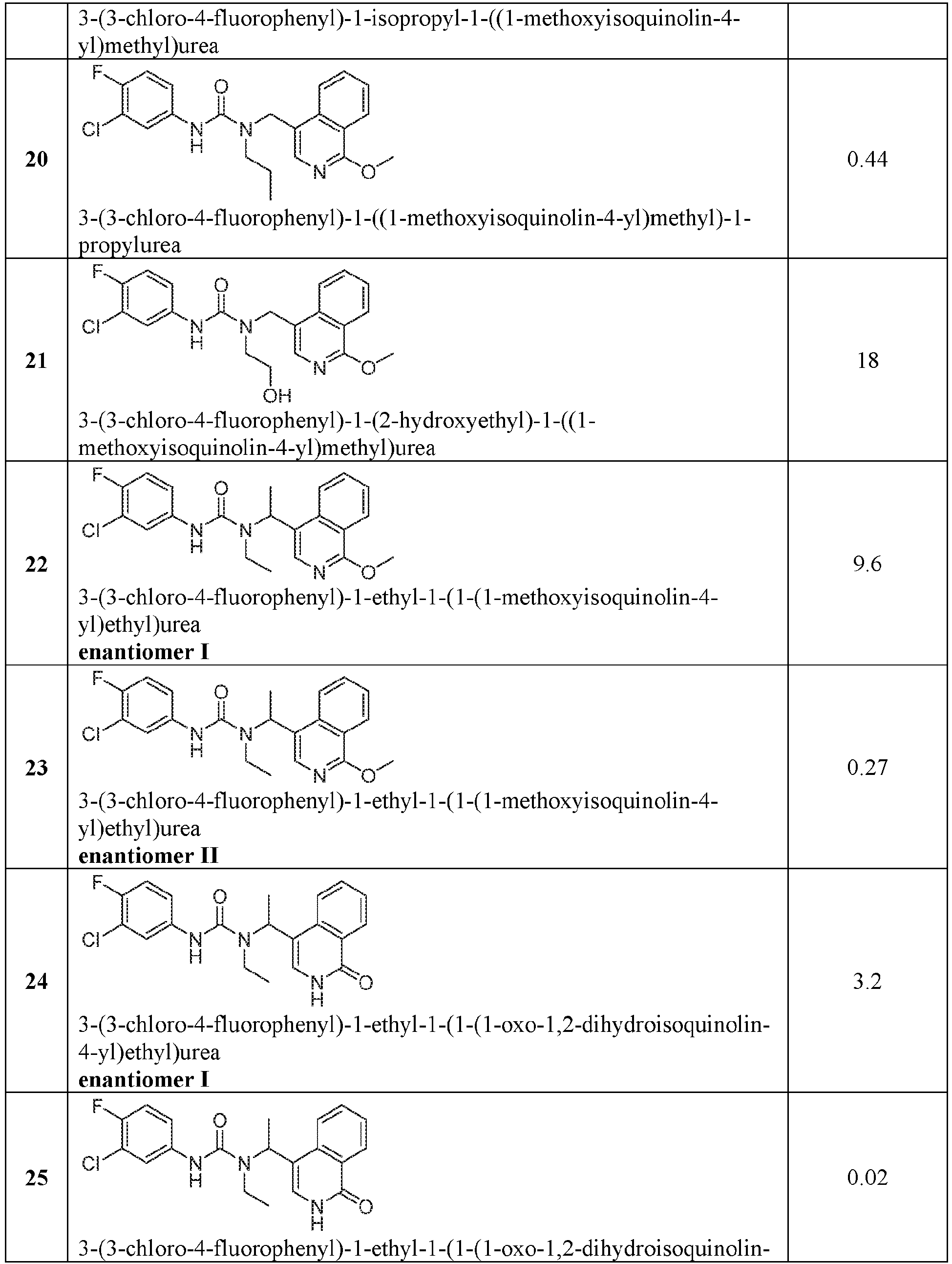

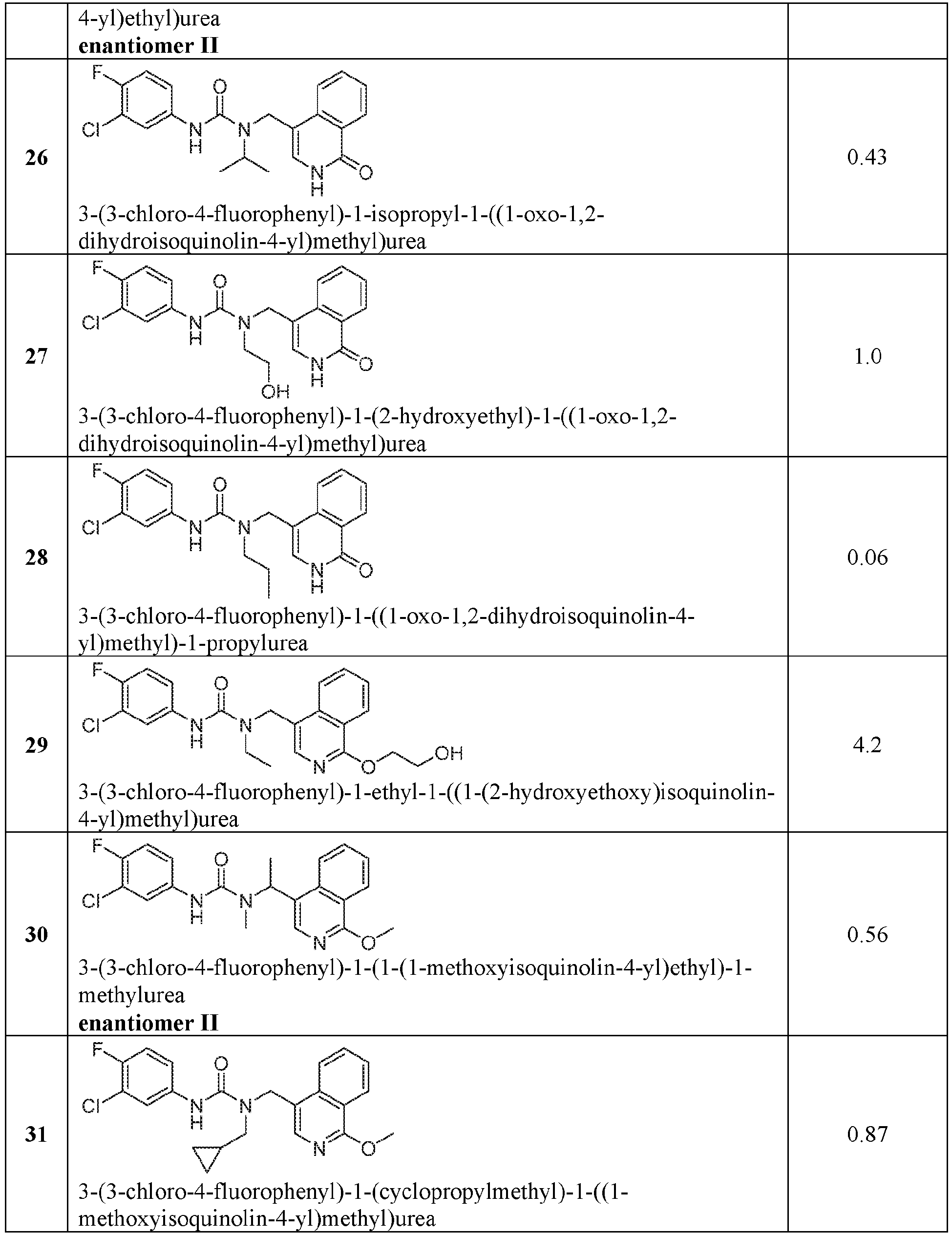

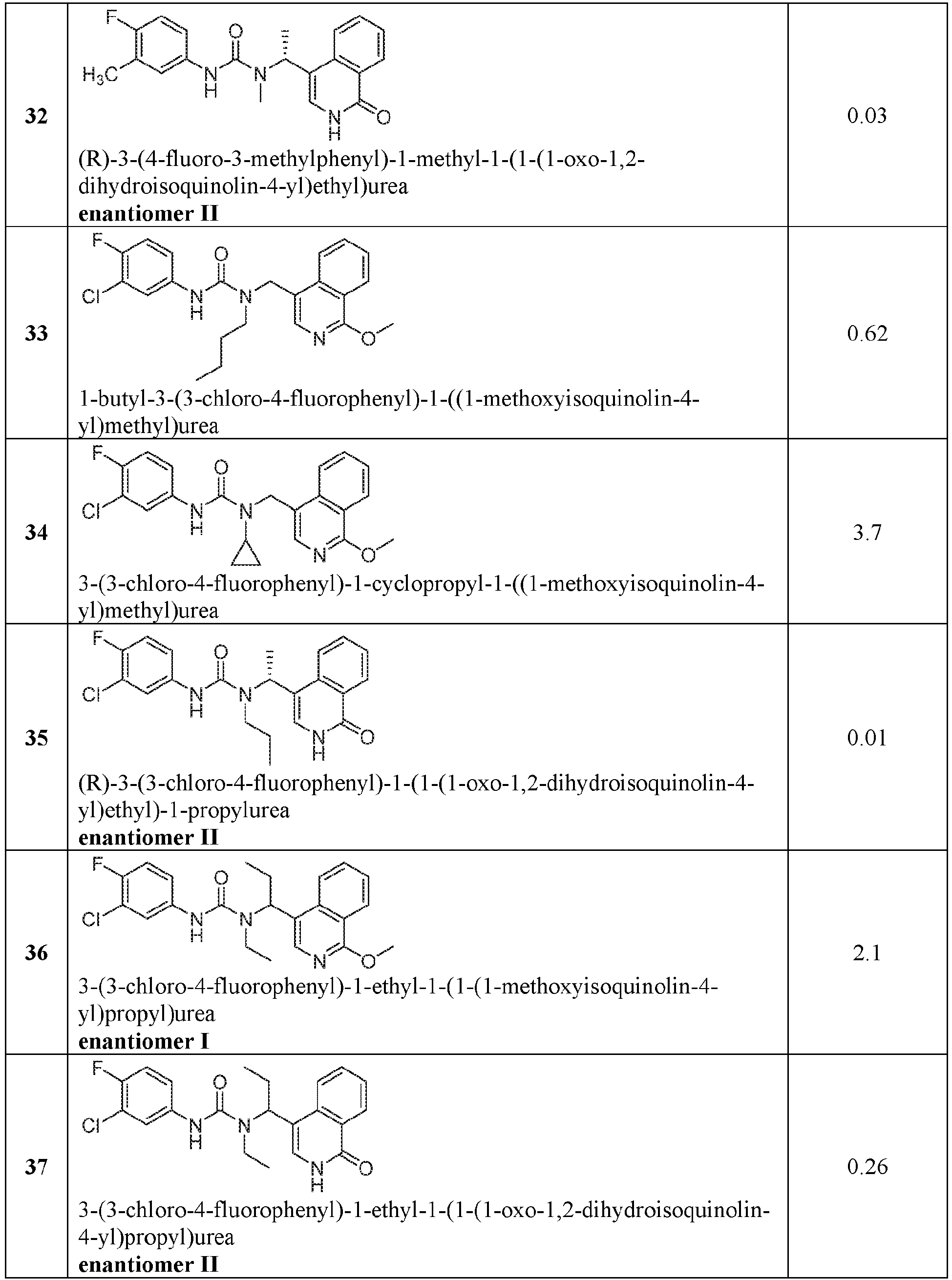

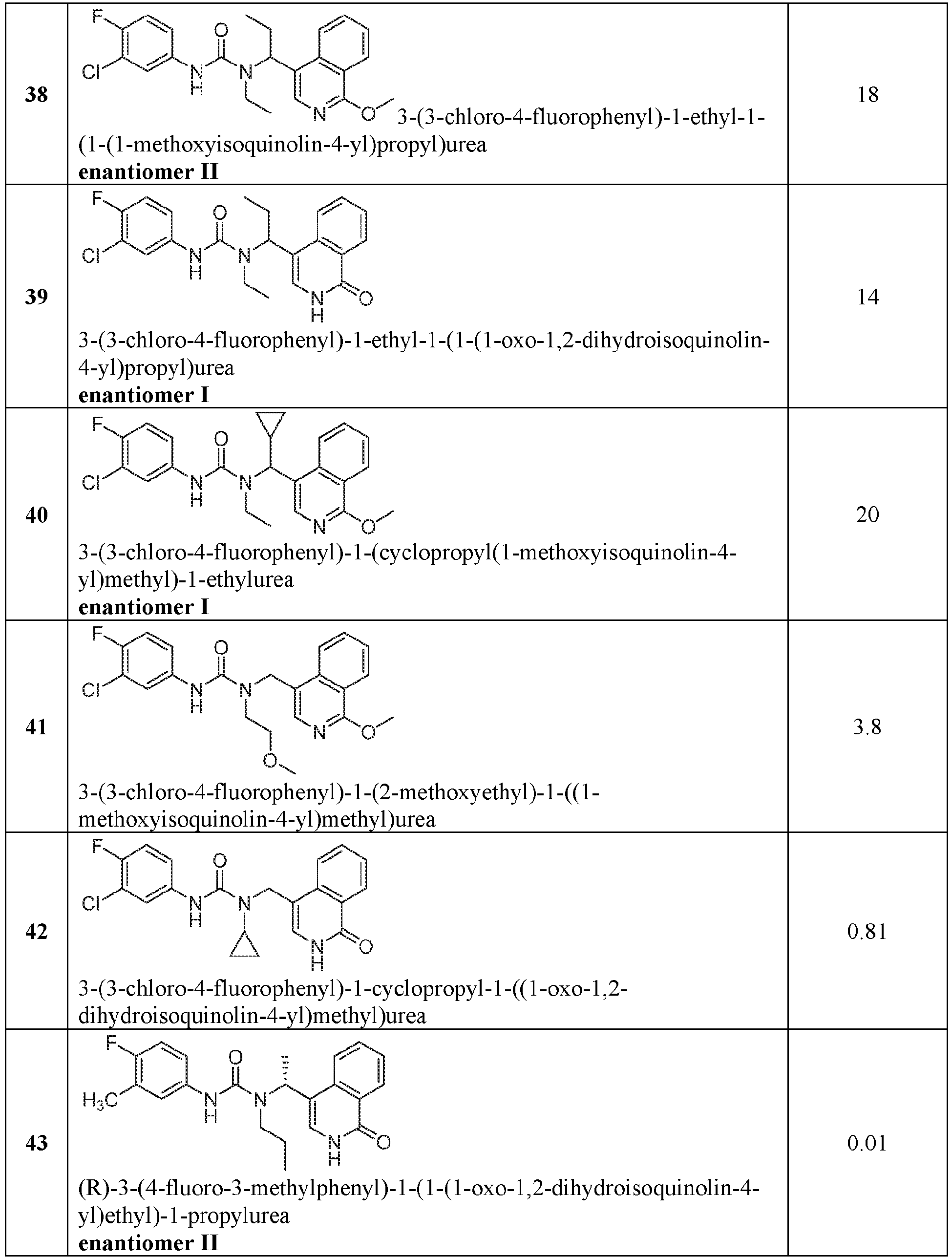

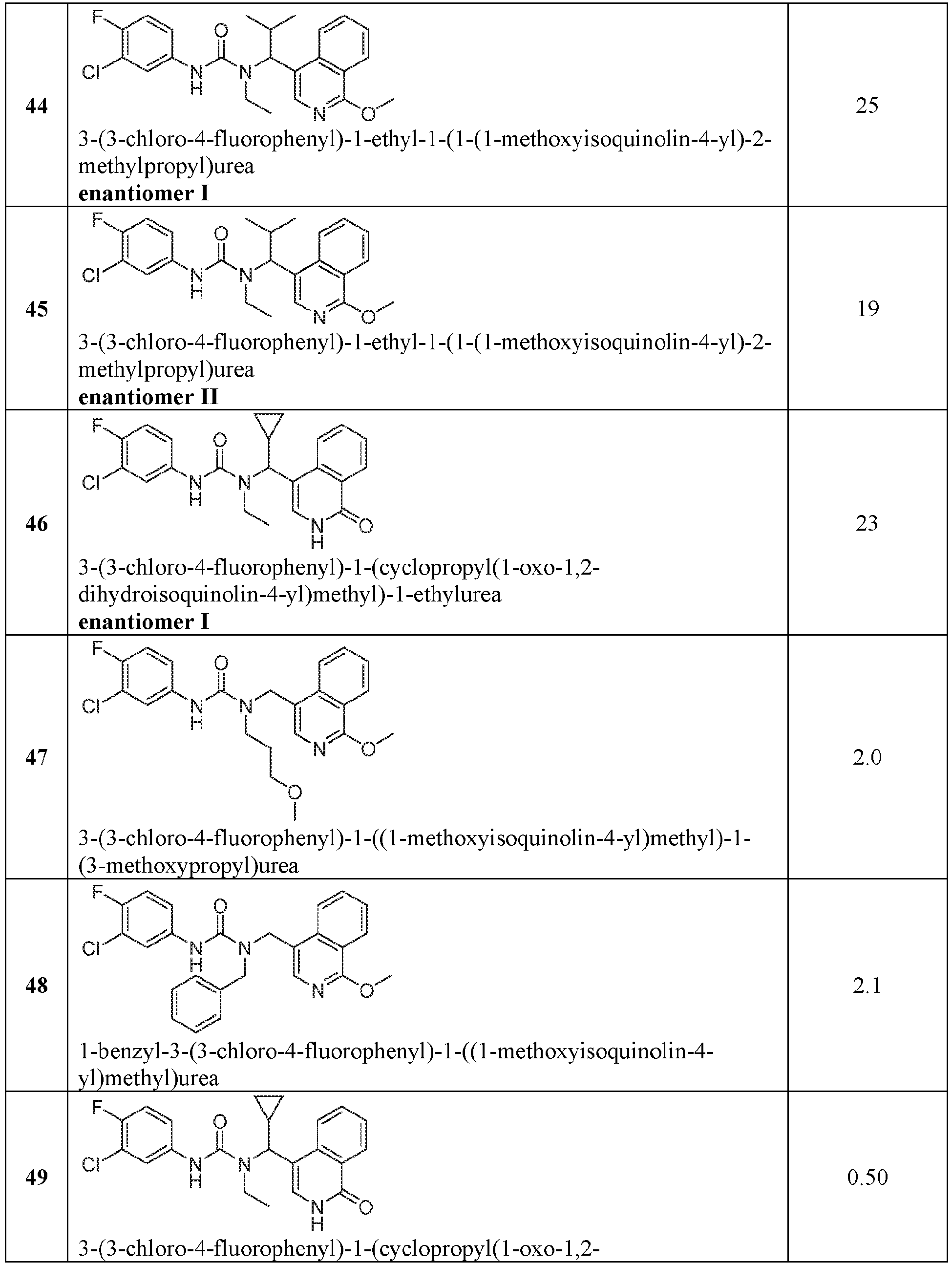

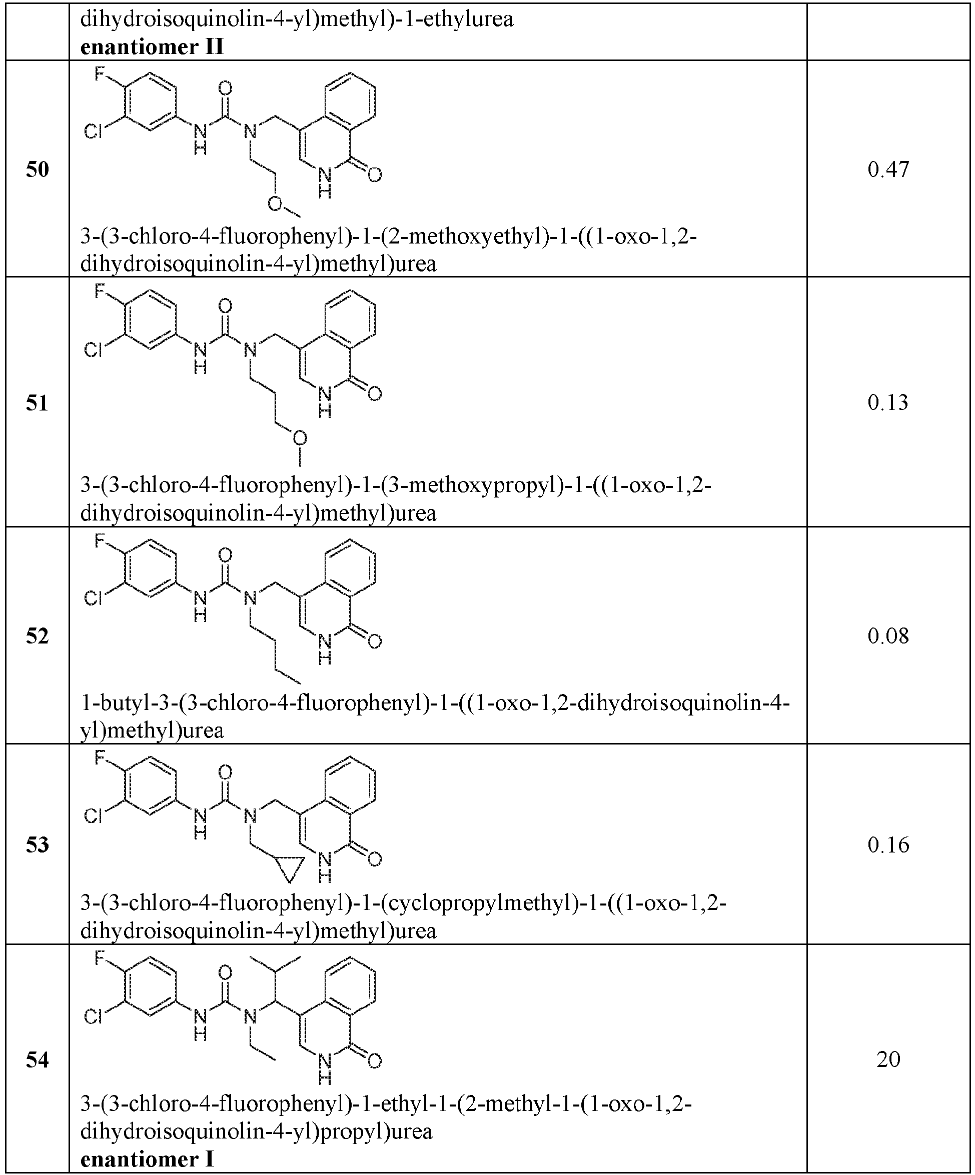

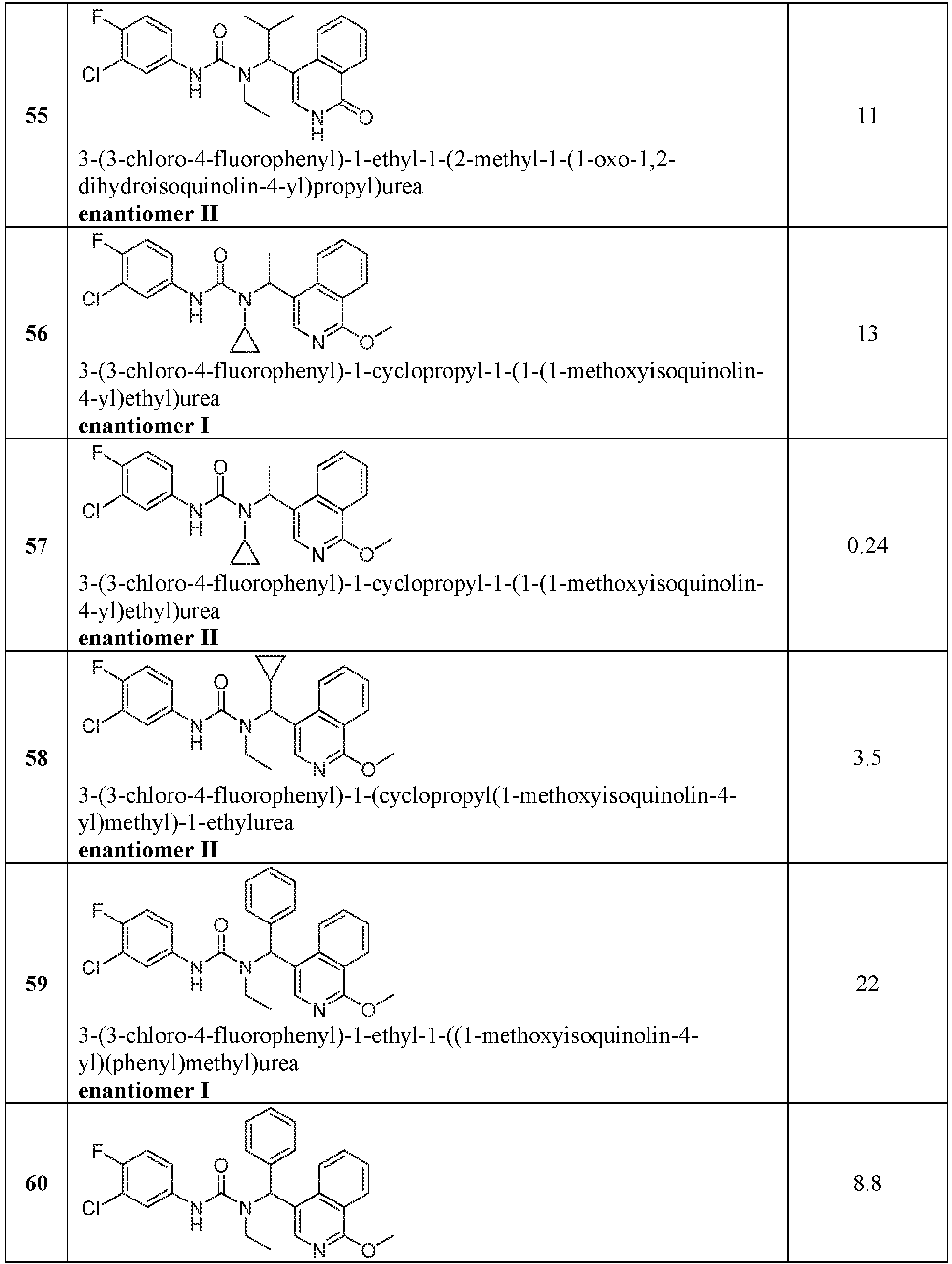

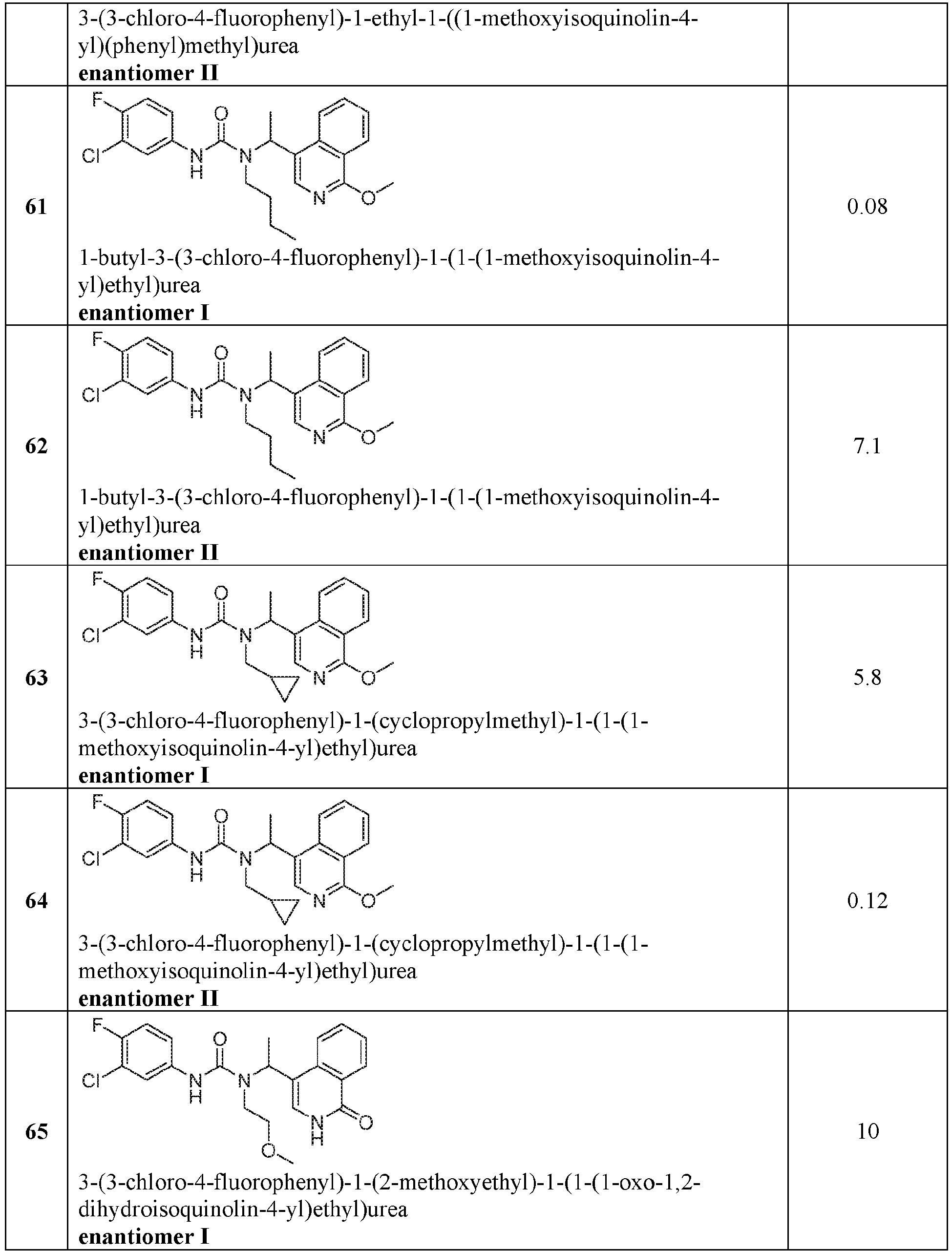

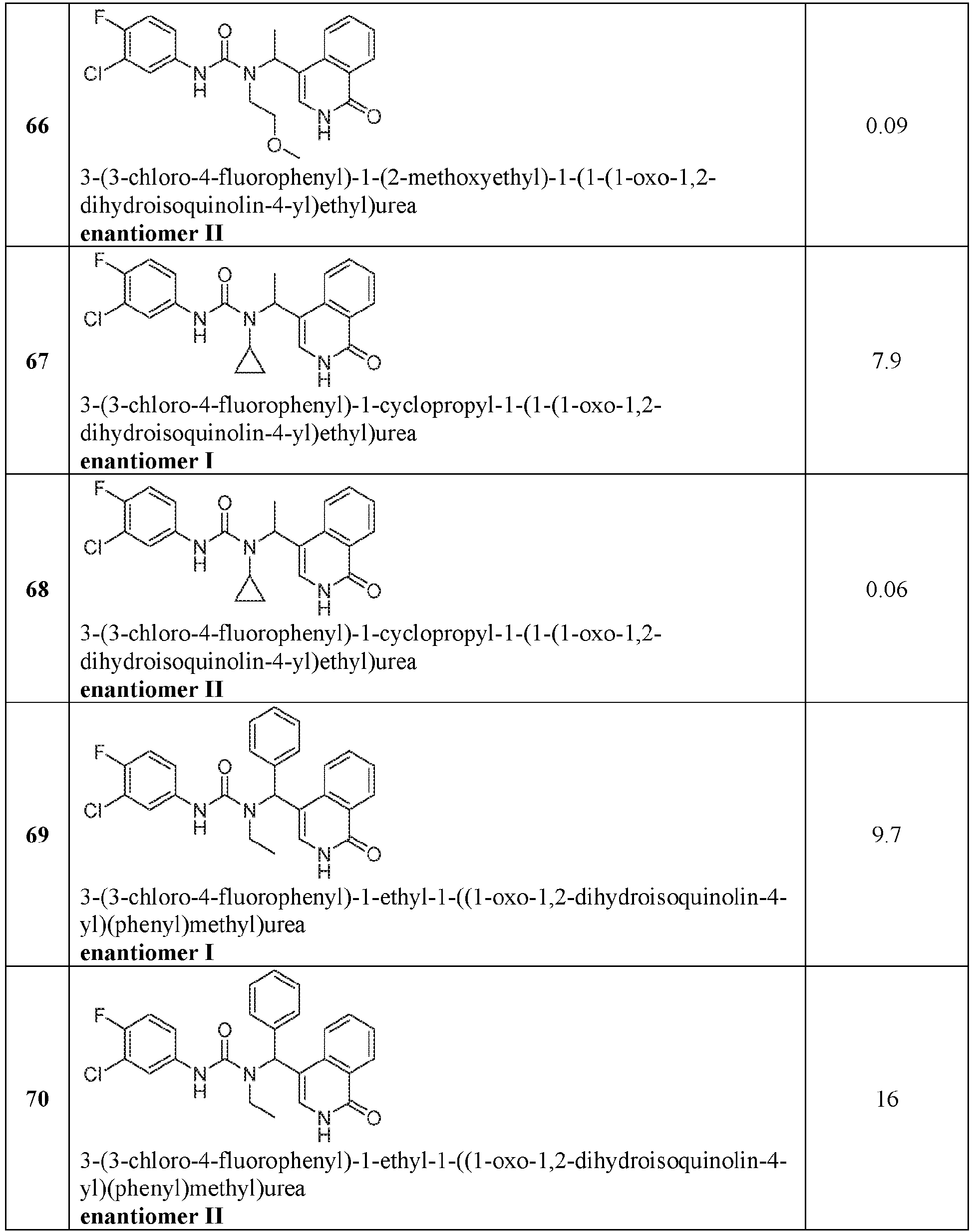

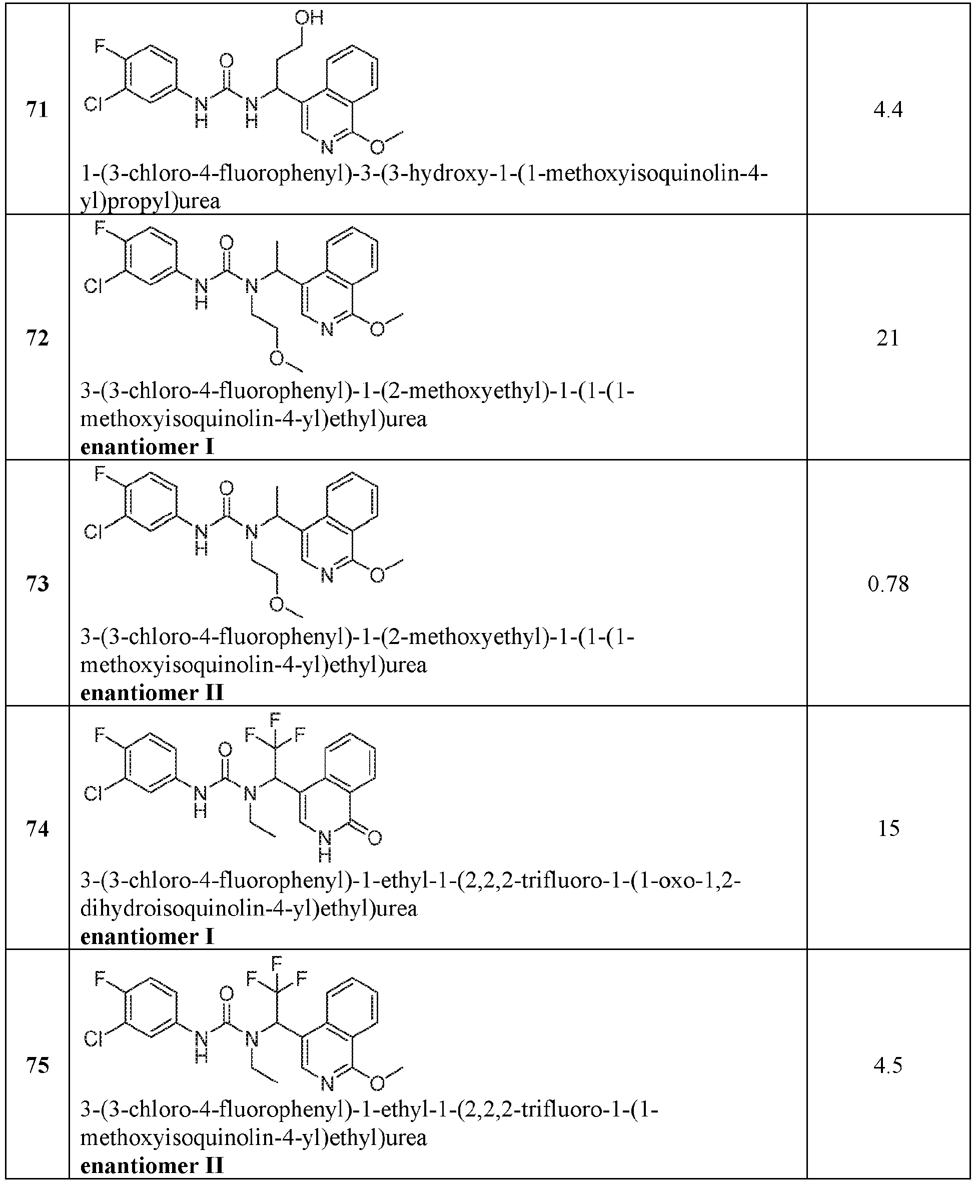

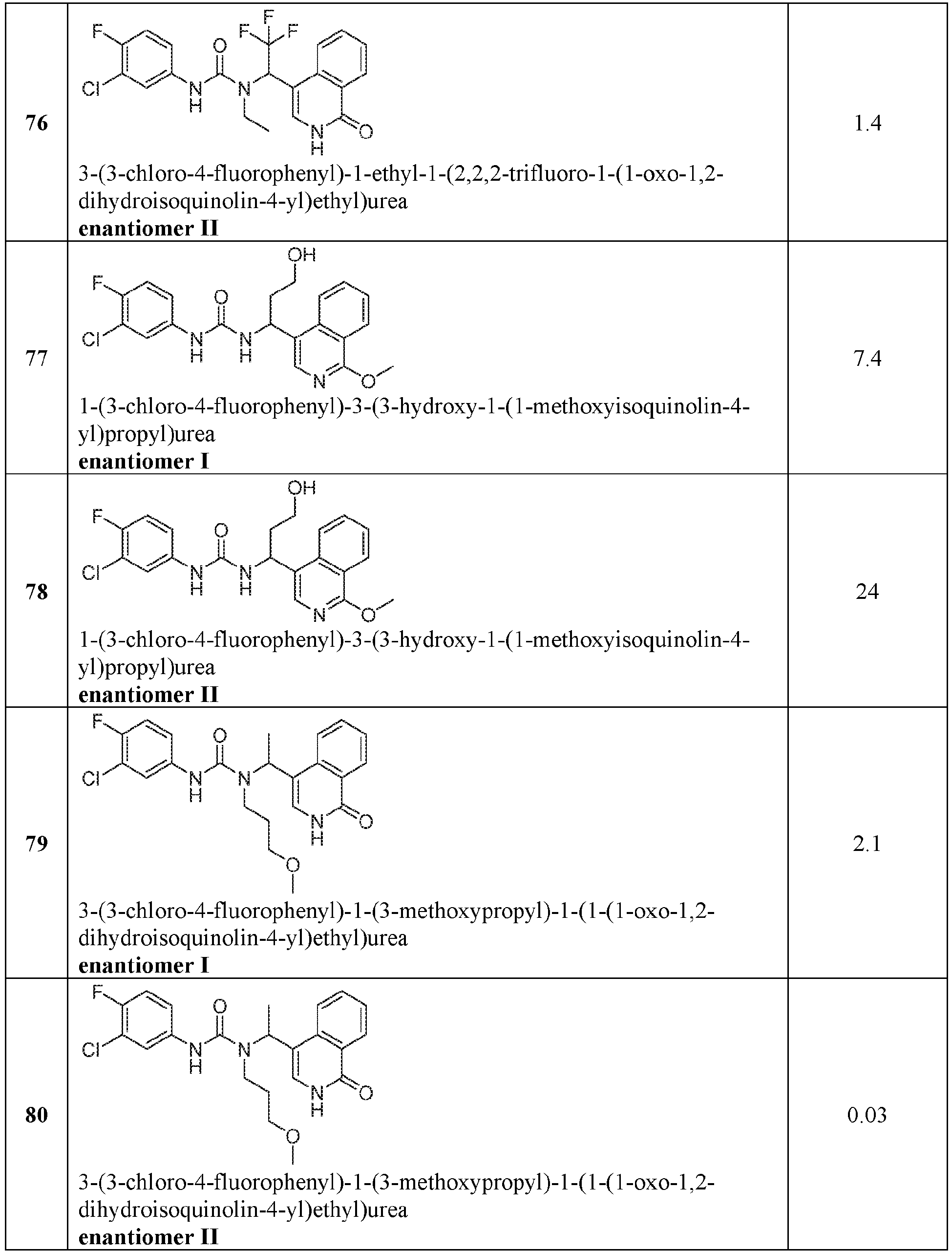

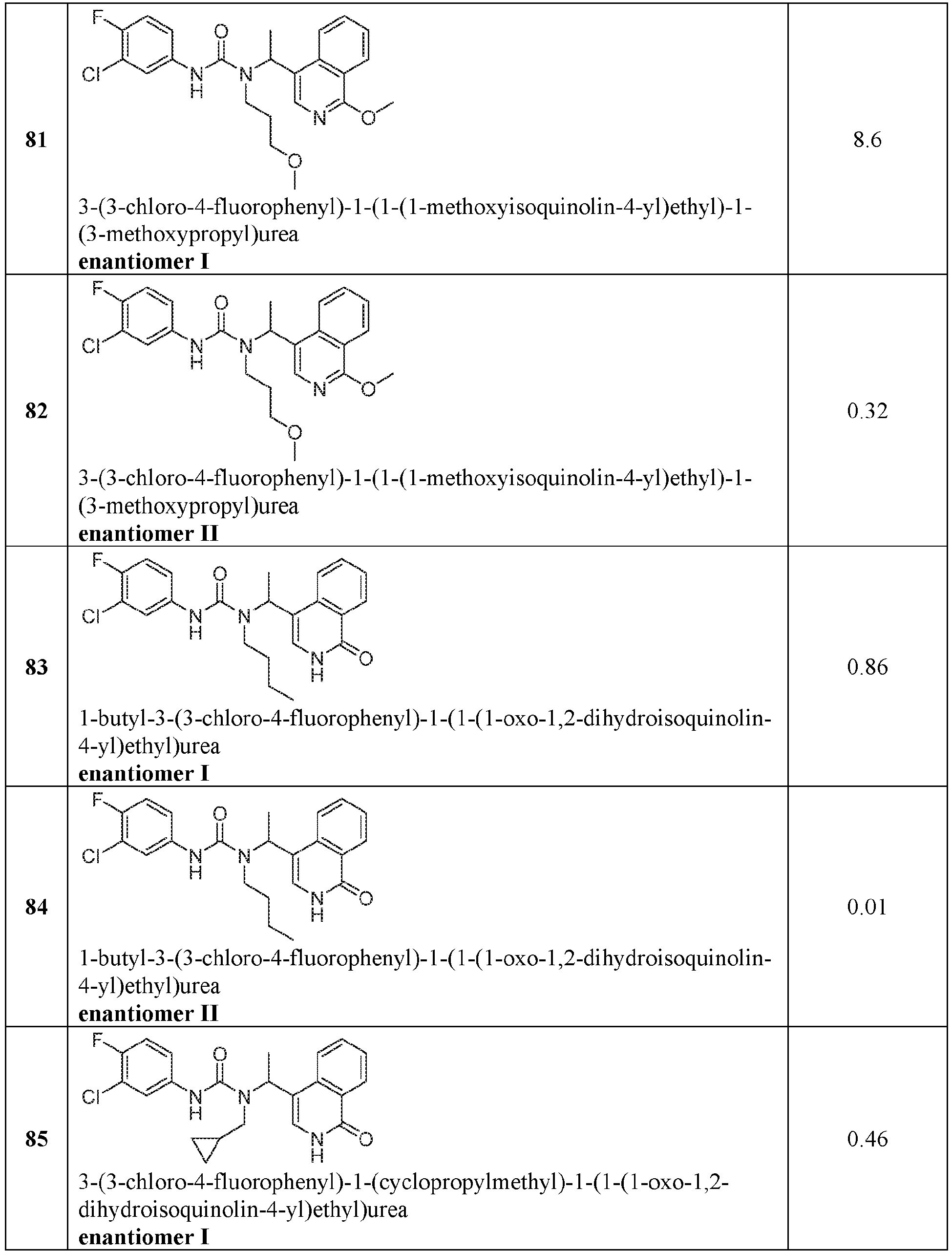

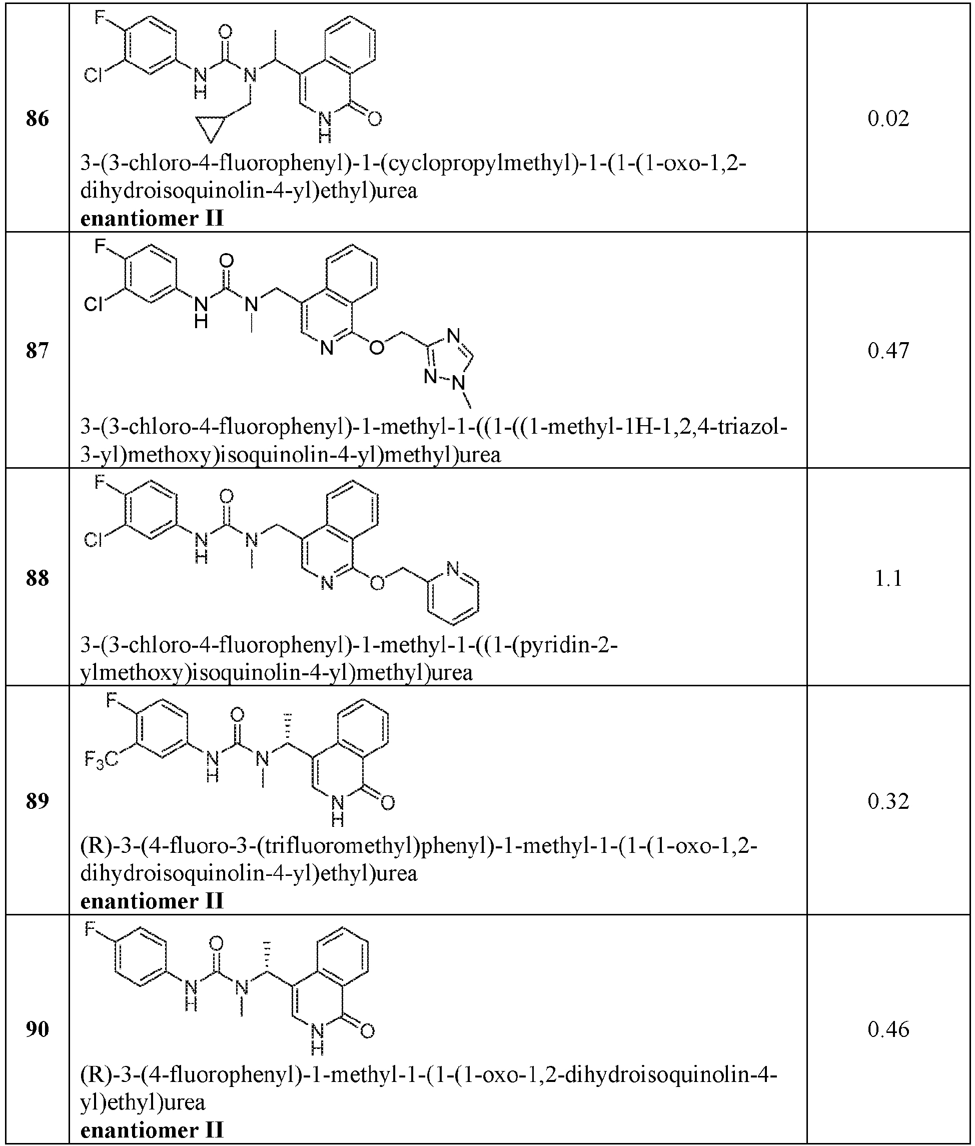

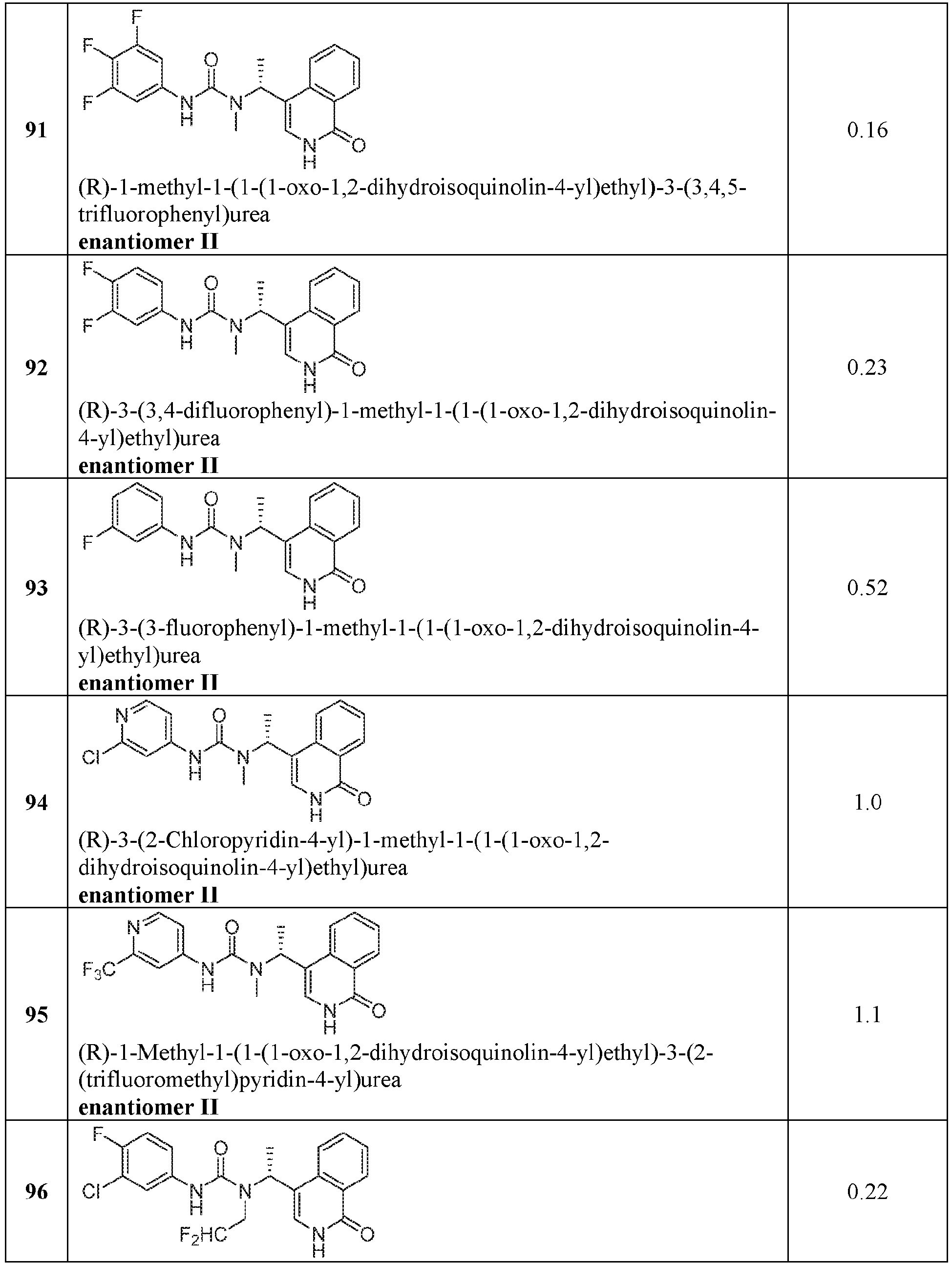

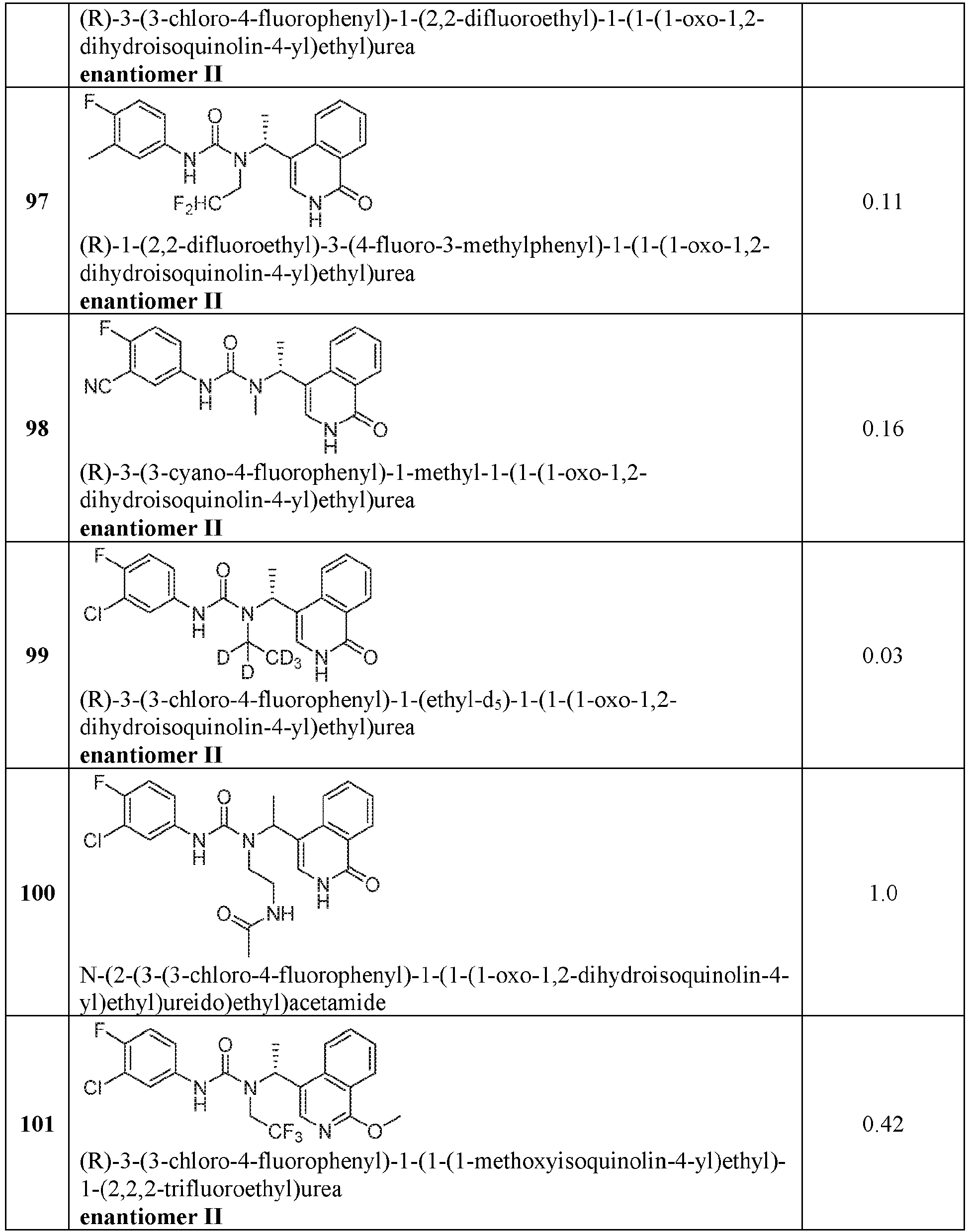

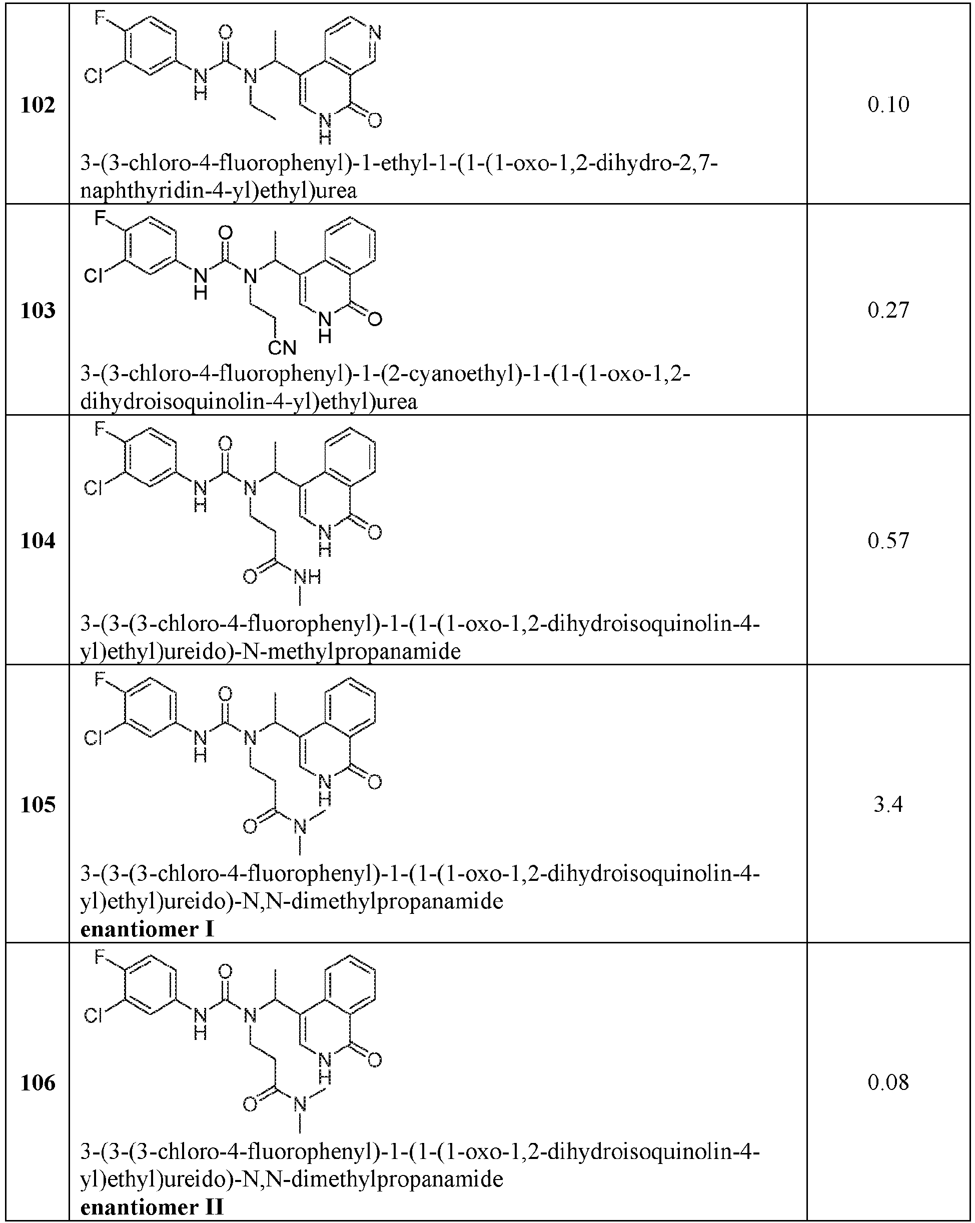

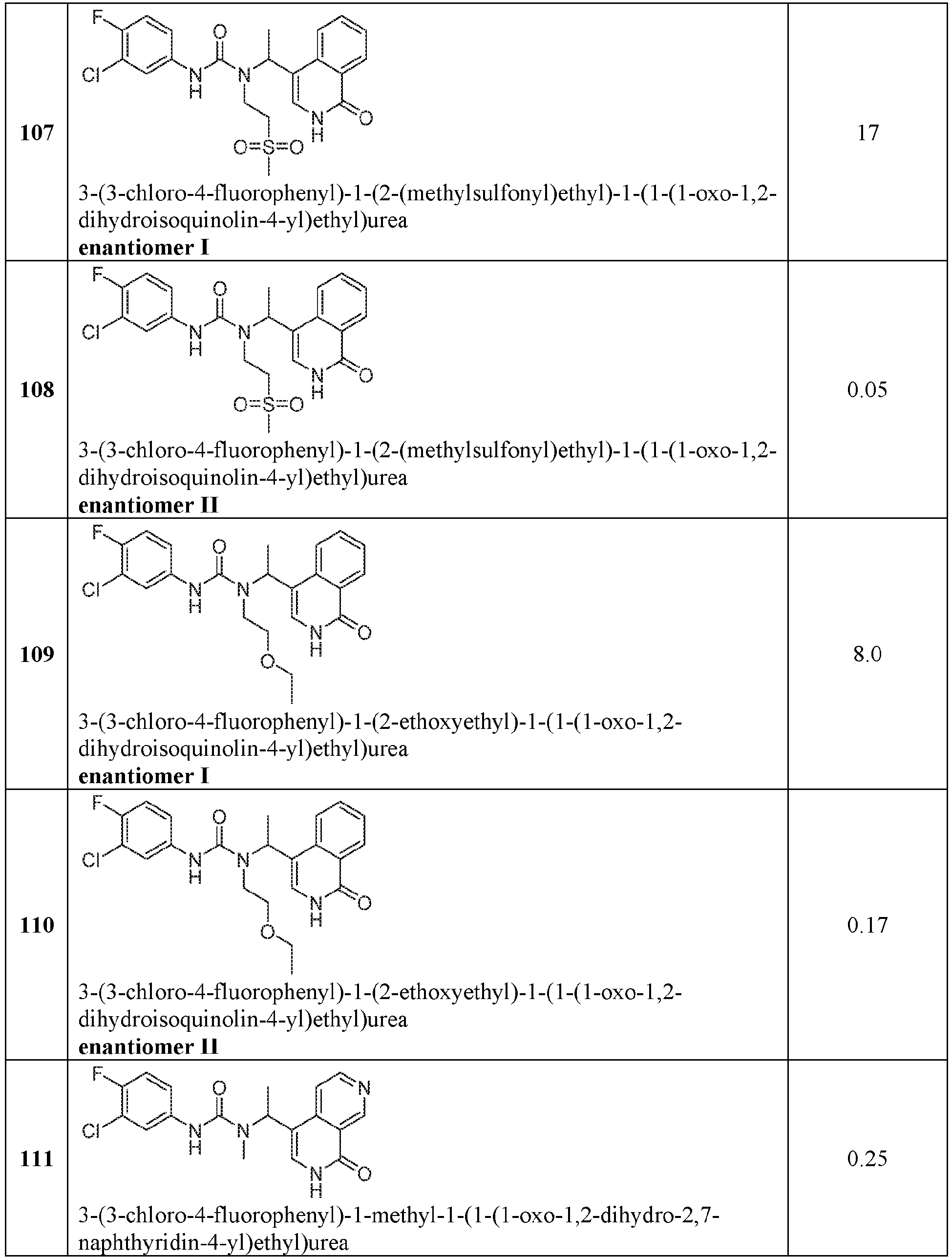

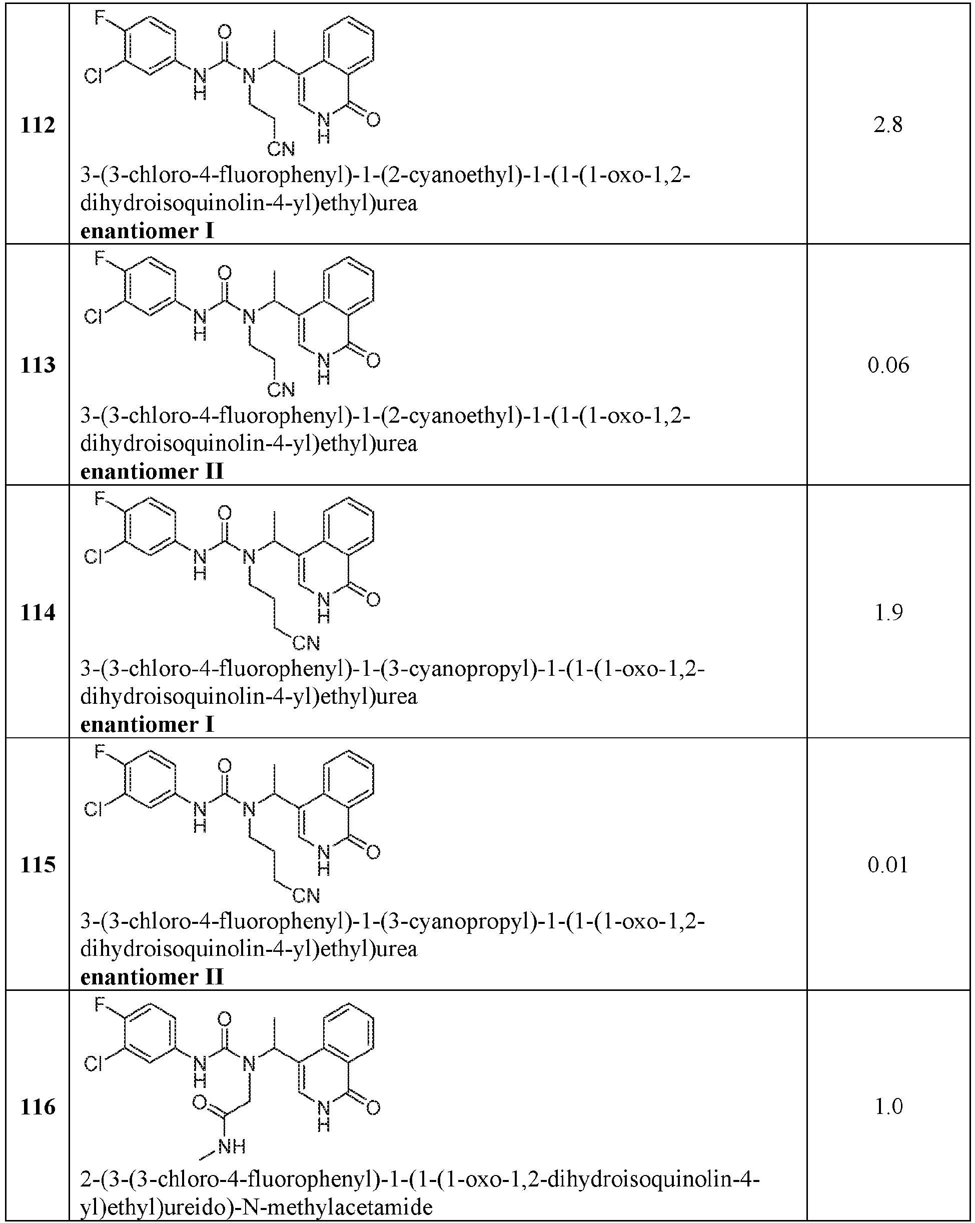

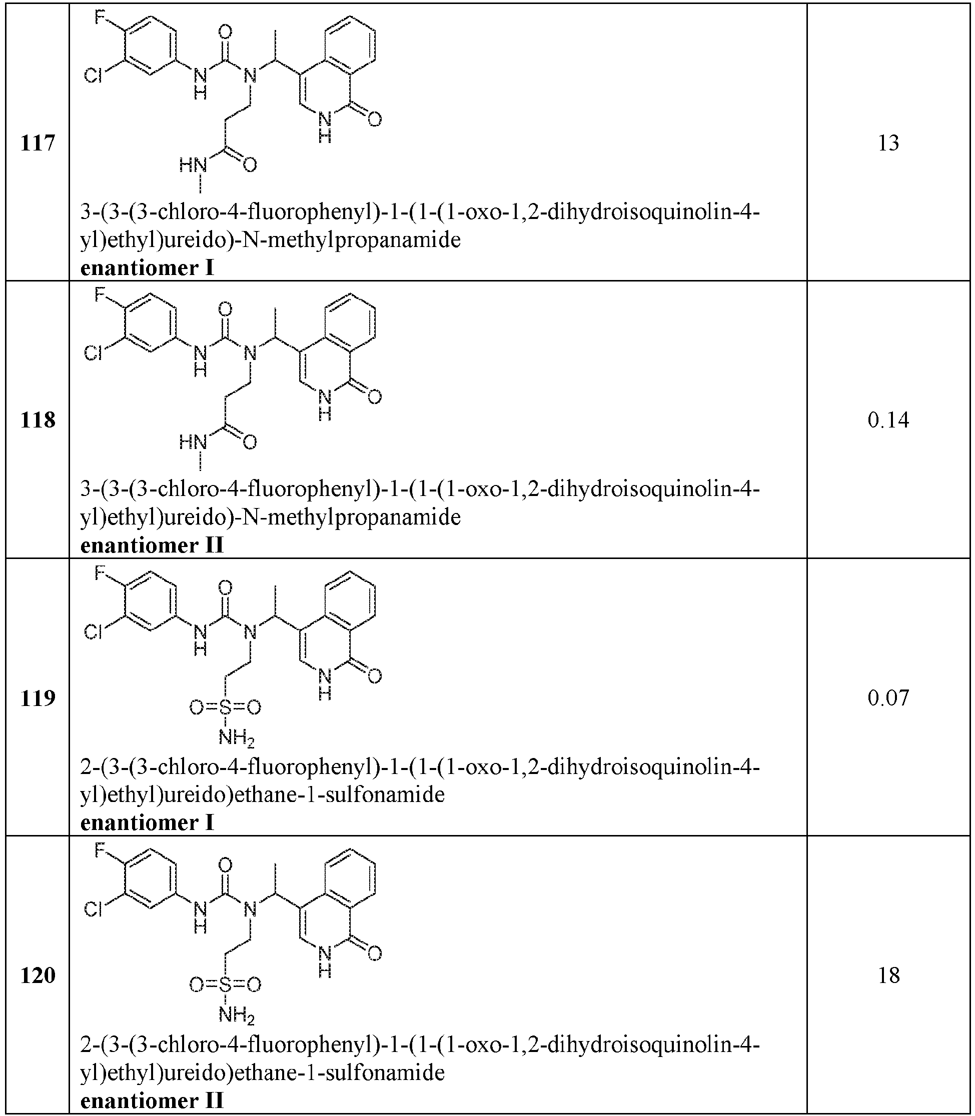

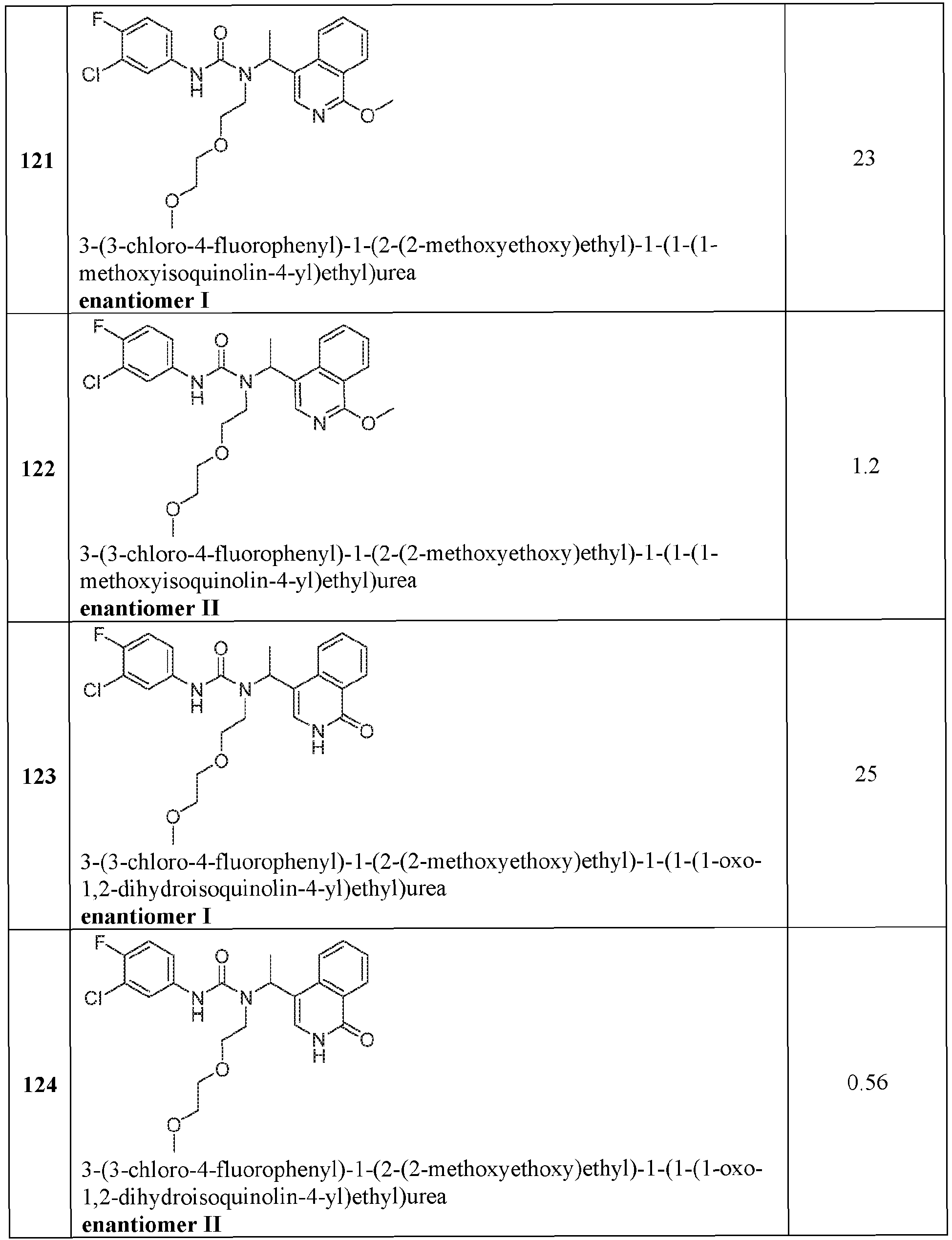

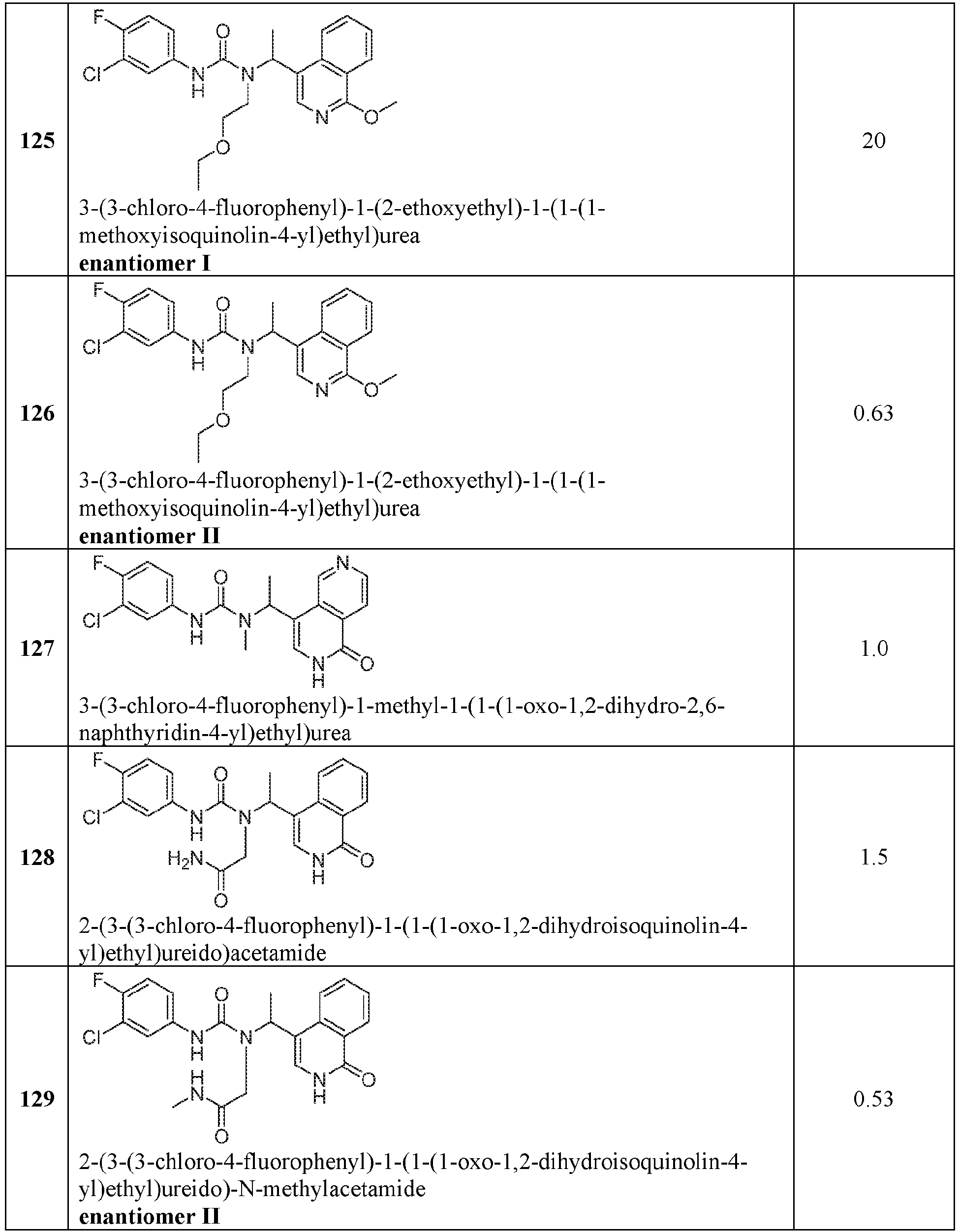

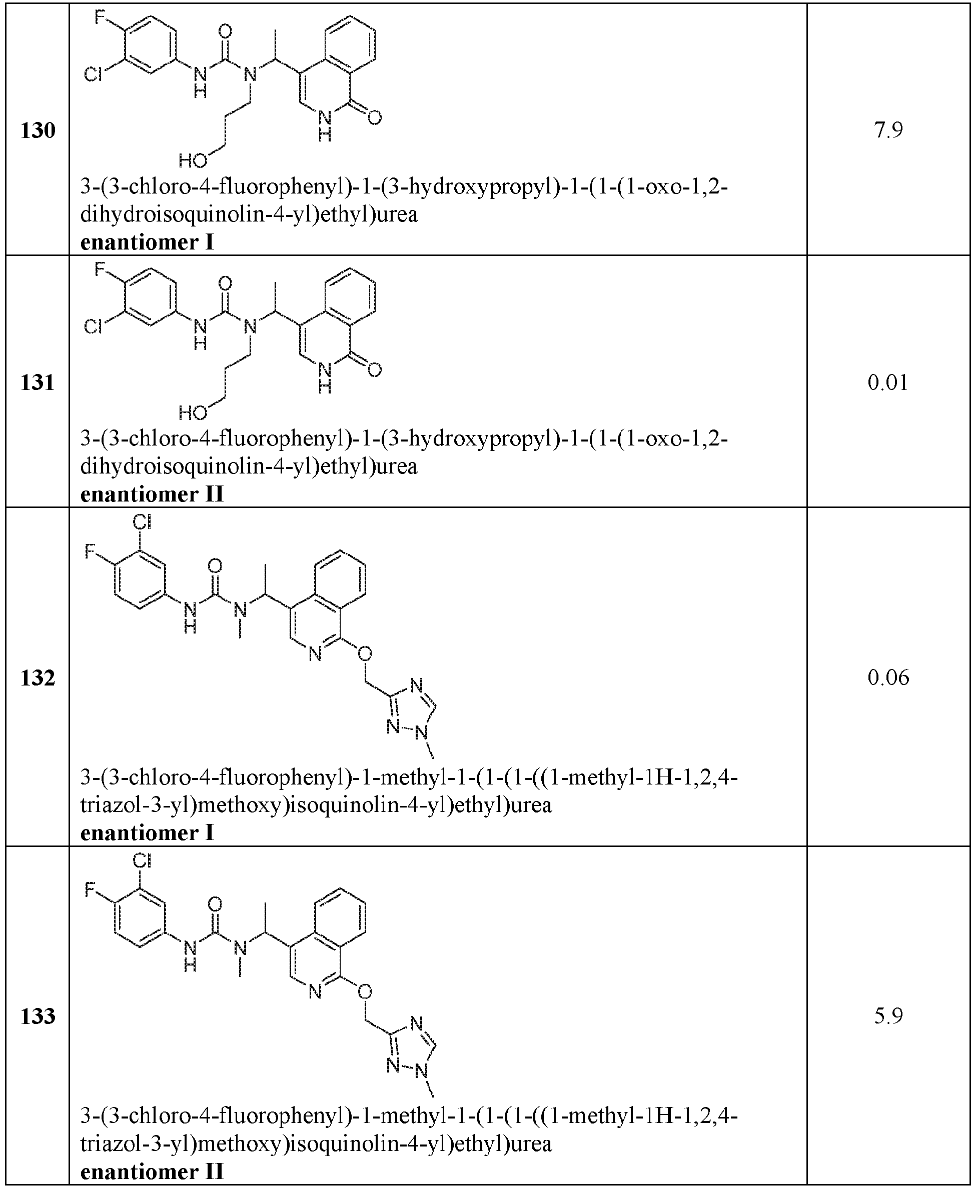

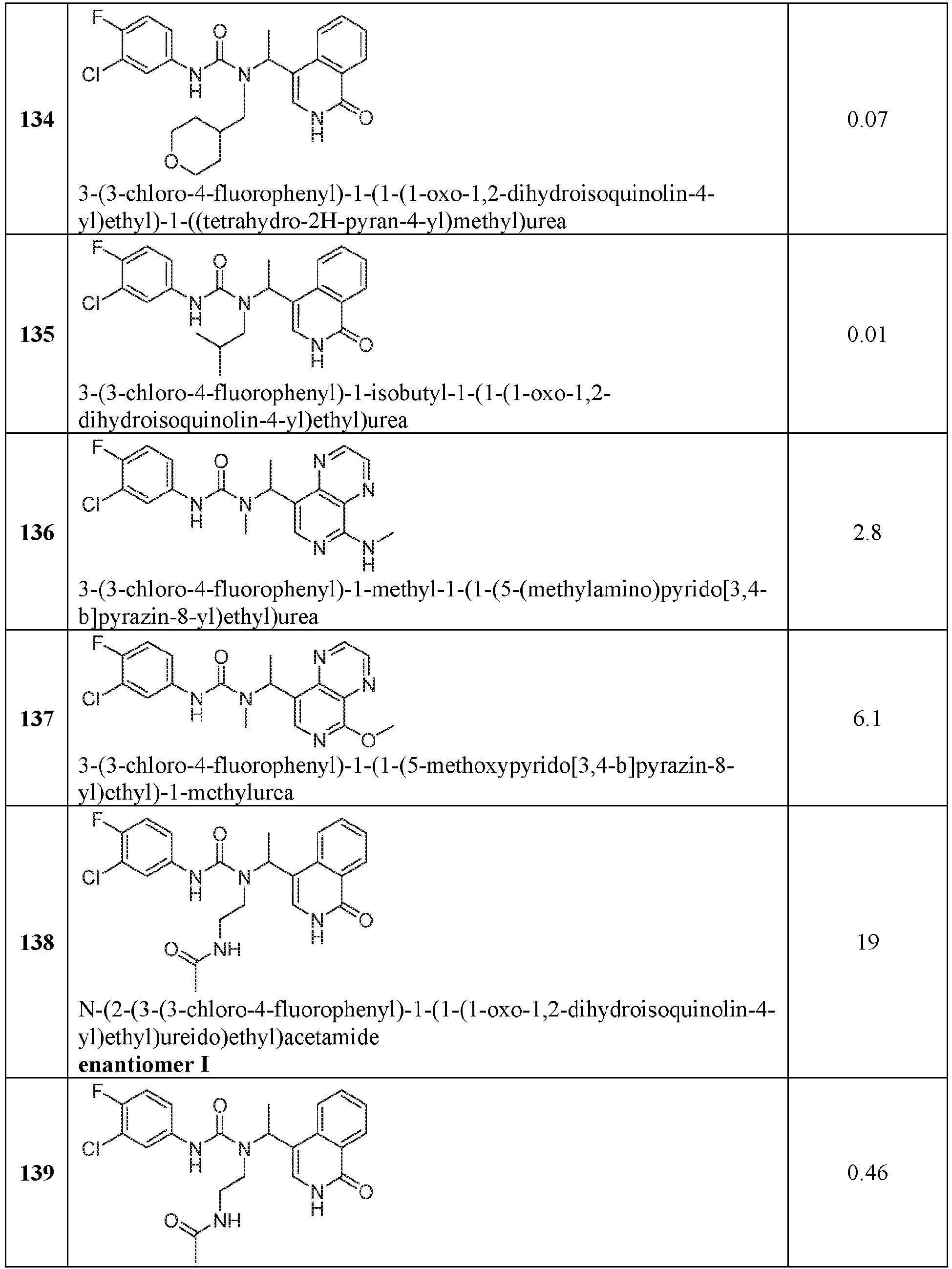

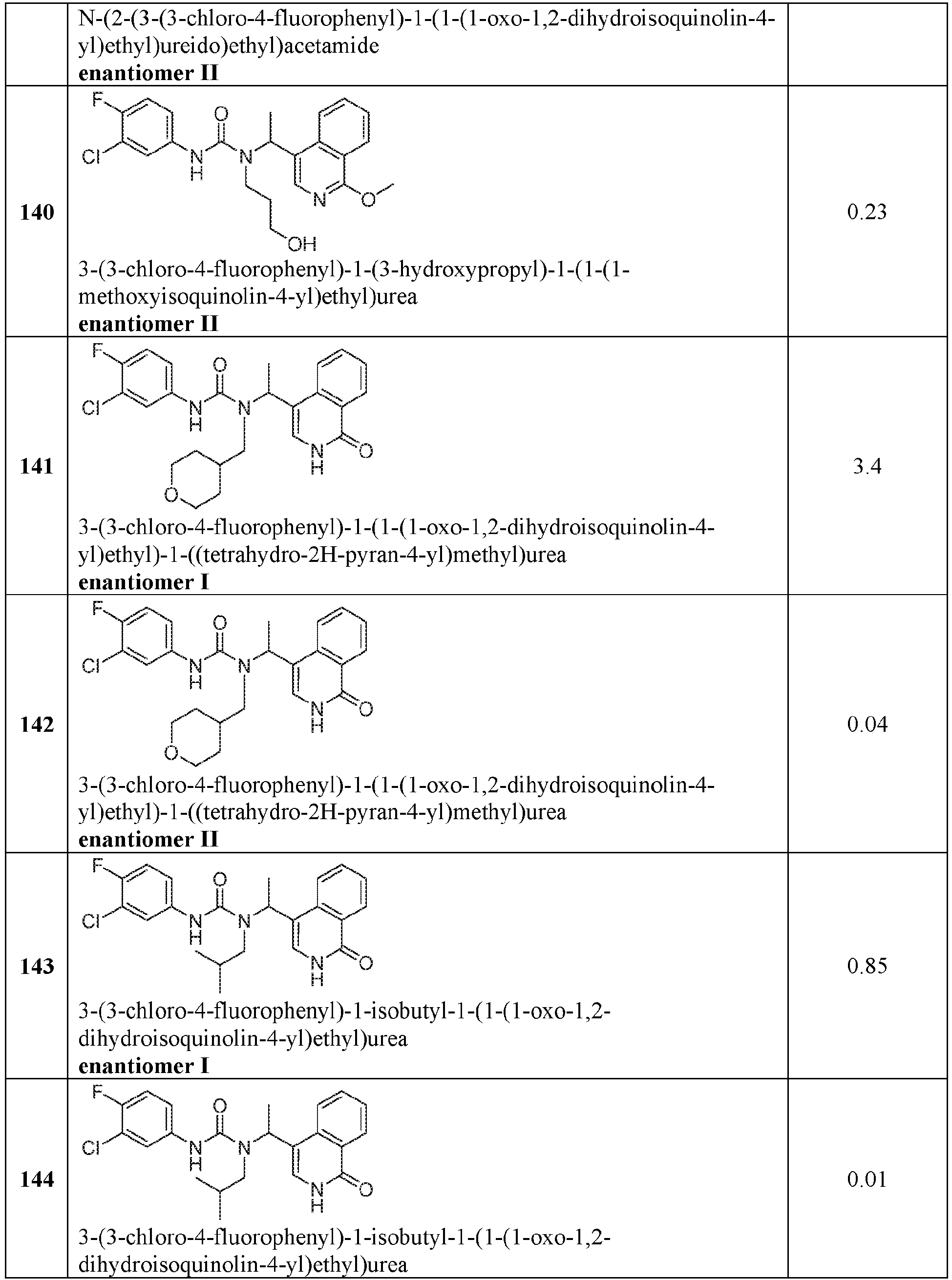

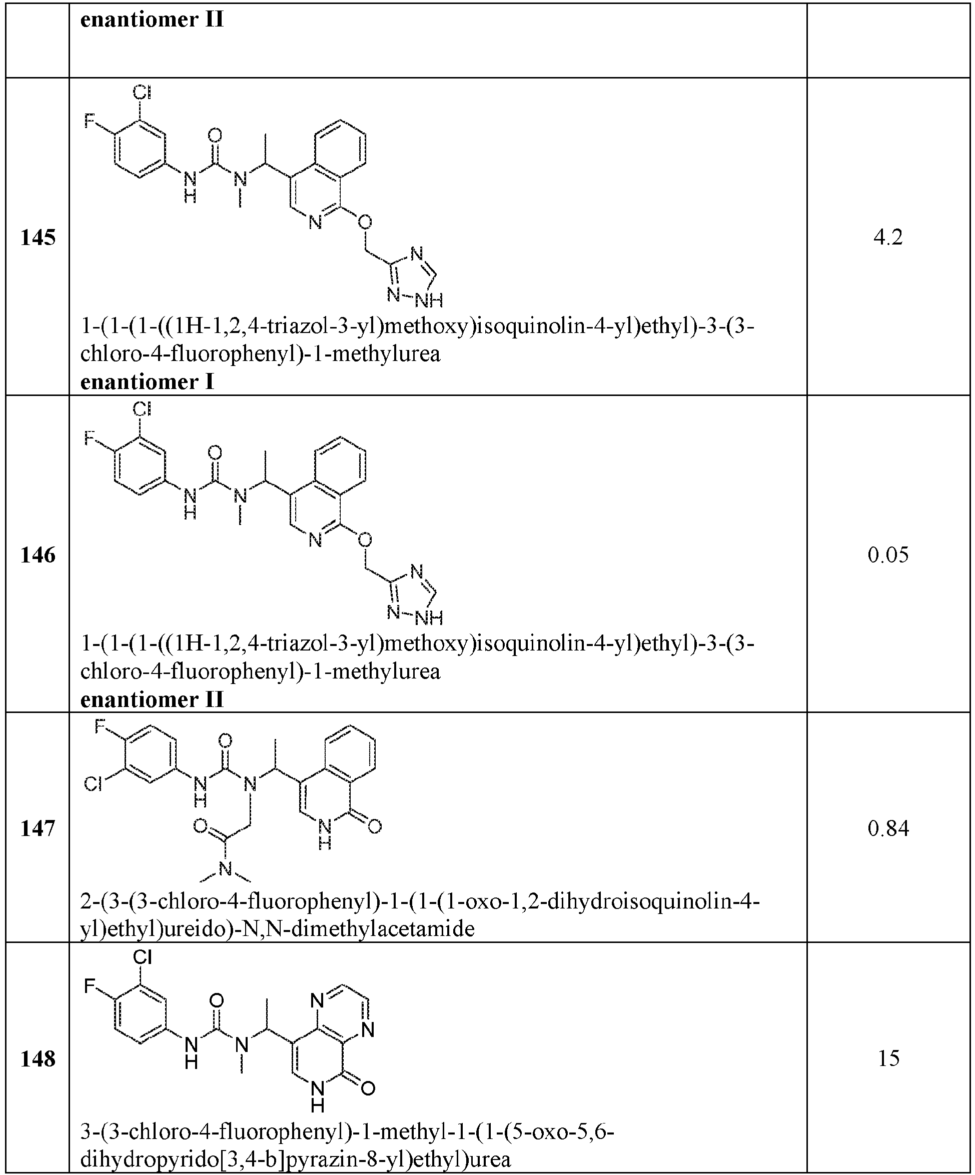

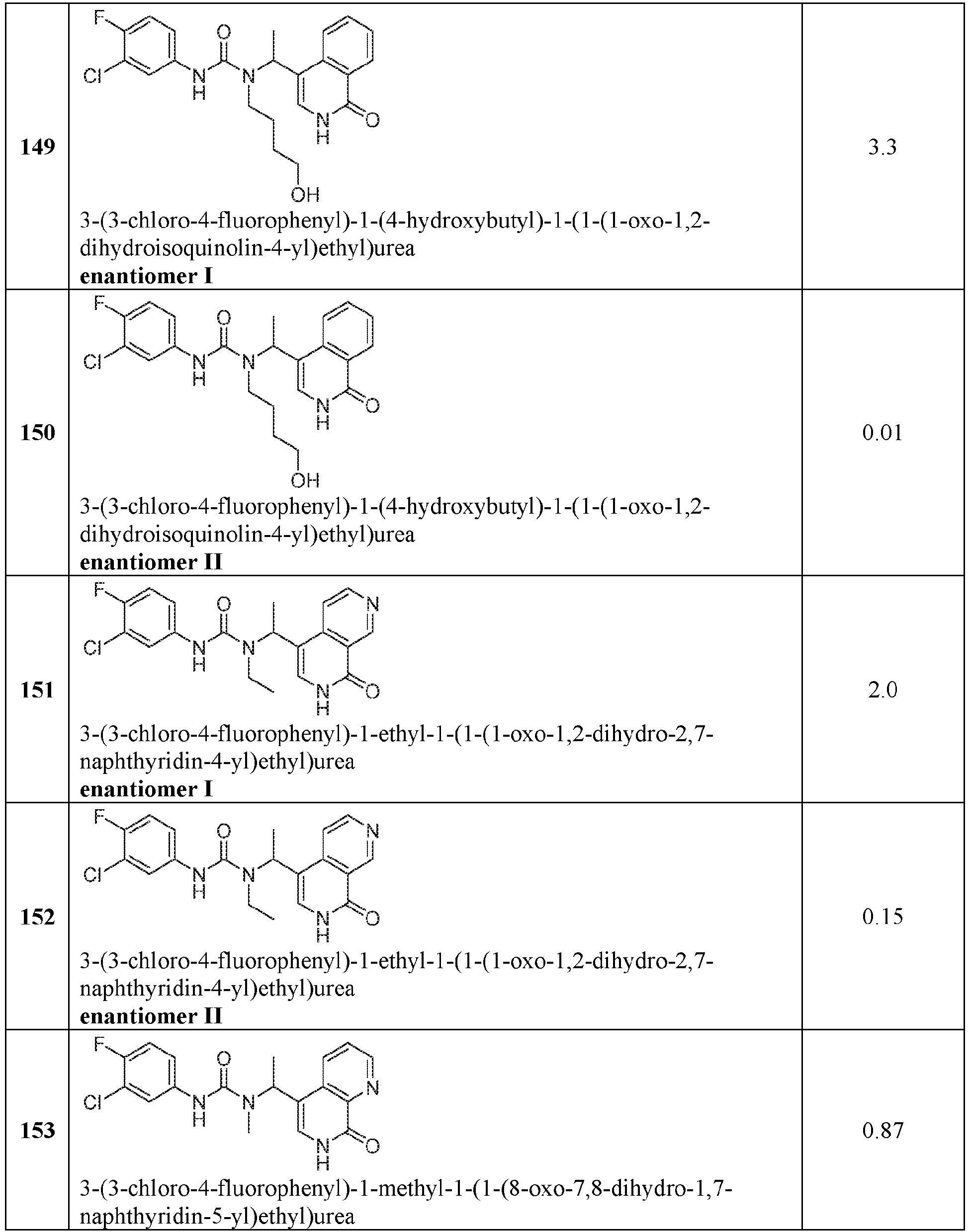

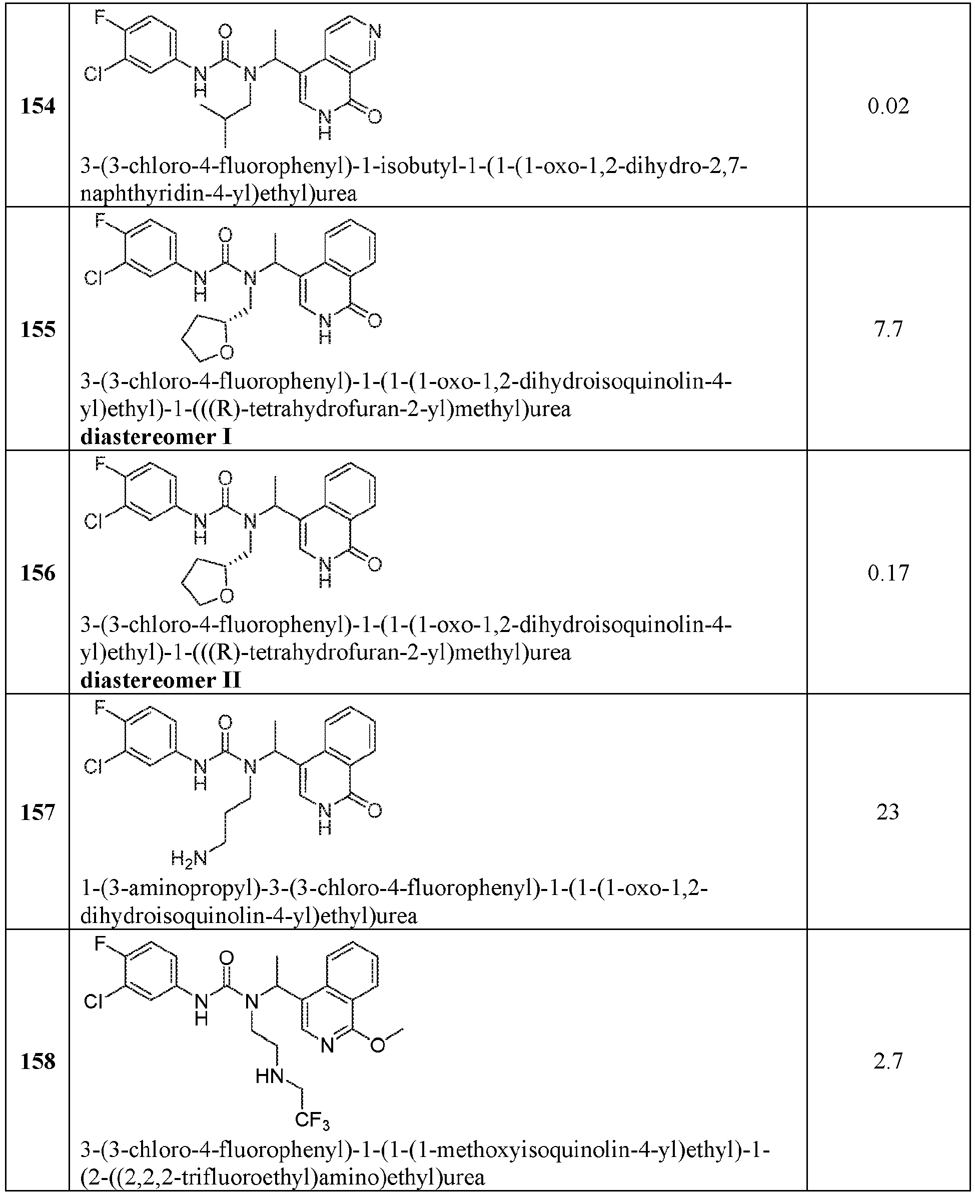

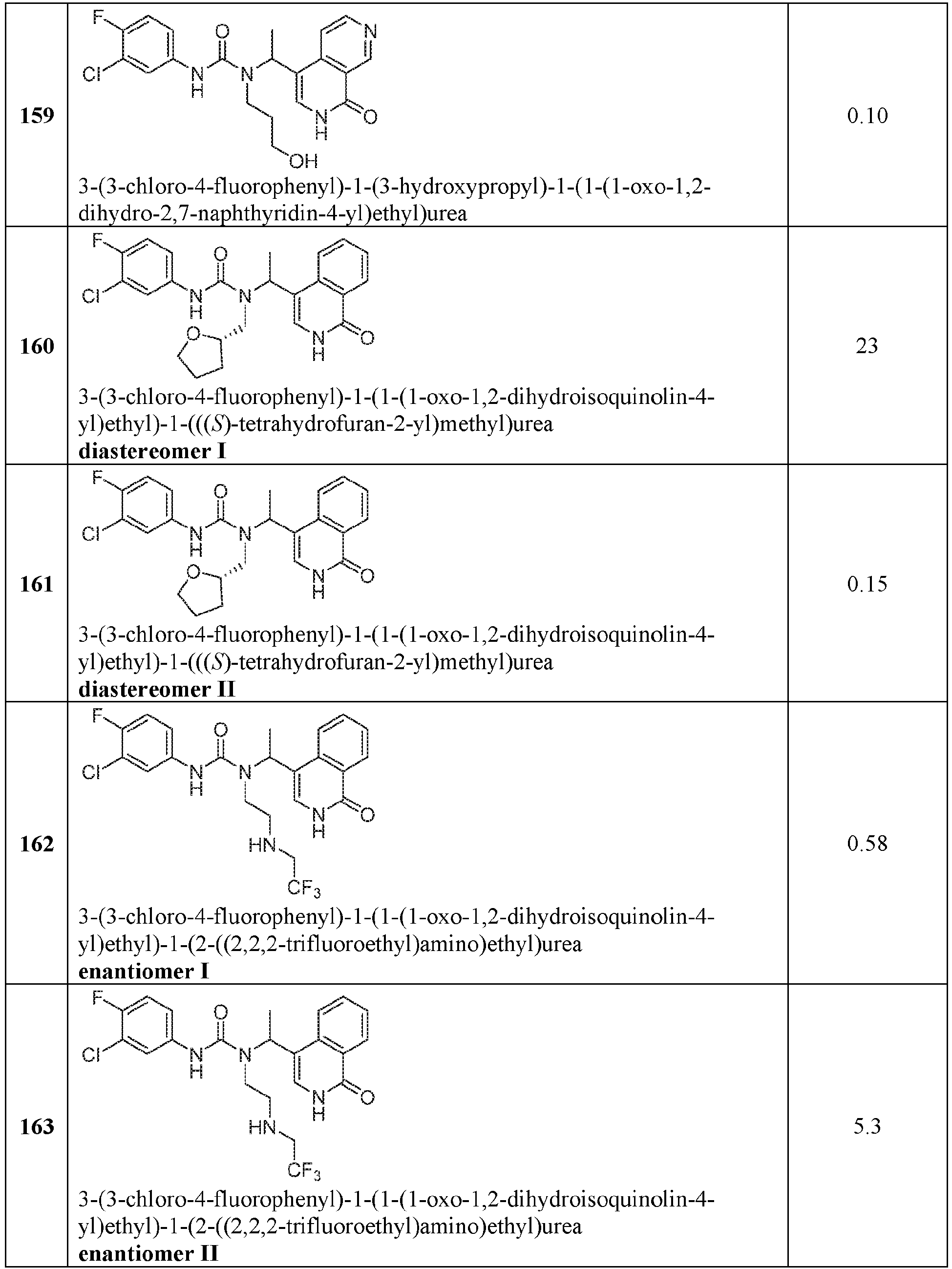

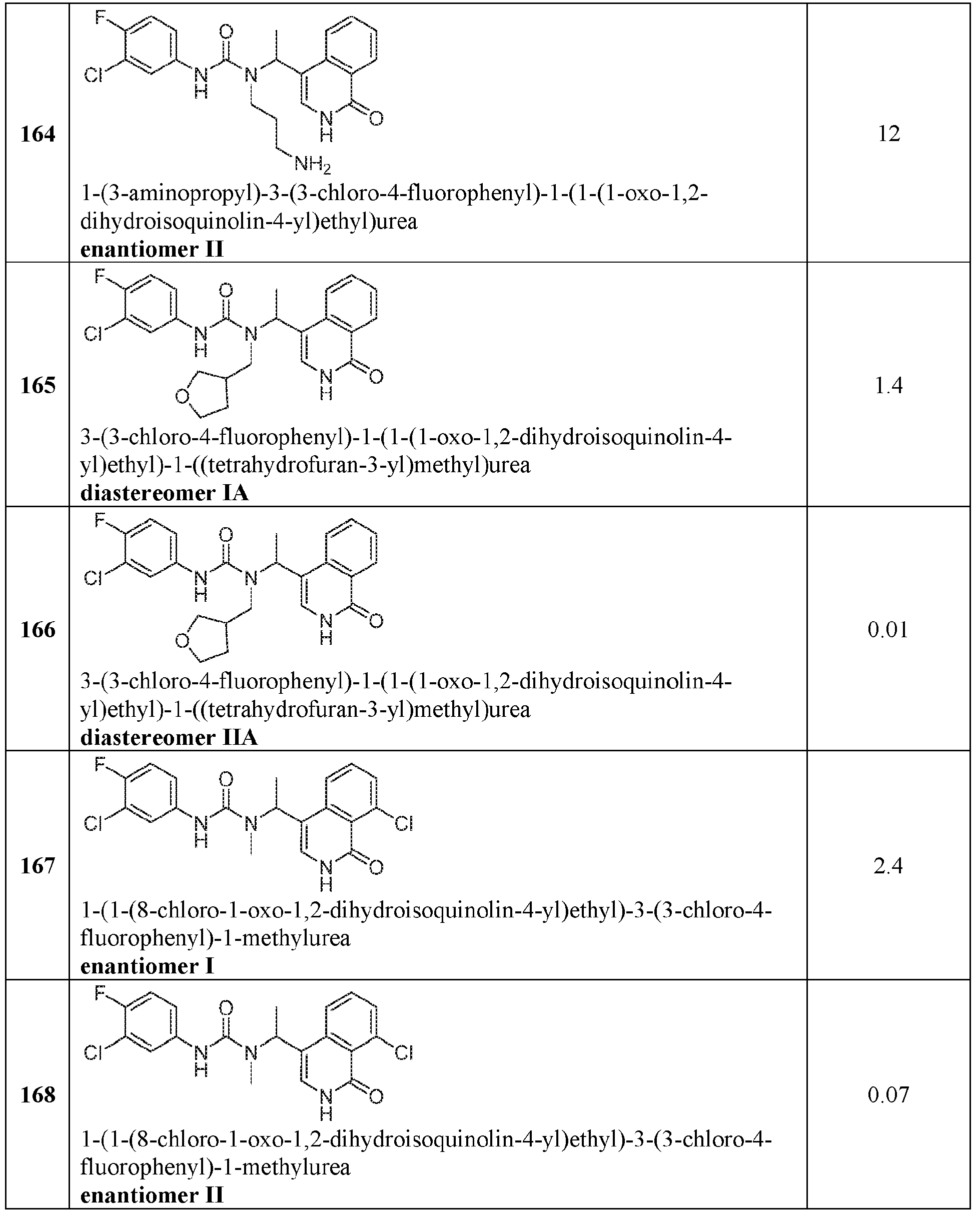

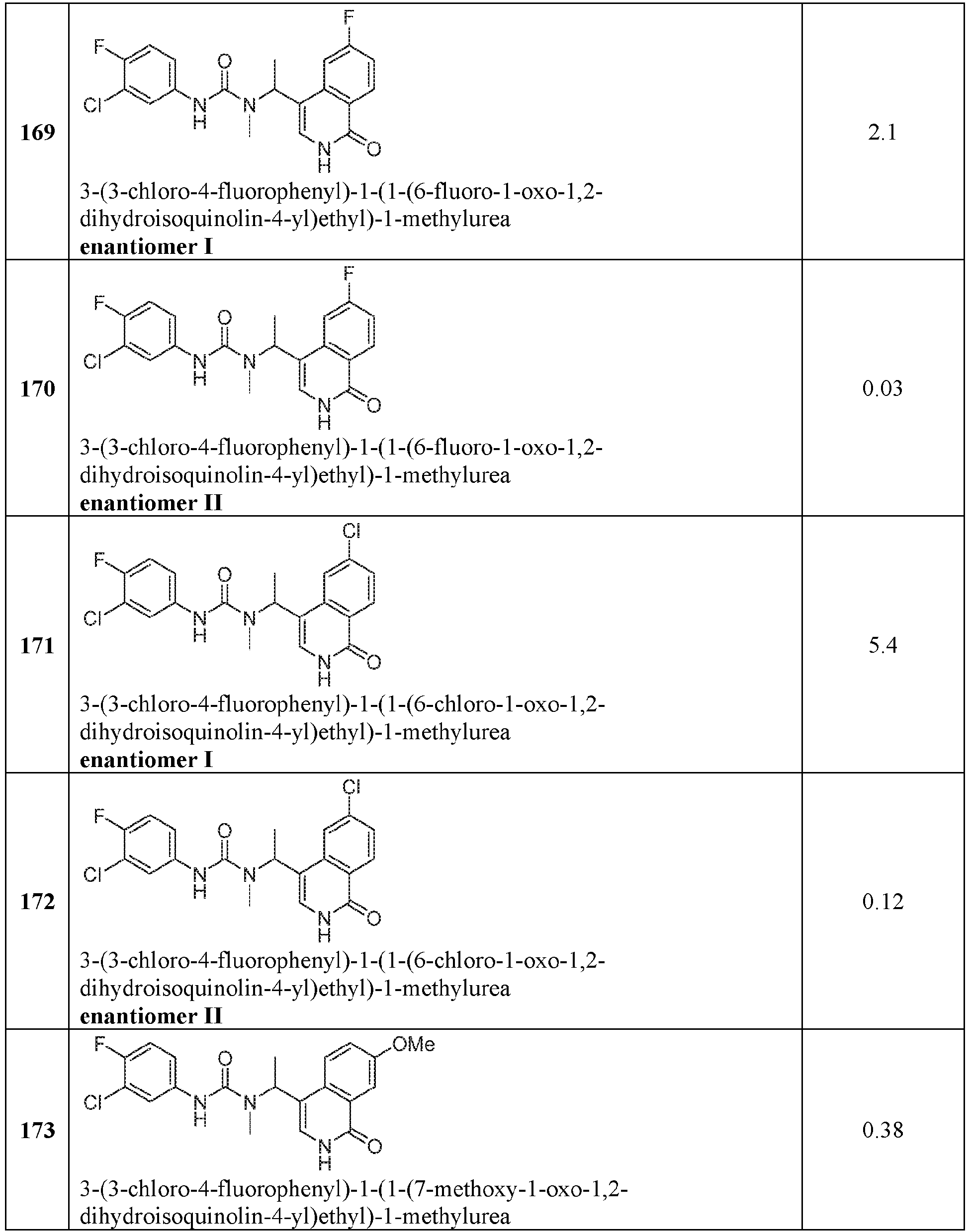

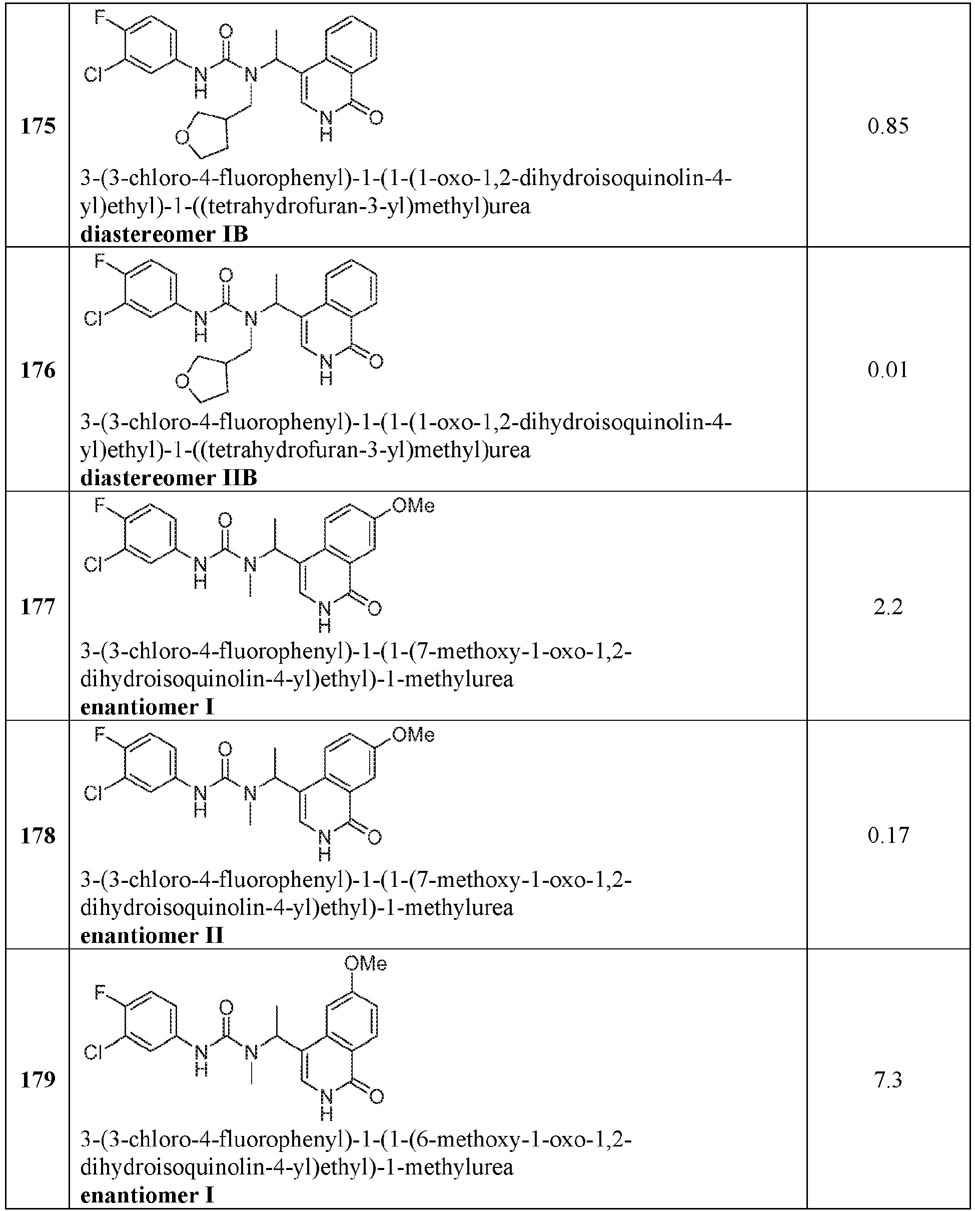

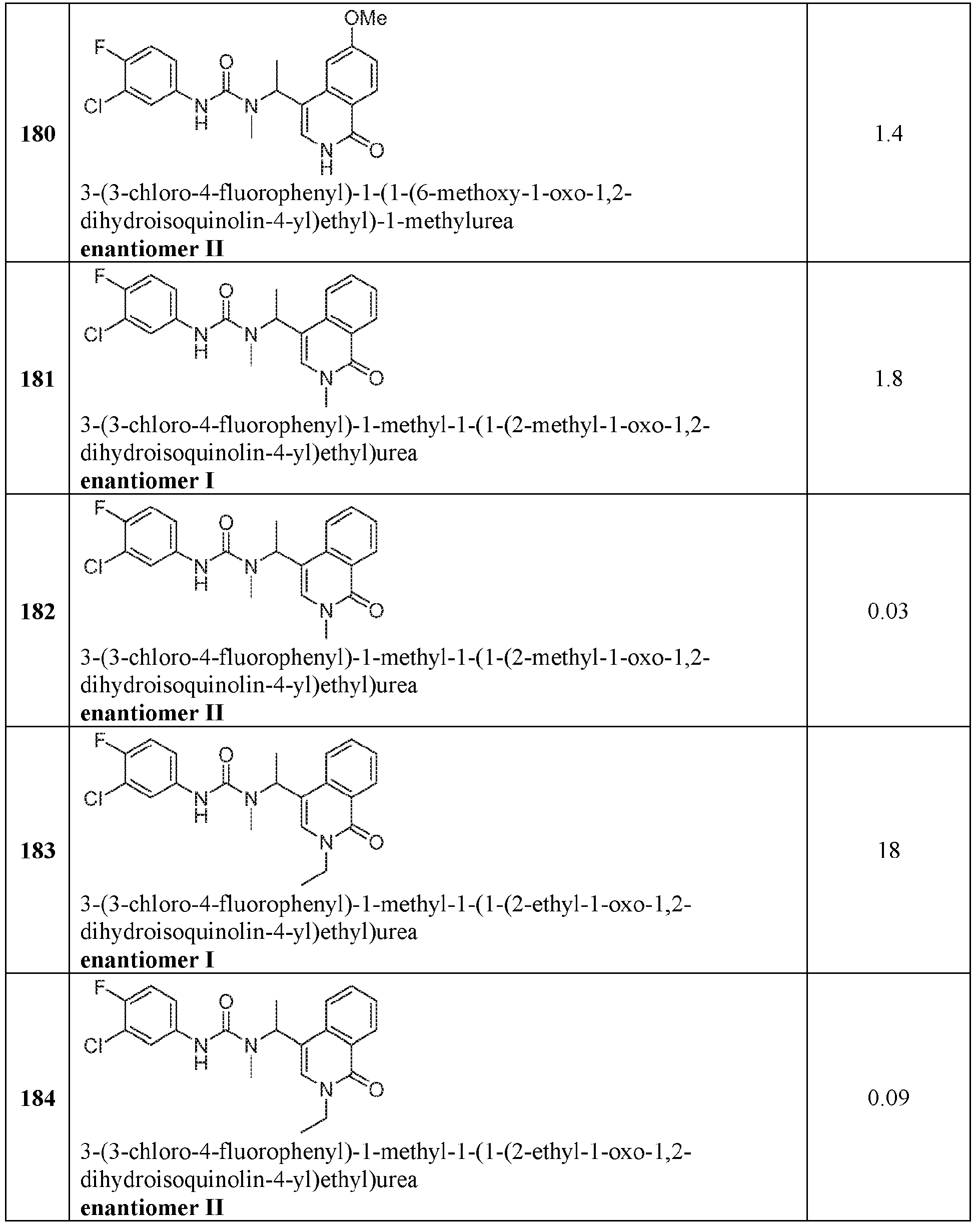

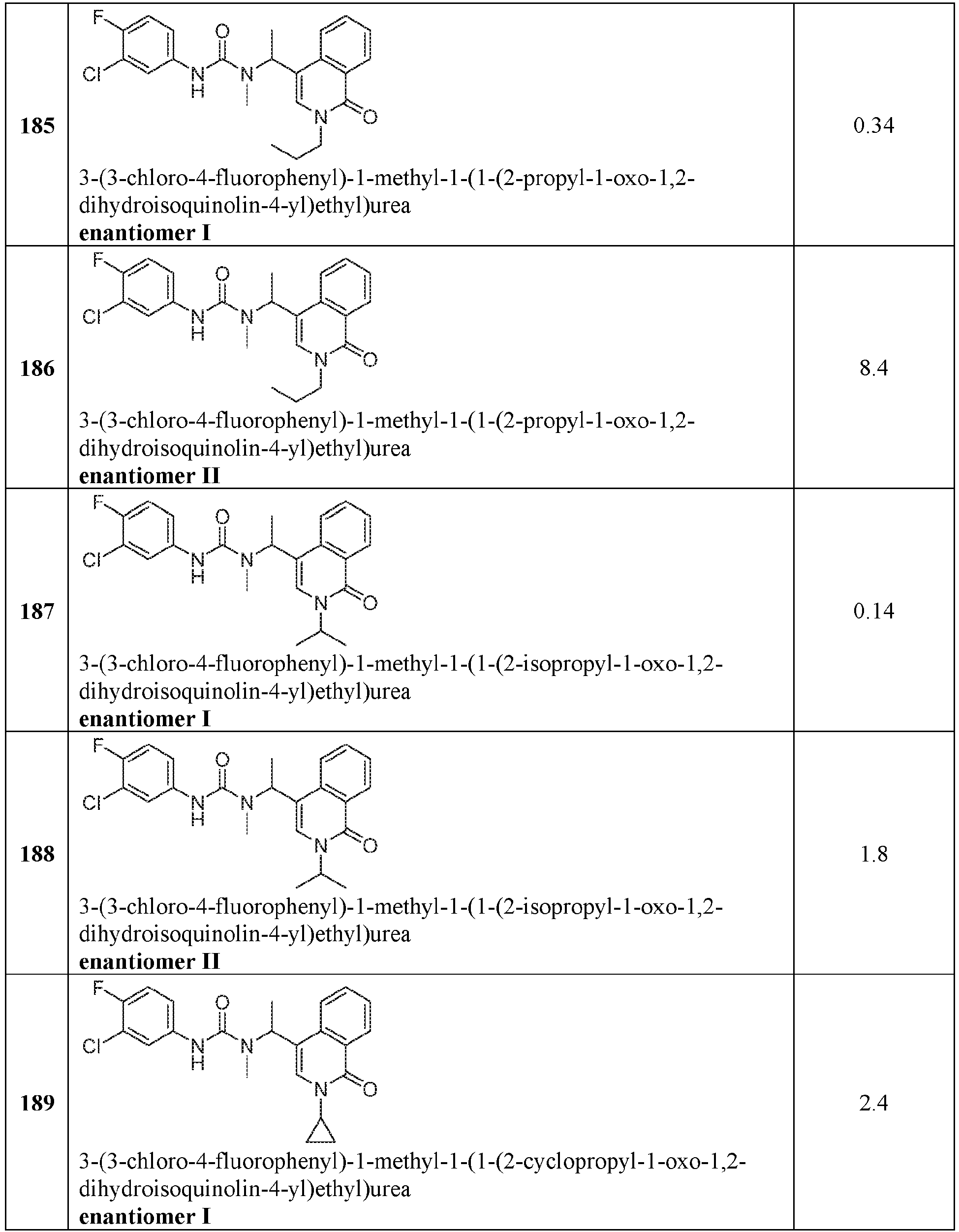

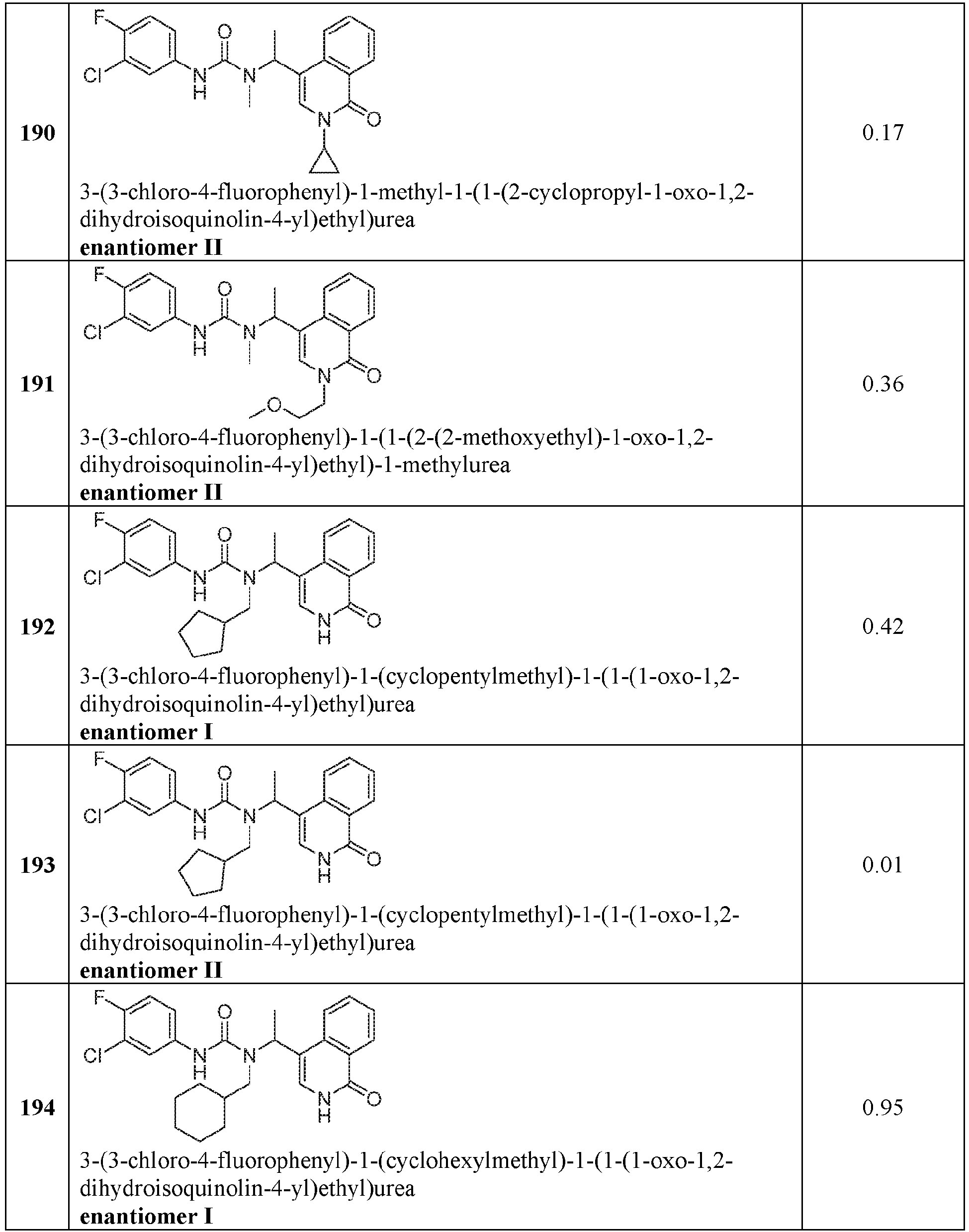

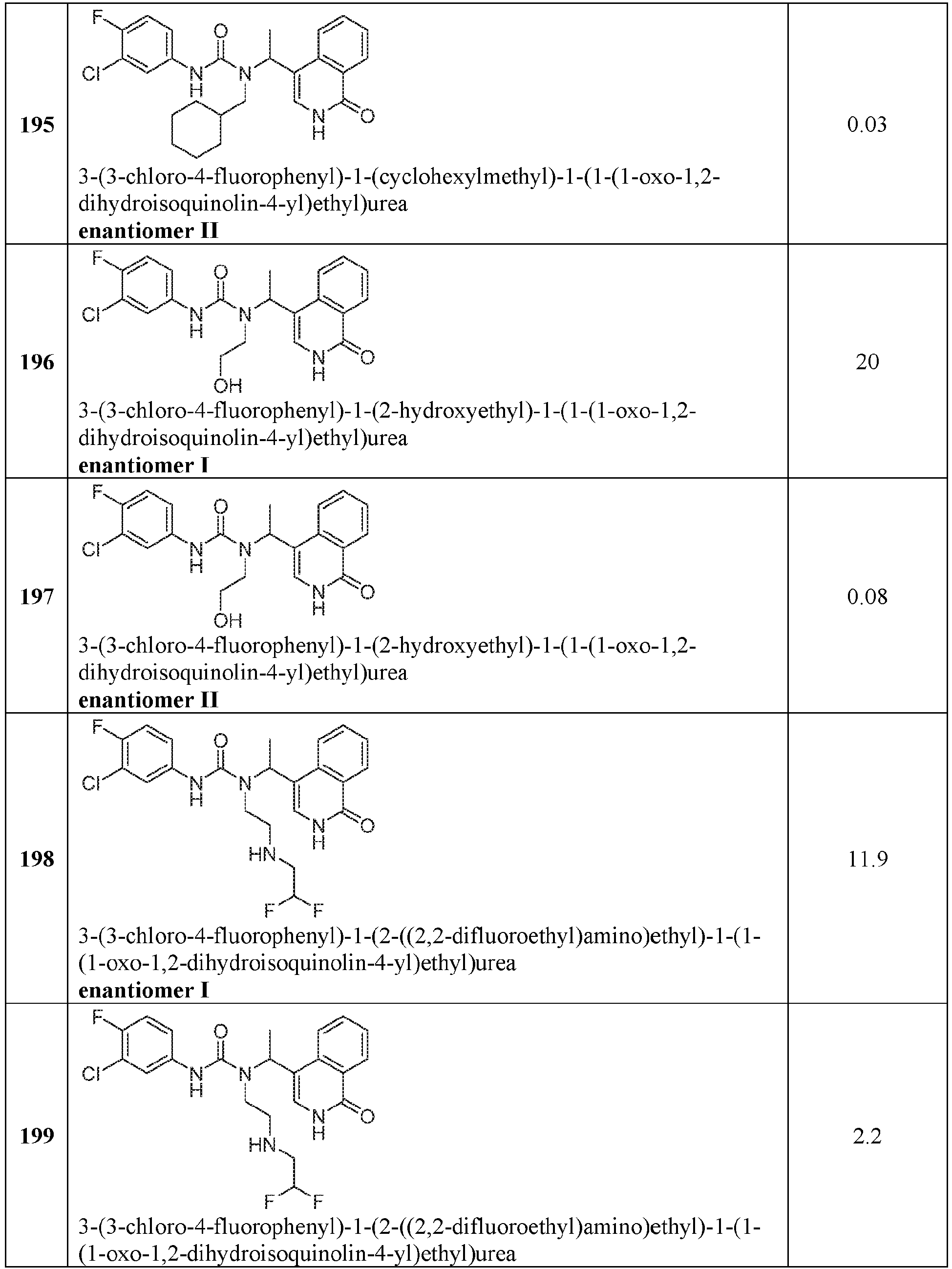

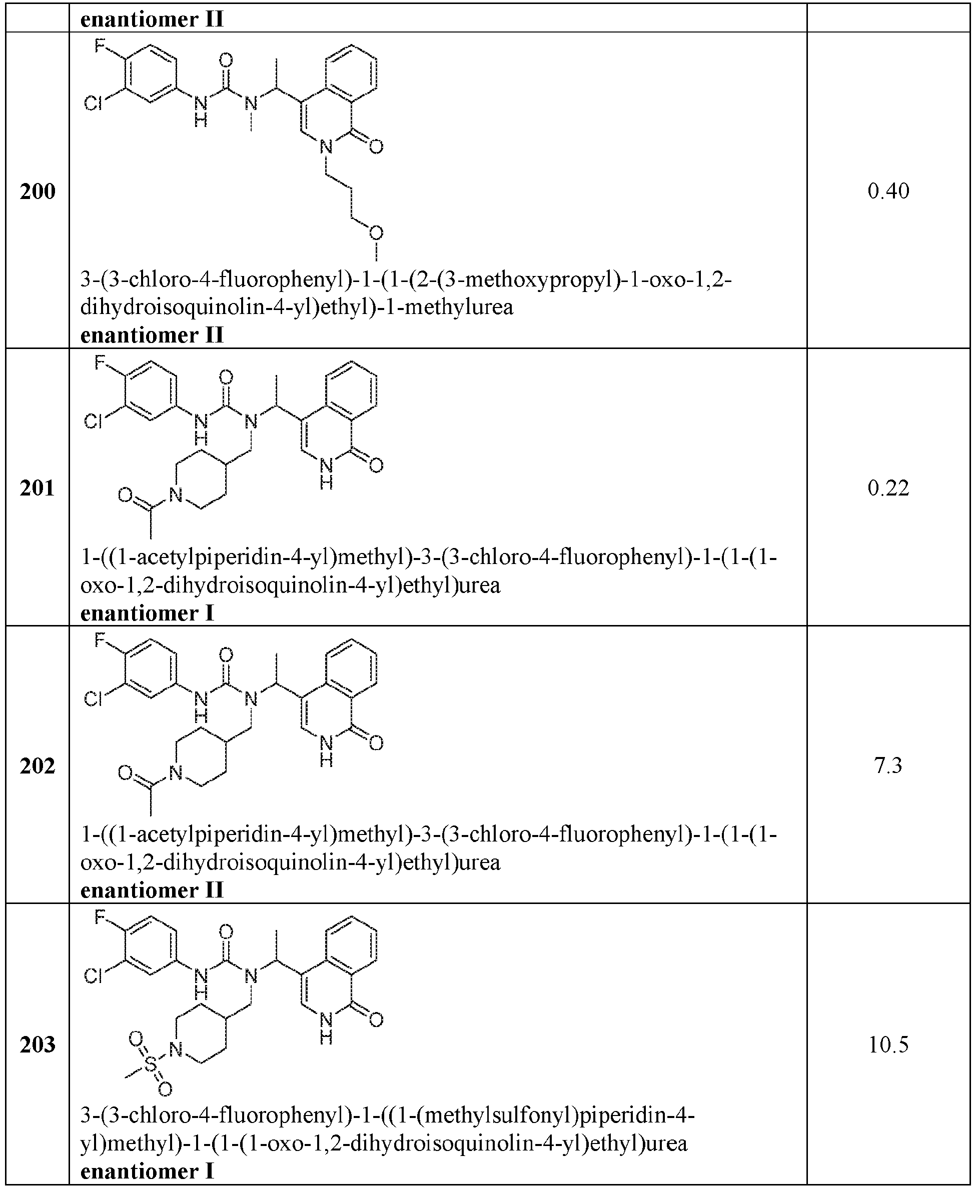

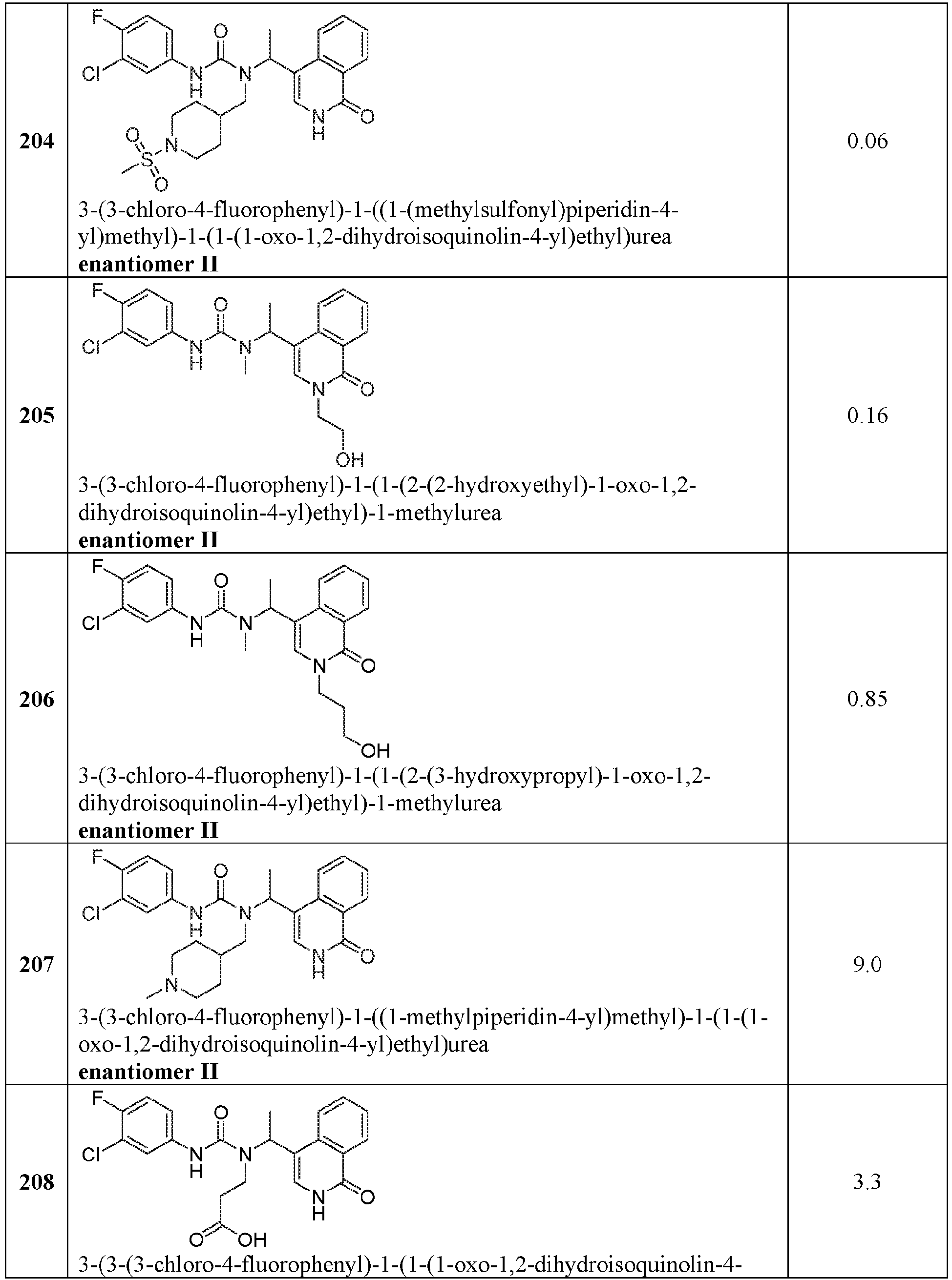

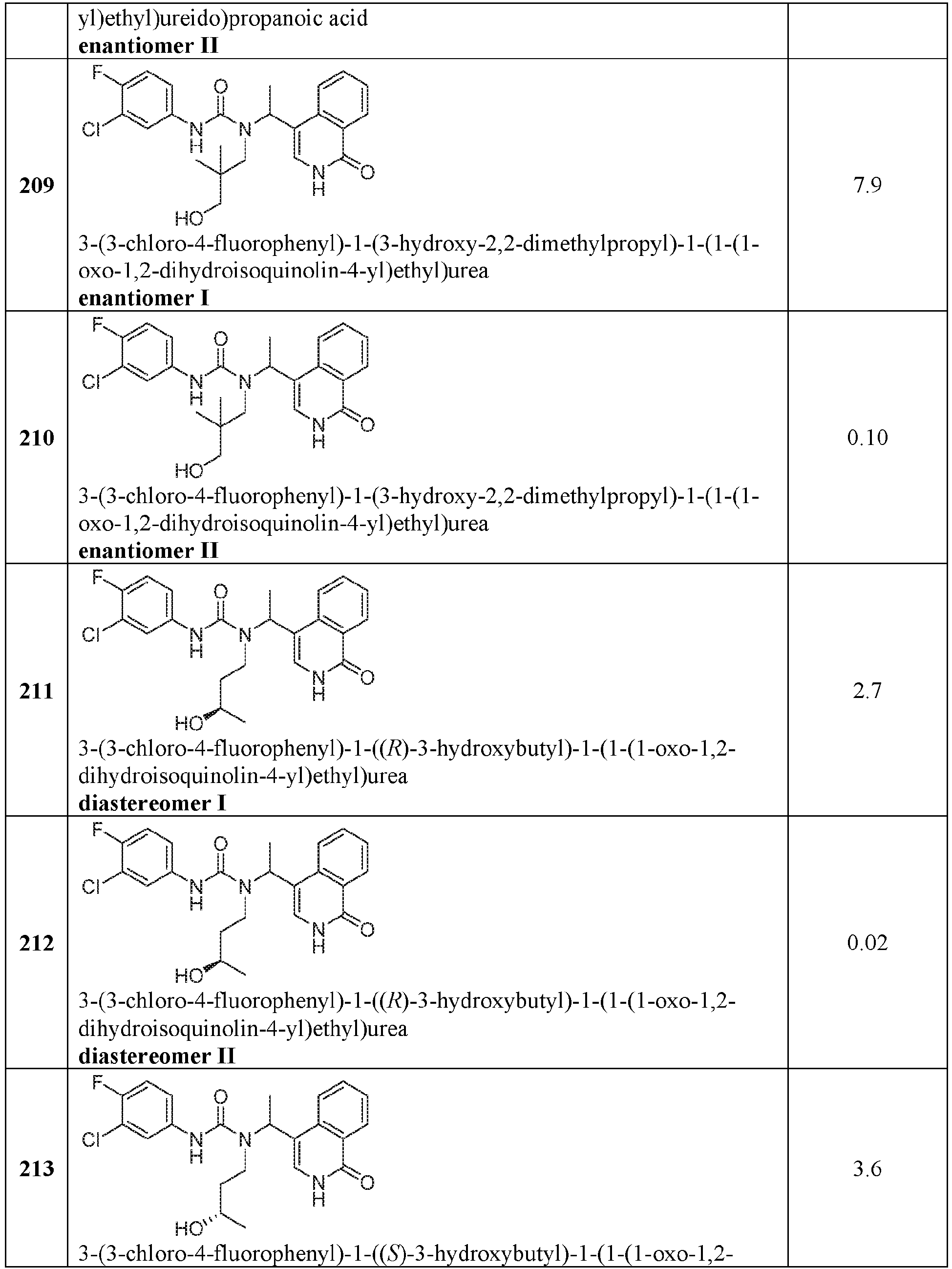

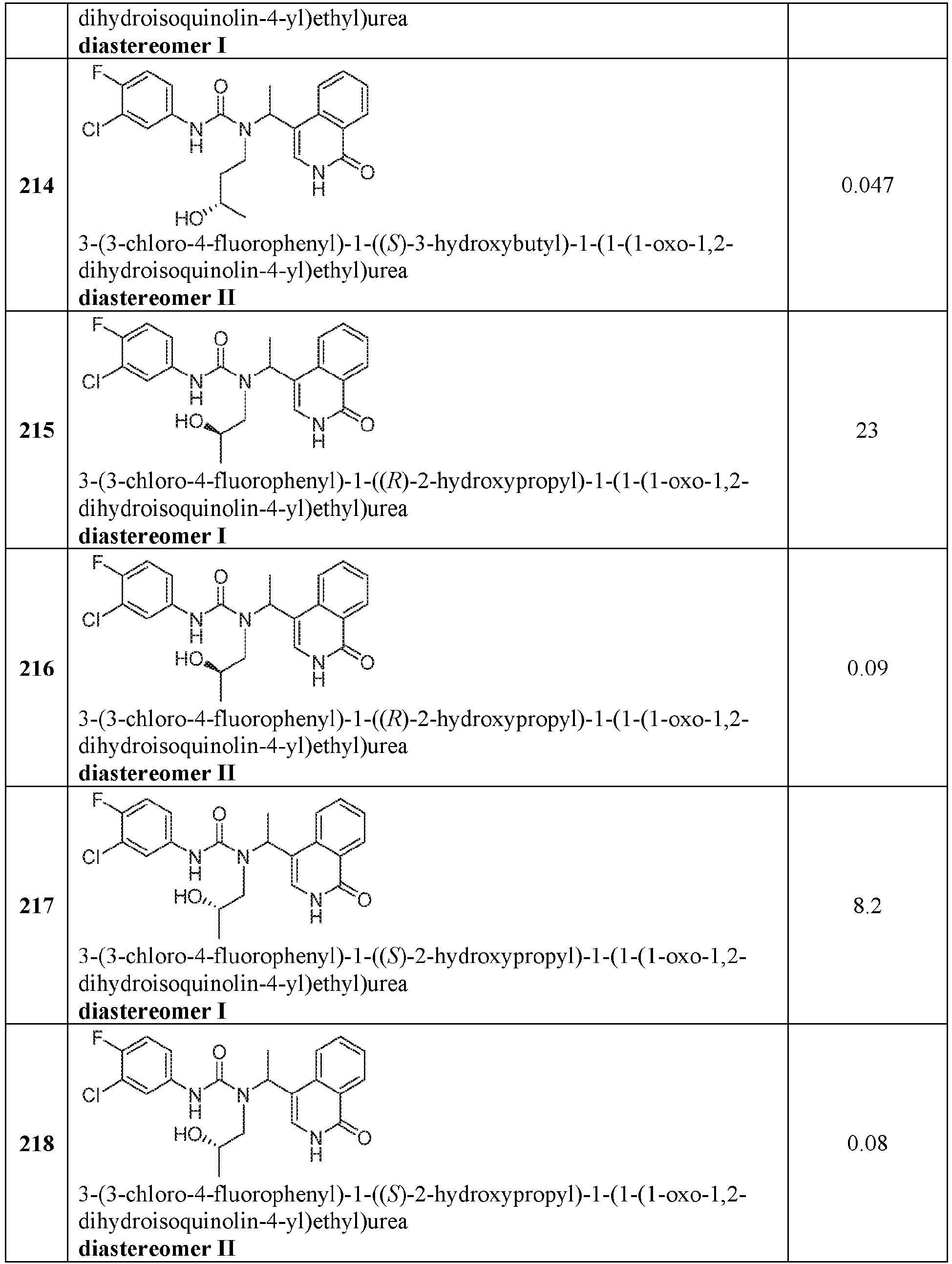

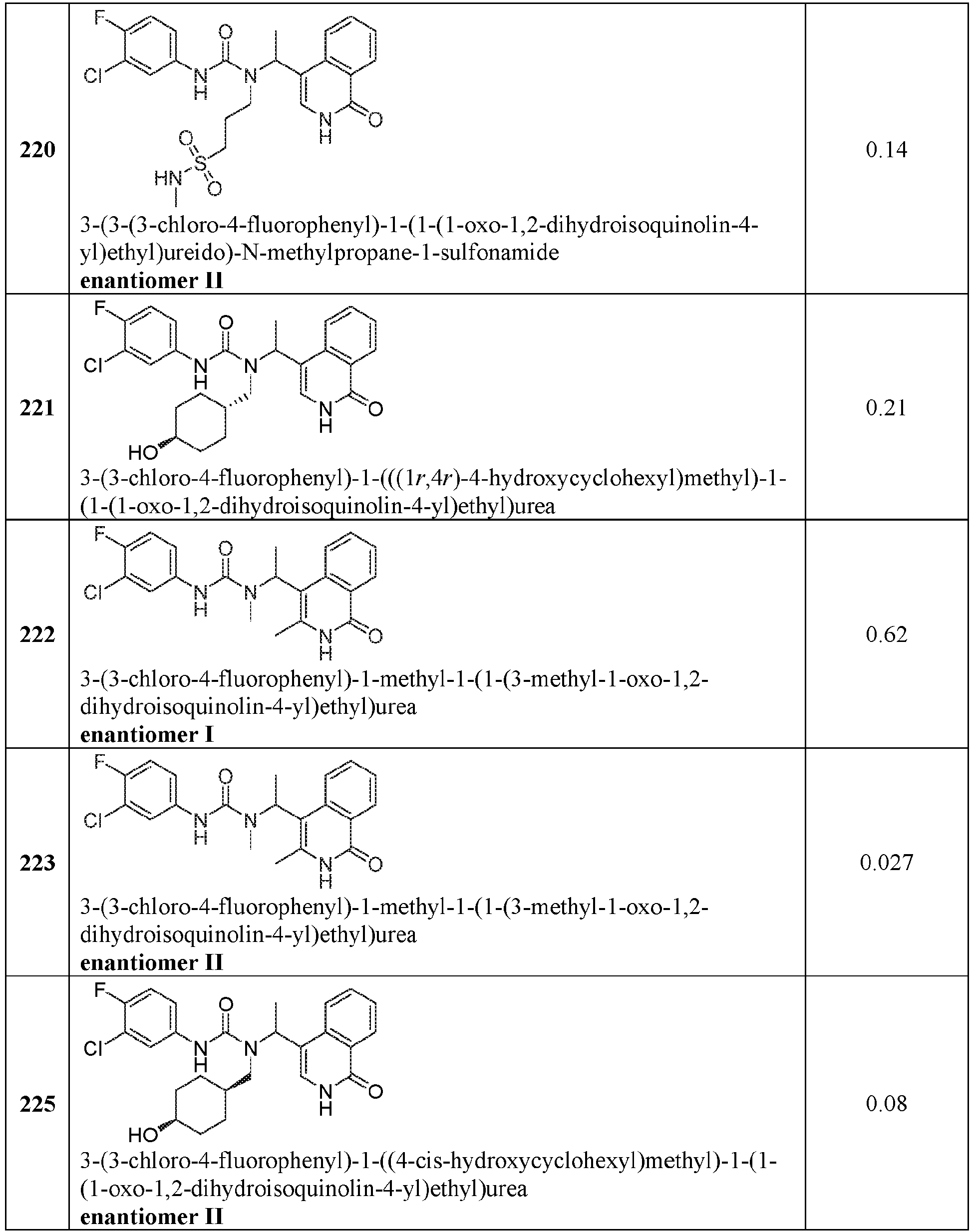

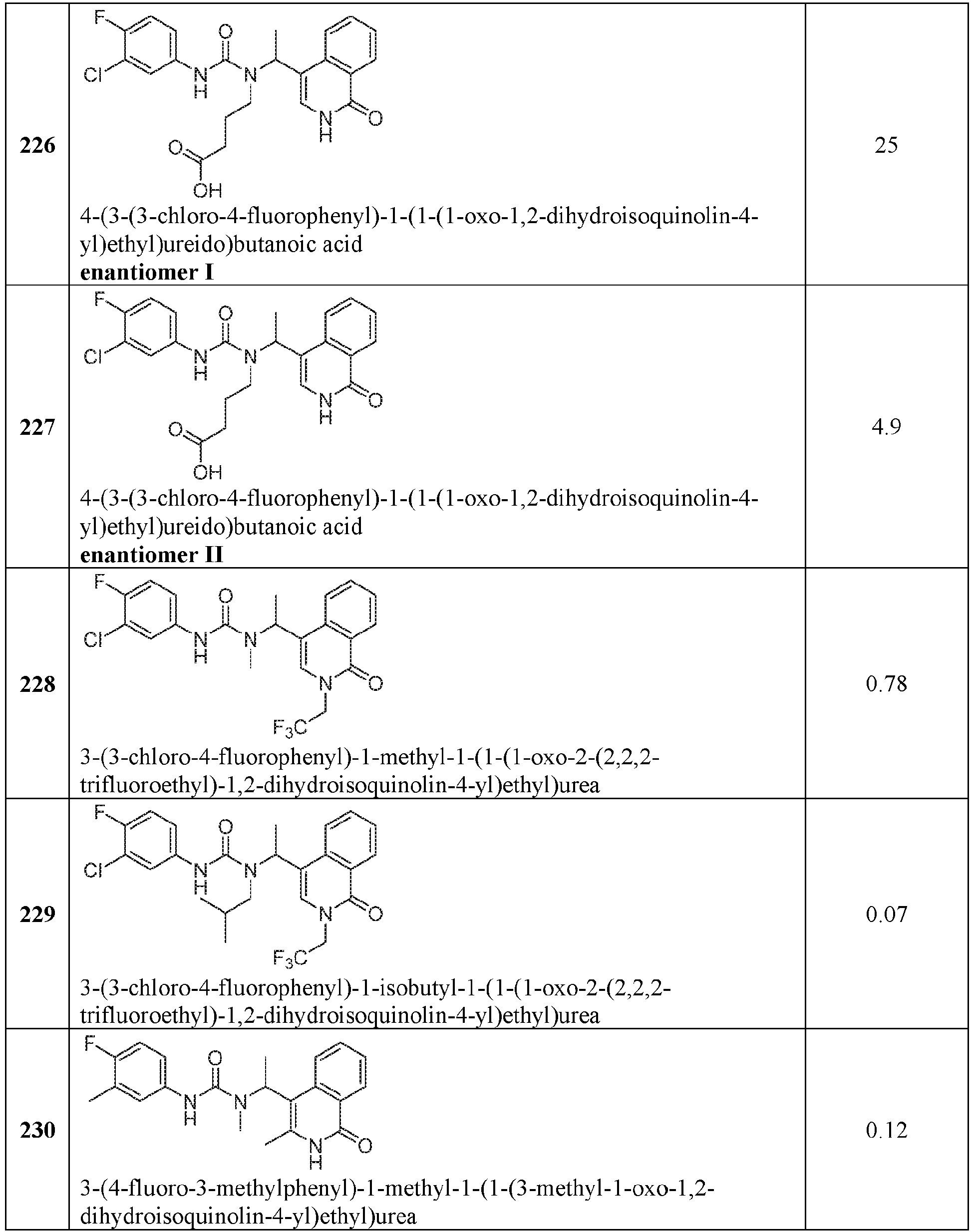

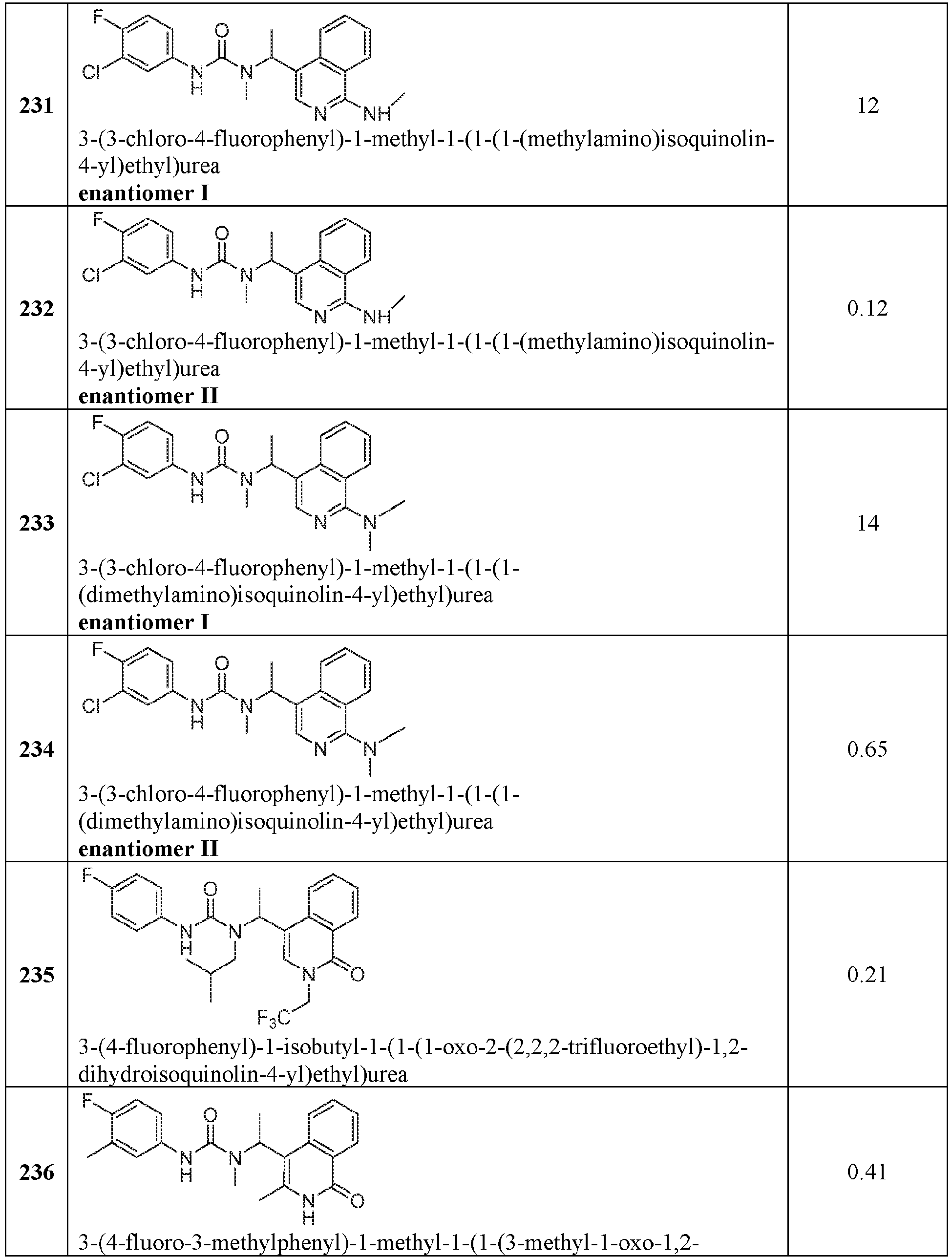

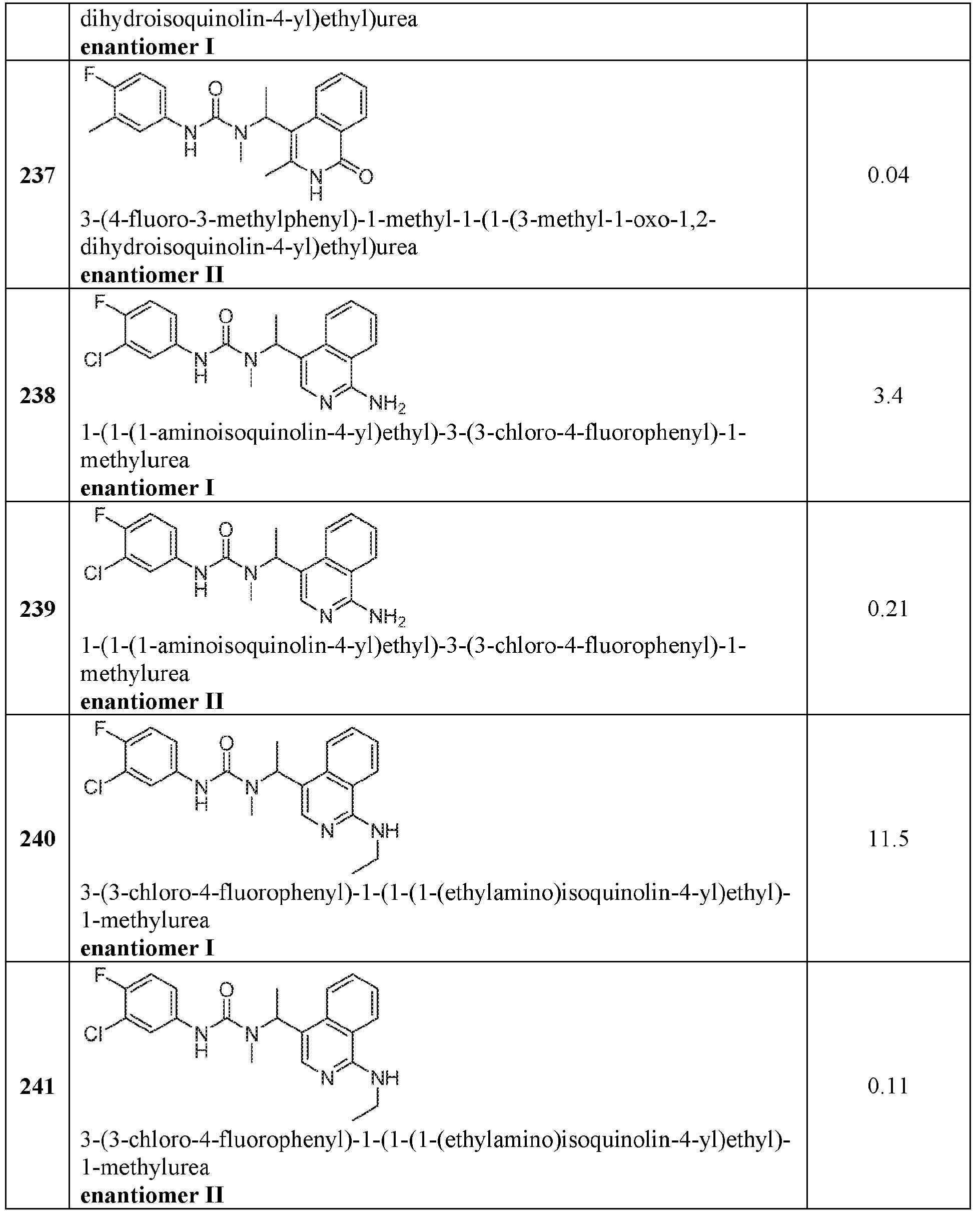

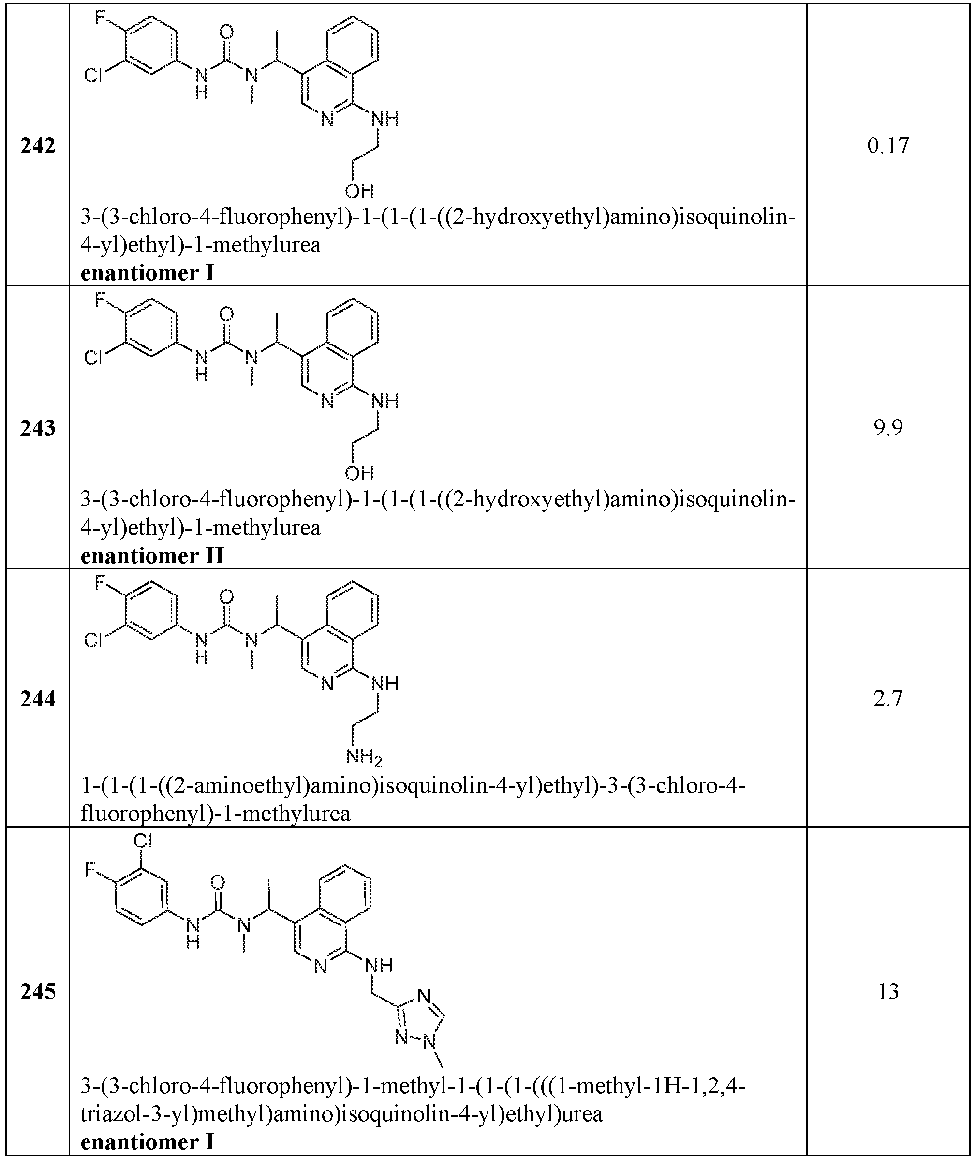

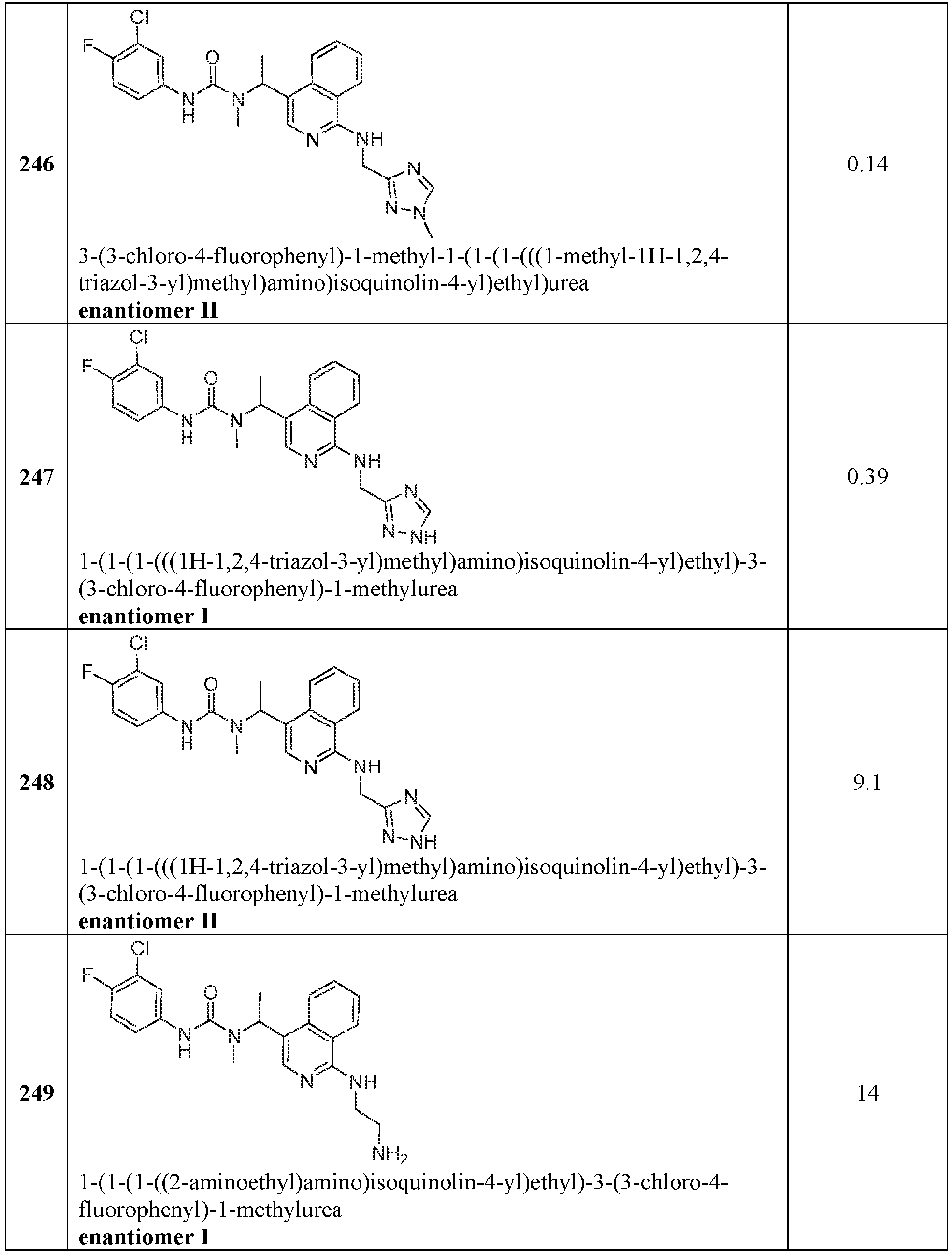

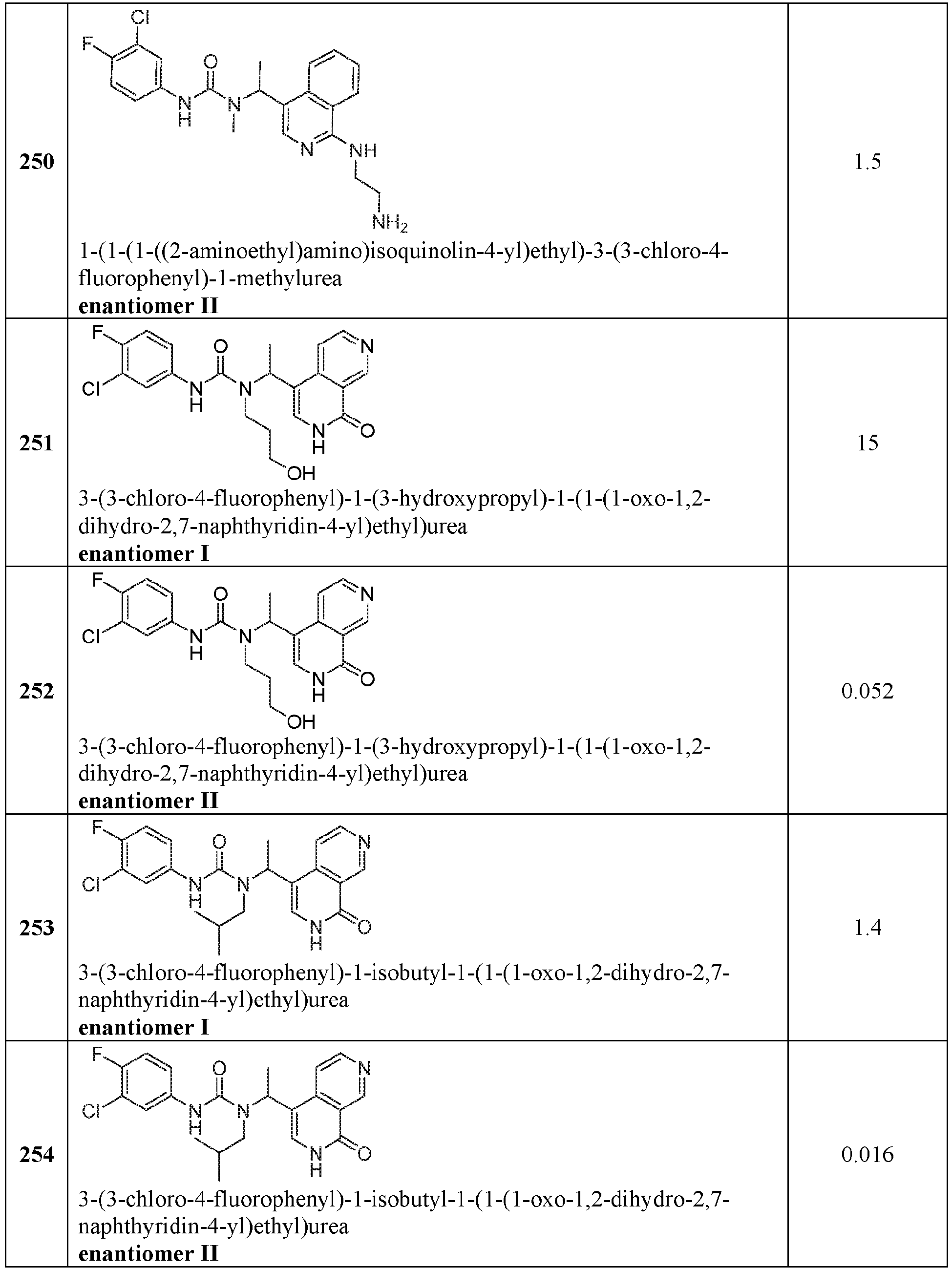

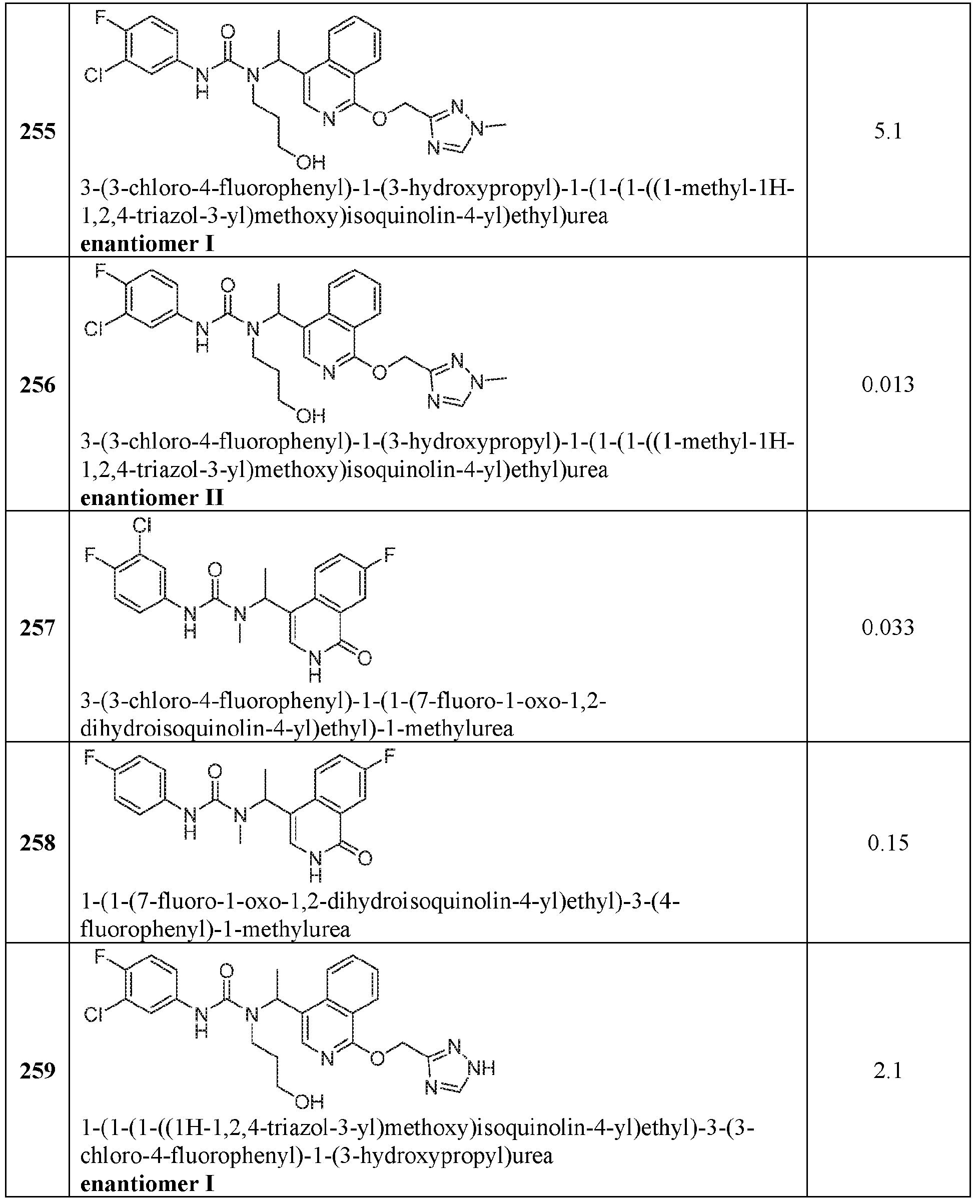

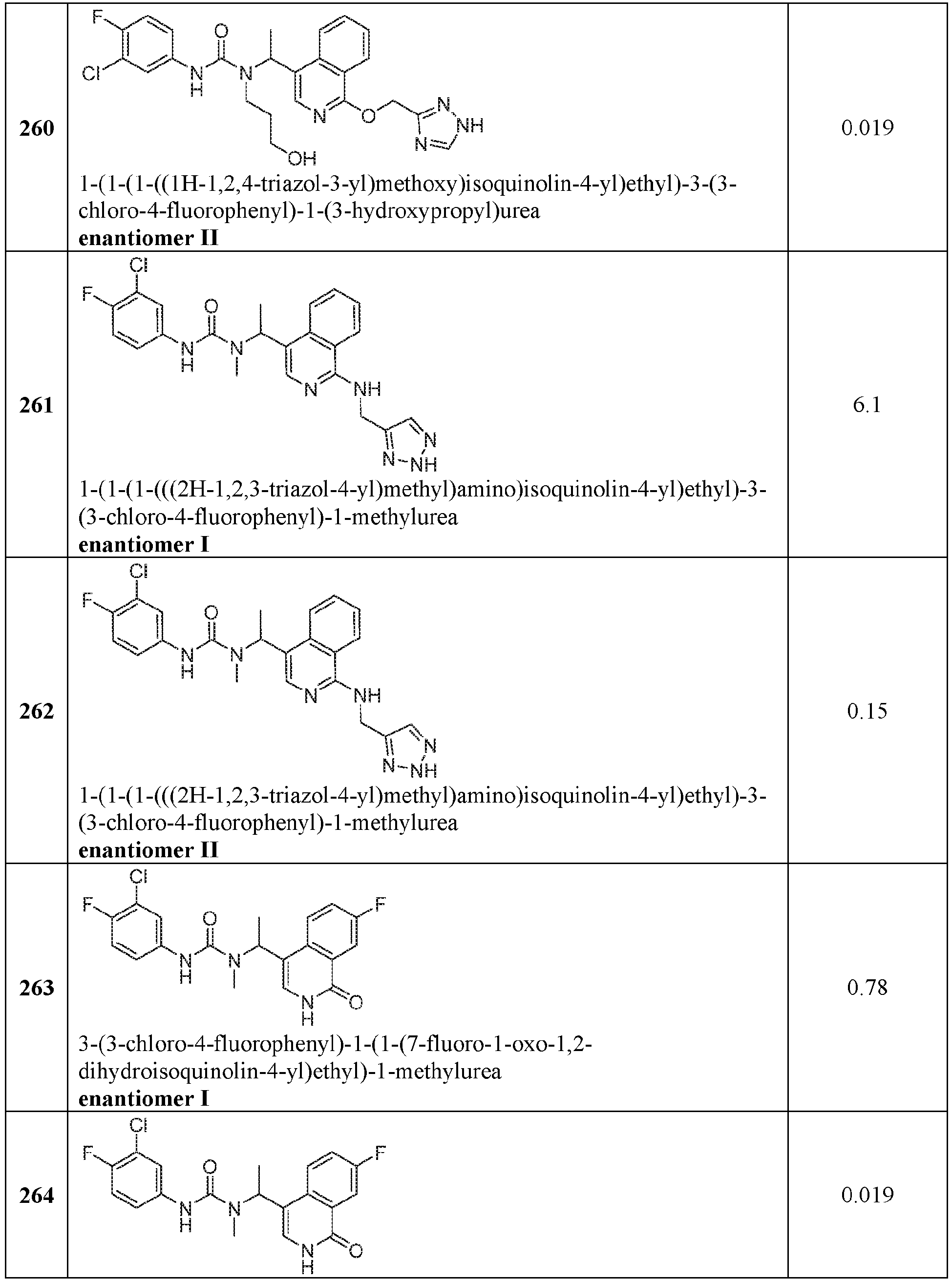

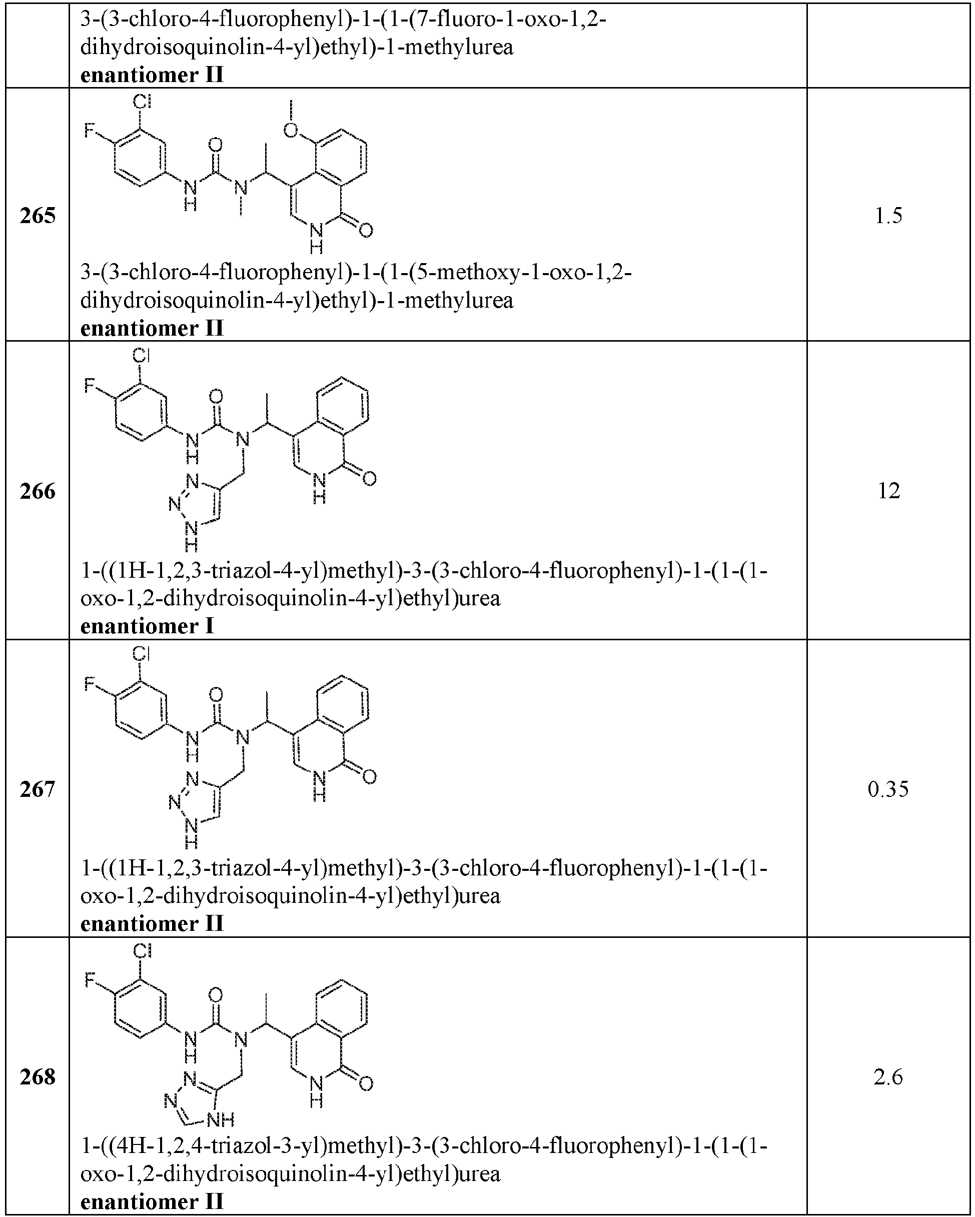

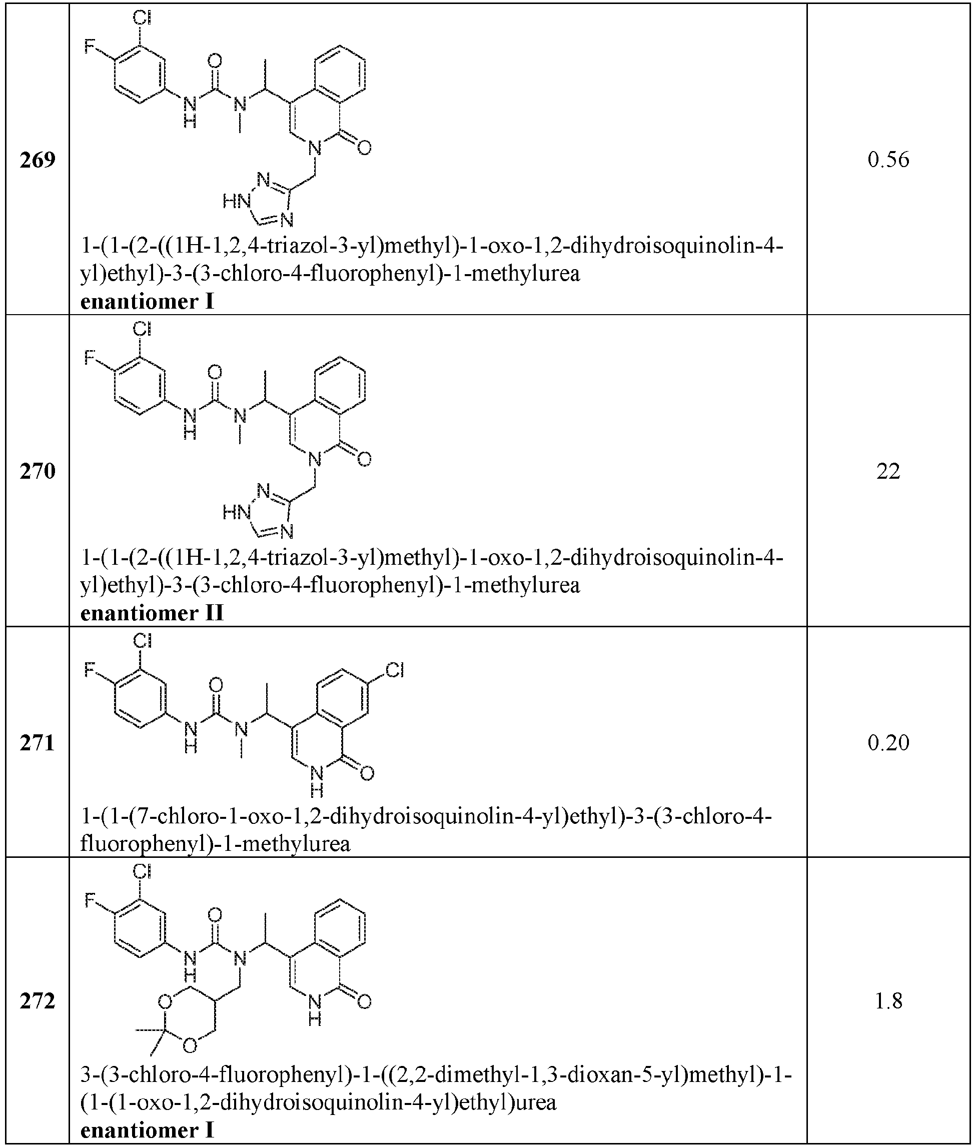

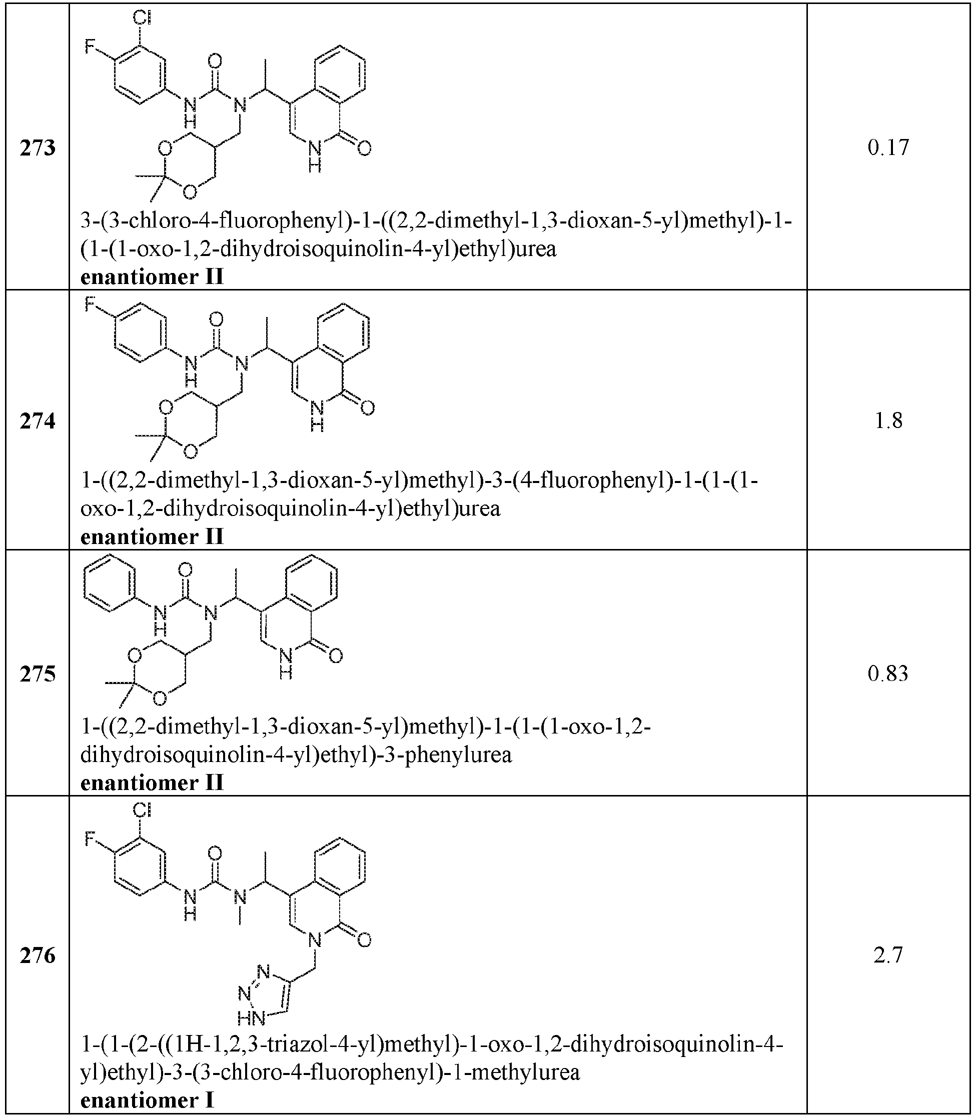

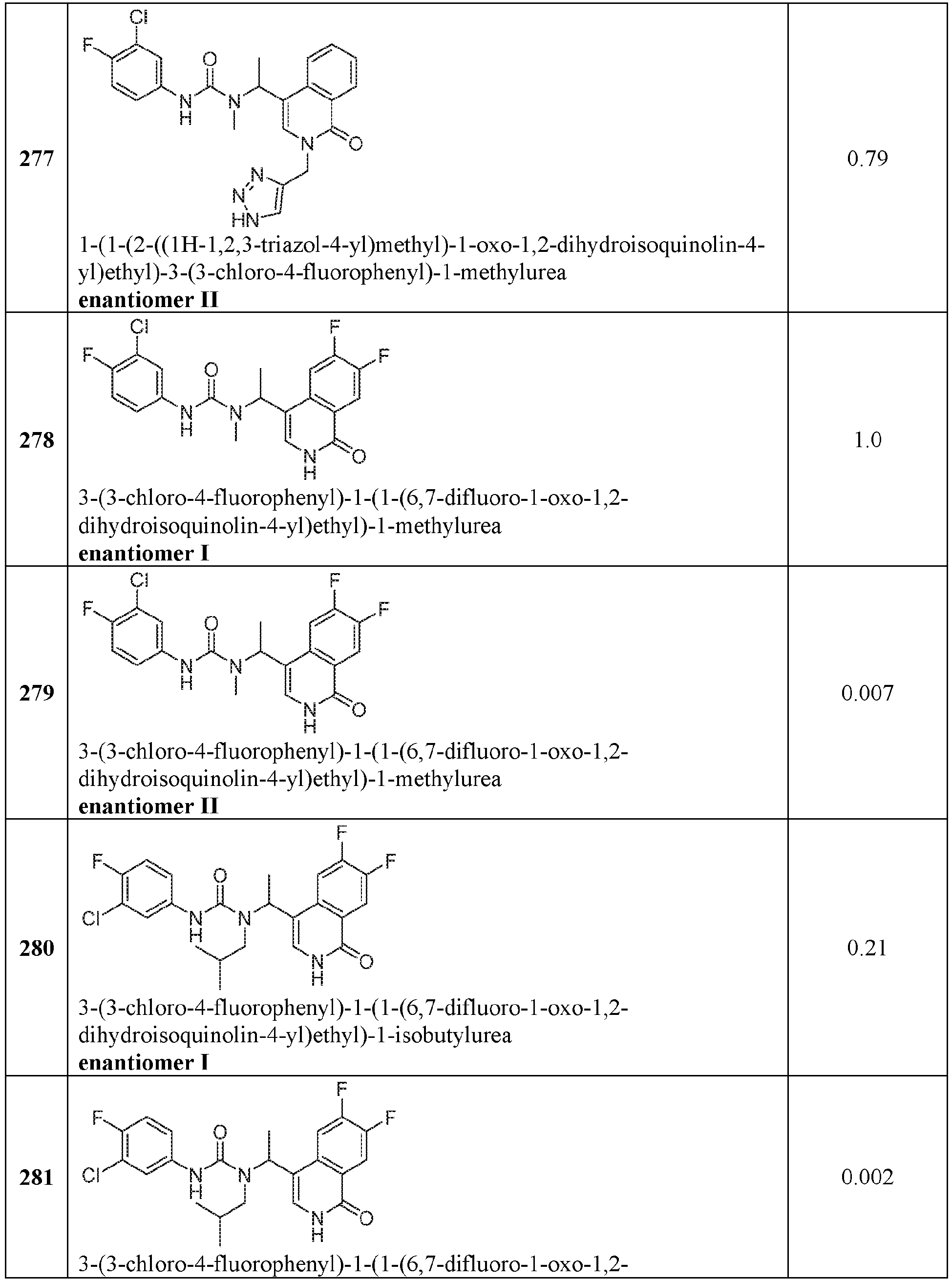

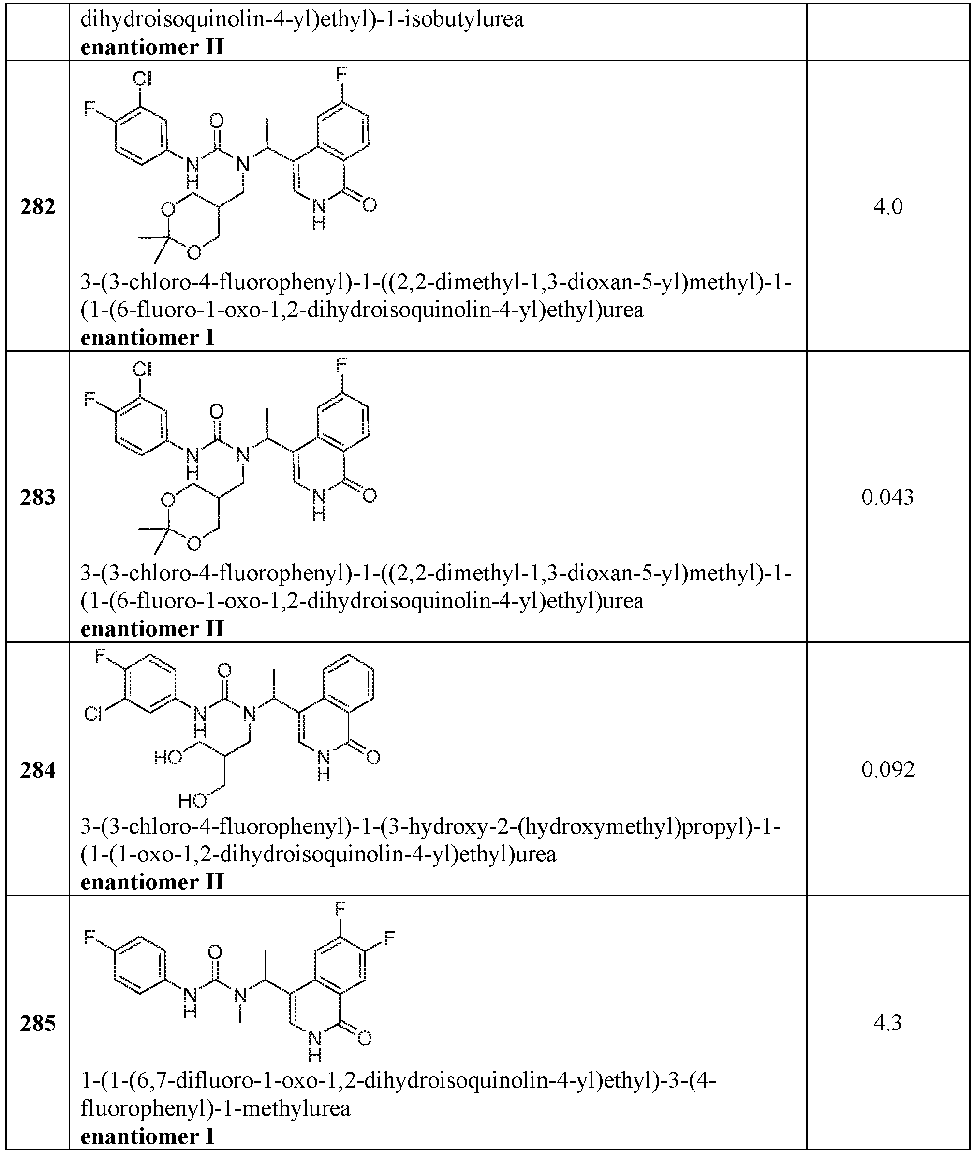

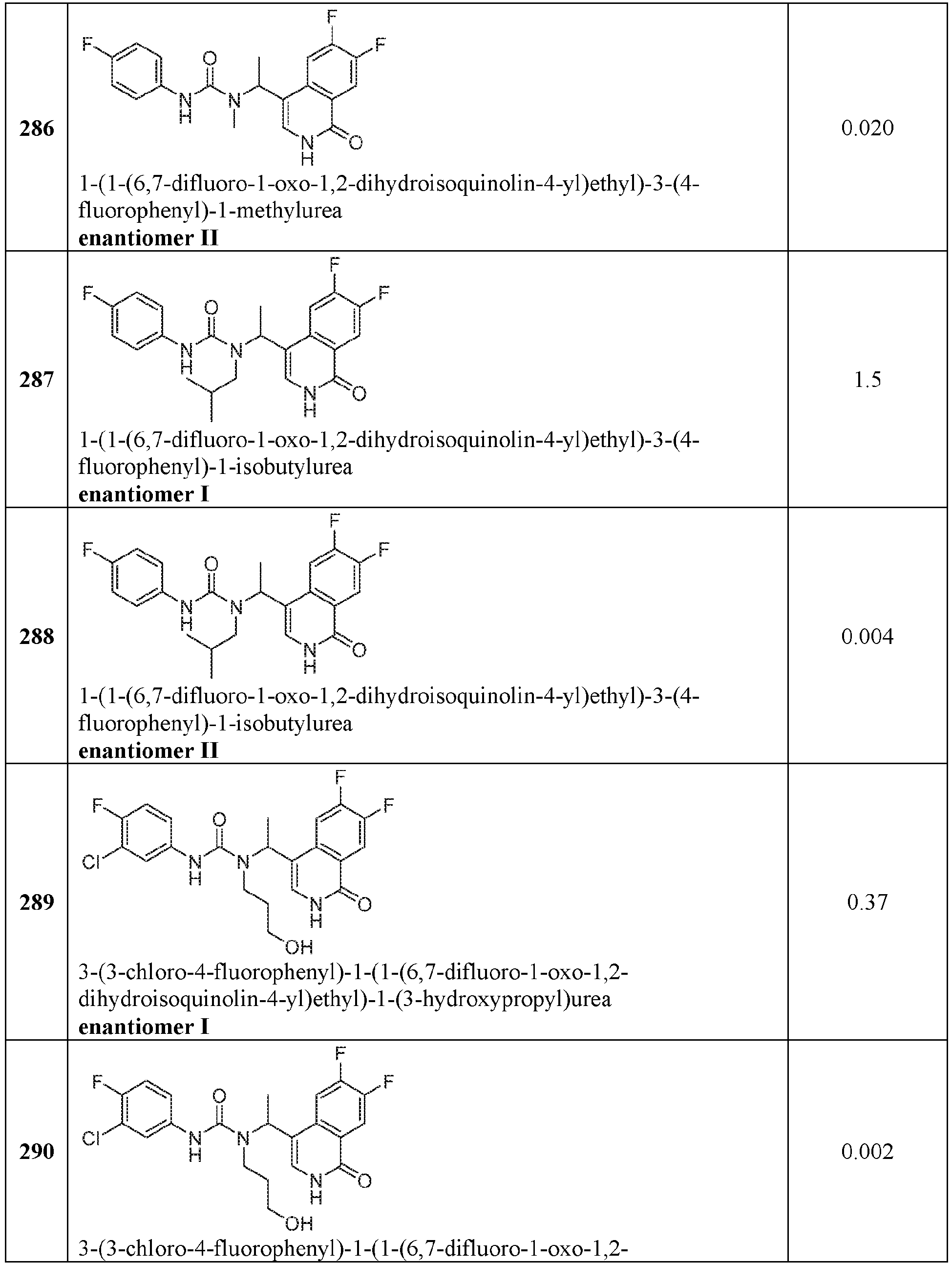

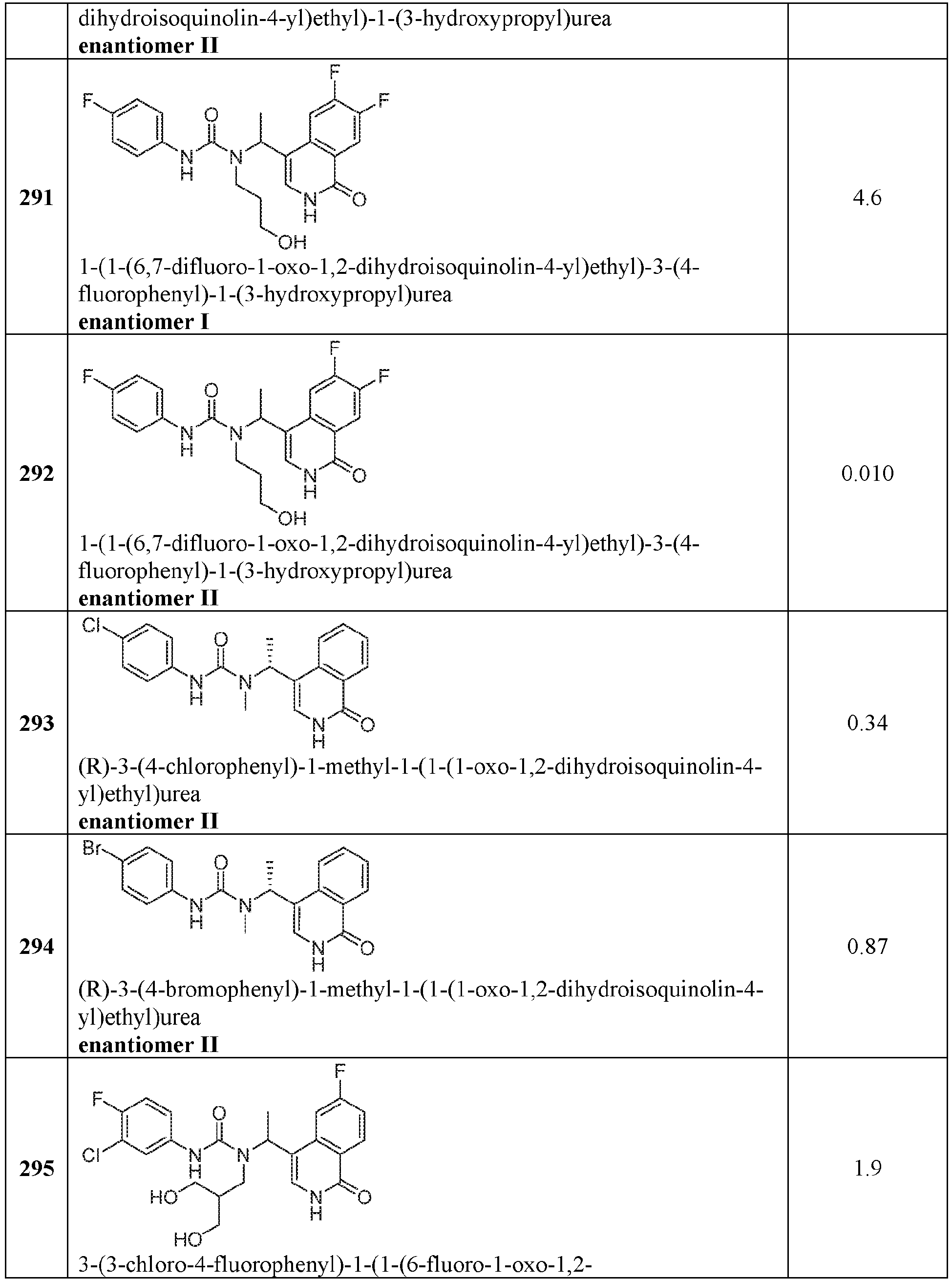

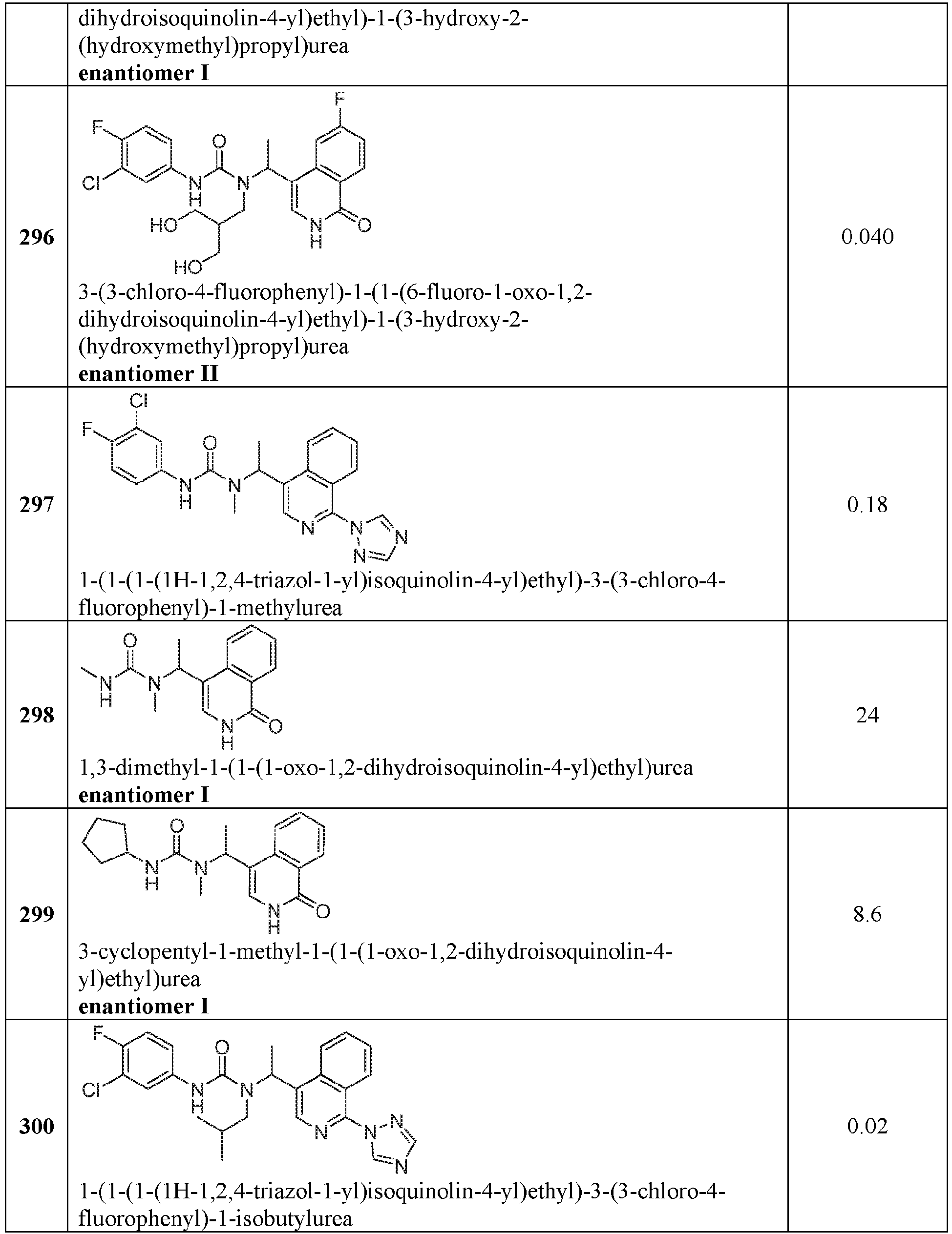

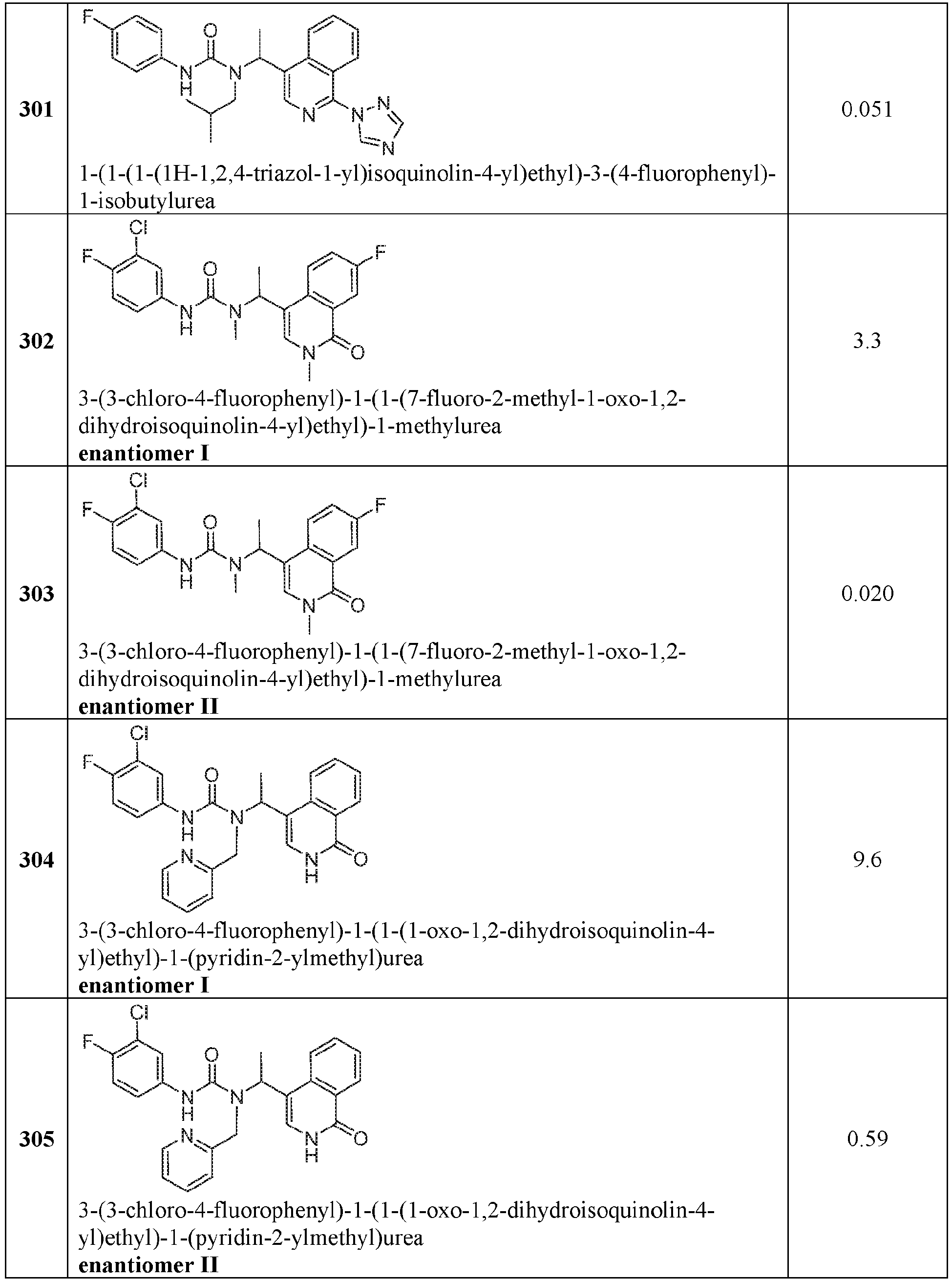

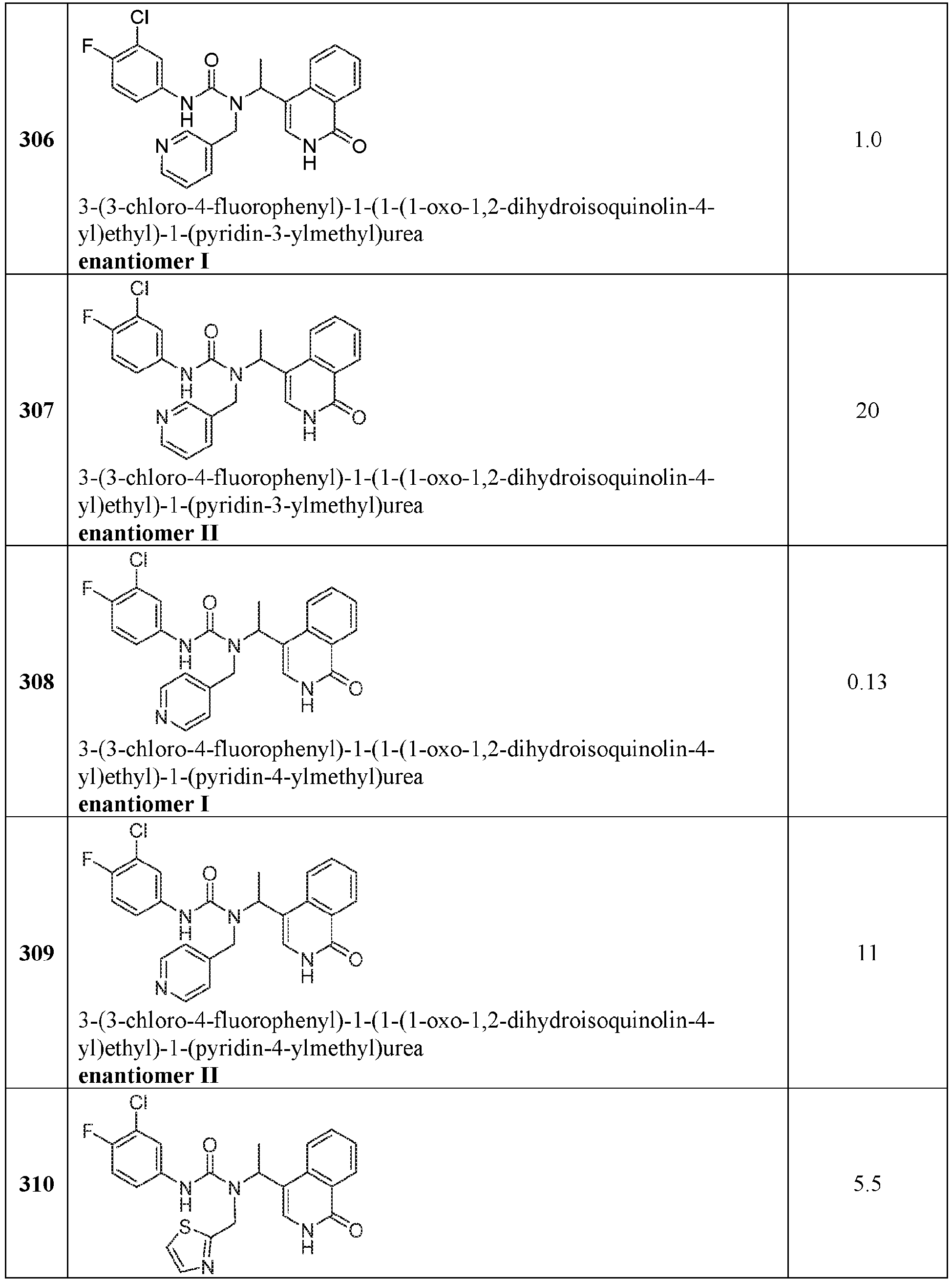

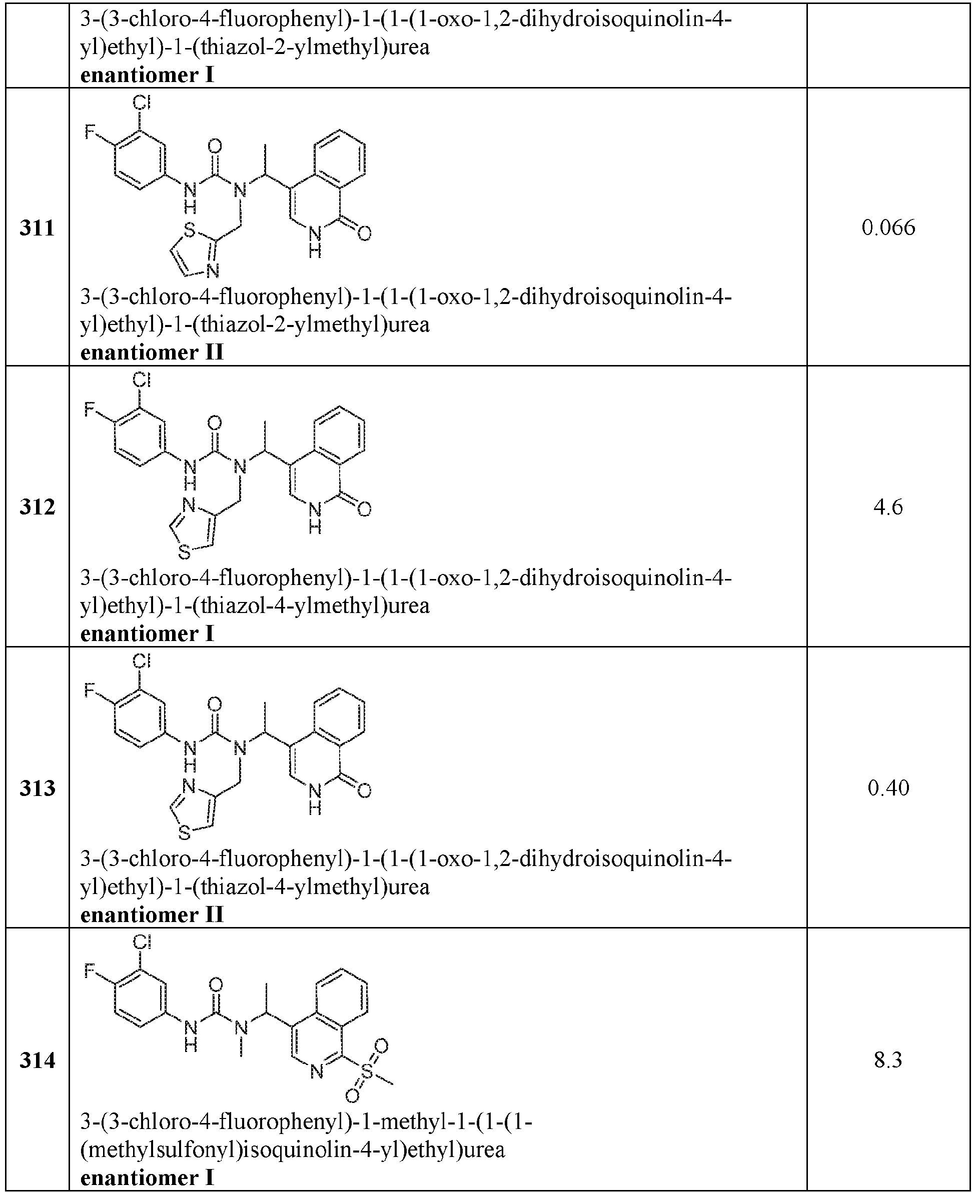

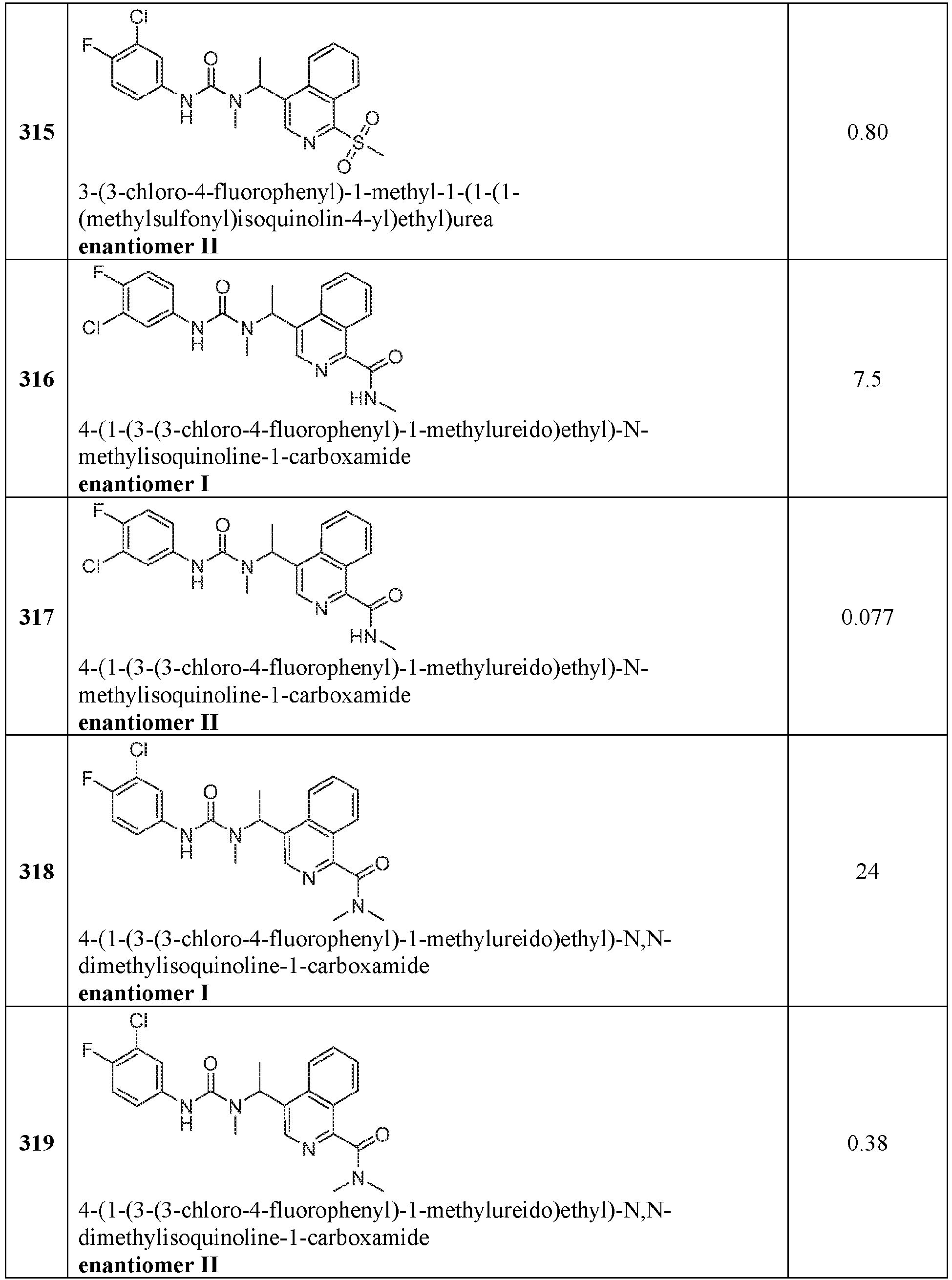

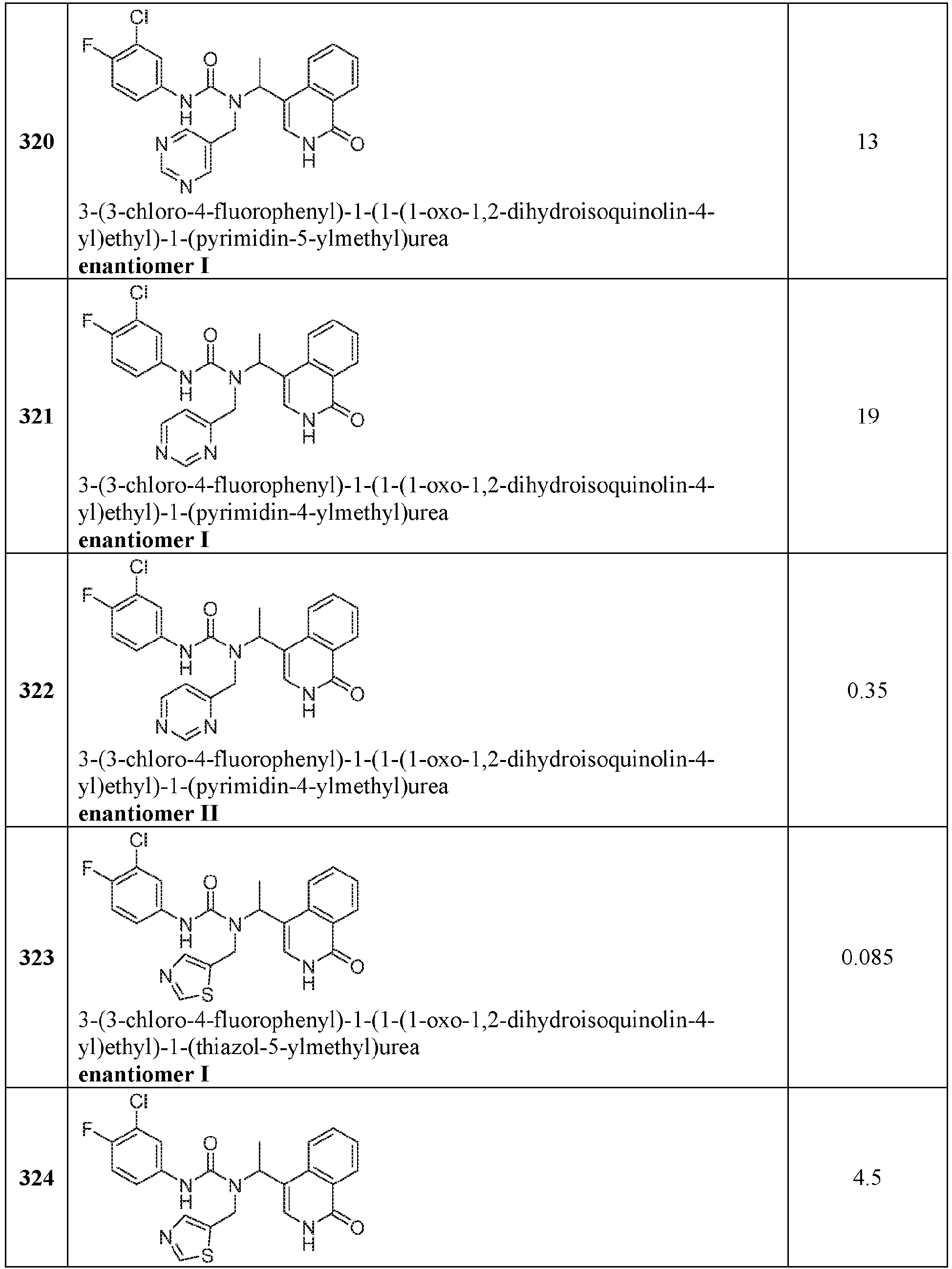

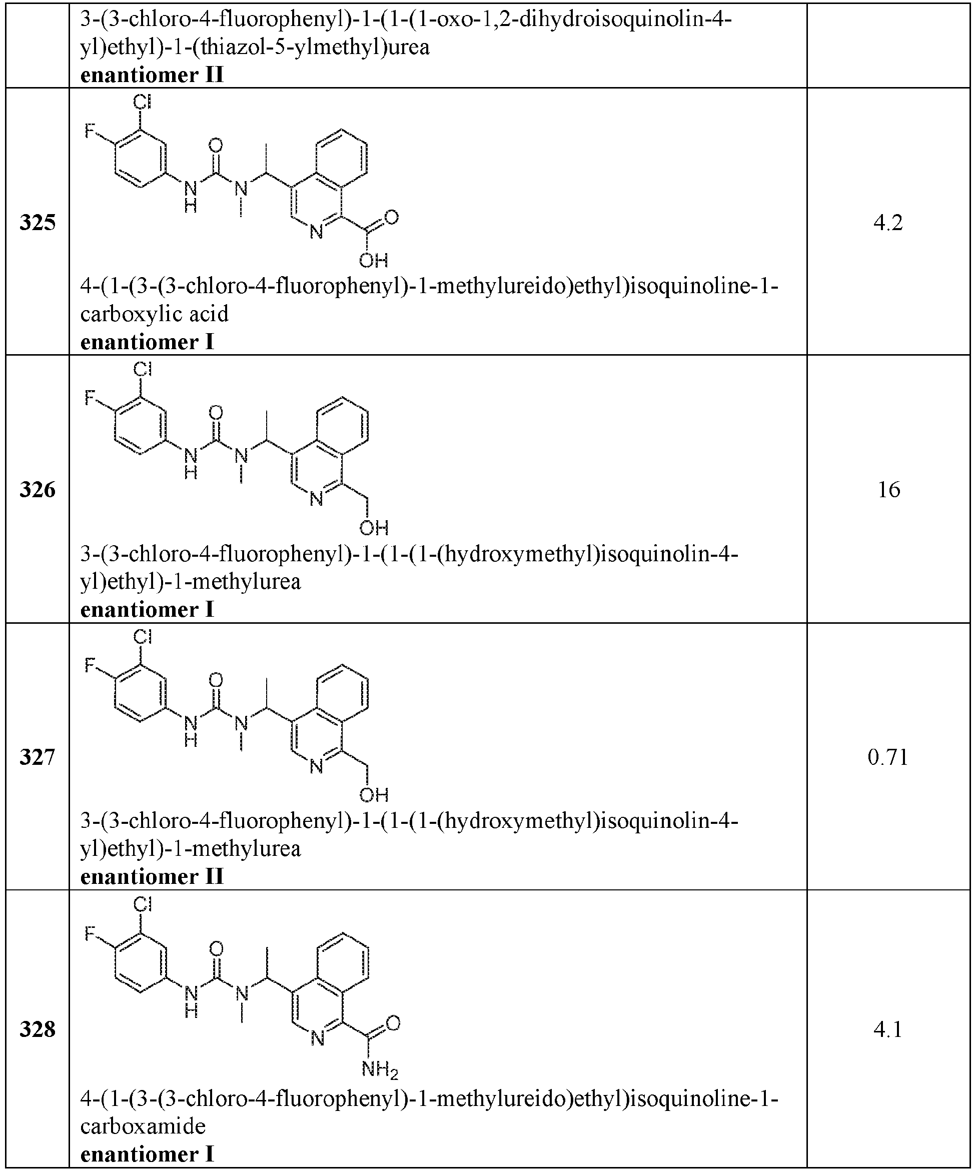

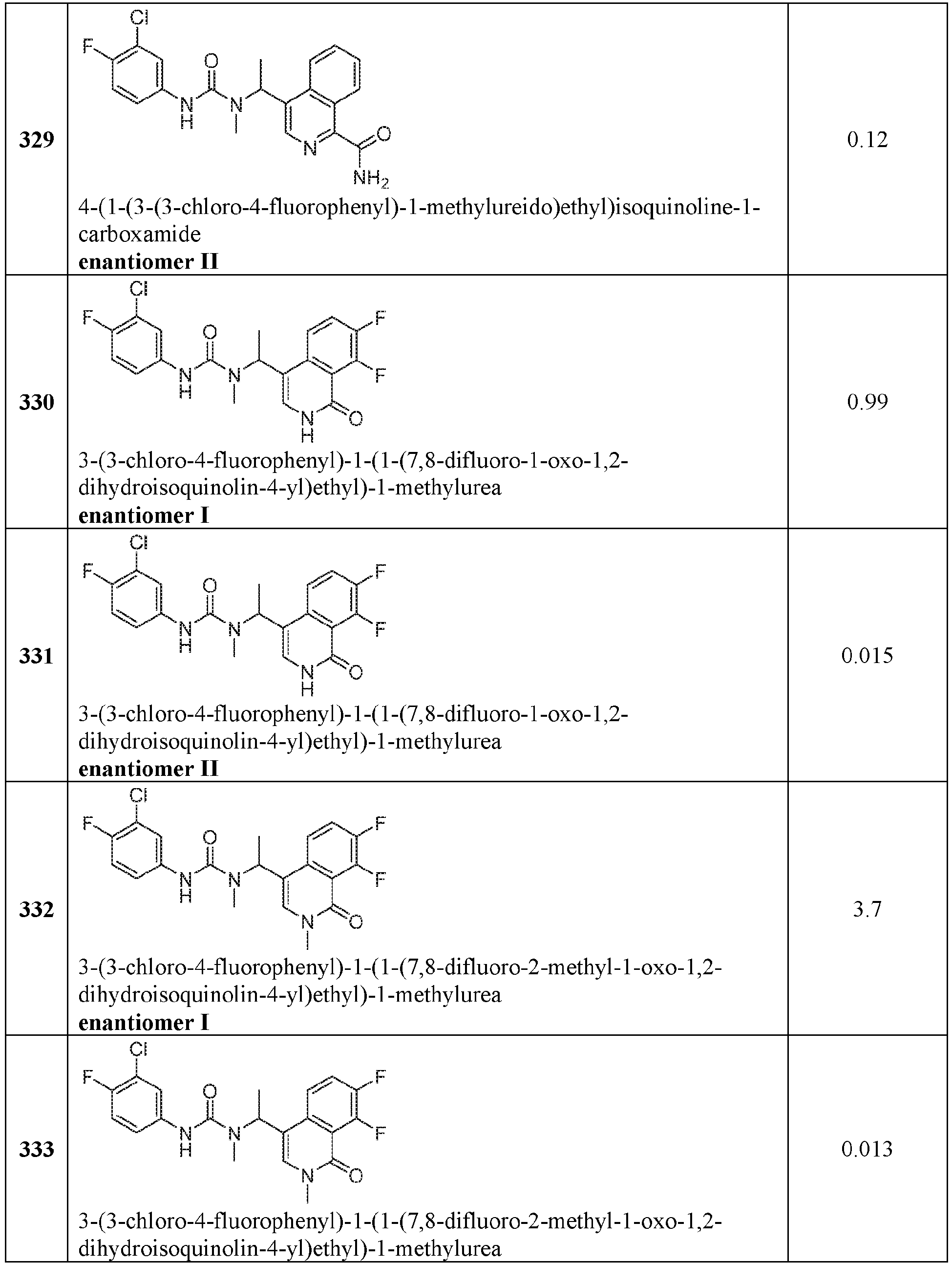

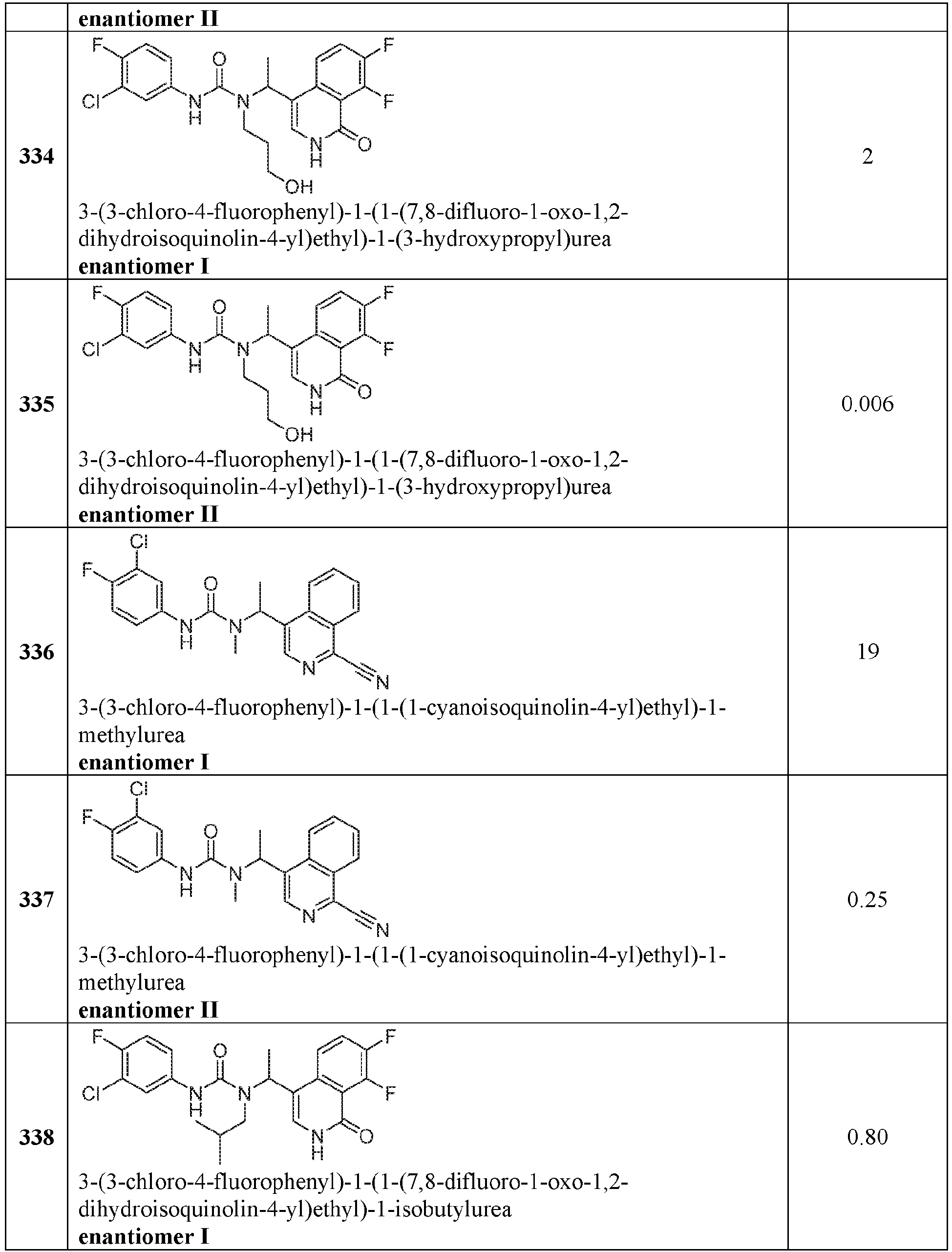

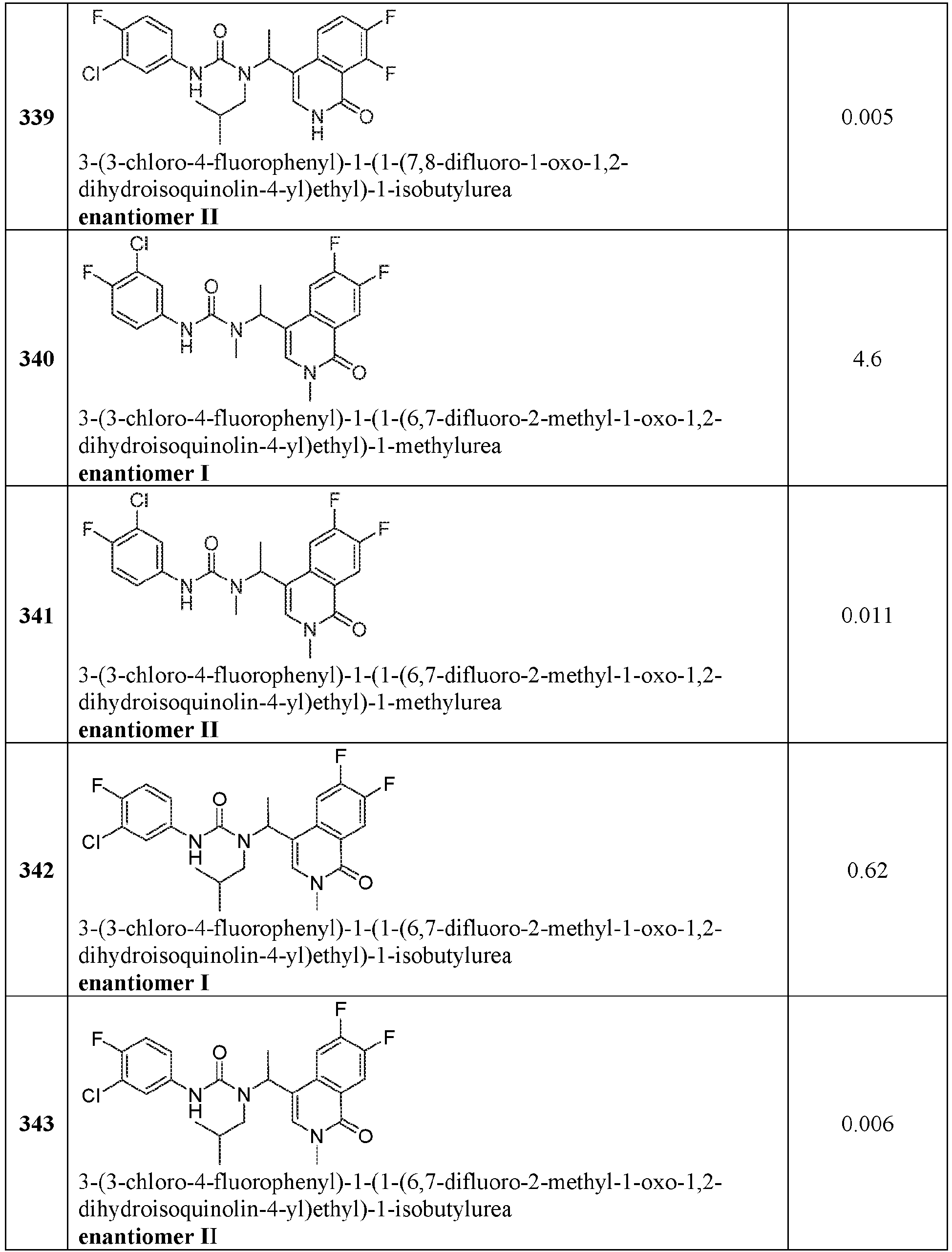

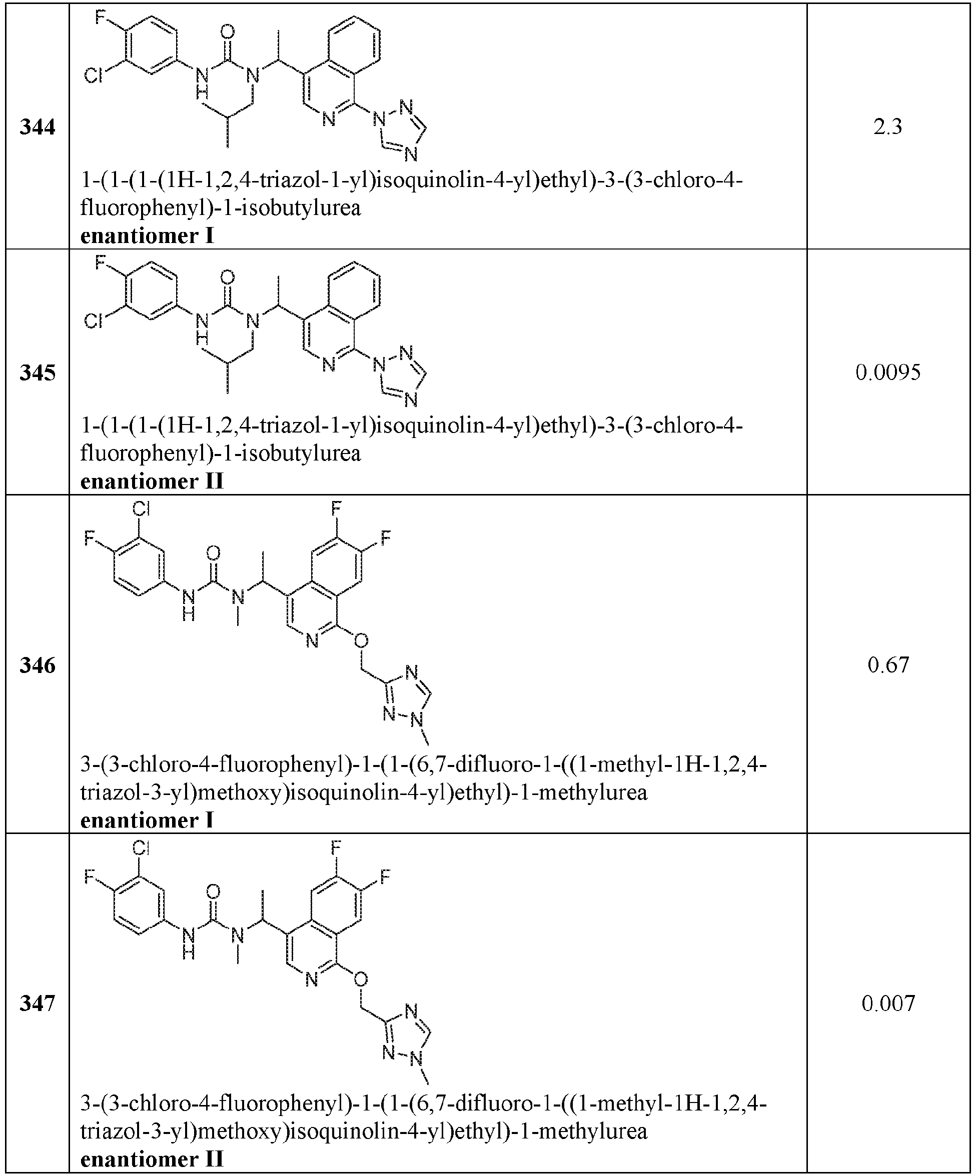

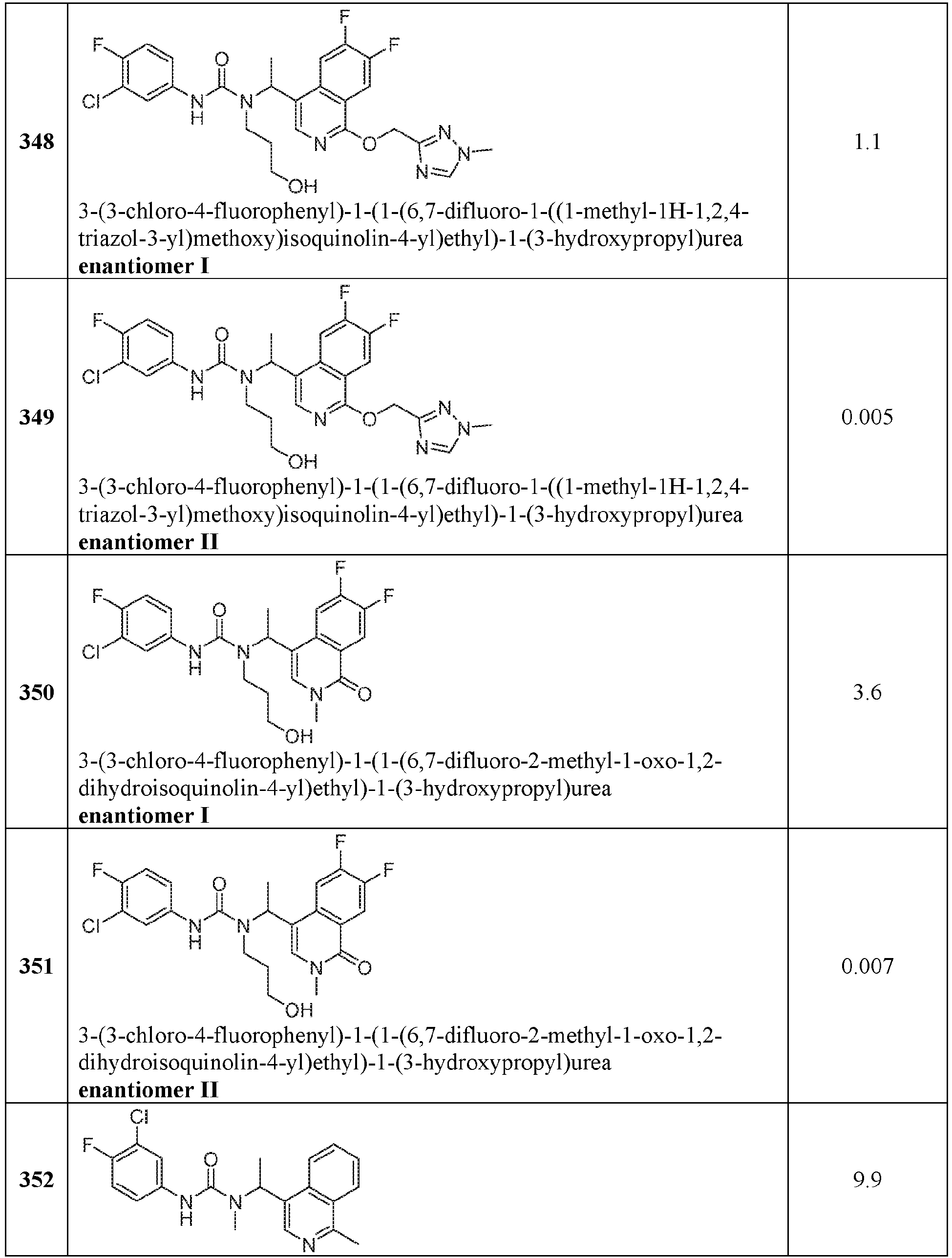

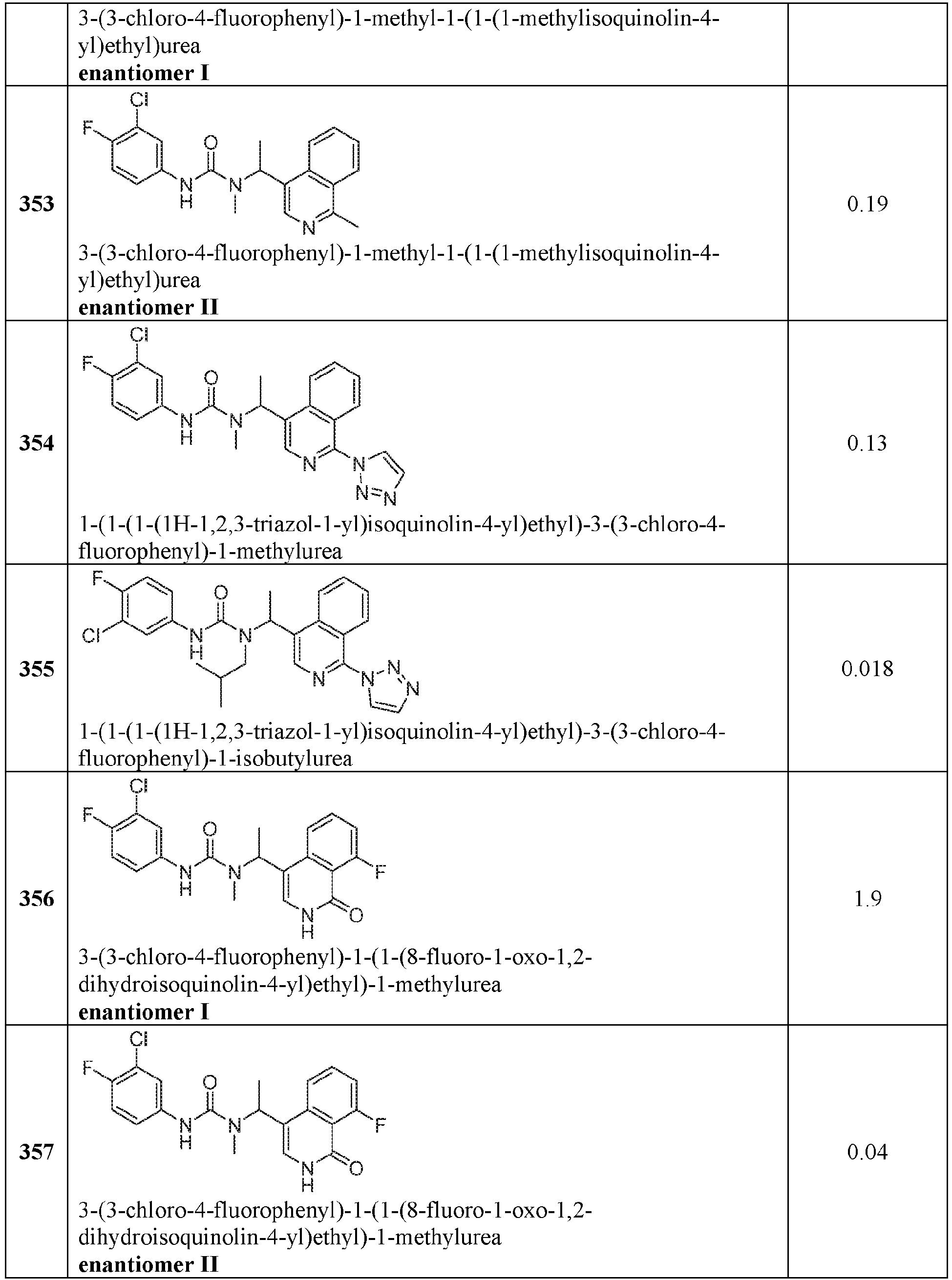

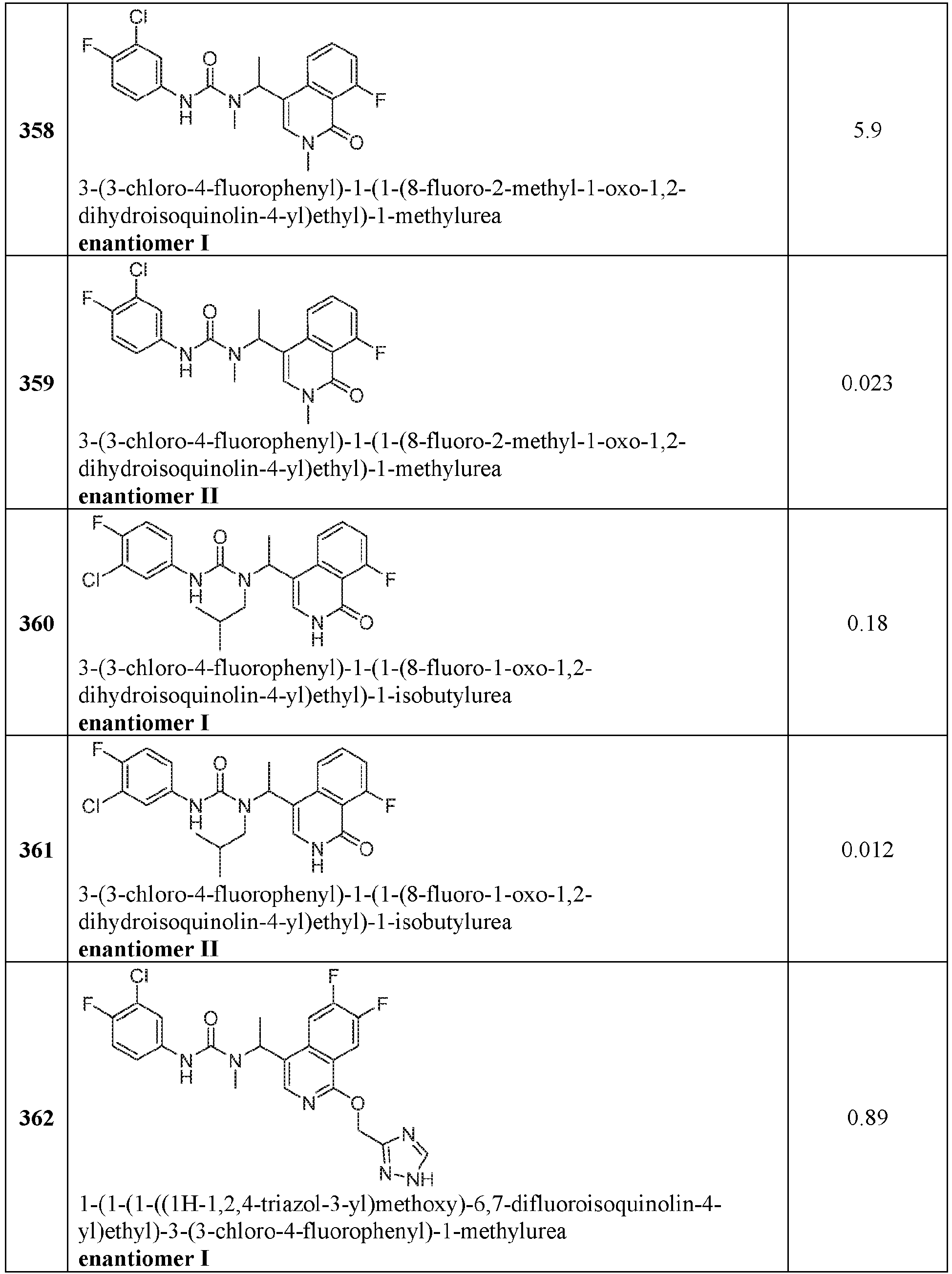

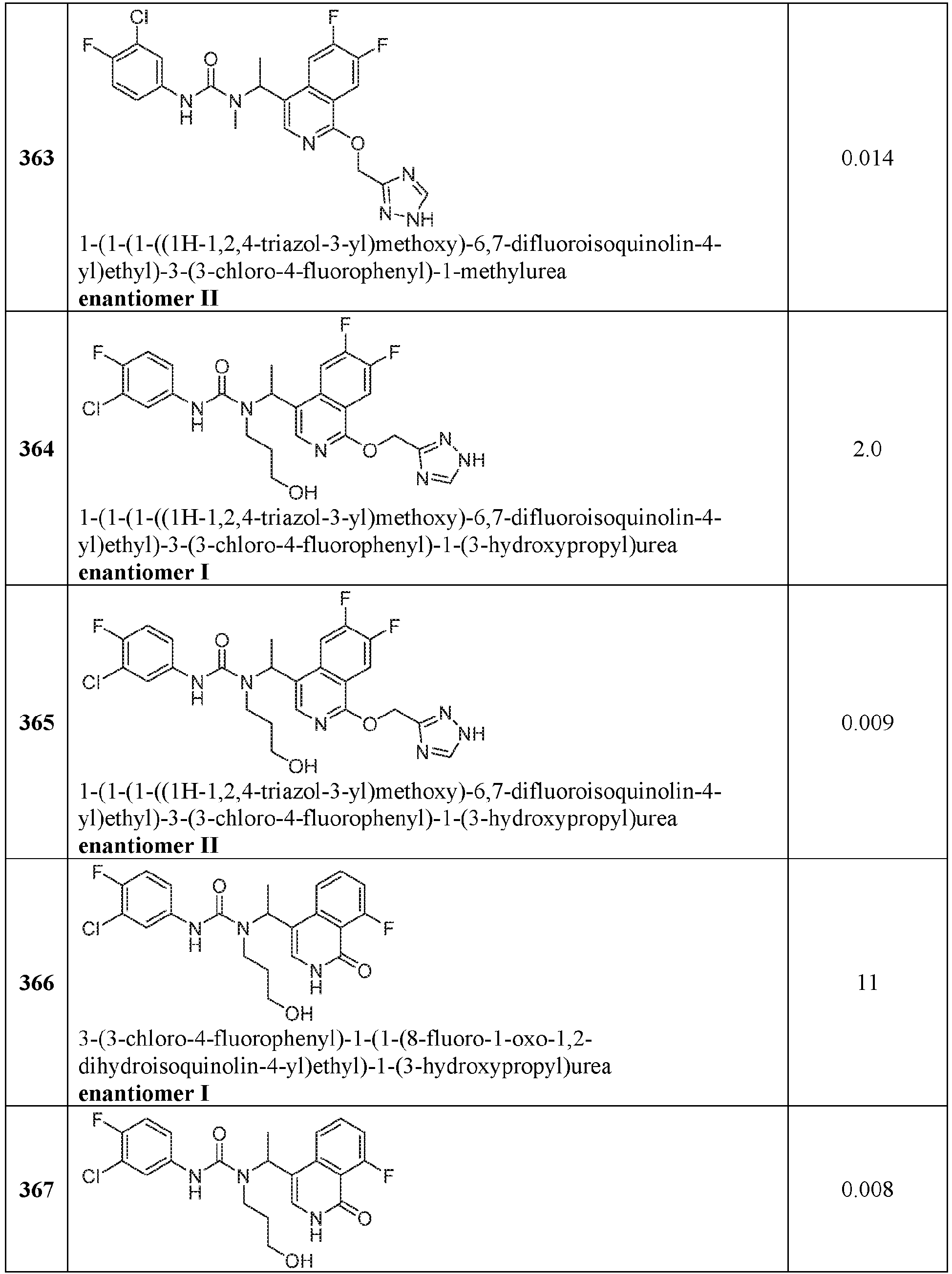

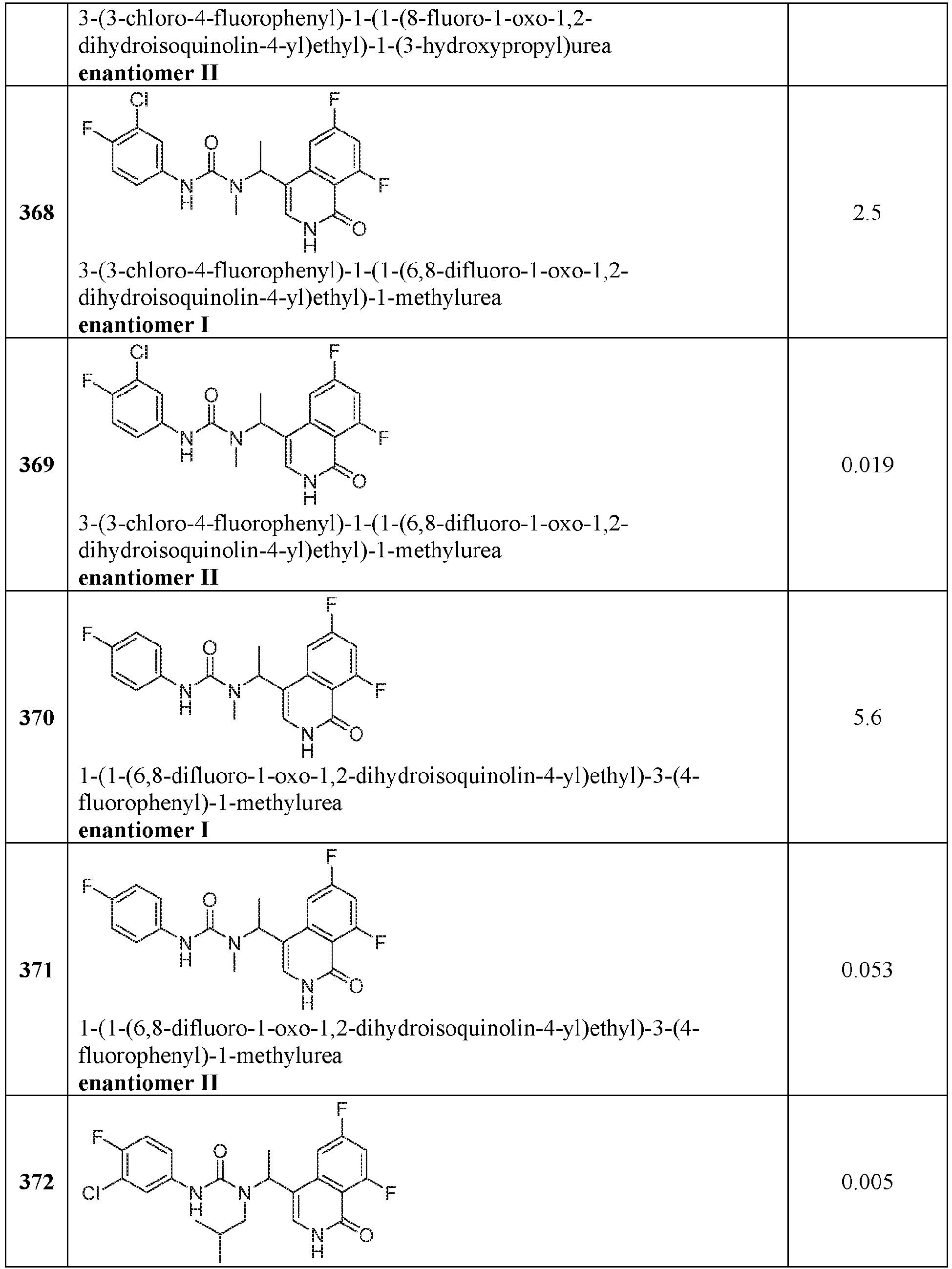

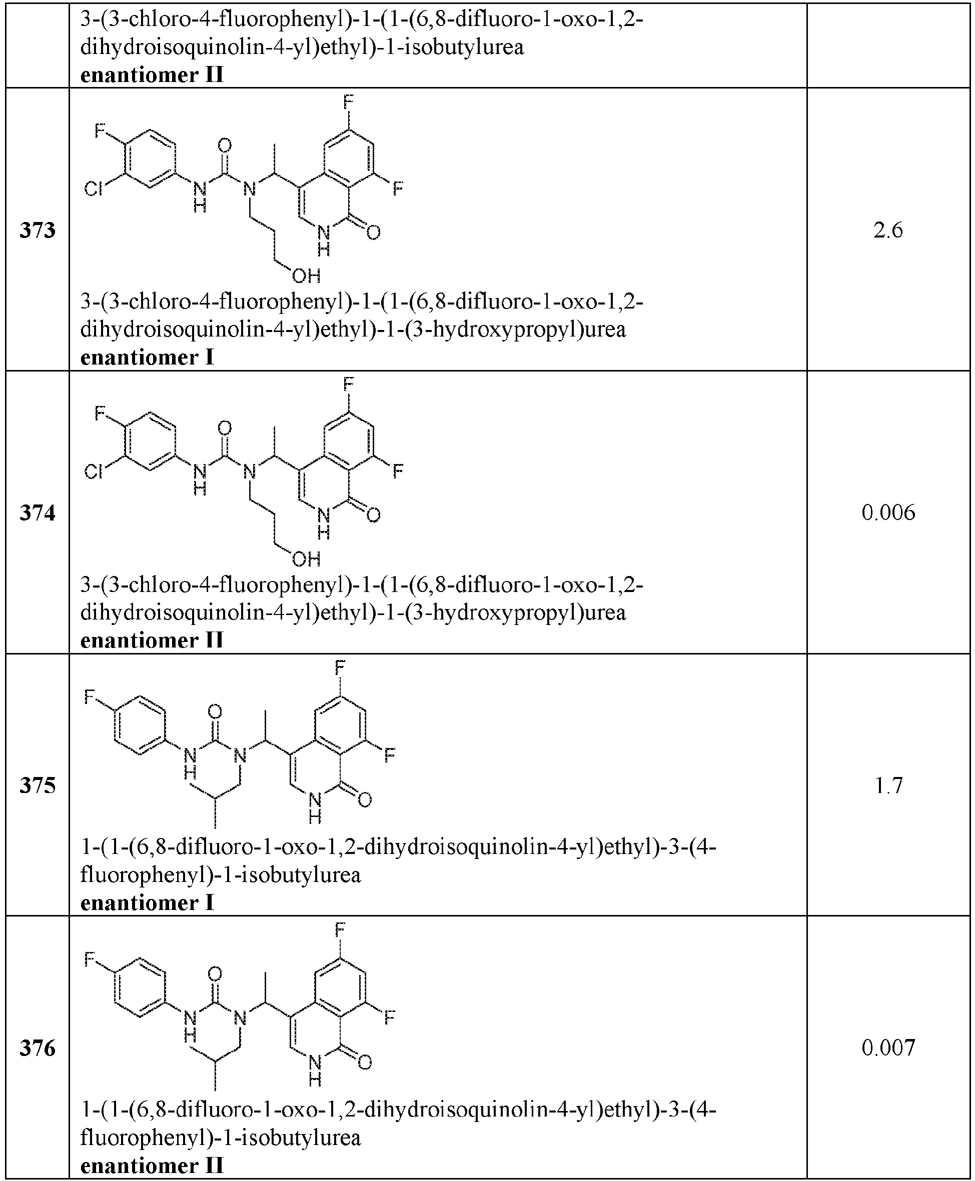

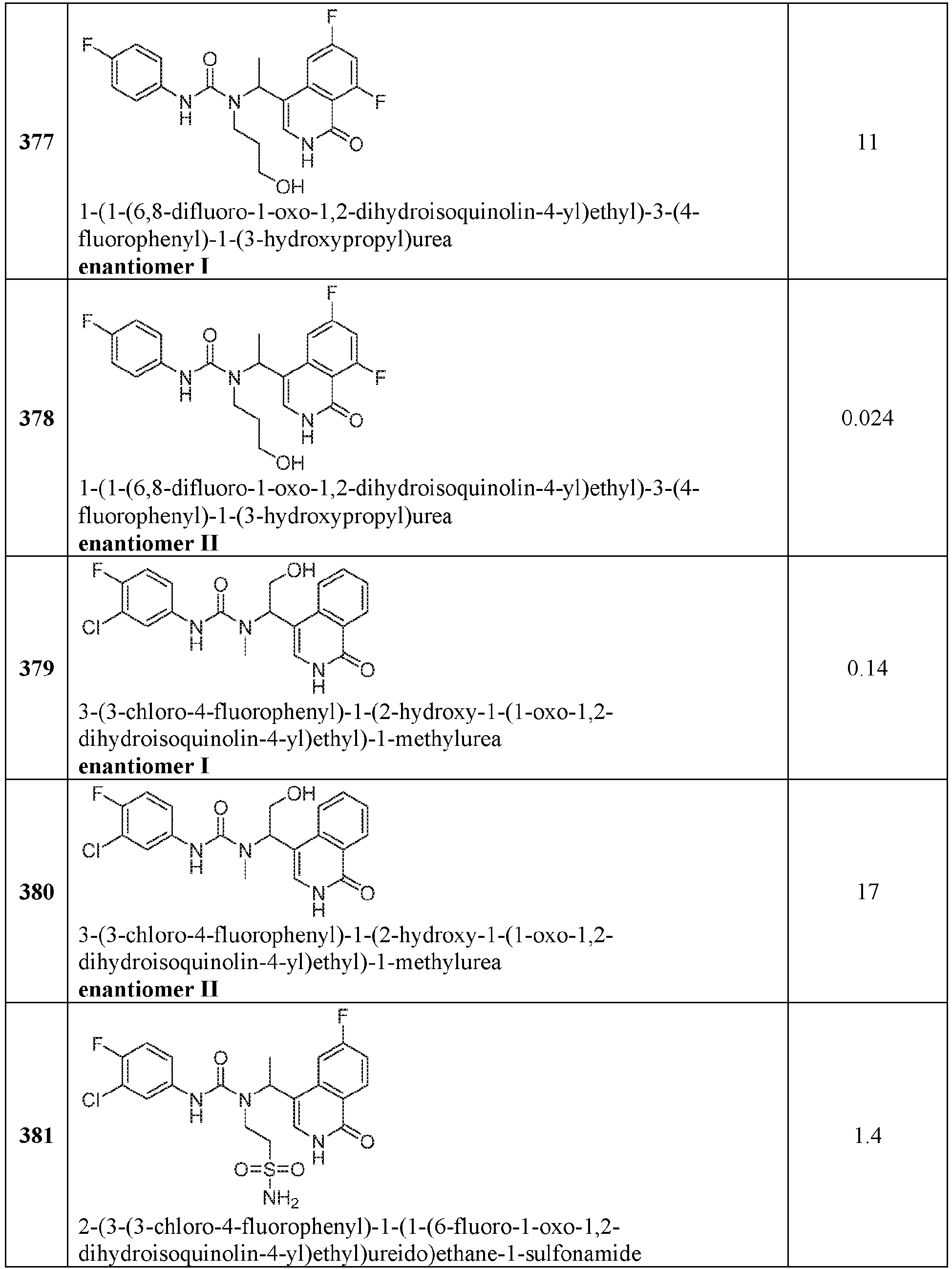

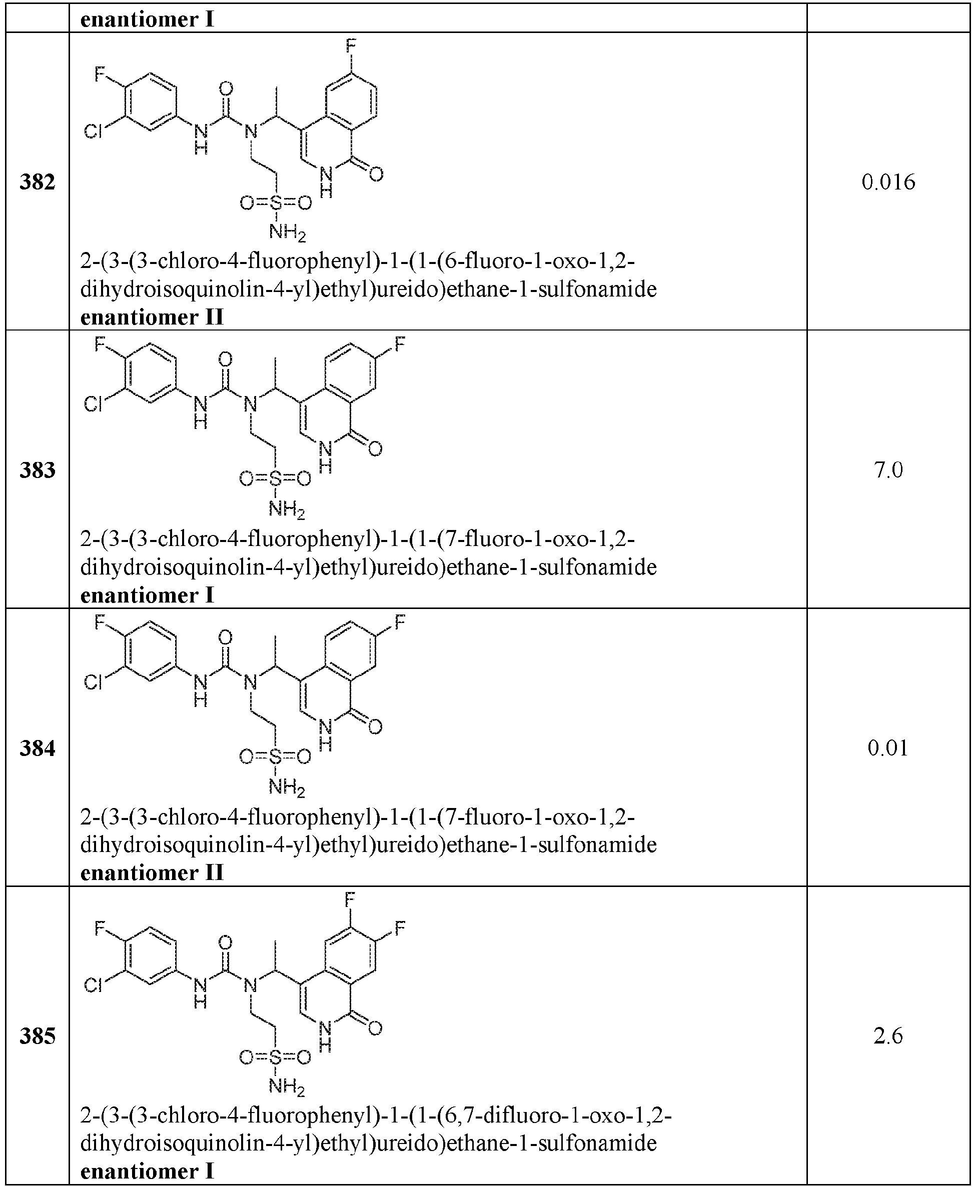

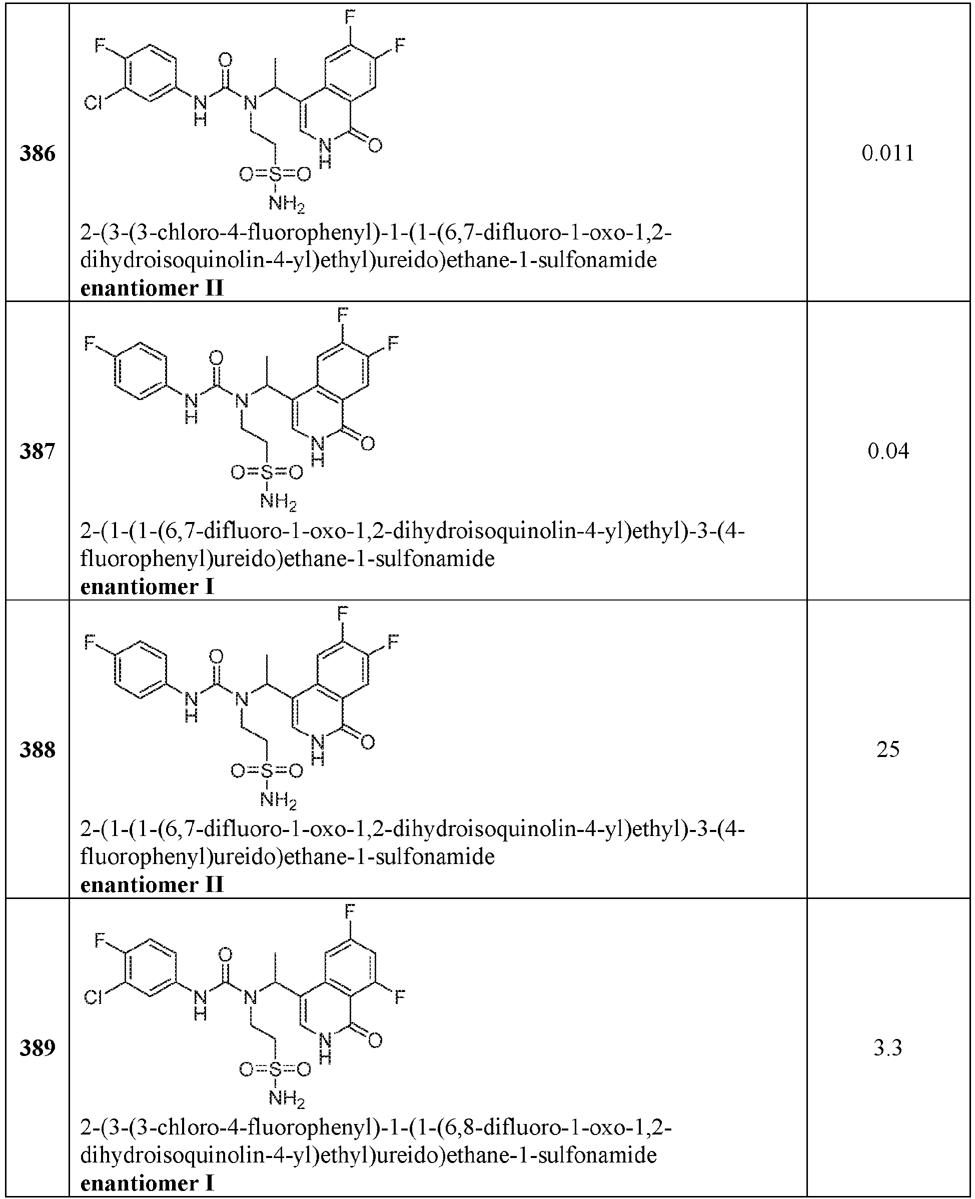

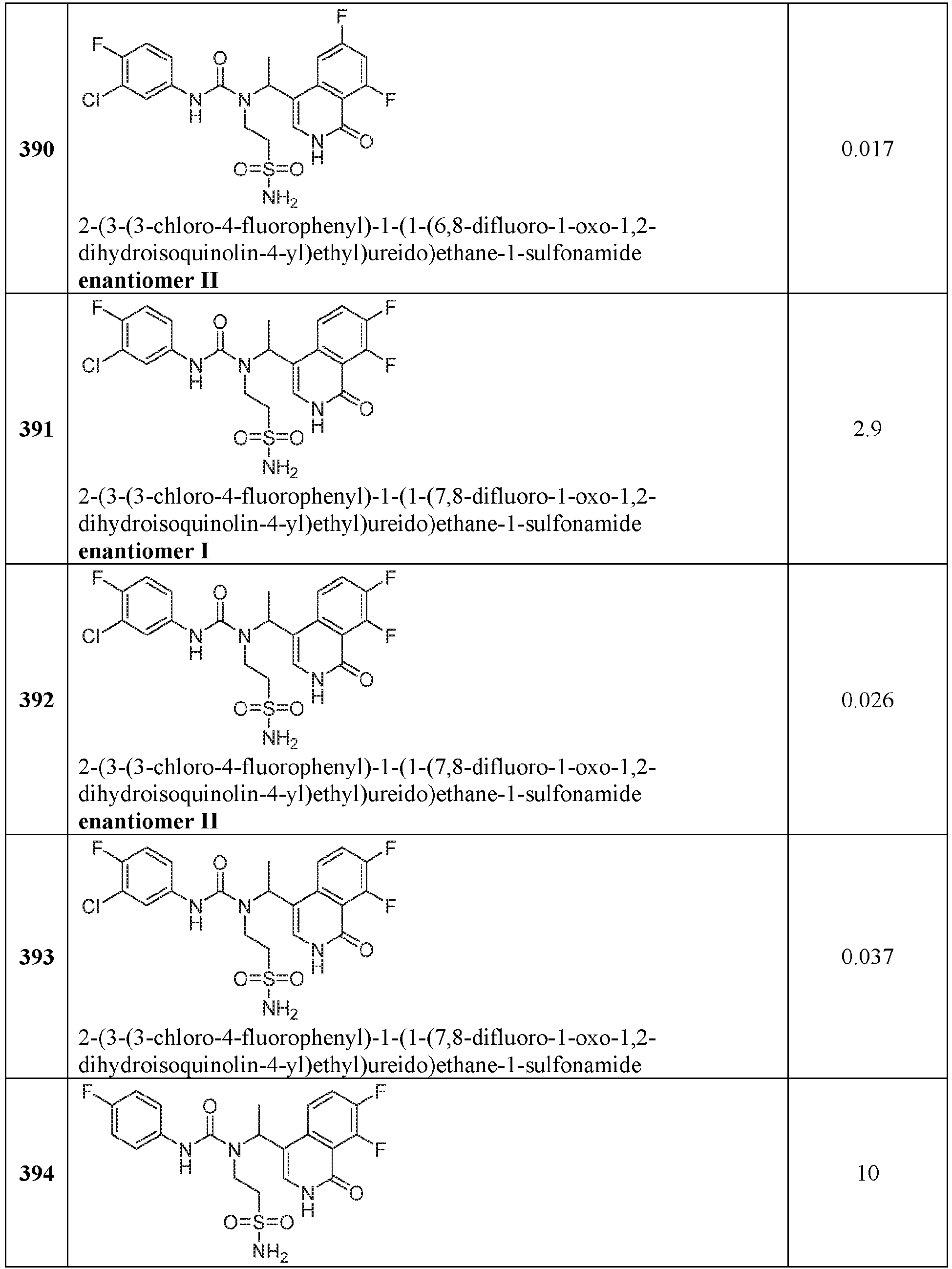

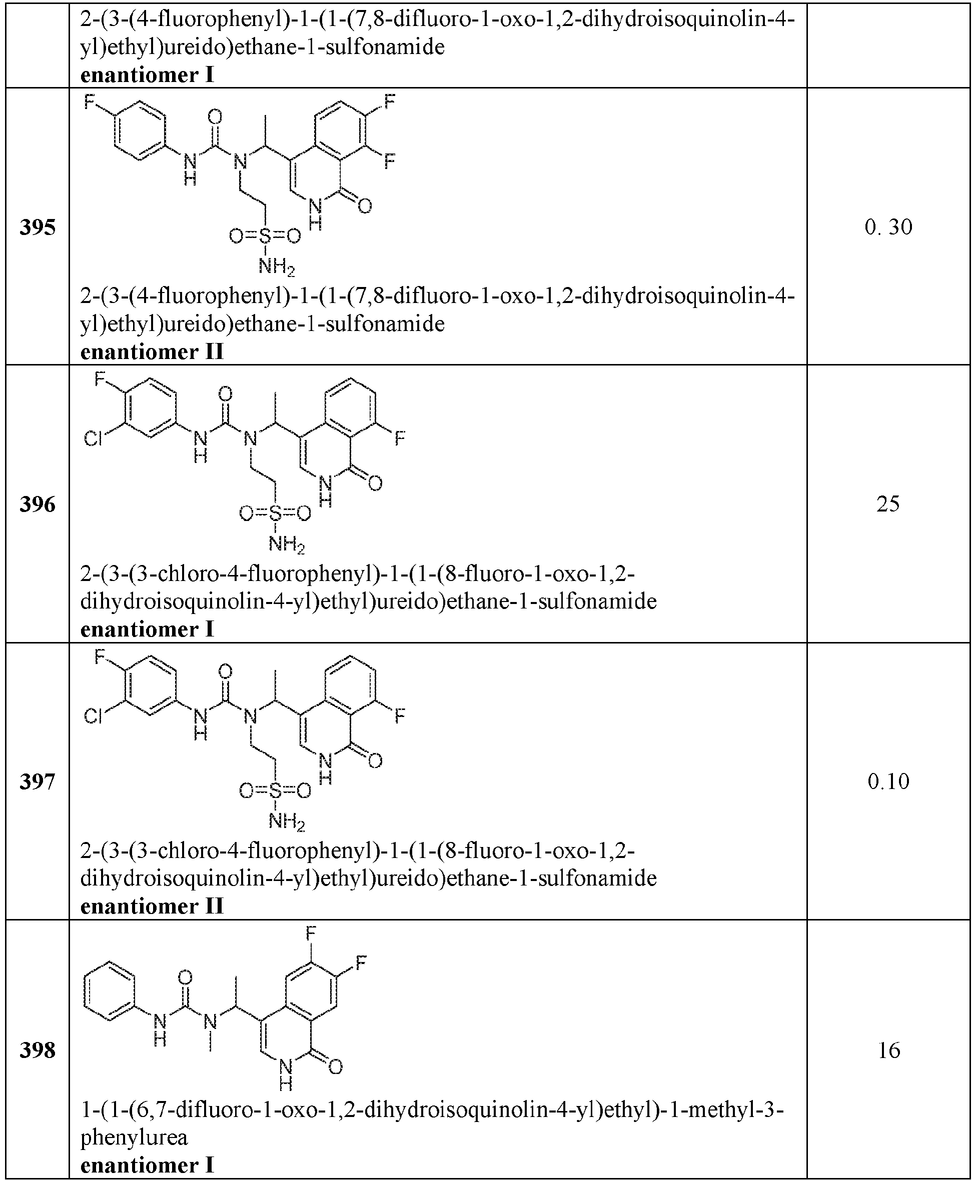

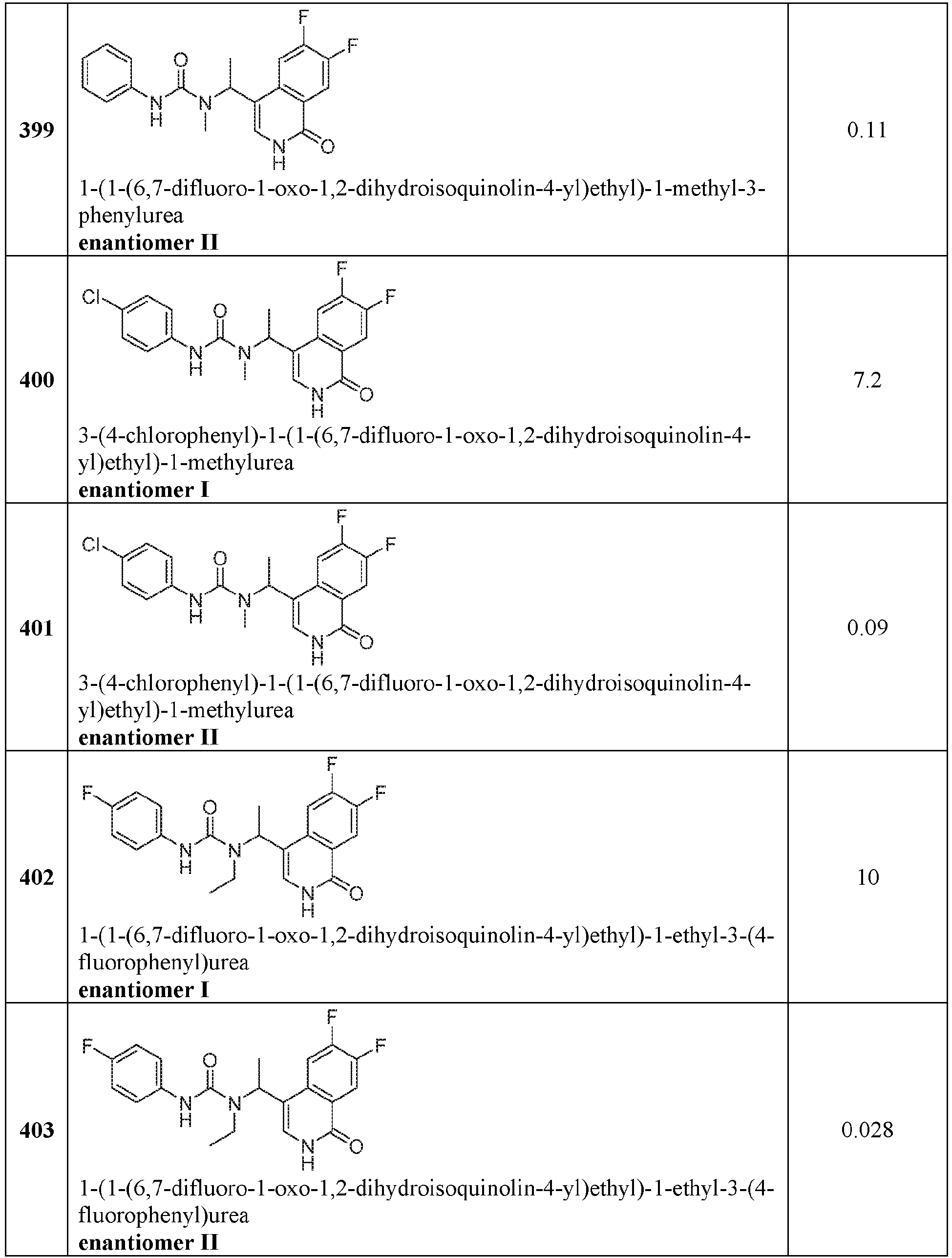

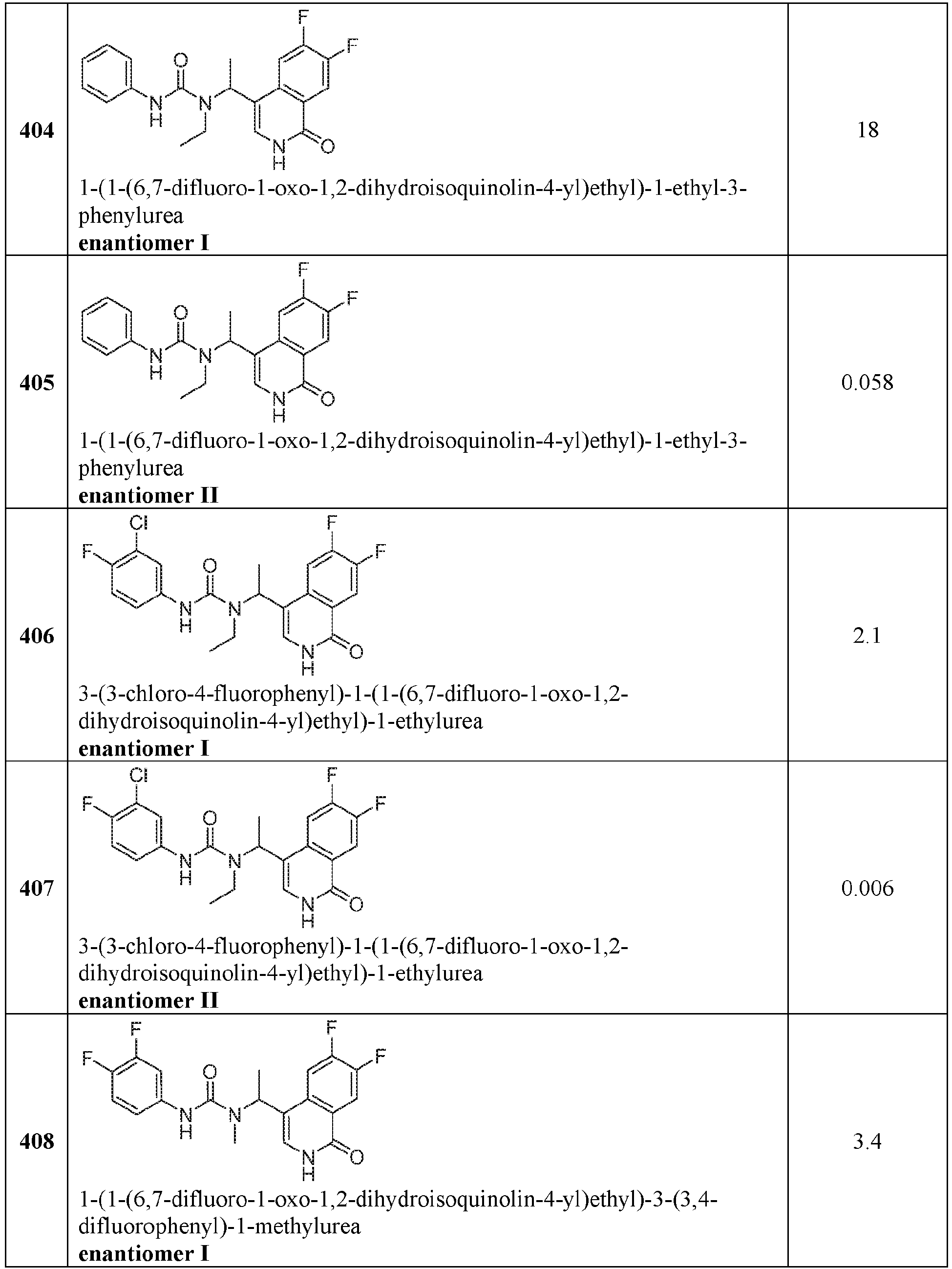

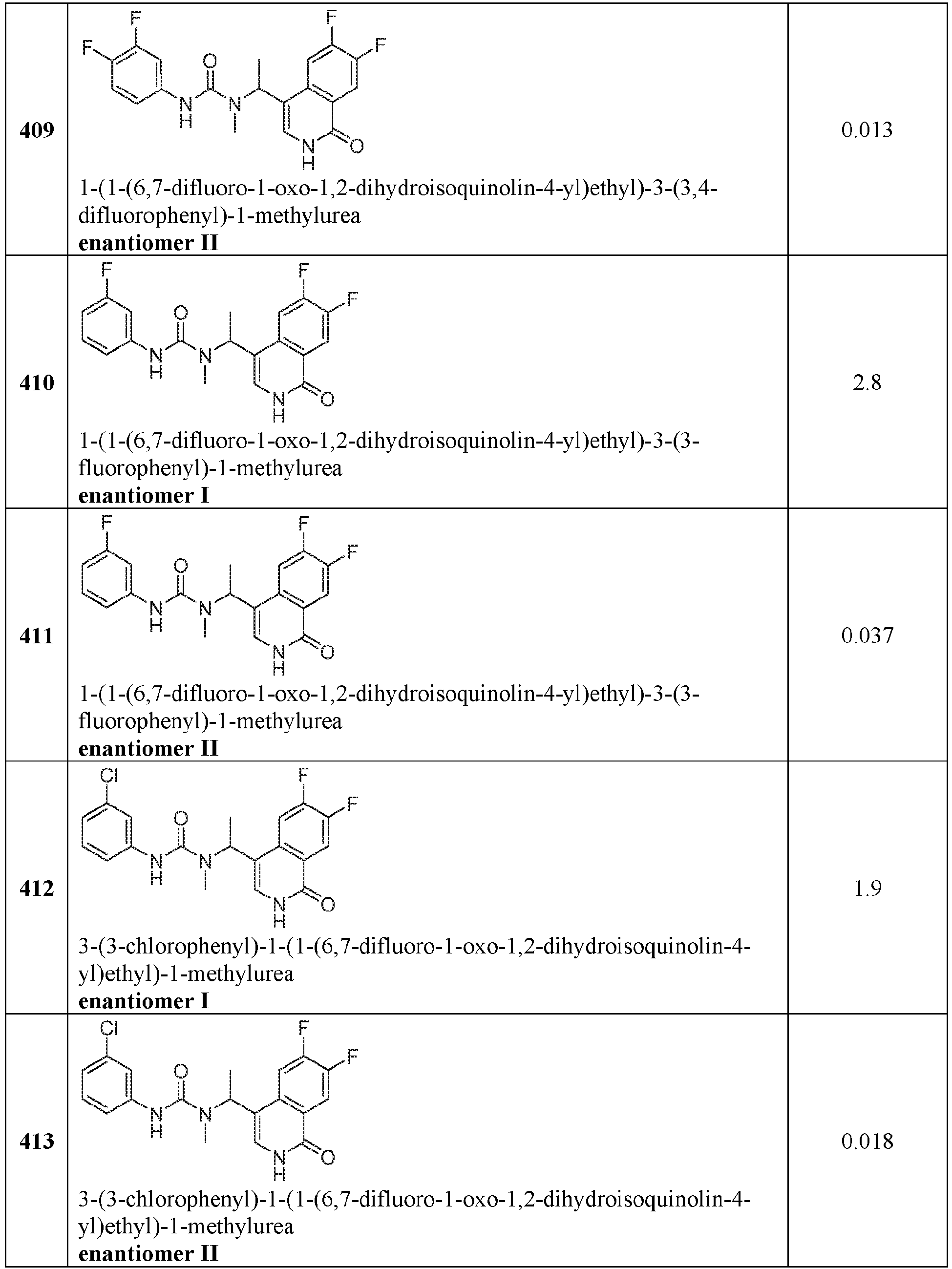

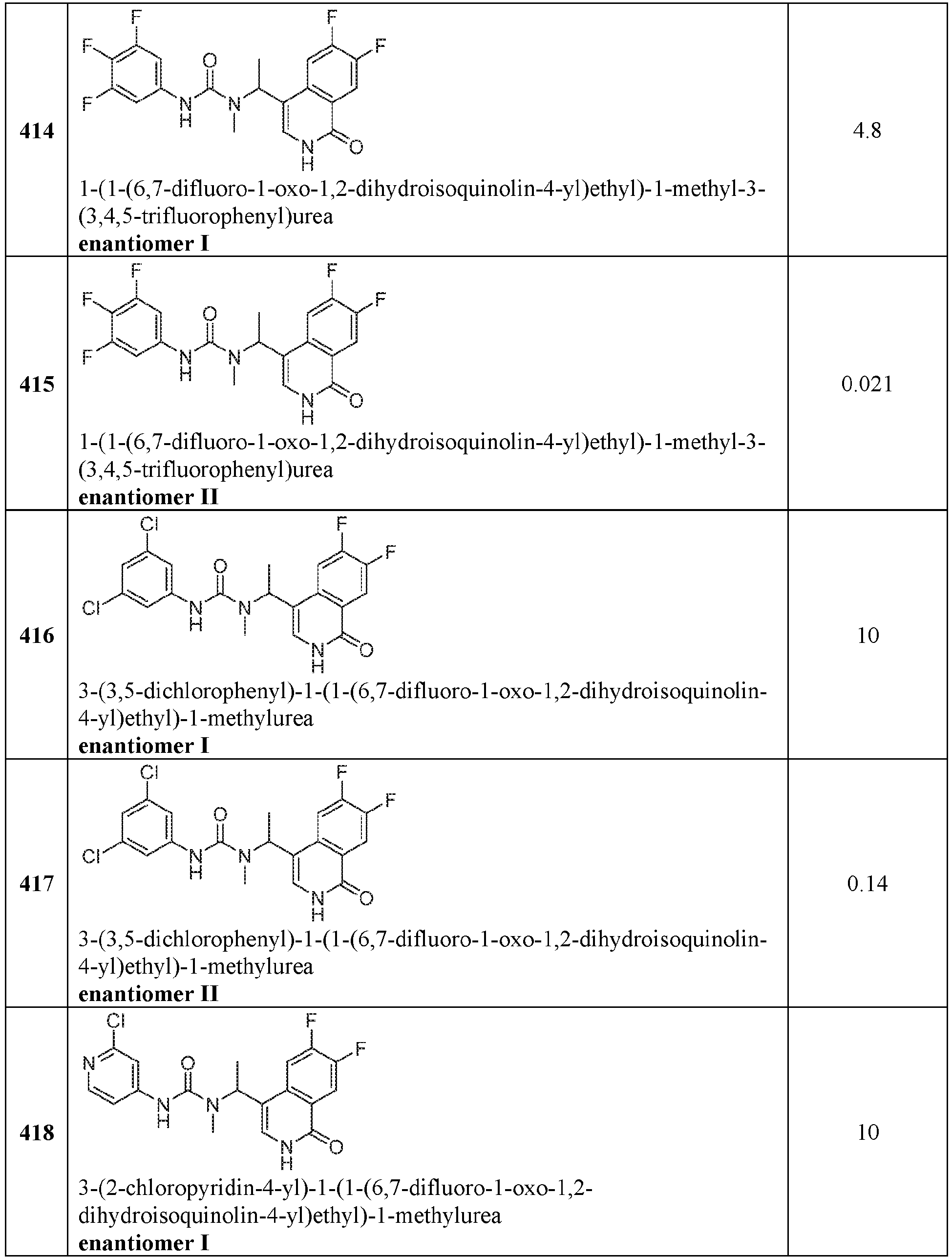

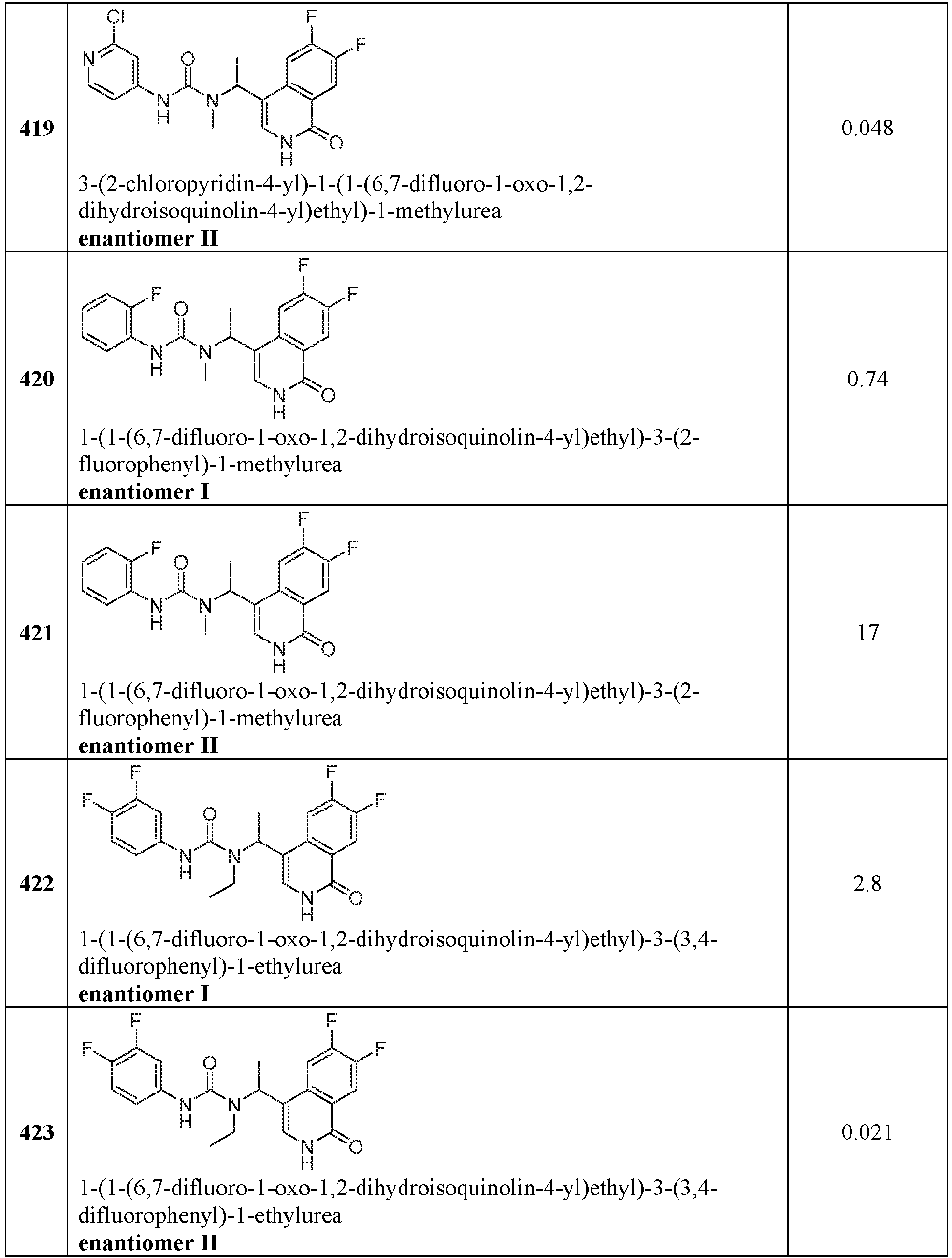

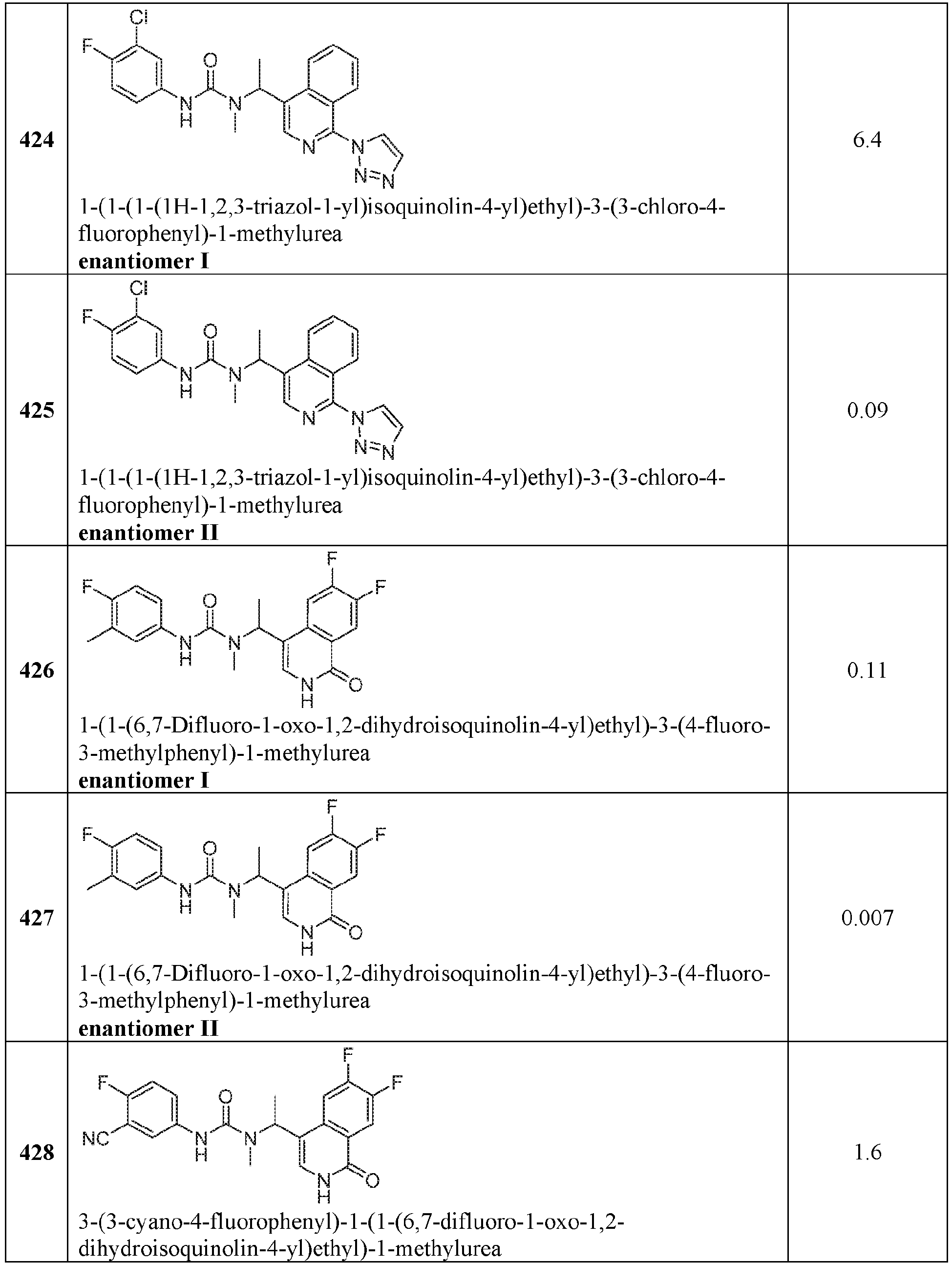

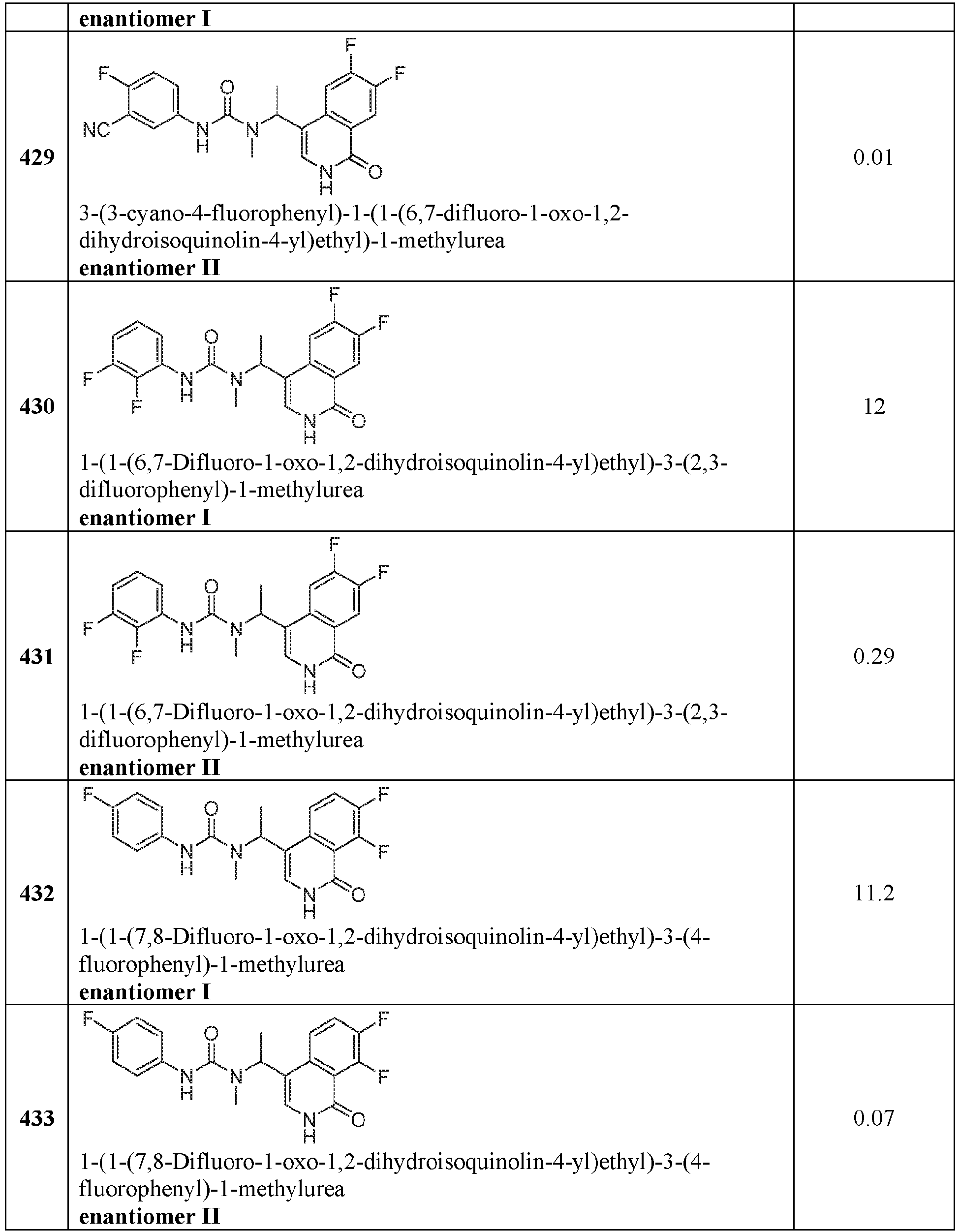

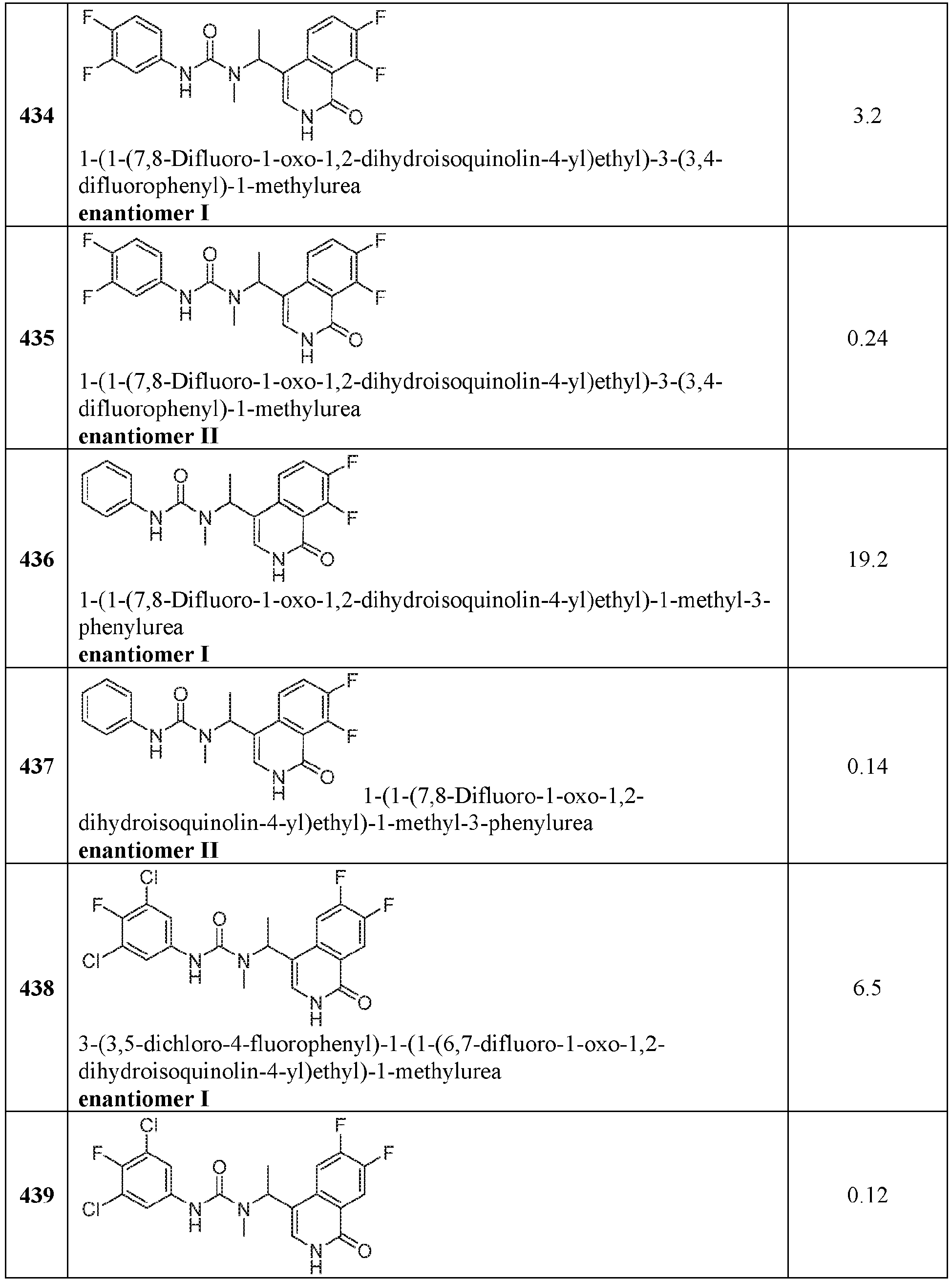

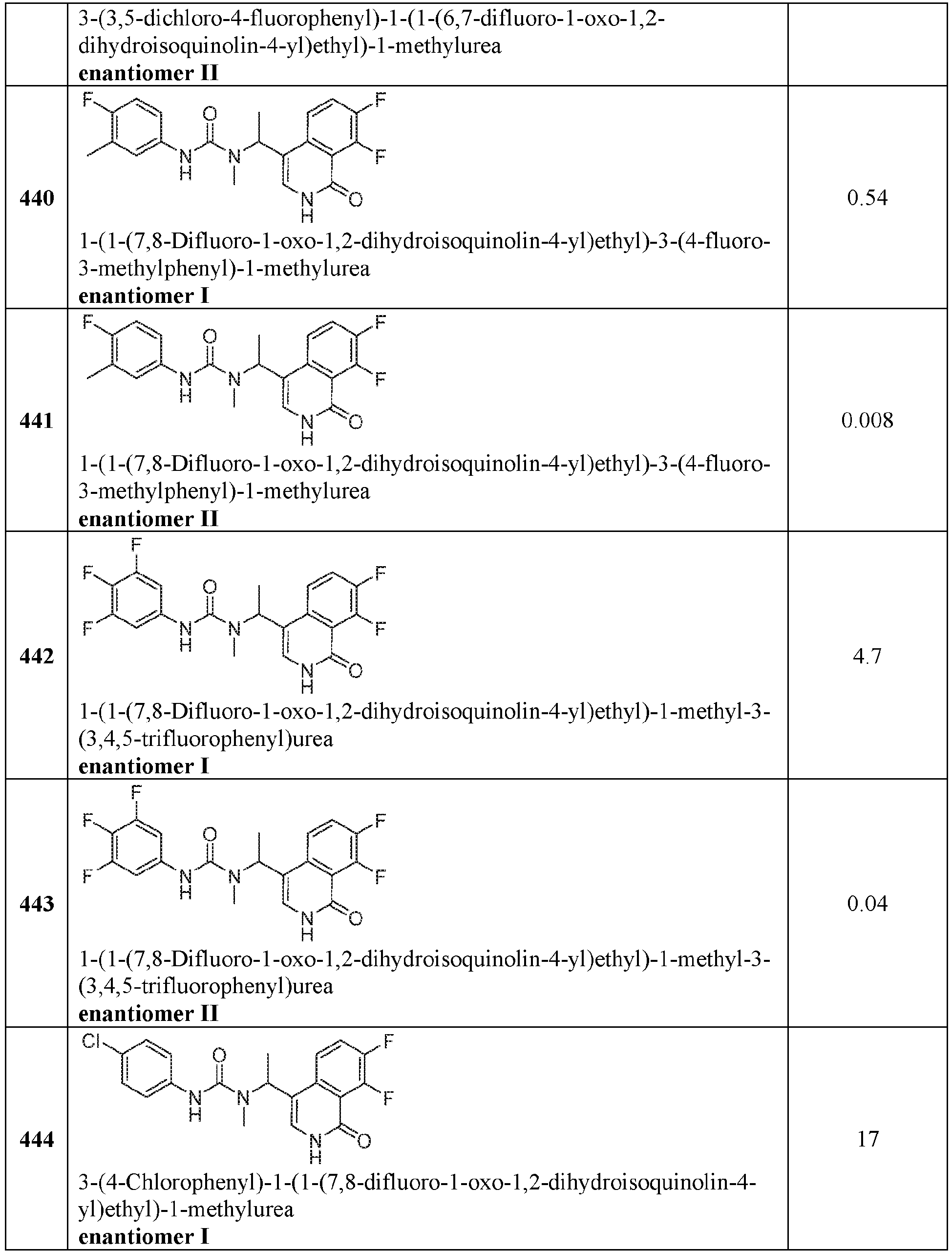

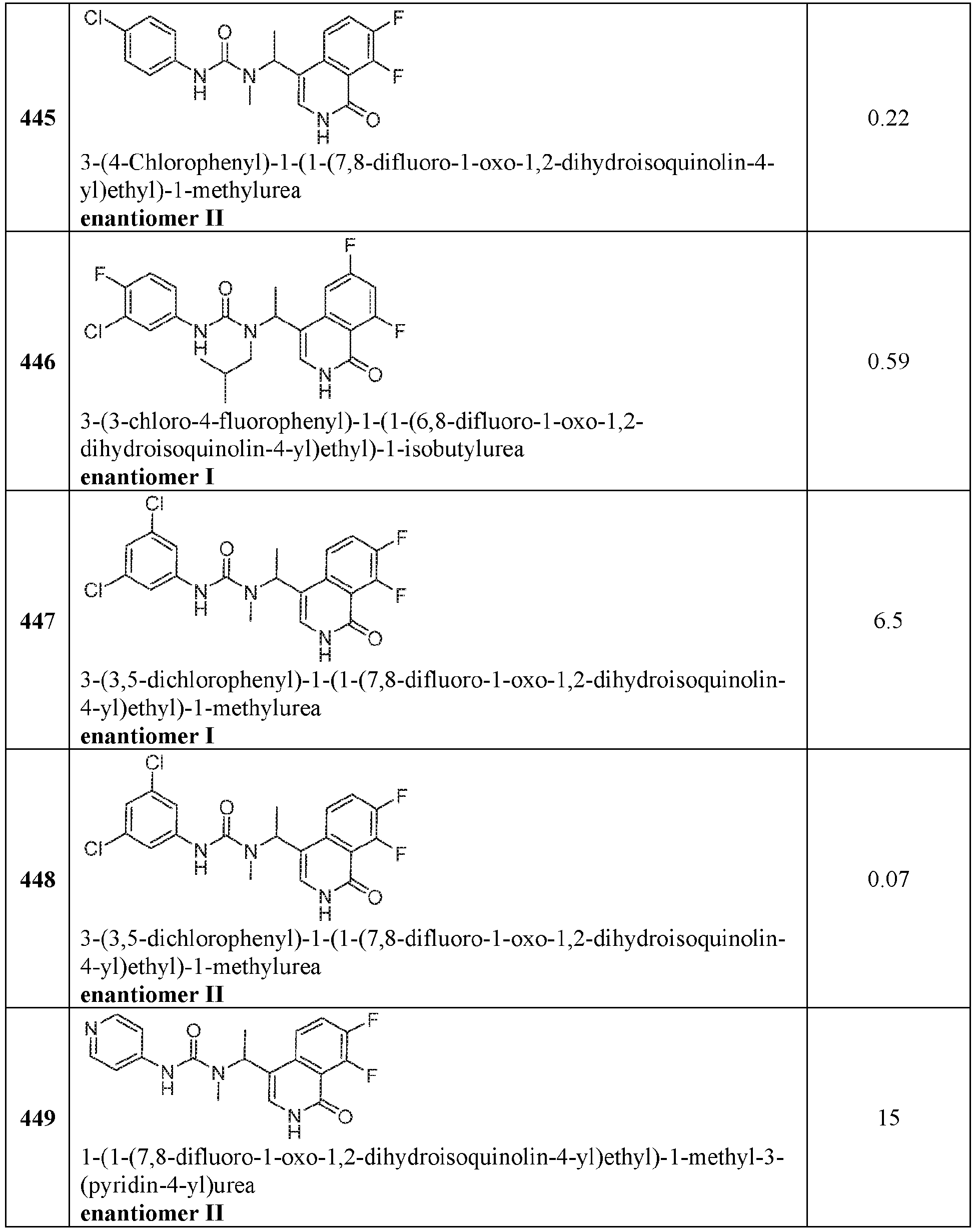

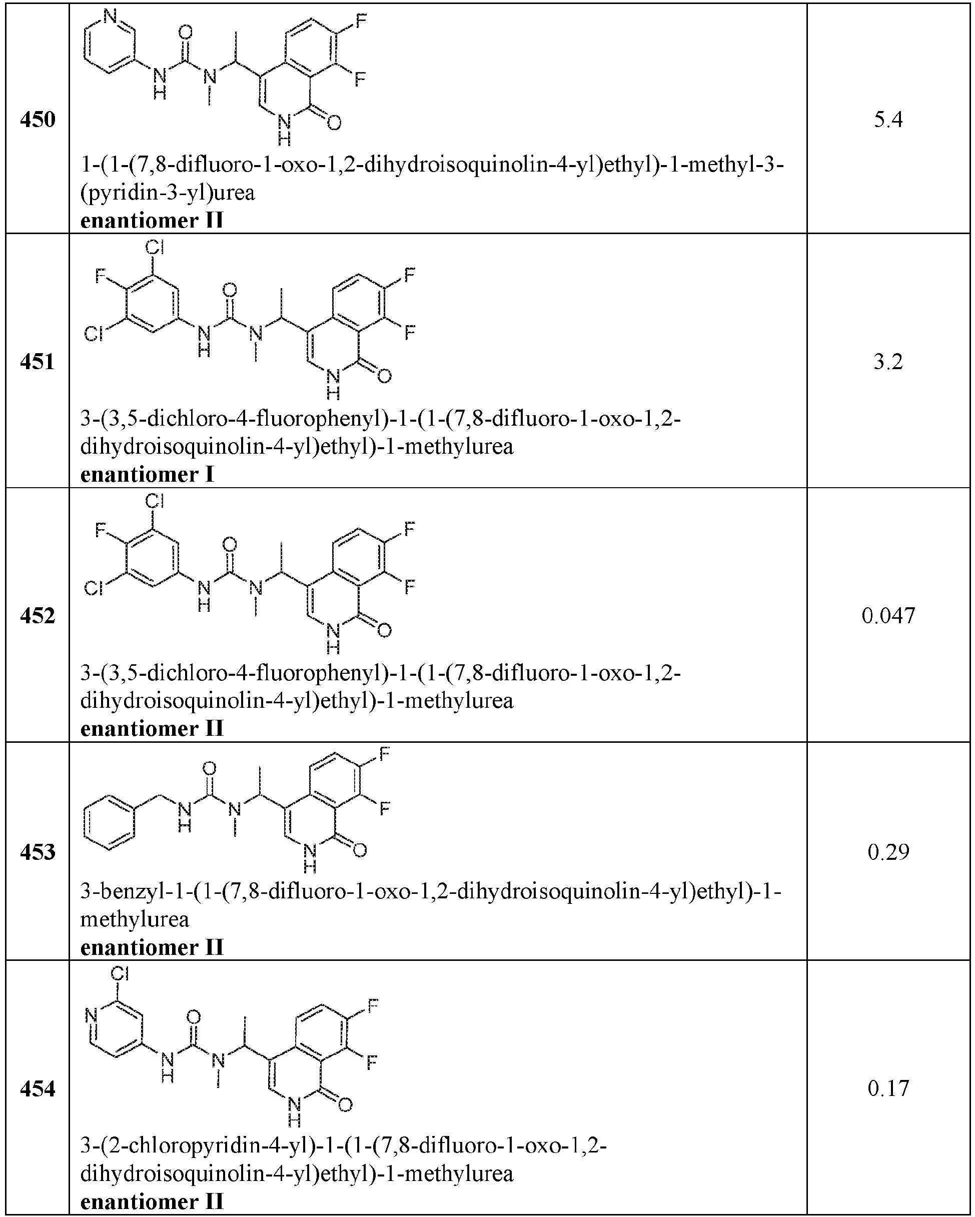

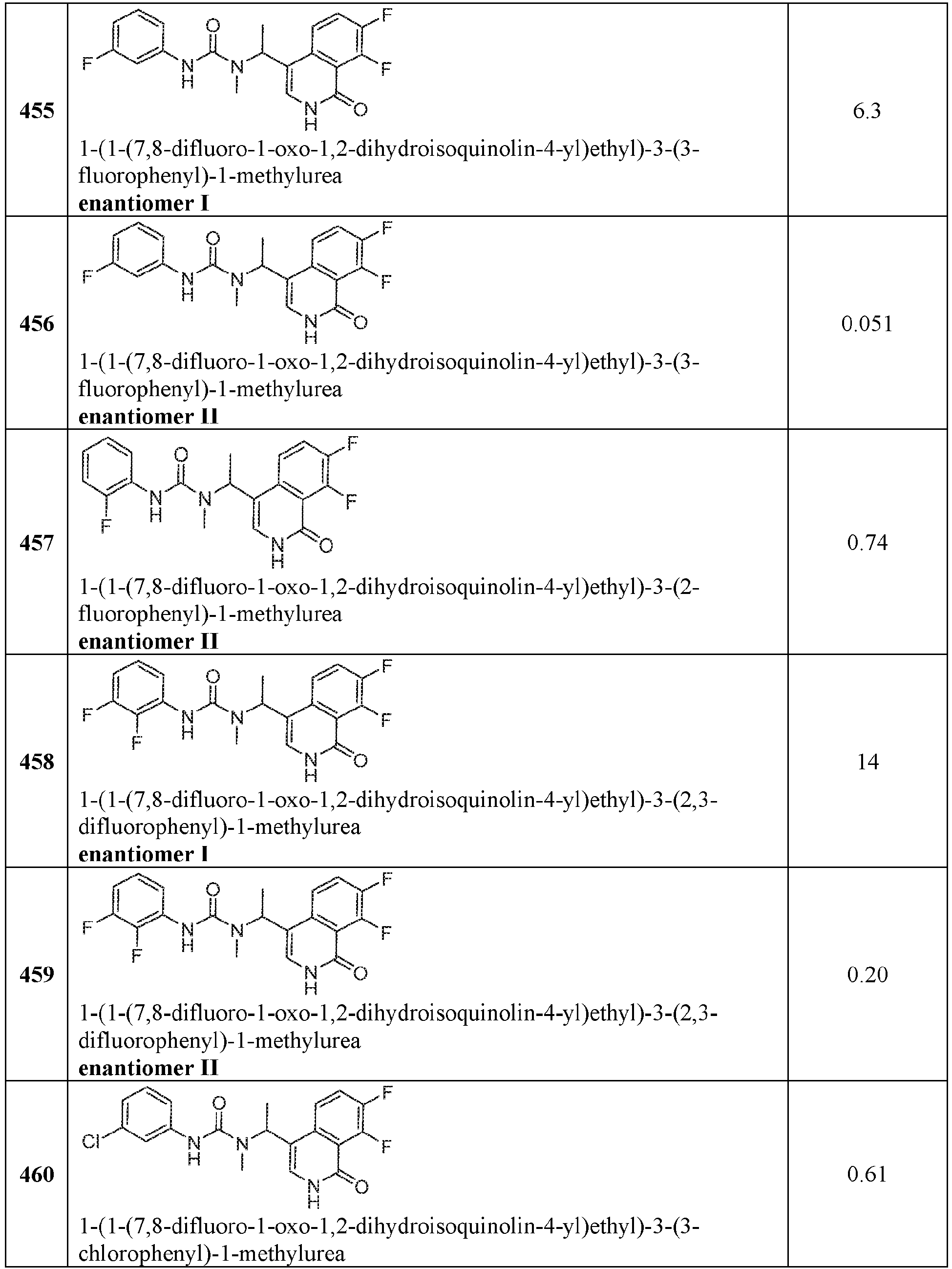

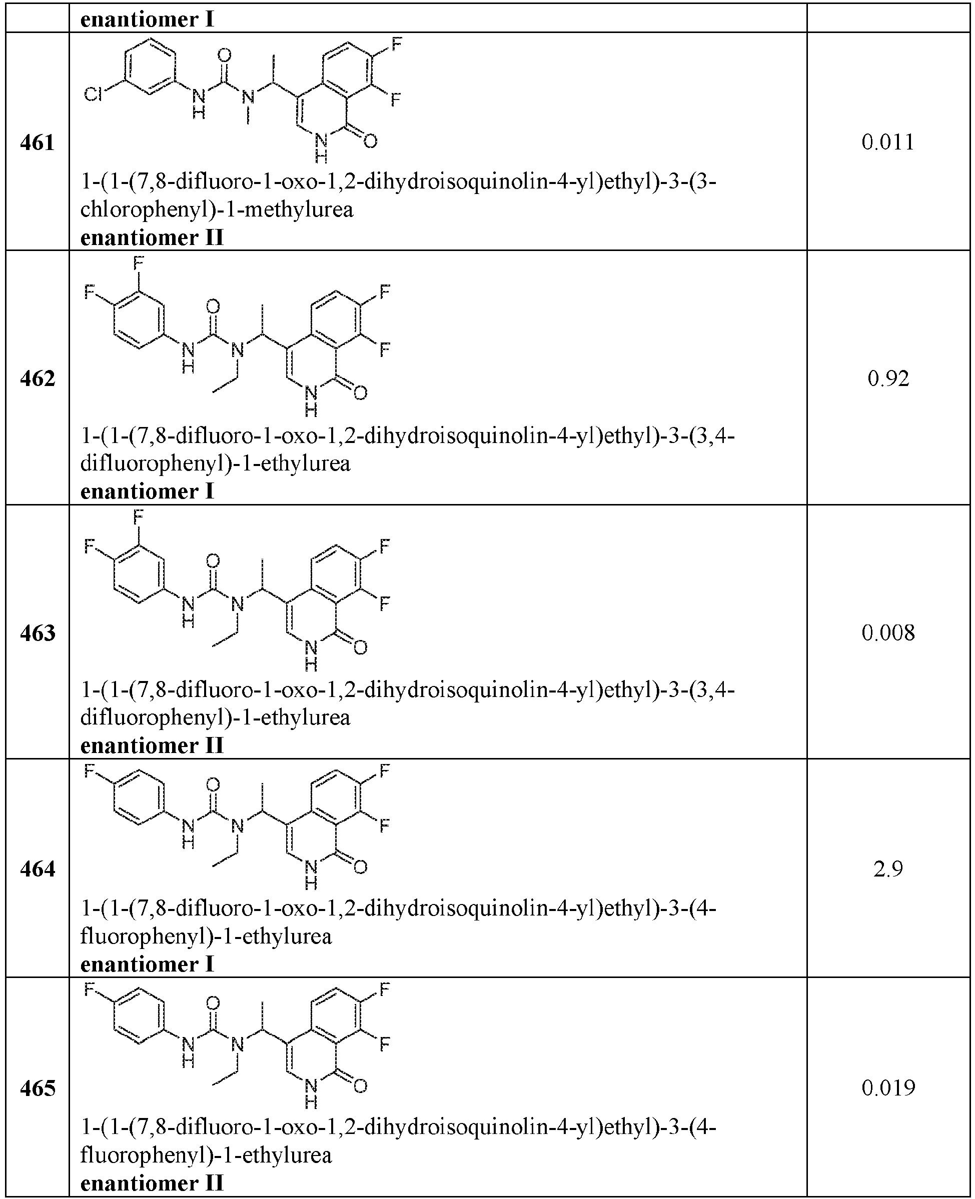

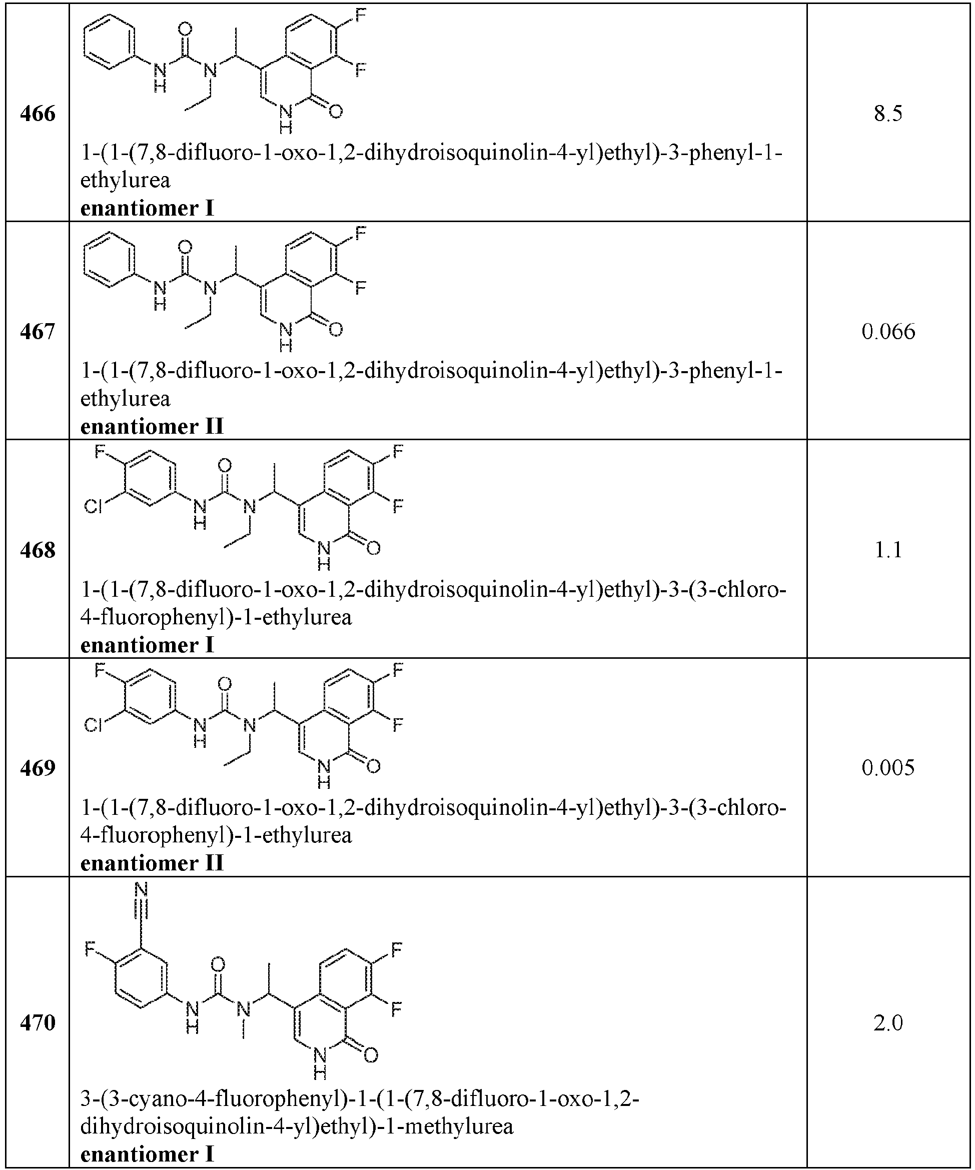

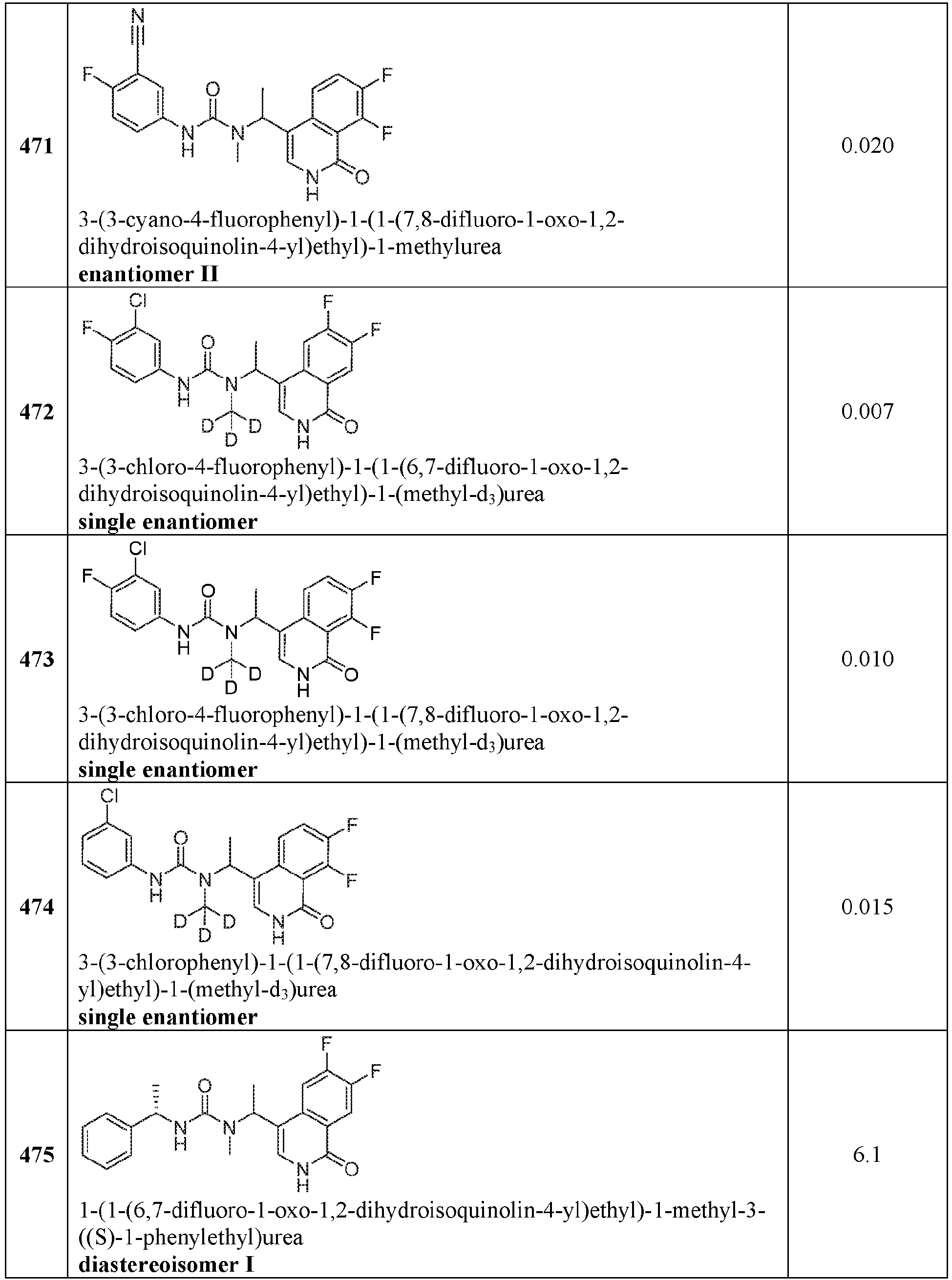

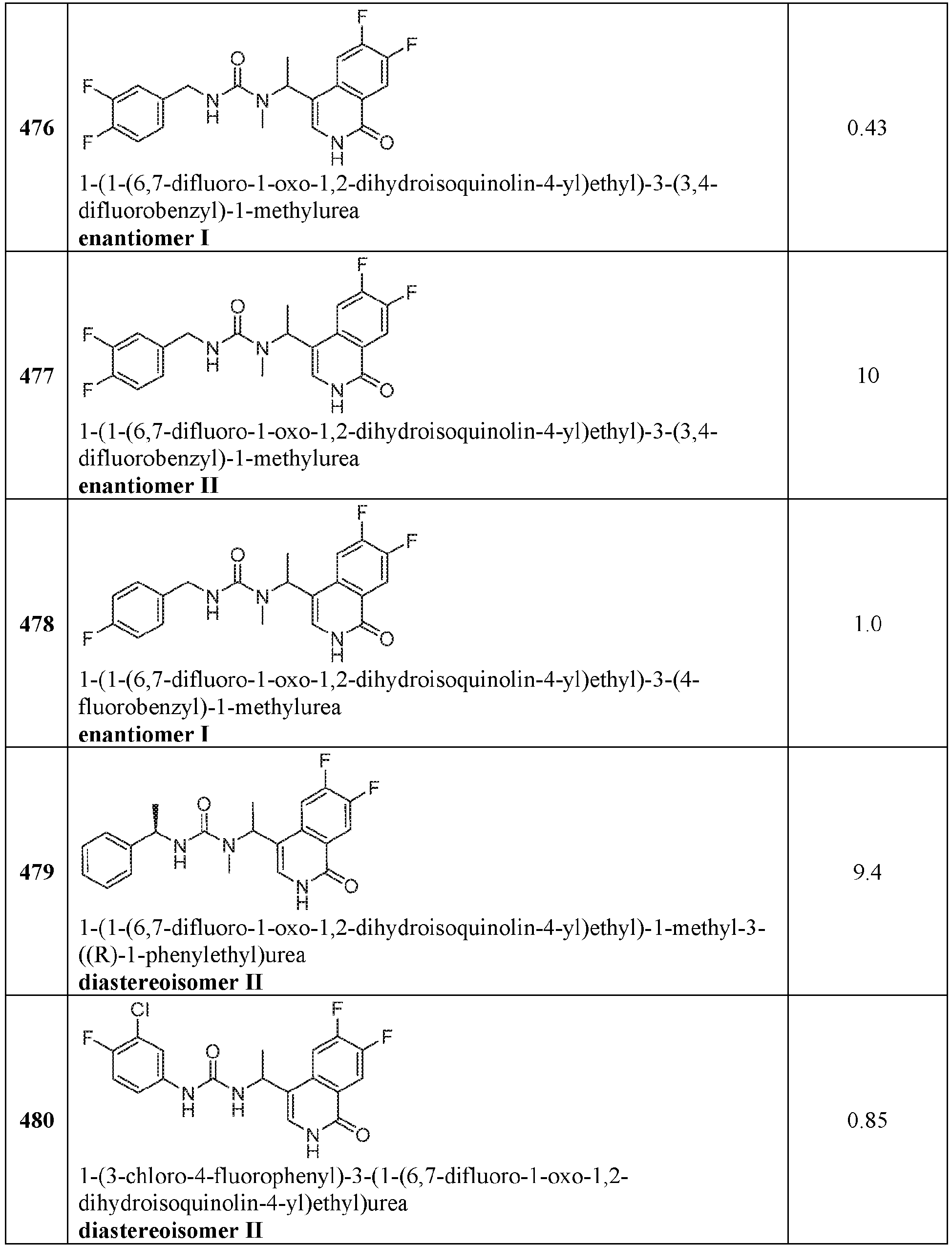

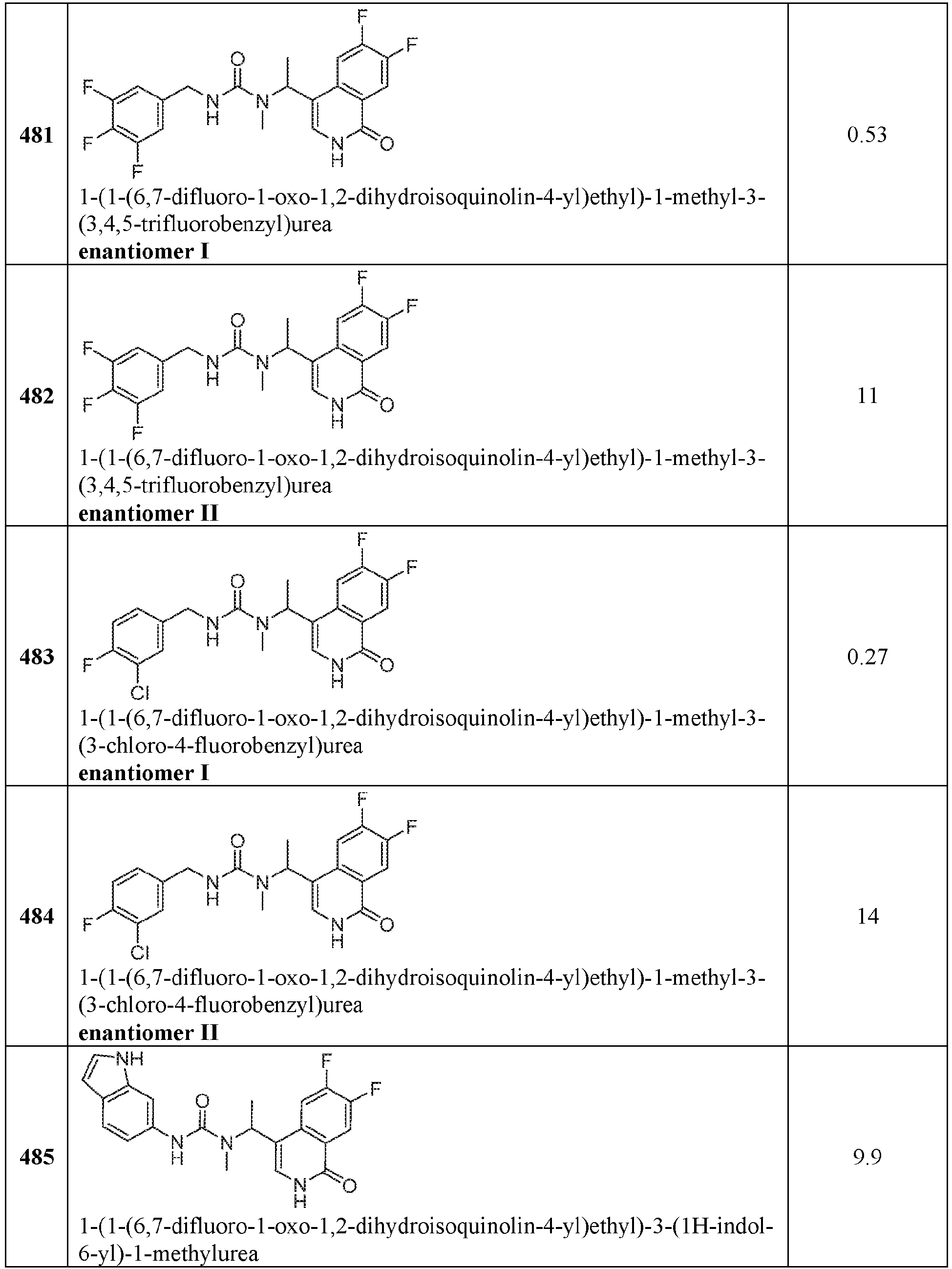

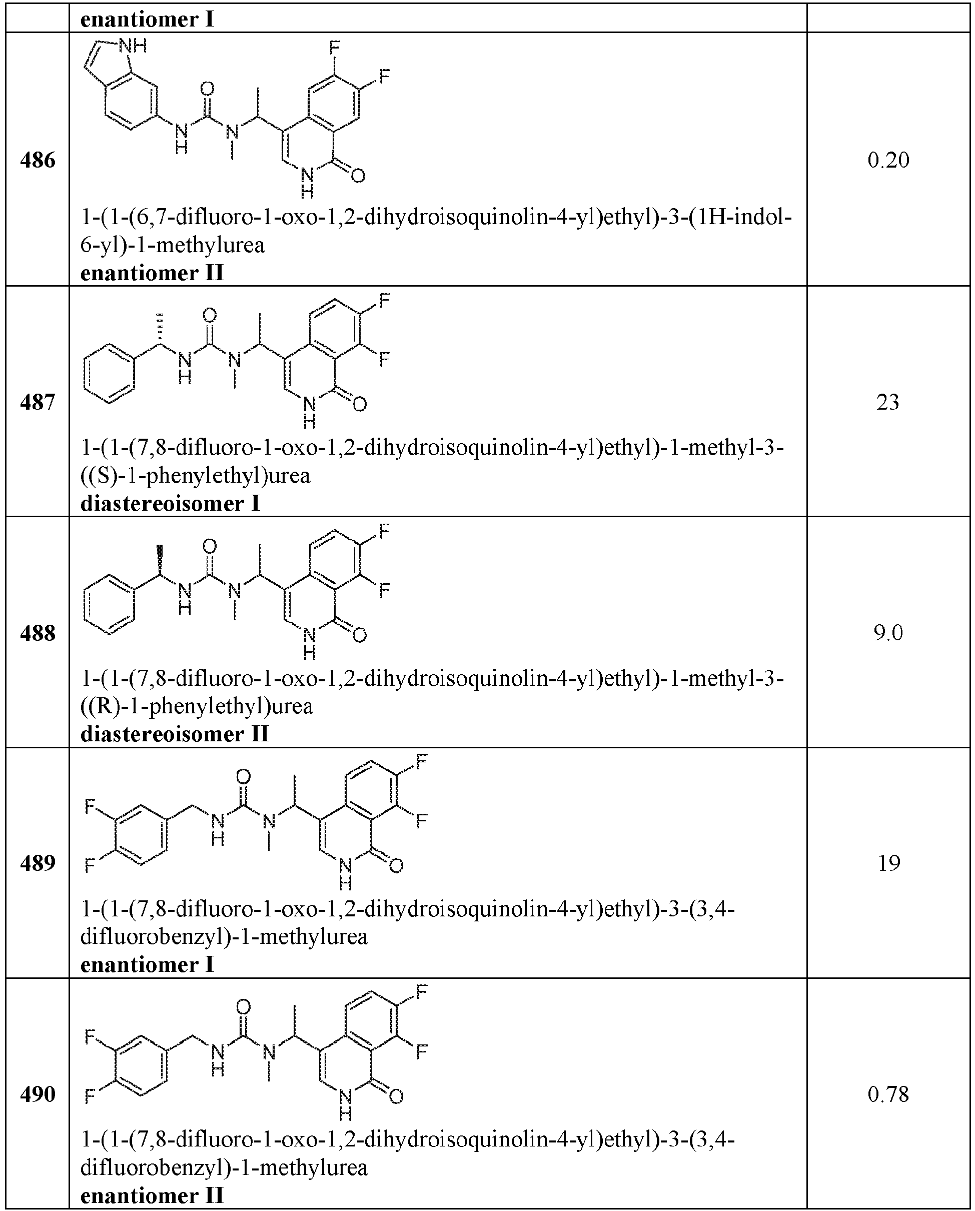

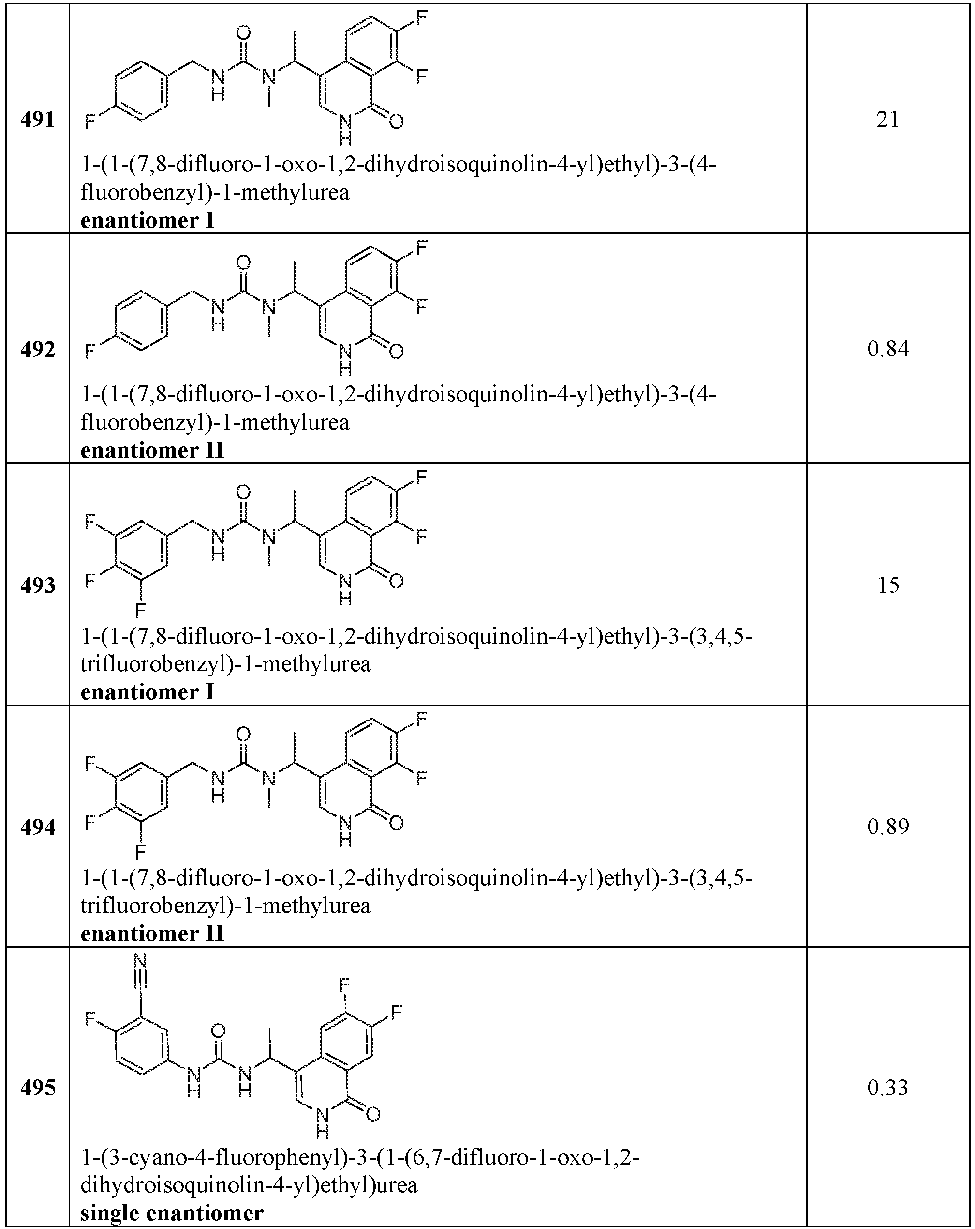

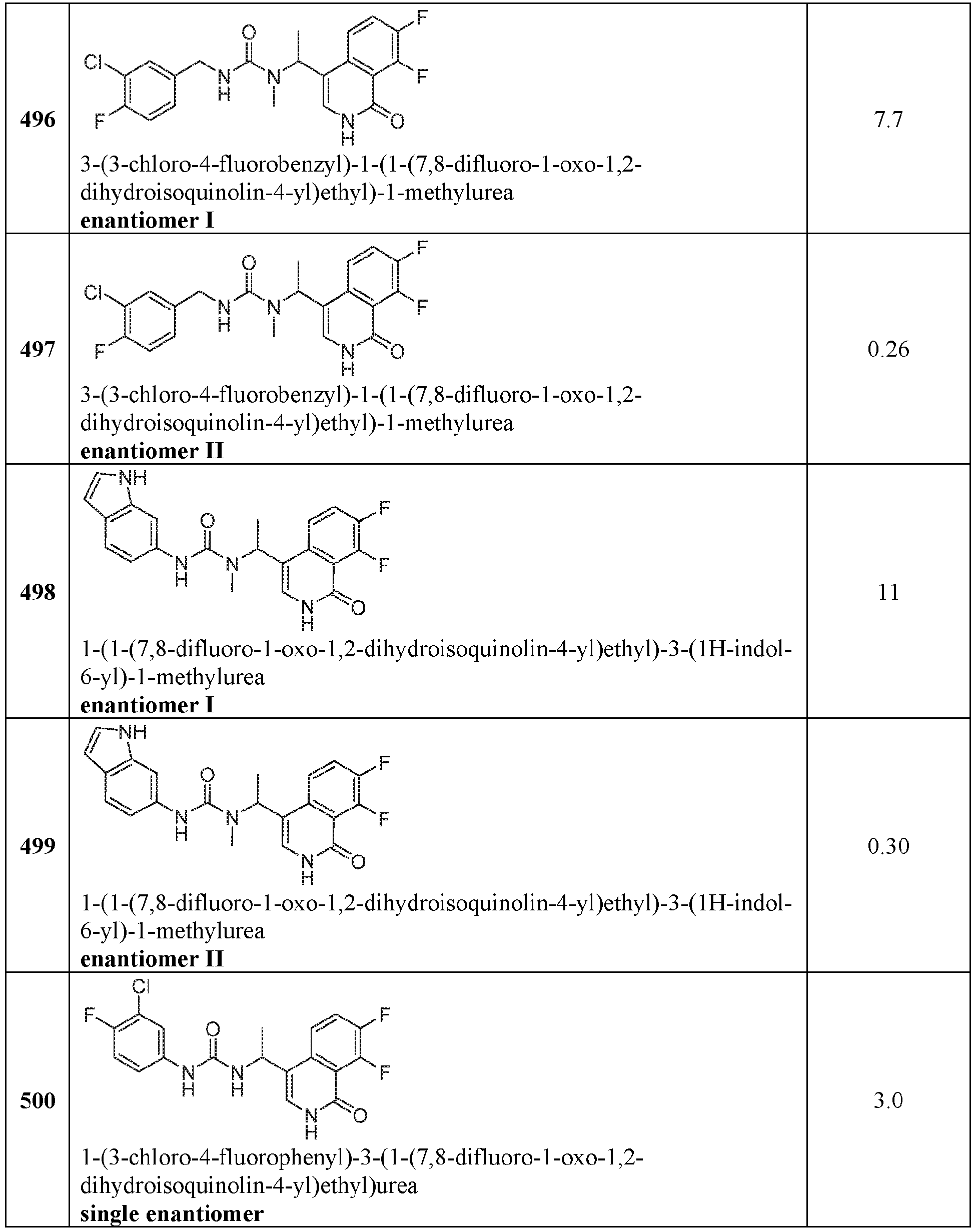

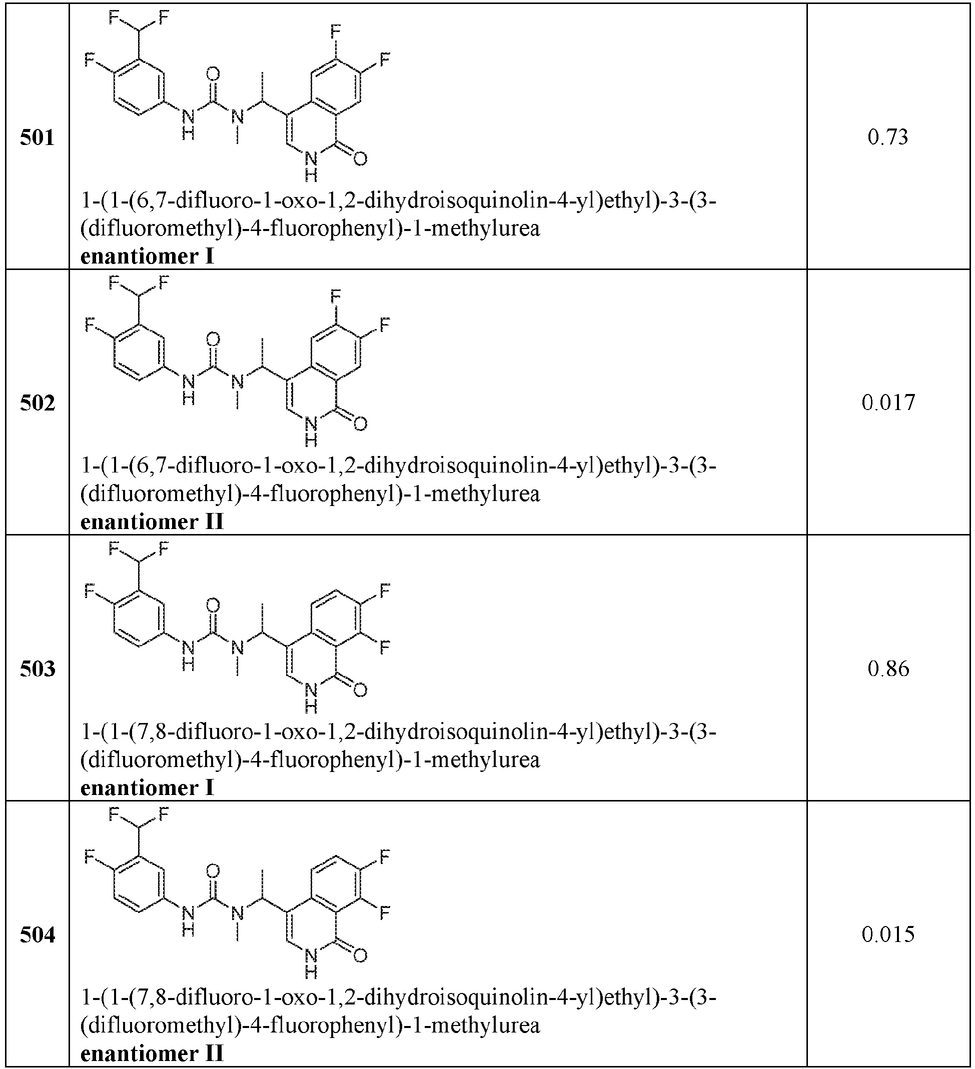

## Claims

1. A compound of formula (I), or a salt, solvate, stereoisomer, tautomer, or isotopically labelled derivative thereof, or any mixtures thereof: 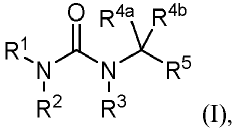 wherein in (I):
R¹ is selected from the group consisting of optionally substituted phenyl, optionally substituted C₃-C₈ cycloalkyl, optionally substituted benzyl, optionally substituted heteroaryl, and -(CH₂)(optionally substituted heteroaryl);
each occurrence of R² is independently selected from the group consisting of H and C₁-C₆ alkyl;
R³ is selected from the group consisting of C₁-C₆ alkyl, and C₃-C₈ cycloalkyl, wherein the alkyl or cycloalkyl is optionally substituted with at least one substituent selected from the group consisting of C₁-C₆ alkyl, C₃-C₈ cycloalkyl, halogen, cyano, -OH, C₁-C₆ alkoxy, C₃-C₈ cycloalkoxy, C₁-C₆ haloalkoxy, C₃-C₈ halocycloalkoxy, optionally substituted phenyl, optionally substituted heteroaryl, optionally substituted heterocyclyl, -C(=O)OR⁶, -OC(=O)R⁶, -SR⁶, -S(=O)R⁶, -S(=O)₂R⁶, - S(=O)₂NR⁶R⁶, -N(R⁶)S(=O)₂R⁶, -N(R⁶)C(=O)R⁶, -C(=O)NR⁶R⁶, and -NR⁶R⁶;
R^{4a} is selected from the group consisting of H, C₁-C₆ alkyl, C₃-C₈ cycloalkyl, and phenyl, wherein the alkyl, cycloalkyl, or phenyl is optionally substituted with at least one substituent selected from the group consisting of C₁-C₆ alkyl, C₃-C₈ cycloalkyl, halogen, cyano, -OH, C₁-C₆ alkoxy, C₃-C₈ cycloalkoxy, C₁-C₆ haloalkoxy, C₃-C₈ halocycloalkoxy, -NR⁶R⁶, and optionally substituted phenyl;
R^{4b} selected from the group consisting of H and optionally substituted C₁-C₆ alkyl;
R⁵ is selected from the group consisting of: 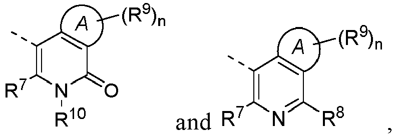 wherein each ring A is independently selected from the group consisting of benzene, pyridine, pyrimidine, pyridazine, and pyrazine;
each occurrence of R⁶ is independently selected from the group consisting of H, optionally substituted C₁-C₆ alkyl, optionally substituted C₃-C₈ cycloalkyl, optionally substituted phenyl, and optionally substituted hetereoaryl;
each occurrence of R⁷ is independently selected from the group consisting of H, halogen, optionally substituted C₁-C₆ alkyl, optionally substituted C₃-C₈ cycloalkyl, optionally substituted C₁-C₆ alkoxy, and optionally substituted C₃-C₈ cycloalkoxy;
each occurrence of R⁸ is independently selected from the group consisting of halogen, -CN, optionally substituted C₁-C₆ alkyl, optionally substituted C₃-C₈ cycloalkyl, optionally substituted C₁-C₆ alkoxy, optionally substituted C₃-C₈ cycloalkoxy, heterocyclyl, heteroaryl, -S(optionally substituted C₁-C₆ alkyl), -SO(optionally substituted C₁-C₆ alkyl), -SO2(optionally substituted C₁-C₆ alkyl), -C(=O)OH, -C(=O)O(optionally substituted C₁-C₆ alkyl), -C(=O)O(optionally substituted C₃-C₈ cycloalkyl), - O(optionally substituted C₁-C₆ alkyl), -O(optionally substituted C₃-C₈ cycloalkyl), -NH₂, - NH(optionally substituted C₁-C₆ alkyl), -NH(optionally substituted C₃-C₈ cycloalkyl), -N(optionally substituted C₁-C₆ alkyl)(optionally substituted C₁-C₆ alkyl), -N(optionally substituted C₃-C₈ cycloalkyl)(optionally substituted C₃-C₈ cycloalkyl), -N(optionally substituted C₁-C₆ alkyl)(optionally substituted C₃-C₈ cycloalkyl), -C(=O)NH₂, -C(=O)NH(optionally substituted C₁-C₆ alkyl), - C(=O)NH(optionally substituted C₃-C₈ cycloalkyl), -C(=O)N(optionally substituted C₁-C₆ alkyl)(optionally substituted C₁-C₆ alkyl), -C(=O)N(optionally substituted C₃-C₈ cycloalkyl)(optionally substituted C₃-C₈ cycloalkyl), and -C(=O)N(optionally substituted C₁-C₆ alkyl)(optionally substituted C₃-C₈ cycloalkyl;
each occurrence of R⁹ is independently selected from the group consisting of H, halogen, optionally substituted C₁-C₆ alkyl, optionally substituted C₃-C₈ cycloalkyl, optionally substituted C₁-C₆ alkoxy, and optionally substituted C₃-C₈ cycloalkoxy;
each occurrence of n is independently 0, 1, 2, 3, or 4; and
R¹⁰ is selected from the group consisting of H, optionally substituted C₁-C₆ alkyl, and optionally substituted C₃-C₈ cycloalkyl.

2. The compound of claim 1, wherein each occurrence of R^{4b} is independently selected from the group consisting of H and CH₃.

3. The compound of claim 1, wherein each occurrence of aryl or heteroaryl is independently optionally substituted with at least one substituent selected from the group consisting of Ci-Ce alkyl, C₃-C₈ cycloalkyl, phenyl, C₁-C₆ hydroxyalkyl, (C₁-C₆ alkoxy)-C₁-C₆ alkyl, C₁-C₆ haloalkyl, C₁-C₆ haloalkoxy, halogen, -CN, -OR^{b}, -N(R^{b})(R^{b}), -NO₂, -C(=O)N(R^{b})(R^{b}), -C(=O)OR^{b}, -OC(=O)R^{b}, -SR^{b}, - S(=O)R^{b}, -S(=O)₂R^{b}, N(R^{b})S(=O)₂R^{b}, -S(=O)₂N(R^{b})(R^{b}), acyl, and C₁-C₆ alkoxycarbonyl, wherein each occurrence of R^{b} is independently H, C₁-C₆ alkyl, or C₃-C₈ cycloalkyl, wherein in R^{b} the alkyl or cycloalkyl is optionally substituted with at least one substituent selected from the group consisting of halogen, -OH, C₁-C₆ alkoxy, and heteroaryl; or substituents on two adjacent carbon atoms combine to form -O(CH₂)₁₋₃O-.

4. The compound of claim 1, wherein each occurrence of alkyl, alkenyl, alkynyl, or cycloalkyl is independently optionally substituted with at least one substituent selected from the group consisting of C₁-C₆ alkyl, C₃-C₈ cycloalkyl, halogen, cyano (-CN), -OR^{a}, optionally substituted phenyl, optionally substituted heteroaryl, optionally substituted heterocyclyl, -C(=O)OR^{a}, -OC(=O)R^{a}, -SR^{a}, -S(=O)R^{a}, - S(=O)₂R^{a}, -S(=O)₂NR^{a}R^{a}, -N(R^{a})S(=O)₂R^{a}, -N(R^{a})C(=O)R^{a}, -C(=O)NR^{a}R^{a}, and -N(R^{a})(R^{a}), wherein each occurrence of R^{a} is independently H, optionally substituted C₁-C₆ alkyl, optionally substituted C₃-C₈ cycloalkyl, optionally substituted aryl, or optionally substituted heteroaryl, or two R^{a} groups combine with the N to which they are bound to form a heterocycle.

5. The compound of claim 1, wherein R¹ is phenyl optionally substituted with at least one substituent selected from the group consisting of C₁-C₆ alkyl, halogen, C₁-C₃ haloalkyl, and -CN, preferably R¹ is selected from the group consisting of phenyl, 3-chlorophenyl, 4-chlorophenyl, 3-fluorophenyl, 4-fluorophenyl, 3,4-difluorophenyl, 3,5-difluorophenyl, 2,4,5-trifluorophenyl, 3,4,5-trifluorophenyl, 3,4-dichlorophenyl, 3-chloro-4-fluorophenyl, 4-chloro-3-fluorophenyl, 4-chloro-3-methylphenyl, 3-chloro-4-methylphenyl, 4-fluoro-3-methylphenyl, 3-fluoro-4-methylphenyl, 4-chloro-3-methoxyphenyl, 3-chloro-4-methoxyphenyl, 4-fluoro-3-methoxyphenyl, 3-fluoro-4-methoxyphenyl, 3-trifluoromethylphenyl, 4-trifluoromethylphenyl, 3-trifluoromethyl-4-fluorophenyl, 4-trifluoromethyl-3-fluorophenyl, 3-cyanophenyl, 4-cyanophenyl, 3-cyano-4-fluorophenyl, 4-cyano-3-fluorophenyl, 3-difluoromethyl-4-fluorophenyl, and 4-difluoromethyl-3-fluorophenyl.

6. The compound of claim 1, wherein R² is selected from the group consisting of H and methyl.

7. The compound of claim 1, which is selected from the group consisting of:

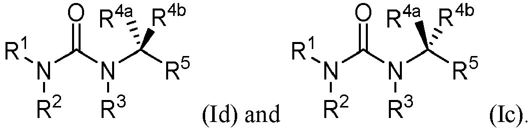

8. The compound of claim 1, which is selected from the group consisting of:

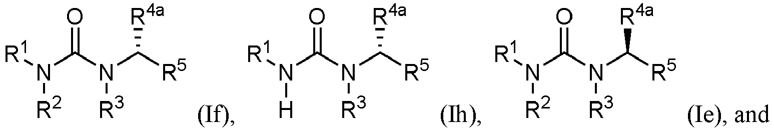

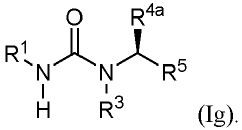

9. The compound of claim 1, wherein R⁵ is selected from the group consisting of:

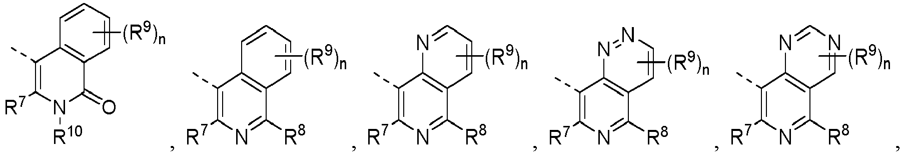

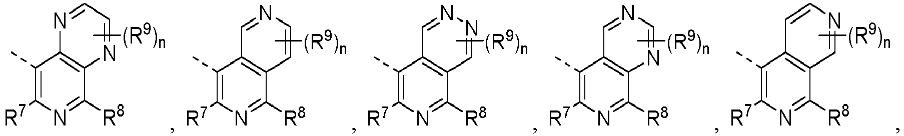

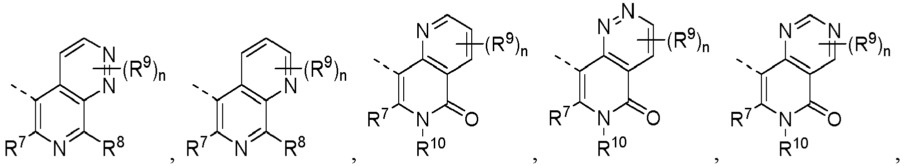

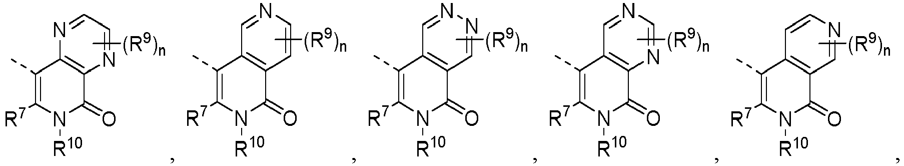

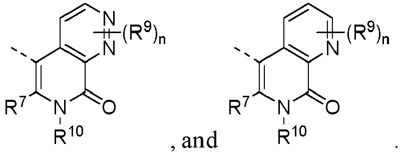

10. The compound of claim 1, which is selected from the group consisting of:
3-(3-chloro-4-fluorophenyl)-1-methyl-1-((1-oxo-1,2-dihydroisoquinolin-4-yl)methyl)urea;
3-(3-chloro-4-fluorophenyl)-1-((1-methoxyisoquinolin-4-yl)methyl)-1-methylurea;
3-(4-fluoro-3-methylphenyl)-1-((1-methoxyisoquinolin-4-yl)methyl)-1-methylurea;
3-(4-fluoro-3-methylphenyl)-1-methyl-1-((1-oxo-1,2-dihydroisoquinolin-4-yl)methyl)urea;
3-(3-chloro-4-fluorophenyl)-1-ethyl-1-((1-oxo-1,2-dihydroisoquinolin-4-yl)methyl)urea;
3-(3-chloro-4-fluorophenyl)-1-methyl-1-(1-(1-oxo-1,2-dihydroisoquinolin-4-yl)ethyl)urea;
3-(3-chloro-4-fluorophenyl)-1-ethyl-1-((1-methoxyisoquinolin-4-yl)methyl)urea;
3-(3-chloro-4-fluorophenyl)-1-ethyl-1-(isoquinolin-4-ylmethyl)urea;
3-(3-chloro-4-fluorophenyl)-1-((1-ethoxyisoquinolin-4-yl)methyl)-1-ethylurea;
3-(3-chloro-4-fluorophenyl)-1-ethyl-1-((2-methyl-1-oxo-1,2-dihydroisoquinolin-4-yl)methyl)urea;
3-(3-chloro-4-fluorophenyl)-1-isopropyl-1-((1-methoxyisoquinolin-4-yl)methyl)urea;
3-(3 -chloro-4-fluorophenyl)-1-((1-methoxyisoquinolin-4-yl)methyl)-1-propylurea;
3-(3-chloro-4-fluorophenyl)-1-(2-hydroxyethyl)-1-((1-methoxyisoquinolin-4-yl)methyl)urea;
3-(3-chloro-4-fluorophenyl)-1-ethyl-1-(1-(1-methoxylsoquinolin-4-yl)ethyl)urea;
3-(3-chloro-4-fluorophenyl)-1-ethyl-1-(1-(1-oxo-1,2-dihydroisoquinolin-4-yl)ethyl)urea;
3-(3-chloro-4-fluorophenyl)-1-isopropyl-1-((1-oxo-11,2-dihydroisoquinolin-4-yl)methyl)urea;
3-(3-chloro-4-fluorophenyl)-1-(2-hydroxyethyl)-1-((1-oxo-1,2-dihydroisoquinolin-4-yl)methyl)urea;
3-(3 -chloro-4-fluorophenyl)-1-((1-oxo-1,2-dihydroisoquinolin-4-yl)methyl)-1-propylurea;
3-(3-chloro-4-fluorophenyl)-1-ethyl-1-((1-(2-hydroxyethoxy)isoquinolin-4-yl)methyl)urea;
3-(3-chloro-4-fluorophenyl)-1-(1-(1-methoxyisoquinolin-4-yl)ethyl)-1-methylurea;
3-(3-chloro-4-fluorophenyl)-1-(cyclopropylmethyl)-1-((1-methoxylsoquinolin-4-yl)methyl)urea;
3-(4-fluoro-3-methylphenyl)-1-methyl-1-(1-(1-oxo-1,2-dihydroisoquinolin-4-yl)ethyl)urea;
1-butyl-3-(3-chloro-4-fluorophenyl)-1-((1-methoxyisoquinolin-4-yl)methyl)urea;
3-(3-chloro-4-fluorophenyl)-1-cyclopropyl-1-((1-methoxylsoquinolin-4-yl)methyl)urea;
(3-(3-chloro-4-fluorophenyl)-1-(1-(1-oxo-1,2-dihydroisoquinolin-4-yl)ethyl)-1-propylurea;
3-(3-chloro-4-fluorophenyl)-1-ethyl-1-(1-(1-methoxyisoquinolin-4-yl)propyl)urea
3-(3-chloro-4-fluorophenyl)-1-ethyl-1-(1-(1-oxo-1,2-dihydroisoquinolin-4-yl)propyl)urea;
3-(3-chloro-4-fluorophenyl)-1-(cyclopropyl(1-methoxyisoquinolin-4-yl)methyl)-1-ethylurea;
3-(3-chloro-4-fluorophenyl)-1-(2-methoxyethyl)-1-((1-methoxyisoquinolin-4-yl)methyl)urea;
3-(3-chloro-4-fluorophenyl)-1-cyclopropyl-1-((1-oxo-1,2-dihydroisoquinolin-4-yl)methyl)urea;
3-(4-fluoro-3-methylphenyl)-1-(1-(1-oxo-1,2-dihydroisoquinolin-4-yl)ethyl)-1-propylurea;
3-(3-chloro-4-fluorophenyl)-1-ethyl-1-(1-(1-methoxylsoquinolin-4-yl)-2-methylpropyl)urea;
3-(3-chloro-4-fluorophenyl)-1-(cyclopropyl(1-oxo-1,2-dihydroisoquinolin-4-yl)methyl)-1-ethylurea;
3-(3-chloro-4-fluorophenyl)-1-((1-methoxyisoquinolin-4-yl)methyl)-1-(3-methoxypropyl)urea;
1-benzyl-3-(3-chloro-4-fluorophenyl)-1-((1-methoxylsoquinolin-4-yl)methyl)urea;
3-(3-chloro-4-fluorophenyl)-1-(2-methoxyethyl)-1-((1-oxo-1,2-dlhydrolsoquinolin-4-yl)methyl)urea;
3-(3-chloro-4-fluorophenyl)-1-(3-methoxypropyl)-1-((1-oxo-1,2-dihydrolsoquinolin-4-yl)methyl)urea;
1-butyl-3-(3-chloro-4-fluorophenyl)-1-((1-oxo-1,2-dihydroisoquinolin-4-yl)methyl)urea;
3-(3-chloro-4-fluorophenyl)-1-(cyclopropylmethyl)-1-((1-oxo-1,2-dihydroisoquinolin-4-yl)methyl)urea;
3-(3 -chloro-4-fluorophenyl)-1-ethyl-1-(2-methyl-1-(1-oxo-1,2-dihydroisoquinolin-4-yl)propyl)urea;
3-(3-chloro-4-fluorophenyl)-1-cyclopropyl-1-(1-(1-methoxyisoquinolin-4-yl)ethyl)urea;
3-(3-chloro-4-fluorophenyl)-1-ethyl-1-((1-methoxylsoquinolin-4-yl)(phenyl)methyl)urea;
1-butyl-3-(3-chloro-4-fluorophenyl)-1-(1-(1-methoxyisoquinolin-4-yl)ethyl)urea;
3-(3-chloro-4-fluorophenyl)-1-(cyclopropylmethyl)-1-(1-(1-methoxyisoquinolin-4-yl)ethyl)urea;
3-(3-chloro-4-fluorophenyl)-1-(2-methoxyethyl)-1-(1-(1-oxo-1,2-dlhydrolsoquinolin-4-yl)ethyl)urea;
3-(3-chloro-4-fluorophenyl)-1-cyclopropyl-1-(1-(1-oxo-1,2-dihydroisoquinolin-4-yl)ethyl)urea;
3-(3-chloro-4-fluorophenyl)-1-ethyl-1-((1-oxo-1,2-dihydroisoquinolin-4-yl)(phenyl)methyl)urea;
3-(3-chloro-4-fluorophenyl)-1-(2-methoxyethyl)-1-(1-(1-methoxyisoquinolin-4-yl)ethyl)urea;
3-(3-chloro-4-fluorophenyl)-1-ethyl-1-(2,2,2-triffuoro-1-(1-oxo-1,2-dihydroisoquinolin-4-yl)ethyl)urea;
3-(3-chloro-4-fluorophenyl)-1-ethyl-1-(2,2,2-trifluoro-1-(1-methoxyisoquinolin-4-yl)ethyl)urea;
3-(3-chloro-4-fluorophenyl)-1-(3-methoxypropyl)-1-(1-(1-oxo-1,2-dlhydrolsoquinolin-4-yl)ethyl)urea;
3-(3-chloro-4-fluorophenyl)-1-(1-(1-methoxyisoquinolin-4-yl)ethyl)-1-(3-methoxypropyl)urea;
1-butyl-3-(3-chloro-4-fluorophenyl)-1-(1-(1-oxo-1,2-dihydroisoquinolin-4-yl)ethyl)urea;
3-(3-chloro-4-fluorophenyl)-1-(cyclopropylmethyl)-1-(1-(1-oxo-1,2-dihydroisoquinolin-4-yl)ethyl)urea;
3-(3-chloro-4-fluorophenyl)-1-methyl-1-((1-((1-methyl-1H-1,2,4-triazol-3-yl)methoxy)isoquinolin-4-yl)methyl)urea;
3-(3-chloro-4-fluorophenyl)-1-methyl-1-((1-(pyridin-2-ylmethoxy)lsoquinolin-4-yl)methyl)urea;
3-(4-fluoro-3-(trifluoromethyl)phenyl)-1-methyl-1-(1-(1-oxo-1,2-dihydroisoquinolin-4-yl)ethyl)urea;
3-(4-fluorophenyl)-1-methyl-1-(1-(1-oxo-1,2-dihydroisoquinolin-4-yl)ethyl)urea;
1-methyl-1-(1-(1-oxo-1,2-dihydroisoquinolin-4-yl)ethyl)-3-(3,4,5-trifluorophenyl)urea;
3-(3,4-difluorophenyl)-1-methyl-1-(1-(1-oxo-1,2-dihydroisoquinolin-4-yl)ethyl)urea;
3-(3-fluorophenyl)-1-methyl-1-(1-(1-oxo-1,2-dihydroisoquinolin-4-yl)ethyl)urea;
3-(2-Chloropyridin-4-yl)-1-methyl-1-(1-(1-oxo-1,2-dihydroisoquinolin-4-yl)ethyl)urea;
1-Methyl-1-(1-(1-oxo-1,2-dihydroisoquinolin-4-yl)ethyl)-3-(2-(trifluoromethyl)pyridin-4-yl)urea;
3-(3-chloro-4-fluorophenyl)-1-(2,2-difluoroethyl)-1-(1-(1-oxo-1,2-dihydroisoquinolin-4-yl)ethyl)urea;
1-(2,2-difluoroethyl)-3-(4-fluoro-3-methylphenyl)-1-(1-(1-oxo-1,2-dihydroisoquinolin-4-yl)ethyl)urea;
3-(3-cyano-4-fluorophenyl)-1-methyl-1-(1-(1-oxo-1,2-dihydroisoquinolin-4-yl)ethyl)urea;
3-(3 -chloro-4-fluorophenyl)-1-(ethyl-d5)-1-(1-(1-oxo-1,2-dihydroisoquinolin-4-yl)ethyl)urea;
N-(2-(3-(3-chloro-4-fluorophenyl)-1-(1-(1-oxo-1,2-dihydroisoquinolin-4-yl)ethyl)ureido)ethyl)acetamide;
(3-(3-chloro-4-fluorophenyl)-1-(1-(1-methoxyisoquinolin-4-yl)ethyl)-1-(2,2,2-trifluoroethyl)urea;
3-(3-chloro-4-fluorophenyl)-1-ethyl-1-(1-(1-oxo-1,2-dihydro-2,7-naphthyridin-4-yl)ethyl)urea;
3-(3-chloro-4-fluorophenyl)-1-(2-cyanoethyl)-1-(1-(1-oxo-1,2-dihydroisoquinolin-4-yl)ethyl)urea;
3-(3-(3-chloro-4-fluorophenyl)-1-(1-(1-oxo-1,2-dihydroisoquinolin-4-yl)ethyl)ureido)-N-methylpropanamide;
3-(3-(3-chloro-4-fluorophenyl)-1-(1-(1-oxo-1,2-dihydroisoquinolin-4-yl)ethyl)ureido)-N,N-dimethylpropanamide;
3-(3 -chloro-4-fluorophenyl)-1-(2-(methylsulfonyl)ethyl)-1-(1-(1-oxo-1,2-dihydroisoquinolin-4-yl)ethyl)urea;
3-(3-chloro-4-fluorophenyl)-1-(2-ethoxyethyl)-1-(1-(1-oxo-1,2-dihydroisoquinolin-4-yl)ethyl)urea;
3-(3-chloro-4-fluorophenyl)-1-methyl-1-(1-(1-oxo-1,2-dihydro-2,7-naphthyridin-4-yl)ethyl)urea;
3-(3-chloro-4-fluorophenyl)-1-(3-cyanopropyl)-1-(1-(1-oxo-1,2-dihydroisoquinolin-4-yl)ethyl)urea;
2-(3-(3-chloro-4-fluorophenyl)-1-(1-(1-oxo-1,2-dihydroisoquinolin-4-yl)ethyl)ureido)-N-methylacetamide;
2-(3 -(3-chloro-4-ffuorophenyl)-1-(1-(1-oxo-1,2-dihydroisoquinolin-4-yl)ethyl)ureido)ethane-1-sulfonamide;
3-(3-chloro-4-fluorophenyl)-1-(2-(2-methoxyethoxy)ethyl)-1-(1-(1-methoxyisoquinolin-4-yl)ethyl)urea;
3-(3-chloro-4-fluorophenyl)-1-(2-(2-methoxyethoxy)ethyl)-1-(1-(1-oxo-1,2-dihydroisoquinolin-4-yl)ethyl)urea;
3-(3-chloro-4-fluorophenyl)-1-(2-ethoxyethyl)-1-(1-(1-methoxyisoquinolin-4-yl)ethyl)urea;
3-(3-chloro-4-fluorophenyl)-1-methyl-1-(1-(1-oxo-1,2-dihydro-2,6-naphthyridin-4-yl)ethyl)urea;
2-(3-(3-chloro-4-ffuorophenyl)-1-(1-(1-oxo-1,2-dihydroisoquinolin-4-yl)ethyl)ureido)acetamide;
3-(3-chloro-4-fluorophenyl)-1-(3-hydroxypropyl)-1-(1-(1-oxo-1,2-dihydroisoquinolin-4-yl)ethyl)urea;
3-(3-chloro-4-fluorophenyl)-1-methyl-1-(1-(1-((1-methyl-1H-1,2,4-triazol-3-yl)methoxy)isoquinolin-4-yl)ethyl)urea;
3-(3-chloro-4-fluorophenyl)-1-(1-(1-oxo-1,2-dihydroisoquinolin-4-yl)ethyl)-1-((tetrahydro-2H-pyran-4-yl)methyl)urea;
3-(3-chloro-4-fluorophenyl)-1-isobutyl-1-(1-(1-oxo-1,2-dihydroisoquinolin-4-yl)ethyl)urea;
3-(3-chloro-4-fluorophenyl)-1-methyl-1-(1-(5-(methylamino)pyrido[3,4-b]pyrazin-8-yl)ethyl)urea;
3-(3-chloro-4-fluorophenyl)-1-(1-(5-methoxypyrido[3,4-b]pyrazin-8-yl)ethyl)-1-methylurea;
3-(3-chloro-4-fluorophenyl)-1-(3-hydroxypropyl)-1-(1-(1-methoxyisoquinolin-4-yl)ethyl)urea;
1-(1-(1-((1H-1,2,4-triazol-3-yl)methoxy)isoquinolin-4-yl)ethyl)-3-(3-chloro-4-fluorophenyl)-1-methylurea;
2-(3-(3-chloro-4-fluorophenyl)-1-(1-(1-oxo-1,2-dihydroisoquinolin-4-yl)ethyl)ureido)-N,N-dimethylacetamide;
3-(3 -chloro-4-fluorophenyl)-1-methyl-1-(1-(5-oxo-5,6-dihydropyrido[3,4-b]pyrazin-8-yl)ethyl)urea;
3-(3-chloro-4-fluorophenyl)-1-(4-hydroxybutyl)-1-(1-(1-oxo-1,2-dihydroisoquinolin-4-yl)ethyl)urea;
3-(3-chloro-4-fluorophenyl)-1-methyl-1-(1-(8-oxo-7,8-dihydro-1,7-naphthyridin-5-yl)ethyl)urea;
3-(3-chloro-4-fluorophenyl)-1-isobutyl-1-(1-(1-oxo-1,2-dihydro-2,7-naphthyridin-4-yl)ethyl)urea;
3-(3-chloro-4-fluorophenyl)-1-(1-(1-oxo-1,2-dihydroisoquinolin-4-yl)ethyl)-1-(((R)-tetrahydrofuran-2-yl)methyl)urea;
1-(3-aminopropyl)-3-(3-chloro-4-fluorophenyl)-1-(1-(1-oxo-1,2-dihydroisoquinolin-4-yl)ethyl)urea;
3-(3-chloro-4-fluorophenyl)-1-(1-(1-methoxylsoquinolin-4-yl)ethyl)-1-(2-((2,2,2-trifluoroethyl)amino)ethyl)urea;
1-(3-hydroxypropyl)-1-(1-(1-oxo-1,2-dihydro-2,7-naphthyridin-4-yl)ethyl)urea;
3-(3-chloro-4-fluorophenyl)-1-(1-(1-oxo-1,2-dihydroisoquinolin-4-yl)ethyl)-1-(((S)-tetrahydrofuran-2-yl)methyl)urea;
3-(3-chloro-4-fluorophenyl)-1-(1-(1-oxo-1,2-dihydroisoquinolin-4-yl)ethyl)-1-(2-((2,2,2-trifluoroethyl)amino)ethyl)urea;
3-(3-chloro-4-fluorophenyl)-1-(1-(1-oxo-1,2-dihydroisoquinolin-4-yl)ethyl)-1-((tetrahydrofuran-3-yl)methyl)urea;
1-(1-(8-chloro-1-oxo-1,2-dihydroisoquinolin-4-yl)ethyl)-3-(3-chloro-4-fluorophenyl)-1-methylurea;
3-(3-chloro-4-fluorophenyl)-1-(1-(6-fluoro-1-oxo-1,2-dihydroisoquinolin-4-yl)ethyl)-1-methylurea;
3-(3-chloro-4-fluorophenyl)-1-(1-(6-chloro-1-oxo-1,2-dihydroisoquinolin-4-yl)ethyl)-1-methylurea;
3-(3-chloro-4-fluorophenyl)-1-(1-(7-methoxy-1-oxo-1,2-dihydroisoquinolin-4-yl)ethyl)-1-methylurea;
3-(3-chloro-4-fluorophenyl)-1-(1-(6-methoxy-1-oxo-1,2-dihydroisoquinolin-4-yl)ethyl)-1-methylurea;
3-(3-chloro-4-fluorophenyl)-1-methyl-1-(1-(2-methyl-1-oxo-1,2-dihydroisoquinolin-4-yl)ethyl)urea;
3-(3-chloro-4-fluorophenyl)-1-methyl-1-(1-(2-ethyl-1-oxo-1,2-dihydroisoquinolin-4-yl)ethyl)urea;
3-(3-chloro-4-fluorophenyl)-1-methyl-1-(1-(2-propyl-1-oxo-1,2-dihydroisoquinolin-4-yl)ethyl)urea;
3-(3 -chloro-4-fluorophenyl)-1-methyl-1-(1-(2-isopropyl-1-oxo-1,2-dihydroisoquinolin-4-yl)ethyl)urea;
3-(3-chloro-4-fluorophenyl)-1-methyl-1-(1-(2-cyclopropyl-1-oxo-1,2-dihydroisoquinolin-4-yl)ethyl)urea;
3-(3-chloro-4-fluorophenyl)-1-(1-(2-(2-methoxyethyl)-1-oxo-1,2-dihydroisoquinolin-4-yl)ethyl)-1-methylurea;
3-(3 -chloro-4-fluorophenyl)-1-(cyclopentylmethyl)-1-(1-(1-oxo-1,2-dihydroisoquinolin-4-yl)ethyl)urea;
3-(3-chloro-4-fluorophenyl)-1-(cyclohexylmethyl)-1-(1-(1-oxo-1,2-dlhydrolsoquinolin-4-yl)ethyl)urea;
3-(3-chloro-4-fluorophenyl)-1-(2-hydroxyethyl)-1-(1-(1-oxo-1,2-dihydroisoquinolin-4-yl)ethyl)urea;
3-(3 -chloro-4-fluorophenyl)-1-(2-((2,2-difluoroethyl)amino)ethyl)-1-(1-(1-oxo-1,2-dihydroisoquinolin-4-yl)ethyl)urea;
3-(3-chloro-4-fluorophenyl)-1-(1-(2-(3-methoxypropyl)-1-oxo-1,2-dihydroisoquinolin-4-yl)ethyl)-1-methylurea;
1-((1-acetylpiperidin-4-yl)methyl)-3-(3-chloro-4-fluorophenyl)-1-(1-(1-oxo-1,2-dihydroisoquinolin-4-yl)ethyl)urea;
3-(3-chloro-4-fluorophenyl)-1-((1-(methylsulfonyl)piperidin-4-yl)methyl)-1-(1-(1-oxo-1,2-dihydroisoquinolin-4-yl)ethyl)urea;
3-(3 -chloro-4-fluorophenyl)-1-(1-(2-(2-hydroxyethyl)-1-oxo-1,2-dihydroisoquinolin-4-yl)ethyl)-1-methylurea;
3-(3-chloro-4-fluorophenyl)-1-(1-(2-(3-hydroxypropyl)-1-oxo-1,2-dihydroisoquinolin-4-yl)ethyl)-1-methylurea;
3-(3-chloro-4-fluorophenyl)-1-((1-methylpiperidin-4-yl)methyl)-1-(1-(1-oxo-1,2-dihydroisoquinolin-4-yl)ethyl)urea;
3-(3-(3-chloro-4-fluorophenyl)-1-(1-(1-oxo-1,2-dihydroisoquinolin-4-yl)ethyl)ureido)propanoic acid;
3-(3-chloro-4-fluorophenyl)-1-(3-hydroxy-2,2-dimethylpropyl)-1-(1-(1-oxo-1,2-dihydroisoquinolin-4-yl)ethyl)urea;
3-(3-chloro-4-fluorophenyl)-1-((R)-3-hydroxybutyl)-1-(1-(1-oxo-1,2-dihydroisoquinolin-4-yl)ethyl)urea;
3-(3 -chloro-4-fluorophenyl)-1 -((S)-3 -hydroxybutyl)-1 -(1 -(1 -oxo-1,2-dihydroisoquinolin-4-yl)ethyl)urea;
3-(3-chloro-4-fluorophenyl)-1-((R)-2-hydroxypropyl)-1-(1-(1-oxo-1,2-dihydroisoquinolin-4-yl)ethyl)urea;
3-(3-chloro-4-fluorophenyl)-1-((S)-2-hydroxypropyl)-1-(1-(1-oxo-1,2-dihydroisoquinolin-4-yl)ethyl)urea;
3-(3-(3-chloro-4-fluorophenyl)-1-(1-(1-oxo-1,2-dihydroisoquinolin-4-yl)ethyl)ureido)-N-methylpropane-1-sulfonamide;
3-(3-chloro-4-fluorophenyl)-1-(((1r,4r)-4-hydroxycyclohexyl)methyl)-1-(1-(1-oxo-1,2-dihydroisoquinolin-4-yl)ethyl)urea;
3-(3-chloro-4-fluorophenyl)-1-methyl-1-(1-(3-methyl-1-oxo-1,2-dihydroisoquinolin-4-yl)ethyl)urea;
3-(3-chloro-4-fluorophenyl)-1-((4-cis-hydroxycyclohexyl)methyl)-1-(1-(1-oxo-1,2-dihydrolsoquinolin-4-yl)ethyl)urea;
4-(3-(3-chloro-4-fluorophenyl)-1-(1-(1-oxo-1,2-dihydroisoquinolin-4-yl)ethyl)ureido)butanoic acid;
3-(3-chloro-4-fluorophenyl)-1-methyl-1-(1-(1-oxo-2-(2,2,2-triffuoroethyl)-1,2-dihydroisoquinolin-4-yl)ethyl)urea;
3-(3 -chloro-4-fluorophenyl)-1-isobutyl-1-(1-(1-oxo-2-(2,2,2-trifluoroethyl)-1,2-dihydroisoquinolin-4-yl)ethyl)urea;
3-(4-fluoro-3-methylphenyl)-1-methyl-1-(1-(3-methyl-1-oxo-1,2-dihydroisoquinolin-4-yl)ethyl)urea;
3-(3-chloro-4-fluorophenyl)-1-methyl-1-(1-(1-(methylamino)isoquinolin-4-yl)ethyl)urea;
3-(3-chloro-4-fluorophenyl)-1-methyl-1-(1-(1-(dimethylamino)isoquinolin-4-yl)ethyl)urea;
3-(4-fluorophenyl)-1-isobutyl-1-(1-(1-oxo-2-(2,2,2-trifluoroethyl)-1,2-dihydroisoquinolin-4-yl)ethyl)urea;
3-(4-fluoro-3-methylphenyl)-1-methyl-1-(1-(3-methyl-1-oxo-1,2-dihydroisoquinolin-4-yl)ethyl)urea;
1-(1-(1-aminoisoquinolin-4-yl)ethyl)-3-(3-chloro-4-fluorophenyl)-1-methylurea;
3-(3-chloro-4-fluorophenyl)-1-(1-(1-(ethylamino)isoquinolin-4-yl)ethyl)-1-methylurea;
3-(3-chloro-4-fluorophenyl)-1-(1-(1-((2-hydroxyethyl)amino)isoquinolin-4-yl)ethyl)-1-methylurea;
1-(1-(1-((2-aminoethyl)amino)isoquinolin-4-yl)ethyl)-3-(3-chloro-4-fluorophenyl)-1-methylurea;
3-(3-chloro-4-fluorophenyl)-1-methyl-1-(1-(1-(((1-methyl-1H-1,2,4-triazol-3-yl)methyl)amino)isoquinolin-4-yl)ethyl)urea;
1-(1-(1-(((1H-1,2,4-triazol-3-yl)methyl)amino)isoquinolin-4-yl)ethyl)-3-(3-chloro-4-fluorophenyl)-1-methylurea;
3-(3-chloro-4-fluorophenyl)-1-(3-hydroxypropyl)-1-(1-(1-((1-methyl-1H-1,2,4-triazol-3-yl)methoxy)isoquinolin-4-yl)ethyl)urea;
3-(3-chloro-4-fluorophenyl)-1-(1-(7-fluoro-1-oxo-1,2-dihydroisoquinolin-4-yl)ethyl)-1-methylurea;
1-(1-(7-fluoro-1-oxo-1,2-dihydroisoquinolin-4-yl)ethyl)-3-(4-fluorophenyl)-1-methylurea;
1-(1-(1-((1H-1,2,4-triazol-3-yl)methoxy)isoquinolin-4-yl)ethyl)-3-(3-chloro-4-fluorophenyl)-1-(3-hydroxypropyl)urea;
1-(1-(1-(((2H-1,2,3-triazol-4-yl)methyl)amino)isoquinolin-4-yl)ethyl)-3-(3-chloro-4-ffuorophenyl)-1-methylurea;
3-(3-chloro-4-fluorophenyl)-1-(1-(5-methoxy-1-oxo-1,2-dihydroisoquinolin-4-yl)ethyl)-1-methylurea;
1-((1H-1,2,3-triazol-4-yl)methyl)-3-(3 -chloro-4-fluorophenyl)-1-(1-(1-oxo-1,2-dihydroisoquinolin-4-yl)ethyl)urea;
1-((4H-1,2,4-triazol-3-yl)methyl)-3-(3-chloro-4-fluorophenyl)-1-(1-(1-oxo-1,2-dihydroisoquinolin-4-yl)ethyl)urea;
1-(1-(2-((1H-1,2,4-triazol-3-yl)methyl)-1-oxo-1,2-dihydroisoquinolin-4-yl)ethyl)-3-(3-chloro-4-fluorophenyl)-1-methylurea;
1-(1-(7-chloro-1-oxo-1,2-dihydroisoquinolin-4-yl)ethyl)-3-(3-chloro-4-fluorophenyl)-1-methylurea;
3-(3-chloro-4-fluorophenyl)-1-((2,2-dimethyl-1,3-dioxan-5-yl)methyl)-1-(1-(1-oxo-1,2-dihydroisoquinolin-4-yl)ethyl)urea;
1-((2,2-dimethyl-1,3-dioxan-5-yl)methyl)-3-(4-fluorophenyl)-1-(1-(1-oxo-1,2-dihydroisoquinolin-4-yl)ethyl)urea;
1-((2,2-dimethyl-1,3-dioxan-5-yl)methyl)-1-(1-(1-oxo-1,2-dihydroisoquinolin-4-yl)ethyl)-3-phenylurea;
1-(1-(2-((1H-1,2,3-triazol-4-yl)methyl)-1-oxo-1,2-dihydroisoquinolin-4-yl)ethyl)-3-(3-chloro-4-fluorophenyl)-1-methylurea;
3-(3-chloro-4-fluorophenyl)-1-(1-(6,7-difluoro-1-oxo-1,2-dihydroisoquinolin-4-yl)ethyl)-1-methylurea;
3-(3-chloro-4-fluorophenyl)-1-(1-(6,7-difluoro-1-oxo-1,2-dihydroisoquinolin-4-yl)ethyl)-1-isobutylurea;
3-(3 -chloro-4-fluorophenyl)-1-((2,2-dimethyl-1,3 -dioxan-5-yl)methyl)-1-(1-(6-ffuoro-1-oxo-1,2-dihydroisoquinolin-4-yl)ethyl)urea;
3-(3-chloro-4-fluorophenyl)-1-(3-hydroxy-2-(hydroxymethyl)propyl)-1-(1-(1-oxo-1,2-dihydroisoquinolin-4-yl)ethyl)urea;
1-(1-(6,7-diffuoro-1-oxo-1,2-dihydroisoquinolin-4-yl)ethyl)-3-(4-fluorophenyl)-1-methylurea;
1-(1-(6,7-difluoro-1-oxo-1,2-dihydroisoquinolin-4-yl)ethyl)-3-(4-fluorophenyl)-1-isobutylurea;
3-(3-chloro-4-fluorophenyl)-1-(1-(6,7-difluoro-1-oxo-1,2-dihydroisoquinolin-4-yl)ethyl)-1-(3-hydroxypropyl)urea;
1-(1-(6,7-diffuoro-1-oxo-1,2-dihydroisoquinolin-4-yl)ethyl)-3-(4-fluorophenyl)-1-(3-hydroxypropyl)urea;
3-(4-chlorophenyl)-1-methyl-1-(1-(1-oxo-1,2-dihydroisoquinolin-4-yl)ethyl)urea;
3-(4-bromophenyl)-1-methyl-1-(1-(1-oxo-1,2-dihydroisoquinolin-4-yl)ethyl)urea;
3-(3-chloro-4-fluorophenyl)-1-(1-(6-fluoro-1-oxo-1,2-dihydroisoquinolin-4-yl)ethyl)-1-(3-hydroxy-2-(hydroxymethyl)propyl)urea;
1-(1-(1-(1H-1,2,4-triazol-1-yl)isoquinolin-4-yl)ethyl)-3-(3-chloro-4-fluorophenyl)-1-methylurea;
3-cyclopentyl-1-methyl-1-(1-(1-oxo-1,2-dihydroisoquinolin-4-yl)ethyl)urea;
1-(1-(1-(1H-1,2,4-triazol-1-yl)isoquinolin-4-yl)ethyl)-3-(3-chloro-4-fluorophenyl)-1-isobutylurea;
1-(1-(1-(1H-1,2,4-triazol-1-yl)isoquinolin-4-yl)ethyl)-3-(4-fluorophenyl)-1-isobutylurea;
3-(3-chloro-4-fluorophenyl)-1-(1-(7-fluoro-2-methyl-1-oxo-1,2-dihydroisoquinolin-4-yl)ethyl)-1-methylurea;
3-(3-chloro-4-fluorophenyl)-1-(1-(1-oxo-1,2-dihydroisoquinolin-4-yl)ethyl)-1-(pyridin-2-ylmethyl)urea;
3-(3-chloro-4-fluorophenyl)-1-(1-(1-oxo-1,2-dihydroisoquinolin-4-yl)ethyl)-1-(pyridin-3-ylmethyl)urea;
3-(3-chloro-4-fluorophenyl)-1-(1-(1-oxo-1,2-dihydroisoquinolin-4-yl)ethyl)-1-(pyridin-4-ylmethyl)urea;
3-(3-chloro-4-fluorophenyl)-1-(1-(1-oxo-1,2-dihydroisoquinolin-4-yl)ethyl)-1-(thiazol-2-ylmethyl)urea;
3-(3-chloro-4-fluorophenyl)-1-(1-(1-oxo-1,2-dihydroisoquinolin-4-yl)ethyl)-1-(thiazol-4-ylmethyl)urea;
3-(3-chloro-4-fluorophenyl)-1-methyl-1-(1-(1-(methylsulfonyl)isoquinolin-4-yl)ethyl)urea;
4-(1-(3-(3-chloro-4-fluorophenyl)-1-methylureido)ethyl)-N-methylisoquinoline-1-carboxamide;
4-(1-(3-(3-chloro-4-fluorophenyl)-1-methylureldo)ethyl)-N,N-dimethylisoquinoline-1-carboxamide;
3-(3-chloro-4-fluorophenyl)-1-(1-(1-oxo-1,2-dihydroisoquinolin-4-yl)ethyl)-1-(pyrimidin-5-ylmethyl)urea;
3-(3-chloro-4-fluorophenyl)-1-(1-(1-oxo-1,2-dihydroisoquinolin-4-yl)ethyl)-1-(pyrimidin-4-ylmethyl)urea;
3-(3-chloro-4-fluorophenyl)-1-(1-(1-oxo-1,2-dihydroisoquinolin-4-yl)ethyl)-1-(thiazol-5-ylmethyl)urea;
4-(1-(3-(3-chloro-4-fluorophenyl)-1-methylureido)ethyl)isoquinoline-1-carboxylic acid;
3-(3-chloro-4-fluorophenyl)-1-(1-(1-(hydroxymethyl)isoquinolin-4-yl)ethyl)-1-methylurea;
4-(1-(3-(3 -chloro-4-fluorophenyl)-1-methylureido)ethyl)isoquinoline-1-carboxamide;
3-(3 -chloro-4-fluorophenyl)-1-(1-(7, 8-difluoro-1-oxo-1,2-dihydroisoquinolin-4-yl)ethyl)-1-methylurea;
3-(3-chloro-4-fluorophenyl)-1-(1-(7,8-difluoro-2-methyl-1-oxo-1,2-dihydroisoquinolin-4-yl)ethyl)-1-methylurea;
3-(3 -chloro-4-fluorophenyl)-1-(1-(7, 8-difluoro-1-oxo-1,2-dihydroisoquinolin-4-yl)ethyl)-1-(3-hydroxypropyl)urea;
3-(3-chloro-4-fluorophenyl)-1-(1-(1-cyanoisoquinolin-4-yl)ethyl)-1-methylurea;
3-(3-chloro-4-fluorophenyl)-1-(1-(7,8-difluoro-1-oxo-1,2-dihydroisoquinolin-4-yl)ethyl)-1-isobutylurea;
3-(3-chloro-4-fluorophenyl)-1-(1-(6,7-difluoro-2-methyl-1-oxo-1,2-dihydroisoquinolin-4-yl)ethyl)-1-methylurea;
3-(3-chloro-4-fluorophenyl)-1-(1-(6,7-difluoro-2-methyl-1-oxo-1,2-dihydroisoquinolin-4-yl)ethyl)-1-isobutylurea;
3-(3-chloro-4-fluorophenyl)-1-(1-(6,7-difluoro-1-((1-methyl-1H-1,2,4-triazol-3-yl)methoxy)isoquinolin-4-yl)ethyl)-1-methylurea;
3-(3-chloro-4-fluorophenyl)-1-(1-(6,7-difluoro-1-((1-methyl-1H-1,2,4-triazol-3-yl)methoxy)isoquinolin-4-yl)ethyl)-1 -(3 -hydroxypropyl)urea;
3-(3-chloro-4-fluorophenyl)-1-(1-(6,7-difluoro-2-methyl-1-oxo-1,2-dihydroisoquinolin-4-yl)ethyl)-1-(3-hydroxypropyl)urea;
3-(3-chloro-4-fluorophenyl)-1-methyl-1-(1-(1-methylisoquinolin-4-yl)ethyl)urea;
1-(1-(1-(1H-1,2,3-triazol-1-yl)isoquinolin-4-yl)ethyl)-3-(3-chloro-4-fluorophenyl)-1-methylurea;
1-(1-(1-(1H-1,2,3-triazol-1-yl)isoquinolin-4-yl)ethyl)-3-(3-chloro-4-fluorophenyl)-1-isobutylurea;
3-(3 -chloro-4-fluorophenyl)-1-(1-(8-fluoro-1-oxo-1,2-dihydroisoquinolin-4-yl)ethyl)-1-methylurea;
3-(3 -chloro-4-fluorophenyl)-1-(1-(8-fluoro-2-methyl-1-oxo-1,2-dihydroisoquinolin-4-yl)ethyl)-1-methylurea;
3-(3 -chloro-4-fluorophenyl)-1-(1-(8-fluoro-1-oxo-1,2-dihydroisoquinolin-4-yl)ethyl)-1-isobutylurea;
1-(1-(1-((1H-1,2,4-triazol-3-yl)methoxy)-6,7-difluoroisoquinolin-4-yl)ethyl)-3-(3-chloro-4-fluorophenyl)-1-methylurea;
1-(1-(1-((1H-1,2,4-triazol-3-yl)methoxy)-6,7-difluoroisoquinolin-4-yl)ethyl)-3-(3-chloro-4-fluorophenyl)-1-(3-hydroxypropyl)urea;
3-(3-chloro-4-fluorophenyl)-1-(1-(8-fluoro-1-oxo-1,2-dihydroisoquinolin-4-yl)ethyl)-1-(3-hydroxypropyl)urea;
3-(3-chloro-4-fluorophenyl)-1-(1-(6,8-difluoro-1-oxo-1,2-dihydroisoquinolin-4-yl)ethyl)-1-methylurea;
1-(1-(6,8-difluoro-1-oxo-1,2-dihydroisoquinolin-4-yl)ethyl)-3-(4-fluorophenyl)-1-methylurea;
3-(3-chloro-4-fluorophenyl)-1-(1-(6,8-difluoro-1-oxo-1,2-dihydroisoquinolin-4-yl)ethyl)-1-(3-hydroxypropyl)urea;
1-(1-(6,8-diffuoro-1-oxo-1,2-dihydroisoquinolin-4-yl)ethyl)-3-(4-fluorophenyl)-1-isobutylurea;
1-(1-(6,8-difluoro-1-oxo-1,2-dihydroisoquinolin-4-yl)ethyl)-3-(4-fluorophenyl)-1-(3-hydroxypropyl)urea;
3-(3-chloro-4-fluorophenyl)-1-(2-hydroxy-1-(1-oxo-1,2-dihydroisoquinolin-4-yl)ethyl)-1-methylurea;
2-(3-(3-chloro-4-fluorophenyl)-1-(1-(6-fluoro-1-oxo-1,2-dihydroisoquinolin-4-yl)ethyl)ureido)ethane-1-sulfonamide;
2-(3-(3-chloro-4-fluorophenyl)-1-(1-(7-fluoro-1-oxo-1,2-dihydroisoquinolin-4-yl)ethyl)ureido)ethane-1-sulfonamide;
2-(3-(3-chloro-4-fluorophenyl)-1-(1-(6,7-diffuoro-1-oxo-1,2-dihydroisoquinolin-4-yl)ethyl)ureido)ethane-1 -sulfonamide;
2-(1-(1-(6,7-difluoro-1-oxo-1,2-dihydroisoquinolin-4-yl)ethyl)-3-(4-fluorophenyl)ureido)ethane-1-sulfonamide;
2-(3-(3-chloro-4-fluorophenyl)-1-(1-(6,8-difluoro-1-oxo-1,2-dihydroisoquinolin-4-yl)ethyl)ureido)ethane-1-sulfonamide;
2-(3-(3-chloro-4-fluorophenyl)-1-(1-(7,8-difluoro-1-oxo-1,2-dihydroisoquinolin-4-yl)ethyl)ureido)ethane-1 -sulfonamide;
2-(3-(4-fluorophenyl)-1-(1-(7,8-difluoro-1-oxo-1,2-dihydroisoquinolin-4-yl)ethyl)ureido)ethane-1-sulfonamide;
2-(3-(3-chloro-4-fluorophenyl)-1-(1-(8-fluoro-1-oxo-1,2-dihydroisoquinolin-4-yl)ethyl)ureido)ethane-1-sulfonamide;
1-(1-(6,7-difluoro-1-oxo-1,2-dihydroisoquinolin-4-yl)ethyl)-1-methyl-3-phenylurea;
3-(4-chlorophenyl)-1-(1-(6,7-difluoro-1-oxo-1,2-dihydroisoquinolin-4-yl)ethyl)-1-methylurea;
1-(1-(6,7-difluoro-1-oxo-1,2-dihydroisoquinolin-4-yl)ethyl)-1-ethyl-3-(4-fluorophenyl)urea;
1-(1-(6,7-difluoro-1-oxo-1,2-dihydroisoquinolin-4-yl)ethyl)-1-ethyl-3-phenylurea;
3-(3-chloro-4-fluorophenyl)-1-(1-(6,7-difluoro-1-oxo-1,2-dihydroisoquinolin-4-yl)ethyl)-1-ethylurea;
1-(1-(6,7-difluoro-1-oxo-1,2-dihydroisoquinolin-4-yl)ethyl)-3-(3,4-difluorophenyl)-1-methylurea;
1-(1-(6,7-diffuoro-1-oxo-1,2-dihydroisoquinolin-4-yl)ethyl)-3-(3-fluorophenyl)-1-methylurea;
3-(3-chlorophenyl)-1-(1-(6,7-difluoro-1-oxo-1,2-dihydroisoquinolin-4-yl)ethyl)-1-methylurea;
1-(1-(6,7-difluoro-1-oxo-1,2-dihydroisoquinolin-4-yl)ethyl)-1-methyl-3-(3,4,5-trifluorophenyl)urea;
3-(3,5-dichlorophenyl)-1-(1-(6,7-difluoro-1-oxo-1,2-dihydroisoquinolin-4-yl)ethyl)-1-methylurea;
3-(2-chloropyridin-4-yl)-1-(1-(6,7-difluoro-1-oxo-1,2-dihydroisoquinolin-4-yl)ethyl)-1-methylurea;
1-(1-(6,7-diffuoro-1-oxo-1,2-dihydroisoquinolin-4-yl)ethyl)-3-(2-fluorophenyl)-1-methylurea;
1-(1-(6,7-diffuoro-1-oxo-1,2-dihydroisoquinolin-4-yl)ethyl)-3-(3,4-difluorophenyl)-1-ethylurea;
1-(1-(6,7-Difluoro-1-oxo-1,2-dihydroisoquinolin-4-yl)ethyl)-3-(4-fluoro-3-methylphenyl)-1-methylurea;
3-(3-cyano-4-fluorophenyl)-1-(1-(6,7-difluoro-1-oxo-1,2-dihydroisoquinolin-4-yl)ethyl)-1-methylurea;
1-(1-(6,7-Difluoro-1-oxo-1,2-dihydroisoquinolin-4-yl)ethyl)-3-(2,3-difluorophenyl)-1-methylurea;
1-(1-(7,8-Difluoro-1-oxo-1,2-dihydroisoquinolin-4-yl)ethyl)-3-(4-fluorophenyl)-1-methylurea;
1-(1-(7,8-Difluoro-1-oxo-1,2-dihydroisoquinolin-4-yl)ethyl)-3-(3,4-difluorophenyl)-1-methylurea;
1-(1-(7,8-Difluoro-1-oxo-1,2-dihydroisoquinolin-4-yl)ethyl)-1-methyl-3-phenylurea;
3-(3,5-dichloro-4-fluorophenyl)-1-(1-(6,7-difluoro-1-oxo-1,2-dihydroisoquinolin-4-yl)ethyl)-1-methylurea;
1-(1-(7,8-Difluoro-1-oxo-1,2-dihydroisoquinolin-4-yl)ethyl)-3-(4-fluoro-3-methylphenyl)-1-methylurea;
1-(1-(7,8-Difluoro-1-oxo-1,2-dihydroisoquinolin-4-yl)ethyl)-1-methyl-3-(3,4,5-trifluorophenyl)urea;
3-(4-Chlorophenyl)-1-(1-(7,8-difluoro-1-oxo-1,2-dihydroisoquinolin-4-yl)ethyl)-1-methylurea;
3-(3-chloro-4-fluorophenyl)-1-(1-(6,8-difluoro-1-oxo-1,2-dihydroisoquinolin-4-yl)ethyl)-1-isobutylurea;
3-(3,5-dichlorophenyl)-1-(1-(7,8-difluoro-1-oxo-1,2-dihydroisoquinolin-4-yl)ethyl)-1-methylurea;
1-(1-(7,8-difluoro-1-oxo-1,2-dihydroisoquinolin-4-yl)ethyl)-1-methyl-3-(pyridin-4-yl)urea;
1-(1-(7,8-difluoro-1-oxo-1,2-dihydroisoquinolin-4-yl)ethyl)-1-methyl-3-(pyridin-3-yl)urea;
3-(3,5-dichloro-4-fluorophenyl)-1-(1-(7, 8-difluoro-1-oxo-1,2-dihydroisoquinolin-4-yl)ethyl)-1-methylurea;
3-benzyl-1-(1-(7,8-difluoro-1-oxo-1,2-dihydroisoquinolin-4-yl)ethyl)-1-methylurea;
3-(2-chloropyridin-4-yl)-1-(1-(7, 8-difluoro-1-oxo-1,2-dihydroisoquinolin-4-yl)ethyl)-1-methylurea;
1-(1-(7,8-difluoro-1-oxo-1,2-dihydroisoquinolin-4-yl)ethyl)-3-(3-fluorophenyl)-1-methylurea;
1-(1-(7,8-difluoro-1-oxo-1,2-dihydroisoquinolin-4-yl)ethyl)-3-(2-fluorophenyl)-1-methylurea;
1-(1-(7,8-difluoro-1-oxo-1,2-dihydroisoquinolin-4-yl)ethyl)-3-(2,3-difluorophenyl)-1-methylurea;
1-(1-(7,8-difluoro-1-oxo-1,2-dihydroisoquinolin-4-yl)ethyl)-3-(3-chlorophenyl)-1-methylurea;
1-(1-(7,8-difluoro-1-oxo-1,2-dihydroisoquinolin-4-yl)ethyl)-3-(3,4-difluorophenyl)-1-ethylurea;
1-(1-(7,8-difluoro-1-oxo-1,2-dihydroisoquinolin-4-yl)ethyl)-3-(4-fluorophenyl)-1-ethylurea;
1-(1-(7,8-difluoro-1-oxo-1,2-dihydroisoquinolin-4-yl)ethyl)-3-phenyl-1-ethylurea;
1-(1-(7,8-difluoro-1-oxo-1,2-dihydroisoquinolin-4-yl)ethyl)-3-(3-chloro-4-fluorophenyl)-1-ethylurea;
3-(3-cyano-4-fluorophenyl)-1-(1-(7,8-difluoro-1-oxo-1,2-dihydroisoquinolin-4-yl)ethyl)-1-methylurea;
3-(3 -chloro-4-fluorophenyl)-1-(1-(6,7-difluoro-1-oxo-1,2-dihydroisoquinolin-4-yl)ethyl)-1-(methyl)urea;
3-(3 -chloro-4-fluorophenyl)-1-(1-(6,7-difluoro-1-oxo-1,2-dihydroisoquinolin-4-yl)ethyl)-1-(methyl-d₃)urea;
3-(3-chloro-4-fluorophenyl)-1-(1-(7,8-difluoro-1-oxo-1,2-dihydroisoquinolin-4-yl)ethyl)-1-(methyl)urea;
3-(3-chloro-4-fluorophenyl)-1-(1-(7,8-difluoro-1-oxo-1,2-dihydroisoquinolin-4-yl)ethyl)-1-(methyl-d₃)urea;
3-(3-chlorophenyl)-1-(1-(7,8-difluoro-1-oxo-1,2-dihydroisoquinolin-4-yl)ethyl)-1-(methyl)urea;
3-(3-chlorophenyl)-1-(1-(7,8-difluoro-1-oxo-1,2-dihydroisoquinolin-4-yl)ethyl)-1-(methyl-d₃)urea;
1-(1-(6,7-diffuoro-1-oxo-1,2-dihydroisoquinolin-4-yl)ethyl)-1-methyl-3-((S)-1-phenylethyl)urea;
1-(1-(6,7-difluoro-1-oxo-1,2-dihydroisoquinolin-4-yl)ethyl)-3-(3,4-difluorobenzyl)-1-methylurea;
1-(1-(6,7-difluoro-1-oxo-1,2-dihydroisoquinolin-4-yl)ethyl)-3-(4-fluorobenzyl)-1-methylurea;
1-(1-(6,7-difluoro-1-oxo-1,2-dihydroisoquinolin-4-yl)ethyl)-1-methyl-3-((R)-1-phenylethyl)urea;
1-(1-(6,7-difluoro-1-oxo-1,2-dihydroisoquinolin-4-yl)ethyl)-1-methyl-3-(3,4,5-trifluorobenzyl)urea;
1-(1-(6,7-difluoro-1-oxo-1,2-dihydroisoquinolin-4-yl)ethyl)-1-methyl-3-(3-chloro-4-fluorobenzyl)urea;
1-(1-(6,7-diffuoro-1-oxo-1,2-dihydroisoquinolin-4-yl)ethyl)-3-(1H-indol-6-yl)-1-methylurea;
1-(1-(7,8-difluoro-1-oxo-1,2-dihydroisoquinolin-4-yl)ethyl)-1-methyl-3-((S)-1-phenylethyl)urea;
1-(1-(7,8-difluoro-1-oxo-1,2-dihydroisoquinolin-4-yl)ethyl)-1-methyl-3-((R)-1-phenylethyl)urea;
1-(1-(7,8-difluoro-1-oxo-1,2-dihydroisoquinolin-4-yl)ethyl)-3-(3,4-difluorobenzyl)-1-methylurea;
1-(1-(7,8-difluoro-1-oxo-1,2-dihydroisoquinolin-4-yl)ethyl)-3-(4-fluorobenzyl)-1-methylurea;
1-(1-(7,8-difluoro-1-oxo-1,2-dihydroisoquinolin-4-yl)ethyl)-3-(3,4,5-trifluorobenzyl)-1-methylurea;
3-(3-chloro-4-fluorobenzyl)-1-(1-(7,8-difluoro-1-oxo-1,2-dihydroisoquinolin-4-yl)ethyl)-1-methylurea;
1-(1-(7, 8-diffuoro-1-oxo-1,2-dihydroisoquinolin-4-yl)ethyl)-3-(1H-indol-6-yl)-1-methylurea;
1-(1-(6,7-difluoro-1-oxo-1,2-dihydroisoquinolin-4-yl)ethyl)-3-(3-(difluoromethyl)-4-fluorophenyl)-1-methylurea;
1-(1-(7,8-difluoro-1-oxo-1,2-dihydroisoquinolin-4-yl)ethyl)-3-(3-(difluoromethyl)-4-fluorophenyl)-1-methylurea;
or a salt, solvate, isotopically labelled derivative, stereoisomer, or tautomer thereof, or any mixtures thereof.

11. The compound of claim 1, which is selected from the group consisting of:
(R)-3-(3-chloro-4-fluorophenyl)-1-methyl-1-(1-(1-oxo-1,2-dihydroisoquinolin-4-yl)ethyl)urea;
(S)-3-(3-chloro-4-fluorophenyl)-1-methyl-1-(1-(1-oxo-1,2-dihydroisoquinolin-4-yl)ethyl)urea;
(R)-3-(4-fluoro-3-methylphenyl)-1-methyl-1-(1-(1-oxo-1,2-dihydroisoquinolin-4-yl)ethyl)urea;
(R)-3-(3-chloro-4-fluorophenyl)-1-(1-(1-oxo-1,2-dihydroisoquinolin-4-yl)ethyl)-1-propylurea;
(R)-3-(4-fluoro-3-methylphenyl)-1-(1-(1-oxo-1-oxo-1,2-dihydroisoquinolin-4-yl)ethyl)-1-propylurea;
(R)-3-(4-fluoro-3-(trifluoromethyl)phenyl)-1-methyl-1-(1-(1-oxo-1,2-dihydroisoquinolin-4-yl)ethyl)urea;
(R)-3-(4-fluorophenyl)-1-methyl-1-(1-(1-oxo-1,2-dihydroisoquinolin-4-yl)ethyl)urea;
(R)-1-methyl-1-(1-(1-oxo-1,2-dihydroisoquinolin-4-yl)ethyl)-3-(3,4,5-trifluorophenyl)urea;
(R)-3-(3,4-difluorophenyl)-1-methyl-1-(1-(1-oxo-1,2-dihydroisoquinolin-4-yl)ethyl)urea;
(R)-3-(3 -fluorophenyl)-1 -methyl-1 -(1 -(1 -oxo-1,2-dihydroisoquinolin-4-yl)ethyl)urea;
(R)-3-(2-Chloropyridin-4-yl)-1-methyl-1-(1-(1-oxo-1,2-dihydroisoquinolin-4-yl)ethyl)urea;
(R)-1-Methyl-1-(1-(1-oxo-1,2-dihydroisoquinolin-4-yl)ethyl)-3-(2-(trifluoromethyl)pyridin-4-yl)urea;
(R)-3-(3-chloro-4-fluorophenyl)-1-(2,2-difluoroethyl)-1-(1-(1-oxo-1,2-dihydroisoquinolin-4-yl)ethyl)urea;
(R)-1-(2,2-difluoroethyl)-3-(4-fluoro-3-methylphenyl)-1-(1-(1-oxo-1,2-dihydroisoquinolin-4-yl)ethyl)urea;
(R)-3-(3-cyano-4-fluorophenyl)-1-methyl-1-(1-(1-oxo-1,2-dihydroisoquinolin-4-yl)ethyl)urea;
(R)-3-(3-chloro-4-fluorophenyl)-1-(ethyl-d5)-1-(1-(1-oxo-1,2-dihydroisoquinolin-4-yl)ethyl)urea;
(R)-3-(3-chloro-4-fluorophenyl)-1-(1-(1-methoxyisoquinolin-4-yl)ethyl)-1-(2,2,2-trifluoroethyl)urea;
(R)-3-(4-chlorophenyl)-1-methyl-1-(1-(1-oxo-1-oxo-1,2-dihydroisoquinolin-4-yl)ethyl)urea;
(R)-3-(4-bromophenyl)-1-methyl-1-(1-(1-oxo-1,2-dihydroisoquinolin-4-yl)ethyl)urea;
(R)-1-(3-chloro-4-fluorophenyl)-3-(1-(1-oxo-1,2-dihydroisoquinolin-4-yl)ethyl)urea;
(S)-1-(3-chloro-4-fluorophenyl)-3-(1-(1-oxo-1,2-dihydroisoquinolin-4-yl)ethyl)urea;
(R)-3-(3-chloro-4-fluorophenyl)-1-ethyl-1-(1-(1-methoxyisoquinolin-4-yl)ethyl)urea;
(S)-3-(3-chloro-4-fluorophenyl)-1-ethyl-1-(1-(1-methoxyisoquinolin-4-yl)ethyl)urea;
(R)-3-(3-chloro-4-fluorophenyl)-1-ethyl-1-(1-(1-oxo-1,2-dihydroisoquinolin-4-yl)ethyl)urea;
(S)-3-(3-chloro-4-fluorophenyl)-1-ethyl-1-(1-(1-oxo-1,2-dihydroisoquinolin-4-yl)ethyl)urea;
(R)-3-(3-chloro-4-fluorophenyl)-1-ethyl-1-(1-(1-methoxyisoquinolin-4-yl)propyl)urea;
(S)-3-(3-chloro-4-fluorophenyl)-1-ethyl-1-(1-(1-methoxylsoquinolin-4-yl)propyl)urea;
(R)-3-(3-chloro-4-fluorophenyl)-1-ethyl-1-(1-(1-oxo-1,2-dihydroisoquinolin-4-yl)propyl)urea;
(S)-3-(3-chloro-4-fluorophenyl)-1-ethyl-1-(1-(1-oxo-1,2-dihydroisoquinolin-4-yl)propyl)urea;
(R)-3-(3-chloro-4-fluorophenyl)-1-ethyl-1-(1-(1-methoxyisoquinolin-4-yl)-2-methylpropyl)urea;
(S)-3-(3-chloro-4-fluorophenyl)-1-ethyl-1-(1-(1-methoxyisoquinolin-4-yl)-2-methylpropyl)urea;
(R)-3-(3 -chloro-4-fluorophenyl)-1-(cyclopropyl(1-methoxyisoquinolin-4-yl)methyl)-1-ethylurea;
(S)-3-(3-chloro-4-fluorophenyl)-1-(cyclopropyl(1-methoxyisoquinolin-4-yl)methyl)-1-ethylurea;
(R)-3-(3 -chloro-4-fluorophenyl)-1-(cyclopropyl(1-oxo-1,2-dihydroisoquinolin-4-yl)methyl)-1-ethylurea;
(S)-3-(3-chloro-4-fluorophenyl)-1-(cyclopropyl(1-oxo-1,2-dihydroisoquinolin-4-yl)methyl)-1-ethylurea;
(R)-3-(3 -chloro-4-fluorophenyl)-1-ethyl-1-(2-methyl-1-(1-oxo-1,2-dihydroisoquinolin-4-yl)propyl)urea;
(S)-3-(3 -chloro-4-fluorophenyl)-1-ethyl-1-(2-methyl-1-(1-oxo-1,2-dihydroisoquinolin-4-yl)propyl)urea;
(R)-3-(3-chloro-4-fluorophenyl)-1-cyclopropyl-1-(1-(1-methoxyisoquinolin-4-yl)ethyl)urea;
(S)-3-(3-chloro-4-fluorophenyl)-1-cyclopropyl-1-(1-(1-methoxyisoquinolin-4-yl)ethyl)urea;
(R)-3-(3-chloro-4-fluorophenyl)-1-ethyl-1-((1-methoxyisoquinolin-4-yl)(phenyl)methyl)urea;
(S)-3-(3-chloro-4-fluorophenyl)-1-ethyl-1-((1-methoxyisoquinolin-4-yl)(phenyl)methyl)urea;
(R)-1-butyl-3-(3-chloro-4-fluorophenyl)-1-(1-(1-methoxyisoquinolin-4-yl)ethyl)urea;
(S)-1-butyl-3-(3-chloro-4-fluorophenyl)-1-(1-(1-methoxyisoquinolin-4-yl)ethyl)urea;
(R)-3-(3 -chloro-4-fluorophenyl)-1-(cyclopropylmethyl)-1-(1-(1-methoxyisoquinolin-4-yl)ethyl)urea;
(S)-3-(3-chloro-4-fluorophenyl)-1-(cyclopropylmethyl)-1-(1-(1-methoxylsoquinolin-4-yl)ethyl)urea;
(R)-3-(3-chloro-4-fluorophenyl)-1-(2-methoxyethyl)-1-(1-(1-oxo-1,2-dihydroisoquinolin-4-yl)ethyl)urea;
(S)-3-(3-chloro-4-fluorophenyl)-1-(2-methoxyethyl)-1-(1-(1-oxo-1,2-dihydroisoquinolin-4-yl)ethyl)urea;
(R)-3-(3-chloro-4-fluorophenyl)-1-cyclopropyl-1-(1-(1-oxo-1,2-dihydroisoquinolin-4-yl)ethyl)urea;
(S)-3-(3-chloro-4-fluorophenyl)-1-cyclopropyl-1-(1-(1-oxo-1,2-dihydroisoquinolin-4-yl)ethyl)urea;
(R)-3-(3 -chloro-4-fluorophenyl)-1-ethyl-1-((1-oxo-1,2-dihydroisoquinolin-4-yl)(phenyl)methyl)urea;
(S)-3-(3 -chloro-4-fluorophenyl)-1-ethyl-1-((1 -oxo-1,2-dihydroisoquinolin-4-yl)(phenyl)methyl)urea;
(R)-3-(3-chloro-4-fluorophenyl)-1-(2-methoxyethyl)-1-(1-(1-methoxyisoquinolin-4-yl)ethyl)urea;
(S)-3-(3-chloro-4-fluorophenyl)-1-(2-methoxyethyl)-1-(1-(1-methoxyisoquinolin-4-yl)ethyl)urea;
(R)-3-(3 -chloro-4-fluorophenyl)-1-ethyl-1-(2,2,2-trifluoro-1-(1-oxo-1,2-dihydroisoquinolin-4-yl)ethyl)urea;
(S)-3-(3-chloro-4-fluorophenyl)-1-ethyl-1-(2,2,2-trifluoro-1-(1-oxo-1,2-dihydroisoquinolin-4-yl)ethyl)urea;
(R)-3-(3-chloro-4-fluorophenyl)-1-ethyl-1-(2,2,2-trifluoro-1-(1-methoxyisoquinolin-4-yl)ethyl)urea;
(S)-3-(3-chloro-4-fluorophenyl)-1-ethyl-1-(2,2,2-trifluoro-1-(1-methoxyisoquinolin-4-yl)ethyl)urea;
(R)-3-(3-chloro-4-fluorophenyl)-1-(3-methoxypropyl)-1-(1-(1-oxo-1,2-dihydroisoquinolin-4-yl)ethyl)urea;
(S)-3-(3-chloro-4-fluorophenyl)-1-(3-methoxypropyl)-1-(1-(1-oxo-1-oxo-1,2-dihydroisoquinolin-4-yl)ethyl)urea;
(R)-3-(3-chloro-4-fluorophenyl)-1-(1-(1-methoxyisoquinolin-4-yl)ethyl)-1-(3-methoxypropyl)urea;
(S)-3-(3-chloro-4-fluorophenyl)-1-(1-(1-methoxyisoquinolin-4-yl)ethyl)-1-(3-methoxypropyl)urea;
(R)-1-butyl-3-(3-chloro-4-fluorophenyl)-1-(1-(1-oxo-1,2-dihydroisoquinolin-4-yl)ethyl)urea;
(S)-1-butyl-3-(3-chloro-4-fluorophenyl)-1-(1-(1-oxo-1,2-dihydroisoquinolin-4-yl)ethyl)urea;
(R)-3-(3-chloro-4-fluorophenyl)-1-(cyclopropylmethyl)-1-(1-(1-oxo-1-oxo-1,2-dihydroisoquinolin-4-yl)ethyl)urea;
(S)-3-(3-chloro-4-fluorophenyl)-1-(cyclopropylmethyl)-1-(1-(1-oxo-1,2-dihydroisoquinolin-4-yl)ethyl)urea;
(R)-3-(3-(3-chloro-4-fluorophenyl)-1-(1-(1-oxo-1,2-dihydroisoquinolin-4-yl)ethyl)ureido)-N,N-dimethylpropanamide;
(S)-3-(3-(3-chloro-4-fluorophenyl)-1-(1-(1-oxo-1,2-dihydroisoquinolin-4-yl)ethyl)ureido)-N,N-dimethylpropanamide;
(R)-3-(3-chloro-4-fluorophenyl)-1-(2-(methylsulfonyl)ethyl)-1-(1-(1-oxo-1,2-dihydroisoquinolin-4-yl)ethyl)urea;
(S)-3-(3-chloro-4-fluorophenyl)-1-(2-(methylsulfonyl)ethyl)-1-(1-(1-oxo-1,2-dihydroisoquinolin-4-yl)ethyl)urea;
(R)-3-(3-chloro-4-fluorophenyl)-1-(2-ethoxyethyl)-1-(1-(1-oxo-1,2-dihydroisoquinolin-4-yl)ethyl)urea;
(S)-3-(3-chloro-4-fluorophenyl)-1-(2-ethoxyethyl)-1-(1-(1-oxo-1,2-dihydroisoquinolin-4-yl)ethyl)urea;
(R)-3-(3-chloro-4-fluorophenyl)-1-(2-cyanoethyl)-1-(1-(1-oxo-1,2-dihydroisoquinolin-4-yl)ethyl)urea;
(S)-3 -(3 -chloro-4-fluorophenyl)-1 -(2-cyanoethyl)-1 -(1 -(1 -oxo-1,2-dihydroisoquinolin-4-yl)ethyl)urea;
(R)-3-(3-chloro-4-fluorophenyl)-1-(3-cyanopropyl)-1-(1-(1-oxo-1,2-dihydroisoquinolin-4-yl)ethyl)urea;
(S)-3-(3 -chloro-4-fluorophenyl)-1-(3-cyanopropyl)-1-(1-(1-oxo-1,2-dihydroisoquinolin-4-yl)ethyl)urea;
(R)-3-(3-(3-chloro-4-fluorophenyl)-1-(1-(1-oxo-1,2-dihydroisoquinolin-4-yl)ethyl)ureido)-N-methylpropanamide;
(S)-3-(3-(3-chloro-4-fluorophenyl)-1-(1-(1-oxo-1,2-dihydroisoquinolin-4-yl)ethyl)ureido)-N-methylpropanamide;
(R)-2-(3 -(3-chloro-4-ffuorophenyl)-1-(1-(1-oxo-1,2-dihydroisoquinolin-4-yl)ethyl)ureido)ethane-1-sulfonamide;
(S)-2-(3-(3-chloro-4-fluorophenyl)-1-(1-(1-oxo-1,2-dihydroisoquinolin-4-yl)ethyl)ureido)ethane-1-sulfonamide;
(R)-3-(3-chloro-4-fluorophenyl)-1-(2-(2-methoxyethoxy)ethyl)-1-(1-(1-methoxyisoquinolin-4-yl)ethyl)urea;
(S)-3-(3-chloro-4-fluorophenyl)-1-(2-(2-methoxyethoxy)ethyl)-1-(1-(1-methoxyisoquinolin-4-yl)ethyl)urea;
(R)-3-(3 -chloro-4-fluorophenyl)-1-(2-(2-methoxyethoxy)ethyl)-1-(1-(1-oxo-1,2-dihydroisoquinolin-4-yl)ethyl)urea;
(S)-3-(3-chloro-4-fluorophenyl)-1-(2-(2-methoxyethoxy)ethyl)-1-(1-(1-oxo-1,2-dihydrolsoquinolin-4-yl)ethyl)urea;
(R)-3-(3-chloro-4-fluorophenyl)-1-(2-ethoxyethyl)-1-(1-(1-methoxyisoquinolin-4-yl)ethyl)urea;
(S)-3-(3 -chloro-4-fluorophenyl)-1-(2-ethoxyethyl)-1-(1-(1-methoxyisoquinolin-4-yl)ethyl)urea;
(R)-3-(3-chloro-4-fluorophenyl)-1-(3-hydroxypropyl)-1-(1-(1-oxo-1-oxo-1,2-dihydroisoquinolin-4-yl)ethyl)urea;
(S)-3-(3-chloro-4-fluorophenyl)-1-(3-hydroxypropyl)-1-(1-(1-oxo-1-oxo-1,2-dihydroisoquinolin-4-yl)ethyl)urea;
(R)-3-(3-chloro-4-fluorophenyl)-1-methyl-1-(1-(1-((1-methyl-1H-1,2,4-triazol-3-yl)methoxy)isoquinolin-4-yl)ethyl)urea;
(S)-3-(3-chloro-4-fluorophenyl)-1-methyl-1-(1-(1-((1-methyl-1H-1,2,4-triazol-3-yl)methoxy)isoquinolin-4-yl)ethyl)urea;
(R)-N-(2-(3-(3-chloro-4-fluorophenyl)-1-(1-(1-oxo-1,2-dihydroisoquinolin-4-yl)ethyl)ureido)ethyl)acetamide;
(S)-N-(2-(3-(3-chloro-4-fluorophenyl)-1-(1-(1-oxo-1,2-dihydroisoquinolin-4-yl)ethyl)ureido)ethyl)acetamide;
(R)-3-(3-chloro-4-fluorophenyl)-1-(1-(1-oxo-1,2-dihydroisoquinolin-4-yl)ethyl)-1-((tetrahydro-2H-pyran-4-yl)methyl)urea;
(S)-3-(3-chloro-4-fluorophenyl)-1-(1-(1-oxo-1,2-dihydroisoquinolin-4-yl)ethyl)-1-((tetrahydro-2H-pyran-4-yl)methyl)urea;
(R)-3-(3-chloro-4-fluorophenyl)-1-isobutyl-1-(1-(1-oxo-1,2-dihydroisoquinolin-4-yl)ethyl)urea;
(S)-3-(3-chloro-4-fluorophenyl)-1-isobutyl-1-(1-(1-oxo-1,2-dihydroisoquinolin-4-yl)ethyl)urea;
(R)-3-(3-chloro-4-fluorophenyl)-1-methyl-1-(1-(5-(methylamino)pyrido[3,4-b]pyrazin-8-yl)ethyl)urea;
(S)-3-(3 -chloro-4-fluorophenyl)-1-methyl-1-(1-(5-(methylamino)pyrido[3,4-b]pyrazin-8-yl)ethyl)urea;
(R)-3-(3 -chloro-4-fluorophenyl)-1-(1-(5-methoxypyrido[3,4-b]pyrazin-8-yl)ethyl)-1-methylurea;
(S)-3-(3-chloro-4-fluorophenyl)-1-(1-(5-methoxypyrido[3,4-b]pyrazin-8-yl)ethyl)-1-methylurea;
(R)-3-(3-chloro-4-fluorophenyl)-1-(3-hydroxypropyl)-1-(1-(1-methoxyisoquinolin-4-yl)ethyl)urea;
(S)-3-(3-chloro-4-fluorophenyl)-1-(3-hydroxypropyl)-1-(1-(1-methoxyisoquinolin-4-yl)ethyl)urea;
(R)-1-(1-(1-((1H-1 ,2,4-triazol-3-yl)methoxy)isoquinolin-4-yl)ethyl)-3 -(3-chloro-4-fluorophenyl)-1-methylurea;
(S)-1-(1-(1-((1H-1,2,4-triazol-3-yl)methoxy)isoquinolin-4-yl)ethyl)-3-(3-chloro-4-fluorophenyl)-1-methylurea;
(R)-(3-(3-chloro-4-fluorophenyl)-1-(1-(1-methoxyisoquinolin-4-yl)ethyl)-1-(2,2,2-trifluoroethyl)urea;
(S)-(3-(3-chloro-4-fluorophenyl)-1-(1-(1-methoxyisoquinolin-4-y1)ethyl)-1-(2,2,2-trifluoroethyl)urea;
(R)-3-(3-chloro-4-fluorophenyl)-1-ethyl-1-(1-(1-oxo-1,2-dihydro-2,7-naphthyridin-4-yl)ethyl)urea;
(S)-3-(3-chloro-4-fluorophenyl)-1-ethyl-1-(1-(1-oxo-1,2-dihydro-2,7-naphthyridin-4-yl)ethyl)urea;
(R)-3-(3-chloro-4-fluorophenyl)-1-(1-(1-oxo-1,2-dihydroisoquinolin-4-yl)ethyl)-1-(2-((2,2,2-trifluoroethyl)amino)ethyl)urea;
(S)-3-(3-chloro-4-fluorophenyl)-1-(1-(1-oxo-1,2-dihydroisoquinolin-4-yl)ethyl)-1-(2-((2,2,2-trifluoroethyl)amino)ethyl)urea;
(R)-1-(1-(8-chloro-1-oxo-1,2-dihydroisoquinolin-4-yl)ethyl)-3-(3-chloro-4-fluorophenyl)-1-methylurea;
(S)-1-(1-(8-chloro-1-oxo-1,2-dihydroisoquinolin-4-yl)ethyl)-3-(3-chloro-4-ffuorophenyl)-1-methylurea;
(R)-3-(3-chloro-4-fluorophenyl)-1-(1-(6-fluoro-1-oxo-1,2-dihydroisoquinolin-4-yl)ethyl)-1-methylurea;
(S)-3-(3 -chloro-4-fluorophenyl)-1-(1-(6-fluoro-1-oxo-1,2-dihydroisoquinolin-4-yl)ethyl)-1-methylurea;
(R)-3-(3 -chloro-4-fluorophenyl)-1-(1-(6-chloro-1-oxo-1,2-dihydroisoquinolin-4-yl)ethyl)-1-methylurea;
(S)-3-(3 -chloro-4-fluorophenyl)-1-(1-(6-chloro-1-oxo-1,2-dihydroisoquinolin-4-yl)ethyl)-1-methylurea;
(R)-3-(3-chloro-4-fluorophenyl)-1-(1-(7-methoxy-1-oxo-1,2-dihydroisoquinolin-4-yl)ethyl)-1-methylurea;
(S)-3-(3 -chloro-4-fluorophenyl)-1 -(1 -(7-methoxy-1 -oxo-1,2-dihydroisoquinolin-4-yl)ethyl)-1 - methylurea;
(R)-3-(3-chloro-4-fluorophenyl)-1-(1-(6-methoxy-1-oxo-1,2-dihydroisoquinolin-4-yl)ethyl)-1-methylurea;
(S)-3-(3-chloro-4-fluorophenyl)-1-(1-(6-methoxy-1-oxo-1,2-dihydroisoquinolin-4-yl)ethyl)-1-methylurea;
(R)-3-(3-chloro-4-fluorophenyl)-1-methyl-1-(1-(2-methyl-1-oxo-1-oxo-1,2-dihydroisoquinolin-4-yl)ethyl)urea;
(S)-3-(3-chloro-4-fluorophenyl)-1-methyl-1-(1-(2-methyl-1-oxo-1,2-dihydroisoquinolin-4-yl)ethyl)urea;
(R)-3-(3-chloro-4-fluorophenyl)-1-methyl-1-(1-(2-ethyl-1-oxo-1,2-dihydroisoquinolin-4-yl)ethyl)urea;
(S)-3-(3-chloro-4-fluorophenyl)-1-methyl-1-(1-(2-ethyl-1-oxo-1,2-dihydroisoquinolin-4-yl)ethyl)urea;
(R)-3-(3-chloro-4-fluorophenyl)-1-methyl-1-(1-(2-propyl-1-oxo-1,2-dihydroisoquinolin-4-yl)ethyl)urea;
(S)-3-(3-chloro-4-fluorophenyl)-1-methyl-1-(1-(2-propyl-1-oxo-1,2-dihydroisoquinolin-4-yl)ethyl)urea;
(R)-3-(3-chloro-4-fluorophenyl)-1-methyl-1-(1-(2-isopropyl-1-oxo-1,2-dihydroisoquinolin-4-yl)ethyl)urea;
(S)-3-(3-chloro-4-fluorophenyl)-1-methyl-1-(1-(2-isopropyl-1-oxo-1,2-dihydroisoquinolin-4-yl)ethyl)urea;
(R)-3-(3-chloro-4-fluorophenyl)-1-methyl-1-(1-(2-cyclopropyl-1-oxo-1,2-dihydroisoquinolin-4-yl)ethyl)urea;
(S)-3-(3-chloro-4-fluoropheny1)-1-methyl-1-(1-(2-cyclopropyl-1-oxo-1,2-dihydroisoquinolin-4-yl)ethyl)urea;
(R)-3-(3-chloro-4-fluorophenyl)-1 -(cyclopentylmethyl)-1-(1 -(1 -oxo-1-oxo-1,2-dihydroisoquinolin-4-yl)ethyl)urea;
(S)-3-(3-chloro-4-fluorophenyl)-1-(cyclopentylmethyl)-1-(1-(1-oxo-1,2-dihydroisoquinolin-4-yl)ethyl)urea;
(R)-3-(3-chloro-4-fluorophenyl)-1-(cyclohexylmethyl)-1-(1-(1-oxo-1,2-dihydroisoquinolin-4-yl)ethyl)urea;
(S)-3-(3-chloro-4-fluorophenyl)-1-(cyclohexylmethyl)-1-(1-(1-oxo-1,2-dihydroisoquinolin-4-yl)ethyl)urea;
(R)-3-(3-chloro-4-fluorophenyl)-1-(2-hydroxyethyl)-1-(1-(1-oxo-1,2-dihydroisoquinolin-4-yl)ethyl)urea;
(S)-3-(3-chloro-4-fluorophenyl)-1-(2-hydroxyethyl)-1-(1-(1-oxo-1-oxo-1,2-dihydroisoquinolin-4-yl)ethyl)urea;
(R)-3-(3-chloro-4-fluorophenyl)-1-(2-((2,2-difluoroethyl)amino)ethyl)-1-(1-(1-oxo-1,2-dihydroisoquinolin-4-yl)ethyl)urea;
(S)-3-(3-chloro-4-fluorophenyl)-1-(2-((2,2-difluoroethyl)amino)ethyl)-1-(1-(1-oxo-1,2-dihydroisoquinolin-4-yl)ethyl)urea;
(R)-1-((1-acetylpiperidin-4-yl)methyl)-3-(3-chloro-4-fluorophenyl)-1-(1-(1-oxo-1,2-dihydroisoquinolin-4-yl)ethyl)urea;
(S)-1-((1-acetylpiperidin-4-yl)methyl)-3-(3-chloro-4-fluorophenyl)-1-(1-(1-oxo-1,2-dihydroisoquinolin-4-yl)ethyl)urea;
(R)-3-(3-chloro-4-fluorophenyl)-1-((1-(methylsulfonyl)piperidin-4-yl)methyl)-1-(1-(1-oxo-1,2-dihydroisoquinolin-4-yl)ethyl)urea;
(S)-3-(3-chloro-4-fluorophenyl)-1-((1-(methylsulfonyl)piperidin-4-yl)methyl)-1-(1-(1-oxo-1,2-dihydroisoquinolin-4-yl)ethyl)urea;
(R)-3-(3-chloro-4-fluorophenyl)-1-(3-hydroxy-2,2-dimethylpropyl)-1-(1-(1-oxo-1,2-dihydroisoquinolin-4-yl)ethyl)urea;
(S)-3-(3-chloro-4-fluorophenyl)-1-(3-hydroxy-2,2-dimethylpropyl)-1-(1-(1-oxo-1,2-dihydroisoquinolin-4-yl)ethyl)urea;
3-(3-chloro-4-fluorophenyl)-1-((R)-3-hydroxybutyl)-1-(1(R)-(1-oxo-1,2-dihydroisoquinolin-4-yl)ethyl)urea;
3-(3-chloro-4-fluorophenyl)-1-((R)-3-hydroxybutyl)-1-(1(S)-(1-oxo-1,2-dihydroisoquinolin-4-yl)ethyl)urea;
3-(3-chloro-4-fluorophenyl)-1-((S)-3-hydroxybutyl)-1-(1(R)-(1-oxo-1,2-dihydroisoquinolin-4-yl)ethyl)urea;
3-(3-chloro-4-fluorophenyl)-1-((S)-3-hydroxybutyl)-1-(1(S)-(1-oxo-1,2-dihydroisoquinolin-4-yl)ethyl)urea;
3-(3-chloro-4-fluorophenyl)-1((R)-2-hydroxypropyl)-1-(1(R)-(1-oxo-1,2-dihydroisoquinolin-4-yl)ethyl)urea;
3-(3-chloro-4-fluorophenyl)-1-((R)-2-hydroxypropyl)-1-((S)1-(1 -oxo-1,2-dihydroisoquinolin-4-yl)ethyl)urea;
3-(3-chloro-4-fluorophenyl)-1-((S)-2-hydroxypropyl)-1-(1(R)-(1-oxo-1,2-dihydroisoquinolin-4-yl)ethyl)urea;
3-(3-chloro-4-fluorophenyl)-1-((S)-2-hydroxypropyl)-1-(1(S)-(1-oxo-1,2-dihydroisoquinolin-4-yl)ethyl)urea;
(R)-3-(3-chloro-4-fluorophenyl)-1-methyl-1-(1 -(3-methyl-1-oxo-1-oxo-1,2-dihydroisoquinolin-4-yl)ethyl)urea;
(S)-3-(3-chloro-4-fluorophenyl)-1-methyl-1-(1-(3-methyl-1-oxo-1,2-dihydroisoquinolin-4-yl)ethyl)urea;
(R)-4-(3-(3-chloro-4-fluorophenyl)-1-(1-(1-oxo-1,2-dihydroisoquinolin-4-yl)ethyl)ureido)butanoic acid;
(S)-4-(3-(3-chloro-4-fluorophenyl)-1-(1-(1-oxo-1,2-dihydroisoquinolin-4-yl)ethyl)ureido)butanoic acid;
(R)-3-(3 -chloro-4-fluorophenyl)-1-methyl-1-(1-(1-(methylamino)isoquinolin-4-yl)ethyl)urea;
(S)-3-(3-chloro-4-fluorophenyl)-1-methyl-1-(1-(1-(methylamino)isoquinolin-4-yl)ethyl)urea;
(R)-3-(3 -chloro-4-fluorophenyl)-1-methyl-1-(1-(1-(dimethylamino)isoquinolin-4-yl)ethyl)urea;
(S)-3-(3-chloro-4-fluorophenyl)-1-methyl-1-(1-(1-(dimethylamino)isoquinolin-4-yl)ethyl)urea;
(R)-3-(4-fluoro-3-methylphenyl)-1-methyl-1-(1-(3-methyl-1-oxo-1,2-dihydroisoquinolin-4-yl)ethyl)urea;
(S)-3-(4-fluoro-3-methylphenyl)-1-methyl-1-(1-(3-methyl-1-oxo-11,2-dihydroisoquinolin-4-yl)ethyl)urea;
(R)-1-(1-(1-aminoisoquinolin-4-yl)ethyl)-3-(3-chloro-4-fluorophenyl)-1-methylurea;
(S)-1-(1-(1-aminoisoquinolin-4-yl)ethyl)-3-(3 -chloro-4-fluorophenyl)-1-methylurea;
(R)-3-(3-chloro-4-fluorophenyl)-1-(1-(1-(ethylamino)isoquinolin-4-yl)ethyl)-1-methylurea;
(S)-3-(3-chloro-4-fluorophenyl)-1-(1-(1-(ethylamino)isoquinolin-4-yl)ethyl)-1-methylurea;
(R)-3-(3-chloro-4-fluorophenyl)-1-(1-(1-((2-hydroxyethyl)amino)isoquinolin-4-yl)ethyl)-1-methylurea;
(S)-3-(3-chloro-4-fluorophenyl)-1-(1-(1-((2-hydroxyethyl)amino)isoquinolin-4-yl)ethyl)-1-methylurea;
(R)-3-(3-chloro-4-fluorophenyl)-1-methyl-1-(1-(1-(((1-methyl-1H-1,2,4-triazol-3 - yl)methyl)amino)isoquinolin-4-yl)ethyl)urea;
(S)-3-(3-chloro-4-fluorophenyl)-1-methyl-1-(1-(1-(((1-methyl-1H-1,2,4-triazol-3-yl)methyl)amino)isoquinolin-4-yl)ethyl)urea;
(R)-1-(1-(1-(((1H-1,2,4-triazol-3-yl)methyl)amino)isoquinolin-4-yl)ethyl)-3-(3-chloro-4-fluorophenyl)-1-methylurea;
(S)-1-(1-(1-(((1H-1,2,4-triazol-3-yl)methyl)amino)isoquinolin-4-yl)ethyl)-3-(3-chloro-4-fluorophenyl)-1-methylurea;
(R)-3-(3-chloro-4-fluorophenyl)-1-(3-hydroxypropyl)-1-(1-(1-oxo-1,2-dihydro-2,7-naphthyridin-4-yl)ethyl)urea;
(S)-3-(3-chloro-4-fluorophenyl)-1-(3-hydroxypropyl)-1-(1-(1-oxo-1,2-dihydro-2,7-naphthyridin-4-yl)ethyl)urea;
(R)-3-(3-chloro-4-fluorophenyl)-1-isobutyl-1-(1-(1-oxo-1,2-dihydro-2,7-naphthyridin-4-yl)ethyl)urea;
(S)-3-(3-chloro-4-fluorophenyl)-1-isobutyl-1-(1-(1-oxo-1,2-dlhydro-2,7-naphthyridin-4-yl)ethyl)urea;
(R)-3-(3-chloro-4-fluorophenyl)-1-(3-hydroxypropyi)-1-(1-(1-((1-methyl-1H-1,2,4-triazol-3-yl)methoxy)isoquinolin-4-yl)ethyl)urea;
(S)-3-(3-chloro-4-fluorophenyl)-1-(3-hydroxypropyl)-1-(1-(1-((1-methyl-1H-1,2,4-triazol-3-yl)methoxy)isoquinolin-4-yl)ethyl)urea;
(R)-1-(1-(1-((1H-1,2,4-triazol-3-yl)methoxy)isoquinolin-4-yl)ethyl)-3-(3-chloro-4-fluorophenyl)-1-(3-hydroxypropyl)urea;
(S)-1-(1-(1-((1H-1,2,4-triazol-3-yl)methoxy)isoquinolin-4-yl)ethyl)-3-(3-chloro-4-fluorophenyl)-1-(3-hydroxypropyl)urea;
(R)-1-(1-(1-(((2H-1,2,3-triazol-4-yl)methyl)amino)isoquinolin-4-yl)ethyl)-3-(3-chloro-4-fluorophenyl)-1-methylurea;
(S)-1-(1-(1-(((2H-1,2,3-triazol-4-yl)methyl)amino)isoquinolin-4-yl)ethyl)-3-(3-chloro-4-fluorophenyl)-1-methylurea;
(R)-3-(3 -chloro-4-fluorophenyl)-1-(1-(7-fluoro-1-oxo-1,2-dihydroisoquinolin-4-yl)ethyl)-1-methylurea;
(S)-3-(3 -chloro-4-fluorophenyl)-1-(1-(7-fluoro-1-oxo-1,2-dihydroisoquinolin-4-yl)ethyl)-1-methylurea;
(R)-1-((1H-1,2,3-triazol-4-yl)methyl)-3-(3-chloro-4-fluorophenyl)-1-(1-(1-oxo-1,2-dihydroisoquinolin-4-yl)ethyl)urea;
(S)-1-((1H-1,2,3-triazol-4-yl)methyl)-3-(3-chloro-4-fluorophenyl)-1-(1-(1-oxo-1,2-dihydroisoquinolin-4-yl)ethyl)urea;
(R)-1-(1-(2-((1H-1,2,4-triazol-3-yl)methyl)-1-oxo-1,2-dihydroisoquinolin-4-yl)ethyl)-3-(3-chloro-4-fluorophenyl)-1-methylurea;
(S)-1-(1-(2-((1H-1,2,4-triazol-3-yl)methyl)-1-oxo-1,2-dihydroisoquinolin-4-yl)ethyl)-3-(3-chloro-4-fluorophenyl)-1-methylurea;
(R)-3-(3 -chloro-4-fluorophenyl)-1-((2,2-dimethyl-1,3-dioxan-5-yl)methyl)-1-(1-(1-oxo-1,2-dihydroisoquinolin-4-yl)ethyl)urea;
(S)-3-(3-chloro-4-fluorophenyl)-1-((2,2-dimethyl-1,3-dioxan-5-yl)methyl)-1-(1-(1-oxo-1,2-dihydroisoquinolin-4-yl)ethyl)urea;
(R)-1-(1-(2-((1H-1,2,3-triazol-4-yl)methyl)-1-oxo-1,2-dihydroisoquinolin-4-yl)ethyl)-3-(3-chloro-4-fluorophenyl)-1-methylurea;
(S)-1-(1-(2-((1H-1,2,3-triazol-4-yl)methyl)-1-oxo-1,2-dihydroisoquinolin-4-yl)ethyl)-3 -(3-chloro-4-fluorophenyl)-1-methylurea;
(R)-3-(3 -chloro-4-fluorophenyl)-1-(1-(6,7-difluoro-1-oxo-1,2-dihydroisoquinolin-4-yl)ethyl)-1-methylurea;
(S)-3-(3 -chloro-4-fluorophenyl)-1-(1-(6,7-difluoro-1-oxo-1,2-dihydroisoquinolin-4-yl)ethyl)-1-methylurea;
(R)-3-(3 -chloro-4-fluorophenyl)-1-(1-(6,7-difluoro-1-oxo-1,2-dihydroisoquinolin-4-yl)ethyl)-1-isobutylurea;
(S)-3-(3 -chloro-4-fluorophenyl)-1-(1-(6,7-difluoro-1-oxo-1,2-dihydroisoquinolin-4-yl)ethyl)-1-isobutylurea;
(R)-3-(3 -chloro-4-fluorophenyl)-1-((2,2-dimethyl-1,3-dioxan-5-yl)methyl)-1-(1-(6-ffuoro-1-oxo-1,2-dihydroisoquinolin-4-yl)ethyl)urea;
(S)-3-(3-chloro-4-fluorophenyl)-1-((2,2-dimethyl-1,3-dioxan-5-yl)methyl)-1-(1-(6-fluoro-1-oxo-1,2-dihydroisoquinolin-4-yl)ethyl)urea;
(R)-1-(1-(6,7-diffuoro-1-oxo-1,2-dihydroisoquinolin-4-yl)ethyl)-3-(4-fluorophenyl)-1-methylurea;
(S)-1-(1-(6,7-difluoro-1-oxo-1,2-dihydroisoquinolin-4-yl)ethyl)-3-(4-fluorophenyl)-1 -methylurea;
(R)-1-(1-(6,7-diffuoro-1-oxo-1,2-dihydroisoquinolin-4-yl)ethyl)-3-(4-fluorophenyl)-1-isobutylurea;
(S)-1-(1-(6,7-difluoro-1-oxo-1,2-dihydroisoquinolin-4-yl)ethyl)-3-(4-fluorophenyl)-1-isobutylurea;
(R)-3-(3-chloro-4-fluorophenyl)-1-(1-(6,7-difluoro-1-oxo-1,2-dihydroisoquinolin-4-yl)ethyl)-1 -(3-hydroxypropyl)urea;
(S)-3-(3-chloro-4-fluorophenyl)-1-(1-(6,7-difluoro-1-oxo-1,2-dihydroisoquinolin-4-yl)ethyl)-1-(3-hydroxypropyl)urea;
(R)-1-(1-(6,7-difluoro-1-oxo-1,2-dihydroisoquinolin-4-yl)ethyl)-3-(4-fluorophenyl)-1-(3- hydroxypropyl)urea;
(S)-1-(1-(6,7-difluoro-1-oxo-1,2-dihydroisoquinolin-4-yl)ethyl)-3-(4-fluorophenyl)-1-(3-hydroxypropyl)urea;
(R)-3-(3 -chloro-4-fluorophenyl)-1-(1-(6-fluoro-1-oxo-1,2-dihydroisoquinolin-4-yl)ethyl)-1-(3-hydroxy-2-(hydroxymethyl)propyl)urea;
(S)-3-(3-chloro-4-fluorophenyl)-1-(1-(6-fluoro-1-oxo-1,2-dihydroisoquinolin-4-yl)ethyl)-1-(3-hydroxy-2-(hydroxymethyl)propyl)urea;
(R)-3-(3-chloro-4-fluorophenyl)-1-(1-(7-fluoro-2-methyl-1-oxo-1,2-dihydroisoquinolin-4-yl)ethyl)-1-methylurea;
(S)-3-(3-chloro-4-fluorophenyl)-1-(1-(7-fluoro-2-methyl-1-oxo-1,2-dihydroisoquinolin-4-yl)ethyl)-1-methylurea;
(R)-3-(3-chloro-4-fluorophenyl)-1-(1-(1-oxo-1,2-dihydroisoquinolin-4-yl)ethyl)-1-(pyridin-2-ylmethyl)urea;
(S)-3-(3 -chloro-4-fluorophenyl)-1 -(1 -(1 -oxo-1,2-dihydroisoquinolin-4-yl)ethyl)-1 -(pyridin-2-ylmethyl)urea;
(R)-3-(3-chloro-4-fluorophenyl)-1-(1-(1-oxo-1,2-dihydroisoquinolin-4-yl)ethyl)-1-(pyridin-3-ylmethyl)urea;
(S)-3-(3-chloro-4-fluorophenyl)-1-(1-(1-oxo-1,2-dihydroisoquinolin-4-yl)ethyl)-1-(pyridin-3-ylmethyl)urea;
(R)-3-(3-chloro-4-fluorophenyl)-1-(1-(1-oxo-1,2-dihydroisoquinolin-4-yl)ethyl)-1-(pyridin-4-ylmethyl)urea;
(S)-3-(3 -chloro-4-fluorophenyl)-1 -(1 -(1 -oxo-1,2-dihydroisoquinolin-4-yl)ethyl)-1 -(pyridin-4-ylmethyl)urea;
(R)-3-(3-chloro-4-fluorophenyl)-1-(1-(1-oxo-1,2-dihydroisoquinolin-4-yl)ethyl)-1-(thiazol-2-ylmethyl)urea;
(S)-3-(3 -chloro-4-fluorophenyl)-1 -(1 -(1 -oxo-1,2-dihydroisoquinolin-4-yl)ethyl)-1 -(thiazol-2-ylmethyl)urea;
(R)-3-(3-chloro-4-fluorophenyl)-1-(1-(1-oxo-1,2-dihydroisoquinolin-4-yl)ethyl)-1-(thiazol-4-ylmethyl)urea;
(S)-3-(3 -chloro-4-fluorophenyl)-1 -(1 -(1 -oxo-1,2-dihydroisoquinolin-4-yl)ethyl)-1 -(thiazol-4-ylmethyl)urea;
(R)-3-(3-chloro-4-fluorophenyl)-1-methyl-1-(1-(1-(methylsulfonyl)isoquinolin-4-yl)ethyl)urea;
(S)-3-(3-chloro-4-fluorophenyl)-1-methyl-1-(1-(1-(methylsulfonyl)isoquinolin-4-yl)ethyl)urea;
(R)-4-(1-(3-(3-chloro-4-fluorophenyl)-1-methylureido)ethyl)-N-methylisoquinoline-1-carboxamide;
(S)-4-(1-(3-(3-chloro-4-fluorophenyl)-1-methylureido)ethyl)-N-methylisoquinoline-1-carboxamide;
(R)-4-(1-(3-(3-chloro-4-fluorophenyl)-1-methylureido)ethyl)-N,N-dimethylisoquinoline-1-carboxamide;
(S)-4-(1-(3-(3 -chloro-4-fluorophenyl)-1-methylureido)ethyl)-N,N-dimethylisoquinoline-1-carboxamide;
(R)-3-(3-chloro-4-fluorophenyl)-1-(1-(1-oxo-1,2-dihydroisoquinolin-4-yl)ethyl)-1-(pyrimidin-4-ylmethyl)urea;
(S)-3-(3-chloro-4-fluorophenyl)-1-(1-(1-oxo-1,2-dihydroisoquinolin-4-yl)ethyl)-1-(pyrimidin-4-ylmethyl)urea;
(R)-3-(3-chloro-4-fluorophenyl)-1-(1-(1-oxo-1,2-dihydroisoquinolin-4-yl)ethyl)-1-(thiazol-5-ylmethyl)urea;
(S)-3-(3-chloro-4-fluorophenyl)-1-(1-(1-oxo-1-oxo-1,2-dihydroisoquinolin-4-yl)ethyl)-1-(thiazol-5-ylmethyl)urea;
(R)-3-(3 -chloro-4-fluorophenyl)-1-(1-(1-(hydroxymethyl)isoquinolin-4-yl)ethyl)-1-methylurea;
(S)-3-(3 -chloro-4-fluorophenyl)-1-(1-(1-(hydroxymethyl)isoquinolin-4-yl)ethyl)-1-methylurea;
(R)-4-(1-(3-(3-chloro-4-fluorophenyl)-1-methylureido)ethyl)isoquinoline-1-carboxamide;
(S)-4-(1-(3-(3 -chloro-4-fluorophenyl)-1-methylureido)ethyl)isoquinoline-1-carboxamide;
(R)-3-(3 -chloro-4-fluorophenyl)-1-(1-(7,8-difluoro-1-oxo-1,2-dihydroisoquinolin-4-yl)ethyl)-1-methylurea;
(S)-3-(3 -chloro-4-fluorophenyl)-1-(1-(7,8-difluoro-1-oxo-1,2-dihydroisoquinolin-4-yl)ethyl)-1-methylurea;
(R)-3-(3 -chloro-4-fluorophenyl)-1-(1-(7,8-difluoro-2-methyl-1-oxo-1,2-dihydroisoquinolin-4-yl)ethyl)-1-methylurea;
(S)-3-(3 -chloro-4-fluorophenyl)-1-(1-(7,8-difluoro-2-methyl-1-oxo-1,2-dihydroisoquinolin-4-yl)ethyl)-1-methylurea;
(R)-3-(3 -chloro-4-fluorophenyl)-1-(1-(7,8-difluoro-1-oxo-1,2-dihydroisoquinolin-4-yl)ethyl)-1-(3-hydroxypropyl)urea;
(S)-3-(3 -chloro-4-fluorophenyl)-1-(1-(7,8-difluoro-1-oxo-1,2-dihydroisoquinolin-4-yl)ethyl)-1-(3-hydroxypropyl)urea;
(R)-3-(3 -chloro-4-fluorophenyl)-1-(1-(1-cyanoisoquinolin-4-yl)ethyl)-1-methylurea;
(S)-3-(3 -chloro-4-fluorophenyl)-1-(1-(1-cyanoisoquinolin-4-yl)ethyl)-1-methylurea;
(R)-3-(3 -chloro-4-fluorophenyl)-1-(1-(7,8-difluoro-1-oxo-1,2-dihydroisoquinolin-4-yl)ethyl)-1-isobutylurea;
(S)-3-(3 -chloro-4-fluorophenyl)-1-(1-(7,8-difluoro-1-oxo-1,2-dihydroisoquinolin-4-yl)ethyl)-1-isobutylurea;
(R)-3-(3 -chloro-4-fluorophenyl)-1-(1-(6,7-difluoro-2-methyl-1-oxo-1,2-dihydroisoquinolin-4-yl)ethyl)-1-methylurea;
(S)-3-(3-chloro-4-fluorophenyl)-1-(1-(6,7-difluoro-2-methyl-1-oxo-1,2-dihydroisoquinolin-4-yl)ethyl)-1-methylurea;
(R)-3-(3 -chloro-4-fluorophenyl)-1-(1-(6,7-difluoro-2-methyl-1-oxo-1,2-dihydroisoquinolin-4-yl)ethyl)-1-isobutylurea;
(S)-3-(3-chloro-4-fluorophenyl)-1-(1-(6,7-difluoro-2-methyl-1-oxo-1,2-dihydroisoquinolin-4-yl)ethyl)-1-isobutylurea;
(R)-1-(1-(1-(1H-1,2,4-triazol-1-yl)isoquinolin-4-yl)ethyl)-3-(3 -chloro-4-fluorophenyl)-1-isobutylurea;
(S)-1-(1-(1-(1H-1,2,4-triazol-1-yl)isoquinolin-4-yl)ethyl)-3-(3-chloro-4-fluorophenyl)-1-isobutylurea;
(R)-3-(3-chloro-4-fluorophenyl)-1-(1-(6,7-difluoro-1-((1-methyl-1H-1,2,4-triazol-3-yl)methoxy)isoquinolin-4-yl)ethyl)-1-methylurea;
(S)-3-(3-chloro-4-fluorophenyl)-1-(1-(6,7-difluoro-1-((1-methyl-1H-1,2,4-triazol-3-yl)methoxy)isoquinolin-4-yl)ethyl)-1-methylurea;
(R)-3-(3-chloro-4-fluorophenyl)-1-(1-(6,7-difluoro-1-((1-methyl-1H-1,2,4-triazol-3-yl)methoxy)isoquinolin-4-yl)ethyl)-1-(3-hydroxypropyl)urea;
(S)-3-(3-chloro-4-fluorophenyl)-1-(1-(6,7-difluoro-1-((1-methyl-1H-1,2,4-triazol-3-yl)methoxy)isoquinolin-4-yl)ethyl)-1-(3-hydroxypropyl)urea;
(R)-3-(3 -chloro-4-fluorophenyl)-1-(1-(6,7-difluoro-2-methyl-1-oxo-1,2-dihydroisoquinolin-4-yl)ethyl)-1-(3-hydroxypropyl)urea;
(S)-3-(3 -chloro-4-fluorophenyl)-1-(1-(6,7-difluoro-2-methyl-1-oxo-1,2-dihydroisoquinolin-4-yl)ethyl)-1-(3-hydroxypropyl)urea;
(R)-3-(3-chloro-4-fluorophenyl)-1-methyl-1-(1-(1-methylisoquinolin-4-yl)ethyl)urea;
(S)-3-(3-chloro-4-fluorophenyl)-1-methyl-1-(1-(1-methylisoquinolin-4-yl)ethyl)urea;
(R)-3-(3-chloro-4-fluorophenyl)-1-(1-(8-fluoro-1-oxo-1,2-dihydroisoquinolin-4-yl)ethyl)-1-methylurea;
(S)-3-(3-chloro-4-fluorophenyl)-1-(1-(8-fluoro-1-oxo-1,2-dihydroisoquinolin-4-yl)ethyl)-1-methylurea;
(R)-3-(3-chloro-4-fluorophenyl)-1-(1-(8-fluoro-2-methyl-1-oxo-1,2-dihydroisoquinolin-4-yl)ethyl)-1-methylurea;
(S)-3-(3 -chloro-4-fluorophenyl)-1-(1-(8-fluoro-2-methyl-1-oxo-1,2-dihydroisoquinolin-4-yl)ethyl)-1-methylurea;
(R)-3-(3 -chloro-4-fluorophenyl)-1-(1-(8-fluoro-1-oxo-1,2-dihydroisoquinolin-4-yl)ethyl)-1-isobutylurea;
(S)-3-(3 -chloro-4-fluorophenyl)-1-(1-(8-fluoro-1-oxo-1,2-dihydroisoquinolin-4-yl)ethyl)-1-isobutylurea;
(R)-1-(1-(1-((1H-1,2,4-triazol-3-yl)methoxy)-6,7-difluoroisoquinolin-4-yl)ethyl)-3-(3-chloro-4-fluorophenyl)-1-methylurea;
(S)-1-(1-(1-((1H-1,2,4-triazol-3-yl)methoxy)-6,7-difluoroisoquinolin-4-yl)ethyl)-3-(3-chloro-4-fluorophenyl)-1-methylurea;
(R)-1-(1-(1-((1H-1,2,4-triazol-3-yl)methoxy)-6,7-difluoroisoquinolin-4-yl)ethyl)-3-(3-chloro-4-fluorophenyl)-1-(3-hydroxypropyl)urea;
(S)-1-(1-(1-((1H-1,2,4-triazol-3-yl)methoxy)-6,7-difluoroisoquinolin-4-yl)ethyl)-3-(3-chloro-4-fluorophenyl)-1-(3-hydroxypropyl)urea;
(R)-3-(3 -chloro-4-fluorophenyl)-1-(1-(8-fluoro-1-oxo-1,2-dihydroisoquinolin-4-yl)ethyl)-1-(3-hydroxypropyl)urea;
(S)-3-(3 -chloro-4-fluorophenyl)-1-(1-(8-fluoro-1-oxo-1,2-dihydroisoquinolin-4-yl)ethyl)-1-(3-hydroxypropyl)urea;
(R)-3-(3 -chloro-4-fluorophenyl)-1-(1-(6,8-difluoro-1-oxo-1,2-dihydroisoquinolin-4-yl)ethyl)-1-methylurea;
(S)-3-(3 -chloro-4-fluorophenyl)-1-(1-(6,8-difluoro-1-oxo-1,2-dihydroisoquinolin-4-yl)ethyl)-1-methylurea;
(R)-1-(1-(6,8-diffuoro-1-oxo-1,2-dihydroisoquinolin-4-yl)ethyl)-3-(4-fluorophenyl)-1-methylurea;
(S)-1-(1-(6, 8-difluoro-1-oxo-1,2-dihydroisoquinolin-4-yl)ethyl)-3-(4-fluorophenyl)-1-methylurea;
(R)-3-(3 -chloro-4-fluorophenyl)-1-(1-(6,8-difluoro-1 -oxo-1,2-dihydroisoquinolin-4-yl)ethyl)-1-(3-hydroxypropyl)urea;
(S)-3-(3 -chloro-4-fluorophenyl)-1-(1-(6,8-difluoro-1-oxo-1,2-dihydroisoquinolin-4-yl)ethyl)-1-(3-hydroxypropyl)urea;
(R)-1-(1-(6,8-diffuoro-1-oxo-1,2-dihydroisoquinolin-4-yl)ethyl)-3-(4-fluorophenyl)-1-isobutylurea;
(S)-1-(1-(6, 8-difluoro-1-oxo-1,2-dihydroisoquinolin-4-yl)ethyl)-3-(4-fluorophenyl)-1-isobutylurea;
(R)-1-(1-(6,8-difluoro-1-oxo-1,2-dihydroisoquinolin-4-yl)ethyl)-3-(4-fluorophenyl)-1-(3 - hydroxypropyl)urea;
(S)-1-(1-(6, 8-difluoro-1-oxo-1,2-dihydroisoquinolin-4-yl)ethyl)-3-(4-fluorophenyl)-1-(3-hydroxypropyl)urea;
(R)-3-(3-chloro-4-fluorophenyl)-1-(2-hydroxy-1-(1-oxo-1,2-dihydroisoquinolin-4-yl)ethyl)-1-methylurea;
(S)-3-(3-chloro-4-fluorophenyl)-1-(2-hydroxy-1-(1-oxo-1,2-dihydroisoquinolin-4-yl)ethyl)-1-methylurea;
(R)-2-(3-(3-chloro-4-fluorophenyl)-1-(1-(6-fluoro-1-oxo-1,2-dihydroisoquinolin-4-yl)ethyl)ureido)ethane-1-sulfonamide;
(S)-2-(3-(3-chloro-4-fluoropheny1)-1-(1-(6-fluoro-1-oxo-1,2-dihydroisoquinolin-4-yl)ethyl)ureido)ethane-1-sulfonamide;
(R)-2-(3-(3-chloro-4-fluorophenyl)-1-(1-(7-fluoro-1-oxo-1,2-dihydroisoquinolin-4-yl)ethyl)ureido)ethane-1-sulfonamide;
(S)-2-(3-(3-chloro-4-fluoropheny1)-1-(1-(7-fluoro-1-oxo-1,2-dihydroisoquinolin-4-yl)ethyl)ureido)ethane-1-sulfonamide;
(R)-2-(3-(3-chloro-4-fluorophenyl)-1-(1-(6,7-diffuoro-1-oxo-1,2-dihydroisoquinolin-4-yl)ethyl)ureido)ethane-1-sulfonamide;
(S)-2-(3-(3-chloro-4-fluorophenyl)-1-(1-(6,7-difluoro-1-oxo-1,2-dihydroisoquinolin-4-yl)ethyl)ureido)ethane-1-sulfonamide;
(R)-2-(1-(1-(6,7-difluoro-1-oxo-1,2-dihydroisoquinolin-4-yl)ethyl)-3-(4-fluorophenyl)ureido)ethane-1 - sulfonamide;
(S)-2-(1-(1-(6,7-difluoro-1-oxo-1,2-dihydroisoquinolin-4-yl)ethyl)-3-(4-fluorophenyl)ureido)ethane-1-sulfonamide;
(R)-2-(3-(3-chloro-4-fluorophenyl)-1-(1-(6,8-difluoro-1-oxo-1,2-dihydroisoquinolin-4-yl)ethyl)ureido)ethane-1-sulfonamide;
(S)-2-(3 -(3-chloro-4-fluorophenyl)-1-(1-(6, 8-difluoro-1-oxo-1,2-dihydroisoquinolin-4-yl)ethyl)ureido)ethane-1-sulfonamide;
(R)-2-(3-(3-chloro-4-fluorophenyl)-1-(1-(7,8-difluoro-1-oxo-1,2-dihydroisoquinolin-4-yl)ethyl)ureido)ethane-1-sulfonamide;
(S)-2-(3 -(3-chloro-4-fluorophenyl)-1-(1-(7,8-difluoro-1-oxo-1 ,2-dihydroisoquinolin-4-yl)ethyl)ureido)ethane-1-sulfonamide;
(R)-2-(3-(4-fluorophenyl)-1-(1-(7,8-difluoro-1-oxo-1,2-dihydroisoquinolin-4-yl)ethyl)ureido)ethane-1-sulfonamide;
(S)-2-(3 -(4-fluorophenyl)-1-(1-(7, 8-diffuoro-1-oxo-1,2-dihydroisoquinolin-4-yl)ethyl)ureido)ethane-1-sulfonamide;
(R)-2-(3 -(3-chloro-4-fluorophenyl)-1-(1-(8-fluoro-1-oxo-1 ,2-dihydroisoquinolin-4-yl)ethyl)ureido)ethane-1-sulfonamide;
(S)-2-(3 -(3-chloro-4-fluorophenyl)-1-(1-(8-fluoro-1-oxo-1 ,2-dihydroisoquinolin-4-yl)ethyl)ureido)ethane-1-sulfonamide;
(R)-1-(1-(6,7-difluoro-1-oxo-1,2-dihydroisoquinolin-4-yl)ethyl)-1 -methyl-3-phenylurea;
(S)-1-(1-(6,7-difluoro-1-oxo-1,2-dihydroisoquinolin-4-yl)ethyl)-1-methyl-3 -phenylurea;
(R)-3-(4-chlorophenyl)-1-(1-(6,7-difluoro-1-oxo-1,2-dihydroisoquinolin-4-yl)ethyl)-1-methylurea;
(S)-3-(4-chlorophenyl)-1-(1-(6,7-difluoro-1-oxo-1,2-dihydroisoquinolin-4-yl)ethyl)-1 -methylurea;
(R)-1-(1-(6,7-difluoro-1-oxo-1,2-dihydroisoquinolin-4-yl)ethyl)-1-ethyl-3-(4-fluorophenyl)urea;
(S)-1-(1-(6,7-difluoro-1-oxo-1,2-dihydroisoquinolin-4-yl)ethyl)-1-ethyl-3-(4-fluorophenyl)urea;
(R)-1-(1-(6,7-difluoro-1-oxo-1,2-dihydroisoquinolin-4-yl)ethyl)-1-ethyl-3-phenylurea;
(S)-1-(1-(6,7-difluoro-1-oxo-1,2-dihydroisoquinolin-4-yl)ethyl)-1-ethyl-3-phenylurea;
(R)-3-(3-chloro-4-fluorophenyl)-1-(1-(6,7-difluoro-1-oxo-1,2-dihydroisoquinolin-4-yl)ethyl)-1-ethylurea;
(S)-3-(3-chloro-4-fluorophenyl)-1-(1-(6,7-difluoro-1-oxo-1,2-dihydroisoquinolin-4-yl)ethyl)-1-ethylurea;
(R)-1-(1-(6,7-diffuoro-1-oxo-1,2-dihydroisoquinolin-4-yl)ethyl)-3-(3,4-difluorophenyl)-1-methylurea;
(S)-1-(1-(6,7-difluoro-1-oxo-1,2-dihydroisoquinolin-4-yl)ethyl)-3-(3,4-difluorophenyl)-1-methylurea;
(R)-1-(1-(6,7-difluoro-1-oxo-1,2-dihydroisoquinolin-4-yl)ethyl)-3-(3-fluorophenyl)-1-methylurea;
(S)-1-(1-(6,7-difluoro-1-oxo-1,2-dihydroisoquinolin-4-yl)ethyl)-3-(3-fluorophenyl)-1-methylurea;
(R)-3-(3 -chlorophenyl)-1-(1-(6,7-difluoro-1-oxo-1,2-dihydroisoquinolin-4-yl)ethyl)-1-methylurea;
(S)-3-(3 -chlorophenyl)-1-(1-(6,7-difluoro-1-oxo-1,2-dihydroisoquinolin-4-yl)ethyl)-1-methylurea;
(R)-1-(1-(6,7-difluoro-1-oxo-1,2-dihydroisoquinolin-4-yl)ethyl)-1-methyl-3-(3,4,5-trifluorophenyl)urea;
(S)-1-(1-(6,7-difluoro-1-oxo-1,2-dihydroisoquinolin-4-yl)ethyl)-1-methyl-3-(3,4,5-trifluorophenyl)urea;
(R)-3-(3,5-dichlorophenyl)-1-(1-(6,7-difluoro-1-oxo-1,2-dihydroisoquinolin-4-yl)ethyl)-1-methylurea;
(S)-3-(3,5-dichlorophenyl)-1-(1-(6,7-difluoro-1-oxo-1,2-dihydroisoquinolin-4-yl)ethyl)-1-methylurea;
(R)-3-(2-chloropyridin-4-yl)-1-(1-(6,7-difluoro-1-oxo-1,2-dihydroisoquinolin-4-yl)ethyl)-1-methylurea;
(S)-3-(2-chloropyridin-4-yl)-1-(1-(6,7-difluoro-1-oxo-1,2-dihydroisoquinolin-4-yl)ethyl)-1-methylurea;
(R)-1-(1-(6,7-diffuoro-1-oxo-1,2-dihydroisoquinolin-4-yl)ethyl)-3-(2-fluorophenyl)-1-methylurea;
(S)-1-(1-(6,7-difluoro-1-oxo-1,2-dihydroisoquinolin-4-yl)ethyl)-3-(2-fluorophenyl)-1-methylurea;
(R)-1-(1-(6,7-difluoro-1-oxo-1,2-dihydroisoquinolin-4-yl)ethyl)-3-(3,4-difluorophenyl)-1-ethylurea;
(S)-1-(1-(6,7-difluoro-1-oxo-1,2-dihydroisoquinolin-4-yl)ethyl)-3-(3,4-difluorophenyl)-1-ethylurea;
(R)-1-(1-(1-(1H-1,2,3-triazol-1-yl)isoquinolin-4-yl)ethyl)-3-(3-chloro-4-fluorophenyl)-1-methylurea;
(S)-1-(1-(1-(1H-1,2,3-triazol-1-yl)isoquinolin-4-yl)ethyl)-3-(3-chloro-4-fluorophenyl)-1-methylurea;
(R)-1-(1-(6,7-Difluoro-1-oxo-1,2-dihydroisoquinolin-4-yl)ethyl)-3-(4-fluoro-3-methylphenyl)-1-methylurea;
(S)-1-(1-(6,7-Difluoro-1-oxo-1,2-dihydroisoquinolin-4-yl)ethyl)-3 -(4-ffuoro-3-methylphenyl)-1-methylurea;
(R)-3-(3 -cyano-4-fluorophenyl)-1-(1-(6,7-difluoro-1-oxo-1,2-dihydroisoquinolin-4-yl)ethyl)-1-methylurea;
(S)-3-(3-cyano-4-fluorophenyl)-1-(1-(6,7-difluoro-1-oxo-1,2-dihydroisoquinolin-4-yl)ethyl)-1-methylurea;
(R)-1-(1-(6,7-Difluoro-1-oxo-1,2-dihydroisoquinolin-4-yl)ethyl)-3-(2,3-difluorophenyl)-1-methylurea;
(S)-1-(1-(6,7-Difluoro-1-oxo-1,2-dihydroisoquinolin-4-yl)ethyl)-3-(2,3-difluorophenyl)-1-methylurea;
(R)-1-(1-(7,8-Difluoro-1-oxo-1,2-dihydroisoquinolin-4-yl)ethyl)-3-(4-fluorophenyl)-1-methylurea;
(S)-1-(1-(7,8-Difluoro-1-oxo-1,2-dihydroisoquinolin-4-yl)ethyl)-3-(4-fluorophenyl)-1-methylurea;
(R)-1-(1-(7,8-Difluoro-1-oxo-1,2-dihydroisoquinolin-4-yl)ethyl)-3-(3,4-difluorophenyl)-1-methylurea;
(S)-1-(1-(7,8-Difluoro-1-oxo-1 ,2-dihydroisoquinolin-4-yl)ethyl)-3 -(3,4-difluorophenyl)-1-methylurea;
(R)-1-(1-(7,8-Difluoro-1-oxo-1,2-dihydroisoquinolin-4-yl)ethyl)-1-methyl-3-phenylurea;
(S)-1-(1-(7, 8-Difluoro-1-oxo-1,2-dihydroisoquinolin-4-yl)ethyl)-1-methyl-3-phenylurea;
(R)-3-(3,5-dichloro-4-fluorophenyl)-1-(1-(6,7-difluoro-1-oxo-1,2-dihydroisoquinolin-4-yl)ethyl)-1-methylurea;
(S)-3-(3,5-dichloro-4-fluorophenyl)-1-(1-(6,7-difluoro-1-oxo-1,2-dihydroisoquinolin-4-yl)ethyl)-1-methylurea;
(R)-1-(1-(7, 8-Difluoro-1-oxo-1,2-dihydroisoquinolin-4-yl)ethyl)-3-(4-fluoro-3-methylphenyl)-1-methylurea;
(S)-1-(1-(7,8-Difluoro-1-oxo-1,2-dihydroisoquinolin-4-yl)ethyl)-3-(4-fluoro-3-methylphenyl)-1-methylurea;
(R)-1-(1-(7,8-Difluoro-1-oxo-1,2-dihydroisoquinolin-4-yl)ethyl)-1-methyl-3-(3,4,5-trifluorophenyl)urea;
(S)-1-(1-(7,8-Difluoro-1-oxo-1,2-dihydroisoquinolin-4-yl)ethyl)-1-methyl-3-(3,4,5-trifluorophenyl)urea;
(R)-3-(4-Chlorophenyl)-1-(1-(7,8-difluoro-1-oxo-1,2-dihydroisoquinolin-4-yl)ethyl)-1-methylurea;
(S)-3-(4-Chlorophenyl)-1-(1-(7,8-diffuoro-1-oxo-1,2-dihydroisoquinolin-4-yl)ethyl)-1-methylurea;
(R)-3-(3 -chloro-4-fluorophenyl)-1-(1-(6,8-difluoro-1-oxo-1,2-dihydroisoquinolin-4-yl)ethyl)-1-isobutylurea;
(S)-3-(3 -chloro-4-fluorophenyl)-1-(1-(6,8-difluoro-1-oxo-1,2-dihydroisoquinolin-4-yl)ethyl)-1-isobutylurea;
(R)-3-(3,5-dichlorophenyl)-1-(1-(7, 8-difluoro-1-oxo-1,2-dihydroisoquinolin-4-yl)ethyl)-1-methylurea;
(S)-3-(3,5-dichlorophenyl)-1-(1-(7,8-difluoro-1-oxo-1,2-dihydroisoquinolin-4-yl)ethyl)-1-methylurea;
(R)-3-(3,5-dichloro-4-fluorophenyl)-1-(1-(7,8-difluoro-1-oxo-1,2-dihydroisoquinolin-4-yl)ethyl)-1-methylurea;
(S)-3-(3,5-dichloro-4-fluorophenyl)-1-(1-(7,8-difluoro-1-oxo-1,2-dihydroisoquinolin-4-yl)ethyl)-1-methylurea;
(R)-1-(1-(7,8-difluoro-1-oxo-1,2-dihydroisoquinolin-4-yl)ethyl)-3-(3-fluorophenyl)-1-methylurea;
(S)-1-(1-(7, 8-difluoro-1-oxo-1,2-dihydroisoquinolin-4-yl)ethyl)-3-(3-fluorophenyl)-1-methylurea;
(R)-1-(1-(7,8-difluoro-1-oxo-1,2-dihydroisoquinolin-4-yl)ethyl)-3-(2,3-difluorophenyl)-1-methylurea;
(S)-1-(1-(7, 8-difluoro-1-oxo-1,2-dihydroisoquinolin-4-yl)ethyl)-3-(2,3-difluorophenyl)-1-methylurea;
(R)-1-(1-(7,8-difluoro-1-oxo-1,2-dihydroisoquinolin-4-yl)ethyl)-3-(3-chlorophenyl)-1-methylurea;
(S)-1-(1-(7,8-difluoro-1-oxo-1,2-dihydroisoquinolin-4-yl)ethyl)-3-(3-chlorophenyl)-1-methylurea;
(R)-1-(1-(7,8-diffuoro-1-oxo-1,2-dihydroisoquinolin-4-yl)ethyl)-3-(3,4-difluorophenyl)-1-ethylurea;
(S)-1-(1-(7, 8-difluoro-1-oxo-1,2-dihydroisoquinolin-4-yl)ethyl)-3-(3,4-difluorophenyl)-1-ethylurea;
(R)-1-(1-(7,8-diffuoro-1-oxo-1,2-dihydroisoquinolin-4-yl)ethyl)-3-(4-fluorophenyl)-1-ethylurea;
(S)-1-(1-(7, 8-difluoro-1-oxo-1,2-dihydroisoquinolin-4-yl)ethyl)-3-(4-fluorophenyl)-1-ethylurea;
(R)-1-(1-(7,8-difluoro-1-oxo-1,2-dihydroisoquinolin-4-yl)ethyl)-3-phenyl-1-ethylurea;
(S)-1-(1-(7, 8-difluoro-1-oxo-1,2-dihydroisoquinolin-4-yl)ethyl)-3-phenyl-1-ethylurea;
(R)-1-(1-(7,8-difluoro-1-oxo-1 ,2-dihydroisoquinolin-4-yl)ethyl)-3 -(3-chloro-4-fluorophenyl)-1-ethylurea;
(S)-1-(1-(7, 8-difluoro-1-oxo-1,2-dihydroisoquinolin-4-yl)ethyl)-3-(3-chloro-4-fluorophenyl)-1-ethylurea;
(R)-3-(3 -cyano-4-fluorophenyl)-1-(1-(7, 8-difluoro-1-oxo-1,2-dihydroisoquinolin-4-yl)ethyl)-1-methylurea;
(S)-3-(3 -cyano-4-fluorophenyl)-1-(1-(7,8-difluoro-1-oxo-1 ,2-dihydroisoquinolin-4-yl)ethyl)-1-methylurea;
(R)-1-(1-(6,7-difluoro-1-oxo-1,2-dihydroisoquinolin-4-yl)ethyl)-3-(3,4-difluorobenzyl)-1-methylurea;
(S)-1-(1-(6,7-difluoro-1-oxo-1,2-dihydroisoquinolin-4-yl)ethyl)-3-(3,4-difluorobenzyl)-1-methylurea;
(R)-1-(1-(6,7-difluoro-1-oxo-1,2-dihydroisoquinolin-4-yl)ethyl)-1-methyl-3-(3,4,5-trifluorobenzyl)urea;
(S)1-(1-(6,7-difluoro-1-oxo-1,2-dihydroisoquinolin-4-yl)ethyl)-1-methyl-3-(3,4,5-trifluorobenzyl)urea;
(R)-1-(1-(6,7-diffuoro-1-oxo-1,2-dihydroisoquinolin-4-yl)ethyl)-1-methyl-3-(3-chloro-4-fluorobenzyl)urea;
(S)-1-(1-(6,7-difluoro-1-oxo-1,2-dihydroisoquinolin-4-yl)ethyl)-1-methyl-3 -(3-chloro-4-fluorobenzyl)urea;
(R)-1-(1-(6,7-diffuoro-1-oxo-1,2-dihydroisoquinolin-4-yl)ethyl)-3-(1H-indol-6-yl)-1-methylurea;
(S)-1-(1-(6,7-difluoro-1-oxo-1,2-dihydroisoquinolin-4-yl)ethyl)-3-(1H-indol-6-yl)-1-methylurea;
(R)-1-(1-(7,8-difluoro-1-oxo-1 ,2-dihydroisoquinolin-4-yl)ethyl)-3 -(3,4-difluorobenzyl)-1-methylurea;
(S)-1-(1-(7, 8-difluoro-1-oxo-1,2-dihydroisoquinolin-4-yl)ethyl)-3-(3,4-difluorobenzyl)-1-methylurea;
(R)-1-(1-(7,8-difluoro-1-oxo-1,2-dihydroisoquinolin-4-yl)ethyl)-3-(4-fluorobenzyl)-1-methylurea;
(S)-1-(1-(7, 8-difluoro-1-oxo-1,2-dihydroisoquinolin-4-yl)ethyl)-3-(4-fluorobenzyl)-1-methylurea;
(R)-1-(1-(7,8-difluoro-1-oxo-1,2-dihydroisoquinolin-4-yl)ethyl)-3-(3,4,5-trifluorobenzyl)-1-methylurea;
(S)-1-(1-(7,8-difluoro-1-oxo-1,2-dihydroisoquinolin-4-yl)ethyl)-3-(3,4,5-trifluorobenzyl)-1-methylurea;
(R)-3-(3-chloro-4-fluorobenzyl)-1-(1-(7,8-difluoro-1-oxo-1,2-dihydroisoquinolin-4-yl)ethyl)-1-methylurea;
(S)-3-(3 -chloro-4-fluorobenzyl)-1-(1-(7, 8-difluoro-1-oxo-1,2-dihydroisoquinolin-4-yl)ethyl)-1-methylurea;
(R)-1-(1-(7,8-difluoro-1-oxo-1,2-dihydroisoquinolin-4-yl)ethyl)-3-(1H-indol-6-yl)-1-methylurea;
(S)-1-(1-(7, 8-difluoro-1-oxo-1,2-dihydroisoquinolin-4-yl)ethyl)-3-(1H-indol-6-yl)-1-methylurea;
(R)-1-(1-(6,7-diffuoro-1-oxo-1,2-dihydroisoquinolin-4-yl)ethyl)-3-(3-(difluoromethyl)-4-fluorophenyl)-1-methylurea;
(S)-1-(1-(6,7-difluoro-1-oxo-1,2-dihydroisoquinolin-4-yl)ethyl)-3-(3-(difluoromethyl)-4-fluorophenyl)-1-methylurea;
(R)-1-(1-(7,8-difluoro-1-oxo-1,2-dihydroisoquinolin-4-yl)ethyl)-3-(3-(difluoromethyl)-4-fluorophenyl)-1-methylurea; and
(S)-1-(1-(7,8-difluoro-1-oxo-1,2-dihydroisoquinolin-4-yl)ethyl)-3-(3-(difluoromethyl)-4-fluorophenyl)-1-methylurea;
or a salt, solvate, isotopically labelled derivative, stereoisomer, or tautomer thereof, or any mixtures thereof.

12. A compound selected from the group consisting of:
1-(4-fluoro-3-methylphenyl)-3-((1-oxo-1,2-dihydroisoquinolin-4-yl)methyl)urea;
1-(3-chloro-4-fluorophenyl)-3-((1-methoxylsoquinolin-4-yl)methyl)urea;
1-(4-fluoro-3-methylphenyl)-3-((1-methoxylsoquinolin-4-yl)methyl)urea;
1-(3-chloro-4-fluorophenyl)-3-((1-oxo-1,2-dihydroisoquinolin-4-yl)methyl)urea;
1-(3-chloro-4-fluorophenyl)-3-(1-(1-oxo-1,2-dihydroisoquinolin-4-yl)ethyl)urea;
1-(3-chloro-4-fluorophenyl)-3-(3-hydroxy-1-(1-methoxyisoquinolin-4-yl)propyl)urea;
1-(3-chloro-4-fluorophenyl)-3-(1-(6,7-difluoro-1-oxo-1,2-dihydroisoquinolin-4-yl)ethyl)urea;
1-(3-cyano-4-fluorophenyl)-3-(1-(6,7-difluoro-1-oxo-1,2-dihydroisoquinolin-4-yl)ethyl)urea; and
1-(3-chloro-4-fluorophenyl)-3-(1-(7,8-difluoro-1-oxo-1,2-dihydroisoquinolin-4-yl)ethyl)urea;
or a salt, solvate, isotopically labelled derivative, stereoisomer, or tautomer thereof, or any mixtures thereof.

13. A pharmaceutical composition comprising at least one compound of any one of claims 1 to 12 and a pharmaceutically acceptable carrier.

14. At least one compound of any one of claims 1 to 12 for use in treating or preventing hepatitis B virus (HBV) infection in a subject, or for inhibiting expression and/or function of a viral capsid protein directly or indirectly in a hepatitis B virus-infected subject.

15. The at least one compound of any one of claims 1 to 12 for use according to claim 14, wherein the subject is further infected with hepatitis D virus (HDV).

16. The pharmaceutical composition of claim 13 or the at least one compound of any one of claims 1 to 12 for use according to claim 14, further comprising at least one additional agent useful for treating the hepatitis B viral infection, preferably co-formulated or co-administered to the subject, wherein the at least one additional agent comprises at least one agent selected from the group consisting of reverse transcriptase inhibitor, capsid inhibitor, cccDNA formation inhibitor, RNA destabilizer, oligomeric nucleotides targeted against the HBV genome, immunostimulators, GalNAc-siRNA conjugate targeted against an HBV gene transcript.

17. At least one compound of any one of claims 1 to 12 for use according to claim 14, wherein the subject is a mammal, preferably a human.

## Patentansprüche

1. Eine Verbindung der Formel (I), oder ein Salz, Solvat, Stereoisomer, Tautomer oder Isotopen-markiertes Derivat davon, oder eine beliebige Mischung davon: 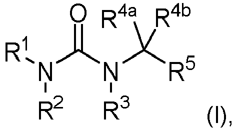 worin in (I):
R¹ ausgewählt ist aus der Gruppe bestehend aus gegebenenfalls substituiertem Phenyl, gegebenenfalls substituiertem C₃-C₈ Cycloalkyl, gegebenenfalls substituiertem Benzyl, gegebenenfalls substituiertem Heteroaryl, und -(CH₂)(gegebenenfalls substituiertem Heteroaryl);
R² jeweils unabhängig voneinander ausgewählt ist aus der Gruppe bestehend aus H und C₁-C₆ Alkyl;
R³ ausgewählt ist aus der Gruppe bestehend aus C₁-C₆ Alkyl, und C₃-C₈ Cycloalkyl, worin das Alkyl oder Cycloalkyl gegebenenfalls substituiert ist mit mindestens einem Substituenten, der ausgewählt ist aus der Gruppe bestehend aus C₁-C₆ Alkyl, C₃-C₈ Cycloalkyl, Halogen, Cyano, - OH, C₁-C₆ Alkoxy, C₃-C₈ Cycloalkoxy, C₁-C₆ Haloalkoxy, C₃-C₈ Halocycloalkoxy, gegebenenfalls substituiertem Phenyl, gegebenenfalls substituiertem Heteroaryl, gegebenenfalls substituiertem Heterocyclyl, -C(=O)OR⁶, -OC(=O)R⁶, -SR⁶, -S(=O)R⁶, -S(=O)₂R⁶, -S(=O)₂NR⁶R⁶, -N(R⁶)S(=O)₂R⁶, - N(R⁶)C(=O)R⁶, -C(=O)NR⁶R⁶ und -NR⁶R⁶;
R^{4a} ausgewählt ist aus der Gruppe bestehend aus H, C₁-C₆ Alkyl, C₃-C₈ Cycloalkyl und Phenyl, worin das Alkyl, Cycloalkyl oder Phenyl gegebenenfalls substituiert ist mit mindestens einem Substituenten, der ausgewählt ist aus der Gruppe bestehend aus C₁-C₆ Alkyl, C₃-C₈ Cycloalkyl, Halogen, Cyano, -OH, C₁-C₆ Alkoxy, C₃-C₈ Cycloalkoxy, C₁-C₆ Haloalkoxy, C₃-C₈ Halocycloalkoxy, - NR⁶R⁶ und gegebenenfalls substituiertem Phenyl;
R^{4b} ausgewählt ist aus der Gruppe bestehend aus H und gegebenenfalls substituiertem C₁-C₆ Alkyl;
R⁵ ausgewählt ist aus der Gruppe bestehend aus: 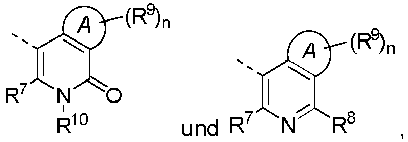 wobei Ring A jeweils unabhängig voneinander ausgewählt ist aus der Gruppe bestehend aus Benzol, Pyridin, Pyrimidin, Pyridazin und Pyrazin;
R⁶ jeweils unabhängig voneinander ausgewählt ist aus der Gruppe bestehend aus H, gegebenenfalls substituiertem C₁-C₆ Alkyl, gegebenenfalls substituiertem C₃-C₈ Cycloalkyl, gegebenenfalls substituiertem Phenyl und gegebenenfalls substituiertem Hetereoaryl;
R⁷ jeweils unabhängig voneinander ausgewählt ist aus der Gruppe bestehend aus H, Halogen, gegebenenfalls substituiertem C₁-C₆ Alkyl, gegebenenfalls substituiertem C₃-C₈ Cycloalkyl, gegebenenfalls substituiertem C₁-C₆ Alkoxy und gegebenenfalls substituiertem C₃-C₈ Cycloalkoxy;
R⁸ jeweils unabhängig voneinander ausgewählt ist aus der Gruppe bestehend aus Halogen, - CN, gegebenenfalls substituiertem C₁-C₆ Alkyl, gegebenenfalls substituiertem C₃-C₈ Cycloalkyl, gegebenenfalls substituiertem C₁-C₆ Alkoxy, gegebenenfalls substituiertem C₃-C₈ Cycloalkoxy, Heterocyclyl, Heteroaryl, -S(gegebenenfalls substituiertes C₁-C₆ Alkyl), -SO(gegebenenfalls substituiertes C₁-C₆ Alkyl), -SO₂(gegebenenfalls substituiertes C₁-C₆ Alkyl), -C(=O)OH, -C(=O)O(gegebenenfalls substituiertes C₁-C₆ Alkyl), -C(=O)O(gegebenenfalls substituiertes C₃-C₈ Cycloalkyl), -O(gegebenenfalls substituiertes C₁-C₆ Alkyl), -O(gegebenenfalls substituiertes C₃-C₈ Cycloalkyl),
-NH₂, -NH(gegebenenfalls substituiertes C₁-C₆ Alkyl), -NH(gegebenenfalls substituiertes C₃-C₈ Cycloalkyl), -N(gegebenenfalls substituiertes C₁-C₆ Alkyl)(gegebenenfalls substituiertes C₁-C₆ Alkyl), -N(gegebenenfalls substituiertes C₃-C₈ Cycloalkyl)(gegebenenfalls substituiertes C₃-C₈ Cycloalkyl), -N(gegebenenfalls substituiertes C₁-C₆ Alkyl)(gegebenenfalls substituiertes C₃-C₈ Cycloalkyl), -C(=O)NH₂, -C(=O)NH(gegebenenfalls substituiertes C₁-C₆Alkyl), -C(=O)NH(gegebenenfalls substituiertes C₃-C₈ Cycloalkyl), -C(=O)N(gegebenenfalls substituiertes C₁-C₆ Alkyl)(gegebenenfalls substituiertes C₁-C₆ Alkyl), -C(=O)N(gegebenenfalls substituiertes C₃-C₈ Cycloalkyl)(gegebenenfalls substituiertes C₃-C₈ Cycloalkyl) und -C(=O)N(gegebenenfalls substituiertes C₁-C₆ Alkyl)(gegebenenfalls substituiertes C₃-C₈ cycloalkyl;
R⁹ jeweils unabhängig voneinander ausgewählt ist aus der Gruppe bestehend aus H, Halogen, gegebenenfalls substituiertem C₁-C₆ Alkyl, gegebenenfalls substituiertem C₃-C₈ Cycloalkyl, gegebenenfalls substituiertem C₁-C₆ Alkoxy und gegebenenfalls substituiertem C₃-C₈ Cycloalkoxy;
n jeweils unabhängig voneinander 0, 1, 2, 3 oder 4 ist; und
R¹⁰ ausgewählt ist aus der Gruppe bestehend aus H, gegebenenfalls substituiertem C₁-C₆ Alkyl und gegebenenfalls substituiertem C₃-C₈ Cycloalkyl.

2. Die Verbindung nach Anspruch 1, worin jedes R^{4b} jeweils unabhängig voneinander ausgewählt ist aus der Gruppe bestehend aus H und CH₃.

3. Die Verbindung nach Anspruch 1, worin jedes Aryl oder Heteroaryl jeweils unabhängig voneinander gegebenenfalls substituiert ist mit mindestens einem Substituenten, der ausgewählt ist aus der Gruppe bestehend aus C₁-C₆ Alkyl, C₃-C₈ Cycloalkyl, Phenyl, C₁-C₆ Hydroxyalkyl, (C₁-C₆ Alkoxy)-C₁-C₆ alkyl, C₁-C₆ Haloalkyl, C₁-C₆ Haloalkoxy, Halogen, -CN, -OR^{b}, -N(R^{b})(R^{b}), - NO₂, -C(=O)N(R^{b})(R^{b}), -C(=O)OR^{b}, -OC(=O)R^{b}, -SR^{b}, -S(=O)R^{b}, -S(=O)₂R^{b}, N(R^{b})S(=O)₂R^{b}, - S(=O)₂N(R^{b})(R^{b}), Acyl und C₁-C₆ Alkoxycarbonyl, worin jedes R^{b} jeweils unabhängig voneinander H, C₁-C₆ Alkyl oder C₃-C₈ Cycloalkyl ist, worin in R^{b} das Alkyl oder Cycloalkyl gegebenenfalls substituiert ist mit mindestens einem Substituenten, der ausgewählt ist aus der Gruppe bestehend aus Halogen, -OH, C₁-C₆ Alkoxy und Heteroaryl; oder Substituenten auf zwei benachbarten Kohlenstoffatomen bilden zusammen -O(CH₂)₁₋₃O-.

4. Die Verbindung nach Anspruch 1, worin jedes Alkyl, Alkenyl, Alkinyl oder Cycloalkyl jeweils unabhängig voneinander gegebenenfalls substituiert ist mit mindestens einem Substituenten, der ausgewählt ist aus der Gruppe bestehend aus C₁-C₆ Alkyl, C₃-C₈ Cycloalkyl, Halogen, Cyano (-CN), -OR^{a}, gegebenenfalls substituiertem Phenyl, gegebenenfalls substituiertem Heteroaryl, gegebenenfalls substituiertem Heterocyclyl, -C(=O)OR^{a}, -OC(=O)R^{a}, -SR^{a}, -S(=O)R^{a},-S(=O)₂R^{a}, -S(=O)₂NR^{a}R^{a}, -N(R^{a})S(=O)₂R^{a}, -N(R^{a})C(=O)R^{a}, -C(=O)NR^{a}R^{a} und -N(R^{a})(R^{a}), worin jedes R^{a} jeweils unabhängig voneinander H, gegebenenfalls substituiertes C₁-C₆ Alkyl, gegebenenfalls substituiertes C₃-C₈ Cycloalkyl, gegebenenfalls substituiertes Aryl oder gegebenenfalls substituiertes Heteroaryl ist, oder zwei R^{a} Gruppen, die mit dem N an das sie gebunden sind zusammen einen Heterozyklus bilden.

5. Die Verbindung nach Anspruch 1, worin R¹ Phenyl ist, das gegebenenfalls mit mindestens einem Substituenten substituiert ist, der ausgewählt ist aus der Gruppe bestehend aus C₁-C₆ Alkyl, Halogen, C₁-C₃ Haloalkyl und -CN, vorzugsweise ist R¹ ausgewählt aus der Gruppe bestehend aus Phenyl, 3-Chlorphenyl, 4-Chlorphenyl, 3-phenyl, 4-Fluorphenyl, 3,4-Difluorphenyl, 3,5-Difluorphenyl, 2,4,5-Trifluorphenyl, 3,4,5-Trifluorphenyl, 3,4-Dichlorphenyl, 3-Chlor-4-Fluorphenyl, 4-Chlor-3-fluorphenyl, 4-Chlor-3-methylphenyl, 3-Chlor-4-methylphenyl, 4-Fluor-3-methylphenyl, 3-Fluor-4-methylphenyl, 4-Chlor-3-methoxyphenyl, 3-Chlor-4-methoxyphenyl, 4-Fluor-3-methoxyphenyl, 3-Fluor-4-methoxyphenyl, 3-Trifluormethylphenyl, 4-Trifluormethylphenyl, 3-Trifluormethyl-4-fluorphenyl, 4-Trifluormethyl-3-fluorphenyl, 3-Cyanophenyl, 4-Cyanophenyl, 3-Cyano-4-fluorphenyl, 4-Cyano-3-fluorphenyl, 3-Difluormethyl-4-fluorphenyl und 4-Difluormethyl-3-fluorphenyl.

6. Die Verbindung nach Anspruch 1, worin R² ausgewählt ist aus der Gruppe bestehend aus H und Methyl.

7. Die Verbindung nach Anspruch 1, die ausgewählt ist aus der Gruppe bestehend aus:

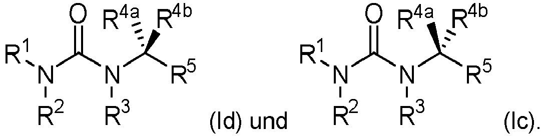

8. Die Verbindung nach Anspruch 1, die ausgewählt ist aus der Gruppe bestehend aus:

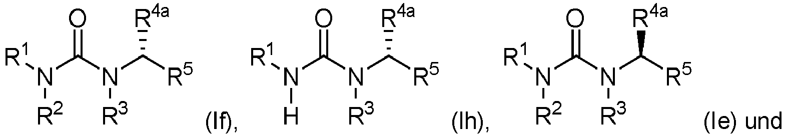

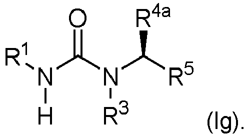

9. Die Verbindung nach Anspruch 1, worin R⁵ ausgewählt ist aus der Gruppe bestehend aus:

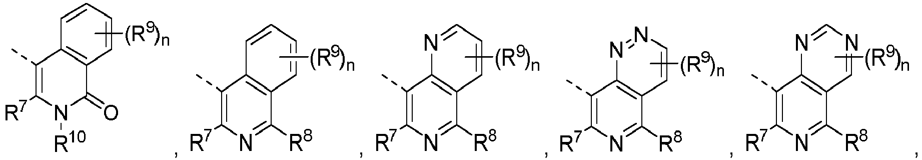

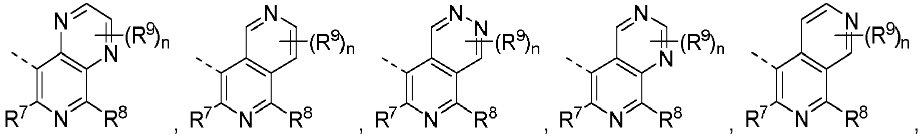

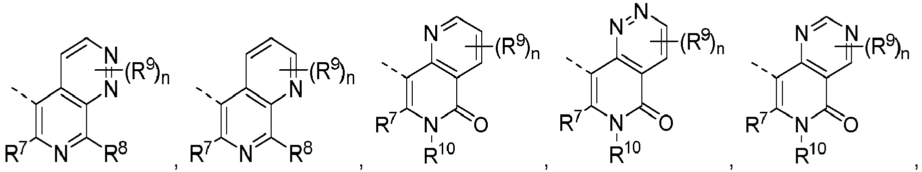

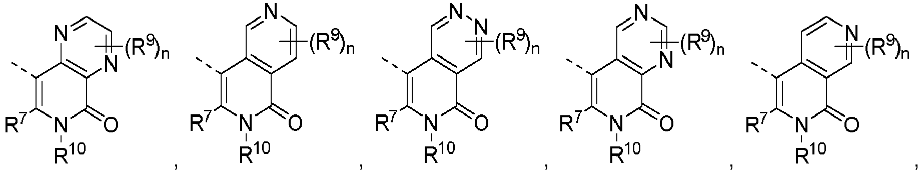

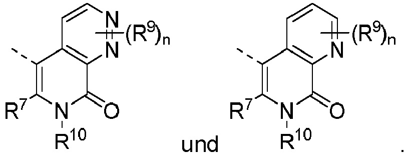

10. Die Verbindung nach Anspruch 1, die ausgewählt ist aus der Gruppe bestehend aus:
3-(3-Chlor-4-fluorphenyl)-1-methyl-1-((1-oxo-1,2-dihydroisochinolin-4-yl)methyl)harnstoff;
3-(3-Chlor-4-fluorphenyl)-1-((1-methoxyisochinolin-4-yl)methyl)-1-methylharnstoff;
3-(4-Fluor-3-methylphenyl)-1-((1-methoxyisochinolin-4-yl)methyl)-1-methylharnstoff;
3-(4-Fluor-3-methylphenyl)-1-methyl-1-((1-oxo-1,2-dihydroisochinolin-4-yl)methyl)harnstoff;
3-(3-Chlor-4-fluorphenyl)-1-ethyl-1-((1-oxo-1,2-dihydroisochinolin-4-yl)methyl)harnstoff;
3-(3-Chlor-4-fluorphenyl)-1-methyl-1-(1-(1-oxo-1,2-dihydroisochinolin-4-yl)ethyl)harnstoff;
3-(3-Chlor-4-fluorphenyl)-1-ethyl-1-((1-methoxyisochinolin-4-yl)methyl)harnstoff;
3-(3-Chlor-4-fluorphenyl)-1-ethyl-1-(isochinolin-4-ylmethyl)harnstoff;
3-(3-Chlor-4-fluorphenyl)-1-((1-ethoxyisochinolin-4-yl)methyl)-1-ethylhamstoff;
3-(3-Chlor-4-fluorphenyl)-1-ethyl-1-((2-methyl-1-oxo-1,2-dihydroisochinolin-4-yl)methyl)harnstoff;
3-(3-Chlor-4-fluorphenyl)-1-isopropyl-1-((1-methoxyisochinolin-4-yl)methyl)harnstoff;
3-(3-Chlor-4-fluorphenyl)-1-((1-methoxyisochinolin-4-yl)methyl)-1-propylharnstoff;
3-(3-Chlor-4-fluorphenyl)-1-(2-hydroxyethyl)-1-((1-methoxyisochinolin-4-yl)methyl)harnstoff;
3-(3-Chlor-4-fluorphenyl)-1-ethyl-1-(1-(1-methoxyisochinolin-4-yl)ethyl)harnstoff;
3-(3-Chlor-4-fluorphenyl)-1-ethyl-1-(1-(1-oxo-1,2-dihydroisochinolin-4-yl)ethyl)harnstoff;
3-(3-Chlor-4-fluorphenyl)-1-isopropyl-1-((1-oxo-1,2-dihydroisochinolin-4-yl)methyl)harnstoff;
3-(3-Chlor-4-fluorphenyl)-1-(2-hydroxyethyl)-1-((1-oxo-1,2-dihydroisochinolin-4-yl)methyl)harnstoff;
3-(3-Chlor-4-fluorphenyl)-1-((1-oxo-1,2-dihydroisochinolin-4-yl)methyl)-1-propylharnstoff;
3-(3-Chlor-4-fluorphenyl)-1-ethyl-1-((1-(2-hydroxyethoxy)isochinolin-4-yl)methyl)harnstoff;
3-(3-Chlor-4-fluorphenyl)-1-(1-(1-methoxyisochinolin-4-yl)ethyl)-1-methylharnstoff;
3-(3-Chlor-4-fluorphenyl)-1-(cyclopropylmethyl)-1-((1-methoxyisochinolin-4-yl)methyl)harnstoff;
3-(4-Fluor-3-methylphenyl)-1-methyl-1-(1-(1-oxo-1,2-dihydroisochinolin-4-yl)ethyl)harnstoff;
1-Butyl-3-(3-chlor-4-fluorphenyl)-1-((1-methoxyisochinolin-4-yl)methyl)harnstoff;
3-(3-Chlor-4-fluorphenyl)-1-cyclopropyl-1-((1-methoxyisochinolin-4-yl)methyl)harnstoff;
(3-(3-Chlor-4-fluorphenyl)-1-(1-(1-oxo-1,2-dihydroisochinolin-4-yl)ethyl)-1-propylharnstoff;
3-(3-Chlor-4-fluorphenyl)-1-ethyl-1-(1-(1-methoxyisochinolin-4-yl)propyl)harnstoff
3-(3-Chlor-4-fluorphenyl)-1-ethyl-1-(1-(1-oxo-1,2-dihydroisochinolin-4-yl)propyl)harnstoff;
3-(3-Chlor-4-fluorphenyl)-1-(cyclopropyl(1-methoxyisochinolin-4-yl)methyl)-1-ethylharnstoff;
3-(3-Chlor-4-fluorphenyl)-1-(2-methoxyethyl)-1-((1-methoxyisochinolin-4-yl)methyl)harnstoff;
3-(3-Chlor-4-fluorphenyl)-1-cyclopropyl-1-((1-oxo-1,2-dihydroisochinolin-4-yl)methyl)harnstoff;
3-(4-Fluor-3-methylphenyl)-1-(1-(1-oxo-1,2-dihydroisochinolin-4-yl)ethyl)-1-propylharnstoff;
3-(3-Chlor-4-fluorphenyl)-1-ethyl-1-(1-(1-methoxyisochinolin-4-yl)-2-methylpropyl)harnstoff;
3-(3-Chlor-4-fluorphenyl)-1-(cyclopropyl(1-oxo-1,2-dihydroisochinolin-4-yl)methyl)-1-ethylharnstoff;
3-(3-Chlor-4-fluorphenyl)-1-((1-methoxyisochinolin-4-yl)methyl)-1-(3-methoxypropyl)harnstoff;
1-Benzyl-3-(3-chlor-4-fluorphenyl)-1-((1-methoxyisochinolin-4-yl)methyl)harnstoff;
3-(3-Chlor-4-fluorphenyl)-1-(2-methoxyethyl)-1-((1-oxo-1,2-dihydroisochinolin-4-yl)methyl)harnstoff;
3-(3-Chlor-4-fluorphenyl)-1-(3-methoxypropyl)-1-((1-oxo-1,2-dihydroisochinolin-4-yl)methyl)harnstoff;
1-Butyl-3-(3-chlor-4-fluorphenyl)-1-((1-oxo-1,2-dihydroisochinolin-4-yl)methyl)harnstoff;
3-(3-Chlor-4-fluorphenyl)-1-(cyclopropylmethyl)-1-((1-oxo-1,2-dihydroisochinolin-4-yl)methyl)harnstoff;
3-(3-Chlor-4-fluorphenyl)-1-ethyl-1-(2-methyl-1-(1-oxo-1,2-dihydroisochinolin-4-yl)propyl)harnstoff;
3-(3-Chlor-4-fluorphenyl)-1-cyclopropyl-1-(1-(1-methoxyisochinolin-4-yl)ethyl)harnstoff;
3-(3-Chlor-4-fluorphenyl)-1-ethyl-1-((1-methoxyisochinolin-4-yl)(phenyl)methyl)hamstoff;
1-Butyl-3-(3-chlor-4-fluorphenyl)-1-(1-(1-methoxyisochinolin-4-yl)ethyl)harnstoff;
3-(3-Chlor-4-fluorphenyl)-1-(cyclopropylmethyl)-1-(1-(1-methoxyisochinolin-4-yl)ethyl)harnstoff;
3-(3-Chlor-4-fluorphenyl)-1-(2-methoxyethyl)-1-(1-(1-oxo-1,2-dihydroisochinolin-4-yl)ethyl)harnstoff;
3-(3-Chlor-4-fluorphenyl)-1-cyclopropyl-1-(1-(1-oxo-1,2-dihydroisochinolin-4-yl)ethyl)harnstoff;
3-(3-Chlor-4-fluorphenyl)-1-ethyl-1-((1-oxo-1,2-dihydroisochinolin-4-yl)(phenyl)methyl)harnstoff;
3-(3-Chlor-4-fluorphenyl)-1-(2-methoxyethyl)-1-(1-(1-methoxyisochinolin-4-yl)ethyl)harnstoff;
3-(3-Chlor-4-fluorphenyl)-1-ethyl-1-(2,2,2-trifluor-1-(1-oxo-1,2-dihydroisochinolin-4-yl)ethyl)harnstoff;
3-(3-Chlor-4-fluorphenyl)-1-ethyl-1-(2,2,2-trifluor-1-(1-methoxyisochinolin-4-yl)ethyl)harnstoff;
3-(3-Chlor-4-fluorphenyl)-1-(3-methoxypropyl)-1-(1-(1-oxo-1,2-dihydroisochinolin-4-yl)ethyl)harnstoff;
3-(3-Chlor-4-fluorphenyl)-1-(1-(1-methoxyisochinolin-4-yl)ethyl)-1-(3-methoxypropyl)harnstoff;
1-Butyl-3-(3-chlor-4-fluorphenyl)-1-(1-(1-oxo-1,2-dihydroisochinolin-4-yl)ethyl)harnstoff;
3-(3-Chlor-4-fluorphenyl)-1-(cyclopropylmethyl)-1-(1-(1-oxo-1,2-dihydroisochinolin-4-yl)ethyl)harnstoff;
3-(3-Chlor-4-fluorphenyl)-1-methyl-1-((1-((1-methyl-1H-1,2,4-triazol-3-yl)methoxy)isochinolin-4-yl)methyl)hamstoff;
3-(3-Chlor-4-fluorphenyl)-1-methyl-1-((1-(pyridin-2-ylmethoxy)isochinolin-4-yl)methyl)harnstoff;
3-(4-Fluor-3-(trifluormethyl)phenyl)-1-methyl-1-(1-(1-oxo-1,2-dihydroisochinolin-4-yl)ethyl)harnstoff;
3-(4-Fluorphenyl)-1-methyl-1-(1-(1-oxo-1,2-dihydroisochinolin-4-yl)ethyl)harnstoff;
1-Methyl-1-(1-(1-oxo-1,2-dihydroisochinolin-4-yl)ethyl)-3-(3,4,5-trifluorphenyl)harnstoff;
3-(3,4-Difluorphenyl)-1-methyl-1-(1-(1-oxo-1,2-dihydroisochinolin-4-yl)ethyl)harnstoff;
3-(3-Fluorphenyl)-1-methyl-1-(1-(1-oxo-1,2-dihydroisochinolin-4-yl)ethyl)harnstoff;
3-(2-Chlorpyridin-4-yl)-1-methyl-1-(1-(1-oxo-1,2-dihydroisochinolin-4-yl)ethyl)harnstoff;
1-Methyl-1-(1-(1-oxo-1,2-dihydroisochinolin-4-yl)ethyl)-3-(2-(trifluormethyl)pyridin-4-yl)harnstoff;
3-(3-Chlor-4-fluorphenyl)-1-(2,2-difluorethyl)-1-(1-(1-oxo-1,2-dihydroisochinolin-4-yl)ethyl)harnstoff;
1-(2,2-Difluorethyl)-3-(4-fluor-3-methylphenyl)-1-(1-(1-oxo-1,2-dihydroisochinolin-4-yl)ethyl)harnstoff;
3-(3-Cyano-4-fluorphenyl)-1-methyl-1-(1-(1-oxo-1,2-dihydroisochinolin-4-yl)ethyl)harnstoff;
3-(3-Chlor-4-fluorphenyl)-1-(ethyl-d5)-1-(1-(1-oxo-1,2-dihydroisochinolin-4-yl)ethyl)harnstoff;
N-(2-(3-(3-Chlor-4-fluorphenyl)-1-(1-(1-oxo-1,2-dihydroisochinolin-4-yl)ethyl)ureido)ethyl)acetamid;
(3-(3-Chlor-4-fluorphenyl)-1-(1-(1-methoxyisochinolin-4-yl)ethyl)-1-(2,2,2-trifluorethyl)harnstoff;
3-(3-Chlor-4-fluorphenyl)-1-ethyl-1-(1-(1-oxo-1,2-dihydro-2,7-naphthyridin-4-yl)ethyl)harnstoff;
3-(3-Chlor-4-fluorphenyl)-1-(2-cyanoethyl)-1-(1-(1-oxo-1,2-dihydroisochinolin-4-yl)ethyl)harnstoff;
3-(3-(3-Chlor-4-fluorphenyl)-1-(1-(1-oxo-1,2-dihydroisochinolin-4-yl)ethyl)ureido)-N-methylpropanamid;
3-(3-(3-Chlor-4-fluorphenyl)-1-(1-(1-oxo-1,2-dihydroisochinolin-4-yl)ethyl)ureido)-N,N-dimethylpropanamid;
3-(3-Chlor-4-fluorphenyl)-1-(2-(methylsulfonyl)ethyl)-1-(1-(1-oxo-1,2-dihydroisochinolin-4-yl)ethyl)harnstoff;
3-(3-Chlor-4-fluorphenyl)-1-(2-ethoxyethyl)-1-(1-(1-oxo-1,2-dihydroisochinolin-4-yl)ethyl)harnstoff;
3-(3-Chlor-4-fluorphenyl)-1-methyl-1-(1-(1-oxo-1,2-dihydro-2,7-naphthyridin-4-yl)ethyl)harnstoff;
3-(3-Chlor-4-fluorphenyl)-1-(3-cyanopropyl)-1-(1-(1-oxo-1,2-dihydroisochinolin-4-yl)ethyl)hamstoff;
2-(3-(3-Chlor-4-fluorphenyl)-1-(1-(1-oxo-1,2-dihydroisochinolin-4-yl)ethyl)ureido)-N-methyla-cetamid;
2-(3-(3-Chlor-4-fluorphenyl)-1-(1-(1-oxo-1,2-dihydroisochinolin-4-yl)ethyl)ureido)ethane-1-sulfonamid;
3-(3-Chlor-4-fluorphenyl)-1-(2-(2-methoxyethoxy)ethyl)-1-(1-(1-methoxyisochinolin-4-yl)ethyl)harnstoff;
3-(3-Chlor-4-fluorphenyl)-1-(2-(2-methoxyethoxy)ethyl)-1-(1-(1-oxo-1,2-dihydroisochinolin-4-yl)ethyl)harnstoff;
3-(3-Chlor-4-fluorphenyl)-1-(2-ethoxyethyl)-1-(1-(1-methoxyisochinolin-4-yl)ethyl)harnstoff;
3-(3-Chlor-4-fluorphenyl)-1-methyl-1-(1-(1-oxo-1,2-dihydro-2,6-naphthyridin-4-yl)ethyl)harnstoff;
2-(3-(3-Chlor-4-fluorphenyl)-1-(1-(1-oxo-1,2-dihydroisochinolin-4-yl)ethyl)ureido)acetamid;
3-(3-Chlor-4-fluorphenyl)-1-(3-hydroxypropyl)-1-(1-(1-oxo-1,2-dihydroisochinolin-4-yl)ethyl)harnstoff;
3-(3-Chlor-4-fluorphenyl)-1-methyl-1-(1-(1-((1-methyl-1H-1,2,4-triazol-3-yl)methoxy)isochinolin-4-yl)ethyl)harnstoff;
3-(3-Chlor-4-fluorphenyl)-1-(1-(1-oxo-1,2-dihydroisochinolin-4-yl)ethyl)-1-((tetrahydro-2H-pyran-4-yl)methyl)harnstoff;
3-(3-Chlor-4-fluorphenyl)-1-isobutyl-1-(1-(1-oxo-1,2-dihydroisochinolin-4-yl)ethyl)harnstoff;
3-(3-Chlor-4-fluorphenyl)-1-methyl-1-(1-(5-(methylamino)pyrido[3,4-b]pyrazin-8-yl)ethyl)harnstoff;
3-(3-Chlor-4-fluorphenyl)-1-(1-(5-methoxypyrido[3,4-b]pyrazin-8-yl)ethyl)-1-methylharnstoff;
3-(3-Chlor-4-fluorphenyl)-1-(3-hydroxypropyl)-1-(1-(1-methoxyisochinolin-4-yl)ethyl)harnstoff;
1- (1-(1-((1H-1,2,4-Triazol-3-yl)methoxy)isochinolin-4-yl)ethyl)-3-(3-chlor-4-fluorphenyl)-1-methylharnstoff;
2-(3-(3-Chlor-4-fluorphenyl)-1-(1-(1-oxo-1,2-dihydroisochinolin-4-yl)ethyl)ureido)-N, N-dimethylacetamid;
3-(3-Chlor-4-fluorphenyl)-1-methyl-1-(1-(5-oxo-5,6-dihydropyrido[3,4-b]pyrazin-8-yl)ethyl)harnstoff;
3-(3-Chlor-4-fluorphenyl)-1-(4-hydroxybutyl)-1-(1-(1-oxo-1,2-dihydroisochinolin-4-yl)ethyl)harnstoff;
3-(3-Chlor-4-fluorphenyl)-1-methyl-1-(1-(8-oxo-7,8-dihydro-1,7-naphthyridin-5-yl)ethyl)harnstoff;
3-(3-Chlor-4-fluorphenyl)-1-isobutyl-1-(1-(1-oxo-1,2-dihydro-2,7-naphthyridin-4-yl)ethyl)harnstoff;
3-(3-Chlor-4-fluorphenyl)-1-(1-(1-oxo-1,2-dihydroisochinolin-4-yl)ethyl)-1-(((R)-tetrahydrofuran-2-yl)methyl)hamstoff;
1-(3-Aminopropyl)-3-(3-chlor-4-fluorphenyl)-1-(1-(1-oxo-1,2-dihydroisochinolin-4-yl)ethyl)hamstoff;
3-(3-Chlor-4-fluorphenyl)-1-(1-(1-methoxyisochinolin-4-yl)ethyl)-1-(2-((2,2,2-trifluorethyl)amino)ethyl)harnstoff;
3-(3-Chlor-4-fluorphenyl)-1-(3-hydroxypropyl)-1-(1-(1-oxo-1,2-dihydro-2,7-naphthyridin-4-yl)ethyl)harnstoff;
3-(3-Chlor-4-fluorphenyl)-1-(1-(1-oxo-1,2-dihydroisochinolin-4-yl)ethyl)-1-(((S)-tetrahydrofuran-2-yl)methyl)hamstoff;
3-(3-Chlor-4-fluorphenyl)-1-(1-(1-oxo-1,2-dihydroisochinolin-4-yl)ethyl)-1-(2-((2,2,2-trifluorethyl)amino)ethyl)harnstoff;
3-(3-Chlor-4-fluorphenyl)-1-(1-(1-oxo-1,2-dihydroisochinolin-4-yl)ethyl)-1-((tetrahydrofuran-3-yl)methyl)hamstoff;
1-(1-(8-Chlor-1-oxo-1,2-dihydroisochinolin-4-yl)ethyl)-3-(3-chlor-4-fluorphenyl)-1-methylharnstoff;
3-(3-Chlor-4-fluorphenyl)-1-(1-(6-fluor-1-oxo-1,2-dihydroisochinolin-4-yl)ethyl)-1-methylharnstoff;
3-(3-Chlor-4-fluorphenyl)-1-(1-(6-chlor-1-oxo-1,2-dihydroisochinolin-4-yl)ethyl)-1-methylharnstoff;
3-(3-Chlor-4-fluorphenyl)-1-(1-(7-methoxy-1-oxo-1,2-dihydroisochinolin-4-yl)ethyl)-1-methylharnstoff;
3-(3-Chlor-4-fluorphenyl)-1-(1-(6-methoxy-1-oxo-1,2-dihydroisochinolin-4-yl)ethyl)-1-methylharnstoff;
3-(3-Chlor-4-fluorphenyl)-1-methyl-1-(1-(2-methyl-1-oxo-1,2-dihydroisochinolin-4-yl)ethyl)hamstoff;
3-(3-Chlor-4-fluorphenyl)-1-methyl-1-(1-(2-ethyl-1-oxo-1,2-dihydroisochinolin-4-yl)ethyl)harnstoff;
3-(3-Chlor-4-fluorphenyl)-1-methyl-1-(1-(2-propyl-1-oxo-1,2-dihydroisochinolin-4-yl)ethyl)hamstoff;
3-(3-Chlor-4-fluorphenyl)-1-methyl-1-(1-(2-isopropyl-1-oxo-1,2-dihydroisochinolin-4-yl)ethyl)harnstoff;
3-(3-Chlor-4-fluorphenyl)-1-methyl-1-(1-(2-cyclopropyl-1-oxo-1,2-dihydroisochinolin-4-yl)ethyl)harnstoff;
3-(3-Chlor-4-fluorphenyl)-1-(1-(2-(2-methoxyethyl)-1-oxo-1,2-dihydroisochinolin-4-yl)ethyl)-1-methylharnstoff;
3-(3-Chlor-4-fluorphenyl)-1-(cyclopentylmethyl)-1-(1-(1-oxo-1,2-dihydroisochinolin-4-yl)ethyl)harnstoff;
3-(3-Chlor-4-fluorphenyl)-1-(cyclohexylmethyl)-1-(1-(1-oxo-1,2-dihydroisochinolin-4-yl)ethyl)harnstoff;
3-(3-Chlor-4-fluorphenyl)-1-(2-hydroxyethyl)-1-(1-(1-oxo-1,2-dihydroisochinolin-4-yl)ethyl)harnstoff;
3-(3-Chlor-4-fluorphenyl)-1-(2-((2,2-difluorethyl)amino)ethyl)-1-(1-(1-oxo-1,2-dihydroisochinolin-4-yl)ethyl)harnstoff;
3-(3-Chlor-4-fluorphenyl)-1-(1-(2-(3-methoxypropyl)-1-oxo-1,2-dihydroisochinolin-4-yl)ethyl)-1-methylharnstoff;
1-((1-Acetylpiperidin-4-yl)methyl)-3-(3-chlor-4-fluorphenyl)-1-(1-(1-oxo-1,2-dihydroisochinolin-4-yl)ethyl)harnstoff;
3-(3-Chlor-4-fluorphenyl)-1-((1-(methylsulfonyl)piperidin-4-yl)methyl)-1-(1-(1-oxo-1,2-dihydroisochinolin-4-yl)ethyl)harnstoff;
3-(3-Chlor-4-fluorphenyl)-1-(1-(2-(2-hydroxyethyl)-1-oxo-1,2-dihydroisochinolin-4-yl)ethyl)-1-methylharnstoff;
3-(3-Chlor-4-fluorphenyl)-1-(1-(2-(3-hydroxypropyl)-1-oxo-1,2-dihydroisochinolin-4-yl)ethyl)-1-methylharnstoff;
3-(3-Chlor-4-fluorphenyl)-1-((1-methylpiperidin-4-yl)methyl)-1-(1-(1-oxo-1,2-dihydroisochinolin-4-yl)ethyl)harnstoff;
3-(3-(3-Chlor-4-fluorphenyl)-1-(1-(1-oxo-1,2-dihydroisochinolin-4-yl)ethyl)ureido)propansäure;
3-(3-Chlor-4-fluorphenyl)-1-(3-hydroxy-2,2-dimethylpropyl)-1-(1-(1-oxo-1,2-dihydroisochinolin-4-yl)ethyl)harnstoff;
3-(3-Chlor-4-fluorphenyl)-1-((R)-3-hydroxybutyl)-1-(1-(1-oxo-1,2-dihydroisochinolin-4-yl)ethyl)harnstoff;
3-(3-Chlor-4-fluorphenyl)-1-((S)-3-hydroxybutyl)-1-(1-(1-oxo-1,2-dihydroisochinolin-4-yl)ethyl)harnstoff;
3-(3-Chlor-4-fluorphenyl)-1-((R)-2-hydroxypropyl)-1-(1-(1-oxo-1,2-dihydroisochinolin-4-yl)ethyl)harnstoff;
3-(3-Chlor-4-fluorphenyl)-1-((S)-2-hydroxypropyl)-1-(1-(1-oxo-1,2-dihydroisochinolin-4-yl)ethyl)harnstoff;
3-(3-(3-Chlor-4-fluorphenyl)-1-(1-(1-oxo-1,2-dihydroisochinolin-4-yl)ethyl)ureido)-N-methylpropane-1-sulfonamid;
3-(3-Chlor-4-fluorphenyl)-1-(((1r,4r)-4-hydroxycyclohexyl)methyl)-1-(1-(1-oxo-1,2-dihydroisochinolin-4-yl)ethyl)harnstoff;
3-(3-Chlor-4-fluorphenyl)-1-methyl-1-(1-(3-methyl-1-oxo-1,2-dihydroisochinolin-4-yl)ethyl)hamstoff;
3-(3-Chlor-4-fluorphenyl)-1-((4-cis-hydroxycyclohexyl)methyl)-1-(1-(1-oxo-1,2-dihydroisochinolin-4-yl)ethyl)harnstoff;
4-(3-(3-Chlor-4-fluorphenyl)-1-(1-(1-oxo-1,2-dihydroisochinolin-4-yl)ethyl)ureido)buttersäure;
3-(3-Chlor-4-fluorphenyl)-1-methyl-1-(1-(1-oxo-2-(2,2,2-trifluorethyl)-1,2-dihydroisochinolin-4-yl)ethyl)harnstoff;
3-(3-Chlor-4-fluorphenyl)-1-isobutyl-1-(1-(1-oxo-2-(2,2,2-trifluorethyl)-1,2-dihydroisochinolin-4-yl)ethyl)harnstoff;
3-(4-Fluor-3-methylphenyl)-1-methyl-1-(1-(3-methyl-1-oxo-1,2-dihydroisochinolin-4-yl)ethyl)harnstoff;
3-(3-Chlor-4-fluorphenyl)-1-methyl-1-(1-(1-(methylamino)isochinolin-4-yl)ethyl)harnstoff;
3-(3-Chlor-4-fluorphenyl)-1-methyl-1-(1-(1-(dimethylamino)isochinolin-4-yl)ethyl)harnstoff;
3-(4-Fluorphenyl)-1-isobutyl-1-(1-(1-oxo-2-(2,2,2-trifluorethyl)-1,2-dihydroisochinolin-4-yl)ethyl)harnstoff;
3-(4-Fluor-3-methylphenyl)-1-methyl-1-(1-(3-methyl-1-oxo-1,2-dihydroisochinolin-4-yl)ethyl)harnstoff;
1-(1-(1-Aminoisochinolin-4-yl)ethyl)-3-(3-chlor-4-fluorphenyl)-1-methylharnstoff;
3-(3-Chlor-4-Fluorphenyl)-1-(1-(1-(ethylamino)isochinolin-4-yl)ethyl)-1-methylharnstoff;
3-(3-Chlor-4-Fluorphenyl)-1-(1-(1-((2-hydroxyethyl)amino)isochinolin-4-yl)ethyl)-1-methylharnstoff;
1-(1-(1-((2-Aminoethyl)amino)isochinolin-4-yl)ethyl)-3-(3-chlor-4-fluorphenyl)-1-methylharnstoff;
3-(3-Chlor-4-fluorphenyl)-1-methyl-1-(1-(1-(((1-methyl-1H-1,2,4-triazol-3-yl)methyl)amino)isochinolin-4-yl)ethyl)harnstoff;
1-(1-(1-(((1H-1,2,4-Triazol-3-yl)methyl)amino)isochinolin-4-yl)ethyl)-3-(3-chlor-4-fluorphenyl)-1-methylharnstoff;
3-(3-Chlor-4-fluorphenyl)-1-(3-hydroxypropyl)-1-(1-(1-((1-methyl-1H-1,2,4-triazol-3-yl)methoxy)isochinolin-4-yl)ethyl)harnstoff;
3-(3-Chlor-4-fluorphenyl)-1-(1-(7-fluor-1-oxo-1,2-dihydroisochinolin-4-yl)ethyl)-1-methylharnstoff;
1-(1-(7-Fluor-1-oxo-1,2-dihydroisochinolin-4-yl)ethyl)-3-(4-fluorphenyl)-1-methylharnstoff;
1-(1-(1-((1H-1,2,4-triazol-3-yl)methoxy)isochinolin-4-yl)ethyl)-3-(3-chlor-4-fluorphenyl)-1-(3-hydroxypropyl)harnstoff;
1-(1-(1-(((2H-1,2,3-Triazol-4-yl)methyl)amino)isochinolin-4-yl)ethyl)-3-(3-chlor-4-fluorphenyl)-1-methylharnstoff;
3-(3-Chlor-4-fluorphenyl)-1-(1-(5-methoxy-1-oxo-1,2-dihydroisochinolin-4-yl)ethyl)-1-methylharnstoff;
1-((1H-1,2,3-Triazol-4-yl)methyl)-3-(3-chlor-4-fluorphenyl)-1-(1-(1-oxo-1,2-dihydroisochinolin-4-yl)ethyl)harnstoff;
1-((4H-1,2,4-Triazol-3-yl)methyl)-3-(3-chlor-4-fluorphenyl)-1-(1-(1-oxo-1,2-dihydroisochinolin-4-yl)ethyl)harnstoff;
1-(1-(2-((1H-1,2,4-Triazol-3-yl)methyl)-1-oxo-1,2-dihydroisochinolin-4-yl)ethyl)-3-(3-chlor-4-fluorphenyl)-1-methylharnstoff;
1-(1-(7-Chlor-1-oxo-1,2-dihydroisochinolin-4-yl)ethyl)-3-(3-chlor-4-fluorphenyl)-1-methylharnstoff;
3-(3-Chlor-4-fluorphenyl)-1-((2,2-dimethyl-1,3-dioxan-5-yl)methyl)-1-(1-(1-oxo-1,2-dihydroisochinolin-4-yl)ethyl)harnstoff;
1-((2,2-Dimethyl-1,3-dioxan-5-yl)methyl)-3-(4-fluorphenyl)-1-(1-(1-oxo-1,2-dihydroisochinolin-4-yl)ethyl)harnstoff;
1-((2,2-Dimethyl-1,3-dioxan-5-yl)methyl)-1-(1-(1-oxo-1,2-dihydroisochinolin-4-yl)ethyl)-3-phenylharnstoff;
1-(1-(2-((1H-1,2,3-Triazol-4-yl)methyl)-1-oxo-1,2-dihydroisochinolin-4-yl)ethyl)-3-(3-chlor-4-fluorphenyl)-1-methylharnstoff;
3-(3-Chlor-4-fluorphenyl)-1-(1-(6,7-difluor-1-oxo-1,2-dihydroisochinolin-4-yl)ethyl)-1-methylharnstoff;
3-(3-Chlor-4-fluorphenyl)-1-(1-(6,7-difluor-1-oxo-1,2-dihydroisochinolin-4-yl)ethyl)-1-isobutylharnstoff;
3-(3-Chlor-4-fluorphenyl)-1-((2,2-dimethyl-1,3-dioxan-5-yl)methyl)-1-(1-(6-fluor-1-oxo-1,2-dihydroisochinolin-4-yl)ethyl)harnstoff;
3-(3-Chlor-4-fluorphenyl)-1-(3-hydroxy-2-(hydroxymethyl)propyl)-1-(1-(1-oxo-1,2-dihydroisochinolin-4-yl)ethyl)harnstoff;
1-(1-(6,7-Difluor-1-oxo-1,2-dihydroisochinolin-4-yl)ethyl)-3-(4-fluorphenyl)-1-methylharnstoff;
1-(1-(6,7-Difluor-1-oxo-1,2-dihydroisochinolin-4-yl)ethyl)-3-(4-fluorphenyl)-1-isobutylharnstoff;
3-(3-Chlor-4-fluorphenyl)-1-(1-(6,7-difluor-1-oxo-1,2-dihydroisochinolin-4-yl)ethyl)-1-(3-hydroxypropyl)harnstoff;
1-(1-(6,7-Difluor-1-oxo-1,2-dihydroisochinolin-4-yl)ethyl)-3-(4-fluorphenyl)-1-(3-hydroxypropyl)harnstoff;
3-(4-Chlorphenyl)-1-methyl-1-(1-(1-oxo-1,2-dihydroisochinolin-4-yl)ethyl)harnstoff;
3-(4-Bromphenyl)-1-methyl-1-(1-(1-oxo-1,2-dihydroisochinolin-4-yl)ethyl)harnstoff;
3-(3-Chlor-4-fluorphenyl)-1-(1-(6-fluor-1-oxo-1,2-dihydroisochinolin-4-yl)ethyl)-1-(3-hydroxy-2-(hydroxymethyl) propyl)harnstoff;
1-(1-(1-(1H-1,2,4-Triazol-1-yl)isochinolin-4-yl)ethyl)-3-(3-chlor-4-fluorphenyl)-1-methylharnstoff;
3-Cyclopentyl-1-methyl-1-(1-(1-oxo-1,2-dihydroisochinolin-4-yl)ethyl)harnstoff;
1-(1-(1-(1H-1,2,4-Triazol-1-yl)isochinolin-4-yl)ethyl)-3-(3-chlor-4-fluorphenyl)-1-isobutylharnstoff;
1-(1-(1-(1H-1,2,4-Triazol-1-yl)isochinolin-4-yl)ethyl)-3-(4-fluorphenyl)-1-isobutylharnstoff;
3-(3-Chlor-4-fluorphenyl)-1-(1-(7-fluor-2-methyl-1-oxo-1,2-dihydroisochinolin-4-yl)ethyl)-1-methylharnstoff;
3-(3-Chlor-4-fluorphenyl)-1-(1-(1-oxo-1,2-dihydroisochinolin-4-yl)ethyl)-1-(pyridin-2-ylmethyl)harnstoff;
3-(3-Chlor-4-fluorphenyl)-1-(1-(1-oxo-1,2-dihydroisochinolin-4-yl)ethyl)-1-(pyridin-3-ylmethyl)harnstoff;
3-(3-Chlor-4-fluorphenyl)-1-(1-(1-oxo-1,2-dihydroisochinolin-4-yl)ethyl)-1-(pyridin-4-ylmethyl)harnstoff;
3-(3-Chlor-4-fluorphenyl)-1-(1-(1-oxo-1,2-dihydroisochinolin-4-yl)ethyl)-1-(thiazol-2-ylmethyl)harnstoff;
3-(3-Chlor-4-fluorphenyl)-1-(1-(1-oxo-1,2-dihydroisochinolin-4-yl)ethyl)-1-(thiazol-4-ylmethyl)harnstoff;
3-(3-Chlor-4-fluorphenyl)-1-methyl-1-(1-(1-(methylsulfonyl)isochinolin-4-yl)ethyl)harnstoff;
4-(1-(3-(3-Chlor-4-fluorphenyl)-1-methylureido)ethyl)-N-methylisochinolin-1-carboxamid;
4-(1-(3-(3-Chlor-4-fluorphenyl)-1-methylureido)ethyl)-N,N-dimethylisochinolin-1-carboxamid;
3-(3-Chlor-4-fluorphenyl)-1-(1-(1-oxo-1,2-dihydroisochinolin-4-yl)ethyl)-1-(pyrimidin-5-ylmethyl)harnstoff;
3-(3-Chlor-4-fluorphenyl)-1-(1-(1-oxo-1,2-dihydroisochinolin-4-yl)ethyl)-1-(pyrimidin-4-ylmethyl)harnstoff;
3-(3-Chlor-4-fluorphenyl)-1-(1-(1-oxo-1,2-dihydroisochinolin-4-yl)ethyl)-1-(thiazol-5-ylmethyl)harnstoff;
4-(1-(3-(3-Chlor-4-fluorphenyl)-1-methylureido)ethyl)isochinolin-1-carbonsäure;
3-(3-Chlor-4-fluorphenyl)-1-(1-(1-(hydroxymethyl)isochinolin-4-yl)ethyl)-1-methylharnstoff;
4-(1-(3-(3-Chlor-4-fluorphenyl)-1-methylureido)ethyl)isochinolin-1-carboxamid;
3-(3-Chlor-4-fluorphenyl)-1-(1-(7,8-difluor-1-oxo-1,2-dihydroisochinolin-4-yl)ethyl)-1-methylharnstoff;
3-(3-Chlor-4-fluorphenyl)-1-(1-(7,8-difluor-2-methyl-1-oxo-1,2-dihydroisochinolin-4-yl)ethyl)-1-methylharnstoff;
3-(3-Chlor-4-fluorphenyl)-1-(1-(7,8-difluor-1-oxo-1,2-dihydroisochinolin-4-yl)ethyl)-1-(3-hydroxypropyl)harnstoff;
3-(3-Chlor-4-fluorphenyl)-1-(1-(1-cyanoisochinolin-4-yl)ethyl)-1-methylharnstoff;
3-(3-Chlor-4-fluorphenyl)-1-(1-(7,8-difluor-1-oxo-1,2-dihydroisochinolin-4-yl)ethyl)-1-isobutylharnstoff;
3-(3-Chlor-4-fluorphenyl)-1-(1-(6,7-difluor-2-methyl-1-oxo-1,2-dihydroisochinolin-4-yl)ethyl)-1-methylharnstoff;
3-(3-Chlor-4-fluorphenyl)-1-(1-(6,7-difluor-2-methyl-1-oxo-1,2-dihydroisochinolin-4-yl)ethyl)-1-isobutylharnstoff;
3-(3-Chlor-4-fluorphenyl)-1-(1-(6,7-difluor-1-((1-methyl-1H-1,2,4-triazol-3-yl)methoxy)isochinolin-4-yl)ethyl)-1-methylharnstoff;
3-(3-Chlor-4-fluorphenyl)-1-(1-(6,7-difluor-1-((1-methyl-1H-1,2,4-triazol-3-yl)methoxy)isochinolin-4-yl)ethyl)-1-(3-hydroxypropyl)harnstoff;
3-(3-Chlor-4-fluorphenyl)-1-(1-(6,7-difluor-2-methyl-1-oxo-1,2-dihydroisochinolin-4-yl)ethyl)-1-(3-hydroxypropyl)harnstoff;
3-(3-Chlor-4-fluorphenyl)-1-methyl-1-(1-(1-methylisochinolin-4-yl)ethyl)harnstoff;
1-(1-(1-(1H-1,2,3-triazol-1-yl)isochinolin-4-yl)ethyl)-3-(3-chlor-4-fluorphenyl)-1-methylharnstoff;
1-(1-(1-(1H-1,2,3-triazol-1-yl)isochinolin-4-yl)ethyl)-3-(3-chlor-4-fluorphenyl)-1-isobutylharnstoff;
3-(3-Chlor-4-fluorphenyl)-1-(1-(8-fluor-1-oxo-1,2-dihydroisochinolin-4-yl)ethyl)-1-methylharnstoff;
3-(3-Chlor-4-fluorphenyl)-1-(1-(8-fluor-2-methyl-1-oxo-1,2-dihydroisochinolin-4-yl)ethyl)-1-methylharnstoff;
3-(3-Chlor-4-fluorphenyl)-1-(1-(8-fluor-1-oxo-1,2-dihydroisochinolin-4-yl)ethyl)-1-isobutylharnstoff;
1-(1-(1-((1H-1,2,4-Triazol-3-yl)methoxy)-6,7-difluorisochinolin-4-yl)ethyl)-3-(3-chlor-4-fluorphenyl)-1-methylharnstoff;
1-(1-(1-((1H-1,2,4-Triazol-3-yl)methoxy)-6,7-difluorisochinolin-4-yl)ethyl)-3-(3-chlor-4-fluorphenyl)-1-(3-hydroxypropyl)harnstoff;
3-(3-Chlor-4-fluorphenyl)-1-(1-(8-fluor-1-oxo-1,2-dihydroisochinolin-4-yl)ethyl)-1-(3-hydroxypropyl)harnstoff;
3-(3-Chlor-4-fluorphenyl)-1-(1-(6,8-difluor-1-oxo-1,2-dihydroisochinolin-4-yl)ethyl)-1-methylharnstoff;
1-(1-(6,8-Difluor-1-oxo-1,2-dihydroisochinolin-4-yl)ethyl)-3-(4-fluorphenyl)-1-methylharnstoff;
3-(3-Chlor-4-fluorphenyl)-1-(1-(6,8-difluor-1-oxo-1,2-dihydroisochinolin-4-yl)ethyl)-1-(3-hydroxypropyl)harnstoff;
1-(1-(6,8-Difluor-1-oxo-1,2-dihydroisochinolin-4-yl)ethyl)-3-(4-fluorphenyl)-1-isobutylharnstoff;
1-(1-(6,8-Difluor-1-oxo-1,2-dihydroisochinolin-4-yl)ethyl)-3-(4-fluorphenyl)-1-(3-hydroxypropyl)harnstoff;
3-(3-Chlor-4-fluorphenyl)-1-(2-hydroxy-1-(1-oxo-1,2-dihydroisochinolin-4-yl)ethyl)-1-methylharnstoff;
2-(3-(3-Chlor-4-fluorphenyl)-1-(1-(6-fluor-1-oxo-1,2-dihydroisochinolin-4-yl)ethyl)ureido)ethane-1-sulfonamid;
2-(3-(3-Chlor-4-fluorphenyl)-1-(1-(7-fluor-1-oxo-1,2-dihydroisochinolin-4-yl)ethyl)ureido)ethane-1-sulfonamid;
2-(3-(3-Chlor-4-fluorphenyl)-1-(1-(6,7-difluor-1-oxo-1,2-dihydroisochinolin-4-yl)ethyl)ureido)ethane-1-sulfonamid;
2-(1-(1-(6,7-Difluor-1-oxo-1,2-dihydroisochinolin-4-yl)ethyl)-3-(4-fluorphenyl)ureido)ethane-1-sulfonamid;
2-(3-(3-Chlor-4-fluorphenyl)-1-(1-(6,8-difluor-1-oxo-1,2-dihydroisochinolin-4-yl)ethyl)ureido)ethane-1-sulfonamid;
2-(3-(3-Chlor-4-fluorphenyl)-1-(1-(7,8-difluor-1-oxo-1,2-dihydroisochinolin-4-yl)ethyl)ureido)ethane-1-sulfonamid;
2-(3-(4-Fluorphenyl)-1-(1-(7,8-difluor-1-oxo-1,2-dihydroisochinolin-4-yl)ethyl)ureido)ethane-1-sulfonamid;
2-(3-(3-Chlor-4-fluorphenyl)-1-(1-(8-fluor-1-oxo-1,2-dihydroisochinolin-4-yl)ethyl)ureido)ethane-1-sulfonamid;
1-(1-(6,7-Difluor-1-oxo-1,2-dihydroisochinolin-4-yl)ethyl)-1-methyl-3-phenylharnstoff;
3-(4-Chlorphenyl)-1-(1-(6,7-difluor-1-oxo-1,2-dihydroisochinolin-4-yl)ethyl)-1-methylharnstoff;
1-(1-(6,7-Difluor-1-oxo-1,2-dihydroisochinolin-4-yl)ethyl)-1-ethyl-3-(4-fluorphenyl)harnstoff;
1-(1-(6,7-Difluor-1-oxo-1,2-dihydroisochinolin-4-yl)ethyl)-1-ethyl-3-phenylharnstoff;
3-(3-Chlor-4-fluorphenyl)-1-(1-(6,7-difluor-1-oxo-1,2-dihydroisochinolin-4-yl)ethyl)-1-ethylharnstoff;
1-(1-(6,7-Difluor-1-oxo-1,2-dihydroisochinolin-4-yl)ethyl)-3-(3,4-difluorphenyl)-1-methylharnstoff;
1-(1-(6,7-Difluor-1-oxo-1,2-dihydroisochinolin-4-yl)ethyl)-3-(3-fluorphenyl)-1-methylharnstoff;
3-(3-Chlorphenyl)-1-(1-(6,7-difluor-1-oxo-1,2-dihydroisochinolin-4-yl)ethyl)-1-methylharnstoff;
1-(1-(6,7-Difluor-1-oxo-1,2-dihydroisochinolin-4-yl)ethyl)-1-methyl-3-(3,4,5-trifluorphenyl)harnstoff;
3-(3,5-Dichlorphenyl)-1-(1-(6,7-difluor-1-oxo-1,2-dihydroisochinolin-4-yl)ethyl)-1-methylharnstoff;
3-(2-Chlorpyridin-4-yl)-1-(1-(6,7-difluor-1-oxo-1,2-dihydroisochinolin-4-yl)ethyl)-1-methylharnstoff;
1-(1-(6,7-Difluor-1-oxo-1,2-dihydroisochinolin-4-yl)ethyl)-3-(2-fluorphenyl)-1-methylharnstoff;
1-(1-(6,7-Difluor-1-oxo-1,2-dihydroisochinolin-4-yl)ethyl)-3-(3,4-difluorphenyl)-1-ethylharnstoff;
1-(1-(6,7-Difluor-1-oxo-1,2-dihydroisochinolin-4-yl)ethyl)-3-(4-fluor-3-methylphenyl)-1-methylharnstoff;
3-(3-Cyano-4-fluorphenyl)-1-(1-(6,7-difluor-1-oxo-1,2-dihydroisochinolin-4-yl)ethyl)-1-methylhamstoff;
1-(1-(6,7-Difluor-1-oxo-1,2-dihydroisochinolin-4-yl)ethyl)-3-(2,3-difluorphenyl)-1-methylharnstoff;
1-(1-(7,8-Difluor-1-oxo-1,2-dihydroisochinolin-4-yl)ethyl)-3-(4-fluorphenyl)-1-methylharnstoff;
1-(1-(7,8-Difluor-1-oxo-1,2-dihydroisochinolin-4-yl)ethyl)-3-(3,4-difluorphenyl)-1-methylharnstoff;
1-(1-(7,8-Difluor-1-oxo-1,2-dihydroisochinolin-4-yl)ethyl)-1-methyl-3-phenylharnstoff;
3-(3,5-Dichlor-4-fluorphenyl)-1-(1-(6,7-difluor-1-oxo-1,2-dihydroisochinolin-4-yl)ethyl)-1-methylharnstoff;
1-(1-(7,8-Difluor-1-oxo-1,2-dihydroisochinolin-4-yl)ethyl)-3-(4-fluor-3-methylphenyl)-1-methylharnstoff;
1-(1-(7,8-Difluor-1-oxo-1,2-dihydroisochinolin-4-yl)ethyl)-1-methyl-3-(3,4,5-trifluorphenyl)harnstoff;
3-(4-Chlorphenyl)-1-(1-(7,8-difluor-1-oxo-1,2-dihydroisochinolin-4-yl)ethyl)-1-methylharnstoff;
3-(3-Chlor-4-fluorphenyl)-1-(1-(6,8-difluor-1-oxo-1,2-dihydroisochinolin-4-yl)ethyl)-1-isobutylharnstoff;
3-(3,5-Dichiorphenyl)-1-(1-(7,8-difluor-1-oxo-1,2-dihydroisochinolin-4-yl)ethyl)-1-methylharnstoff;
1-(1-(7,8-Difluor-1-oxo-1,2-dihydroisochinolin-4-yl)ethyl)-1-methyl-3-(pyridin-4-yl)harnstoff;
1-(1-(7,8-Difluor-1-oxo-1,2-dihydroisochinolin-4-yl)ethyl)-1-methyl-3-(pyridin-3-yl)harnstoff;
3-(3,5-Dichlor-4-fluorphenyl)-1-(1-(7,8-difluor-1-oxo-1,2-dihydroisochinolin-4-yl)ethyl)-1-methylharnstoff;
3-Benzyl-1-(1-(7,8-difluor-1-oxo-1,2-dihydroisochinolin-4-yl)ethyl)-1-methylharnstoff;
3-(2-Chlorpyridin-4-yl)-1-(1-(7,8-difluor-1-oxo-1,2-dihydroisochinolin-4-yl)ethyl)-1-methylharnstoff;
1-(1-(7,8-Difluor-1-oxo-1,2-dihydroisochinolin-4-yl)ethyl)-3-(3-fluorphenyl)-1-methylharnstoff;
1-(1-(7,8-Difluor-1-oxo-1,2-dihydroisochinolin-4-yl)ethyl)-3-(2-fluorphenyl)-1-methylharnstoff;
1-(1-(7,8-Difluor-1-oxo-1,2-dihydroisochinolin-4-yl)ethyl)-3-(2,3-difluorphenyl)-1-methylharnstoff;
1-(1-(7,8-Difluor-1-oxo-1,2-dihydroisochinolin-4-yl)ethyl)-3-(3-chlorphenyl)-1-methylharnstoff;
1-(1-(7,8-Difluor-1-oxo-1,2-dihydroisochinolin-4-yl)ethyl)-3-(3,4-difluorphenyl)-1-ethylharnstoff;
1-(1-(7,8-Difluor-1-oxo-1,2-dihydroisochinolin-4-yl)ethyl)-3-(4-fluorphenyl)-1-ethylharnstoff;
1-(1-(7,8-Difluor-1-oxo-1,2-dihydroisochinolin-4-yl)ethyl)-3-phenyl-1-ethylharnstoff;
1-(1-(7,8-Difluor-1-oxo-1,2-dihydroisochinolin-4-yl)ethyl)-3-(3-chlor-4-fluorphenyl)-1-ethylharnstoff;
3-(3-Cyano-4-fluorphenyl)-1-(1-(7,8-difluor-1-oxo-1,2-dihydroisochinolin-4-yl)ethyl)-1-methylhamstoff;
3-(3-Chlor-4-fluorphenyl)-1-(1-(6,7-difluor-1-oxo-1,2-dihydroisochinolin-4-yl)ethyl)-1-(methyl)harnstoff;
3-(3-Chlor-4-fluorphenyl)-1-(1-(6,7-difluor-1-oxo-1,2-dihydroisochinolin-4-yl)ethyl)-1-(methyld₃)harnstoff;
3-(3-Chlor-4-fluorphenyl)-1-(1-(7,8-difluor-1-oxo-1,2-dihydroisochinolin-4-yl)ethyl)-1-(methyl)harnstoff;
3-(3-Chlor-4-fluorphenyl)-1-(1-(7,8-difluor-1-oxo-1,2-dihydroisochinolin-4-yl)ethyl)-1-(methyld₃)harnstoff;
3-(3-Chlorphenyl)-1-(1-(7,8-difluor-1-oxo-1,2-dihydroisochinolin-4-yl)ethyl)-1-(methyl)harnstoff;
3-(3-Chlorphenyl)-1-(1-(7,8-difluor-1-oxo-1,2-dihydroisochinolin-4-yl)ethyl)-1-(methyl-d₃)hamstoff;
1-(1-(6,7-Difluor-1-oxo-1,2-dihydroisochinolin-4-yl)ethyl)-1-methyl-3-((S)-1-phenylethyl)harnstoff;
1-(1-(6,7-Difluor-1-oxo-1,2-dihydroisochinolin-4-yl)ethyl)-3-(3,4-difluorbenzyl)-1-methylharnstoff;
1-(1-(6,7-Difluor-1-oxo-1,2-dihydroisochinolin-4-yl)ethyl)-3-(4-fluorbenzyl)-1-methylharnstoff;
1-(1-(6,7-Difluor-1-oxo-1,2-dihydroisochinolin-4-yl)ethyl)-1-methyl-3-((R)-1-phenylethyl)harnstoff;
1-(1-(6,7-Difluor-1-oxo-1,2-dihydroisochinolin-4-yl)ethyl)-1-methyl-3-(3,4,5-trifluorbenzyl)hamstoff;
1-(1-(6,7-Difluor-1-oxo-1,2-dihydroisochinolin-4-yl)ethyl)-1-methyl-3-(3-chlor-4-fluorbenzyl)harnstoff;
1-(1-(6,7-Difluor-1-oxo-1,2-dihydroisochinolin-4-yl)ethyl)-3-(1H-indol-6-yl)-1-methylharnstoff;
1-(1-(7,8-Difluor-1-oxo-1,2-dihydroisochinolin-4-yl)ethyl)-1-methyl-3-((S)-1-phenylethyl)harnstoff;
1-(1-(7,8-Difluor-1-oxo-1,2-dihydroisochinolin-4-yl)ethyl)-1-methyl-3-((R)-1-phenylethyl)harnstoff;
1-(1-(7,8-Difluor-1-oxo-1,2-dihydroisochinolin-4-yl)ethyl)-3-(3,4-difluorbenzyl)-1-methylharnstoff;
1-(1-(7,8-Difluor-1-oxo-1,2-dihydroisochinolin-4-yl)ethyl)-3-(4-fluorbenzyl)-1-methylharnstoff;
1-(1-(7,8-Difluor-1-oxo-1,2-dihydroisochinolin-4-yl)ethyl)-3-(3,4,5-trifluorbenzyl)-1-methylharnstoff;
3-(3-Chlor-4-fluorbenzyl)-1-(1-(7,8-difluor-1-oxo-1,2-dihydroisochinolin-4-yl)ethyl)-1-methylharnstoff;
1-(1-(7,8-Difluor-1-oxo-1,2-dihydroisochinolin-4-yl)ethyl)-3-(1H-indol-6-yl)-1-methylharnstoff;
1-(1-(6,7-Difluor-1-oxo-1,2-dihydroisochinolin-4-yl)ethyl)-3-(3-(difluormethyl)-4-fluorphenyl)-1-methylharnstoff;
1-(1-(7,8-Difluor-1-oxo-1,2-dihydroisochinolin-4-yl)ethyl)-3-(3-(difluormethyl)-4-fluorphenyl)-1-methylharnstoff;
oder ein Salz, Solvat, Stereoisomer, Tautomer oder Isotopen-markiertes Derivat davon, oder eine beliebige Mischung davon.

11. Die Verbindung nach Anspruch 1, die ausgewählt ist aus der Gruppe bestehend aus:
(R)-3-(3-Chlor-4-fluorphenyl)-1-methyl-1-(1-(1-oxo-1,2-dihydroisochinolin-4-yl)ethyl)harnstoff;
(S)-3-(3-Chlor-4-fluorphenyl)-1-methyl-1-(1-(1-oxo-1,2-dihydroisochinolin-4-yl)ethyl)harnstoff;
(R)-3-(4-Fluor-3-methylphenyl)-1-methyl-1-(1-(1-oxo-1,2-dihydroisochinolin-4-yl)ethyl)harnstoff;
(R)-3-(3-Chlor-4-fluorphenyl)-1-(1-(1-oxo-1,2-dihydroisochinolin-4-yl)ethyl)-1 -propylharnstoff;
(R)-3-(4-Fluor-3-methylphenyl)-1-(1-(1-oxo-1,2-dihydroisochinolin-4-yl)ethyl)-1 -propylharnstoff;
(R)-3-(4-Fluor-3-(trifluormethyl)phenyl)-1-methyl-1-(1-(1-oxo-1,2-dihydroisochinolin-4-yl)ethyl)harnstoff;
(R)-3-(4-Fluorphenyl)-1-methyl-1-(1-(1-oxo-1,2-dihydroisochinolin-4-yl)ethyl)harnstoff;
(R)-1-Methyl-1-(1-(1-oxo-1,2-dihydroisochinolin-4-yl)ethyl)-3-(3,4,5-trifluorphenyl)harnstoff;
(R)-3-(3,4-Difluorphenyl)-1-methyl-1-(1-(1-oxo-1,2-dihydroisochinolin-4-yl)ethyl)harnstoff;
(R)-3-(3-Fluorphenyl)-1-methyl-1-(1-(1 -oxo-1,2-dihydroisochinolin-4-yl)ethyl)harnstoff;
(R)-3-(2-Chlorpyridin-4-yl)-1-methyl-1-(1-(1-oxo-1,2-dihydroisochinolin-4-yl)ethyl)harnstoff;
(R)-1-Methyl-1-(1-(1-oxo-1,2-dihydroisochinolin-4-yl)ethyl)-3-(2-(trifluormethyl)pyridin-4-yl)harnstoff;
(R)-3-(3-Chlor-4-fluorphenyl)-1-(2,2-difluorethyl)-1-(1-(1-oxo-1,2-dihydroisochinolin-4-yl)ethyl)harnstoff;
(R)-1-(2,2-Difluorethyl)-3-(4-fluor-3-methylphenyl)-1-(1-(1-oxo-1,2-dihydroisochinolin-4-yl)ethyl)harnstoff;
(R)-3-(3-Cyan-4-fluorphenyl)-1-methyl-1-(1-(1-oxo-1,2-dihydroisochinolin-4-yl)ethyl)harnstoff;
(R)-3-(3-Chlor-4-fluorphenyl)-1-(ethyl-d5)-1-(1-(1-oxo-1,2-dihydroisochinolin-4-yl)ethyl)harnstoff;
(R)-3-(3-Chlor-4-fluorphenyl)-1-(1-(1-methoxyisochinolin-4-yl)ethyl)-1-(2,2,2-trifluorethyl)harnstoff;
(R)-3-(4-Chlorphenyl)-1-methyl-1-(1-(1-oxo-1,2-dihydroisochinolin-4-yl)ethyl)harnstoff;
(R)-3-(4-Bromphenyl)-1-methyl-1-(1-(1-oxo-1,2-dihydroisochinolin-4-yl)ethyl)harnstoff;
(R)-1-(3-Chlor-4-fluorphenyl)-3-(1-(1-oxo-1,2-dihydroisochinolin-4-yl)ethyl)harnstoff;
(S)-1-(3-Chlor-4-fluorphenyl)-3-(1-(1-oxo-1,2-dihydroisochinolin-4-yl)ethyl)hamstoff;
(R)-3-(3-Chlor-4-fluorphenyl)-1-ethyl-1-(1-(1-methoxyisochinolin-4-yl)ethyl)harnstoff;
(S)-3-(3-Chlor-4-fluorphenyl)-1-ethyl-1-(1-(1-methoxyisochinolin-4-yl)ethyl)harnstoff;
(R)-3-(3-Chlor-4-fluorphenyl)-1-ethyl-1-(1-(1-oxo-1,2-dihydroisochinolin-4-yl)ethyl)harnstoff;
(S)-3-(3-Chlor-4-fluorphenyl)-1-ethyl-1-(1-(1-oxo-1,2-dihydroisochinolin-4-yl)ethyl)harnstoff;
(R)-3-(3-Chlor-4-fluorphenyl)-1-ethyl-1-(1-(1-methoxyisochinolin-4-yl)propyl)harnstoff;
(S)-3-(3-Chlor-4-fluorphenyl)-1-ethyl-1-(1-(1-methoxyisochinolin-4-yl)propyl)hamstoff;
(R)-3-(3-Chlor-4-fluorphenyl)-1-ethyl-1-(1-(1-oxo-1,2-dihydroisochinolin-4-yl)propyl)harnstoff;
(S)-3-(3-Chlor-4-fluorphenyl)-1-ethyl-1-(1-(1-oxo-1,2-dihydroisochinolin-4-yl)propyl)harnstoff;
(R)-3-(3-Chlor-4-fluorphenyl)-1-ethyl-1-(1-(1-methoxyisochinolin-4-yl)-2-methylpropyl)harnstoff;
(S)-3-(3-Chlor-4-fluorphenyl)-1-ethyl-1-(1-(1-methoxyisochinolin-4-yl)-2-methylpropyl)harnstoff;
(R)-3-(3-Chlor-4-fluorphenyl)-1-(cyclopropyl(1-methoxyisochinolin-4-yl)methyl)-1-ethylharnstoff;
(S)-3-(3-Chlor-4-fluorphenyl)-1-(cyclopropyl(1-methoxyisochinolin-4-yl)methyl)-1-ethylharnstoff;
(R)-3-(3-Chlor-4-fluorphenyl)-1-(cyclopropyl(1-oxo-1,2-dihydroisochinolin-4-yl)methyl)-1-ethylharnstoff;
(S)-3-(3-Chlor-4-fluorphenyl)-1-(cyclopropyl(1-oxo-1,2-dihydroisochinolin-4-yl)methyl)-1-ethylharnstoff;
(R)-3-(3-Chlor-4-fluorphenyl)-1-ethyl-1-(2-methyl-1-(1-oxo-1,2-dihydroisochinolin-4-yl)propyl)harnstoff;
(S)-3-(3-Chlor-4-fluorphenyl)-1-ethyl-1-(2-methyl-1-(1-oxo-1,2-dihydroisochinolin-4-yl)propyl)harnstoff;
(R)-3-(3-Chlor-4-fluorphenyl)-1-cyclopropyl-1-(1-(1-methoxyisochinolin-4-yl)ethyl)harnstoff;
(S)-3-(3-Chlor-4-fluorphenyl)-1-cyclopropyl-1-(1-(1-methoxyisochinolin-4-yl)ethyl)harnstoff;
(R)-3-(3-Chlor-4-fluorphenyl)-1-ethyl-1-((1-methoxyisochinolin-4-yl)(phenyl)methyl)harnstoff;
(S)-3-(3-Chlor-4-fluorphenyl)-1-ethyl-1-((1-methoxyisochinolin-4-yl)(phenyl)methyl)harnstoff;
(R)-1-Butyl-3-(3-chlor-4-fluorphenyl)-1-(1-(1-methoxyisochinolin-4-yl)ethyl)harnstoff;
(S)-1-Butyl-3-(3-chlor-4-fluorphenyl)-1-(1-(1-methoxyisochinolin-4-yl)ethyl)harnstoff;
(R)-3-(3-Chlor-4-fluorphenyl)-1-(cyclopropylmethyl)-1-(1-(1-methoxyisochinolin-4-yl)ethyl)harnstoff;
(S)-3-(3-Chlor-4-fluorphenyl)-1-(cyclopropylmethyl)-1-(1-(1-methoxyisochinolin-4-yl)ethyl)harnstoff;
(R)-3-(3-Chlor-4-fluorphenyl)-1-(2-methoxyethyl)-1-(1-(1-oxo-1,2-dihydroisochinolin-4-yl)ethyl)harnstoff;
(S)-3-(3-Chlor-4-fluorphenyl)-1-(2-methoxyethyl)-1-(1-(1-oxo-1,2-dihydroisochinolin-4-yl)ethyl)harnstoff;
(R)-3-(3-Chlor-4-fluorphenyl)-1-cyclopropyl-1-(1-(1-oxo-1,2-dihydroisochinolin-4-yl)ethyl)harnstoff;
(S)-3-(3-Chlor-4-fluorphenyl)-1-cyclopropyl-1-(1-(1-oxo-1,2-dihydroisochinolin-4-yl)ethyl)harnstoff;
(R)-3-(3-Chlor-4-fluorphenyl)-1-ethyl-1-((1-oxo-1,2-dihydroisochinolin-4-yl)(phenyl)methyl)harnstoff;
(S)-3-(3-Chlor-4-fluorphenyl)-1-ethyl-1-((1-oxo-1,2-dihydroisochinolin-4-yl)(phenyl)methyl)harnstoff;
(R)-3-(3-Chlor-4-fluorphenyl)-1-(2-methoxyethyl)-1-(1-(1-methoxyisochinolin-4-yl)ethyl)harnstoff;
(S)-3-(3-Chlor-4-fluorphenyl)-1-(2-methoxyethyl)-1-(1-(1-methoxyisochinolin-4-yl)ethyl)harnstoff;
(R)-3-(3-Chlor-4-fluorphenyl)-1-ethyl-1-(2,2,2-trifluor-1-(1-oxo-1,2-dihydroisochinolin-4-yl)ethyl)harnstoff;
(S)-3-(3-Chlor-4-fluorphenyl)-1-ethyl-1-(2,2,2-trifluor-1-(1-oxo-1,2-dihydroisochinolin-4-yl)ethyl)harnstoff;
(R)-3-(3-Chlor-4-fluorphenyl)-1-ethyl-1-(2,2,2-trifluor-1-(1-methoxyisochinolin-4-yl)ethyl)harnstoff;
(S)-3-(3-Chlor-4-fluorphenyl)-1-ethyl-1-(2,2,2-trifluor-1-(1-methoxyisochinolin-4-yl)ethyl)harnstoff;
(R)-3-(3-Chlor-4-fluorphenyl)-1-(3-methoxypropyl)-1-(1-(1-oxo-1,2-dihydroisochinolin-4-yl)ethyl)harnstoff;
(S)-3-(3-Chlor-4-fluorphenyl)-1-(3-methoxypropyl)-1-(1-(1-oxo-1,2-dihydroisochinolin-4-yl)ethyl)harnstoff;
(R)-3-(3-Chlor-4-fluorphenyl)-1-(1-(1-methoxyisochinolin-4-yl)ethyl)-1-(3-methoxypropyl)harnstoff;
(S)-3-(3-Chlor-4-fluorphenyl)-1-(1-(1-methoxyisochinolin-4-yl)ethyl)-1-(3-methoxypropyl)harnstoff;
(R)-1-Butyl-3-(3-chlor-4-fluorphenyl)-1-(1-(1-oxo-1,2-dihydroisochinolin-4-yl)ethyl)harnstoff;
(S)-1-Butyl-3-(3-chlor-4-fluorphenyl)-1-(1-(1-oxo-1,2-dihydroisochinolin-4-yl)ethyl)harnstoff;
(R)-3-(3-Chlor-4-fluorphenyl)-1-(cyclopropylmethyl)-1-(1-(1-oxo-1,2-dihydroisochinolin-4-yl)ethyl)harnstoff;
(S)-3-(3-Chlor-4-fluorphenyl)-1-(cyclopropylmethyl)-1-(1-(1-oxo-1,2-dihydroisochinolin-4-yl)ethyl)harnstoff;
(R)-3-(3-(3-Chlor-4-fluorphenyl)-1-(1-(1-oxo-1,2-dihydroisochinolin-4-yl)ethyl)ureido)-N, N-dimethylpropanamid;
(S)-3-(3-(3-Chlor-4-fluorphenyl)-1-(1-(1-oxo-1,2-dihydroisochinolin-4-yl)ethyl)ureido)-N,N-dime-thylpropanamid;
(R)-3-(3-Chlor-4-fluorphenyl)-1-(2-(methylsulfonyl)ethyl)-1-(1-(1-oxo-1,2-dihydroisochinolin-4-yl)ethyl)harnstoff;
(S)-3-(3-Chlor-4-fluorphenyl)-1-(2-(methylsulfonyl)ethyl)-1-(1-(1-oxo-1,2-dihydroisochinolin-4-yl)ethyl)harnstoff;
(R)-3-(3-Chlor-4-fluorphenyl)-1-(2-ethoxyethyl)-1-(1-(1-oxo-1,2-dihydroisochinolin-4-yl)ethyl)harnstoff;
(S)-3-(3-Chlor-4-fluorphenyl)-1-(2-ethoxyethyl)-1-(1-(1-oxo-1,2-dihydroisochinolin-4-yl)ethyl)harnstoff;
(R)-3-(3-Chlor-4-fluorphenyl)-1-(2-cyanoethyl)-1-(1-(1-oxo-1,2-dihydroisochinolin-4-yl)ethyl)hamstoff;
(S)-3-(3-Chlor-4-fluorphenyl)-1-(2-cyanoethyl)-1-(1-(1-oxo-1,2-dihydroisochinolin-4-yl)ethyl)harnstoff;
(R)-3-(3-Chlor-4-fluorphenyl)-1-(3-cyanopropyl)-1-(1-(1-oxo-1,2-dihydroisochinolin-4-yl)ethyl)harnstoff;
(S)-3-(3-Chlor-4-fluorphenyl)-1-(3-cyanopropyl)-1-(1-(1-oxo-1,2-dihydroisochinolin-4-yl)ethyl)harnstoff;
(R)-3-(3-(3-Chlor-4-fluorphenyl)-1-(1-(1-oxo-1,2-dihydroisochinolin-4-yl)ethyl)ureido)-N-methylpropanamid;
(S)-3-(3-(3-Chlor-4-fluorphenyl)-1-(1-(1-oxo-1,2-dihydroisochinolin-4-yl)ethyl)ureido)-N-methylpropanamid;
(R)-2-(3-(3-Chlor-4-fluorphenyl)-1-(1-(1-oxo-1,2-dihydroisochinolin-4-yl)ethyl)ureido)ethane-1-sulfonamid;
(S)-2-(3-(3-Chlor-4-fluorphenyl)-1-(1-(1-oxo-1,2-dihydroisochinolin-4-yl)ethyl)ureido)ethane-1-sulfonamid;
(R)-3-(3-Chlor-4-fluorphenyl)-1-(2-(2-methoxyethoxy)ethyl)-1-(1-(1-methoxyisochinolin-4-yl)ethyl)harnstoff;
(S)-3-(3-Chlor-4-fluorphenyl)-1-(2-(2-methoxyethoxy)ethyl)-1-(1-(1-methoxyisochinolin-4-yl)ethyl)harnstoff;
(R)-3-(3-Chlor-4-fluorphenyl)-1-(2-(2-methoxyethoxy)ethyl)-1-(1-(1-oxo-1,2-dihydroisochinolin-4-yl)ethyl)harnstoff;
(S)-3-(3-Chlor-4-fluorphenyl)-1-(2-(2-methoxyethoxy)ethyl)-1-(1-(1-oxo-1,2-dihydroisochinolin-4-yl)ethyl)harnstoff;
(R)-3-(3-Chlor-4-fluorphenyl)-1-(2-ethoxyethyl)-1-(1-(1-methoxyisochinolin-4-yl)ethyl)harnstoff;
(S)-3-(3-Chlor-4-fluorphenyl)-1-(2-ethoxyethyl)-1-(1-(1-methoxyisochinolin-4-yl)ethyl)harnstoff;
(R)-3-(3-Chlor-4-fluorphenyl)-1-(3-hydroxypropyl)-1-(1-(1-oxo-1,2-dihydroisochinolin-4-yl)ethyl)harnstoff;
(S)-3-(3-Chlor-4-fluorphenyl)-1-(3-hydroxypropyl)-1-(1-(1-oxo-1,2-dihydroisochinolin-4-yl)ethyl)harnstoff;
(R) -3-(3-Chlor-4-fluorphenyl)-1-methyl-1-(1-(1-((1-methyl-1H-1,2,4-triazol-3-yl)methoxy)isochinolin-4-yl)ethyl)harnstoff;
(S)-3-(3-Chlor-4-fluorphenyl)-1-methyl-1-(1-(1-((1-methyl-1H-1,2,4-triazol-3-yl)methoxy)isochinolin-4-yl)ethyl)harnstoff;
(R) -N-(2-(3-(3-Chlor-4-fluorphenyl)-1-(1-(1-oxo-1,2-dihydroisochinolin-4-yl)ethyl)ureido)ethyl)acetamid;
(S)-N-(2-(3-(3-Chlor-4-fluorphenyl)-1-(1-(1-oxo-1,2-dihydroisochinolin-4-yl)ethyl)ureido)ethyl)acetamid;
(R)-3-(3-Chlor-4-fluorphenyl)-1-(1-(1-oxo-1,2-dihydroisochinolin-4-yl)ethyl)-1-((tetrahydro-2H-pyran-4-yl)methyl)harnstoff;
(S)-3-(3-Chlor-4-fluorphenyl)-1-(1-(1-oxo-1,2-dihydroisochinolin-4-yl)ethyl)-1-((tetrahydro-2H-pyran-4-yl) methyl)harnstoff;
(R)-3-(3-Chlor-4-fluorphenyl)-1-isobutyl-1-(1-(1-oxo-1,2-dihydroisochinolin-4-yl)ethyl)harnstoff;
(S)-3-(3-Chlor-4-fluorphenyl)-1-isobutyl-1-(1-(1-oxo-1,2-dihydroisochinolin-4-yl)ethyl)harnstoff;
(R)-3-(3-Chlor-4-fluorphenyl)-1-methyl-1-(1-(5-(methylamino)pyrido[3,4-b]pyrazin-8-yl)ethyl)harnstoff;
(S)-3-(3-Chlor-4-fluorphenyl)-1-methyl-1-(1-(5-(methylamino)pyrido[3,4-b]pyrazin-8-yl)ethyl)harnstoff;
(R)-3-(3-Chlor-4-fluorphenyl)-1-(1-(5-methoxypyrido[3,4-b]pyrazin-8-yl)ethyl)-1-methylharnstoff;
(S)-3-(3-Chlor-4-fluorphenyl)-1-(1-(5-methoxypyrido[3,4-b]pyrazin-8-yl)ethyl)-1-methylharnstoff;
(R)-3-(3-Chlor-4-fluorphenyl)-1-(3-hydroxypropyl)-1-(1-(1-methoxyisochinolin-4-yl)ethyl)harnstoff;
(S)-3-(3-Chlor-4-fluorphenyl)-1-(3-hydroxypropyl)-1-(1-(1-methoxyisochinolin-4-yl)ethyl)harnstoff;
(R) -1-(1-(1-((1H-1,2,4-triazol-3-yl)methoxy)isochinolin-4-yl)ethyl)-3-(3-chlor-4-fluorphenyl)-1-methylharnstoff;
(S) -1-(1-(1-((1H-1,2,4-Triazol-3-yl)methoxy)isochinolin-4-yl)ethyl)-3-(3-chlor-4-fluorphenyl)-1-methylharnstoff;
(R)-(3-(3-Chlor-4-fluorphenyl)-1-(1-(1-methoxyisochinolin-4-yl)ethyl)-1-(2,2,2-trifluorethyl)harnstoff;
(S)-(3-(3-Chlor-4-fluorphenyl)-1-(1-(1-methoxyisochinolin-4-yl)ethyl)-1-(2,2,2-trifluorethyl)harnstoff;
(R)-3-(3-Chlor-4-fluorphenyl)-1-ethyl-1-(1-(1-oxo-1,2-dihydro-2,7-naphthyridin-4-yl)ethyl)harnstoff;
(S)-3-(3-Chlor-4-fluorphenyl)-1-ethyl-1-(1-(1-oxo-1,2-dihydro-2,7-naphthyridin-4-yl)ethyl)harnstoff;
(R)-3-(3-Chlor-4-fluorphenyl)-1-(1-(1-oxo-1,2-dihydroisochinolin-4-yl)ethyl)-1-(2-((2,2,2-trifluorethyl)amino)ethyl)harnstoff;
(S)-3-(3-Chlor-4-fluorphenyl)-1-(1-(1-oxo-1,2-dihydroisochinolin-4-yl)ethyl)-1-(2-((2,2,2-trifluorethyl)amino)ethyl)harnstoff;
(R)-1-(1-(8-Chlor-1-oxo-1,2-dihydroisochinolin-4-yl)ethyl)-3-(3-chlor-4-fluorphenyl)-1-methylharnstoff;
(S)-1-(1-(8-Chlor-1-oxo-1,2-dihydroisochinolin-4-yl)ethyl)-3-(3-chlor-4-fluorphenyl)-1-methylharnstoff;
(R)-3-(3-Chlor-4-fluorphenyl)-1-(1-(6-fluor-1-oxo-1,2-dihydroisochinolin-4-yl)ethyl)-1-methylharnstoff;
(S)-3-(3-Chlor-4-fluorphenyl)-1-(1-(6-fluor-1-oxo-1,2-dihydroisochinolin-4-yl)ethyl)-1-methylharnstoff;
(R)-3-(3-Chlor-4-fluorphenyl)-1-(1-(6-chlor-1-oxo-1,2-dihydroisochinolin-4-yl)ethyl)-1-methylharnstoff;
(S)-3-(3-Chlor-4-fluorphenyl)-1-(1-(6-chlor-1-oxo-1,2-dihydroisochinolin-4-yl)ethyl)-1-methylharnstoff;
(R)-3-(3-Chlor-4-fluorphenyl)-1-(1-(7-methoxy-1-oxo-1,2-dihydroisochinolin-4-yl)ethyl)-1-methylharnstoff;
(S)-3-(3-Chlor-4-fluorphenyl)-1-(1-(7-methoxy-1-oxo-1,2-dihydroisochinolin-4-yl)ethyl)-1-methylharnstoff;
(R)-3-(3-Chlor-4-fluorphenyl)-1-(1-(6-methoxy-1-oxo-1,2-dihydroisochinolin-4-yl)ethyl)-1-methylharnstoff;
(S)-3-(3-Chlor-4-fluorphenyl)-1-(1-(6-methoxy-1-oxo-1,2-dihydroisochinolin-4-yl)ethyl)-1-methylharnstoff;
(R)-3-(3-Chlor-4-fluorphenyl)-1-methyl-1-(1-(2-methyl-1-oxo-1,2-dihydroisochinolin-4-yl)ethyl)harnstoff;
(S)-3-(3-Chlor-4-fluorphenyl)-1-methyl-1-(1-(2-methyl-1-oxo-1,2-dihydroisochinolin-4-yl)ethyl)harnstoff;
(R)-3-(3-Chlor-4-fluorphenyl)-1-methyl-1-(1-(2-ethyl-1-oxo-1,2-dihydroisochinolin-4-yl)ethyl)harnstoff;
(S)-3-(3-Chlor-4-fluorphenyl)-1-methyl-1-(1-(2-ethyl-1-oxo-1,2-dihydroisochinolin-4-yl)ethyl)harnstoff;
(R)-3-(3-Chlor-4-fluorphenyl)-1-methyl-1-(1-(2-propyl-1-oxo-1,2-dihydroisochinolin-4-yl)ethyl)harnstoff;
(S)-3-(3-Chlor-4-fluorphenyl)-1-methyl-1-(1-(2-propyl-1-oxo-1,2-dihydroisochinolin-4-yl)ethyl)harnstoff;
(R)-3-(3-Chlor-4-fluorphenyl)-1-methyl-1-(1-(2-isopropyl-1-oxo-1,2-dihydroisochinolin-4-yl)ethyl)harnstoff;
(S)-3-(3-Chlor-4-fluorphenyl)-1-methyl-1-(1-(2-isopropyl-1-oxo-1,2-dihydroisochinolin-4-yl)ethyl)harnstoff;
(R)-3-(3-Chlor-4-fluorphenyl)-1-methyl-1-(1-(2-cyclopropyl-1-oxo-1,2-dihydroisochinolin-4-yl)ethyl)harnstoff;
(S)-3-(3-Chlor-4-fluorphenyl)-1-methyl-1-(1-(2-cyclopropyl-1-oxo-1,2-dihydroisochinolin-4-yl)ethyl)harnstoff;
(R)-3-(3-Chlor-4-fluorphenyl)-1-(cyclopentylmethyl)-1-(1-(1-oxo-1,2-dihydroisochinolin-4-yl)ethyl)harnstoff;
(S)-3-(3-Chlor-4-fluorphenyl)-1-(cyclopentylmethyl)-1-(1-(1-oxo-1,2-dihydroisochinolin-4-yl)ethyl)harnstoff;
(R)-3-(3-Chlor-4-fluorphenyl)-1-(cyclohexylmethyl)-1-(1-(1-oxo-1,2-dihydroisochinolin-4-yl)ethyl)harnstoff;
(S)-3-(3-Chlor-4-fluorphenyl)-1-(cyclohexylmethyl)-1-(1-(1-oxo-1,2-dihydroisochinolin-4-yl)ethyl)harnstoff;
(R)-3-(3-Chlor-4-fluorphenyl)-1-(2-hydroxyethyl)-1-(1-(1-oxo-1,2-dihydroisochinolin-4-yl)ethyl)harnstoff;
(S)-3-(3-Chlor-4-fluorphenyl)-1-(2-hydroxyethyl)-1-(1-(1-oxo-1,2-dihydroisochinolin-4-yl)ethyl)harnstoff;
(R)-3-(3-Chlor-4-fluorphenyl)-1 -(2-((2,2-difluorethyl)amino)ethyl)-1-(1-(1-oxo-1,2-dihydroisochinolin-4-yl)ethyl)harnstoff;
(S)-3-(3-Chlor-4-fluorphenyl)-1-(2-((2,2-difluorethyl)amino)ethyl)-1-(1-(1-oxo-1,2-dihydroisochinolin-4-yl)ethyl)harnstoff;
(R)-1-((1-Acetylpiperidin-4-yl)methyl)-3-(3-chlor-4-fluorphenyl)-1-(1-(1-oxo-1,2-dihydroisochinolin-4-yl)ethyl)harnstoff;
(S)-1-((1-Acetylpiperidin-4-yl)methyl)-3-(3-chlor-4-fluorphenyl)-1-(1-(1-oxo-1,2-dihydroisochinolin-4-yl)ethyl)harnstoff;
(R)-3-(3-Chlor-4-fluorphenyl)-1-((1-(methylsulfonyl)piperidin-4-yl)methyl)-1-(1-(1-oxo-1,2-dihydroisochinolin-4-yl)ethyl)harnstoff;
(S)-3-(3-Chlor-4-fluorphenyl)-1-((1-(methylsulfonyl)piperidin-4-yl)methyl)-1-(1-(1-oxo-1,2-dihydroisochinolin-4-yl)ethyl)harnstoff;
(R)-3-(3-Chlor-4-fluorphenyl)-1-(3-hydroxy-2,2-dimethylpropyl)-1-(1-(1-oxo-1,2-dihydroisochinolin-4-yl)ethyl)harnstoff;
(S)-3-(3-Chlor-4-fluorphenyl)-1-(3-hydroxy-2,2-dimethylpropyl)-1-(1-(1-oxo-1,2-dihydroisochinolin-4-yl)ethyl)harnstoff;
3-(3-Chlor-4-fluorphenyl)-1-((R)-3-hydroxybutyl)-1-(1(R)-(1-oxo-1,2-dihydroisochinolin-4-yl)ethyl)harnstoff;
3-(3-Chlor-4-fluorphenyl)-1-((R)-3-hydroxybutyl)-1-(1(S)-(1-oxo-1 ,2-dihydroisochinolin-4-yl)ethyl)harnstoff;
3-(3-Chlor-4-fluorphenyl)-1-((S)-3-hydroxybutyl)-1-(1(R)-(1-oxo-1,2-dihydroisochinolin-4-yl)ethyl)harnstoff;
3-(3-Chlor-4-fluorphenyl)-1-((S)-3-hydroxybutyl)-1-(1(S)-(1-oxo-1,2-dihydroisochinolin-4-yl)ethyl)harnstoff;
3-(3-Chlor-4-fluorphenyl)-1-((R)-2-hydroxypropyl)-1-(1(R)-(1-oxo-1,2-dihydroisochinolin-4-yl)ethyl)harnstoff;
3-(3-Chlor-4-fluorphenyl)-1-((R)-2-hydroxypropyl)-1-((S)1-(1-oxo-1,2-dihydroisochinolin-4-yl)ethyl)harnstoff;
3-(3-Chlor-4-fluorphenyl)-1-((S)-2-hydroxypropyl)-1-(1(R)-(1-oxo-1,2-dihydroisochinolin-4-yl)ethyl)harnstoff;
3-(3-Chlor-4-fluorphenyl)-1-((S)-2-hydroxypropyl)-1-(1(S)-(1-oxo-1,2-dihydroisochinolin-4-yl)ethyl)harnstoff;
(R)-3-(3-Chlor-4-fluorphenyl)-1-methyl-1-(1-(3-methyl-1-oxo-1,2-dihydroisochinolin-4-yl)ethyl)harnstoff;
(S)-3-(3-Chlor-4-fluorphenyl)-1-methyl-1-(1-(3-methyl-1-oxo-1,2-dihydroisochinolin-4-yl)ethyl)harnstoff;
(R)-4-(3-(3-Chlor-4-fluorphenyl)-1-(1-(1-oxo-1,2-dihydroisochinolin-4-yl)ethyl)ureido)buttersäure;
(S)-4-(3-(3-Chlor-4-fluorphenyl)-1-(1-(1-oxo-1,2-dihydroisochinolin-4-yl)ethyl)ureido)buttersäure;
(R)-3-(3-Chlor-4-fluorphenyl)-1-methyl-1-(1-(1-(methylamino)isochinolin-4-yl)ethyl)harnstoff;
(S)-3-(3-Chlor-4-fluorphenyl)-1-methyl-1-(1-(1-(methylamino)isochinolin-4-yl)ethyl)harnstoff;
(R)-3-(3-Chlor-4-fluorphenyl)-1-methyl-1-(1-(1-(dimethylamino)isochinolin-4-yl)ethyl)harnstoff;
(S)-3-(3-Chlor-4-fluorphenyl)-1-methyl-1-(1-(1-(dimethylamino)isochinolin-4-yl)ethyl)harnstoff;
(R)-3-(4-Fluor-3-methylphenyl)-1-methyl-1-(1-(3-methyl-1-oxo-1,2-dihydroisochinolin-4-yl)ethyl)harnstoff;
(S)-3-(4-Fluor-3-methylphenyl)-1-methyl-1-(1-(3-methyl-1-oxo-1,2-dihydroisochinolin-4-yl)ethyl)harnstoff;
(R)-1-(1-(1-Aminoisochinolin-4-yl)ethyl)-3-(3-chlor-4-fluorphenyl)-1-methylharnstoff;
(S)-1-(1-(1-Aminoisochinolin-4-yl)ethyl)-3-(3-chlor-4-fluorphenyl)-1-methylharnstoff;
(R)-3-(3-Chlor-4-fluorphenyl)-1-(1-(1-(ethylamino)isochinolin-4-yl)ethyl)-1-methylharnstoff;
(S)-3-(3-Chlor-4-fluorphenyl)-1-(1-(1-(ethylamino)isochinolin-4-yl)ethyl)-1-methylharnstoff;
(R)-3-(3-Chlor-4-fluorphenyl)-1-(1-(1-((2-hydroxyethyl)amino)isochinolin-4-yl)ethyl)-1-methylharnstoff;
(S)-3-(3-Chlor-4-fluorphenyl)-1-(1-(1-((2-hydroxyethyl)amino)isochinolin-4-yl)ethyl)-1-methylharnstoff;
(R)-3-(3-Chlor-4-fluorphenyl)-1-methyl-1-(1-(1-(((1-methyl-1H-1,2,4-triazol-3-yl)methyl)amino)isochinolin-4-yl)ethyl)harnstoff;
(S)-3-(3-Chlor-4-fluorphenyl)-1-methyl-1-(1-(1-(((1-methyl-1H-1,2,4-triazol-3-yl)methyl)amino)isochinolin-4-yl)ethyl)harnstoff;
(R) -1-(1-(1-(((1H-1,2,4-Triazol-3-yl)methyl)amino)isochinolin-4-yl)ethyl)-3-(3-chlor-4-fluorphenyl)-1-methylharnstoff;
(S) -1-(1-(1-(((1H-1,2,4-Triazol-3-yl)methyl)amino)isochinolin-4-yl)ethyl)-3-(3-chlor-4-fluorphenyl)-1-methylharnstoff;
(R)-3-(3-Chlor-4-fluorphenyl)-1-(3-hydroxypropyl)-1-(1-(1-oxo-1,2-dihydro-2,7-naphthyridin-4-yl)ethyl)harnstoff;
(S)-3-(3-Chlor-4-fluorphenyl)-1-(3-hydroxypropyl)-1-(1-(1-oxo-1,2-dihydro-2,7-naphthyridin-4-yl)ethyl)harnstoff;
(R)-3-(3-Chlor-4-fluorphenyl)-1-isobutyl-1-(1-(1-oxo-1,2-dihydro-2,7-naphthyridin-4-yl)ethyl)harnstoff;
(S)-3-(3-Chlor-4-fluorphenyl)-1-isobutyl-1-(1-(1-oxo-1,2-dihydro-2,7-naphthyridin-4-yl)ethyl)harnstoff;
(R)-3-(3-Chlor-4-fluorphenyl)-1-(3-hydroxypropyl)-1-(1-(1-((1-methyl-1H-1,2,4-triazol-3-yl)methoxy)isochinolin-4-yl)ethyl)harnstoff;
(S)-3-(3-Chlor-4-fluorphenyl)-1-(3-hydroxypropyl)-1-(1-(1-((1-methyl-1H-1,2,4-triazol-3-yl)methoxy)isochinolin-4-yl)ethyl)harnstoff;
(R) -1-(1-(1-((1H-1,2,4-Triazol-3-yl)methoxy)isochinolin-4-yl)ethyl)-3-(3-chlor-4-fluorphenyl)-1-(3-hydroxypropyl)hamstoff;
(S)-1-(1-(1-((1H-1,2,4-Triazol-3-yl)methoxy)isochinolin-4-yl)ethyl)-3-(3-chlor-4-fluorphenyl)-1-(3-hydroxypropyl)hamstoff;
(R)-1-(1-(1-(((2H-1,2,3-Triazol-4-yl)methyl)amino)isochinolin-4-yl)ethyl)-3-(3-chlor-4-fluorphenyl)-1-methylharnstoff;
(S)-1-(1-(1-(((2H-1,2,3-Triazol-4-yl)methyl)amino)isochinolin-4-yl)ethyl)-3-(3-chlor-4-fluorphenyl)-1-methylharnstoff;
(R)-3-(3-Chlor-4-fluorphenyl)-1-(1-(7-fluor-1-oxo-1,2-dihydroisochinolin-4-yl)ethyl)-1-methylharnstoff;
(S)-3-(3-Chlor-4-fluorphenyl)-1-(1-(7-fluor-1-oxo-1,2-dihydroisochinolin-4-yl)ethyl)-1-methylharnstoff;
(R)-1-((1H-1,2,3-Triazol-4-yl)methyl)-3-(3-chlor-4-fluorphenyl)-1-(1-(1-oxo-1,2-dihydroisochinolin-4-yl)ethyl)harnstoff;
(S)-1-((1H-1,2,3-Triazol-4-yl)methyl)-3-(3-chlor-4-fluorphenyl)-1-(1-(1-oxo-1,2-dihydroisochinolin-4-yl)ethyl)harnstoff;
(R)-1-(1-(2-((1H-1,2,4-Triazol-3-yl)methyl)-1-oxo-1,2-dihydroisochinolin-4-yl)ethyl)-3-(3-chlor-4-fluorphenyl)-1-methylharnstoff;
(S)-1-(1-(2-((1H-1,2,4-Triazol-3-yl)methyl)-1-oxo-1,2-dihydroisochinolin-4-yl)ethyl)-3-(3-chlor-4-fluorphenyl)-1-methylharnstoff;
(R)-3-(3-Chlor-4-fluorphenyl)-1-((2,2-dimethyl-1,3-dioxan-5-yl)methyl)-1-(1-(1-oxo-1,2-dihydroisochinolin-4-yl)ethyl)harnstoff;
(S)-3-(3-Chlor-4-fluorphenyl)-1-((2,2-dimethyl-1,3-dioxan-5-yl)methyl)-1-(1-(1-oxo-1,2-dihydroisochinolin-4-yl)ethyl)harnstoff;
(R)-1-(1-(2-((1H-1,2,3-Triazol-4-yl)methyl)-1-oxo-1,2-dihydroisochinolin-4-yl)ethyl)-3-(3-chlor-4-fluorphenyl)-1-methylharnstoff;
(S)-1-(1-(2-((1H-1,2,3-Triazol-4-yl)methyl)-1-oxo-1,2-dihydroisochinolin-4-yl)ethyl)-3-(3-chlor-4-fluorphenyl)-1-methylharnstoff;
(R)-3-(3-Chlor-4-fluorphenyl)-1-(1-(6,7-difluor-1-oxo-1,2-dihydroisochinolin-4-yl)ethyl)-1-methylharnstoff;
(S)-3-(3-Chlor-4-fluorphenyl)-1-(1-(6,7-difluor-1-oxo-1,2-dihydroisochinolin-4-yl)ethyl)-1-methylharnstoff;
(R)-3-(3-Chlor-4-fluorphenyl)-1-(1-(6,7-difluor-1-oxo-1,2-dihydroisochinolin-4-yl)ethyl)-1-isobutylharnstoff;
(S)-3-(3-Chlor-4-fluorphenyl)-1-(1-(6,7-difluor-1-oxo-1,2-dihydroisochinolin-4-yl)ethyl)-1-isobutylharnstoff;
(R)-3-(3-Chlor-4-fluorphenyl)-1-((2,2-dimethyl-1 ,3-dioxan-5-yl)methyl)-1-(1-(6-fluor-1-oxo-1 ,2-dihydroisochinolin-4-yl)ethyl)harnstoff;
(S)-3-(3-Chlor-4-fluorphenyl)-1-((2,2-dimethyl-1,3-dioxan-5-yl)methyl)-1-(1-(6-fluor-1-oxo-1,2-dihydroisochinolin-4-yl)ethyl)harnstoff;
(R)-1-(1-(6,7-Difluor-1-oxo-1,2-dihydroisochinolin-4-yl)ethyl)-3-(4-fluorphenyl)-1-methylharnstoff;
(S)-1-(1-(6,7-Difluor-1-oxo-1,2-dihydroisochinolin-4-yl)ethyl)-3-(4-fluorphenyl)-1-methylharnstoff;
(R)-1-(1-(6,7-Difluor-1-oxo-1,2-dihydroisochinolin-4-yl)ethyl)-3-(4-fluorphenyl)-1-isobutylharnstoff;
(S)-1-(1-(6,7-Difluor-1-oxo-1,2-dihydroisochinolin-4-yl)ethyl)-3-(4-fluorphenyl)-1-isobutylharnstoff;
(R)-3-(3-Chlor-4-fluorphenyl)-1-(1-(6,7-difluor-1-oxo-1,2-dihydroisochinolin-4-yl)ethyl)-1-(3-hydroxypropyl)harnstoff;
(S)-3-(3-Chlor-4-fluorphenyl)-1-(1-(6,7-difluor-1-oxo-1,2-dihydroisochinolin-4-yl)ethyl)-1-(3-hydroxypropyl)harnstoff;
(R)-1-(1-(6,7-Difluor-1-oxo-1,2-dihydroisochinolin-4-yl)ethyl)-3-(4-fluorphenyl)-1-(3-hydroxypropyl)harnstoff;
(S)-1-(1-(6,7-Difluor-1-oxo-1,2-dihydroisochinolin-4-yl)ethyl)-3-(4-fluorphenyl)-1-(3-hydroxypropyl)harnstoff;
(R)-3-(3-Chlor-4-fluorphenyl)-1-(1-(6-fluor-1-oxo-1,2-dihydroisochinolin-4-yl)ethyl)-1-(3-hydroxy-2-(hydroxymethyl)propyl)harnstoff;
(S)-3-(3-Chlor-4-fluorphenyl)-1-(1-(6-fluor-1-oxo-1,2-dihydroisochinolin-4-yl)ethyl)-1-(3-hydroxy-2-(hydroxymethyl)propyl)harnstoff;
(R)-3-(3-Chlor-4-fluorphenyl)-1-(1-(7-fluor-2-methyl-1-oxo-1,2-dihydroisochinolin-4-yl)ethyl)-1-methylharnstoff;
(S)-3-(3-Chlor-4-fluorphenyl)-1-(1-(7-fluor-2-methyl-1-oxo-1,2-dihydroisochinolin-4-yl)ethyl)-1-methylharnstoff;
(R)-3-(3-Chlor-4-fluorphenyl)-1-(1-(1-oxo-1,2-dihydroisochinolin-4-yl)ethyl)-1-(pyridin-2-ylmethyl)harnstoff;
(S)-3-(3-Chlor-4-fluorphenyl)-1-(1-(1-oxo-1,2-dihydroisochinolin-4-yl)ethyl)-1-(pyridin-2-ylmethyl)harnstoff;
(R)-3-(3-Chlor-4-fluorphenyl)-1-(1-(1-oxo-1,2-dihydroisochinolin-4-yl)ethyl)-1-(pyridin-3-ylmethyl)harnstoff;
(S)-3-(3-Chlor-4-fluorphenyl)-1-(1-(1-oxo-1,2-dihydroisochinolin-4-yl)ethyl)-1-(pyridin-3-ylmethyl)harnstoff;
(R)-3-(3-Chlor-4-fluorphenyl)-1-(1-(1-oxo-1,2-dihydroisochinolin-4-yl)ethyl)-1-(pyridin-4-ylmethyl)harnstoff;
(S)-3-(3-Chlor-4-fluorphenyl)-1-(1-(1-oxo-1,2-dihydroisochinolin-4-yl)ethyl)-1-(pyridin-4-ylmethyl)harnstoff;
(R)-3-(3-Chlor-4-fluorphenyl)-1-(1-(1-oxo-1,2-dihydroisochinolin-4-yl)ethyl)-1-(thiazol-2-ylmethyl)harnstoff;
(S)-3-(3-Chlor-4-fluorphenyl)-1-(1-(1-oxo-1,2-dihydroisochinolin-4-yl)ethyl)-1-(thiazol-2-ylmethyl)harnstoff;
(R)-3-(3-Chlor-4-fluorphenyl)-1-(1-(1-oxo-1,2-dihydroisochinolin-4-yl)ethyl)-1-(thiazol-4-ylmethyl)harnstoff;
(S)-3-(3-Chlor-4-fluorphenyl)-1-(1-(1-oxo-1,2-dihydroisochinolin-4-yl)ethyl)-1-(thiazol-4-ylmethyl)harnstoff;
(R)-3-(3-Chlor-4-fluorphenyl)-1-methyl-1-(1-(1-(methylsulfonyl)isochinolin-4-yl)ethyl)harnstoff;
(S)-3-(3-Chlor-4-fluorphenyl)-1-methyl-1-(1-(1-(methylsulfonyl)isochinolin-4-yl)ethyl)harnstoff;
(R)-4-(1-(3-(3-Chlor-4-fluorphenyl)-1-methylureido)ethyl)-N-methylisochinolin-1-carboxamid;
(S)-4-(1-(3-(3-Chlor-4-fluorphenyl)-1-methylureido)ethyl)-N-methylisochinolin-1-carboxamid;
(R)-4-(1-(3-(3-Chlor-4-fluorphenyl)-1-methylureido)ethyl)-N, N-dimethylisochinolin-1-carboxamid;
(S)-4-(1-(3-(3-Chlor-4-fluorphenyl)-1-methylureido)ethyl)-N, N-dimethylisochinolin-1-carboxamid;
(R)-3-(3-Chlor-4-fluorphenyl)-1-(1-(1-oxo-1,2-dihydroisochinolin-4-yl)ethyl)-1-(pyrimidin-4-ylmethyl)harnstoff;
(S)-3-(3-Chlor-4-fluorphenyl)-1-(1-(1-oxo-1,2-dihydroisochinolin-4-yl)ethyl)-1-(pyrimidin-4-ylmethyl)harnstoff;
(R)-3-(3-Chlor-4-fluorphenyl)-1-(1-(1-oxo-1,2-dihydroisochinolin-4-yl)ethyl)-1-(thiazol-5-ylmethyl)harnstoff;
(S)-3-(3-Chlor-4-fluorphenyl)-1-(1-(1-oxo-1,2-dihydroisochinolin-4-yl)ethyl)-1-(thiazol-5-ylmethyl)harnstoff;
(R)-3-(3-Chlor-4-fluorphenyl)-1-(1-(1-(hydroxymethyl)isochinolin-4-yl)ethyl)-1-methylharnstoff;
(S)-3-(3-Chlor-4-fluorphenyl)-1-(1-(1-(hydroxymethyl)isochinolin-4-yl)ethyl)-1-methylharnstoff;
(R)-4-(1-(3-(3-Chlor-4-fluorphenyl)-1-methylureido)ethyl)isochinolin-1-carboxamid;
(S)-4-(1-(3-(3-Chlor-4-fluorphenyl)-1-methylureido)ethyl)isochinolin-1-carboxamid;
(R)-3-(3-Chlor-4-fluorphenyl)-1-(1-(7,8-difluor-1-oxo-1,2-dihydroisochinolin-4-yl)ethyl)-1-methylharnstoff;
(S)-3-(3-Chlor-4-fluorphenyl)-1-(1-(7,8-difluor-1-oxo-1,2-dihydroisochinolin-4-yl)ethyl)-1-methylharnstoff;
(R)-3-(3-Chlor-4-fluorphenyl)-1-(1-(7,8-difluor-2-methyl-1-oxo-1,2-dihydroisochinolin-4-yl)ethyl)-1-methylharnstoff;
(S)-3-(3-Chlor-4-fluorphenyl)-1-(1-(7,8-difluor-2-methyl-1-oxo-1,2-dihydroisochinolin-4-yl)ethyl)-1-methylharnstoff;
(R)-3-(3-Chlor-4-fluorphenyl)-1-(1-(7,8-difluor-1-oxo-1,2-dihydroisochinolin-4-yl)ethyl)-1-(3-hydroxypropyl)harnstoff;
(S)-3-(3-Chlor-4-fluorphenyl)-1-(1-(7,8-difluor-1-oxo-1,2-dihydroisochinolin-4-yl)ethyl)-1-(3-hydroxypropyl)harnstoff;
(R)-3-(3-Chlor-4-fluorphenyl)-1-(1-(1-cyanoisochinolin-4-yl)ethyl)-1-methylharnstoff;
(S)-3-(3-Chlor-4-fluorphenyl)-1-(1-(1-cyanoisochinolin-4-yl)ethyl)-1-methylharnstoff;
(R)-3-(3-Chlor-4-fluorphenyl)-1-(1-(7,8-difluor-1-oxo-1,2-dihydroisochinolin-4-yl)ethyl)-1-isobutylharnstoff;
(S)-3-(3-Chlor-4-fluorphenyl)-1-(1-(7,8-difluor-1-oxo-1,2-dihydroisochinolin-4-yl)ethyl)-1-isobutylharnstoff;
(R)-3-(3-Chlor-4-fluorphenyl)-1-(1-(6,7-difluor-2-methyl-1-oxo-1,2-dihydroisochinolin-4-yl)ethyl)-1-methylharnstoff;
(S)-3-(3-Chlor-4-fluorphenyl)-1-(1-(6,7-difluor-2-methyl-1-oxo-1,2-dihydroisochinolin-4-yl)ethyl)-1-methylharnstoff;
(R)-3-(3-Chlor-4-fluorphenyl)-1-(1-(6,7-difluor-2-methyl-1-oxo-1,2-dihydroisochinolin-4-yl)ethyl)-1-isobutylharnstoff;
(S)-3-(3-Chlor-4-fluorphenyl)-1-(1-(6,7-difluor-2-methyl-1-oxo-1,2-dihydroisochinolin-4-yl)ethyl)-1-isobutylharnstoff;
(R)-1-(1-(1-(1H-1,2,4-Triazol-1-yl)isochinolin-4-yl)ethyl)-3-(3-chlor-4-fluorphenyl)-1-isobutylharnstoff;
(S)-1-(1-(1-(1H-1,2,4-Triazol-1-yl)isochinolin-4-yl)ethyl)-3-(3-chlor-4-fluorphenyl)-1-isobutylhamstoff;
(R) -3-(3-Chlor-4-fluorphenyl)-1-(1-(6,7-difluor-1-((1-methyl-1H-1,2,4-triazol-3-yl)methoxy)isochinolin-4-yl)ethyl)-1-methylharnstoff;
(S)-3-(3-Chlor-4-fluorphenyl)-1-(1-(6,7-difluor-1-((1-methyl-1H-1,2,4-triazol-3-yl)methoxy)isochinolin-4-yl)ethyl)-1-methylharnstoff;
(R)-3-(3-Chlor-4-fluorphenyl)-1-(1-(6,7-difluor-1-((1-methyl-1H-1,2,4-triazol-3-yl)methoxy)isochinolin-4-yl)ethyl)-1-(3-hydroxypropyl)harnstoff;
(S)-3-(3-Chlor-4-fluorphenyl)-1-(1-(6,7-difluor-1-((1-methyl-1H-1,2,4-triazol-3-yl)methoxy)isochinolin-4-yl)ethyl)-1-(3-hydroxypropyl)harnstoff;
(R)-3-(3-Chlor-4-fluorphenyl)-1-(1-(6,7-difluor-2-methyl-1-oxo-1,2-dihydroisochinolin-4-yl)ethyl)-1-(3-hydroxypropyl)harnstoff;
(S)-3-(3-Chlor-4-fluorphenyl)-1-(1-(6,7-difluor-2-methyl-1-oxo-1,2-dihydroisochinolin-4-yl)ethyl)-1-(3-hydroxypropyl)harnstoff;
(R)-3-(3-Chlor-4-fluorphenyl)-1-methyl-1-(1-(1-methylisochinolin-4-yl)ethyl)harnstoff;
(S)-3-(3-Chlor-4-fluorphenyl)-1-methyl-1-(1-(1-methylisochinolin-4-yl)ethyl)harnstoff;
(R)-3-(3-Chlor-4-fluorphenyl)-1-(1-(8-fluor-1-oxo-1,2-dihydroisochinolin-4-yl)ethyl)-1-methylharnstoff;
(S)-3-(3-Chlor-4-fluorphenyl)-1-(1-(8-fluor-1-oxo-1,2-dihydroisochinolin-4-yl)ethyl)-1-methylharnstoff;
(R)-3-(3-Chlor-4-fluorphenyl)-1-(1-(8-fluor-2-methyl-1-oxo-1,2-dihydroisochinolin-4-yl)ethyl)-1-methylharnstoff;
(S)-3-(3-Chlor-4-fluorphenyl)-1-(1-(8-fluor-2-methyl-1-oxo-1,2-dihydroisochinolin-4-yl)ethyl)-1-methylharnstoff;
(R)-3-(3-Chlor-4-fluorphenyl)-1-(1-(8-fluor-1-oxo-1,2-dihydroisochinolin-4-yl)ethyl)-1-isobutylharnstoff;
(S)-3-(3-Chlor-4-fluorphenyl)-1-(1-(8-fluor-1-oxo-1,2-dihydroisochinolin-4-yl)ethyl)-1-isobutylharnstoff;
(R)-1-(1-(1-((1H-1,2,4-Triazol-3-yl)methoxy)-6,7-difluorisochinolin-4-yl)ethyl)-3-(3-chlor-4-fluorphenyl)-1-methylharnstoff;
(S)-1-(1-(1-((1H-1,2,4-Triazol-3-yl)methoxy)-6,7-difluorisochinolin-4-yl)ethyl)-3-(3-chlor-4-fluorphenyl)-1-methylharnstoff;
(R)-1-(1-(1-((1H-1,2,4-Triazol-3-yl)methoxy)-6,7-difluorisochinolin-4-yl)ethyl)-3-(3-chlor-4-fluorphenyl)-1-(3-hydroxypropyl)harnstoff;
(S)-1-(1-(1-((1H-1,2,4-Triazol-3-yl)methoxy)-6,7-difluorisochinolin-4-yl)ethyl)-3-(3-chlor-4-fluorphenyl)-1-(3-hydroxypropyl)harnstoff;
(R)-3-(3-Chlor-4-fluorphenyl)-1-(1-(8-fluor-1-oxo-1,2-dihydroisochinolin-4-yl)ethyl)-1-(3-hydroxypropyl)harnstoff;
(S)-3-(3-Chlor-4-fluorphenyl)-1-(1-(8-fluor-1-oxo-1,2-dihydroisochinolin-4-yl)ethyl)-1-(3-hydroxypropyl)harnstoff;
(R)-3-(3-Chlor-4-fluorphenyl)-1-(1-(6,8-difluor-1-oxo-1,2-dihydroisochinolin-4-yl)ethyl)-1-methylharnstoff;
(S)-3-(3-Chlor-4-fluorphenyl)-1-(1-(6,8-difluor-1-oxo-1,2-dihydroisochinolin-4-yl)ethyl)-1-methylharnstoff;
(R)-1-(1-(6,8-Difluor-1-oxo-1,2-dihydroisochinolin-4-yl)ethyl)-3-(4-fluorphenyl)-1-methylharnstoff;
(S)-1-(1-(6,8-Difluor-1-oxo-1,2-dihydroisochinolin-4-yl)ethyl)-3-(4-fluorphenyl)-1-methylharnstoff;
(R)-3-(3-Chlor-4-fluorphenyl)-1-(1-(6,8-difluor-1-oxo-1,2-dihydroisochinolin-4-yl)ethyl)-1-(3-hydroxypropyl)harnstoff;
(S)-3-(3-Chlor-4-fluorphenyl)-1-(1-(6,8-difluor-1-oxo-1,2-dihydroisochinolin-4-yl)ethyl)-1-(3-hydroxypropyl)harnstoff;
(R)-1-(1-(6,8-Difluor-1-oxo-1,2-dihydroisochinolin-4-yl)ethyl)-3-(4-fluorphenyl)-1-isobutylharnstoff;
(S)-1-(1-(6,8-Difluor-1-oxo-1,2-dihydroisochinolin-4-yl)ethyl)-3-(4-fluorphenyl)-1-isobutylharnstoff;
(R)-1-(1-(6,8-Difluor-1-oxo-1,2-dihydroisochinolin-4-yl)ethyl)-3-(4-fluorphenyl)-1-(3-hydroxypropyl)harnstoff;
(S)-1-(1-(6,8-Difluor-1-oxo-1,2-dihydroisochinolin-4-yl)ethyl)-3-(4-fluorphenyl)-1-(3-hydroxypropyl)harnstoff;
(R)-3-(3-Chlor-4-fluorphenyl)-1-(2-hydroxy-1-(1-oxo-1,2-dihydroisochinolin-4-yl)ethyl)-1-methylharnstoff;
(S)-3-(3-Chlor-4-fluorphenyl)-1-(2-hydroxy-1-(1-oxo-1,2-dihydroisochinolin-4-yl)ethyl)-1-methylharnstoff;
(R)-2-(3-(3-Chlor-4-fluorphenyl)-1-(1-(6-fluor-1-oxo-1,2-dihydroisochinolin-4-yl)ethyl)ureido)ethane-1-sulfonamid;
(S)-2-(3-(3-Chlor-4-fluorphenyl)-1-(1-(6-fluor-1-oxo-1,2-dihydroisochinolin-4-yl)ethyl)ureido)ethane-1-sulfonamid;
(R)-2-(3-(3-Chlor-4-fluorphenyl)-1-(1-(7-fluor-1-oxo-1,2-dihydroisochinolin-4-yl)ethyl)ureido)ethane-1-sulfonamid;
(S)-2-(3-(3-Chlor-4-fluorphenyl)-1-(1-(7-fluor-1-oxo-1,2-dihydroisochinolin-4-yl)ethyl)ureido)ethane-1-sulfonamid;
(R)-2-(3-(3-Chlor-4-fluorphenyl)-1-(1-(6,7-difluor-1-oxo-1,2-dihydroisochinolin-4-yl)ethyl)ureido)ethane-1-sulfonamid;
(S)-2-(3-(3-Chlor-4-fluorphenyl)-1-(1-(6,7-difluor-1-oxo-1,2-dihydroisochinolin-4-yl)ethyl)ureido)ethane-1-sulfonamid;
(R)-2-(1-(1-(6,7-Difluor-1-oxo-1,2-dihydroisochinolin-4-yl)ethyl)-3-(4-fluorphenyl)ureido)ethane-1-sulfonamid;
(S)-2-(1-(1-(6,7-Difluor-1-oxo-1,2-dihydroisochinolin-4-yl)ethyl)-3-(4-fluorphenyl)ureido)ethane-1-sulfonamid;
(R)-2-(3-(3-Chlor-4-fluorphenyl)-1-(1-(6,8-difluor-1-oxo-1,2-dihydroisochinolin-4-yl)ethyl)ureido)ethane-1-sulfonamid;
(S)-2-(3-(3-Chlor-4-fluorphenyl)-1-(1-(6,8-difluor-1-oxo-1,2-dihydroisochinolin-4-yl)ethyl)ureido)ethane-1-sulfonamid;
(R)-2-(3-(3-Chlor-4-fluorphenyl)-1-(1-(7,8-difluor-1-oxo-1,2-dihydroisochinolin-4-yl)ethyl)ureido)ethane-1-sulfonamid;
(S)-2-(3-(3-Chlor-4-fluorphenyl)-1-(1-(7,8-difluor-1-oxo-1,2-dihydroisochinolin-4-yl)ethyl)ureido)ethane-1-sulfonamid;
(R)-2-(3-(4-Fluorphenyl)-1-(1-(7,8-difluor-1-oxo-1,2-dihydroisochinolin-4-yl)ethyl)ureido)ethane-1-sulfonamid;
(S)-2-(3-(4-Fluorphenyl)-1-(1-(7,8-difluor-1-oxo-1,2-dihydroisochinolin-4-yl)ethyl)ureido)ethane-1-sulfonamid;
(R)-2-(3-(3-Chlor-4-fluorphenyl)-1-(1-(8-fluor-1-oxo-1,2-dihydroisochinolin-4-yl)ethyl)ureido)ethane-1-sulfonamid;
(S)-2-(3-(3-Chlor-4-fluorphenyl)-1-(1-(8-fluor-1-oxo-1,2-dihydroisochinolin-4-yl)ethyl)ureido)ethane-1-sulfonamid;
(R)-1-(1-(6,7-Difluor-1-oxo-1,2-dihydroisochinolin-4-yl)ethyl)-1-methyl-3-phenylharnstoff;
(S)-1-(1-(6,7-Difluor-1-oxo-1,2-dihydroisochinolin-4-yl)ethyl)-1-methyl-3-phenylharnstoff;
(R)-3-(4-Chlorphenyl)-1-(1-(6,7-difluor-1-oxo-1,2-dihydroisochinolin-4-yl)ethyl)-1-methylharnstoff;
(S)-3-(4-Chlorphenyl)-1-(1-(6,7-difluor-1-oxo-1,2-dihydroisochinolin-4-yl)ethyl)-1-methylharnstoff;
(R)-1-(1-(6,7-Difluor-1-oxo-1,2-dihydroisochinolin-4-yl)ethyl)-1-ethyl-3-(4-fluorphenyl)harnstoff;
(S)-1-(1-(6,7-Difluor-1-oxo-1,2-dihydroisochinolin-4-yl)ethyl)-1-ethyl-3-(4-fluorphenyl)harnstoff;
(R)-1-(1-(6,7-Difluor-1-oxo-1,2-dihydroisochinolin-4-yl)ethyl)-1-ethyl-3-phenylharnstoff;
(S)-1-(1-(6,7-Difluor-1-oxo-1,2-dihydroisochinolin-4-yl)ethyl)-1-ethyl-3-phenylharnstoff;
(R)-3-(3-Chlor-4-fluorphenyl)-1-(1-(6,7-difluor-1-oxo-1,2-dihydroisochinolin-4-yl)ethyl)-1-ethylharnstoff;
(S)-3-(3-Chlor-4-fluorphenyl)-1-(1-(6,7-difluor-1-oxo-1,2-dihydroisochinolin-4-yl)ethyl)-1-ethylharnstoff;
(R)-1-(1-(6,7-Difluor-1-oxo-1,2-dihydroisochinolin-4-yl)ethyl)-3-(3,4-difluorphenyl)-1-methylharnstoff;
(S)-1-(1-(6,7-Difluor-1-oxo-1,2-dihydroisochinolin-4-yl)ethyl)-3-(3,4-difluorphenyl)-1-methylharnstoff;
(R)-1-(1-(6,7-Difluor-1-oxo-1,2-dihydroisochinolin-4-yl)ethyl)-3-(3-fluorphenyl)-1-methylharnstoff;
(S)-1-(1-(6,7-Difluor-1-oxo-1,2-dihydroisochinolin-4-yl)ethyl)-3-(3-fluorphenyl)-1-methylharnstoff;
(R)-3-(3-Chlorphenyl)-1-(1-(6,7-difluor-1-oxo-1,2-dihydroisochinolin-4-yl)ethyl)-1-methylharnstoff;
(S)-3-(3-Chlorphenyl)-1-(1-(6,7-difluor-1-oxo-1,2-dihydroisochinolin-4-yl)ethyl)-1-methylharnstoff;
(R)-1-(1-(6,7-Difluor-1-oxo-1,2-dihydroisochinolin-4-yl)ethyl)-1-methyl-3-(3,4,5-trifluorphenyl)harnstoff;
(S)-1-(1-(6,7-Difluor-1-oxo-1,2-dihydroisochinolin-4-yl)ethyl)-1-methyl-3-(3,4,5-trifluorphenyl)harnstoff;
(R)-3-(3,5-Dichlorphenyl)-1-(1-(6,7-difluor-1-oxo-1,2-dihydroisochinolin-4-yl)ethyl)-1-methylharnstoff;
(S)-3-(3,5-Dichlorphenyl)-1-(1-(6,7-difluor-1-oxo-1,2-dihydroisochinolin-4-yl)ethyl)-1-methylharnstoff;
(R)-3-(2-Chlorpyridin-4-yl)-1-(1-(6,7-difluor-1-oxo-1,2-dihydroisochinolin-4-yl)ethyl)-1-methylharnstoff;
(S)-3-(2-Chlorpyridin-4-yl)-1-(1-(6,7-difluor-1-oxo-1,2-dihydroisochinolin-4-yl)ethyl)-1-methylhamstoff;
(R)-1-(1-(6,7-Difluor-1-oxo-1,2-dihydroisochinolin-4-yl)ethyl)-3-(2-fluorphenyl)-1-methylharnstoff;
(S)-1-(1-(6,7-Difluor-1-oxo-1,2-dihydroisochinolin-4-yl)ethyl)-3-(2-fluorphenyl)-1-methylharnstoff;
(R)-1-(1-(6,7-Difluor-1-oxo-1,2-dihydroisochinolin-4-yl)ethyl)-3-(3,4-difluorphenyl)-1-ethylharnstoff;
(S)-1-(1-(6,7-Difluor-1-oxo-1,2-dihydroisochinolin-4-yl)ethyl)-3-(3,4-difluorphenyl)-1-ethylharnstoff;
(R)-1-(1-(1-(1H-1,2,3-Triazol-1-yl)isochinolin-4-yl)ethyl)-3-(3-chlor-4-fluorphenyl)-1-methylharnstoff;
(S) -1-(1-(1-(1H-1,2,3-Triazol-1-yl)isochinolin-4-yl)ethyl)-3-(3-chlor-4-fluorphenyl)-1-methylharnstoff;
(R)-1-(1-(6,7-Difluor-1-oxo-1,2-dihydroisochinolin-4-yl)ethyl)-3-(4-fluor-3-methylphenyl)-1-methylharnstoff;
(S)-1-(1-(6,7-Difluor-1-oxo-1,2-dihydroisochinolin-4-yl)ethyl)-3-(4-fluor-3-methylphenyl)-1-methylharnstoff;
(R)-3-(3-Cyan-4-fluorphenyl)-1-(1-(6,7-difluor-1-oxo-1,2-dihydroisochinolin-4-yl)ethyl)-1-methylharnstoff;
(S)-3-(3-Cyan-4-fluorphenyl)-1-(1-(6,7-difluor-1-oxo-1,2-dihydroisochinolin-4-yl)ethyl)-1-methylharnstoff;
(R)-1-(1-(6,7-Difluor-1-oxo-1,2-dihydroisochinolin-4-yl)ethyl)-3-(2,3-difluorphenyl)-1-methylharnstoff;
(S)-1-(1-(6,7-Difluor-1-oxo-1,2-dihydroisochinolin-4-yl)ethyl)-3-(2,3-difluorphenyl)-1-methylharnstoff;
(R)-1-(1-(7,8-Difluor-1-oxo-1,2-dihydroisochinolin-4-yl)ethyl)-3-(4-fluorphenyl)-1-methylharnstoff;
(S)-1-(1-(7,8-Difluor-1-oxo-1,2-dihydroisochinolin-4-yl)ethyl)-3-(4-fluorphenyl)-1-methylharnstoff;
(R)-1-(1-(7,8-Difluor-1-oxo-1,2-dihydroisochinolin-4-yl)ethyl)-3-(3,4-difluorphenyl)-1-methylharnstoff;
(S)-1-(1-(7,8-Difluor-1-oxo-1,2-dihydroisochinolin-4-yl)ethyl)-3-(3,4-difluorphenyl)-1-methylharnstoff;
(R)-1-(1-(7,8-Difluor-1-oxo-1,2-dihydroisochinolin-4-yl)ethyl)-1-methyl-3-phenylharnstoff;
(S)-1-(1-(7,8-Difluor-1-oxo-1,2-dihydroisochinolin-4-yl)ethyl)-1-methyl-3-phenylharnstoff;
(R)-3-(3,5-Dichlor-4-fluorphenyl)-1-(1-(6,7-difluor-1-oxo-1,2-dihydroisochinolin-4-yl)ethyl)-1-methylharnstoff;
(S)-3-(3,5-Dichlor-4-fluorphenyl)-1-(1-(6,7-difluor-1-oxo-1,2-dihydroisochinolin-4-yl)ethyl)-1-methylharnstoff;
(R)-1-(1-(7,8-Difluor-1-oxo-1,2-dihydroisochinolin-4-yl)ethyl)-3-(4-fluor-3-methylphenyl)-1-methylharnstoff;
(S)-1-(1-(7,8-Difluor-1-oxo-1,2-dihydroisochinolin-4-yl)ethyl)-3-(4-fluor-3-methylphenyl)-1-methylharnstoff;
(R)-1-(1-(7,8-Difluor-1-oxo-1,2-dihydroisochinolin-4-yl)ethyl)-1-methyl-3-(3,4,5-trifluorphenyl)harnstoff;
(S)-1-(1-(7,8-Difluor-1-oxo-1,2-dihydroisochinolin-4-yl)ethyl)-1-methyl-3-(3,4,5-trifluorphenyl)harnstoff;
(R)-3-(4-Chlorphenyl)-1-(1-(7,8-difluor-1-oxo-1,2-dihydroisochinolin-4-yl)ethyl)-1-methylharnstoff;
(S)-3-(4-Chlorphenyl)-1-(1-(7,8-difluor-1-oxo-1,2-dihydroisochinolin-4-yl)ethyl)-1-methylharnstoff;
(R)-3-(3-Chlor-4-fluorphenyl)-1-(1-(6,8-difluor-1-oxo-1,2-dihydroisochinolin-4-yl)ethyl)-1-isobutylharnstoff;
(S)-3-(3-Chlor-4-fluorphenyl)-1-(1-(6,8-difluor-1-oxo-1,2-dihydroisochinolin-4-yl)ethyl)-1-isobutylharnstoff;
(R)-3-(3,5-Dichlorphenyl)-1-(1-(7,8-difluor-1-oxo-1,2-dihydroisochinolin-4-yl)ethyl)-1-methylharnstoff;
(S)-3-(3,5-Dichlorphenyl)-1-(1-(7,8-difluor-1-oxo-1,2-dihydroisochinolin-4-yl)ethyl)-1-methylharnstoff;
(R)-3-(3,5-Dichlor-4-fluorphenyl)-1-(1-(7,8-difluor-1-oxo-1,2-dihydroisochinolin-4-yl)ethyl)-1-methylharnstoff;
(S)-3-(3,5-Dichlor-4-fluorphenyl)-1-(1-(7,8-difluor-1-oxo-1,2-dihydroisochinolin-4-yl)ethyl)-1-methylharnstoff;
(R)-1-(1-(7,8-Difluor-1-oxo-1,2-dihydroisochinolin-4-yl)ethyl)-3-(3-fluorphenyl)-1-methylharnstoff;
(S)-1-(1-(7,8-Difluor-1-oxo-1,2-dihydroisochinolin-4-yl)ethyl)-3-(3-fluorphenyl)-1-methylharnstoff;
(R)-1-(1-(7,8-Difluor-1-oxo-1,2-dihydroisochinolin-4-yl)ethyl)-3-(2,3-difluorphenyl)-1-methylharnstoff;
(S)-1-(1-(7,8-Difluor-1-oxo-1,2-dihydroisochinolin-4-yl)ethyl)-3-(2,3-difluorphenyl)-1-methylharnstoff;
(R)-1-(1-(7,8-Difluor-1-oxo-1,2-dihydroisochinolin-4-yl)ethyl)-3-(3-chlorphenyl)-1-methylharnstoff;
(S)-1-(1-(7,8-Difluor-1-oxo-1,2-dihydroisochinolin-4-yl)ethyl)-3-(3-chlorphenyl)-1-methylharnstoff;
(R)-1-(1-(7,8-Difluor-1-oxo-1,2-dihydroisochinolin-4-yl)ethyl)-3-(3,4-difluorphenyl)-1-ethylharnstoff;
(S)-1-(1-(7,8-Difluor-1-oxo-1,2-dihydroisochinolin-4-yl)ethyl)-3-(3,4-difluorphenyl)-1-ethylharnstoff;
(R)-1-(1-(7,8-Difluor-1-oxo-1,2-dihydroisochinolin-4-yl)ethyl)-3-(4-fluorphenyl)-1-ethylharnstoff;
(S)-1-(1-(7,8-Difluor-1-oxo-1,2-dihydroisochinolin-4-yl)ethyl)-3-(4-fluorphenyl)-1-ethylharnstoff;
(R)-1-(1-(7,8-Difluor-1-oxo-1,2-dihydroisochinolin-4-yl)ethyl)-3-phenyl-1-ethylharnstoff;
(S)-1-(1-(7,8-Difluor-1-oxo-1,2-dihydroisochinolin-4-yl)ethyl)-3-phenyl-1-ethylharnstoff;
(R)-1-(1-(7,8-Difluor-1-oxo-1,2-dihydroisochinolin-4-yl)ethyl)-3-(3-chlor-4-fluorphenyl)-1-ethylharnstoff;
(S)-1-(1-(7,8-Difluor-1-oxo-1,2-dihydroisochinolin-4-yl)ethyl)-3-(3-chlor-4-fluorphenyl)-1-ethylharnstoff;
(R)-3-(3-Cyan-4-fluorphenyl)-1-(1-(7,8-difluor-1-oxo-1,2-dihydroisochinolin-4-yl)ethyl)-1-methylharnstoff;
(S)-3-(3-Cyan-4-fluorphenyl)-1-(1-(7,8-difluor-1-oxo-1,2-dihydroisochinolin-4-yl)ethyl)-1-methylharnstoff;
(R)-1-(1-(6,7-Difluor-1-oxo-1,2-dihydroisochinolin-4-yl)ethyl)-3-(3,4-difluorbenzyl)-1-methylharnstoff;
(S)-1-(1-(6,7-Difluor-1-oxo-1,2-dihydroisochinolin-4-yl)ethyl)-3-(3,4-difluorbenzyl)-1-methylharnstoff;
(R)-1-(1-(6,7-Difluor-1-oxo-1,2-dihydroisochinolin-4-yl)ethyl)-1-methyl-3-(3,4,5-trifluorbenzyl)harnstoff;
(S)1-(1-(6,7-Difluor-1-oxo-1,2-dihydroisochinolin-4-yl)ethyl)-1-methyl-3-(3,4,5-trifluorbenzyl)harnstoff;
(R)-1-(1-(6,7-Difluor-1-oxo-1,2-dihydroisochinolin-4-yl)ethyl)-1-methyl-3-(3-chlor-4-fluorbenzyl)harnstoff;
(S)-1-(1-(6,7-Difluor-1-oxo-1,2-dihydroisochinolin-4-yl)ethyl)-1-methyl-3-(3-chlor-4-fluorbenzyl)harnstoff;
(R)-1-(1-(6,7-Difluor-1-oxo-1,2-dihydroisochinolin-4-yl)ethyl)-3-(1H-indol-6-yl)-1-methylharnstoff;
(S)-1-(1-(6,7-Difluor-1-oxo-1,2-dihydroisochinolin-4-yl)ethyl)-3-(1H-indol-6-yl)-1-methylharnstoff;
(R)-1-(1-(7,8-Difluor-1-oxo-1,2-dihydroisochinolin-4-yl)ethyl)-3-(3,4-difluorbenzyl)-1-methylharnstoff;
(S)-1-(1-(7,8-Difluor-1-oxo-1,2-dihydroisochinolin-4-yl)ethyl)-3-(3,4-difluorbenzyl)-1-methylharnstoff;
(R)-1-(1-(7,8-Difluor-1-oxo-1,2-dihydroisochinolin-4-yl)ethyl)-3-(4-fluorbenzyl)-1-methylharnstoff;
(S)-1-(1-(7,8-Difluor-1-oxo-1,2-dihydroisochinolin-4-yl)ethyl)-3-(4-fluorbenzyl)-1-methylharnstoff;
(R)-1-(1-(7,8-Difluor-1-oxo-1,2-dihydroisochinolin-4-yl)ethyl)-3-(3,4,5-trifluorbenzyl)-1-methylharnstoff;
(S)-1-(1-(7,8-Difluor-1-oxo-1,2-dihydroisochinolin-4-yl)ethyl)-3-(3,4,5-trifluorbenzyl)-1-methylharnstoff;
(R)-3-(3-Chlor-4-fluorbenzyl)-1-(1-(7,8-difluor-1-oxo-1,2-dihydroisochinolin-4-yl)ethyl)-1-methylharnstoff;
(S)-3-(3-Chlor-4-fluorbenzyl)-1-(1-(7,8-difluor-1-oxo-1,2-dihydroisochinolin-4-yl)ethyl)-1-methylharnstoff;
(R)-1-(1-(7,8-Difluor-1-oxo-1,2-dihydroisochinolin-4-yl)ethyl)-3-(1H-indol-6-yl)-1-methylharnstoff;
(S)-1-(1-(7,8-Difluor-1-oxo-1,2-dihydroisochinolin-4-yl)ethyl)-3-(1H-indol-6-yl)-1-methylharnstoff;
(R)-1-(1-(6,7-Difluor-1-oxo-1,2-dihydroisochinolin-4-yl)ethyl)-3-(3-(difluormethyl)-4-fluorphenyl)-1-methylharnstoff;
(S)-1-(1-(6,7-Difluor-1-oxo-1,2-dihydroisochinolin-4-yl)ethyl)-3-(3-(difluormethyl)-4-fluorphenyl)-1-methylharnstoff;
(R)-1-(1-(7,8-Difluor-1-oxo-1,2-dihydroisochinolin-4-yl)ethyl)-3-(3-(difluormethyl)-4-fluorphenyl)-1-methylharnstoff; and
(S)-1-(1-(7,8-Difluor-1-oxo-1,2-dihydroisochinolin-4-yl)ethyl)-3-(3-(difluormethyl)-4-fluorphenyl)-1-methylharnstoff;
oder ein Salz, Solvat, Stereoisomer, Tautomer oder Isotopen-markiertes Derivat davon, oder eine beliebige Mischung davon.

12. Eine Verbindung ausgewählt aus der Gruppe bestehend aus:
1-(4-Fluor-3-methylphenyl)-3-((1-oxo-1,2-dihydroisochinolin-4-yl)methyl)harnstoff;
1-(3-Chlor-4-fluorphenyl)-3-((1-methoxyisochinolin-4-yl)methyl)harnstoff;
1-(4-Fluor-3-methylphenyl)-3-((1-methoxyisochinolin-4-yl)methyl)harnstoff;
1-(3-Chlor-4-fluorphenyl)-3-((1-oxo-1,2-dihydroisochinolin-4-yl)methyl)harnstoff;
1-(3-Chlor-4-fluorphenyl)-3-(1-(1-oxo-1,2-dihydroisochinolin-4-yl)ethyl)harnstoff;
1-(3-Chlor-4-fluorphenyl)-3-(3-hydroxy-1-(1-methoxyisochinolin-4-yl)propyl)harnstoff;
1-(3-Chlor-4-fluorphenyl)-3-(1-(6,7-difluor-1-oxo-1,2-dihydroisochinolin-4-yl)ethyl)harnstoff;
1-(3-Cyan-4-fluorphenyl)-3-(1-(6,7-difluor-1-oxo-1,2-dihydroisochinolin-4-yl)ethyl)harnstoff; und
1-(3-Chlor-4-fluorphenyl)-3-(1-(7,8-difluor-1-oxo-1,2-dihydroisochinolin-4-yl)ethyl)harnstoff,
oder ein Salz, Solvat, Stereoisomer, Tautomer oder Isotopen-markiertes Derivat davon, oder
eine beliebige Mischung davon.

13. Eine pharmazeutische Zusammensetzung, die mindestens eine Verbindung nach einem der Ansprüche 1 bis 12 und einen pharmazeutisch verträglichen Träger umfasst.

14. Mindestens eine Verbindung nach einem der Ansprüche 1 bis 12 zur Verwendung bei der Behandlung oder Prävention einer Hepatitis B-Virus (HBV)-Infektion in einem Individuum, oder zur direkten oder indirekten Inhibierung der Expression und/oder Funktion eines viralen Kapsidproteins in einem Individuum, das mit Hepatitis B-Virus infiziert ist.

15. Mindestens eine Verbindung nach einem der Ansprüche 1 bis 12 zur Verwendung nach Anspruch 14, wobei das Individuum weiterhin mit Hepatitis D-Virus (HDV) infiziert ist.

16. Die pharmazeutische Zusammensetzung nach Anspruch 12 oder die mindestens eine Verbindung nach einem der Ansprüche 1 bis 12 zur Verwendung nach Anspruch 14, die mindestens ein zusätzliches Mittel, das für die Behandlung einer Hepatitis B-Virusinfektion geeignet ist, umfassen, vorzugsweise zusammen formuliert oder dem Individuum gleichzeitig verabreicht, wobei das mindestens eine zusätzliche Mittel mindestens ein Mittel umfasst, das ausgewählt ist aus der Gruppe bestehend aus einem Inhibitor der reversen Transkriptase, einem Kap-sidinhibitor, einem Inhibitor der cccDNA-Bildung, einem RNA-Destabilisator, oligomeren Nukleotiden, die gegen das HBV-Genom gerichtet sind, Immunstimulatoren, einem GaINAc-siRNA-Konjugat, das gegen das HBV-Gentranskript gerichtet ist.

17. Mindestens eine Verbindung nach einem der Ansprüche 1 bis 12 zur Verwendung nach Anspruch 14, wobei das Individuum ein Säuger ist, vorzugsweise ein Mensch.

## Revendications

1. Composé de formule (I), ou son sel, solvate, stéréoisomère, tautomère, ou dérivé isotopiquement marqué, ou n'importe quels mélanges de ceux-ci : 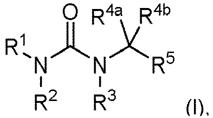 dans (I) :
R¹ étant sélectionné dans le groupe constitué des radicaux phényle éventuellement substitué, cycloalkyle en C₃ à C₈ éventuellement substitué, benzyle éventuellement substitué, hétéroaryle éventuellement substitué, et - (CH₂) (hétéroaryle éventuellement substitué) ;
chaque présence de R² étant indépendamment sélectionnée dans le groupe constitué des radicaux H et alkyle en C₁ à C₆ ;
R³ étant sélectionné dans le groupe constitué des radicaux alkyle en C₁ à C₆, et cycloalkyle en C₃ à C₈, le radical alkyle ou cycloalkyle étant éventuellement substitué par au moins un substituant sélectionné dans le groupe constitué des radicaux alkyle en C₁ à C₆, cycloalkyle en C₃ à C₈, halogéno, cyano, -OH, alcoxy en C₁ à C₆, cycloalcoxy en C₃ à C₈, halogénoalcoxy en C₁ à C₆, halogénocycloalcoxy en C₃ à C₈, phényle éventuellement substitué, hétéroaryle éventuellement substitué, hétérocyclyle éventuellement substitué, -C(=O)OR⁶, - OC(=O)R⁶, -SR⁶, -S(=O)R⁶, -S(=O)₂R6, -S(=O)₂NR⁶R⁶, - N(R⁶)S(=O)₂R⁶, -N(R⁶)C(=O)R⁶, -C(=O)NR⁶R⁶, et -NR⁶R⁶ ;
R^{4a} étant sélectionné dans le groupe constitué des radicaux H, alkyle en C₁ à C₆, cycloalkyle en C₃ à C₈, et phényle, le radical alkyle, cycloalkyle, ou phényle étant éventuellement substitué par au moins un substituant sélectionné dans le groupe constitué des radicaux alkyle en C₁ à C₆, cycloalkyle en C₃ à C₈, halogéno, cyano, -OH, alcoxy en C₁ à C₆, cycloalcoxy en C₃ à C₈, halogénoalcoxy en C₁ à C₆, halogénocycloalcoxy en C₃ à C₈, -NR⁶R⁶, et phényle éventuellement substitué ;
R^{4b} sélectionné dans le groupe constitué des radicaux H et alkyle en C₁ à C₆ éventuellement substitué ;
R⁵ étant sélectionné dans le groupe constitué de : 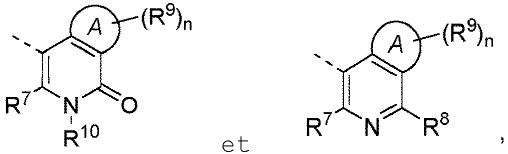
chaque cycle A étant indépendamment sélectionné dans le groupe constitué des cycles benzène, pyridine, pyrimidine, pyridazine, et pyrazine ;
chaque présence de R⁶ étant indépendamment sélectionnée dans le groupe constitué des radicaux H, alkyle en C₁ à C₆ éventuellement substitué, cycloalkyle en C₃ à C₈ éventuellement substitué, phényle éventuellement substitué, et hétéroaryle éventuellement substitué ;
chaque présence de R⁷ étant indépendamment sélectionnée dans le groupe constitué des radicaux H, halogéno, alkyle en C₁ à C₆ éventuellement substitué, cycloalkyle en C₃ à C₈ éventuellement substitué, alcoxy en C₁ à C₆ éventuellement substitué, et cycloalcoxy en C₃ à C₈ éventuellement substitué ;
chaque présence de R⁸ étant indépendamment sélectionnée dans le groupe constitué des radicaux halogéno, -CN, alkyle en C₁ à C₆ éventuellement substitué, cycloalkyle en C₃ à C₈ éventuellement substitué, alcoxy en C₁ à C₆ éventuellement substitué, cycloalcoxy en C₃ à C₈ éventuellement substitué, hétérocyclyle, hétéroaryle, -S(alkyle en C₁ à C₆ éventuellement substitué), -SO(alkyle en C₁ à C₆ éventuellement substitué), -SO₂(alkyle en C₁ à C₆ éventuellement substitué), -C(=O)OH, -C(=O)O(alkyle en C₁ à C₆ éventuellement substitué), -C(=O)O(cycloalkyle en C₃ à C₈ éventuellement substitué), -O(alkyle en C₁ à C₆ éventuellement substitué), -O(cycloalkyle en C₃ à C₈ éventuellement substitué), -NH₂, -NH(alkyle en C₁ à C₆ éventuellement substitué), -NH(cycloalkyle en C₃ à C₈ éventuellement substitué), -N(alkyle en C₁ à C₆ éventuellement substitué)(alkyle en C₁ à C₆ éventuellement substitué), -N(cycloalkyle en C₃ à C₈ éventuellement substitué) (cycloalkyle en C₃ à C₈ éventuellement substitué), -N(alkyle en C₁ à C₆ éventuellement substitué) (cycloalkyle en C₃ à C₈ éventuellement substitué), -C(=O)NH₂, -C (=0) NH (alkyle en C₁ à C₆ éventuellement substitué), -C(=O)NH(cycloalkyle en C₃ à C₈ éventuellement substitué), -C(=O)N(alkyle en C₁ à C₆ éventuellement substitué)(alkyle en C₁ à C₆ éventuellement substitué), -C(=0)N(cycloalkyle en C₃ à C₈ éventuellement substitué) (cycloalkyle en C₃ à C₈ éventuellement substitué), et -C(=O)N(alkyle en C₁ à C₆ éventuellement substitué)(cycloalkyle en C₃ à C₈ éventuellement substitué ;
chaque présence de R⁹ étant indépendamment sélectionnée dans le groupe constitué des radicaux H, halogéno, alkyle en C₁ à C₆ éventuellement substitué, cycloalkyle en C₃ à C₈ éventuellement substitué, alcoxy en C₁ à C₆ éventuellement substitué, et cycloalcoxy en C₃ à C₈ éventuellement substitué ;
chaque présence de n étant indépendamment 0, 1, 2, 3, ou 4 ; et
R¹⁰ étant sélectionné dans le groupe constitué des radicaux H, alkyle en C₁ à C₆ éventuellement substitué, et cycloalkyle en C₃ à C₈ éventuellement substitué.

2. Composé selon la revendication 1, chaque présence de R^{4b} étant indépendamment sélectionnée dans le groupe constitué des radicaux H et CH₃.

3. Composé selon la revendication 1, chaque présence du radical aryle ou hétéroaryle étant indépendamment éventuellement substituée par au moins un substituant sélectionné dans le groupe constitué des radicaux alkyle en C₁ à C₆, cycloalkyle en C₃ à C₈, phényle, hydroxyalkyle en C₁ à C₆, (alcoxy en C₁ à C₆) -alkyle en C₁ à C₆, halogénoalkyle en C₁ à C₆, halogénoalcoxy en C₁ à C₆, halogéno, -CN, -OR^{b}, -N(R^{b})(R^{b}), -NO₂, -C(=O)N(R^{b})(R^{b}), - C(=O)OR^{b}, -OC(=O)R^{b}, -SR^{b}, -S(=O)R^{b}, -S(=O)₂R^{b}, N(R^{b})S(=O)₂R^{b}, -S(=O)₂N(R^{b})(R^{b}), acyle, et alcoxycarbonyle en C₁ à C₆, chaque présence de R^{b} étant indépendamment un radical H, alkyle en C₁ à C₆, ou cycloalkyle en C₃ à C₈, dans R^{b} le radical alkyle ou cycloalkyle étant éventuellement substitué par au moins un substituant sélectionné dans le groupe constitué des radicaux halogéno, -OH, alcoxy en C₁ à C₆, et hétéroaryle ; ou des substituants sur deux atomes de carbone adjacents se combinent pour former le radical -O(CH₂)₁₋₃O-.

4. Composé selon la revendication 1, chaque présence du radical alkyle, alcényle, alcynyle, ou cycloalkyle étant indépendamment éventuellement substituée par au moins un substituant sélectionné dans le groupe constitué des radicaux alkyle en C₁ à C₆, cycloalkyle en C₃ à C₈, halogéno, cyano (-CN), -OR^{a}, phényle éventuellement substitué, hétéroaryle éventuellement substitué, hétérocyclyle éventuellement substitué, -C(=O)OR^{a}, - OC(=O)R^{a}, -SR^{a}, -S(=O)R^{a}, -S(=O)₂R^{a}, -S(=O)₂NR^{a}R^{a}, - N(R^{a})S(=O)₂R^{a}, -N(R^{a})C(=O)R^{a}, -C(=O)NR^{a}R^{a}, et -N(R^{a})(R^{a}), chaque présence de R^{a} étant indépendamment un radical H, alkyle en C₁ à C₆ éventuellement substitué, cycloalkyle en C₃ à C₈ éventuellement substitué, aryle éventuellement substitué, ou hétéroaryle éventuellement substitué, ou deux groupes R^{a} se combinent au radical N auquel ils sont liés pour former un hétérocycle.

5. Composé selon la revendication 1, R¹ étant un radical phényle éventuellement substitué par au moins un substituant sélectionné dans le groupe constitué des radicaux alkyle en C₁ à C₆, halogéno, halogénoalkyle en C₁ à C₃, et -CN, préférablement R¹ étant sélectionné dans le groupe constitué des radicaux phényle, 3-chlorophényle, 4-chlorophényle, 3-fluorophényle, 4-fluorophényle, 3,4-difluorophényle, 3,5-difluorophényle, 2,4,5-trifluorophényle, 3,4,5-trifluorophényle, 3,4-dichlorophényle, 3-chloro-4-fluorophényle, 4-chloro-3-fluorophényle, 4-chloro-3-méthylphényle, 3-chloro-4-méthylphényle, 4-fluoro-3-méthylphényle, 3-fluoro-4-méthylphényle, 4-chloro-3-méthoxyphényle, 3-chloro-4-méthoxyphényle, 4-fluoro-3-méthoxyphényle, 3-fluoro-4-méthoxyphényle, 3-trifluorométhylphényle, 4-trifluorométhylphényle, 3-trifluorométhyl-4-fluorophényle, 4-trifluorométhyl-3-fluorophényle, 3-cyanophényle, 4-cyanophényle, 3-cyano-4-fluorophényle, 4-cyano-3-fluorophényle, 3-difluorométhyl-4-fluorophényle, et 4-difluorométhyl-3-fluorophényle.

6. Composé selon la revendication 1, R² étant sélectionné dans le groupe constitué des radicaux H et méthyle.

7. Composé selon la revendication 1, qui est sélectionné dans le groupe constitué de :

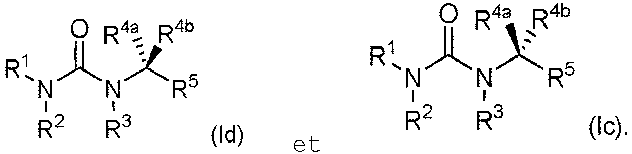

8. Composé selon la revendication 1, qui est sélectionné dans le groupe constitué de : 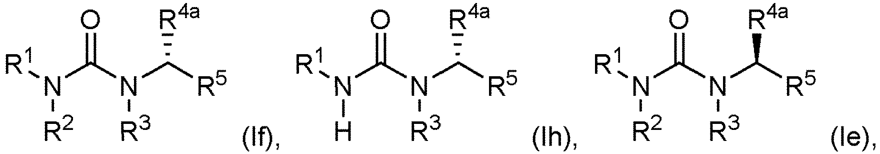 et

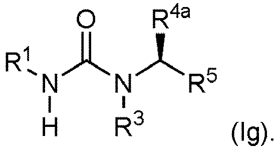

9. Composé selon la revendication 1, R⁵ étant sélectionné dans le groupe constitué de :

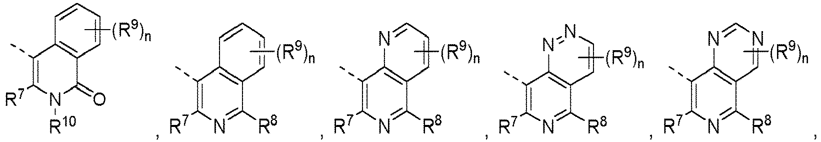

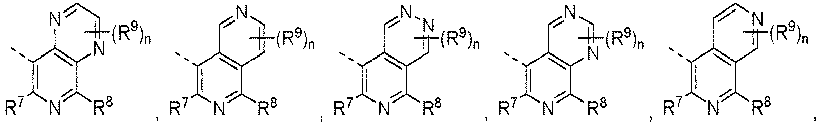

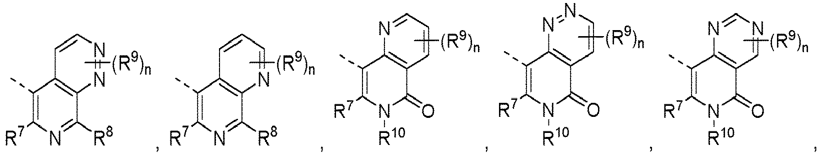

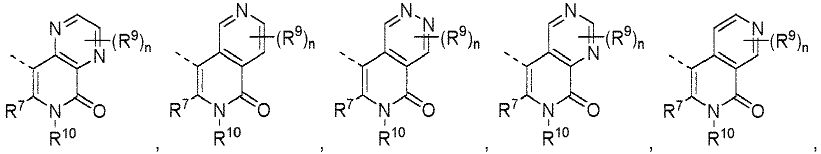

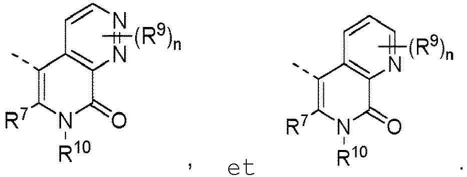

10. Composé selon la revendication 1, qui est sélectionné dans le groupe constitué de la :
3-(3-chloro-4-fluorophényl)-1-méthyl-1-((1-oxo-1,2-dihydroisoquinoléin-4-yl)méthyl)urée ;
3-(3-chloro-4-fluorophényl)-1-((1-méthoxyisoquinoléin-4-yl)méthyl)-1-méthylurée ;
3-(4-fluoro-3-méthylphényl)-1-((1-méthoxyisoquinoléin-4-yl)méthyl)-1-méthylurée ;
3-(4-fluoro-3-méthylphényl)-1-méthyl-1-((1-oxo-1,2-dihydroisoquinoléin-4-yl)méthyl)urée ;
3-(3-chloro-4-fluorophényl)-1-éthyl-1-((1-oxo-1,2-dihydroisoquinoléin-4-yl)méthyl)urée ;
3-(3-chloro-4-fluorophényl)-1-méthyl-1-(1-(1-oxo-1,2-dihydroisoquinoléin-4-yl)éthyl)urée ;
3-(3-chloro-4-fluorophényl)-1-éthyl-1-((1-méthoxyisoquinoléin-4-yl)méthyl)urée ;
3-(3-chloro-4-fluorophényl)-1-éthyl-1-(isoquinoléin-4-ylméthyl)urée ;
3-(3-chloro-4-fluorophényl)-1-((1-éthoxyisoquinoléin-4-yl)méthyl)-1-éthylurée ;
3-(3-chloro-4-fluorophényl)-1-éthyl-1-((2-méthyl-1-oxo-1,2-dihydroisoquinoléin-4-yl)méthyl)urée ;
3-(3-chloro-4-fluorophényl)-1-isopropyl-1-((1-méthoxyisoquinoléin-4-yl)méthyl)urée ;
3-(3-chloro-4-fluorophényl)-1-((1-méthoxyisoquinoléin-4-yl)méthyl)-1-propylurée ;
3-(3-chloro-4-fluorophényl)-1-(2-hydroxyéthyl)-1-((1-méthoxyisoquinoléin-4-yl)méthyl)urée ;
3-(3-chloro-4-fluorophényl)-1-éthyl-1-(1-(1-méthoxyisoquinoléin-4-yl)éthyl)urée ;
3-(3-chloro-4-fluorophényl)-1-éthyl-1-(1-(1-oxo-1,2-dihydroisoquinoléin-4-yl)éthyl)urée ;
3-(3-chloro-4-fluorophényl)-1-isopropyl-1-((1-oxo-1,2-dihydroisoquinoléin-4-yl)méthyl)urée ;
3-(3-chloro-4-fluorophényl)-1-(2-hydroxyéthyl)-1-((1-oxo-1,2-dihydroisoquinoléin-4-yl)méthyl)urée ;
3-(3-chloro-4-fluorophényl)-1-((1-oxo-1,2-dihydroisoquinoléin-4-yl)méthyl)-1-propylurée ;
3-(3-chloro-4-fluorophényl)-1-éthyl-1-((1-(2-hydroxyéthoxy)isoquinoléin-4-yl)méthyl)urée ;
3-(3-chloro-4-fluorophényl)-1-(1-(1-méthoxyisoquinoléin-4-yl)éthyl)-1-méthylurée ;
3-(3-chloro-4-fluorophényl)-1-(cyclopropylméthyl)-1-((1-méthoxyisoquinoléin-4-yl)méthyl)urée ;
3-(4-fluoro-3-méthylphényl)-1-méthyl-1-(1-(1-oxo-1,2-dihydroisoquinoléin-4-yl)éthyl)urée ;
1-butyl-3-(3-chloro-4-fluorophényl)-1-((1-méthoxyisoquinoléin-4-yl)méthyl)urée ;
3-(3-chloro-4-fluorophényl)-1-cyclopropyl-1-((1-méthoxyisoquinoléin-4-yl)méthyl)urée ;
(3-(3-chloro-4-fluorophényl)-1-(1-(1-oxo-1,2-dihydroisoquinoléin-4-yl)éthyl)-1-propylurée ;
3-(3-chloro-4-fluorophényl)-1-éthyl-1-(1-(1-méthoxyisoquinoléin-4-yl)propyl)urée
3-(3-chloro-4-fluorophényl)-1-éthyl-1-(1-(1-oxo-1,2-dihydroisoquinoléin-4-yl)propyl)urée ;
3-(3-chloro-4-fluorophényl)-1-(cyclopropyl(1-méthoxyisoquinoléin-4-yl)méthyl)-1-éthylurée ;
3-(3-chloro-4-fluorophényl)-1-(2-méthoxyéthyl)-1-((1-méthoxyisoquinoléin-4-yl)méthyl)urée ;
3-(3-chloro-4-fluorophényl)-1-cyclopropyl-1-((1-oxo-1,2-dihydroisoquinoléin-4-yl)méthyl)urée ;
3-(4-fluoro-3-méthylphényl)-1-(1-(1-oxo-1,2-dihydroisoquinoléin-4-yl)éthyl)-1-propylurée ;
3-(3-chloro-4-fluorophényl)-1-éthyl-1-(1-(1-méthoxyisoquinoléin-4-yl)-2-méthylpropyl)urée ;
3-(3-chloro-4-fluorophényl)-1-(cyclopropyl(1-oxo-1,2-dihydroisoquinoléin-4-yl)méthyl)-1-éthylurée ;
3-(3-chloro-4-fluorophényl)-1-((1-méthoxyisoquinoléin-4-yl)méthyl)-1-(3-méthoxypropyl)urée ;
1-benzyl-3-(3-chloro-4-fluorophényl)-1-((1-méthoxyisoquinoléin-4-yl)méthyl)urée ;
3-(3-chloro-4-fluorophényl)-1-(2-méthoxyéthyl)-1-((1-oxo-1,2-dihydroisoquinoléin-4-yl)méthyl)urée ;
3-(3-chloro-4-fluorophényl)-1-(3-méthoxypropyl)-1-((1-oxo-1,2-dihydroisoquinoléin-4-yl)méthyl)urée ;
1-butyl-3-(3-chloro-4-fluorophényl)-1-((1-oxo-1,2-dihydroisoquinoléin-4-yl)méthyl)urée ;
3-(3-chloro-4-fluorophényl)-1-(cyclopropylméthyl)-1-((1-oxo-1,2-dihydroisoquinoléin-4-yl)méthyl)urée ;
3-(3-chloro-4-fluorophényl)-1-éthyl-1-(2-méthyl-1-(1-oxo-1,2-dihydroisoquinoléin-4-yl)propyl)urée ;
3-(3-chloro-4-fluorophényl)-1-cyclopropyl-1-(1-(1-méthoxyisoquinoléin-4-yl)éthyl)urée ;
3-(3-chloro-4-fluorophényl)-1-éthyl-1-((1-méthoxyisoquinoléin-4-yl)(phényle)méthyl)urée ;
1-butyl-3-(3-chloro-4-fluorophényl)-1-(1-(1-méthoxyisoquinoléin-4-yl)éthyl)urée ;
3-(3-chloro-4-fluorophényl)-1-(cyclopropylméthyl)-1-(1-(1-méthoxyisoquinoléin-4-yl)éthyl)urée ;
3-(3-chloro-4-fluorophényl)-1-(2-méthoxyéthyl)-1-(1-(1-oxo-1,2-dihydroisoquinoléin-4-yl)éthyl)urée ;
3-(3-chloro-4-fluorophényl)-1-cyclopropyl-1-(1-(1-oxo-1,2-dihydroisoquinoléin-4-yl)éthyl)urée ;
3-(3-chloro-4-fluorophényl)-1-éthyl-1-((1-oxo-1,2-dihydroisoquinoléin-4-yl)(phényle)méthyl)urée ;
3-(3-chloro-4-fluorophényl)-1-(2-méthoxyéthyl)-1-(1-(1-méthoxyisoquinoléin-4-yl)éthyl)urée ;
3-(3-chloro-4-fluorophényl)-1-éthyl-1-(2,2,2-trifluoro-1-(1-oxo-1,2-dihydroisoquinoléin-4-yl)éthyl)urée ;
3-(3-chloro-4-fluorophényl)-1-éthyl-1-(2,2,2-trifluoro-1-(1-méthoxyisoquinoléin-4-yl)éthyl)urée ;
3-(3-chloro-4-fluorophényl)-1-(3-méthoxypropyl)-1-(1-(1-oxo-1,2-dihydroisoquinoléin-4-yl)éthyl)urée ;
3-(3-chloro-4-fluorophényl)-1-(1-(1-méthoxyisoquinoléin-4-yl)éthyl)-1-(3-méthoxypropyl)urée ;
1-butyl-3-(3-chloro-4-fluorophényl)-1-(1-(1-oxo-1,2-dihydroisoquinoléin-4-yl)éthyl)urée ;
3-(3-chloro-4-fluorophényl)-1-(cyclopropylméthyl)-1-(1-(1-oxo-1,2-dihydroisoquinoléin-4-yl)éthyl)urée ;
3-(3-chloro-4-fluorophényl)-1-méthyl-1-((1-((1-méthyl-1H-1,2,4-triazol-3-yl)méthoxy)isoquinoléin-4-yl)méthyl)urée ;
3-(3-chloro-4-fluorophényl)-1-méthyl-1-((1-(pyridin-2-ylméthoxy)isoquinoléin-4-yl)méthyl)urée ;
3-(4-fluoro-3-(trifluorométhyl)phényle)-1-méthyl-1-(1-(1-oxo-1,2-dihydroisoquinoléin-4-yl)éthyl)urée ;
3-(4-fluorophényl)-1-méthyl-1-(1-(1-oxo-1,2-dihydroisoquinoléin-4-yl)éthyl)urée ;
1-méthyl-1-(1-(1-oxo-1,2-dihydroisoquinoléin-4-yl)éthyl)-3-(3,4,5-trifluorophényl)urée ;
3-(3,4-difluorophényl)-1-méthyl-1-(1-(1-oxo-1,2-dihydroisoquinoléin-4-yl)éthyl)urée ;
3-(3-fluorophényl)-1-méthyl-1-(1-(1-oxo-1,2-dihydroisoquinoléin-4-yl)éthyl)urée ;
3-(2-chloropyridin-4-yl)-1-méthyl-1-(1-(1-oxo-1,2-dihydroisoquinoléin-4-yl)éthyl)urée ;
1-méthyl-1-(1-(1-oxo-1,2-dihydroisoquinoléin-4-yl)éthyl)-3-(2-(trifluorométhyl)pyridin-4-yl)urée ;
3-(3-chloro-4-fluorophényl)-1-(2,2-difluoroéthyl)-1-(1-(1-oxo-1,2-dihydroisoquinoléin-4-yl)éthyl)urée ;
1-(2,2-difluoroéthyl)-3-(4-fluoro-3-méthylphényl)-1-(1-(1-oxo-1,2-dihydroisoquinoléin-4-yl)éthyl)urée ;
3-(3-cyano-4-fluorophényl)-1-méthyl-1-(1-(1-oxo-1,2-dihydroisoquinoléin-4-yl)éthyl)urée ;
3-(3-chloro-4-fluorophényl)-1-(éthyl-d5)-1-(1-(1-oxo-1,2-dihydroisoquinoléin-4-yl)éthyl)urée ;
du N-(2-(3-(3-chloro-4-fluorophényl)-1-(1-(1-oxo-1,2-dihydroisoquinoléin-4-yl)éthyl)uréido)éthyl)acétamide ;
de la (3-(3-chloro-4-fluorophényl)-1-(1-(1-méthoxyisoquinoléin-4-yl)éthyl)-1-(2,2,2-trifluoroéthyl)urée ;
3-(3-chloro-4-fluorophényl)-1-éthyl-1-(1-(1-oxo-1,2-dihydro-2,7-naphtyridin-4-yl)éthyl)urée ;
3-(3-chloro-4-fluorophényl)-1-(2-cyanoéthyl)-1-(1-(1-oxo-1,2-dihydroisoquinoléin-4-yl)éthyl)urée ;
du 3-(3-(3-chloro-4-fluorophényl)-1-(1-(1-oxo-1,2-dihydroisoquinoléin-4-yl)éthyl)uréido)-N-méthylpropanamide ;
3-(3-(3-chloro-4-fluorophényl)-1-(1-(1-oxo-1,2-dihydroisoquinoléin-4-yl)éthyl)uréido)-N,N-diméthylpropanamide ;
de la 3-(3-chloro-4-fluorophényl)-1-(2-(méthylsulfonyl)éthyl)-1-(1-(1-oxo-1,2-dihydroisoquinoléin-4-yl)éthyl)urée ;
3-(3-chloro-4-fluorophényl)-1-(2-éthoxyéthyl)-1-(1-(1-oxo-1,2-dihydroisoquinoléin-4-yl)éthyl)urée ;
3-(3-chloro-4-fluorophényl)-1-méthyl-1-(1-(1-oxo-1,2-dihydro-2,7-naphtyridin-4-yl)éthyl)urée ;
3-(3-chloro-4-fluorophényl)-1-(3-cyanopropyl)-1-(1-(1-oxo-1,2-dihydroisoquinoléin-4-yl)éthyl)urée ;
du 2-(3-(3-chloro-4-fluorophényl)-1-(1-(1-oxo-1,2-dihydroisoquinoléin-4-yl)éthyl)uréido)-N-méthylacétamide ;
2-(3-(3-chloro-4-fluorophényl)-1-(1-(1-oxo-1,2-dihydroisoquinoléin-4-yl)éthyl)uréido)éthane-1-sulfonamide ;
de la 3-(3-chloro-4-fluorophényl)-1-(2-(2-méthoxyéthoxy)éthyl)-1-(1-(1-méthoxyisoquinoléin-4-yl)éthyl)urée ;
3-(3-chloro-4-fluorophényl)-1-(2-(2-méthoxyéthoxy)éthyl)-1-(1-(1-oxo-1,2-dihydroisoquinoléin-4-yl)éthyl)urée ;
3-(3-chloro-4-fluorophényl)-1-(2-éthoxyéthyl)-1-(1-(1-méthoxyisoquinoléin-4-yl)éthyl)urée ;
3-(3-chloro-4-fluorophényl)-1-méthyl-1-(1-(1-oxo-1,2-dihydro-2,6-naphtyridin-4-yl)éthyl)urée ;
du 2-(3-(3-chloro-4-fluorophényl)-1-(1-(1-oxo-1,2-dihydroisoquinoléin-4-yl)éthyl)uréido)acétamide ;
de la 3-(3-chloro-4-fluorophényl)-1-(3-hydroxypropyl)-1-(1-(1-oxo-1,2-dihydroisoquinoléin-4-yl)éthyl)urée ;
3-(3-chloro-4-fluorophényl)-1-méthyl-1-(1-(1-((1-méthyl-1H-1,2,4-triazol-3-yl)méthoxy)isoquinoléin-4-yl)éthyl)urée ;
3-(3-chloro-4-fluorophényl)-1-(1-(1-oxo-1,2-dihydroisoquinoléin-4-yl)éthyl)-1-((tétrahydro-2H-pyran-4-yl)méthyl)urée ;
3-(3-chloro-4-fluorophényl)-1-isobutyl-1-(1-(1-oxo-1,2-dihydroisoquinoléin-4-yl)éthyl)urée ;
3-(3-chloro-4-fluorophényl)-1-méthyl-1-(1-(5-(méthylamino)pyrido[3,4-b]pyrazin-8-yl)éthyl)urée ;
3-(3-chloro-4-fluorophényl)-1-(1-(5-méthoxypyrido[3,4-b]pyrazin-8-yl)éthyl)-1-méthylurée ;
3-(3-chloro-4-fluorophényl)-1-(3-hydroxypropyl)-1-(1-(1-méthoxyisoquinoléin-4-yl)éthyl)urée ;
1-(1-(1-((1H-1,2,4-triazol-3-yl)méthoxy)isoquinoléin-4-yl)éthyl)-3-(3-chloro-4-fluorophényl)-1-méthylurée ;
du 2-(3-(3-chloro-4-fluorophényl)-1-(1-(1-oxo-1,2-dihydroisoquinoléin-4-yl)éthyl)uréido)-N,N-diméthylacétamide ;
de la 3-(3-chloro-4-fluorophényl)-1-méthyl-1-(1-(5-oxo-5,6-dihydropyrido[3,4-b]pyrazin-8-yl)éthyl)urée ;
3-(3-chloro-4-fluorophényl)-1-(4-hydroxybutyl)-1-(1-(1-oxo-1,2-dihydroisoquinoléin-4-yl)éthyl)urée ;
3-(3-chloro-4-fluorophényl)-1-méthyl-1-(1-(8-oxo-7,8-dihydro-1,7-naphtyridin-5-yl)éthyl)urée ;
3-(3-chloro-4-fluorophényl)-1-isobutyl-1-(1-(1-oxo-1,2-dihydro-2,7-naphtyridin-4-yl)éthyl)urée ;
3-(3-chloro-4-fluorophényl)-1-(1-(1-oxo-1,2-dihydroisoquinoléin-4-yl)éthyl)-1-(((R)-tétrahydrofuran-2-yl)méthyl)urée ;
1-(3-aminopropyl)-3-(3-chloro-4-fluorophényl)-1-(1-(1-oxo-1,2-dihydroisoquinoléin-4-yl)éthyl)urée ;
3-(3-chloro-4-fluorophényl)-1-(1-(1-méthoxyisoquinoléin-4-yl)éthyl)-1-(2-((2,2,2-trifluoroéthyl)amino)éthyl)urée ;
3-(3-chloro-4-fluorophényl)-1-(3-hydroxypropyl)-1-(1-(1-oxo-1,2-dihydro-2,7-naphtyridin-4-yl)éthyl)urée ;
3-(3-chloro-4-fluorophényl)-1-(1-(1-oxo-1,2-dihydroisoquinoléin-4-yl)éthyl)-1-(((S)-tétrahydrofuran-2-yl)méthyl)urée ;
3-(3-chloro-4-fluorophényl)-1-(1-(1-oxo-1,2-dihydroisoquinoléin-4-yl)éthyl)-1-(2-((2,2,2-trifluoroéthyl)amino)éthyl)urée ;
3-(3-chloro-4-fluorophényl)-1-(1-(1-oxo-1,2-dihydroisoquinoléin-4-yl)éthyl)-1-((tétrahydrofuran-3-yl)méthyl)urée ;
1-(1-(8-chloro-1-oxo-1,2-dihydroisoquinoléin-4-yl)éthyl)-3-(3-chloro-4-fluorophényl)-1-méthylurée ;
3-(3-chloro-4-fluorophényl)-1-(1-(6-fluoro-1-oxo-1,2-dihydroisoquinoléin-4-yl)éthyl)-1-méthylurée ;
3-(3-chloro-4-fluorophényl)-1-(1-(6-chloro-1-oxo-1,2-dihydroisoquinoléin-4-yl)éthyl)-1-méthylurée ;
3-(3-chloro-4-fluorophényl)-1-(1-(7-méthoxy-1-oxo-1,2-dihydroisoquinoléin-4-yl)éthyl)-1-méthylurée ;
3-(3-chloro-4-fluorophényl)-1-(1-(6-méthoxy-1-oxo-1,2-dihydroisoquinoléin-4-yl)éthyl)-1-méthylurée ;
3-(3-chloro-4-fluorophényl)-1-méthyl-1-(1-(2-méthyl-1-oxo-1,2-dihydroisoquinoléin-4-yl)éthyl)urée ;
3-(3-chloro-4-fluorophényl)-1-méthyl-1-(1-(2-éthyl-1-oxo-1,2-dihydroisoquinoléin-4-yl)éthyl)urée ;
3-(3-chloro-4-fluorophényl)-1-méthyl-1-(1-(2-propyl-1-oxo-1,2-dihydroisoquinoléin-4-yl)éthyl)urée ;
3-(3-chloro-4-fluorophényl)-1-méthyl-1-(1-(2-isopropyl-1-oxo-1,2-dihydroisoquinoléin-4-yl)éthyl)urée ;
3-(3-chloro-4-fluorophényl)-1-méthyl-1-(1-(2-cyclopropyl-1-oxo-1,2-dihydroisoquinoléin-4-yl)éthyl)urée ;
3-(3-chloro-4-fluorophényl)-1-(1-(2-(2-méthoxyéthyl)-1-oxo-1,2-dihydroisoquinoléin-4-yl)éthyl)-1-méthylurée ;
3-(3-chloro-4-fluorophényl)-1-(cyclopentylméthyl)-1-(1-(1-oxo-1,2-dihydroisoquinoléin-4-yl)éthyl)urée ;
3-(3-chloro-4-fluorophényl)-1-(cyclohexylméthyl)-1-(1-(1-oxo-1,2-dihydroisoquinoléin-4-yl)éthyl)urée ;
3-(3-chloro-4-fluorophényl)-1-(2-hydroxyéthyl)-1-(1-(1-oxo-1,2-dihydroisoquinoléin-4-yl)éthyl)urée ;
3-(3-chloro-4-fluorophényl)-1-(2-((2,2-difluoroéthyl)amino)éthyl)-1-(1-(1-oxo-1,2-dihydroisoquinoléin-4-yl)éthyl)urée ;
3-(3-chloro-4-fluorophényl)-1-(1-(2-(3-méthoxypropyl)-1-oxo-1,2-dihydroisoquinoléin-4-yl)éthyl)-1-méthylurée ;
1-((1-acetylpiperidin-4-yl)méthyl)-3-(3-chloro-4-fluorophényl)-1-(1-(1-oxo-1,2-dihydroisoquinoléin-4-yl)éthyl)urée ;
3-(3-chloro-4-fluorophényl)-1-((1-(méthylsulfonyl)piperidin-4-yl)méthyl)-1-(1-(1-oxo-1,2-dihydroisoquinoléin-4-yl)éthyl)urée ;
3-(3-chloro-4-fluorophényl)-1-(1-(2-(2-hydroxyéthyl)-1-oxo-1,2-dihydroisoquinoléin-4-yl)éthyl)-1-méthylurée ;
3-(3-chloro-4-fluorophényl)-1-(1-(2-(3-hydroxypropyl)-1-oxo-1,2-dihydroisoquinoléin-4-yl)éthyl)-1-méthylurée ;
3-(3-chloro-4-fluorophényl)-1-((1-méthylpiperidin-4-yl)méthyl)-1-(1-(1-oxo-1,2-dihydroisoquinoléin-4-yl)éthyl)urée ;
de l'acide 3-(3-(3-chloro-4-fluorophényl)-1-(1-(1-oxo-1,2-dihydroisoquinoléin-4-yl)éthyl)uréido)propanoïque ;
de la 3-(3-chloro-4-fluorophényl)-1-(3-hydroxy-2,2-diméthylpropyl)-1-(1-(1-oxo-1,2-dihydroisoquinoléin-4-yl)éthyl)urée ;
3-(3-chloro-4-fluorophényl)-1-((R)-3-hydroxybutyl)-1-(1-(1-oxo-1,2-dihydroisoquinoléin-4-yl)éthyl)urée ;
3-(3-chloro-4-fluorophényl)-1-((S)-3-hydroxybutyl)-1-(1-(1-oxo-1,2-dihydroisoquinoléin-4-yl)éthyl)urée ;
3-(3-chloro-4-fluorophényl)-1-((R)-2-hydroxypropyl)-1-(1-(1-oxo-1,2-dihydroisoquinoléin-4-yl)éthyl)urée ;
3-(3-chloro-4-fluorophényl)-1-((S)-2-hydroxypropyl)-1-(1-(1-oxo-1,2-dihydroisoquinoléin-4-yl)éthyl)urée ;
du 3-(3-(3-chloro-4-fluorophényl)-1-(1-(1-oxo-1,2-dihydroisoquinoléin-4-yl)éthyl)uréido)-N-méthylpropane-1-sulfonamide ;
de la 3-(3-chloro-4-fluorophényl)-1-(((1r,4r)-4-hydroxycyclohexyl)méthyl)-1-(1-(1-oxo-1,2-dihydroisoquinoléin-4-yl)éthyl)urée ;
3-(3-chloro-4-fluorophényl)-1-méthyl-1-(1-(3-méthyl-1-oxo-1,2-dihydroisoquinoléin-4-yl)éthyl)urée ;
3-(3-chloro-4-fluorophényl)-1-((4-cis-hydroxycyclohexyl)méthyl)-1-(1-(1-oxo-1,2-dihydroisoquinoléin-4-yl)éthyl)urée ;
de l'acide 4-(3-(3-chloro-4-fluorophényl)-1-(1-(1-oxo-1,2-dihydroisoquinoléin-4-yl)éthyl)uréido)butanoïque ;
de la 3-(3-chloro-4-fluorophényl)-1-méthyl-1-(1-(1-oxo-2-(2,2,2-trifluoroéthyl)-1,2-dihydroisoquinoléin-4-yl)éthyl)urée ;
3-(3-chloro-4-fluorophényl)-1-isobutyl-1-(1-(1-oxo-2-(2,2,2-trifluoroéthyl)-1,2-dihydroisoquinoléin-4-yl)éthyl)urée ;
3-(4-fluoro-3-méthylphényl)-1-méthyl-1-(1-(3-méthyl-1-oxo-1,2-dihydroisoquinoléin-4-yl)éthyl)urée ;
3-(3-chloro-4-fluorophényl)-1-méthyl-1-(1-(1-(méthylamino)isoquinoléin-4-yl)éthyl)urée ;
3-(3-chloro-4-fluorophényl)-1-méthyl-1-(1-(1-(diméthylamino)isoquinoléin-4-yl)éthyl)urée ;
3-(4-fluorophényl)-1-isobutyl-1-(1-(1-oxo-2-(2,2,2-trifluoroéthyl)-1,2-dihydroisoquinoléin-4-yl)éthyl)urée ;
3-(4-fluoro-3-méthylphényl)-1-méthyl-1-(1-(3-méthyl-1-oxo-1,2-dihydroisoquinoléin-4-yl)éthyl)urée ;
1-(1-(1-aminoisoquinoléin-4-yl)éthyl)-3-(3-chloro-4-fluorophényl)-1-méthylurée ;
3-(3-chloro-4-fluorophényl)-1-(1-(1-(éthylamino)isoquinoléin-4-yl)éthyl)-1-méthylurée ;
3-(3-chloro-4-fluorophényl)-1-(1-(1-((2-hydroxyéthyl)amino)isoquinoléin-4-yl)éthyl)-1-méthylurée ;
1-(1-(1-((2-aminoéthyl)amino)isoquinoléin-4-yl)éthyl)-3-(3-chloro-4-fluorophényl)-1-méthylurée ;
3-(3-chloro-4-fluorophényl)-1-méthyl-1-(1-(1-(((1-méthyl-1H-1,2,4-triazol-3-yl)méthyl)amino)isoquinoléin-4-yl)éthyl)urée ;
1-(1-(1-(((1H-1,2,4-triazol-3-yl)méthyl)amino)isoquinoléin-4-yl)éthyl)-3-(3-chloro-4-fluorophényl)-1-méthylurée ;
3-(3-chloro-4-fluorophényl)-1-(3-hydroxypropyl)-1-(1-(1-((1-méthyl-1H-1,2,4-triazol-3-yl)méthoxy)isoquinoléin-4-yl)éthyl)urée ;
3-(3-chloro-4-fluorophényl)-1-(1-(7-fluoro-1-oxo-1,2-dihydroisoquinoléin-4-yl)éthyl)-1-méthylurée ;
1-(1-(7-fluoro-1-oxo-1,2-dihydroisoquinoléin-4-yl)éthyl)-3-(4-fluorophényl)-1-méthylurée ;
1-(1-(1-((1H-1,2,4-triazol-3-yl)méthoxy)isoquinoléin-4-yl)éthyl)-3-(3-chloro-4-fluorophényl)-1-(3-hydroxypropyl)urée ;
1-(1-(1-(((2H-1,2,3-triazol-4-yl)méthyl)amino)isoquinoléin-4-yl)éthyl)-3-(3-chloro-4-fluorophényl)-1-méthylurée ;
3-(3-chloro-4-fluorophényl)-1-(1-(5-méthoxy-1-oxo-1,2-dihydroisoquinoléin-4-yl)éthyl)-1-méthylurée ;
1-((1H-1,2,3-triazol-4-yl)méthyl)-3-(3-chloro-4-fluorophényl)-1-(1-(1-oxo-1,2-dihydroisoquinoléin-4-yl)éthyl)urée ;
1-((4H-1,2,4-triazol-3-yl)méthyl)-3-(3-chloro-4-fluorophényl)-1-(1-(1-oxo-1,2-dihydroisoquinoléin-4-yl)éthyl)urée ;
1-(1-(2-((1H-1,2,4-triazol-3-yl)méthyl)-1-oxo-1,2-dihydroisoquinoléin-4-yl)éthyl)-3-(3-chloro-4-fluorophényl)-1-méthylurée ;
1-(1-(7-chloro-1-oxo-1,2-dihydroisoquinoléin-4-yl)éthyl)-3-(3-chloro-4-fluorophényl)-1-méthylurée ;
3-(3-chloro-4-fluorophényl)-1-((2,2-diméthyl-1,3-dioxan-5-yl)méthyl)-1-(1-(1-oxo-1,2-dihydroisoquinoléin-4-yl)éthyl)urée ;
1-((2,2-diméthyl-1,3-dioxan-5-yl)méthyl)-3-(4-fluorophényl)-1-(1-(1-oxo-1,2-dihydroisoquinoléin-4-yl)éthyl)urée ;
1-((2,2-diméthyl-1,3-dioxan-5-yl)méthyl)-1-(1-(1-oxo-1,2-dihydroisoquinoléin-4-yl)éthyl)-3-phénylurée ;
1-(1-(2-((1H-1,2,3-triazol-4-yl)méthyl)-1-oxo-1,2-dihydroisoquinoléin-4-yl)éthyl)-3-(3-chloro-4-fluorophényl)-1-méthylurée ;
3-(3-chloro-4-fluorophényl)-1-(1-(6,7-difluoro-1-oxo-1,2-dihydroisoquinoléin-4-yl)éthyl)-1-méthylurée ;
3-(3-chloro-4-fluorophényl)-1-(1-(6,7-difluoro-1-oxo-1,2-dihydroisoquinoléin-4-yl)éthyl)-1-isobutylurée ;
3-(3-chloro-4-fluorophényl)-1-((2,2-diméthyl-1,3-dioxan-5-yl)méthyl)-1-(1-(6-fluoro-1-oxo-1,2-dihydroisoquinoléin-4-yl)éthyl)urée ;
3-(3-chloro-4-fluorophényl)-1-(3-hydroxy-2-(hydroxyméthyl)propyl)-1-(1-(1-oxo-1,2-dihydroisoquinoléin-4-yl)éthyl)urée ;
1-(1-(6,7-difluoro-1-oxo-1,2-dihydroisoquinoléin-4-yl)éthyl)-3-(4-fluorophényl)-1-méthylurée ;
1-(1-(6,7-difluoro-1-oxo-1,2-dihydroisoquinoléin-4-yl)éthyl)-3-(4-fluorophényl)-1-isobutylurée ;
3-(3-chloro-4-fluorophényl)-1-(1-(6,7-difluoro-1-oxo-1,2-dihydroisoquinoléin-4-yl)éthyl)-1-(3-hydroxypropyl)urée ;
1-(1-(6,7-difluoro-1-oxo-1,2-dihydroisoquinoléin-4-yl)éthyl)-3-(4-fluorophényl)-1-(3-hydroxypropyl)urée ;
3-(4-chlorophényl)-1-méthyl-1-(1-(1-oxo-1,2-dihydroisoquinoléin-4-yl)éthyl)urée ;
3-(4-bromophényl)-1-méthyl-1-(1-(1-oxo-1,2-dihydroisoquinoléin-4-yl)éthyl)urée ;
3-(3-chloro-4-fluorophényl)-1-(1-(6-fluoro-1-oxo-1,2-dihydroisoquinoléin-4-yl)éthyl)-1-(3-hydroxy-2-(hydroxyméthyl)propyl)urée ;
1-(1-(1-(1H-1,2,4-triazol-1-yl)isoquinoléin-4-yl)éthyl)-3-(3-chloro-4-fluorophényl)-1-méthylurée ;
3-cyclopentyl-1-méthyl-1-(1-(1-oxo-1,2-dihydroisoquinoléin-4-yl)éthyl)urée ;
1-(1-(1-(1H-1,2,4-triazol-1-yl)isoquinoléin-4-yl)éthyl)-3-(3-chloro-4-fluorophényl)-1-isobutylurée ;
1-(1-(1-(1H-1,2,4-triazol-1-yl)isoquinoléin-4-yl)éthyl)-3-(4-fluorophényl)-1-isobutylurée ;
3-(3-chloro-4-fluorophényl)-1-(1-(7-fluoro-2-méthyl-1-oxo-1,2-dihydroisoquinoléin-4-yl)éthyl)-1-méthylurée ;
3-(3-chloro-4-fluorophényl)-1-(1-(1-oxo-1,2-dihydroisoquinoléin-4-yl)éthyl)-1-(pyridin-2-ylméthyl)urée ;
3-(3-chloro-4-fluorophényl)-1-(1-(1-oxo-1,2-dihydroisoquinoléin-4-yl)éthyl)-1-(pyridin-3-ylméthyl)urée ;
3-(3-chloro-4-fluorophényl)-1-(1-(1-oxo-1,2-dihydroisoquinoléin-4-yl)éthyl)-1-(pyridin-4-ylméthyl)urée ;
3-(3-chloro-4-fluorophényl)-1-(1-(1-oxo-1,2-dihydroisoquinoléin-4-yl)éthyl)-1-(thiazol-2-ylméthyl)urée ;
3-(3-chloro-4-fluorophényl)-1-(1-(1-oxo-1,2-dihydroisoquinoléin-4-yl)éthyl)-1-(thiazol-4-ylméthyl)urée ;
3-(3-chloro-4-fluorophényl)-1-méthyl-1-(1-(1-(méthylsulfonyl)isoquinoléin-4-yl)éthyl)urée ;
du 4-(1-(3-(3-chloro-4-fluorophényl)-1-méthyluréido)éthyl)-N-méthylisoquinoléine-1-carboxamide ;
de la 4-(1-(3-(3-chloro-4-fluorophényl)-1-méthyluréido)éthyl)-N,N-diméthylisoquinoléine-1-carboxamide ;
3-(3-chloro-4-fluorophényl)-1-(1-(1-oxo-1,2-dihydroisoquinoléin-4-yl)éthyl)-1-(pyrimidin-5-ylméthyl)urée ;
3-(3-chloro-4-fluorophényl)-1-(1-(1-oxo-1,2-dihydroisoquinoléin-4-yl)éthyl)-1-(pyrimidin-4-ylméthyl)urée ;
3-(3-chloro-4-fluorophényl)-1-(1-(1-oxo-1,2-dihydroisoquinoléin-4-yl)éthyl)-1-(thiazol-5-ylméthyl)urée ;
de l'acide 4-(1-(3-(3-chloro-4-fluorophényl)-1-méthyluréido)éthyl)isoquinoléine-1-carboxylique ;
de la 3-(3-chloro-4-fluorophényl)-1-(1-(1-(hydroxyméthyl)isoquinoléin-4-yl)éthyl)-1-méthylurée ;
du 4-(1-(3-(3-chloro-4-fluorophényl)-1-méthyluréido)éthyl)isoquinoléine-1-carboxamide ;
de la 3-(3-chloro-4-fluorophényl)-1-(1-(7,8-difluoro-1-oxo-1,2-dihydroisoquinoléin-4-yl)éthyl)-1-méthylurée ;
3-(3-chloro-4-fluorophényl)-1-(1-(7,8-difluoro-2-méthyl-1-oxo-1,2-dihydroisoquinoléin-4-yl)éthyl)-1-méthylurée ;
3-(3-chloro-4-fluorophényl)-1-(1-(7,8-difluoro-1-oxo-1,2-dihydroisoquinoléin-4-yl)éthyl)-1-(3-hydroxypropyl)urée ;
3-(3-chloro-4-fluorophényl)-1-(1-(1-cyanoisoquinoléin-4-yl)éthyl)-1-méthylurée ;
3-(3-chloro-4-fluorophényl)-1-(1-(7,8-difluoro-1-oxo-1,2-dihydroisoquinoléin-4-yl)éthyl)-1-isobutylurée ;
3-(3-chloro-4-fluorophényl)-1-(1-(6,7-difluoro-2-méthyl-1-oxo-1,2-dihydroisoquinoléin-4-yl)éthyl)-1-méthylurée ;
3-(3-chloro-4-fluorophényl)-1-(1-(6,7-difluoro-2-méthyl-1-oxo-1,2-dihydroisoquinoléin-4-yl)éthyl)-1-isobutylurée ;
3-(3-chloro-4-fluorophényl)-1-(1-(6,7-difluoro-1-((1-méthyl-1H-1,2,4-triazol-3-yl)méthoxy)isoquinoléin-4-yl)éthyl)-1-méthylurée ;
3-(3-chloro-4-fluorophényl)-1-(1-(6,7-difluoro-1-((1-méthyl-1H-1,2,4-triazol-3-yl)méthoxy)isoquinoléin-4-yl)éthyl)-1-(3-hydroxypropyl)urée ;
3-(3-chloro-4-fluorophényl)-1-(1-(6,7-difluoro-2-méthyl-1-oxo-1,2-dihydroisoquinoléin-4-yl)éthyl)-1-(3-hydroxypropyl)urée ;
3-(3-chloro-4-fluorophényl)-1-méthyl-1-(1-(1-méthylisoquinoléin-4-yl)éthyl)urée ;
1-(1-(1-(1H-1,2,3-triazol-1-yl)isoquinoléin-4-yl)éthyl)-3-(3-chloro-4-fluorophényl)-1-méthylurée ;
1-(1-(1-(1H-1,2,3-triazol-1-yl)isoquinoléin-4-yl)éthyl)-3-(3-chloro-4-fluorophényl)-1-isobutylurée ;
3-(3-chloro-4-fluorophényl)-1-(1-(8-fluoro-1-oxo-1,2-dihydroisoquinoléin-4-yl)éthyl)-1-méthylurée ;
3-(3-chloro-4-fluorophényl)-1-(1-(8-fluoro-2-méthyl-1-oxo-1,2-dihydroisoquinoléin-4-yl)éthyl)-1-méthylurée ;
3-(3-chloro-4-fluorophényl)-1-(1-(8-fluoro-1-oxo-1,2-dihydroisoquinoléin-4-yl)éthyl)-1-isobutylurée ;
1-(1-(1-((1H-1,2,4-triazol-3-yl)méthoxy)-6,7-difluoroisoquinoléin-4-yl)éthyl)-3-(3-chloro-4-fluorophényl)-1-méthylurée ;
1-(1-(1-((1H-1,2,4-triazol-3-yl)méthoxy)-6,7-difluoroisoquinoléin-4-yl)éthyl)-3-(3-chloro-4-fluorophényl)-1-(3-hydroxypropyl)urée ;
3-(3-chloro-4-fluorophényl)-1-(1-(8-fluoro-1-oxo-1,2-dihydroisoquinoléin-4-yl)éthyl)-1-(3-hydroxypropyl)urée ;
3-(3-chloro-4-fluorophényl)-1-(1-(6,8-difluoro-1-oxo-1,2-dihydroisoquinoléin-4-yl)éthyl)-1-méthylurée ;
1-(1-(6,8-difluoro-1-oxo-1,2-dihydroisoquinoléin-4-yl)éthyl)-3-(4-fluorophényl)-1-méthylurée ;
3-(3-chloro-4-fluorophényl)-1-(1-(6,8-difluoro-1-oxo-1,2-dihydroisoquinoléin-4-yl)éthyl)-1-(3-hydroxypropyl)urée ;
1-(1-(6,8-difluoro-1-oxo-1,2-dihydroisoquinoléin-4-yl)éthyl)-3-(4-fluorophényl)-1-isobutylurée ;
1-(1-(6,8-difluoro-1-oxo-1,2-dihydroisoquinoléin-4-yl)éthyl)-3-(4-fluorophényl)-1-(3-hydroxypropyl)urée ;
3-(3-chloro-4-fluorophényl)-1-(2-hydroxy-1-(1-oxo-1,2-dihydroisoquinoléin-4-yl)éthyl)-1-méthylurée ;
du 2-(3-(3-chloro-4-fluorophényl)-1-(1-(6-fluoro-1-oxo-1,2-dihydroisoquinoléin-4-yl)éthyl)uréido)éthane-1-sulfonamide ;
2-(3-(3-chloro-4-fluorophényl)-1-(1-(7-fluoro-1-oxo-1,2-dihydroisoquinoléin-4-yl)éthyl)uréido)éthane-1-sulfonamide ;
2-(3-(3-chloro-4-fluorophényl)-1-(1-(6,7-difluoro-1-oxo-1,2-dihydroisoquinoléin-4-yl)éthyl)uréido)éthane-1-sulfonamide ;
2-(1-(1-(6,7-difluoro-1-oxo-1,2-dihydroisoquinoléin-4-yl)éthyl)-3-(4-fluorophényl)uréido)éthane-1-sulfonamide ;
2-(3-(3-chloro-4-fluorophényl)-1-(1-(6,8-difluoro-1-oxo-1,2-dihydroisoquinoléin-4-yl)éthyl)uréido)éthane-1-sulfonamide ;
2-(3-(3-chloro-4-fluorophényl)-1-(1-(7,8-difluoro-1-oxo-1,2-dihydroisoquinoléin-4-yl)éthyl)uréido)éthane-1-sulfonamide ;
2-(3-(4-fluorophényl)-1-(1-(7,8-difluoro-1-oxo-1,2-dihydroisoquinoléin-4-yl)éthyl)uréido)éthane-1-sulfonamide ;
2-(3-(3-chloro-4-fluorophényl)-1-(1-(8-fluoro-1-oxo-1,2-dihydroisoquinoléin-4-yl)éthyl)uréido)éthane-1-sulfonamide ;
de la 1-(1-(6,7-difluoro-1-oxo-1,2-dihydroisoquinoléin-4-yl)éthyl)-1-méthyl-3-phénylurée ;
3-(4-chlorophényl)-1-(1-(6,7-difluoro-1-oxo-1,2-dihydroisoquinoléin-4-yl)éthyl)-1-méthylurée ;
1-(1-(6,7-difluoro-1-oxo-1,2-dihydroisoquinoléin-4-yl)éthyl)-1-éthyl-3-(4-fluorophényl)urée ;
1-(1-(6,7-difluoro-1-oxo-1,2-dihydroisoquinoléin-4-yl)éthyl)-1-éthyl-3-phénylurée ;
3-(3-chloro-4-fluorophényl)-1-(1-(6,7-difluoro-1-oxo-1,2-dihydroisoquinoléin-4-yl)éthyl)-1-éthylurée ;
1-(1-(6,7-difluoro-1-oxo-1,2-dihydroisoquinoléin-4-yl)éthyl)-3-(3,4-difluorophényl)-1-méthylurée ;
1-(1-(6,7-difluoro-1-oxo-1,2-dihydroisoquinoléin-4-yl)éthyl)-3-(3-fluorophényl)-1-méthylurée ;
3-(3-chlorophényl)-1-(1-(6,7-difluoro-1-oxo-1,2-dihydroisoquinoléin-4-yl)éthyl)-1-méthylurée ;
1-(1-(6,7-difluoro-1-oxo-1,2-dihydroisoquinoléin-4-yl)éthyl)-1-méthyl-3-(3,4,5-trifluorophényl)urée ;
3-(3,5-dichlorophényl)-1-(1-(6,7-difluoro-1-oxo-1,2-dihydroisoquinoléin-4-yl)éthyl)-1-méthylurée ;
3-(2-chloropyridin-4-yl)-1-(1-(6,7-difluoro-1-oxo-1,2-dihydroisoquinoléin-4-yl)éthyl)-1-méthylurée ;
1-(1-(6,7-difluoro-1-oxo-1,2-dihydroisoquinoléin-4-yl)éthyl)-3-(2-fluorophényl)-1-méthylurée ;
1-(1-(6,7-difluoro-1-oxo-1,2-dihydroisoquinoléin-4-yl)éthyl)-3-(3,4-difluorophényl)-1-éthylurée ;
1-(1-(6,7-difluoro-1-oxo-1,2-dihydroisoquinoléin-4-yl)éthyl)-3-(4-fluoro-3-méthylphényl)-1-méthylurée ;
3-(3-cyano-4-fluorophényl)-1-(1-(6,7-difluoro-1-oxo-1,2-dihydroisoquinoléin-4-yl)éthyl)-1-méthylurée ;
1-(1-(6,7-difluoro-1-oxo-1,2-dihydroisoquinoléin-4-yl)éthyl)-3-(2,3-difluorophényl)-1-méthylurée ;
1-(1-(7,8-difluoro-1-oxo-1,2-dihydroisoquinoléin-4-yl)éthyl)-3-(4-fluorophényl)-1-méthylurée ;
1-(1-(7,8-difluoro-1-oxo-1,2-dihydroisoquinoléin-4-yl)éthyl)-3-(3,4-difluorophényl)-1-méthylurée ;
1-(1-(7,8-difluoro-1-oxo-1,2-dihydroisoquinoléin-4-yl)éthyl)-1-méthyl-3-phénylurée ;
3-(3,5-dichloro-4-fluorophényl)-1-(1-(6,7-difluoro-1-oxo-1,2-dihydroisoquinoléin-4-yl)éthyl)-1-méthylurée ;
1-(1-(7,8-difluoro-1-oxo-1,2-dihydroisoquinoléin-4-yl)éthyl)-3-(4-fluoro-3-méthylphényl)-1-méthylurée ;
1-(1-(7,8-difluoro-1-oxo-1,2-dihydroisoquinoléin-4-yl)éthyl)-1-méthyl-3-(3,4,5-trifluorophényl)urée ;
3-(4-chlorophényl)-1-(1-(7,8-difluoro-1-oxo-1,2-dihydroisoquinoléin-4-yl)éthyl)-1-méthylurée ;
3-(3-chloro-4-fluorophényl)-1-(1-(6,8-difluoro-1-oxo-1,2-dihydroisoquinoléin-4-yl)éthyl)-1-isobutylurée ;
3-(3,5-dichlorophényl)-1-(1-(7,8-difluoro-1-oxo-1,2-dihydroisoquinoléin-4-yl)éthyl)-1-méthylurée ;
1-(1-(7,8-difluoro-1-oxo-1,2-dihydroisoquinoléin-4-yl)éthyl)-1-méthyl-3-(pyridin-4-yl)urée ;
1-(1-(7,8-difluoro-1-oxo-1,2-dihydroisoquinoléin-4-yl)éthyl)-1-méthyl-3-(pyridin-3-yl)urée ;
3-(3,5-dichloro-4-fluorophényl)-1-(1-(7,8-difluoro-1-oxo-1,2-dihydroisoquinoléin-4-yl)éthyl)-1-méthylurée ;
3-benzyl-1-(1-(7,8-difluoro-1-oxo-1,2-dihydroisoquinoléin-4-yl)éthyl)-1-méthylurée ;
3-(2-chloropyridin-4-yl)-1-(1-(7,8-difluoro-1-oxo-1,2-dihydroisoquinoléin-4-yl)éthyl)-1-méthylurée ;
1-(1-(7,8-difluoro-1-oxo-1,2-dihydroisoquinoléin-4-yl)éthyl)-3-(3-fluorophényl)-1-méthylurée ;
1-(1-(7,8-difluoro-1-oxo-1,2-dihydroisoquinoléin-4-yl)éthyl)-3-(2-fluorophényl)-1-méthylurée ;
1-(1-(7,8-difluoro-1-oxo-1,2-dihydroisoquinoléin-4-yl)éthyl)-3-(2,3-difluorophényl)-1-méthylurée ;
1-(1-(7,8-difluoro-1-oxo-1,2-dihydroisoquinoléin-4-yl)éthyl)-3-(3-chlorophényl)-1-méthylurée ;
1-(1-(7,8-difluoro-1-oxo-1,2-dihydroisoquinoléin-4-yl)éthyl)-3-(3,4-difluorophényl)-1-éthylurée ;
1-(1-(7,8-difluoro-1-oxo-1,2-dihydroisoquinoléin-4-yl)éthyl)-3-(4-fluorophényl)-1-éthylurée ;
1-(1-(7,8-difluoro-1-oxo-1,2-dihydroisoquinoléin-4-yl)éthyl)-3-phényle-1-éthylurée ;
1-(1-(7,8-difluoro-1-oxo-1,2-dihydroisoquinoléin-4-yl)éthyl)-3-(3-chloro-4-fluorophényl)-1-éthylurée ;
3-(3-cyano-4-fluorophényl)-1-(1-(7,8-difluoro-1-oxo-1,2-dihydroisoquinoléin-4-yl)éthyl)-1-méthylurée ;
3-(3-chloro-4-fluorophényl)-1-(1-(6,7-difluoro-1-oxo-1,2-dihydroisoquinoléin-4-yl)éthyl)-1-(méthyl)urée ;
3-(3-chloro-4-fluorophényl)-1-(1-(6,7-difluoro-1-oxo-1,2-dihydroisoquinoléin-4-yl)éthyl)-1-(méthyl-d3)urée ;
3-(3-chloro-4-fluorophényl)-1-(1-(7,8-difluoro-1-oxo-1,2-dihydroisoquinoléin-4-yl)éthyl)-1-(méthyl)urée ;
3-(3-chloro-4-fluorophényl)-1-(1-(7,8-difluoro-1-oxo-1,2-dihydroisoquinoléin-4-yl)éthyl)-1-(méthyl-d3)urée ;
3-(3-chlorophényl)-1-(1-(7,8-difluoro-1-oxo-1,2-dihydroisoquinoléin-4-yl)éthyl)-1-(méthyl)urée ;
3-(3-chlorophényl)-1-(1-(7,8-difluoro-1-oxo-1,2-dihydroisoquinoléin-4-yl)éthyl)-1-(méthyl-d3)urée ;
1-(1-(6,7-difluoro-1-oxo-1,2-dihydroisoquinoléin-4-yl)éthyl)-1-méthyl-3-((S)-1-phényléthyl)urée ;
1-(1-(6,7-difluoro-1-oxo-1,2-dihydroisoquinoléin-4-yl)éthyl)-3-(3,4-difluorobenzyl)-1-méthylurée ;
1-(1-(6,7-difluoro-1-oxo-1,2-dihydroisoquinoléin-4-yl)éthyl)-3-(4-fluorobenzyl)-1-méthylurée ;
1-(1-(6,7-difluoro-1-oxo-1,2-dihydroisoquinoléin-4-yl)éthyl)-1-méthyl-3-((R)-1-phényléthyl)urée ;
1-(1-(6,7-difluoro-1-oxo-1,2-dihydroisoquinoléin-4-yl)éthyl)-1-méthyl-3-(3,4,5-trifluorobenzyl)urée ;
1-(1-(6,7-difluoro-1-oxo-1,2-dihydroisoquinoléin-4-yl)éthyl)-1-méthyl-3-(3-chloro-4-fluorobenzyl)urée ;
1-(1-(6,7-difluoro-1-oxo-1,2-dihydroisoquinoléin-4-yl)éthyl)-3-(1H-indol-6-yl)-1-méthylurée ;
1-(1-(7,8-difluoro-1-oxo-1,2-dihydroisoquinoléin-4-yl)éthyl)-1-méthyl-3-((S)-1-phényléthyl)urée ;
1-(1-(7,8-difluoro-1-oxo-1,2-dihydroisoquinoléin-4-yl)éthyl)-1-méthyl-3-((R)-1-phényléthyl)urée ;
1-(1-(7,8-difluoro-1-oxo-1,2-dihydroisoquinoléin-4-yl)éthyl)-3-(3,4-difluorobenzyl)-1-méthylurée ;
1-(1-(7,8-difluoro-1-oxo-1,2-dihydroisoquinoléin-4-yl)éthyl)-3-(4-fluorobenzyl)-1-méthylurée ;
1-(1-(7,8-difluoro-1-oxo-1,2-dihydroisoquinoléin-4-yl)éthyl)-3-(3,4,5-trifluorobenzyl)-1-méthylurée ;
3-(3-chloro-4-fluorobenzyl)-1-(1-(7,8-difluoro-1-oxo-1,2-dihydroisoquinoléin-4-yl)éthyl)-1-méthylurée ;
1-(1-(7,8-difluoro-1-oxo-1,2-dihydroisoquinoléin-4-yl)éthyl)-3-(1H-indol-6-yl)-1-méthylurée ;
1-(1-(6,7-difluoro-1-oxo-1,2-dihydroisoquinoléin-4-yl)éthyl)-3-(3-(difluorométhyl)-4-fluorophényl)-1-méthylurée ;
1-(1-(7,8-difluoro-1-oxo-1,2-dihydroisoquinoléin-4-yl)éthyl)-3-(3-(difluorométhyl)-4-fluorophényl)-1-méthylurée ;
ou son sel, solvate, dérivé isotopiquement marqués, stéréoisomère, ou tautomère, ou de n'importe quels mélanges de ceux-ci.

11. Composé selon la revendication 1, qui est sélectionné dans le groupe constitué de la :
(R)-3-(3-chloro-4-fluorophényl)-1-méthyl-1-(1-(1-oxo-1,2-dihydroisoquinoléin-4-yl)éthyl)urée ;
(S)-3-(3-chloro-4-fluorophényl)-1-méthyl-1-(1-(1-oxo-1,2-dihydroisoquinoléin-4-yl)éthyl)urée ;
(R)-3-(4-fluoro-3-méthylphényl)-1-méthyl-1-(1-(1-oxo-1,2-dihydroisoquinoléin-4-yl)éthyl)urée ;
(R)-3-(3-chloro-4-fluorophényl)-1-(1-(1-oxo-1,2-dihydroisoquinoléin-4-yl)éthyl)-1-propylurée ;
(R)-3-(4-fluoro-3-méthylphényl)-1-(1-(1-oxo-1,2-dihydroisoquinoléin-4-yl)éthyl)-1-propylurée ;
(R)-3-(4-fluoro-3-(trifluorométhyl)phényle)-1-méthyl-1-(1-(1-oxo-1,2-dihydroisoquinoléin-4-yl)éthyl)urée ;
(R)-3-(4-fluorophényl)-1-méthyl-1-(1-(1-oxo-1,2-dihydroisoquinoléin-4-yl)éthyl)urée ;
(R)-1-méthyl-1-(1-(1-oxo-1,2-dihydroisoquinoléin-4-yl)éthyl)-3-(3,4,5-trifluorophényl)urée ;
(R)-3-(3,4-difluorophényl)-1-méthyl-1-(1-(1-oxo-1,2-dihydroisoquinoléin-4-yl)éthyl)urée ;
(R)-3-(3-fluorophényl)-1-méthyl-1-(1-(1-oxo-1,2-dihydroisoquinoléin-4-yl)éthyl)urée ;
(R)-3-(2-chloropyridin-4-yl)-1-méthyl-1-(1-(1-oxo-1,2-dihydroisoquinoléin-4-yl)éthyl)urée ;
(R)-1-méthyl-1-(1-(1-oxo-1,2-dihydroisoquinoléin-4-yl)éthyl)-3-(2-(trifluorométhyl)pyridin-4-yl)urée ;
(R)-3-(3-chloro-4-fluorophényl)-1-(2,2-difluoroéthyl)-1-(1-(1-oxo-1,2-dihydroisoquinoléin-4-yl)éthyl)urée ;
(R)-1-(2,2-difluoroéthyl)-3-(4-fluoro-3-méthylphényl)-1-(1-(1-oxo-1,2-dihydroisoquinoléin-4-yl)éthyl)urée ;
(R)-3-(3-cyano-4-fluorophényl)-1-méthyl-1-(1-(1-oxo-1,2-dihydroisoquinoléin-4-yl)éthyl)urée ;
(R)-3-(3-chloro-4-fluorophényl)-1-(éthyl-d5)-1-(1-(1-oxo-1,2-dihydroisoquinoléin-4-yl)éthyl)urée ;
(R)-3-(3-chloro-4-fluorophényl)-1-(1-(1-méthoxyisoquinoléin-4-yl)éthyl)-1-(2,2,2-trifluoroéthyl)urée ;
(R)-3-(4-chlorophényl)-1-méthyl-1-(1-(1-oxo-1,2-dihydroisoquinoléin-4-yl)éthyl)urée ;
(R)-3-(4-bromophényl)-1-méthyl-1-(1-(1-oxo-1,2-dihydroisoquinoléin-4-yl)éthyl)urée ;
(R)-1-(3-chloro-4-fluorophényl)-3-(1-(1-oxo-1,2-dihydroisoquinoléin-4-yl)éthyl)urée ;
(S)-1-(3-chloro-4-fluorophényl)-3-(1-(1-oxo-1,2-dihydroisoquinoléin-4-yl)éthyl)urée ;
(R)-3-(3-chloro-4-fluorophényl)-1-éthyl-1-(1-(1-méthoxyisoquinoléin-4-yl)éthyl)urée ;
(S)-3-(3-chloro-4-fluorophényl)-1-éthyl-1-(1-(1-méthoxyisoquinoléin-4-yl)éthyl)urée ;
(R)-3-(3-chloro-4-fluorophényl)-1-éthyl-1-(1-(1-oxo-1,2-dihydroisoquinoléin-4-yl)éthyl)urée ;
(S)-3-(3-chloro-4-fluorophényl)-1-éthyl-1-(1-(1-oxo-1,2-dihydroisoquinoléin-4-yl)éthyl)urée ;
(R)-3-(3-chloro-4-fluorophényl)-1-éthyl-1-(1-(1-méthoxyisoquinoléin-4-yl)propyl)urée ;
(S)-3-(3-chloro-4-fluorophényl)-1-éthyl-1-(1-(1-méthoxyisoquinoléin-4-yl)propyl)urée ;
(R)-3-(3-chloro-4-fluorophényl)-1-éthyl-1-(1-(1-oxo-1,2-dihydroisoquinoléin-4-yl)propyl)urée ;
(S)-3-(3-chloro-4-fluorophényl)-1-éthyl-1-(1-(1-oxo-1,2-dihydroisoquinoléin-4-yl)propyl)urée ;
(R)-3-(3-chloro-4-fluorophényl)-1-éthyl-1-(1-(1-méthoxyisoquinoléin-4-yl)-2-méthylpropyl)urée ;
(S)-3-(3-chloro-4-fluorophényl)-1-éthyl-1-(1-(1-méthoxyisoquinoléin-4-yl)-2-méthylpropyl)urée ;
(R)-3-(3-chloro-4-fluorophényl)-1-(cyclopropyl(1-méthoxyisoquinoléin-4-yl)méthyl)-1-éthylurée ;
(S)-3-(3-chloro-4-fluorophényl)-1-(cyclopropyl(1-méthoxyisoquinoléin-4-yl)méthyl)-1-éthylurée ;
(R)-3-(3-chloro-4-fluorophényl)-1-(cyclopropyl(1-oxo-1,2-dihydroisoquinoléin-4-yl)méthyl)-1-éthylurée ;
(S)-3-(3-chloro-4-fluorophényl)-1-(cyclopropyl(1-oxo-1,2-dihydroisoquinoléin-4-yl)méthyl)-1-éthylurée ;
(R)-3-(3-chloro-4-fluorophényl)-1-éthyl-1-(2-méthyl-1-(1-oxo-1,2-dihydroisoquinoléin-4-yl)propyl)urée ;
(S)-3-(3-chloro-4-fluorophényl)-1-éthyl-1-(2-méthyl-1-(1-oxo-1,2-dihydroisoquinoléin-4-yl)propyl)urée ;
(R)-3-(3-chloro-4-fluorophényl)-1-cyclopropyl-1-(1-(1-méthoxyisoquinoléin-4-yl)éthyl)urée ;
(S)-3-(3-chloro-4-fluorophényl)-1-cyclopropyl-1-(1-(1-méthoxyisoquinoléin-4-yl)éthyl)urée ;
(R)-3-(3-chloro-4-fluorophényl)-1-éthyl-1-((1-méthoxyisoquinoléin-4-yl)(phényle)méthyl)urée ;
(S)-3-(3-chloro-4-fluorophényl)-1-éthyl-1-((1-méthoxyisoquinoléin-4-yl)(phényle)méthyl)urée ;
(R)-1-butyl-3-(3-chloro-4-fluorophényl)-1-(1-(1-méthoxyisoquinoléin-4-yl)éthyl)urée ;
(S)-1-butyl-3-(3-chloro-4-fluorophényl)-1-(1-(1-méthoxyisoquinoléin-4-yl)éthyl)urée ;
(R)-3-(3-chloro-4-fluorophényl)-1-(cyclopropylméthyl)-1-(1-(1-méthoxyisoquinoléin-4-yl)éthyl)urée ;
(S)-3-(3-chloro-4-fluorophényl)-1-(cyclopropylméthyl)-1-(1-(1-méthoxyisoquinoléin-4-yl)éthyl)urée ;
(R)-3-(3-chloro-4-fluorophényl)-1-(2-méthoxyéthyl)-1-(1-(1-oxo-1,2-dihydroisoquinoléin-4-yl)éthyl)urée ;
(S)-3-(3-chloro-4-fluorophényl)-1-(2-méthoxyéthyl)-1-(1-(1-oxo-1,2-dihydroisoquinoléin-4-yl)éthyl)urée ;
(R)-3-(3-chloro-4-fluorophényl)-1-cyclopropyl-1-(1-(1-oxo-1,2-dihydroisoquinoléin-4-yl)éthyl)urée ;
(S)-3-(3-chloro-4-fluorophényl)-1-cyclopropyl-1-(1-(1-oxo-1,2-dihydroisoquinoléin-4-yl)éthyl)urée ;
(R)-3-(3-chloro-4-fluorophényl)-1-éthyl-1-((1-oxo-1,2-dihydroisoquinoléin-4-yl)(phényle)méthyl)urée ;
(S)-3-(3-chloro-4-fluorophényl)-1-éthyl-1-((1-oxo-1,2-dihydroisoquinoléin-4-yl)(phényle)méthyl)urée ;
(R)-3-(3-chloro-4-fluorophényl)-1-(2-méthoxyéthyl)-1-(1-(1-méthoxyisoquinoléin-4-yl)éthyl)urée ;
(S)-3-(3-chloro-4-fluorophényl)-1-(2-méthoxyéthyl)-1-(1-(1-méthoxyisoquinoléin-4-yl)éthyl)urée ;
(R)-3-(3-chloro-4-fluorophényl)-1-éthyl-1-(2,2,2-trifluoro-1-(1-oxo-1,2-dihydroisoquinoléin-4-yl)éthyl)urée ;
(S)-3-(3-chloro-4-fluorophényl)-1-éthyl-1-(2,2,2-trifluoro-1-(1-oxo-1,2-dihydroisoquinoléin-4-yl)éthyl)urée ;
(R)-3-(3-chloro-4-fluorophényl)-1-éthyl-1-(2,2,2-trifluoro-1-(1-méthoxyisoquinoléin-4-yl)éthyl)urée ;
(S)-3-(3-chloro-4-fluorophényl)-1-éthyl-1-(2,2,2-trifluoro-1-(1-méthoxyisoquinoléin-4-yl)éthyl)urée ;
(R)-3-(3-chloro-4-fluorophényl)-1-(3-méthoxypropyl)-1-(1-(1-oxo-1,2-dihydroisoquinoléin-4-yl)éthyl)urée ;
(S)-3-(3-chloro-4-fluorophényl)-1-(3-méthoxypropyl)-1-(1-(1-oxo-1,2-dihydroisoquinoléin-4-yl)éthyl)urée ;
(R)-3-(3-chloro-4-fluorophényl)-1-(1-(1-méthoxyisoquinoléin-4-yl)éthyl)-1-(3-méthoxypropyl)urée ;
(S)-3-(3-chloro-4-fluorophényl)-1-(1-(1-méthoxyisoquinoléin-4-yl)éthyl)-1-(3-méthoxypropyl)urée ;
(R)-1-butyl-3-(3-chloro-4-fluorophényl)-1-(1-(1-oxo-1,2-dihydroisoquinoléin-4-yl)éthyl)urée ;
(S)-1-butyl-3-(3-chloro-4-fluorophényl)-1-(1-(1-oxo-1,2-dihydroisoquinoléin-4-yl)éthyl)urée ;
(R)-3-(3-chloro-4-fluorophényl)-1-(cyclopropylméthyl)-1-(1-(1-oxo-1,2-dihydroisoquinoléin-4-yl)éthyl)urée ;
(S)-3-(3-chloro-4-fluorophényl)-1-(cyclopropylméthyl)-1-(1-(1-oxo-1,2-dihydroisoquinoléin-4-yl)éthyl)urée ;
du (R)-3-(3-(3-chloro-4-fluorophényl)-1-(1-(1-oxo-1,2-dihydroisoquinoléin-4-yl)éthyl)uréido)-N,N-diméthylpropanamide ;
(S)-3-(3-(3-chloro-4-fluorophényl)-1-(1-(1-oxo-1,2-dihydroisoquinoléin-4-yl)éthyl)uréido)-N,N-diméthylpropanamide ;
de la (R)-3-(3-chloro-4-fluorophényl)-1-(2-(méthylsulfonyl)éthyl)-1-(1-(1-oxo-1,2-dihydroisoquinoléin-4-yl)éthyl)urée ;
(S)-3-(3-chloro-4-fluorophényl)-1-(2-(méthylsulfonyl)éthyl)-1-(1-(1-oxo-1,2-dihydroisoquinoléin-4-yl)éthyl)urée ;
(R)-3-(3-chloro-4-fluorophényl)-1-(2-éthoxyéthyl)-1-(1-(1-oxo-1,2-dihydroisoquinoléin-4-yl)éthyl)urée ;
(S)-3-(3-chloro-4-fluorophényl)-1-(2-éthoxyéthyl)-1-(1-(1-oxo-1,2-dihydroisoquinoléin-4-yl)éthyl)urée ;
(R)-3-(3-chloro-4-fluorophényl)-1-(2-cyanoéthyl)-1-(1-(1-oxo-1,2-dihydroisoquinoléin-4-yl)éthyl)urée ;
(S)-3-(3-chloro-4-fluorophényl)-1-(2-cyanoéthyl)-1-(1-(1-oxo-1,2-dihydroisoquinoléin-4-yl)éthyl)urée ;
(R)-3-(3-chloro-4-fluorophényl)-1-(3-cyanopropyl)-1-(1-(1-oxo-1,2-dihydroisoquinoléin-4-yl)éthyl)urée ;
(S)-3-(3-chloro-4-fluorophényl)-1-(3-cyanopropyl)-1-(1-(1-oxo-1,2-dihydroisoquinoléin-4-yl)éthyl)urée ;
du (R)-3-(3-(3-chloro-4-fluorophényl)-1-(1-(1-oxo-1,2-dihydroisoquinoléin-4-yl)éthyl)uréido)-N-méthylpropanamide ;
(S)-3-(3-(3-chloro-4-fluorophényl)-1-(1-(1-oxo-1,2-dihydroisoquinoléin-4-yl)éthyl)uréido)-N-méthylpropanamide ;
(R)-2-(3-(3-chloro-4-fluorophényl)-1-(1-(1-oxo-1,2-dihydroisoquinoléin-4-yl)éthyl)uréido)éthane-1-sulfonamide ;
(S)-2-(3-(3-chloro-4-fluorophényl)-1-(1-(1-oxo-1,2-dihydroisoquinoléin-4-yl)éthyl)uréido)éthane-1-sulfonamide ;
de la (R)-3-(3-chloro-4-fluorophényl)-1-(2-(2-méthoxyéthoxy)éthyl)-1-(1-(1-méthoxyisoquinoléin-4-yl)éthyl)urée ;
(S)-3-(3-chloro-4-fluorophényl)-1-(2-(2-méthoxyéthoxy)éthyl)-1-(1-(1-méthoxyisoquinoléin-4-yl)éthyl)urée ;
(R)-3-(3-chloro-4-fluorophényl)-1-(2-(2-méthoxyéthoxy)éthyl)-1-(1-(1-oxo-1,2-dihydroisoquinoléin-4-yl)éthyl)urée ;
(S)-3-(3-chloro-4-fluorophényl)-1-(2-(2-méthoxyéthoxy)éthyl)-1-(1-(1-oxo-1,2-dihydroisoquinoléin-4-yl)éthyl)urée ;
(R)-3-(3-chloro-4-fluorophényl)-1-(2-éthoxyéthyl)-1-(1-(1-méthoxyisoquinoléin-4-yl)éthyl)urée ;
(S)-3-(3-chloro-4-fluorophényl)-1-(2-éthoxyéthyl)-1-(1-(1-méthoxyisoquinoléin-4-yl)éthyl)urée ;
(R)-3-(3-chloro-4-fluorophényl)-1-(3-hydroxypropyl)-1-(1-(1-oxo-1,2-dihydroisoquinoléin-4-yl)éthyl)urée ;
(S)-3-(3-chloro-4-fluorophényl)-1-(3-hydroxypropyl)-1-(1-(1-oxo-1,2-dihydroisoquinoléin-4-yl)éthyl)urée ;
(R)-3-(3-chloro-4-fluorophényl)-1-méthyl-1-(1-(1-((1-méthyl-1H-1,2,4-triazol-3-yl)méthoxy)isoquinoléin-4-yl)éthyl)urée ;
(S)-3-(3-chloro-4-fluorophényl)-1-méthyl-1-(1-(1-((1-méthyl-1H-1,2,4-triazol-3-yl)méthoxy)isoquinoléin-4-yl)éthyl)urée ;
du (R)-N-(2-(3-(3-chloro-4-fluorophényl)-1-(1-(1-oxo-1,2-dihydroisoquinoléin-4-yl)éthyl)uréido)éthyl)acétamide ;
(S)-N-(2-(3-(3-chloro-4-fluorophényl)-1-(1-(1-oxo-1,2-dihydroisoquinoléin-4-yl)éthyl)uréido)éthyl)acétamide ;
de la (R)-3-(3-chloro-4-fluorophényl)-1-(1-(1-oxo-1,2-dihydroisoquinoléin-4-yl)éthyl)-1-((tétrahydro-2H-pyran-4-yl)méthyl)urée ;
(S)-3-(3-chloro-4-fluorophényl)-1-(1-(1-oxo-1,2-dihydroisoquinoléin-4-yl)éthyl)-1-((tétrahydro-2H-pyran-4-yl)méthyl)urée ;
(R)-3-(3-chloro-4-fluorophényl)-1-isobutyl-1-(1-(1-oxo-1,2-dihydroisoquinoléin-4-yl)éthyl)urée ;
(S)-3-(3-chloro-4-fluorophényl)-1-isobutyl-1-(1-(1-oxo-1,2-dihydroisoquinoléin-4-yl)éthyl)urée ;
(R)-3-(3-chloro-4-fluorophényl)-1-méthyl-1-(1-(5-(méthylamino)pyrido[3,4-b]pyrazin-8-yl)éthyl)urée ;
(S)-3-(3-chloro-4-fluorophényl)-1-méthyl-1-(1-(5-(méthylamino)pyrido[3,4-b]pyrazin-8-yl)éthyl)urée ;
(R)-3-(3-chloro-4-fluorophényl)-1-(1-(5-méthoxypyrido[3,4-b]pyrazin-8-yl)éthyl)-1-méthylurée ;
(S)-3-(3-chloro-4-fluorophényl)-1-(1-(5-méthoxypyrido[3,4-b]pyrazin-8-yl)éthyl)-1-méthylurée ;
(R)-3-(3-chloro-4-fluorophényl)-1-(3-hydroxypropyl)-1-(1-(1-méthoxyisoquinoléin-4-yl)éthyl)urée ;
(S)-3-(3-chloro-4-fluorophényl)-1-(3-hydroxypropyl)-1-(1-(1-méthoxyisoquinoléin-4-yl)éthyl)urée ;
(R)-1-(1-(1-((1H-1,2,4-triazol-3-yl)méthoxy)isoquinoléin-4-yl)éthyl)-3-(3-chloro-4-fluorophényl)-1-méthylurée ;
(S)-1-(1-(1-((1H-1,2,4-triazol-3-yl)méthoxy)isoquinoléin-4-yl)éthyl)-3-(3-chloro-4-fluorophényl)-1-méthylurée ;
(R)-(3-(3-chloro-4-fluorophényl)-1-(1-(1-méthoxyisoquinoléin-4-yl)éthyl)-1-(2,2,2-trifluoroéthyl)urée ;
(S)-(3-(3-chloro-4-fluorophényl)-1-(1-(1-méthoxyisoquinoléin-4-yl)éthyl)-1-(2,2,2-trifluoroéthyl)urée ;
(R)-3-(3-chloro-4-fluorophényl)-1-éthyl-1-(1-(1-oxo-1,2-dihydro-2,7-naphtyridin-4-yl)éthyl)urée ;
(S)-3-(3-chloro-4-fluorophényl)-1-éthyl-1-(1-(1-oxo-1,2-dihydro-2,7-naphtyridin-4-yl)éthyl)urée ;
(R)-3-(3-chloro-4-fluorophényl)-1-(1-(1-oxo-1,2-dihydroisoquinoléin-4-yl)éthyl)-1-(2-((2,2,2-trifluoroéthyl)amino)éthyl)urée ;
(S)-3-(3-chloro-4-fluorophényl)-1-(1-(1-oxo-1,2-dihydroisoquinoléin-4-yl)éthyl)-1-(2-((2,2,2-trifluoroéthyl)amino)éthyl)urée ;
(R)-1-(1-(8-chloro-1-oxo-1,2-dihydroisoquinoléin-4-yl)éthyl)-3-(3-chloro-4-fluorophényl)-1-méthylurée ;
(S)-1-(1-(8-chloro-1-oxo-1,2-dihydroisoquinoléin-4-yl)éthyl)-3-(3-chloro-4-fluorophényl)-1-méthylurée ;
(R)-3-(3-chloro-4-fluorophényl)-1-(1-(6-fluoro-1-oxo-1,2-dihydroisoquinoléin-4-yl)éthyl)-1-méthylurée ;
(S)-3-(3-chloro-4-fluorophényl)-1-(1-(6-fluoro-1-oxo-1,2-dihydroisoquinoléin-4-yl)éthyl)-1-méthylurée ;
(R)-3-(3-chloro-4-fluorophényl)-1-(1-(6-chloro-1-oxo-1,2-dihydroisoquinoléin-4-yl)éthyl)-1-méthylurée ;
(S)-3-(3-chloro-4-fluorophényl)-1-(1-(6-chloro-1-oxo-1,2-dihydroisoquinoléin-4-yl)éthyl)-1-méthylurée ;
(R)-3-(3-chloro-4-fluorophényl)-1-(1-(7-méthoxy-1-oxo-1,2-dihydroisoquinoléin-4-yl)éthyl)-1-méthylurée ;
(S)-3-(3-chloro-4-fluorophényl)-1-(1-(7-méthoxy-1-oxo-1,2-dihydroisoquinoléin-4-yl)éthyl)-1-méthylurée ;
(R)-3-(3-chloro-4-fluorophényl)-1-(1-(6-méthoxy-1-oxo-1,2-dihydroisoquinoléin-4-yl)éthyl)-1-méthylurée ;
(S)-3-(3-chloro-4-fluorophényl)-1-(1-(6-méthoxy-1-oxo-1,2-dihydroisoquinoléin-4-yl)éthyl)-1-méthylurée ;
(R)-3-(3-chloro-4-fluorophényl)-1-méthyl-1-(1-(2-méthyl-1-oxo-1,2-dihydroisoquinoléin-4-yl)éthyl)urée ;
(S)-3-(3-chloro-4-fluorophényl)-1-méthyl-1-(1-(2-méthyl-1-oxo-1,2-dihydroisoquinoléin-4-yl)éthyl)urée ;
(R)-3-(3-chloro-4-fluorophényl)-1-méthyl-1-(1-(2-éthyl-1-oxo-1,2-dihydroisoquinoléin-4-yl)éthyl)urée ;
(S)-3-(3-chloro-4-fluorophényl)-1-méthyl-1-(1-(2-éthyl-1-oxo-1,2-dihydroisoquinoléin-4-yl)éthyl)urée ;
(R)-3-(3-chloro-4-fluorophényl)-1-méthyl-1-(1-(2-propyl-1-oxo-1,2-dihydroisoquinoléin-4-yl)éthyl)urée ;
(S)-3-(3-chloro-4-fluorophényl)-1-méthyl-1-(1-(2-propyl-1-oxo-1,2-dihydroisoquinoléin-4-yl)éthyl)urée ;
(R)-3-(3-chloro-4-fluorophényl)-1-méthyl-1-(1-(2-isopropyl-1-oxo-1,2-dihydroisoquinoléin-4-yl)éthyl)urée ;
(S)-3-(3-chloro-4-fluorophényl)-1-méthyl-1-(1-(2-isopropyl-1-oxo-1,2-dihydroisoquinoléin-4-yl)éthyl)urée ;
(R)-3-(3-chloro-4-fluorophényl)-1-méthyl-1-(1-(2-cyclopropyl-1-oxo-1,2-dihydroisoquinoléin-4-yl)éthyl)urée ;
(S)-3-(3-chloro-4-fluorophényl)-1-méthyl-1-(1-(2-cyclopropyl-1-oxo-1,2-dihydroisoquinoléin-4-yl)éthyl)urée ;
(R)-3-(3-chloro-4-fluorophényl)-1-(cyclopentylméthyl)-1-(1-(1-oxo-1,2-dihydroisoquinoléin-4-yl)éthyl)urée ;
(S)-3-(3-chloro-4-fluorophényl)-1-(cyclopentylméthyl)-1-(1-(1-oxo-1,2-dihydroisoquinoléin-4-yl)éthyl)urée ;
(R)-3-(3-chloro-4-fluorophényl)-1-(cyclohexylméthyl)-1-(1-(1-oxo-1,2-dihydroisoquinoléin-4-yl)éthyl)urée ;
(S)-3-(3-chloro-4-fluorophényl)-1-(cyclohexylméthyl)-1-(1-(1-oxo-1,2-dihydroisoquinoléin-4-yl)éthyl)urée ;
(R)-3-(3-chloro-4-fluorophényl)-1-(2-hydroxyéthyl)-1-(1-(1-oxo-1,2-dihydroisoquinoléin-4-yl)éthyl)urée ;
(S)-3-(3-chloro-4-fluorophényl)-1-(2-hydroxyéthyl)-1-(1-(1-oxo-1,2-dihydroisoquinoléin-4-yl)éthyl)urée ;
(R)-3-(3-chloro-4-fluorophényl)-1-(2-((2,2-difluoroéthyl)amino)éthyl)-1-(1-(1-oxo-1,2-dihydroisoquinoléin-4-yl)éthyl)urée ;
(S)-3-(3-chloro-4-fluorophényl)-1-(2-((2,2-difluoroéthyl)amino)éthyl)-1-(1-(1-oxo-1,2-dihydroisoquinoléin-4-yl)éthyl)urée ;
(R)-1-((1-acetylpiperidin-4-yl)méthyl)-3-(3-chloro-4-fluorophényl)-1-(1-(1-oxo-1,2-dihydroisoquinoléin-4-yl)éthyl)urée ;
(S)-1-((1-acetylpiperidin-4-yl)méthyl)-3-(3-chloro-4-fluorophényl)-1-(1-(1-oxo-1,2-dihydroisoquinoléin-4-yl)éthyl)urée ;
(R)-3-(3-chloro-4-fluorophényl)-1-((1-(méthylsulfonyl)piperidin-4-yl)méthyl)-1-(1-(1-oxo-1,2-dihydroisoquinoléin-4-yl)éthyl)urée ;
(S)-3-(3-chloro-4-fluorophényl)-1-((1-(méthylsulfonyl)piperidin-4-yl)méthyl)-1-(1-(1-oxo-1,2-dihydroisoquinoléin-4-yl)éthyl)urée ;
(R)-3-(3-chloro-4-fluorophényl)-1-(3-hydroxy-2,2-diméthylpropyl)-1-(1-(1-oxo-1,2-dihydroisoquinoléin-4-yl)éthyl)urée ;
(S)-3-(3-chloro-4-fluorophényl)-1-(3-hydroxy-2,2-diméthylpropyl)-1-(1-(1-oxo-1,2-dihydroisoquinoléin-4-yl)éthyl)urée ;
3-(3-chloro-4-fluorophényl)-1-((R)-3-hydroxybutyl)-1-(1(R)-(1-oxo-1,2-dihydroisoquinoléin-4-yl)éthyl)urée ;
3-(3-chloro-4-fluorophényl)-1-((R)-3-hydroxybutyl)-1-(1(S)-(1-oxo-1,2-dihydroisoquinoléin-4-yl)éthyl)urée ;
3-(3-chloro-4-fluorophényl)-1-((S)-3-hydroxybutyl)-1-(1(R)-(1-oxo-1,2-dihydroisoquinoléin-4-yl)éthyl)urée ;
3-(3-chloro-4-fluorophényl)-1-((S)-3-hydroxybutyl)-1-(1(S)-(1-oxo-1,2-dihydroisoquinoléin-4-yl)éthyl)urée ;
3-(3-chloro-4-fluorophényl)-1-((R)-2-hydroxypropyl)-1-(1(R)-(1-oxo-1,2-dihydroisoquinoléin-4-yl)éthyl)urée ;
3-(3-chloro-4-fluorophényl)-1-((R)-2-hydroxypropyl)-1-((S)₁-(1-oxo-1,2-dihydroisoquinoléin-4-yl)éthyl)urée ;
3-(3-chloro-4-fluorophényl)-1-((S)-2-hydroxypropyl)-1-(1(R)-(1-oxo-1,2-dihydroisoquinoléin-4-yl)éthyl)urée ;
3-(3-chloro-4-fluorophényl)-1-((S)-2-hydroxypropyl)-1-(1(S)-(1-oxo-1,2-dihydroisoquinoléin-4-yl)éthyl)urée ;
(R)-3-(3-chloro-4-fluorophényl)-1-méthyl-1-(1-(3-méthyl-1-oxo-1,2-dihydroisoquinoléin-4-yl)éthyl)urée ;
(S)-3-(3-chloro-4-fluorophényl)-1-méthyl-1-(1-(3-méthyl-1-oxo-1,2-dihydroisoquinoléin-4-yl)éthyl)urée ;
de l'acide (R)-4-(3-(3-chloro-4-fluorophényl)-1-(1-(1-oxo-1,2-dihydroisoquinoléin-4-yl)éthyl)uréido)butanoïque ;
de l'acide (S)-4-(3-(3-chloro-4-fluorophényl)-1-(1-(1-oxo-1,2-dihydroisoquinoléin-4-yl)éthyl)urde l'éido)butanoïque ;
de la (R)-3-(3-chloro-4-fluorophényl)-1-méthyl-1-(1-(1-(méthylamino)isoquinoléin-4-yl)éthyl)urée ;
(S)-3-(3-chloro-4-fluorophényl)-1-méthyl-1-(1-(1-(méthylamino)isoquinoléin-4-yl)éthyl)urée ;
(R)-3-(3-chloro-4-fluorophényl)-1-méthyl-1-(1-(1-(diméthylamino)isoquinoléin-4-yl)éthyl)urée ;
(S)-3-(3-chloro-4-fluorophényl)-1-méthyl-1-(1-(1-(diméthylamino)isoquinoléin-4-yl)éthyl)urée ;
(R)-3-(4-fluoro-3-méthylphényl)-1-méthyl-1-(1-(3-méthyl-1-oxo-1,2-dihydroisoquinoléin-4-yl)éthyl)urée ;
(S)-3-(4-fluoro-3-méthylphényl)-1-méthyl-1-(1-(3-méthyl-1-oxo-1,2-dihydroisoquinoléin-4-yl)éthyl)urée ;
(R)-1-(1-(1-aminoisoquinoléin-4-yl)éthyl)-3-(3-chloro-4-fluorophényl)-1-méthylurée ;
(S)-1-(1-(1-aminoisoquinoléin-4-yl)éthyl)-3-(3-chloro-4-fluorophényl)-1-méthylurée ;
(R)-3-(3-chloro-4-fluorophényl)-1-(1-(1-(éthylamino)isoquinoléin-4-yl)éthyl)-1-méthylurée ;
(S)-3-(3-chloro-4-fluorophényl)-1-(1-(1-(éthylamino)isoquinoléin-4-yl)éthyl)-1-méthylurée ;
(R)-3-(3-chloro-4-fluorophényl)-1-(1-(1-((2-hydroxyéthyl)amino)isoquinoléin-4-yl)éthyl)-1-méthylurée ;
(S)-3-(3-chloro-4-fluorophényl)-1-(1-(1-((2-hydroxyéthyl)amino)isoquinoléin-4-yl)éthyl)-1-méthylurée ;
(R)-3-(3-chloro-4-fluorophényl)-1-méthyl-1-(1-(1-(((1-méthyl-1H-1,2,4-triazol-3-yl)méthyl)amino)isoquinoléin-4-yl)éthyl)urée ;
(S)-3-(3-chloro-4-fluorophényl)-1-méthyl-1-(1-(1-(((1-méthyl-1H-1,2,4-triazol-3-yl)méthyl)amino)isoquinoléin-4-yl)éthyl)urée ;
(R)-1-(1-(1-(((1H-1,2,4-triazol-3-yl)méthyl)amino)isoquinoléin-4-yl)éthyl)-3-(3-chloro-4-fluorophényl)-1-méthylurée ;
(S)-1-(1-(1-((1H-1,2,4-triazol-3-yl)méthyl)amino)isoquinoléin-4-yl)éthyl)-3-(3-chloro-4-fluorophényl)-1-méthylurée ;
(R)-3-(3-chloro-4-fluorophényl)-1-(3-hydroxypropyl)-1-(1-(1-oxo-1,2-dihydro-2,7-naphtyridin-4-yl)éthyl)urée ;
(S)-3-(3-chloro-4-fluorophényl)-1-(3-hydroxypropyl)-1-(1-(1-oxo-1,2-dihydro-2,7-naphtyridin-4-yl)éthyl)urée ;
(R)-3-(3-chloro-4-fluorophényl)-1-isobutyl-1-(1-(1-oxo-1,2-dihydro-2,7-naphtyridin-4-yl)éthyl)urée ;
(S)-3-(3-chloro-4-fluorophényl)-1-isobutyl-1-(1-(1-oxo-1,2-dihydro-2,7-naphtyridin-4-yl)éthyl)urée ;
(R)-3-(3-chloro-4-fluorophényl)-1-(3-hydroxypropyl)-1-(1-(1-((1-méthyl-1H-1,2,4-triazol-3-yl)méthoxy)isoquinoléin-4-yl)éthyl)urée ;
(S)-3-(3-chloro-4-fluorophényl)-1-(3-hydroxypropyl)-1-(1-(1-((1-méthyl-1H-1,2,4-triazol-3-yl)méthoxy)isoquinoléin-4-yl)éthyl)urée ;
(R)-1-(1-(1-((1H-1,2,4-triazol-3-yl)méthoxy)isoquinoléin-4-yl)éthyl)-3-(3-chloro-4-fluorophényl)-1-(3-hydroxypropyl)urée ;
(S)-1-(1-(1-((1H-1,2,4-triazol-3-yl)méthoxy)isoquinoléin-4-yl)éthyl)-3-(3-chloro-4-fluorophényl)-1-(3-hydroxypropyl)urée ;
(R)-1-(1-(1-(((2H-1,2,3-triazol-4-yl)méthyl)amino)isoquinoléin-4-yl)éthyl)-3-(3-chloro-4-fluorophényl)-1-méthylurée ;
(S)-1-(1-(1-(((2H-1,2,3-triazol-4-yl)méthyl)amino)isoquinoléin-4-yl)éthyl)-3-(3-chloro-4-fluorophényl)-1-méthylurée ;
(R)-3-(3-chloro-4-fluorophényl)-1-(1-(7-fluoro-1-oxo-1,2-dihydroisoquinoléin-4-yl)éthyl)-1-méthylurée ;
(S)-3-(3-chloro-4-fluorophényl)-1-(1-(7-fluoro-1-oxo-1,2-dihydroisoquinoléin-4-yl)éthyl)-1-méthylurée ;
(R)-1-((1H-1,2,3-triazol-4-yl)méthyl)-3-(3-chloro-4-fluorophényl)-1-(1-(1-oxo-1,2-dihydroisoquinoléin-4-yl)éthyl)urée ;
(S)-1-((1H-1,2,3-triazol-4-yl)méthyl)-3-(3-chloro-4-fluorophényl)-1-(1-(1-oxo-1,2-dihydroisoquinoléin-4-yl)éthyl)urée ;
(R)-1-(1-(2-((1H-1,2,4-triazol-3-yl)méthyl)-1-oxo-1,2-dihydroisoquinoléin-4-yl)éthyl)-3-(3-chloro-4-fluorophényl)-1-méthylurée ;
(S)-1-(1-(2-((1H-1,2,4-triazol-3-yl)méthyl)-1-oxo-1,2-dihydroisoquinoléin-4-yl)éthyl)-3-(3-chloro-4-fluorophényl)-1-méthylurée ;
(R)-3-(3-chloro-4-fluorophényl)-1-((2,2-diméthyl-1,3-dioxan-5-yl)méthyl)-1-(1-(1-oxo-1,2-dihydroisoquinoléin-4-yl)éthyl)urée ;
(S)-3-(3-chloro-4-fluorophényl)-1-((2,2-diméthyl-1,3-dioxan-5-yl)méthyl)-1-(1-(1-oxo-1,2-dihydroisoquinoléin-4-yl)éthyl)urée ;
(R)-1-(1-(2-((1H-1,2,3-triazol-4-yl)méthyl)-1-oxo-1,2-dihydroisoquinoléin-4-yl)éthyl)-3-(3-chloro-4-fluorophényl)-1-méthylurée ;
(S)-1-(1-(2-((1H-1,2,3-triazol-4-yl)méthyl)-1-oxo-1,2-dihydroisoquinoléin-4-yl)éthyl)-3-(3-chloro-4-fluorophényl)-1-méthylurée ;
(R)-3-(3-chloro-4-fluorophényl)-1-(1-(6,7-difluoro-1-oxo-1,2-dihydroisoquinoléin-4-yl)éthyl)-1-méthylurée ;
(S)-3-(3-chloro-4-fluorophényl)-1-(1-(6,7-difluoro-1-oxo-1,2-dihydroisoquinoléin-4-yl)éthyl)-1-méthylurée ;
(R)-3-(3-chloro-4-fluorophényl)-1-(1-(6,7-difluoro-1-oxo-1,2-dihydroisoquinoléin-4-yl)éthyl)-1-isobutylurée ;
(S)-3-(3-chloro-4-fluorophényl)-1-(1-(6,7-difluoro-1-oxo-1,2-dihydroisoquinoléin-4-yl)éthyl)-1-isobutylurée ;
(R)-3-(3-chloro-4-fluorophényl)-1-((2,2-diméthyl-1,3-dioxan-5-yl)méthyl)-1-(1-(6-fluoro-1-oxo-1,2-dihydroisoquinoléin-4-yl)éthyl)urée ;
(S)-3-(3-chloro-4-fluorophényl)-1-((2,2-diméthyl-1,3-dioxan-5-yl)méthyl)-1-(1-(6-fluoro-1-oxo-1,2-dihydroisoquinoléin-4-yl)éthyl)urée ;
(R)-1-(1-(6,7-difluoro-1-oxo-1,2-dihydroisoquinoléin-4-yl)éthyl)-3-(4-fluorophényl)-1-méthylurée ;
(S)-1-(1-(6,7-difluoro-1-oxo-1,2-dihydroisoquinoléin-4-yl)éthyl)-3-(4-fluorophényl)-1-méthylurée ;
(R)-1-(1-(6,7-difluoro-1-oxo-1,2-dihydroisoquinoléin-4-yl)éthyl)-3-(4-fluorophényl)-1-isobutylurée ;
(S)-1-(1-(6,7-difluoro-1-oxo-1,2-dihydroisoquinoléin-4-yl)éthyl)-3-(4-fluorophényl)-1-isobutylurée ;
(R)-3-(3-chloro-4-fluorophényl)-1-(1-(6,7-difluoro-1-oxo-1,2-dihydroisoquinoléin-4-yl)éthyl)-1-(3-hydroxypropyl)urée ;
(S)-3-(3-chloro-4-fluorophényl)-1-(1-(6,7-difluoro-1-oxo-1,2-dihydroisoquinoléin-4-yl)éthyl)-1-(3-hydroxypropyl)urée ;
(R)-1-(1-(6,7-difluoro-1-oxo-1,2-dihydroisoquinoléin-4-yl)éthyl)-3-(4-fluorophényl)-1-(3-hydroxypropyl)urée ;
(S)-1-(1-(6,7-difluoro-1-oxo-1,2-dihydroisoquinoléin-4-yl)éthyl)-3-(4-fluorophényl)-1-(3-hydroxypropyl)urée ;
(R)-3-(3-chloro-4-fluorophényl)-1-(1-(6-fluoro-1-oxo-1,2-dihydroisoquinoléin-4-yl)éthyl)-1-(3-hydroxy-2-(hydroxyméthyl)propyl)urée ;
(S)-3-(3-chloro-4-fluorophényl)-1-(1-(6-fluoro-1-oxo-1,2-dihydroisoquinoléin-4-yl)éthyl)-1-(3-hydroxy-2-(hydroxyméthyl)propyl)urée ;
(R)-3-(3-chloro-4-fluorophényl)-1-(1-(7-fluoro-2-méthyl-1-oxo-1,2-dihydroisoquinoléin-4-yl)éthyl)-1-méthylurée ;
(S)-3-(3-chloro-4-fluorophényl)-1-(1-(7-fluoro-2-méthyl-1-oxo-1,2-dihydroisoquinoléin-4-yl)éthyl)-1-méthylurée ;
(R)-3-(3-chloro-4-fluorophényl)-1-(1-(1-oxo-1,2-dihydroisoquinoléin-4-yl)éthyl)-1-(pyridin-2-ylméthyl)urée ;
(S)-3-(3-chloro-4-fluorophényl)-1-(1-(1-oxo-1,2-dihydroisoquinoléin-4-yl)éthyl)-1-(pyridin-2-ylméthyl)urée ;
(R)-3-(3-chloro-4-fluorophényl)-1-(1-(1-oxo-1,2-dihydroisoquinoléin-4-yl)éthyl)-1-(pyridin-3-ylméthyl)urée ;
(S)-3-(3-chloro-4-fluorophényl)-1-(1-(1-oxo-1,2-dihydroisoquinoléin-4-yl)éthyl)-1-(pyridin-3-ylméthyl)urée ;
(R)-3-(3-chloro-4-fluorophényl)-1-(1-(1-oxo-1,2-dihydroisoquinoléin-4-yl)éthyl)-1-(pyridin-4-ylméthyl)urée ;
(S)-3-(3-chloro-4-fluorophényl)-1-(1-(1-oxo-1,2-dihydroisoquinoléin-4-yl)éthyl)-1-(pyridin-4-ylméthyl)urée ;
(R)-3-(3-chloro-4-fluorophényl)-1-(1-(1-oxo-1,2-dihydroisoquinoléin-4-yl)éthyl)-1-(thiazol-2-ylméthyl)urée ;
(S)-3-(3-chloro-4-fluorophényl)-1-(1-(1-oxo-1,2-dihydroisoquinoléin-4-yl)éthyl)-1-(thiazol-2-ylméthyl)urée ;
(R)-3-(3-chloro-4-fluorophényl)-1-(1-(1-oxo-1,2-dihydroisoquinoléin-4-yl)éthyl)-1-(thiazol-4-ylméthyl)urée ;
(S)-3-(3-chloro-4-fluorophényl)-1-(1-(1-oxo-1,2-dihydroisoquinoléin-4-yl)éthyl)-1-(thiazol-4-ylméthyl)urée ;
(R)-3-(3-chloro-4-fluorophényl)-1-méthyl-1-(1-(1-(méthylsulfonyl)isoquinoléin-4-yl)éthyl)urée ;
(S)-3-(3-chloro-4-fluorophényl)-1-méthyl-1-(1-(1-(méthylsulfonyl)isoquinoléin-4-yl)éthyl)urée ;
du (R)-4-(1-(3-(3-chloro-4-fluorophényl)-1-méthyluréido)éthyl)-N-méthylisoquinoléine-1-carboxamide ;
(S)-4-(1-(3-(3-chloro-4-fluorophényl)-1-méthyluréido)éthyl)-N-méthylisoquinoléine-1-carboxamide ;
(R)-4-(1-(3-(3-chloro-4-fluorophényl)-1-méthyluréido)éthyl)-N,N-diméthylisoquinoléine-1-carboxamide ;
(S)-4-(1-(3-(3-chloro-4-fluorophényl)-1-méthyluréido)éthyl)-N,N-diméthylisoquinoléine-1-carboxamide ;
de la (R)-3-(3-chloro-4-fluorophényl)-1-(1-(1-oxo-1,2-dihydroisoquinoléin-4-yl)éthyl)-1-(pyrimidin-4-ylméthyl)urée ;
(S)-3-(3-chloro-4-fluorophényl)-1-(1-(1-oxo-1,2-dihydroisoquinoléin-4-yl)éthyl)-1-(pyrimidin-4-ylméthyl)urée ;
(R)-3-(3-chloro-4-fluorophényl)-1-(1-(1-oxo-1,2-dihydroisoquinoléin-4-yl)éthyl)-1-(thiazol-5-ylméthyl)urée ;
(S)-3-(3-chloro-4-fluorophényl)-1-(1-(1-oxo-1,2-dihydroisoquinoléin-4-yl)éthyl)-1-(thiazol-5-ylméthyl)urée ;
(R)-3-(3-chloro-4-fluorophényl)-1-(1-(1-(hydroxyméthyl)isoquinoléin-4-yl)éthyl)-1-méthylurée ;
(S)-3-(3-chloro-4-fluorophényl)-1-(1-(1-(hydroxyméthyl)isoquinoléin-4-yl)éthyl)-1-méthylurée ;
du (R)-4-(1-(3-(3-chloro-4-fluorophényl)-1-méthyluréido)éthyl)isoquinoléine-1-carboxamide ;
(S)-4-(1-(3-(3-chloro-4-fluorophényl)-1-méthyluréido)éthyl)isoquinoléine-1-carboxamide ;
de la (R)-3-(3-chloro-4-fluorophényl)-1-(1-(7,8-difluoro-1-oxo-1,2-dihydroisoquinoléin-4-yl)éthyl)-1-méthylurée ;
(S)-3-(3-chloro-4-fluorophényl)-1-(1-(7,8-difluoro-1-oxo-1,2-dihydroisoquinoléin-4-yl)éthyl)-1-méthylurée ;
(R)-3-(3-chloro-4-fluorophényl)-1-(1-(7,8-difluoro-2-méthyl-1-oxo-1,2-dihydroisoquinoléin-4-yl)éthyl)-1-méthylurée ;
(S)-3-(3-chloro-4-fluorophényl)-1-(1-(7,8-difluoro-2-méthyl-1-oxo-1,2-dihydroisoquinoléin-4-yl)éthyl)-1-méthylurée ;
(R)-3-(3-chloro-4-fluorophényl)-1-(1-(7,8-difluoro-1-oxo-1,2-dihydroisoquinoléin-4-yl)éthyl)-1-(3-hydroxypropyl)urée ;
(S)-3-(3-chloro-4-fluorophényl)-1-(1-(7,8-difluoro-1-oxo-1,2-dihydroisoquinoléin-4-yl)éthyl)-1-(3-hydroxypropyl)urée ;
(R)-3-(3-chloro-4-fluorophényl)-1-(1-(1-cyanoisoquinoléin-4-yl)éthyl)-1-méthylurée ;
(S)-3-(3-chloro-4-fluorophényl)-1-(1-(1-cyanoisoquinoléin-4-yl)éthyl)-1-méthylurée ;
(R)-3-(3-chloro-4-fluorophényl)-1-(1-(7,8-difluoro-1-oxo-1,2-dihydroisoquinoléin-4-yl)éthyl)-1-isobutylurée ;
(S)-3-(3-chloro-4-fluorophényl)-1-(1-(7,8-difluoro-1-oxo-1,2-dihydroisoquinoléin-4-yl)éthyl)-1-isobutylurée ;
(R)-3-(3-chloro-4-fluorophényl)-1-(1-(6,7-difluoro-2-méthyl-1-oxo-1,2-dihydroisoquinoléin-4-yl)éthyl)-1-méthylurée ;
(S)-3-(3-chloro-4-fluorophényl)-1-(1-(6,7-difluoro-2-méthyl-1-oxo-1,2-dihydroisoquinoléin-4-yl)éthyl)-1-méthylurée ;
(R)-3-(3-chloro-4-fluorophényl)-1-(1-(6,7-difluoro-2-méthyl-1-oxo-1,2-dihydroisoquinoléin-4-yl)éthyl)-1-isobutylurée ;
(S)-3-(3-chloro-4-fluorophényl)-1-(1-(6,7-difluoro-2-méthyl-1-oxo-1,2-dihydroisoquinoléin-4-yl)éthyl)-1-isobutylurée ;
(R)-1-(1-(1-(1H-1,2,4-triazol-1-yl)isoquinoléin-4-yl)éthyl)-3-(3-chloro-4-fluorophényl)-1-isobutylurée ;
(S)-1-(1-(1-(1H-1,2,4-triazol-1-yl)isoquinoléin-4-yl)éthyl)-3-(3-chloro-4-fluorophényl)-1-isobutylurée ;
(R)-3-(3-chloro-4-fluorophényl)-1-(1-(6,7-difluoro-1-((1-méthyl-1H-1,2,4-triazol-3-yl)méthoxy)isoquinoléin-4-yl)éthyl)-1-méthylurée ;
(S)-3-(3-chloro-4-fluorophényl)-1-(1-(6,7-difluoro-1-((1-méthyl-1H-1,2,4-triazol-3-yl)méthoxy)isoquinoléin-4-yl)éthyl)-1-méthylurée ;
(R)-3-(3-chloro-4-fluorophényl)-1-(1-(6,7-difluoro-1-((1-méthyl-1H-1,2,4-triazol-3-yl)méthoxy)isoquinoléin-4-yl)éthyl)-1-(3-hydroxypropyl)urée ;
(S)-3-(3-chloro-4-fluorophényl)-1-(1-(6,7-difluoro-1-((1-méthyl-1H-1,2,4-triazol-3-yl)méthoxy)isoquinoléin-4-yl)éthyl)-1-(3-hydroxypropyl)urée ;
(R)-3-(3-chloro-4-fluorophényl)-1-(1-(6,7-difluoro-2-méthyl-1-oxo-1,2-dihydroisoquinoléin-4-yl)éthyl)-1-(3-hydroxypropyl)urée ;
(S)-3-(3-chloro-4-fluorophényl)-1-(1-(6,7-difluoro-2-méthyl-1-oxo-1,2-dihydroisoquinoléin-4-yl)éthyl)-1-(3-hydroxypropyl)urée ;
(R)-3-(3-chloro-4-fluorophényl)-1-méthyl-1-(1-(1-méthylisoquinoléin-4-yl)éthyl)urée ;
(S)-3-(3-chloro-4-fluorophényl)-1-méthyl-1-(1-(1-méthylisoquinoléin-4-yl)éthyl)urée ;
(R)-3-(3-chloro-4-fluorophényl)-1-(1-(8-fluoro-1-oxo-1,2-dihydroisoquinoléin-4-yl)éthyl)-1-méthylurée ;
(S)-3-(3-chloro-4-fluorophényl)-1-(1-(8-fluoro-1-oxo-1,2-dihydroisoquinoléin-4-yl)éthyl)-1-méthylurée ;
(R)-3-(3-chloro-4-fluorophényl)-1-(1-(8-fluoro-2-méthyl-1-oxo-1,2-dihydroisoquinoléin-4-yl)éthyl)-1-méthylurée ;
(S)-3-(3-chloro-4-fluorophényl)-1-(1-(8-fluoro-2-méthyl-1-oxo-1,2-dihydroisoquinoléin-4-yl)éthyl)-1-méthylurée ;
(R)-3-(3-chloro-4-fluorophényl)-1-(1-(8-fluoro-1-oxo-1,2-dihydroisoquinoléin-4-yl)éthyl)-1-isobutylurée ;
(S)-3-(3-chloro-4-fluorophényl)-1-(1-(8-fluoro-1-oxo-1,2-dihydroisoquinoléin-4-yl)éthyl)-1-isobutylurée ;
(R) -1-(1-(1-((1H-1,2,4-triazol-3-yl)méthoxy)-6,7-difluoroisoquinoléin-4-yl)éthyl)-3-(3-chloro-4-fluorophényl)-1-méthylurée ;
(S)-1-(1-(1-((1H-1,2,4-triazol-3-yl)méthoxy)-6,7-difluoroisoquinoléin-4-yl)éthyl)-3-(3-chloro-4-fluorophényl)-1-méthylurée ;
(R) -1-(1-(1-((1H-1,2,4-triazol-3-yl)méthoxy)-6,7-difluoroisoquinoléin-4-yl)éthyl)-3-(3-chloro-4-fluorophényl)-1-(3-hydroxypropyl)urée ;
(S)-1-(1-(1-((1H-1,2,4-triazol-3-yl)méthoxy)-6,7-difluoroisoquinoléin-4-yl)éthyl)-3-(3-chloro-4-fluorophényl)-1-(3-hydroxypropyl)urée ;
(R)-3-(3-chloro-4-fluorophényl)-1-(1-(8-fluoro-1-oxo-1,2-dihydroisoquinoléin-4-yl)éthyl)-1-(3-hydroxypropyl)urée ;
(S)-3-(3-chloro-4-fluorophényl)-1-(1-(8-fluoro-1-oxo-1,2-dihydroisoquinoléin-4-yl)éthyl)-1-(3-hydroxypropyl)urée ;
(R)-3-(3-chloro-4-fluorophényl)-1-(1-(6,8-difluoro-1-oxo-1,2-dihydroisoquinoléin-4-yl)éthyl)-1-méthylurée ;
(S)-3-(3-chloro-4-fluorophényl)-1-(1-(6,8-difluoro-1-oxo-1,2-dihydroisoquinoléin-4-yl)éthyl)-1-méthylurée ;
(R)-1-(1-(6,8-difluoro-1-oxo-1,2-dihydroisoquinoléin-4-yl)éthyl)-3-(4-fluorophényl)-1-méthylurée ;
(S)-1-(1-(6,8-difluoro-1-oxo-1,2-dihydroisoquinoléin-4-yl)éthyl)-3-(4-fluorophényl)-1-méthylurée ;
(R)-3-(3-chloro-4-fluorophényl)-1-(1-(6,8-difluoro-1-oxo-1,2-dihydroisoquinoléin-4-yl)éthyl)-1-(3-hydroxypropyl)urée ;
(S)-3-(3-chloro-4-fluorophényl)-1-(1-(6,8-difluoro-1-oxo-1,2-dihydroisoquinoléin-4-yl)éthyl)-1-(3-hydroxypropyl)urée ;
(R)-1-(1-(6,8-difluoro-1-oxo-1,2-dihydroisoquinoléin-4-yl)éthyl)-3-(4-fluorophényl)-1-isobutylurée ;
(S)-1-(1-(6,8-difluoro-1-oxo-1,2-dihydroisoquinoléin-4-yl)éthyl)-3-(4-fluorophényl)-1-isobutylurée ;
(R)-1-(1-(6,8-difluoro-1-oxo-1,2-dihydroisoquinoléin-4-yl)éthyl)-3-(4-fluorophényl)-1-(3-hydroxypropyl)urée ;
(S)-1-(1-(6,8-difluoro-1-oxo-1,2-dihydroisoquinoléin-4-yl)éthyl)-3-(4-fluorophényl)-1-(3-hydroxypropyl)urée ;
(R)-3-(3-chloro-4-fluorophényl)-1-(2-hydroxy-1-(1-oxo-1,2-dihydroisoquinoléin-4-yl)éthyl)-1-méthylurée ;
(S)-3-(3-chloro-4-fluorophényl)-1-(2-hydroxy-1-(1-oxo-1,2-dihydroisoquinoléin-4-yl)éthyl)-1-méthylurée ;
du (R)-2-(3-(3-chloro-4-fluorophényl)-1-(1-(6-fluoro-1-oxo-1,2-dihydroisoquinoléin-4-yl)éthyl)uréido)éthane-1-sulfonamide ;
(S)-2-(3-(3-chloro-4-fluorophényl)-1-(1-(6-fluoro-1-oxo-1,2-dihydroisoquinoléin-4-yl)éthyl)uréido)éthane-1-sulfonamide ;
(R)-2-(3-(3-chloro-4-fluorophényl)-1-(1-(7-fluoro-1-oxo-1,2-dihydroisoquinoléin-4-yl)éthyl)uréido)éthane-1-sulfonamide ;
(S)-2-(3-(3-chloro-4-fluorophényl)-1-(1-(7-fluoro-1-oxo-1,2-dihydroisoquinoléin-4-yl)éthyl)uréido)éthane-1-sulfonamide ;
(R)-2-(3-(3-chloro-4-fluorophényl)-1-(1-(6,7-difluoro-1-oxo-1,2-dihydroisoquinoléin-4-yl)éthyl)uréido)éthane-1-sulfonamide ;
(S)-2-(3-(3-chloro-4-fluorophényl)-1-(1-(6,7-difluoro-1-oxo-1,2-dihydroisoquinoléin-4-yl)éthyl)uréido)éthane-1-sulfonamide ;
(R)-2-(1-(1-(6,7-difluoro-1-oxo-1,2-dihydroisoquinoléin-4-yl)éthyl)-3-(4-fluorophényl)uréido)éthane-1-sulfonamide ;
(S)-2-(1-(1-(6,7-difluoro-1-oxo-1,2-dihydroisoquinoléin-4-yl)éthyl)-3-(4-fluorophényl)uréido)éthane-1-sulfonamide ;
(R)-2-(3-(3-chloro-4-fluorophényl)-1-(1-(6,8-difluoro-1-oxo-1,2-dihydroisoquinoléin-4-yl)éthyl)uréido)éthane-1-sulfonamide ;
(S)-2-(3-(3-chloro-4-fluorophényl)-1-(1-(6,8-difluoro-1-oxo-1,2-dihydroisoquinoléin-4-yl)éthyl)uréido)éthane-1-sulfonamide ;
(R)-2-(3-(3-chloro-4-fluorophényl)-1-(1-(7,8-difluoro-1-oxo-1,2-dihydroisoquinoléin-4-yl)éthyl)uréido)éthane-1-sulfonamide ;
(S)-2-(3-(3-chloro-4-fluorophényl)-1-(1-(7,8-difluoro-1-oxo-1,2-dihydroisoquinoléin-4-yl)éthyl)uréido)éthane-1-sulfonamide ;
(R)-2-(3-(4-fluorophényl)-1-(1-(7,8-difluoro-1-oxo-1,2-dihydroisoquinoléin-4-yl)éthyl)uréido)éthane-1-sulfonamide ;
(S)-2-(3-(4-fluorophényl)-1-(1-(7,8-difluoro-1-oxo-1,2-dihydroisoquinoléin-4-yl)éthyl)uréido)éthane-1-sulfonamide ;
(R)-2-(3-(3-chloro-4-fluorophényl)-1-(1-(8-fluoro-1-oxo-1,2-dihydroisoquinoléin-4-yl)éthyl)uréido)éthane-1-sulfonamide ;
(S)-2-(3-(3-chloro-4-fluorophényl)-1-(1-(8-fluoro-1-oxo-1,2-dihydroisoquinoléin-4-yl)éthyl)uréido)éthane-1-sulfonamide ;
de la (R)-1-(1-(6,7-difluoro-1-oxo-1,2-dihydroisoquinoléin-4-yl)éthyl)-1-méthyl-3-phénylurée ;
(S)-1-(1-(6,7-difluoro-1-oxo-1,2-dihydroisoquinoléin-4-yl)éthyl)-1-méthyl-3-phénylurée ;
(R)-3-(4-chlorophényl)-1-(1-(6,7-difluoro-1-oxo-1,2-dihydroisoquinoléin-4-yl)éthyl)-1-méthylurée ;
(S)-3-(4-chlorophényl)-1-(1-(6,7-difluoro-1-oxo-1,2-dihydroisoquinoléin-4-yl)éthyl)-1-méthylurée ;
(R)-1-(1-(6,7-difluoro-1-oxo-1,2-dihydroisoquinoléin-4-yl)éthyl)-1-éthyl-3-(4-fluorophényl)urée ;
(S)-1-(1-(6,7-difluoro-1-oxo-1,2-dihydroisoquinoléin-4-yl)éthyl)-1-éthyl-3-(4-fluorophényl)urée ;
(R)-1-(1-(6,7-difluoro-1-oxo-1,2-dihydroisoquinoléin-4-yl)éthyl)-1-éthyl-3-phénylurée ;
(S)-1-(1-(6,7-difluoro-1-oxo-1,2-dihydroisoquinoléin-4-yl)éthyl)-1-éthyl-3-phénylurée ;
(R)-3-(3-chloro-4-fluorophényl)-1-(1-(6,7-difluoro-1-oxo-1,2-dihydroisoquinoléin-4-yl)éthyl)-1-éthylurée ;
(S)-3-(3-chloro-4-fluorophényl)-1-(1-(6,7-difluoro-1-oxo-1,2-dihydroisoquinoléin-4-yl)éthyl)-1-éthylurée ;
(R)-1-(1-(6,7-difluoro-1-oxo-1,2-dihydroisoquinoléin-4-yl)éthyl)-3-(3,4-difluorophényl)-1-méthylurée ;
(S)-1-(1-(6,7-difluoro-1-oxo-1,2-dihydroisoquinoléin-4-yl)éthyl)-3-(3,4-difluorophényl)-1-méthylurée ;
(R)-1-(1-(6,7-difluoro-1-oxo-1,2-dihydroisoquinoléin-4-yl)éthyl)-3-(3-fluorophényl)-1-méthylurée ;
(S)-1-(1-(6,7-difluoro-1-oxo-1,2-dihydroisoquinoléin-4-yl)éthyl)-3-(3-fluorophényl)-1-méthylurée ;
(R)-3-(3-chlorophényl)-1-(1-(6,7-difluoro-1-oxo-1,2-dihydroisoquinoléin-4-yl)éthyl)-1-méthylurée ;
(S)-3-(3-chlorophényl)-1-(1-(6,7-difluoro-1-oxo-1,2-dihydroisoquinoléin-4-yl)éthyl)-1-méthylurée ;
(R)-1-(1-(6,7-difluoro-1-oxo-1,2-dihydroisoquinoléin-4-yl)éthyl)-1-méthyl-3-(3,4,5-trifluorophényl)urée ;
(S)-1-(1-(6,7-difluoro-1-oxo-1,2-dihydroisoquinoléin-4-yl)éthyl)-1-méthyl-3-(3,4,5-trifluorophényl)urée ;
(R)-3-(3,5-dichlorophényl)-1-(1-(6,7-difluoro-1-oxo-1,2-dihydroisoquinoléin-4-yl)éthyl)-1-méthylurée ;
(S)-3-(3,5-dichlorophényl)-1-(1-(6,7-difluoro-1-oxo-1,2-dihydroisoquinoléin-4-yl)éthyl)-1-méthylurée ;
(R)-3-(2-chloropyridin-4-yl)-1-(1-(6,7-difluoro-1-oxo-1,2-dihydroisoquinoléin-4-yl)éthyl)-1-méthylurée ;
(S)-3-(2-chloropyridin-4-yl)-1-(1-(6,7-difluoro-1-oxo-1,2-dihydroisoquinoléin-4-yl)éthyl)-1-méthylurée ;
(R)-1-(1-(6,7-difluoro-1-oxo-1,2-dihydroisoquinoléin-4-yl)éthyl)-3-(2-fluorophényl)-1-méthylurée ;
(S)-1-(1-(6,7-difluoro-1-oxo-1,2-dihydroisoquinoléin-4-yl)éthyl)-3-(2-fluorophényl)-1-méthylurée ;
(R)-1-(1-(6,7-difluoro-1-oxo-1,2-dihydroisoquinoléin-4-yl)éthyl)-3-(3,4-difluorophényl)-1-éthylurée ;
(S)-1-(1-(6,7-difluoro-1-oxo-1,2-dihydroisoquinoléin-4-yl)éthyl)-3-(3,4-difluorophényl)-1-éthylurée ;
(R)-1-(1-(1-(1H-1,2,3-triazol-1-yl)isoquinoléin-4-yl)éthyl)-3-(3-chloro-4-fluorophényl)-1-méthylurée ;
(S)-1-(1-(1-(1H-1,2,3-triazol-1-yl)isoquinoléin-4-yl)éthyl)-3-(3-chloro-4-fluorophényl)-1-méthylurée ;
(R)-1-(1-(6,7-difluoro-1-oxo-1,2-dihydroisoquinoléin-4-yl)éthyl)-3-(4-fluoro-3-méthylphényl)-1-méthylurée ;
(S)-1-(1-(6,7-difluoro-1-oxo-1,2-dihydroisoquinoléin-4-yl)éthyl)-3-(4-fluoro-3-méthylphényl)-1-méthylurée ;
(R)-3-(3-cyano-4-fluorophényl)-1-(1-(6,7-difluoro-1-oxo-1,2-dihydroisoquinoléin-4-yl)éthyl)-1-méthylurée ;
(S)-3-(3-cyano-4-fluorophényl)-1-(1-(6,7-difluoro-1-oxo-1,2-dihydroisoquinoléin-4-yl)éthyl)-1-méthylurée ;
(R)-1-(1-(6,7-difluoro-1-oxo-1,2-dihydroisoquinoléin-4-yl)éthyl)-3-(2,3-difluorophényl)-1-méthylurée ;
(S)-1-(1-(6,7-difluoro-1-oxo-1,2-dihydroisoquinoléin-4-yl)éthyl)-3-(2,3-difluorophényl)-1-méthylurée ;
(R)-1-(1-(7,8-difluoro-1-oxo-1,2-dihydroisoquinoléin-4-yl)éthyl)-3-(4-fluorophényl)-1-méthylurée ;
(S)-1-(1-(7,8-difluoro-1-oxo-1,2-dihydroisoquinoléin-4-yl)éthyl)-3-(4-fluorophényl)-1-méthylurée ;
(R)-1-(1-(7,8-difluoro-1-oxo-1,2-dihydroisoquinoléin-4-yl)éthyl)-3-(3,4-difluorophényl)-1-méthylurée ;
(S)-1-(1-(7,8-difluoro-1-oxo-1,2-dihydroisoquinoléin-4-yl)éthyl)-3-(3,4-difluorophényl)-1-méthylurée ;
(R)-1-(1-(7,8-difluoro-1-oxo-1,2-dihydroisoquinoléin-4-yl)éthyl)-1-méthyl-3-phénylurée ;
(S)-1-(1-(7,8-difluoro-1-oxo-1,2-dihydroisoquinoléin-4-yl)éthyl)-1-méthyl-3-phénylurée ;
(R)-3-(3,5-dichloro-4-fluorophényl)-1-(1-(6,7-difluoro-1-oxo-1,2-dihydroisoquinoléin-4-yl)éthyl)-1-méthylurée ;
(S)-3-(3,5-dichloro-4-fluorophényl)-1-(1-(6,7-difluoro-1-oxo-1,2-dihydroisoquinoléin-4-yl)éthyl)-1-méthylurée ;
(R)-1-(1-(7,8-difluoro-1-oxo-1,2-dihydroisoquinoléin-4-yl)éthyl)-3-(4-fluoro-3-méthylphényl)-1-méthylurée ;
(S)-1-(1-(7,8-difluoro-1-oxo-1,2-dihydroisoquinoléin-4-yl)éthyl)-3-(4-fluoro-3-méthylphényl)-1-méthylurée ;
(R)-1-(1-(7,8-difluoro-1-oxo-1,2-dihydroisoquinoléin-4-yl)éthyl)-1-méthyl-3-(3,4,5-trifluorophényl)urée ;
(S)-1-(1-(7,8-difluoro-1-oxo-1,2-dihydroisoquinoléin-4-yl)éthyl)-1-méthyl-3-(3,4,5-trifluorophényl)urée ;
(R)-3-(4-chlorophényl)-1-(1-(7,8-difluoro-1-oxo-1,2-dihydroisoquinoléin-4-yl)éthyl)-1-méthylurée ;
(S)-3-(4-chlorophényl)-1-(1-(7,8-difluoro-1-oxo-1,2-dihydroisoquinoléin-4-yl)éthyl)-1-méthylurée ;
(R)-3-(3-chloro-4-fluorophényl)-1-(1-(6,8-difluoro-1-oxo-1,2-dihydroisoquinoléin-4-yl)éthyl)-1-isobutylurée ;
(S)-3-(3-chloro-4-fluorophényl)-1-(1-(6,8-difluoro-1-oxo-1,2-dihydroisoquinoléin-4-yl)éthyl)-1-isobutylurée ;
(R)-3-(3,5-dichlorophényl)-1-(1-(7,8-difluoro-1-oxo-1,2-dihydroisoquinoléin-4-yl)éthyl)-1-méthylurée ;
(S)-3-(3,5-dichlorophényl)-1-(1-(7,8-difluoro-1-oxo-1,2-dihydroisoquinoléin-4-yl)éthyl)-1-méthylurée ;
(R)-3-(3,5-dichloro-4-fluorophényl)-1-(1-(7,8-difluoro-1-oxo-1,2-dihydroisoquinoléin-4-yl)éthyl)-1-méthylurée ;
(S)-3-(3,5-dichloro-4-fluorophényl)-1-(1-(7,8-difluoro-1-oxo-1,2-dihydroisoquinoléin-4-yl)éthyl)-1-méthylurée ;
(R)-1-(1-(7,8-difluoro-1-oxo-1,2-dihydroisoquinoléin-4-yl)éthyl)-3-(3-fluorophényl)-1-méthylurée ;
(S)-1-(1-(7,8-difluoro-1-oxo-1,2-dihydroisoquinoléin-4-yl)éthyl)-3-(3-fluorophényl)-1-méthylurée ;
(R)-1-(1-(7,8-difluoro-1-oxo-1,2-dihydroisoquinoléin-4-yl)éthyl)-3-(2,3-difluorophényl)-1-méthylurée ;
(S)-1-(1-(7,8-difluoro-1-oxo-1,2-dihydroisoquinoléin-4-yl)éthyl)-3-(2,3-difluorophényl)-1-méthylurée ;
(R)-1-(1-(7,8-difluoro-1-oxo-1,2-dihydroisoquinoléin-4-yl)éthyl)-3-(3-chlorophényl)-1-méthylurée ;
(S)-1-(1-(7,8-difluoro-1-oxo-1,2-dihydroisoquinoléin-4-yl)éthyl)-3-(3-chlorophényl)-1-méthylurée ;
(R)-1-(1-(7,8-difluoro-1-oxo-1,2-dihydroisoquinoléin-4-yl)éthyl)-3-(3,4-difluorophényl)-1-éthylurée ;
(S)-1-(1-(7,8-difluoro-1-oxo-1,2-dihydroisoquinoléin-4-yl)éthyl)-3-(3,4-difluorophényl)-1-éthylurée ;
(R)-1-(1-(7,8-difluoro-1-oxo-1,2-dihydroisoquinoléin-4-yl)éthyl)-3-(4-fluorophényl)-1-éthylurée ;
(S)-1-(1-(7,8-difluoro-1-oxo-1,2-dihydroisoquinoléin-4-yl)éthyl)-3-(4-fluorophényl)-1-éthylurée ;
(R)-1-(1-(7,8-difluoro-1-oxo-1,2-dihydroisoquinoléin-4-yl)éthyl)-3-phényle-1-éthylurée ;
(S)-1-(1-(7,8-difluoro-1-oxo-1,2-dihydroisoquinoléin-4-yl)éthyl)-3-phényle-1-éthylurée ;
(R)-1-(1-(7,8-difluoro-1-oxo-1,2-dihydroisoquinoléin-4-yl)éthyl)-3-(3-chloro-4-fluorophényl)-1-éthylurée ;
(S)-1-(1-(7,8-difluoro-1-oxo-1,2-dihydroisoquinoléin-4-yl)éthyl)-3-(3-chloro-4-fluorophényl)-1-éthylurée ;
(R)-3-(3-cyano-4-fluorophényl)-1-(1-(7,8-difluoro-1-oxo-1,2-dihydroisoquinoléin-4-yl)éthyl)-1-méthylurée ;
(S)-3-(3-cyano-4-fluorophényl)-1-(1-(7,8-difluoro-1-oxo-1,2-dihydroisoquinoléin-4-yl)éthyl)-1-méthylurée ;
(R)-1-(1-(6,7-difluoro-1-oxo-1,2-dihydroisoquinoléin-4-yl)éthyl)-3-(3,4-difluorobenzyl)-1-méthylurée ;
(S)-1-(1-(6,7-difluoro-1-oxo-1,2-dihydroisoquinoléin-4-yl)éthyl)-3-(3,4-difluorobenzyl)-1-méthylurée ;
(R)-1-(1-(6,7-difluoro-1-oxo-1,2-dihydroisoquinoléin-4-yl)éthyl)-1-méthyl-3-(3,4,5-trifluorobenzyl)urée ;
(S)₁-(1-(6,7-difluoro-1-oxo-1,2-dihydroisoquinoléin-4-yl)éthyl)-1-méthyl-3-(3,4,5-trifluorobenzyl)urée ;
(R)-1-(1-(6,7-difluoro-1-oxo-1,2-dihydroisoquinoléin-4-yl)éthyl)-1-méthyl-3-(3-chloro-4-fluorobenzyl)urée ;
(S)-1-(1-(6,7-difluoro-1-oxo-1,2-dihydroisoquinoléin-4-yl)éthyl)-1-méthyl-3-(3-chloro-4-fluorobenzyl)urée ;
(R)-1-(1-(6,7-difluoro-1-oxo-1,2-dihydroisoquinoléin-4-yl)éthyl)-3-(1H-indol-6-yl)-1-méthylurée ;
(S)-1-(1-(6,7-difluoro-1-oxo-1,2-dihydroisoquinoléin-4-yl)éthyl)-3-(1H-indol-6-yl)-1-méthylurée ;
(R)-1-(1-(7,8-difluoro-1-oxo-1,2-dihydroisoquinoléin-4-yl)éthyl)-3-(3,4-difluorobenzyl)-1-méthylurée ;
(S)-1-(1-(7,8-difluoro-1-oxo-1,2-dihydroisoquinoléin-4-yl)éthyl)-3-(3,4-difluorobenzyl)-1-méthylurée ;
(R)-1-(1-(7,8-difluoro-1-oxo-1,2-dihydroisoquinoléin-4-yl)éthyl)-3-(4-fluorobenzyl)-1-méthylurée ;
(S)-1-(1-(7,8-difluoro-1-oxo-1,2-dihydroisoquinoléin-4-yl)éthyl)-3-(4-fluorobenzyl)-1-méthylurée ;
(R)-1-(1-(7,8-difluoro-1-oxo-1,2-dihydroisoquinoléin-4-yl)éthyl)-3-(3,4,5-trifluorobenzyl)-1-méthylurée ;
(S)-1-(1-(7,8-difluoro-1-oxo-1,2-dihydroisoquinoléin-4-yl)éthyl)-3-(3,4,5-trifluorobenzyl)-1-méthylurée ;
(R)-3-(3-chloro-4-fluorobenzyl)-1-(1-(7,8-difluoro-1-oxo-1,2-dihydroisoquinoléin-4-yl)éthyl)-1-méthylurée ;
(S)-3-(3-chloro-4-fluorobenzyl)-1-(1-(7,8-difluoro-1-oxo-1,2-dihydroisoquinoléin-4-yl)éthyl)-1-méthylurée ;
(R)-1-(1-(7,8-difluoro-1-oxo-1,2-dihydroisoquinoléin-4-yl)éthyl)-3-(1H-indol-6-yl)-1-méthylurée ;
(S)-1-(1-(7,8-difluoro-1-oxo-1,2-dihydroisoquinoléin-4-yl)éthyl)-3-(1H-indol-6-yl)-1-méthylurée ;
(R)-1-(1-(6,7-difluoro-1-oxo-1,2-dihydroisoquinoléin-4-yl)éthyl)-3-(3-(difluorométhyl)-4-fluorophényl)-1-méthylurée ;
(S)-1-(1-(6,7-difluoro-1-oxo-1,2-dihydroisoquinoléin-4-yl)éthyl)-3-(3-(difluorométhyl)-4-fluorophényl)-1-méthylurée ;
(R)-1-(1-(7,8-difluoro-1-oxo-1,2-dihydroisoquinoléin-4-yl)éthyl)-3-(3-(difluorométhyl)-4-fluorophényl)-1-méthylurée ; et
de la (S)-1-(1-(7,8-difluoro-1-oxo-1,2-dihydroisoquinoléin-4-yl)éthyl)-3-(3-(difluorométhyl)-4-fluorophényl)-1-méthylurée ;
ou son sel, solvate, dérivé isotopiquement marqué, stéréoisomère, ou tautomère, ou de n'importe quels mélanges de ceux-ci.

12. Composé, qui est sélectionné dans le groupe constitué de la :
1-(4-fluoro-3-methylphenyl)-3-((1-oxo-1,2-dihydroisoquinoléin-4-yl)methyl)urée;
1-(3-chloro-4-fluorophényl)-3-((1-methoxyisoquinoléin-4-yl)methyl)urée;
1-(4-fluoro-3-methylphenyl)-3-((1-methoxyisoquinoléin-4-yl)methyl)urée;
1-(3-chloro-4-fluorophényl)-3-((1-oxo-1,2-dihydroisoquinoléin-4-yl)methyl)urée;
1-(3-chloro-4-fluorophényl)-3-(1-(1-oxo-1,2-dihydroisoquinoléin-4-yl)ethyl)urée;
1-(3-chloro-4-fluorophényl)-3-(3-hydroxy-1-(1-methoxyisoquinoléin-4-yl)propyl)urée;
1-(3-chloro-4-fluorophényl)-3-(1-(6,7-difluoro-1-oxo-1,2-dihydroisoquinoléin-4-yl)ethyl)urée;
1-(3-cyano-4-fluorophényl)-3-(1-(6,7-difluoro-1-oxo-1,2-dihydroisoquinoléin-4-yl)ethyl)urée; et
1-(3-chloro-4-fluorophényl)-3-(1-(7,8-difluoro-1-oxo-1,2-dihydroisoquinoléin-4-yl)ethyl)urée;
ou son sel, solvate, dérivé isotopiquement marqué, stéréoisomère, ou tautomère, ou de n'importe quels mélanges de ceux-ci.

13. Composition pharmaceutique comprenant au moins un composé selon l'une quelconque des revendications 1 à 12 et un support pharmaceutiquement acceptable.

14. Au moins un composé selon l'une quelconque des revendications 1 à 12 pour l'utilisation dans le traitement ou la prévention de l'infection par le virus de l'hépatite B (VHB) chez un sujet, ou d'inhibition de l'expression et/ou d'une fonction d'une protéine de capside virale directement ou indirectement chez un sujet infecté du virus de l'hépatite B.

15. Au moins un composé selon l'une quelconque des revendications 1 à 12 pour l'utilisation selon la revendication 14, le sujet étant en outre infecté par un virus de l'hépatite D (VHD).

16. Composition pharmaceutique selon la revendication 13 ou ledit composé selon l'une quelconque des revendications 1 à 12 pour l'utilisation selon la revendication 14, comprenant en outre au moins un agent additionnel utile pour traiter l'infection virale hépatite B, préférablement co-formulé ou co-administré au sujet, ledit agent additionnel comprenant au moins un agent sélectionné dans le groupe constitué d'un inhibiteur de transcriptase inverse, inhibiteur de capside, inhibiteur de formation d'ADNccc, agent de déstabilisation de l'ARN, nucléotides oligomères ciblés contre le génome du VHB, des immunostimulateurs, d'un conjugué GalNAc-siRNA ciblé contre un produit de transcription de gène de VHB.

17. Au moins un composé selon l'une quelconque des revendications 1 à 12 pour l'utilisation selon la revendication 14, le sujet étant un mammifère, préférablement un être humain.
